# EUROPEAN PATENT APPLICATION

(11) **EP 1 806 413 A1**
(43) Date of publication of application: **11.07.2007**
(21) Application number: 06000275.5
(22) Date of filing: 06.01.2006
(51) Int. Cl.: C12Q 1/68

(54) **An in-vitro-method and means for determination of different tumor types and predicting success of surgical procedures in ovarian cancer**

(71) Applicant: Oligene GmbH, 10178 Berlin (DE)
(72) Inventor: Budczies, Jan, 10437 Berlin (DE); Dietel, Manfred, 14193 Berlin (DE); Denkert, Carsten, 10405 Berlin (DE); Lage, Hermann, 13465 Berlin (DE)
(74) Representative: Engelhard, Markus

(57) **Abstract**

The present invention relates to a composition of nucleic acids useful as markers for the response to a multimodal therapeutic strategy in ovarian cancer, to a composition of nucleic acids useful as markers for different tumor types of ovarian carcinoma and useful as markers for predicting the outcome of a surgical procedure in ovarian cancer and assessment of patient prognosis. The present invention also relates to a matrix on which such nucleic acid compositions have been immobilized. Furthermore, the present invention relates to uses of such nucleic acid compositions. Moreover, the present invention relates to compositions of proteins encoded by said nucleic acid compositions, and also relates to kits comprising such nucleic acid compositions, matrices and/or protein compositions. Furthermore, the present invention relates to a method of classifying a patient with ovarian cancer as belonging to a group amenable or not amenable to surgical procedure, and to a method of predicting the outcome of such surgical procedure.

SEQUENCE LISTING

<110>  oligene GmbH

<120>  An in-vitro-method and means for determination of different tumor types and
       predicting success of surgical procedures in ovarian cancer

<130>  FB16827

<160>  368

<170>  PatentIn version 3.3

<210>  1
<211>  1879
<212>  DNA
<213>  Homo sapiens

<400>  1
   gccagcaagt gtggctgagc tccggggtgt gtggacgccg ctttgttgcc tgaggtgggt      60
   ggcggtggaa gttaagggag tcaggggcta tcgctcctcg agactcgcag tcgcggccac     120
   tgcagtcact tcgccagtta gcccttaggg taggagtcgc gccggcagca gccatgagcg     180
   gcggcgtgta cgggggagat gaagttggag cccttgtttt tgacattgga tcctatactg     240
   tgagagctgg ttatgctggt gaggactgcc ccaaggtgga ttttcctaca gctattggta     300
   tggtggtaga aagagatgac ggaagcacat taatggaaat agatggcgat aaaggcaaac     360
   aaggcggtcc cacctactac atagatacta atgctctgcg tgttccgagg gagaatatgg     420
   aggccatttc acctctaaaa aatgggatgg ttgaagactg ggatagtttc caagctattt     480
   tggatcatac ctacaaaatg catgtcaaat cagaagccag tctccatcct gttctcatgt     540
   cagaggcacc gtggaatact agagcaaaga gagagaaact gacagagtta atgtttgaac     600
   actacaacat ccctgccttc ttcctttgca aaactgcagt tttgacagca tttgctaatg     660
   gtcgttctac tgggctgatt ttggacagtg gagccactca taccactgca attccagtcc     720
   acgatggcta tgtccttcaa caaggcattg tgaaatcccc tcttgctgga gactttatta     780
   ctatgcagtg cagagaactc ttccaagaaa tgaatattga attggttcct ccatatatga     840
   ttgcatcaaa agaagctgtt cgtgaaggat ctccagcaaa ctggaaaaga aaagagaagt     900
   tgcctcaggt tacgaggtct tggcacaatt atatgtgtaa ttgtgttatc caggattttc     960
   aagcttcggt acttcaagtg tcagattcaa cttatgatga acaagtggct gcacagatgc    1020
   caactgttca ttatgaattc cccaatggct acaattgtga ttttggtgca gagcggctaa    1080
   agattccaga aggattattt gacccttcca atgtaaaggg gttatcagga aacacaatgt    1140
   taggagtcag tcatgttgtc accacaagtg ttgggatgtg tgatattgat atcagaccag    1200
   gtctctatgg cagtgtaata gtggcaggag gaaacacact aatacagagt tttactgaca    1260
   ggttgaatag agagctgtct cagaaaactc ctccaagtat gcggttgaaa ttgattgcaa    1320
   ataatacaac agtggaacgg aggtttagct catggattgg cggctccatt ctagcctctt    1380
   tgggtacctt tcaacagatg tggatttcca agcaagaata tgaagaagga gggaagcagt    1440
   gtgtagaaag aaaatgccct tgagaaagag ttcccaagct tctaccttcc ttttgtcacc    1500
   ttacgtttca tagctttagt atactcagga aaagaatgac catcttttgt agaatgttta    1560
   tacatttttg catatttcaa tttccactta aattttttaa agctttaact ggctctataa 1620
   attaagtttg tgctttcctt gaaatgcact tattcttatt acaagcattt tataattttg 1680
   tataaatgtc tattttctct aaatattttg ctttcagtaa aatgctttcc aactctgttt 1740
   agtgtattaa ttaccagtgg attggtagaa ctgcttttta ttgactagta aaagttactg 1800
   cctatgcttt ttaccttagg cttacagaat taaataaaaa ttagccattc cagaaatata 1860
   aaaaaaaaaa aaaaaaaaa 1879

<210> 2
<211> 1737
<212> DNA
<213> Homo sapiens

<400> 2
gccagcaagt gtggctgagc tccggggtgt gtggacgccg ctttgttgcc tgagatgaag 60

ttggagccct tgtttttgac attggatcct atactgtgag agctggttat gctggtgagg 120

actgccccaa ggtggatttt cctacagcta ttggtatggt ggtagaaaga gatgacggaa 180

gcacattaat ggaaatagat ggcgataaag gcaaacaagg cggtcccacc tactacatag 240

atactaatgc tctgcgtgtt ccgagggaga atatggaggc catttcacct ctaaaaaatg 300

ggatggttga agactgggat agtttccaag ctattttgga tcatacctac aaaatgcatg 360

tcaaatcaga agccagtctc catcctgttc tcatgtcaga ggcaccgtgg aatactagag 420

caaagagaga gaaactgaca gagttaatgt ttgaacacta caacatccct gccttcttcc 480

tttgcaaaac tgcagttttg acagcatttg ctaatggtcg ttctactggg ctgattttgg 540

acagtggagc cactcatacc actgcaattc cagtccacga tggctatgtc cttcaacaag 600

gcattgtgaa atcccctctt gctggagact ttattactat gcagtgcaga gaactcttcc 660

aagaaatgaa tattgaattg gttcctccat atatgattgc atcaaaagaa gctgttcgtg 720

aaggatctcc agcaaactgg aaaagaaaag agaagttgcc tcaggttacg aggtcttggc 780

acaattatat gtgtaattgt gttatccagg attttcaagc ttcggtactt caagtgtcag 840

attcaactta tgatgaacaa gtggctgcac agatgccaac tgttcattat gaattcccca 900

atggctacaa ttgtgatttt ggtgcagagc ggctaaagat tccagaagga ttatttgacc 960

cttccaatgt aaaggggtta tcaggaaaca caatgttagg agtcagtcat gttgtcacca 1020

caagtgttgg gatgtgtgat attgatatca gaccaggtct ctatggcagt gtaatagtgg 1080

caggaggaaa cacactaata cagagtttta ctgacaggtt gaatagagag ctgtctcaga 1140

aaactcctcc aagtatgcgg ttgaaattga ttgcaaataa tacaacagtg gaacggaggt 1200

ttagctcatg gattggcggc tccattctag cctctttggg tacctttcaa cagatgtgga 1260

tttccaagca agaatatgaa gaaggaggga agcagtgtgt agaaagaaaa tgcccttgag 1320

aaagagttcc caagcttcta ccttcctttt gtcaccttac gtttcatagc tttagtatac 1380

tcaggaaaag aatgaccatc ttttgtagaa tgtttataca tttttgcata tttcaatttc 1440

cacttaaatt ttttaaagct ttaactggct ctataaatta agtttgtgct ttccttgaaa 1500

tgcacttatt cttattacaa gcattttata attttgtata aatgtctatt ttctctaaat 1560

attttgcttt cagtaaaatg ctttccaact ctgtttagtg tattaattac cagtggattg 1620

gtagaactgc tttttattga ctagtaaaag ttactgccta tgctttttac cttaggctta 1680

cagaattaaa taaaaattag ccattccaga aatataaaaa aaaaaaaaaa aaaaaaa 1737

<210> 3
<211> 1925
<212> DNA
<213> Homo sapiens

<400> 3
   gccagcaagt gtggctgagc tccggggtgt gtggacgccg ctttgttgcc tgaggtgggt 60
   ggcggtggaa gttaagggag tcaggggcta tcgctcctcg agactcgcag tcgcggccac 120
   tgcagtcact tcgccagtta gcccttaggg taggagtcgc gccggcagca gccatgagcg 180
   gcggcgtgta cgggggaggt gagtgagtgc ggccggacga gagagcgcgc cttttcggcg 240
   tgtgggatga agttggagcc cttgtttttg acattggatc ctatactgtg agagctggtt 300
   atgctggtga ggactgcccc aaggtggatt ttcctacagc tattggtatg gtggtagaaa 360
   gagatgacgg aagcacatta atggaaatag atggcgataa aggcaaacaa ggcggtccca 420
   cctactacat agatactaat gctctgcgtg ttccgaggga gaatatggag gccatttcac 480
   ctctaaaaaa tgggatggtt gaagactggg atagtttcca agctattttg gatcatacct 540
   acaaaatgca tgtcaaatca gaagccagtc tccatcctgt tctcatgtca gaggcaccgt 600
   ggaatactag agcaaagaga gagaaactga cagagttaat gtttgaacac tacaacatcc 660
   ctgccttctt cctttgcaaa actgcagttt tgacagcatt tgctaatggt cgttctactg 720
   ggctgatttt ggacagtgga gccactcata ccactgcaat tccagtccac gatggctatg 780
   tccttcaaca aggcattgtg aaatcccctc ttgctggaga ctttattact atgcagtgca 840
   gagaactctt ccaagaaatg aatattgaat tggttcctcc atatatgatt gcatcaaaag 900
   aagctgttcg tgaaggatct ccagcaaact ggaaaagaaa agagaagttg cctcaggtta 960
   cgaggtcttg gcacaattat atgtgtaatt gtgttatcca ggattttcaa gcttcggtac 1020
   ttcaagtgtc agattcaact tatgatgaac aagtggctgc acagatgcca actgttcatt 1080
   atgaattccc caatggctac aattgtgatt ttggtgcaga gcggctaaag attccagaag 1140
   gattatttga cccttccaat gtaaaggggt tatcaggaaa cacaatgtta ggagtcagtc 1200
   atgttgtcac cacaagtgtt gggatgtgtg atattgatat cagaccaggt ctctatggca 1260
   gtgtaatagt ggcaggagga aacacactaa tacagagttt tactgacagg ttgaatagag 1320
   agctgtctca gaaaactcct ccaagtatgc ggttgaaatt gattgcaaat aatacaacag 1380
   tggaacggag gtttagctca tggattggcg gctccattct agcctctttg ggtacctttc 1440
   aacagatgtg gatttccaag caagaatatg aagaaggagg gaagcagtgt gtagaaagaa 1500
   aatgcccttg agaaagagtt cccaagcttc taccttcctt ttgtcacctt acgtttcata 1560
   gctttagtat actcaggaaa agaatgacca tcttttgtag aatgtttata catttttgca 1620
   tatttcaatt tccacttaaa ttttttaaag ctttaactgg ctctataaat taagtttgtg 1680
   ctttccttga aatgcactta ttcttattac aagcatttta taattttgta taaatgtcta 1740
   ttttctctaa atattttgct ttcagtaaaa tgctttccaa ctctgtttag tgtattaatt 1800
   accagtggat tggtagaact gctttttatt gactagtaaa agttactgcc tatgcttttt 1860
   accttaggct tacagaatta aataaaaatt agccattcca gaaatataaa aaaaaaaaaa 1920
   aaaaa 1925

<210> 4
<211> 6594
<212> DNA
<213> Homo sapiens

<400> 4
   ccggtctata ggtgcatacc accacaccag gctaattttt tttttttgag acggagtctc 60
   actctgtcac ccaggctgga gtgcaatggc gcgatctttg ctcactgcaa cctctgccac 120
   ccgggttcaa gcaattctcc tgcctcagct cctgagtagc tgggattaca ggcgcccgcc 180
   accacgcccg gctaattttt gtatttttag tagagacgag gtttcaccat cttggccagg 240
   ctggtcttga actcctgacc ttgtgatcta cctgccaggg ccttccaaag tgctgggatt 300
   acggacgtga gccaccacgc ctggcccagg ctaatttttt gaaattttaa tagaggcagg 360
   gtcttgctgt attgcccagg ctgctctcac attcctagcc ttaagccatc ctcctatctc 420
   ggcctcacaa agtgctggga ttacaggtgt aagccactgt gcctggtttt gggcaagttt 480
   cttgtcctga ctgtgtcttg ggttctcacc tgttgattgg ggataccctt gttgcctacc 540
   tccaagtaat tttatgagga tgaaactcat agcttgtcag tgggaagtgc ttaggtaatg 600
   agctactagt ggtggcagtg gctgtgatgg tggtattaat accactccta ctggatggtc 660
   tctaatacta tctccttcct tccctaccag caacatgtca tggtttagtg gcctcctggt 720
   ccctaaagtg gatgaacgga aaacagcctg gggcgaacgc aatgggcaga agcgttcgcg 780
   gcgccgtggc actcgggcag gtggcttctg cacgccccgc tatatgagct gcctccggga 840
   tgcagagcca cccagcccca cccctgccgg cccccctcgg tgcccctggc aggatgacgc 900
   cttcatccgg aggggcggcc caggcaaggg caaggagctg gggctgcggg cagtggccct 960
   gggcttcgag gataccgagg tgacaacgac agcgggcggg acggctgagg tggcgcccga 1020
   cgcggtgccc aggagtgggc gatcctgctg gcgccgtctg gtgcaggtgt tccagtcgaa 1080
   gcagttccgt tcggccaagc tggagcgcct gtaccagcgg tacttcttcc agatgaacca 1140
   gagcagcctg acgctgctga tggcggtgct ggtgctgctc acagcggtgc tgctggcttt 1200
   ccacgccgca cccgcccgcc ctcagcctgc ctatgtggca ctgttggcct gtgccgccgc 1260
   cctgttcgtg gggctcatgg tggtgtgtaa ccggcatagc ttccgccagg actccatgtg 1320
   ggtggtgagc tacgtggtgc tgggcatcct ggcggcagtg caggtcgggg gcgctctcgc 1380
   agcagacccg cgcagcccct ctgcgggcct ctggtgccct gtgttctttg tctacatcgc 1440
   ctacacgctc ctccccatcc gcatgcgggc tgccgtcctc agcggcctgg gcctctccac 1500
   cttgcatttg atcttggcct ggcaacttaa ccgtggtgat gccttcctct ggaagcagct 1560
   cggtgccaat gtgctgctgt tcctctgcac caacgtcatt ggcatctgca cacactatcc 1620
   agcagaggtg tctcagcgcc aggcctttca ggagacccgc ggttacatcc aggcccggct 1680
   ccacctgcag catgagaatc ggcagcagga gcggctgctg ctgtcggtat tgccccagca 1740
   cgttgccatg gagatgaaag aagacatcaa cacaaaaaaa gaagacatga tgttccacaa 1800
   gatctacata cagaagcatg acaatgtcag catcctgttt gcagacattg agggcttcac 1860
   cagcctggca tcccagtgca ctgcgcagga gctggtcatg accctgaatg agctctttgc 1920
   ccggtttgac aagctggctg cggagaatca ctgcctgagg atcaagatct tgggggactg 1980
   ttactactgt gtgtcagggc tgccggaggc ccgggccgac catgcccact gctgtgtgga 2040
   gatgggggta gacatgattg aggccatctc gctggtacgt gaggtgacag gtgtgaatgt 2100
   gaacatgcgc gtgggcatcc acagcgggcg cgtgcactgc ggcgtccttg gcttgcggaa 2160
   atggcagttc gatgtgtggt ccaatgatgt gaccctggcc aaccacatgg aggcaggagg 2220
   ccgggctggc cgcatccaca tcactcgggc aacactgcag tacctgaacg gggactacga 2280
   ggtggagcca ggccgtggtg gcgagcgcaa cgcgtacctc aaggagcagc acattgagac 2340
   tttcctcatc ctgggcgcca gccagaaacg gaaagaggag aaggccatgc tggccaagct 2400
   gcagcggact cgggccaact ccatggaagg gctgatgccg cgctgggttc ctgatcgtgc 2460
   cttctcccgg accaaggact ccaaggcctt ccgccagatg ggcattgatg attccagcaa 2520
   agacaaccgg ggcacccaag atgccctgaa ccctgaggat gaggtggatg agttcctgag 2580
   ccgtgccatc gatgcccgca gcattgatca gctgcggaag gaccatgtgc gccggtttct 2640
   gctcaccttc cagagagagg atcttgagaa gaagtactcc cggaaggtgg atccccgctt 2700
   cggagcctac gttgcctgtg ccctgttggt cttctgcttc atctgcttca tccagcttct 2760
   catcttccca cactccaccc tgatgcttgg gatctatgcc agcatcttcc tgctgctgct 2820
   aatcaccgtg ctgatctgtg ctgtgtactc ctgtggttct ctgttcccta aggccctgca 2880
   acgtctgtcc cgcagcattg tccgctcacg ggcacatagc accgcagttg gcatcttttc 2940
   cgtcctgctt gtgtttactt ctgccattgc caacatgttc acctgtaacc acacccccat 3000
   acggagctgt gcagcccgga tgctgaattt aacacctgct gacatcactg cctgccacct 3060
   gcagcagctc aattactctc tgggcctgga tgctcccctg tgtgagggca ccatgcccac 3120
   ctgcagcttt cctgagtact tcatcgggaa catgctgctg agtctcttgg ccagctctgt 3180
   cttcctgcac atcagcagca tcgggaagtt ggccatgatc tttgtcttgg ggctcatcta 3240
   tttggtgctg cttctgctgg gtcccccagc caccatcttt gacaactatg acctactgct 3300
   tggcgtccat ggcttggctt cttccaatga gacctttgat gggctggact gtccagctgc 3360
   agggagggtg gccctcaaat atatgacccc tgtgattctg ctggtgtttg cgctggcgct 3420
   gtatctgcat gctcagcagg tggagtcgac tgcccgccta gacttcctct ggaaactaca 3480
   ggcaacaggg gagaaggagg agatggagga gctacaggca tacaaccgga ggctgctgca 3540
   taacattctg cccaaggacg tggcggccca cttcctggcc cgggagcgcc gcaatgatga 3600
   actctactat cagtcgtgtg agtgtgtggc tgttatgttt gcctccattg ccaacttctc 3660
   tgagttctat gtggagctgg aggcaaacaa tgagggtgtc gagtgcctgc ggctgctcaa 3720
   cgagatcatc gctgactttg atgagattat cagcgaggag cggttccggc agctggaaaa 3780
   gatcaagacg attggtagca cctacatggc tgcctcaggg ctgaacgcca gcacctacga 3840
   tcaggtgggc cgctcccaca tcactgccct ggctgactac gccatgcggc tcatggagca 3900
   gatgaagcac atcaatgagc actccttcaa caatttccag atgaagattg ggctgaacat 3960
   gggcccagtc gtggcaggtg tcatcggggc tcggaagcca cagtatgaca tctgggggaa 4020
   cacagtgaat gtctctagtc gtatggacag cacgggggtc cccgaccgaa tccaggtgac 4080
   cacggacctg taccaggttc tagctgccaa gggctaccag ctggagtgtc gaggggtggt 4140
   caaggtgaag ggcaaggggg agatgaccac ctacttcctc aatgggggcc ccagcagtta 4200
   acagggccca gccacaaatt cagctgaagg gaccaaggtg ggcattgagt ggactctgtg 4260
   ctcactgggt ggagctgtgg cagggggcac tgagcctcca gaccctgcta accacaaaag 4320
   ggaacatccc agcaggctgt gcttggatca tgctcgtctg ccctcaagct ggaaaacaag 4380
   gggctaccta ccgagaggat tatgcaagtg actttctttc ttacttgggg tagggctgtt 4440
   ccctctccaa tcttccagcc tttgggagca ggggaggggt cagtagcaga agcagaggga 4500
   ggcctcttgc ctgagggatt aaaatggcag cttgccatgc ctacccttcc ctgtctgtct 4560
   gggcagcagg ttcagggctg agcccttctt ttccctcttt tttcctggga atattttgta 4620
   caatattttg tacaaagaca ggcatgagga gtgcctattc catgcttgcc tttgcaatac 4680
   ctgcatcccc agcactggtc ctgggcactt ccccacccca gccaggtgtc cctcctatgc 4740
   acagagcaga ggagggagaa gctctgggga gccagctttg gccatatttc aggagaatgt 4800
   ttccatgtgc caaatcttag tcccatgatc tgtccccaaa ggggaacaaa gggacctctg 4860
   acagcttaga tttagcccca gttcctgcac gctccaggga acggggtgtc tggcctcact 4920
   ggtactgtga aaaatgctca gagagcaagc ctgtgtgtgg ggatgtcagg tcaggagctg 4980
   gaagttcacc tgcaggtgcc aaagagcagg ccggccaggg ctggggcagt gccagactct 5040
   gatctgagga ccccgtcggg gtccagatca ggtcactctg ccccagtgct ctcttgctgt 5100
   ctgctgacaa gggggcatgg agcatctctt cctcttctgt tgccaaatag aaaagggtca 5160
   gggcatggag aaaggtgacc ctgatcccaa acctgccctc ccaagtctct ggtgttgggg 5220
   agggcccgtg tgtttgtgta actgtgtgtg catgttggtc tttgtgtgca tatctgtttt 5280
   ccaggtctat gtgagtcctt gtgctcctgc tcctcagctc tccaccccag gttgcctctc 5340
   tcctgtgggc ctctgtcttc tgggaataaa gcagggtttc ctacttcagg ggatgtagag 5400
   agatgcccag gttgcacagg agtgggatgg ggtgtggtag caaaaggagg gagaggagtc 5460
   ctttttgtgc caaatcccta agtgccgttc gggggccatg tgtgcagcat gactctccct 5520
   gtctgtggca gggacccaag cgcttgctta agccccagtg ctccatgcca gcacttgaac 5580
   tgtctggggt ttgatggaca gaggctgagg aatttcctgg cttccccaga tagtgtcctg 5640
   ggacatggta tgctttgggg ctggggtagc atggaatccc tctgaggacc tggatactgg 5700
   tactacgggg tggggaagag gaaccttaaa cttggctttc cccagccttc agcctgagtc 5760
   tagcatgttt ctagctcccc agtcccttgt gaagccttga gggctggcag aagggttagg 5820
   aggttgaact ctagattccc ttcctgtctt tgccttcctt ttaccccttt ccctgcaacc 5880
   tccttgactc tggcctgaat ttgttggtgc ctcagtttct ctgtctgtac ctatttaagc 5940
   caaaggcact agcctgaatt ttgcttgaag atcactttgt cttggaaatg actagagagg 6000
   cagaggagaa gggtttccag agttgctagg tttgggagtg gaaggggcag gcagtgcact 6060
   tgcccctcct catgcccctt ctgacaccag ctccctgtgg aggcctggtt tctgggtaat 6120
   gcctcccttg ggcatcttca tgcatcaacc aaatgggcca tcaggtactt cattagtcat 6180
   ggcagaagga ggggaaaaga cttgttttcc agacagaaaa tctactcccc tgtccccagc 6240
   catatccctg gataggaagg gataggaaga gactacttgg tgccatgggg taggggtgag 6300
   ggtataagta gatcagagtg ggaagacctc agccttgggt ggcttgtctc tgcttcttgc 6360
   caggtgggag ggcctgtcca caccctggat ccccgtacca cagtgccagc catgcccttc 6420
   ccctgggcta ccattgtccc tttcctcacc cagttggtag aggagtcagg aggtgggagg 6480
   ccgtgggctt tggttttata atgtaaccac tgtgggggtg ggggaggatg gtgaaccatg 6540
   tatttcagtg aaatatttaa tatatttaaa tatcaataaa atcaaactct ttgt 6594

<210> 5
<211> 5877
<212> DNA
<213> Homo sapiens

<400> 5
cggacggcgg acggcgggcg ggacggccag gacgcgcggg ctcggaggac cgcgggacgg 60

ccggccggcc ggccggagcc cgcgggggcg gcggggcggg ggcccagggc aggcgcggag 120

ccgggccggc agcaacatgt catggtttag tggcctcctg gtccctaaag tggatgaacg 180

gaaaacagcc tggggtgaac gcaatgggca gaagcgttcg cggcgccgtg gcactcgggc 240

aggtggcttc tgcacgcccc gctatatgag ctgcctccgg gatgcagagc cacccagccc 300

cacccctgcg ggcccccctc ggtgcccctg gcaggatgac gccttcatcc ggaggggcgg 360

cccaggcaag ggcaaggagc tggggctgcg ggcagtggcc ctgggcttcg aggataccga 420

ggtgacaacg acagcgggcg ggacggctga ggtggcgccc gacgcggtgc ccaggagtgg 480

gcgatcctgc tggcgccgtc tggtgcaggt gttccagtcg aagcagttcc gttcggccaa 540

gctggagcgc ctgtaccagc ggtacttctt ccagatgaac cagagcagcc tgacgctgct 600

gatggcggtg ctggtgctgc tcacagcggt gctgctggct ttccacgccg cacccgcccg 660

ccctcagcct gcctatgtgg cactgttggc ctgtgccgcc gccctgttcg tggggctcat 720

ggtggtgtgt aaccggcata gcttccgcca ggactccatg tgggtggtga gctacgtggt 780

gctgggcatc ctggcggcag tgcaggtcgg gggcgctctc gcagcagacc cgcgcagccc 840

ctctgcgggc ctctggtgcc ctgtgttctt tgtctacatc gcctacacgc tcctccccat 900

ccgcatgcgg gctgccgtcc tcagcggcct gggcctctcc accttgcatt tgatcttggc 960

ctggcaactt aaccgtggtg atgccttcct ctggaagcag ctcggtgcca atgtgctgct 1020

gttcctctgc accaacgtca ttggcatctg cacacactat ccagcagagg tgtctcagcg 1080

ccaggccttt caggagaccc gcggttacat ccaggcccgg ctccacctgc agcatgagaa 1140

tcggcagcag gagcggctgc tgctgtcggt attgccccag cacgttgcca tggagatgaa 1200

agaagacatc aacacaaaaa aagaagacat gatgttccac aagatctaca tacagaagca 1260

tgacaatgtc agcatcctgt ttgcagacat tgagggcttc accagcctgg catcccagtg 1320

cactgcgcag gagctggtca tgaccctgaa tgagctcttt gcccggtttg acaagctggc 1380

tgcggagaat cactgcctga ggatcaagat cttgggggac tgttactact gtgtgtcagg 1440

gctgccggag gcccgggccg accatgccca ctgctgtgtg gagatggggg tagacatgat 1500

tgaggccatc tcgctggtac gtgaggtgac aggtgtgaat gtgaacatgc gcgtgggcat 1560

ccacagcggg cgcgtgcact gcggcgtcct tggcttgcgg aaatggcagt tcgatgtgtg 1620

gtccaatgat gtgaccctgg ccaaccacat ggaggcagga ggccgggctg gccgcatcca 1680

catcactcgg gcaacactgc agtacctgaa cggggactac gaggtggagc caggccgtgg 1740

tggcgagcgc aacgcgtacc tcaaggagca gcacattgag actttcctca tcctgggcgc 1800

cagccagaaa cggaaagagg agaaggccat gctggccaag ctgcagcgga ctcgggccaa 1860

ctccatggaa gggctgatgc cgcgctgggt tcctgatcgt gccttctccc ggaccaagga 1920

ctccaaggcc ttccgccaga tgggcattga tgattccagc aaagacaacc ggggcaccca 1980

agatgccctg aaccctgagg atgaggtgga tgagttcctg agccgtgcca tcgatgcccg 2040

cagcattgat cagctgcgga aggaccatgt gcgccggttt ctgctcacct tccagagaga 2100

ggatcttgag aagaagtact cccggaaggt ggatccccgc ttcggagcct acgttgcctg 2160

tgccctgttg gtcttctgct tcatctgctt catccagctt ctcatcttcc cacactccac 2220

cctgatgctt gggatctatg ccagcatctt cctgctgctg ctaatcaccg tgctgatctg 2280

tgctgtgtac tcctgtggtt ctctgttccc taaggccctg caacgtctgt cccgcagcat 2340

tgtccgctca cgggcacata gcaccgcagt tggcatcttt tccgtcctgc ttgtgtttac 2400

ttctgccatt gccaacatgt acttcatcgg gaacatgctg ctgagtctct tggccagctc 2460

tgtcttcctg cacatcagca gcatcgggaa gttggccatg atctttgtct tggggctcat 2520

ctatttggtg ctgcttctgc tgggtccccc agccaccatc tttgacaact atgacctact 2580

gcttggcgtc catggcttgg cttcttccaa tgagaccttt gatgggctgg actgtccagc 2640

tgcagggagg gtggccctca aatatatgac ccctgtgatt ctgctggtgt ttgcgctggc 2700

gctgtatctg catgctcagc aggtggagtc gactgcccgc ctagacttcc tctggaaact 2760

acaggcaaca ggggagaagg aggagatgga ggagctacag gcatacaacc ggaggctgct 2820

gcataacatt ctgcccaagg acgtggcggc ccacttcctg gcccgggagc gccgcaatga 2880

tgaactctac tatcagtcgt gtgagtgtgt ggctgttatg tttgcctcca ttgccaactt 2940

ctctgagttc tatgtggagc tggaggcaaa caatgagggt gtcgagtgcc tgcggctgct 3000

caacgagatc atcgctgact ttgatgagat tatcagcgag gagcggttcc ggcagctgga 3060

aaagatcaag acgattggta gcacctacat ggctgcctca gggctgaacg ccagcaccta 3120

cgatcaggtg ggccgctccc acatcactgc cctggctgac tacgccatgc ggctcatgga 3180

gcagatgaag cacatcaatg agcactcctt caacaatttc cagatgaaga ttgggctgaa 3240
catgggccca gtcgtggcag gtgtcatcgg ggctcggaag ccacagtatg acatctgggg 3300
gaacacagtg aatgtctcta gtcgtatgga cagcacgggg gtccccgacc gaatccaggt 3360
gaccacggac ctgtaccagg ttctagctgc caagggctac cagctggagt gtcgaggggt 3420
ggtcaaggtg aagggcaagg gggagatgac cacctacttc ctcaatgggg gccccagcag 3480
ttaacagggc ccagccacaa attcagctga agggaccaag gtgggcattg agtggactct 3540
gtgctcactg ggtggagctg tggcaggggg cactgagcct ccagaccctg ctaaccacaa 3600
aagggaacat cccagcaggc tgtgcttgga tcatgctcgt ctgccctcaa gctggaaaac 3660
aaggggctac ctaccgagag gattatgcaa gtgactttct ttcttacttg gggtagggct 3720
gttccctctc caatcttcca gcctttggga gcaggggagg ggtcagtagc agaagcagag 3780
ggaggcctct tgcctgaggg attaaaatgg cagcttgcca tgcctaccct tccctgtctg 3840
tctgggcagc aggttcaggg ctgagccctt cttttccctc ttttttcctg ggaatatttt 3900
gtacaatatt ttgtacaaag acaggcatga ggagtgccta ttccatgctt gcctttgcaa 3960
tacctgcatc cccagcactg gtcctgggca cttccccacc ccagccaggt gtccctccta 4020
tgcacagagc agaggaggga gaagctctgg ggagccagct ttggccatat ttcaggagaa 4080
tgtttccatg tgccaaatct tagtcccatg atctgtcccc aaaggggaac aaagggacct 4140
ctgacagctt agatttagcc ccagttcctg cacgctccag ggaacggggt gtctggcctc 4200
actggtactg tgaaaaatgc tcagagagca agcctgtgtg tggggatgtc aggtcaggag 4260
ctggaagttc acctgcaggt gccaaagagc aggccggcca gggctggggc agtgccagac 4320
tctgatctga ggaccccgtc ggggtccaga tcaggtcact ctgccccagt gctctcttgc 4380
tgtctgctga caagggggca tggagcatct cttcctcttc tgttgccaaa tagaaaaggg 4440
tcagggcatg gagaaaggtg accctgatcc caaacctgcc ctcccaagtc tctggtgttg 4500
gggagggccc gtgtgtttgt gtaactgtgt gtgcatgttg gtctttgtgt gcatatctgt 4560
tttccaggtc tatgtgagtc cttgtgctcc tgctcctcag ctctccaccc caggttgcct 4620
ctctcctgtg ggcctctgtc ttctgggaat aaagcagggt ttcctacttc aggggatgta 4680
gagagatgcc caggttgcac aggagtggga tggggtgtgg tagcaaaagg agggagagga 4740
gtcctttttg tgccaaatcc ctaagtgccg ttcgggggcc atgtgtgcag catgactctc 4800
cctgtctgtg gcagggaccc aagcgcttgc ttaagcccca gtgctccatg ccagcacttg 4860
aactgtctgg ggtttgatgg acagaggctg aggaatttcc tggcttcccc agatagtgtc 4920
ctgggacatg gtatgctttg gggctggggt agcatggaat ccctctgagg acctggatac 4980
tggtactacg gggtggggaa gaggaacctt aaacttggct ttccccagcc ttcagcctga 5040
gtctagcatg tttctagctc cccagtccct tgtgaagcct tgagggctgg cagaagggtt 5100
aggaggttga actctagatt cccttcctgt ctttgccttc cttttacccc tttccctgca 5160
acctccttga ctctggcctg aatttgttgg tgcctcagtt tctctgtctg tacctattta 5220
agccaaaggc actagcctga attttgcttg aagatcactt tgtcttggaa atgactagag 5280

aggcagagga gaagggtttc cagagttgct aggtttggga gtggaagggg caggcagtgc 5340

acttgcccct cctcatgccc cttctgacac cagctccctg tggaggcctg gtttctgggt 5400

aatgcctccc ttgggcatct tcatgcatca accaaatggg ccatcaggta cttcattagt 5460

catggcagaa ggaggggaaa agacttgttt tccagacaga aaatctactc ccctgtcccc 5520

agccatatcc ctggatagga agggatagga agagactact tggtgccatg gggtaggggt 5580

gagggtataa gtagatcaga gtgggaagac ctcagccttg ggtggcttgt ctctgcttct 5640

tgccaggtgg gagggcctgt ccacaccctg gatccccgta ccacagtgcc agccatgccc 5700

ttcccctggg ctaccattgt ccctttcctc acccagttgg tagaggagtc aggaggtggg 5760

aggccgtggg ctttggtttt ataatgtaac cactgtgggg gtgggggagg atggtgaacc 5820

atgtatttca gtgaaatatt taatatattt aaatatcaat aaaatcaaac tctttgt 5877

<210> 6
<211> 2537
<212> DNA
<213> Homo sapiens

<400> 6
cccagaaggc cgcggggggt ggaccgccta agagggcgtg cgctcccgac atgccccgcg 60

gcgcgccatt aaccgccaga tttgaatcgc gggacccgtt ggcagaggtg gcggcggcgg 120

catgggtgcc ccgacgttgc cccctgcctg gcagcccttt ctcaaggacc accgcatctc 180

tacattcaag aactggccct tcttggaggg ctgcgcctgc accccggagc ggatggccga 240

ggctggcttc atccactgcc ccactgagaa cgagccagac ttggcccagt gtttcttctg 300

cttcaaggag ctggaaggct gggagccaga tgacgacccc atgcaaagga aaccaacaat 360

aagaagaaag aatttgagga aactgcggag aaagtgcgcc gtgccatcga gcagctggct 420

gccatggatt gaggcctctg gccggagctg cctggtccca gagtggctgc accacttcca 480

gggtttattc cctggtgcca ccagccttcc tgtgggcccc ttagcaatgt cttaggaaag 540

gagatcaaca ttttcaaatt agatgtttca actgtgctct tgttttgtct tgaaagtggc 600

accagaggtg cttctgcctg tgcagcgggt gctgctggta acagtggctg cttctctctc 660

tctctctctt ttttgggggc tcatttttgc tgttttgatt cccgggctta ccaggtgaga 720

agtgagggag gaagaaggca gtgtcccttt tgctagagct gacagctttg ttcgcgtggg 780

cagagccttc cacagtgaat gtgtctggac ctcatgttgt tgaggctgtc acagtcctga 840

gtgtggactt ggcaggtgcc tgttgaatct gagctgcagg ttccttatct gtcacacctg 900

tgcctcctca gaggacagtt tttttgttgt tgtgtttttt tgtttttttt tttttggtag 960

atgcatgact tgtgtgtgat gagagaatgg agacagagtc cctggctcct ctactgttta 1020

acaacatggc tttcttattt tgtttgaatt gttaattcac agaatagcac aaactacaat 1080

taaaactaag cacaaagcca ttctaagtca ttggggaaac ggggtgaact tcaggtggat 1140

gaggagacag aatagagtga taggaagcgt ctggcagata ctccttttgc cactgctgtg 1200

tgattagaca ggcccagtga gccgcggggc acatgctggc cgctcctccc tcagaaaaag 1260

gcagtggcct aaatcctttt taaatgactt ggctcgatgc tgtgggggac tggctgggct 1320

gctgcaggcc gtgtgtctgt cagcccaacc ttcacatctg tcacgttctc cacacggggg 1380

agagacgcag tccgcccagg tccccgcttt ctttggaggc agcagctccc gcagggctga 1440

agtctggcgt aagatgatgg atttgattcg ccctcctccc tgtcatagag ctgcagggtg 1500

gattgttaca gcttcgctgg aaacctctgg aggtcatctc ggctgttcct gagaaataaa 1560

aagcctgtca tttcaaacac tgctgtggac cctactgggt ttttaaaata ttgtcagttt 1620

ttcatcgtcg tccctagcct gccaacagcc atctgcccag acagccgcag tgaggatgag 1680

cgtcctggca gagacgcagt tgtctctggg cgcttgccag agccacgaac cccagacctg 1740

tttgtatcat ccgggctcct tccgggcaga aacaactgaa aatgcacttc agacccactt 1800

atttctgcca catctgagtc ggcctgagat agacttttcc ctctaaactg ggagaatatc 1860

acagtggttt ttgttagcag aaaatgcact ccagcctctg tactcatcta agctgcttat 1920

ttttgatatt tgtgtcagtc tgtaaatgga tacttcactt taataactgt tgcttagtaa 1980

ttggctttgt agagaagctg gaaaaaaatg gttttgtctt caactccttt gcatgccagg 2040

cggtgatgtg gatctcggct tctgtgagcc tgtgctgtgg gcagggctga gctggagccg 2100

cccctctcag cccgcctgcc acggcctttc cttaaaggcc atccttaaaa ccagaccctc 2160

atggctacca gcacctgaaa gcttcctcga catctgttaa taaagccgta ggcccttgtc 2220

taagtgcaac cgcctagact ttctttcaga tacatgtcca catgtccatt tttcaggttc 2280

tctaagttgg agtggagtct gggaagggtt gtgaatgagg cttctgggct atgggtgagg 2340

ttccaatggc aggttagagc ccctcgggcc aactgccatc ctggaaagta gagacagcag 2400

tgcccgctgc ccagaagaga ccagcaagcc aaactggagc ccccattgca ggctgtcgcc 2460

atgtggaaag agtaactcac aattgccaat aaagtctcat gtggttttat ctaaaaaaaa 2520

aaaaaaaaaa aaaaaaa 2537

<210> 7
<211> 2724
<212> DNA
<213> Homo sapiens

<400> 7
cccagaaggc cgcggggggt ggaccgccta agagggcgtg cgctcccgac atgccccgcg 60

gcgcgccatt aaccgccaga tttgaatcgc gggacccgtt ggcagaggtg gcggcggcgg 120

catgggtgcc ccgacgttgc cccctgcctg gcagcccttt ctcaaggacc accgcatctc 180

tacattcaag aactggccct tcttggaggg ctgcgcctgc accccggagc ggatggccga 240

ggctggcttc atccactgcc ccactgagaa cgagccagac ttggcccagt gtttcttctg 300

cttcaaggag ctggaaggct gggagccaga tgacgacccc attgggccgg gcacggtggc 360

ttacgcctgt aataccagca ctttgggagg ccgaggcggg cggatcacga gagaggaaca 420

taaaaagcat tcgtccggtt gcgctttcct ttctgtcaag aagcagtttg aagaattaac 480

ccttggtgaa tttttgaaac tggacagaga aagagccaag aacaaaattg caaaggaaac 540

caacaataag aagaaagaat ttgaggaaac tgcggagaaa gtgcgccgtg ccatcgagca 600

gctggctgcc atggattgag gcctctggcc ggagctgcct ggtcccagag tggctgcacc 660
acttccaggg tttattccct ggtgccacca gccttcctgt gggcccctta gcaatgtctt 720
aggaaaggag atcaacattt tcaaattaga tgtttcaact gtgctcttgt tttgtcttga 780
aagtggcacc agaggtgctt ctgcctgtgc agcgggtgct gctggtaaca gtggctgctt 840
ctctctctct ctctcttttt tgggggctca tttttgctgt tttgattccc gggcttacca 900
ggtgagaagt gagggaggaa gaaggcagtg tcccttttgc tagagctgac agctttgttc 960
gcgtgggcag agccttccac agtgaatgtg tctggacctc atgttgttga ggctgtcaca 1020
gtcctgagtg tggacttggc aggtgcctgt tgaatctgag ctgcaggttc cttatctgtc 1080
acacctgtgc ctcctcagag gacagttttt ttgttgttgt gtttttttgt tttttttttt 1140
ttggtagatg catgacttgt gtgtgatgag agaatggaga cagagtccct ggctcctcta 1200
ctgtttaaca acatggcttt cttattttgt ttgaattgtt aattcacaga atagcacaaa 1260
ctacaattaa aactaagcac aaagccattc taagtcattg gggaaacggg gtgaacttca 1320
ggtggatgag gagacagaat agagtgatag gaagcgtctg gcagatactc cttttgccac 1380
tgctgtgtga ttagacaggc ccagtgagcc gcggggcaca tgctggccgc tcctccctca 1440
gaaaaaggca gtggcctaaa tcctttttaa atgacttggc tcgatgctgt gggggactgg 1500
ctgggctgct gcaggccgtg tgtctgtcag cccaaccttc acatctgtca cgttctccac 1560
acgggggaga gacgcagtcc gcccaggtcc ccgctttctt tggaggcagc agctcccgca 1620
gggctgaagt ctggcgtaag atgatggatt tgattcgccc tcctccctgt catagagctg 1680
cagggtggat tgttacagct tcgctggaaa cctctggagg tcatctcggc tgttcctgag 1740
aaataaaaag cctgtcattt caaacactgc tgtggaccct actgggtttt taaaatattg 1800
tcagtttttc atcgtcgtcc ctagcctgcc aacagccatc tgcccagaca gccgcagtga 1860
ggatgagcgt cctggcagag acgcagttgt ctctgggcgc ttgccagagc cacgaacccc 1920
agacctgttt gtatcatccg ggctccttcc gggcagaaac aactgaaaat gcacttcaga 1980
cccacttatt tctgccacat ctgagtcggc ctgagataga cttttccctc taaactggga 2040
gaatatcaca gtggtttttg ttagcagaaa atgcactcca gcctctgtac tcatctaagc 2100
tgcttatttt tgatatttgt gtcagtctgt aaatggatac ttcactttaa taactgttgc 2160
ttagtaattg gctttgtaga gaagctggaa aaaaatggtt ttgtcttcaa ctcctttgca 2220
tgccaggcgg tgatgtggat ctcggcttct gtgagcctgt gctgtgggca gggctgagct 2280
ggagccgccc ctctcagccc gcctgccacg gcctttcctt aaaggccatc cttaaaacca 2340
gaccctcatg gctaccagca cctgaaagct tcctcgacat ctgttaataa agccgtaggc 2400
ccttgtctaa gtgcaaccgc ctagactttc tttcagatac atgtccacat gtccattttt 2460
caggttctct aagttggagt ggagtctggg aagggttgtg aatgaggctt ctgggctatg 2520
ggtgaggttc caatggcagg ttagagcccc tcgggccaac tgccatcctg gaaagtagag 2580
acagcagtgc ccgctgccca gaagagacca gcaagccaaa ctggagcccc cattgcaggc 2640
tgtcgccatg tggaaagagt aactcacaat tgccaataaa gtctcatgtg gttttatcta 2700

aaaaaaaaaa aaaaaaaaaa aaaa                                          2724

<210>  8
<211>  2655
<212>  DNA
<213>  Homo sapiens

<400>  8
cccagaaggc cgcggggggt ggaccgccta agagggcgtg cgctcccgac atgccccgcg      60

gcgcgccatt aaccgccaga tttgaatcgc gggacccgtt ggcagaggtg gcggcggcgg     120

catgggtgcc ccgacgttgc cccctgcctg gcagcccttt ctcaaggacc accgcatctc     180

tacattcaag aactggccct tcttggaggg ctgcgcctgc accccggagc ggatggccga     240

ggctggcttc atccactgcc ccactgagaa cgagccagac ttggcccagt gtttcttctg     300

cttcaaggag ctggaaggct gggagccaga tgacgacccc atagaggaac ataaaaagca     360

ttcgtccggt tgcgctttcc tttctgtcaa gaagcagttt gaagaattaa cccttggtga     420

atttttgaaa ctggacagag aaagagccaa gaacaaaatt gcaaaggaaa ccaacaataa     480

gaagaaagaa tttgaggaaa ctgcggagaa agtgcgccgt gccatcgagc agctggctgc     540

catggattga ggcctctggc cggagctgcc tggtcccaga gtggctgcac cacttccagg     600

gtttattccc tggtgccacc agccttcctg tgggcccctt agcaatgtct taggaaagga     660

gatcaacatt ttcaaattag atgtttcaac tgtgctcttg ttttgtcttg aaagtggcac     720

cagaggtgct tctgcctgtg cagcgggtgc tgctggtaac agtggctgct tctctctctc     780

tctctctttt ttgggggctc atttttgctg ttttgattcc cgggcttacc aggtgagaag     840

tgagggagga agaaggcagt gtcccttttg ctagagctga cagctttgtt cgcgtgggca     900

gagccttcca cagtgaatgt gtctggacct catgttgttg aggctgtcac agtcctgagt     960

gtggacttgg caggtgcctg ttgaatctga gctgcaggtt ccttatctgt cacacctgtg    1020

cctcctcaga ggacagtttt tttgttgttg tgtttttttg tttttttttt tttggtagat    1080

gcatgacttg tgtgtgatga gagaatggag acagagtccc tggctcctct actgtttaac    1140

aacatggctt tcttattttg tttgaattgt taattcacag aatagcacaa actacaatta    1200

aaactaagca caaagccatt ctaagtcatt ggggaaacgg ggtgaacttc aggtggatga    1260

ggagacagaa tagagtgata ggaagcgtct ggcagatact ccttttgcca ctgctgtgtg    1320

attagacagg cccagtgagc cgcggggcac atgctggccg ctcctccctc agaaaaaggc    1380

agtggcctaa atccttttta aatgacttgg ctcgatgctg tgggggactg gctgggctgc    1440

tgcaggccgt gtgtctgtca gcccaacctt cacatctgtc acgttctcca cacgggggag    1500

agacgcagtc cgcccaggtc cccgctttct ttggaggcag cagctcccgc agggctgaag    1560

tctggcgtaa gatgatggat ttgattcgcc ctcctccctg tcatagagct gcagggtgga    1620

ttgttacagc ttcgctggaa acctctggag gtcatctcgg ctgttcctga gaaataaaaa    1680

gcctgtcatt tcaaacactg ctgtggaccc tactgggttt ttaaaatatt gtcagttttt    1740

catcgtcgtc cctagcctgc caacagccat ctgcccagac agccgcagtg aggatgagcg    1800

tcctggcaga gacgcagttg tctctgggcg cttgccagag ccacgaaccc cagacctgtt 1860

tgtatcatcc gggctccttc cgggcagaaa caactgaaaa tgcacttcag acccacttat 1920

ttctgccaca tctgagtcgg cctgagatag acttttccct ctaaactggg agaatatcac 1980

agtggttttt gttagcagaa aatgcactcc agcctctgta ctcatctaag ctgcttattt 2040

ttgatatttg tgtcagtctg taaatggata cttcacttta ataactgttg cttagtaatt 2100

ggctttgtag agaagctgga aaaaaatggt tttgtcttca actcctttgc atgccaggcg 2160

gtgatgtgga tctcggcttc tgtgagcctg tgctgtgggc agggctgagc tggagccgcc 2220

cctctcagcc cgcctgccac ggcctttcct taaaggccat ccttaaaacc agaccctcat 2280

ggctaccagc acctgaaagc ttcctcgaca tctgttaata aagccgtagg cccttgtcta 2340

agtgcaaccg cctagacttt ctttcagata catgtccaca tgtccatttt tcaggttctc 2400

taagttggag tggagtctgg gaagggttgt gaatgaggct tctgggctat gggtgaggtt 2460

ccaatggcag gttagagccc ctcgggccaa ctgccatcct ggaaagtaga gacagcagtg 2520

cccgctgccc agaagagacc agcaagccaa actggagccc ccattgcagg ctgtcgccat 2580

gtggaaagag taactcacaa ttgccaataa agtctcatgt ggttttatct aaaaaaaaaa 2640

aaaaaaaaaa aaaaa 2655

<210> 9
<211> 3486
<212> DNA
<213> Homo sapiens

<400> 9
gagccgactg cggctgcgcg gggtattcga atcggcggcg gcttctagtt tgcggttcag 60

gtttggccgc tgccggccag cgtcctctgg ccatggacac cccggaaaat gtccttcaga 120

tgcttgaagc ccacatgcag agctacaagg gcaatgaccc tcttggtgaa tgggaaagat 180

acatacagtg ggtagaagag aattttcctg agaataaaga atacttgata actttactag 240

aacatttaat gaaggaattt ttagataaga agaaatacca caatgaccca agattcatca 300

gttattgttt aaaatttgct gagtacaaca gtgacctcca tcaatttttt gagtttctgt 360

acaaccatgg gattggaacc ctgtcatccc ctctgtacat tgcctgggcg gggcatctgg 420

aagcccaagg agagctgcag catgccagtg ctgtccttca gagaggaatt caaaaccagg 480

ctgaacccag agagttcctg caacaacaat acaggttatt tcagacacgc ctcactgaaa 540

cccatttgcc agctcaagct agaacctcag aacctctgca taatgttcag gttttaaatc 600

aaatgataac atcaaaatca aatccaggaa ataacatggc ctgcatttct aagaatcagg 660

gttcagagct ttctggagtg atatcttcag cttgtgataa agagtcaaat atggaacgaa 720

gagtgatcac gatttctaaa tcagaatatt ctgtgcactc atctttggca tccaaagttg 780

atgttgagca ggttgttatg tattgcaagg agaagcttat tcgtggggaa tcagaatttt 840

cctttgaaga attgagagcc cagaaataca atcaacggag aaagcatgag caatgggtaa 900

atgaagacag acattatatg aaaaggaaag aagcaaatgc ttttgaagaa cagctattaa 960

aacagaaaat ggatgaactt cataagaagt tgcatcaggt ggtggagaca tcccatgagg 1020

atctgcccgc ttcccaggaa aggtccgagg ttaatccagc acgtatgggg ccaagtgtag 1080
gctcccagca ggaactgaga gcgccatgtc ttccagtaac ctatcagcag acaccagtga 1140
acatggaaaa gaacccaaga gaggcacctc ctgttgttcc tcctttggca aatgctattt 1200
ctgcagcttt ggtgtcccca gccaccagcc agagcattgc tcctcctgtt cctttgaaag 1260
cccagacagt aacagactcc atgtttgcag tggccagcaa agatgctgga tgtgtgaata 1320
agagtactca tgaattcaag ccacagagtg gagcagagat caaagaaggg tgtgaaacac 1380
ataaggttgc caacacaagt tcttttcaca caactccaaa cacatcactg ggaatggttc 1440
aggcaacgcc atccaaagtg cagccatcac ccaccgtgca cacaaaagaa gcattaggtt 1500
tcatcatgaa tatgtttcag gctcctacac ttcctgatat ttctgatgac aaagatgaat 1560
ggcaatctct agatcaaaat gaagatgcat ttgaagccca gtttcaaaaa aatgtaaggt 1620
catctggggc ttggggagtc aataagatca tctcttcttt gtcatctgct tttcatgtgt 1680
ttgaagatgg aaacaaagaa aattatggat taccacagcc taaaaataaa cccacaggag 1740
ccaggacctt tggagaacgc tctgtcagca gacttccttc aaaaccaaag gaggaagtgc 1800
ctcatgctga agagtttttg gatgactcaa ctgtatgggg tattcgctgc aacaaaaccc 1860
tggcacccag tcctaagagc ccaggagact tcacatctgc tgcacaactt gcgtctacac 1920
cattccacaa gcttccagtg gagtcagtgc acattttaga agataaagaa aatgtggtag 1980
caaaacagtg tacccaggcg actttggatt cttgtgagga aaacatggtg gtgccttcaa 2040
gggatggaaa attcagtcca attcaagaga aaagcccaaa acaggccttg tcgtctcaca 2100
tgtattcagc atccttactt cgtctgagcc agcctgctgc aggtggggta cttacctgtg 2160
aggcagagtt gggcgttgag gcttgcagac tcacagacac tgacgctgcc attgcagaag 2220
atccaccaga tgctattgct gggctccaag cagaatggat gcagatgagt tcacttggga 2280
ctgttgatgc tccaaacttc attgttggga acccatggga tgataagctg attttcaaac 2340
ttttatctgg gctttctaaa ccagtgagtt cctatccaaa tacttttgaa tggcaatgta 2400
aacttccagc catcaagccc aagactgaat ttcaattggg ttctaagctg gtctatgtcc 2460
atcaccttct tggagaagga gcctttgccc aggtgtacga agctacccag ggagatctga 2520
atgatgctaa aaataaacag aaatttgttt taaaggtcca aaagcctgcc aacccctggg 2580
aattctacat tgggacccag ttgatggaaa gactaaagcc atctatgcag cacatgttta 2640
tgaagttcta ttctgcccac ttattccaga atggcagtgt attagtagga gagctctaca 2700
gctatggaac attattaaat gccattaacc tctataaaaa tacccctgaa aaagtgatgc 2760
ctcaaggtct tgtcatctct tttgctatga gaatgcttta catgattgag caagtgcatg 2820
actgtgaaat cattcatgga gacattaaac cagacaattt catacttgga aacggatttt 2880
tggaacagga tgatgaagat gatttatctg ctggcttggc actgattgac ctgggtcaga 2940
gtatagatat gaaacttttt ccaaaaggaa ctatattcac agcaaagtgt gaaacatctg 3000
gttttcagtg tgttgagatg ctcagcaaca aaccatggaa ctaccagatc gattactttg 3060
gggttgctgc aacagtatat tgcatgctct ttggcactta catgaaagtg aaaaatgaag 3120

gaggagagtg taagcctgaa ggtcttttta gaaggcttcc tcatttggat atgtggaatg 3180

aattttttca tgttatgttg aatattccag attgtcatca tcttccatct ttggatttgt 3240

taaggcaaaa gctgaagaaa gtatttcaac aacactatac taacaagatt agggccctac 3300

gtaataggct aattgtactg ctcttagaat gtaagcgttc acgaaaataa aatttggata 3360

tagacagtcc ttaaaaatca cactgtaaat atgaatctgc tcactttaaa cctgtttttt 3420

tttcatttat tgtttatgta aatgtttgtt aaaaataaat cccatggaat atttccatgt 3480

aaaaaa 3486

<210> 10
<211> 3721
<212> DNA
<213> Homo sapiens

<400> 10
accgttaaat ttgaaacttg gcggctaggg gtgtgggctt gaggtggccg gtttgttagg 60

gagtcgtgta cgtgccttgg tcgcttctgt agctccgagg gcaggttgcg gaagaaagcc 120

caggcggtct gtggcccaga ggaaaggcct gcagcaggac gaggacctga gccaggaatg 180

caggatggcg gcggtgaaga aggaaggggg tgctctgagt gaagccatgt ccctggaggg 240

agatgaatgg gaactgagta aagaaaatgt acaaccttta aggcaagggc ggatcatgtc 300

cacgcttcag ggagcactgg cacaagaatc tgcctgtaac aatactcttc agcagcagaa 360

acgggcattt gaatatgaaa ttcgatttta cactggaaat gaccctctgg atgtttggga 420

taggtatatc agctggacag agcagaacta tcctcaaggt gggaaggaga gtaatatgtc 480

aacgttatta gaaagagctg tagaagcact acaaggagaa aaacgatatt atagtgatcc 540

tcgatttctc aatctctggc ttaaattagg gcgtttatgc aatgagcctt tggatatgta 600

cagttacttg cacaaccaag ggattggtgt ttcacttgct cagttctata tctcatgggc 660

agaagaatat gaagctagag aaaactttag gaaagcagat gcgatatttc aggaagggat 720

tcaacagaag gctgaaccac tagaaagact acagtcccag caccgacaat tccaagctcg 780

agtgtctcgg caaactctgt tggcacttga gaaagaagaa gaggaggaag tttttgagtc 840

ttctgtacca caacgaagca cactagctga actaaagagc aaagggaaaa agacagcaag 900

agctccaatc atccgtgtag gaggtgctct caaggctcca agccagaaca gaggactcca 960

aaatccattt cctcaacaga tgcaaaataa tagtagaatt actgtttttg atgaaaatgc 1020

tgatgaggct tctacagcag agttgtctaa gcctacagtc cagccatgga tagcaccccc 1080

catgcccagg gccaaagaga atgagctgca agcaggccct tggaacacag gcaggtcctt 1140

ggaacacagg cctcgtggca atacagcttc actgatagct gtacccgctg tgcttcccag 1200

tttcactcca tatgtggaag agactgcaca acagccagtt atgacaccat gtaaaattga 1260

acctagtata aaccacatcc taagcaccag aaagcctgga aaggaagaag gagatcctct 1320

acaaagggtt cagagccatc agcaagcgtc tgaggagaag aaagagaaga tgatgtattg 1380

taaggagaag atttatgcag gagtagggga attctccttt gaagaaattc gggctgaagt 1440

tttccggaag aaattaaaag agcaaaggga agccgagcta ttgaccagtg cagagaagag 1500
agcagaaatg cagaaacaga ttgaagagat ggagaagaag ctaaaagaaa tccaaactac 1560
tcagcaagaa agaacaggtg atcagcaaga agagacgatg cctacaaagg agacaactaa 1620
actgcaaatt gcttccgagt ctcagaaaat accaggaatg actctatcca gttctgtttg 1680
tcaagtaaac tgttgtgcca gagaaacttc acttgcggag aacatttggc aggaacaacc 1740
tcattctaaa ggtcccagtg tacctttctc catttttgat gagtttcttc tttcagaaaa 1800
gaagaataaa agtcctcctg cagatccccc acgagtttta gctcaacgaa gaccccttgc 1860
agttctcaaa acctcagaaa gcatcacctc aaatgaagat gtgtctccag atgtttgtga 1920
tgaatttaca ggaattgaac ccttgagcga ggatgccatt atcacaggct tcagaaatgt 1980
aacaatttgt cctaacccag aagacacttg tgactttgcc agagcagctc gttttgtatc 2040
cactcctttt catgagataa tgtccttgaa ggatctccct tctgatcctg agagactgtt 2100
accggaagaa gatctagatg taaagacctc tgaggaccag cagacagctt gtggcactat 2160
ctacagtcag actctcagca tcaagaagct gagcccaatt attgaagaca gtcgtgaagc 2220
cacacactcc tctggcttct ctggttcttc tgcctcggtt gcaagcacct cctccatcaa 2280
atgtcttcaa attcctgaga aactagaact tactaatgag acttcagaaa accctactca 2340
gtcaccatgg tgttcacagt atcgcagaca gctactgaag tccctaccag agttaagtgc 2400
ctctgcagag ttgtgtatag aagacagacc aatgcctaag ttggaaattg agaaggaaat 2460
tgaattaggt aatgaggatt actgcattaa acgagaatac ctaatatgtg aagattacaa 2520
gttattctgg gtggcgccaa gaaactctgc agaattaaca gtaataaagg tatcttctca 2580
acctgtccca tgggactttt atatcaacct caagttaaag gaacgtttaa atgaagattt 2640
tgatcatttt tgcagctgtt atcaatatca agatggctgt attgtttggc accaatatat 2700
aaactgcttc acccttcagg atcttctcca acacagtgaa tatattaccc atgaaataac 2760
agtgttgatt atttataacc ttttgacaat agtggagatg ctacacaaag cagaaatagt 2820
ccatggtgac ttgagtccaa ggtgtctgat tctcagaaac agaatccacg atccctatga 2880
ttgtaacaag aacaatcaag ctttgaagat agtggacttt tcctacagtg ttgaccttag 2940
ggtgcagctg gatgttttta ccctcagcgg ctttcggact gtacagatcc tggaaggaca 3000
aaagatcctg gctaactgtt cttctcccta ccaggtagac ctgtttggta tagcagattt 3060
agcacattta ctattgttca aggaacacct acaggtcttc tgggatgggt ccttctggaa 3120
acttagccaa aatatttctg agctaaaaga tggtgaattg tggaataaat tctttgtgcg 3180
gattctgaat gccaatgatg aggccacagt gtctgttctt ggggagcttg cagcagaaat 3240
gaatggggtt tttgacacta cattccaaag tcacctgaac aaagccttat ggaaggtagg 3300
gaagttaact agtcctgggg ctttgctctt tcagtgagct aggcaatcaa gtctcacaga 3360
ttgctgcctc agagcaatgg ttgtattgtg gaacactgaa actgtatgtg ctgtaattta 3420
atttaggaca catttagatg cactaccatt gctgttctac tttttggtac aggtatattt 3480
tgacgtcact gatatttttt atacagtgat atacttactc atggccttgt ctaacttttg 3540

tgaagaacta ttttattcta aacagactca ttacaaatgg ttaccttgtt atttaaccca 3600

tttgtctcta cttttccctg tacttttccc atttgtaatt tgtaaaatgt tctcttatga 3660

tcaccatgta ttttgtaaat aataaaatag tatctgttaa atttgtgctt ctaaaaaaaa 3720

a 3721

<210> 11
<211> 7108
<212> DNA
<213> Homo sapiens

<400> 11
gcgcgcccga ggctcctacg ctgccgcgcc cggcttctct ccagcgcccc gcgccgttag 60

ccacgtggac cgactccggc gcgccgtcct cacgtggttc cagtggagtt tgcagtcctt 120

cccgcttctc cgtactcgcc cccgcctctg agctcccttc ccatggcggc cctagtgttg 180

gaggacgggt cggtcctgcg gggccagccc tttggggccg ccgtgtcgac tgccggggaa 240

gtggtgtttc aaaccggcat ggtcggctac cccgaggccc tcactgatcc ctcctacaag 300

gcacagatct tagtgctcac ctatcctctg atcggcaact atggcatccc cccagatgaa 360

atggatgagt tcggtctctg caagtggttt gaatcctcgg gcatccacgt agcagcactg 420

gtagtgggag agtgctgtcc tactcccagc cactggagtg ccacccgcac cctgcatgag 480

tggctgcagc agcatggcat ccctggcttg caaggagtag acactcggga gctgaccaag 540

aagttgcggg aacaggggtc tctgctgggg aagctggtcc agaatggaac agaaccttca 600

tccctgccat tcttggaccc caatgcccgc cccctggtac cagaggtctc cattaagact 660

ccacgggtat tcaatacagg gggtgcccct cggatccttg ctttggactg tggcctcaag 720

tataatcaga tccgatgcct ctgccagcgt ggggctgagg tcactgtggt accctgggac 780

catgcactag acagccaaga gtatgagggt ctcttcttaa gtaatgggcc tggtgaccct 840

gcctcctatc ccagtgtcgt atccacactg agccgtgttt tatctgagcc taatccccga 900

cctgtctttg ggatctgcct gggacaccag ctattggcct tagccattgg ggccaagact 960

tacaagatga gatatgggaa ccgaggccat aaccagccct gcttgttggt gggctctggg 1020

cgctgctttc tgacatccca gaaccatggg tttgctgtgg agacagactc actgccagca 1080

gactgggctc ctctcttcac caacgccaat gatggttcca atgaaggcat tgtgcacaac 1140

agcttgcctt tcttcagtgt ccagtttcac ccagagcacc aagctggccc ttcagatatg 1200

gaactgcttt tcgatatctt tctggaaact gtgaaagagg ccacagctgg gaaccctggg 1260

ggccagacag ttagagagcg gctgactgag cgcctctgtc cccctgggat tcccactccc 1320

ggctctggac ttccaccacc acgaaaggtt ctgatcctgg gctcaggggg cctctccatt 1380

ggccaagctg gagaatttga ctactcgggc tctcaggcaa ttaaggccct gaaggaggaa 1440

aacatccaga cgttgctgat caaccccaat attgccacag tgcagacctc ccaggggctg 1500

gccgacaagg tctattttct tcccataaca cctcattatg taacccaggt gatacgtaat 1560

gaacgccccg atggtgtgtt actgactttt gggggccaga ctgctctgaa ctgtggtgtg 1620

gagctgacca aggccggggt gctggctcgg tatggggtcc gggtcctggg cacaccagtg 1680

gagaccattg agctgaccga ggatcgacgg gcctttgctg ccagaatggc agagatcgga 1740

gagcatgtgg ccccgagcga ggcagcaaat tctcttgaac aggcccaggc agccgctgaa 1800

cggctggggt accctgtgct agtgcgtgca gcctttgccc tgggtggcct gggctctggc 1860

tttgcctcta acagggagga gctctctgct ctcgtggccc cagcttttgc ccataccagc 1920

caagtgctag tagacaagtc tctgaaggga tggaaggaga ttgagtacga ggtggtgaga 1980

gacgcctatg gcaactgtgt cacggtgtgt aacatggaga acttggaccc actgggcatc 2040

cacactggtg agtccatagt ggtggcccct agccagacac tgaatgacag ggagtatcag 2100

ctcctgaggc agacagctat caaggtgacc cagcacctgg gaattgttgg ggagtgcaat 2160

gtgcagtatg ccttgaaccc tgagtctgag cagtattaca tcattgaagt gaatgccagg 2220

ctctctcgca gctctgccct ggccagtaag gccacaggtt atccactggc ttatgtggca 2280

gccaagctag cattgggcat ccctttgcct gagctcagga actctgtgac agggggtaca 2340

gcagcctttg aacccagcgt ggattattgt gtggtgaaga ttcctcgatg ggaccttagc 2400

aagttcctgc gagtcagcac aaagattggg agctgcatga agagcgttgg tgaagtcatg 2460

ggcattgggc gttcatttga ggaggccttc cagaaggccc tgcgcatggt ggatgagaac 2520

tgtgtgggct ttgatcacac agtgaaacca gtcagcgata tggagttgga gactccaaca 2580

gataagcgga tttttgtggt ggcagctgct ttgtgggctg gttattcagt ggaccgcctg 2640

tatgagctca cacgcatcga ccgctggttc ctgcaccgaa tgaagcgtat catcgcacat 2700

gcccagctgc tagaacaaca ccgtggacag cctttgccgc cagacctgct gcaacaggcc 2760

aagtgtcttg gcttctcaga caaacagatt gcccttgcag ttctgagcac agagctggct 2820

gttcgcaagc tgcgtcagga actggggatc tgtccagcag tgaaacagat tgacacagtt 2880

gcagctgagt ggccagccca gacaaattac ctatacctaa cgtattgggg caccacccat 2940

gacctcacct ttcgaacacc tcatgtccta gtccttggct ctggcgtcta ccgtattggc 3000

tctagcgttg aatttgactg gtgtgctgta ggctgcatcc agcagctccg aaagatggga 3060

tataagacca tcatggtgaa ctataaccca gagacagtca gcaccgacta tgacatgtgt 3120

gatcgactct actttgatga gatctctttt gaggtggtga tggacatcta tgagctcgag 3180

aaccctgaag gtgtgatcct atccatgggt ggacagctgc ccaacaacat ggccatggcg 3240

ttgcatcggc agcagtgccg ggtgctgggc acctcccctg aagccattga ctcggctgag 3300

aaccgtttca agttttcccg gctccttgac accattggta tcagccagcc tcagtggagg 3360

gagctcagtg acctcgagtc tgctcgccaa ttctgccaga ccgtggggta cccctgtgtg 3420

gtgcgcccct cctatgtgct gagcggtgct gctatgaatg tggcctacac ggatggagac 3480

ctggagcgct tcctgagcag cgcagcagcc gtctccaaag agcatcccgt ggtcatctcc 3540

aagttcatcc aggaggctaa ggagattgac gtggatgccg tggcctctga tggtgtggtg 3600

gcagccatcg ccatctctga gcatgtggag aatgcaggtg tgcattcagg tgatgcgacg 3660

ctggtgaccc ccccacaaga tatcactgcc aaaaccctgg agcggatcaa agccattgtg 3720

catgctgtgg gccaggagct acaggtcaca ggacccttca atctgcagct cattgccaag 3780

gatgaccagc tgaaagttat tgaatgcaac gtacgtgtct ctcgctcctt ccccttcgtt 3840

tccaagacac tgggtgtgga cctagtagcc ttggccacgc gggtcatcat gggggaagaa 3900

gtggaacctg tggggctaat gactggttct ggagtcgtgg gagtaaaggt gcctcagttc 3960

tccttctccc gcttggcggg tgctgacgtg gtgttgggtg tggaaatgac cagtactggg 4020

gaggtggccg gctttgggga gagccgctgt gaggcatacc tcaaggccat gctaagcact 4080

ggctttaaga tccccaagaa gaatatcctg ctgaccattg gcagctataa gaacaaaagc 4140

gagctgctcc caactgtgcg gctactggag agcctgggct acagcctcta tgccagtctc 4200

ggcacagctg acttctacac tgagcatggc gtcaaggtaa cagctgtgga ctggcacttt 4260

gaggaggctg tggatggtga gtgcccacca cagcggagca tcctggagca gctagctgag 4320

aaaaactttg agctggtgat taacctgtca atgcgtggag ctgggggccg gcgtctctct 4380

tcctttgtca ccaaggccta ccgcacccga cgcttggccg ctgacttctc cgtgccccta 4440

atcatcgata tcaagtgcac caaactcttt gtggaggccc taggccagat cgggccagcc 4500

cctcctttga aggtgcatgt tgactgtatg acctcccaaa agcttgtgcg actgccggga 4560

ttgattgatg tccatgtgca cctgcgggaa ccaggtggga cacataagga ggactttgct 4620

tcaggcacag ccgctgccct ggctgggggt atcaccatgg tgtgtgccat gcctaatacc 4680

cggcccccca tcattgacgc ccctgctctg gccctggccc agaagctggc agaggctggc 4740

gcccggtgcg actttgcgct attccttggg gcctcgtctg aaaatgcagg aaccttgggc 4800

accgtggccg ggtctgcagc cgggctgaag ctttacctca atgagacctt ctctgagctg 4860

cggctggaca gcgtggtcca gtggatggag catttcgaga catggccctc ccacctcccc 4920

attgtggctc acgcagagca gcaaaccgtg gctgctgtcc tcatggtggc tcagctcact 4980

cagcgctcag tgcacatatg tcacgtggca cggaaggagg agatcctgct aattaaagct 5040

gcaaaggcac ggggcttgcc agtgacctgc gaggtggctc cccaccacct gttcctaagc 5100

catgatgacc tggagcgcct ggggcctggg aagggggagg tccggcctga gcttggctcc 5160

cgccaggatg tggaagccct gtgggagaac atggctgtca tcgactgctt tgcctcagac 5220

catgctcccc ataccttgga ggagaagtgt gggtccaggc ccccacctgg gttcccaggg 5280

ttagagacca tgctgccact actcctgacg gctgtaagcg agggccggct cagcctggac 5340

gacctgctgc agcgattgca ccacaatcct cggcgcatct ttcacctgcc cccgcaggag 5400

gacacctatg tggaggtgga tctggagcat gagtggacaa ttcccagcca catgcccttc 5460

tccaaggccc actggacacc ttttgaaggg cagaaagtga agggcaccgt ccgccgtgtg 5520

gtcctgcgag gggaggttgc ctatatcgat gggcaggttc tggtaccccc gggctatgga 5580

caggatgtac ggaagtggcc acagggggct gttcctcagc tcccaccctc agcccctgcc 5640

actagtgaga tgaccacgac acctgaaaga ccccgccgtg gcatcccagg gcttcctgat 5700

ggccgcttcc atctgccgcc ccgaatccat cgagcctccg acccaggttt gccagctgag 5760

gagccaaagg agaagtcctc tcggaaggta gccgagccag agctgatggg aacccctgat 5820

ggcacctgct accctccacc accagtaccg agacaggcat ctccccagaa cctggggacc 5880

cctggcttgc tgcaccccca gacctcaccc ctgctgcact cattagtggg ccaacatatc 5940

ctgtccgtcc agcagttcac caaggatcag atgtctcacc tgttcaatgt ggcacacaca 6000

ctgcgtatga tggtgcagaa ggagcggagc ctcgacatcc tgaaggggaa ggtcatggcc 6060

tccatgttct atgaagtgag cacacggacc agcagctcct ttgcagcagc catggcccgg 6120

ctgggaggtg ctgtgctcag cttctcggaa gccacatcgt ccgtccagaa gggcgaatcc 6180

ctggctgact ccgtgcagac catgagctgc tatgccgacg tcgtcgtgct ccggcacccc 6240

cagcctggag cagtggagct ggccgccaag cactgccgga ggccagtgat caatgctggg 6300

gatggggtcg gagagcaccc cacccaggcc ctgctggaca tcttcaccat ccgtgaggag 6360

ctgggaactg tcaatggcat gacgatcacg atggtgggtg acctgaagca cggacgcaca 6420

gtacattccc tggcctgcct gctcacccag tatcgtgtca gcctgcgcta cgtggcacct 6480

cccagcctgc gcatgccacc cactgtgcgg gccttcgtgg cctcccgcgg caccaagcag 6540

gaggaattcg agagcattga ggaggcgctg cctgacactg atgtgctcta catgactcga 6600

atccagaagg aacgatttgg ctctacccag gagtacgaag cttgctttgg tcagttcatc 6660

ctcactcccc acatcatgac ccgggccaag aagaagatgg tggtgatgca cccgatgccc 6720

cgtgtcaacg agataagcgt ggaagtggac tcggatcccc gcgcagccta cttccgccag 6780

gctgagaacg gcatgtacat ccgcatggct ctgttagcca ccgtgctggg ccgtttctag 6840

ggcctggctt cctcagcctc ttctctttag gcccagctgc tgggcaagga attccagtgc 6900

ctcctacggg ggcagcacac ttagatattc ctggacatcc agatagctca catgtgctga 6960

ccacacttca ggctctggac tggagctctc tggcatgggg gtggggcctc agatgctggg 7020

gcccagtctg ccccatcttc attcctgcac cttaaacctg tacagtcatt tttctactga 7080

cttaataaac agccgagctg tcccttga 7108

<210> 12
<211> 2101
<212> DNA
<213> Homo sapiens

<400> 12
acgaacaggc caataaggag ggagcagtgc ggggtttaaa tctgaggcta ggctggctct 60

tctcggcgtg ctgcggcgga acggctgttg gtttctgctg ggtgtaggtc cttggctggt 120

cgggcctccg gtgttctgct tctccccgct gagctgctgc ctggtgaaga ggaagccatg 180

gcgctccgag tcaccaggaa ctcgaaaatt aatgctgaaa ataaggcgaa gatcaacatg 240

gcaggcgcaa agcgcgttcc tacggcccct gctgcaacct ccaagcccgg actgaggcca 300

agaacagctc ttggggacat tggtaacaaa gtcagtgaac aactgcaggc caaaatgcct 360

atgaagaagg aagcaaaacc ttcagctact ggaaaagtca ttgataaaaa actaccaaaa 420

cctcttgaaa aggtacctat gctggtgcca gtgccagtgt ctgagccagt gccagagcca 480

gaacctgagc cagaacctga gcctgttaaa gaagaaaaac tttcgcctga gcctattttg 540

gttgatactg cctctccaag cccaatggaa acatctggat gtgcccctgc agaagaagac 600

ctgtgtcagg ctttctctga tgtaattctt gcagtaaatg atgtggatgc agaagatgga 660

gctgatccaa acctttgtag tgaatatgtg aaagatattt atgcttatct gagacaactt 720

gaggaagagc aagcagtcag accaaaatac ctactgggtc gggaagtcac tggaaacatg 780

agagccatcc taattgactg gctagtacag gttcaaatga aattcaggtt gttgcaggag 840

accatgtaca tgactgtctc cattattgat cggttcatgc agaataattg tgtgcccaag 900

aagatgctgc agctggttgg tgtcactgcc atgtttattg caagcaaata tgaagaaatg 960

taccctccag aaattggtga ctttgctttt gtgactgaca acacttatac taagcaccaa 1020

atcagacaga tggaaatgaa gattctaaga gctttaaact ttggtctggg tcggcctcta 1080

cctttgcact tccttcggag agcatctaag attggagagg ttgatgtcga gcaacatact 1140

ttggccaaat acctgatgga actaactatg ttggactatg acatggtgca ctttcctcct 1200

tctcaaattg cagcaggagc tttttgctta gcactgaaaa ttctggataa tggtgaatgg 1260

acaccaactc tacaacatta cctgtcatat actgaagaat ctcttcttcc agttatgcag 1320

cacctggcta agaatgtagt catggtaaat caaggactta caaagcacat gactgtcaag 1380

aacaagtatg ccacatcgaa gcatgctaag atcagcactc taccacagct gaattctgca 1440

ctagttcaag atttagccaa ggctgtggca aaggtgtaac ttgtaaactt gagttggagt 1500

actatattta caaataaaat tggcaccatg tgccatctgt acatattact gttgcattta 1560

cttttaataa agcttgtggc cccttttact tttttatagc ttaactaatt tgaatgtggt 1620

tacttcctac tgtagggtag cggaaaagtt gtcttaaaag gtatggtggg gatattttta 1680

aaaactcctt ttggtttacc tggggatcca attgatgtat atgtttatat actgggttct 1740

tgttttatat acctggcttt tactttatta atatgagtta ctgaaggtga tggaggtatt 1800

tgaaaatttt acttccatag gacatactgc atgtaagcca agtcatggag aatctgctgc 1860

atagctctat tttaaagtaa aagtctacca ccgaatccct agtccccctg ttttctgttt 1920

cttcttgtga ttgctgccat aattctaagt tatttacttt taccactatt taagttatca 1980

actttagcta gtatcttcaa actttcactt tgaaaaatga gaattttata ttctaagcca 2040

gttttcattt tggttttgtg ttttggttaa taaaacaata ctcaaataca aaaaaaaaaa 2100

a 2101

<210> 13
<211> 1235
<212> DNA
<213> Homo sapiens

<400> 13
gggggggggg ggcacttggc ttcaaagctg gctcttggaa attgagcgga gagcgacgcg 60

gttgttgtag ctgccgctgc ggccgccgcg gaataataag ccgggatcta ccatacccat 120

tgactaacta tggaagatta taccaaaata gagaaaattg gagaaggtac ctatggagtt 180

gtgtataagg gtagacacaa aactacaggt caagtggtag ccatgaaaaa aatcagacta 240

gaaagtgaag aggaaggggt tcctagtact gcaattcggg aaatttctct attaaaggaa 300

cttcgtcatc caaatatagt cagtcttcag gatgtgctta tgcaggattc caggttatat 360

ctcatctttg agtttctttc catggatctg aagaaatact tggattctat ccctcctggt 420

cagtacatgg attcttcact tgttaagagt tatttatacc aaatcctaca ggggattgtg 480

ttttgtcact ctagaagagt tcttcacaga gacttaaaac ctcaaaatct cttgattgat 540

gacaaaggaa caattaaact ggctgatttt ggccttgcca gagcttttgg aatacctatc 600

agagtatata cacatgaggt agtaacactc tggtacagat ctccagaagt attgctgggg 660

tcagctcgtt actcaactcc agttgacatt tggagtatag gcaccatatt tgctgaacta 720

gcaactaaga aaccactttt ccatggggat tcagaaattg atcaactctt caggattttc 780

agagctttgg gcactcccaa taatgaagtg tggccagaag tggaatcttt acaggactat 840

aagaatacat ttcccaaatg gaaaccagga agcctagcat cccatgtcaa aaacttggat 900

gaaaatggct tggatttgct ctcgaaaatg ttaatctatg atccagccaa acgaatttct 960

ggcaaaatgg cactgaatca tccatatttt aatgatttgg acaatcagat taagaagatg 1020

tagctttctg acaaaaagtt tccatatgtt atgtcaacag atagttgtgt ttttattgtt 1080

aactcttgtc tatttttgtc ttatatatat ttctttgtta tcaaacttca gctgtacttc 1140

gtcttctaat ttcaaaaata taacttaaaa atgtaaatat tctatatgaa tttaaatata 1200

attctgtaaa tgtgaaaaaa aaaaaaaaaa aaaaa 1235

<210> 14
<211> 948
<212> DNA
<213> Homo sapiens

<400> 14
   ccattgacta actatggaag attataccaa aatagagaaa attggagaag gtacctatgg 60
   agttgtgtat aagggtagac acaaaactac aggtcaagtg gtagccatga aaaaaatcag 120
   actagaaagt gaagaggaag gggttcctag tactgcaatt cgggaaattt ctctattaaa 180
   ggaacttcgt catccaaata tagtcagtct tcaggatgtg cttatgcagg attccaggtt 240
   atatctcatc tttgagtttc tttccatgga tctgaagaaa tacttggatt ctatccctcc 300
   tggtcagtac atggattctt cacttgttaa ggtagtaaca ctctggtaca gatctccaga 360
   agtattgctg gggtcagctc gttactcaac tccagttgac atttggagta taggcaccat 420
   atttgctgaa ctagcaacta agaaaccact tttccatggg gattcagaaa ttgatcaact 480
   cttcaggatt ttcagagctt tgggcactcc caataatgaa gtgtggccag aagtggaatc 540
   tttacaggac tataagaata catttcccaa atggaaacca ggaagcctag catcccatgt 600
   caaaaacttg gatgaaaatg gcttggattt gctctcgaaa atgttaatct atgatccagc 660
   caaacgaatt tctggcaaaa tggcactgaa tcatccatat tttaatgatt tggacaatca 720
   gattaagaag atgtagcttt ctgacaaaaa gtttccatat gttatgtcaa cagatagttg 780
   tgtttttatt gttaactctt gtctattttt gtcttatata tatttctttg ttatcaaact 840
   tcagctgtac ttcgtcttct aatttcaaaa atataactta aaaatgtaaa tattctatat 900
   gaatttaaat ataattctgt aaatgtgaaa aaaaaaaaaa aaaaaaaa 948

<210> 15
<211> 1686
<212> DNA
<213> Homo sapiens

<400> 15
ccacgcgtcc gggcgtaagc caggcgtgtt aaagccggtc ggaactgctc cggagggcac 60

gggctccgta ggcaccaact gcaaggaccc ctccccctgc gggcgctccc atggcacagt 120

tcgcgttcga gagtgacctg cactcgctgc ttcagctgga tgcacccatc cccaatgcac 180

cccctgcgcg ctggcagcgc aaagccaagg aagccgcagg cccggccccc tcacccatgc 240

gggccgccaa ccgatcccac agcgccggca ggactccggg ccgaactcct ggcaaatcca 300

gttccaaggt tcagaccact cctagcaaac ctggcggtga ccgctatatc ccccatcgca 360

gtgctgccca gatggaggtg gccagcttcc tcctgagcaa ggagaaccag tctgaaaaca 420

gccagacgcc caccaagaag gaacatcaga aagcctgggc tttgaacctg aacggttttg 480

atgtagagga agccaagatc cttcggctca gtggaaaacc acaaaatgcg ccagagggtt 540

atcagaacag actgaaagta ctctacagcc aaaaggccac tcctggctcc agccggaaga 600

cctgccgtta cattccttcc ctgccagacc gtatcctgga tgcgcctgaa atccgaaatg 660

actattacct gaaccttgtg gattggagtt ctgggaatgt actggccgtg gcactggaca 720

acagtgtgta cctgtggagt gcaagctctg gtgacatcct gcagcttttg caaatggagc 780

agcctgggga atatatatcc tctgtggcct ggatcaaaga gggcaactac ttggctgtgg 840

gcaccagcag tgctgaggtg cagctatggg atgtgcagca gcagaaacgg cttcgaaata 900

tgaccagtca ctctgcccga gtgggctccc taagctggaa cagctatatc ctgtccagtg 960

gttcacgttc tggccacatc caccaccatg atgttcgggt agcagaacac catgtggcca 1020

cactgagtgg ccacagccag gaagtgtgtg ggctgcgctg ggccccagat ggacgacatt 1080

tggccagtgg tggtaatgat aacttggtca atgtgtggcc tagtgctcct ggagagggtg 1140

gctgggttcc tctgcagaca ttcacccagc atcaaggggc tgtcaaggcc gtagcatggt 1200

gtccctggca gtccaatgtc ctggcaacag gagggggcac cagtgatcga cacattcgca 1260

tctggaatgt gtgctctggg gcctgtctga gtgccgtgga tgcccattcc caggtgtgct 1320

ccatcctctg gtctccccat tacaaggagc tcatctcagg ccatggcttt gcacagaacc 1380

agctagttat ttggaagtac ccaaccatgg ccaaggtggc tgaactcaaa ggtcacacat 1440

cccgggtcct gagtctgacc atgagcccag atggggccac agtggcatcc gcagcagcag 1500

atgagaccct gaggctatgg cgctgttttg agttggaccc tgcgcggcgg cgggagcggg 1560

agaaggccag tgcagccaaa agcagcctca tccaccaagg catccgctga agaccaaccc 1620

atcacctcag ttgtttttta tttttctaat aaagtcatgt ctcccttcat gttttttttt 1680

ttaaaa 1686

<210> 16
<211> 1389
<212> DNA
<213> Homo sapiens

<400>  16
cgcggacttc tgccaagcac cggctcatgt gaggctcgcg gcacagcgtt ctctgggctc      60

cccagaagcc agcctttcgc tcccggaccc ggcagcccga gcaggagccg tgggaccggg     120

cgccagcacc ctctgcggcg tgtcatgggc ccgcgccgcc ggagccgaaa gcccgaggcc     180

ccgaggaggc gcagcccgag cccgaccccg acccccggcc cctcccggcg gggcccctcc     240

ttaggcgctt cctcccatca acacagtcgg cggagacaag gttggctaaa ggagatccga     300

aagcttcaga agagcacaca cctcttgata aggaagctgc ccttcagccg cctggcaaga     360

gaaatatgtg ttaaattcac tcgtggtgtg gacttcaatt ggcaagccca ggccctattg     420

gccctacaag aggcagcaga agcatttcta gttcatctct ttgaggacgc ctatctcctc     480

accttacatg caggccgagt tactctcttc ccaaaggatg tgcaactggc ccggaggatc     540

cggggccttg aggagggact cggctgagct cctgcaccca gtgtttctgt cagtctttcc     600

tgctcagcca ggggggatga taccggggac tctccagagc catgactaga tccaatggat     660

tctgcgatgc tgtctggact ttgctgtctc tgaacagtat gtgtgtgttg ctttaaatat     720

ttttcttttt tttgagaagg agaagactgc atgactttcc tctgtaacag aggtaatata     780

tgagacaatc aacaccgttc caaaggcctg aaaataattt tcagataaag agactccaag     840

gttgacttta gtttgtgagt tactcatgtg actatttgag gattttgaaa acatcagatt     900

tgctgtggta tgggagaaaa ggttatgtac ttattatttt agctctttct gtaatattta     960

cattttttac catatgtaca tttgtacttt tattttacac ataagggaaa aaataagacc    1020

actttgagca gttgcctgga aggctgggca tttccatcat atagacctct gcccttcaga    1080

gtagcctcac cattagtggc agcatcatgt aactgagtgg actgtgcttg tcaacggatg    1140

tgtagctttt cagaaactta attggggatg aatagaaaac ctgtaagctt tgatgttctg    1200

gttacttcta gtaaattcct gtcaaaatca attcagaaat tctaacttgg agaatttaac    1260

attttactct tgtaaatcat agaagatgta tcataacagt tcagaatttt aaagtacatt    1320

ttcgatgctt ttatgggtat tttgtagttt tctttgtaga gagataataa aaatcaaaat    1380

atttaatga                                                            1389

<210>  17
<211>  10316
<212>  DNA
<213>  Homo sapiens

<400>  17
gagaccagaa gcgggcgaat tgggcaccgg tggcggctgc gggcagtttg aattagactc      60

tgggctccag cccgccgaag ccgcgccaga actgtactct ccgagaggtc gttttcccgt     120

ccccgagagc aagtttattt acaaatgttg gagtaataaa gaaggcagaa caaaatgagc     180

tgggctttgg aagaatggaa agaagggctg cctacaagag ctcttcagaa aattcaagag     240

cttgaaggac agcttgacaa actgaagaag gaaaagcagc aaaggcagtt tcagcttgac     300

agtctcgagg ctgcgctgca gaagcaaaaa cagaaggttg aaaatgaaaa aaccgagggt     360

acaaacctga aaagggagaa tcaaagattg atggaaatat gtgaaagtct ggagaaaact     420

aagcagaaga tttctcatga acttcaagtc aaggagtcac aagtgaattt ccaggaagga 480

caactgaatt caggcaaaaa acaaatagaa aaactggaac aggaacttaa aaggtgtaaa 540

tctgagcttg aaagaagcca acaagctgcg cagtctgcag atgtctctct gaatccatgc 600

aatacaccac aaaaaatttt tacaactcca ctaacaccaa gtcaatatta tagtggttcc 660

aagtatgaag atctaaaaga aaaatataat aaagaggttg aagaacgaaa aagattagag 720

gcagaggtta aagccttgca ggctaaaaaa gcaagccaga ctcttccaca agccaccatg 780

aatcaccgcg acattgcccg gcatcaggct tcatcatctg tgttctcatg gcagcaagag 840

aagaccccaa gtcatctttc atctaattct caaagaactc caattaggag agatttctct 900

gcatcttact tttctgggga acaagaggtg actccaagtc gatcaacttt gcaaataggg 960

aaaagagatg ctaatagcag tttctttgac aattctagca gtcctcatct tttggatcaa 1020

ttaaaagcgc agaatcaaga gctaagaaac aagattaatg agttggaact acgcctgcaa 1080

ggacatgaaa aagaaatgaa aggccaagtg aataagtttc aagaactcca actccaactg 1140

gagaaagcaa aagtggaatt aattgaaaaa gagaaagttt tgaacaaatg tagggatgaa 1200

ctagtgagaa caacagcaca atacgaccag gcgtcaacca agtatactgc attggaacaa 1260

aaactgaaaa aattgacgga agatttgagt tgtcagcgac aaaatgcaga aagtgccaga 1320

tgttctctgg aacagaaaat taaggaaaaa gaaaaggagt ttcaagagga gctctcccgt 1380

caacagcgtt ctttccaaac actggaccag gagtgcatcc agatgaaggc cagactcacc 1440

caggagttac agcaagccaa gaatatgcac aacgtcctgc aggctgaact ggataaactc 1500

acatcagtaa agcaacagct agaaaacaat ttggaagagt ttaagcaaaa gttgtgcaga 1560

gctgaacagg cgttccaggc gagtcagatc aaggagaatg agctgaggag aagcatggag 1620

gaaatgaaga aggaaaacaa cctccttaag agtcactctg agcaaaaggc cagagaagtc 1680

tgccacctgg aggcagaact caagaacatc aaacagtgtt taaatcagag ccagaatttt 1740

gcagaagaaa tgaaagcgaa gaatacctct caggaaacca tgttaagaga tcttcaagaa 1800

aaaataaatc agcaagaaaa ctccttgact ttagaaaaac tgaagcttgc tgtggctgat 1860

ctggaaaagc agcgagattg ttctcaagac cttttgaaga aaagagaaca tcacattgaa 1920

caacttaatg ataagttaag caagacagag aaagagtcca aagccttgct gagtgcttta 1980

gagttaaaaa agaaagaata tgaagaattg aaagaagaga aaactctgtt ttcttgttgg 2040

aaaagtgaaa acgaaaaact tttaactcag atggaatcag aaaaggaaaa cttgcagagt 2100

aaaattaatc acttggaaac ttgtctgaag acacagcaaa taaaaagtca tgaatacaac 2160

gagagagtaa gaacgctgga gatggacaga gaaaacctaa gtgtcgagat cagaaacctt 2220

cacaacgtgt tagacagtaa gtcagtggag gtagagaccc agaaactagc ttatatggag 2280

ctacagcaga aagctgagtt ctcagatcag aaacatcaga aggaaataga aaatatgtgt 2340

ttgaagactt ctcagcttac tgggcaagtt gaagatctag aacacaagct tcagttactg 2400

tcaaatgaaa taatggacaa agaccggtgt taccaagact tgcatgccga atatgagagc 2460

ctcagggatc tgctaaaatc caaagatgct tctctggtga caaatgaaga tcatcagaga 2520

agtcttttgg cttttgatca gcagcctgcc atgcatcatt cctttgcaaa tataattgga 2580

gaacaaggaa gcatgccttc agagaggagt gaatgtcgtt tagaagcaga ccaaagtccg 2640

aaaaattctg ccatcctaca aaatagagtt gattcacttg aattttcatt agagtctcaa 2700

aaacagatga actcagacct gcaaaagcag tgtgaagagt tggtgcaaat caaaggagaa 2760

atagaagaaa atctcatgaa agcagaacag atgcatcaaa gttttgtggc tgaaacaagt 2820

cagcgcatta gtaagttaca ggaagacact tctgctcacc agaatgttgt tgctgaaacc 2880

ttaagtgccc ttgagaacaa ggaaaaagag ctgcaacttt taaatgataa ggtagaaact 2940

gagcaggcag agattcaaga attaaaaaag agcaaccatc tacttgaaga ctctctaaag 3000

gagctacaac ttttatccga aaccctaagc ttggagaaga aagaaatgag ttccatcatt 3060

tctctaaata aaagggaaat tgaagagctg acccaagaga atgggactct taaggaaatt 3120

aatgcatcct taaatcaaga gaagatgaac ttaatccaga aaagtgagag ttttgcaaac 3180

tatatagatg aaagggagaa aagcatttca gagttatctg atcagtacaa gcaagaaaaa 3240

cttattttac tacaaagatg tgaagaaacc ggaaatgcat atgaggatct tagtcaaaaa 3300

tacaaagcag cacaggaaaa gaattctaaa ttagaatgct tgctaaatga atgcactagt 3360

ctttgtgaaa ataggaaaaa tgagttggaa cagctaaagg aagcatttgc aaaggaacac 3420

caagaattct taacaaaatt agcatttgct gaagaaagaa atcagaatct gatgctagag 3480

ttggagacag tgcagcaagc tctgagatct gagatgacag ataaccaaaa caattctaag 3540

agcgaggctg gtggtttaaa gcaagaaatc atgactttaa aggaagaaca aaacaaaatg 3600

caaaaggaag ttaatgactt attacaagag aatgaacagc tgatgaaggt aatgaagact 3660

aaacatgaat gtcaaaatct agaatcagaa ccaattagga actctgtgaa agaaagagag 3720

agtgagagaa atcaatgtaa ttttaaacct cagatggatc ttgaagttaa agaaatttct 3780

ctagatagtt ataatgcgca gttggtgcaa ttagaagcta tgctaagaaa taaggaatta 3840

aaacttcagg aaagtgagaa ggagaaggag tgcctgcagc atgaattaca gacaattaga 3900

ggagatcttg aaaccagcaa tttgcaagac atgcagtcac aagaaattag tggccttaaa 3960

gactgtgaaa tagatgcgga agaaaagtat atttcagggc ctcatgagtt gtcaacaagt 4020

caaaacgaca atgcacacct tcagtgctct ctgcaaacaa caatgaacaa gctgaatgag 4080

ctagagaaaa tatgtgaaat actgcaggct gaaaagtatg aactcgtaac tgagctgaat 4140

gattcaaggt cagaatgtat cacagcaact aggaaaatgg cagaagaggt agggaaacta 4200

ctaaatgaag ttaaaatatt aaatgatgac agtggtcttc tccatggtga gttagtggaa 4260

gacataccag gaggtgaatt tggtgaacaa ccaaatgaac agcaccctgt gtctttggct 4320

ccattggacg agagtaattc ctacgagcac ttgacattgt cagacaaaga agttcaaatg 4380

cactttgccg aattgcaaga gaaattctta tctttacaaa gtgaacacaa aattttacat 4440

gatcagcact gtcagatgag ctctaaaatg tcagagctgc agacctatgt tgactcatta 4500

aaggccgaaa atttggtctt gtcaacgaat ctgagaaact ttcaaggtga cttggtgaag 4560

gagatgcagc tgggcttgga ggaggggctc gttccatccc tgtcatcctc ttgtgtgcct 4620

gacagctcta gtcttagcag tttgggagac tcctcctttt acagagctct tttagaacag 4680

acaggagata tgtctctttt gagtaattta gaaggggctg tttcagcaaa ccagtgcagt 4740

gtagatgaag tattttgcag cagtctgcag gaggagaatc tgaccaggaa agaaacccct 4800

tcggccccag cgaagggtgt tgaagagctt gagtccctct gtgaggtgta ccggcagtcc 4860

ctcgagaagc tagaagagaa aatggaaagt caagggatta tgaaaaataa ggaaattcaa 4920

gagctcgagc agttattaag ttctgaaagg caagagcttg actgccttag gaagcagtat 4980

ttgtcagaaa atgaacagtg gcaacagaag ctgacaagcg tgactctgga gatggagtcc 5040

aagttggcgg cagaaaagaa acagacggaa caactgtcac ttgagctgga agtagcacga 5100

ctccagctac aaggtctgga cttaagttct cggtctttgc ttggcatcga cacagaagat 5160

gctattcaag gccgaaatga gagctgtgac atatcaaaag aacatacttc agaaactaca 5220

gaaagaacac caaagcatga tgttcatcag atttgtgata aagatgctca gcaggacctc 5280

aatctagaca ttgagaaaat aactgagact ggtgcagtga aacccacagg agagtgctct 5340

ggggaacagt ccccagatac caattatgag cctccagggg aagataaaac ccagggctct 5400

tcagaatgca tttctgaatt gtcattttct ggtcctaatg ctttggtacc tatggatttc 5460

ctggggaatc aggaagatat ccataatctt caactgcggg taaaagagac atcaaatgag 5520

aatttgagat tacttcatgt gatagaggac cgtgacagaa aagttgaaag tttgctaaat 5580

gaaatgaaag aattagactc aaaactccat ttacaggagg tacaactaat gaccaaaatt 5640

gaagcatgca tagaattgga aaaaatagtt ggggaactta agaaagaaaa ctcagattta 5700

agtgaaaaat tggaatattt ttcttgtgat caccaggagt tactccagag agtagaaact 5760

tctgaaggcc tcaattctga tttagaaatg catgcagata aatcatcacg tgaagatatt 5820

ggagataatg tggccaaggt gaatgacagc tggaaggaga gatttcttga tgtggaaaat 5880

gagctgagta ggatcagatc ggagaaagct agcattgagc atgaagccct ctacctggag 5940

gctgacttag aggtagttca aacagagaag ctatgtttag aaaaagacaa tgaaaataag 6000

cagaaggtta ttgtctgcct tgaagaagaa ctctcagtgg tcacaagtga gagaaaccag 6060

cttcgtggag aattagatac tatgtcaaaa aaaaccacgg cactggatca gttgtctgaa 6120

aaaatgaagg agaaaacaca agagcttgag tctcatcaaa gtgagtgtct ccattgcatt 6180

caggtggcag aggcagaggt gaaggaaaag acggaactcc ttcagacttt gtcctctgat 6240

gtgagtgagc tgttaaaaga caaaactcat ctccaggaaa agctgcagag tttggaaaag 6300

gactcacagg cactgtcttt gacaaaatgt gagctggaaa accaaattgc acaactgaat 6360

aaagagaaag aattgcttgt caaggaatct gaaagcctgc aggccagact gagtgaatca 6420

gattatgaaa agctgaatgt ctccaaggcc ttggaggccg cactggtgga gaaaggtgag 6480

ttcgcattga ggctgagctc aacacaggag gaagtgcatc agctgagaag aggcatcgag 6540

aaactgagag ttcgcattga ggccgatgaa aagaagcagc tgcacatcgc agagaaactg 6600

aaagaacgcg agcgggagaa tgattcactt aaggataaag ttgagaacct tgaaagggaa 6660

ttgcagatgt cagaagaaaa ccaggagcta gtgattcttg atgccgagaa ttccaaagca 6720

gaagtagaga ctctaaaaac acaaatagaa gagatggcca gaagcctgaa agtttttgaa 6780

ttagaccttg tcacgttaag gtctgaaaaa gaaaatctga caaaacaaat acaagaaaaa 6840

caaggtcagt tgtcagaact agacaagtta ctctcttcat ttaaaagtct gttagaagaa 6900

aaggagcaag cagagataca gatcaaagaa gaatctaaaa ctgcagtgga gatgcttcag 6960

aatcagttaa aggagctaaa tgaggcagta gcagccttgt gtggtgacca agaaattatg 7020

aaggccacag aacagagtct agacccacca atagaggaag agcatcagct gagaaatagc 7080

attgaaaagc tgagagcccg cctagaagct gatgaaaaga agcagctctg tgtcttacaa 7140

caactgaagg aaagtgagca tcatgcagat ttacttaagg gtagagtgga gaaccttgaa 7200

agagagctag agatagccag gacaaaccaa gagcatgcag ctcttgaggc agagaattcc 7260

aaaggagagg tagagaccct aaaagcaaaa atagaaggga tgacccaaag tctgagaggt 7320

ctggaattag atgttgttac tataaggtca gaaaaagaaa atctgacaaa tgaattacaa 7380

aaagagcaag agcgaatatc tgaattagaa ataataaatt catcatttga aaatattttg 7440

caagaaaaag agcaagagaa agtacagatg aaagaaaaat caagcactgc catggagatg 7500

cttcaaacac aattaaaaga gctcaatgag agagtggcag ccctgcataa tgaccaagaa 7560

gcctgtaagg ccaaagagca gaatcttagt agtcaagtag agtgtcttga acttgagaag 7620

gctcagttgc tacaaggcct tgatgaggcc aaaaataatt atattgtttt gcaatcttca 7680

gtgaatggcc tcattcaaga agtagaagat ggcaagcaga aactggagaa gaaggatgaa 7740

gaaatcagta gactgaaaaa tcaaattcaa gaccaagagc agcttgtctc taaactgtcc 7800

caggtggaag gagagcacca actttggaag gagcaaaact tagaactgag aaatctgaca 7860

gtggaattgg agcagaagat ccaagtgcta caatccaaaa atgcctcttt gcaggacaca 7920

ttagaagtgc tgcagagttc ttacaagaat ctagagaatg agcttgaatt gacaaaaatg 7980

gacaaaatgt cctttgttga aaaagtaaac aaaatgactg caaaggaaac tgagctgcag 8040

agggaaatgc atgagatggc acagaaaaca gcagagctgc aagaagaact cagtggagag 8100

aaaaataggc tagctggaga gttgcagtta ctgttggaag aaataaagag cagcaaagat 8160

caattgaagg agctcacact agaaaatagt gaattgaaga agagcctaga ttgcatgcac 8220

aaagaccagg tggaaaagga agggaaagtg agagaggaaa tagctgaata tcagctacgg 8280

cttcatgaag ctgaaaagaa acaccaggct ttgcttttgg acacaaacaa acagtatgaa 8340

gtagaaatcc agacataccg agagaaattg acttctaaag aagaatgtct cagttcacag 8400

aagctggaga tagacctttt aaagtctagt aaagaagagc tcaataattc attgaaagct 8460

actactcaga ttttggaaga attgaagaaa accaagatgg acaatctaaa atatgtaaat 8520

cagttgaaga aggaaaatga acgtgcccag gggaaaatga agttgttgat caaatcctgt 8580

aaacagctgg aagaggaaaa ggagatactg cagaaagaac tctctcaact tcaagctgca 8640

caggagaagc agaaaacagg tactgttatg gataccaagg tcgatgaatt aacaactgag 8700

atcaaagaac tgaaagaaac tcttgaagaa aaaaccaagg aggcagatga atacttggat 8760

aagtactgtt ccttgcttat aagccatgaa aagttagaga aagctaaaga gatgttagag 8820

acacaagtgg cccatctgtg ttcacagcaa tctaaacaag attcccgagg gtctcctttg 8880

ctaggtccag ttgttccagg accatctcca atcccttctg ttactgaaaa gaggttatca 8940

tctggccaaa ataaagcttc aggcaagagg caaagatcca gtggaatatg ggagaatggt 9000

agaggaccaa cacctgctac cccagagagc ttttctaaaa aaagcaagaa agcagtcatg 9060

agtggtattc accctgcaga agacacggaa ggtactgagt ttgagccaga gggacttcca 9120

gaagttgtaa agaaagggtt tgctgacatc ccgacaggaa agactagccc atatatcctg 9180

cgaagaacaa ccatggcaac tcggaccagc ccccgcctgg ctgcacagaa gttagcgcta 9240

tccccactga gtctcggcaa agaaaatctt gcagagtcct ccaaaccaac agctggtggc 9300

agcagatcac aaaaggtcaa agttgctcag cggagcccag tagattcagg caccatcctc 9360

cgagaaccca ccacgaaatc cgtcccagtc aataatcttc ctgagagaag tccgactgac 9420

agccccagag agggcctgag ggtcaagcga ggccgacttg tccccagccc caaagctgga 9480

ctggagtcca acggcagtga gaactgtaag gtccagtgaa ggcactttgt gtgtcagtac 9540

ccctgggagg tgccagtcat tgaatagata aggctgtgcc tacaggactt ctctttagtc 9600

agggcatgct ttattagtga ggagaaaaca attccttaga agtcttaaat atattgtact 9660

ctttagatct cccatgtgta ggtattgaaa aagtttggaa gcactgatca cctgttagca 9720

ttgccattcc tctactgcaa tgtaaatagt ataaagctat gtatataaag ctttttggta 9780

atatgttaca attaaaatga caagcactat atcacaatct ctgtttgtat gtgggtttta 9840

cactaaaaaa atgcaaaaca cattttattc ttctaattaa cagctcctag gaaaatgtag 9900

acttttgctt tatgatattc tatctgtagt atgaggcatg gaatagtttt gtatcgggaa 9960

tttctcagag ctgagtaaaa tgaaggaaaa gcatgttatg tgtttttaag gaaaatgtgc 10020

acacatatac atgtaggagt gtttatcttt ctcttacaat ctgttttaga catctttgct 10080

tatgaaacct gtacatatgt gtgtgtgggt atgtgtttat ttccagtgag ggctgcaggc 10140

ttcctagagg tgtgctatac catgcgtctg tcgttgtgct tttttctgtt tttagaccaa 10200

ttttttacag ttctttggta agcattgtcg tatctggtga tggattaaca tatagccttt 10260

gttttctaat aaaatagtcg ccttcgtttt ctgtaaaaaa aaaaaaaaaa aaaaaa 10316

<210> 18
<211> 2042
<212> DNA
<213> Homo sapiens

<400> 18
ggggccagtc gttcgccgga aagcatttgt ctcccacctc atcataacaa caattaattt 60

cctctggggc ctgaggaggg cagaatttca accttcggtg tgcttgggag tggcgattgt 120

gatttacacg acaaaatgcc gaggtgctcg gtggagtcat ggcagtgccc tttgtggaag 180

actgggactt ggtgcaaacc ctgggagaag gtgcctatgg agaagttcaa cttgctgtga 240

atagagtaac tgaagaagca gtcgcagtga agattgtaga tatgaagcgt gccgtagact 300

gtccagaaaa tattaagaaa gagatctgta tcaataaaat gctaaatcat gaaaatgtag 360

taaaattcta tggtcacagg agagaaggca atatccaata tttatttctg gagtactgta 420

gtggaggaga gctttttgac agaatagagc cagacatagg catgcctgaa ccagatgctc 480

agagattctt ccatcaactc atggcagggg tggtttatct gcatggtatt ggaataactc 540

acagggatat taaaccagaa aatcttctgt tggatgaaag ggataacctc aaaatctcag 600

actttggctt ggcaacagta tttcggtata ataatcgtga gcgtttgttg aacaagatgt 660

gtggtacttt accatatgtt gctccagaac ttctgaagag aagagaattt catgcagaac 720

cagttgatgt ttggtcctgt ggaatagtac ttactgcaat gctcgctgga gaattgccat 780

gggaccaacc cagtgacagc tgtcaggagt attctgactg gaaagaaaaa aaaacatacc 840

tcaacccttg gaaaaaaatc gattctgctc ctctagctct gctgcataaa atcttagttg 900

agaatccatc agcaagaatt accattccag acatcaaaaa agatagatgg tacaacaaac 960

ccctcaagaa aggggcaaaa aggccccgag tcacttcagg tggtgtgtca gagtctccca 1020

gtggattttc taagcacatt caatccaatt tggacttctc tccagtaaac agtgcttcta 1080

gtgaagaaaa tgtgaagtac tccagttctc agccagaacc ccgcacaggt ctttccttat 1140

gggataccag cccctcatac attgataaat tggtacaagg gatcagcttt tcccagccca 1200

catgtcctga tcatatgctt ttgaatagtc agttacttgg caccccagga tcctcacaga 1260

acccctggca gcggttggtc aaaagaatga cacgattctt taccaaattg gatgcagaca 1320

aatcttatca atgcctgaaa gagacttgtg agaagttggg ctatcaatgg aagaaaagtt 1380

gtatgaatca ggttactata tcaacaactg ataggagaaa caataaactc attttcaaag 1440

tgaatttgtt agaaatggat gataaaatat tggttgactt ccggctttct aagggtgatg 1500

gattggagtt caagagacac ttcctgaaga ttaaagggaa gctgattgat attgtgagca 1560

gccagaaggt ttggcttcct gccacatgat cggaccatcg gctctgggga atcctggtga 1620

atatagtgct gctatgttga cattattctt cctagagaag attatcctgt cctgcaaact 1680

gcaaatagta gttcctgaag tgttcacttc cctgtttatc caaacatctt ccaatttatt 1740

ttgtttgttc ggcatacaaa taatacctat atcttaattg taagcaaaac tttggggaaa 1800

ggatgaatag aattcatttg attatttctt catgtgtgtt tagtatctga atttgaaact 1860

catctggtgg aaaccaagtt tcaggggaca tgagttttcc agcttttata cacacgtatc 1920

tcatttttat caaaacattt tgtttaattc aaaaagtaca tatttcttcc atgttgattt 1980

aattctaaga tgaaccaata aagacataat tcttgcaaaa aaaaaaaaaa aaaaaaaaaa 2040

aa 2042

<210> 19
<211> 717
<212> DNA
<213> Homo sapiens

<400> 19
agagcgatca tgtcgcacaa acaaatttac tattcggaca aatacgacga cgaggagttt 60

gagtatcgac atgtcatgct gcccaaggac atagccaagc tggtccctaa aacccatctg 120

atgtctgaat ctgaatggag gaatcttggc gttcagcaga gtcagggatg ggtccattat 180

atgatccatg aaccagaacc tcacatcttg ctgttccggc gcccactacc caagaaacca 240

aagaaatgaa gctggcaagc tacttttcag cctcaagctt tacacagctg tccttacttc 300

ctaacatctt tctgataaca ttattatgtt gccttcttgt ttctcacttt gatatttaaa 360

agatgttcaa tacactgttt gaatgtgctg gtaactgctt tgcttcttga gtagagccac 420

caccaccata gcccagccag atgagtgctc tgtggaccca cagcctaagc tgagtgtgac 480

cccagaagcc acgatgtgct ctgtatccag aacacacttg gcagatggag gaagcatctg 540

agtttgagac catggctgtt acagggatca tgtaaacttg ctgtttttgt tttttctgcc 600

gggtgttgta tgtgtggtga cttgcggatt tatgtttcag tgtactggaa actttccatt 660

ttattcaaga aatctgttca tgttaaaagc cttgattaaa gaggaagttt ttataat 717

<210> 20
<211> 2732
<212> DNA
<213> Homo sapiens

<400> 20
   cccgcctcct ggtaggaggg ggtttccgct tccggcagca gcggctgcag cctcgctctg 60
   gtccctgcgg ctggcggccg agccgtgtgt ctcctcctcc atcgccgcca tattgtctgt 120
   gtgagcagag gggagagcgg ccgccgccgc tgccgcttcc accacagttt gaagaaaaca 180
   ggtctgaaac aaggtcttac ccccagctgc ttctgaacac agtgactgcc agatctccaa 240
   acatcaagtc cagctttgtc cgccaacctg tctgacatgt cgggacccgt gccaagcagg 300
   gccagagttt acacagatgt taatacacac agacctcgag aatactggga ttacgagtca 360
   catgtggtgg aatggggaaa tcaagatgac taccagctgg ttcgaaaatt aggccgaggt 420
   aaatacagtg aagtatttga agccatcaac atcacaaata atgaaaaagt tgttgttaaa 480
   attctcaagc cagtaaaaaa gaagaaaatt aagcgtgaaa taaagatttt ggagaatttg 540
   agaggaggtc ccaacatcat cacactggca gacattgtaa aagaccctgt gtcacgaacc 600
   cccgccttgg tttttgaaca cgtaaacaac acagacttca agcaattgta ccagacgtta 660
   acagactatg atattcgatt ttacatgtat gagattctga aggccctgga ttattgtcac 720
   agcatgggaa ttatgcacag agatgtcaag ccccataatg tcatgattga tcatgagcac 780
   agaaagctac gactaataga ctgggggttg gctgagtttt atcatcctgg ccaagaatat 840
   aatgtccgag ttgcttcccg atacttcaaa ggtcctgagc tacttgtaga ctatcagatg 900
   tacgattata gtttggatat gtggagtttg ggttgtatgc tggcaagtat gatctttcgg 960
   aaggagccat ttttccatgg acatgacaat tatgatcagt tggtgaggat agccaaggtt 1020
   ctggggacag aagatttata tgactatatt gacaaataca acattgaatt agatccacgt 1080
   ttcaatgata tcttgggcag acactctcga aagcgatggg aacgctttgt ccacagtgaa 1140
   aatcagcacc ttgtcagccc tgaggccttg gatttcctgg acaaactgct gcgatatgac 1200
   caccagtcac ggcttactgc aagagaggca atggagcacc cctatttcta cactgttgtg 1260
   aaggaccagg ctcgaatggg ttcatctagc atgccagggg gcagtacgcc cgtcagcagc 1320
   gccaatatga tgtcagggat ttcttcagtg ccaacccctt caccccttgg acctctggca 1380
   ggctcaccag tgattgctgc tgccaacccc cttgggatgc ctgttccagc tgccgctggc 1440
   gctcagcagt aacggcccta tctgtctcct gatgcctgag cagaggtggg ggagtccacc 1500
   ctctccttga tgcagcttgc gcctggcggg gaggggtgaa acacttcaga agcaccgtgt 1560
   ctgaaccgtt gcttgtggat ttatagtagt tcagtcataa aaaaaaaatt ataataggct 1620
   gatttctttt tttctttttt tttttaactc gaacttttca taactcaggg gattccctga 1680
   aaaattacct gcaggtggaa tatttcatgg acaaattttt ttttctcccc tcccaaattt 1740
   agttcctcat cacaaaagaa caaagataaa ccagcctcaa tcccggctgc tgcatttagg 1800
   tggagacttc ttcccattcc caccattgtt cctccaccgt cccacacttt agggggttgg 1860
   tatctcgtgc tcttctccag agattacaaa aatgtagctt ctcaggggag gcaggaagaa 1920
   aggaaggaag gaaagaagga agggaggacc caatctatag gagcagtgga ctgcttgctg 1980
   gtcgcttaca tcactttact ccataagcgc ttcagtgggg ttatcctagt ggctcttgtg 2040
   gaagtgtgtc ttagttacat caagatgttg aaaatctacc caaaatgcag acagatacta 2100
   aaaacttctg ttcagtaaga atcatgtctt actgatctaa ccctaaatcc aactcattta 2160
   tacttttatt tttagttcag tttaaaatgt tgataccttc cctcccaggc tccttacctt 2220
   ggtcttttcc ctgttcatct cccaacatgc tgtgctccat agctggtagg agagggaagg 2280
   caaaatcttt cttagttttc tttgtcttgg ccattttgaa ttcatttagt tactgggcat 2340
   aacttactgc tttttacaaa agaaacaaac attgtctgta caggtttcat gctagagcta 2400
   atgggagatg tggccacact gacttccatt ttaagctttc taccttcttt tcctccgacc 2460
   gtccccttcc ctcacatgcc atccagtgag aagacctgct cctcagtctt gtaaatgtat 2520
   cttgagaggt aggagcagag ccactatctc cattgaagct gaaatggtag acctgtaatt 2580
   gtgggaaaac tataaactct cttgttacag ccccgccacc ccttgctgtg tgtatatata 2640
   taatactttg tccttcatat gtgaaagatc cagtgttgga attctttggt gtaaataaac 2700
   gtttggtttt atttatcaaa aaaaaaaaaa aa 2732

<210> 21
<211> 2849
<212> DNA
<213> Homo sapiens

<400> 21
cccgcctcct ggtaggaggg ggtttccgct tccggcagca gcggctgcag cctcgctctg 60

gtccctgcgg ctggcggccg agccgtgtgt ctcctcctcc atcgccgcca tattgtctgt 120

gtgagcagag gggagagcgg ccgccgccgc tgccgcttcc accacagctc tatcaaggct 180

tgtcaagcag tgtgctcatc acatggtaaa tcatgcagcg tggaacctca taaaatctcc 240

aagaaacatc attcacccat actgactagt ttcacatctc tttgtttgaa gaaaacaggt 300

ctgaaacaag gtcttacccc cagctgcttc tgaacacagt gactgccaga tctccaaaca 360

tcaagtccag ctttgtccgc caacctgtct gacatgtcgg gacccgtgcc aagcagggcc 420

agagtttaca cagatgttaa tacacacaga cctcgagaat actgggatta cgagtcacat 480

gtggtggaat ggggaaatca agatgactac cagctggttc gaaaattagg ccgaggtaaa 540
tacagtgaag tatttgaagc catcaacatc acaaataatg aaaaagttgt tgttaaaatt 600
ctcaagccag taaaaaagaa gaaaattaag cgtgaaataa agattttgga gaatttgaga 660
ggaggtccca acatcatcac actggcagac attgtaaaag accctgtgtc acgaaccccc 720
gccttggttt ttgaacacgt aaacaacaca gacttcaagc aattgtacca gacgttaaca 780
gactatgata ttcgatttta catgtatgag attctgaagg ccctggatta ttgtcacagc 840
atgggaatta tgcacagaga tgtcaagccc cataatgtca tgattgatca tgagcacaga 900
aagctacgac taatagactg gggtttggct gagttttatc atcctggcca agaatataat 960
gtccgagttg cttcccgata cttcaaaggt cctgagctac ttgtagacta tcagatgtac 1020
gattatagtt tggatatgtg gagtttgggt tgtatgctgg caagtatgat ctttcggaag 1080
gagccatttt tccatggaca tgacaattat gatcagttgg tgaggatagc caaggttctg 1140
gggacagaag atttatatga ctatattgac aaatacaaca ttgaattaga tccacgtttc 1200
aatgatatct tgggcagaca ctctcgaaag cgatgggaac gctttgtcca cagtgaaaat 1260
cagcaccttg tcagccctga ggccttggat ttcctggaca aactgctgcg atatgaccac 1320
cagtcacggc ttactgcaag agaggcaatg gagcacccct atttctacac tgttgtgaag 1380
gaccaggctc gaatgggttc atctagcatg ccagggggca gtacgcccgt cagcagcgcc 1440
aatatgatgt cagggatttc ttcagtgcca accccttcac cccttggacc tctggcaggc 1500
tcaccagtga ttgctgctgc caaccccctt gggatgcctg ttccagctgc cgctggcgct 1560
cagcagtaac ggccctatct gtctcctgat gcctgagcag aggtggggga gtccaccctc 1620
tccttgatgc agcttgcgcc tggcggggag gggtgaaaca cttcagaagc accgtgtctg 1680
aaccgttgct tgtggattta tagtagttca gtcataaaaa aaaaattata ataggctgat 1740
tttctttttt cttttttttt ttaactcgaa cttttcataa ctcaggggat tccctgaaaa 1800
attacctgca ggtggaatat ttcatggaca aatttttttt tctcccctcc caaatttagt 1860
tcctcatcac aaaagaacaa agataaacca gcctcaatcc cggctgctgc atttaggtgg 1920
agacttcttc ccattcccac cattgttcct ccaccgtccc acactttagg gggttggtat 1980
ctcgtgctct tctccagaga ttacaaaaat gtagcttctc aggggaggca ggaagaaagg 2040
aaggaaggaa agaaggaagg gaggacccaa tctataggag cagtggactg cttgctggtc 2100
gcttacatca ctttactcca taagcgcttc agtggggtta tcctagtggc tcttgtggaa 2160
gtgtgtctta gttacatcaa gatgttgaaa atctacccaa aatgcagaca gatactaaaa 2220
acttctgttc agtaagaatc atgtcttact gatctaaccc taaatccaac tcatttatac 2280
ttttattttt agttcagttt aaaatgttga taccttccct cccaggctcc ttaccttggt 2340
cttttccctg ttcatctccc aacatgctgt gctccatagc tggtaggaga gggaaggcaa 2400
aatctttctt agttttcttt gtcttggcca ttttgaattc atttagttac tgggcataac 2460
ttactgcttt ttacaaaaga aacaaacatt gtctgtacag gtttcatgct agagctaatg 2520
ggagatgtgg ccacactgac ttccatttta agctttctac cttcttttcc tccgaccgtc 2580

cccttccctc acatgccatc cagtgagaag acctgctcct cagtcttgta aatgtatctt 2640

gagaggtagg agcagagcca ctatctccat tgaagctgaa atggtagacc tgtaattgtg 2700

ggaaaactat aaactctctt gttacagccc cgccacccct tgctgtgtgt atatatataa 2760

tactttgtcc ttcatatgtg aaagatccag tgttggaatt ctttggtgta aataaacgtt 2820

tggttttatt tatcaaaaaa aaaaaaaaa 2849

<210> 22
<211> 2522
<212> DNA
<213> Homo sapiens

<400> 22
cccgcctcct ggtaggaggg ggtttccgct tccggcagca gcggctgcag cctcgctctg 60

gtccctgcgg ctggcggccg agccgtgtgt ctcctcctcc atcgccgcca tattgtctgt 120

gtgagcagag gggagagcgg ccgccgccgc tgccgcttcc accacagaaa tcaagatgac 180

taccagctgg ttcgaaaatt aggccgaggt aaatacagtg aagtatttga agccatcaac 240

atcacaaata atgaaaaagt tgttgttaaa attctcaagc cagtaaaaaa gaagaaaatt 300

aagcgtgaaa taaagatttt ggagaatttg agaggaggtc ccaacatcat cacactggca 360

gacattgtaa aagaccctgt gtcacgaacc cccgccttgg tttttgaaca cgtaaacaac 420

acagacttca agcaattgta ccagacgtta acagactatg atattcgatt ttacatgtat 480

gagattctga aggccctgga ttattgtcac agcatgggaa ttatgcacag agatgtcaag 540

ccccataatg tcatgattga tcatgagcac agaaagctac gactaataga ctggggtttg 600

gctgagtttt atcatcctgg ccaagaatat aatgtccgag ttgcttcccg atacttcaaa 660

ggtcctgagc tacttgtaga ctatcagatg tacgattata gtttggatat gtggagtttg 720

ggttgtatgc tggcaagtat gatctttcgg aaggagccat ttttccatgg acatgacaat 780

tatgatcagt tggtgaggat agccaaggtt ctggggacag aagatttata tgactatatt 840

gacaaataca acattgaatt agatccacgt ttcaatgata tcttgggcag acactctcga 900

aagcgatggg aacgctttgt ccacagtgaa aatcagcacc ttgtcagccc tgaggccttg 960

gatttcctgg acaaactgct gcgatatgac caccagtcac ggcttactgc aagagaggca 1020

atggagcacc cctatttcta cactgttgtg aaggaccagg ctcgaatggg ttcatctagc 1080

atgccagggg gcagtacgcc cgtcagcagc gccaatatga tgtcagggat ttcttcagtg 1140

ccaacccctt caccccttgg acctctggca ggctcaccag tgattgctgc tgccaacccc 1200

cttgggatgc ctgttccagc tgccgctggc gctcagcagt aacggcccta tctgtctcct 1260

gatgcctgag cagaggtggg ggagtccacc ctctccttga tgcagcttgc gcctggcggg 1320

gaggggtgaa acacttcaga agcaccgtgt ctgaaccgtt gcttgtggat ttatagtagt 1380

tcagtcataa aaaaaaaatt ataataggct gattttcttt tttctttttt tttttaactc 1440

gaacttttca taactcaggg gattccctga aaaattacct gcaggtggaa tatttcatgg 1500

acaaattttt ttttctcccc tcccaaattt agttcctcat cacaaaagaa caaagataaa 1560

ccagcctcaa tcccggctgc tgcatttagg tggagacttc ttcccattcc caccattgtt 1620

cctccaccgt cccacacttt agggggttgg tatctcgtgc tcttctccag agattacaaa 1680

aatgtagctt ctcaggggag gcaggaagaa aggaaggaag gaaagaagga agggaggacc 1740

caatctatag gagcagtgga ctgcttgctg gtcgcttaca tcactttact ccataagcgc 1800

ttcagtgggg ttatcctagt ggctcttgtg gaagtgtgtc ttagttacat caagatgttg 1860

aaaatctacc caaaatgcag acagatacta aaaacttctg ttcagtaaga atcatgtctt 1920

actgatctaa ccctaaatcc aactcattta tacttttatt tttagttcag tttaaaatgt 1980

tgataccttc cctcccaggc tccttacctt ggtcttttcc ctgttcatct cccaacatgc 2040

tgtgctccat agctggtagg agagggaagg caaaatcttt cttagttttc tttgtcttgg 2100

ccattttgaa ttcatttagt tactgggcat aacttactgc tttttacaaa agaaacaaac 2160

attgtctgta caggtttcat gctagagcta atgggagatg tggccacact gacttccatt 2220

ttaagctttc taccttcttt tcctccgacc gtccccttcc ctcacatgcc atccagtgag 2280

aagacctgct cctcagtctt gtaaatgtat cttgagaggt aggagcagag ccactatctc 2340

cattgaagct gaaatggtag acctgtaatt gtgggaaaac tataaactct cttgttacag 2400

ccccgccacc ccttgctgtg tgtatatata taatactttg tccttcatat gtgaaagatc 2460

cagtgttgga attctttggt gtaaataaac gtttggtttt atttatcaaa aaaaaaaaaa 2520

aa 2522

<210> 23
<211> 4353
<212> DNA
<213> Homo sapiens

<400> 23
acccactccc gctgccccgt ccggcccgcg ccgcttcctc gcagcagctg ctcccggctc 60

cgcggccgca gcccgcgtgg acgctccgag cgccccccga cggacgggac cggctccctg 120

gcggtcgggc gagcgggcgg caacgctgcc cggccggcag cgctggggtt aagtggccca 180

agtaaaccta gctcggcgat cggcgccgga gattcgcgag cccagcgccc tgcacggccg 240

ccagccggcc tcccgccagc cagccccgac ccgcggctcc gccgcccagc cgcgccccag 300

ccagccctgc ggcaggaaag catgaaggga gacaccaggc atctcaatgg agaggaggac 360

gccggcggga gggaagactc gatcctcgtc aacggggcct gcagcgacca gtcctccgac 420

tcgcccccaa tcctggaggc tatccgcacc ccggagatca gaggccgaag atcaagctcg 480

cgactctcca agagggaggt gtccagtctg ctaagctaca cacaggactt gacaggcgat 540

ggcgacgggg aagatgggga tggctctgac accccagtca tgccaaagct cttccgggaa 600

accaggactc gttcagaaag cccagctgtc cgaactcgaa ataacaacag tgtctccagc 660

cgggagaggc acaggccttc cccacgttcc acccgaggcc ggcagggccg caaccatgtg 720

gacgagtccc ccgtggagtt cccggctacc aggtccctga gacggcgggc aacagcatcg 780

gcaggaacgc catggccgtc ccctcccagc tcttacctta ccatcgacct cacagacgac 840

acagaggaca cacatgggac gccccagagc agcagtaccc cctacgcccg cctagcccag 900

gacagccagc aggggggcat ggagtccccg caggtggagg cagacagtgg agatggagac 960

agttcagagt atcaggatgg gaaggagttt ggaatagggg acctcgtgtg ggaaaagatc 1020

aagggcttct cctggtggcc cgccatggtg gtgtcttgga aggccacctc caagcgacag 1080

gctatgtctg gcatgcggtg ggtccagtgg tttggcgatg gcaagttctc cgaggtctct 1140

gcagacaaac tggtggcact ggggctgttc agccagcact ttaatttggc caccttcaat 1200

aagctcgtct cctatcgaaa agccatgtac catgctctgg agaaagctag ggtgcgagct 1260

ggcaagacct tccccagcag ccctggagac tcattggagg accagctgaa gcccatgttg 1320

gagtgggccc acgggggctt caagcccact gggatcgagg gcctcaaacc caacaacacg 1380

caaccagtgg ttaataagtc gaaggtgcgt cgtgcaggca gtaggaaatt agaatcaagg 1440

aaatacgaga acaagactcg aagacgcaca gctgacgact cagccacctc tgactactgc 1500

cccgcaccca agcgcctcaa gacaaattgc tataacaacg gcaaagaccg aggggatgaa 1560

gatcagagcc gagaacaaat ggcttcagat gttgccaaca acaagagcag cctggaagat 1620

ggctgtttgt cttgtggcag gaaaaacccc gtgtccttcc accctctctt tgaggggggg 1680

ctctgtcaga catgccggga tcgcttcctt gagctgtttt acatgtatga tgacgatggc 1740

tatcagtctt actgcactgt gtgctgcgag ggccgagagc tgctgctttg cagcaacacg 1800

agctgctgcc ggtgtttctg tgtggagtgc ctggaggtgc tggtgggcac aggcacagcg 1860

gccgaggcca agcttcagga gccctggagc tgttacatgt gtctcccgca gcgctgtcat 1920

ggcgtcctgc ggcgccggaa ggactggaac gtgcgcctgc aggccttctt caccagtgac 1980

acggggcttg aatatgaagc ccccaagctg taccctgcca ttcccgcagc ccgaaggcgg 2040

cccattcgag tcctgtcatt gtttgatggc atcgcgacag gctacctagt cctcaaagag 2100

ttgggcataa aggtaggaaa gtacgtcgct tctgaagtgt gtgaggagtc cattgctgtt 2160

ggaaccgtga agcacgaggg gaatatcaaa tacgtgaacg acgtgaggaa catcacaaag 2220

aaaaatattg aagaatgggg cccatttgac ttggtgattg gcggaagccc atgcaacgat 2280

ctctcaaatg tgaatccagc caggaaaggc ctgtatgagg gtacaggccg gctcttcttc 2340

gaattttacc acctgctgaa ttactcacgc cccaaggagg gtgatgaccg gccgttcttc 2400

tggatgtttg agaatgttgt agccatgaag gttggcgaca agagggacat ctcacggttc 2460

ctggagtgta atccagtgat gattgatgcc atcaaagttt ctgctgctca cagggcccga 2520

tacttctggg gcaacctacc cgggatgaac aggcccgtga tagcatcaaa gaatgataaa 2580

ctcgagctgc aggactgctt ggaatacaat aggatagcca agttaaagaa agtacagaca 2640

ataaccacca agtcgaactc gatcaaacag gggaaaaacc aacttttccc tgttgtcatg 2700

aatggcaaag aagatgtttt gtggtgcact gagctcgaaa ggatctttgg ctttcctgtg 2760

cactacacag acgtgtccaa catgggccgt ggtgcccgcc agaagctgct gggaaggtcc 2820

tggagcgtgc ctgtcatccg acacctcttc gcccctctga aggactactt tgcatgtgaa 2880

tagttccagc caggccccaa gcccactggg gtgtgtggca gagccaggac ccaggaggtg 2940

tgattcctga aggcatcccc aggccctgct cttcctcagc tgtgtgggtc ataccgtgta 3000

cctcagttcc ctcttgctca gtgggggcag agccacctga ctcttgcagg ggtagcctga 3060
ggtgccgcct ccttgtgcac aaatcagacc tggctgcttg gagcagccta acacggtgct 3120
catttttctt tctcctaaaa ctttaaaact tgaagtaggt agcaacgtgg cttttttttt 3180
ttcccttcct gggtctacca ctcagagaaa caatggctaa gataccaaaa ccacagtgcc 3240
gacagctctc caatactcag gttaatgctg aaaaatcatc caagacagtt attgcaagag 3300
tttaattttt gaaaactggc tactgctctg tgtttacaga cgtgtgcagt tgtaggcatg 3360
tagctacagg acatttttaa gggcccagga tcgttttttc ccagggcaag cagaagagaa 3420
aatgttgtat atgtctttta cccggcacat tccccttgcc taaatacaag ggctggagtc 3480
tgcacgggac ctattagagt attttccaca atgatgatga tttcagcagg gatgacgtca 3540
tcatcacatt cagggctatt ttttccccca caaacccaag ggcaggggcc actcttagct 3600
aaatccctcc ccgtgactgc aatagaaccc tctggggagc tcaggaaggg gtgtgctgag 3660
ttctataata taagctgcca tatattttgt agacaagtat ggctcctcca tatctccctc 3720
ttccctagga gaggagtgtg aagcaaggag cttagataag acaccccctc aaacccattc 3780
cctctccagg agacctaccc tccacaggca caggtcccca gatgagaagt ctgctaccct 3840
catttctcat ctttttacta aactcagagg cagtgacagc agtcagggac agacatacat 3900
ttctcatacc ttccccacat ctgagagatg acagggaaaa ctgcaaagct cggtgctccc 3960
tttggagatt ttttaatcct tttttattcc ataagaagtc gtttttaggg agaacgggaa 4020
ttcagacaag ctgcatttca gaaatgctgt cataatggtt tttaacacct tttactcttc 4080
ttactggtgc tattttgtag aataaggaac aacgttgaca agttttgtgg ggctttttat 4140
acacttttta aaatctcaaa cttctatttt tatgtttaac gttttcatta aaattttttt 4200
tgtaactgga gccacgacgt aacaaatatg gggaaaaaac tgtgccttgt ttcaacagtt 4260
tttgctaatt tttaggctga aagatgacgg atgcctagag tttaccttat gtttaattaa 4320
aatcagtatt tgtctaaaaa aaaaaaaaaa aaa 4353

<210> 24
<211> 4293
<212> DNA
<213> Homo sapiens

<400> 24
acccactccc gctgccccgt ccggcccgcg ccgcttcctc gcagcagctg ctcccggctc 60
cgcggccgca gcccgcgtgg acgctccgag cgccccccga cggacgggac cggctccctg 120
gcggtcgggc gagcgggcgg caacgctgcc cggccggcag cgctggggtt aagtggccca 180
agtaaaccta gctcggcgat cggcgccgga gattcgcgag cccagcgccc tgcacggccg 240
ccagccggcc tcccgccagc cagccccgac ccgcggctcc gccgcccagc cgcgccccag 300
ccagccctgc ggcaggaaag catgaaggga gacaccaggc atctcaatgg agaggaggac 360
gccggcggga gggaagactc gatcctcgtc aacggggcct gcagcgacca gtcctccgac 420
tcgcccccaa tcctggaggc tatccgcacc ccggagatca gaggccgaag atcaagctcg 480

cgactctcca agagggaggt gtccagtctg ctaagctaca cacaggactt gacaggcgat 540
ggcgacgggg aagatgggga tggctctgac accccagtca tgccaaagct cttccgggaa 600
accaggactc gttcagaaag cccagctgtc cgaactcgaa ataacaacag tgtctccagc 660
cgggagaggc acaggccttc cccacgttcc acccgaggcc ggcagggccg caaccatgtg 720
gacgagtccc ccgtggagtt cccggctacc aggtccctga gacggcgggc aacagcatcg 780
gcaggaacgc catggccgtc ccctcccagc tcttacctta ccatcgacct cacagacgac 840
acagaggaca cacatgggac gccccagagc agcagtaccc cctacgcccg cctagcccag 900
gacagccagc aggggggcat ggagtccccg caggtggagg cagacagtgg agatggagac 960
agttcagagt atcaggatgg gaaggagttt ggaatagggg acctcgtgtg gggaaagatc 1020
aagggcttct cctggtggcc cgccatggtg gtgtcttgga aggccacctc caagcgacag 1080
gctatgtctg gcatgcggtg ggtccagtgg tttggcgatg gcaagttctc cgaggtctct 1140
gcagacaaac tggtggcact ggggctgttc agccagcact ttaatttggc caccttcaat 1200
aagctcgtct cctatcgaaa agccatgtac catgctctgg agaaagctag ggtgcgagct 1260
ggcaagacct tccccagcag ccctggagac tcattggagg accagctgaa gcccatgttg 1320
gagtgggccc acgggggctt caagcccact gggatcgagg gcctcaaacc caacaacacg 1380
caaccagaga acaagactcg aagacgcaca gctgacgact cagccacctc tgactactgc 1440
cccgcaccca agcgcctcaa gacaaattgc tataacaacg gcaaagaccg aggggatgaa 1500
gatcagagcc gagaacaaat ggcttcagat gttgccaaca acaagagcag cctggaagat 1560
ggctgtttgt cttgtggcag gaaaaacccc gtgtccttcc accctctctt tgaggggggg 1620
ctctgtcaga catgccggga tcgcttcctt gagctgtttt acatgtatga tgacgatggc 1680
tatcagtctt actgcactgt gtgctgcgag ggccgagagc tgctgctttg cagcaacacg 1740
agctgctgcc ggtgtttctg tgtggagtgc ctggaggtgc tggtgggcac aggcacagcg 1800
gccgaggcca agcttcagga gccctggagc tgttacatgt gtctcccgca gcgctgtcat 1860
ggcgtcctgc ggcgccggaa ggactggaac gtgcgcctgc aggccttctt caccagtgac 1920
acggggcttg aatatgaagc ccccaagctg taccctgcca ttcccgcagc ccgaaggcgg 1980
cccattcgag tcctgtcatt gtttgatggc atcgcgacag gctacctagt cctcaaagag 2040
ttgggcataa aggtaggaaa gtacgtcgct tctgaagtgt gtgaggagtc cattgctgtt 2100
ggaaccgtga agcacgaggg gaatatcaaa tacgtgaacg acgtgaggaa catcacaaag 2160
aaaaatattg aagaatgggg cccatttgac ttggtgattg gcggaagccc atgcaacgat 2220
ctctcaaatg tgaatccagc caggaaaggc ctgtatgagg gtacaggccg gctcttcttc 2280
gaattttacc acctgctgaa ttactcacgc cccaaggagg gtgatgaccg gccgttcttc 2340
tggatgtttg agaatgttgt agccatgaag gttggcgaca agagggacat ctcacggttc 2400
ctggagtgta atccagtgat gattgatgcc atcaaagttt ctgctgctca cagggcccga 2460
tacttctggg gcaacctacc cgggatgaac aggcccgtga tagcatcaaa gaatgataaa 2520
ctcgagctgc aggactgctt ggaatacaat aggatagcca agttaaagaa agtacagaca 2580

ataaccacca agtcgaactc gatcaaacag ggaaaaaacc aacttttccc tgttgtcatg 2640

aatggcaaag aagatgtttt gtggtgcact gagctcgaaa ggatctttgg ctttcctgtg 2700

cactacacag acgtgtccaa catgggccgt ggtgcccgcc agaagctgct gggaaggtcc 2760

tggagcgtgc ctgtcatccg acacctcttc gcccctctga aggactactt tgcatgtgaa 2820

tagttccagc caggccccaa gcccactggg gtgtgtggca gagccaggac ccaggaggtg 2880

tgattcctga aggcatcccc aggccctgct cttcctcagc tgtgtgggtc ataccgtgta 2940

cctcagttcc ctcttgctca gtgggggcag agccacctga ctcttgcagg ggtagcctga 3000

ggtgccgcct ccttgtgcac aaatcagacc tggctgcttg gagcagccta acacggtgct 3060

cattttttct tctcctaaaa ctttaaaact tgaagtaggt agcaacgtgg cttttttttt 3120

ttcccttcct gggtctacca ctcagagaaa caatggctaa gataccaaaa ccacagtgcc 3180

gacagctctc caatactcag gttaatgctg aaaaatcatc caagacagtt attgcaagag 3240

tttaattttt gaaaactggc tactgctctg tgtttacaga cgtgtgcagt tgtaggcatg 3300

tagctacagg acatttttaa gggcccagga tcgttttttc ccagggcaag cagaagagaa 3360

aatgttgtat atgtctttta cccggcacat tccccttgcc taaatacaag ggctggagtc 3420

tgcacgggac ctattagagt attttccaca atgatgatga tttcagcagg gatgacgtca 3480

tcatcacatt cagggctatt ttttccccca caaacccaag ggcaggggcc actcttagct 3540

aaatccctcc ccgtgactgc aatagaaccc tctggggagc tcaggaaggg gtgtgctgag 3600

ttctataata taagctgcca tatattttgt agacaagtat ggctcctcca tatctccctc 3660

ttccctagga gaggagtgtg aagcaaggag cttagataag acaccccctc aaacccattc 3720

cctctccagg agacctaccc tccacaggca caggtcccca gatgagaagt ctgctaccct 3780

catttctcat ctttttacta aactcagagg cagtgacagc agtcagggac agacatacat 3840

ttctcatacc ttccccacat ctgagagatg acagggaaaa ctgcaaagct cggtgctccc 3900

tttggagatt ttttaatcct tttttattcc ataagaagtc gtttttaggg agaacgggaa 3960

ttcagacaag ctgcatttca gaaatgctgt cataatggtt tttaacacct tttactcttc 4020

ttactggtgc tattttgtag aataaggaac aacgttgaca agttttgtgg ggctttttat 4080

acacttttta aaatctcaaa cttctatttt tatgtttaac gttttcatta aaattttttt 4140

tgtaactgga gccacgacgt aacaaatatg ggaaaaaaac tgtgccttgt ttcaacagtt 4200

tttgctaatt tttaggctga aagatgacgg atgcctagag tttaccttat gtttaattaa 4260

aatcagtatt tgtctaaaaa aaaaaaaaaa aaa 4293

<210> 25
<211> 4104
<212> DNA
<213> Homo sapiens

<400> 25
acccactccc gctgccccgt ccggcccgcg ccgcttcctc gcagcagctg ctcccggctc 60

cgcggccgca gcccgcgtgg acgctccgag cgccccccga cggacgggac cggctccctg 120

gcggtcgggc gagcgggcgg caacgctgcc cggccggcag cgctggggtt aagtggccca 180

agtaaaccta gctcggcgat cggcgccgga gattcgcgag cccagcgccc tgcacggccg 240

ccagccggcc tcccgccagc cagccccgac ccgcggctcc gccgcccagc cgcgccccag 300

ccagccctgc ggcaggaaag catgaaggga gacaccaggc atctcaatgg agaggaggac 360

gccggcggga gggaagactc gatcctcgtc aacggggcct gcagcgacca gtcctccgac 420

tcgcccccaa tcctggaggc tatccgcacc ccggagatca gaggccgaag atcaagctcg 480

cgactctcca agagggaggt gtccagtctg ctaagctaca cacaggactt gacaggcgat 540

ggcgacgggg aagatgggga tggctctgac accccagtca tgccaaagct cttccgggaa 600

accaggactc gttcagaaag cccagctgtc cgaactcgaa ataacaacag tgtctccagc 660

cgggagaggc acaggccttc cccacgttcc acccgaggcc ggcagggccg caaccatgtg 720

gacgagtccc ccgtggagtt cccggctacc aggtccctga gacggcgggc aacagcatcg 780

gcaggaacgc catggccgtc ccctcccagc tcttacctta ccatcgacct cacagacgac 840

acagaggaca cacatgggac gccccagagc agcagtaccc cctacgcccg cctagcccag 900

gacagccagc aggggggcat ggagtccccg caggtggagg cagacagtgg agatggagac 960

agttcagagt atcaggatgg gaaggagttt ggaatagggg acctcgtgtg ggaaaagatc 1020

aagggcttct cctggtggcc cgccatggtg gtgtcttgga aggccacctc caagcgacag 1080

gctatgtctg gcatgcggtg ggtccagtgg tttggcgatg gcaagttctc cgaggtctct 1140

gcagacaaac tggtggcact ggggctgttc agccagcact ttaatttggc caccttcaat 1200

aagctcgtct cctatcgaaa agccatgtac catgctctgg agaaagctag ggtgcgagct 1260

ggcaagacct tccccagcag ccctggagac tcattggagg accagctgaa gcccatgttg 1320

gagtgggccc acgggggctt caagcccact gggatcgagg gcctcaaacc caacaacacg 1380

caaccagaga acaagactcg aagacgcaca gctgacgact cagccacctc tgactactgc 1440

cccgcaccca agcgcctcaa gacaaattgc tataacaacg gcaaagaccg aggggatgaa 1500

gatcagagcc gagaacaaat ggcttcagat gttgccaaca acaagagcag cctggaagat 1560

ggctgtttgt cttgtggcag gaaaaacccc gtgtccttcc accctctctt tgaggggggg 1620

ctctgtcaga catgccggga tcgcttcctt gagctgtttt acatgtatga tgacgatggc 1680

tatcagtctt actgcactgt gtgctgcgag ggccgagagc tgctgctttg cagcaacacg 1740

agctgctgcc ggtgtttctg tgtggagtgc ctggaggtgc tggtgggcac aggcacagcg 1800

gccgaggcca agcttcagga gccctggagc tgttacatgt gtctcccgca gcgctgtcat 1860

ggcgtcctgc ggcgccggaa ggactggaac gtgcgcctgc aggccttctt caccagtgac 1920

acggggcttg aatatgaagc ccccaagctg taccctgcca ttcccgcagc ccgaaggcgg 1980

cccattcgag tcctgtcatt gtttgatggc atcgcgacag gctacctagt cctcaaagag 2040

ttgggcataa aggtaggaaa gtacgtcgct tctgaagtgt gtgaggagtc cattgctgtt 2100

ggaaccgtga agcacgaggg gaatatcaaa tacgtgaacg acgtgaggaa catcacaaag 2160

aaaaatattg aagaatgggg cccatttgac ttggtgattg gcggaagccc atgcaacgat 2220

ctctcaaatg tgaatccagc caggaaaggc ctgtatgagg gtacaggccg gctcttcttc 2280

gaattttacc acctgctgaa ttactcacgc cccaaggagg gtgatgaccg gccgttcttc 2340

tggatgtttg agaatgttgt agccatgaag gttggcgaca agagggacat ctcacggttc 2400

ctggagtgta atccagtgat gattgatgcc atcaaagttt ctgctgctca cagggcccga 2460

tacttctggg gcaacctacc cgggatgaac aggatctttg gctttcctgt gcactacaca 2520

gacgtgtcca acatgggccg tggtgcccgc cagaagctgc tgggaaggtc ctggagcgtg 2580

cctgtcatcc gacacctctt cgcccctctg aaggactact ttgcatgtga atagttccag 2640

ccaggcccca agcccactgg ggtgtgtggc agagccagga cccaggaggt gtgattcctg 2700

aaggcatccc caggccctgc tcttcctcag ctgtgtgggt cataccgtgt acctcagttc 2760

cctcttgctc agtgggggca gagccacctg actcttgcag gggtagcctg aggtgccgcc 2820

tccttgtgca caaatcagac ctggctgctt ggagcagcct aacacggtgc tcattttttc 2880

ttctcctaaa actttaaaac ttgaagtagg tagcaacgtg gctttttttt tttcccttcc 2940

tgggtctacc actcagagaa acaatggcta agataccaaa accacagtgc cgacagctct 3000

ccaatactca ggttaatgct gaaaaatcat ccaagacagt tattgcaaga gtttaatttt 3060

tgaaaactgg ctactgctct gtgtttacag acgtgtgcag ttgtaggcat gtagctacag 3120

gacattttta agggcccagg atcgtttttt cccagggcaa gcagaagaga aaatgttgta 3180

tatgtctttt acccggcaca ttccccttgc ctaaatacaa gggctggagt ctgcacggga 3240

cctattagag tattttccac aatgatgatg atttcagcag ggatgacgtc atcatcacat 3300

tcagggctat tttttccccc acaaacccaa gggcaggggc cactcttagc taaatccctc 3360

cccgtgactg caatagaacc ctctggggag ctcaggaagg ggtgtgctga gttctataat 3420

ataagctgcc atatattttg tagacaagta tggctcctcc atatctccct cttccctagg 3480

agaggagtgt gaagcaagga gcttagataa gacaccccct caaacccatt ccctctccag 3540

gagacctacc ctccacaggc acaggtcccc agatgagaag tctgctaccc tcatttctca 3600

tctttttact aaactcagag gcagtgacag cagtcaggga cagacataca tttctcatac 3660

cttccccaca tctgagagat gacagggaaa actgcaaagc tcggtgctcc ctttggagat 3720

tttttaatcc ttttttattc cataagaagt cgtttttagg gagaacggga attcagacaa 3780

gctgcatttc agaaatgctg tcataatggt ttttaacacc ttttactctt cttactggtg 3840

ctattttgta gaataaggaa caacgttgac aagttttgtg gggcttttta tacacttttt 3900

aaaatctcaa acttctattt ttatgtttaa cgttttcatt aaaatttttt ttgtaactgg 3960

agccacgacg taacaaatat ggggaaaaaa ctgtgccttg tttcaacagt ttttgctaat 4020

ttttaggctg aaagatgacg gatgcctaga gtttacctta tgtttaatta aaatcagtat 4080

ttgtctaaaa aaaaaaaaaa aaaa 4104

<210> 26
<211> 4267
<212> DNA
<213> Homo sapiens

<400> 26

gaagggagag agcaaacaaa ggggtcagga gggaaaataa tgcactggct tcctgagccc 60

ctgcagaggc tgagcaggga gagggggcca gggccagagg ggacagaggc tggcggcaga 120

cgggccggga caggcaggtc ctaaatggca ttgtttgaag gggccggcta attgcacaga 180

gcagtctgag cctgagaccc cagccctggc ctccccactc tgtcctgggt gctggcgtct 240

gagccttcgg gacagcctgt ccacatggaa ccaagtcctg agcctccaag cttggaaagc 300

atgaagggag acaccaggca tctcaatgga gaggaggacg ccggcgggag ggaagactcg 360

atcctcgtca acggggcctg cagcgaccag tcctccgact cgcccccaat cctggaggct 420

atccgcaccc cggagatcag aggccgaaga tcaagctcgc gactctccaa gagggaggtg 480

tccagtctgc taagctacac acaggacttg acaggcgatg gcgacgggga agatggggat 540

ggctctgaca ccccagtcat gccaaagctc ttccgggaaa ccaggactcg ttcagaaagc 600

ccagctgtcc gaactcgaaa taacaacagt gtctccagcc gggagaggca caggccttcc 660

ccacgttcca cccgaggccg gcagggccgc aaccatgtgg acgagtcccc cgtggagttc 720

ccggctacca ggtccctgag acggcgggca acagcatcgg caggaacgcc atggccgtcc 780

cctcccagct cttaccttac catcgacctc acagacgaca cagaggacac acatgggacg 840

ccccagagca gcagtacccc ctacgcccgc ctagcccagg acagccagca ggggggcatg 900

gagtccccgc aggtggaggc agacagtgga gatggagaca gttcagagta tcaggatggg 960

aaggagtttg gaatagggga cctcgtgtgg ggaaagatca agggcttctc ctggtggccc 1020

gccatggtgg tgtcttggaa ggccacctcc aagcgacagg ctatgtctgg catgcggtgg 1080

gtccagtggt ttggcgatgg caagttctcc gaggtctctg cagacaaact ggtggcactg 1140

gggctgttca gccagcactt taatttggcc accttcaata agctcgtctc ctatcgaaaa 1200

gccatgtacc atgctctgga gaaagctagg gtgcgagctg gcaagacctt ccccagcagc 1260

cctggagact cattggagga ccagctgaag cccatgttgg agtgggccca cgggggcttc 1320

aagcccactg ggatcgaggg cctcaaaccc aacaacacgc aaccagagaa caagactcga 1380

agacgcacag ctgacgactc agccacctct gactactgcc ccgcacccaa gcgcctcaag 1440

acaaattgct ataacaacgg caaagaccga ggggatgaag atcagagccg agaacaaatg 1500

gcttcagatg ttgccaacaa caagagcagc ctggaagatg gctgtttgtc ttgtggcagg 1560

aaaaaccccg tgtccttcca ccctctcttt gagggggggc tctgtcagac atgccgggat 1620

cgcttccttg agctgtttta catgtatgat gacgatggct atcagtctta ctgcactgtg 1680

tgctgcgagg gccgagagct gctgctttgc agcaacacga gctgctgccg gtgtttctgt 1740

gtggagtgcc tggaggtgct ggtgggcaca ggcacagcgg ccgaggccaa gcttcaggag 1800

ccctggagct gttacatgtg tctcccgcag cgctgtcatg gcgtcctgcg gcgccggaag 1860

gactggaacg tgcgcctgca ggccttcttc accagtgaca cggggcttga atatgaagcc 1920

cccaagctgt accctgccat tcccgcagcc cgaaggcggc ccattcgagt cctgtcattg 1980

tttgatggca tcgcgacagg ctacctagtc ctcaaagagt tgggcataaa ggtaggaaag 2040

tacgtcgctt ctgaagtgtg tgaggagtcc attgctgttg gaaccgtgaa gcacgagggg 2100

aatatcaaat acgtgaacga cgtgaggaac atcacaaaga aaaatattga agaatggggc 2160
ccatttgact tggtgattgg cggaagccca tgcaacgatc tctcaaatgt gaatccagcc 2220
aggaaaggcc tgtatgaggg tacaggccgg ctcttcttcg aattttacca cctgctgaat 2280
tactcacgcc ccaaggaggg tgatgaccgg ccgttcttct ggatgtttga gaatgttgta 2340
gccatgaagg ttggcgacaa gagggacatc tcacggttcc tggagtgtaa tccagtgatg 2400
attgatgcca tcaaagtttc tgctgctcac agggcccgat acttctgggg caacctaccc 2460
gggatgaaca ggcccgtgat agcatcaaag aatgataaac tcgagctgca ggactgcttg 2520
gaatacaata ggatagccaa gttaaagaaa gtacagacaa taaccaccaa gtcgaactcg 2580
atcaaacagg ggaaaaacca acttttccct gttgtcatga atggcaaaga agatgttttg 2640
tggtgcactg agctcgaaag gatctttggc tttcctgtgc actacacaga cgtgtccaac 2700
atgggccgtg gtgcccgcca gaagctgctg ggaaggtcct ggagcgtgcc tgtcatccga 2760
cacctcttcg cccctctgaa ggactacttt gcatgtgaat agttccagcc aggccccaag 2820
cccactgggg tgtgtggcag agccaggacc caggaggtgt gattcctgaa ggcatcccca 2880
ggccctgctc ttcctcagct gtgtgggtca taccgtgtac ctcagttccc tcttgctcag 2940
tgggggcaga gccacctgac tcttgcaggg gtagcctgag gtgccgcctc cttgtgcaca 3000
aatcagacct ggctgcttgg agcagcctaa cacggtgctc attttttctt ctcctaaaac 3060
tttaaaactt gaagtaggta gcaacgtggc tttttttttt tcccttcctg ggtctaccac 3120
tcagagaaac aatggctaag ataccaaaac cacagtgccg acagctctcc aatactcagg 3180
ttaatgctga aaaatcatcc aagacagtta ttgcaagagt ttaatttttg aaaactggct 3240
actgctctgt gtttacagac gtgtgcagtt gtaggcatgt agctacagga catttttaag 3300
ggcccaggat cgttttttcc cagggcaagc agaagagaaa atgttgtata tgtcttttac 3360
ccggcacatt ccccttgcct aaatacaagg gctggagtct gcacgggacc tattagagta 3420
ttttccacaa tgatgatgat ttcagcaggg atgacgtcat catcacattc agggctattt 3480
tttcccccac aaacccaagg gcaggggcca ctcttagcta aatccctccc cgtgactgca 3540
atagaaccct ctggggagct caggaagggg tgtgctgagt tctataatat aagctgccat 3600
atattttgta gacaagtatg gctcctccat atctccctct tccctaggag aggagtgtga 3660
agcaaggagc ttagataaga caccccctca aacccattcc ctctccagga gacctaccct 3720
ccacaggcac aggtccccag atgagaagtc tgctaccctc atttctcatc tttttactaa 3780
actcagaggc agtgacagca gtcagggaca gacatacatt tctcatacct tccccacatc 3840
tgagagatga cagggaaaac tgcaaagctc ggtgctccct ttggagattt tttaatcctt 3900
ttttattcca taagaagtcg tttttaggga gaacgggaat tcagacaagc tgcatttcag 3960
aaatgctgtc ataatggttt ttaacacctt ttactcttct tactggtgct attttgtaga 4020
ataaggaaca acgttgacaa gttttgtggg gctttttata cactttttaa aatctcaaac 4080
ttctattttt atgtttaacg ttttcattaa aatttttttt gtaactggag ccacgacgta 4140
acaaatatgg ggaaaaaact gtgccttgtt tcaacagttt ttgctaattt ttaggctgaa 4200

agatgacgga tgcctagagt ttaccttatg tttaattaaa atcagtattt gtctaaaaaa 4260

aaaaaaa 4267

<210> 27
<211> 3916
<212> DNA
<213> Homo sapiens

<400> 27
agagtgctga tttagaagaa tacaaatcat ggctgaaaat agtgtattaa catccactac 60

tgggaggact agcttggcag actcttccat ttttgattct aaagttactg agatttccaa 120

ggaaaactta cttattggat ctacttcata tgtagaagaa gagatgcctc agattgaaac 180

aagagtgata ttggttcaag aagctggaaa acaagaagaa cttataaaag ccttaaagga 240

cattaaagtg ggctttgtaa agatggagtc agtggaagaa tttgaaggtt tggattctcc 300

ggaatttgaa aatgtatttg tagtcacgga ctttcaggat tctgtcttta atgacctcta 360

caaggctgat tgtagagtta ttggaccacc agttgtatta aattgttcac aaaaaggaga 420

gcctttgcca ttttcatgtc gcccgttgta ttgtacaagt atgatgaatc tagtactatg 480

ctttactgga tttaggaaaa aagaagaact agtcaggttg gtgacattgg tccatcacat 540

gggtggagtt attcgaaaag actttaattc aaaagttaca catttggtgg caaattgtac 600

acaaggagaa aaattcaggg ttgctgtgag tctaggtact ccaattatga agccagaatg 660

gatttataaa gcttgggaaa ggcggaatga acaggatttc tatgcagcag ttgatgactt 720

tagaaatgaa tttaaagttc ctccatttca agattgtatt ttaagtttcc tgggattttc 780

agatgaagag aaaaccaata tggaagaaat gactgaaatg caaggaggta aatatttacc 840

gcttggagat gaaagatgca ctcaccttgt agttgaagag aatatagtaa aagatcttcc 900

ctttgaacct tcaaagaaac tttatgttgt caagcaagag tggttctggg gaagcattca 960

aatggatgcc cgagctggag aaactatgta tttatatgaa aaggcaaata ctcctgagct 1020

caagaaatca gtgtcaatgc tttctctaaa tacccctaac agcaatcgca aacgacgtcg 1080

tttaaaagaa acacttgctc agctttcaag agagacagac gtgtcaccat ttccaccccg 1140

taagcgccca tcagctgagc attccctttc catagggtca ctcctagata tctccaacac 1200

accagagtct agcattaact atggagacac cccaaagtct tgtactaagt cttctaaaag 1260

ctccactcca gttccttcaa agcagtcagc aaggtggcaa gttgcaaaag agctttatca 1320

aactgaaagt aattatgtta atatattggc aacaattatt cagttatttc aagtaccatt 1380

ggaagaggaa ggacaacgtg gtggacctat ccttgcacca gaggagatta agactatttt 1440

tggtagcatc ccagatatct ttgatgtaca cactaagata aaggatgatc ttgaagacct 1500

tatagttaat tgggatgaga gcaaaagcat tggtgacatt tttctgaaat attcaaaaga 1560

tttggtaaaa acctaccctc cctttgtaaa cttctttgaa atgagcaagg aaacaattat 1620

taaatgtgaa aaacagaaac caagatttca tgcttttctc aagataaacc aagcaaaacc 1680

agaatgtgga cggcagagcc ttgttgaact tcttatccga ccagtacaga ggttacccag 1740

tgttgcatta cttttaaatg atcttaagaa gcatacagct gatgaaaatc cagacaaaag 1800

cactttagaa aaagctattg gatcactgaa ggaagtaatg acgcatatta atgaggataa 1860

gagaaaaaca gaagctcaaa agcaaatttt tgatgttgtt tatgaagtag atggatgccc 1920

agctaatctt ttatcttctc accgaagctt agtacagcgg gttgaaacaa tttctctagg 1980

tgagcacccc tgtgacagag gagaacaagt aactctcttc ctcttcaatg attgcctaga 2040

gatagcaaga aaacggcaca aggttattgg cacttttagg agtcctcatg gccaaacccg 2100

acccccagct tctcttaagc atattcacct aatgcctctt tctcagatta agaaggtatt 2160

ggacataaga gagacagaag attgccataa tgcttttgcc ttgcttgtga ggccaccaac 2220

agagcaggca aatgtgctac tcagtttcca gatgacatca gatgaacttc caaaagaaaa 2280

ctggctaaag atgctgtgtc gacatgtagc taacaccatt tgtaaagcag atgctgagaa 2340

tcttatttat actgctgatc cagaatcctt tgaagtaaat acaaaagata tggacagtac 2400

attgagtaga gcatcaagag caataaaaaa gacttcaaaa aaggttacaa gagcattctc 2460

tttctccaaa actccaaaaa gagctcttcg aagggctctt atgacatccc acggctcagt 2520

ggagggaaga agtccttcca gcaatgataa gcatgtaatg agtcgtcttt ctagcacatc 2580

atcattagca ggtatccctt ctccctccct tgtcagcctt ccttccttct ttgaaaggag 2640

aagtcatacg ttaagtagat ctacaactca tttgatatga agcgttacca aaatcttaaa 2700

ttatagaaat gtatagacac ctcatactca aataagaaac tgacttaaat ggtacttgta 2760

attagcactt ggtgaaagct ggaaggaaga taaataacac taaactatgc tatttgattt 2820

ttcttcttga aagagtaagg tttacctgtt acattttcaa gttaattcat gtaaaaaatg 2880

atagtgattt tgatgtaatt tatctcttgt ttgaatctgt cattcaaagg ccaataattt 2940

aagttgctat cagctgatat tagtagcttt gcaaccctga tagagtaaat aaattttatg 3000

ggcgggtgcc aaatactgct gtgaatctat ttgtatagta tccatgaatg aatttatgga 3060

aatagatatt tgtgcagctc aatttatgca gagattaaat gacatcataa tactggatga 3120

aaacttgcat agaattctga ttaaatagtg ggtctgtttc acatgtgcag tttgaagtat 3180

ttaaataacc actcctttca cagtttattt tcttctcaag cgttttcaag atctagcatg 3240

tggattttaa aagatttgcc ctcattaaca agaataacat ttaaaggaga ttgtttcaaa 3300

atatttttgc aaattgagat aaggacagaa agattgagaa acattgtata ttttgcaaaa 3360

acaagatgtt tgtagctgtt tcagagagag tacggtatat ttatggtaat tttatccact 3420

agcaaatctt gatttagttt gatagtgtgt ggaattttat tttgaaggat aagaccatgg 3480

gaaaattgtg gtaaagactg tttgtaccct tcatgaaata attctgaagt tgccatcagt 3540

tttactaatc ttctgtgaaa tgcatagata tgcgcatgtt caacttttta ttgtggtctt 3600

ataattaaat gtaaaattga aaattcattt gctgtttcaa agtgtgatat ctttcacaat 3660

agccttttta tagtcagtaa ttcagaataa tcaagttcat atggataaat gcatttttat 3720

ttcctatttc tttagggagt gctacaaatg tttgtcactt aaatttcaag tttctgtttt 3780

aatagttaac tgactataga ttgttttcta tgccatgtat gtgccacttc tgagagtagt 3840

aaatgactct ttgctacatt ttaaaagcaa ttgtattagt aagaactttg taaataaata 3900

cctaaaaccc aagtgt                                                    3916

<210>  28
<211>  2695
<212>  DNA
<213>  Homo sapiens

<400>  28
   caaataaaag cgatggcgat tgggctgccg cgtttggcgc tcggtccggt cgcgtccgac      60
   acccggtggg actcagaagg cagtggagcc ccggcggcgg cggcggcggc gcgcgggggc     120
   gacgcgcggg aacaacgcga gtcggcgcgc gggacgaaga ataatcatgg gccagactgg     180
   gaagaaatct gagaagggac cagtttgttg gcggaagcgt gtaaaatcag agtacatgcg     240
   actgagacag ctcaagaggt tcagacgagc tgatgaagta aagagtatgt ttagttccaa     300
   tcgtcagaaa attttggaaa gaacggaaat cttaaaccaa gaatggaaac agcgaaggat     360
   acagcctgtg cacatcctga cttctgtgag ctcattgcgc gggactaggg agtgttcggt     420
   gaccagtgac ttggattttc caacacaagt catcccatta aagactctga atgcagttgc     480
   ttcagtaccc ataatgtatt cttggtctcc cctacagcag aattttatgg tggaagatga     540
   aactgtttta cataacattc cttatatggg agatgaagtt ttagatcagg atggtacttt     600
   cattgaagaa ctaataaaaa attatgatgg gaaagtacac ggggatagag aatgtgggtt     660
   tataaatgat gaaatttttg tggagttggt gaatgccctt ggtcaatata atgatgatga     720
   cgatgatgat gatggagacg atcctgaaga aagagaagaa aagcagaaag atctggagga     780
   tcaccgagat gataaagaaa gccgcccacc tcggaaattt ccttctgata aaatttttga     840
   agccatttcc tcaatgtttc cagataaggg cacagcagaa gaactaaagg aaaaatataa     900
   agaactcacc gaacagcagc tcccaggcgc acttcctcct gaatgtaccc ccaacataga     960
   tggaccaaat gctaaatctg ttcagagaga gcaaagctta cactcctttc atacgctttt    1020
   ctgtaggcga tgttttaaat atgactgctt cctacatcgt aagtgcaatt attcttttca    1080
   tgcaacaccc aacacttata agcggaagaa cacagaaaca gctctagaca acaaaccttg    1140
   tggaccacag tgttaccagc atttggaggg agcaaaggag tttgctgctg ctctcaccgc    1200
   tgagcggata aagaccccac caaaacgtcc aggaggccgc agaagaggac ggcttcccaa    1260
   taacagtagc aggcccagca cccccaccat taatgtgctg gaatcaaagg atacagacag    1320
   tgatagggaa gcagggactg aaacgggggg agagaacaat gataaagaag aagaagagaa    1380
   gaaagatgaa acttcgagct cctctgaagc aaattctcgg tgtcaaacac caataaagat    1440
   gaagccaaat attgaacctc ctgagaatgt ggagtggagt ggtgctgaag cctcaatgtt    1500
   tagagtcctc attggcactt actatgacaa tttctgtgcc attgctaggt taattgggac    1560
   caaaacatgt agacaggtgt atgagtttag agtcaaagaa tctagcatca tagctccagc    1620
   tcccgctgag gatgtggata ctcctccaag gaaaaagaag aggaaacacc ggttgtgggc    1680
   tgcacactgc agaaagatac agctgaaaaa ggacggctcc tctaaccatg tttacaacta    1740
   tcaaccctgt gatcatccac ggcagccttg tgacagttcg tgcccttgtg tgatagcaca    1800
   aaatttttgt gaaaagtttt gtcaatgtag ttcagagtgt caaaaccgct ttccgggatg 1860
   ccgctgcaaa gcacagtgca acaccaagca gtgcccgtgc tacctggctg tccgagagtg 1920
   tgaccctgac ctctgtctta cttgtggagc cgctgaccat tgggacagta aaaatgtgtc 1980
   ctgcaagaac tgcagtattc agcggggctc caaaaagcat ctattgctgg caccatctga 2040
   cgtggcaggc tgggggattt ttatcaaaga tcctgtgcag aaaaatgaat tcatctcaga 2100
   atactgtgga gagattattt ctcaagatga agctgacaga agagggaaag tgtatgataa 2160
   atacatgtgc agctttctgt tcaacttgaa caatgatttt gtggtggatg caacccgcaa 2220
   gggtaacaaa attcgttttg caaatcattc ggtaaatcca aactgctatg caaaagttat 2280
   gatggttaac ggtgatcaca ggataggtat ttttgccaag agagccatcc agactggcga 2340
   agagctgttt tttgattaca gatacagcca ggctgatgcc ctgaagtatg tcggcatcga 2400
   aagagaaatg gaaatccctt gacatctgct acctcctccc ccctcctctg aaacagctgc 2460
   cttagcttca ggaacctcga gtactgtggg caatttagaa aaagaacatg cagtttgaaa 2520
   ttctgaattt gcaaagtact gtaagaataa tttatagtaa tgagtttaaa aatcaacttt 2580
   ttattgcctt ctcaccagct gcaaagtgtt ttgtaccagt gaatttttgc aataatgcag 2640
   tatggtacat ttttcaactt tgaataaaga atacttgaac ttgtcaaaaa aaaaa 2695

<210> 29
<211> 2563
<212> DNA
<213> Homo sapiens

<400> 29
caaataaaag cgatggcgat tgggctgccg cgtttggcgc tcggtccggt cgcgtccgac 60

acccggtggg actcagaagg cagtggagcc ccggcggcgg cggcggcggc gcgcgggggc 120

gacgcgcggg aacaacgcga gtcggcgcgc gggacgaaga ataatcatgg gccagactgg 180

gaagaaatct gagaagggac cagtttgttg gcggaagcgt gtaaaatcag agtacatgcg 240

actgagacag ctcaagaggt tcagacgagc tgatgaagta aagagtatgt ttagttccaa 300

tcgtcagaaa attttggaaa gaacggaaat cttaaaccaa gaatggaaac agcgaaggat 360

acagcctgtg cacatcctga cttctgtgag ctcattgcgc gggactaggg aggtggaaga 420

tgaaactgtt ttacataaca ttccttatat gggagatgaa gttttagatc aggatggtac 480

tttcattgaa gaactaataa aaaattatga tgggaaagta cacggggata gagaatgtgg 540

gtttataaat gatgaaattt ttgtggagtt ggtgaatgcc cttggtcaat ataatgatga 600

tgacgatgat gatgatggag acgatcctga agaaagagaa gaaaagcaga aagatctgga 660

ggatcaccga gatgataaag aaagccgccc acctcggaaa tttccttctg ataaaatttt 720

tgaagccatt tcctcaatgt ttccagataa gggcacagca gaagaactaa aggaaaaata 780

taaagaactc accgaacagc agctcccagg cgcacttcct cctgaatgta cccccaacat 840

agatggacca aatgctaaat ctgttcagag agagcaaagc ttacactcct ttcatacgct 900

tttctgtagg cgatgtttta aatatgactg cttcctacat ccttttcatg caacacccaa 960

cacttataag cggaagaaca cagaaacagc tctagacaac aaaccttgtg gaccacagtg 1020

ttaccagcat ttggagggag caaaggagtt tgctgctgct ctcaccgctg agcggataaa 1080

gaccccacca aaacgtccag gaggccgcag aagaggacgg cttcccaata acagtagcag 1140

gcccagcacc cccaccatta atgtgctgga atcaaaggat acagacagtg atagggaagc 1200

agggactgaa acggggggag agaacaatga taaagaagaa gaagagaaga aagatgaaac 1260

ttcgagctcc tctgaagcaa attctcggtg tcaaacacca ataaagatga agccaaatat 1320

tgaacctcct gagaatgtgg agtggagtgg tgctgaagcc tcaatgttta gagtcctcat 1380

tggcacttac tatgacaatt tctgtgccat tgctaggtta attgggacca aaacatgtag 1440

acaggtgtat gagtttagag tcaaagaatc tagcatcata gctccagctc ccgctgagga 1500

tgtggatact cctccaagga aaaagaagag gaaacaccgg ttgtgggctg cacactgcag 1560

aaagatacag ctgaaaaagg acggctcctc taaccatgtt tacaactatc aaccctgtga 1620

tcatccacgg cagccttgtg acagttcgtg cccttgtgtg atagcacaaa atttttgtga 1680

aaagttttgt caatgtagtt cagagtgtca aaaccgcttt ccgggatgcc gctgcaaagc 1740

acagtgcaac accaagcagt gcccgtgcta cctggctgtc cgagagtgtg accctgacct 1800

ctgtcttact tgtggagccg ctgaccattg ggacagtaaa aatgtgtcct gcaagaactg 1860

cagtattcag cggggctcca aaaagcatct attgctggca ccatctgacg tggcaggctg 1920

gggatttttt atcaaagatc ctgtgcagaa aaatgaattc atctcagaat actgtggaga 1980

gattatttct caagatgaag ctgacagaag agggaaagtg tatgataaat acatgtgcag 2040

ctttctgttc aacttgaaca atgattttgt ggtggatgca acccgcaagg gtaacaaaat 2100

tcgttttgca aatcattcgg taaatccaaa ctgctatgca aaagttatga tggttaacgg 2160

tgatcacagg ataggtattt ttgccaagag agccatccag actggcgaag agctgttttt 2220

tgattacaga tacagccagg ctgatgccct gaagtatgtc ggcatcgaaa gagaaatgga 2280

aatcccttga catctgctac ctcctccccc ctcctctgaa acagctgcct tagcttcagg 2340

aacctcgagt actgtgggca atttagaaaa agaacatgca gtttgaaatt ctgaatttgc 2400

aaagtactgt aagaataatt tatagtaatg agtttaaaaa tcaacttttt attgccttct 2460

caccagctgc aaagtgtttt gtaccagtga atttttgcaa taatgcagta tggtacattt 2520

ttcaactttg aataaagaat acttgaactt gtcaaaaaaa aaa 2563

<210> 30
<211> 2649
<212> DNA
<213> Homo sapiens

<400> 30
aggaggacct gggggtgtgg cagcgaggaa gggccgagcc acggactgtg gggccgaaac 60

tcgctcccgc ccaccctttc tcgaggctgt ggcctccgcg agagccgagc gggccgcacc 120

gccggccgtg cgactgcccc agtcagacac gaccccggct tctagcccgc ctaagcctgt 180

ttggggttgc tgactcgttt cctccccgag tttcccgcgg gaactaactc ttcaagagga 240

ccaaccgcag cccagagctt cgcagacccg gccaaccaga ggcgaggttg agagcccggc 300

gggccgcggg gagagagcgt cccatctgtc ctggaaagcc tgggcgggtg gattgggacc 360
ccgagagaag caggggagct cggcggggtg cagaagtgcc caggcccctc cccgctgggg 420
ttgggagctt gggcaggcca gcttcaccct tcctaagtcc gcttctggtc tccgggccca 480
gcctcggcca ccatgtcccg ccagaccacc tctgtgggct ccagctgcct ggacctgtgg 540
agggaaaaga atgaccggct cgttcgacag gccaaggtgg ctcagaactc cggtctgact 600
ctgaggcgac agcagttggc tcaggatgca ctggaagggc tcagagggct cctccatagt 660
ctgcaagggc tccctgcagc tgttcctgtt cttcccttgg agctgactgt cacctgcaac 720
ttcattatcc tgagggcaag cttggcccag ggtttcacag aggatcaggc ccaggatatc 780
cagcggagcc tagagagagt gctggagaca caggagcagc aggggcccag gttggaacag 840
gggctcaggg agctgtggga ctctgtcctt cgtgcttcct gccttctgcc ggagctgctg 900
tctgccctgc accgcctggt tggcctgcag gctgccctct ggttgagtgc tgaccgtctt 960
ggggacctgg ccttgttact agagaccctg aatggcagcc agagtggagc ctctaaggat 1020
ctgctgttac ttctgaaaac ttggagtccc ccagctgagg aattagatgc tccattgacc 1080
ctgcaggatg cccagggatt gaaggatgtc ctcctgacag catttgccta ccgccaaggt 1140
ctccaggagc tgatcacagg gaacccagac aaggcactaa gcagccttca tgaagcggcc 1200
tcaggcctgt gtccacggcc tgtgttggtc caggtgtaca cagcactggg gtcctgtcac 1260
cgtaagatgg gaaatccaca gagagcactg ttgtacttgg ttgcagccct gaaagaggga 1320
tcagcctggg gtcctccact tctggaggcc tctaggctct atcagcaact gggggacaca 1380
acagcagagc tggagagtct ggagctgcta gttgaggcct tgaatgtccc atgcagttcc 1440
aaagccccgc agtttctcat tgaggtagaa ttactactgc caccacctga cctagcctca 1500
ccccttcatt gtggcactca gagccagacc aagcacatac tagcaagcag gtgcctacag 1560
acggggaggg caggagacgc tgcagagcat tacttggacc tgctggccct gttgctggat 1620
agctcggagc caaggttctc cccacccccc tcccctccag ggccctgtat gcctgaggtg 1680
tttttggagg cagcggtagc actgatccag gcaggcagag cccaagatgc cttgactcta 1740
tgtgaggagt tgctcagccg cacatcatct ctgctaccca agatgtcccg gctgtgggaa 1800
gatgccagaa aaggaaccaa ggaactgcca tactgcccac tctgggtctc tgccacccac 1860
ctgcttcagg gccaggcctg ggttcaactg ggtgcccaaa aagtggcaat tagtgaattt 1920
agcaggtgcc tcgagctgct cttccgggcc acacctgagg aaaaagaaca aggggcagct 1980
ttcaactgtg agcagggatg taagtcagat gcggcactgc agcagcttcg ggcagccgcc 2040
ctaattagtc gtggactgga atgggtagcc agcggccagg ataccaaagc cttacaggac 2100
ttcctcctca gtgtgcagat gtgcccaggt aatcgagaca cttactttca cctgcttcag 2160
actctgaaga ggctagatcg gagggatgag gccactgcac tctggtggag gctggaggcc 2220
caaactaagg ggtcacatga agatgctctg tggtctctcc ccctgtacct agaaagctat 2280
ttgagctgga tccgtccctc tgatcgtgac gccttccttg aagaatttcg gacatctctg 2340
ccaaagtctt gtgacctgta gctgccacgt tttgaagagc ttgagctggg tccccagtgg 2400

gctgtctctc tgtggggagg gctttctgct tcaccatcat taggaatgtg accattccta 2460

tataattcct ggactggtga gattggtggt aggcctgtga aatttgccct agttactacc 2520

attctcgttt tggaggaaac aatctctgcc accaccaagt cattgacttt gctcgaggca 2580

ccttttttcc tgtttctcct tttctgttgt cgagtaaaat ttcatattta taaaaaaaaa 2640

aaaaaaaaa 2649

<210> 31
<211> 2265
<212> DNA
<213> Homo sapiens

<400> 31
agtcctgcga tttcgggtgt agagggagca ggggcctgcg gggacctggt gtgggtggag 60

tggggacaag cggtggagaa gggtacgcca gggtcgctga gagactctgt tctccctgga 120

gggactggtt gccatgagag cagccgtctg aggggacgca gcctgcacta cgcgccccaa 180

gaggctgtgc gtggcgagca ggtcacgtga cgggagcgcg ggctttggaa ggcggctgaa 240

cgtcaggcca cccgccgcta agctgagaag ggagagcgag cttaggaccg cctgcccggg 300

gcaaccccga accaagcttt agccgccgag gccgcgtgtc ccaaaggcca gtcatccctc 360

ctctgtgttg ccatgggaat tcaaggcctg gccaaactaa ttgctgatgt ggcccccagt 420

gccatccggg agaatgacat caagagctac tttggccgta aggtggccat tgatgcctct 480

atgagcattt atcagttcct gattgctgtt cgccagggtg gggatgtgct gcagaatgag 540

gagggtgaga ccaccagcca cctgatgggc atgttctacc gcaccattcg catgatggag 600

aacggcatca agcccgtgta tgtctttgat ggcaagccgc cacagctcaa gtcaggcgag 660

ctggccaaac gcagtgagcg gcgggctgag gcagagaagc agctgcagca ggctcaggct 720

gctggggccg agcaggaggt ggaaaaattc actaagcggc tggtgaaggt cactaagcag 780

cacaatgatg agtgcaaaca tctgctgagc ctcatgggca tcccttatct tgatgcaccc 840

agtgaggcag aggccagctg tgctgccctg gtgaaggctg gcaaagtcta tgctgcggct 900

accgaggaca tggactgcct caccttcggc agccctgtgc taatgcgaca cctgactgcc 960

agtgaagcca aaaagctgcc aatccaggaa ttccacctga gccggattct gcaggagctg 1020

ggcctgaacc aggaacagtt tgtggatctg tgcatcctgc taggcagtga ctactgtgag 1080

agtatccggg gtattgggcc caagcgggct gtggacctca tccagaagca caagagcatc 1140

gaggagatcg tgcggcgact tgaccccaac aagtaccctg tgccagaaaa ttggctccac 1200

aaggaggctc accagctctt cttggaacct gaggtgctgg acccagagtc tgtggagctg 1260

aagtggagcg agccaaatga agaagagctg atcaagttca tgtgtggtga aaagcagttc 1320

tctgaggagc gaatccgcag tggggtcaag aggctgagta agagccgcca aggcagcacc 1380

cagggccgcc tggatgattt cttcaaggtg accggctcac tctcttcagc taagcgcaag 1440

gagccagaac ccaagggatc cactaagaag aaggcaaaga ctggggcagc agggaagttt 1500

aaaaggggaa aataaatgtg tttccccatt atacctcctt caccccagaa tatttgccgt 1560

cttgtaccct taagagctac agctagagaa accttcacgg ggtggagaga ggattctaag 1620

gcttttctag cgtgaccctt ttcagtagtg ctagtccctt ttttacttga tcttaatggc 1680

aagaaggcca cagaggtact tttccttttt tagctcagga aaatatgtca ggctcaaacc 1740

acttctcagg cagtttaatg gacactaagt ccattgttac atgaaagtga tagatagcaa 1800

caagttttgg agaagagaga gggagataaa agggggagac aaaagatgta cagaaatgat 1860

ttcctggctg gccaactggt ggccagtggg aggtgatggt ggacctagac tgtgcttttc 1920

tgtcttgttc agccttgacc caccttgaga gagagccacc aggaaggcgc atcttagcag 1980

atgggaggaa ctgctgagag aagatgggca gaaagctgga gcccctggag ttggctgtgt 2040

ctgtgtttgt gactgattac tggctgtgtc ttgggtgggc agaaactcga acttgctatg 2100

taatttgtgt ctagttattc agaggagtaa gatggtgatg ttcacctggc aatcagctga 2160

gttgagactt tggaataaga cactggtttt catgcgctgt ttttgtttta aagttatgaa 2220

gaaaaaagtc aataaaattc taaaagtaaa aaaaaaaaaa aaaaa 2265

<210> 32
<211> 3527
<212> DNA
<213> Homo sapiens

<400> 32
actgaaagct ccggtgccag accccacccc cggccccggc ccgggacccc ctcccctccc 60

gggatccccc ggggttccca ccccgcccgc accgccgggg acccggccgg tccggcgcga 120

gcccccgtcc ggggccctgg ctcggccccc aggttggagg agcccggagc ccgccttcgg 180

agctacggcc taacggcggc ggcgactgca gtctggaggg tccacacttg tgattctcaa 240

tggagagtga aaacgcagat tcataatgaa aactagcccc cgtcggccac tgattctcaa 300

aagacggagg ctgccccttc ctgttcaaaa tgccccaagt gaaacatcag aggaggaacc 360

taagagatcc cctgcccaac aggagtctaa tcaagcagag gcctccaagg aagtggcaga 420

gtccaactct tgcaagtttc cagctgggat caagattatt aaccacccca ccatgcccaa 480

cacgcaagta gtggccatcc ccaacaatgc taatattcac agcatcatca cagcactgac 540

tgccaaggga aaagagagtg gcagtagtgg gcccaacaaa ttcatcctca tcagctgtgg 600

gggagcccca actcagcctc caggactccg gcctcaaacc caaaccagct atgatgccaa 660

aaggacagaa gtgaccctgg agaccttggg accaaaacct gcagctaggg atgtgaatct 720

tcctagacca cctggagccc tttgcgagca gaaacgggag acctgtgcag atggtgaggc 780

agcaggctgc actatcaaca atagcctatc caacatccag tggcttcgaa agatgagttc 840

tgatggactg ggctcccgca gcatcaagca agagatggag gaaaaggaga attgtcacct 900

ggagcagcga caggttaagg ttgaggagcc ttcgagacca tcagcgtcct ggcagaactc 960

tgtgtctgag cggccaccct actcttacat ggccatgata caattcgcca tcaacagcac 1020

tgagaggaag cgcatgactt tgaaagacat ctatacgtgg attgaggacc actttcccta 1080

ctttaagcac attgccaagc caggctggaa gaactccatc cgccacaacc tttccctgca 1140

cgacatgttt gtccgggaga cgtctgccaa tggcaaggtc tccttctgga ccattcaccc 1200

cagtgccaac cgctacttga cattggacca ggtgtttaag ccactggacc cagggtctcc 1260
acaattgccc gagcacttgg aatcacagca gaaacgaccg aatccagagc tccgccggaa 1320
catgaccatc aaaaccgaac tccccctggg cgcacggcgg aagatgaagc cactgctacc 1380
acgggtcagc tcatacctgg tacctatcca gttcccggtg aaccagtcac tggtgttgca 1440
gccctcggtg aaggtgccat tgcccctggc ggcttccctc atgagctcag agcttgcccg 1500
ccatagcaag cgagtccgca ttgcccccaa ggtgctgcta gctgaggagg ggatagctcc 1560
tctttcttct gcaggaccag ggaaagagga gaaactcctg tttggagaag ggttttctcc 1620
tttgcttcca gttcagacta tcaaggagga agaaatccag cctggggagg aaatgccaca 1680
cttagcgaga cccatcaaag tggagagccc tcccttggaa gagtggccct ccccggcccc 1740
atctttcaaa gaggaatcat ctcactcctg ggaggattcg tcccaatctc ccaccccaag 1800
acccaagaag tcctacagtg ggcttaggtc cccaacccgg tgtgtctcgg aaatgcttgt 1860
gattcaacac agggagagga gggagaggag ccggtctcgg aggaaacagc atctactgcc 1920
tccctgtgtg gatgagccgg agctgctctt ctcagagggg cccagtactt cccgctgggc 1980
cgcagagctc ccgttcccag cagactcctc tgaccctgcc tcccagctca gctactccca 2040
ggaagtggga ggacctttta agacacccat taaggaaacg ctgcccatct cctccacccc 2100
gagcaaatct gtcctcccca gaacccctga atcctggagg ctcacgcccc cagccaaagt 2160
agggggactg gatttcagcc cagtacaaac ctcccagggt gcctctgacc ccttgcctga 2220
ccccctgggg ctgatggatc tcagcaccac tcccttgcaa agtgctcccc cccttgaatc 2280
accgcaaagg ctcctcagtt cagaaccctt agacctcatc tccgtcccct ttggcaactc 2340
ttctccctca gatatagacg tccccaagcc aggctccccg gagccacagg tttctggcct 2400
tgcagccaat cgttctctga cagaaggcct ggtcctggac acaatgaatg acagcctcag 2460
caagatcctg ctggacatca gctttcctgg cctggacgag gacccactgg gccctgacaa 2520
catcaactgg tcccagttta ttcctgagct acagtagagc cctgcccttg cccctgtgct 2580
caagctgtcc accatcccgg gcactccaag gctcagtgca ccccaagcct ctgagtgagg 2640
acagcaggca gggactgttc tgctcctcat agctccctgc tgcctgatta tgcaaaagta 2700
gcagtcacac cctagccact gctgggacct tgtgttcccc aagagtatct gattcctctg 2760
ctgtccctgc caggagctga agggtgggaa caacaaaggc aatggtgaaa agagattagg 2820
aaccccccag cctgtttcca ttctctgccc agcagtctct taccttccct gatctttgca 2880
gggtggtccg tgtaaatagt ataaattctc caaattatcc tctaattata aatgtaagct 2940
tatttcctta gatcattatc cagagactgc cagaaggtgg gtaggatgac ctggggtttc 3000
aattgacttc tgttccttgc ttttagtttt gatagaaggg aagacctgca gtgcacggtt 3060
tcttccaggc tgaggtacct ggatcttggg ttcttcactg cagggaccca gacaagtgga 3120
tctgcttgcc agagtccttt ttgcccctcc ctgccacctc cccgtgtttc caagtcagct 3180
ttcctgcaag aagaaatcct ggttaaaaaa gtcttttgta ttgggtcagg agttgaattt 3240
ggggtgggag gatggatgca actgaagcag agtgtgggtg cccagatgtg cgctattaga 3300

tgtttctctg ataatgtccc caatcatacc agggagactg gcattgacga gaactcaggt 3360

ggaggcttga gaaggccgaa agggcccctg acctgcctgg cttccttagc ttgcccctca 3420

gctttgcaaa gagccaccct aggccccagc tgaccgcatg ggtgtgagcc agcttgagaa 3480

cactaactac tcaataaaag cgaaggtgga caaaaaaaaa aaaaaaa 3527

<210> 33
<211> 3641
<212> DNA
<213> Homo sapiens

<400> 33
actgaaagct ccggtgccag accccacccc cggccccggc ccgggacccc ctcccctccc 60

gggatccccc ggggttccca ccccgcccgc accgccgggg acccggccgg tccggcgcga 120

gcccccgtcc ggggccctgg ctcggccccc aggttggagg agcccggagc ccgccttcgg 180

agctacggcc taacggcggc ggcgactgca gtctggaggg tccacacttg tgattctcaa 240

tggagagtga aaacgcagat tcataatgaa aactagcccc cgtcggccac tgattctcaa 300

aagacggagg ctgccccttc ctgttcaaaa tgccccaagt gaaacatcag aggaggaacc 360

taagagatcc cctgcccaac aggagtctaa tcaagcagag gcctccaagg aagtggcaga 420

gtccaactct tgcaagtttc cagctgggat caagattatt aaccacccca ccatgcccaa 480

cacgcaagta gtggccatcc ccaacaatgc taatattcac agcatcatca cagcactgac 540

tgccaaggga aaagagagtg gcagtagtgg gcccaacaaa ttcatcctca tcagctgtgg 600

gggagcccca actcagcctc caggactccg gcctcaaacc caaaccagct atgatgccaa 660

aaggacagaa gtgaccctgg agaccttggg accaaaacct gcagctaggg atgtgaatct 720

tcctagacca cctggagccc tttgcgagca gaaacgggag acctgtgcag atggtgaggc 780

agcaggctgc actatcaaca atagcctatc caacatccag tggcttcgaa agatgagttc 840

tgatggactg ggctcccgca gcatcaagca agagatggag gaaaaggaga attgtcacct 900

ggagcagcga caggttaagg ttgaggagcc ttcgagacca tcagcgtcct ggcagaactc 960

tgtgtctgag cggccaccct actcttacat ggccatgata caattcgcca tcaacagcac 1020

tgagaggaag cgcatgactt tgaaagacat ctatacgtgg attgaggacc actttcccta 1080

ctttaagcac attgccaagc caggctggaa gaactccatc cgccacaacc tttccctgca 1140

cgacatgttt gtccgggaga cgtctgccaa tggcaaggtc tccttctgga ccattcaccc 1200

cagtgccaac cgctacttga cattggacca ggtgtttaag ccactggacc cagggtctcc 1260

acaattgccc gagcacttgg aatcacagca gaaacgaccg aatccagagc tccgccggaa 1320

catgaccatc aaaaccgaac tccccctggg cgcacggcgg aagatgaagc cactgctacc 1380

acgggtcagc tcatacctgg tacctatcca gttcccggtg aaccagtcac tggtgttgca 1440

gccctcggtg aaggtgccat tgcccctggc ggcttccctc atgagctcag agcttgcccg 1500

ccatagcaag cgagtccgca ttgcccccaa ggtttttggg gaacaggtgg tgtttggtta 1560

catgagtaag ttctttagtg gcgatctgcg agattttggt acacccatca ccagcttgtt 1620

taattttatc tttctttgtt tatcagtgct gctagctgag gaggggatag ctcctctttc 1680

ttctgcagga ccagggaaag aggagaaact cctgtttgga gaagggtttt ctcctttgct 1740

tccagttcag actatcaagg aggaagaaat ccagcctggg gaggaaatgc cacacttagc 1800

gagacccatc aaagtggaga gccctccctt ggaagagtgg ccctccccgg ccccatcttt 1860

caaagaggaa tcatctcact cctgggagga ttcgtcccaa tctcccaccc caagacccaa 1920

gaagtcctac agtgggctta ggtccccaac ccggtgtgtc tcggaaatgc ttgtgattca 1980

acacagggag aggagggaga ggagccggtc tcggaggaaa cagcatctac tgcctccctg 2040

tgtggatgag ccggagctgc tcttctcaga ggggcccagt acttcccgct gggccgcaga 2100

gctcccgttc ccagcagact cctctgaccc tgcctcccag ctcagctact cccaggaagt 2160

gggaggacct tttaagacac ccattaagga aacgctgccc atctcctcca ccccgagcaa 2220

atctgtcctc cccagaaccc ctgaatcctg gaggctcacg cccccagcca aagtaggggg 2280

actggatttc agcccagtac aaacctccca gggtgcctct gaccccttgc ctgaccccct 2340

ggggctgatg gatctcagca ccactccctt gcaaagtgct cccccccttg aatcaccgca 2400

aaggctcctc agttcagaac ccttagacct catctccgtc ccctttggca actcttctcc 2460

ctcagatata gacgtcccca agccaggctc cccggagcca caggtttctg gccttgcagc 2520

caatcgttct ctgacagaag gcctggtcct ggacacaatg aatgacagcc tcagcaagat 2580

cctgctggac atcagctttc ctggcctgga cgaggaccca ctgggccctg acaacatcaa 2640

ctggtcccag tttattcctg agctacagta gagccctgcc cttgcccctg tgctcaagct 2700

gtccaccatc ccgggcactc caaggctcag tgcaccccaa gcctctgagt gaggacagca 2760

ggcagggact gttctgctcc tcatagctcc ctgctgcctg attatgcaaa agtagcagtc 2820

acaccctagc cactgctggg accttgtgtt ccccaagagt atctgattcc tctgctgtcc 2880

ctgccaggag ctgaagggtg ggaacaacaa aggcaatggt gaaaagagat taggaacccc 2940

ccagcctgtt tccattctct gcccagcagt ctcttacctt ccctgatctt tgcagggtgg 3000

tccgtgtaaa tagtataaat tctccaaatt atcctctaat tataaatgta agcttatttc 3060

cttagatcat tatccagaga ctgccagaag gtgggtagga tgacctgggg tttcaattga 3120

cttctgttcc ttgcttttag ttttgataga agggaagacc tgcagtgcac ggtttcttcc 3180

aggctgaggt acctggatct tgggttcttc actgcaggga cccagacaag tggatctgct 3240

tgccagagtc ctttttgccc ctccctgcca cctccccgtg tttccaagtc agctttcctg 3300

caagaagaaa tcctggttaa aaaagtcttt tgtattgggt caggagttga atttggggtg 3360

ggaggatgga tgcaactgaa gcagagtgtg ggtgcccaga tgtgcgctat tagatgtttc 3420

tctgataatg tccccaatca taccagggag actggcattg acgagaactc aggtggaggc 3480

ttgagaaggc cgaaagggcc cctgacctgc ctggcttcct tagcttgccc ctcagctttg 3540

caaagagcca ccctaggccc cagctgaccg catgggtgtg agccagcttg agaacactaa 3600

ctactcaata aaagcgaagg tggacaaaaa aaaaaaaaaa a 3641

<210> 34
<211> 3487

<212>  DNA
<213>  Homo sapiens

<400>  34
actgaaagct ccggtgccag accccacccc cggccccggc ccgggacccc ctcccctccc      60

gggatccccc ggggttccca ccccgcccgc accgccgggg acccggccgg tccggcgcga     120

gcccccgtcc ggggccctgg ctcggccccc aggttggagg agcccggagc ccgccttcgg     180

agctacggcc taacggcggc ggcgactgca gtctggaggg tccacacttg tgattctcaa     240

tggagagtga aaacgcagat tcataatgaa aactagcccc cgtcggccac tgattctcaa     300

aagacggagg ctgccccttc ctgttcaaaa tgccccaagt gaaacatcag aggaggaacc     360

taagagatcc cctgcccaac aggagtctaa tcaagcagag gcctccaagg aagtggcaga     420

gtccaactct tgcaagtttc cagctgggat caagattatt aaccacccca ccatgcccaa     480

cacgcaagta gtggccatcc ccaacaatgc taatattcac agcatcatca cagcactgac     540

tgccaaggga aaagagagtg gcagtagtgg gcccaacaaa ttcatcctca tcagctgtgg     600

gggagcccca actcagcctc caggactccg gcctcaaacc caaaccagct atgatgccaa     660

aaggacagaa gtgaccctgg agaccttggg accaaaacct gcagctaggg atgtgaatct     720

tcctagacca cctggagccc tttgcgagca gaaacgggag acctgtgcag atggtgaggc     780

agcaggctgc actatcaaca atagcctatc caacatccag tggcttcgaa agatgagttc     840

tgatggactg ggctcccgca gcatcaagca agagatggag gaaaaggaga attgtcacct     900

ggagcagcga caggttaagg ttgaggagcc ttcgagacca tcagcgtcct ggcagaactc     960

tgtgtctgag cggccaccct actcttacat ggccatgata caattcgcca tcaacagcac    1020

tgagaggaag cgcatgactt tgaaagacat ctatacgtgg attgaggacc actttcccta    1080

ctttaagcac attgccaagc caggctggaa gaactccatc cgccacaacc tttccctgca    1140

cgacatgttt gtccgggaga cgtctgccaa tggcaaggtc tccttctgga ccattcaccc    1200

cagtgccaac cgctacttga cattggacca ggtgtttaag cagcagaaac gaccgaatcc    1260

agagctccgc cggaacatga ccatcaaaac cgaactcccc ctgggcgcac ggcggaagat    1320

gaagccactg ctaccacggg tcagctcata cctggtacct atccagttcc cggtgaacca    1380

gtcactggtg ttgcagccct cggtgaaggt gccattgccc ctggcggctt ccctcatgag    1440

ctcagagctt gcccgccata gcaagcgagt ccgcattgcc cccaaggtgc tgctagctga    1500

ggaggggata gctcctcttt cttctgcagg accagggaaa gaggagaaac tcctgtttgg    1560

agaagggttt tctcctttgc ttccagttca gactatcaag gaggaagaaa tccagcctgg    1620

ggaggaaatg ccacacttag cgagacccat caaagtggag agccctcccc tggaagagtg    1680

gccctccccg gccccatctt tcaaagagga atcatctcac tcctgggagg attcgtccca    1740

atctcccacc ccaagaccca agaagtccta cagtgggctt aggtccccaa cccggtgtgt    1800

ctcggaaatg cttgtgattc aacacaggga gaggagggag aggagccggt ctcggaggaa    1860

acagcatcta ctgcctccct gtgtggatga gccggagctg ctcttctcag aggggcccag    1920

tacttcccgc tgggccgcag agctcccgtt cccagcagac tcctctgacc ctgcctccca    1980

gctcagctac tcccaggaag tgggaggacc ttttaagaca cccattaagg aaacgctgcc 2040

catctcctcc accccgagca aatctgtcct ccccagaacc cctgaatcct ggaggctcac 2100

gcccccagcc aaagtagggg gactggattt cagcccagta caaacctccc agggtgcctc 2160

tgaccccttg cctgaccccc tggggctgat ggatctcagc accactccct tgcaaagtgc 2220

tccccccctt gaatcaccgc aaaggctcct cagttcagaa cccttagacc tcatctccgt 2280

cccctttggc aactcttctc cctcagatat agacgtcccc aagccaggct ccccggagcc 2340

acaggtttct ggccttgcag ccaatcgttc tctgacagaa ggcctggtcc tggacacaat 2400

gaatgacagc ctcagcaaga tcctgctgga catcagcttt cctggcctgg acgaggaccc 2460

actgggccct gacaacatca actggtccca gtttattcct gagctacagt agagccctgc 2520

ccttgcccct gtgctcaagc tgtccaccat cccgggcact ccaaggctca gtgcacccca 2580

agcctctgag tgaggacagc aggcagggac tgttctgctc ctcatagctc cctgctgcct 2640

gattatgcaa aagtagcagt cacaccctag ccactgctgg gaccttgtgt tccccaagag 2700

tatctgattc ctctgctgtc cctgccagga gctgaagggt gggaacaaca aaggcaatgg 2760

tgaaaagaga ttaggaaccc cccagcctgt ttccattctc tgcccagcag tctcttacct 2820

tccctgatct ttgcagggtg gtccgtgtaa atagtataaa ttctccaaat tatcctctaa 2880

ttataaatgt aagcttattt ccttagatca ttatccagag actgccagaa ggtgggtagg 2940

atgacctggg gtttcaattg acttctgttc cttgctttta gttttgatag aagggaagac 3000

ctgcagtgca cggtttcttc caggctgagg tacctggatc ttgggttctt cactgcaggg 3060

acccagacaa gtggatctgc ttgccagagt cctttttgcc cctccctgcc acctccccgt 3120

gtttccaagt cagctttcct gcaagaagaa atcctggtta aaaaagtctt ttgtattggg 3180

tcaggagttg aatttggggt gggaggatgg atgcaactga agcagagtgt gggtgcccag 3240

atgtgcgcta ttagatgttt ctctgataat gtccccaatc ataccaggga gactggcatt 3300

gacgagaact caggtggagg cttgagaagg ccgaaaggcc ccctgacctg cctggcttcc 3360

ttagcttgcc cctcagcttt gcaaagagcc accctaggcc ccagctgacc gcatgggtgt 3420

gagccagctt gagaacacta actactcaat aaaagcgaag gtggacaaaa aaaaaaaaaa 3480

aaaaaaa 3487

<210> 35
<211> 951
<212> DNA
<213> Homo sapiens

<400> 35
attggtggga tgagcaatcc gagttcccgg atgagggaac attctgcagt ataaagggag 60

cagggaaggc gggagacagc gcagtttgaa tcgcggtgcg acgaaggagt aggtggtggg 120

atctcaccgt gggtccgatt agccttttct ctgccttgct tgcttgagct tcagcggaat 180

tcgaaatggc tggcggtaag gctggaaagg actccggaaa ggccaagaca aaggcggttt 240

cccgctcgca gagagccggc ttgcagttcc cagtgggccg tattcatcga cacctaaaat 300

ctaggacgac cagtcatgga cgtgtgggcg cgactgccgc tgtgtacagc gcagccatcc 360

tggagtacct caccgcagag gtacttgaac tggcaggaaa tgcatcaaaa gacttaaagg 420

taaagcgtat tacccctcgt cacttgcaac ttgctattcg tggagatgaa gaattggatt 480

ctctcatcaa ggctacaatt gctggtggtg gtgtcattcc acacatccac aaatctctga 540

ttgggaagaa aggacaacag aagactgtct aaaggatgcc tggattcctt gttatctcag 600

gactctaaat actctaacag ctgtccagtg ttggtgattc cagtggactg tatctctgtg 660

aaaaacacaa ttttgccttt ttgtaattct atttgagcaa gttggaagtt taattagctt 720

tccaaccaac caaatttctg cattcgagtc ttaaccatat ttaagtgtta ctgtggcttc 780

aaagaagcta ttgattctga agtagtgggt tttgattgag ttgactgttt ttaaaaaact 840

gtttggattt taattgtgat gcagaagtta tagtaacaaa catttggttt tgtacagaca 900

ttatttccac tctggtggat aagttcaata aaggtcatat cccaaacaaa a 951

<210> 36
<211> 576
<212> DNA
<213> Homo sapiens

<400> 36
actcttctgg tccccacaga ctcagagaga acccaccatg gtgctgtctc ctgccgacaa 60

gaccaacgtc aaggccgcct ggggtaaggt cggcgcgcac gctggcgagt atggtgcgga 120

ggccctggag aggatgttcc tgtccttccc caccaccaag acctacttcc cgcacttcga 180

cctgagccac ggctctgccc aggttaaggg ccacggcaag aaggtggccg acgcgctgac 240

caacgccgtg gcgcacgtgg acgacatgcc caacgcgctg tccgccctga gcgacctgca 300

cgcgcacaag cttcgggtgg acccggtcaa cttcaagctc ctaagccact gcctgctggt 360

gaccctggcc gcccacctcc ccgccgagtt cacccctgcg gtgcacgcct ccctggacaa 420

gttcctggct tctgtgagca ccgtgctgac ctccaaatac cgttaagctg gagcctcggt 480

ggccatgctt cttgcccctt gggcctcccc ccagcccctc ctccccttcc tgcacccgta 540

cccccgtggt ctttgaataa agtctgagtg ggcggc 576

<210> 37
<211> 575
<212> DNA
<213> Homo sapiens

<400> 37
actcttctgg tccccacaga ctcagagaga acccaccatg gtgctgtctc ctgccgacaa 60

gaccaacgtc aaggccgcct ggggtaaggt cggcgcgcac gctggcgagt atggtgcgga 120

ggccctggag aggatgttcc tgtccttccc caccaccaag acctacttcc cgcacttcga 180

cctgagccac ggctctgccc aggttaaggg ccacggcaag aaggtggccg acgcgctgac 240

caacgccgtg gcgcacgtgg acgacatgcc caacgcgctg tccgccctga gcgacctgca 300

cgcgcacaag cttcgggtgg acccggtcaa cttcaagctc ctaagccact gcctgctggt 360

gaccctggcc gcccacctcc ccgccgagtt cacccctgcg gtgcacgcct ccctggacaa 420

gttcctggct tctgtgagca ccgtgctgac ctccaaatac cgttaagctg gagcctcggt 480

agccgttcct cctgcccgct gggcctccca acgggccctc ctcccctcct tgcaccggcc    540

cttcctggtc tttgaataaa gtctgagtgg gcggc                              575

<210>  38
<211>  626
<212>  DNA
<213>  Homo sapiens

<400>  38
acatttgctt ctgacacaac tgtgttcact agcaacctca aacagacacc atggtgcatc     60

tgactcctga ggagaagtct gccgttactg ccctgtgggg caaggtgaac gtggatgaag    120

ttggtggtga ggccctgggc aggctgctgg tggtctaccc ttggacccag aggttctttg    180

agtcctttgg ggatctgtcc actcctgatg ctgttatggg caaccctaag gtgaaggctc    240

atggcaagaa agtgctcggt gcctttagtg atggcctggc tcacctggac aacctcaagg    300

gcacctttgc cacactgagt gagctgcact gtgacaagct gcacgtggat cctgagaact    360

tcaggctcct gggcaacgtg ctggtctgtg tgctggccca tcactttggc aaagaattca    420

ccccaccagt gcaggctgcc tatcagaaag tggtggctgg tgtggctaat gccctggccc    480

acaagtatca ctaagctcgc tttcttgctg tccaatttct attaaaggtt cctttgttcc    540

ctaagtccaa ctactaaact gggggatatt atgaagggcc ttgagcatct ggattctgcc    600

taataaaaaa catttatttt cattgc                                        626

<210>  39
<211>  1637
<212>  DNA
<213>  Homo sapiens

<400>  39
ggcaagacgc ctcttcagtt gtctgctact cagaggaagg ggcggttggt gcggcctcca     60

ttgttcgtgt tttaaggcgc catgaggggt gacagaggcc gtggtcgtgg tgggcgcttt    120

ggttccagag gaggcccagg aggagggttc aggccctttg taccacatat cccatttgac    180

ttctatttgt gtgaaatggc ctttccccgg gtcaagccag cacctgatga aacttccttc    240

agtgaggcct tgctgaagag gaatcaggac ctggctccca attctgctga acaggcatct    300

atcctttctc tggtgacaaa aataaacaat gtgattgata atctgattgt ggctccaggg    360

acatttgaag tgcaaattga agaagttcga caggtgggat cctataaaaa ggggacaatg    420

actacaggac acaatgtggc tgacctggtg gtgatactca agattctgcc aacgttggaa    480

gctgttgctg ccctggggaa caaagtcgtg gaaagcctaa gagcacagga tccttctgaa    540

gttttaacca tgctgaccaa cgaaactggc tttgaaatca gttcttctga tgctacagtg    600

aagattctca ttacaacagt gccacccaat cttcgaaaac tggatccaga actccatttg    660

gatatcaaag tattgcagag tgccttagca gccatccgac atgcccgctg gttcgaggaa    720

aatgcttctc agtccacagt taaagttctc atcagactac tgaaggactt gaggattcgt    780

tttcctggct ttgagcccct cacaccctgg atccttgacc tactaggcca ttatgctgtg    840

atgaacaacc ccaccagaca gcctttggcc ctaaacgttg catacaggcg ctgcttgcag    900

attctggctg caggactgtt cctgccaggt tcagtgggta tcactgaccc ctgtgagagt 960

ggcaacttta gagtacacac agtcatgacc ctagaacagc aggacatggt ctgctataca 1020

gctcagactc tcgtccgaat cctctcacat ggtggcttta ggaagatcct tggccaggag 1080

ggtgatgcca gctatcttgc ttctgaaata tctacctggg atggagtgat agtaacacct 1140

tcagaaaagg cttatgagaa gccaccagag aagaaggaag gagaggaaga agaggagaat 1200

acagaagaac cacctcaagg agaggaagaa gaaagcatgg aaactcagga gtgacattcc 1260

cttcactcct tttcctaccc aagggggaag actggagcct aagctgcctg ctactgggct 1320

ttacatggtg acagacattt ccgtgggata gggaagatag caggaagaaa agtaaactcc 1380

atagaagtgt cattccactg ggttttgata ttggcttagc tgccagtctc ccatttgtga 1440

cctatgccat ccatctataa tggaggatac caacatttct tcctaatatt ctataatctc 1500

caactcctga aaacccctct ctcaactaat actttgctgt tgaaatgttg tgaaatgtta 1560

agtgtctgga aatttttttt tctaagaaaa actattaaag tacttcctag taaaaaaaaa 1620

aaaaaaaaaa aaaaaaa 1637

<210> 40
<211> 4908
<212> DNA
<213> Homo sapiens

<400> 40
acctgcgtgc agtcggtcct ccaggccacg cagcgcccga gagtaccagg gagactccgg 60

cccctgtcgg cgccaagccc ctccgcccct cacagcgccc aggtccgcgg ccgggccttg 120

attttttggc ggggaccgtc atggcgtcgc agccaaattc gtctgcgaag aagaaagagg 180

agaaggggaa gaacatccag gtggtggtga gatgcagacc atttaatttg gcagagcgga 240

aagctagcgc ccattcaata gtagaatgtg atcctgtacg aaaagaagtt agtgtacgaa 300

ctggaggatt ggctgacaag agctcaagga aaacatacac ttttgatatg gtgtttggag 360

catctactaa acagattgat gtttaccgaa gtgttgtttg tccaattctg gatgaagtta 420

ttatgggcta taattgcact atctttgcgt atggccaaac tggcactgga aaaactttta 480

caatggaagg tgaaaggtca cctaatgaag agtatacctg ggaagaggat cccttggctg 540

gtataattcc acgtaccctt catcaaattt ttgagaaact tactgataat ggtactgaat 600

tttcagtcaa agtgtctctg ttggagatct ataatgaaga gctttttgat cttcttaatc 660

catcatctga tgtttctgag agactacaga tgtttgatga tccccgtaac aagagaggag 720

tgataattaa aggtttagaa gaaattacag tacacaacaa ggatgaagtc tatcaaattt 780

tagaaaaggg ggcagcaaaa aggacaactg cagctactct gatgaatgca tactctagtc 840

gttcccactc agttttctct gttacaatac atatgaaaga aactacgatt gatggagaag 900

agcttgttaa aatcggaaag ttgaacttgg ttgatcttgc aggaagtgaa aacattggcc 960

gttctggagc tgttgataag agagctcggg aagctggaaa tataaatcaa tccctgttga 1020

ctttgggaag ggtcattact gcccttgtag aaagaacacc tcatgttcct tatcgagaat 1080

ctaaactaac tagaatcctc caggattctc ttggagggcg tacaagaaca tctataattg 1140

caacaatttc tcctgcatct ctcaatcttg aggaaactct gagtacattg gaatatgctc 1200

atagagcaaa gaacatattg aataagcctg aagtgaatca gaaactcacc aaaaaagctc 1260

ttattaagga gtatacggag gagatagaac gtttaaaacg agatcttgct gcagcccgtg 1320

agaaaaatgg agtgtatatt tctgaagaaa attttagagt catgagtgga aaattaactg 1380

ttcaagaaga gcagattgta gaattgattg aaaaaattgg tgctgttgag gaggagctga 1440

atagggttac agagttgttt atggataata aaaatgaact tgaccagtgt aaatctgacc 1500

tgcaaaataa aacacaagaa cttgaaacca ctcaaaaaca tttgcaagaa actaaattac 1560

aacttgttaa agaagaatat atcacatcag ctttggaaag tactgaggag aaacttcatg 1620

atgctgccag caagctgctt aacacagttg aagaaactac aaaagatgta tctggtctcc 1680

attccaaact ggatcgtaag aaggcagttg accaacacaa tgcagaagct caggatattt 1740

ttggcaaaaa cctgaatagt ctgtttaata atatggaaga attaattaag gatggcagct 1800

caaagcaaaa ggccatgcta gaagtacata agaccttatt tggtaatctg ctgtcttcca 1860

gtgtctctgc attagatacc attactacag tagcacttgg atctctcaca tctattccag 1920

aaaatgtgtc tactcatgtt tctcagattt ttaatatgat actaaaagaa caatcattag 1980

cagcagaaag taaaactgta ctacaggaat tgattaatgt actcaagact gatcttctaa 2040

gttcactgga aatgatttta tccccaactg tggtgtctat actgaaaatc aatagtcaac 2100

taaagcatat tttcaagact tcattgacag tggccgataa gatagaagat caaaaaaagg 2160

aactagatgg ctttctcagt atactgtgta acaatctaca tgaactacaa gaaaatacca 2220

tttgttcctt ggttgagtca caaaagcaat gtggaaacct aactgaagac ctgaagacaa 2280

taaagcagac ccattcccag gaactttgca agttaatgaa tctttggaca gagagattct 2340

gtgctttgga ggaaaagtgt gaaaatatac agaaaccact tagtagtgtc caggaaaata 2400

tacagcagaa atctaaggat atagtcaaca aaatgacttt tcacagtcaa aaattttgtg 2460

ctgattctga tggcttctca caggaactca gaaattttaa ccaagaaggt acaaaattgg 2520

ttgaagaatc tgtgaaacac tctgataaac tcaatggcaa cctggaaaaa atatctcaag 2580

agactgaaca gagatgtgaa tctctgaaca caagaacagt ttatttttct gaacagtggg 2640

tatcttcctt aaatgaaagg gaacaggaac ttcacaactt attggaggtt gtaagccaat 2700

gttgtgaggc ttcaagttca gacatcactg agaaatcaga tggacgtaag gcagctcatg 2760

agaaacagca taacattttt cttgatcaga tgactattga tgaagataaa ttgatagcac 2820

aaaatctaga acttaatgaa accataaaaa ttggtttgac taagcttaat tgctttctgg 2880

aacaggatct gaaactggat atcccaacag gtacgacacc acagaggaaa agttatttat 2940

acccatcaac actggtaaga actgaaccac gtgaacatct ccttgatcag ctgaaaagga 3000

aacagcctga gctgttaatg atgctaaact gttcagaaaa caacaaagaa gagacaattc 3060

cggatgtgga tgtagaagag gcagttctgg ggcagtatac tgaagaacct ctaagtcaag 3120

agccatctgt agatgctggt gtggattgtt catcaattgg cggggttcca tttttccagc 3180

ataaaaaatc acatggaaaa gacaaagaaa acagaggcat taacacactg gagaggtcta 3240

aagtggaaga aactacagag cacttggtta caaagagcag attacctctg cgagcccaga 3300

tcaaccttta attcacttgg gggttggcaa tttatttttt aaagaaaact taaaaataaa 3360

acctgaaacc ccagaacttg agccttgtgt atagatttta aaagaatata tatatcagcc 3420

gggcgcggtg gctcatgcct gtaatcccag cactttggga ggctgaggcg ggtggattgc 3480

ttgagcccag gagtttgaga ccagcctggc caacgtggca aaacctcgtc tctgttaaaa 3540

attagccggg cgtggtggca cactcctgta atcccagcta ctggggaggc tgaggcacga 3600

gaatcacttg aacccaggaa gcggggttgc agtgagccaa aggtacacca ctacactcca 3660

gcctgggcaa cagagcaaga ctcggtctca aaaacaaaat ttaaaaaaga tataaggcag 3720

tactgtaaat tcagttgaat tttgatatct acccattttt ctgtcatccc tatagttcac 3780

tttgtattaa attgggtttc atttgggatt tgcaatgtaa atacgtattt ctagttttca 3840

tataaagtag ttcttttata acaaatgaaa agtatttttc ttgtatatta ttaagtaatg 3900

aatatataag aactgtactc ttctcagctt gagcttaaca taggtaaata tcaccaacat 3960

ctgtccttag aaaggaccat ctcatgtttt ttttcttgct atgacttgtg tattttcttg 4020

catcctccct agacttccct atttcgcttt ctcctcggct cactttctcc ctttttattt 4080

ttcaccaaac catttgtaga gctacaaaac ctatcctttc ttattttcag tagtcagaat 4140

tttatctaga aatcttttaa caccttttta gtggttattt ctaaaatcac tgtcaacaat 4200

aaatctaacc ctagttgtat ccctccttta agtatttaaa acttgttgcc ccaaatgtga 4260

aagcatttaa ttcctttaag aggcctaact cattcaccct gacagagttc acaaaaagcc 4320

cactttagag tatacattgc tattatggga gaccacccag acatctgact aatggctctg 4380

tgccacactc caagacctgt gccttttaga gaagctcaca atgatttaag gactgtttga 4440

aacttccaat tatgtctata atttatattc ttttgtttac atgatgaaac tttttgttgt 4500

tgcttgtttg tatataatac aatgtgtaca tgtatctttt tctcgattca aatcttaacc 4560

cttaggactc tggtattttt gatctggcaa ccatatttct ggaagttgag atgtttcagc 4620

ttgaagaacc aaaacagaag gaatatgtac aaagaataaa ttttctgctc acgatgagtt 4680

tagtgtgtaa agtttagaga catctgactt tgatagctaa attaaaccaa accctattga 4740

agaattgaat atatgctact tcaagaaact aaattgatct cgtagaatta tcttaataaa 4800

ataatggcta taatttctct gcaaaatcag atgtcagcat aagcgatgga taatacctaa 4860

taaactgccc tcagtaaatc catggttaat aaatgtggtt tctacatt 4908

<210> 41
<211> 2391
<212> DNA
<213> Homo sapiens

<400> 41
tcaaaagtgg cgggtgtggc gcggggctgg tagcggccgg agccgtgcga gttctctacc 60

ctgcttcgcg agcgggcgag agaacgcgag tcccaggatc cccggcaccc agttctcttc 120

cactgcattc ccccggcgcg tgtgggaccg aggtggacat ggatccgcag aggtcccccc 180

tattggaagt aaaggggaac atagaactga agagacctct gattaaggcc ccttcccagc 240
tgcctctctc aggaagcaga ctcaagagga ggcctgacca gatggaagat ggcctggagc 300
ctgagaagaa acggacaaga ggcctgggtg caacgaccaa aattaccaca tcccacccaa 360
gagttccatc cctcactaca gtgccacaga cacaaggcca gaccacagct caaaaagttt 420
ccaagaagac aggaccccgg tgttccacag ctattgccac agggttgaag aaccagaagc 480
cagttcctgc tgttcctgtc cagaagtctg gcacatcagg tgttcctccc atggcaggag 540
ggaagaaacc cagcaaacgt ccagcctggg acttaaaggg tcagttatgt gacctaaatg 600
cagaactaaa acggtgccgt gagaggactc aaacgttgga ccaagagaac cagcagcttc 660
aggaccagct cagagatgcc cagcagcagg tcaaggccct ggggacagag cgcacaacac 720
tggaggggca tttagccaag gtacaggccc aggctgagca gggccaacag gagctgaaga 780
acttgcgtgc ttgtgtcctg gagctggaag agcggctgag cacgcaggag ggcttggtgc 840
aagagcttca gaaaaaacag gtggaattgc aggaagaacg gaggggactg atgtcccaac 900
tagaggagaa ggagaggagg ctgcagacat cagaagcagc cctgtcaagc agccaagcag 960
aggtggcatc tctgcggcag gagactgtgg cccaggcagc cttactgact gagcgggaag 1020
aacgtcttca tgggctagaa atggagcgcc ggcgactgca caaccagctg caggaactca 1080
agggcaacat ccgtgtattc tgccgggtcc gccctgtcct gccgggggag cccactccac 1140
cccctggcct cctcctgttt ccctctggcc ctggtgggcc ctctgatcct ccaacccgcc 1200
ttagcctctc ccggtctgac gagcggcgtg ggaccctgag tggggcacca gctcccccac 1260
ctcgccatga tttttccttt gaccgggtat tcccaccagg aagtggacag gatgaagtgt 1320
ttgaagagat tgccatgctt gtccagtcag ccctggatgg ctatccagta tgcatctttg 1380
cctatggcca gacaggcagt ggcaagacct tcacaatgga gggtgggcct gggggagacc 1440
cccagttgga ggggctgatc cctcgggccc tgcggcacct cttctctgtg gctcaggagc 1500
tgagtggtca gggctggacc tacagctttg tagcaagcta cgtagagatc tacaatgaga 1560
ctgtccggga cctgctggcc actggaaccc ggaagggtca agggggcgag tgtgagattc 1620
gccgtgcagg gccagggagt gaggagctca ctgtcaccaa tgctcgatat gtccctgtct 1680
cctgtgagaa agaagtggac gccctgcttc atctggcccg ccagaatcgg gctgtggccc 1740
gcacagccca gaatgaacgg tcatcacgca gccacagtgt attccagcta cagatttctg 1800
gggagcactc cagccgaggc ctgcagtgtg gggcccccct cagtcttgtg gacctggccg 1860
ggagtgagcg acttgacccc ggcttagccc tcggccccgg ggagcgggaa cgccttcggg 1920
aaacacaggc cattaacagc agcctgtcca cgctggggct ggttatcatg gccctgagca 1980
acaaggagtc ccacgtgcct taccggaaca gcaaactgac ctacctgctg cagaactctc 2040
tgggtggtag tgctaagatg ctcatgtttg tgaacatttc tccactggaa gagaacgtct 2100
ccgagtccct caactctcta cgctttgcct ccaaggtgaa ccagtgtgtt attggtactg 2160
ctcaggccaa caggaagtga agacggatcc agatctgtgt gtgtgtgtgt gtgtgtgtgt 2220
gtgtgtgtgt gtgtgtgtgt gtgtgtgtcc ctatgtctat gtatcgggtg aggggtggga 2280

gggttgctgg agggtgcttt attgggtgga gggcaccatg tcccagggct atcaaataaa 2340

gaatagtttg gttttttttt taaaaaaaaa aaaaaaaaaa aaaaaaaaaa a 2391

<210> 42
<211> 2011
<212> DNA
<213> Homo sapiens

<400> 42
gccacacggt ctttgagctg agtcgaggtg gaccctttga acgcagtcgc cctacagccg 60

ctgattcccc ccgcatcgcc tcccgtggaa gcccaggccc gcttcgcagc tttctccctt 120

tgtctcataa ccatgtccac caacgagaat gctaatacac cagctgcccg tcttcacaga 180

ttcaagaaca agggaaaaga cagtacagaa atgaggcgtc gcagaataga ggtcaatgtg 240

gagctgagga aagctaagaa ggatgaccag atgctgaaga ggagaaatgt aagctcattt 300

cctgatgatg ctacttctcc gctgcaggaa aaccgcaaca accagggcac tgtaaattgg 360

tctgttgatg acattgtcaa aggcataaat agcagcaatg tggaaaatca gctccaagct 420

actcaagctg ccaggaaact actttccaga gaaaaacagc cccccataga caacataatc 480

cgggctggtt tgattccgaa atttgtgtcc ttcttgggca gaactgattg tagtcccatt 540

cagtttgaat ctgcttgggc actcactaac attgcttctg ggacatcaga acaaaccaag 600

gctgtggtag atggaggtgc catcccagca ttcatttctc tgttggcatc tccccatgct 660

cacatcagtg aacaagctgt ctgggctcta ggaaacattg caggtgatgg ctcagtgttc 720

cgagacttgg ttattaagta cggtgcagtt gacccactgt tggctctcct tgcagttcct 780

gatatgtcat ctttagcatg tggctactta cgtaatctta cctggacact ttctaatctt 840

tgccgcaaca agaatcctgc acccccgata gatgctgttg agcagattct tcctacctta 900

gttcggctcc tgcatcatga tgatccagaa gtattagcag atacctgctg ggctatttcc 960

taccttactg atggtccaaa tgaacgaatt ggcatggtgg tgaaaacagg agttgtgccc 1020

caacttgtga agcttctagg agcttctgaa ttgccaattg tgactcctgc cctaagagcc 1080

atagggaata ttgtcactgg tacagatgaa cagactcagg ttgtgattga tgcaggagca 1140

ctcgccgtct ttcccagcct gctcaccaac cccaaaacta acattcagaa ggaagctacg 1200

tggacaatgt caaacatcac agccggccgc caggaccaga tacagcaagt tgtgaatcat 1260

ggattagtcc cattccttgt cagtgttctc tctaaggcag attttaagac acaaaaggaa 1320

gctgtgtggg ccgtgaccaa ctataccagt ggtggaacag ttgaacagat tgtgtacctt 1380

gttcactgtg gcataataga accgttgatg aacctcttaa ctgcaaaaga taccaagatt 1440

attctggtta tcctggatgc catttcaaat atctttcagg ctgctgagaa actaggtgaa 1500

actgagaaac ttagtataat gattgaagaa tgtggaggct tagacaaaat tgaagctcta 1560

caaaaccatg aaaatgagtc tgtgtataag gcttcgttaa gcttaattga gaagtatttc 1620

tctgtagagg aagaggaaga tcaaaacgtt gtaccagaaa ctacctctga aggctacact 1680

ttccaagttc aggatggggc tcctgggacc tttaactttt agatcatgta gctgagacat 1740

aaatttgttg tgtactacgt ttggtatttt gtcttattgt ttctctacta agaactcttt 1800

cttaaatgtg gtttgttact gtagcacttt ttacactgaa actatacttg aacagttcca 1860

actgtacata catactgtat gaagcttgtc ctctgactag gtttctaatt tctatgtgga 1920

atttcctatc ttgcagcatc ctgtaaataa acattcaagt ccaccctttt cttgacttca 1980

aaaaaaaaaa aaaaaaaaaa aaaaaaaaaa a 2011

<210> 43
<211> 2882
<212> DNA
<213> Homo sapiens

<400> 43
gtgcagcctg agaggaaaca aagtgctgcg agcaggagac ggcggcggcg cgaaccctgc 60

tgggcctcca gtcaccctcg tcttgcattt tcccgcgtgc gtgtgtgagt gggtgtgtgt 120

gttttcttac aaagggtatt tcgcgatcga tcgattgatt cgtagttccc ccccgcgcgc 180

ctttgccctt tgtgctgtaa tcgagctccc gccatcccag gtgcttctcc gttcctctaa 240

acgccagcgt ctggacgtga gcgcaggtcg ccggtttgtg ccttcggtcc ccgcttcgcc 300

ccctgccgtc ccctccttat cacggtcccg ctcgcggcct cgccgccccg ctgtctccgc 360

cgcccgccat ggcgactgcg acccccgtgc cgccgcggat gggcagccgc gctggcggcc 420

ccaccacgcc gctgagcccc acgcgcctgt cgcggctcca ggagaaggag gagctgcgcg 480

agctcaatga ccggctggcg gtgtacatcg acaaggtgcg cagcctggag acggagaaca 540

gcgcgctgca gctgcaggtg acggagcgcg aggaggtgcg cggccgtgag ctcaccggcc 600

tcaaggcgct ctacgagacc gagctggccg acgcgcgacg cgcgctcgac gacacggccc 660

gcgagcgcgc caagctgcag atcgagctgg gcaagtgcaa ggcggaacac gaccagctgc 720

tcctcaacta tgctaagaag gaatctgatc ttaatggcgc ccagatcaag cttcgagaat 780

atgaagcagc actgaattcg aaagatgcag ctcttgctac tgcacttggt gacaaaaaaa 840

gtttagaggg agatttggag gatctgaagg atcagattgc ccagttggaa gcctccttag 900

ctgcagccaa aaaacagtta gcagatgaaa ctttacttaa agtagatttg gagaatcgtt 960

gtcagagcct tactgaggac ttggagtttc gcaaaagcat gtatgaagag gagattaacg 1020

agaccagaag gaagcatgaa acgcgcttgg tagaggtgga ttctgggcgt caaattgagt 1080

atgagtacaa gctggcgcaa gcccttcatg agatgagaga gcaacatgat gcccaagtga 1140

ggctgtataa ggaggagctg gagcagactt accatgccaa acttgagaat gccagactgt 1200

catcagagat gaatacttct actgtcaaca gtgccaggga agaactgatg gaaagccgca 1260

tgagaattga gagcctttca tcccagcttt ctaatctaca gaaagagtct agagcatgtt 1320

tggaaaggat tcaagaatta gaggacttgc ttgctaaaga aaaagacaac tctcgtcgca 1380

tgctgacaga caaagagaga gagatggcgg aaataaggga tcaaatgcag caacagctga 1440

atgactatga acagcttctt gatgtaaagt tagccctgga catggaaatc agtgcttaca 1500

ggaaactctt agaaggcgaa gaagagaggt tgaagctgtc tccaagccct tcttcccgtg 1560

tgacagtatc ccgagcatcc tcaagtcgta gtgtacgtac aactagagga aagcggaaga 1620

gggttgatgt ggaagaatca gaggcgagta gtagtgttag catctctcat tccgcctcag 1680

ccactggaaa tgtttgcatc gaagaaattg atgttgatgg gaaatttatc cgcttgaaga 1740

acacttctga acaggatcaa ccaatgggag gctgggagat gatcagaaaa attggagaca 1800

catcagtcag ttataaatat acctcaagat atgtgctgaa ggcaggccag actgttacaa 1860

tttgggctgc aaacgctggt gtcacagcca gccccccaac tgacctcatc tggaagaacc 1920

agaactcgtg gggcactggc gaagatgtga aggttatatt gaaaaattct cagggagagg 1980

aggttgctca aagaagtaca gtctttaaaa caaccatacc tgaagaagag gaggaggagg 2040

aagaagcagc tggagtggtt gttgaggaag aacttttcca ccagcaggga accccaagag 2100

catccaatag aagctgtgca attatgtaaa attttcaact gtcttcctca aaataaagaa 2160

gtatggtaat ccttacctgt atacagtgca gagccttctc agaagcacag aatattttta 2220

tatttccttt atgtgaattt ttaagctgca aatctgatgg ccttaatttc ctttttgaca 2280

ctgaaagttt tgtaaaagaa atcatgtcca tacactttgt tgcaagatgt gaattattga 2340

cactgaactt aataactgtg tactgttcgg aaggggttcc tcaaattttt tgactttttt 2400

tgtatgtgtg ttttttcttt ttttttaagt tcttatgagg aggggagggt aaataaacca 2460

ctgtgcgtct tggtgtaatt tgaagattgc cccatctaga ctagcaatct cttcattatt 2520

ctctgctata tataaaacgg tgctgtgagg gaggggaaaa gcatttttca atatattgaa 2580

cttttgtact gaattttttt gtaataagca atcaaggtta taattttttt taaaatagaa 2640

attttgtaag aaggcaatat taacctaatc accatgtaag cactctggat gatggattcc 2700

acaaaacttg gttttatggt tacttcttct cttagattct taattcatga ggagggtggg 2760

ggagggaggt ggagggaggg aagggtttct ctattaaaat gcattcgttg tgttttttaa 2820

gatagtgtaa cttgcttaaa tttcttatgt gacattaaca aataaaaaag ctcttttaat 2880

at 2882

<210> 44
<211> 1390
<212> DNA
<213> Homo sapiens

<400> 44
gggaagtgct gttggagccg ctgtggttgc tgtccgcgga gtggaagcgc gtgcttttgt 60

ttgtgtccct ggccatggcg ctgcagctct cccgggagca gggaatcacc ctgcgcggga 120

gcgccgaaat cgtggccgag ttcttctcat tcggcatcaa cagcatttta tatcagcgtg 180

gcatatatcc atctgaaacc tttactcgag tgcagaaata cggactcacc ttgcttgtaa 240

ctactgatct tgagctcata aaatacctaa ataatgtggt ggaacaactg aaagattggt 300

tatacaagtg ttcagttcag aaactggttg tagttatctc aaatattgaa agtggtgagg 360

tcctggaaag atggcagttt gatattgagt gtgacaagac tgcaaaagat gacagtgcac 420

ccagagaaaa gtctcagaaa gctatccagg atgaaatccg ttcagtgatc agacagatca 480

cagctacggt gacatttctg ccactgttgg aagtttcttg ttcatttgat ctgctgattt 540

atacagacaa agatttggtt gtacctgaaa aatgggaaga gtcgggacca cagtttatta 600

ccaattctga ggaagtccgc cttcgttcat ttactactac aatccacaaa gtaaatagca 660

tggtggccta caaaattcct gtcaatgact gaggatgaca tgaggaaaat aatgtaattg 720

taattttgaa atgtggtttt cctgaaatca ggtcatctat agttgatatg ttttatttca 780

ttggttaatt tttacatgga gaaaaccaaa atgatactta ctgaactgtg tgtaattgtt 840

cctttatttt tttggtacct atttgactta ccatggagtt aacatcatga atttattgca 900

cattgttcaa aaggaaccag gaggtttttt tgtcaacatt gtgatgtata ttcctttgaa 960

gatagtaact gtagatggaa aaacttgtgc tataaagcta gatgctttcc taaatcagat 1020

gttttggtca agtagtttga ctcagtatag gtagggagat atttaagtat aaaatacaac 1080

aaaggaagtc taaatattca gaatctttgt taaggtcctg aaagtaactc ataatctata 1140

aacaatgaaa tattgctgta tagctccttt tgaccttcat ttcatgtata gttttcccta 1200

ttgaatcagt ttccaattat ttgactttaa tttatgtaac ttgaacctat gaagcaatgg 1260

atatttgtac tgtttaatgt tctgtgatac agaactctta aaatgttttt ttcatgtgtt 1320

ttataaaatc aagttttaag tgaaagtgag gaaataaagt taagtttgtt ttaaaaaaaa 1380

aaaaaaaaaa 1390

<210> 45
<211> 3453
<212> DNA
<213> Homo sapiens

<400> 45
acttttcgcg cgaaacctgg ttgttgctgt agtggcggag aggatcgtgg tactgctatg 60

gcggaatcat cggaatcctt caccatggca tccagcccgg cccagcgtcg gcgaggcaat 120

gatcctctca cctccagccc tggccgaagc tcccggcgta ctgatgccct cacctccagc 180

cctggccgtg accttccacc atttgaggat gagtccgagg ggctcctagg cacagagggg 240

cccctggagg aagaagagga tggagaggag ctcattggag atggcatgga aagggactac 300

cgcgccatcc cagagctgga cgcctatgag gccgagggac tggctctgga tgatgaggac 360

gtagaggagc tgacggccag tcagagggag gcagcagagc gggccatgcg gcagcgtgac 420

cgggaggctg gccggggcct gggccgcatg cgccgtgggc tcctgtatga cagcgatgag 480

gaggacgagg agcgccctgc ccgcaagcgc cgccaggtgg agcgggccac ggaggacggc 540

gaggaggacg aggagatgat cgagagcatc gagaacctgg aggatctcaa aggccactct 600

gtgcgcgagt gggtgagcat ggcgggcccc cggctggaga tccaccaccg cttcaagaac 660

ttcctgcgca ctcacgtcga cagccacggc cacaacgtct tcaaggagcg catcagcgac 720

atgtgcaaag agaaccgtga gagcctggtg gtgaactatg aggacttggc agccagggag 780

cacgtgctgg cctacttcct gcctgaggca ccggcggagc tgctgcagat ctttgatgag 840

gctgccctgg aggtggtact ggccatgtac cccaagtacg accgcatcac caaccacatc 900

catgtccgca tctcccacct gcctctggtg gaggagctgc gctcgctgag gcagctgcat 960

ctgaaccagc tgatccgcac cagtggggtg gtgaccagct gcactggcgt cctgccccag 1020

ctcagcatgg tcaagtacaa ctgcaacaag tgcaatttcg tcctgggtcc tttctgccag 1080

tcccagaacc aggaggtgaa accaggctcc tgtcctgagt gccagtcggc cggccccttt 1140

gaggtcaaca tggaggagac catctatcag aactaccagc gtatccgaat ccaggagagt 1200

ccaggcaaag tggcggctgg ccggctgccc cgctccaagg acgccattct cctcgcagat 1260

ctggtggaca gctgcaagcc aggagacgag atagagctga ctggcatcta tcacaacaac 1320

tatgatggct ccctcaacac tgccaatggc ttccctgtct ttgccactgt catcctagcc 1380

aaccacgtgg ccaagaagga caacaaggtt gctgtagggg aactgaccga tgaagatgtg 1440

aagatgatca ctagcctctc caaggatcag cagatcggag agaagatctt tgccagcatt 1500

gctccttcca tctatggtca tgaagacatc aagagaggcc tggctctggc cctgttcgga 1560

ggggagccca aaaacccagg tggcaagcac aaggtacgtg gtgatatcaa cgtgctcttg 1620

tgcggagacc ctggcacagc gaagtcgcag tttctcaagt atattgagaa agtgtccagc 1680

cgagccatct tcaccactgg ccagggggcg tcggctgtgg gcctcacggc gtatgtccag 1740

cggcaccctg tcagcaggga gtggaccttg gaggctgggg ccctggttct ggctgaccga 1800

ggagtgtgtc tcattgatga atttgacaag atgaatgacc aggacagaac cagcatccat 1860

gaggccatgg agcaacagag catctccatc tcgaaggctg gcatcgtcac ctccctgcag 1920

gctcgctgca cggtcattgc tgccgccaac cccataggag ggcgctacga cccctcgctg 1980

actttctctg agaacgtgga cctcacagag cccatcatct cacgctttga catcctgtgt 2040

gtggtgaggg acaccgtgga cccagtccag gacgagatgc tggcccgctt cgtggtgggc 2100

agccacgtca gacaccaccc cagcaacaag gaggaggagg ggctggccaa tggcagcgct 2160

gctgagcccg ccatgcccaa cacgtatggc gtggagcccc tgccccagga ggtcctgaag 2220

aagtacatca tctacgccaa ggagagggtc cacccgaagc tcaaccagat ggaccaggac 2280

aaggtggcca agatgtacag tgacctgagg aaagaatcta tggcgacagg cagcatcccc 2340

attacggtgc ggcacatcga gtccatgatc cgcatggcgg aggcccacgc gcgcatccat 2400

ctgcgggact atgtgatcga agacgacgtc aacatggcca tccgcgtgat gctggagagc 2460

ttcatagaca cacagaagtt cagcgtcatg cgcagcatgc gcaagacttt tgcccgctac 2520

ctttcattcc ggcgtgacaa caatgagctg ttgctcttca tactgaagca gttagtggca 2580

gagcaggtga catatcagcg caaccgcttt ggggcccagc aggacactat tgaggtccct 2640

gagaaggact tggtggataa ggctcgtcag atcaacatcc acaacctctc tgcattttat 2700

gacagtgagc tcttcaggat gaacaagttc agccacgacc tgaaaaggaa aatgatcctg 2760

cagcagttct gaggccctat gccatccata aggattcctt gggattctgg tttggggtgg 2820

tcagtgccct ctgtgcttta tggacacaaa accagagcac ttgatgaact cggggtacta 2880

gggtcagggc ttatagcagg atgtctggct gcacctggca tgactgtttg tttctccaag 2940

cctgctttgt gcttctcacc tttgggtggg atgccttgcc agtgtgtctt acttggttgc 3000

tgaacatctt gccacctccg agtgctttgt ctccactcag taccttggat cagagctgct 3060

gagttcagga tgcctgcgtg tggtttaggt gttagccttc ttacatggat gtcaggagag 3120

ctgctgccct cttggcgtga gttgcgtatt caggctgctt ttgctgcctt tggccagaga 3180

gctggttgaa gatgtttgta atcgttttca gtctcctgca ggtttctgtg cccctgtggt 3240

ggaagagggc acgacagtgc cagcgcagcg ttctgggctc ctcagtcgca ggggtgggat 3300

gtgagtcatg cggattatcc actcgccaca gttatcagct gccattgctc cctgtctgtt 3360

tccccactct cttatttgtg cattcggttt ggtttctgta gttttaattt ttaataaagt 3420

tgaataaaat ataaaaaaaa aaaaaaaaaa aaa 3453

<210> 46
<211> 3143
<212> DNA
<213> Homo sapiens

<400> 46
ctcgaggtaa cctttggtgg cgggaagagt tcggaagttt tcgcgccggg gtggagtcat 60

cctgggaacc tccacgcgac tttggtggag gtagttcttt ggcagcgggc atggcgggta 120

ccgtggtgct ggacgatgtg gagctgcggg aggctcagag agattacctg gacttcctgg 180

acgacgagga agaccaggga atttatcaga gcaaagttcg ggagctgatc agtgacaacc 240

aataccggct gattgtcaat gtgaatgacc tgcgcaggaa aaacgagaag agggctaacc 300

ggcttctgaa caatgccttt gaggagctgg ttgccttcca gcgggcctta aaggattttg 360

tggcctccat tgatgctacc tatgccaagc agtatgagga gttctacgta ggactggaag 420

gcagctttgg ctccaagcac gtctccccgc ggactcttac ctcctgcttc ctcagctgtg 480

tggtctgtgt ggagggcatt gtcactaaat gttctctagt tcgtcccaaa gtcgtccgca 540

gtgtccacta ctgtcctgct actaagaaga ccatagagcg acgttattct gatctcacca 600

ccctggtggc ctttccctcc agctctgtct atcctaccaa ggatgaggag aacaatcccc 660

ttgagacaga atatggcctt tctgtctaca aggatcacca gaccatcacc atccaggaga 720

tgccggagaa ggccccagcc ggccagctcc cccgctctgt ggacgtcatt ctggatgatg 780

acttggtgga taaagcgaag cctggtgacc gggttcaggt ggtgggaacc taccgttgcc 840

ttcctggaaa gaagggaggc tacacctctg ggaccttcag gactgtcctg attgcctgta 900

atgttaagca gatgagcaag gatgctcagc cctctttctc tgctgaggat atagccaaga 960

tcaagaagtt cagtaaaacc cgatccaagg atatctttga ccagctggcc aagtcattgg 1020

ccccaagtat ccatgggcat gactatgtca agaaagcaat cctctgcttg ctcttgggag 1080

gggtggaacg agacctagaa aatggcagcc acatccgtgg ggacatcaat attcttctaa 1140

taggagaccc atccgttgcc aagtctcagc ttctgcggta tgtgctttgc actgcacccc 1200

gagctatccc caccactggc cggggctcct ctggagtggg tctgacggct gctgtcacca 1260

cagaccagga aacaggagag cgccgtctgg aagcaggggc catggtcctg gctgaccgag 1320

gcgtggtttg cattgatgaa tttgacaaaa tgtctgacat ggatcgcaca gccatccatg 1380

aagtgatgga gcagggtcga gtgaccattg ccaaggctgg catccatgct cggctgaatg 1440

cccgctgcag tgttttggca gctgccaacc ctgtctacgg caggtatgac cagtataaga 1500

ctccaatgga gaacattggg ctacaggact cactgctgtc acgatttgac ttgctcttca 1560

tcatgctgga tcagatggat cctgagcagg atcgggagat ctcagaccat gtccttcgga 1620

tgcaccgtta cagagcacct ggggagcagg atggcgatgc tatgcccttg ggtagtgctg 1680

tggatatcct ggccacagat gatcccaact ttagccagga agatcagcag gacacccaga 1740

tttatgagaa gcatgacaac cttctacatg ggaccaagaa gaaaaaggag aagatggtga 1800

gtgcagcatt catgaagaag tacatccatg tggccaaaat catcaagcct gtcctgacac 1860

aggagtcggc cacctacatt gcagaagagt attcacgcct gcgcagccag gatagcatga 1920

gctcagacac cgccaggaca tctccagtta cagcccgaac actggaaact ctgattcgac 1980

tggccacagc ccatgcgaag gcccgcatga gcaagactgt ggacctgcag gatgcagagg 2040

aagctgtgga gttggtccag tatgcttact ttaagaaggt tctggagaag gagaagaaac 2100

gtaagaagcg aagtgaggat gaatcagaga cagaagatga agaggagaaa agccaagagg 2160

accaggagca gaagaggaag agaaggaaga ctcgccagcc agatgccaaa gatggggatt 2220

catacgaccc ctatgacttc agtgacacag aggaggaaat gcctcaagta cacactccaa 2280

agacggcaga ctcacaggag accaaggaat cccagaaagt ggagttgagt gaatccaggt 2340

tgaaggcatt caaggtggcc ctcttggatg tgttccggga agctcatgcg cagtcaatcg 2400

gcatgaatcg cctcacagaa tccatcaacc gggacagcga agagcccttc tcttcagttg 2460

agatccaggc tgctctgagc aagatgcagg atgacaatca ggtcatggtg tctgagggca 2520

tcatcttcct catctgagga ggcctcgtct ctgaacttgg gttgtgccga gagagtttgt 2580

tctgtgtttc ccaccctctc cctgacccaa gtctttgcct ctactccctt aacagtgttg 2640

aattcaactg aaggcgagga atgttggtga tgaagctgag ttcaggactc ggtggaccct 2700

ttgggaatgg gtcatgaaag ctgccatggg gtgaggaaag aggagacagt gggagaggac 2760

aatgactatt gcatcttcat tgcaaaagca ctggctcatc cgccctactt cccatcccac 2820

acaaacccaa ttgtaaataa catatgactt ctgagtactt ttgggggcac aactgttttc 2880

tgtttgctgt ttttttgttt tgtttttttt ctccagagca ctttggtcta gactaggctt 2940

tgggtggttc caattggtgg agagaagctc tgaggcacgt catgcaggtc aagaaagctt 3000

tctttgcagt agcaccagtt aaggtgaata tgtattgtat cacaaaacaa acccaatatc 3060

cagatgaata tccgagatgt tgaataaact tagccatttc gtacacatgg taaaaaaaaa 3120

aaaaaaaaaa aaaaaaaaaa aaa 3143

<210> 47
<211> 3769
<212> DNA
<213> Homo sapiens

<400> 47
aaagctgcag cgtctggaaa aaagcgactt gtggcggtcg agcgtggcgc aggcgaatcc 60

tcggcactaa gcaaatatgg acctcgcggc ggcagcggag ccgggcgccg gcagccagca 120

cctggaggtc cgcgacgagg tggccgagaa gtgccagaaa ctgttcctgg acttcttgga 180

ggagtttcag agcagcgatg gagaaattaa atacttgcaa ttagcagagg aactgattcg 240

tcctgagaga aacacattgg ttgtgagttt tgtggacctg gaacaattta accagcaact 300
ttccaccacc attcaagagg agttctatag agtttaccct tacctgtgtc gggccttgaa 360
aacattcgtc aaagaccgta aagagatccc tcttgccaag gatttttatg ttgcattcca 420
agacctgcct accagacaca agattcgaga gctcacctca tccagaattg gtttgctcac 480
tcgcatcagt gggcaggtgg tgcggactca cccagttcac ccagagcttg tgagcggaac 540
ttttctgtgc ttggactgtc agacagtgat cagggatgta gaacagcagt tcaaatacac 600
acagccaaac atctgccgaa atccagtttg tgccaacagg aggagattct tactggatac 660
aaataaatca agatttgttg attttcaaaa ggttcgtatt caagagaccc aagctgagct 720
tcctcgaggg agtatccccc gcagtttaga agtaatttta agggctgaag ctgtggaatc 780
agctcaagct ggtgacaagt gtgactttac agggacactg attgttgtgc ctgacgtctc 840
caagcttagc acaccaggag cacgtgcaga aactaattcc cgtgtcagtg gtgttgatgg 900
atatgagaca gaaggcattc gaggactccg ggcccttggt gttagggacc tttcttatag 960
gctggtcttt cttgcctgct gtgttgcgcc aaccaaccca aggtttgggg ggaaagagct 1020
cagagatgag gaacagacag ctgagagcat taagaaccaa atgactgtga aagaatggga 1080
gaaagtgttt gagatgagtc aagataaaaa tctataccac aatctttgta ccagcctgtt 1140
ccctactata catggcaatg atgaagtaaa acggggtgtc ctgctgatgc tctttggtgg 1200
cgttccaaag acaacaggag aagggacctc tcttcgaggg gacataaatg tttgcattgt 1260
tggtgaccca agtacagcta agagccaatt tctcaagcac gtggaggagt tcagccccag 1320
agctgtctac accagtggta aagcgtccag tgctgctggc ttaacagcag ctgttgtgag 1380
agatgaagaa tctcatgagt ttgtcattga ggctggagct ttgatgttgg ctgataatgg 1440
tgtgtgttgt attgatgaat ttgataagat ggacgtgcgg gatcaagttg ctattcatga 1500
agctatggaa cagcagacca tatccatcac taaagcagga gtgaaggcta ctctgaacgc 1560
ccggacgtcc attttggcag cagcaaaccc aatcagtgga cactatgaca gatcaaaatc 1620
attgaaacag aatataaatt tgtcagctcc catcatgtcc cgattcgatc tcttctttat 1680
ccttgtggat gaatgtaatg aggttacaga ttatgccatt gccaggcgca tagtagattt 1740
gcattcaaga attgaggaat caattgatcg tgtctattcc ctcgatgata tcagaagata 1800
tcttctcttt gcaagacagt ttaaacccaa gatttccaaa gagtcagagg acttcattgt 1860
ggagcaatat aaacatctcc gccagagaga tggttctgga gtgaccaagt cttcatggag 1920
gattacagtg cgacagcttg agagcatgat tcgtctctct gaagctatgg ctcggatgca 1980
ctgctgtgat gaggtccaac ctaaacatgt gaaggaagct ttccggttac tgaataaatc 2040
aatcatccgt gtggaaacac ctgatgtcaa tctagatcaa gaggaagaga tccagatgga 2100
ggtagatgag ggtgctggtg gcatcaatgg tcatgctgac agccctgctc ctgtgaacgg 2160
gatcaatggc tacaatgaag acataaatca agagtctgct cccaaagcct ccttaaggct 2220
gggcttctct gagtactgcc gaatctctaa ccttattgtg cttcacctca gaaaggtgga 2280
agaagaagag gacgagtcag cattaaagag gagcgagctt gttaactggt acttgaagga 2340

aatcgaatca gagatagact ctgaagaaga acttataaat aaaaaaagaa tcatagagaa 2400

agttattcat cgactcacac actatgatca tgttctaatt gagctcaccc aggctggatt 2460

gaaaggctcc acagagggaa gtgagagcta tgaagaagat ccctacttgg tagttaaccc 2520

taactacttg ctcgaagatt gagatagtga aagtaactga ccagagctga ggaactgtgg 2580

cacagcacct cgtggcctgg agcctggctg gagctctgct agggacagaa gtgtttctgg 2640

aagtgatgct tccaggattt gttttcagaa acaagaattg agttgatggt cctatgtgtc 2700

acattcatca caggtttcat accaacacag gcttcagcac ttcctttggt gtgtttcctg 2760

tcccagtgaa gttggaacca aataatgtgt agtctctata accaatacct ttgttttcat 2820

gtgtaagaaa aggcccatta cttttaaggt atgtgctgtc ctattgagca aataactttt 2880

tttcaattgc cagctactgc ttttattcat caaaataaaa taacttgttc tgaagttgtc 2940

tattggattt ctttctactg taccctgatt attacttcca tctacttctg aatgtgagac 3000

tttccctttt tgcttaacct ggagtgaaga ggtagaactg tggtattatg gatgaggttt 3060

ctatgagaag gagtcattag agaactcata tgaaagctag aggccttaga gatgactttc 3120

caaggttaat tccagttgtt tttttttttt tttaagttta taaaagttta ttatactttt 3180

ttaaaattac tctttagtaa tttattttac ttctgtgtcc taagggtaat ttctcaggat 3240

tgttttcaaa ttgctttttt aggggaaata ggtcatttgc tatattacaa gcaatcccca 3300

aattttatgg tcttccagga aaagttatta ccgtttatga tactaacagt tcctgagact 3360

tagctatgat cagtatgttc atgaggtgga gcagttcctg tgttgcagct tttaacaaca 3420

gatggcattc attaaatcac aaagtatgtt aaaggtcaca aaagcaaaat aactgtctga 3480

ggctaaggcc cacgtgggac agtctaatac ccatgagtac tcaacttgcc ttgatgtctg 3540

agctttccag tgcaatgtga atttgagcag ccagaaatct attagtagaa agcaagacag 3600

attaatatag gttaaaacaa tgatttaaat atgtttctcc caataattat ctctttccct 3660

ggaatcaact tgtatgaaac cttgtcaaaa tgtactccac aagtatgtac aattaagtat 3720

tttaaaaata aatggcaaac attaaaaaca aaaaaaaaaa aaaaaaaaa 3769

<210> 48
<211> 2731
<212> DNA
<213> Homo sapiens

<400> 48
gcgcttggcg ggagatagaa aagtgcttca acccgcgccg gcggcgactg cagttcctgc 60

gagcgaggag cgcgggacct gctgacacgc tgacgccttc gagcgcggcc cggggcccgg 120

agcggccgga gcagcccggg tcctgacccc ggcccggctc ccgctccggg ctctgccggc 180

gggcgggcga gcgcggcgcg gtccgggccg gggggatgtc tcggcggacg cgctgcgagg 240

atctggatga gctgcactac caggacacag attcagatgt gccggagcag agggatagca 300

agtgcaaggt caaatggacc catgaggagg acgagcagct gagggccctg gtgaggcagt 360

ttggacagca ggactggaag ttcctggcca gccacttccc taaccgcact gaccagcaat 420

gccagtacag gtggctgaga gttttgaatc cagaccttgt caaggggcca tggaccaaag 480
aggaagacca aaaagtcatc gagctggtta agaagtatgg cacaaagcag tggacactga 540
ttgccaagca cctgaagggc cggctgggga agcagtgccg tgaacgctgg cacaaccacc 600
tcaaccctga ggtgaagaag tcttgctgga ccgaggagga ggaccgcatc atctgcgagg 660
cccacaaggt gctgggcaac cgctgggccg agatcgccaa gatgttgcca gggaggacag 720
acaatgctgt gaagaatcac tggaactcta ccatcaaaag gaaggtggac acaggaggct 780
tcttgagcga gtccaaagac tgcaagcccc cagtgtactt gctgctggag ctcgaggaca 840
aggacggcct ccagagtgcc cagcccacgg aaggccaggg aagtcttctg accaactggc 900
cctccgtccc tcctaccata aaggaggagg aaaacagtga ggaggaactt gcagcagcca 960
ccacatcgaa ggaacaggag cccatcggta cagatctgga cgcagtgcga acaccagagc 1020
ccttggagga attcccgaag cgtgaggacc aggaaggctc cccaccagaa acgagcctgc 1080
cttacaagtg ggtggtggag gcagctaacc tcctcatccc cgctgtgggt tctagcctct 1140
ctgaagccct ggacttgatc gagtcggacc ctgatgcttg gtgtgacctg agtaaatttg 1200
acctccctga ggaaccatct gcagaggaca gtatcaacaa cagcctagtg cagctgcaag 1260
cgtcacatca gcagcaagtc ctgccacccc gccagccttc cgccctggtg cccagtgtga 1320
ccgagtaccg cctggatggc cacaccatct cagacctgag ccggagcagc cggggcgagc 1380
tgatccccat ctcccccagc actgaagtcg gggctctggc cattggcaca ccgccctctg 1440
tgctcaagcg gcagaggaag aggcgtgtgg ctctgtcccc tgtcactgag aatagcacca 1500
gtctgtcctt cctggattcc tgtaacagcc tcacgcccaa gagcacacct gttaagaccc 1560
tgcccttctc gccctcccag tttctgaact tctggaacaa acaggacaca ttggagctgg 1620
agagcccctc gctgacatcc accccagtgt gcagccagaa ggtggtggtc accacaccac 1680
tgcaccggga caagacaccc ctgcaccaga aacatgctgc gtttgtaacc ccagatcaga 1740
agtactccat ggacaacact ccccacacgc caaccccgtt caagaacgcc ctggagaagt 1800
acggacccct gaagcccctg ccacagaccc cgcacctgga ggaggacttg aaggaggtgc 1860
tgcgttctga ggctggcatc gaactcatca tcgaggacga catcaggccc gagaagcaga 1920
agaggaagcc tgggctgcgg cggagcccca tcaagaaagt ccggaagtct ctggctcttg 1980
acattgtgga tgaggatgtg aagctgatga tgtccacact gcccaagtct ctatccttgc 2040
cgacaactgc cccttcaaac tcttccagcc tcaccctgtc aggtatcaaa gaagacaaca 2100
gcttgctcaa ccagggcttc ttgcaggcca agcccgagaa ggcagcagtg gcccagaagc 2160
cccgaagcca cttcacgaca cctgccccta tgtccagtgc ctggaagacg gtggcctgcg 2220
gggggaccag ggaccagctt ttcatgcagg agaaagcccg gcagctcctg ggccgcctga 2280
agcccagcca cacatctcgg accctcatct tgtcctgagg tgttgagggt gtcacgagcc 2340
cattctcatg tttacagggg ttgtgggggc agagggggtc tgtgaatctg agagtcattc 2400
aggtgacctc ctgcagggag ccttctgcca ccagcccctc cccagactct caggtggagg 2460
caacagggcc atgtgctgcc ctgttgccga gcccagctgt gggcggctcc tggtgctaac 2520

aacaaagttc cacttccagg tctgcctggt tccctcccca aggccacagg gagctccgtc 2580

agcttctccc aagcccacgt caggcctggc ctcatctcag accctgctta ggatggggga 2640

tgtggccagg ggtgctcctg tgctcaccct ctcttggtgc atttttttgg aagaataaaa 2700

ttgcctctct cttaaaaaaa aaaaaaaaaa a 2731

<210> 49
<211> 1121
<212> DNA
<213> Homo sapiens

<400> 49
ctagctctgt gggaaggttt tgggctctct ggctcggatt ttgcaatttc tccctgggga 60

ctgccgtgga gccgcatcca ctgtggatta taattgcaac atgacgctgg aagagctcgt 120

ggcgtgcgac aacgcggcgc agaagatgca gacggtgacc gccgcggtgg aggagctttt 180

ggtggccgct cagcgccagg atcgcctcac agtgggggtg tacgagtcgg ccaagttgat 240

gaatgtggac ccagacagcg tggtcctctg cctcttggcc attgacgagg aggaggagga 300

tgacatcgcc ctgcaaatcc acttcacgct catccagtcc ttctgctgtg acaacgacat 360

caacatcgtg cgggtgtcgg gcaatgcgcg cctggcgcag ctcctgggag agccggccga 420

gacccagggc accaccgagg cccgagacct ccactgtctt cccttcctac agaaccctca 480

cacggacgcc tggaagagcc acggcttggt ggaggtggcc agctactgcg aagaaagccg 540

gggcaacaac cagtgggtcc cctacatctc tcttcaggaa cgctgaggcc cttcccagca 600

gcagaatctg ttgagttgct gccaacaaac aaaaaataca ataaatattt gaaccccctc 660

ccccccagca caaccccccc aaaacaaccc aacccacgag gaccatcggg ggcaggtcgt 720

tggagactga agagaaagag agagaggaga agggagtgag gggccgctgc cgccttcccc 780

atcacggagg gtccagactg tccactcggg ggtggagtga gactgactgc aagccccacc 840

ctccttgaga ctggagctga gcgtctgcat acgagagact tggttgaaac ttggttggtc 900

cttgtctgca ccctcgacaa gaccacactt tgggacttgg gagctggggc tgaagttgct 960

ctgtacccat gaactcccag tttgcgaatt aataagagac aatctatttt gttacttgca 1020

cttgttattc gaaccactga gagcgagatg ggaagcatag atatctatat ttttatttct 1080

actatgaggg ccttgtaata aatttctaaa gcctcaaaaa a 1121

<210> 50
<211> 3323
<212> DNA
<213> Homo sapiens

<400> 50
aatctgccat tttctgtccc tgagtgagtc tctggcgtcc caaattgcct gtttttctcg 60

caggctctat tccgttcgct ggttcgccac ctcaggggaa cgatggccat ggagtccaca 120

gccactgccg ccgtcgccgc ggagctggtt tctgccgaca aaattgaaga tgttcctgct 180

ccttctacat ctgcagataa agtggagagt ctggatgtgg atagtgaagc taagaaacta 240

ttgggtttag gacagaaaca tctggtgatg ggggatattc cagcagctgt caatgcattc 300

caggaagcag ctagtctttt aggtaagaag tatggagaga cagctaatga gtgtggagaa 360
gccttctttt tctatgggaa atcacttctg gagttggcaa gaatggagaa tggtgtgttg 420
ggaaacgcct tggaaggtgt gcatgtggaa gaggaagaag gagaaaaaac agaagatgaa 480
tctctggtag aaaataatga taacatagat gaggaagcaa gggaagagtt gagagaacag 540
gtttatgacg ccatgggaga aaaagaagaa gccaaaaaaa cagaagacaa gtctttggca 600
aagcctgaaa ctgataaaga acaggacagt gaaatggaga agggtggaag agaagatatg 660
gatataagta aatctgcaga ggagccacag gaaaaagttg acttgactct agattggtta 720
actgaaacct ctgaagaggc aaaaggagga gcagcaccag aaggaccgaa tgaagctgag 780
gtcacttctg ggaagccaga acaggaagta ccagatgctg aggaagaaaa atcagtttct 840
ggaactgatg tccaagaaga gtgcagagaa aaaggaggtc aggagaagca gggagaggta 900
attgtgagca tagaggagaa gccaaaagaa gtttcagaag agcagcctgt ggtgactcta 960
gaaaagcagg gcactgcagt ggaggtagaa gcagagtctt tagacccgac agtcaagcca 1020
gtggatgtgg gtggggacga gccagaggag aaggtagtta cctctgaaaa cgaggcagga 1080
aaggcggttc ttgaacaact ggtaggtcaa gaagtaccac ctgctgaaga gtcaccagag 1140
gtgacaacag aggctgcaga ggcctcagct gtagaggctg gatcagaagt ctctgaaaag 1200
cctgggcagg aggctccagt tctccctaag gatggtgcag tcaatggacc gtcagttgta 1260
ggagatcaga ctcctattga accacagact tctatagaaa gactgacaga aacaaaagat 1320
ggctcaggac tagaggagaa ggtcagggca aagctggttc ctagtcagga ggagactaag 1380
ctgtctgtag aagagtctga ggcagctgga gatggggttg ataccaaggt agcccaggga 1440
gctactgaga aatcacctga agacaaagtt cagatagctg ctaatgaaga gacacaagag 1500
agagaagaac agatgaaaga gggtgaagaa actgaaggct cagaagagga tgataaagaa 1560
aatgataaga ccgaagaaat gccaaatgat tcagtccttg aaaacaagtc tcttcaagaa 1620
aatgaggagg aggagattgg gaacctagag cttgcctggg atatgctgga tttagcaaag 1680
atcattttta aaaggcaaga aacaaaagaa gcacagcttt atgctgccca ggcacatctt 1740
aaactcggag aagttagtgt tgaatctgaa aactatgtgc aagctgtgga ggagttccag 1800
tcctgcctta acctgcagga acagtacctg gaagcccacg accgtctcct tgcagagacc 1860
cactaccagc tgggcttggc ttatgggtac aactctcagt atgatgaggc agtggcacag 1920
ttcagcaaat ctattgaagt cattgagaac agaatggctg tactaaacga gcaggtgaag 1980
gaggctgaag gatcgtctgc tgaatacaag aaagaaattg aggaactaaa ggaactgcta 2040
cccgaaatta gagagaagat agaagatgca aaggagtctc agcgtagtgg gaatgtagct 2100
gaactggctc tgaaagctac tctggtggag agttctactt caggtttcac tcctggtgga 2160
ggaggctctt cagtctccat gattgccagt agaaagccaa cagacggtgc ttcctcatca 2220
aattgtgtga ctgatatttc ccaccttgtc agaaagaaga ggaaaccaga ggaagagagt 2280
ccccggaaag atgatgcaaa gaaagccaaa caagagccgg aggtgaacgg aggcagtggg 2340
gatgctgtcc ccagtggaaa tgaagtttcg gaaaacatgg aggaggaggc tgagaatcag 2400

gctgaaagcc gggcagcagt ggaggggaca gtggaggctg gagctacagt tgaaagcact 2460

gcatgttaag agggggcaca gccctcctcc caagggaaag tgtttttgta tataatgtat 2520

tttttcactt ttggaggatt ctttttgtat aacttcaata aagattgtaa gcaaaggttg 2580

aggctttgat ggtttttttc ttaattattg gctgaatctg ccttggagca ctgctggttt 2640

tatatattag ccaaaggttt tgttctggcc ttctgtactg atctgtgttc ctgatcctaa 2700

ttcctatctg tctaacgtgg aggtgatcaa gtgtggctgt aggcctttgt tttccaatgg 2760

tgctatattc tgttttcaaa cacttcactg aacccagctg tcttgcaaac tttcagtggt 2820

gctgtccctg gatgggggct acaaaaacaa gaattggtga agatcttgct cttcagtgct 2880

gaaaatggat gatggacttt ggctgtgagc caggcctagg atggttcttg tcctatatcc 2940

acctagtctt cacctggggc tataattctg tcctggaaaa agaactctga aaacctgggt 3000

caggggaatg attcctaagg aaaacggtct gcatttgagc tctggtttga aagtagccaa 3060

ggggactgat ggtggacact ccagatgtgg ttggaagcat atgtggggag gctggctggt 3120

tgagttttgt tattttctgt atagaaaggt tgagatatat caacacttgg aattgttacc 3180

catctgcaga attgacttct caaataaaga tgctaaaaat ctcaaaaaaa aaaaaaaaaa 3240

aaaaaaaaaa aaaaaaaaaa aaaaaaaaaa aaaaaaaaaa aaaaaaaaaa aaaaaaaaaa 3300

aaaaaaaaaa aaaaaaaaaa aaa 3323

<210> 51
<211> 2306
<212> DNA
<213> Homo sapiens

<400> 51
   aatctgccat tttctgtccc tgagtgagtc tctggcgtcc caaattgcct gtttttctcg 60
   caggctctat tccgttcgct ggttcgccac ctcaggggaa cgatggccat ggagtccaca 120
   gccactgccg ccgtcgccgc ggagctggtt tctgccgaca aaattgaaga tgttcctgct 180
   ccttctacat ctgcagataa agtggagagt ctggatgtgg atagtgaagc taagaaacta 240
   ttgggtttag gacagaaaca tctggtgatg ggggatattc cagcagctgt caatgcattc 300
   caggaagcag ctagtctttt aggtaagaag tatggagaga cagctaatga gtgtggagaa 360
   gccttctttt tctatggaaa atcacttctg gagttggcaa gaatggagaa tggtgtgttg 420
   ggaaacgcct tggaaggtgt gcatgtggaa gaggaagaag gagaaaaaac agaagatgaa 480
   tctctggtag aaaataatga taacatagat gaaactgaag gctcagaaga ggatgataaa 540
   gaaaatgata agaccgaaga aatgccaaat gattcagtcc ttgaaaacaa gtctcttcaa 600
   gaaaatgagg aggaggagat tgggaaccta gagcttgcct gggatatgct ggatttagca 660
   aagatcattt ttaaaaggca agaaacaaaa gaagcacagc tttatgctgc ccaggcacat 720
   cttaaactcg gagaagttag tgttgaatct gaaaactatg tgcaagctgt ggaggagttc 780
   cagtcctgcc ttaacctgca ggaacagtac ctggaagccc acgaccgtct ccttgcagag 840
   acccactacc agctgggctt ggcttatggg tacaactctc agtatgatga ggcagtggca 900
   cagttcagca aatctattga agtcattgag aacagaatgg ctgtactaaa cgagcaggtg 960
   aaggaggctg aaggatcgtc tgctgaatac aagaaagaaa ttgaggaact aaaggaactg 1020
   ctacccgaaa ttagagagaa gatagaagat gcaaaggagt ctcagcgtag tgggaatgta 1080
   gctgaactgg ctctgaaagc tactctggtg gagagttcta cttcaggttt cactcctggt 1140
   ggaggaggct cttcagtctc catgattgcc agtagaaagc caacagacgg tgcttcctca 1200
   tcaaattgtg tgactgatat ttcccacctt gtcagaaaga agaggaaacc agaggaagag 1260
   agtccccgga aagatgatgc aaagaaagcc aaacaagagc cggaggtgaa cggaggcagt 1320
   ggggatgctg tccccagtgg aaatgaagtt tcggaaaaca tggaggagga ggctgagaat 1380
   caggctgaaa gccgggcagc agtggagggg acagtggagg ctggagctac agttgaaagc 1440
   actgcatgtt aagagggggc acagccctcc tcccaaggga aagtgttttt gtatataatg 1500
   tattttttca cttttggagg attctttttg tataacttca ataaagattg taagcaaagg 1560
   ttgaggcttt gatggttttt ttcttaatta ttggctgaat ctgccttgga gcactgctgg 1620
   ttttatatat tagccaaagg ttttgttctg gccttctgta ctgatctgtg ttcctgatcc 1680
   taattcctat ctgtctaacg tggaggtgat caagtgtggc tgtaggcctt tgttttccaa 1740
   tggtgctata ttctgttttc aaacacttca ctgaacccag ctgtcttgca aactttcagt 1800
   ggtgctgtcc ctggatgggg gctacaaaaa caagaattgg tgaagatctt gctcttcagt 1860
   gctgaaaatg gatgatggac tttggctgtg agccaggcct aggatggttc ttgtcctata 1920
   tccacctagt cttcacctgg ggctataatt ctgtcctgga aaaagaactc tgaaaacctg 1980
   ggtcagggga atgattccta aggaaaacgg tctgcatttg agctctggtt tgaaagtagc 2040
   caaggggact gatggtggac actccagatg tggttggaag catatgtggg gaggctggct 2100
   ggttgagttt tgttattttc tgtatagaaa ggttgagata tatcaacact tggaattgtt 2160
   acccatctgc agaattgact tctcaaataa agatgctaaa aatctcaaaa aaaaaaaaaa 2220
   aaaaaaaaaa aaaaaaaaaa aaaaaaaaaa aaaaaaaaaa aaaaaaaaaa aaaaaaaaaa 2280
   aaaaaaaaaa aaaaaaaaaa aaaaaa 2306

<210> 52
<211> 3396
<212> DNA
<213> Homo sapiens

<400> 52
aatctgccat tttctgtccc tgagtgagtc tctggcgtcc caaattgcct gtttttctcg 60

caggctctat tccgttcgct ggttcgccac ctcaggggaa cgatggccat ggagtccaca 120

gccactgccg ccgtcgccgc ggagctggtt tctgccgaca aaattgaaga tgttcctgct 180

ccttctacat ctgcagataa agtggagagc aaccattaat ctgctttctg tcactgaaga 240

tgggttgcat tttgtagaat attatctaaa tagaatcata catctggatg tggatagtga 300

agctaagaaa ctattgggtt taggacagaa acatctggtg atgggggata ttccagcagc 360

tgtcaatgca ttccaggaag cagctagtct tttaggtaag aagtatggag agacagctaa 420

tgagtgtgga gaagccttct ttttctatgg gaaatcactt ctggagttgg caagaatgga 480

gaatggtgtg ttgggaaacg ccttggaagg tgtgcatgtg gaagaggaag aaggagaaaa 540

aacagaagat gaatctctgg tagaaaataa tgataacata gatgaggaag caagggaaga 600

gttgagagaa caggtttatg acgccatggg agaaaaagaa gaagccaaaa aaacagaaga 660

caagtctttg gcaaagcctg aaactgataa agaacaggac agtgaaatgg agaagggtgg 720

aagagaagat atggatataa gtaaatctgc agaggagcca caggaaaaag ttgacttgac 780

tctagattgg ttaactgaaa cctctgaaga ggcaaaagga ggagcagcac cagaaggacc 840

gaatgaagct gaggtcactt ctgggaagcc agaacaggaa gtaccagatg ctgaggaaga 900

aaaatcagtt tctggaactg atgtccaaga agagtgcaga gaaaaaggag gtcaggagaa 960

gcagggagag gtaattgtga gcatagagga gaagccaaaa gaagtttcag aagagcagcc 1020

tgtggtgact ctagaaaagc agggcactgc agtggaggta gaagcagagt ctttagaccc 1080

gacagtcaag ccagtggatg tgggtgggga cgagccagag gagaaggtag ttacctctga 1140

aaacgaggca ggaaaggcgg ttcttgaaca actggtaggt caagaagtac cacctgctga 1200

agagtcacca gaggtgacaa cagaggctgc agaggcctca gctgtagagg ctggatcaga 1260

agtctctgaa aagcctgggc aggaggctcc agttctccct aaggatggtg cagtcaatgg 1320

accgtcagtt gtaggagatc agactcctat tgaaccacag acttctatag aaagactgac 1380

agaaacaaaa gatggctcag gactagagga gaaggtcagg gcaaagctgg ttcctagtca 1440

ggaggagact aagctgtctg tagaagagtc tgaggcagct ggagatgggg ttgataccaa 1500

ggtagcccag ggagctactg agaaatcacc tgaagacaaa gttcagatag ctgctaatga 1560

agagacacaa gagagagaag aacagatgaa agagggtgaa gaaactgaag gctcagaaga 1620

ggatgataaa gaaaatgata agaccgaaga aatgccaaat gattcagtcc ttgaaaacaa 1680

gtctcttcaa gaaaatgagg aggaggagat tgggaaccta gagcttgcct gggatatgct 1740

ggatttagca aagatcattt ttaaaaggca agaaacaaaa gaagcacagc tttatgctgc 1800

ccaggcacat cttaaactcg gagaagttag tgttgaatct gaaaactatg tgcaagctgt 1860

ggaggagttc cagtcctgcc ttaacctgca ggaacagtac ctggaagccc acgaccgtct 1920

ccttgcagag acccactacc agctgggctt ggcttatggg tacaactctc agtatgatga 1980

ggcagtggca cagttcagca aatctattga agtcattgag aacagaatgg ctgtactaaa 2040

cgagcaggtg aaggaggctg aaggatcgtc tgctgaatac aagaaagaaa ttgaggaact 2100

aaaggaactg ctacccgaaa ttagagagaa gatagaagat gcaaaggagt ctcagcgtag 2160

tgggaatgta gctgaactgg ctctgaaagc tactctggtg gagagttcta cttcaggttt 2220

cactcctggt ggaggaggct cttcagtctc catgattgcc agtagaaagc caacagacgg 2280

tgcttcctca tcaaattgtg tgactgatat ttcccacctt gtcagaaaga agaggaaacc 2340

agaggaagag agtccccgga aagatgatgc aaagaaagcc aaacaagagc cggaggtgaa 2400

cggaggcagt ggggatgctg tccccagtgg aaatgaagtt tcggaaaaca tggaggagga 2460

ggctgagaat caggctgaaa gccgggcagc agtggagggg acagtggagg ctggagctac 2520

agttgaaagc actgcatgtt aagagggggc acagccctcc tcccaaggga aagtgttttt 2580

gtatataatg tattttttca cttttggagg attctttttg tataacttca ataaagattg 2640

taagcaaagg ttgaggcttt gatggttttt ttcttaatta ttggctgaat ctgccttgga 2700

gcactgctgg ttttatatat tagccaaagg ttttgttctg gccttctgta ctgatctgtg 2760

ttcctgatcc taattcctat ctgtctaacg tggaggtgat caagtgtggc tgtaggcctt 2820

tgttttccaa tggtgctata ttctgttttc aaacacttca ctgaacccag ctgtcttgca 2880

aactttcagt ggtgctgtcc ctggatgggg gctacaaaaa caagaattgg tgaagatctt 2940

gctcttcagt gctgaaaatg gatgatggac tttggctgtg agccaggcct aggatggttc 3000

ttgtcctata tccacctagt cttcacctgg ggctataatt ctgtcctgga aaaagaactc 3060

tgaaaacctg ggtcagggga atgattccta aggaaaacgg tctgcatttg agctctggtt 3120

tgaaagtagc caaggggact gatggtggac actccagatg tggttggaag catatgtggg 3180

gaggctggct ggttgagttt tgttattttc tgtatagaaa ggttgagata tatcaacact 3240

tggaattgtt acccatctgc agaattgact tctcaaataa agatgctaaa aatctcaaaa 3300

aaaaaaaaaa aaaaaaaaaa aaaaaaaaaa aaaaaaaaaa aaaaaaaaaa aaaaaaaaaa 3360

aaaaaaaaaa aaaaaaaaaa aaaaaaaaaa aaaaaa 3396

<210> 53
<211> 2130
<212> DNA
<213> Homo sapiens

<400> 53
ggttaaacgg ggcccaaggc aggggtggcg ggtcagtgct gctcgggggc ttctccatcc 60

aggtccctgg agttcctggt ccctggagct ccgcacttgg cggcgcaacc tgcgtgaggc 120

agcgcgactc tggcgactgg ccggccatgc cttcccgggc tgaggactat gaagtgttgt 180

acaccattgg cacaggctcc tacggccgct gccagaagat ccggaggaag agtgatggca 240

agatattagt ttggaaagaa cttgactatg gctccatgac agaagctgag aaacagatgc 300

ttgtttctga agtgaatttg cttcgtgaac tgaaacatcc aaacatcgtt cgttactatg 360

atcggattat tgaccggacc aatacaacac tgtacattgt aatggaatat tgtgaaggag 420

gggatctggc tagtgtaatt acaaagggaa ccaaggaaag gcaatactta gatgaagagt 480

ttgttcttcg agtgatgact cagttgactc tggccctgaa ggaatgccac agacgaagtg 540

atggtggtca taccgtattg catcgggatc tgaaaccagc caatgttttc ctggatggca 600

agcaaaacgt caagcttgga gactttgggc tagctagaat attaaaccat gacacgagtt 660

ttgcaaaaac atttgttggc acaccttatt acatgtctcc tgaacaaatg aatcgcatgt 720

cctacaatga gaaatcagat atctggtcat tgggctgctt gctgtatgag ttatgtgcat 780

taatgcctcc atttacagct tttagccaga aagaactcgc tgggaaaatc agagaaggca 840

aattcaggcg aattccatac cgttactctg atgaattgaa tgaaattatt acgaggatgt 900

taaacttaaa ggattaccat cgaccttctg ttgaagaaat tcttgagaac cctttaatag 960

cagatttggt tgcagacgag caaagaagaa atcttgagag aagagggcga caattaggag 1020

agccagaaaa atcgcaggat tccagccctg tattgagtga gctgaaactg aaggaaattc 1080

agttacagga gcgagagcga gctctcaaag caagagaaga aagattggag cagaaagaac 1140

aggagctttg tgttcgtgag agactagcag aggacaaact ggctagagca gaaaatctgt 1200

tgaagaacta cagcttgcta aaggaacgga agttcctgtc tctggcaagt aatccagaac 1260

ttcttaatct tccatcctca gtaattaaga agaaagttca tttcagtggg gaaagtaaag 1320

agaacatcat gaggagtgag aattctgaga gtcagctcac atctaagtcc aagtgcaagg 1380

acctgaagaa aaggcttcac gctgcccagc tgcgggctca agccctgtca gatattgaga 1440

aaaattacca actgaaaagc agacagatcc tgggcatgcg ctagccaggt agagagacac 1500

agagctgtgt acaggatgta atattaccaa cctttaaaga ctgatattca aatgctgtag 1560

tgttgaatac ttggttccat gagccatgcc tttctgtata gtacacatga tatttcggaa 1620

ttggttttac tgttcttcag caactattgt acaaaatgtt cacatttaat ttttctttct 1680

tcttttaaga acatattata aaaagaatac tttcttggtt gggcttttaa tcctgtgtgt 1740

gattactagt aggaacatga gatgtgacat tctaaatctt gggagaaaaa ataatgttag 1800

gaaaaaaata tttatgcagg aagagtagca ctcactgaat agttttaaat gactgagtgg 1860

tatgcttaca attgtcatgt ctagatttaa attttaagtc tgagatttta aatgtttttg 1920

agcttagaaa acccagttag atgcaatttg gtcattaata ccatgacatc ttgcttataa 1980

atattccatt gctctgtagt tcaaatctgt tagctttgtg aaaattcatc actgtgatgt 2040

ttgtattctt tttttttttc tgtttaacag aatatgagct gtctgtcatt tacctacttc 2100

tttcccacta aataaaagaa ttcttcagtt 2130

<210> 54
<211> 1021
<212> DNA
<213> Homo sapiens

<400> 54
cgggaagcgc cgggcgagcc cactgtgcgg ccgtcgtggg ggaagcgaag gttcctgatt 60

ccacctacct cctaagtgaa ggcttccgtc cctggagagg aggcggcgcc ctgtatcggc 120

cttcgtcctc cgcgggtgtg ctagcgttgg gacggtcctt tgttgccgcg aggggtagga 180

gtgggcgtgg cggagccagc tccgttcgga acactcccgg gccgacccga ctcgctcatc 240

ctgcaggagc tgcggcgcca agatgagtgg agaggagaac ccagccagca agcccacgcc 300

ggtgcaggac gtacagggcg acgggcgctg gatgtccctg caccatcggt tcgtggctga 360

cagcaaagat aaggaacccg aagtcgtctt catcggggac tccttggtcc agctcatgca 420

ccagtgcgag atctggcgcg agctcttctc tcctctgcat gcacttaact ttggcattgg 480

tggtgacggc acacagcatg tactgtggcg gctggagaat ggggagctgg aacacatccg 540

gcccaagatt gtggtggtct gggtgggcac caacaaccac ggacacacag cagagcaggt 600

gactggtggc atcaaggcca ttgtgcaact ggtgaatgag cgacagcccc aggcccgggt 660

tgtggtgctg ggcctgcttc cgcgaggcca acatcccaac ccacttcggg agaagaaccg 720

acaggtgaac gagctggtac gggcggcact ggctggccac cctcgggccc acttcctaga 780

tgccgaccct ggctttgtgc actcagatgg caccatcagc catcatgaca tgtatgatta 840

cctgcatctg agccgcctgg gctacacacc tgtttgccgg gctctgcact ccctgcttct 900

gcgtctgctg gcccaagacc agggccaagg tgctcccctg ctggagcccg caccctaagc 960

atcctgctgc cttcccacaa cattaaactc tccttcctca gtgaaaaaaa aaaaaaaaaa 1020

a 1021

<210> 55
<211> 2547
<212> DNA
<213> Homo sapiens

<400> 55
ccacgcgtcc gcggacgcgt gggcgagaag tgcttccaag cgtccatttt gagccttgga 60

aactacgacg accaaagggc cacgggttcc tgggtcgttt ctcatttccg tcgagttaaa 120

cgtctggggc tgcttctgag gaatcagctt ggctggccag caagttcagc tccggcaagt 180

catttgattc acccggtgat gaaatggggg tcaaccagtc tgtgggcttt ccacctgtca 240

caggacccca cctcgtaggc tgtggggatg tgatggaggg tcagaatctc caggggagct 300

tctttcgact cttctacccc tgccaaaagg cagaggagac catggagcag cccctgtgga 360

ttccccgcta tgagtactgc actggcctgg ccgagtacct gcagtttaat aagcgctgcg 420

ggggcttgct gttcaacctg gcggtgggat cttgtcgcct gcctgttagc tggaatggcc 480

cctttaagac aaaggactct ggatacccct tgatcatctt ctcccatggc ctaggagcct 540

tcaggacttt gtattcagcc ttctgcatgg agctggcctc acgtggcttt gtggttgctg 600

tgccagagca cagggaccgg tcagcggcaa ccacctattt ctgcaagcag gccccagaag 660

agaaccagcc caccaatgaa tcgctgcagg aggaatggat ccctttccgt cgagttgagg 720

aaggggagaa ggaatttcat gttcggaatc cccaggtgca tcagcgggta agcgagtgtt 780

tacgggtgtt gaagatcctg caagaggtca ctgctgggca gactgtcttc aacatcttgc 840

ctggtggctt ggatctgatg actttgaagg gcaacattga catgagccgt gtggctgtga 900

tgggacattc atttggaggg gccacagcta ttctggcttt ggccaaggag acccaatttc 960

ggtgtgcggt ggctctggat gcttggatgt ttcctctgga acgtgacttt taccccaagg 1020

cccgaggacc tgtgttcttt atcaatactg agaaattcca gacaatggag agtgtcaatt 1080

tgatgaagaa gatatgtgcc cagcatgaac agtctaggat cataaccgtt cttggttctg 1140

ttcatcggag tcaaactgac tttgcttttg tgactggcaa cttgattggt aaattcttct 1200

ccactgaaac ccgtgggagc ctggacccct atgaagggca ggaggttatg gtacgggcca 1260

tgttggcctt cctgcagaag cacctcgacc tgaaagaaga ctataatcaa tggaacaacc 1320

ttattgaagg cattggaccg tcgctcaccc caggggcccc ccaccatctg tccagcctgt 1380

aggcacaact ggccatttgt aaagtcactt cagccaagtt ttcatttggg agctacccaa 1440

gggcacccat gagctcctat caagaagtga tcaacgtgac cccttttcac agattgaaag 1500

gtgtaatcac actgctgctt ggataactgg gtactttgat cttagatttg atcttaaaat 1560

cactttggga ctgggatccc ttgctgattg acaaacagac tttctgggac cttgatggag 1620

tggggaacaa gcagtagagt gggactgggg gagacccagg ccccgggctg agcactgtga 1680

ggcctggatg tgaagactca gcccagcgaa gctcattccc ttacccccgg ccagtgctgc 1740

tgcttcagtg gaagagatga agccaaagga cagaatgaaa atccctacct tcagagactc 1800

tagcccagcc caacaccatc tcttcctacc tctcagcctt ctccctcccc agggccactt 1860

gttgaagtct gagcacttta tgtaaatttc taggtgtgag ccgtgatcac attttctatt 1920

tatttccaag tcttctcatt gtatggaaca tagtactact tatacttaca gtagtaagtt 1980

atacttgtga gcccacagag tggcagacag catggctctc acagcacagg gagaaaaact 2040

gaggtacaca gaggtacctc agaagctctg gatgtctttg gggtttttgc taagtgtatc 2100

ttgataggaa acaacaaaag caggttgaga tggggaagat gacagaacaa cagtgttaaa 2160

tggccatttg cacaggcctt tgccacaaca gagaagtagt ttggtcagct aaaactcagc 2220

tgcagcctgg acagtagagc gagaccccat cttaaaaata aagaaggctg ggcgtggtgg 2280

ctcatgcctg taatcccagc actttgggag gccaaggcag gcagatcact taaggccagg 2340

agttcaagac cacctggcca acatggtgaa accccgtctc tactaaaaat acaaaaaatt 2400

agcctggcgt aatggcaggc gcctataatc ccagctactc aggaggctga agcagaagaa 2460

tcacttgaac ctaggaggcg gaggttgcag tgagtcaaga tcgcgccact gcactccagc 2520

ctgggtgaca gagcaagact ctgtctt 2547

<210> 56
<211> 1355
<212> DNA
<213> Homo sapiens

<400> 56
ggatggccgg agctggcgcc ctggttctgg aggtaaccgg ttactgaggg cgagaagcgc 60

cacccggagg ctctagcctg acaaatgctt gctgacctgg gccagagctc ttcccttacg 120

caagtctcag ccggtcgtcg cgacgttcgc ccgctcgctc tgaggctcct gaagccgaaa 180

ccagctagac tttcctcctt cccgcctgcc tgtagcggcg ttgttgccac tccgccacca 240

tgttcgaggc gcgcctggtc cagggctcca tcctcaagaa ggtgttggag gcactcaagg 300

acctcatcaa cgaggcctgc tgggatatta gctccagcgg tgtaaacctg cagagcatgg 360

actcgtccca cgtctctttg gtgcagctca ccctgcggtc tgagggcttc gacacctacc 420

gctgcgaccg caacctggcc atgggcgtga acctcaccag tatgtccaaa atactaaaat 480

gcgccggcaa tgaagatatc attacactaa gggccgaaga taacgcggat accttggcgc 540

tagtatttga agcaccaaac caggagaaag tttcagacta tgaaatgaag ttgatggatt 600

tagatgttga acaacttgga attccagaac aggagtacag ctgtgtagta aagatgcctt 660

ctggtgaatt tgcacgtata tgccgagatc tcagccatat tggagatgct gttgtaattt 720

cctgtgcaaa agacggagtg aaattttctg caagtggaga acttggaaat ggaaacatta 780

aattgtcaca gacaagtaat gtcgataaag aggaggaagc tgttaccata gagatgaatg 840

aaccagttca actaactttt gcactgaggt acctgaactt ctttacaaaa gccactccac 900

tctcttcaac ggtgacactc agtatgtctg cagatgtacc ccttgttgta gagtataaaa 960

ttgcggatat gggacactta aaatactact tggctcccaa gatcgaggat gaagaaggat 1020

cttaggcatt cttaaaattc aagaaaataa aactaagctc tttgagaact gcttctaaga 1080

tgccagcata tactgaagtc ttttctgtca ccaaatttgt acctctaagt acatatgtag 1140

atattgtttt ctgtaaataa cctatttttt tctctattct ctgcaatttg tttaaagaat 1200

aaagtccaaa gtcagatctg gtctagttaa cctagaagta tttttgtctc ttagaaatac 1260

ttgtgatttt tataatacaa aagggtcttg actctaaatg cagttttaag aattgttttt 1320

gaatttaaat aaagttactt gaatttcaaa catca 1355

<210> 57
<211> 1319
<212> DNA
<213> Homo sapiens

<400> 57
aacgcggcgc agggtgagag cgcgcgcttg cggacgcggc ggcattaaac ggttgcaggc 60

gtagcagagt ggtcgttgtc tttctaggtc tcagccggtc gtcgcgacgt tcgcccgctc 120

gctctgaggc tcctgaagcc gaaaccagct agactttcct ccttcccgcc tgcctgtagc 180

ggcgttgttg ccactccgcc accatgttcg aggcgcgcct ggtccagggc tccatcctca 240

agaaggtgtt ggaggcactc aaggacctca tcaacgaggc ctgctgggat attagctcca 300

gcggtgtaaa cctgcagagc atggactcgt cccacgtctc tttggtgcag ctcaccctgc 360

ggtctgaggg cttcgacacc taccgctgcg accgcaacct ggccatgggc gtgaacctca 420

ccagtatgtc caaaatacta aaatgcgccg gcaatgaaga tatcattaca ctaagggccg 480

aagataacgc ggataccttg gcgctagtat ttgaagcacc aaaccaggag aaagtttcag 540

actatgaaat gaagttgatg gatttagatg ttgaacaact tggaattcca gaacaggagt 600

acagctgtgt agtaaagatg ccttctggtg aatttgcacg tatatgccga gatctcagcc 660

atattggaga tgctgttgta atttcctgtg caaaagacgg agtgaaattt tctgcaagtg 720

gagaacttgg aaatggaaac attaaattgt cacagacaag taatgtcgat aaagaggagg 780

aagctgttac catagagatg aatgaaccag ttcaactaac ttttgcactg aggtacctga 840

acttctttac aaaagccact ccactctctt caacggtgac actcagtatg tctgcagatg 900

taccccttgt tgtagagtat aaaattgcgg atatgggaca cttaaaatac tacttggctc 960

ccaagatcga ggatgaagaa ggatcttagg cattcttaaa attcaagaaa ataaaactaa 1020

gctctttgag aactgcttct aagatgccag catatactga agtcttttct gtcaccaaat 1080

ttgtacctct aagtacatat gtagatattg ttttctgtaa ataacctatt tttttctcta 1140

ttctctgcaa tttgtttaaa gaataaagtc caaagtcaga tctggtctag ttaacctaga 1200

agtatttttg tctcttagaa atacttgtga tttttataat acaaaagggt cttgactcta 1260

aatgcagttt taagaattgt ttttgaattt aaataaagtt acttgaattt caaacatca 1319

<210> 58
<211> 2204

<212>  DNA
<213>  Homo sapiens

<400>  58
   gagcggtgcg gaggctctgc tcggatcgag gtctgcagcg cagcttcggg agcatgagtg      60
   ctgcagtgac tgcagggaag ctggcacggg caccggccga ccctgggaaa gccggggtcc     120
   ccggagttgc agctcccgga gctccggcgg cggctccacc ggcgaaagag atcccggagg     180
   tcctagtgga cccacgcagc cggcggcgct atgtgcgggg ccgctttttg ggcaagggcg     240
   gctttgccaa gtgcttcgag atctcggacg cggacaccaa ggaggtgttc gcgggcaaga     300
   ttgtgcctaa gtctctgctg ctcaagccgc accagaggga gaagatgtcc atggaaatat     360
   ccattcaccg cagcctcgcc caccagcacg tcgtaggatt ccacggcttt ttcgaggaca     420
   acgacttcgt gttcgtggtg ttggagctct gccgccggag gtctctcctg gagctgcaca     480
   agaggaggaa agccctgact gagcctgagg cccgatacta cctacggcaa attgtgcttg     540
   gctgccagta cctgcaccga aaccgagtta ttcatcgaga cctcaagctg ggcaaccttt     600
   tcctgaatga agatctggag gtgaaaatag gggattttgg actggcaacc aaagtcgaat     660
   atgacgggga gaggaagaag accctgtgtg ggactcctaa ttacatagct cccgaggtgc     720
   tgagcaagaa agggcacagt ttcgaggtgg atgtgtggtc cattgggtgt atcatgtata     780
   ccttgttagt gggcaaacca ccttttgaga cttcttgcct aaaagagacc tacctccgga     840
   tcaagaagaa tgaatacagt attcccaagc acatcaaccc cgtggccgcc tccctcatcc     900
   agaagatgct tcagacagat cccactgccc gcccaaccat taacgagctg cttaatgacg     960
   agttctttac ttctggctat atccctgccc gtctccccat cacctgcctg accattccac    1020
   caaggttttc gattgctccc agcagcctgg accccagcaa ccggaagccc ctcacagtcc    1080
   tcaataaagg cttggagaac cccctgcctg agcgtccccg ggaaaaagaa gaaccagtgg    1140
   ttcgagagac aggtgaggtg gtcgactgcc acctcagtga catgctgcag cagctgcaca    1200
   gtgtcaatgc ctccaagccc tcggagcgtg ggctggtcag gcaagaggag gctgaggatc    1260
   ctgcctgcat ccccatcttc tgggtcagca agtgggtgga ctattcggac aagtacggcc    1320
   ttgggtatca gctctgtgat aacagcgtgg gggtgctctt caatgactca acacgcctca    1380
   tcctctacaa tgatggtgac agcctgcagt acatagagcg tgacggcact gagtcctacc    1440
   tcaccgtgag ttcccatccc aactccttga tgaagaagat caccctcctt aaatatttcc    1500
   gcaattacat gagcgagcac ttgctgaagg caggtgccaa catcacgccg cgcgaaggtg    1560
   atgagctcgc ccggctgccc tacctacgga cctggttccg cacccgcagc gccatcatcc    1620
   tgcacctcag caacggcagc gtgcagatca acttcttcca ggatcacacc aagctcatct    1680
   tgtgcccact gatggcagcc gtgacctaca tcgacgagaa gcgggacttc cgcacatacc    1740
   gcctgagtct cctggaggag tacggctgct gcaaggagct ggccagccgg ctccgctacg    1800
   cccgcactat ggtggacaag ctgctgagct cacgctcggc cagcaaccgt ctcaaggcct    1860
   cctaatagct gccctcccct ccggactggt gccctcctca ctcccacctg catctggggc    1920
   ccatactggt tggctcccgc ggtgccatgt ctgcagtgtg ccccccagcc ccggtggctg    1980
   ggcagagctg catcatcctt gcaggtgggg gttgctgtgt aagttatttt tgtacatgtt 2040
   cgggtgtggg ttctacagcc ttgtccccct ccccctcaac cccaccatat gaattgtaca 2100
   gaatatttct attgaattcg gaactgtcct ttccttggct ttatgcacat taaacagatg 2160
   tgaatattca aaaaaaaaaa aaaaaaaaaa aaaaaaaaaa aaaa 2204

<210> 59
<211> 1644
<212> DNA
<213> Homo sapiens

<400> 59
gatgacgtaa ttttcctgcg cctcggggcg agcagcggcg cgcaaggaaa gatcgggttc 60

cgtttttccc gcggattctg gtgcctgtgg ggccggtgac ccaacaccat gaaggaaacg 120

ccactctcaa actgcgaacg ccgcttccta ctccgtgcca tcgaagagaa gaagcggctg 180

gatggcagac aaacctatga ttataggaac atcaggatct catttggaac agattacgga 240

tgctgcattg tggaacttgg aaaaacaaga gttcttggac aggtttcctg tgaacttgtg 300

tctccaaaac tcaatcgggc aacagaaggt attctttttt ttaaccttga actctctcag 360

atggccgctc cagctttcga acctggcagg cagtcagatc tcttggtgaa gttgaatcga 420

ctcatggaaa gatgtctaag aaattcgaag tgtatagaca ctgagtctct ctgtgttgtt 480

gctggtgaaa aggtttggca aatacgtgta gacctacatt tattaaatca tgatggaaat 540

attattgatg ctgccagcat tgctgcaatc gtggccttat gtcatttccg aagacctgat 600

gtctctgtcc aaggagatga agtaacactg tatacacctg aagagcgtga tcctgtacca 660

ttaagtatcc accacatgcc catttgtgtc agttttgcct ttttccagca aggaacatat 720

ttattggtgg atcccaatga acgagaagaa cgtgtgatgg atggcttgct ggtgattgcc 780

atgaacaaac atcgagagat ttgtactatc cagtccagtg gtgggataat gctactaaaa 840

gatcaagttc tgagatgcag taaaatcgct ggtgtgaaag tagcagaaat tacagagcta 900

atattgaaag ctttggagaa tgaccaaaaa gtaaggaaag aaggtggaaa gtttggtttt 960

gcagagtcta tagcaaatca aaggatcaca gcatttaaaa tggaaaaggc ccctattgat 1020

acctcggatg tagaagaaaa agcagaagaa atcattgctg aagcagaacc tccttcagaa 1080

gttgtttcta cacctgtgct atggactcct ggaactgccc aaattggaga gggagtagaa 1140

aactcctggg gtgatcttga agactctgag aaggaagatg atgaaggcgg tggtgatcaa 1200

gctatcattc ttgatggtat aaaaatggac actggagtag aagtctctga tattggaagc 1260

caagagctgg ggtttcacca tgttggccag actggactcg agttcctgac ctcagatgct 1320

cccataatac tctcagatag tgaagaagaa gaaatgatca ttttggaacc agacaagaat 1380

ccaaagaaaa taagaacaca gaccaccagt gcaaaacaag aaaaagcacc aagtaaaaag 1440

ccagtgaaaa gaagaaaaaa gaagagagct gccaattaaa gctaacagtt gtatatctgt 1500

atatataact attaaaaggg atatttattc cattctgaga accctgggta ttttttattc 1560

acaaatccat tataaaatct agcaggattt taaaaatagt tttttgtttt taatgtgctt 1620

taaaataata aaccttctgg agca 1644

<210> 60
<211> 1593
<212> DNA
<213> Homo sapiens

<400> 60
gatgacgtaa ttttcctgcg cctcggggcg agcagcggcg cgcaaggaaa gatcgggttc 60

cgtttttccc gcggattctg gtgcctgtgg ggccggtgac ccaacaccat gaaggaaacg 120

ccactctcaa actgcgaacg ccgcttccta ctccgtgcca tcgaagagaa gaagcggctg 180

gatggcagac aaacctatga ttataggaac atcaggatct catttggaac agattacgga 240

tgctgcattg tggaacttgg aaaaacaaga gttcttggac aggtttcctg tgaacttgtg 300

tctccaaaac tcaatcgggc aacagaaggt attctttttt ttaaccttga actctctcag 360

atggccgctc cagctttcga acctggcagg cagtcagatc tcttggtgaa gttgaatcga 420

ctcatggaaa gatgtctaag aaattcgaag tgtatagaca ctgagtctct ctgtgttgtt 480

gctggtgaaa aggtttggca aatacgtgta gacctacatt tattaaatca tgatggaaat 540

attattgatg ctgccagcat tgctgcaatc gtggccttat gtcatttccg aagacctgat 600

gtctctgtcc aaggagatga agtaacactg tatacacctg aagagcgtga tcctgtacca 660

ttaagtatcc accacatgcc catttgtgtc agttttgcct tttccagcaa aggaacatat 720

ttattggtgg atcccaatga acgagaagaa cgtgtgatgg atggcttgct ggtgattgcc 780

atgaacaaac atcgagagat ttgtactatc cagtccagtg gtgggataat gctactaaaa 840

gatcaagttc tgagatgcag taaaatcgct ggtgtgaaag tagcagaaat tacagagcta 900

atattgaaag ctttggagaa tgaccaaaaa gtaaggaaag aaggtggaaa gtttggtttt 960

gcagagtcta tagcaaatca aaggatcaca gcatttaaaa tggaaaaggc ccctattgat 1020

acctcggatg tagaagaaaa agcagaagaa atcattgctg aagcagaacc tccttcagaa 1080

gttgtttcta cacctgtgct atggactcct ggaactgccc aaattggaga gggagtagaa 1140

aactcctggg gtgatcttga agactctgag aaggaagatg atgaaggcgg tggtgatcaa 1200

gctatcattc ttgatggtat aaaaatggac actggagtag aagtctctga tattggaagc 1260

caagatgctc ccataatact ctcagatagt gaagaagaag aaatgatcat tttggaacca 1320

gacaagaatc caaagaaaat aagaacacag accaccagtg caaaacaaga aaaagcacca 1380

agtaaaaagc cagtgaaaag aagaaaaaag aagagagctg ccaattaaag ctaacagttg 1440

tatatctgta tatataacta ttaaaaggga tatttattcc attctgagaa ccctgggtat 1500

tttttattca caaatccatt ataaaatcta gcaggatttt aaaaatagtt ttttgttttt 1560

aatgtgcttt aaaataataa accttctgga gca 1593

<210> 61
<211> 2380
<212> DNA
<213> Homo sapiens

<400> 61
ggaaaacttg tctctgcgtt gtggggagga cgcgcgctcg cgcgggattt tcaagcgtag 60

gcccccggga actcgagctg ccatgagcct ctgggtggac aagtatcggc cctgctcctt 120

gggacggctg gactatcaca aggagcaggc ggcccagctg cggaacctgg tgcagtgtgg 180

tgactttcct catctgttag tgtacggacc atcaggtgct ggaaaaaaga caagaattat 240

gtgtattcta cgtgaacttt atggtgttgg agtggaaaaa ttgagaattg aacatcagac 300

catcacaact ccatctaaaa aaaaaattga aattagcacc attgcaagta actaccacct 360

tgaagttaat cctagtgatg ctggaaatag tgaccgagta gtcattcagg agatgttgaa 420

aacagtggca caatcacaac aacttgaaac aaactctcaa agggatttta aagtggtatt 480

attgacagaa gttgacaaac tcaccaaaga tgctcagcat gccttgcgaa gaaccatgga 540

aaaatatatg tctacctgca gattgatctt gtgctgcaat tctacatcta aagtgatccc 600

acctattcgt agtaggtgct tggcggttcg tgtgcctgct cccagcattg aagatatttg 660

ccacgtgtta tctactgtgt gtaagaagga aggtctgaat cttccttcac aactggctca 720

tagacttgca gagaagtctt gtagaaatct cagaaaagcc ctgcttatgt gtgaagcctg 780

cagagtgcaa caatatcctt ttactgcaga tcaagaaatc cctgagacag attgggaggt 840

gtatctgagg gagactgcaa atgctattgt cagtcagcaa actccacaaa ggctccttga 900

agttcgtgga aggctgtatg agcttctaac tcattgtatt cctcctgaga taataatgaa 960

gggccttctc tcagaactgt tacataattg tgatggacaa ctgaaagggg aggtggcaca 1020

aatggcagct tactatgagc atcgtctaca gctgggtagc aaagccattt atcacttgga 1080

agcgtttgtg gccaaattca tggcacttta taagaagttc atggaggatg gattggaagg 1140

catgatgttc tgacttctgt cagttattct tgcaaagatt tctcagtatc agtatttaca 1200

tacagcttat attaaaagag ctgtgggtaa attaactgaa cttaatcatg tcgtatttgc 1260

gtttttttgg taataacttc tctgtgaact attaatcatc ctctgagtta aataattgct 1320

cctatactat tgaagtatgt agttttgtac ataacttaga gactttagag tctaagaaaa 1380

tgatcttaat ttactttaag cattggttat tcaagtattc attgttgatc ctcctattct 1440

cttccgtcta atctctcacc tgctaaagga gatttacaca ttagaaagca aagattattt 1500

tcatttatcc agatgaccat tttctgccac aggtaacatg attgtttgac acaccattat 1560

atttaattct agtttctctc aatgaataat tgtatttttg taggaaatgt aagatttcat 1620

tctgaaacat aattattggt atggacaaaa ttgcagatac catttctgtt gaggctgcag 1680

atttccaact tttatttcag tggttcagat tagtattagg tcggtactaa gaaataagca 1740

tgttttcact aatttaagta cttgagactc ttgaagaaaa ttcagaatga agttctggag 1800

aaaggtatgt tactgtagta attactcttt gaacaggttt tgtgttttgt cattagctct 1860

gcccttttta attaaatatt ttggttcatg gaccaaaggg tttacttgac aaatttgtgt 1920

gacagactcc gaacaattcc tttactacga agtataattt ataaaataaa atatacccat 1980

tttaagggta cagtttgatt tttgaccagt gaaactatga tcccaatcaa ggtatagatg 2040

ccgtcacccc aaaaagttcc ctccatatcc ctttgcagtc agttcatccc taccctggcc 2100

cagatgatca ctgatcttgt cattatagat gagttttgcc agttcaagaa tttaatggaa 2160

tcagatattg taagcattct tgtgtaatac ttcattctct ctcattattg agattcatcc 2220

atattgttga atgtttcact agttaatgtt tattgttcaa tatttttgta tatactttta 2280

aagcctattc acttgctgat ggatctttgg tttgtttcca ggtttggtta ttatgaataa 2340

agttgctgtg aatacttacg tgtaaaaaaa aaaaaaaaaa 2380

<210> 62
<211> 1403
<212> DNA
<213> Homo sapiens

<400> 62
ggaaaacttg tctctgcgtt gtggggagga cgcgcgctcg cgcgggattt tcaagcgtag 60

gcccccggga actcgagctg ccatgagcct ctgggtggac aagtatcggc cctgctcctt 120

gggacggctg gactatcaca aggagcaggc ggcccagctg cggaacctgg tgcagtgtgg 180

tgactttcct catctgttag tgtacggacc atcaggtgct ggaaaaaaga caagaattat 240

gtgtattcta cgtgaacttt atggtgttgg agtggaaaaa ttgagaattg aacatcagac 300

catcacaact ccatctaaaa aaaaaattga aattagcacc attgcaagta actaccacct 360

tgaagttaat cctagtgatg ctggaaatag tgaccgagta gtcattcagg agatgttgaa 420

aacagtggca caatcacaac aacttgaaac aaactctcaa agggatttta aagtggtatt 480

attgacagaa gttgacaaac tcaccaaaga tgctcagcat gccttgcgaa gaaccatgga 540

aaaatatatg tctacctgca gattgatctt gtgctgcaat tctacatcta aagtgatccc 600

acctattcgt agtaggtgct tggcggttcg tgtgcctgct cccagcattg aagatggaag 660

gtctgaatct tccttcacaa ctggctcata gacttgcaga gaagtcttgt agaaatctca 720

gaaaagccct gcttatgtgt gaagcctgca gagtgcaaca atatcctttt actgcagatc 780

aagaaatccc tgagacagat tgggaggtgt atctgaggga gactgcaaat gctattgtca 840

gtcagcaaac tccacaaagg ctccttgaag ttcgtggaag gctgtatgag cttctaactc 900

attgtattcc tcctgagata ataatgaagg catgtaagga ggaatcaaga agctgtgaca 960

tattctgagg acctccaaag aacaacttgc tactgactgg acttctctca tcttttagca 1020

tgtgaagttt tgcaaatata atccactttg ctctgaatgt cctgacacct gcttcagagc 1080

tcaagattca gactccagcc tgaatgttat ttcctgagaa aatccctccc tgacccactg 1140

tgctggttag gtccacctgc taatcacatc cacaaaaccc tctgcttctc cttcaaaaca 1200

ctcatctcct ctgtaatgat cagatgaact catgtctttc tcagaggaca ataaatttga 1260

tgaatgggga ccatggaagt ttatttccat ctgtatccct acatctaact catctgctgg 1320

tacctatttg tcatgtattt aataagtaca actacctatg ccaccctgca ataaaatgat 1380

attgttactt tctcttcttt aaa 1403

<210> 63
<211> 962
<212> DNA
<213> Homo sapiens

<400>  63
cttcttttcc gacaaaacac caaatggcgg atgacgccgg tgcagcgggg gggcccgggg      60

gccctggtgg ccctgggatg gggaaccgcg gtggcttccg cggaggtttc ggcagtggca      120

tccggggccg gggtcgcggc cgtggacggg gccggggccg aggccgcgga gctcgcggag      180

gcaaggccga ggataaggag tggatgcccg tcaccaagtt gggccgcttg gtcaaggaca      240

tgaagatcaa gtccctggag gagatctatc tcttctccct gcctattaag gaatcagaga      300

tcattgattt cttcctgggg gcctctctca aggatgaggt tttgaagatt atgccagtgc      360

agaagcagac ccgtgccggc cagcgcacca ggttcaaggc atttgttgct atcggggact      420

acaatggcca cgtcggtctg ggtgttaagt gctccaagga ggtggccacc gccatccgtg      480

gggccatcat cctggccaag ctctccatcg tccccgtgcg cagaggctac tgggggaaca      540

agatcggcaa gccccacact gtcccttgca aggtgacagg ccgctgcggc tctgtgctgg      600

tacgcctcat ccctgcaccc aggggcactg gcatcgtctc cgcacctgtg cctaagaagc      660

tgctcatgat ggctggtatc gatgactgct acacctcagc ccggggctgc actgccaccc      720

tgggcaactt cgccaaggcc acctttgatg ccatttctaa gacctacagc tacctgaccc      780

ccgacctctg gaaggagact gtattcacca agtctcccta tcaggagttc actgaccacc      840

tcgtcaagac ccacaccaga gtctccgtgc agcggactca ggctccagct gtggctacaa      900

catagggttt ttatacaaga aaaataaagt gaattaagcg tgaaaaaaaa aaaaaaaaaa      960

aa                                                                    962

<210>  64
<211>  5681
<212>  DNA
<213>  Homo sapiens

<400>  64
cgccgcggtg ctgaggggga ggggagccgg cgagcgcgcg cgcagcgggg gcgcgggtgg      60

cgcgcgtgtg tgtgaagggg gggcggtggc cgaggcgggc gggcgcgcgc gcgaggcttc      120

ccctcgtttg gcggcggcgg cggcttcttt gtttcgtgaa gagaagcgag acgcccattc      180

tgcccccggc cccgcgcgga ggggcggggg aggcgccggg aagtcgacgg cgccggcggc      240

tcctgcagga ggccactgtc tgcagctccc gtgaagatgt ccactccaga cccacccctg      300

ggcggaactc ctcggccagg tccttccccg ggccctggcc cttcccctgg agccatgctg      360

ggccctagcc cgggtccctc gccgggctcc gcccacagca tgatggggcc cagcccaggg      420

ccgccctcag caggacaccc catccccacc caggggcctg gagggtaccc tcaggacaac      480

atgcaccaga tgcacaagcc catggagtcc atgcatgaga agggcatgtc ggacgacccg      540

cgctacaacc agatgaaagg aatggggatg cggtcagggg gccatgctgg gatggggccc      600

ccgcccagcc ccatggacca gcactcccaa ggttacccct cgcccctggg tggctctgag      660

catgcctcta gtccagttcc agccagtggc ccgtcttcgg ggccccagat gtcttccggg      720

ccaggaggtg ccccgctgga tggtgctgac ccccaggcct tggggcagca gaaccggggc      780

ccaaccccat ttaaccagaa ccagctgcac cagctcagag ctcagatcat ggcctacaag      840

atgctggcca gggggcagcc cctccccgac cacctgcaga tggcggtgca gggcaagcgg 900

ccgatgcccg ggatgcagca gcagatgcca acgctacctc caccctcggt gtccgcaaca 960

ggacccggcc ctggccctgg ccctggcccc ggcccgggtc ccggcccggc acctccaaat 1020

tacagcaggc ctcatggtat gggagggccc aacatgcctc ccccaggacc ctcgggcgtg 1080

ccccccggga tgccaggcca gcctcctgga gggcctccca agccctggcc tgaaggaccc 1140

atggcgaatg ctgctgcccc cacgagcacc cctcagaagc tgattccccc gcagccaacg 1200

ggccgccctt cccccgcgcc ccctgccgtc ccacccgccg cctcgcccgt gatgccaccg 1260

cagacccagt cccccgggca gccggcccag cccgcgccca tggtgccact gcaccagaag 1320

cagagccgca tcacccccat ccagaagccg cggggcctcg accctgtgga gatcctgcag 1380

gagcgcgagt acaggctgca ggctcgcatc gcacaccgaa ttcaggaact tgaaaacctt 1440

cccgggtccc tggccgggga tttgcgaacc aaagcgacca ttgagctcaa ggccctcagg 1500

ctgctgaact tccagaggca gctgcgccag gaggtggtgg tgtgcatgcg gagggacaca 1560

gcgctggaga cagccctcaa tgctaaggcc tacaagcgca gcaagcgcca gtccctgcgc 1620

gaggcccgca tcactgagaa gctggagaag cagcagaaga tcgagcagga gcgcaagcgc 1680

cggcagaagc accaggaata cctcaatagc attctccagc atgccaagga tttcaaggaa 1740

tatcacagat ccgtcacagg caaaatccag aagctgacca aggcagtggc cacgtaccat 1800

gccaacacgg agcgggagca gaagaaagag aacgagcgga tcgagaagga gcgcatgcgg 1860

aggctcatgg ctgaagatga ggaggggtac cgcaagctca tcgaccagaa gaaggacaag 1920

cgcctggcct acctcttgca gcagacagac gagtacgtgg ctaacctcac ggagctggtg 1980

cggcagcaca aggctgccca ggtcgccaag gagaaaaaga agaaaaagaa aaagaagaag 2040

gcagaaaatg cagaaggaca gacgcctgcc attgggccgg atggcgagcc tctggacgag 2100

accagccaga tgagcgacct cccggtgaag gtgatccacg tggagagtgg gaagatcctc 2160

acaggcacag atgcccccaa agccgggcag ctggaggcct ggctcgagat gaacccgggg 2220

tatgaagtag ctccgaggtc tgatagtgaa gaaagtggct cagaagaaga ggaagaggag 2280

gaggaggaag agcagccgca ggcagcacag cctcccaccc tgcccgtgga ggagaagaag 2340

aagattccag atccagacag cgatgacgtc tctgaggtgg acgcgcggca catcattgag 2400

aatgccaagc aagatgtcga tgatgaatat ggcgtgtccc aggcccttgc acgtggcctg 2460

cagtcctact atgccgtggc ccatgctgtc actgagagag tggacaagca gtcagcgctt 2520

atggtcaatg gtgtcctcaa acagtaccag atcaaaggtt tggagtggct ggtgtccctg 2580

tacaacaaca acctgaacgg catcctggcc gacgagatgg gcctggggaa gaccatccag 2640

accatcgcgc tcatcacgta cctcatggag cacaaacgca tcaatgggcc cttcctcatc 2700

atcgtgcctc tctcaacgct gtccaactgg gcgtacgagt ttgacaagtg ggccccctcc 2760

gtggtgaagg tgtcttacaa gggatcccca gcagcaagac gggcctttgt cccccagctc 2820

cggagtggga agttcaacgt cttgctgacg acgtacgagt acatcatcaa agacaagcac 2880

atcctcgcca agatccgttg gaagtacatg attgtggacg aaggtcaccg catgaagaac 2940

caccactgca agctgacgca ggtgctcaac acgcactatg tggcaccccg ccgcctgctg 3000
ctgacgggca caccgctgca gaacaagctt cccgagctct gggcgctgct caacttcctg 3060
ctgcccacca tcttcaagag ctgcagcacc ttcgagcagt ggtttaacgc accctttgcc 3120
atgaccgggg aaaaggtgga cctgaatgag gaggaaacca ttctcatcat ccggcgtctc 3180
cacaaagtgc tgcggccctt cttgctccga cgactcaaga aggaagtcga ggcccagttg 3240
cccgaaaagg tggagtacgt catcaagtgc gacatgtctg cgctgcagcg agtgctctac 3300
cgccacatgc aggccaaggg cgtgctgctg actgatggct ccgagaagga caagaagggc 3360
aaaggcggca ccaagaccct gatgaacacc atcatgcagc tgcggaagat ctgcaaccac 3420
ccctacatgt tccagcacat cgaggagtcc ttttccgagc acttggggtt cactggcggc 3480
attgtccaag ggctggacct gtaccgagcc tcgggtaaat ttgagcttct tgatagaatt 3540
cttcccaaac tccgagcaac caaccacaaa gtgctgctgt tctgccaaat gacctccctc 3600
atgaccatca tggaagatta ctttgcgtat cgcggcttta aatacctcag gcttgatgga 3660
accacgaagg cggaggaccg gggcatgctg ctgaaaacct tcaacgagcc cggctctgag 3720
tacttcatct tcctgctcag cacccgggct gggggctcgg gcctgaacct ccagtcggca 3780
gacactgtga tcatttttga cagcgactgg aatcctcacc aggacctgca agcgcaggac 3840
cgagcccacc gcatcgggca gcagaacgag gtgcgtgtgc tccgcctctg caccgtcaac 3900
agcgtggagg agaagatcct agctgcagcc aagtacaagc tcaacgtgga ccagaaggtg 3960
atccaggccg gcatgttcga ccagaagtcc tccagccatg agcggcgcgc cttcctgcag 4020
gccatcctgg agcacgagga gcaggatgag agcagacact gcagcacggg cagcggcagt 4080
gccagcttcg cccacactgc ccctccgcca gcgggcgtca accccgactt ggaggagcca 4140
cctctaaagg aggaagacga ggtgcccgac gacgagaccg tcaaccagat gatcgcccgg 4200
cacgaggagg agtttgatct gttcatgcgc atggacctgg accgcaggcg cgaggaggcc 4260
cgcaacccca agcggaagcc gcgcctcatg gaggaggacg agctcccctc gtggatcatc 4320
aaggacgacg cggaggtgga gcggctgacc tgtgaggagg aggaggagaa gatgttcggc 4380
cgtggctccc gccaccgcaa ggaggtggac tacagcgact cactgacgga gaagcagtgg 4440
ctcaaggcca tcgaggaggg cacgctggag gagatcgaag aggaggtccg gcagaagaaa 4500
tcatcacgga agcgcaagcg agacagcgac gccggctcct ccaccccgac caccagcacc 4560
cgcagccgcg acaaggacga cgagagcaag aagcagaaga agcgcgggcg gccgcctgcc 4620
gagaaactct cccctaaccc acccaacctc accaagaaga tgaagaagat tgtggatgcc 4680
gtgatcaagt acaaggacag cagcagtgga cgtcagctca gcgaggtctt catccagctg 4740
ccctcgcgaa aggagctgcc cgagtactac gagctcatcc gcaagcccgt ggacttcaag 4800
aagataaagg agcgcattcg caaccacaag taccgcagcc tcaacgacct agagaaggac 4860
gtcatgctcc tgtgccagaa cgcacagacc ttcaacctgg agggctccct gatctatgaa 4920
gactccatcg tcttgcagtc ggtcttcacc agcgtgcggc agaaaatcga gaaggaggat 4980
gacagtgaag gcgaggagag tgaggaggag gaagagggcg aggaggaagg ctccgaatcc 5040

gaatctcggt ccgtcaaagt gaagatcaag cttggccgga aggagaaggc acaggaccgg 5100

ctgaagggcg gccggcggcg gccgagccga gggtcccgag ccaagccggt cgtgagtgac 5160

gatgacagtg aggaggaaca agaggaggac cgctcaggaa gtggcagcga agaagactga 5220

gccccgacat tccagtctcg accccgagcc cctcgttcca gagctgagat ggcataggcc 5280

ttagcagtaa cgggtagcag cagatgtagt ttcagacttg gagtaaaact gtataaacaa 5340

aagaatcttc catatttata cagcagagaa gctgtaggac tgtttgtgac tggccctgtc 5400

ctggcatcag tagcatctgt aacagcatta actgtcttaa agagagagag agagaattcc 5460

gaattgggga acacacgata cctgtttttc ttttccgttg ctggcagtac tgttgcgccg 5520

cagtttggag tcactgtagt taagtgtgga tgcatgtgcg tcaccgtcca ctcctcctac 5580

tgtattttat tggacaggtc agactcgccg ggggcccggc gagggtatgt cagtgtcact 5640

ggatgtcaaa cagtaataaa ttaaaccaac aacaaaacgc a 5681

<210> 65
<211> 1153
<212> DNA
<213> Homo sapiens

<400> 65
aactccaggg ctagtgagct ggaccggaag taggtttcta cccgaccgca ttttacgtgg 60

tgctgcattt ccggtagcgg cggcgggaaa tcggctgtgg gagagaggct aggcctctga 120

ggaggcgaat ccggcgggta tcagagccat cagaaccgcc accatgacgg tgggcaagag 180

cagcaagatg ctgcagcata ttgattacag gatgaggtgc atcctgcagg acggccggat 240

cttcattggc accttcaagg cttttgacaa gcacatgaat ttgatcctct gtgactgtga 300

tgagttcaga aagatcaagc caaagaactc caaacaagca gaaagggaag agaagcgagt 360

cctcggtctg gtgctgctgc gaggggagaa tctggtctca atgacagtag agggacctcc 420

tcccaaagat actggtattg ctcgagttcc acttgctgga gctgccgggg gcccagggat 480

cggcagggct gctggcagag gaatcccagc tggggttccc atgccccagg ctcctgcagg 540

acttgctggg ccagtccgtg gggttggcgg gccatcccaa caggtgatga ccccacaagg 600

aagaggtact gttgcagccg ctgcagctgc tgccacagcc agtattgccg gggctccaac 660

ccagtaccca cctggccgtg ggggtcctcc cccacctatg ggccgaggag caccccctcc 720

aggcatgatg ggcccacctc ctggtatgag acctcctatg ggtcccccaa tggggatccc 780

ccctggaaga gggactccaa tgggcatgcc ccctccggga atgcggcctc ctccccctgg 840

gatgcgaggc cttctttgac ccttggccac agagtatgga agtagctccg cagaggcgtg 900

ggctcgattc ctcagggcca cgttaccaca gacctgtttg tttcttatgc tgttgttcgt 960

ggagtctcat gggattgtct ggtttccctt acagggcccc ctcccccggg aatgcgccca 1020

ccaaggccct agactcatct tggccctcct cagctccctg cctgtttccc gtaaggctgt 1080

acatagtcct tttatctcct tgtggcctat gaaactggtt tataataaac tcttaagaga 1140

acattataat tgc 1153

<210> 66
<211> 1007
<212> DNA
<213> Homo sapiens

<400> 66
aactccaggg ctagtgagct ggaccggaag taggtttcta cccgaccgca ttttacgtgg 60

tgctgcattt ccggtagcgg cggcgggaaa tcggctgtgg gagagaggct aggcctctga 120

ggaggcgaat ccggcgggta tcagagccat cagaaccgcc accatgacgg tgggcaagag 180

cagcaagatg ctgcagcata ttgattacag gatgaggtgc atcctgcagg acggccggat 240

cttcattggc accttcaagg cttttgacaa gcacatgaat ttgatcctct gtgactgtga 300

tgagttcaga aagatcaagc caaagaactc caaacaagca gaaagggaag agaagcgagt 360

cctcggtctg gtgctgctgc gaggggagaa tctggtctca atgacagtag agggacctcc 420

tcccaaagat actggtattg ctcgagttcc acttgctgga gctgccgggg gcccagggat 480

cggcagggct gctggcagag gaatcccagc tggggttccc atgccccagg ctcctgcagg 540

acttgctggg ccagtccgtg gggttggcgg gccatcccaa caggtgatga ccccacaagg 600

aagaggtact gttgcagccg ctgcagctgc tgccacagcc agtattgccg gggctccaac 660

ccagtaccca cctggccgtg ggggtcctcc cccacctatg ggccgaggag caccccctcc 720

aggcatgatg ggcccacctc ctggtatgag acctcctatg ggtcccccaa tggggatccc 780

ccctggaaga gggactccaa tgggcatgcc ccctccggga atgcggcctc ctccccctgg 840

gatgcgaggg ccccctcccc cgggaatgcg cccaccaagg ccctagactc atcttggccc 900

tcctcagctc cctgcctgtt tcccgtaagg ctgtacatag tccttttatc tccttgtggc 960

ctatgaaact ggtttataat aaactcttaa gagaacatta taattgc 1007

<210> 67
<211> 2346
<212> DNA
<213> Homo sapiens

<400> 67
   acaaggcagc ctcgctcgag cgcaggccaa tcggctttct agctagaggg tttaactcct 60
   atttaaaaag aagaaccttt gaattctaac ggctgagctc ttggaagact tgggtccttg 120
   ggtcgcaggt gggagccgac gggtgggtag accgtggggg atatctcagt ggcggacgag 180
   gacggcgggg acaaggggcg gctggtcgga gtggcggagc gtcaagtccc ctgtcggttc 240
   ctccgtccct gagtgtcctt ggcgctgcct tgtgcccgcc cagcgccttt gcatccgctc 300
   ctgggcaccg aggcgccctg taggatactg cttgttactt attacagcta gaggcatcat 360
   ggaccgatct aaagaaaact gcatttcagg acctgttaag gctacagctc cagttggagg 420
   tccaaaacgt gttctcgtga ctcagcaatt tccttgtcag aatccattac ctgtaaatag 480
   tggccaggct cagcgggtct tgtgtccttc aaattcttcc cagcgcattc ctttgcaagc 540
   acaaaagctt gtctccagtc acaagccggt tcagaatcag aagcagaagc aattgcaggc 600
   aaccagtgta cctcatcctg tctccaggcc actgaataac acccaaaaga gcaagcagcc 660
   cctgccatcg gcacctgaaa ataatcctga ggaggaactg gcatcaaaac agaaaaatga 720
   agaatcaaaa aagaggcagt gggctttgga agactttgaa attggtcgcc ctctgggtaa 780
   aggaaagttt ggtaatgttt atttggcaag agaaaagcaa agcaagttta ttctggctct 840
   taaagtgtta tttaaagctc agctggagaa agccggagtg gagcatcagc tcagaagaga 900
   agtagaaata cagtcccacc ttcggcatcc taatattctt agactgtatg gttatttcca 960
   tgatgctacc agagtctacc taattctgga atatgcacca cttggaacag tttatagaga 1020
   acttcagaaa ctttcaaagt ttgatgagca gagaactgct acttatataa cagaattggc 1080
   aaatgccctg tcttactgtc attcgaagag agttattcat agagacatta agccagagaa 1140
   cttacttctt ggatcagctg gagagcttaa aattgcagat tttgggtggt cagtacatgc 1200
   tccatcttcc aggaggacca ctctctgtgg caccctggac tacctgcccc ctgaaatgat 1260
   tgaaggtcgg atgcatgatg agaaggtgga tctctggagc cttggagttc tttgctatga 1320
   atttttagtt gggaagcctc cttttgaggc aaacacatac caagagacct acaaaagaat 1380
   atcacgggtt gaattcacat tccctgactt tgtaacagag ggagccaggg acctcatttc 1440
   aagactgttg aagcataatc ccagccagag gccaatgctc agagaagtac ttgaacaccc 1500
   ctggatcaca gcaaattcat caaaaccatc aaattgccaa aacaaagaat cagctagcaa 1560
   acagtcttag gaatcgtgca gggggagaaa tccttgagcc agggctgcca tataacctga 1620
   caggaacatg ctactgaagt ttattttacc attgactgct gccctcaatc tagaacgcta 1680
   cacaagaaat atttgtttta ctcagcaggt gtgccttaac ctccctattc agaaagctcc 1740
   acatcaataa acatgacact ctgaagtgaa agtagccacg agaattgtgc tacttatact 1800
   ggttcataat ctggaggcaa ggttcgactg cagccgcccc gtcagcctgt gctaggcatg 1860
   gtgtcttcac aggaggcaaa tccagagcct ggctgtgggg aaagtgacca ctctgccctg 1920
   accccgatca gttaaggagc tgtgcaataa ccttcctagt acctgagtga gtgtgtaact 1980
   tattgggttg gcgaagcctg gtaaagctgt tggaatgagt atgtgattct ttttaagtat 2040
   gaaaataaag atatatgtac agacttgtat tttttctctg gtggcattcc tttaggaatg 2100
   ctgtgtgtct gtccggcacc ccggtaggcc tgattgggtt tctagtcctc cttaaccact 2160
   tatctcccat atgagagtgt gaaaaatagg aacacgtgct ctacctccat ttagggattt 2220
   gcttgggata cagaagaggc catgtgtctc agagctgtta agggcttatt tttttaaaac 2280
   attggagtca tagcatgtgt gtaaacttta aatatgcaaa taaataagta tctatgtcta 2340
   aaaaaa 2346

<210> 68
<211> 2554
<212> DNA
<213> Homo sapiens

<400> 68
acaaggcagc ctcgctcgag cgcaggccaa tcggctttct agctagaggg tttaactcct 60

atttaaaaag aagaaccttt gaattctaac ggctgagctc ttggaagact tgggtccttg 120

ggtcgcaggt gggagccgac gggtgggtag accgtggggg atatctcagt ggcggacgag 180

gacggcgggg acaaggggcg gctggtcgga gtggcggagc gtcaagtccc ctgtcggttc 240
ctccgtccct gagtgtcctt ggcgctgcct tgtgcccgcc cagcgccttt gcatccgctc 300
ctgggcaccg aggcgccctg taggatactg cttgttactt attacagcta gagggtctca 360
ctccattgcc caggccagag tgcggggata tttgataaga aacttcagtg aaggccgggc 420
gcggtggctc atgcccgtaa tcccagcatt ttcggaggcc gaggctggag tgcaatggtg 480
tgatctcagc tcactgcaac ctctgcttcc tgggtttaag tgattctcct gcctcagcct 540
cccgagtagc tgggattaca ggcatcatgg accgatctaa agaaaactgc atttcaggac 600
ctgttaaggc tacagctcca gttggaggtc caaaacgtgt tctcgtgact cagcaatttc 660
cttgtcagaa tccattacct gtaaatagtg gccaggctca gcgggtcttg tgtccttcaa 720
attcttccca gcgcattcct ttgcaagcac aaaagcttgt ctccagtcac aagccggttc 780
agaatcagaa gcagaagcaa ttgcaggcaa ccagtgtacc tcatcctgtc tccaggccac 840
tgaataacac ccaaaagagc aagcagcccc tgccatcggc acctgaaaat aatcctgagg 900
aggaactggc atcaaaacag aaaaatgaag aatcaaaaaa gaggcagtgg gctttggaag 960
actttgaaat tggtcgccct ctgggtaaag gaaagtttgg taatgtttat ttggcaagag 1020
aaaagcaaag caagtttatt ctggctctta aagtgttatt taaagctcag ctggagaaag 1080
ccggagtgga gcatcagctc agaagagaag tagaaataca gtcccacctt cggcatccta 1140
atattcttag actgtatggt tatttccatg atgctaccag agtctaccta attctggaat 1200
atgcaccact tggaacagtt tatagagaac ttcagaaact ttcaaagttt gatgagcaga 1260
gaactgctac ttatataaca gaattggcaa atgccctgtc ttactgtcat tcgaagagag 1320
ttattcatag agacattaag ccagagaact tacttcttgg atcagctgga gagcttaaaa 1380
ttgcagattt tgggtggtca gtacatgctc catcttccag gaggaccact ctctgtggca 1440
ccctggacta cctgccccct gaaatgattg aaggtcggat gcatgatgag aaggtggatc 1500
tctggagcct tggagttctt tgctatgaat ttttagttgg gaagcctcct tttgaggcaa 1560
acacatacca agagacctac aaaagaatat cacgggttga attcacattc cctgactttg 1620
taacagaggg agccagggac ctcatttcaa gactgttgaa gcataatccc agccagaggc 1680
caatgctcag agaagtactt gaacacccct ggatcacagc aaattcatca aaaccatcaa 1740
attgccaaaa caaagaatca gctagcaaac agtcttagga atcgtgcagg gggagaaatc 1800
cttgagccag ggctgccata taacctgaca ggaacatgct actgaagttt attttaccat 1860
tgactgctgc cctcaatcta gaacgctaca caagaaatat ttgttttact cagcaggtgt 1920
gccttaacct ccctattcag aaagctccac atcaataaac atgacactct gaagtgaaag 1980
tagccacgag aattgtgcta cttatactgg ttcataatct ggaggcaagg ttcgactgca 2040
gccgccccgt cagcctgtgc taggcatggt gtcttcacag gaggcaaatc cagagcctgg 2100
ctgtggggaa agtgaccact ctgccctgac cccgatcagt taaggagctg tgcaataacc 2160
ttcctagtac ctgagtgagt gtgtaactta ttgggttggc gaagcctggt aaagctgttg 2220
gaatgagtat gtgattcttt ttaagtatga aaataaagat atatgtacag acttgtattt 2280

tttctctggt ggcattcctt taggaatgct gtgtgtctgt ccggcacccc ggtaggcctg 2340

attgggtttc tagtcctcct taaccactta tctcccatat gagagtgtga aaaataggaa 2400

cacgtgctct acctccattt agggatttgc ttgggataca gaagaggcca tgtgtctcag 2460

agctgttaag ggcttatttt tttaaaacat tggagtcata gcatgtgtgt aaactttaaa 2520

tatgcaaata aataagtatc tatgtctaaa aaaa 2554

<210> 69
<211> 2245
<212> DNA
<213> Homo sapiens

<400> 69
acaaggcagc ctcgctcgag cgcaggccaa tcggctttct agctagaggg tttaactcct 60

atttaaaaag aagaaccttt gaattctaac ggctgagctc ttggaagact tgggtccttg 120

ggtcgcaggt gggagccgac ggggtctcac tccattgccc aggccagagt gcggggatat 180

ttgataagaa acttcagtga aggccgggcg cggtggctca tgcccgtaat cccagcattt 240

tcggaggccg aggcatcatg gaccgatcta aagaaaactg catttcagga cctgttaagg 300

ctacagctcc agttggaggt ccaaaacgtg ttctcgtgac tcagcaattt ccttgtcaga 360

atccattacc tgtaaatagt ggccaggctc agcgggtctt gtgtccttca aattcttccc 420

agcgcattcc tttgcaagca caaaagcttg tctccagtca caagccggtt cagaatcaga 480

agcagaagca attgcaggca accagtgtac ctcatcctgt ctccaggcca ctgaataaca 540

cccaaaagag caagcagccc ctgccatcgg cacctgaaaa taatcctgag gaggaactgg 600

catcaaaaca gaaaaatgaa gaatcaaaaa agaggcagtg ggctttggaa gactttgaaa 660

ttggtcgccc tctgggtaaa ggaaagtttg gtaatgttta tttggcaaga gaaaagcaaa 720

gcaagtttat tctggctctt aaagtgttat ttaaagctca gctggagaaa gccggagtgg 780

agcatcagct cagaagagaa gtagaaatac agtcccacct tcggcatcct aatattctta 840

gactgtatgg ttatttccat gatgctacca gagtctacct aattctggaa tatgcaccac 900

ttggaacagt ttatagagaa cttcagaaac tttcaaagtt tgatgagcag agaactgcta 960

cttatataac agaattggca aatgccctgt cttactgtca ttcgaagaga gttattcata 1020

gagacattaa gccagagaac ttacttcttg gatcagctgg agagcttaaa attgcagatt 1080

ttgggtggtc agtacatgct ccatcttcca ggaggaccac tctctgtggc accctggact 1140

acctgccccc tgaaatgatt gaaggtcgga tgcatgatga gaaggtggat ctctggagcc 1200

ttggagttct ttgctatgaa tttttagttg ggaagcctcc ttttgaggca aacacatacc 1260

aagagaccta caaaagaata tcacgggttg aattcacatt ccctgacttt gtaacagagg 1320

gagccaggga cctcatttca agactgttga agcataatcc cagccagagg ccaatgctca 1380

gagaagtact tgaacacccc tggatcacag caaattcatc aaaaccatca aattgccaaa 1440

acaaagaatc agctagcaaa cagtcttagg aatcgtgcag ggggagaaat ccttgagcca 1500

gggctgccat ataacctgac aggaacatgc tactgaagtt tattttacca ttgactgctg 1560

ccctcaatct agaacgctac acaagaaata tttgttttac tcagcaggtg tgccttaacc 1620

tccctattca gaaagctcca catcaataaa catgacactc tgaagtgaaa gtagccacga 1680

gaattgtgct acttatactg gttcataatc tggaggcaag gttcgactgc agccgccccg 1740

tcagcctgtg ctaggcatgg tgtcttcaca ggaggcaaat ccagagcctg gctgtgggga 1800

aagtgaccac tctgccctga ccccgatcag ttaaggagct gtgcaataac cttcctagta 1860

cctgagtgag tgtgtaactt attgggttgg cgaagcctgg taaagctgtt ggaatgagta 1920

tgtgattctt tttaagtatg aaaataaaga tatatgtaca gacttgtatt ttttctctgg 1980

tggcattcct ttaggaatgc tgtgtgtctg tccggcaccc cggtaggcct gattgggttt 2040

ctagtcctcc ttaaccactt atctcccata tgagagtgtg aaaaatagga acacgtgctc 2100

tacctccatt tagggatttg cttgggatac agaagaggcc atgtgtctca gagctgttaa 2160

gggcttattt ttttaaaaca ttggagtcat agcatgtgtg taaactttaa atatgcaaat 2220

aaataagtat ctatgtctaa aaaaa 2245

<210> 70
<211> 2135
<212> DNA
<213> Homo sapiens

<400> 70
acaaggcagc ctcgctcgag cgcaggccaa tcggctttct agctagaggg tttaactcct 60

atttaaaaag aagaaccttt gaattctaac ggctgagctc ttggaagact tgggtccttg 120

ggtcgcaggt gggagccgac gggcatcatg gaccgatcta aagaaaactg catttcagga 180

cctgttaagg ctacagctcc agttggaggt ccaaaacgtg ttctcgtgac tcagcaattt 240

ccttgtcaga atccattacc tgtaaatagt ggccaggctc agcgggtctt gtgtccttca 300

aattcttccc agcgcattcc tttgcaagca caaaagcttg tctccagtca caagccggtt 360

cagaatcaga agcagaagca attgcaggca accagtgtac ctcatcctgt ctccaggcca 420

ctgaataaca cccaaaagag caagcagccc ctgccatcgg cacctgaaaa taatcctgag 480

gaggaactgg catcaaaaca gaaaaatgaa gaatcaaaaa agaggcagtg ggctttggaa 540

gactttgaaa ttggtcgccc tctgggtaaa ggaaagtttg gtaatgttta tttggcaaga 600

gaaaagcaaa gcaagtttat tctggctctt aaagtgttat ttaaagctca gctggagaaa 660

gccggagtgg agcatcagct cagaagagaa gtagaaatac agtcccacct tcggcatcct 720

aatattctta gactgtatgg ttatttccat gatgctacca gagtctacct aattctggaa 780

tatgcaccac ttggaacagt ttatagagaa cttcagaaac tttcaaagtt tgatgagcag 840

agaactgcta cttatataac agaattggca aatgccctgt cttactgtca ttcgaagaga 900

gttattcata gagacattaa gccagagaac ttacttcttg gatcagctgg agagcttaaa 960

attgcagatt ttgggtggtc agtacatgct ccatcttcca ggaggaccac tctctgtggc 1020

accctggact acctgccccc tgaaatgatt gaaggtcgga tgcatgatga gaaggtggat 1080

ctctggagcc ttggagttct ttgctatgaa tttttagttg ggaagcctcc ttttgaggca 1140

aacacatacc aagagaccta caaaagaata tcacgggttg aattcacatt ccctgacttt 1200

gtaacagagg gagccaggga cctcatttca agactgttga agcataatcc cagccagagg 1260

ccaatgctca gagaagtact tgaacacccc tggatcacag caaattcatc aaaaccatca 1320

aattgccaaa acaaagaatc agctagcaaa cagtcttagg aatcgtgcag ggggagaaat 1380

ccttgagcca gggctgccat ataacctgac aggaacatgc tactgaagtt tattttacca 1440

ttgactgctg ccctcaatct agaacgctac acaagaaata tttgttttac tcagcaggtg 1500

tgccttaacc tccctattca gaaagctcca catcaataaa catgacactc tgaagtgaaa 1560

gtagccacga gaattgtgct acttatactg gttcataatc tggaggcaag gttcgactgc 1620

agccgccccg tcagcctgtg ctaggcatgg tgtcttcaca ggaggcaaat ccagagcctg 1680

gctgtgggga aagtgaccac tctgccctga ccccgatcag ttaaggagct gtgcaataac 1740

cttcctagta cctgagtgag tgtgtaactt attgggttgg cgaagcctgg taaagctgtt 1800

ggaatgagta tgtgattctt tttaagtatg aaaataaaga tatatgtaca gacttgtatt 1860

ttttctctgg tggcattcct ttaggaatgc tgtgtgtctg tccggcaccc cggtaggcct 1920

gattgggttt ctagtcctcc ttaaccactt atctcccata tgagagtgtg aaaaatagga 1980

acacgtgctc tacctccatt tagggatttg cttgggatac agaagaggcc atgtgtctca 2040

gagctgttaa gggcttattt ttttaaaaca ttggagtcat agcatgtgtg taaactttaa 2100

atatgcaaat aaataagtat ctatgtctaa aaaaa 2135

<210> 71
<211> 2231
<212> DNA
<213> Homo sapiens

<400> 71
acaaggcagc ctcgctcgag cgcaggccaa tcggctttct agctagaggg tttaactcct 60

atttaaaaag aagaaccttt gaattctaac ggctgagctc ttggaagact tgggtccttg 120

ggtcgcaggg tctcactcca ttgcccaggc cagagtgcgg ggatatttga taagaaactt 180

cagtgaaggc cgggcgcggt ggctcatgcc cgtaatccca gcattttcgg aggccgaggc 240

atcatggacc gatctaaaga aaactgcatt tcaggacctg ttaaggctac agctccagtt 300

ggaggtccaa aacgtgttct cgtgactcag caatttcctt gtcagaatcc attacctgta 360

aatagtggcc aggctcagcg ggtcttgtgt ccttcaaatt cttcccagcg cattcctttg 420

caagcacaaa agcttgtctc cagtcacaag ccggttcaga atcagaagca gaagcaattg 480

caggcaacca gtgtacctca tcctgtctcc aggccactga ataacaccca aaagagcaag 540

cagcccctgc catcggcacc tgaaaataat cctgaggagg aactggcatc aaaacagaaa 600

aatgaagaat caaaaaagag gcagtgggct ttggaagact ttgaaattgg tcgccctctg 660

ggtaaaggaa agtttggtaa tgtttatttg gcaagagaaa agcaaagcaa gtttattctg 720

gctcttaaag tgttatttaa agctcagctg gagaaagccg gagtggagca tcagctcaga 780

agagaagtag aaatacagtc ccaccttcgg catcctaata ttcttagact gtatggttat 840

ttccatgatg ctaccagagt ctacctaatt ctggaatatg caccacttgg aacagtttat 900

agagaacttc agaaactttc aaagtttgat gagcagagaa ctgctactta tataacagaa 960

ttggcaaatg ccctgtctta ctgtcattcg aagagagtta ttcatagaga cattaagcca 1020

gagaacttac ttcttggatc agctggagag cttaaaattg cagattttgg gtggtcagta 1080

catgctccat cttccaggag gaccactctc tgtggcaccc tggactacct gccccctgaa 1140

atgattgaag gtcggatgca tgatgagaag gtggatctct ggagccttgg agttctttgc 1200

tatgaatttt tagttgggaa gcctcctttt gaggcaaaca cataccaaga gacctacaaa 1260

agaatatcac gggttgaatt cacattccct gactttgtaa cagagggagc cagggacctc 1320

atttcaagac tgttgaagca taatcccagc cagaggccaa tgctcagaga agtacttgaa 1380

cacccctgga tcacagcaaa ttcatcaaaa ccatcaaatt gccaaaacaa agaatcagct 1440

agcaaacagt cttaggaatc gtgcaggggg agaaatcctt gagccagggc tgccatataa 1500

cctgacagga acatgctact gaagtttatt ttaccattga ctgctgccct caatctagaa 1560

cgctacacaa gaaatatttg ttttactcag caggtgtgcc ttaacctccc tattcagaaa 1620

gctccacatc aataaacatg acactctgaa gtgaaagtag ccacgagaat tgtgctactt 1680

atactggttc ataatctgga ggcaaggttc gactgcagcc gccccgtcag cctgtgctag 1740

gcatggtgtc ttcacaggag gcaaatccag agcctggctg tggggaaagt gaccactctg 1800

ccctgacccc gatcagttaa ggagctgtgc aataaccttc ctagtacctg agtgagtgtg 1860

taacttattg ggttggcgaa gcctggtaaa gctgttggaa tgagtatgtg attcttttta 1920

agtatgaaaa taaagatata tgtacagact tgtatttttt ctctggtggc attcctttag 1980

gaatgctgtg tgtctgtccg gcaccccggt aggcctgatt gggtttctag tcctccttaa 2040

ccacttatct cccatatgag agtgtgaaaa ataggaacac gtgctctacc tccatttagg 2100

gatttgcttg ggatacagaa gaggccatgt gtctcagagc tgttaagggc ttattttttt 2160

aaaacattgg agtcatagca tgtgtgtaaa ctttaaatat gcaaataaat aagtatctat 2220

gtctaaaaaa a 2231

<210> 72
<211> 2121
<212> DNA
<213> Homo sapiens

<400> 72
acaaggcagc ctcgctcgag cgcaggccaa tcggctttct agctagaggg tttaactcct 60

atttaaaaag aagaaccttt gaattctaac ggctgagctc ttggaagact tgggtccttg 120

ggtcgcaggc atcatggacc gatctaaaga aaactgcatt tcaggacctg ttaaggctac 180

agctccagtt ggaggtccaa aacgtgttct cgtgactcag caatttcctt gtcagaatcc 240

attacctgta aatagtggcc aggctcagcg ggtcttgtgt ccttcaaatt cttcccagcg 300

cattcctttg caagcacaaa agcttgtctc cagtcacaag ccggttcaga atcagaagca 360

gaagcaattg caggcaacca gtgtacctca tcctgtctcc aggccactga ataacaccca 420

aaagagcaag cagcccctgc catcggcacc tgaaaataat cctgaggagg aactggcatc 480

aaaacagaaa aatgaagaat caaaaaagag gcagtgggct ttggaagact ttgaaattgg 540

tcgccctctg ggtaaaggaa agtttggtaa tgtttatttg gcaagagaaa agcaaagcaa 600

gtttattctg gctcttaaag tgttatttaa agctcagctg gagaaagccg gagtggagca 660

tcagctcaga agagaagtag aaatacagtc ccaccttcgg catcctaata ttcttagact 720

gtatggttat ttccatgatg ctaccagagt ctacctaatt ctggaatatg caccacttgg 780

aacagtttat agagaacttc agaaactttc aaagtttgat gagcagagaa ctgctactta 840

tataacagaa ttggcaaatg ccctgtctta ctgtcattcg aagagagtta ttcatagaga 900

cattaagcca gagaacttac ttcttggatc agctggagag cttaaaattg cagattttgg 960

gtggtcagta catgctccat cttccaggag gaccactctc tgtggcaccc tggactacct 1020

gccccctgaa atgattgaag gtcggatgca tgatgagaag gtggatctct ggagccttgg 1080

agttctttgc tatgaatttt tagttgggaa gcctcctttt gaggcaaaca cataccaaga 1140

gacctacaaa agaatatcac gggttgaatt cacattccct gactttgtaa cagagggagc 1200

cagggacctc atttcaagac tgttgaagca taatcccagc cagaggccaa tgctcagaga 1260

agtacttgaa cacccctgga tcacagcaaa ttcatcaaaa ccatcaaatt gccaaaacaa 1320

agaatcagct agcaaacagt cttaggaatc gtgcaggggg agaaatcctt gagccagggc 1380

tgccatataa cctgacagga acatgctact gaagtttatt ttaccattga ctgctgccct 1440

caatctagaa cgctacacaa gaaatatttg ttttactcag caggtgtgcc ttaacctccc 1500

tattcagaaa gctccacatc aataaacatg acactctgaa gtgaaagtag ccacgagaat 1560

tgtgctactt atactggttc ataatctgga ggcaaggttc gactgcagcc gccccgtcag 1620

cctgtgctag gcatggtgtc ttcacaggag gcaaatccag agcctggctg tggggaaagt 1680

gaccactctg ccctgacccc gatcagttaa ggagctgtgc aataaccttc ctagtacctg 1740

agtgagtgtg taacttattg ggttggcgaa gcctggtaaa gctgttggaa tgagtatgtg 1800

attcttttta agtatgaaaa taaagatata tgtacagact tgtatttttt ctctggtggc 1860

attcctttag gaatgctgtg tgtctgtccg gcaccccggt aggcctgatt gggtttctag 1920

tcctccttaa ccacttatct cccatatgag agtgtgaaaa ataggaacac gtgctctacc 1980

tccatttagg gatttgcttg ggatacagaa gaggccatgt gtctcagagc tgttaagggc 2040

ttattttttt aaaacattgg agtcatagca tgtgtgtaaa ctttaaatat gcaaataaat 2100

aagtatctat gtctaaaaaa a 2121

<210> 73
<211> 2701
<212> DNA
<213> Homo sapiens

<400> 73
ggttggtgcg agcttccagc ttggccgcag ttggttcgta gttcggctct ggggtctttt 60

gtgtccgggt ctggcttggc tttgtgtccg cgagtttttg ttccgctccg cagcgctctt 120

cccgggcagg agccgtgagg ctcggaggcg gcagcgcggt ccccggccag gagcaagcgc 180

gccggcgtga gcggcggcgg caaaggctgt ggggaggggg cttcgcagat ccccgagatg 240

ccggagttcc tggaagaccc ctcggtcctg acaaaagaca agttgaagag tgagttggtc 300

gccaacaatg tgacgctgcc ggccggggag cagcgcaaag acgtgtacgt ccagctctac 360

ctgcagcacc tcacggctcg caaccggccg ccgctccccg ccggcaccaa cagcaagggg 420

cccccggact tctccagtga cgaagagcgc gagcccaccc cggtcctcgg ctctggggcc 480

gccgccgcgg gccggagccg agcagccgtc ggcaggaaag ccacaaaaaa aactgataaa 540

cccagacaag aagataaaga tgatctagat gtaacagagc tcactaatga agatcttttg 600

gatcagcttg tgaaatacgg agtgaatcct ggtcctattg tgggaacaac caggaagcta 660

tatgagaaaa agcttttgaa actgagggaa caaggaacag aatcaagatc ttctactcct 720

ctgccaacaa tttcttcttc agcagaaaat acaaggcaga atggaagtaa tgattctgac 780

agatacagtg acaatgaaga agactctaaa atagagctca agcttgagaa gagagaacca 840

ctaaagggca gagcaaagac tccagtaaca ctcaagcaaa gaagagttga gcacaatcag 900

agctattctc aagctggaat aactgagact gaatggacaa gtggatcttc aaaaggcgga 960

cctctgcagg cattaactag ggaatctaca agagggtcaa gaagaactcc aaggaaaagg 1020

gtggaaactt cagaacattt tcgtatagat ggtccagtaa tttcagagag tactcccata 1080

gctgaaacta taatggcttc aagcaacgaa tccttagttg tcaatagggt gactggaaat 1140

ttcaagcatg catctcctat tctgccaatc actgaattct cagacatacc cagaagagca 1200

ccaaagaaac cattgacaag agctgaagtg ggagaaaaaa cagaggaaag aagagtagaa 1260

agggatattc ttaaggaaat gttcccctat gaagcatcta caccaacagg aattagtgct 1320

agttgccgca gaccaatcaa aggggctgca ggccggccat tagaactcag tgatttcagg 1380

atggaggagt ctttttcatc taaatatgtt cctaagtatg ttcccttggc agatgtcaag 1440

tcagaaaaga caaaaaaggg acgctccatt cccgtatgga taaaaatttt gctgtttgtt 1500

gttgtggcag tttttttgtt tttggtctat caagctatgg aaaccaacca agtaaatccc 1560

ttctctaatt ttcttcatgt tgaccctaga aaatccaact gaatggtatc tctttggcac 1620

gttcaacttg gtctcctatt ttcaataact gttgaaaaac atttgtgtac acttgttgac 1680

tccaagaact aaaaataatg tgatttcgcc tcaataaatg tagtatttca ttgaaaagca 1740

aacaaaatat atataaatgg acttcattaa aatgtttttg aactttggac tagtaggaga 1800

tcactttgtg ccatatgaat aatctttttt agctctggaa ctttttgtag gctttatttt 1860

tttaatgtgg gcatcttatt tcatttttga aaaaatgtat atgttttttg tgtatttggg 1920

aaacgaaggg tgaaacatgg tagtataatg tgaagctaca catttaaata cttagaattc 1980

ttacagaaaa gattttaaga attattctct gctgaataaa aactgcaaat atgtgaaaca 2040

taatgaaatt cagtaagagg aaaagtaact tggttgtact ttttgtaact gcaacaaagt 2100

ttgatggtgt ttatgaggaa aagtacagca ataatctctt ctgtaacctt tattaatagt 2160

aatgttgttg tagccctatc atactcactt tttaagacac agtatcatga aagtcctatt 2220

tcagtaagac ccatttacat acagtagatt tttagcagag atcttttagt gtaacataca 2280

tattttagag aattgttggc tagctgtaca tgttttgaaa agctgtttag ctagctataa 2340

ggctataatt ggaaatttgt atttttattt tacagcaaaa catttattca gtcatccagt 2400

ttgctaccaa aatatgtttt agataagtgt gtgtatgttt gtttagaagt tagaaattgt 2460

aaacactggt cttatgtttc atttggattc attattgcat tgtcttgtta ccagaaacaa 2520

attttgccga gctttttttg ccctatattt cccagcataa tttgattaga aagtacaaaa 2580

agggccgggc gcggtggctt acgcctgtaa tcccagcact ttgggaggcc agggcgggtg 2640

gatcacgagg tcaggagatc gggaccatcc tggccaacat ggtgaaaccc cgtctctact 2700

a 2701

<210> 74
<211> 2374
<212> DNA
<213> Homo sapiens

<400> 74
ggttggtgcg agcttccagc ttggccgcag ttggttcgta gttcggctct ggggtctttt 60

gtgtccgggt ctggcttggc tttgtgtccg cgagtttttg ttccgctccg cagcgctctt 120

cccgggcagg agccgtgagg ctcggaggcg gcagcgcggt ccccggccag gagcaagcgc 180

gccggcgtga gcggcggcgg caaaggctgt ggggaggggg cttcgcagat ccccgagatg 240

ccggagttcc tggaagaccc ctcggtcctg acaaaagaca agttgaagag tgagttggtc 300

gccaacaatg tgacgctgcc ggccggggag cagcgcaaag acgtgtacgt ccagctctac 360

ctgcagcacc tcacggctcg caaccggccg ccgctccccg ccggcaccaa cagcaagggg 420

cccccggact tctccagtga cgaagagcgc gagcccaccc cggtcctcgg ctctggggcc 480

gccgccgcgg gccggagccg agcagccgtc ggcaggaaag ccacaaaaaa aactgataaa 540

cccagacaag aagataaaga tgatctagat gtaacagagc tcactaatga agatcttttg 600

gatcagcttg tgaaatacgg agtgaatcct ggtcctattg tgggaacaac caggaagcta 660

tatgagaaaa agcttttgaa actgagggaa caaggaacag aatcaagatc ttctactcct 720

ctgccaacaa tttcttcttc agcagaaaat acaaggcaga atggaagtaa tgattctgac 780

agatacagtg acaatgaaga agactctaaa atagagctca agcttgagaa gagagaacca 840

ctaaagggca gagcaaagac tccagtaaca ctcaagcaaa gaagagttga gcacaatcag 900

gtgggagaaa aaacagagga aagaagagta gaaagggata ttcttaagga aatgttcccc 960

tatgaagcat ctacaccaac aggaattagt gctagttgcc gcagaccaat caaaggggct 1020

gcaggccggc cattagaact cagtgatttc aggatggagg agtctttttc atctaaatat 1080

gttcctaagt atgttccctt ggcagatgtc aagtcagaaa agacaaaaaa gggacgctcc 1140

attcccgtat ggataaaaat tttgctgttt gttgttgtgg cagttttttt gtttttggtc 1200

tatcaagcta tggaaaccaa ccaagtaaat cccttctcta attttcttca tgttgaccct 1260

agaaaatcca actgaatggt atctctttgg cacgttcaac ttggtctcct attttcaata 1320

actgttgaaa aacatttgtg tacacttgtt gactccaaga actaaaaata atgtgatttc 1380

gcctcaataa atgtagtatt tcattgaaaa gcaaacaaaa tatatataaa tggacttcat 1440

taaaatgttt ttgaactttg gactagtagg agatcacttt gtgccatatg aataatcttt 1500

tttagctctg gaactttttg taggctttat ttttttaatg tgggcatctt atttcatttt 1560

tgaaaaaatg tatatgtttt ttgtgtattt gggaaacgaa gggtgaaaca tggtagtata 1620

atgtgaagct acacatttaa atacttagaa ttcttacaga aaagatttta agaattattc 1680

tctgctgaat aaaaactgca aatatgtgaa acataatgaa attcagtaag aggaaaagta 1740

acttggttgt actttttgta actgcaacaa agtttgatgg tgtttatgag gaaaagtaca 1800

gcaataatct cttctgtaac ctttattaat agtaatgttg ttgtagccct atcatactca 1860

ctttttaaga cacagtatca tgaaagtcct atttcagtaa gacccattta catacagtag 1920

atttttagca gagatctttt agtgtaacat acatatttta gagaattgtt ggctagctgt 1980

acatgttttg aaaagctgtt tagctagcta taaggctata attggaaatt tgtatttttt 2040

atttacagca aaacatttat tcagtcatcc agtttgctac caaaatatgt tttagataag 2100

tgtgtgtatg tttgtttaga agttagaaat tgtaaacact ggtcttatgt ttcatttgga 2160

ttcattattg cattgtcttg ttaccagaaa caaattttgc cgagcttttt ttgccctata 2220

tttcccagca taatttgatt agaaagtaca aaaagggccg ggcgcggtgg cttacgcctg 2280

taatcccagc actttgggag gccagggcgg gtggatcacg aggtcaggag atcgggacca 2340

tcctggccaa catggtgaaa ccccgtctct acta 2374

<210> 75
<211> 2490
<212> DNA
<213> Homo sapiens

<400> 75
gttcgtagtt cggctctggg gtcttttgtg tccgggtctg gcttggcttt gtgtccgcga 60

gtttttgttc cgctccgcag cgctcttccc gggcaggagc cgtgaggctc ggaggcggca 120

gcgcggtccc cggccaggag caagcgcgcc ggcgtgagcg gcggcggcaa aggctgtggg 180

gaggggcctt cgcagatccc cgagatgccg gagttcctgg aagacccctc ggtcctgaca 240

aaagacaagt tgaagagtga gttggtcgcc aacaatgtga cgctgccggc cggggagcag 300

cgcaaagacg tgtacgtcca gctctacctg cagcacctca cggctcgcaa ccggccgccg 360

ctccccgccg gcaccaacag caaggggccc ccggacttct ccagtgacga agagcgcgag 420

cccaccccgg tcctcggctc tggggccgcc gccgcgggcc ggagccgagc agccgtcggc 480

aggaaagcca caaaaaaaac tgataaaccc agacaagaag ataaagatga tctagatgta 540

acagagctca ctaatgaaga tcttttggat cagcttgtga aatacggagt gaatcctggt 600

cctattgtgg gaacaaccag gaagctatat gagaaaaagc ttttgaaact gagggaacaa 660

ggaacagaat caagatcttc tactcctctg ccaacaattt cttcttcagc agaaaataca 720

aggcagaatg gaagtaatga ttctgacaga tacagtgaca atgaagaagg aaagaagaaa 780

gaacacaaga aagtgaagtc cactagggat attgttcctt tttctgaact tggaactact 840

ccctctggtg gtggattttt tcagggtatt tcttttcctg aaatctccac ccgtcctcct 900

ttgggcagta ccgaactaca ggcagctaag aaagtacata cttctaaggg agacctacct 960

agggagcctc ttgttgccac aaacttgcct ggcaggggac agttgcagaa gttagcctct 1020

gaaaggaatt tgtttatttc atgcaagtct agccatgata ggtgtttaga gaaaagttct 1080

tcgtcatctt ctcagcctga acacagtgcc atgttggtct ctactgcagc ttctccttca 1140

ctgattaaag aaaccaccac tggttactat aaagacatag tagaaaatat ttgcggtaga 1200

gagaaaagtg gaattcaacc attatgtcct gagaggtccc atatttcaga tcaatcgcct 1260

ctctccagta aaaggaaagc actagaagag tctgagagct cacaactaat ttctccgcca 1320

cttgcccagg caatcagaga ttatgtcaat tctctgttgg tccagggtgg ggtaggtagt 1380

ttgcctggaa cttctaactc tatgccccca ctggatgtag aaaacataca gaagagaatt 1440

gatcagtcta agtttcaaga aactgaattc ctgtctcctc caagaaaagt ccctagactg 1500

agtgagaagt cagtggagga aagggattca ggttcctttg tggcatttca gaacatacct 1560

ggatccgaac tgatgtcttc ttttgccaaa actgttgtct ctcattcact cactacctta 1620

ggtctagaag tggctaagca atcacagcat gataaaatag atgcctcaga actatctttt 1680

cccttccatg aatctatttt aaaagtaatt gaagaagaat ggcagcaagt tgacaggcag 1740

ctgccttcac tggcatgcaa atatccagtt tcttccaggg aggcaacaca gatattatca 1800

gttccaaaag tagatgatga aatcctaggg tttatttctg aagccactcc actaggaggt 1860

attcaagcag cctccactga gtcttgcaat cagcagttgg acttagcact ctgtagagca 1920

tatgaagctg cagcatcagc attgcagatt gcaactcaca ctgcctttgt agctaaggct 1980

atgcaggcag acattagtca agctgcacag attcttagct cagatcctag tcgtacccac 2040

caagcgcttg ggattctgag caaaacatat gatgcagcct catatatttg tgaagctgca 2100

tttgatgaag tgaagatggc tgcccatacc atgggaaatg ccactgtagg tcgtcgatac 2160

ctctggctga aggattgcaa aattaattta gcttctaaga ataagctggc ttccactccc 2220

tttaaaggtg gaacattatt tggaggagaa gtatgcaaag taattaaaaa gcgtggaaat 2280

aaacactagt aaaattaagg acaaaaagac atctatctta tctttcaggt actttatgcc 2340

aacattttct tttctgttaa ggttgtttta gtttccagat agggctaatt acaaaatgtt 2400

aagcttctac ccatcaaatt acagtataaa agtaattgcc tgtgtagaac tacttgtctt 2460

ttctaaagat ttgcgtagat aggaagcctg 2490

<210> 76
<211> 5698
<212> DNA
<213> Homo sapiens

<400> 76
aggttcaagt ggagctctcc taaccgacgc gcgtctgtgg agaagcggct tggtcggggg 60

tggtctcgtg gggtcctgcc tgtttagtcg ctttcagggt tcttgagccc cttcacgacc 120

gtcaccatgg aagtgtcacc attgcagcct gtaaatgaaa atatgcaagt caacaaaata 180

aagaaaaatg aagatgctaa gaaaagactg tctgttgaaa gaatctatca aaagaaaaca 240

caattggaac atattttgct ccgcccagac acctacattg gttctgtgga attagtgacc 300

cagcaaatgt gggtttacga tgaagatgtt ggcattaact atagggaagt cacttttgtt 360

cctggtttgt acaaaatctt tgatgagatt ctagttaatg ctgcggacaa caaacaaagg 420

gacccaaaaa tgtcttgtat tagagtcaca attgatccgg aaaacaattt aattagtata 480

tggaataatg gaaaaggtat tcctgttgtt gaacacaaag ttgaaaagat gtatgtccca 540

gctctcatat ttggacagct cctaacttct agtaactatg atgatgatga aaagaaagtg 600

acaggtggtc gaaatggcta tggagccaaa ttgtgtaaca tattcagtac caaatttact 660

gtggaaacag ccagtagaga atacaagaaa atgttcaaac agacatggat ggataatatg 720

ggaagagctg gtgagatgga actcaagccc ttcaatggag aagattatac atgtatcacc 780

tttcagcctg atttgtctaa gtttaaaatg caaagcctgg acaaagatat tgttgcacta 840

atggtcagaa gagcatatga tattgctgga tccaccaaag atgtcaaagt ctttcttaat 900

ggaaataaac tgccagtaaa aggatttcgt agttatgtgg acatgtattt gaaggacaag 960

ttggatgaaa ctggtaactc cttgaaagta atacatgaac aagtaaacca caggtgggaa 1020

gtgtgtttaa ctatgagtga aaaaggcttt cagcaaatta gctttgtcaa cagcattgct 1080

acatccaagg gtggcagaca tgttgattat gtagctgatc agattgtgac taaacttgtt 1140

gatgttgtga agaagaagaa caagggtggt gttgcagtaa aagcacatca ggtgaaaaat 1200

cacatgtgga tttttgtaaa tgccttaatt gaaaacccaa cctttgactc tcagacaaaa 1260

gaaaacatga ctttacaacc caagagcttt ggatcaacat gccaattgag tgaaaaattt 1320

atcaaagctg ccattggctg tggtattgta gaaagcatac taaactgggt gaagtttaag 1380

gcccaagtcc agttaaacaa gaagtgttca gctgtaaaac ataatagaat caagggaatt 1440

cccaaactcg atgatgccaa tgatgcaggg ggccgaaact ccactgagtg tacgcttatc 1500

ctgactgagg gagattcagc caaaactttg gctgtttcag gccttggtgt ggttgggaga 1560

gacaaatatg gggttttccc tcttagagga aaaatactca atgttcgaga agcttctcat 1620

aagcagatca tggaaaatgc tgagattaac aatatcatca agattgtggg tcttcagtac 1680

aagaaaaact atgaagatga agattcattg aagacgcttc gttatgggaa gataatgatt 1740

atgacagatc aggaccaaga tggttcccac atcaaaggct tgctgattaa ttttatccat 1800

cacaactggc cctctcttct gcgacatcgt tttctggagg aatttatcac tcccattgta 1860

aaggtatcta aaaacaagca agaaatggca ttttacagcc ttcctgaatt tgaagagtgg 1920

aagagttcta ctccaaatca taaaaaatgg aaagtcaaat attacaaagg tttgggcacc 1980

agcacatcaa aggaagctaa agaatacttt gcagatatga aaagacatcg tatccagttc 2040

aaatattctg gtcctgaaga tgatgctgct atcagcctgg cctttagcaa aaaacagata 2100

gatgatcgaa aggaatggtt aactaatttc atggaggata gaagacaacg aaagttactt 2160

gggcttcctg aggattactt gtatggacaa actaccacat atctgacata taatgacttc 2220

atcaacaagg aacttatctt gttctcaaat tctgataacg agagatctat cccttctatg 2280

gtggatggtt tgaaaccagg tcagagaaag gttttgttta cttgcttcaa acggaatgac 2340

aagcgagaag taaaggttgc ccaattagct ggatcagtgg ctgaaatgtc ttcttatcat 2400

catggtgaga tgtcactaat gatgaccatt atcaatttgg ctcagaattt tgtgggtagc 2460

aataatctaa acctcttgca gcccattggt cagtttggta ccaggctaca tggtggcaag 2520

gattctgcta gtccacgata catctttaca atgctcagct ctttggctcg attgttattt 2580

ccaccaaaag atgatcacac gttgaagttt ttatatgatg acaaccagcg tgttgagcct 2640

gaatggtaca ttcctattat tcccatggtg ctgataaatg gtgctgaagg aatcggtact 2700

gggtggtcct gcaaaatccc caactttgat gtgcgtgaaa ttgtaaataa catcaggcgt 2760

ttgatggatg gagaagaacc tttgccaatg cttccaagtt acaagaactt caagggtact 2820

attgaagaac tggctccaaa tcaatatgtg attagtggtg aagtagctat tcttaattct 2880

acaaccattg aaatctcaga gcttcccgtc agaacatgga cccagacata caaagaacaa 2940

gttctagaac ccatgttgaa tggcaccgag aagacacctc ctctcataac agactatagg 3000

gaataccata cagataccac tgtgaaattt gttgtgaaga tgactgaaga aaaactggca 3060

gaggcagaga gagttggact acacaaagtc ttcaaactcc aaactagtct cacatgcaac 3120

tctatggtgc tttttgacca cgtaggctgt ttaaagaaat atgacacggt gttggatatt 3180

ctaagagact tttttgaact cagacttaaa tattatggat taagaaaaga atggctccta 3240

ggaatgcttg gtgctgaatc tgctaaactg aataatcagg ctcgctttat cttagagaaa 3300

atagatggca aaataatcat tgaaaataag cctaagaaag aattaattaa agttctgatt 3360

cagaggggat atgattcgga tcctgtgaag gcctggaaag aagcccagca aaaggttcca 3420

gatgaagaag aaaatgaaga gagtgacaac gaaaaggaaa ctgaaaagag tgactccgta 3480

acagattctg gaccaacctt caactatctt cttgatatgc ccctttggta tttaaccaag 3540

gaaaagaaag atgaactctg caggctaaga aatgaaaaag aacaagagct ggacacatta 3600

aaaagaaaga gtccatcaga tttgtggaaa gaagacttgg ctacatttat tgaagaattg 3660

gaggctgttg aagccaagga aaaacaagat gaacaagtcg gacttcctgg gaaagggggg 3720

aaggccaagg ggaaaaaaac acaaatggct gaagttttgc cttctccgcg tggtcaaaga 3780

gtcattccac gaataaccat agaaatgaaa gcagaggcag aaaagaaaaa taaaaagaaa 3840

attaagaatg aaaatactga aggaagccct caagaagatg gtgtggaact agaaggccta 3900

aaacaaagat tagaaaagaa acagaaaaga gaaccaggta caaagacaaa gaaacaaact 3960

acattggcat ttaagccaat caaaaaagga aagaagagaa atccctggtc tgattcagaa 4020

tcagatagga gcagtgacga aagtaatttt gatgtccctc cacgagaaac agagccacgg 4080

agagcagcaa caaaaacaaa attcacaatg gatttggatt cagatgaaga tttctcagat 4140

tttgatgaaa aaactgatga tgaagatttt gtcccatcag atgctagtcc acctaagacc 4200

aaaacttccc caaaacttag taacaaagaa ctgaaaccac agaaaagtgt cgtgtcagac 4260

cttgaagctg atgatgttaa gggcagtgta ccactgtctt caagccctcc tgctacacat 4320

ttcccagatg aaactgaaat tacaaaccca gttcctaaaa agaatgtgac agtgaagaag 4380

acagcagcaa aaagtcagtc ttccacctcc actaccggtg ccaaaaaaag ggctgcccca 4440

aaaggaacta aaagggatcc agctttgaat tctggtgtct ctcaaaagcc tgatcctgcc 4500

aaaaccaaga atcgccgcaa aaggaagcca tccacttctg atgattctga ctctaatttt 4560

gagaaaattg tttcgaaagc agtcacaagc aagaaatcca agggggagag tgatgacttc 4620

catatggact ttgactcagc tgtggctcct cgggcaaaat ctgtacgggc aaagaaacct 4680

ataaagtacc tggaagagtc agatgaagat gatctgtttt aaaatgtgag gcgattattt 4740

taagtaatta tcttaccaag cccaagactg gttttaaagt tacctgaagc tcttaacttc 4800

ctcccctctg aatttagttt ggggaaggtg tttttagtac aagacatcaa agtgaagtaa 4860

agcccaagtg ttctttagct ttttataata ctgtctaaat agtgaccatc tcatgggcat 4920

tgttttcttc tctgctttgt ctgtgttttg agtctgcttt cttttgtctt taaaacctga 4980

tttttaagtt cttctgaact gtagaaatag ctatctgatc acttcagcgt aaagcagtgt 5040

gtttattaac catccactaa gctaaaacta gagcagtttg atttaaaagt gtcactcttc 5100

ctccttttct actttcagta gatatgagat agagcataat tatctgtttt atcttagttt 5160

tatacataat ttaccatcag atagaacttt atggttctag tacagatact ctactacact 5220

cagcctctta tgtgccaagt ttttctttaa gcaatgagaa attgctcatg ttcttcatct 5280

tctcaaatca tcagaggcca aagaaaaaca ctttggctgt gtctataact tgacacagtc 5340

aatagaatga agaaaattag agtagttatg tgattatttc agctcttgac ctgtcccctc 5400

tggctgcctc tgagtctgaa tctcccaaag agagaaacca atttctaaga ggactggatt 5460

gcagaagact cggggacaac atttgatcca agatcttaaa tgttatattg ataaccatgc 5520

tcagcaatga gctattagat tcattttggg aaatctccat aatttcaatt tgtaaacttt 5580

gttaagacct gtctacattg ttatatgtgt gtgacttgag taatgttatc aacgtttttg 5640

taaatattta ctatgttttt ctattagcta aattccaaca attttgtact ttaataaa 5698

<210> 77
<211> 2984
<212> DNA
<213> Homo sapiens

<400> 77
ggaaattcaa acgtgtttgc ggaaaggagt ttgggttcca tcttttcatt tccccagcgc 60

agctttctgt agaaatggaa tccgaggatt taagtggcag agaattgaca attgattcca 120

taatgaacaa agtgagagac attaaaaata agtttaaaaa tgaagacctt actgatgaac 180

taagcttgaa taaaatttct gctgatacta cagataactc gggaactgtt aaccaaatta 240

tgatgatggc aaacaaccca gaggactggt tgagtttgtt gctcaaacta gagaaaaaca 300

gtgttccgct aagtgatgct cttttaaata aattgattgg tcgttacagt caagcaattg 360

aagcgcttcc cccagataaa tatggccaaa atgagagttt tgctagaatt caagtgagat 420

ttgctgaatt aaaagctatt caagagccag atgatgcacg tgactacttt caaatggcca 480

gagcaaactg caagaaattt gcttttgttc atatatcttt tgcacaattt gaactgtcac 540

aaggtaatgt caaaaaaagt aaacaacttc ttcaaaaagc tgtagaacgt ggagcagtac 600

cactagaaat gctggaaatt gccctgcgga atttaaacct ccaaaaaaag cagctgcttt 660

cagaggagga aaagaagaat ttatcagcat ctacggtatt aactgcccaa gaatcatttt 720

ccggttcact tgggcattta cagaatagga acaacagttg tgattccaga ggacagacta 780

ctaaagccag gtttttatat ggagagaaca tgccaccaca agatgcagaa ataggttacc 840

ggaattcatt gagacaaact aacaaaacta aacagtcatg cccatttgga agagtcccag 900

ttaaccttct aaatagccca gattgtgatg tgaagacaga tgattcagtt gtaccttgtt 960

ttatgaaaag acaaacctct agatcagaat gccgagattt ggttgtgcct ggatctaaac 1020

caagtggaaa tgattcctgt gaattaagaa atttaaagtc tgttcaaaat agtcatttca 1080

aggaacctct ggtgtcagat gaaaagagtt ctgaacttat tattactgat tcaataaccc 1140

tgaagaataa aacggaatca agtcttctag ctaaattaga agaaactaaa gagtatcaag 1200

aaccagaggt tccagagagt aaccagaaac agtggcaatc taagagaaag tcagagtgta 1260

ttaaccagaa tcctgctgca tcttcaaatc actggcagat tccggagtta gcccgaaaag 1320

ttaatacaga gcagaaacat accacttttg agcaacctgt cttttcagtt tcaaaacagt 1380

caccaccaat atcaacatct aaatggtttg acccaaaatc tatttgtaag acaccaagca 1440

gcaatacctt ggatgattac atgagctgtt ttagaactcc agttgtaaag aatgactttc 1500

cacctgcttg tcagttgtca acaccttatg gccaacctgc ctgtttccag cagcaacagc 1560

atcaaatact tgccactcca cttcaaaatt tacaggtttt agcatcttct tcagcaaatg 1620

aatgcatttc ggttaaagga agaatttatt ccattttaaa gcagatagga agtggaggtt 1680

caagcaaggt atttcaggtg ttaaatgaaa agaaacagat atatgctata aaatatgtga 1740

acttagaaga agcagataac caaactcttg atagttaccg gaacgaaata gcttatttga 1800

ataaactaca acaacacagt gataagatca tccgacttta tgattatgaa atcacggacc 1860

agtacatcta catggtaatg gagtgtggaa atattgatct taatagttgg cttaaaaaga 1920

aaaaatccat tgatccatgg gaacgcaaga gttactggaa aaatatgtta gaggcagttc 1980

acacaatcca tcaacatggc attgttcaca gtgatcttaa accagctaac tttctgatag 2040

ttgatggaat gctaaagcta attgattttg ggattgcaaa ccaaatgcaa ccagatacaa 2100

caagtgttgt taaagattct caggttggca cagttaatta tatgccacca gaagcaatca 2160

aagatatgtc ttcctccaga gagaatggga aatctaagtc aaagataagc cccaaaagtg 2220

atgtttggtc cttaggatgt attttgtact atatgactta cgggaaaaca ccatttcagc 2280

agataattaa tcagatttct aaattacatg ccataattga tcctaatcat gaaattgaat 2340

ttcccgatat tccagagaaa gatcttcaag atgtgttaaa gtgttgttta aaaagggacc 2400

caaaacagag gatatccatt cctgagctcc tggctcatcc ctatgttcaa attcaaactc 2460

atccagttaa ccaaatggcc aagggaacca ctgaagaaat gaaatatgtt ctgggccaac 2520

ttgttggtct gaattctcct aactccattt tgaaagctgc taaaacttta tatgaacact 2580

atagtggtgg tgaaagtcat aattcttcat cctccaagac ttttgaaaaa aaaaggggaa 2640

aaaaatgatt tgcagttatt cgtaatgtca aataccacct ataaaatata ttggactgtt 2700

atactcttga atccctgtgg aaatctacat ttgaagacaa catcactctg aagtgttatc 2760

agcaaaaaaa attcagtaga ttatctttaa aagaaaactg taaaaatagc aaccacttat 2820

ggtactgtat atattgtaga cttgttttct ctgttttatg ctcttgtgta atctacttga 2880

catcatttta ctcttggaat agtgggtgga tagcaagtat attctaaaaa actttgtaaa 2940

taaagttttg tggctaaaat gacactaaaa aaaaaaaaaa aaaa 2984

<210> 78
<211> 1536
<212> DNA
<213> Homo sapiens

<400> 78
gggggggggg ggaccacttg gcctgcctcc gtcccgccgc gccacttggc ctgcctccgt 60

cccgccgcgc cacttcgcct gcctccgtcc cccgcccgcc gcgccatgcc tgtggccggc 120

tcggagctgc cgcgccggcc cttgcccccc gccgcacagg agcgggacgc cgagccgcgt 180

ccgccgcacg gggagctgca gtacctgggg cagatccaac acatcctccg ctgcggcgtc 240

aggaaggacg accgcacggg caccggcacc ctgtcggtat tcggcatgca ggcgcgctac 300

agcctgagag atgaattccc tctgctgaca accaaacgtg tgttctggaa gggtgttttg 360

gaggagttgc tgtggtttat caagggatcc acaaatgcta aagagctgtc ttccaaggga 420

gtgaaaatct gggatgccaa tggatcccga gactttttgg acagcctggg attctccacc 480

agagaagaag gggacttggg cccagtttat ggcttccagt ggaggcattt tggggcagaa 540

tacagagata tggaatcaga ttattcagga cagggagttg accaactgca aagagtgatt 600

gacaccatca aaaccaaccc tgacgacaga agaatcatca tgtgcgcttg gaatccaaga 660

gatcttcctc tgatggcgct gcctccatgc catgccctct gccagttcta tgtggtgaac 720

agtgagctgt cctgccagct gtaccagaga tcgggagaca tgggcctcgg tgtgcctttc 780

aacatcgcca gctacgccct gctcacgtac atgattgcgc acatcacggg cctgaagcca 840

ggtgacttta tacacacttt gggagatgca catatttacc tgaatcacat cgagccactg 900

aaaattcagc ttcagcgaga acccagacct ttcccaaagc tcaggattct tcgaaaagtt 960

gagaaaattg atgacttcaa agctgaagac tttcagattg aagggtacaa tccgcatcca 1020

actattaaaa tggaaatggc tgtttagggt gctttcaaag gagcttgaag gatattgtca 1080

gtctttaggg gttgggctgg atgccgaggt aaaagttctt tttgctctaa aagaaaaagg 1140

aactaggtca aaaatctgtc cgtgacctat cagttattaa tttttaagga tgttgccact 1200

ggcaaatgta actgtgccag ttctttccat aataaaaggc tttgagttaa ctcactgagg 1260

gtatctgaca atgctgaggt tatgaacaaa gtgaggagaa tgaaatgtat gtgctcttag 1320

caaaaacatg tatgtgcatt tcaatcccac gtacttataa agaaggttgg tgaatttcac 1380

aagctatttt tggaatattt ttagaatatt ttaagaattt cacaagctat tccctcaaat 1440

ctgagggagc tgagtaacac catcgatcat gatgtagagt gtggttatga actttatagt 1500

tgttttatat gttgctataa taaagaagtg ttctgc 1536

<210> 79
<211> 2081
<212> DNA
<213> Homo sapiens

<400> 79
tcgggaagcc atagggcgcc tcccagcccg tctccccgct ccagtttaga acctaattcc 60

caattcccgg accgggccca gccctgggct cttactgtcc gcttttgctg ggacctgttc 120

cacaaatggg cgtcttctgc cttgggccgt gggggttggg ccggaagctg cggacgcctg 180

ggaaggggcc gctgcagctc ttgagccgcc tctgcgggga ccacttgcag gccatcccag 240

ccaagaaggc cccggctggg caggaggagc ctgggacgcc gccctcctcg ccgctgagtg 300

ccgagcagtt ggaccggatc cagaggaaca aggccgcggc cctgctcaga ctcgcggccc 360

gcaacgtgcc cgtgggcttt ggagagagct ggaagaagca cctcagcggg gagttcggga 420

aaccgtattt tatcaagcta atgggatttg ttgcagaaga aagaaagcat tacactgttt 480

atccaccccc acaccaagtc ttcacctgga cccagatgtg tgacataaaa gatgtgaagg 540

ttgtcatcct gggacaggat ccatatcatg gacctaatca agctcacggg ctctgcttta 600

gtgttcaaag gcctgttccg cctccgccca gtttggagaa catttataaa gagttgtcta 660

cagacataga ggattttgtt catcctggcc atggagattt atctgggtgg gccaagcaag 720

gtgttctcct tctcaacgct gtcctcacgg ttcgtgccca tcaagccaac tctcataagg 780

agcgaggctg ggagcagttc actgatgcag ttgtgtcctg gctaaatcag aactcgaatg 840

gccttgtttt cttgctctgg ggctcttatg ctcagaagaa gggcagtgcc attgatagga 900

agcggcacca tgtactacag acggctcatc cctccccttt gtcagtgtat agagggttct 960

ttggatgtag acacttttca aagaccaatg agctgctgca gaagtctggc aagaagccca 1020

ttgactggaa ggagctgtga tcatcagctg aggggtggcc tttgagaagc tgctgttaac 1080

gtatttgcca gttacgaagt tccactgaaa attttcctat taattcttaa gtactctgca 1140

taagggggaa aagcttccag aaagcagcca tgaaccaggc tgtccaggaa tggcagctgt 1200

atccaaccac aaacaacaaa ggctaccctt tgaccaaatg tctttctctg caacatggct 1260

tcggcctaaa atatgcagaa gacagatgag gtcaaatact cagttggctc tctttatctc 1320

ccttgccttt atggtgaaac aggggagatg tgcacctttc aggcacagcc ctagtttggc 1380

gcctgctgct ccttggtttt gcctggttag actttcagtg acagatgttg ggtgtttttt 1440

gcttagaaag gtccccttgt ctcagccttg cagggcaggc atgccagtct ctgccagttc 1500

cactgccccc ttgatctttg aaggagtcct caggcccctc gcagcataag gatgttttgc 1560

aactttccag aatctggccc agaaattagg gctcaatttc ctgattgtag tagaggttaa 1620

gattgctgtg agctttatca gataagagac cgagagaagt aagctgggtc ttgttattcc 1680

ttgggtgttg gtggaataag cagtggaatt tgaacaagga agaggagaaa agggaatttt 1740

gtctttatgg ggtggggtga ttttctccta gggttatgtc cagttggggt ttttaaggca 1800

gcacagactg ccaagtactg ttttttttaa ccgactgaaa tcactttggg atattttttc 1860

ctgcaacact ggaaagtttt agttttttaa gaagtactca tgcagatata tatatatata 1920

tttttcccag tccttttttt aagagacggt ctttattggg tctgcacctc catccttgat 1980

cttgttagca atgctgtttt tgctgttagt cgggttagag ttggctctac gcgaggtttg 2040

ttaataaaag tttgttaaaa gtttaaaaaa aaaaaaaaaa a 2081

<210> 80
<211> 2053
<212> DNA
<213> Homo sapiens

<400> 80
cacagccaca gccagggcta gcctcgccgg ttcccgggtg gcgcgcgttc gctgcctcct 60

cagctccagg atgatcggcc agaagacgct ctactccttt ttctccccca gccccgccag 120

gaagcgacac gcccccagcc ccgagccggc cgtccagggg accggcgtgg ctggggtgcc 180

tgaggaaagc ggagatgcgg cggccatccc agccaagaag gccccggctg ggcaggagga 240

gcctgggacg ccgccctcct cgccgctgag tgccgagcag ttggaccgga tccagaggaa 300

caaggccgcg gccctgctca gactcgcggc ccgcaacgtg cccgtgggct ttggagagag 360

ctggaagaag cacctcagcg gggagttcgg gaaaccgtat tttatcaagc taatgggatt 420

tgttgcagaa gaaagaaagc attacactgt ttatccaccc ccacaccaag tcttcacctg 480

gacccagatg tgtgacataa aagatgtgaa ggttgtcatc ctgggacagg atccatatca 540

tggacctaat caagctcacg ggctctgctt tagtgttcaa aggcctgttc cgcctccgcc 600

cagtttggag aacatttata aagagttgtc tacagacata gaggattttg ttcatcctgg 660

ccatggagat ttatctgggt gggccaagca aggtgttctc cttctcaacg ctgtcctcac 720

ggttcgtgcc catcaagcca actctcataa ggagcgaggc tgggagcagt tcactgatgc 780

agttgtgtcc tggctaaatc agaactcgaa tggccttgtt ttcttgctct ggggctctta 840

tgctcagaag aagggcagtg ccattgatag gaagcggcac catgtactac agacggctca 900

tccctcccct ttgtcagtgt atagagggtt ctttggatgt agacactttt caaagaccaa 960

tgagctgctg cagaagtctg gcaagaagcc cattgactgg aaggagctgt gatcatcagc 1020

tgaggggtgg cctttgagaa gctgctgtta acgtatttgc cagttacgaa gttccactga 1080

aaattttcct attaattctt aagtactctg cataaggggg aaaagcttcc agaaagcagc 1140

catgaaccag gctgtccagg aatggcagct gtatccaacc acaaacaaca aaggctaccc 1200

tttgaccaaa tgtctttctc tgcaacatgg cttcggccta aaatatgcag aagacagatg 1260

aggtcaaata ctcagttggc tctctttatc tcccttgcct ttatggtgaa acaggggaga 1320

tgtgcacctt tcaggcacag ccctagtttg gcgcctgctg ctccttggtt ttgcctggtt 1380

agactttcag tgacagatgt tggggtgttt ttgcttagaa aggtcccctt gtctcagcct 1440

tgcagggcag gcatgccagt ctctgccagt tccactgccc ccttgatctt tgaaggagtc 1500

ctcaggcccc tcgcagcata aggatgtttt gcaactttcc agaatctggc ccagaaatta 1560

gggctcaatt tcctgattgt agtagaggtt aagattgctg tgagctttat cagataagag 1620

accgagagaa gtaagctggg tcttgttatt ccttgggtgt tggtggaata agcagtggaa 1680

tttgaacaag gaagaggaga aaagggaatt ttgtctttat ggggtggggt gattttctcc 1740

tagggttatg tccagttggg gtttttaagg cagcacagac tgccaagtac tgtttttttt 1800

aaccgactga aatcactttg ggatattttt tcctgcaaca ctggaaagtt ttagtttttt 1860

aagaagtact catgcagata tatatatata tatttttccc agtccttttt ttaagagacg 1920

gtctttattg ggtctgcacc tccatccttg atcttgttag caatgctgtt tttgctgtta 1980

gtcgggttag agttggctct acgcgaggtt tgttaataaa agtttgttaa aagtttaaaa 2040

aaaaaaaaaa aaa 2053

<210> 81
<211> 1720
<212> DNA
<213> Homo sapiens

<400> 81
actgcagggt gcgaaggggc cggcgccgct gccgagttac gagtcggcga aagcggcggg 60

aagttcgtac tgggcagaac gcgacgggtc tgcggcttag gtgaaaatgc ctcgtgtaaa 120

agcagctcaa gctggaagac agagctctgc aaagagacat cttgcagaac aatttgcagt 180

tggagagata ataactgaca tggcaaaaaa ggaatggaaa gtaggattac ccattggcca 240

aggaggcttt ggctgtatat atcttgctga tatgaattct tcagagtcag ttggcagtga 300

tgcaccttgt gttgtaaaag tggaacccag tgacaatgga cctcttttta ctgaattaaa 360

gttctaccaa cgagctgcaa aaccagagca aattcagaaa tggattcgta cccgtaagct 420

gaagtacctg ggtgttccta agtattgggg gtctggtcta catgacaaaa atggaaaaag 480

ttacaggttt atgataatgg atcgctttgg gagtgacctt cagaaaatat atgaagcaaa 540

tgccaaaagg ttttctcgga aaactgtctt gcagctaagc ttaagaattc tggatattct 600

ggaatatatt cacgagcatg agtatgtgca tggagatatc aaggcctcaa atcttcttct 660

gaactacaag aatcctgacc aggtgtactt ggtagattat ggccttgctt atcggtactg 720

cccagaagga gttcataaag aatacaaaga agaccccaaa agatgtcacg atggcactat 780

tgaattcacg agcatcgatg cacacaatgg cgtggcccca tcaagacgtg gtgatttgga 840

aatacttggt tattgcatga tccaatggct tactggccat cttccttggg aggataattt 900

gaaagatcct aaatatgtta gagattccaa aattagatac agagaaaata ttgcaagttt 960

gatggacaaa tgttttcctg agaaaaacaa accaggtgaa attgccaaat acatggaaac 1020

agtgaaatta ctagactaca ctgaaaaacc tctttatgaa aatttacgtg acattctttt 1080

gcaaggacta aaagctatag gaagtaagga tgatggcaaa ttggacctca gtgttgtgga 1140

gaatggaggt ttgaaagcaa aaacaataac aaagaagcga aagaaagaaa ttgaagaaag 1200

caaggaacct ggtgttgaag atacggaatg gtcaaacaca cagacagagg aggccataca 1260

gacccgttca agaaccagaa agagagtcca gaagtaattc agatgctgtg aaccagattt 1320

ccttttcttt gttttctttt gacttttttc tccttttcta tttgaactgt tttattttcc 1380

tgtgagtctt gcgaggtgga agtaatgatt aaatactcat gtgttcagaa aacataaact 1440

ttttttataa aaatattttg tacaattcat taaaggctaa tttatgaaat ttgaaaatct 1500

tcaggttata ctccttaagt tatcccaaag ccgtgtgttt gtgatgtttt ggagtacata 1560

tatatgaaaa ttattatgac acgcactttt ctaatcattg tacatttctc agagtggata 1620

aaaatgtttg acaaagtcct cacttttaag gaaatgcaaa gcttaaaata aaactctctt 1680

ttgtttgatg caaacacaca gtaaaaaaaa aaaaaaaaaa 1720

<210> 82
<211> 1568
<212> DNA
<213> Homo sapiens

<400> 82
gccccgccga gagcgtgaac tatcgctgcg gaggggcaga cctggaccgg tggaaggccg 60

ggcggaagtg cgcgcctggg gccgccttgg ttaccgcgtt ttccgctcct cgctacgtca 120

tcgttgtgag cccgctatca gcggccagcg cgggcgcggc cggagaccgt ggggcccccg 180

gttgccgccc cctcgggagc caccatgttg gtgatacccc ccggactgag cgaggaagag 240

gaggctctgc agaagaaatt caacaagctc aagaaaaaga aaaaggcatt gctggctctg 300

aagaagcaaa gtagcagcag cacaaccagc caaggtggtg tcaaacgctc actatcagag 360

cagcctgtca tggacacagc cacagcaaca gagcaggcaa agcagctggt gaagtcagga 420

gccatcagtg ccatcaaggc tgagaccaag aactcaggct tcaagcgttc tcgaaccctt 480

gaggggaagt taaaggaccc cgagaaggga ccagtcccca ctttccagcc gttccagagg 540

agcatatctg ctgatgatga cctgcaagag tcatccagac gtccccagag gaaatctctg 600

tatgagagct ttgtgtcttc tagtgatcga cttcgagaac taggaccaga tggagaagag 660

gcagagggcc caggggctgg tgatggtccc cctcgaagct ttgactgggg ctatgaagaa 720

cgcagtggtg cccactcctc agcctcccct ccccgaagcc gcagccggga ccgcagccat 780

gagaggaacc gggacagaga ccgagatcgg gagcgggatc gagaccggga tcgagacaga 840

gacagagagc gggacaggga tcgggatcgg gatcgagatc gagaccggga acgggacagg 900

gatcgggagc gggatcgaga ccgagaccga gagggtcctt tccgcaggtc ggattcattc 960

cctgaacggc gagcccctag gaaagggaat actctctatg tatatggaga agacatgaca 1020

cccacccttc tccgtggggc cttctctcct tttggaaaca tcattgacct ctccatggac 1080

ccacccagaa actgtgcctt cgtcacctat gaaaagatgg agtcagcaga tcaggccgtt 1140

gctgagctca acgggaccca ggtggagtct gtacagctca aagtcaacat agcccgaaaa 1200

cagcccatgc tggatgccgc tactggcaag tctgtctggg gctccctcgc tgtccagaac 1260

agccctaagg gttgccaccg ggacaagagg acccagattg tctacagtga tgacgtctac 1320

aaggaaaacc ttgtggatgg cttctaggga acagagctgg attccttgtg cctcatatgc 1380

cccaatgctg gtctcagtaa aacactgagg tggaagctta cacatctccc tcagcctctg 1440

gtttttcagc acttgggatt ggggttaaac ctttaaaaac ggctgtcagg tttgatctca 1500

gtgtaacaac atggccagtg cctgttcccc actcccttgc cccaaaagga tctggaacac 1560

aaaaaaaa 1568

<210> 83
<211> 2457
<212> DNA
<213> Homo sapiens

<400> 83
gagggccggc cggtttggcg gcggctgcgg agggtatctg aggctcggct gccaagccga 60
acgggctctg gctcctccca gtggccggcc ggggcggaag caggaggcgg gggcagcgtg 120
cgcgctgctg gtcttctctc ccgcggcgct ggggcccgcg ctccgctgct gttgctccat 180
tcggcgcttt tctggcggct ggctcctctc cgctgccggc tgctcctcga ccaggcctcc 240
ttctcaacct cagcccgcgg cgccgaccct tccggcaccc tcccgccccg tctcgtactg 300
tcgccgtcac cgccgcggct ccggccctgg ccccgatggc tctgtgcaac ggagactcca 360
agctggagaa tgctggagga gaccttaagg atggccacca ccactatgaa ggagctgttg 420
tcattctgga tgctggtgct cagtacggga aagtcataga ccgaagagtg agggaactgt 480
tcgtgcagtc tgaaattttc cccttggaaa caccagcatt tgctataaag gaacaaggat 540
tccgtgctat tatcatctct ggaggaccta attctgtgta tgctgaagat gctccctggt 600
ttgatccagc aatattcact attggcaagc ctgttcttgg aatttgctat ggtatgcaga 660
tgatgaataa ggtatttgga ggtactgtgc acaaaaaaag tgtcagagaa gatggagttt 720
tcaacattag tgtggataat acatgttcat tattcagggg ccttcagaag gaagaagttg 780
ttttgcttac acatggagat agtgtagaca aagtagctga tggattcaag gttgtggcac 840
gttctggaaa catagtagca ggcatagcaa atgaatctaa aaagttatat ggagcacagt 900
tccaccctga agttggcctt acagaaaatg gaaaagtaat actgaagaat ttcctttatg 960
atatagctgg atgcagtgga accttcaccg tgcagaacag agaacttgag tgtattcgag 1020
agatcaaaga gagagtaggc acgtcaaaag ttttggtttt actcagtggt ggagtagact 1080
caacagtttg tacagctttg ctaaatcgtg ctttgaacca agaacaagtc attgctgtgc 1140
acattgataa tggctttatg agaaaacgag aaagccagtc tgttgaagag gccctcaaaa 1200
agcttggaat tcaggtcaaa gtgataaatg ctgctcattc tttctacaat ggaacaacaa 1260
ccctaccaat atcagatgaa gatagaaccc cacggaaaag aattagcaaa acgttaaata 1320
tgaccacaag tcctgaagag aaaagaaaaa tcattgggga tacttttgtt aagattgcca 1380
atgaagtaat tggagaaatg aacttgaaac cagaggaggt tttccttgcc caaggtactt 1440
tacggcctga tctaattgaa agtgcatccc ttgttgcaag tggcaaagct gaactcatca 1500
aaacccatca caatgacaca gagctcatca gaaagttgag agaggaggga aaagtaatag 1560
aacctctgaa agattttcat aaagatgaag tgagaatttt gggcagagaa cttggacttc 1620
cagaagagtt agtttccagg catccatttc caggtcctgg cctggcaatc agagtaatat 1680
gtgctgaaga accttatatt tgtaaggact ttcctgaaac caacaatatt ttgaaaatag 1740
tagctgattt ttctgcaagt gttaaaaagc cacataccct attacagaga gtcaaagcct 1800
gcacaacaga agaggatcag gagaagctga tgcaaattac cagtctgcat tcactgaatg 1860
ccttcttgct gccaattaaa actgtaggtg tgcagggtga ctgtcgttcc tacagttacg 1920
tgtgtggaat ctccagtaaa gatgaacctg actgggaatc acttattttt ctggctaggc 1980
ttatacctcg catgtgtcac aacgttaaca gagttgttta tatatttggc ccaccagtta 2040
aagaacctcc tacagatgtt actcccactt tcttgacaac aggggtgctc agtactttac 2100

gccaagctga ttttgaggcc cataacattc tcagggagtc tgggtatgct gggaaaatca 2160

gccagatgcc ggtgattttg acaccattac attttgatcg ggacccactt caaaagcagc 2220

cttcatgcca gagatctgtg gttattcgaa cctttattac tagtgacttc atgactggta 2280

tacctgcaac acctggcaat gagatccctg tagaggtggt attaaagatg gtcactgaga 2340

ttaagaagat tcctggtatt tctcgaatta tgtatgactt aacatcaaag cccccaggaa 2400

ctactgagtg ggagtaataa acttcttgtt ctattaaagt accgtgtgca gtttaaa 2457

<210> 84
<211> 4409
<212> DNA
<213> Homo sapiens

<400> 84
   tggcggaaag gctgcggccc cgcgccagag ggccggtggc tcctccgggc ttctgaggac 60
   ccgcagggag cgggcctcat catttctgtc tcctgtttct ccacttcctt ctctattggt 120
   tggtccactg atgtatggac ttgcacatga tgaactgtga acttctagcc acatgtagtg 180
   cccttgggta cttggaggga gacacttacc ataaggaacc agattgctta gagagcgtga 240
   aggatctgat ccgctatttg aggcatgagg atgagacacg agatgtgcgg cagcagctgg 300
   gggcagccca gatcctacag agcgaccttc tgcccatcct cacccagcac caccaggaca 360
   agcctctctt tgatgctgtt atcagactga tggtgaactt gacacaacca gccttgctct 420
   gttttggcaa tctgcctaag gagcccagct ttcggcacca ttttttgcag gtgctaactt 480
   atttgcaggc ctacaaagag gcctttgcca gtgagaaggc ttttggagtc ctcagtgaaa 540
   ccttgtatga gctgctgcag ctgggctggg aggaacggca ggaggaagac aacttgctga 600
   ttgaacggat cctactgctg gtcagaaata ttctccatgt cccagctgac cttgatcagg 660
   agaagaagat tgatgatgac gccagtgccc atgaccagct cctctgggcg attcacctca 720
   gcggcctgga tgacctgctc ctctttctgg ccagctcgtc tgctgaggag caatggagcc 780
   tacatgtgct agagattgtc tcccttatgt ttcgtgacca gaaccccgag cagctggcgg 840
   gagtagggca gggacgctta gctcaggagc ggagtgcaga ttttgcagaa ctggaggtat 900
   tgcgccagcg agagatggca gaaaagaaga ctcgagccct ccagcgaggc aacaggcatt 960
   ctcgatttgg gggctcctat attgtccagg ggttgaaatc cattggggag agggacctca 1020
   tctttcacaa aggccttcac aacctacgaa actacagttc agatttggga aagcagccga 1080
   aaaaggtgcc taaacgtcgc caggccgccc gagagctgtc cattcagcgc cgttctgccc 1140
   tcaatgtgag gctcttcctc agagacttct gctctgagtt cctggagaac tgttacaacc 1200
   ggctcatggg atcagtaaag gatcacctgc ttcgggagaa agctcagcag catgatgaga 1260
   cctattatat gtgggccttg gctttcttca tggccttcaa ccgagctgcc tccttccggc 1320
   caggcctggt ttctgagacc ctcagtgtcc gtaccttcca cttcattgag cagaacctca 1380
   ccaactacta tgagatgatg ctgactgacc gcaaggaagc tgcctcctgg gcacgccgga 1440
   tgcacttggc tctgaaggcc tatcaggagc tgctggcaac agtgaatgag atggacttat 1500
   ctccagatga ggctgtgagg gagagcagcc gcatcatcaa gaacaatatt ttctatgtga 1560
   tggagtaccg agaactattc ctggcacttt ttcgaaagtt tgatgagaga tgccagcccc 1620
   gctctttcct tcgtgacctg gtggagacca cccacctctt cctcaaaatg ttggagcgat 1680
   tctgtcggag ccgtgggaac ctggtggtgc agaacaaaca aaagaagaga aggaagaaga 1740
   agaagaaggt cctagaccag gccattgttt ctggtaatgt cccatctagc ccagaagaag 1800
   tggaggctgt gtggccagcc ctggctgagc agctacagtg ctgtgcccag aattctgagc 1860
   tcagcatgga ctccgtggtt ccctttgatg cggcctcaga ggtgccagtg gaggagcagc 1920
   gggcagaagc tatggtacgg atccaagact gtctcctggc tggccaggcc ccacaggccc 1980
   tgactctcct gaggtctgct cgggaggtgt ggcctgaagg agatgtgttt ggctctcaag 2040
   acatttctcc agaggaagag atccagttgc tgaaacaaat cctctctgct ccacttcccc 2100
   ggcagcaggg cccagaggaa cgtggggcag aggaagaaga agaggaggag gaggaggaag 2160
   aggaggagtt gcaagtggtc caggtgtcgg agaaagaatt taattttctg gactacctga 2220
   aacgctttgc atgttcaact gtcgttcgag cctatgtgct gctactaagg agctaccagc 2280
   agaatagtgc ccacactaac cattgcattg tgaagatgct gcaccggctg gcccatgacc 2340
   tcaaaatgga agccctactt tttcagctgt cagtcttctg cctcttcaat cgtctgctta 2400
   gtgaccctgc tgctggagcc tacaaagagc tagtgacttt tgccaaatac atcctgggca 2460
   aattttttgc actggctgca gtcaaccaaa aagcctttgt ggagctgttg ttctggaaga 2520
   acacagctgt ggttcgagag atgactgagg gctatggctc cctggatgac aggtcttcca 2580
   gtcgcagagc acctacatgg agccccgaag aagaggctca tcttcaggag ctgtacctcg 2640
   ccaataagga cgtggaaggg caggatgtgg tggaagccat cttggcccac ctgaatactg 2700
   ttcctcgaac acgcaagcag atcatccacc atctggtaca gatgggactg gctgacagtg 2760
   tcaaggactt ccaaaggaaa ggaacccata ttgtactgtg gacgggggat caggagttgg 2820
   agctgcagcg gctttttgag gaattccggg actcagatga tgtcctgggt catatcatga 2880
   agaatatcac agccaaacgc tcacgggccc gaatagtgga taaactcttg gctctggggc 2940
   tggtggctga gcggcgggag ctgtacaaga aacggcagaa aaagttggca tcctccatct 3000
   tgccaaatgg agcggagtcc ctgaaagatt tttgccagga agatctggaa gaagaggaaa 3060
   acctgcctga ggaagacagc gaagaggaag aagaaggggg ctcagaagca gaacaagtcc 3120
   agggtagctt agtcctttca aatgaaaacc ttggtcaaag cctgcatcag gaaggctttt 3180
   ctatcccgct cctatggctc cagaactgcc tgatccgagc agctgatgat cgggaagagg 3240
   atggctgctc ccaggccgtt ccattggtgc cactcacaga ggaaaatgag gaagccatgg 3300
   aaaacgaaca gtttcagcag ctgttgcgca agctaggggt tcggccccct gcctctgggc 3360
   aggaaacctt ctggcgaatt ccagccaagc tgagtcctac ccagctccgg agggcagcag 3420
   cttctttgag tcaaccagag gaggaacaga agctgcagcc agagctgcag cctaaagtcc 3480
   ctggagagca aggctctgat gaggagcact gtaaagagca ccgagcacaa gccctgaggg 3540
   ccctcttgct agcccacaag aagaaagcgg gcctggcatc cccagaggag gaagacgctg 3600
   ttggtaaaga gccgctgaag gcagcaccca agaaacgaca attgctggac agcgacgagg 3660
   aacaggaaga agatgagggc aggaacagag caccagagtt gggagctcca ggaatccaaa 3720
   agaagaaacg ataccagatt gaggatgatg aggatgactg aagagctaag aagcctaggg 3780
   gtagagatag acacgttttt agatggtgca ttcaagtcag agttggaagg gtcatacagg 3840
   acccagaagc tcctcttact ggacagagca gtagaatccc aggctctttc acggatttag 3900
   gctgagaaac ttcagcagca gtattggact tctctgctgt cagtctttgt ttctcccagt 3960
   ttttatcggt atgccccagc agctctctgg agatttagtt ctcttcttct aggaaccatt 4020
   tcttttggtt attggagctc cttagcaaat aagaggagtg agtgaaatag agaaagcacc 4080
   ccggacccct tttattggtc tgttcctttg gctttctctt ggaggtgggt cgcagcacca 4140
   gatggggagc tttatgacat gacaggcggg agttttgtac cacttttaga agataccaga 4200
   tgcctctgtc ctcattttgt ttcctatgat catcctctag ataccagata gaggcccatt 4260
   cctgcatttg agatagggtt ggaatatagt actctagatg atgaatgtgt ttgctttgtg 4320
   tttggtatta aacatgtaag attagaagtt ctcaaaaaaa aaaaaaaaaa aaaaaaaaaa 4380
   aaaaaaaaaa aaaaaaaaaa aaaaaaaaa 4409

<210> 85
<211> 3128
<212> DNA
<213> Homo sapiens

<400> 85
gcttcgcccc gtggcgcggt ttgaaatttt gcggggctca acggctcgcg gagcggctac 60

gcggagtgac atcgccggtg tttgcgggtg gttgttgctc tcggggccgt gtggagtagg 120

tctggacctg gactcacggc tgcttggagc gtccgccatg aggagaagtg aggtgctggc 180

ggaggagtcc atagtatgtc tgcagaaagc cctaaatcac cttcgggaaa tatgggagct 240

aattgggatt ccagaggacc agcggttaca aagaactgag gtggtaaaga agcatatcaa 300

ggaactcctg gatatgatga ttgctgaaga ggaaagcctg aaggaaagac tcatcaaaag 360

catatccgtc tgtcagaaag agctgaacac tctgtgcagc gagttacatg ttgagccatt 420

tcaggaagaa ggagagacga ccatcttgca actagaaaaa gatttgcgca cccaagtgga 480

attgatgcga aaacagaaaa aggagagaaa acaggaactg aagctacttc aagagcaaga 540

tcaagaactg tgcgaaattc tttgtatgcc ccactatgat attgacagtg cctcagtgcc 600

cagcttagaa gagctgaacc agttcaggca acatgtgaca actttgaggg aaacaaaggc 660

ttctaggcgt gaggagtttg tcagtataaa gagacagatc atactgtgta tggaagaatt 720

agaccacacc ccagacacaa gctttgaaag agatgtggtg tgtgaagacg aagatgcctt 780

ttgtttgtct ttggagaata ttgcaacact acaaaagttg ctacggcagc tggaaatgca 840

gaaatcacaa aatgaagcag tgtgtgaggg gctgcgtact caaatccgag agctctggga 900

caggttgcaa atacctgaag aagaaagaga agctgtggcc accattatgt ctgggtcaaa 960

ggccaaggtc cggaaagcgc tgcaattaga agtggatcgg ttggaagaac tgaaaatgca 1020

aaacatgaag aaagtgattg aggcaattcg agtggagctg gttcagtact gggaccagtg 1080

cttttatagc caggagcaga gacaagcttt tgcccctttc tgtgctgagg actacacaga 1140

aagtctgctc cagctccacg atgctgagat tgtgcggtta aaaaactact atgaagttca 1200

caaggaactc tttgaaggtg tccagaagtg ggaagaaacc tggaggcttt tcttagagtt 1260

tgagagaaaa gcttcagatc caaatcgatt tacaaaccga ggaggaaatc ttctaaaaga 1320

agaaaaacaa cgagccaagc tccagaaaat gctgcccaag ctggaagaag agttgaaggc 1380

acgaattgaa ttgtgggaac aggaacattc aaaggcattt atggtgaatg ggcagaaatt 1440

catggagtat gtggcagaac aatgggagat gcatcgattg gagaaagaga gagccaagca 1500

ggaaagacaa ctgaagaaca aaaaacagac agagacagag atgctgtatg gcagcgctcc 1560

tcgaacacct agcaagcggc gaggactggc tcccaataca ccgggcaaag cacgtaagct 1620

gaacactacc accatgtcca atgctacggc caatagtagc attcggccta tctttggagg 1680

gacagtctac cactcccccg tgtctcgact tcctccttct ggcagcaagc cagtcgctgc 1740

ttccacctgt tcagggaaga aaacaccccg tactggcagg catggagcca acaaggagaa 1800

cctggagctc aacggcagca tcctgagtgg tgggtaccct ggctcggccc ccctccagcg 1860

caacttcagc attaattctg ttgccagcac ctattctgag tttgcgaagg atccgtccct 1920

ctctgacagt tccactgttg ggcttcagcg agaactttca aaggcttcca aatctgatgc 1980

tacttctgga atcctcaatt caaccaacat ccagtcctga gaagccctga tcagtcaacc 2040

agctgtggct tcctgtgcct agactggacc taattatatg ggggtgactt tagtttttct 2100

tcagcttagg cgtgcttgaa accttggcca ggttccatga ccatgggcct aacttaaaga 2160

tgtgaatgag tgttacagtt gaaagcccat cataggttta gtggtcctag gagacttggt 2220

tttgacttat atacatgaaa agtttatggc aagaagtgca aattttagca tatggggcct 2280

gacttctcta ccacataatt ctacttgctg aagcatgatc aaagcttgtt ttatttcacc 2340

actgtaggaa aatgattgac tatgcccatc cctgggggta attttggcat gtatacctgt 2400

aactagtaat taacatcttt tttgtttagg catgttcaat taatgctgta gctatcatag 2460

ctttgctctt acctgaagcc ttgtccccac cacacaggac agccttcctc ctgaagagaa 2520

tgtctttgtg tgtccgaagt tgagatggcc tgccctactg ccaaagaggt gacaggaagg 2580

ctgggagcag ctttgttaaa ttgtgttcag ttctgttaca cagtgcattg ccctttgttg 2640

ggggtatgca tgtatgaaca cacatgcttg tcggaacgct ttctcggcgt ttgtcccttg 2700

gctctcatct cccccattcc tgtgcctact ttgcctgagt tcttctaccc ccgcagttgc 2760

cagccacatt gggagtctgt ttgttccaat gggttgagct gtctttgtcg tggagatctg 2820

gaactttgca catgtcacta ctggggaggt gttcctgctc tagcttccac gatgaggcgc 2880

cctctttacc tatcctctca atcactactc ttcttgaagc actattattt attcttccgc 2940

tgtctgcctg cagcagtact actgtcaaca tagtgtaaat ggttctcaaa agcttaccag 3000

tgtggacttg gtgttagcca cgctgtttac tcatacagta cgtgtcctgt ttttaaaata 3060

tacaattatt cttaaaaata aattaaaatc tgtatactta catttcaaaa agaaaaaaaa 3120

aaaaaaaa 3128

<210> 86
<211> 3091
<212> DNA
<213> Homo sapiens

<400> 86
gcttcgcccc gtggcgcggt ttgaaatttt gcggggctca acggctcgcg gagcggctac 60

gcggagtgac atcgccggtg tttgcgggtg gttgttgctc tcggggccgt gtggagtagg 120

tctggacctg gactcacggc tgcttggagc gtccgccatg aggagaagtg aggtgctggc 180

ggaggagtcc atagtatgtc tgcagaaagc cctaaatcac cttcgggaaa tatgggagct 240

aattgggatt ccagaggacc agcggttaca aagaactgag gtggtaaaga agcatatcaa 300

ggaactcctg gatatgatga ttgctgaaga ggaaagcctg aaggaaagac tcatcaaaag 360

catatccgtc tgtcagaaag agctgaacac tctgtgcagc gagttacatg ttgagccatt 420

tcaggaagaa ggagagacga ccatcttgca actagaaaaa gatttgcgca cccaagtgga 480

attgatgcga aaacagaaaa aggagagaaa acaggaactg aagctacttc aagagcaaga 540

tcaagaactg tgcgaaattc tttgtatgcc ccactatgat attgacagtg cctcagtgcc 600

cagcttagaa gagctgaacc agttcaggca acatgtgaca actttgaggg aaacaaaggc 660

ttctaggcgt gaggagtttg tcagtataaa gagacagatc atactgtgta tggaagaatt 720

agaccacacc ccagacacaa gctttgaaag agatgtggtg tgtgaagacg aagatgcctt 780

ttgtttgtct ttggagaata ttgcaacact acaaaagttg ctacggcagc tggaaatgca 840

gaaatcacaa aatgaagcag tgtgtgaggg gctgcgtact caaatccgag agctctggga 900

caggttgcaa atacctgaag aagaaagaga agctgtggcc accattatgt ctgggtcaaa 960

ggccaaggtc cggaaagcgc tgcaattaga agtggatcgg ttggaagaac tgaaaatgca 1020

aaacatgaag aaagtgattg aggcaattcg agtggagctg gttcagtact gggaccagtg 1080

cttttatagc caggagcaga gacaagcttt tgcccctttc tgtgctgagg actacacaga 1140

aagtctgctc cagctccacg atgctgagat tgtgcggtta aaaaactact atgaagttca 1200

caaggaactc tttgaaggtg tccagaagtg ggaagaaacc tggaggcttt tcttagagtt 1260

tgagagaaaa gcttcagatc caaatcgatt tacaaaccga ggaggaaatc ttctaaaaga 1320

agaaaaacaa cgagccaagc tccagaaaat gctgcccaag ctggaagaag agttgaaggc 1380

acgaattgaa ttgtgggaac aggaacattc aaaggcattt atggtgaatg ggcagaaatt 1440

catggagtat gtggcagaac aatgggagat gcatcgattg gagaaagaga gagccaagca 1500

ggaaagacaa ctgaagaaca aaaaacagac agagacagag atgctgtatg gcagcgctcc 1560

tcgaacacct agcaagcggc gaggactggc tcccaataca ccgggcaaag cacgtaagct 1620

gaacactacc accatgtcca atgctacggc caatagtagc attcggccta tctttggagg 1680

gacagtctac cactcccccg tgtctcgact tcctccttct ggcagcaagc cagtcgctgc 1740

ttccacctgt tcagggaaga aaacaccccg tactggcagg catggagcca acaaggagaa 1800

cctggagctc aacggcagca tcctgagtgg tgggtaccct ggctcggccc ccctccagcg 1860

caacttcagc attaattctg ttgccagcac ctattctgag tttgcgcgag aactttcaaa 1920

ggcttccaaa tctgatgcta cttctggaat cctcaattca accaacatcc agtcctgaga 1980

agccctgatc agtcaaccag ctgtggcttc ctgtgcctag actggaccta attatatggg 2040

ggtgacttta gtttttcttc agcttaggcg tgcttgaaac cttggccagg ttccatgacc 2100

atgggcctaa cttaaagatg tgaatgagtg ttacagttga aagcccatca taggtttagt 2160

ggtcctagga gacttggttt tgacttatat acatgaaaag tttatggcaa gaagtgcaaa 2220

ttttagcata tggggcctga cttctctacc acataattct acttgctgaa gcatgatcaa 2280

agcttgtttt atttcaccac tgtaggaaaa tgattgacta tgcccatccc tgggggtaat 2340

tttggcatgt atacctgtaa ctagtaatta acatcttttt tgtttaggca tgttcaatta 2400

atgctgtagc tatcatagct ttgctcttac ctgaagcctt gtccccacca cacaggacag 2460

ccttcctcct gaagagaatg tctttgtgtg tccgaagttg agatggcctg ccctactgcc 2520

aaagaggtga caggaaggct gggagcagct ttgttaaatt gtgttcagtt ctgttacaca 2580

gtgcattgcc ctttgttggg ggtatgcatg tatgaacaca catgcttgtc ggaacgcttt 2640

ctcggcgttt gtcccttggc tctcatctcc cccattcctg tgcctacttt gcctgagttc 2700

ttctaccccc gcagttgcca gccacattgg gagtctgttt gttccaatgg gttgagctgt 2760

ctttgtcgtg gagatctgga actttgcaca tgtcactact ggggaggtgt tcctgctcta 2820

gcttccacga tgaggcgccc tctttaccta tcctctcaat cactactctt cttgaagcac 2880

tattatttat tcttccgctg tctgcctgca gcagtactac tgtcaacata gtgtaaatgg 2940

ttctcaaaag cttaccagtg tggacttggt gttagccacg ctgtttactc atacagtacg 3000

tgtcctgttt ttaaaatata caattattct taaaaataaa ttaaaatctg tatacttaca 3060

tttcaaaaag aaaaaaaaaa aaaaaaaaaa a 3091

<210> 87
<211> 3011
<212> DNA
<213> Homo sapiens

<400> 87
gcttcgcccc gtggcgcggt ttgaaatttt gcggggctca acggctcgcg gagcggctac 60

gcggagtgac atcgccggtg tttgcgggtg gttgttgctc tcggggccgt gtggagtagg 120

tctggacctg gactcacggc tgcttggagc gtccgccatg aggagaagtg aggtgctggc 180

ggaggagtcc atagtatgtc tgcagaaagc cctaaatcac cttcgggaaa tatgggagct 240

aattgggatt ccagaggacc agcggttaca aagaactgag gtggtaaaga agcatatcaa 300

ggaactcctg gatatgatga ttgctgaaga ggaaagcctg aaggaaagac tcatcaaaag 360

catatccgtc tgtcagaaag agctgaacac tctgtgcagc gagttacatg ttgagccatt 420

tcaggaagaa ggagagacga ccatcttgca actagaaaaa gatttgcgca cccaagtgga 480

attgatgcga aaacagaaaa aggagagaaa acaggaactg aagctacttc aagagcaaga 540

tcaagaactg tgcgaaattc tttgtatgcc ccactatgat attgacagtg cctcagtgcc 600

cagcttagaa gagctgaacc agttcaggca acatgtgaca actttgaggg aaacaaaggc 660

ttctaggcgt gaggagtttg tcagtataaa gagacagatc atactgtgta tggaagaatt 720

agaccacacc ccagacacaa gctttgaaag agatgtggtg tgtgaagacg aagatgcctt 780

ttgtttgtct ttggagaata ttgcaacact acaaaagttg ctacggcagc tggaaatgca 840

gaaatcacaa aatgaagcag tgtgtgaggg gctgcgtact caaatccgag agctctggga 900

caggttgcaa atacctgaag aagaaagaga agctgtggcc accattatgt ctgggtcaaa 960

ggccaaggtc cggaaagcgc tgcaattaga agtggatcgg ttggaagaac tgaaaatgca 1020

aaacatgaag aaagtgattg aggcaattcg agtggagctg gttcagtact gggaccagtg 1080

cttttatagc caggagcaga gacaagcttt tgcccctttc tgtgctgagg actacacaga 1140

aagtctgctc cagctccacg atgctgagat tgtgcggtta aaaaactact atgaagttca 1200

caaggaactc tttgaaggtg tccagaagtg ggaagaaacc tggaggcttt tcttagagtt 1260

tgagagaaaa gcttcagatc caaatcgatt tacaaaccga ggaggaaatc ttctaaaaga 1320

agaaaaacaa cgagccaagc tccagaaaat gctgcccaag ctggaagaag agttgaaggc 1380

acgaattgaa ttgtgggaac aggaacattc aaaggcattt atggtgaatg ggcagaaatt 1440

catggagtat gtggcagaac aatgggagat gcatcgattg gagaaagaga gagccaagca 1500

ggaaagacaa ctgaagaaca aaaaacagac agagacagag atgctgtatg gcagcgctcc 1560

tcgaacacct agcaagcggc gaggactggc tcccaataca ccgggcaaag cacgtaagct 1620

gaacactacc accatgtcca atgctacggc caatagtagc attcggccta tctttggagg 1680

gacagtctac cactcccccg tgtctcgact tcctccttct ggcagcaagc cagtcgctgc 1740

ttccacctgt tcagggaaga aaacaccccg tactggcagg catggagcca acaaggagaa 1800

cctggagctc aacggcagca tcctgagtgc gagaactttc aaaggcttcc aaatctgatg 1860

ctacttctgg aatcctcaat tcaaccaaca tccagtcctg agaagccctg atcagtcaac 1920

cagctgtggc ttcctgtgcc tagactggac ctaattatat gggggtgact ttagtttttc 1980

ttcagcttag gcgtgcttga aaccttggcc aggttccatg accatgggcc taacttaaag 2040

atgtgaatga gtgttacagt tgaaagccca tcataggttt agtggtccta ggagacttgg 2100

ttttgactta tatacatgaa aagtttatgg caagaagtgc aaattttagc atatggggcc 2160

tgacttctct accacataat tctacttgct gaagcatgat caaagcttgt tttatttcac 2220

cactgtagga aaatgattga ctatgcccat ccctgggggt aattttggca tgtatacctg 2280

taactagtaa ttaacatctt ttttgtttag gcatgttcaa ttaatgctgt agctatcata 2340

gctttgctct tacctgaagc cttgtcccca ccacacagga cagccttcct cctgaagaga 2400

atgtctttgt gtgtccgaag ttgagatggc ctgccctact gccaaagagg tgacaggaag 2460

gctgggagca gctttgttaa attgtgttca gttctgttac acagtgcatt gccctttgtt 2520

gggggtatgc atgtatgaac acacatgctt gtcggaacgc tttctcggcg tttgtccctt 2580

ggctctcatc tcccccattc ctgtgcctac tttgcctgag ttcttctacc cccgcagttg 2640

ccagccacat tgggagtctg tttgttccaa tgggttgagc tgtctttgtc gtggagatct 2700

ggaactttgc acatgtcact actggggagg tgttcctgct ctagcttcca cgatgaggcg 2760

ccctctttac ctatcctctc aatcactact cttcttgaag cactattatt tattcttccg 2820

ctgtctgcct gcagcagtac tactgtcaac atagtgtaaa tggttctcaa aagcttacca 2880

gtgtggactt ggtgttagcc acgctgttta ctcatacagt acgtgtcctg tttttaaaat 2940

atacaattat tcttaaaaat aaattaaaat ctgtatactt acatttcaaa aagaaaaaaa 3000

aaaaaaaaaa a 3011

<210> 88
<211> 1530
<212> DNA
<213> Homo sapiens

<400> 88
aatcctggaa caaggctaca gcgtcgaaga tccccagcgc tgcgggctcg gagagcagtc 60

ctaacggcgc ctcgtacgct agtgtcctcc cttttcagtc cgcgtccctc cctgggccgg 120

gctggcactc ttgccttccc cgtccctcat ggcgctgctc cgacgcccga cggtgtccag 180

tgatttggag aatattgaca caggagttaa ttctaaagtt aagagtcatg tgactattag 240

gcgaactgtt ttagaagaaa ttggaaatag agttacaacc agagcagcac aagtagctaa 300

gaaagctcag aacaccaaag ttccagttca acccaccaaa acaacaaatg tcaacaaaca 360

actgaaacct actgcttctg tcaaaccagt acagatggaa aagttggctc caaagggtcc 420

ttctcccaca cctgaggatg tctccatgaa ggaagagaat ctctgccaag ctttttctga 480

tgccttgctc tgcaaaatcg aggacattga taacgaagat tgggagaacc ctcagctctg 540

cagtgactac gttaaggata tctatcagta tctcaggcag ctggaggttt tgcagtccat 600

aaacccacat ttcttagatg gaagagatat aaatggacgc atgcgtgcca tcctagtgga 660

ttggctggta caagtccact ccaagtttag gcttctgcag gagactctgt acatgtgcgt 720

tggcattatg gatcgatttt tacaggttca gccagtttcc cggaagaagc ttcaattagt 780

tgggattact gctctgctct tggcttccaa gtatgaggag atgttttctc caaatattga 840

agactttgtt tacatcacag acaatgctta taccagttcc caaatccgag aaatggaaac 900

tctaattttg aaagaattga aatttgagtt gggtcgaccc ttgccactac acttcttaag 960

gcgagcatca aaagccgggg aggttgatgt tgaacagcac actttagcca agtatttgat 1020

ggagctgact ctcatcgact atgatatggt gcattatcat ccttctaagg tagcagcagc 1080

tgcttcctgc ttgtctcaga aggttctagg acaaggaaaa tggaacttaa agcagcagta 1140

ttacacagga tacacagaga atgaagtatt ggaagtcatg cagcacatgg ccaagaatgt 1200

ggtgaaagta aatgaaaact taactaaatt catcgccatc aagaataagt atgcaagcag 1260

caaactcctg aagatcagca tgatccctca gctgaactca aaagccgtca aagaccttgc 1320

ctccccactg ataggaaggt cctaggctgc cgtgggccct ggggatgtgt gcttcattgt 1380

gccctttttc ttattggttt agaactcttg attttgtaca tagtcctctg gtctatctca 1440

tgaaacctct tctcagacca gttttctaaa catatattga ggaaaaataa agcgattggt 1500

ttttcttaag gtaaaaaaaa aaaaaaaaaa 1530

<210> 89
<211> 3138
<212> DNA
<213> Homo sapiens

<400> 89
   cccgtgttct gcgttgccgg ccgtgggtgc tctggccaca gtgagttagg ggcgtcggag 60
   cgggtttctc caaccgcaat cggctccgct caaggggagg aggagagtcc cttctcggaa 120
   ggcctaagga aacgtgtcgt ctggaatggg cttgggggcc acgcctgcac atctccgcga 180
   gacagaggga taaagtgaag atggtgctgt tattgttacc tcgagtgcca catgcgacct 240
   ctgagatatg tacacagtca ttcttactat cgcactcagc cattcttact acgctaaaga 300
   agaaataatt attcgaggat atttgcctgg cccagaagaa acttatgtaa atttcatgaa 360
   ctattatatc cgttttcctc ggagtgagag aaaactcttt ttagatatca tctgagaggt 420
   agttaatttg gcaccatggg gatacaggga ttgctacaat ttatcaaaga agcttcagaa 480
   cccatccatg tgaggaagta taaagggcag gtagtagctg tggatacata ttgctggctt 540
   cacaaaggag ctattgcttg tgctgaaaaa ctagccaaag gtgaacctac tgataggtat 600
   gtaggatttt gtatgaaatt tgtaaatatg ttactatctc atgggatcaa gcctattctc 660
   gtatttgatg gatgtacttt accttctaaa aaggaagtag agagatctag aagagaaaga 720
   cgacaagcca atcttcttaa gggaaagcaa cttcttcgtg aggggaaagt ctcggaagct 780
   cgagagtgtt tcacccggtc tatcaatatc acacatgcca tggcccacaa agtaattaaa 840
   gctgcccggt ctcagggggt agattgcctc gtggctccct atgaagctga tgcgcagttg 900
   gcctatctta acaaagcggg aattgtgcaa gccataatta cagaggactc ggatctccta 960
   gcttttggct gtaaaaaggt aattttaaag atggaccagt ttggaaatgg acttgaaatt 1020
   gatcaagctc ggctaggaat gtgcagacag cttggggatg tattcacgga agagaagttt 1080
   cgttacatgt gtattctttc aggttgtgac tacctgtcat cactgcgtgg gattggatta 1140
   gcaaaggcat gcaaagtcct aagactagcc aataatccag atatagtaaa ggttatcaag 1200
   aaaattggac attatctcaa gatgaatatc acggtaccag aggattacat caacgggttt 1260
   attcgggcca acaatacctt cctctatcag ctagtttttg atcccatcaa aaggaaactt 1320
   attcctctga acgcctatga agatgatgtt gatcctgaaa cactaagcta cgctgggcaa 1380
   tatgttgatg attccatagc tcttcaaata gcacttggaa ataaagatat aaatactttt 1440
   gaacagatcg atgactacaa tccagacact gctatgcctg cccattcaag aagtcatagt 1500
   tgggatgaca aaacatgtca aaagtcagct aatgttagca gcatttggca taggaattac 1560
   tctcccagac cagagtcggg tactgtttca gatgccccac aattgaagga aaatccaagt 1620
   actgtgggag tggaacgagt gattagtact aaagggttaa atctcccaag gaaatcatcc 1680
   attgtgaaaa gaccaagaag tgcagagctg tcagaagatg acctgttgag tcagtattct 1740
   ctttcattta cgaagaagac caagaaaaat agctctgaag gcaataaatc attgagcttt 1800
   tctgaagtgt ttgtgcctga cctggtaaat ggacctacta acaaaaagag tgtaagcact 1860
   ccacctagga cgagaaataa atttgcaaca tttttacaaa ggaaaaatga agaaagtggt 1920
   gcagttgtgg ttccagggac cagaagcagg tttttttgca gttcagattc tactgactgt 1980
   gtatcaaaca aagtgagcat ccagcctctg gatgaaactg ctgtcacaga taaagagaac 2040
   aatctgcatg aatcagagta tggagaccaa gaaggcaaga gactggttga cacagatgta 2100
   gcacgtaatt caagtgatga cattccgaat aatcatattc caggtgatca tattccagac 2160
   aaggcaacag tgtttacaga tgaagagtcc tactcttttg agagcagcaa atttacaagg 2220
   accatttcac cacccacttt gggaacacta agaagttgtt ttagttggtc tggaggtctt 2280
   ggagattttt caagaacgcc gagcccctct ccaagcacag cattgcagca gttccgaaga 2340
   aagagcgatt cccccacctc tttgcctgag aataatatgt ctgatgtgtc gcagttaaag 2400
   agcgaggagt ccagtgacga tgagtctcat cccttacgag aaggggcatg ttcttcacag 2460
   tcccaggaaa gtggagaatt ctcactgcag agttcaaatg catcaaagct ttctcagtgc 2520
   tctagtaagg actctgattc agaggaatct gattgcaata ttaagttact tgacagtcaa 2580
   agtgaccaga cctccaagct atgtttatct catttctcaa aaaaagacac acctctaagg 2640
   aacaaggttc ctgggctata taagtccagt tctgcagact ctctttctac aaccaagatc 2700
   aaacctctag gacctgccag agccagtggg ctgagcaaga agccggcaag catccagaag 2760
   agaaagcatc ataatgccga gaacaagccg gggttacaga tcaaactcaa tgagctctgg 2820
   aaaaactttg gatttaaaaa attctgaaaa gcttcctcct tgtaagaaac ccctgtcccc 2880
   agtcagagat aacatccaac taactccaga agcggaagag gatatattta acaaacctga 2940
   atgtggccgt gttcaaagag caatattcca gtaaatgcag actgctgcaa agcttttgcc 3000
   tgcaagagaa tctgatcaat ttgaagtccc tgtttgggaa tgaggcactt atcagcatga 3060
   agaatttttt ctcattctgt gccattttaa aaatagaata cattttgtat attaacttta 3120
   aaaaaaaaaa aaaaaaaa 3138

<210> 90
<211> 3140
<212> DNA
<213> Homo sapiens

<400> 90
cccgtgttct gcgttgccgg ccgtgggtgc tctggccaca gtgagttagg ggcgtcggag 60

cgggtttctc caaccgcaat cggctccgct caaggggagg aggagagtcc cttctcggaa 120

ggcctaagga aacgtgtcgt ctggaatggg cttgggggcc acgcctgcac atctccgcga 180

gacagaggga taaagtgaag atggtgctgt tattgttacc tcgagtgcca catgcgacct 240

ctgagatatg tacacagtca ttcttactat cgcactcagc cattcttact acgctaaaga 300

agaaataatt attcgaggat atttgcctgg cccagaagaa acttatgtaa atttcatgaa 360

ctattatatc cgttttcctc ggagtgagag aaaactcttt ttagatatca tctgagaggt 420

agttaatttg gcaccatggg gatacaggga ttgctacaat ttatcaaaga agcttcagaa 480

cccatccatg tgaggaagta taaagggcag gtagtagctg tggatacata ttgctggctt 540

cacaaaggag ctattgcttg tgctgaaaaa ctagccaaag gtgaacctac tgataggtat 600

gtaggatttt gtatgaaatt tgtaaatatg ttactatctc atgggatcaa gcctattctc 660
gtatttgatg gatgtacttt accttctaaa aaggaagtag agagatctag aagagaaaga 720
cgacaagcca atcttcttaa gggaaagcaa cttcttcgtg aggggaaagt ctcggaagct 780
cgagagtgtt tcacccggtc tatcaatatc acacatgcca tggcccacaa agtaattaaa 840
gctgcccggt ctcagggggt agattgcctc gtggctccct atgaagctga tgcgcagttg 900
gcctatctta acaaagcggg aattgtgcaa gccataatta cagaggactc ggatctccta 960
gcttttggct gtaaaaaggt aattttaaag atggaccagt ttggaaatgg acttgaaatt 1020
gatcaagctc ggctaggaat gtgcagacag cttggggatg tattcacgga agagaagttt 1080
cgttacatgt gtattctttc aggttgtgac tacctgtcat cactgcgtgg gattggatta 1140
gcaaaggcat gcaaagtcct aagactagcc aataatccag atatagtaaa ggttatcaag 1200
aaaattggac attatctcaa gatgaatatc acggtaccag aggattacat caacgggttt 1260
attcgggcca acaatacctt cctctatcag ctagtttttg atcccatcaa aaggaaactt 1320
attcctctga acgcctatga agatgatgtt gatcctgaaa cactaagcta cgctgggcaa 1380
tatgttgatg attccatagc tcttcaaata gcacttggaa ataaagatat aaatactttt 1440
gaacagatcg atgactacaa tccagacact gctatgcctg cccattcaag aagtcatagt 1500
tgggatgaca aaacatgtca aaagtcagct aatgttagca gcatttggca taggaattac 1560
tctcccagac cagagtcggg tactgtttca gatgccccac aattgaagga aaatccaagt 1620
actgtgggag tggaacgagt gattagtact aaagggttaa atctcccaag gaaatcatcc 1680
attgtgaaaa gaccaagaag tgcagagctg tcagaagatg acctgttgag tcagtattct 1740
ctttcattta cgaagaagac caagaaaaat agctctgaag gcaataaatc attgagcttt 1800
tctgaagtgt ttgtgcctga cctggtaaat ggacctacta acaaaaagag tgtaagcact 1860
ccacctagga cgagaaataa atttgcaaca tttttacaaa ggaaaaatga agaaagtggt 1920
gcagttgtgg ttccagggac cagaagcagg tttttttgca gttcagattc tactgactgt 1980
gtatcaaaca aagtgagcat ccagcctctg gatgaaactg ctgtcacaga taaagagaac 2040
aatctgcatg aatcagagta tggagaccaa gaaggcaaga gactggttga cacagatgta 2100
gcacgtaatt caagtgatga cattccgaat aatcatattc caggtgatca tattccagac 2160
aaggcaacag tgtttacaga tgaagagtcc tactcttttg agagcagcaa atttacaagg 2220
accatttcac cacccacttt gggaacacta agaagttgtt ttagttggtc tggaggtctt 2280
ggagattttt caagaacgcc gagcccctct ccaagcacag cattgcagca gttccgaaga 2340
aagagcgatt cccccacctc tttgcctgag aataatatgt ctgatgtgtc gcagttaaag 2400
agcgaggagt ccagtgacga tgagtctcat cccttacgag aaggggcatg ttcttcacag 2460
tcccaggaaa gtggagaatt ctcactgcag agttcaaatg catcaaagct ttctcagtgc 2520
tctagtaagg actctgattc agaggaatct gattgcaata ttaagttact tgacagtcaa 2580
agtgaccaga cctccaagct atgtttatct catttctcaa aaaaagacac acctctaagg 2640
aacaaggttc ctgggctata taagtccagt tctgcagact ctctttctac aaccaagatc 2700

aaacctctag gacctgccag agccagtggg ctgagcaaga agccggcaag catccagaag 2760

agaaagcatc ataatgccga gaacaagccg gggttacaga tcaaactcaa tgagctctgg 2820

aaaaactttg gatttaaaaa agattctgaa aagcttcctc cttgtaagaa acccctgtcc 2880

ccagtcagag ataacatcca actaactcca gaagcggaag aggatatatt taacaaacct 2940

gaatgtggcc gtgttcaaag agcaatattc cagtaaatgc agactgctgc aaagcttttg 3000

cctgcaagag aatctgatca atttgaagtc cctgtttggg aatgaggcac ttatcagcat 3060

gaagaatttt ttctcattct gtgccatttt aaaaatagaa tacattttgt atattaactt 3120

taaaaaaaaa aaaaaaaaaa 3140

<210> 91
<211> 3245
<212> DNA
<213> Homo sapiens

<400> 91
cccgtgttct gcgttgccgg ccgtgggtgc tctggccaca gtgagttagg ggcgtcggag 60

cgggtttctc caaccgcaat cggctccgct caaggggagg aggagagtcc cttctcggaa 120

ggcctaagga aacgtgtcgt ctggaatggg cttgggggcc acgcctgcac atctccgcga 180

gacagaggga taaagtgaag atggtgctgt tattgttacc tcgagtgcca catgcgacct 240

ctgagatatg tacacagtca ttcttactat cgcactcagc cattcttact acgctaaaga 300

agaaataatt attcgaggat atttgcctgg cccagaagaa acttatgtaa atttcatgaa 360

ctattatatc cgttttcctc ggagtgagag aaaactcttt ttagatatca tctgagagaa 420

ctagtgaatc ccagtcactg agtggagttg agagtctaag aacctctgaa atttgagaac 480

tgctggacca gagcctttag agctctgata aggtgtcaac agggtagtta atttggcacc 540

atggggatac agggattgct acaatttatc aaagaagctt cagaacccat ccatgtgagg 600

aagtataaag ggcaggtagt agctgtggat acatattgct ggcttcacaa aggagctatt 660

gcttgtgctg aaaaactagc caaaggtgaa cctactgata ggtatgtagg attttgtatg 720

aaatttgtaa atatgttact atctcatggg atcaagccta ttctcgtatt tgatggatgt 780

actttacctt ctaaaaagga agtagagaga tctagaagag aaagacgaca agccaatctt 840

cttaagggaa agcaacttct tcgtgagggg aaagtctcgg aagctcgaga gtgtttcacc 900

cggtctatca atatcacaca tgccatggcc cacaaagtaa ttaaagctgc ccggtctcag 960

ggggtagatt gcctcgtggc tccctatgaa gctgatgcgc agttggccta tcttaacaaa 1020

gcgggaattg tgcaagccat aattacagag gactcggatc tcctagcttt tggctgtaaa 1080

aaggtaattt taaagatgga ccagtttgga aatggacttg aaattgatca agctcggcta 1140

ggaatgtgca gacagcttgg ggatgtattc acggaagaga agtttcgtta catgtgtatt 1200

ctttcaggtt gtgactacct gtcatcactg cgtgggattg gattagcaaa ggcatgcaaa 1260

gtcctaagac tagccaataa tccagatata gtaaaggtta tcaagaaaat tggacattat 1320

ctcaagatga atatcacggt accagaggat tacatcaacg ggtttattcg ggccaacaat 1380

accttcctct atcagctagt ttttgatccc atcaaaagga aacttattcc tctgaacgcc 1440

tatgaagatg atgttgatcc tgaaacacta agctacgctg ggcaatatgt tgatgattcc 1500

atagctcttc aaatagcact tggaaataaa gatataaata cttttgaaca gatcgatgac 1560

tacaatccag acactgctat gcctgcccat tcaagaagtc atagttggga tgacaaaaca 1620

tgtcaaaagt cagctaatgt tagcagcatt tggcatagga attactctcc cagaccagag 1680

tcgggtactg tttcagatgc cccacaattg aaggaaaatc caagtactgt gggagtggaa 1740

cgagtgatta gtactaaagg gttaaatctc ccaaggaaat catccattgt gaaaagacca 1800

agaagtgcag agctgtcaga agatgacctg ttgagtcagt attctctttc atttacgaag 1860

aagaccaaga aaaatagctc tgaaggcaat aaatcattga gcttttctga agtgtttgtg 1920

cctgacctgg taaatggacc tactaacaaa aagagtgtaa gcactccacc taggacgaga 1980

aataaatttg caacattttt acaaaggaaa aatgaagaaa gtggtgcagt tgtggttcca 2040

gggaccagaa gcaggttttt ttgcagttca gattctactg actgtgtatc aaacaaagtg 2100

agcatccagc ctctggatga aactgctgtc acagataaag agaacaatct gcatgaatca 2160

gagtatggag accaagaagg caagagactg gttgacacag atgtagcacg taattcaagt 2220

gatgacattc cgaataatca tattccaggt gatcatattc cagacaaggc aacagtgttt 2280

acagatgaag agtcctactc ttttgagagc agcaaattta caaggaccat ttcaccaccc 2340

actttgggaa cactaagaag ttgttttagt tggtctggag gtcttggaga tttttcaaga 2400

acgccgagcc cctctccaag cacagcattg cagcagttcc gaagaaagag cgattccccc 2460

acctctttgc ctgagaataa tatgtctgat gtgtcgcagt taaagagcga ggagtccagt 2520

gacgatgagt ctcatccctt acgagaaggg gcatgttctt cacagtccca ggaaagtgga 2580

gaattctcac tgcagagttc aaatgcatca aagctttctc agtgctctag taaggactct 2640

gattcagagg aatctgattg caatattaag ttacttgaca gtcaaagtga ccagacctcc 2700

aagctatgtt tatctcattt ctcaaaaaaa gacacacctc taaggaacaa ggttcctggg 2760

ctatataagt ccagttctgc agactctctt tctacaacca agatcaaacc tctaggacct 2820

gccagagcca gtgggctgag caagaagccg gcaagcatcc agaagagaaa gcatcataat 2880

gccgagaaca agccggggtt acagatcaaa ctcaatgagc tctggaaaaa ctttggattt 2940

aaaaaagatt ctgaaaagct tcctccttgt aagaaacccc tgtccccagt cagagataac 3000

atccaactaa ctccagaagc ggaagaggat atatttaaca aacctgaatg tggccgtgtt 3060

caaagagcaa tattccagta aatgcagact gctgcaaagc ttttgcctgc aagagaatct 3120

gatcaatttg aagtccctgt ttgggaatga ggcacttatc agcatgaaga attttttctc 3180

attctgtgcc attttaaaaa tagaatacat tttgtatatt aactttaaaa aaaaaaaaaa 3240

aaaaa 3245

<210> 92
<211> 728
<212> DNA
<213> Homo sapiens

<400> 92
   gcggcctcag atgaatgcgg ctgttaagac ctgcaataat ccagaatggc tactctgatc 60
   tatgttgata aggaaaatgg agaaccaggc acccgtgtgg ttgctaagga tgggctgaag 120
   ctggggtctg gaccttcaat caaagcctta gatgggagat ctcaagtttc aacaccacgt 180
   tttggcaaaa cgttcgatgc cccaccagcc ttacctaaag ctactagaaa ggctttggga 240
   actgtcaaca gagctacaga aaagtctgta aagaccaagg gacccctcaa acaaaaacag 300
   ccaagctttt ctgccaaaaa gatgactgag aagactgtta aagcaaaaag ctctgttcct 360
   gcctcagatg atgcctatcc agaaatagaa aaattctttc ccttcaatcc tctagacttt 420
   gagagttttg acctgcctga agagcaccag attgcgcacc tccccttgag tggagtgcct 480
   ctcatgatcc ttgacgagga gagagagctt gaaaagctgt ttcagctggg ccccccttca 540
   cctgtgaaga tgccctctcc accatgggaa tccaatctgt tgcagtctcc ttcaagcatt 600
   ctgtcgaccc tggatgttga attgccacct gtttgctgtg acatagatat ttaaatttct 660
   tagtgcttca gagtttgtgt gtatttgtat taataaagca ttcttcaaca gaaaaaaaaa 720
   aaaaaaaa 728

<210> 93
<211> 1783
<212> DNA
<213> Homo sapiens

<400> 93
gttttggcgt ccgtttgctg cggtaggaac atggcggatc ggctcacgca gcttcaggac 60

gctgtgaatt cgcttgcaga tcagttttgt aatgccattg gagtattgca gcaatgtggt 120

cctcctgcct ctttcaataa tattcagaca gcaattaaca aagaccagcc agctaaccct 180

acagaagagt atgcccagct ttttgcagca ctgattgcac gaacagcaaa agacattgat 240

gttttgatag attccttacc cagtgaagaa tctacagctg ctttacaggc tgctagcttg 300

tataagctag aagaagaaaa ccatgaagct gctacatgtc tggaggatgt tgtttatcga 360

ggagacatgc ttctggagaa gatacaaagc gcacttgctg atattgcaca gtcacagctg 420

aagacaagaa gtggtaccca tagccagtct cttccagact catagcatca gtggatacca 480

tgtggctgag aaaagaactg tttgagtgcc attaagaatt ctgcatcaga cttagataca 540

agccttacca acaattacag aaacattaaa cactatgaca cattaccttt ttagctattt 600

ttaatagtct tctattttca ctcttgataa gcttataaaa tcatgattga atcagcttta 660

aagcatcata ccatcatttt ttaactgagt gaaattatta aggcatgtaa tacattaatg 720

aacataatat aaggaaacat atgtaaaatt ctgttatgac ataatttatg tctccatttt 780

gttgtattgg ccagtacttt tacaaatcaa aacatctctc aagccaaagg agaagacagt 840

aagaacagac ataagggaca ttttagttta ggctagtgtc ctgcctctta gaggtggcat 900

tgtgtaaatc tgagctttga gcaagaaagt tttggaaatt gtgttgcttt taagaaatag 960

agttagtgtg gctggataag aaagtcacat ttatgcaaat gtttcttctg ctagaacctc 1020

atacctgttc tagttcatct ccacgtttat tttaccaaga gatcctctgt caagagaatt 1080

tcctggctgt tgtgaaagaa tttttctaca tcctgaacta tttttcctat ttcttttcac 1140

cttgcttttt ttcatcctta atttgcttgt catcacaatg aagtatgttt tttgaatcat 1200

caattctttc tcattctcca tttatctgaa ggttcttttg cccttattaa cctctcaact 1260

tttattgctg tattctagtc agaccagaac atacttccac tacatcagtt acttggcatc 1320

atttcacctc agtttattat tgccaaataa taatttgtgt cagcattttc aactatggtt 1380

attcatccaa cccttggatc tctttgagtc tactgataat ctccactgga aaggtggaat 1440

tgaaatgtgg tccacatttt aactagctat ttcctggggc tgtatttttc agaatatgct 1500

gagctacatt tgctgcaatc tgttgctatt cagagtttaa gtttcaggag aaaacaggaa 1560

caatagaaca ctctgcctgt tatttttgtt gtaatcaagc ttttccacag ttcttgaaaa 1620

gtactatgtt tcaaatttca ggaacaccag cgttagctgt aaaagttgca gcaatttatt 1680

ggctagtcat agaaaatttt tgaactttta actgtatttt aattgatgtt tattaaaaac 1740

actttgctat caggaaaaaa aaaaaaaaaa aaaaaaaaaa aaa 1783

<210> 94
<211> 3324
<212> DNA
<213> Homo sapiens

<400> 94
gcagagcacc gcgccttagc cgcgaagttc tagttcttgc tgccggtcct aacgtcccgc 60

agtcttcgcc agccagccgt cccgcatgcg cgtttgggcg gcgtggagcc tgctgccatg 120

aagtcagcga gagctaagac accccggaaa cctaccgtga aaaaagggtc ccaaacgaac 180

cttaaagacc cagttggggt atactgtagg gtgcgcccac tgggctttcc tgatcaagag 240

tgttgcatag aagtgatcaa taatacaact gttcagcttc atactcctga gggctacaga 300

ctcaaccgaa atggagacta taaggagact cagtattcat ttaaacaagt atttggcact 360

cacaccaccc agaaggaact ctttgatgtt gtggctaatc ccttggtcaa tgacctcatt 420

catggcaaaa atggtcttct ttttacatat ggtgtgacgg gaagtggaaa aactcacaca 480

atgactggtt ctccagggga aggagggctg cttcctcgtt gtttggacat gatctttaac 540

agtatagggt catttcaagc taaacgatat gttttcaaat ctaatgatag gaatagtatg 600

gatatacagt gtgaggttga tgccttatta gaacgtcaga aaagagaagc tatgcccaat 660

ccaaagactt cttctagcaa acgacaagta gatccagagt ttgcagatat gataactgta 720

caagaattct gcaaagcaga agaggttgat gaagatagtg tctatggtgt atttgtctct 780

tatattgaaa tatataataa ttacatatat gatctattgg aagaggtgcc gtttgatccc 840

ataaaaccca aacctccaca atctaaattg cttcgtgaag ataagaacca taacatgtat 900

gttgcaggat gtacagaagt tgaagtgaaa tctactgagg aggcttttga agttttctgg 960

agaggccaga aaaagagacg tattgctaat acccatttga atcgtgagtc cagccgttcc 1020

catagcgtgt tcaacattaa attagttcag gctcccttgg atgcagatgg agacaatgtc 1080

ttacaggaaa aagaacaaat cactataagt cagttgtcct tggtagatct tgctggaagt 1140

gaaagaacta accggaccag agcagaaggg aacagattac gtgaagctgg taatattaat 1200

cagtcactaa tgacgctaag aacatgtatg gatgtcctaa gagagaacca aatgtatgga 1260

actaacaaga tggttccata tcgagattca aagttaaccc atctgttcaa gaactacttt 1320

gatggggaag gaaaagtgcg gatgatcgtg tgtgtgaacc ccaaggctga agattatgaa 1380

gaaaacttgc aagtcatgag atttgcggaa gtgactcaag aagttgaagt agcaagacct 1440

gtagacaagg caatatgtgg tttaacgcct gggaggagat acagaaacca gcctcgaggt 1500

ccagttggaa atgaaccatt ggttactgac gtggttttgc agagttttcc acctttgccg 1560

tcatgcgaaa ttttggatat caacgatgag cagacacttc caaggctgat tgaagcctta 1620

gagaaacgac ataacttacg acaaatgatg attgatgagt ttaacaaaca atctaatgct 1680

tttaaagctt tgttacaaga atttgacaat gctgttttaa gtaaagaaaa ccacatgcaa 1740

gggaaactaa atgaaaagga gaagatgatc tcaggacaga aattggaaat agaacgactg 1800

gaaaagaaaa acaaaacttt agaatataag attgagattt tagagaaaac aactactatc 1860

tatgaggaag ataaacgcaa tttgcaacag gaacttgaaa ctcagaacca gaaacttcag 1920

cgacagtttt ctgacaaacg cagattagaa gccaggttgc aaggcatggt gacagaaacg 1980

acaatgaagt gggagaaaga atgtgagcgt agagtggcag ccaaacagct ggagatgcag 2040

aataaactct gggttaaaga tgaaaagctg aaacaactga aggctattgt tactgaacct 2100

aaaactgaga agccagagag accctctcgg gagcgagatc gagaaaaagt tactcaaaga 2160

tctgtttctc catcacctgt gcctttactc tttcaacctg atcagaacgc accaccaatt 2220

cgtctccgac acagacgatc acgctctgca ggagacagat gggtagatca taagcccgcc 2280

tctaacatgc aaactgaaac agtcatgcag ccacatgtcc ctcatgccat cacagtatct 2340

gttgcaaatg aaaaggcact agctaagtgt gagaagtaca tgctgaccca ccaggaacta 2400

gcctccgatg gggagattga aactaaacta attaagggtg atatttataa aacaaggggt 2460

ggtggacaat ctgttcagtt tactgatatt gagactttaa agcaagaatc accaaatggt 2520

agtcgaaaac gaagatcttc cacagtagca cctgcccaac cagatggtgc agagtctgaa 2580

tggaccgatg tagaaacaag gtgttctgtg gctgtggaga tgagagcagg atcccagctg 2640

ggacctggat atcagcatca cgcacaaccc aagcgcaaaa agccatgaac tgacagtccc 2700

agtactgaaa gaacattttc atttgtgtgg atgatttctc gaaagccatg ccagaagcag 2760

tcttccaggt catcttgtag aactccagct ttgttgaaaa tcacggacct cagctacatc 2820

atacactgac ccagagcaaa gctttcccta tggttccaaa gacaactagt attcaacaaa 2880

ccttgtatag tatatgtttt gccatattta atattaatag cagaggaaga ctcctttttt 2940

catcactgta tgaatttttt ataatgtttt tttaaaatat atttcatgta tacttataaa 3000

ctaattcaca caagtgtttg tcttagatga ttaaggaaga ctatatctag atcatgtctg 3060

attttttatt gtgacttctc cagccctggt ctgaatttct taaggtttta taaacaaatg 3120

ctgctattta ttagctgcaa gaatgcactt tagaactatt tgacaattca gactttcaaa 3180

ataaagatgt aaatgactgg ccaataataa ccattttagg aaggtgtttt gaattctgta 3240

tgtatatatt cactttctga catttagata tgccaaaaga attaaaatca aaagcactaa 3300

gaaataaaaa aaaaaaaaaa aaaa                                          3324

<210>  95
<211>  3636
<212>  DNA
<213>  Homo sapiens

<400>  95
gcagagcacc gcgccttagc cgcgaagttc tagttcttgc tgccggtcct aacgtcccgc      60

agtcttcgcc agccagccgt cccgcatgcg cgtttgggcg gcgtggagcc tgctgccatg     120

aagtcagcga gagctaagac accccggaaa cctaccgtga aaaaagggtc ccaaacgaac     180

cttaaagacc cagttggggt atactgtagg gtgcgcccac tgggctttcc tgatcaagag     240

tgttgcatag aagtgatcaa taatacaact gttcagcttc atactcctga gggctacaga     300

ctcaaccgaa atggagacta taaggagact cagtattcat ttaaacaagt atttggcact     360

cacaccaccc agaaggaact ctttgatgtt gtggctaatc ccttggtcaa tgacctcatt     420

catggcaaaa atggtcttct ttttacatat ggtgtgacgg gaagtggaaa aactcacaca     480

atgactggtt ctccagggga aggagggctg cttcctcgtt gtttggacat gatctttaac     540

agtatagggt catttcaagc taaacgatat gttttcaaat ctaatgatag gaatagtatg     600

gatatacagt gtgaggttga tgccttatta gaacgtcaga aaagagaagc tatgcccaat     660

ccaaagactt cttctagcaa acgacaagta gatccagagt ttgcagatat gataactgta     720

caagaattct gcaaagcaga agaggttgat gaagatagtg tctatggtgt atttgtctct     780

tatattgaaa tatataataa ttacatatat gatctattgg aagaggtgcc gtttgatccc     840

ataaaaccca aacctccaca atctaaattg cttcgtgaag ataagaacca taacatgtat     900

gttgcaggat gtacagaagt tgaagtgaaa tctactgagg aggcttttga agttttctgg     960

agaggccaga aaaagagacg tattgctaat acccatttga atcgtgagtc cagccgttcc    1020

catagcgtgt tcaacattaa attagttcag gctcccttgg atgcagatgg agacaatgtc    1080

ttacaggaaa aagaacaaat cactataagt cagttgtcct tggtagatct tgctggaagt    1140

gaaagaacta accggaccag agcagaaggg aacagattac gtgaagctgg taatattaat    1200

cagtcactaa tgacgctaag aacatgtatg gatgtcctaa gagagaacca aatgtatgga    1260

actaacaaga tggttccata tcgagattca aagttaaccc atctgttcaa gaactacttt    1320

gatggggaag gaaaagtgcg gatgatcgtg tgtgtgaacc ccaaggctga agattatgaa    1380

gaaaacttgc aagtcatgag atttgcggaa gtgactcaag aagttgaagt agcaagacct    1440

gtagacaagg caatatgtgg tttaacgcct gggaggagat acagaaacca gcctcgaggt    1500

ccagttggaa atgaaccatt ggttactgac gtggttttgc agagttttcc acctttgccg    1560

tcatgcgaaa ttttggatat caacgatgag cagacacttc caaggctgat tgaagcctta    1620

gagaaacgac ataacttacg acaaatgatg attgatgagt ttaacaaaca atctaatgct    1680

tttaaagctt tgttacaaga atttgacaat gctgttttaa gtaaagaaaa ccacatgcaa    1740

gggaaactaa atgaaaagga gaagatgatc tcaggacaga aattggaaat agaacgactg    1800

gaaaagaaaa acaaaacttt agaatataag attgagattt tagagaaaac aactactatc    1860

tatgaggaag ataaacgcaa tttgcaacag gaacttgaaa ctcagaacca gaaacttcag 1920

cgacagtttt ctgacaaacg cagattagaa gccaggttgc aaggcatggt gacagaaacg 1980

acaatgaagt gggagaaaga atgtgagcgt agagtggcag ccaaacagct ggagatgcag 2040

aataaactct gggttaaaga tgaaaagctg aaacaactga aggctattgt tactgaacct 2100

aaaactgaga agccagagag accctctcgg gagcgagatc gagaaaaagt tactcaaaga 2160

tctgtttctc catcacctgt gcctctttct agtaactata ttgctcagat ttccaacggc 2220

cagcaactca tgagccagcc acagctacat aggcgctcta actcttgcag cagcatttct 2280

gtagcttcct gtatttcgga atgggagcag aaaattccta cgtacaacac acctctcaaa 2340

gtcacatcta ttgcaaggcg taggcagcag gagccaggac aaagcaaaac ttgtatcgtg 2400

tcagacagaa ggcgagggat gtactggact gaaggcaggg aggtggttcc tacattcaga 2460

aatgagatag aaatagaaga ggatcattgc ggcaggttac tctttcaacc tgatcagaac 2520

gcaccaccaa ttcgtctccg acacagacga tcacgctctg caggagacag atgggtagat 2580

cataagcccg cctctaacat gcaaactgaa acagtcatgc agccacatgt ccctcatgcc 2640

atcacagtat ctgttgcaaa tgaaaaggca ctagctaagt gtgagaagta catgctgacc 2700

caccaggaac tagcctccga tggggagatt gaaactaaac taattaaggg tgatatttat 2760

aaaacaaggg gtggtggaca atctgttcag tttactgata ttgagacttt aaagcaagaa 2820

tcaccaaatg gtagtcgaaa acgaagatct tccacagtag cacctgccca accagatggt 2880

gcagagtctg aatggaccga tgtagaaaca aggtgttctg tggctgtgga gatgagagca 2940

ggatcccagc tgggacctgg atatcagcat cacgcacaac ccaagcgcaa aaagccatga 3000

actgacagtc ccagtactga aagaacattt tcatttgtgt ggatgatttc tcgaaagcca 3060

tgccagaagc agtcttccag gtcatcttgt agaactccag ctttgttgaa aatcacggac 3120

ctcagctaca tcatacactg acccagagca aagctttccc tatggttcca aagacaacta 3180

gtattcaaca aaccttgtat agtatatgtt ttgccatatt taatattaat agcagaggaa 3240

gactcctttt ttcatcactg tatgaatttt ttataatgtt tttttaaaat atatttcatg 3300

tatacttata aactaattca cacaagtgtt tgtcttagat gattaaggaa gactatatct 3360

agatcatgtc tgatttttta ttgtgacttc tccagccctg gtctgaattt cttaaggttt 3420

tataaacaaa tgctgctatt tattagctgc aagaatgcac tttagaacta tttgacaatt 3480

cagactttca aaataaagat gtaaatgact ggccaataat aaccatttta ggaaggtgtt 3540

ttgaattctg tatgtatata ttcactttct gacatttaga tatgccaaaa gaattaaaat 3600

caaaagcact aagaaataaa aaaaaaaaaa aaaaaa 3636

<210> 96
<211> 6552
<212> DNA
<213> Homo sapiens

<400> 96
atgaggagct tcaaaagagt caactttggg actctgctaa gcagccagaa ggaggctgaa 60

gagttgctgc ccgacttgaa ggagttcctg tccaaccctc cagctggttt tcccagcagc 120
cgatctgatg ctgagaggag acaagcttgt gatgccatcc tgagggcttg caaccagcag 180
ctgactgcta agctagcttg ccctaggcat ctggggagcc tgctggagct ggcagagctg 240
gcctgtgatg gctacttagt gtctacccca cagcgtcctc ccctctacct ggaacgaatt 300
ctctttgtct tactgcggaa tgctgctgca caaggaagcc cagaggccac actccgcctt 360
gctcagcccc tccatgcctg cttggtgcag tgctctcgcg aggctgctcc ccaggactat 420
gaggccgtgg ctcggggcag cttttctctg ctttggaagg gggcagaagc cctgttggaa 480
cggcgagctg catttgcagc tcggctgaag gccttgagct tcctagtact cttggaggat 540
gaaagtaccc cttgtgaggt tcctcacttt gcttctccaa cagcctgtcg agcggtagct 600
gcccatcagc tatttgatgc cagtggccat ggtctaaatg aagcagatgc tgatttccta 660
gatgacctgc tctccaggca cgtgatcaga gccttggtgg gtgagagagg gagctcttct 720
gggcttcttt ctccccagag ggccctctgc ctcttggagc tcaccttgga acactgccgt 780
cgcttttgct ggagccgcca ccatgacaaa gccatcagcg cagtggagaa ggctcacagt 840
tacctaagga acaccaatct agcccctagc cttcagctat gtcagctggg ggttaagctg 900
ctgcaggttg gggaggaagg acctcaggca gtggccaagc ttctgatcaa ggcatcagct 960
gtcctgagca agagtatgga ggcaccatca cccccacttc gggcattgta tgagagctgc 1020
cagttcttcc tttcaggcct ggaacgaggc accaagaggc gctatagact tgatgccatt 1080
ctgagcctct ttgcttttct tggagggtac tgctctcttc tgcagcagct gcgggatgat 1140
ggtgtgtatg ggggctcctc caagcaacag cagtcttttc ttcagatgta ctttcaggga 1200
cttcacctct acactgtggt ggtttatgac tttgcccaag gctgtcagat agttgatttg 1260
gctgacctga cccaactagt ggacagttgt aaatctaccg ttgtctggat gctggaggcc 1320
ttagagggcc tgtcgggcca agagctgacg gaccacatgg ggatgaccgc ttcttacacc 1380
agtaatttgg cctacagctt ctatagtcac aagctctatg ccgaggcctg tgccatctct 1440
gagccgctct gtcagcacct gggtttggtg aagccaggca cttatcccga ggtgcctcct 1500
gagaagttgc acaggtgctt ccggctacaa gtagagagtt tgaagaaact gggtaaacag 1560
gcccagggct gcaagatggt gattttgtgg ctggcagccc tgcaaccctg tagccctgaa 1620
cacatggctg agccagtcac tttctgggtt cgggtcaaga tggatgcggc cagggctgga 1680
gacaaggagc tacagctaaa gactctgcga gacagcctca gtggctggga cccggagacc 1740
ctggccctcc tgctgaggga ggagctgcag gcctacaagg cggtgcgggc cgacactgga 1800
caggaacgct tcaacatcat ctgtgacctc ctggagctga gccccgagga gacaccagcc 1860
ggggcctggg cacgagccac ccacctggta gaactggctc aggtgctctg ctaccacgac 1920
tttacgcagc agaccaactg ctctgctctg gatgctatcc gggaagccct gcagcttctg 1980
gactctgtga ggcctgaggc ccaggccaga gatcagcttc tggacgataa agcacaggcc 2040
ttgctgtggc tttacatctg tactctggaa gccaaaatac aggaaggtat cgagcgggat 2100
cggagagccc aggcccctgg taacttggag gaatttgaag tcaatgacct gaactatgaa 2160

gataaactcc aggaagatcg tttcctatac agtaacattg ccttcaacct ggctgcagat 2220

gctgctcagt ccaaatgcct ggaccaagcc ctggccctgt ggaaggagct gcttacaaag 2280

gggcaggccc cagctgtacg gtgtctccag cagacagcag cctcactgca gatcctagca 2340

gccctctacc agctggtggc aaagcccatg caggctctgg aggtcctcct gctgctacgg 2400

attgtctctg agagactgaa ggaccactcg aaggcagctg gctcctcctg ccacatcacc 2460

cagctcctcc tgaccctcgg ctgtcccagc tatgcccagt tacacctgga agaggcagca 2520

tcgagcctga agcatctcga tcagactact gacacatacc tgctcctttc cctgacctgt 2580

gatctgcttc gaagtcaact ctactggact caccagaagg tgaccaaggg tgtctctctg 2640

ctgctgtctg tgcttcggga tcctgccctc cagaagtcct ccaaggcttg gtacttgctg 2700

cgtgtccagg tcctgcagct ggtggcagct taccttagcc tcccgtcaaa caacctctca 2760

cactccctgt gggagcagct ctgtgcccaa ggctggcaga cacctgagat agctctcata 2820

gactcccata agctcctccg aagcatcatc ctcctgctga tgggcagtga cattctctca 2880

actcagaaag cagctgtgga gacatcgttt ttggactatg gtgaaaatct ggtacaaaaa 2940

tggcaggttc tttcagaggt gctgagctgc tcagagaagc tggtctgcca cctgggccgc 3000

ctgggtagtg tgagtgaagc caaggccttt tgcttggagg ccctaaaact tacaacaaag 3060

ctgcagatac cacgccagtg tgccctgttc ctggtgctga agggcgagct ggagctggcc 3120

cgcaatgaca ttgatctctg tcagtcggac ctgcagcagg ttctgttctt gcttgagtct 3180

tgcacagagt ttggtggggt gactcagcac ctggactctg tgaagaaggt ccacctgcag 3240

aaggggaagc agcaggccca ggtcccctgt cctccacagc tcccagagga ggagctcttc 3300

ctaagaggcc ctgctctaga gctggtggcc actgtggcca aggagcctgg ccccatagca 3360

ccttctacaa actcctcccc agtcttgaaa accaagcccc agcccatacc caacttcctg 3420

tcccattcac ccacctgtga ctgctcgctc tgcgccagcc ctgtcctcac agcagtctgt 3480

ctgcgctggg tattggtcac ggcaggggtg aggctggcca tgggccacca agcccagggt 3540

ctggatctgc tgcaggtcgt gctgaagggc tgtcctgaag ccgctgagcg cctcacccaa 3600

gctctccaag cttccctgaa tcataaaaca cccccctcct tggttccaag cctcttggat 3660

gagatcttgg ctcaagcata cacactgttg gcactggagg gcctgaacca gccatcaaac 3720

gagagcctgc agaaggttct acagtcaggg ctgaagtttg tagcagcacg gataccccac 3780

ctagagccct ggcgagccag cctgctcttg atttgggccc tcacaaaact aggtggcctc 3840

agctgctgta ctacccaact ttttgcaagc tcctggggct ggcagccacc attaataaaa 3900

agtgtccctg gctcagagcc ctctaagact cagggccaaa aacgttctgg acgagggcgc 3960

caaaagttag cctctgctcc cctgcgcctc aataatacct ctcagaaagg tctggaaggt 4020

agaggactgc cctgcacacc taaaccccca gaccggatca ggcaagctgg ccctcatgtc 4080

cccttcacgg tgtttgagga agtctgccct acagagagca agcctgaagt accccaggcc 4140

cccagggtac aacagagagt ccagacgcgc ctcaaggtga acttcagtga tgacagtgac 4200

ttggaagacc ctgtctcagc tgaggcctgg ctggcagagg agcctaagag acggggcact 4260

gcttcccggg gccgggggcg agcaaggaag ggcctgagcc taaagacgga tgccgtggtt 4320

gccccaggta gtgcccctgg gaaccctggc ctgaatggca ggagccggag ggccaagaag 4380

gtggcatcaa gacattgtga ggagcggcgt ccccagaggg ccagtgacca ggccaggcct 4440

ggccctgaga tcatgaggac catccctgag gaagaactga ctgacaactg gagaaaaatg 4500

agctttgaga tcctcagggg ctctgacggg gaagactcag cctcaggtgg gaagactcca 4560

gctccgggcc ctgaggcagc ttctggagaa tgggagctgc tgaggctgga ttccagcaag 4620

aagaagctgc ccagcccatg cccagacaag gagagtgaca aggaccttgg tcctcggctc 4680

cagctcccct cagcccccgt agccactggt ctttctaccc tggactccat ctgtgactcc 4740

ctgagtgttg ctttccgggg cattagtcac tgtcctccta gtgggctcta tgcccacctc 4800

tgccgcttcc tggccttgtg cctgggccac cgggatcctt atgccactgc tttccttgtc 4860

accgagtctg tctccatcac ctgtcgccac cagctgctca cccacctcca cagacagctc 4920

agcaaggccc agaagcaccg aggatcactt gaaatagcag accagctgca ggggctgagc 4980

cttcaggaga tgcctggaga tgtccccctg gcccgcatcc agcgcctctt ttccttcagg 5040

gctttggaat ctggccactt cccccagcct gaaaaggaga gtttccagga gcgcctggct 5100

ctgatcccca gtggggtgac tgtgtgtgtg ttggccctgg ccaccctcca gcccggaacc 5160

gtgggcaaca ccctcctgct gacccggctg gaaaaggaca gtcccccagt cagtgtgcag 5220

attcccactg gccagaacaa gcttcatctg cgttcagtcc tgaatgagtt tgatgccatc 5280

cagaaggcac agaaagagaa cagcagctgt actgacaagc gagaatggtg gacagggcgg 5340

ctggcactgg accacaggat ggaggttctc atcgcttccc tagagaagtc tgtgctgggc 5400

tgctggaagg ggctgctgct gccgtccagt gaggagcccg gccctgccca ggaggcctcc 5460

cgcctacagg agctgctaca ggactgtggc tggaaatatc ctgaccgcac tctgctgaaa 5520

atcatgctca gtggtgccgg tgccctcacc cctcaggaca ttcaggccct ggcctacggg 5580

ctgtgcccaa cccagccaga gcgagcccag gagctcctga atgaggcagt aggacgtcta 5640

cagggcctga cagtaccaag caatagccac cttgtcttgg tcctagacaa ggacttgcag 5700

aagctgccgt gggaaagcat gcccagcctc caagcactgc ctgtcacccg gctgccctcc 5760

ttccgcttcc tactcagcta ctccatcatc aaagagtatg gggcctcgcc agtgctgagt 5820

caaggggtgg atccacgaag taccttctat gtcctgaacc ctcacaataa cctgtcaagc 5880

acagaggagc aatttcgagc caatttcagc agtgaagctg gctggagagg agtggttggg 5940

gaggtgccaa gacctgaaca ggtgcaggaa gccctgacaa agcatgattt gtatatctat 6000

gcagggcatg gggctggtgc ccgcttcctt gatgggcagg ctgtcctgcg gctgagctgt 6060

cgggcagtgg ccctgctgtt tggctgtagc agtgcggccc tggctgtgca tggaaacctg 6120

gagggggctg gcatcgtgct caagtacatc atggctggtt gccccttgtt tctgggtaat 6180

ctctgggatg tgactgaccg cgacattgac cgctacacgg aagctctgct gcaaggctgg 6240

cttggagcag gcccaggggc cccccttctc tactatgtaa accaggcccg ccaagctccc 6300

cgactcaagt atcttattgg ggctgcacct atagcctatg gcttgcctgt ctctctgcgg 6360

taaccccatg gagctgtctt attgatgcta gaagcctcat aactgttcta cctccaaggt 6420

tagatttaat ccttaggata actcttttaa agtgattttc cccagtgttt tatatgaaac 6480

atttcctttt gatttaacct cagtataata aagatacatc atttaaaccc tgaaaaaaaa 6540

aaaaaaaaaa aa 6552

<210> 97
<211> 6972
<212> DNA
<213> Homo sapiens

<400> 97
ggcatggaac ctaaagacta gaggcggttg tgtgagtcag gaagaggggc cagatatctg 60

agtgttcctc tttagtttct tcaattgcag ataatatggt gtctaatttt atgttgttca 120

ggaaagacag tggttcctga ctcaggaaga cagtctcaga aacatgtgga atgatattga 180

gctgctaaca aatgatgata ccggaagtgg gtacctgagt gtcggttcaa gaaaagaaca 240

tggaactgct ttatatcaag tagatttgct agtgaagatc tcttctgaaa aggcctcatt 300

aaatccaaag atacaggcat gcagcttaag tgatgggttt attattgtag ccgaccaatc 360

agtgatattg cttgacagta tttgtagatc acttcaattg catcttgtct ttgatactga 420

agtggatgta gttggccttt gtcaagaagg aaagtttctt ttggttggcg agagaagtgg 480

caacctacat cttattcatg taacatcaaa acaaacacta ctcactaatg catttgttca 540

gaaagctaac gatgaaaatc ggcggactta ccagaatctt gtcattgaga aggatggttc 600

aaatgaaggt acctattata tgctacttct tacatacagt ggattttttt gtattacaaa 660

ccttcagctt ttaaaaattc aacaagcaat tgagaatgta gacttcagta cagcaaaaaa 720

gttacaagga caaatcaagt ccagttttat ttctactgaa aattatcata ctcttggttg 780

tctcagtctt gtggctggag atttagcaag tgaagttcct gtgataattg ggggaaccgg 840

taattgtgca ttctcaaaat gggaaccaga ttcttccaag aaaggaatga cagttaagaa 900

ccttattgat gcagagatta ttaaaggtgc aaagaagttc cagctgatag acaatctact 960

ttttgttctt gatactgata acgtgctgag tttatgggat atttacactc taactcctgt 1020

atggaactgg ccctctcttc acgtagaaga gtttcttctt actacagaag cagactctcc 1080

ttcatcagtc acgtggcaag gaattacaaa tctcaaatta atagctctga cagcttcagc 1140

taataagaag atgaaaaacc tcatggttta ttcattacct acaatggaaa tactatattc 1200

tttggaagta tctagtgttt cttctctggt ccaaacagga attagcacag ataccatata 1260

ccttttagaa ggagtttgca aaaatgatcc aaaattgtct gaagactcag tctctgtgtt 1320

agtactcaga tgtcttacgg aagctttacc agaaaacaga ttgagtcggt tacttcacaa 1380

acacagattt gctgaagctg agagttttgc cattcagttt ggactagatg ttgagcttgt 1440

ttacaaggtc aagtcaaatc atatattgga gaaactggca ttgagttctg tggatgccag 1500

tgaacagacc gaatggcaac aacttgtaga cgacgctaag gaaaatctac ataagatcca 1560

ggatgatgaa tttgtggtga attactgcct gaaagctcag tggataacct atgaaaccac 1620

tcaagagatg ctgaattatg ccaaaaccag gcttttgaag aaagaagata aaactgctct 1680

catttattct gatggcttga aagaggtgct aagagctcat gcaaaattga ctacttttta 1740

tggagcattt ggaccagaaa aattcagtgg cagttcttgg attgaatttc taaataatga 1800

agatgatctt aaagatattt ttttacagct aaaagaagga aaccttgttt gtgcacagta 1860

tctttggctt cgacatcggg caaactttga aagcagattt gatgtgaaaa tgctggagag 1920

cttgctcaac tcaatgtctg catcagtctc tttgcaaaag ctgtgtccat ggtttaaaaa 1980

tgatgtgatt ccatttgtaa gaaggactgt gcctgaagga cagataattc ttgcaaaatg 2040

gttggaacaa gcagccagga accttgaatt aactgataag gcaaattggc cagaaaatgg 2100

acttcaattg gcagagatat tttttacagc agaaaaaaca gacgagttgg gattggcatc 2160

ttcctggcat tggatttcct tgaaagatta tcagaacaca gaggaagtat gtcagctaag 2220

gactttggta aataacttgc gagagttgat cacgttgcat aggaagtaca actgcaaatt 2280

agccctctct gattttgaga aggaaaatac aaccaccata gtgttccgaa tgtttgataa 2340

agtgctggcc ccagagctta ttccctccat cttagagaag tttataagag tttacatgag 2400

agaacatgac ttgcaagagg aggaacttct cttgctgtac atagaggatt tactgaatag 2460

atgcagctca aagtccacat cactctttga aacagcatgg gaagcaaagg ccatggcagt 2520

aatagcgtgt ttatctgaca cggacctcat atttgatgcc gtgctcaaga tcatgtatgc 2580

ggcagtggtt ccttggagtg cagctgtgga gcaactggtg aaacagcacc tggaaatgga 2640

ccatcccaaa gtcaagttat tacaggaaag ttacaaacta atggagatga aaaaactttt 2700

acgaggctat ggaataagag aggtaaatct cttaaacaag gaaataatga gagtggttag 2760

atacattctc aaacaagatg tcccatcttc tttagaagat gctttaaagg tagcccaagc 2820

gtttatgtta tctgatgatg agatctacag tctaagaatt attgacctga ttgatagaga 2880

acagggtgaa gactgtctcc ttctgttgaa gtctttgcct cctgctgaag ctgagaaaac 2940

tgcagaaaga gtcatcatat gggcacgact ggcattacaa gaagagccag atcattctaa 3000

agagggcaag gcctggagaa tgtctgtagc gaagacatcc gtggacattc ttaagatact 3060

atgtgacatt cagaaagaca atctgcagaa gaaggacgaa tgtgaagaaa tgttgaaact 3120

atttaaagag gttgctagct tacaggagaa ctttgaggtc tttctttcat ttgaagatta 3180

tagcaatagt tccctggtag cagatctccg tgagcagcac attaaagctc acgaagttgc 3240

acaggcgaaa cacaaacctg ggagcacccc agagcccata gctgctgagg tgaggagccc 3300

aagcatggaa tcaaagctgc acagacaggc actggccctg cagatgtcca aacaagagct 3360

ggaggcagag ctgaccttga gagccttaaa agatgggaac atcaaaacag cactgaaaaa 3420

atgcagcgac ttgtttaagt atcactgcaa tgctgacact gggaaattgc tatttctgac 3480

atgtcagaag ctttgtcaga tgttggctga taatgtccca gtgacagtgc ctgtgggact 3540

gaatcttcct tccatgatac atgatctagc aagccaagct gccaccattt gcagtccaga 3600

ttttttacta gatgctttag aactatgtaa acatacttta atggctgtag agctttccag 3660

acaatgccaa atggatgact gtggaatcct catgaaagct tcttttggga cacataaaga 3720

tccatatgaa gagtggtctt acagtgactt cttcagtgaa gatggaattg ttcttgagtc 3780

acagatggtg cttccagtga tttatgaact gatttcatct cttgtgcctc tagctgaaag 3840

caagagatat cccttggagt ctaccagttt gccatactgc tcccttaatg aaggagatgg 3900

ccttgtttta cctgttataa attccatctc tgccctgctt cagaatcttc aggaatctag 3960

ccagtgggag ctagccctaa gatttgtggt tggttcattt ggtacctgtc ttcagcactc 4020

tgtgtcaaac ttcatgaatg ccactttgag tgaaaagtta tttggagaga ctacattagt 4080

taaatcaagg catgttgtta tggaattgaa agaaaaagct gttatattta tcagggaaaa 4140

tgctacaaca ctactgcaca aagtatttaa ttgtcgcttg gtagatcttg acctggcgtt 4200

gggttactgc actctcttac ctcaaaaaga tgtgtttgaa aatctctgga agctcataga 4260

taaagcatgg cagaattacg acaaaatctt ggcaatatct ctggtgggct ctgagctggc 4320

aagtctctat caggaaatag aaatggggct taagttccgt gaactcagta ctgatgccca 4380

gtggggcatt cgtcttggta aacttggtat ttcttttcaa ccagttttca ggcaacattt 4440

tctcaccaag aaagacctca ttaaagctct tgtggagaat atagatatgg acacaagcct 4500

cattttggaa tattgcagca catttcagtt ggactgcgat gcagttcttc agctcttcat 4560

tgaaacgctg ctccacaaca caaatgccgg ccaaggccag ggagatgcaa gcatggactc 4620

tgcaaagcgg cggcatccca aactcctggc caaagccctt gagatggttc ctttactgac 4680

gagcacaaaa gatttggtca tcagtcttag tggaatacta cataagttgg atccttatga 4740

ctatgaaatg attgaagttg tcttgaaagt tatagaacga gctgatgaaa agataaccaa 4800

tattaatatt aatcaggcat tgagtattct gaaacatttg aagtcataca gaagaatttc 4860

tcctcccgtg gatctagaat atcagtatat gttggaacat gtcataactt tgccatcagc 4920

tgcccaaact agactgcctt ttcacctgat attctttggc acagcacaga acttctggaa 4980

aattctctct acagaactca gtgaagaatc tttcccaaca ttgctcttaa tttcgaaatt 5040

aatgaagttc tctctggaca ctctgtacgt gtctacagca aaacacgttt tcgaaaaaaa 5100

actgaagcca aagctcctga agttaacaca agctaaatcc tcaacactga ttaacaagga 5160

aataactaag atcacgcaga ccatcgaatc ctgcttactc tctatagtca acccagagtg 5220

ggctgtagct attgccatca gccttgccca ggatatccct gaaggttcct tcaagatatc 5280

tgctttgaaa ttctgccttt atttagctga gagatggcta cagaatatcc catcgcagga 5340

cgaaaaacgt gaaaaagccg aggctttgtt gaagaagctt catatccagt accggcgatc 5400

gggcacagaa gctgtgctca tagcccacaa gctgaacact gaggaatatt taagagtgat 5460

cggaaagcca gcacatctta ttgtcagtct ctacgaacat cctagcatca atcaaagaat 5520

tcagaattca tctggcacag attatcctga tattcatgca gcagctaaag aaatagccga 5580

agtcaatgaa attaatttgg aaaaagtctg ggacatgttg ttggaaaaat ggctatgccc 5640

ttcaacaaaa cctggtgaaa aaccatcaga attatttgaa cttcaagaag atgaagccct 5700

acgaagagtg cagtatctcc tcctgtctcg tccaattgat tatagttcaa gaatgctgtt 5760

tgtatttgca acatcaacta caaccacatt aggtatgcat cagttaactt ttgcccatag 5820

aactcgagct cttcagtgtc tcttctattt ggctgacaag gaaactatag aatctctctt 5880

taaaaaaccc attgaagaag tgaaatctta tttgagatgt ataacttttc tggcatcatt 5940

tgagactttg aatatcccca tcacatatga attattttgc agcagtccta aagaaggaat 6000

gattaagggt ctgtggaaaa accacagcca cgagtccatg gcagtaagat tggtgactga 6060

gctgtgttta gaatacaaaa tctatgacct gcagctttgg aatggactct tgcaaaagct 6120

tctgggcttc aatatgattc cttatctaag gaaagtttta aaagccatct ccagtatcca 6180

ttctttatgg caggttccct acttcagcaa agcgtggcag cgtgtgatac agataccact 6240

gctttcagcc tcttgtcctt taagtcctga tcagctgtca gattgttctg agagtctcat 6300

cgctgtcctc gaatgtccag tctcaggtga tcttgacctg atcggagtcg ccaggcagta 6360

tatccagtta gaacttccgg cttttgcatt agcttgtctg atgctcatgc cccactcaga 6420

gaaaagacac cagcaaatta agaattttct gggttcctgt gaccctcagg ttattttaaa 6480

gcaattggaa gagcatatga acacgggcca gctagcagga ttttcacatc aaattagaag 6540

tctgattttg aataatatca tcaataagaa ggagtttggg attttggcaa agaccaaata 6600

ctttcaaatg ttgaagatgc atgcgatgaa taccaacaat atcactgagc tagtgaacta 6660

tttggcaaat gacttaagtt tagatgaagc ttcagtcttg ataactgaat attcaaagca 6720

ctgcgggaaa cctgtgcctc cagacactgc tccctgtgaa attctgaaga tgtttcttag 6780

tggattatcg taaatcactg aacctttttt tcaagaagga caagaatttt ggagtctgct 6840

attaatggac catatttatt acagttttta aattgtacaa tctctgtatt atagctattt 6900

gtctaacatt accccacatg taataaataa aacaatatga gcataattgc cccataaaga 6960

actcaaaaaa aa 6972

<210> 98
<211> 2979
<212> DNA
<213> Homo sapiens

<400> 98
agcaaaccaa tcgcaagcct cgttgagtgg aaggggtggg atcttccccg gaagttttgg 60

ttaaagcccc tccaatcagc ggctcggtgc ggcaagtttg aatttcgtgg aggctcgggt 120

tgtgagggtt cctgcttcgg agtcggcggt ggtcgtccag accgagtgtt ctttactttt 180

tgtttggttg aggtttcacg ctagaaggtg gctcaggatg tcttcatcac attttgccag 240

tcgacacagg aaggatataa gtactgaaat gattagaact aaaattgctc ataggaaatc 300

actgtctcag aaagaaaata gacataagga atacgaacga aatagacact ttggtttgaa 360

agatgtaaac attccaacct tggaaggtag aattcttgtt gaattagatg agacatctca 420

agagcttgtt ccagaaaaga ccaatgttaa gccaagggca atgaaaacta ttctaggtga 480

tcaacgaaaa cagatgctcc aaaaatacaa agaagaaaag caacttcaaa aattgaaaga 540

gcagagagag aaagctaaac gaggaatatt taaagtgggt cgttatagac ctgatatgcc 600

ttgttttctt ttatcaaacc agaatgctgt gaaagctgag ccaaaaaagg ctattccatc 660

ttctgtacgg attacaaggt caaaggccaa agaccaaatg gagcagacta agattgataa 720

cgagagtgat gttcgagcaa tccgacctgg tccaagacaa acttctgaaa agaaagtgtc 780

agacaaagag aaaaaagttg tgcagcctgt aatgcccacg tcgttgagaa tgactcgatc 840

agctactcaa gcagcaaagc aggttcccag aacagtctca tctaccacag caagaaagcc 900

agtcacaaga gctgctaatg aaaacgaacc agaaggaaag gtgccaagta aaggaagacc 960

tgccaaaaat gtagaaacaa aacccgacaa gggtatttct tgtaaagtcg atagtgaaga 1020

aaatactttg aattcacaaa ctaatgcaac aagtggaatg aatccagatg gagtcttatc 1080

aaaaatggaa aacttacctg agataaatac tgcaaaaata aaagggaaga attccttcgc 1140

acctaaggat tttatgtttc agccactgga tggtctgaag acctatcaag taacacctat 1200

gactcccaga agtgccaatg cttttttgac acccagttac acctggactc ctttaaaaac 1260

agaagttgat gagtctcaag caacaaaaga aattttggca caaaaatgta aaacttactc 1320

taccaagaca atacagcaag attcaaataa attgccatgt cctttgggtc ctctaactgt 1380

ttggcatgaa gaacatgttt taaataaaaa tgaagctact actaaaaatt taaatggcct 1440

tccaataaaa gaagtcccat cacttgaaag aaatgaaggt cgaattgctc agccccacca 1500

tggtgtgcca tatttcagaa atatcctcca gtcagaaact gagaaattaa cttcacattg 1560

cttcgagtgg gacaggaaac ttgaattgga cattccagat gatgctaaag atcttattcg 1620

cacagcagtt ggtcaaacaa gactccttat gaaggaaagg tttaaacagt ttgaaggact 1680

ggttgatgat tgtgaatata aacgaggtat aaaggagact acctgtacag atctggatgg 1740

attttgggat atggttagtt ttcagataga agatgtaatc cacaaattca acaatctgat 1800

caaacttgag gaatctgggt ggcaagtcaa taataatatg aatcataata tgaacaaaaa 1860

tgtctttagg aaaaaagttg tctcaggtat agcaagtaaa ccaaaacagg atgatgctgg 1920

aagaattgca gcgagaaatc gcctagctgc cataaaaaat gcaatgagag agagaattag 1980

gcaggaagaa tgtgctgaaa cagcagtttc tgtgatacca aaggaagttg ataaaatagt 2040

gttcgatgct ggatttttca gagttgaaag tcctgttaaa ttattctcag gactttctgt 2100

ctcttctgaa ggcccttctc aaagacttgg aacacctaag tctgtcaaca aagctgtatc 2160

tcagagtaga aatgagatgg gcattccaca acaaactaca tcaccagaaa atgccggtcc 2220

tcagaatacg aaaagtgaac atgtgaagaa gactttgttt ttgagtattc ctgaaagcag 2280

gagcagcata gaagatgctc agtgtcctgg attaccagat ttaattgaag aaaaccatgt 2340

tgtaaataag acagacttga aggtggattg tttatccagt gagagaatga gtttgcctct 2400

tcttgctggt ggagtagcag atgatattaa tactaacaaa aaagaaggaa tttcagatgt 2460

tgtggaagga atggaactga attcttcaat tacatcacag gatgttttga tgagtagccc 2520

tgaaaaaaat acagcttcac aaaatagcat cttagaagaa ggggaaacta aaatttctca 2580

gtcagaacta tttgataata aaagtctcac tactgaatgc caccttcttg attcaccagg 2640

tctaaactgc agtaatccat ttactcagct ggagaggaga catcaagaac atgccagaca 2700

catttctttt ggtggtaacc tgattacttt ttcacctcta caaccaggag aattttgaat 2760

ttaaaaataa atccaaacat tttccttcat attatcaatg cttatatatt ccttagacta 2820

ttgaaatttt ggagaaaatg tatttgtgtt cacttctata gcatataatg ttttaatatt 2880

ctgtgttcat caaagtgtat tttagatata ctctttctca agggaagtgg ggatattttg 2940

tacattttca acacagaata aaaaatgtac tgtgccttg 2979

<210> 99
<211> 2501
<212> DNA
<213> Homo sapiens

<400> 99
cgaaaagatt cttaggaacg ccgtaccagc cgcgtctctc aggacagcag gcccctgtcc 60

ttctgtcggg cgccgctcag ccgtgccctc cgcccctcag gttctttttc taattccaaa 120

taaacttgca agaggactat gaaagattat gatgaacttc tcaaatatta tgaattacat 180

gaaactattg ggacaggtgg ctttgcaaag gtcaaacttg cctgccatat ccttactgga 240

gagatggtag ctataaaaat catggataaa aacacactag ggagtgattt gccccggatc 300

aaaacggaga ttgaggcctt gaagaacctg agacatcagc atatatgtca actctaccat 360

gtgctagaga cagccaacaa aatattcatg gttcttgagt actgccctgg aggagagctg 420

tttgactata taatttccca ggatcgcctg tcagaagagg agacccgggt tgtcttccgt 480

cagatagtat ctgctgttgc ttatgtgcac agccagggct atgctcacag ggacctcaag 540

ccagaaaatt tgctgtttga tgaatatcat aaattaaagc tgattgactt tggtctctgt 600

gcaaaaccca agggtaacaa ggattaccat ctacagacat gctgtgggag tctggcttat 660

gcagcacctg agttaataca aggcaaatca tatcttggat cagaggcaga tgtttggagc 720

atgggcatac tgttatatgt tcttatgtgt ggatttctac catttgatga tgataatgta 780

atggctttat acaagaagat tatgagagga aaatatgatg ttcccaagtg gctctctccc 840

agtagcattc tgcttcttca acaaatgctg caggtggacc caaagaaacg gatttctatg 900

aaaaatctat tgaaccatcc ctggatcatg caagattaca actatcctgt tgagtggcaa 960

agcaagaatc cttttattca cctcgatgat gattgcgtaa cagaactttc tgtacatcac 1020

agaaacaaca ggcaaacaat ggaggattta atttcactgt ggcagtatga tcacctcacg 1080

gctacctatc ttctgcttct agccaagaag gctcggggaa aaccagttcg tttaaggctt 1140

tcttctttct cctgtggaca agccagtgct accccattca cagacatcaa gtcaaataat 1200

tggagtctgg aagatgtgac cgcaagtgat aaaaattatg tggcgggatt aatagactat 1260

gattggtgtg aagatgattt atcaacaggt gctgctactc cccgaacatc acagtttacc 1320

aagtactgga cagaatcaaa tggggtggaa tctaaatcat taactccagc cttatgcaga 1380

acacctgcaa ataaattaaa gaacaaagaa aatgtatata ctcctaagtc tgctgtaaag 1440

aatgaagagt actttatgtt tcctgagcca aagactccag ttaataagaa ccagcataag 1500

agagaaatac tcactacgcc aaatcgttac actacaccct caaaagctag aaaccagtgc 1560

ctgaaagaaa ctccaattaa aataccagta aattcaacag gaacagacaa gttaatgaca 1620

ggtgtcatta gccctgagag gcggtgccgc tcagtggaat tggatctcaa ccaagcacat 1680

atggaggaga ctccaaaaag aaagggagcc aaagtgtttg ggagccttga aagggggttg 1740

gataaggtta tcactgtgct caccaggagc aaaaggaagg gttctgccag agacgggccc 1800

agaagactaa agcttcacta taatgtgact acaactagat tagtgaatcc agatcaactg 1860

ttgaatgaaa taatgtctat tcttccaaag aagcatgttg actttgtaca aaagggttat 1920

acactgaagt gtcaaacaca gtcagatttt gggaaagtga caatgcaatt tgaattagaa 1980

gtgtgccagc ttcaaaaacc cgatgtggtg ggtatcagga ggcagcggct taagggcgat 2040

gcctgggttt acaaaagatt agtggaagac atcctatcta gctgcaaggt ataattgatg 2100

gattcttcca tcctgccgga tgagtgtggg tgtgatacag cctacataaa gactgttatg 2160

atcgctttga ttttaaagtt cattggaact accaacttgt ttctaaagag ctatcttaag 2220

accaatatct ctttgttttt aaacaaaaga tattattttg tgtatgaatc taaatcaagc 2280

ccatctgtca ttatgttact gtctttttta atcatgtggt tttgtatatt aataattgtt 2340

gactttctta gattcacttc catatgtgaa tgtaagctct taactatgtc tctttgtaat 2400

gtgtaatttc tttctgaaat aaaaccattt gtgaatataa aaaaaaaaaa aaaaaaaaaa 2460

aaaaaaaaaa aaaaaaaaaa aaaaaaaaaa aaaaaaaaaa a 2501

<210> 100
<211> 1861
<212> DNA
<213> Homo sapiens

<400> 100
gggagcgcgg agcggaggcc gaggcgagag cctggcgctg taggactaga acgaaaggag 60

tgaggcgccg agagcccaga taccattttg gcgtgagagc tggtggttgg caaggccgcg 120

ggagtgggaa gcgtccgcca tgttctgcga aaaagccatg gaactgatcc gcgagctgca 180

tcgcgcgccc gaagggcaac tgcctgcctt caacgaggat ggactcagac aagttctgga 240

ggagatgaaa gctttgtatg aacaaaacca gtctgatgtg aatgaagcaa agtcaggtgg 300

acgaagtgat ttgataccaa ctatcaaatt tcgacactgt tctctgttaa gaaatcgacg 360

ctgcactgta gcatacctgt atgaccgctt gcttcggatc agagcactca gatgggaata 420

tggtagcatc ttgccaaatg cattacgatt tcacatggct gctgaagaaa tggagtggtt 480

taataattat aaaagatctc ttgctactta tatgaggtca ctgggaggag atgaaggttt 540

ggacattaca caggatatga aaccaccaaa aagcctatat attgaagtcc ggtgtctaaa 600

agactatgga gaatttgaag ttgatgatgg cacttcagtc ctattaaaaa aaaatagcca 660

gcacttttta cctcgatgga aatgtgagca gctgatcaga caaggagtcc tggagcacat 720

cctgtcatga ccatgcgccg aggcacttcc aggcttcact caactcatgg actcctctgt 780

actcactctc tccaccactc ccttcacctc cctctttgat tttagaagct atagacattg 840

tttaagataa ctaagaatac ttggctaaga agtataattt gctaactatt aaggactttc 900

tttttttaat gttgtacact attcttccta ctcttttttg gttttggttt tgttttgtag 960

agactgtctc actatgttgc ccaagctggt ctcaaactcc tggcctcaag cagtcctccc 1020

accttagctt ctcaaagtgt tgagatcaca ggcgtgagcc actgcacccg gcccctactc 1080

ctttttctaa taagctgtat ctgtaatcac agcattccta cagttgttac agtgtgtttt 1140

ttaaatgaaa gtaaacatgg ttacatttga atctcttaaa taagcagtca cttggctgga 1200

caggaagaag gtagatcctg tgtgtcttgt tttctggtca tgtgtattgt acaagctaga 1260

gagctgaatt tctgagatac acattttcaa atcacatgca agtgaagatg atggtctgta 1320

gaaattttca gtatatataa tgtttaatga catactaatt tatcatctgg ctatttggga 1380

aggaaggaca cacatggatt ttgcacattt ccaccatggt ggctggtgtg gcttgtggct 1440

atggggtgat caccagtatc accactttgg aaggggacag tgaaattggg gctagagaag 1500

gaactttgta cagttttccc tgagattcag attgactgaa aagtcacatg aagagttgat 1560

tgtcttttaa tggtatgttt taaacagctg acattttaaa ttttgatgaa atccagttta 1620

ttcgtttgtt cttttatgct ttgggtgttg catccgagaa atcttttccc atcccaagat 1680

cacaattttt tttccttttt acttctagaa gtgttataat tttaagcttt atactttggt 1740

ctatgacccg tttttttttt tgttttgttt tgttttttcg tttgtttctt tgttttgaga 1800

tggagcctgg gtgatagagt gagacactgt cttgccaaaa aaaaaaaaaa aaaaaaaaaa 1860

a 1861

<210> 101
<211> 4513
<212> DNA
<213> Homo sapiens

<400> 101
ccacgcgtcc gcagagccgc gggtgagatc cccagccctg tgagcctgta ggagtagaat 60

ggctccccaa atgtatgagt tccatctgcc attatcccca gaggagttgt tgaaaagtgg 120

aggggtgaat cagtatgttg tgcaagaggt actgtccatc aaacatcttc caccacagct 180

tagagctttt caggctgcct ttcgagctca ggggcccctg gctatgctgc agcactttga 240

tactatctac agcattttgc atcactttcg aagtatagat cctggcctca aagaagatac 300

tctggaattc ctgataaaag tggtatcccg ccactcccag gagcttccag ctatcctgga 360

tgatacaact tttgagtgga tcagatagaaa cgcccatcta aatgccctca aaatgaactg 420

ttatgctctg atacgtctcc tggaatcctt tgagaccatg gccagccaga caaaccttgt 480

ggacctggac cttggtggga agggtaagaa agctcggacc aaggcagccc atggctttga 540

ctgggaagaa gagaggcaac caattcttca gcttttaaca cagctacttc agttggacat 600

ccgtcacctg tggaaccact caataattga agaagaattt gtcagtttgg ttactggctg 660

ttgctaccgc cttctggaga atcccaccat taatcaccag aagaaccgcc ccactcggga 720

agccataaca cacctgcttg gtgtagcctt gacccgttat aaccatatgc tcagtgctac 780

agtgaagatc atccagatgc tgcagcactt tgaacacctg gcacctgtac tggttgcagc 840

cgtgagtcta tgggcaactg actatggaat gaagagcata gtgggagaga ttgtaagaga 900

gattggacaa aagtgtcccc aagagctgag tcgagaccct tcagggacaa agggctttgc 960

agcattcctg acagaactag cagaacgtgt cccagctatc ctgatgtcca gcatgtgcat 1020

tttgctagat cacctggatg gagaaaatta catgatgcgt aatgctgtgc tggcagccat 1080

ggcggagatg gtgctgcagg ttctcagtgg cgatcaactg gaagcagcag cccgagacac 1140

cagagaccag ttcttggata ctttacaagc ccatggccat gatgtcaact cctttgtgcg 1200

gagccgtgtt ttgcagctct tcacccgaat tgtccagcag aaggctctcc ccctgacacg 1260

tttccaggca gtggtggctt tagctgtggg acgtctggca gacaagtcag tgctagtatg 1320

taaaaatgcc atccagctgc tggccagttt tctagccaat aatcctttct cctgcaagct 1380

tagtgatgct gaccttgccg gaccactgca gaaggagacc cagaaattac aagagatgag 1440

ggcccagagg cgaactgcag cagcttctgc agtgctggac ccagaggagg agtgggaagc 1500

catgctgcca gagttgaagt ctaccctgca gcagcttcta cagcttcccc agggagagga 1560

ggagattcct gagcaaattg ccaatacaga gacaactgaa gatgtgaaag gacgcatcta 1620

tcaactgctt gccaaagcta gttacaaaaa ggccatcatt ctcactcgag aagccacagg 1680

ccacttccag gagtccgaac ccttcagtca tatagaccca gaggagtcag aggagaccag 1740

gctcttgaat atcttaggac ttatcttcaa aggcccagca gcttccacac aagaaaagaa 1800

tccccgggag tctacaggaa acatggtcac aggacagact gtctgtaaaa ataaacccaa 1860

tatgtcggat cctgaggaat ccaggggaaa tgatgaacta gtgaagcagg agatgctggt 1920

acagtatctg caggatgcct acagcttctc ccggaagatt acagaggcca ttggcataat 1980

cagcaagatg atgtatgaaa acacaactac agtggtgcag gaggtgattg aattctttgt 2040

gatggtcttc caatttgggg taccccaggc cctgtttggg gtgcgccgta tgctgcctct 2100

catctggtct aaggagcctg gtgtccggga agccgtgctt aatgcctacc gccaactcta 2160

cctcaacccc aaaggggact ctgccagagc caaggcccag gctttgattc agaatctctc 2220

tctgctgcta gtggatgcct cggttgggac cattcagtgt cttgaggaaa ttctctgtga 2280

gtttgtgcag aaggatgagt tgaaaccagc agtgacccag ctgctgtggg agcgggccac 2340

cgagaaggtc gcctgctgtc ctctggagcg ctgttcctct gtcatgcttc ttggcatgat 2400

ggcacgagga aagccagaaa ttgtgggaag caatttagac acactggtga gcatagggct 2460

ggatgagaag tttccacagg actacaggct ggcccagcag gtgtgccatg ccattgccaa 2520

catctcggac aggagaaagc cttctctggg caaacgtcac ccccccttcc ggctgcctca 2580

ggaacacagg ttgtttgagc gactgcggga gacagtcaca aaaggctttg tccacccaga 2640

cccactctgg atcccattca aagaggtggc agtgaccctc atttaccaac tggcagaggg 2700

ccccgaagtg atctgtgccc agatattgca gggctgtgca aaacaggccc tggagaagct 2760

agaagagaag agaaccagtc aggaggaccc gaaggagtcc cccgcaatgc tccccacttt 2820

cctgttgatg aacctgctgt ccctggctgg ggatgtggct ctgcagcagc tggtccactt 2880

ggagcaggca gtgagtggag agctctgccg gcgccgagtt ctccgggaag aacaggagca 2940

caagaccaaa gatcccaagg agaagaatac gagctctgag accaccatgg aggaggagct 3000

ggggctggtt ggggcaacag cagatgacac agaggcagaa ctaatccgtg gcatctgcga 3060

gatggaactg ttggatggca aacagacact ggctgccttt gttccactct tgcttaaagt 3120

ctgtaacaac ccaggcctct atagcaaccc agacctctct gcagctgctt cacttgccct 3180

tggcaagttc tgcatgatca gtgccacttt ctgcgactcc cagcttcgtc ttctgttcac 3240

catgctggaa aagtctccac ttcccattgt ccggtctaac ctcatggttg ccactgggga 3300

tctggccatc cgctttccca atctggtgga cccctggact cctcatctgt atgctcgcct 3360

ccgggaccct gctcagcaag tgcggaaaac agcggggctg gtgatgaccc acctgatcct 3420

caaggacatg gtgaaggtga aggggcaggt cagcgagatg gcggtgctgc tcatcgaccc 3480

cgagcctcag attgctgccc tggccaagaa cttcttcaat gagctctccc acaagggcaa 3540

cgcaatctat aatctccttc cagatatcat cagccgcctg tcagaccccg agctgggggt 3600

ggaggaagag cctttccaca ccatcatgaa acagctcctc tcctacatca ccaaggacaa 3660

gcagacagag agcctggtgg aaaagctgtg tcagcggttc cgcacatccc gaactgagcg 3720

gcagcagcga gacctggcct actgtgtgtc acagctgccc ctcacagagc gaggcctccg 3780

taagatgctt gacaattttg actgttttgg agacaaactg tcagatgagt ccatcttcag 3840

tgcttttttg tcagttgtgg gcaagctgcg acgtggggcc aagcctgagg gcaaggctat 3900

aatagatgaa tttgagcaga agcttcgggc ctgtcatacc agaggtttgg atggaatcaa 3960

ggagcttgag attggccaag caggtagcca gagagcgcca tcagccaaga aaccatccag 4020

tggttctagg taccagcctc tggcttctac agcctcagac aatgactttg tcacaccaga 4080

gccccgccgt actacccgtc ggcatccaaa cacccagcag cgagcttcca aaaagaaacc 4140

caaagttgtc ttctcaagtg atgagtccag tgaggaagat ctttcagcag agatgacaga 4200

agacgagaca cccaagaaaa caactcccat tctcagagca tcggctcgca ggcacagatc 4260

ctaggaagtc tgttcctgtc ctccctgtgc agggtatcct gtagggtgac ctggaattcg 4320

aattctgttt cccttgtaaa atatttgtct gtctcttttt ttaaaaaaaa aaaaaaaagg 4380

ccgggcactg tggctcacgc ctgtaatccc agcactttgc gataccaagg cgggtggata 4440

acctgaggta gggagttcga gaccagcctg accaacatgg agaaacccca tctctactaa 4500

aaaaaaaaaa aaa 4513

<210> 102
<211> 6586
<212> DNA
<213> Homo sapiens

<400> 102
ctggggagcc ggcgctggag gtggtgagtg gcgtggggac tgtgtcgagg gggtccccaa 60

ggtgccggac cctgcggagg ggcgaagttt cggcactggg gagggcgtgc ggacgctttc 120

cctacaggcg accactgctc tgcgggcggg tggtcttagc tccagtcccc cattcagttc 180

ctcagcattc caggtcggcg gcgaaggggt ccccgaacga agggcgcaag gcagcgtctc 240

tgctgggacc gggaagccgg acttcagggc ctctcggccc gtgggcttct ccccgagtct 300

ccccgagtcg gttggcatta agagtttagc agatactttc agaaatggat acataagaaa 360

tggctggaaa tcaaatgaat gtccaaagaa gagcttaggg tcttagtaac attctttttt 420

aaaataactg tctgccaaaa tgtcattaca cagtactcat aatagaaata acagcggtga 480

tattcttgat attccttctt cccaaaatag ttcatcactg aatgccctca cccacagtag 540
ccgacttaag ctgcatttga agtcggatat gtcagaatgt gaaaatgatg atccattatt 600
gagatctgca ggtaaagtca gagacataaa tagaacttat gttatttctg ccagtagaaa 660
aacagcagac atgcccctta cccctaatcc tgtaggtaga ttggcacttc agaggagaac 720
tacaaggaac aaagaatcat ctttgcttgt tagtgagttg gaagacacaa ctgaaaaaac 780
agcagaaaca cgtcttacat tacaacgtcg tgctaaaaca gattctgcag aaaagtggaa 840
aacagctgaa atagattctg tcaaaatgac actgaatgtg ggaggtgaaa cagaaaataa 900
tggtgtttct aaggaaagta gaacaaatgt aaggattgta aataatgcta aaaactcttt 960
tgttgcctct tctgtacctt tagatgaaga tccacaggtc attgaaatga tggctgataa 1020
gaaatacaaa gaaacatttt ctgcccccag tagagcaaat gaaaatgttg cacttaagta 1080
ctcaagtaat agaccaccca ttgcttccct gagtcagact gaagttgtta gatcaggaca 1140
cttgacaacg aaacctactc agagcaagtt ggatatcaaa gtgttgggaa caggaaactt 1200
gtatcataga agtattggga aggaaattgc aaaaacttca aataaatttg ggagcttaga 1260
aaaaagaaca cctacaaaat gtacaacaga acacaaactg acaacaaagt gcagcctgcc 1320
tcagcttaag agcccagctc catcaatact gaagaataga atgtctaacc ttcaagttaa 1380
acaaagacca aaaagttcct ttcttgcaaa taaacaggaa agatccgcag aaaatacaat 1440
tcttcccgaa gaagaaactg tagttcagaa cacctctgca ggaaaagacc ccttaaaagt 1500
agagaatagt caagtgacag tggcagtacg cgtaagacct ttcaccaaga gagagaagat 1560
tgaaaaagca tcccaggtag tcttcatgag tgggaaagaa ataactgtgg aacaccctga 1620
cacgaaacaa gtttataatt ttatttatga tgtttcattc tggtcttttg atgaatgtca 1680
tcctcactac gctagccaga caactgtcta tgagaagcta gcagcaccac tcctagaaag 1740
agccttcgaa ggcttcaata cctgtctttt tgcttatggt cagactggct ctggaaaatc 1800
atatacgatg atgggattta gtgaagaacc aggaataatt ccaagatttt gtgaagatct 1860
tttttctcaa gtagccagaa aacaaaccca agaggtcagc tatcacattg aaatgagctt 1920
ctttgaagta tataatgaaa aaattcacga ccttctggtt tgtaaagatg aaaatgggca 1980
gagaaagcaa ccactgagag tgagggaaca tcctgtttat ggaccatatg ttgaagcact 2040
gtcaatgaac attgtcagtt cttacgctga tatccagagt tggctagaat tgggaaataa 2100
acaaagagct actgctgcta ctggtatgaa tgataaaagt tcccgatctc attcagtttt 2160
caccctggtg atgacccaga ccaagacaga atttgtggaa ggggaagaac acgatcacag 2220
aataacaagt cgaattaacc taatagatct ggcaggcagt gagcgctgct ctacggctca 2280
cactaatgga gatcgactaa aggaaggtgt gagtattaat aagtccttgc taactttggg 2340
aaaagttata tctgcacttt cggaacaagc aaaccaaagg agtgttttta ttccttatcg 2400
tgaatctgtt cttacatggc tgttaaaaga aagtctgggt ggaaattcaa aaactgcaat 2460
gattgctacg attagtcccg ctgccagcaa catagaagaa acattaagca cacttagata 2520
tgctaaccaa gcccgtttaa tagtcaacat tgctaaagta aatgaagata tgaacgctaa 2580

gttaattaga gaattgaagg cagaaattgc aaagctaaaa gctgctcaga gaaacagtcg 2640

gaatattgac cctgaacgat acaggctctg tcggcaagaa ataacatcct taagaatgaa 2700

actgcatcaa caggagagag acatggcaga aatgcaaaga gtgtggaaag aaaagtttga 2760

acaagctgaa aaaagaaaac ttcaagaaac aaaagagtta cagaaagcag gaattatgtt 2820

tcaaatggac aatcatttac caaaccttgt taatctgaat gaagatccac aactatctga 2880

gatgctgcta tatatgataa aagaaggaac aactacagtt ggaaagtata aaccaaactc 2940

aagccatgat attcagttat ctggggtgct gattgctgat gatcattgta ctatcaaaaa 3000

ttttggtggg acagtgagta ttatcccagt tggggaagca aagacatatg taaatggaaa 3060

acatattttg gaaatcacag tattacgtca tggtgatcga gtgattcttg gtggagatca 3120

ttattttaga tttaatcatc cagtagaagt ccagaaagga aaaaggccat ctggaagaga 3180

tactcctata agtgagggtc caaaagactt tgaatttgca aaaaatgagt tgctcatggc 3240

acagagatca caacttgaag cagaaataaa agaggctcag ttgaaggcaa aggaagaaat 3300

gatgcaagga atccagattg caaaagaaat ggctcagcaa gagctttctt ctcaaaaagc 3360

tgcatatgaa agcaaaataa aagcactgga agcagaactg agagaagagt ctcaaaggaa 3420

aaaaatgcag gaaataaata accagaaggc taatcacaaa attgaggaat tagaaaaggc 3480

aaagcagcat cttgaacagg aaatatatgt caacaaaaag cgattagaaa tggagacatt 3540

ggctacaaaa caggctttag aagaccatag catccgccat gcaagaattc tggaagcttt 3600

agaaactgaa aagcaaaaaa ttgctaaaga agtacaaatt ctacagcaga atcggaataa 3660

tagggataaa acttttacag tgcagacaac ttggagctct atgaaactct caatgatgat 3720

tcaggaagcc aatgctatca gcagcaaatt gaaaacatac tatgtttttg gcagacatga 3780

tatatcagat aaaagtagtt ctgacacttc tattcgggtt cgtaacctga aactaggaat 3840

ctcaacattc tggagtctgg aaaagtttga atctaaactt gcagcaatga aagaacttta 3900

tgagagtaat ggtagtaaca ggggtgaaga tgccttttgt gatcctgaag atgaatggga 3960

acccgacatt acagatgcac cagtttcttc actttctaga aggaggagta ggagtttgat 4020

gaagaacaga agaatttctg gttgtttaca tgacatacaa gtccatccaa ttaagaattt 4080

gcattcttca cattcatcag gtttaatgga caaatcaagc actatttact caaattcagc 4140

agagtccttt cttcctggaa tttgcaaaga attgattggt tcttcgttag atttttttgg 4200

acagagttat gatgaagaaa gaactatagc agacagccta attaatagtt ttcttaaaat 4260

ttataatggg ctatttgcca tttccaaggc tcatgaagaa caagatgaag aaagtcaaga 4320

taacttgttt tcttctgatc gagcaatcca gtcacttact attcagactg catgtgcttt 4380

tgagcagcta gtagtgctaa tgaaacactg gctgagtgat ttactgcctt gtaccaacat 4440

agcaagactt gaggatgagt tgagacaaga agttaaaaaa ctgggaggct acttacagtt 4500

atttttgcag ggatgctgtt tggatatttc atcaatgata aaagaggctc aaaagaatgc 4560

aatccaaatt gtacaacaag ctgtaaagta tgtggggcag ttagcagttc tgaaaggagg 4620

caagctacat tttctagaaa acggtaacaa taaagctgcc agtgtccagg aggaattcat 4680

ggatgctgtt tgtgatggtg taggcttagg aatgaagatt ttattagatt ctggactgga 4740
aaaagcaaaa gaacttcagc atgaactctt taggcagtgt acaaaaaatg aggttaccaa 4800
agaaatgaaa actaatgcca tgggattgat tagatctctt gaaaacatct ttgctgaatc 4860
gaaaattaaa agtttcagaa ggcaagtaca agaagaaaac tttgaatacc aagatttcaa 4920
gaggatggtt aatcgtgctc cagaattctt aaagttaaaa cattgcttag agaaagctat 4980
tgaaattatt atttctgcac tgaaaggatg ccatagtgat ataaatcttc tccagacttg 5040
tgttgaaagt attcgcaact tggccagtga tttttacagt gacttcagtg tgccttctac 5100
ttctgttggc agctatgaga gtagagtaac tcacattgtc caccaggaac tagaatctct 5160
agctaagtct ctcctctttt gttttgaatc tgaagaaagc cctgatttgt tgaaaccctg 5220
ggaaacttat aatcaaaata ccaaagaaga acaccaacaa tctaaatcaa gcgggattga 5280
cggcagtaag aataaaggtg taccaaagcg tgtctatgag ctccatggct catccccagc 5340
agtgagctca gaggaatgca cacccagtag gattcagtgg gtgtgaatac tgatgtgtag 5400
gcacttttat gaccacccat gaaagaaaaa gaacacttgc tcggtaattt tctttatgca 5460
ggagagttta agagaaatca gcacagatat ttcaaaaaag tccatgtctt tttatcttta 5520
aaatatctat ttatcaaagg ccagacacag tggctcacgc ctgtaatccc agcactttgg 5580
gaggcgggca gatcacaagg tcaggagttt gagaccggcc tggccaacat ggtgaaaccc 5640
cgtctctact aaaaatacaa aaatttgctg ggcatggtgg cgcgtgcctg taatcccagc 5700
tactaggggg gctgaggcag gaggatcgct tgaacctgag aggcagaggt tgcagtgagc 5760
caagatcatg ccactttact ccagtctgag caacagaacg agacttagtc aaaataaata 5820
aataaataag taaataaata aataaataaa atatctttta tctttaaagt gtttaacatt 5880
ggtatactgt ctgtagttgg ttcattagtc gtttataaag ggttattttc tcatgagtgg 5940
aaacctgaac aatcagttac ctttgtgcct atgccttctc tctcctcaga cagctgggat 6000
gtttatggtg aaatggcctg tacaagttta actaagacaa cttaacttgc attgttaatc 6060
aaaaattctt ttctcaaagg gttaactggt tgccattttg aatagtatgt tcaagggtgt 6120
agcttcctgt ttctttccaa attataagta gctacctaaa tatagtataa ttatatatta 6180
ataatatggc ttgctggcac agtagtttac cctgttatct gtgtttcata atgggggctg 6240
tatgaatatt atttaaaact aataaaatgt tgccagaatt atactaaact gttggatgag 6300
attaggagat cagaggctgg accttctctt gataatgctt gttttgttaa aggtataatg 6360
aaataatttg tatatgattt gatgaagatt aaagaccctt attttccaca gctttaaaaa 6420
aaaaccttta tttatgatca agtaataaag ataatattct acttgtggga tcttacatta 6480
tggaaatagt ttgacgtttt tgacctcaag agtatgtata atttgaagag atactttgta 6540
actatgcttg ggtgatattg agcagttcct aaagaataat tcattt 6586

<210> 103
<211> 2561
<212> DNA
<213> Homo sapiens

<400> 103
   cgccaggaac agcttgaggt acctgagccc tgccctccag cagcacccga gagggtcagg 60
   agaaaagcgg aggaagctgg gtaggccctg aggggcctcg gtaagccatc atgaccaccc 120
   ggcaagccac gaaggatccc ctcctccggg gtgtatctcc tacccctagc aagattccgg 180
   tacgctctca gaaacgcacg cctttcccca ctgttacatc gtgcgccgtg gaccaggaga 240
   accaagatcc aaggagatgg gtgcagaaac caccgctcaa tattcaacgc cccctcgttg 300
   attcagcagg ccccaggccg aaagccaggc accaggcaga gacatcacaa agattggtgg 360
   ggatcagtca gcctcggaac cccttggaag agctcaggcc tagccctagg ggtcaaaatg 420
   tggggcctgg gccccctgcc cagacagagg ctccagggac catagagttt gtggctgacc 480
   ctgcagccct ggccaccatc ctgtcaggtg agggtgtgaa gagctgtcac ctggggcgcc 540
   agcctagtct ggctaaaaga gtactggttc gaggaagtca gggaggcacc acccagaggg 600
   tccagggtgt tcgggcctct gcatatttgg cccccagaac ccccacccac cgactggacc 660
   ctgccagggc ttcctgcttc tctaggctgg agggaccagg acctcgaggc cggacattgt 720
   gcccccagag gctacaggct ctgatttcac cttcaggacc ttcctttcac ccttccactc 780
   accccagttt ccaggagcta agaagggaga cagctggcag cagccggact tcagtgagcc 840
   aggcctcagg attgctcctg gagaccccag tccagcctgc tttctctctt cctaaaggag 900
   aacgcgaggt tgtcactcac tcagatgaag gaggtgtggc ctctcttggt ctggcccagc 960
   gagtaccatt aagagaaaac cgagaaatgt cacataccag ggacagccat gactcccacc 1020
   tgatgccctc ccctgcccct gtggcccagc ccttgcctgg ccatgtggtg ccatgtccat 1080
   caccctttgg acgggctcag cgtgtaccct ccccaggccc tccaactctg acctcatatt 1140
   cagtgttgcg gcgtctcacc gttcaaccta aaacccggtt cacacccatg ccatcaaccc 1200
   ccagagttca gcaggcccag tggctgcgtg gtgtctcccc tcagtcctgc tctgaagatc 1260
   ctgccctgcc ctgggagcag gttgccgtcc ggttgtttga ccaggagagt tgtataaggt 1320
   cactggaggg ttctgggaaa ccaccggtgg ccactccttc tggaccccac tctaacagaa 1380
   cccccagcct ccaggaggtg aagattcaac gcatcggtat cctgcaacag ctgttgagac 1440
   aggaagtaga ggggctggta gggggccagt gtgtccctct taatggaggc tcttctctgg 1500
   atatggttga acttcagccc ctgctgactg agatttctag aactctgaat gccacagagc 1560
   ataactctgg gacttcccac cttcctggac tgttaaaaca ctcagggctg ccaaagccct 1620
   gtcttccaga ggagtgcggg gaaccacagc cctgccctcc ggcagagcct gggcccccag 1680
   aggccttctg taggagtgag cctgagatac cagagccctc cctccaggaa cagcttgaag 1740
   taccagagcc ctaccctcca gcagaaccca ggcccctaga gtcctgctgt aggagtgagc 1800
   ctgagatacc ggagtcctct cgccaggaac agcttgaggt acctgagccc tgccctccag 1860
   cagaacccag gcccctagag tcctactgta ggattgagcc tgagataccg gagtcctctc 1920
   gccaggaaca gcttgaggta cctgagccct gccctccagc agaacccggg ccccttcagc 1980
   ccagcaccca ggggcagtct ggacccccag ggccctgccc tagggtagag ctgggggcat 2040
   cagagccctg caccctggaa catagaagtc tagagtccag tctaccaccc tgctgcagtc 2100
   agtgggctcc agcaaccacc agcctgatct tctcttccca acacccgctt tgtgccagcc 2160
   cccctatctg ctcactccag tctttgagac ccccagcagg ccaggcaggc ctcagcaatc 2220
   tggcccctcg aaccctagcc ctgagggagc gcctcaaatc gtgtttaacc gccatccact 2280
   gcttccacga ggctcgtctg gacgatgagt gtgcctttta caccagccga gcccctccct 2340
   caggccccac ccgggtctgc accaaccctg tggctacatt actcgaatgg caggatgccc 2400
   tgtgtttcat tccagttggt tctgctgccc cccagggctc tccatgatga gacaaccact 2460
   cctgccctgc cgtacttctt ccttttagcc cttatttatt gtcggtctgc ccatgggact 2520
   gggagccgcc cacttttgtc ctcaataaag tttctaaagt a 2561

<210> 104
<211> 3344
<212> DNA
<213> Homo sapiens

<400> 104
   gcggcagcag cggcgcgggc gggagggcga agagcagcgg ccgcctgagg ggagcccgcg 60
   cctccgccgc ctgagaggag gtcgagctgc cgccggggcg atgctggagg agctggagtg 120
   cggggcgccc ggcgccaggg gagccgccac agccatggat tgcaaagata gaccagcttt 180
   tccagttaag aagttaatac aagcccgtct gccgtttaag cgcctgaatc ttgtcccaaa 240
   ggggaaagcc gatgacatgt cagacgatca gggtacttct gtgcaaagta aaagccccga 300
   tttagaggcc tctttggaca ccttggaaaa caactgtcat gtgggttctg acatagactt 360
   tagaccgaaa cttgtcaacg ggaagggtcc cttagataac tttttaagaa atagaatcga 420
   aaccagtatt ggccagagca cagtcatcat tgatttgaca gaggactcga atgagcagcc 480
   agacagtctt gtggaccaca ataaactaaa ttctgaagcc tctccctcca gggaggcaat 540
   aaatggccag cgagaagaca ctggggatca gcaggggttg ttgaaggcca ttcagaacga 600
   caagttggca tttcctggag agaccctttc agacattcct tgcaaaacag aggaggaggg 660
   tgttggctgt ggaggtgcag ggaggagagg cgactcccag gaatgttcgc cacggagctg 720
   cccggagctg acgagtggcc cgagaatgtg ccccagaaag gagcaggaca gttggagtga 780
   agctgggggc atcctgttca aagggaaggt gcctatggtg gtcttgcagg acatcttggc 840
   tgtgagacca ccgcaaatca agtcccttcc agccacaccc caaggcaaga acatgacccc 900
   tgagagtgag gtgctggaat ctttccccga agaagactct gtactcagcc attcgtccct 960
   gagctctccc tcttccacca gctcgcccga ggggccgcct gctcccccaa agcagcacag 1020
   cagtaccagt cccttcccca cctccacgcc cctccgcaga ataactaaga aattcgtcaa 1080
   aggctctaca gagaagaaca agctcagact gcaaagagat caggagcgtc tgggcaagca 1140
   gctcaagtta cgtgcagaaa gggaagaaaa ggagaagctg aaagaggagg ccaagcgggc 1200
   caaggaggag gccaagaaga agaaggagga agagaaggag cttaaggaaa aggagaggcg 1260
   ggagaagcgg gagaaggatg agaaggagaa ggcggagaag cagcggctca aggaggagcg 1320
   gcgcaaggag agacaggaag ccctggaggc taaacttgag gaaaaaagga aaaaggaaga 1380
   agagaaacgg ttaagagaag aagagaagcg cattaaagca gagaaggccg aaatcacgag 1440
   gttcttccag aaaccaaaga ctccacaggc ccccaagacc ctggccggct cctgtgggaa 1500
   gtttgccccc tttgaaatta aagagcacat ggtcctggcc cctcggcgtc ggaccgcttt 1560
   ccatccagac ctctgcagtc agctggacca gctcctccag cagcagagcg gcgagttctc 1620
   cttcttgaaa gacctcaaag gccggcagcc cctgaggtcc ggacccacgc acgtttccac 1680
   ccggaatgca gatattttta acagtgatgt cgtcatcgtg gagcgtggga agggcgacgg 1740
   tgttcccgag aggaggaagt ttggcaggat gaagctcctg cagttctgtg agaaccaccg 1800
   gcctgcctac tggggtacct ggaataagaa gacggcactc atccgcgcgc gagacccctg 1860
   ggcccaggac acgaagctcc tggactatga ggtggacagt gatgaggagt gggaagaaga 1920
   ggagcctggg gagtccctgt cccacagtga gggggatgat gatgacgaca tgggagagga 1980
   tgaagatgag gacgatggtt tctttgtgcc ccatgggtac ctgtctgagg acgaaggtgt 2040
   gacagaggag tgtgccgacc ctgagaacca taaggtccgc cagaaactga aggccaagga 2100
   gtgggacgag ttcctggcta aggggaagcg ctttcgcgtc ctgcaacctg tgaagatcgg 2160
   ctgcgtgtgg gcggctgaca gagactgcgc aggcgatgac ctgaaggtac tgcagcagtt 2220
   cgcagcctgc ttcctggaga ccctgccggc ccaggaggag cagacgccca aggcctccaa 2280
   gcgggagagg agagacgagc agatcctggc ccagctgctg ccgctcctgc acggcaatgt 2340
   gaacgggagc aaggtcatca tccgggagtt ccaggagcac tgccgccggg gactgctcag 2400
   caaccacacc ggcagcccgc ggagcccctc caccacctac ctgcacaccc ccacccccag 2460
   cgaggatgcc gccatcccct ctaagtcccg gctcaagcgg ctcatttccg agaactcagt 2520
   gtatgagaag cggcctgact tcaggatgtg ctggtacgtg cacccgcagg tgctacagag 2580
   cttccagcag gagcacctgc ccgtgccgtg ccagtggagc tatgtgacat cggtgccctc 2640
   ggcccccaaa gaggacagtg gcagcgtccc ctccacgggg cccagccagg gcactcccat 2700
   ctcgctgaag aggaagtcag cgggcagcat gtgcatcacc caattcatga agaagcgcag 2760
   gcacgacggc cagattggtg ctgaagacat ggacggcttc caggcagaca cggaggagga 2820
   ggaagaggag gagggcgact gtatgatcgt ggatgtcccg gatgctgcgg aggtccaagc 2880
   cccgtgtgga gccgcttccg gagctggggg tggtgtgggg gtggacaccg gcaaggccac 2940
   cctgaccgcg agcccactgg gtgcatcctg agagcagggg tgacgtatgt agaatgctta 3000
   gggtgtcctc cccacagagc agatacttga accgactcaa ttcctgtgta aagagcactt 3060
   tgtcctgctt cacggacctc cccaaagtgt gcagagttct atataggatg ctggattagt 3120
   tcctttgata tttgtaaaaa ttcccccaag agccgcatat gaatctgccc tttaataaag 3180
   cattattgag attgctggcc tattggggaa gcctgcgggc acaggagcag gcgtggaatc 3240
   caatacttgt aaatgaattg aagcgtcagg accacccgcc tggccacgtg cgcgggcccc 3300
   tggacctaac gaggcagtgt ataaacttat tctctagccc tgaa 3344

<210> 105
<211> 1887
<212> DNA

<213>  Homo sapiens

<400>  105
ccatggcggc gcggcggcga cggagcaccg gcggcggcag ggcgagagca ttaaatgaaa      60

gcaaaagagt taataatggc aacacggctc cagaagactc ttcccctgcc aagaaaactc     120

gtagatgcca gagacaggag tcgaaaaaga tgcctgtggc tggaggaaaa gctaataagg     180

acaggacaga agacaagcaa gatggtatgc caggaaggtc atgggccagc aaaagggtct     240

ctgaatctgt gaaggccttg ctgttaaagg gcaaagctcc tgtggaccca gagtgtacag     300

ccaaggtggg gaaggctcat gtgtattgtg aaggaaatga tgtctatgat gtcatgctaa     360

atcagaccaa tctccagttc aacaacaaca agtactatct gattcagcta ttagaagatg     420

atgcccagag gaacttcagt gtttggatga gatggggccg agttgggaaa atgggacagc     480

acagcctggt ggcttgttca ggcaatctca acaaggccaa ggaaatcttt cagaagaaat     540

tccttgacaa aacgaaaaac aattgggaag atcgagaaaa gtttgagaag gtgcctggaa     600

aatatgatat gctacagatg gactatgcca ccaatactca ggatgaagag gaaacaaaaa     660

aagaggaatc tcttaaatct cccttgaagc cagagtcaca gctagatctt cgggtacagg     720

agttaataaa gttgatctgt aatgttcagg ccatggaaga aatgatgatg gaaatgaagt     780

ataataccaa gaaagcccca cttgggaagc tgacagtggc acaaatcaag gcaggttacc     840

agtctcttaa gaagattgag gattgtattc gggctggcca gcatggacga gctctcatgg     900

aagcatgcaa tgaattctac accaggattc cgcatgactt tggactccgt actcctccac     960

taatccggac acagaaggaa ctgtcagaaa aaatacaatt actagaggct ttgggagaca    1020

ttgaaattgc tattaagctg gtgaaaacag agctacaaag cccagaacac ccattggacc    1080

aacactatag aaacctacat tgtgccttgc gcccccttga ccatgaaagt tacgagttca    1140

aagtgatttc ccagtaccta caatctaccc atgctcccac acacagcgac tataccatga    1200

ccttgctgga tttgtttgaa gtggagaagg atggtgagaa agaagccttc agagaggacc    1260

ttcataacag gatgcttcta tggcatggtt ccaggatgag taactgggtg ggaatcttga    1320

gccatgggct tcgaattgcc caccctgaag ctcccatcac aggttacatg tttgggaaag    1380

gaatctactt tgctgacatg tcttccaaga gtgccaatta ctgctttgcc tctcgcctaa    1440

agaatacagg actgctgctc ttatcagagg tagctctagg tcagtgtaat gaactactag    1500

aggccaatcc taaggccgaa ggattgcttc aaggtaaaca tagcaccaag gggctgggca    1560

agatggctcc cagttctgcc cacttcgtca ccctgaatgg gagtacagtg ccattaggac    1620

cagcaagtga cacaggaatt ctgaatccag atggttatac cctcaactac aatgaatata    1680

ttgtatataa ccccaaccag gtccgtatgc ggtacctttt aaaggttcag tttaatttcc    1740

ttcagctgtg gtgaatgttg atcttaaata aaccagagat ctgatcttca agcaagaaaa    1800

taagcagtgt tgtacttgtg aattttgtga tattttatgt aataaaaact gtacaggtct    1860

aaaaaaaaaa aaaaaaaaaa aaaaaaa                                        1887

<210>  106
<211>  5071

<212>  DNA
<213>  Homo sapiens

<400>  106
gtattttagg cgccattttc gagtgaagga cccggagccg aaacaccggt aggagcgggg      60

aggtgggtac tacacaaccg tctccagcct tggtctgagt ggactgtcct gcagcgacca     120

tgccccgtaa aggcacccag ccctccactg cccggcgcag agaggaaggg ccgccgccgc     180

cgtcccctga cggcgccagc agcgacgcgg agcctgagcc gccgtccggc cgcacggaga     240

gcccagccac cgccgcagca atgaccaatg aagctggagc tcctcggctt atgataactc     300

atattgtaaa ccagaacttc aaatcctatg ctggggagaa aattctggga cctttccata     360

agcgcttttc ctgtattatc gggccaaatg gcagtggcaa atccaatgtt attgattcta     420

tgctttttgt gtttggctat cgagcacaaa aaataagatc taaaaaactc tcagtattaa     480

tacataattc tgatgaacac aaggacattc agagttgtac agtagaagtt cattttcaaa     540

agataattga taaggaaggg gatgattatg aagtcattcc taacagtaat ttctatgtat     600

ccagaacggc ctgcagagat aatacttctg tctatcacat aagtggaaag aaaaagacat     660

ttaaggatgt tggaaatctt cttcgaagcc atggaattga cttggaccat aatagatttt     720

taattttaca gggtgaagtt gaacaaattg ctatgatgaa accaaaaggc cagactgaac     780

acgatgaggg tatgcttgaa tatttagaag atataattgg ttgtggacgg ctaaatgaac     840

ctattaaagt cttgtgtcgg agagttgaaa tattaaatga acacagagga gagaagttaa     900

acagggtaaa gatggtggaa aaggaaaagg atgccttaga aggagagaaa aacatagcta     960

tcgaatttct taccttggaa aatgaaatat ttagaaaaaa gaatcatgtt tgtcaatatt    1020

atatttatga gttgcagaaa cgaattgctg aaatggaaac tcaaaaggaa aaaattcatg    1080

aagataccaa agaaattaat gagaagagca atatactatc aaatgaaatg aaagctaaga    1140

ataaagatgt aaaagataca gaaaagaaac tgaataaaat tacaaaattt attgaggaga    1200

ataaagaaaa atttacacag ctagatttgg aagatgttca agttagagaa aagttaaaac    1260

atgccacgag taaagccaaa aaactggaga aacaacttca aaaagataaa gaaaaggttg    1320

aagaatttaa aagtatacct gccaagagta acaatatcat taatgaaaca acaaccagaa    1380

acaatgccct cgagaaggaa aaagagaaag aagaaaaaaa attaaaggaa gttatggata    1440

gccttaaaca ggaaacacaa gggcttcaga aagaaaaaga aagtcgagag aaagaactta    1500

tgggtttcag caaatcggta aatgaagcac gttcaaagat ggatgtagcc cagtcagaac    1560

ttgatatcta tctcagtcgt cataatactg cagtgtctca attaactaag gctaaggaag    1620

ctctaattgc agcttctgag actctcaaag aaaggaaagc tgcaatcaga gatatagaag    1680

gaaaactccc tcaaactgaa caagaattaa aggagaaaga aaaagaactt caaaaactta    1740

cacaagaaga aacaaacttt aaaagtttgg ttcatgatct ctttcaaaaa gttgaagaag    1800

caaagagctc attagcaatg aatcgaagta gggggaaagt ccttgatgca ataattcaag    1860

aaaaaaaatc tggcaggatt ccaggaatat atggaagatt gggggactta ggagccattg    1920

atgaaaaata cgacgtggct atatcatcct gttgtcatgc actggactac attgttgttg    1980

attctattga tatagcccaa gaatgtgtaa acttccttaa aagacaaaat attggagttg 2040

caacctttat aggtttagat aagatggctg tatgggcgaa aaagatgacc gaaattcaaa 2100

ctcctgaaaa tactcctcgt ttatttgatt tagtaaaagt aaaagatgag aaaattcgcc 2160

aagcttttta ttttgcttta cgagatacct tagtagctga caacttggat caagccacaa 2220

gagtagcata tcaaaaagat agaagatgga gagtggtaac tttacaggga caaatcatag 2280

aacagtcagg tacaatgact ggtggtggaa gcaaagtaat gaaaggaaga atgggttcct 2340

cacttgttat tgaaatctct gaagaagagg taaacaaaat ggaatcacag ttgcaaaacg 2400

actctaaaaa agcaatgcaa atccaagaac agaaagtaca acttgaagaa agagtagtta 2460

agttacggca tagtgaacga gaaatgagga acacactaga aaaatttact gcaagcatcc 2520

agcgtttaat agagcaagaa gaatatttga atgtccaagt taaggaactt gaagctaatg 2580

tacttgctac agcccctgac aaaaaaaagc agaaattgct agaagaaaac gttagtgctt 2640

tcaaaacaga atatgatgct gtggctgaga aagctggtaa agtagaagct gaggttaaac 2700

gcttacacaa taccatcgta gaaatcaata atcataaact caaggcccaa caagacaaac 2760

ttgataaaat aaataagcaa ttagatgaat gtgcttctgc tattactaaa gcccaagtag 2820

caatcaagac tgctgacaga ccttcaaaag gcacaagact ctgtcttgcg tacagagaaa 2880

gaaataaaag atactgagaa agaggtggat gacctaacag cagagctgaa aagtcttgag 2940

gacaaagcag cagaggtcgt aaagaataca aatgctgcag aggaatcctt accagagatc 3000

cagaaagaac atcgcaatct gcttcaagaa ttaaaagtta ttcaagaaaa tgaacatgct 3060

cttcaaaaag atgcacttag tattaagttg aaacttgaac aaatagatgg tcacattgct 3120

gaacataatt ctaaaataaa atattggcac aaagagattt caaaaatatc actgcatcct 3180

atagaagata atcctattga agagatttcg gttctaagcc cagaggatct tgaagcgatc 3240

aagaatccag attctataac aaatcaaatt gcacttttgg aagcccggtg tcatgaaatg 3300

aaaccaaacc tcggtgccat cgcagagtat aaaaagaagg aagaattgta tttgcaacgg 3360

gtagcagaat tggacaaaat tacttatgaa agagacagtt ttagacaggc atatgaagat 3420

cttcggaaac aaaggcttaa tgaatttatg gcaggttttt atataataac aaataaatta 3480

aaggaaaatt accaaatgct tactttggga ggggacgccg aactcgagct tgtagacagc 3540

ttggatcctt tctctgaagg aatcatgttc agtgttcgac cacctaagaa aagttggaaa 3600

aagatcttca acctttcggg aggagagaaa acacttagtt cattggcttt agtatttgct 3660

cttcaccact acaagcccac tcccctttac ttcatggatg agattgatgc agcccttgat 3720

tttaaaaatg tgtccattgt tgcattttat atatatgaac aaacaaaaaa tgcacagttc 3780

ataataattt ctcttcgaaa taatatgttt gagatttcgg atagacttat tggaatttac 3840

aagacataca acataacaaa aagtgttgct gtaaatccaa aagaaattgc atctaaggga 3900

ctttgttgaa ctttatgctg aagattcttc aagttgattc agtgtattac tgattttttt 3960

ctatttgtaa aggattatga gttgtataaa atacatactc cctaaactag atcatgaaac 4020

tggtttctgt tttatgcagt tgtcatttgt aaagtctaat aaaatattct ctataattgc 4080

ttctagatta caaaaatatg acaatcttgt aagtagcaga ctatggagaa aaatgagtta 4140

cctggagggt caggtaactt gccaaactaa aaagtatgtt agttgaggca aagtcctaag 4200

caaggttgtg ctatcaaggc tcagcatacc ttcgtgggcc tttgatttac caacactgga 4260

aatgcctgcc aactaatctt ggatagattc tttaaggcat tccacttagc ttgccagttg 4320

agacaatcac cacagttatt acccaaatac tatgaacata tttttgtaaa ccagtcattc 4380

tgaattatag tgatgagaat ttaaatatat gcttttctag aatttgatgt ttgaccattt 4440

atgacttaat taccagagag ccagtaaatt aggacagtgt ttcaacaagc ctaggctatc 4500

tcgtaagttg aaaaatatcc cactatagtt gcttcatgag tatgaagtaa gatggcctct 4560

gatttacact ggttcaattt acaaattttc aactttatga taggtttatc cgggtactaa 4620

atgcatttca acttgatagt ttcaacttat gataggttta ccaggatgta gtcccactgt 4680

tgaggagcat ctatttaggg gttaattact ttagtaataa gtggaaagta agataccttg 4740

agtaatgttt gcctataaaa ttgtcagcgt atttttacac tattggctca agaatgttat 4800

aatgctaagg gacataagtt ggcaaccact tggtttttgg aaggactttc ggtattgtat 4860

tagaagtctg ccctagctgt taaatttctg ggtatttatc ctaaggaatt aattaaagag 4920

ttaattgttc ctttcttcag tgggccattg ttttagatat ttaaaaaatc caacagtttc 4980

tatcataatg taactgtaaa aatgtaaaca cattattagc atggactttt aaataaagat 5040

ttaaagaaag caaaaaaaaa aaaaaaaaaa a 5071

<210> 107
<211> 5142
<212> DNA
<213> Homo sapiens

<400> 107
taggcgccat tttcgagtga aggacccgga gccgaaacac cggtaggagc ggggaggtgg 60

gtactacaca accgtctcca gccttggtct gagtggactg tcctgcagcg accatgcccc 120

gtaaaggcac ccagccctcc actgcccggc gcagagagga agggccgccg ccgccgtccc 180

ctgacggcgc cagcagcgac gcggagcctg agccgccgtc cggccgcacg gagagcccag 240

ccaccgccgc agagactgca agtgaggaac ttgataatag aagtttagaa gagattttga 300

acagcattcc tcctcccccg cctccagcaa tgaccaatga agctggagct cctcggctta 360

tgataactca tattgtaaac cagaacttca aatcctatgc tggggagaaa attctgggac 420

ctttccataa gcgcttttcc tgtattatcg ggccaaatgg cagtggcaaa tccaatgtta 480

ttgattctat gctttttgtg tttggctatc gagcacaaaa aataagatct aaaaaactct 540

cagtattaat acataattct gatgaacaca aggacattca gagttgtaca gtagaagttc 600

attttcaaaa gataattgat aaggaagggg atgattatga agtcattcct aacagtaatt 660

tctatgtatc cagaacggcc tgcagagata atacttctgt ctatcacata agtggaaaga 720

aaaagacatt taaggatgtt ggaaatcttc ttcgaagcca tggaattgac ttggaccata 780

atagattttt aattttacag ggtgaagttg aacaaattgc tatgatgaaa ccaaaaggcc 840

agactgaaca cgatgagggt atgcttgaat atttagaaga tataattggt tgtggacggc 900

taaatgaacc tattaaagtc ttgtgtcgga gagttgaaat attaaatgaa cacagaggag 960

agaagttaaa cagggtaaag atggtggaaa aggaaaagga tgccttagaa ggagagaaaa 1020

acatagctat cgaatttctt accttggaaa atgaaatatt tagaaaaaag aatcatgttt 1080

gtcaatatta tatttatgag ttgcagaaac gaattgctga aatggaaact caaaaggaaa 1140

aaattcatga agataccaaa gaaattaatg agaagagcaa tatactatca aatgaaatga 1200

aagctaagaa taaagatgta aaagatacag aaaagaaact gaataaaatt acaaaattta 1260

ttgaggagaa taaagaaaaa tttacacagc tagatttgga agatgttcaa gttagagaaa 1320

agttaaaaca tgccacgagt aaagccaaaa aactggagaa acaacttcaa aaagataaag 1380

aaaaggttga agaatttaaa agtatacctg ccaagagtaa caatatcatt aatgaaacaa 1440

caaccagaaa caatgccctc gagaaggaaa aagagaaaga agaaaaaaaa ttaaaggaag 1500

ttatggatag ccttaaacag gaaacacaag ggcttcagaa agaaaaagaa agtcgagaga 1560

aagaacttat gggtttcagc aaatcggtaa atgaagcacg ttcaaagatg gatgtagccc 1620

agtcagaact tgatatctat ctcagtcgtc ataatactgc agtgtctcaa ttaactaagg 1680

ctaaggaagc tctaattgca gcttctgaga ctctcaaaga aaggaaagct gcaatcagag 1740

atatagaagg aaaactccct caaactgaac aagaattaaa ggagaaagaa aaagaacttc 1800

aaaaacttac acaagaagaa acaaacttta aaagtttggt tcatgatctc tttcaaaaag 1860

ttgaagaagc aaagagctca ttagcaatga atcgaagtag ggggaaagtc cttgatgcaa 1920

taattcaaga aaaaaaatct ggcaggattc caggaatata tggaagattg ggggacttag 1980

gagccattga tgaaaaatac gacgtggcta tatcatcctg ttgtcatgca ctggactaca 2040

ttgttgttga ttctattgat atagcccaag aatgtgtaaa cttccttaaa agacaaaata 2100

ttggagttgc aacctttata ggtttagata agatggctgt atgggcgaaa aagatgaccg 2160

aaattcaaac tcctgaaaat actcctcgtt tatttgattt agtaaaagta aaagatgaga 2220

aaattcgcca agctttttat tttgctttac gagatacctt agtagctgac aacttggatc 2280

aagccacaag agtagcatat caaaaagata gaagatggag agtggtaact ttacagggac 2340

aaatcataga acagtcaggt acaatgactg gtggtggaag caaagtaatg aaaggaagaa 2400

tgggttcctc acttgttatt gaaatctctg aagaagaggt aaacaaaatg gaatcacagt 2460

tgcaaaacga ctctaaaaaa gcaatgcaaa tccaagaaca gaaagtacaa cttgaagaaa 2520

gagtagttaa gttacggcat agtgaacgag aaatgaggaa cacactagaa aaatttactg 2580

caagcatcca gcgtttaata gagcaagaag aatatttgaa tgtccaagtt aaggaacttg 2640

aagctaatgt acttgctaca gcccctgaca aaaaaaagca gaaattgcta gaagaaaacg 2700

ttagtgcttt caaaacagaa tatgatgctg tggctgagaa agctggtaaa gtagaagctg 2760

aggttaaacg cttacacaat accatcgtag aaatcaataa tcataaactc aaggcccaac 2820

aagacaaact tgataaaata aataagcaat tagatgaatg tgcttctgct attactaaag 2880

cccaagtagc aatcaagact gctgacagaa accttcaaaa ggcacaagac tctgtcttgc 2940

gtacagagaa agaaataaaa gatactgaga aagaggtgga tgacctaaca gcagagctga 3000

aaagtcttga ggacaaagca gcagaggtcg taaagaatac aaatgctgca gaggaatcct 3060

taccagagat ccagaaagaa catcgcaatc tgcttcaaga attaaaagtt attcaagaaa 3120

atgaacatgc tcttcaaaaa gatgcactta gtattaagtt gaaacttgaa caaatagatg 3180

gtcacattgc tgaacataat tctaaaataa aatattggca caaagagatt tcaaaaatat 3240

cactgcatcc tatagaagat aatcctattg aagagatttc ggttctaagc ccagaggatc 3300

ttgaagcgat caagaatcca gattctataa caaatcaaat tgcacttttg gaagcccggt 3360

gtcatgaaat gaaaccaaac ctcggtgcca tcgcagagta taaaaagaag gaagaattgt 3420

atttgcaacg ggtagcagaa ttggacaaaa ttacttatga aagagacagt tttagacagg 3480

catatgaaga tcttcggaaa caaaggctta atgaatttat ggcaggtttt tatataataa 3540

caaataaatt aaaggaaaat taccaaatgc ttactttggg aggggacgcc gaactcgagc 3600

ttgtagacag cttggatcct ttctctgaag gaatcatgtt cagtgttcga ccacctaaga 3660

aaagttggaa aaagatcttc aacctttcgg gaggagagaa aacacttagt tcattggctt 3720

tagtatttgc tcttcaccac tacaagccca ctccccttta cttcatggat gagattgatg 3780

cagcccttga ttttaaaaat gtgtccattg ttgcatttta tatatatgaa caaacaaaaa 3840

atgcacagtt cataataatt tctcttcgaa ataatatgtt tgagatttcg gatagactta 3900

ttggaattta caagacatac aacataacaa aaagtgttgc tgtaaatcca aaagaaattg 3960

catctaaggg actttgttga actttatgct gaagattctt caagttgatt cagtgtatta 4020

ctgatttttt tctatttgta aaggattatg agttgtataa aatacatact ccctaaacta 4080

gatcatgaaa ctggtttctg ttttatgcag ttgtcatttg taaagtctaa taaaatattc 4140

tctataattg cttctagatt acaaaaatat gacaatcttg taagtagcag actatggaga 4200

aaaatgagtt acctggaggg tcaggtaact tgccaaacta aaaagtatgt tagttgaggc 4260

aaagtcctaa gcaaggttgt gctatcaagg ctcagcatac cttcgtgggc ctttgattta 4320

ccaacactgg aaatgcctgc caactaatct tggatagatt ctttaaggca ttccacttag 4380

cttgccagtt gagacaatca ccacagttat tacccaaata ctatgaacat atttttgtaa 4440

accagtcatt ctgaattata gtgatgagaa tttaaatata tgcttttcta gaatttgatg 4500

tttgaccatt tatgacttaa ttaccagaga gccagtaaat taggacagtg tttcaacaag 4560

cctaggctat ctcgtaagtt gaaaaatatc ccactatagt tgcttcatga gtatgaagta 4620

agatggcctc tgatttacac tggttcaatt tacaaatttt caactttatg ataggtttat 4680

ccgggtacta aatgcatttc aacttgatag tttcaactta tgataggttt accaggatgt 4740

agtcccactg ttgaggagca tctatttagg ggttaattac tttagtaata agtggaaagt 4800

aagatacctt gagtaatgtt tgcctataaa attgtcagcg tatttttaca ctattggctc 4860

aagaatgtta taatgctaag ggacataagt tggcaaccac ttggtttttg gaaggacttt 4920

cggtattgta ttagaagtct gccctagctg ttaaatttct gggtatttat cctaaggaat 4980

taattaaaga gttaattgtt cctttcttca gtgggccatt gttttagata tttaaaaaat 5040

ccaacagttt ctatcataat gtaactgtaa aaatgtaaac acattattag catggacttt 5100

taaataaaga tttaaagaaa gcaaaaaaaa aaaaaaaaaa aa 5142

<210> 108
<211> 5297
<212> DNA
<213> Homo sapiens

<400> 108
caacaaacta agcagttgct caatcgttga ttgatcctca ggcacgtcta gattttccca 60

cttacatagg tcttgttaag aaaccagtcc tgccttcttg ccactcgtgt ctttcgatgg 120

ctcccttccc gaagtcccgc tgcctctaag cggagtgtta agcggggctc tcggaagccg 180

gtggaccgag atttccccgg gtggggcggg cgcggtgtag cggcccgcgg gctgacttgc 240

tcccggctgt cccccggccc cagcgaccat gccccgtaaa ggcacccagc cctccactgc 300

ccggcgcaga gaggaagggc cgccgccgcc gtcccctgac ggcgccagca gcgacgcgga 360

gcctgagccg ccgtccggcc gcacggagag cccagccacc gccgcagaga ctgcaagtga 420

ggaacttgat aatagaagtt tagaagagat tttgaacagc attcctcctc ccccgcctcc 480

agcaatgacc aatgaagctg gagctcctcg gcttatgata actcatattg taaaccagaa 540

cttcaaatcc tatgctgggg agaaaattct gggacctttc cataagcgct tttcctgtat 600

tatcgggcca aatggcagtg gcaaatccaa tgttattgat tctatgcttt ttgtgtttgg 660

ctatcgagca caaaaaataa gatctaaaaa actctcagta ttaatacata attctgatga 720

acacaaggac attcagagtt gtacagtaga agttcatttt caaaagataa ttgataagga 780

aggggatgat tatgaagtca ttcctaacag taatttctat gtatccagaa cggcctgcag 840

agataatact tctgtctatc acataagtgg aaagaaaaag acatttaagg atgttggaaa 900

tcttcttcga agccatggaa ttgacttgga ccataataga tttttaattt tacagggtga 960

agttgaacaa attgctatga tgaaaccaaa aggccagact gaacacgatg agggtatgct 1020

tgaatattta gaagatataa ttggttgtgg acggctaaat gaacctatta aagtcttgtg 1080

tcggagagtt gaaatattaa atgaacacag aggagagaag ttaaacaggg taaagatggt 1140

ggaaaaggaa aaggatgcct tagaaggaga gaaaaacata gctatcgaat ttcttacctt 1200

ggaaaatgaa atatttagaa aaaagaatca tgtttgtcaa tattatattt atgagttgca 1260

gaaacgaatt gctgaaatgg aaactcaaaa ggaaaaaatt catgaagata ccaaagaaat 1320

taatgagaag agcaatatac tatcaaatga aatgaaagct aagaataaag atgtaaaaga 1380

tacagaaaag aaactgaata aaattacaaa atttattgag gagaataaag aaaaatttac 1440

acagctagat ttggaagatg ttcaagttag agaaaagtta aaacatgcca cgagtaaagc 1500

caaaaaactg gagaaacaac ttcaaaaaga taaagaaaag gttgaagaat ttaaaagtat 1560

acctgccaag agtaacaata tcattaatga aacaacaacc agaaacaatg ccctcgagaa 1620

ggaaaaagag aaagaagaaa aaaaattaaa ggaagttatg gatagcctta aacaggaaac 1680

acaagggctt cagaaagaaa aagaaagtcg agagaaagaa cttatgggtt tcagcaaatc 1740

ggtaaatgaa gcacgttcaa agatggatgt agcccagtca gaacttgata tctatctcag 1800

tcgtcataat actgcagtgt ctcaattaac taaggctaag gaagctctaa ttgcagcttc 1860

tgagactctc aaagaaagga aagctgcaat cagagatata gaaggaaaac tccctcaaac 1920

tgaacaagaa ttaaaggaga aagaaaaaga acttcaaaaa cttacacaag aagaaacaaa 1980

ctttaaaagt ttggttcatg atctctttca aaaagttgaa gaagcaaaga gctcattagc 2040

aatgaatcga agtaggggga aagtccttga tgcaataatt caagaaaaaa aatctggcag 2100

gattccagga atatatggaa gattggggga cttaggagcc attgatgaaa aatacgacgt 2160

ggctatatca tcctgttgtc atgcactgga ctacattgtt gttgattcta ttgatatagc 2220

ccaagaatgt gtaaacttcc ttaaaagaca aaatattgga gttgcaacct ttataggttt 2280

agataagatg gctgtatggg cgaaaaagat gaccgaaatt caaactcctg aaaatactcc 2340

tcgtttattt gatttagtaa aagtaaaaga tgagaaaatt cgccaagctt tttattttgc 2400

tttacgagat accttagtag ctgacaactt ggatcaagcc acaagagtag catatcaaaa 2460

agatagaaga tggagagtgg taactttaca gggacaaatc atagaacagt caggtacaat 2520

gactggtggt ggaagcaaag taatgaaagg aagaatgggt tcctcacttg ttattgaaat 2580

ctctgaagaa gaggtaaaca aaatggaatc acagttgcaa aacgactcta aaaaagcaat 2640

gcaaatccaa gaacagaaag tacaacttga agaaagagta gttaagttac ggcatagtga 2700

acgagaaatg aggaacacac tagaaaaatt tactgcaagc atccagcgtt taatagagca 2760

agaagaatat ttgaatgtcc aagttaagga acttgaagct aatgtacttg ctacagcccc 2820

tgacaaaaaa aagcagaaat tgctagaaga aaacgttagt gctttcaaaa cagaatatga 2880

tgctgtggct gagaaagctg gtaaagtaga agctgaggtt aaacgcttac acaataccat 2940

cgtagaaatc aataatcata aactcaaggc ccaacaagac aaacttgata aaataaataa 3000

gcaattagat gaatgtgctt ctgctattac taaagcccaa gtagcaatca agactgctga 3060

cagaaacctt caaaaggcac aagactctgt cttgcgtaca gagaaagaaa taaaagatac 3120

tgagaaagag gtggatgacc taacagcaga gctgaaaagt cttgaggaca aagcagcaga 3180

ggtcgtaaag aatacaaatg ctgcagagga atccttacca gagatccaga aagaacatcg 3240

caatctgctt caagaattaa aagttattca agaaaatgaa catgctcttc aaaaagatgc 3300

acttagtatt aagttgaaac ttgaacaaat agatggtcac attgctgaac ataattctaa 3360

aataaaatat tggcacaaag agatttcaaa aatatcactg catcctatag aagataatcc 3420

tattgaagag atttcggttc taagcccaga ggatcttgaa gcgatcaaga atccagattc 3480

tataacaaat caaattgcac ttttggaagc ccggtgtcat gaaatgaaac caaacctcgg 3540

tgccatcgca gagtataaaa agaaggaaga attgtatttg caacgggtag cagaattgga 3600

caaaattact tatgaaagag acagttttag acaggcatat gaagatcttc ggaaacaaag 3660

gcttaatgaa tttatggcag gtttttatat aataacaaat aaattaaagg aaaattacca 3720

aatgcttact ttgggagggg acgccgaact cgagcttgta gacagcttgg atcctttctc 3780

tgaaggaatc atgttcagtg ttcgaccacc taagaaaagt tggaaaaaga tcttcaacct 3840

ttcgggagga gagaaaacac ttagttcatt ggctttagta tttgctcttc accactacaa 3900

gcccactccc ctttacttca tggatgagat tgatgcagcc cttgatttta aaaatgtgtc 3960

cattgttgca ttttatatat atgaacaaac aaaaaatgca cagttcataa taatttctct 4020

tcgaaataat atgtttgaga tttcggatag acttattgga atttacaaga catacaacat 4080

aacaaaaagt gttgctgtaa atccaaaaga aattgcatct aagggacttt gttgaacttt 4140

atgctgaaga ttcttcaagt tgattcagtg tattactgat ttttttctat ttgtaaagga 4200

ttatgagttg tataaaatac atactcccta aactagatca tgaaactggt ttctgtttta 4260

tgcagttgtc atttgtaaag tctaataaaa tattctctat aattgcttct agattacaaa 4320

aatatgacaa tcttgtaagt agcagactat ggagaaaaat gagttacctg gagggtcagg 4380

taacttgcca aactaaaaag tatgttagtt gaggcaaagt cctaagcaag gttgtgctat 4440

caaggctcag cataccttcg tgggcctttg atttaccaac actggaaatg cctgccaact 4500

aatcttggat agattcttta aggcattcca cttagcttgc cagttgagac aatcaccaca 4560

gttattaccc aaatactatg aacatatttt tgtaaaccag tcattctgaa ttatagtgat 4620

gagaatttaa atatatgctt ttctagaatt tgatgtttga ccatttatga cttaattacc 4680

agagagccag taaattagga cagtgtttca acaagcctag gctatctcgt aagttgaaaa 4740

atatcccact atagttgctt catgagtatg aagtaagatg gcctctgatt tacactggtt 4800

caatttacaa attttcaact ttatgatagg tttatccggg tactaaatgc atttcaactt 4860

gatagtttca acttatgata ggtttaccag gatgtagtcc cactgttgag gagcatctat 4920

ttaggggtta attactttag taataagtgg aaagtaagat accttgagta atgtttgcct 4980

ataaaattgt cagcgtattt ttacactatt ggctcaagaa tgttataatg ctaaggacca 5040

taagttggca accacttggt ttttggaagg actttcggta ttgtattaga agtctgccct 5100

agctgttaaa tttctgggta tttatcctaa ggaattaatt aaagagttaa ttgttccttt 5160

cttcagtggg ccattgtttt agatatttaa aaaatccaac agtttctatc ataatgtaac 5220

tgtaaaaatg taaacacatt attagcatgg acttttaaat aaagatttaa agaaagcaaa 5280

aaaaaaaaaa aaaaaaa 5297

<210> 109
<211> 2972
<212> DNA
<213> Homo sapiens

<400> 109
tcttcggacc taggctgccc tgccgtcatg tcgcaaggga tcctttctcc gccagcgggc 60

ttgctgtccg atgacgatgt cgtagtttct cccatgtttg agtccacagc tgcagatttg 120

gggtctgtgg tacgcaagaa cctgctatca gactgctctg tcgtctctac ctccctagag 180

gacaagcagc aggttccatc tgaggacagt atggagaagg tgaaagtata cttgagggtt 240

aggcccttgt taccttcaga gttggaacga caggaagatc agggttgtgt ccgtattgag 300

aatgtggaga cccttgttct acaagcaccc aaggactcgt ttgccctgaa gagcaatgaa 360

cggggaattg gccaagccac acacaggttc accttttccc agatctttgg gccagaagtg 420

ggacaggcat ccttcttcaa cctaactgtg aaggagatgg taaaggatgt actcaaaggg 480

cagaactggc tcatctatac atatggagtc actaactcag ggaaaaccca cacgattcaa 540

ggtaccatca aggatggagg gattctcccc cggtccctgg cgctgatctt caatagcctc 600

caaggccaac ttcatccaac acctgatctg aagcccttgc tctccaatga ggtaatctgg 660

ctagacagca agcagatccg acaggaggaa atgaagaagc tgtccctgct aaatggaggc 720

ctccaagagg aggagctgtc cacttccttg aagaggagtg tctacatcga aagtcggata 780

ggtaccagca ccagcttcga cagtggcatt gctgggctct cttctatcag tcagtgtacc 840

agcagtagcc agctggatga aacaagtcat cgatgggcac agccagacac tgccccacta 900

cctgtcccgg caaacattcg cttctccatc tggatctcat tctttgagat ctacaacgaa 960

ctgctttatg acctattaga accgcctagc caacagcgca agaggcagac tttgcggcta 1020

tgcgaggatc aaaatggcaa tccctatgtg aaagatctca actggattca tgtgcaagat 1080

gctgaggagg cctggaagct cctaaaagtg ggtcgtaaga accagagctt tgccagcacc 1140

cacctcaacc agaactccag ccgcagtcac agcatcttct caatcaggat cctacacctt 1200

cagggggaag gagatatagt ccccaagatc agcgagctgt cactctgtga tctggctggc 1260

tcagagcgct gcaaagatca gaagagtggt gaacggttga aggaagcagg aaacattaac 1320

acctctctac acaccctggg ccgctgtatt gctgcccttc gtcaaaacca gcagaaccgg 1380

tcaaagcaga acctggttcc cttccgtgac agcaagttga ctcgagtgtt ccaaggtttc 1440

ttcacaggcc gaggccgttc ctgcatgatt gtcaatgtga atccctgtgc atctacctat 1500

gatgaaactc ttcatgtggc caagttctca gccattgcta gccagcttgt gcatgcccca 1560

cctatgcaac tgggattccc atccctgcac tcgttcatca aggaacatag tcttcaggta 1620

tcccccagct tagagaaagg ggctaaggca gacacaggcc ttgatgatga tattgaaaat 1680

gaagctgaca tctccatgta tggcaaagag gagctcctac aagttgtgga agccatgaag 1740

acactgcttt tgaaggaacg acaggaaaag ctacagctgg agatgcatct ccgagatgaa 1800

atttgcaatg agatggtaga acagatgcaa cagcgggaac agtggtgcag tgaacatttg 1860

gacacccaaa aggaactatt ggaggaaatg tatgaagaaa aactaaatat cctcaaggag 1920

tcactgacaa gtttttacca agaagagatt caggagcggg atgaaaagat tgaagagcta 1980

gaagctctct tgcaggaagc cagacaacag tcagtggccc atcagcaatc agggtctgaa 2040

ttggccctac ggcggtcaca aaggttggca gcttctgcct ccacccagca gcttcaggag 2100

gttaaagcta aattacagca gtgcaaagca gagctaaact ctaccactga agagttgcat 2160

aagtatcaga aaatgttaga accaccaccc tcagccaagc ccttcaccat tgatgtggac 2220

aagaagttag aagagggcca gaagaatata aggctgttgc ggacagagct tcagaaactt 2280

ggtgagtctc tccaatcagc agagagagct tgttgccaca gcactggggc aggaaaactt 2340

cgtcaagcct tgaccacttg tgatgacatc ttaatcaaac aggaccagac tctggctgaa 2400

ctgcagaaca acatggtgct agtgaaactg gaccttcgga agaaggcagc atgtattgct 2460

gagcagtatc atactgtgtt gaaactccaa ggccaggttt ctgccaaaaa gcgccttggt 2520

accaaccagg aaaatcagca accaaaccaa caaccaccag ggaagaaacc attccttcga 2580

aatttacttc cccgaacacc aacctgccaa agctcaacag actgcagccc ttatgcccgg 2640

atcctacgct cacggcgttc ccctttactc aaatctgggc cttttggcaa aaagtactaa 2700

ggctgtgggg aaagagaaga gcagtcatgg ccctgaggtg ggtcagctac tctcctgaag 2760

aaataggtct cttttatgct ttaccatata tcaggaatta tatccaggat gcaatactca 2820

gacactagct tttttctcac ttttgtatta taaccaccta tgtaatctca tgttgttgtt 2880

tttttttatt tacttatatg atttctatgc acacaaaaac agttatatta aagatattat 2940

tgttcacatt ttttattgaa aaaaaaaaaa aa 2972

<210> 110
<211> 1149
<212> DNA
<213> Homo sapiens

<400> 110
   ccgcagccat gaccccgcag cttctcctgg cccttgtcct ctgggccagc tgcccgccct 60
   gcagtggaag gaaagggccc ccagcagctc tgacactgcc ccgggtgcaa tgccgagcct 120
   ctcggtaccc gatcgccgtg gattgctcct ggaccctgcc gcctgctcca aactccacca 180
   gccccgtgtc cttcattgcc acgtacaggc tcggcatggc tgcccggggc cacagctggc 240
   cctgcctgca gcagacgcca acgtccacca gctgcaccat cacggatgtc cagctgttct 300
   ccatggctcc ctacgtgctc aatgtcaccg ccgtccaccc ctggggctcc agcagcagct 360
   tcgtgccttt cataacagag cacatcatca agcccgaccc tccagaaggc gtgcgcctaa 420
   gccccctcgc tgagcgccag ctacaggtgc agtgggagcc tcccgggtcc tggcccttcc 480
   cagagatctt ctcactgaag tactggatcc gttacaagcg tcagggagct gcgcgcttcc 540
   accgggtggg gcccattgaa gccacgtcct tcatcctcag ggctgtgcgg ccccgagcca 600
   ggtactacgt ccaagtggcg gctcaggacc tcacagacta cggggaactg agtgactgga 660
   gtctccccgc cactgccaca atgagcctgg gcaagtagca agggcttccc gctgcctcca 720
   gacagcacct gggtcctcgc caccctaagc cccgggacac ctgttggagg gcggatggga 780
   tctgcctagc ctgggctgga gtccttgctt tgctgctgct gagctgccgg gcaacctcag 840
   atgaccgact tttccctttg agcctcagtt tctctagctg agaaatggag atgtactact 900
   ctctccttta cctttacctt taccacagtg cagggctgac tgaactgtca ctgtgagata 960
   ttttttattg tttaattaga aaagaattgt tgttgggctg ggcgcagtgg atcgcacctg 1020
   taatcccagt cactgggaag ccgacgtggg agggtagctt gaggccagga gctcgaaacc 1080
   agtccgggcc acacagcaag accccatctc taaaaaatta atataaatat aaaataaaaa 1140
   aaaaaaaaa 1149

<210> 111
<211> 2663
<212> DNA
<213> Homo sapiens

<400> 111
cgcgcgcgcc atttctagtc gttttcaaag cgcctcgcgc tgattctcac gggcccggct 60

gccggccccc gctctgccct gcataataaa atggctaatc aggtgaatgg taatgcggta 120

cagttaaaag aagaggaaga accaatggat acttccagtg taactcacac agaacactac 180

aagacactga tagaggcagg cctcccacag aaggtggcag aaagacttga tgaaatattt 240

cagacaggat tggtagctta tgtcgatctt gatgaaagag caattgatgc tctcagggaa 300

tttaatgaag aaggagctct gtctgtacta cagcagttca aggaaagtga cttatcacat 360

gttcagaaca aaagtgcatt tttatgtgga gttatgaaga cctacaggca gagagagaaa 420

caggggagca aggtgcaaga gtccacaaag ggacctgatg aagcgaagat caaggccttg 480

cttgagagaa ctggttatac tctggatgta accacaggac agaggaagta tggtggtcct 540

ccaccagaca gtgtgtactc tggcgtgcaa cctggaattg gaacggaggt atttgtaggc 600

aaaataccaa gggatttata tgaggatgag ttggtgcccc tttttgagaa ggccggaccc 660

atttgggatc tacgtcttat gatggatcca ctgtccggtc agaatagagg gtatgcattt 720

atcaccttct gtggaaagga agctgcacag gaagccgtga aactgtgtga cagctatgaa 780

attcgccctg gtaaacacct tggagtgtgc atttctgtgg caaacaacag actttttgtt 840

ggatccattc cgaagaataa gactaaagaa aacattttgg aagaattcag taaagtcaca 900

gagggtttgg tggacgttat tctctatcat caacccgatg acaaaaagaa gaatcggggg 960

ttctgcttcc ttgaatatga ggatcacaag tcagcagcac aagccagacg ccggctgatg 1020

agtggaaaag taaaagtgtg gggaaatgta gttacagttg aatgggctga ccctgtggaa 1080

gaaccagatc cagaagtcat ggctaaggta aaagttttgt ttgtgagaaa cttggctact 1140

acggtgacag aagaaatatt ggaaaagtca ttttctgaat ttggaaaact cgaaagagta 1200

aagaagttga aagattatgc atttgttcat tttgaagaca gaggagcagc tgttaaggct 1260

atggatgaaa tgaatggcaa agaaatagaa ggggaagaaa ttgaaatagt cttagccaag 1320

ccaccagaca agaaaaggaa agagcgccaa gctgctagac aggcctccag aagcactgcg 1380

tatgaagatt attactacca ccctcctcct cgcatgccac ctccaattag aggtcggggt 1440

cgtggtgggg ggagaggtgg atatggctac cctccagatt actacggcta tgaagattac 1500

tatgatgatt actatggtta tgattatcac gactatcgtg gaggctatga agatccctac 1560

tacggctatg atgatggcta tgcagtaaga ggaagaggag gaggaagggg agggcgaggt 1620

gctccaccac caccaagggg gaggggagca ccacctccaa gaggtagagc tggctattca 1680

cagagggggg cacctttggg accaccaaga ggctctaggg gtggcagagg gggtcctgct 1740

caacagcaga gaggccgtgg ttcccgtgga tctcggggca atcgtggggg caatgtagga 1800

ggcaagagaa aggcagatgg gtacaaccag cctgattcca agcgtcgtca gaccaacaac 1860

caacagaact ggggttccca acccatcgct cagcagccgc ttcagcaagg tggtgactat 1920

tctggtaact atggttacaa taatgacaac caggaatttt atcaggatac ttatgggcaa 1980

cagtggaagt agacaagtaa gggcttgaaa atgatactgg caagatacga ttggctctag 2040

atctacattc ttcaaaaaaa aaaattggct taactgtttc atctttaagt agcattttgc 2100

tgccatttgt attgggctga agaaatcact attgtgtata tactcaagtc tttttatttt 2160

tcctcttttc ataaatgctc ttggacatta ttgggcttgc agagttccct tattctgggg 2220

attacaatgc ttttatcgtt tcaggcttca ttttagcttc aaaacaagct gggcacactg 2280

ttaaatcatg attttgcaga acctttggtt ttggacagtt tcattttttt ggatttggga 2340

tagattacat aggagtatgg agtatgctgt aaataaaaat acaagctagt gctttgtctt 2400

agtagtttta agaaattaaa gcaaacaaat ttaagttttc ttgtattgaa aataacctat 2460

gattgtatgt tttgcattcc tagaagtagg ttaactgtgt ttttaaattg ttataacttc 2520

acaccttttt gaaatctgcc ctacaaaatt tgtttggctt aaacgtcaaa agccgtgaca 2580

atttgttctt tgatgtgatt gtatttccaa tttcttgttc atgtaagatt tcaataaaac 2640

taaaaaatct attcaaaaca tta 2663

<210> 112
<211> 1591
<212> DNA
<213> Homo sapiens

<400> 112
atataagcgt tggcggagcg tcggttgtag cactctgcgc gcccgctctt ctgctgctgt 60

ttgtctactt cctcctgctt ccccgccgcc gccgccgcca tcatgaggga aatcgtgcac 120

ttgcaggccg ggcagtgcgg caaccaaatc ggcgccaagt tttgggaggt gatcagcgat 180

gagcacggca tcgaccccac gggcacctac cacggggaca gcgacctgca gctggaacgc 240

atcaacgtgt actacaatga ggccaccggc ggcaagtacg tgccccgcgc cgtgctcgtg 300

gatctggagc ccggcaccat ggactccgtg cgctcggggc ccttcggcca gatcttccgg 360

ccggacaact tcgttttcgg tcagagtggt gctgggaaca actgggccaa ggggcactac 420

acagaaggcg cggagctggt ggactcggtg ctggatgttg tgagaaagga ggctgagagc 480

tgtgactgcc tgcagggttt ccagctgacc cactccctgg gtggggggac tgggtctggg 540

atgggtaccc tcctcatcag caagatccgg gaggagtacc cagacaggat catgaacacg 600

tttagtgtgg tgccttcgcc caaagtgtca gacacagtgg tggagcccta caacgccacc 660

ctctcagtcc accagctcgt agaaaacaca gacgagacct actgcattga taacgaagct 720

ctctacgaca tttgcttcag aaccctaaag ctgaccacgc ccacctatgg tgacctgaac 780

cacctggtgt ctgctaccat gagtggggtc accacctgcc tgcgcttccc aggccagctc 840

aatgctgacc tgcggaagct ggctgtgaac atggtcccgt ttccccggct gcacttcttc 900

atgcccggct ttgccccact gaccagccgg ggcagccagc agtaccgggc gctgaccgtg 960

cccgagctca cccagcagat gtttgatgcc aagaacatga tggctgcctg cgacccccgc 1020

catggccgct acctgacggt tgccgccgtg ttcaggggcc gcatgtccat gaaggaggtg 1080

gatgagcaaa tgcttaatgt ccaaaacaaa aacagcagct attttgttga gtggatcccc 1140

aacaatgtga aaacggctgt ctgtgacatc ccacctcggg ggctaaaaat gtccgccacc 1200

ttcattggca acagcacggc catccaggag ctgttcaagc gcatctccga gcagttcacg 1260

gccatgttcc ggcgcaaggc cttcctgcac tggtacacgg gcgagggcat ggacgagatg 1320

gagttcaccg aggccgagag caacatgaat gacctggtgt ccgagtacca gcagtaccag 1380

gatgccacag ccgaggagga gggcgagttc gaggaggagg ctgaggagga ggtggcctag 1440

agccttcagt cactggggaa agcagggaag cagtgtgaac tctttattca ctcccagcct 1500

gtcctgtggc ctgtcccact gtgtgcactt gctgttttcc ctgtccacat ccatgctgta 1560

cagacaccac cattaaagca ttttcatagt g 1591

<210> 113
<211> 2150
<212> DNA
<213> Homo sapiens

<400> 113
ctcgagccac gaaggccccg ctgtcctgtc tagcagatac ttgcacggtt tacagaaatt 60

cggtccctgg gtcgtgtcag gaaactggaa aaaaggtcat aagcatgaag cgcagttcag 120

tttccagcgg tggtgctggc cgcctctcca tgcaggagtt aagatcccag gatgtaaata 180

aacaaggcct ctatacccct caaaccaaag agaaaccaac ctttggaaag ttgagtataa 240

acaaaccgac atctgaaaga aaagtctcgc tatttggcaa aagaactagt ggacatggat 300

cccggaatag tcaacttggt atattttcca gttctgagaa aatcaaggac ccgagaccac 360

ttaatgacaa agcattcatt cagcagtgta ttcgacaact ctgtgagttt cttacagaaa 420

atggttatgc acataatgtg tccatgaaat ctctacaagc tccctctgtt aaagacttcc 480

tgaagatctt cacatttctt tatggcttcc tgtgcccctc atacgaactt cctgacacaa 540

agtttgaaga agaggttcca agaatcttta aagaccttgg gtatcctttt gcactatcca 600

aaagctccat gtacacagtg ggggctcctc atacatggcc tcacattgtg gcagccttag 660

tttggctaat agactgcatc aagatacata ctgccatgaa agaaagctca cctttatttg 720

atgatgggca gccttgggga gaagaaactg aagatggaat tatgcataat aagttgtttt 780

tggactacac cataaaatgc tatgagagtt ttatgagtgg tgccgacagc tttgatgaga 840

tgaatgcaga gctgcagtca aaactgaagg atttatttaa tgtggatgct tttaagctgg 900

aatcattaga agcaaaaaac agagcattga atgaacagat tgcaagattg gaacaagaaa 960

gagaaaaaga accgaatcgt ctagagtcgt tgagaaaact gaaggcttcc ttacaaggag 1020

atgttcaaaa gtatcaggca tacatgagca atttggagtc tcattcagcc attcttgacc 1080

agaaattaaa tggtctcaat gaggaaattg ctagagtaga actagaatgt gaaacaataa 1140

aacaggagaa cactcgacta cagaatatca ttgacaacca gaagtactca gttgcagaca 1200

ttgagcgaat aaatcatgaa agaaatgaat tgcagcagac tattaataaa ttaaccaagg 1260

acctggaagc tgaacaacag aagttgtgga atgaggagtt aaaatatgcc agaggcaaag 1320

aagcgattga aacacaatta gcagagtatc acaaattggc tagaaaatta aaacttattc 1380

ctaaaggtgc tgagaattcc aaaggttatg actttgaaat taagtttaat cccgaggctg 1440

gtgccaactg ccttgtcaaa tacagggctc aagtttatgt acctcttaag gaactcctga 1500

atgaaactga agaagaaatt aataaagccc taaataaaaa aatgggtttg gaggatactt 1560

tagaacaatt gaatgcaatg ataacagaaa gcaagagaag tgtgagaact ctgaaagaag 1620

aagttcaaaa gctggatgat ctttaccaac aaaaaattaa ggaagcagag gaagaggatg 1680

aaaaatgtgc cagtgagctt gagtccttgg agaaacacaa gcacctgcta gaaagtactg 1740

ttaaccaggg gctcagtgaa gctatgaatg aattagatgc tgttcagcgg gaataccaac 1800

tagttgtgca aaccacgact gaagaaagac gaaaagtggg aaataacttg caacgtctgt 1860

tagagatggt tgctacacat gttgggtctg tagagaaaca tcttgaggag cagattgcta 1920

aagttgatag agaatatgaa gaatgcatgt cagaagatct ctcggaaaat attaaagaga 1980

ttagagataa gtatgagaag aaagctactc taattaagtc ttctgaagaa tgaagataaa 2040

atgttgatca tgtatatata tccatagtga ataaaattgt ctcagtaaaa aaaaaaaaaa 2100

aaaaaaaaaa aaaaaaaaaa aaaaaaaaaa aaaaaaaaaa aaaaaaaaaa 2150

<210> 114
<211> 1992
<212> DNA
<213> Homo sapiens

<400> 114
cgcgcgccgg agcgcgctag ccgcattgcg agccgaaccc gggagctggc gccatggtgc 60

tgttgcacgt gctgtttgag cacgcggtcg gctacgcgct gctggcgctg aaggaagtgg 120

aggagatcag tctgctgcag ccgcaggtgg aggagtctgt gctcaacctg ggcaaattcc 180

acagcatcgt tcgtctggtg gccttttgtc cctttgcctc atcccaggtt gccttggaaa 240

atgccaacgc cgtgtctgaa ggggttgttc atgaggacct ccgcctgctc ttggagaccc 300

acctgccgtc caaaaagaag aaagtactct tgggagttgg ggatcccaag attggtgccg 360

caatacagga ggagttaggg tacaactgcc agactggagg agtcatagct gagatcctgc 420

gaggagttcg tctgcacttc cacaatctgg tgaagggtct gaccgatctg tcagcttgta 480

aagcacagct ggggctggga cacagctatt cccgtgccaa agttaagttt aatgtgaacc 540

gggtggacaa tatgatcatc cagtccatta gcctcctgga ccagctggat aaggacatca 600

ataccttctc tatgcgtgtc agggagtggt acgggtatca ctttccggag ctggtgaaga 660

tcatcaacga caatgccaca tactgccgtc ttgcccagtt tattggaaac cgaagggaac 720

tgaatgagga caagctggag aagctggagg agctgacaat ggatggggcc aaggctaagg 780

ctattctgga tgcctcacgg tcctccatgg gcatggacat atctgccatt gacttgataa 840

acatcgagag cttctccagt cgtgtggtgt ctttatctga ataccgccag agcctacaca 900

cttacctgcg ctccaagatg agccaagtag cccccagcct gtcagcccta attggggaag 960

cggtaggtgc acgtctcatc gcacatgctg gcagcctcac caacctggcc aagtatccag 1020

catccacagt gcagatcctt ggggctgaaa aggccctgtt cagagccctg aagacaaggg 1080

gtaacactcc aaaatatgga ctcattttcc actccacctt cattggccga gcagctgcca 1140

agaacaaagg ccgcatctcc cgatacctgg caaacaaatg cagtattgcc tcacgaatcg 1200

attgcttctc tgaggtgccc acgagtgtat tcggggagaa gcttcgagaa caagttgaag 1260

agcgactgtc cttctatgag actggagaga taccacgaaa gaatctggat gtcatgaagg 1320

aagcaatggt tcaggcagag gaagcggctg ctgagattac taggaagctg gagaaacagg 1380

agaagaaacg cttaaagaag gaaaagaaac ggctggctgc acttgccctc gcgtcttcag 1440

aaaacagcag tagtactcca gaggagtgtg aggagatgag tgaaaaaccc aaaaagaaga 1500

aaaagcaaaa gccccaggag gttcctcagg agaatggaat ggaagaccca tctatctctt 1560

tctccaaacc caagaaaaag aaatcttttt ccaaggagga gttgatgagt agcgatcttg 1620

aagagaccgc tggcagcacc agtattccca agaggaagaa gtctacaccc aaggaggaaa 1680

cagttaatga ccctgaggag gcaggccaca gaagtggctc caagaaaaag aggaaattct 1740

ccaaagagga gccggtcagc agtgggcctg aagaggcggt tggcaagagc agctccaaga 1800

agaagaaaaa gttccataaa gcatcccagg aagattagaa tgcaaatgga cattctctgg 1860

gaggtggggc ataccatagc ccaaggtgac atttcccacc ctgtgccgtg ttccccaata 1920

aaaacaaatt cacaagaaaa aaaaaaaaaa aaaaaaaaaa aaaaaaaaaa aaaaaaaaaa 1980

aaaaaaaaaa aa 1992

<210> 115
<211> 1148
<212> DNA
<213> Homo sapiens

<400> 115
gcgccgagac ccgctcctgc agtattagtt cttgcagctg gtggtggcgg ctgaggcggc 60

atggatctca gcgagctgga gagagacaat acaggccgct gtcgcctgag ttcgcctgtg 120

cccgcggtgt gccgcaagga gccttgcgtc ctgggcgtcg atgaggcggg caggggcccc 180

gtgctgggcc ccatggtcta cgccatctgt tattgtcccc tgcctcgcct ggcagatctg 240

gaggcgctga aagtggcaga ctcaaagacc ctattggaga gcgagcggga aaggctgttt 300

gcgaaaatgg aggacacgga ctttgtcggc tgggcgctgg atgtgctgtc tccaaacctc 360

atctctacca gcatgcttgg gcgggtcaaa tacaacctga actccctgtc acatgataca 420

gccactgggc ttatacagta tgcattggac cagggcgtga acgtcaccca ggtattcgtg 480

gacaccgtag ggatgccaga gacataccag gcgcggctgc agcaaagttt tcccgggatt 540

gaggtgacgg tcaaggccaa agcagatgcc ctctacccgg tggttagtgc tgccagcatc 600

tgtgccaagg tggcccggga ccaggccgtg aagaaatggc agttcgtgga gaaactgcag 660

gacttggata ctgattatgg ctcaggctac cccaatgatc ccaagacaaa agcgtggttg 720

aaggagcacg tggagcctgt gttcggcttc ccccagtttg tccggttcag ctggcgcacg 780

gcccagacca tcctggagaa agaggcggaa gatgttatat gggaggactc agcatccgag 840

aatcaggagg gactcaggaa gatcacatcc tacttcctca atgaagggtc ccaagcccgt 900

ccccgttctt cccaccgata tttcctggaa cgcggcctgg agtcagcaac cagcctctag 960

cagctgcctc tacgcgctct acctgcttcc ccaacccaga cattaaaatt gtttaaggag 1020

aaccacacgt aggggatgta cttttgggac agaagcaagg tgggagtgtg ctctgcagcc 1080

gggtccagct acttcctttt ggaaccttaa atagaatggg tgttggttga ttaattttat 1140

ttaaaaaa 1148

<210> 116
<211> 1942
<212> DNA
<213> Homo sapiens

<400> 116
gagcatggag gcgggggcgg ctgaggcagc tgtagcggcc gtggaggagg tcggctcagc 60

cgggcagttt gaggagctgc tgcgcctcaa agccaagtcc ctccttgtgg tccatttctg 120

ggcaccatgg gctccacagt gtgcacagat gaacgaagtt atggcagagt tagctaaaga 180

actccctcaa gtttcatttg tgaagttgga agctgaaggt gttcctgaag tatctgaaaa 240

atatgaaatt agctctgttc ccacttttct gtttttcaag aattctcaga aaatcgaccg 300

attagatggt gcacatgccc cagagttgac caaaaaagtt cagcgacatg catctagtgg 360

ctccttccta cccagcgcta atgaacatct taaagaagat ctcaaccttc gcttgaagaa 420

attgactcat gctgccccct gcatgctgtt tatgaaagga actcctcaag aaccacgctg 480

tggtttcagc aagcagatgg tggaaattct tcacaaacat aatattcagt ttagcagttt 540

tgatatcttc tcagatgaag aggttcgaca gggactcaaa gcctattcca gttggcctac 600

ctatcctcag ctctatgttt ctggagagct cataggagga cttgatataa ttaaggagct 660

agaagcatct gaagaactag atacaatttg tcccaaagct cccaaattag aggaaaggct 720

caaagtgctg acaaataaag cttctgtgat gctctttatg aaaggaaaca aacaggaagc 780

aaaatgtgga ttcagcaaac aaattctgga aatactaaat agtactggtg ttgaatatga 840

aacattcgat atattggagg atgaagaagt tcggcaagga ttaaaagctt actcaaattg 900

gccaacatac cctcagctgt atgtgaaagg ggagctggtg ggaggattgg atattgtgaa 960

ggaactgaaa gaaaatggtg aattgctgcc tatactgaga ggagaaaatt aataaatctt 1020

aaacttggtg cccaactatt acggggtctg gctctgtcac ccaggctgga gtgcagtggc 1080

acgattatgg ctcattgcag cctcgacttc tcggggccaa gcgatcctcc tgcctcagcc 1140

ttctgagtag ctgggaccac aggcgtgcac caccatgccc acctaatttt ttatttcttg 1200

tagagatgag gtctcctgcc tcagcctccc aaagtgctgg aatttacagg agtgtcggct 1260

gtgccccttg tgatggatga ggaaaggtat ccacctttct gaatcagaca caagctgcag 1320

tagcttcctg atgcctcatt caccaggatg ggtaacttta aagacaagtt ttataactgg 1380

catcccatct ggtttcagag tcttgggggc ttttttcata cagctttgtc tgatatttag 1440

agacattcag attcagaagc catgagttca gttagctcat gggccgggca cgtgcactga 1500

ctcactggct gcagcagaac acattgaggg agtcttggtg cacttaggtc actgtaaggt 1560

tctagggctg ggagctggga tttgaaccag gatctgtcag cgcagcccca gtgctgtgct 1620

tgaagcctcg tgccacactg gctttgaggt agcacgctgc aagttagtga gtggagttgc 1680

ccaagtctgg ggcttctgaa atgacctggt atttttatgt tcctgttaga aatggtgtgg 1740

ctttcacaaa ttaagtgcca ggctgggcac aatggctcgt gcttgtaatc ccagcacttt 1800

gggaggcgga ggcaggcggg ttgcttgagc tccggagctt gagaccagcc tgagcaacat 1860

ggcgaaacct catatctaca aaaaataaaa aattaactgg tcaaaaaaaa aaaaaaaaaa 1920

aaaaaaaaaa aaaaaaaaaa aa 1942

<210> 117
<211> 2143
<212> DNA
<213> Homo sapiens

<400> 117
gaagccaaca agaatttgag aactgtaaat accaagcctt gaaagggacc atggtgcggc 60

ctgtgagaca taagaaacca gtcaattact cacagtttga ccactctgac agtgatgatg 120

attttgtttc tgcaactgta cctttaaaca agaaatccag aacagcacca aaggagttaa 180

aacaagataa accaaaacct aacttgaaca atctccggaa agaagaaatc ccagtacaag 240

agaaaacccc taaaaaaagg atggctttag atgacaagct ctaccagaga gacttagaag 300

ttgcactagc tttatcagtg aaggaacttc caacagtcac cactaatgtg cagaactctc 360

aagataaaag cattgaaaaa catggcagta gtaaaataga aacaatgaat aagtctcctc 420

atatctctaa ttgcagtgta gccagtgatt atttagattt ggataagatt actgtggaag 480

atgatgttgg tggtgttcaa gggaaaagaa aagcagcatc taaagctgca gcacagcaga 540

ggaagattct tctggaaggc agtgatggtg atagtgctaa tgacactgaa ccagactttg 600

cacctggtga agattctgag gatgattctg atttttgtga gagtgaggat aatgacgaag 660

acttctctat gagaaaaagt aaagttaaag aaattaaaaa gaaagaagtg aaggtaaaat 720

ccccagtaga aaagaaagag aagaaatcta aatccaaatg taatgctttg gtgacttcgg 780

tggactctgc tccagctgcc gtcaaatcag aatctcagtc cttgccaaaa aaggtttctc 840

tgtcttcaga taccactagg aaaccattag aaatacgcag tccttcagct gaaagcaaga 900

aacctaaatg ggtcccacca gcggcatctg gaggtagcag aagtagcagc agcccactgg 960

tggtagtgtc tgtgaagtct cccaatcaga gtctccgcct tggcttgtcc agattagcac 1020

gagttaaacc tttgcatcca aatgccacta gcacctgagt gtggtacagg aggaatgttt 1080

ggttgggaga atcacagctt tacaagggtg tttatatttg atttgtgttt atatttgagg 1140

caggtattgt aatataaagg aatccattac catgtcctat aaatgacctc tagccatttt 1200

atgattatgt tctctgtaaa actcttcaag acttcaatga gaagtttgtt tataagaatt 1260

atcttctcat acctttcctt gtgaagagcg tattctgttt ttctatcagt tcgacatgaa 1320

gtccacatca catgctgttc ttttctagtt acatgatgtg cctttctagc tttgtctagt 1380

ttatagcacc ttaactttaa ctgttcagtt ttatctggca gaggaaacat tcttatttct 1440

ttcagaagac atttctgaaa tcttataagc tacttaagct acgttgtcag ttttatcgca 1500

aagatgtttt gtattttagc caaatctttt tatagtacaa acttagaatt attttacaca 1560

ctaaaatggt tgcagtttta tggcatatgt ctccgattta gatggttgtt ctctagaaaa 1620

tagtatttaa agacatttta tgaaatcttc attgtcaaaa cctttaataa aagtggaaat 1680

attttgaaat gccctttttc ttgataccac tcatccacgt gttcctgatt gtccacattt 1740

catgataaaa tgagagctct gcagagaatg ttagcctttc tgttgtaaat gtaatcttca 1800

agtagtcact ttttgttaag ttctttagaa agtagttgtc aagtacttag tcatccctat 1860

tatgatatga gatagtacag cttttcagga agcttagatc tgaatttact ttgaaaaaca 1920

attgtaatga atattttata tttacattga gaatttcaac tagcttctga tcaattttta 1980

ataaaaaatt ttcaaatcat gttagctgtt aaaaaatgta taataactca gtttttcttg 2040

gtttatggaa atatctatat taatgtgaaa ataattaatt tagaattgtg attaaagtga 2100

gcatttgtct atcaaaaaaa aaaaaaaaaa aaaaaaaaaa aaa 2143

<210> 118
<211> 2234
<212> DNA
<213> Homo sapiens

<400> 118
   tggcgcctgc gctggacgac tcggccggta gtggagatgt ccggccggtc taagcgggag 60
   tctcgcggtt ccactcgcgg gaagcgagag tctgagtcgc ggggcagctc cggtcgcgtc 120
   aagcgggagc gagatcggga gcgggagcct gaggcggcga gctcccgggg cagccctgtg 180
   cgcgtgaagc gggagttcga gccggcgagc gcgcgcgagg ccccggcttc tgttgtcccg 240
   tttgtgcggg tgaagcggga gcgcgaggtc gatgaggact cggagcctga gcgggaggtg 300
   cgagcaaaga atggccgagt ggattctgag gaccggagga gccgccactg cccgtacctg 360
   gacaccatta acaggagtgt gctggacttt gactttgaga aactgtgttc tatctccctc 420
   tcacacatca atgcttatgc ctgtctggtg tgtggcaagt actttcaagg ccggggtttg 480
   aagtctcacg cctacattca cagtgtccag tttagccacc atgttttcct caacctccac 540
   accctcaagt ttactgcctt tccagacaac tatgagatca tcgattcctc attggaggat 600
   atcacgtatg tgttgaagcc cactttcaca aagcagcaaa ttgcaaactt ggacaagcaa 660
   gccaaattgt cccgggcata tgatggtacc acttacctgc cgggtattgt gggactgaat 720
   aacataaagg ccaatgatta tgccaacgct gtccttcagg ctctatctaa tgttcctcct 780
   ctccggaact actttctgga agaagacaat tataagaaca tcaaacgtcc tccaggggat 840
   atcatgttct tgttggtcca gcgttttgga gagctgatga gaaagctctg gaaccctcga 900
   aatttcaagg cacatgtgtc tccccatgag atgcttcagg cagttgtact ttgcagtaag 960
   aagacttttc agatcaccaa acaaggagat ggcgttgact ttctgtcttg gtttctgaat 1020
   gctctgcact cagctctggg gggcacaaag aagaaaaaga agactattgt gactgatgtt 1080
   ttccaggggt ccatgaggat cttcactaaa aagcttcccc atcctgatct gccagcagaa 1140
   gaaaaagagc agttgctcca taatgacgag taccaggaga caatggtgga gtccactttt 1200
   atgtacctga cgctggacct tcctactgcc cccctctaca aggacgagaa ggagcagctc 1260
   atcattcccc aagtgccact cttcaacatc ctggctaagt tcaatggcat cactgagaag 1320
   gaatataaga cttacaagga gaactttctg aagcgcttcc agcttaccaa gttgcctcca 1380
   tatctaatct tttgtatcaa gagattcact aagaacaact tctttgttga gaagaatcca 1440
   actattgtca atttccctat tacaaatgtg gatctgagag aatacttgtc tgaagaagta 1500
   caagcagtac acaagaatac cacctatgac ctcattgcca acatcgtgca tgacggcaag 1560
   ccctccgagg gctcctaccg gatccacgtg cttcatcatg ggacaggcaa atggtatgaa 1620
   ttacaagacc tccaggtgac tgacatcctt ccccagatga tcacactgtc agaggcttac 1680
   attcagattt ggaagaggcg agataatgat gaaaccaacc agcagggggc ttgaaggagg 1740
   cgtctagggc tttgctccca agggctgtgg ctgatgatgg taaataagaa cacagaagct 1800
   gtagctgaac acaggctggc tggtgggctt cctaggccag cccagcttgt atgggttctg 1860
   gctacaccag agcaccaaga gcccacttgc ctgggatggc cccacactgt cactcagctg 1920
   ttctttgatc atttttttct agattgatgc tcctttctcc catgcattga gctcccatct 1980
   agcttcagca gggcagaacc cttctccaga tgtgtgtaac ttatgtcttg agtatctggg 2040
   agtagttgaa gaacagataa ttccttccaa acatcaagcc ttgggattct tggagcaagc 2100
   agaaagccag taacttcgct ctgttagagg tggaggattt tcctatggtt ccccccattt 2160
   cctgatttgt atttttagat ggattaaata gtctcctgtt tttaaaccaa aaaaaaaaaa 2220
   aaaaaaaaaa aaaa 2234

<210> 119
<211> 1488
<212> DNA
<213> Homo sapiens

<400> 119
gttggtgagc atcatggcaa ccgttacagc cacaaccaaa gtcccggaga tccgtgatgt 60

aacaaggatt gagcgaatcg gtgcccactc ccacatccgg ggactggggc tggacgatgc 120

cttggagcct cggcaggctt cgcaaggcat ggtgggtcag ctggcggcac ggcgggcggc 180

tggcgtggtg ctggagatga tccgggaagg gaagattgcc ggtcgggcag tccttattgc 240

tggccagccg ggcacgggga agacggccat cgccatgggc atggcgcagg ccctgggccc 300

tgacacgcca ttcacagcca tcgccggcag tgaaatcttc tccctggaga tgagcaagac 360

cgaggcgctg acgcaggcct tccggcggtc catcggcgtt cgcatcaagg aggagacgga 420

gatcatcgaa ggggaggtgg tggagatcca gattgatcga ccagcaacag ggacgggctc 480

caaggtgggc aaactgaccc tcaagaccac agagatggag accatctacg acctgggcac 540

caagatgatt gagtccctga ccaaggacaa ggtccaggcc ggggacgtga tcaccatcga 600

caaggcgacg ggcaagatct ccaagctggg ccgctccttc acacgcgccc gcgactacga 660

cgctatgggc tcccagacca agttcgtgca gtgcccagat ggggagctcc agaaacgcaa 720

ggaggtggtg cacaccgtgt ccctgcacga gatcgacgtc atcaactctc gcacccaggg 780

cttcctggcg ctcttctcag gtgacacagg ggagatcaag tcagaagtcc gtgagcagat 840

caatgccaag gtggctgagt ggcgcgagga gggcaaggcg gagatcatcc ctggagtgct 900

gttcatcgac gaggtccaca tgctggacat cgagagcttc tccttcctca accgggccct 960

ggagagtgac atggcgcctg tcctgatcat ggccaccaac cgtggcatca cgcgaatccg 1020

gggcaccagc taccagagcc ctcacggcat ccccatagac ctgctggacc ggctgcttat 1080

cgtctccacc accccctaca gcgagaaaga cacgaagcag atcctccgca tccggtgcga 1140

ggaagaagat gtggagatga gtgaggacgc ctacacggtg ctgacccgca tcgggctgga 1200

gacgtcactg cgctacgcca tccagctcat cacagctgcc agcttggtgt gccggaaacg 1260

caagggtaca gaagtgcagg tggatgacat caagcgggtc tactcactct tcctggacga 1320

gtcccgctcc acgcagtaca tgaaggagta ccaggacgcc ttcctcttca acgaactcaa 1380

aggcgagacc atggacacct cctgagttgg atgtcatccc ccgaccccac cctgttttcc 1440

accagagttc tgacactgtg actctgtata aaatggttgg gaagctgc 1488

<210> 120
<211> 2825
<212> DNA
<213> Homo sapiens

<400> 120
gcgaaattga ggtttcttgg tattgcgcgt ttctcttcct tgctgactct ccgaatggcc 60

atggactcgt cgcttcaggc ccgcctgttt cccggtctcg ctatcaagat ccaacgcagt 120

aatggtttaa ttcacagtgc caatgtaagg actgtgaact tggagaaatc ctgtgtttca 180

gtggaatggg cagaaggagg tgccacaaag ggcaaagaga ttgattttga tgatgtggct 240

gcaataaacc cagaactctt acagcttctt cccttacatc cgaaggacaa tctgcccttg 300

caggaaaatg taacaatcca gaaacaaaaa cggagatccg tcaactccaa aattcctgct 360

ccaaaagaaa gtcttcgaag ccgctccact cgcatgtcca ctgtctcaga gcttcgcatc 420

acggctcagg agaatgacat ggaggtggag ctgcctgcag ctgcaaactc ccgcaagcag 480

ttttcagttc ctcctgcccc cactaggcct tcctgccctg cagtggctga aataccattg 540

aggatggtca gcgaggagat ggaagagcaa gtccattcca tccgtggcag ctcttctgca 600

aaccctgtga actcagttcg gaggaaatca tgtcttgtga aggaagtgga aaaaatgaag 660

aacaagcgag aagagaagaa ggcccagaac tctgaaatga gaatgaagag agctcaggag 720

tatgacagta gttttccaaa ctgggaattt gcccgaatga ttaaagaatt tcgggctact 780

ttggaatgtc atccacttac tatgactgat cctatcgaag agcacagaat atgtgtctgt 840

gttaggaaac gcccactgaa taagcaagaa ttggccaaga aagaaattga tgtgatttcc 900

attcctagca agtgtctcct cttggtacat gaacccaagt tgaaagtgga cttaacaaag 960

tatctggaga accaagcatt ctgctttgac tttgcatttg atgaaacagc ttcgaatgaa 1020

gttgtctaca ggttcacagc aaggccactg gtacagacaa tctttgaagg tggaaaagca 1080

acttgttttg catatggcca gacaggaagt ggcaagacac atactatggg cggagacctc 1140

tctgggaaag cccagaatgc atccaaaggg atctatgcca tggcctcccg ggacgtcttc 1200

ctcctgaaga atcaaccctg ctaccggaag ttgggcctgg aagtctatgt gacattcttc 1260

gagatctaca atgggaagct gtttgacctg ctcaacaaga aggccaagct gcgcgtgctg 1320

gaggacggca agcaacaggt gcaagtggtg gggctgcagg agcatctggt taactctgct 1380

gatgatgtca tcaagatgct cgacatgggc agcgcctgca gaacctctgg gcagacattt 1440

gccaactcca attcctcccg ctcccacgcg tgcttccaaa ttattcttcg agctaaaggg 1500

agaatgcatg gcaagttctc tttggtagat ctggcaggga atgagcgagg cgcagacact 1560

tccagtgctg accggcagac ccgcatggag ggcgcagaaa tcaacaagag tctcttagcc 1620

ctgaaggagt gcatcagggc cctgggacag aacaaggctc acaccccgtt ccgtgagagc 1680

aagctgacac aggtgctgag ggactccttc attggggaga actctaggac ttgcatgatt 1740

gccacgatct caccaggcat aagctcctgt gaatatactt taaacaccct gagatatgca 1800

gacagggtca aggagctgag cccccacagt gggcccagtg gagagcagtt gattcaaatg 1860

gaaacagaag agatggaagc ctgctctaac ggggcgctga ttccaggcaa tttatccaag 1920

gaagaggagg aactgtcttc ccagatgtcc agctttaacg aagccatgac tcagatcagg 1980

gagctggagg agaaggctat ggaagagctc aaggagatca tacagcaagg accagactgg 2040

cttgagctct ctgagatgac cgagcagcca gactatgacc tggagacctt tgtgaacaaa 2100

gcggaatctg ctctggccca gcaagccaag catttctcag ccctgcgaga tgtcatcaag 2160

gccttacgcc tggccatgca gctggaagag caggctagca gacaaataag cagcaagaaa 2220

cggccccagt gacgactgca aataaaaatc tgtttggttt gacacccagc ctcttccctg 2280

gccctcccca gagaactttg ggtacctggt gggtctaggc agggtctgag ctgggacagg 2340

ttctggtaaa tgccaagtat gggggcatct gggcccaggg cagctgggga gggggtcaga 2400

gtgacatggg acactccttt tctgttcctc agttgtcgcc ctcacgagag gaaggagctc 2460

ttagttaccc ttttgtgttg cccttctttc catcaagggg aatgttctca gcatagagct 2520

ttctccgcag catcctgcct gcgtggactg gctgctaatg gagagctccc tggggttgtc 2580

ctggctctgg ggagagagac ggagccttta gtacagctat ctgctggctc taaaccttct 2640

acgcctttgg gccgagcact gaatgtcttg tactttaaaa aaatgtttct gagacctctt 2700

tctactttac tgtctcccta gagtcctaga ggatccctac tgttttctgt tttatgtgtt 2760

tatacattgt atgtaacaat aaagagaaaa aataaaaaaa aaaaaaaaaa aaaaaaaaaa 2820

aaaaa 2825

<210> 121
<211> 823
<212> DNA
<213> Homo sapiens

<400> 121
   aaacgcgggc gggcgggccc gcagtcctgc agttgcagtc gtgttctccg agttcctgtc 60
   tctctgccaa cgccgcccgg atggcttccc aaaaccgcga cccagccgcc actagcgtcg 120
   ccgccgcccg taaaggagct gagccgagcg ggggcgccgc ccggggtccg gtgggcaaaa 180
   ggctacagca ggagctgatg accctcatga tgtctggcga taaagggatt tctgccttcc 240
   ctgaatcaga caaccttttc aaatgggtag ggaccatcca tggagcagct ggaacagtat 300
   atgaagacct gaggtataag ctctcgctag agttccccag tggctaccct tacaatgcgc 360
   ccacagtgaa gttcctcacg ccctgctatc accccaacgt ggacacccag ggtaacatat 420
   gcctggacat cctgaaggaa aagtggtctg ccctgtatga tgtcaggacc attctgctct 480
   ccatccagag ccttctagga gaacccaaca ttgatagtcc cttgaacaca catgctgccg 540
   agctctggaa aaaccccaca gcttttaaga agtacctgca agaaacctac tcaaagcagg 600
   tcaccagcca ggagccctga cccaggctgc ccagcctgtc cttgtgtcgt ctttttaatt 660
   tttccttaga tggtctgtcc tttttgtgat ttctgtatag gactctttat cttgagctgt 720
   ggtatttttg ttttgttttt gtcttttaaa ttaagcctcg gttgagccct tgtatattaa 780
   ataaatgcat ttttgtcctt ttttagacaa aaaaaaaaaa aaa 823

<210> 122
<211> 766
<212> DNA
<213> Homo sapiens

<400> 122
aaacgcgggc gggcgggccc gcagtcctgc agttgcagtc gtgttctccg agttcctgtc 60

tctctgccaa cgccgcccgg atggcttccc aaaaccgcga cccagccgcc actagcgtcg 120

ccgccgcccg taaaggagct gagccgagcg ggggcgccgc ccggggtccg gtgggcaaaa 180

ggctacagca ggagctgatg accctcatga tgtctggcga taaagggatt tctgccttcc 240

ctgaatcaga caaccttttc aaatgggtag ggaccatcca tggagcagct ggaacagcag 300

tggggagcat cagaaccagc tcaacagttt gtctactgtc cggtcccaga gaaactcaag 360

attctagcaa gccccttgtg tggggcttgg gttgggacat gaggctgctg ctggagctta 420

ctctgcaact gtttctccaa atgccagaac ccaacattga tagtcccttg aacacacatg 480

ctgccgagct ctggaaaaac cccacagctt ttaagaagta cctgcaagaa acctactcaa 540

agcaggtcac cagccaggag ccctgaccca ggctgcccag cctgtccttg tgtcgtcttt 600

ttaatttttc cttagatggt ctgtcctttt tgtgatttct gtataggact ctttatcttg 660

agctgtggta tttttgtttt gtttttgtct tttaaattaa gcctcggttg agcccttgta 720

tattaaataa atgcattttt gtcctttttt agacaaaaaa aaaaaa 766

<210> 123
<211> 744
<212> DNA
<213> Homo sapiens

<400> 123
aaacgcgggc gggcgggccc gcagtcctgc agttgcagtc gtgttctccg agttcctgtc 60

tctctgccaa cgccgcccgg atggcttccc aaaaccgcga cccagccgcc actagcgtcg 120

ccgccgcccg taaaggagct gagccgagcg ggggcgccgc ccggggtccg gtgggcaaaa 180

ggctacagca ggagctgatg accctcatgg tatatgaaga cctgaggtat aagctctcgc 240

tagagttccc cagtggctac ccttacaatg cgcccacagt gaagttcctc acgccctgct 300

atcaccccaa cgtggacacc cagggtaaca tatgcctgga catcctgaag gaaaagtggt 360

ctgccctgta tgatgtcagg accattctgc tctccatcca gagccttcta ggagaaccca 420

acattgatag tcccttgaac acacatgctg ccgagctctg gaaaaacccc acagctttta 480

agaagtacct gcaagaaacc tactcaaagc aggtcaccag ccaggagccc tgacccaggc 540

tgcccagcct gtccttgtgt cgtcttttta atttttcctt agatggtctg tcctttttgt 600

gatttctgta taggactctt tatcttgagc tgtggtattt ttgttttgtt tttgtctttt 660

aaattaagcc tcggttgagc ccttgtatat taaataaatg catttttgtc cttttttaga 720

caaaaaaaaa aaaaaaaaaa aaaa 744

<210> 124
<211> 936
<212> DNA
<213> Homo sapiens

<400> 124
cgagagatgg gggacaggcc aggagctcag accgctcttt gagactctcc cgaaggagaa 60

tgggagggta ggggcgctgc cagactcctt ccctggtggg cctagatgaa gacgctcaag 120

gaccctcgtg acttggccga gacaggggaa gggagaagtt gagtcgggca aggaagagat 180

gctaaagcct ggggaattaa gaacatgcca gaatcatccc gagggagtct ggaattaggg 240

agggtgagga ctcgctagga tcgtcctgtg gatctggcta cagcaggagc tgatgaccct 300

catgatgtct ggcgataaag ggatttctgc cttccctgaa tcagacaacc ttttcaaatg 360

ggtagggacc atccatggag cagctggaac agtatatgaa gacctgaggt ataagctctc 420

gctagagttc cccagtggct acccttacaa tgcgcccaca gtgaagttcc tcacgccctg 480

ctatcacccc aacgtggaca cccagggtaa catatgcctg gacatcctga aggaaaagtg 540

gtctgccctg tatgatgtca ggaccattct gctctccatc cagagccttc taggagaacc 600

caacattgat agtcccttga acacacatgc tgccgagctc tggaaaaacc ccacagcttt 660

taagaagtac ctgcaagaaa cctactcaaa gcaggtcacc agccaggagc cctgacccag 720

gctgcccagc ctgtccttgt gtcgtctttt taatttttcc ttagatggtc tgtccttttt 780

gtgatttctg tataggactc tttatcttga gctgtggtat ttttgttttg tttttgtctt 840

ttaaattaag cctcggttga gcccttgtat attaaataaa tgcatttttg tcctttttta 900

gacaaaaaaa aaaaaaaaaa aaaaaaaaaa aaaaaa 936

<210> 125
<211> 1490
<212> DNA
<213> Homo sapiens

<400> 125
aaacgcgggc gggcgggccc gcagtcctgc agttgcagtc gtgttctccg agttcctgtc 60

tctctgccaa cgccgcccgg atggcttccc aaaaccgcga cccagccgcc actagcgtcg 120

ccgccgcccg taaaggagct gagccgagcg ggggcgccgc ccggggtccg gtgggcaaaa 180

ggtgagtgat gcggcctacc actcgccggg cctgccatgc cctaggcatt ggtacccaga 240

gcaaagattt ctaggaccac cccccgccgc cacctcctgg agcgggagat ctgcgggtgc 300

aggagaacac accaggagct cggggcctgg cattcccctg ggcatgggtg tcgagggcgg 360

agacttccgg gactcccacc tccttgcgcg ggtgagattc gagagatggg ggacaggcca 420

ggagctcaga ccgctctttg agactctccc gaaggagaat gggagggtag gggcgctgcc 480

agactccttc cctggtgggc ctagatgaag acgctcaagg accctcgtga cttggccgag 540

acaggggaag ggagaagttg agtcgggcaa ggaagagatg ctaaagcctg gggaattaag 600

aacatgccag aatcatcccg agggagtctg gaattaggga gggtgaggac tcgctaggat 660

cgtcctgtgg atctggtgag tataccctga gctgagcctg accttggtga tgtggggagc 720

ctccatgacg gctccccctg agattcaggt gggagaagat gcaccgtctg gaggcctggc 780

ccatccagac tcccaggtaa ccccgaatac tcttttttca ggctacagca ggagctgatg 840

accctcatga tgtctggcga taaagggatt tctgccttcc ctgaatcaga caaccttttc 900

aaatgggtag ggaccatcca tggagcagct ggaacagtat atgaagacct gaggtataag 960

ctctcgctag agttccccag tggctaccct tacaatgcgc ccacagtgaa gttcctcacg 1020

ccctgctatc accccaacgt ggacacccag ggtaacatat gcctggacat cctgaaggaa 1080

aagtggtctg ccctgtatga tgtcaggacc attctgctct ccatccagag ccttctagga 1140

gaacccaaca ttgatagtcc cttgaacaca catgctgccg agctctggaa aaaccccaca 1200

gcttttaaga agtacctgca agaaacctac tcaaagcagg tcaccagcca ggagccctga 1260

cccaggctgc ccagcctgtc cttgtgtcgt ctttttaatt tttccttaga tggtctgtcc 1320

tttttgtgat ttctgtatag gactctttat cttgagctgt ggtatttttg ttttgttttt 1380

gtcttttaaa ttaagcctcg gttgagccct tgtatattaa ataaatgcat tttgtccttt 1440

ttttagacaa aaaaaaaaaa aaaaaaaaaa aaaaaaaaaa aaaaaaaaaa 1490

<210> 126
<211> 520
<212> DNA
<213> Homo sapiens

<400> 126
aaacgcgggc gggcgggccc gcagtcctgc agttgcagtc gtgttctccg agttcctgtc 60

tctctgccaa cgccgcccgg atggcttccc aaaaccgcga cccagccgcc actagcgtcg 120

ccgccgcccg taaaggagct gagccgagcg ggggcgccgc ccggggtccg gtgggcaaaa 180

ggctacagca ggagctgatg accctcatga acccaacatt gatagtccct tgaacacaca 240

tgctgccgag ctctggaaaa accccacagc ttttaagaag tacctgcaag aaacctactc 300

aaagcaggtc accagccagg agccctgacc caggctgccc agcctgtcct tgtgtcgtct 360

ttttaatttt tccttagatg gtctgtcctt tttgtgattt ctgtatagga ctctttatct 420

tgagctgtgg tatttttgtt ttgtttttgt cttttaaatt aagcctcggt tgagcccttg 480

tatattaaat aaatgcattt ttgtcctttt ttagacaaaa 520

<210> 127
<211> 1546
<212> DNA
<213> Homo sapiens

<400> 127
gattgtggga aggcagctga actcggcgcc tggaaagatg gaggcagcgg agacagaggc 60

ggaagctgca gccctagagg tcctggctga ggtggcaggc atcttggaac ctgtaggcct 120

gcaggaggag gcagaactgc cagccaagat cctggttgag tttgtggtgg actctcagaa 180

gaaagacaag ctgctctgca gccagcttca ggtagcggat ttcctgcaga acatcctggc 240

tcaggaggac actgctaagg gtctcgaccc cttggcttct gaagacacga gccgacagaa 300

ggcaattgca gctaaggaac aatggaaaga gctgaaggcc acctacaggg agcacgtaga 360

ggccatcaaa attggcctca ccaaggccct gactcagatg gaggaagccc agaggaaacg 420

gacacaactc cgggaagcct ttgagcagct ccaggccaag aaacaaatgg ccatggagaa 480

acgcagagca gtccagaacc agtggcagct acaacaggag aagcatctgc agcatctggc 540

ggaggtttct gcagaggtta agctgttgtt ccctgaggct gaggctgagg cagagaatct 600

tccagatgat aaaccccagc agccgactcg accccaggag cagagtacag gagacaccat 660

ggggagagac cctggtgtgt ccttcaaggc tgttggtcta caacctgctg gagatgtaaa 720

tttgccatga cttcctggag gacagcagca tggagaaaga tcctagaaaa ggcctctgac 780

ttccctcacc tcccaaccat cattacagga aagactgtga actcctgagt tcagcttgat 840

ttctgactac atcccagcaa gctctggcat ctgtggatta aaatccctgg atctctctca 900

gttgtgtatt tgttcatctt catatgctgg caggaacaac tattaataca gatactcaga 960

agccaataac atgacaggag ctgggactgg tttgaacaca gggtgtgcag atggggaggg 1020

ggtactggcc ttgggcctcc tatgatgcag acatggtgaa tttaattcaa ggaggaggag 1080

aatgttttag gcaggtggtt atatgtggga agataatttt attcatggat ccaaatgttt 1140

gttgagtcct ttctttgtgc taaggttctt gcggtgaacc agaattataa cagtgagctc 1200

atctgactgt tttaggatgt acagcctagt gttaacattc ttggtatctt tttgtgcctt 1260

atctaaaaca tttctcgatc actggtttca gatgttcatt tattatattc ttttcaaaga 1320

ttcagagatt ggcttttgtc atccactatt gtatgttttg tttcattgac ctctagtgat 1380

accttgatct ttcccacttt ctgttttcgg attggagaag atgtaccttt tttgtcaact 1440

cttactttta tcagatgatc aactcacgta tttggatctt tatttgtttt ctcaaataaa 1500

tatttaaggt tatacattta aaaaaaaaaa aaaaaaaaaa aaaaaa 1546

<210> 128
<211> 2002
<212> DNA
<213> Homo sapiens

<400> 128
gattgtggga aggcagctga actcggcgcc tggaaagatg gaggcagcgg agacagaggc 60

ggaagctgca gccctagagg tcctggctga ggtggcaggc atcttggaac ctgtaggcct 120

gcaggaggag gcagaactgc cagccaagat cctggttgag tttgtggtgg tatgtacaag 180

atgagagcga gcctttcaga agccagggtc tggtcctggc tcctcttact gagatacagg 240

gcagcattgg cagagagcag acttaaggaa acctgtaagg aggtaaggat gttccctgtc 300

tctgccctcc ctctgagcag gactctcaga agaaagacaa gctgctctgc agccagcttc 360

aggtagcgga tttcctgcag aacatcctgg ctcaggagga cactgctaag ggtctcgacc 420

ccttggcttc tgaagacacg agccgtgagt aggcagggga ttgtagtagc agctgacctg 480

agtggggaag gaaggaggat ttcagttagg tctcccattt ggctgatggg aaggaggccc 540

agtgttgcag ggaggcaggc tagcaggcca ccctcactca tgtactcatt cctggtggct 600

catctcagga cagaaggcaa ttgcagctaa ggaacaatgg aaagagctga aggccaccta 660

cagggagcac gtagaggcca tcaaaattgg cctcaccaag gccctgactc agatggagga 720

agcccagagg aaacggacac aactccggga agcctttgag cagctccagg ccaagaaaca 780

aatggccatg gagaaacgca gagcagtcca gaaccagtgg cagctacaac aggagaagca 840

tctgcagcat ctggcggagg tttctgcaga ggtgagggag cgtaagacag ggactcagca 900

ggagcttgac agggtgtttc agaaacttgg aaacctgaag cagcaggcag aacaggagcg 960

ggacaagctg cagaggtatc agaccttcct ccagcttctg tataccctgc agggtaagct 1020

gttgttccct gaggctgagg ctgaggcaga gaatcttcca gatgataaac cccagcagcc 1080

gactcgaccc caggagcaga gtacaggaga caccatgggg agagaccctg gtgtgtcctt 1140

caaggctgtt ggtctacaac ctgctggaga tgtaaatttg ccatgacttc ctggaggaca 1200

gcagcatgga gaaagatcct agaaaaggcc tctgacttcc ctcacctccc aaccatcatt 1260

acaggaaaga ctgtgaactc ctgagttcag cttgatttct gactacatcc cagcaagctc 1320

tggcatctgt ggattaaaat ccctggatct ctctcagttg tgtatttgtt catcttcata 1380

tgctggcagg aacaactatt aatacagata ctcagaagcc aataacatga caggagctgg 1440

gactggtttg aacacagggt gtgcagatgg ggagggggta ctggccttgg gcctcctatg 1500

atgcagacat ggtgaattta attcaaggag gaggagaatg ttttaggcag gtggttatat 1560

gtgggaagat aattttattc atggatccaa atgtttgttg agtcctttct ttgtgctaag 1620

gttcttgcgg tgaaccagaa ttataacagt gagctcatct gactgtttta ggatgtacag 1680

cctagtgtta acattcttgg tatctttttg tgccttatct aaaacatttc tcgatcactg 1740

gtttcagatg ttcatttatt atattctttt caaagattca gagattggct tttgtcatcc 1800

actattgtat gttttgtttc attgacctct agtgatacct tgatctttcc cactttctgt 1860

tttcggattg gagaagatgt accttttttg tcaactctta cttttatcag atgatcaact 1920

cacgtatttg gatctttatt tgttttctca aataaatatt taaggttata catttaaaaa 1980

aaaaaaaaaa aaaaaaaaaa aa 2002

<210> 129
<211> 1687
<212> DNA
<213> Homo sapiens

<400> 129
gattgtggga aggcagctga actcggcgcc tggaaagatg gaggcagcgg agacagaggc 60

ggaagctgca gccctagagg tcctggctga ggtggcaggc atcttggaac ctgtaggcct 120

gcaggaggag gcagaactgc cagccaagat cctggttgag tttgtggtgg actctcagaa 180

gaaagacaag ctgctctgca gccagcttca ggtagcggat ttcctgcaga acatcctggc 240

tcaggaggac actgctaagg gtctcgaccc cttggcttct gaagacacga gccgacagaa 300

ggcaattgca gctaaggaac aatggaaaga gctgaaggcc acctacaggg agcacgtaga 360

ggccatcaaa attggcctca ccaaggccct gactcagatg gaggaagccc agaggaaacg 420

gacacaactc cgggaagcct ttgagcagct ccaggccaag aaacaaatgg ccatggagaa 480

acgcagagca gtccagaacc agtggcagct acaacaggag aagcatctgc agcatctggc 540

ggaggtttct gcagaggtga gggagcgtaa gacagggact cagcaggagc ttgacagggt 600

gtttcagaaa cttggaaacc tgaagcagca ggcagaacag gagcgggaca agctgcagag 660

gtatcagacc ttcctccagc ttctgtatac cctgcagggt aagctgttgt tccctgaggc 720

tgaggctgag gcagagaatc ttccagatga taaaccccag cagccgactc gaccccagga 780

gcagagtaca ggagacacca tggggagaga ccctggtgtg tccttcaagg ctgttggtct 840

acaacctgct ggagatgtaa atttgccatg acttcctgga ggacagcagc atggagaaag 900

atcctagaaa aggcctctga cttccctcac ctcccaacca tcattacagg aaagactgtg 960

aactcctgag ttcagcttga tttctgacta catcccagca agctctggca tctgtggatt 1020

aaaatccctg gatctctctc agttgtgtat ttgttcatct tcatatgctg gcaggaacaa 1080

ctattaatac agatactcag aagccaataa catgacagga gctgggactg gtttgaacac 1140

agggtgtgca gatggggagg gggtactggc cttgggcctc ctatgatgca gacatggtga 1200

atttaattca aggaggagga gaatgtttta ggcaggtggt tatatgtggg aagataattt 1260

tattcatgga tccaaatgtt tgttgagtcc tttctttgtg ctaaggttct tgcggtgaac 1320

cagaattata acagtgagct catctgactg ttttaggatg tacagcctag tgttaacatt 1380

cttggtatct ttttgtgcct tatctaaaac atttctcgat cactggtttc agatgttcat 1440

ttattatatt cttttcaaag attcagagat tggcttttgt catccactat tgtatgtttt 1500

gtttcattga cctctagtga taccttgatc tttcccactt tctgttttcg gattggagaa 1560

gatgtacctt ttttgtcaac tcttactttt atcagatgat caactcacgt atttggatct 1620

ttatttgttt tctcaaataa atatttaagg ttatacattt aaaaaaaaaa aaaaaaaaaa 1680

aaaaaaa 1687

<210> 130
<211> 1878
<212> DNA
<213> Homo sapiens

<400> 130
gattgtggga aggcagctga actcggcgcc tggaaagatg gaggcagcgg agacagaggc 60

ggaagctgca gccctagagg tcctggctga ggtggcaggc atcttggaac ctgtaggcct 120

gcaggaggag gcagaactgc cagccaagat cctggttgag tttgtggtgg actctcagaa 180

gaaagacaag ctgctctgca gccagcttca ggtagcggat ttcctgcaga acatcctggc 240

tcaggaggac actgctaagg gtctcgaccc cttggcttct gaagacacga gccgacagaa 300

ggcaattgca gctaaggaac aatggaaaga gctgaaggcc acctacaggg agcacgtaga 360

ggccatcaaa attggcctca ccaaggccct gactcagatg gaggaagccc agaggaaacg 420

gacacaactc cgggaagcct ttgagcagct ccaggccaag aaacaaatgg ccatggagaa 480

acgcagagca gtccagaacc agtggcagct acaacaggag aagcatctgc agcatctggc 540

ggaggtttct gcagaggtga gggagcgtaa gacagggact cagcaggagc ttgacagggt 600

gtttcagaaa cttggaaacc tgaagcagca ggcagaacag gagcgggaca agctgcagag 660

gtatcagacc ttcctccagc ttctgtatac cctgcagggt aagctgttgt tccctgaggc 720

tgaggctgag gcagagaatc ttccagatga taaaccccag cagccgactc gaccccagga 780

gcagagtaca ggagacacca tggggagaga ccctggtgtg tccttcaagg ctgttggtct 840

acaacctgct ggagatgtaa atttgccatg acttcctgga ggacagcagc atggagaaag 900

atcctagaaa aggtcagacc caactcaggc cttggtgtcc ctggactgca agtgtggaag 960

gagggaaagc ctggtttacc tctctctgca tctgagctct gctacccatg gagcagatgg 1020

atggtgggaa caggaaagag cttatgttac acctcattcc catgcttagc ccacccagag 1080

ctaacccctg tcttcttccc caggcctctg acttccctca cctcccaacc atcattacag 1140

gaaagactgt gaactcctga gttcagcttg atttctgact acatcccagc aagctctggc 1200

atctgtggat taaaatccct ggatctctct cagttgtgta tttgttcatc ttcatatgct 1260

ggcaggaaca actattaata cagatactca gaagccaata acatgacagg agctgggact 1320

ggtttgaaca cagggtgtgc agatggggag ggggtactgg ccttgggcct cctatgatgc 1380

agacatggtg aatttaattc aaggaggagg agaatgtttt aggcaggtgg ttatatgtgg 1440

gaagataatt ttattcatgg atccaaatgt ttgttgagtc ctttctttgt gctaaggttc 1500

ttgcggtgaa ccagaattat aacagtgagc tcatctgact gttttaggat gtacagccta 1560

gtgttaacat tcttggtatc tttttgtgcc ttatctaaaa catttctcga tcactggttt 1620

cagatgttca tttattatat tcttttcaaa gattcagaga ttggcttttg tcatccacta 1680

ttgtatgttt tgtttcattg acctctagtg ataccttgat ctttcccact ttctgttttc 1740

ggattggaga agatgtacct tttttgtcaa ctcttacttt tatcagatga tcaactcacg 1800

tatttggatc tttatttgtt ttctcaaata aatatttaag gttatacatt taaaaaaaaa 1860

aaaaaaaaaa aaaaaaaa 1878

<210> 131
<211> 3685
<212> DNA
<213> Homo sapiens

<400> 131
agtggactca cgcaggcgca ggagactaca cttcccagga actccgggcc gcgttgttcg 60

ctggtacctc cttctgactt ccggtattgc tgcggtctgt agggccaatc gggagcctgg 120

aattgctttc ccggcgctct gattggtgca ttcgactagg ctgcctgggt tcaaaatttc 180

aacgatactg aatgagtccc gcggcgggtt ggctcgcgct tcgttgtcag atctgaggcg 240

aggctaggtg agccgtggga agaaaagagg gagcagctag ggcgcgggtc tccctcctcc 300

cggagtttgg aacggctgaa gttcaccttc cagcccctag cgccgttcgc gccgctaggc 360

ctggcttctg aggcggttgc ggtgctcggt cgccgcctag gcggggcagg gtgcgagcag 420

gggcttcggg ccacgcttct cttggcgaca ggattttgct gtgaagtccg tccgggaaac 480

ggaggaaaaa aagagttgcg ggaggctgtc ggctaataac ggttcttgat acatatttgc 540
cagacttcaa gatttcagaa aaggggtgaa agagaagatt gcaactttga gtcagacctg 600
taggcctgat agactgatta aaccacagaa ggtgacctgc tgagaaaagt ggtacaaata 660
ctgggaaaaa cctgctcttc tgcgttaagt gggagacaat gtcacaagtt aaaagctctt 720
attcctatga tgccccctcg gatttcatca atttttcatc cttggatgat gaaggagata 780
ctcaaaacat agattcatgg tttgaggaga aggccaattt ggagaataag ttactgggga 840
agaatggaac tggagggctt tttcagggca aaactccttt gagaaaggct aatcttcagc 900
aagctattgt cacacctttg aaaccagttg acaacactta ctacaaagag gcagaaaaag 960
aaaatcttgt ggaacaatcc attccgtcaa atgcttgttc ttccctggaa gttgaggcag 1020
ccatatcaag aaaaactcca gcccagcctc agagaagatc tcttaggctt tctgctcaga 1080
aggatttgga acagaaagaa aagcatcatg taaaaatgaa agccaagaga tgtgccactc 1140
ctgtaatcat cgatgaaatt ctaccctcta agaaaatgaa agtttctaac aacaaaaaga 1200
agccagagga agaaggcagt gctcatcaag atactgctga aaagaatgca tcttccccag 1260
agaaagccaa gggtagacat actgtgcctt gtatgccacc tgcaaagcag aagtttctaa 1320
aaagtactga ggagcaagag ctggagaaga gtatgaaaat gcagcaagag gtggtggaga 1380
tgcggaaaaa gaatgaagaa ttcaagaaac ttgctctggc tggaataggg caacctgtga 1440
agaaatcagt gagccaggtc accaaatcag ttgacttcca cttccgcaca gatgagcgaa 1500
tcaaacaaca tcctaagaac caggaggaat ataaggaagt gaactttaca tctgaactac 1560
gaaagcatcc ttcatctcct gcccgagtga ctaagggatg taccattgtt aagcctttca 1620
acctgtccca aggaaagaaa agaacatttg atgaaacagt ttctacatat gtgccccttg 1680
cacagcaagt tgaagacttc cataaacgaa cccctaacag atatcatttg aggagcaaga 1740
aggatgatat taacctgtta ccctccaaat cttctgtgac caagatttgc agagacccac 1800
agactcctgt actgcaaacc aaacaccgtg cacgggctgt gacctgcaaa agtacagcag 1860
agctggaggc tgaggagctc gagaaattgc aacaatacaa attcaaagca cgtgaacttg 1920
atcccagaat acttgaaggt gggcccatct tgcccaagaa accacctgtg aaaccaccca 1980
ccgagcctat tggctttgat ttggaaattg agaaaagaat ccaggagcga gaatcaaaga 2040
agaaaacaga ggatgaacac tttgaatttc attccagacc ttgccctact aagattttgg 2100
aagatgttgt gggtgttcct gaaaagaagg tacttccaat caccgtcccc aagtcaccag 2160
cctttgcatt gaagaacaga attcgaatgc ccaccaaaga agatgaggaa gaggacgaac 2220
cggtagtgat aaaagctcaa cctgtgccac attatggggt gccttttaag ccccaaatcc 2280
cagaggcaag aactgtggaa atatgccctt tctcgtttga ttctcgagac aaagaacgtc 2340
agttacagaa ggagaagaaa ataaaagaac tgcagaaagg ggaggtgccc aagttcaagg 2400
cacttccctt gcctcatttt gacaccatta acctgccaga gaagaaggta aagaatgtga 2460
cccagattga acctttctgc ttggagactg acagaagagg tgctctgaag gcacagactt 2520
ggaagcacca gctggaagaa gaactgagac agcagaaaga agcagcttgt ttcaaggctc 2580

gtccaaacac cgtcatctct caggagccct ttgttcccaa gaaagagaag aaatcagttg 2640

ctgagggcct ttctggttct ctagttcagg aaccttttca gctggctact gagaagagag 2700

ccaaagagcg gcaggagctg gagaagagaa tggctgaggt agaagcccag aaagcccagc 2760

agttggagga ggccagacta caggaggaag agcagaaaaa agaggagctg gccaggctac 2820

ggagagaact ggtgcataag gcaaatccaa tacgcaagta ccagggtctg gagataaagt 2880

caagtgacca gcctctgact gtgcctgtat ctcccaaatt ctccactcga ttccactgct 2940

aaactcagct gtgagctgcg gataccgccc ggcaatggga cctgctctta acctcaaacc 3000

taggaccgtc ttgctttgtc attgggcatg gagagaaccc atttctccag acttttacct 3060

acccgtgcct gagaaagcat acttgacaac tgtggactcc agttttgttg agaattgttt 3120

tcttacatta ctaaggctaa taatgagatg taactcatga atgtctcgat tagactccat 3180

gtagttactt cctttaaacc atcagccggc cttttatatg ggtcttcact ctgactagaa 3240

tttagtctct gtgtcagcac agtgtaatct ctattgctat tgccccttac gactctcacc 3300

ctctccccac tttttttaaa aattttaacc agaaaataaa gatagttaaa tcctaagata 3360

gagattaagt catggtttaa atgaggaaca atcagtaaat cagattctgt cctcttctct 3420

gcataccgtg aatttatagt taaggatccc tttgctgtga gggtagaaaa cctcaccaac 3480

tgcaccagtg aggaagaaga ctgcgtggat tcatggggag cctcacagca gccacgcagc 3540

aggctctggg tggggctgcc gttaaggcac gttctttcct tactggtgct gataacaaca 3600

gggaaccgtg cagtgtgcat tttaagacct ggcctggaat aaatacgttt tgtctttccc 3660

tcaaaaaaaa aaaaaaaaaa aaaaa 3685

<210> 132
<211> 14315
<212> DNA
<213> Homo sapiens

<400> 132
gcggccgcag aatcgagctc gggccccggc ccccggcccg cggcgcgggg ctcccgggcc 60

ccgccgcgga cgtcgcgccg gtcgcccctt ccccgtagcc cgtgcgccct cggcgcggag 120

ccccggcccg ccgcggtccc gtctcctggg cctgtcccgc ccgcgccctc cgccggccct 180

caggtataat acttctccac gtctgcttca ggaagaaagt gcctgccatt cttatcattt 240

ctaagcaggt tcatgccagc ccagaacaga gaatcagctg gagcccagat ttcaagtttt 300

gagtaaaata ccttcaagcg aatgggccct attgtgctca cacattcaga acctgttacc 360

caaggaattc cctaaagaat tagaagtgcg tctcaccaac cagccaagat gaacatggtg 420

aagaggatca tggggcggcc gaggcaggag gagtgcagcc cacaagacaa cgccttagga 480

ctgatgcacc tccgccggct cttcacggag ttgtgccatc ctccccggca catgactcag 540

aaggaacaag aagagaaact gtatatgatg ctgccagtgt ttaacagggt ttttggaaat 600

gctccgccga atacaatgac agaaaaattt tctgatcttc tgcagttcac aacacaagtc 660

tcacgactaa tggtgacaga aattcgaagg agagcatcaa acaaatccac agaggctgca 720

agtcgggcca tagttcagtt cctagagatt aatcagagtg aagaagccag tagaggctgg 780
atgcttctaa cgacaattaa tttgttagct tcctctggtc agaaaaccgt ggactgcatg 840
acaacaatgt cagtgccttc caccctggtt aaatgtttat atctgttttt tgaccttcca 900
catgtgcctg aggcagttgg aggtgcacag aatgagctac ctctagcaga acgtcgagga 960
ctactccaga aagtttttgt acagatctta gtgaaactgt gcagttttgt ttcccctgcg 1020
gaggagctgg ctcagaaaga tgatctccag cttctattca gtgcaataac ctcttggtgc 1080
cctccctata acctgccttg gagaaagagt gctggagaag tcctcatgac catatctcgt 1140
catggtctta gtgtcaatgt agtgaagtat attcatgaga aagagtgttt atctacatgt 1200
gttcagaata tgcagcaatc agatgacctg tctcccctag aaattgtcga aatgtttgct 1260
gggctttctt gtttcctcaa agattccagc gatgtttccc aaacacttct ggatgatttt 1320
cggatatggc aaggatataa ttttctttgt gatctcttgc ttagattgga acaagcaaaa 1380
gaggcagaat ccaaagatgc cttgaaagat ctggttaatc tgataacttc cctaacaaca 1440
tatggtgtca gtgaactaaa accagctggt attaccacag gggcaccctt tttattgcct 1500
ggatttgcag tacctcagcc tgcaggcaaa ggtcacagtg tgagaaacgt ccaggccttt 1560
gcagttcttc agaatgcatt tttaaaagca aaaaccagct tccttgccca aatcatcctt 1620
gatgctatca caaatattta catggctgac aatgccaatt acttcatcct agagtcacag 1680
cacacattgt cacagtttgc agagaagatt tctaaactcc cagaagtaca aaacaaatac 1740
tttgagatgc tggagtttgt tgtttttagc ttaaattata taccttgtaa agaacttatt 1800
agtgtcagta tcctcttaaa atctagctct tcttatcact gtagcattat tgcaatgaaa 1860
acacttctta agtttacaag acatgactac atatttaaag acgtgttcag ggaggttggc 1920
cttttggagg tcatggtaaa ccttttgcat aaatatgctg ccctgttgaa ggatccaact 1980
caggcactaa atgaacaagg ggactcaaga aataatagtt cagttgaaga ccaaaaacac 2040
ctggctttat tggttatgga gaccttgaca gtgcttcttc aaggatcaaa cacaaatgca 2100
ggaatttttc gagaatttgg aggtgcaaga tgtgcacata atatagtaaa gtaccctcaa 2160
tgccggcagc atgccttgat gactatccaa cagctggtgc tctccccaaa tggggacgat 2220
gacatgggca ctctcctggg gctaatgcat tcagccccac cgacggaatt gcagttgaag 2280
actgatattt taagggccct cctgtcggtc cttcgagaaa gccatcgttc aagaacagtt 2340
tttaggaaag ttggaggatt tgtgtacatt acatccttgc tcgttgctat ggaaagatct 2400
ttgagctgtc cacccaagaa tggctgggag aaagtgaacc agaatcaagt gtttgaactt 2460
cttcacactg tgttctgcac gttgactgca gcaatgcgct atgagccagc caactctcat 2520
ttcttcaaaa cagagattca gtatgagaag ttggcagatg ctgttcgatt tcttggctgc 2580
ttctcagacc taagaaaaat aagcgccatg aatgtcttcc cctcaaatac acagccattt 2640
caaagacttt tagaggaaga tgtaatctca atagaatcag tgtcacccac gttacggcac 2700
tgcagtaaac tttttattta tctttacaaa gtagccacag attcttttga cagtcgtgca 2760
gaacagatcc ctccttgcct gacaagtgag tcttctctcc cctctccttg gggtacacca 2820

gctttgtcca ggaaaaggca tgcatatcat tctgtttcaa ctccccctgt ttaccctcct 2880

aaaaatgttg ccgacctgaa actacatgtg acaacttcat ctctgcagag ttctgatgca 2940

gtcatcattc atcctggagc catgcttgcc atgctggacc tactggcctc tgttgggtca 3000

gtgacacagc cagaacatgc tttggatctt caacttgccg tggcaaatat tttacaatcc 3060

ctggtgcaca cagaaaggaa ccagcaagtc atgtgtgaag ctggtcttca tgcacgactg 3120

ctgcagaggt gcagtgctgc attggctgat gaggaccact cactgcaccc gcccctgcag 3180

cggatgtttg aacgattagc ctctcaggct ctggaaccca tggtgttgag ggagttttta 3240

cgtttggcaa gtcctttaaa ttgtggtgcc tgggacaaaa aactgctaaa acaatatagg 3300

gtccacaaac caagttcact gagttatgaa ccagaaatga gaagtagtat gatcacatct 3360

ctggaaggtc tgggtactga taatgttttt agcttacatg aagataacca ttaccggata 3420

agcaagagcc tggtaaaatc tgcggaagga agtactgtac ccctgaccag ggtgaagtgt 3480

ctggtctcca tgacaacccc acatgacatc agacttcatg ggtcatcagt tactccagct 3540

tttgttgaat ttgacacatc acttgaaggg tttggatgtc tttttttgcc cagtttggcc 3600

cctcataatg ctcctacaaa taataccgtc acaacaggtc ttattgatgg ggctgtggtc 3660

agtggcattg gttctggtga aagattcttc cctcctccct ccggcttaag ttactctagc 3720

tggttttgta ttgaacattt tagttctcct ccaaataacc accctgtcag acttcttact 3780

gttgtgcgcc gagcaaattc ttctgagcaa cattacgtgt gccttgcaat agttctatca 3840

gcaaaagacc gatctctgat tgtttccacc aaagaggaac tcctccaaaa ttatgttgat 3900

gattttagtg aagagtcctc attttatgaa attctcccat gctgtgctcg ctttcgatgt 3960

ggagagctta tcattgaggg acagtggcat catttggtcc tggtaatgag caaaggcatg 4020

ttgaaaaaca gtactgcagc cctttatatt gatggacagc ttgttaacac tgtaaagctt 4080

cattatgtcc acagtactcc agggggttca ggttcggcaa atccaccagt ggtgagcacg 4140

gtctatgcct acattggtac tccacctgcc caacgccaaa ttgcctcatt ggtttggcgc 4200

ctgggaccca cacattttct agaagaagtt ttaccttctt caaatgttac taccatttat 4260

gaacttggac caaattatgt tggaagcttt caggctgtat gtatgccatg taaagatgca 4320

aaatccgaag gggtggtgcc atcccctgtg tcattagtac cagaggagaa agtgtcattt 4380

ggcctctatg cactctctgt gtcgtctcta acagtggcaa gaatccggaa agtgtataac 4440

aaattggata gcaaagccat tgctaagcag ttaggcattt cctcacatga gaatgccact 4500

cctgtgaagt tgatacacaa ttcagcagga catcttaatg gatctgcacg gacaattggg 4560

gccgctctga ttggatactt gggagtaaga acatttgtcc ctaagcctgt tgccactact 4620

ttgcagtacg ttggtggagc tgcagccatc ctgggcctgg tggccatggc ctctgatgtg 4680

gaagggttat atgcagcagt caaggccctg gtttgtgtgg tcaagagtaa cccactagcc 4740

agcaaagaaa tggaaagaat caagggctac cagttgctgg caatgttgct taagaagaaa 4800

cgttcccttc ttaacagcca catcctccat ctaacttttt ctttggtggg aactgttgat 4860

agtggacatg agacctccat tattccaaat tcaactgctt tccaggacct cctctgtgat 4920

tttgaagtct ggctccatgc accatatgaa cttcatcttt ccttatttga acactttatt 4980

gaactgctca cagagtccag tgaagcctca aagaatgcca aattaatgag agaattccag 5040

ttaatcccaa agctgctcct gactcttcga gatatgtctt tatcccagcc tactattgct 5100

gctattagta atgtcctgag cttcttactg caaggttttc ctagcagcaa tgatctgctc 5160

agatttgggc agtttatttc ttctactttg ccaacctttg cggtttgtga gaaatttgta 5220

gtaatggaaa taaataatga agagaagctt gacactggaa ctgaagagga gtttggaggt 5280

cttgtatcag ctaatcttat acttttgagg aacagacttc tggatatctt gctaaaacta 5340

atttatacat ctaaagaaaa gacaagcatt aatttgcaag cttgtgaaga actggtgaag 5400

acactgggtt ttgactggat catgatgttt atggaggaac acttacattc caccacagtt 5460

acagcagcca tgaggattct tgttgtccta ctaagtaatc agtctattct catcaagttt 5520

aaagaaggac tcagtggtgg aggatggctt gaacagacag attctgtctt aactaataag 5580

attggaactg tattaggatt caacgtgggc agaagtgctg gtgggagatc gacggtcagg 5640

gagattaacc gagatgcttg tcattttcct ggttttccag tccttcagtc attccttcct 5700

aaacacacta atgtccctgc cctctatttt ctcctcatgg ccttgtttct gcagcagcca 5760

gttagtgagc tgcctgagaa cctgcaggtc agtgtgcctg tcatcagctg ccggagtaag 5820

cagggttgcc agtttgattt ggattccatt tggacattca tctttggagt tcctgcctcc 5880

agcggaactg tggtctcttc tatccataac gtatgcacag aagctgtttt tttattattg 5940

ggaatgctcc gcagcatgct gacttcacct tggcaatcag aagaagaggg atcttggctc 6000

cgagaatatc ctgtgaccct gatgcagttc ttcagatatt tgtatcacaa cgtgccagac 6060

cttgcctcca tgtggatgag ccctgacttc ctgtgtgcat tagcagccac cgtcttcccc 6120

ttcaatattc gcccttactc agagatggtg actgaccttg atgatgaagt tggatctcca 6180

gcagaagagt ttaaagcgtt tgcagcagac acagggatga acaggagcca atcagagtac 6240

tgcaatgtgg gcaccaagac atatctgacc aatcacccgg ctaaaaagtt cgtttttgac 6300

ttcatgcggg tcttaatcat agacaacctc tgtctcactc ctgccagcaa gcaaactcca 6360

ctaattgatc ttttgttgga ggcttcccct gaaaggtcta caagaactca gcaaaaagaa 6420

tttcaaactt acatttggga tagcgtgatg gaccatttgc ttgcagctga tgtgttatta 6480

ggggaagatg catctctgcc tattaccagt ggaggaagct accaggtatt ggtgaacaat 6540

gtgttttatt tcacacagcg tgtggtggac aagctttggc aaggcatgtt caacaaagaa 6600

tctaaacttc ttatagattt tataattcaa ctaattgcac agtcaaagag aagatcacag 6660

ggattgtcac tggatgcagt gtatcattgc ctcaatagga ccatcttgta ccagttctca 6720

cgggcacaca aaaccgttcc tcagcaagta gctctgcttg attcactcag ggtcctcact 6780

gtaaacagaa acttgatcct gggacctggg aaccatgacc aagaattcat tagctgtctg 6840

gcccactgct tgataaatct acatgttgga agcaacgtgg atggatttgg actggaagca 6900

gaagcccgca tgaccacatg gcacattatg atcccctcgg acattgaacc agatggtagt 6960

tacagccaag atattagtga agggcgtcag cttctcataa aagctgtcaa cagagtttgg 7020

actgaactga tacatagtaa gaaacaagtc ttagaggaac ttttcaaagt aactctacct 7080
gtgaatgaaa ggggccacgt ggacatagct acagcaaggc cactcattga agaagctgcc 7140
ctgaagtgct ggcagaatca tttggcccat gaaaagaaat gcataagtcg aggagaagct 7200
ttagcgccca ccacacagtc caaattatcc cgtgtcagca gtggctttgg tctttccaag 7260
ttaacaggat caagaaggaa tcgaaaagaa agtggtctta ataaacacag tctttccacc 7320
caggagattt cgcagtggat gtttactcac attgctgttg ttcgtgactt agtagataca 7380
caatataaag aatatcagga gcgtcagcag aatgccctga agtacgtgac agaagagtgg 7440
tgtcagatcg agtgcgagct gttgagggag cgggggctgt ggggccctcc catcggctcc 7500
cacctcgaca agtggatgct ggagatgaca gaagggccct gcaggatgag gaaaaagatg 7560
gtgcgaaatg atatgtttta taaccattac ccttacgtgc cagaaactga gcaagagaca 7620
aatgtggcgt ctgagatccc aagtaaacag cctgagacac ccgatgatat tcctcaaaag 7680
aaacctgctc gatatagaag agccgtaagt tatgacagta aagagtacta catgcgactg 7740
gcctctggca atcccgccat tgtccaagac gccattgtgg agagttcaga aggtgaagct 7800
gctcagcaag aaccagagca tggggaagac actattgcta aagtcaaagg tttggtcaag 7860
cctcctctaa aacgctcccg atctgcacct gatggaggag atgaggagaa ccaggagcag 7920
ctacaagacc agattgctga gggcagctcc atagaagagg aggagaaaac agataatgct 7980
accttactgc gcctgttaga ggaaggagaa aagatccaac acatgtaccg ctgtgctcga 8040
gtccagggcc tagataccag tgaggggctc cttctttttg gtaaagagca tttttatgtg 8100
attgatggat ttaccatgac agcaaccagg gaaataagag atattgaaac cttacctcca 8160
aatatgcatg agcctattat tcctagagga gccaggcaag gccctagtca actcaagaga 8220
acatgcagca tttttgcata tgaagatatc aaggaagttc ataaaaggag atatctcctg 8280
cagcctattg ctgtggaagt tttctctgga gatggacgga attacctcct tgcttttcag 8340
aaaggaatca gaaacaaagt ctatcaaagg tttttggctg tagtgccatc tctaacggac 8400
agttcagaat ctgtatctgg gcaacgacca aacacgagtg tggagcaggg atctgggtta 8460
cttagcactt tggttggaga gaagtctgtg actcagagat gggagagagg tgaaatcagc 8520
aacttccaat atttgatgca tttgaacact ttggctggca gatcatataa tgatctcatg 8580
cagtatcctg tcttcccctg gatccttgca gattatgact cagaggaggt ggatcttact 8640
aatcccaaga cgtttagaaa cctggctaag ccaatgggag cacaaacaga tgaacgatta 8700
gctcagtata agaagcggta taaagactgg gaggatccta atggagaaac tcctgcatac 8760
cactatggga cccactattc atctgcaatg attgtggcct cataccttgt aaggatggag 8820
cctttcacac agatattctt aaggctacag ggtggccact ttgacctggc tgaccggatg 8880
tttcacagtg tgcgcgaggc ctggtattca gcgtcaaagc acaatatggc agatgtaaaa 8940
gaacttatcc cagagttctt ttatttacca gaattcctgt tcaattccaa caactttgat 9000
ctaggctgta aacaaaatgg caccaagctt ggagatgtta tccttccacc ctgggcaaaa 9060
ggggacccac gagaattcat cagagtccat cgtgaggctt tggagtgtga ttacgtgagt 9120

gcccatctac atgagtggat tgacttaatc ttcggttata aacagcaagg ccctgctgca 9180

gtagaagctg taaatgtctt ccatcatctt ttttatgagg gtcaagtgga tatctacaac 9240

atcaatgacc cactaaagga gacagccaca attgggttca ttaataactt cggtcagatc 9300

cctaaacagt tatttaaaaa acctcatcca ccaaagcgag tgagaagtcg actcaatgga 9360

gacaatgcag gaatctctgt cctaccagga tctacaagtg acaagatctt ttttcatcat 9420

ctagacaact tgaggccttc tctaacacct gtaaaagaac tcaaagaacc tgtaggacaa 9480

atcgtatgta cagataaagg tattcttgcg gtggaacaga ataaggttct tatcccacca 9540

acctggaata aaacttttgc ttggggctat gcagacctca gttgcagact gggaacctat 9600

gagtcagaca aggccatgac tgtttatgaa tgcttgtctg agtggggcca gattctctgt 9660

gcaatctgcc ccaaccccaa gctggtcatc acgggtggaa caagcacggt tgtgtgtgtg 9720

tgggagatgg gcacctccaa agaaaaggcc aagaccgtca ccctcaaaca ggccttactg 9780

ggccacactg ataccgtcac ctgcgccaca gcatcattag cctatcacat aattgtcagt 9840

gggtcccgtg atcgaacctg tatcatttgg gatttgaaca aactgtcatt tctaacccag 9900

cttcgagggc atcgagctcc agtttctgct ctttgtatca atgaattaac aggggacatt 9960

gtgtcctgcg ctggcacata tatccatgtg tggagcatca atgggaaccc tatcgtgagt 10020

gtcaacacgt tcacaggtag gagccagcag atcatctgct gctgcatgtc ggagatgaac 10080

gaatgggaca cgcagaacgt catagtgaca ggacactcag atggagtggt tcggttttgg 10140

agaatggaat ttttgcaagt tcctgaaaca ccagctcctg agcctgctga agtcctagaa 10200

atgcaggaag actgtccaga agcacaaata gggcaggaag cccaagacga ggacagcagt 10260

gattcagaag cagatgagca gagcatcagc caggacccta aggacactcc aagccaaccc 10320

agcagcacca gccacaggcc ccgggcagcc tcctgccgcg caacagccgc ctggtgtact 10380

gacagtggct ctgacgactc cagacgctgg tccgaccagc tcagtctaga tgagaaagac 10440

ggcttcatat ttgtgaacta ttcagagggc cagaccagag cccatctgca gggccccctt 10500

agccaccccc accccaatcc cattgaggtg cggaattaca gcagattgaa acctgggtac 10560

cgatgggaac ggcagctggt gttcaggagt aagctgacta tgcacacagc ctttgatcga 10620

aaggacaatg cacacccagc tgaggtcact gccttgggca tctccaagga tcacagtagg 10680

atcctcgttg gtgacagtcg aggccgagtt ttcagctggt ctgtgagtga ccagccaggc 10740

cgttctgctg ctgatcactg ggtgaaggat gaaggtggtg acagctgctc aggctgctcg 10800

gtgaggtttt cactcacaga aagacgacac cattgcagga actgtggtca gctcttctgc 10860

cagaagtgca gtcgctttca atctgaaatc aaacgcttga aaatctcatc cccggtgcgt 10920

gtttgtcaga actgttatta taacttacag catgagagag gttcagaaga tgggcctcga 10980

aattgttgaa gattcaacaa gctgagtgga gaccatggtc tgtagacccc ttcccgattc 11040

tcctgtccca gcttggaagg cattgaaaac agtctccgtt tacacatctc ttcataccac 11100

gtgtttgaag tgttaaaatt caaagggatc attgaataaa acgggtgtag agtacaggaa 11160

tggggcagac gcgattcagg tgaacagcac aagaagaata tgaggtggtt cctaggagca 11220

acactttcga cctccagttc tccctgatga cagtagctgt ctccaagaga aaaatcctca 11280

cttattaact ctcttttctt gcatctcatt tttatagagc tactcatcct tatttggaaa 11340

aaccaacaac aaaaaaggct tttagaaaat ggttgtaaat ctgacttctt tgcaagtaac 11400

tatgtatatt gtaaatagat ataaaaggcc ttttttctaa ataaggactt aactgcctgt 11460

aacatgaaac ttcaaactaa accactaact caatgaacta cttatggttt gtctgacatc 11520

cctcacttac caattaatta taaatatgtt tttttaaatc cccaaagaca ttatctgtgg 11580

tctttttttc ctttcaagct cagcctgtgt gcctgatgtc atttctttca agttgcccac 11640

agtatctcca cttaaactag gctagtaacc aaaataatgt ggaccttctt taggaaacag 11700

tgtgggagaa taggagtcca gccgtaagat aaactggaaa tatttgggcg tcttgtacct 11760

ggctacgcac cacctcagtg ttgttcctac ataaacaggg ccccttttaa acttgtatgt 11820

ggactgctgt ttggtcaaag aataccttct tagcattgca gaaaggtggt cagatgacca 11880

gtgtagtgca ggaaacagcc ctgtctcaac taatggaaat atatttgcat gtaacccaaa 11940

attagcttat cttgcataga acataataag tatgtgtctt tggtgacact aatgttctac 12000

tatagcttat tttcaaacaa ggggtaaaaa aaggaaagaa agaagtgtac agaattaaca 12060

tataaacttt gttgtaaaac tgaatcatgt cagaactgct taaaattaac ctttaccatt 12120

taatgtcatc tacctgaaaa cagtgagatt tatactgtat caatgtctat ttttttgttt 12180

ttgctatgaa tataattaca gtattttaat atttagttat ttaatttgtt ctactagttg 12240

gatacagaac acacaaatcc agggggatta aagctggaag gggctaagag attagtttac 12300

agagaaaagg cttggtggtg ggattttttt aaatgtgtgt tatgtacata tatatatata 12360

tataatatat attaaaaatg aaacaattaa tctagatttt aacattttca gaaacttagt 12420

gataacatta tgaacaattc taaaagccct gtgatttgaa aaatatagaa tcattaatgg 12480

cccaagatag gccttcacac cttcacaggt gcgaaaggaa aggccttcac accctcacag 12540

aggcatcatg caaaggacag cggctttggc ttttccaatt ttccatcttt aggccctggt 12600

gagaggcaca cttatgcact aaaatgcaca tatatgcaca tgcattcaaa aataggcatt 12660

tggtacaatg gtgatcttgt acctgatggg ctgaaaccag cttaagaaca aatttgttct 12720

tcctgatatg ataactaggt ctccaagaga aaatagaaag gctgctttag tgccttacgc 12780

ttactaaatt taaatcttta tttacctggg tttgagccta cagtctattt atgattacat 12840

atcaaaattg attaaaacac ttccatttct aaaagttcaa atatacttgt taataaaagg 12900

attatcggca ttaatacttt aatttaaaga aaagttgtgt tctgttttcc tttctgtgtc 12960

ttactccccc cacactctcc ctcccccatc accatcttca attctaataa ataatgctga 13020

tgttcaacag ttgcagaaat tgtgctatta tgtaactgtg ggccttgccc ctgtctggcc 13080

ctctagatga tttgtagcag tgttattcta cactttttaa aagaagcgtc ctccttttgt 13140

ccatgaatca tgtttacccc atacccagtg gcagaggtgt tctttaaaga cttgaatata 13200

tgaatgtgtg tgtgtagtta cttaaaggtt attcctcttt gtaataggaa actatatggg 13260

atgaacactt ttaaactttc cgacacaact tccattacta actttctaac agaacttcca 13320

taactagaag gtggaaacca aaaccctcat ggtagtattt cctctggcag ctggtgctgt 13380

gggcaactgt tttgttcaat cgggtttctt ttctttttgc ctctaatgca gaaatcaaca 13440

gaatcactca cacatacaag tacactcaca tacataaact aattatttct ctggatatct 13500

ttctgtgttc catgtaaatt tatttaccaa catctattgt caacatgtac atctacctta 13560

gtatggtctg cattcttttt ctgagagtac ctcatagggc tcctgcctga tctttgtagt 13620

ttgttcattc atccatccac ctgttcattt gttcatccat gtattctaac atttctatgt 13680

agtgtgcaac tctaatgtca tgcttttgaa gaagagaata gctgcccata gcagccatcc 13740

gtctggataa tagcaaaaca ctctagataa gttattttgc actttcttat gtataaagtt 13800

ggtagaaact tatttttgct ttgtatcatt taaatacatt ttgttttggt aaatgaactg 13860

tgtataaaat atttatgccg ttaaaactgt ttttagaaag tatttttaat ttcagcaagt 13920

ttggttactt gttgcatgac tcttaacaca gctgactttt tgtgtcagtg caatgtatat 13980

tttttgtcct gttattaact tgtaagccct agtaatggcc aattatttgt acagcaacag 14040

aagtaaattg aagatactgg ctaagactgg attgattgtg gacttttata ctatattgca 14100

gaaaccaata tctgtttctt ggtggttatg taaaagacct gaagaattac tatctagtgt 14160

gcagtctgtg atatctgaat gttcattgta tatttgtctc tgatgcaaaa aggtagagta 14220

acacaattac aatacatgat taaatgcaat agtccaggta cttaagtaat ttttttttca 14280

tttcaaataa atacctattt accaccaaaa gaaag 14315

<210> 133
<211> 3498
<212> DNA
<213> Homo sapiens

<400> 133
ctggagccca gatttcaagt tttgagtaaa ataccttcaa gcgaatgggc cctattgtgc 60

tcacacattc agaacctgtt acccaaggaa ttcccttaaa gaattagaag tgcgtctcac 120

caaccagcca agatgaacat ggtgaagagg atcatggggc ggccgaggca ggaggagtgc 180

agcccacaag acaacgcctt aggactgatg cacctccgcc ggctcttcac ggagttgtgc 240

catcctcccc ggcacatgac tcagaaggaa caagaagaga aactgtatat gatgctgcca 300

gtgtttaaca gggtttttgg aaatgctccg ccgaatacaa tgacagaaaa attttctgat 360

cttctgcagt tcacaacaca agtctcacga ctaatggtga cagaaattcg aaggagagca 420

tcaaacaaat ccacagaggc tgcaagtcgg gccatagttc agttcctaga gattaatcag 480

agtgaagaag ccagtagagg ctggatgctt ctaacgacaa ttaatttgtt agcttcctct 540

ggtcagaaaa ccgtggactg catgacaaca atgtcagtgc cttccaccct ggttaaatgt 600

ttatatctgt tttttgacct tccacatgtg cctgaggcag ttggaggtgc acagaatgag 660

ctacctctag cagaacgtcg aggactactc cagaaagttt ttgtacagat cttagtgaaa 720

ctgtgcagtt ttgtttcccc tgcggaggag ctggctcaga aagatgatct ccagcttcta 780

ttcagtgcaa taacctcttg gtgccctccc tataacctgc cttggagaaa gagtgctgga 840

gaagtcctca tgaccatatc tcgtcatggt cttagtgtca atgtagtgaa gtatattcat 900

gagaaagagt gtttatctac atgtgttcag aatatgcagc aatcagatga cctgtctccc 960
ctagaaattg tcgaaatgtt tgctgggctt tcttgtttcc tcaaagattc cagcgatgtt 1020
tcccaaacac ttctggatga ttttcggata tggcaaggat ataattttct ttgtgatctc 1080
ttgcttagat tggaacaagc aaaagaggca gaatccaaag atgccttgaa agatctggtt 1140
aatctgataa cttccctaac aacatatggt gtcagtgaac taaaaccagc tggtattacc 1200
acaggggcac cctttttatt gcctggattt gcagtacctc agcctgcagg caaaggtcac 1260
agtgtgagaa acgtccaggc ctttgcagtt cttcaaaatg catttttaaa agcaaaaacc 1320
agcttccttg cccaaatcat ccttgatgct atcacaaata tttacatggc tgacaatgcc 1380
aattacttca tcctagagtc acagcacaca ttgtcacagt ttgcagagaa gatttctaaa 1440
ctcccagaag tacaaaacaa atactttgag atgctggagt ttgttgtttt tagcttaaat 1500
tatatacctt gtaaagaact tattagtgtc agtatcctct taaaatctag ctcttcttat 1560
cactgtagca ttattgcaat gaaaacactt cttaagttta caagacatga ctacatattt 1620
aaagacgtgt tcagggaggt tggccttttg gaggtcatgg taaacctttt gcataaatat 1680
gctgccctgt tgaaggatcc aactcaggca ctaaatgaac aaggggactc aagaaataat 1740
agttcagttg aagaccaaaa acacctggct ttattggtta tggagacctt gacagtgctt 1800
cttcaaggat caaacacaaa tgcaggaatt tttcgagaat ttggaggtgc aagatgtgca 1860
cataatatag taaagtaccc tcaatgccgg cagcatgcct tgatgactat ccaacagctg 1920
gtgctctccc caaatgggga cgatgacatg ggcactctcc tggggctaat gcattcagcc 1980
ccaccgacgg aattgcagtt gaagactgat attttaaggg ccctcctgtc ggtccttcga 2040
gaaagccatc gttcaagaac agtttttagg aaagttggag gatttgtgta cattacatcc 2100
ttgctcgttg ctatggaaag atctttgagc tgtccaccca agaatggctg ggagaaagtg 2160
aaccagaatc aagtgtttga acttcttcac actgtgttct gcacgttgac tgcagcaatg 2220
cgctatgagc cagccaactc tcatttcttc aaaacagaga ttcagtatga gaagttggca 2280
gatgctgttc gatttcttgg ctgcttctca gacctaagaa aaataagcgc catgaatgtc 2340
ttcccctcaa atacacagcc atttcaaaga cttttagagg aagatgtaat ctcaatagaa 2400
tcagtgtcac ccacgttacg gcactgcagt aaacttttta tttatcttta caaagtagcc 2460
acagattctt ttgacaggta tggctctgct ctgtcctctg aattaaaggg tgtatactaa 2520
gcatgacttc aacattaaga cttgaattat gagttaatcc aaaagaatat gaaaacagat 2580
gactcttctc ctatttgttt gaagtgtatc actttatttt tgaatattac tcttgctctg 2640
gtattgtttt tttttttttt tagcatggaa gcctgtgtac tatttgtaaa ttcaggttca 2700
attagttctc attgcagtta ttggaacagt ttctatgaag tttcctttaa tgatagtgtt 2760
tactgttgtt ttttttcttt ttagcctcaa aaggtgcatc tggacttatt gatatgtgtt 2820
atcaatagaa catcattatg attattctta tttccagaca cgtactccat cattctccac 2880
atatcatact cctcaaaaaa tgatatagtt cccaaagaga ggtgtcagtg tcttgaaccg 2940
tcaagttcaa gaggccatca gactcaatat ttactcattc cttcatgaaa taagtactta 3000

acaaaaagct gctgcatgcc aagtcactgt gctaggcatt gaggattcag ctctacacag 3060

ggctgtcttg gtccttgcta tcttttagct aaaatgtaga cacataaata aacaattaca 3120

tatagtgtga cacattctac agtgggggaa tccagggttc tcaggcagat tgtaggagag 3180

ccacttcatc tagatcactt attttcagtg cttcaactgt gttttcaatg aagatgtcac 3240

tttgaaaata atacttctaa tttatgcccc aagtgttatt gcttttaact taccatagtt 3300

ttactacata aaataaatga actgccctca tcaatagacc atgtaactac attggataga 3360

gcttgtaata ggttttgtgt ccttttctgt ggaatatctg ctttgaagta tcaactgaga 3420

tatttcctgc ttcttaacaa gatttcttag tcctcagtat ttacatttaa aaatccaaac 3480

agctttgaca acaattgt 3498

<210> 134
<211> 4650
<212> DNA
<213> Homo sapiens

<400> 134
tgcaatctcg gcttactgaa acctctgcct cccaggttca agcaattctc ctgcatcagc 60

ctcacaagta gctggaatta caagcatgca ctgctatgcc cggctaattt ttgtacttct 120

agtagagaca gggtttcacc atgttgacct ggctggtctc aaactcctga cctcaaatga 180

tccgcccatc ttggcttccc aaagtgctgg gattataggc agcactacac ctggcctgaa 240

ccatgttttt aaactaatat gactataatc tttttccctg tactttgaaa cagccattgt 300

gtgcaggcag cccgctgcca catcttccaa gatacgggaa atctaactgt ataacatgat 360

ggaaattaag ttatctgtta accctgacag tctctcttca ttgagtaatg taaattggtt 420

ccttaatctg tttcttaact ttttaatttt tgtacttatt taaatttagt tttggagaat 480

ggaatttttg caagttcctg aaacaccagc tcctgagcct gctgaagtcc tagaaatgca 540

ggaagactgt ccagaagcac aaatagggca ggaagcccaa gacgaggaca gcagtgattc 600

agaagcagat gagcagagca tcagccagga ccctaaggac actccaagcc aacccagcag 660

caccagccac aggccccggg cagcctcctg ccgcgcaaca gccgcctggt gtactgacag 720

tggctctgac gactccagac gctggtccga ccagctcagt ctagatgaga aagacggctt 780

catatttgtg aactattcag agggccagac cagagcccat ctgcagggcc cccttagcca 840

cccccacccc aatcccattg aggtgcggaa ttacagcaga ttgaaacctg ggtaccgatg 900

ggaacggcag ctggtgttca ggagtaagct gactatgcac acagcctttg atcgaaagga 960

caatgcacac ccagctgagg tcactgcctt gggcatctcc aaggatcaca gtaggatcct 1020

cgttggtgac agtcgaggcc gagttttcag ctggtctgtg agtgaccagc caggccgttc 1080

tgctgctgat cactgggtga aggatgaagg tggtgacagc tgctcaggct gctcggtgag 1140

gttttcactc acagaaagac gacaccattg caggaactgt ggtcagcttt tctgccagaa 1200

gtgcagtcgc tttcaatctg aaatcaaacg cttgaaaatc tcatccccgg tgcgtgtttg 1260

tcagaactgt tattataact tacagcatga gagaggttca gaagatgggc ctcgaaattg 1320

ttgaagattc aacaagctga gtggagacca tggtctgtag accccttccc gattctcctg 1380

tcccagcttg gaaggcattg aaaacagtct ccgtttacac atctcttcat accacgtgtt 1440

tgaagtgtta aaattcaaag ggatcattga ataaaacggg tgtagagtac aggaatgggg 1500

cagacgcgat tcaggtgaac agcacaagaa gaatatgagg tggttcctag gagcaacact 1560

ttcgacctcc agttctccct gatgacagta gctgtctcca agagaaaaat cctcacttat 1620

taactctctt ttcttgcatc tcatttttat agagctactc atccttattt ggaaaaacca 1680

acaacaaaaa aggcttttag aaaatggttg taaatctgac ttctttgcaa gtaactatgt 1740

atattgtaaa tagatataaa aggccttttt tctaaataag gacttaactg cctgtaacat 1800

gaaacttcaa actaaaccac taactcaatg aactacttat ggtttgtctg acatccctca 1860

cttaccaatt aattataaat atgttttttt aaatccccaa agacattatc tgtggtcttt 1920

ttttcctttc aagctcagcc tgtgtgcctg atgtcatttc tttcaagttg cccacagtat 1980

ctccacttaa actaggctag taaccaaaat aatgtggacc ttctttagga aacagtgtgg 2040

gagaatagga gtccagccgt aagataaact ggaaatattt gggcgtcttg tacctggcta 2100

cgcaccacct cagtgttgtt cctacataaa cagggcccct tttaaacttg tatgtggact 2160

gctgtttggt caaagaatac cttcttagca ttgcagaaag gtggtcagat gaccagtgta 2220

gtgcaggaaa cagccctgtc tcaactaatg gaaatatatt tgcatgtaac ccaaaattag 2280

cttatcttgc atagaacata ataagtatgt gtctttggtg acactaatgt tctactatag 2340

cttattttca aacaaggggt aaaaaaagga aagaaagaag tgtacagaat taacatataa 2400

actttgttgt aaaactgaat catgtcagaa ctgcttaaaa ttaaccttta ccatttaatg 2460

tcatctacct gaaaacagtg agatttatac tgtatcaatg tctatttttt tgtttttgct 2520

atgaatataa ttacagtatt ttaatattta gttatttaat ttgttctact agttggatac 2580

agaacacaca aatccagggg gattaaagct ggaaggggct aagagattag tttacagaga 2640

aaaggcttgg tggtgggatt tttttaaatg tgtgttatgt acatatatat atatatataa 2700

tatatattaa aaatgaaaca attaatctag attttaacat tttcagaaac ttagtgataa 2760

cattatgaac aattctaaaa gccctgtgat ttgaaaaata tagaatcatt aatggcccaa 2820

gataggcctt cacaccttca caggtgcgaa aggaaaggcc ttcacaccct cacagaggca 2880

tcatgcaaag gacagcggct ttggcttttc caattttcca tctttaggcc ctggtgagag 2940

gcacacttat gcactaaaat gcacatatat gcacatgcat tcaaaaatag gcatttggta 3000

caatggtgat cttgtacctg atgggctgaa accagcttaa gaacaaattt gttcttcctg 3060

atatgataac taggtctcca agagaaaata gaaaggctgc tttagtgcct tacgcttact 3120

aaatttaaat ctttatttac ctgggtttga gcctacagtc tatttatgat tacatatcaa 3180

aattgattaa aacacttcca tttctaaaag ttcaaatata cttgttaata aaaggattat 3240

cggcattaat actttaattt aaagaaaagt tgtgttctgt tttcctttct gtgtcttact 3300

ccccccacac tctccctccc ccatcaccat cttcaattct aataaataat gctgatgttc 3360

aacagttgca gaaattgtgc tattatgtaa ctgtgggcct tgcccctgtc tggccctcta 3420

gatgatttgt agcagtgtta ttctacactt tttaaaagaa gcgtcctcct tttgtccatg 3480

aatcatgttt accccatacc cagtggcaga ggtgttcttt aaagacttga atatatgaat 3540

gtgtgtgtgt agttacttaa aggttattcc tctttgtaat aggaaactat atgggatgaa 3600

cacttttaaa ctttccgaca caacttccat tactaacttt ctaacagaac ttccataact 3660

agaaggtgga aaccaaaacc ctcatggtag tatttcctct ggcagctggt gctgtgggca 3720

actgttttgt tcaatcgggt ttcttttctt tttgcctcta atgcagaaat caacagaatc 3780

actcacacat acaagtacac tcacatacat aaactaatta tttctctgga tatctttctg 3840

tgttccatgt aaatttattt accaacatct attgtcaaca tgtacatcta ccttagtatg 3900

gtctgcattc tttttctgag agtacctcat agggctcctg cctgatcttt gtagtttgtt 3960

cattcatcca tccacctgtt catttgttca tccatgtatt ctaacatttc tatgtagtgt 4020

gcaactctaa tgtcatgctt ttgaagaaga gaatagctgc ccatagcagc catccgtctg 4080

gataatagca aaacactcta gataagttat tttgcacttt cttatgtata aagttggtag 4140

aaacttattt ttgctttgta tcatttaaat acattttgtt ttggtaaatg aactgtgtat 4200

aaaatattta tgccgttaaa actgttttta gaaagtattt ttaatttcag caagtttggt 4260

tacttgttgc atgactctta acacagctga ctttttgtgt cagtgcaatg tatatttttt 4320

gtcctgttat taacttgtaa gccctagtaa tggccaatta tttgtacagc aacagaagta 4380

aattgaagat actggctaag actggattga ttgtggactt ttatactata ttgcagaaac 4440

caatatctgt ttcttggtgg ttatgtaaaa gacctgaaga attactatct agtgtgcagt 4500

ctgtgatatc tgaatgttca ttgtatattt gtctctgatg caaaaaggta gagtaacaca 4560

attacaatac atgattaaat gcaatagtcc aggtacttaa gtaatttttt tttcatttca 4620

aataaatacc tatttaccac caaaagaaag 4650

<210> 135
<211> 6407
<212> DNA
<213> Homo sapiens

<400> 135
agtctgcggg cctccggggc tgcggcgagg ccggagcgtc gcggcggaga ggacgagacc 60

gggacaagac cagggcagga gggagccggc cagccgcgag aaccccgcac gcccggcaag 120

atgctgtcct tgcggctgca gacgggcccc gagaaggccg agctccagga gctcaacgcc 180

cggctctatg actacgtgtg tcgggtgcgg gagctggagc gcgaaaacct actcctggag 240

gaggagctgc gcggccggcg cgggcgagag ggcctgtggg ccgaggggca ggcccgctgc 300

gccgaggagg cgcgcagctt gcggcagcag ctggacgagc tgagctgggc cactgcgctg 360

gcggagggcg agcgggacgc tctgcggcgc gagctgcggg agctgcagcg cctggatgcg 420

gaggagcgcg ccgcccgcgg ccgcctggac gccgagctgg gtgcgcagca gcgcgagctg 480

caggaggcgc tgggcgcgcg cgccgccctc gaggcgctgc tgggccgact gcaggccgag 540

cgccgaggcc tcgacgcggc ccacgaacgc gacgtgaggg agctgcgcgc gcgcgccgcc 600

agccttacca tgcatttccg cgcccgcgcc accggccccg ccgcgccgcc gccacgcctg 660

cgggaggtgc acgacagcta cgcactgctg gtggccgagt cgtggcggga gacggtgcag 720
ctgtacgagg acgaggtgcg cgagctggag gaggcgctgc ggcgcggcca ggagagcaga 780
ctccaggcgg aggaagagac gcggctgtgc gcgcaggagg cagaggcgct gcggcgcgag 840
gcgctcgggt tggagcagct gcgcgcgcgg ctggaggacg cgctgctgcg gatgcgcgag 900
gagtacggga tacaggccga ggagcggcag agagtgattg actgcctgga ggatgagaag 960
gcaaccctca ccttggccat ggctgactgg ctgcgggact atcaggacct cctgcaggtg 1020
aagaccggcc tcagtttgga ggtggcgacc taccgggcct tattggaagg agaaagtaat 1080
ccagagatag tgatctgggc tgagcacgtt gaaaacatgc cgtcagaatt cagaaacaaa 1140
tcctatcact ataccgactc actactacag agggaaaatg aaaggaatct attttcaagg 1200
cagaaagcac ctttggcaag tttcaatcac agctcggcac tgtattctaa cctgtcaggg 1260
caccgtggat ctcagacggg cacatctatt ggaggtgatg ccagaagagg cttcttgggc 1320
tcgggatatt cttcctcggc cactacccag caggaaaact catacggaaa agccgtcagc 1380
agtcaaacca acgtcagaac tttctctcca acctatggcc ttttaagaaa tactgaggct 1440
caagtgaaaa cattccctga cagaccaaaa gccggagata caagggaggt ccccgtttac 1500
ataggtgaag attccacaat tgcccgcgag tcgtaccggg atcgccgaga caaggtggca 1560
gcaggtgctt cggaaagcac acggtcaaat gagaggaccg tcattctggg aaagaaaaca 1620
gaagtgaaag ccacgaggga gcaagaaaga aacagaccag aaaccatccg aacaaagcca 1680
gaagagaaaa tgttcgattc taaagagaag gcttccgagg agagaaacct aagatgggaa 1740
gaattgacaa agttagataa ggaagcgaga cagagagaaa gccagcagat gaaggagaag 1800
gctaaggaga aggactcact gaaggagaag agtgtgcgag agagagaggt gccgattagt 1860
ctagaagtat cccaggacag aagagcagag gtgtccccga aaggtttgca gacgcctgtg 1920
aaggatgctg gtggtgggac cggtagagag gcagaagcaa gagagctacg gttcaggttg 1980
ggcaccagtg atgccactgg ttctctgcaa ggcgattcca tgacagaaac cgtagcagaa 2040
aacatcgtta ccagtatcct gaagcagttc actcagtctc cagagacaga agcatctgct 2100
gattcttttc cagacacaaa agtcacttac gtggacagga aagagcttcc tggggaaagg 2160
aaaacaaaga ctgaaatact tgtggagtct aaactgactg aggatgttga tgtttccgat 2220
gaagctggcc tggactacct tttaagcaag gatattaagg aagtggggct gaaaggcaag 2280
tcagccgagc agatgatagg agacatcatc aacctcggcc tgaaagggag ggaggggaga 2340
gcaaaggtcg tcaacgtgga gatcgtggag gagcccgtga gttatgtcag cggggagaag 2400
ccggaggagt tttccgtccc attcaaagtg gaggaggtcg aagatgtgtc gccaggcccc 2460
tgggggttgg ttaaggagga ggaaggttat ggagaaagcg atgtcacatt ctcagttaat 2520
cagcatcgaa ggaccaagca gcctcaggag aacacgactc acgtggaaga agtgacagag 2580
gcaggtgatt cagagggcga gcagagttat tttgtgtcca ctccagatga acaccccggg 2640
gggcacgaca gagatgacgg ctcggtgtac gggcagatcc acatcgagga ggaatccacc 2700
atcaggtact cttggcagga tgaaatcgtg caggggactc gaaggaggac acagaaggac 2760

ggtgcagtgg gcgagaaggt tgtgaagccc ttggatgtcc cagcgccctc tctggagggg 2820

gacctgggtt ccactcactg gaaagaacaa gctagaagcg gtgaatttca tgccgaaccc 2880

acagtcattg aaaaagaaat taaaataccc cacgaattcc acacctccat gaagggcatc 2940

tcctccaagg agccccggca gcagctggtg gaagtcatcg ggcagctgga ggaaaccctt 3000

cccgagcgca tgagggagga gctgtccgcc ctcaccagag aggggcaggg tgggccgggg 3060

agcgtttccg tggatgtcaa gaaggtccag ggtgctggtg gcagttccgt gaccctggtt 3120

gctgaagtca acgtctcaca aactgtggat gccgatcggt tagacctgga ggaggtgagc 3180

aaagatgagg ccagtgagat ggagaaggct gtggagtcgg tggttcggga gagcctgagc 3240

aggcaacgca gcccagcgcc tggcagccca gatgaggaag gtggagcgga ggcccccgct 3300

gctggcattc gctttaggcg ttgggccacc cgggagctgt acatcccttc aggcgagagc 3360

gaggttgctg gtggggcctc tcacagctcg ggacagcgca ctccccaggg cccagtgtcg 3420

gccactgtgg aggtcagcag ccccacaggc tttgcccagt cacaggtgct ggaggatgtg 3480

agccaggctg caaggcacat aaaactcggc ccctctgaag tctggaggac tgagcgaatg 3540

tcatatgaag gacccactgc agaagtggtg gagatggatg tgagtaacgt agaggcgatc 3600

cgcagccgga cacaggaagc gggagctctc ggtgtgtctg accgtggttc ctggagagac 3660

gcggacagta ggaatgacca ggcagttggt gtgagcttta aggcctctgc tggggaagga 3720

gaccaggccc acagagaaca gggcaaggag caggccatgt ttgataagaa ggtgcagctc 3780

cagagaatgg tagaccaaag gtcggtgatt tcagatgaaa agaaagttgc cctcctctat 3840

ctagacaatg aggaggagga gaatgatggg cattggtttt aataagcaga aacattttgt 3900

tttaatggca gcctgttggc gacgtgccaa catccaaagg ccttaactta ttttaagagg 3960

ccgagggagt ctatgaaaat ctcccctttt ttactttttt aaagagtact cccggcatgg 4020

tcaatttcct ttatagttaa tccgtaaagg tttccagtta attcatgcct taaaaggcac 4080

tgcaatttta tttttgagtt gggactttta caaaacactt ttttccctgg agtcttctct 4140

ccacttctgg agatgaattt ctatgttttg cacctggtca cagacatggc ttgcatctgt 4200

ttgaaactac aattaattat agatgtcaaa acattaacca gattaaagta atatatttaa 4260

gagtaaattt tgcttgcatg tgctaatatg aaataacaga ctaacatttt aggggaaaaa 4320

taaatacaat ttagactcta aaaagtcttt tcaaaaagaa atgggaaata ggcagactgt 4380

ttatgttaaa aaaattcttg ctaaatgatt tcatctttag gaaaaaatta cttgccatat 4440

agagctaaat tcatcttaag acttgaatga attgctttct atgtacagaa ctttaaacaa 4500

tatagtattt atggcgagga cagctgtagt ctgttgtgat atttcacatt ctatttgcac 4560

aggttccctg gcactggtag ggtagatgat tattgggaat cgcttacagt accatttcat 4620

tttttggcac taggtcatta agtagcacac agtctgaatg cccttttctg gagtggccag 4680

ttcctatcag actgtgcaga cttgcgcttc tctgcacctt atcccttagc acccaaacat 4740

ttaatttcac tggtgggagg tagaccttga agacaatgaa gagaatgccg atactcagac 4800

tgcagctgga ccggcaagct ggctgtgtac aggaaaattg gaagcacaca gtggactgtg 4860

cctcttaaag atgcctttcc caaccctcca ttcatgggat gcaggtcttt ctgagctcaa 4920

gggtgaaaga tgaatacaat aacaaccatg aacccacctc acggaagctt tttttgcact 4980

ttgaacagaa gtcattgcag ttggggtgtt ttgtccaggg aaacagttta ttaaatagaa 5040

ggatgttttg gggaaggaac tggatatctc tcctgcagcc cagcaccgag atacccagga 5100

cgggcctggg gggcgagaaa ggcccccatg ctcatgggcc gcggagtgtg gacctgtaga 5160

taggcaccac cgagtttaag atactgggat gagcatgctt cattggattc attttatttt 5220

acacgtcagt attgttttaa agtttctgtc tgtaaagtgt agcatcatat ataaaaagag 5280

tttcgctagc agcgcatttt ttttagttca ggctagcttc tttcacataa tgctgtctca 5340

gctgtatttc cagtaacaca gcatcatcac actgactgtg gcgcactggg gaataacagt 5400

ctgagctagc accaccctca gccaggctac aacgacagca ctggagggtc ttccctctca 5460

gattcacctg gaggccctca gacccccagg gtgcacgtct ccccaggtcc tgggagtggc 5520

taccgcaggt agtttctgga gagcacgttt tcttcattga taagtggagg agaaatgcag 5580

cacagctttc aagatactat tttaaaaaca ccatgaatca gatagggaaa gaaagttgat 5640

tggaatggca agtttaaacc tttgttgtcc atctgccaaa tgaactagtg attgtcagac 5700

tggtatggag gtgactgctt tgtaaggttt tgtcgtttct aatacagaca gagatgtgct 5760

gattttgttt tagctgtaac aggtaatggt ttttggatag atgattgact ggtgagaatt 5820

tggtcaaggt gacagcctcc tgtctgatga caggacagac tggtggtgag gagtctaagt 5880

gggctcagtt tgatgtcagt gtctgggctc atgacttgta aatggaagct gatgtgaaca 5940

ggtaattaat attatgaccc acttctattt actttgggaa atatcttgga tcttaattat 6000

catctgcaag tttcaagaag tattctgcca aaagtattta caagtatgga ctcatgagct 6060

attgttggtt gctaaatgtg aatcacgcgg gagtgagtgt gcccttcaca ctgtgacatt 6120

gtgacattgt gacaagctcc atgtccttta aaatcagtca ctctgcacac aagagaaatc 6180

aacttcgtgg ttggatgggg ccggaacaca accagtcttt ttgtatttat tgttactgag 6240

acaaaacagt actcactgag tgtttttcag tttcctactg gtggttttga tattgtttgt 6300

ttaagatgta tatttagaat gacatcatct aagaagctga ttttgctaaa ctcctgttcc 6360

ctacaatggg aaatgtcaca agaatgtgca aaaataaaaa tctgagg 6407

<210> 136
<211> 7343
<212> DNA
<213> Homo sapiens

<400> 136
agtctgcggg cctccggggc tgcggcgagg ccggagcgtc gcggcggaga ggacgagacc 60

gggacaagac cagggcagga gggagccggc cagccgcgag aaccccgcac gcccggcaag 120

atgctgtcct tgcggctgca gacggccccc gagaaggccg agctccagga gctcaacgcc 180

cggctctatg actacgtgtg tcgggtgcgg gagctggagc gcgaaaacct actcctggag 240

gaggagctgc gcggccggcg cgggcgagag ggcctgtggg ccgaggggca ggcccgctgc 300

gccgaggagg cgcgcagctt gcggcagcag ctggacgagc tgagctgggc cactgcgctg 360

gcggagggcg agcgggacgc tctgcggcgc gagctgcggg agctgcagcg cctggatgcg 420

gaggagcgcg ccgcccgcgg ccgcctggac gccgagctgg gtgcgcagca gcgcgagctg 480

caggaggcgc tgggcgcgcg cgccgccctc gaggcgctgc tgggccgact gcaggccgag 540

cgccgaggcc tcgacgcggc ccacgaacgc gacgtgaggg agctgcgcgc gcgcgccgcc 600

agccttacca tgcatttccg cgcccgcgcc accggccccg ccgcgccgcc gccacgcctg 660

cgggaggtgc acgacagcta cgcactgctg gtggccgagt cgtggcggga gacggtgcag 720

ctgtacgagg acgaggtgcg cgagctggag gaggcgctgc ggcgcggcca ggagagcaga 780

ctccaggcgg aggaagagac gcggctgtgc gcgcaggagg cagaggcgct gcggcgcgag 840

gcgctcgggt tggagcagct gcgcgcgcgg ctggaggacg cgctgctgcg gatgcgcgag 900

gagtacggga tacaggccga ggagcggcag agagtgattg actgcctgga ggatgagaag 960

gcaaccctca ccttggccat ggctgactgg ctgcgggact atcaggacct cctgcaggtg 1020

aagaccggcc tcagtttgga ggtggcgacc taccgggcct tattggaagg agaaagtaat 1080

ccagagatag tgatctgggc tgagcacgtt gaaaacatgc cgtcagaatt cagaaacaaa 1140

tcctatcact ataccgactc actactacag agggaaaatg aaaggaatct attttcaagg 1200

cagaaagcac ctttggcaag tttcaatcac agctcggcac tgtattctaa cctgtcaggg 1260

caccgtggat ctcagacggg cacatctatt ggaggtgatg ccagaagagg cttcttgggc 1320

tcgggatatt cttcctcggc cactacccag caggaaaact catacggaaa agccgtcagc 1380

agtcaaacca acgtcagaac tttctctcca acctatggcc ttttaagaaa tactgaggct 1440

caagtgaaaa cattccctga cagaccaaaa gccggagata caagggaggt ccccgtttac 1500

ataggtgaag attccacaat tgcccgcgag tcgtaccggg atcgccgaga caaggtggca 1560

gcaggtgctt cggaaagcac acggtcaaat gagaggaccg tcattctggg aaagaaaaca 1620

gaagtgaaag ccacgaggga gcaagaaaga aacagaccag aaaccatccg aacaaagcca 1680

gaagagaaaa tgttcgattc taaagagaag gcttccgagg agagaaacct aagatgggaa 1740

gaattgacaa agttagataa ggaagcgaga cagagagaaa gccagcagat gaaggagaag 1800

gctaaggaga aggactcact gaaggagaag agtgtgcgag agagagaggt gccgattagt 1860

ctagaagtat cccaggacag aagagcagag gtgtccccga aaggtttgca gacgcctgtg 1920

aaggatgctg gtggtgggac cggtagagag gcagaagcaa gagagctacg gttcaggttg 1980

ggcaccagtg atgccactgg ttctctgcaa ggcgattcca tgacagaaac cgtagcagaa 2040

aacatcgtta ccagtatcct gaagcagttc actcagtctc cagagacaga agcatctgct 2100

gattcttttc cagacacaaa agtcacttac gtggacagga aagagcttcc tggggaaagg 2160

aaaacaaaga ctgaaatact tgtggagtct aaactgactg aggatgttga tgtttccgat 2220

gaagctggcc tggactacct tttaagcaag gatattaagg aagtggggct gaaaggcaag 2280

tcagccgagc agatgatagg agacatcatc aacctcggcc tgaaagggag ggaggggaga 2340

gcaaaggtcg tcaacgtgga gatcgtggag gagcccgtga gttatgtcag cggggagaag 2400

ccggaggagt tttccgtccc attcaaagtg gaggaggtcg aagatgtgtc gccaggcccc 2460

tgggggttgg ttaaggagga ggaaggttat ggagaaagcg atgtcacatt ctcagttaat 2520

cagcatcgaa ggaccaagca gcctcaggag aacacgactc acgtggaaga agtgacagag 2580

gcaggtgatt cagagggcga gcagagttat tttgtgtcca ctccagatga acaccccggg 2640

gggcacgaca gagatgacgg ctcggtgtac gggcagatcc acatcgagga ggaatccacc 2700

atcaggtact cttggcagga tgaaatcgtg caggggactc gaaggaggac acagaaggac 2760

ggtgcagtgg gcgagaaggt tgtgaagccc ttggatgtcc cagcgccctc tctggagggg 2820

gacctgggtt ccactcactg gaaagaacaa gctagaagcg gtgaatttca tgccgaaccc 2880

acagtcattg aaaaagaaat taaaataccc cacgaattcc acacctccat gaagggcatc 2940

tcctccaagg agccccggca gcagctggtg gaagtcatcg ggcagctgga ggaaaccctt 3000

cccgagcgca tgagggagga gctgtccgcc ctcaccagag aggggcaggg tggccggggg 3060

agcgtttccg tggatgtcaa gaaggtccag ggtgctggtg gcagttccgt gaccctggtt 3120

gctgaagtca acgtctcaca aactgtggat gccgatcggt tagacctgga ggaggtgagc 3180

aaagatgagg ccagtgagat ggagaaggct gtggagtcgg tggttcggga gagcctgagc 3240

aggcaacgca gcccagcgcc tggcagccca gatgaggaag gtggagcgga ggccccggct 3300

gctggcattc gctttaggcg ttgggccacc cgggagctgt acatcccttc aggcgagagc 3360

gaggttgctg gtggggcctc tcacagctcg ggacagcgca ctccccaggg cccagtgtcg 3420

gccactgtgg aggtcagcag ccccacaggc tttgcccagt cacaggtgct ggaggatgtg 3480

agccaggctg caaggcacat aaaactcggc ccctctgaag tctggaggac tgagcgaatg 3540

tcatatgaag gacccactgc agaagtggtg ggggtaagtg cgggaggtga cctaagtcag 3600

gcagcgagcc cgaccggagc cagccggtct gtgaggcatg tcacgctggg tcccggtcaa 3660

agtccactgt ccagagaagt catcttccta ggccctgccc ctgcctgtcc agaggcatgg 3720

ggctcgccag aacctggccc agcagagtct tctgcagata tggacggatc agggaggcac 3780

agcacatttg gctgcagaca atttcatgct gaaaaggaga ttatttttca gggccccatt 3840

tctgctgcag ggaaggttgg tgattatttt gcaacagaag agtcagtggg tacccagact 3900

tctgtcaggc aactccagtt aggccctaaa gaagggttca gtgggcaaat ccagttcaca 3960

gctccacttt cagacaaggt ggagttgggt gtcacaggag attctgtaca catggaaggg 4020

ttgccaggga gcagcacatc catcaggcac atcagcattg ggcctcagag gcatcagacc 4080

acccagcaga tagtttacca tgggctggtt ccccaactgg gggaatctgg tgactcagag 4140

agcactgtgc acggagaggg ctcagcagat gtgcaccagg ccactcacag tcatacctcg 4200

ggtagacaaa ccgttatgac tgaaaagagc accttccaaa gtgtcgtttc tgaatctccc 4260

caggaggata gtgcagagga cacatcaggg gcagaaatga catcgggtgt tagcagatcc 4320

tttaggcaca ttcgactagg tcctacagaa acggaaacct ctgaacacat tgccatccgt 4380

ggacccgtgt ccagaacatt tgtgcttgct ggttcagcgg actcccctga gctaggcaag 4440

ttagcagaca gcagcagaac gctaaggcac attgcaccag ggcccaaaga aacttcgttt 4500

acctttcaga tggatgtgag taacgtagag gcgatccgca gccggacaca ggaagcggga 4560

gctctcggtg tgtctgaccg tggttcctgg agagacgcgg acagtaggaa tgaccaggca 4620

gttggtgtga gctttaaggc ctctgctggg gaaggagacc aggcccacag agaacagggc 4680

aaggagcagg ccatgtttga taagaaggtg cagctccaga gaatggtaga ccaaaggtcg 4740

gtgatttcag atgaaaagaa agttgccctc ctctatctag acaatgagga ggaggagaat 4800

gatgggcatt ggttttaata agcagaaaca ttttgtttta atggcagcct gttggcgacg 4860

tgccaacatc caaaggcctt aacttatttt aagaggccga gggagtctat gaaaatctcc 4920

ccttttttac ttttttaaag agtactcccg gcatggtcaa tttcctttat agttaatccg 4980

taaaggtttc cagttaattc atgccttaaa aggcactgca attttatttt tgagttggga 5040

cttttacaaa acactttttt ccctggagtc ttctctccac ttctggagat gaatttctat 5100

gttttgcacc tggtcacaga catggcttgc atctgtttga aactacaatt aattatagat 5160

gtcaaaacat taaccagatt aaagtaatat atttaagagt aaattttgct tgcatgtgct 5220

aatatgaaat aacagactaa cattttaggg gaaaaataaa tacaatttag actctaaaaa 5280

gtcttttcaa aaagaaatgg gaaataggca gactgtttat gttaaaaaaa ttcttgctaa 5340

atgatttcat ctttaggaaa aaattacttg ccatatagag ctaaattcat cttaagactt 5400

gaatgaattg ctttctatgt acagaacttt aaacaatata gtatttatgg cgaggacagc 5460

tgtagtctgt tgtgatattt cacattctat ttgcacaggt tccctggcac tggtagggta 5520

gatgattatt gggaatcgct tacagtacca tttcattttt tggcactagg tcattaagta 5580

gcacacagtc tgaatgccct tttctggagt ggccagttcc tatcagactg tgcagacttg 5640

cgcttctctg caccttatcc cttagcaccc aaacatttaa tttcactggt gggaggtaga 5700

ccttgaagac aatgaagaga atgccgatac tcagactgca gctggaccgg caagctggct 5760

gtgtacagga aaattggaag cacacagtgg actgtgcctc ttaaagatgc ctttcccaac 5820

cctccattca tgggatgcag gtctttctga gctcaagggt gaaagatgaa tacaataaca 5880

accatgaacc cacctcacgg aagctttttt tgcactttga acagaagtca ttgcagttgg 5940

ggtgttttgt ccagggaaac agtttattaa atagaaggat gttttgggga aggaactgga 6000

tatctctcct gcagcccagc accgagatac ccaggacggg cctggggggc gagaaaggcc 6060

cccatgctca tgggccgcgg agtgtggacc tgtagatagg caccaccgag tttaagatac 6120

tgggatgagc atgcttcatt ggattcattt tattttacac gtcagtattg ttttaaagtt 6180

tctgtctgta aagtgtagca tcatatataa aaagagtttc gctagcagcg catttttttt 6240

agttcaggct agcttctttc acataatgct gtctcagctg tatttccagt aacacagcat 6300

catcacactg actgtggcgc actggggaat aacagtctga gctagcacca ccctcagcca 6360

ggctacaacg acagcactgg agggtcttcc ctctcagatt cacctggagg ccctcagacc 6420

cccagggtgc acgtctcccc aggtcctggg agtggctacc gcaggtagtt tctggagagc 6480

acgttttctt cattgataag tggaggagaa atgcagcaca gctttcaaga tactatttta 6540

aaaacaccat gaatcagata gggaaagaaa gttgattgga atggcaagtt taaacctttg 6600

ttgtccatct gccaaatgaa ctagtgattg tcagactggt atggaggtga ctgctttgta 6660

aggttttgtc gtttctaata cagacagaga tgtgctgatt ttgttttagc tgtaacaggt 6720

aatggttttt ggatagatga ttgactggtg agaatttggt caaggtgaca gcctcctgtc 6780

tgatgacagg acagactggt ggtgaggagt ctaagtgggc tcagtttgat gtcagtgtct 6840

gggctcatga cttgtaaatg gaagctgatg tgaacaggta attaatatta tgacccactt 6900

ctatttactt tgggaaatat cttggatctt aattatcatc tgcaagtttc aagaagtatt 6960

ctgccaaaag tatttacaag tatggactca tgagctattg ttggttgcta aatgtgaatc 7020

acgcgggagt gagtgtgccc ttcacactgt gacattgtga cattgtgaca agctccatgt 7080

cctttaaaat cagtcactct gcacacaaga gaaatcaact tcgtggttgg atggggccgg 7140

aacacaacca gtctttttgt atttattgtt actgagacaa aacagtactc actgagtgtt 7200

tttcagtttc ctactggtgg ttttgatatt gtttgtttaa gatgtatatt tagaatgaca 7260

tcatctaaga agctgatttt gctaaactcc tgttccctac aatgggaaat gtcacaagaa 7320

tgtgcaaaaa taaaaatctg agg 7343

<210> 137
<211> 3812
<212> DNA
<213> Homo sapiens

<400> 137
gggcctactc tgccgccgcc gccgcccgcc cgctccagcc gccgccgccg ccgccaccgc 60

cctccaggct ccgggacccg gcccgcgcca ccgcccccgt gcgcgccccg ccgccgccgc 120

cttcgccttc gccttttgtt tcctccgctc cggcgccccc gccccggctc gcgctttgca 180

ggggacgcag cgcgcgcccc cagcgggccc gggaaaagcc gcggcgcgcg cgcgcgcctg 240

cgcggcggac ccctccttct cctccccgcg tgcgcgtgcc cttcttggct gcgcgccggc 300

gccgcctggc gggcgggagg ggaggtggca ggcgcgtttg caggaggggc gcacctcttc 360

gctcgcgcac ccccccggaa ggtagaccgg gaaggggagg cgggcgggcg gagaggagag 420

agtggcgcgc agtccagcga gggcgggggt tggctatgtg gggggtggtg caccccgcag 480

tctagacagt ctgatccggg ctgggggcgt gtacactcgg cgcacctgcg agactacaga 540

gcctcgggcc ggcacgtgtg gggagtgtgg acacgtctgc tgcgccccgc ttctcgctgc 600

tgaggggaag ggagggggcg ggcaggtgca gcggccgggc tagtgggagg gggcggcggc 660

catggagcgg gtgaacgacg cttcgtgcgg cccgtctggc tgctacacat accaggtgag 720

cagacacagc acggagatgc tgcacaacct gaaccagcag cgcaaaaacg gcgggcgctt 780

ctgcgacgtg ctcttgcggg taggcgacga gagcttccca gcgcaccgcg ccgtgctggc 840

cgcctgcagc gagtactttg agtcggtgtt cagcgcccag ttgggcgacg gcggagctgc 900

ggacgggggt ccggctgatg taggggggcgc gacggcagca ccaggcggcg ggccgggggg 960

cagccgggag ctggagatgc acactatcag ctccaaggta tttggggaca ttctggactt 1020

cgcctacact tcccgcatcg tggtgcgctt ggagagcttt cccgaactca tgacggccgc 1080

caagttcctg ctgatgaggt cggttatcga gatctgccag gaagtcatca aacagtccaa 1140

cgtacagatc ctggtacccc ctgcccgcgc cgatataatg ctctttcgcc cccctgggac 1200

ctcggacttg ggcttccctt tggacatgac caacggggca gccttggcag ccaacagcaa 1260

tggcatcgcc ggcagcatgc agccagagga ggaggcagct cgggcggctg gtgcagccat 1320

tgcaggccaa gcctctttgc ctgtgttacc tggggtggac cgcttgccca tggtggctgg 1380

acccctatcc ccccaactgc tgacttcccc attccccagt gtggcatcca gtgcccctcc 1440

cctgactggc aagcgaggcc ggggccgccc aaggaaggcc aacctgctgg actcaatgtt 1500

tgggtcccca gggggcctga gggaggcagg catccttcca tgcggtctat gtggtaaggt 1560

gttcactgat gccaaccggc tccggcagca cgaggcccag cacggtgtca ccagcctcca 1620

gctgggctac atcgaccttc ctcctccgag gctgggtgag aatgggctac ccatctctga 1680

agaccccgac ggcccccgaa agaggagccg gaccaggaag caggtggctt gtgagatctg 1740

cggcaagatc ttccgtgatg tgtatcatct taaccggcac aagctgtccc actctgggga 1800

gaagccctac tcctgccctg tgtgtgggtt gcggttcaag agaaaagacc gcatgtccta 1860

ccatgtgcgg tcccatgatg ggtccgtggg caagccttac atctgccaga gctgtgggaa 1920

aggcttctcc aggcctgatc acttgaacgg acatatcaag caggtgcaca cttctgagcg 1980

gcctcacaag tgtcagacct gcaatgcttc ttttgccacc cgagaccgtc tgcgctccca 2040

cctggcctgt catgaagaca aggtgccctg ccaggtgtgt gggaagtact tgcgggcagc 2100

atacatggca gaccacctga agaagcacag cgagggggcc agcaacttct gcagtatctg 2160

taaccgaggt ttctcctctg cctcctactt aaaggtccat gttaaaaccc accacggtgt 2220

tccccttccc caggtctcca ggcaccagga gcccatcctg aatgggggag cagcgttcca 2280

ctgcgccagg acctatggca acaaagaagg ccagaaatgc tcacatcagg atccgattga 2340

gagctctgac tcctatggtg acctctcaga tgccagcgac ctgaagacgc cagagaagca 2400

gagtgccaat ggctctttct cctgcgacat ggcagtcccc aaaaacaaaa tggagtctga 2460

tggggagaag aagtacccat gccctgaatg tgggagcttc ttccgctcta agtcctactt 2520

gaacaaacac atccagaagg tgcatgtccg ggctctcggg ggccccctgg gggacctggg 2580

ccctgccctt ggctcacctt tctctcctca gcagaacatg tctctcctcg agtcctttgg 2640

gtttcagatt gttcagtcgg catttgcgtc atctttagta gatcctgagg ttgaccagca 2700

gcccatgggg cctgaaggga aatgaggcag ctgctgtgtc cccacggaaa caaccatctg 2760

gggactgctg ggaaatgctg tgaatgcgga gggaagtgat gtttgggttc tgtagctgag 2820

agatttttat tcatttttaa ctgcccccca accccactcc aactccttct ccaccaccca 2880

ttctcccaat ggtctttaga aatagatttt catctgatat tctgcagaaa tatcaatgag 2940

acttggtatg ggacaggggc agaaaacact acataggcct ccaaggcaaa accagtccca 3000

gtttctttaa tgggaagaag ctggaattcc tggtgctcaa ttcttagtga ccccaatcct 3060

atacccaaat ctatgatatt ctgggacctc agtgattttg gtcccctccc acttctctag 3120

ttcgtcatcc tcccttccca tatccttcaa aagaaccaca ctagggtctc cacctactta 3180

tacaatgcgg atgcccaact gtttttaagg aagccagaag catcccatgg accatggggt 3240

gagtgtcctc caagagcccc ctgagctcag ccctctgcct ggagggctcc agacctttct 3300

gagccctgct tggaggcgag cattttcact gctaggacaa gctcagctgt tgaggacacc 3360

cccaccccaa atttcagttc ttacgtgatt ttaaccattc aacatgctgt tgggttttaa 3420

ttctctaatt attattatta ttgttattat tttttaggac cagttgtagt gaattgctac 3480

tgaaagctat cccaggtgat acagagctct ttgtaaaccg cagtcacaca ttagggttag 3540

tattaaactt tgtttagatg taccataatt aacttggcta gttgattgtt tgaagtctat 3600

ggaagaaata gttttatgca aaattttaaa aaatgccagt ctggtcaggg aagtaggggg 3660

tttcaatgct gttgggaacc aggaaggtgg gacagccggc aggtagggac attgtgtacc 3720

tcagttgtgt cacatgtgag caagcccagg ttgaccttgt gatgtgaatt gatctgatca 3780

gactgtatta aaaatgttag tacattactc ta 3812

<210> 138
<211> 3674
<212> DNA
<213> Homo sapiens

<400> 138
gggcctactc tgccgccgcc gccgcccgcc cgctccagcc gccgccgccg ccgccaccgc 60

cctccaggct ccgggacccg gcccgcgcca ccgcccccgt gcgcgccccg ccgccgccgc 120

cttcgccttc gccttttgtt tcctccgctc cggcgccccc gccccggctc gcgctttgca 180

ggggacgcag cgcgcgcccc cagcgggccc gggaaaagcc gcggcgcgcg cgcgcgcctg 240

cgcggcggac ccctccttct cctccccgcg tgcgcgtgcc cttcttggct gcgcgccggc 300

gccgcctggc gggcgggagg ggaggtggca ggcgcgtttg caggaggggc gcacctcttc 360

gctcgcgcac ccccccggaa ggtagaccgg gaaggggagg cgggcgggcg gagaggagag 420

agtggcgcgc agtccagcga gggcgggggt tggctatgtg gggggtggtg caccccgcag 480

tctagacagt ctgatccggg ctgggggcgt gtacactcgg cgcacctgcg agactacaga 540

gcctcgggcc ggcacgtgtg gggagtgtgg acacgtctgc tgcgccccgc ttctcgctgc 600

tgaggggaag ggagggggcg ggcaggtgca gcggccgggc tagtgggagg gggcggcggc 660

catggagcgg gtgaacgacg cttcgtgcgg cccgtctggc tgctacacat accaggtgag 720

cagacacagc acggagatgc tgcacaacct gaaccagcag cgcaaaaacg gcgggcgctt 780

ctgcgacgtg ctcttgcggg taggcgacga gagcttccca gcgcaccgcg ccgtgctggc 840

cgcctgcagc gagtactttg agtcggtgtt cagcgcccag ttgggcgacg gcggagctgc 900

ggacgggggt ccggctgatg tagggggcgc gacggcagca ccaggcggcg gggccggggg 960

cagccgggag ctggagatgc acactatcag ctccaaggta tttggggaca ttctggactt 1020

cgcctacact tcccgcatcg tggtgcgctt ggagagcttt cccgaactca tgacggccgc 1080

caagttcctg ctgatgaggt cggttatcga gatctgccag gaagtcatca aacagtccaa 1140

cgtacagatc ctggtacccc ctgcccgcgc cgatataatg ctctttcgcc cccctgggac 1200

ctcggacttg ggcttccctt tggacatgac caacggggca gccttggcag ccaacagcaa 1260

tggcatcgcc ggcagcatgc agccagagga ggaggcagct cgggcggctg gtgcagccat 1320

tgcaggccaa gcctctttgc ctgtgttacc tggggtggac cgcttgccca tggtggctgg 1380

acccctatcc ccccaactgc tgacttcccc attccccagt gtggcatcca gtgcccctcc 1440

cctgactggc aagcgaggcc ggggccgccc aaggaaggcc aacctgctgg actcaatgtt 1500

tgggtcccca gggggcctga gggaggcagg catccttcca tgcggtctat gtggtaaggt 1560

gttcactgat gccaaccggc tccggcagca cgaggcccag cacggtgtca ccagcctcca 1620

gctgggctac atcgaccttc ctcctccgag gctgggtgag aatgggctac ccatctctga 1680

agaccccgac ggcccccgaa agaggagccg gaccaggaag caggtggctt gtgagatctg 1740

cggcaagatc ttccgtgatg tgtatcatct taaccggcac aagctgtccc actctgggga 1800

gaagccctac tcctgccctg tgtgtgggtt gcggttcaag agaaaagacc gcatgtccta 1860

ccatgtgcgg tcccatgatg ggtccgtggg caagccttac atctgccaga gctgtgggaa 1920

aggcttctcc aggcctgatc acttgaacgg acatatcaag caggtgcaca cttctgagcg 1980

gcctcacaag tgtcagacct gcaatgcttc ttttgccacc cgagaccgtc tgcgctccca 2040

cctggcctgt catgaagaca aggtgccctg ccaggtgtgt gggaagtact tgcgggcagc 2100

atacatggca gaccacctga agaagcacag cgaggggccc agcaacttct gcagtatctg 2160

taaccgagaa ggccagaaat gctcacatca ggatccgatt gagagctctg actcctatgg 2220

tgacctctca gatgccagcg acctgaagac gccagagaag cagagtgcca atggctcttt 2280

ctcctgcgac atggcagtcc ccaaaaacaa aatggagtct gatggggaga agaagtaccc 2340

atgccctgaa tgtgggagct tcttccgctc taagtcctac ttgaacaaac acatccagaa 2400

ggtgcatgtc cgggctctcg gggcccccct gggggacctg ggccctgccc ttggctcacc 2460

tttctctcct cagcagaaca tgtctctcct cgagtccttt gggtttcaga ttgttcagtc 2520

ggcatttgcg tcatctttag tagatcctga ggttgaccag cagcccatgg ggcctgaagg 2580

gaaatgaggc agctgctgtg tccccacgga aacaaccatc tggggactgc tgggaaatgc 2640

tgtgaatgcg gagggaagtg atgtttgggt tctgtagctg agagattttt attcattttt 2700

aactgccccc caaccccact ccaactcctt ctccaccacc cattctccca atggtcttta 2760

gaaatagatt ttcatctgat attctgcaga aatatcaatg agacttggta tgggacaggg 2820

gcagaaaaca ctacataggc ctccaaggca aaaccagtcc cagtttcttt aatgggaaga 2880

agctggaatt cctggtgctc aattcttagt gaccccaatc ctatacccaa atctatgata 2940

ttctgggacc tcagtgattt tggtcccctc ccacttctct agttcgtcat cctcccttcc 3000

catatccttc aaaagaacca cactagggtc tccacctact tatacaatgc ggatgcccaa 3060

ctgtttttaa ggaagccaga agcatcccat ggaccatggg gtgagtgtcc tccaagagcc 3120

ccctgagctc agccctctgc ctggagggct ccagaccttt ctgagccctg cttggaggcg 3180

agcattttca ctgctaggac aagctcagct gttgaggaca cccccacccc aaatttcagt 3240

tcttacgtga ttttaaccat tcaacatgct gttgggtttt aattctctaa ttattattat 3300

tattgttatt attttttagg accagttgta gtgaattgct actgaaagct atcccaggtg 3360

atacagagct ctttgtaaac cgcagtcaca cattagggtt agtattaaac tttgtttaga 3420

tgtaccataa ttaacttggc tagttgattg tttgaagtct atggaagaaa tagttttatg 3480

caaaatttta aaaaatgcca gtctggtcag ggaagtaggg ggtttcaatg ctgttgggaa 3540

ccaggaaggt gggacagccg gcaggtaggg acattgtgta cctcagttgt gtcacatgtg 3600

agcaagccca ggttgacctt gtgatgtgaa ttgatctgat cagactgtat taaaaatgtt 3660

agtacattac tcta 3674

<210> 139
<211> 3021
<212> DNA
<213> Homo sapiens

<400> 139
gggcctactc tgccgccgcc gccgcccgcc cgctccagcc gccgccgccg ccgccaccgc 60

cctccaggct ccgggacccg gcccgcgcca ccgcccccgt gcgcgccccg ccgccgccgc 120

cttcgccttc gccttttgtt tcctccgctc cggcgccccc gccccggctc gcgctttgca 180

ggggacgcag cgcgcgcccc cagcgggccc gggaaaagcc gcggcgcgcg cgcgcgcctg 240

cgcggcggac ccctccttct cctccccgcg tgcgcgtgcc cttcttggct gcgcgccggc 300

gccgcctggc gggcgggagg ggaggtggca ggcgcgtttg caggaggggc gcacctcttc 360

gctcgcgcac ccccccggaa ggtagaccgg gaaggggagg cgggcgggcg gagaggagag 420

agtggcgcgc agtccagcga gggcgggggt tggctatgtg gggggtggtg caccccgcag 480

tctagacagt ctgatccggg ctgggggcgt gtacactcgg cgcacctgcg agactacaga 540

gcctcgggcc ggcacgtgtg gggagtgtgg acacgtctgc tgcgccccgc ttctcgctgc 600

tgaggggaag ggagggggcg ggcaggtgca gcggccgggc tagtgggagg gggcggcggc 660

catggagcgg gtgaacgacg cttcgtgcgg cccgtctggc tgctacacat accaggtgag 720

cagacacagc acggagatgc tgcacaacct gaaccagcag cgcaaaaacg gcgggcgctt 780

ctgcgacgtg ctcttgcggg taggcgacga gagcttccca gcgcaccgcg ccgtgctggc 840

cgcctgcagc gagtactttg agtcggtgtt cagcgcccag ttgggcgacg gcggagctgc 900

ggacgggggt ccggctgatg tagggggcgc gacggcagca ccaggcggcg gggccggggg 960

cagccgggag ctggagatgc acactatcag ctccaaggta tttggggaca ttctggactt 1020

cgcctacact tcccgcatcg tggtgcgctt ggagagcttt cccgaactca tgacggccgc 1080

caagttcctg ctgatgaggt cggttatcga gatctgccag gaagtcatca aacagtccaa 1140

cgtacagatc ctggtacccc ctgcccgcgc cgatataatg ctctttcgcc cccctgggac 1200

ctcggacttg ggcttccctt tggacatgac caacggggca gccttggcag ccaacagcaa 1260

tggcatcgcc ggcagcatgc agccagagga ggaggcagct cgggcggctg gtgcagccat 1320

tgcaggccaa gcctctttgc ctgtgttacc tggggtggac cgcttgccca tggtggctgg 1380

acccctatcc ccccaactgc tgacttcccc attccccagt gtggcatcca gtgcccctcc 1440

cctgactggc aagcgaggcc ggggccgccc aaggaaggcc aacctgctgg actcaatgtt 1500

tgggtcccca gggggcctga gggaggcagg catccttcca tgcggtctat gtggtaaggt 1560

gttcactgat gccaaccggc tccggcagca cgaggcccag cacggtgtca ccagcctcca 1620
gctgggctac atcgaccttc ctcctccgag gctgggtgag aatgggctac ccatctctga 1680
agaccccgac ggcccccgaa agaggagccg gaccaggaag caggtggctt gtgagatctg 1740
cggcaagatc ttccgtgatg tgtatcatct taaccggcac aagctgtccc actctgggga 1800
gaagccctac tcctgccctg tgtgtgggtt gcggttcaag agaaaagacc gcatgtccta 1860
ccatgtgcgg tcccatgatg ggtccgtggg caagccttac atctgccaga gctgtgggaa 1920
aggcttctcc aggcctgatc acttgaacgg acatatcaag caggtgcaca cttctgagcg 1980
gcctcacaag tgtcaggtgt gggttgggag cagcagcggc ctgccgcccc tggaacctct 2040
tcctagcgac ctgccatcat gggactttgc ccagcctgct ttgtggaggt cgtcccattc 2100
ggttcctgac accgcctttt ccctttctct aaaaaaatca ttcccagccc ttgaaaacct 2160
gggcccagca cactccagca acactctctt ctgcccagcc ccgccgggat atctgaggca 2220
gggctggacc accccagagg gcagcagggc ctttacccag tggcctgttg gctagcctgg 2280
gcctccctgg agagggttga cagtggaagg gaacaggagg ggcatttggc ctgagacccc 2340
tgcttttggg agaggctagc agggtggttc ctgcccagca tgcccagctc ctccctgggt 2400
gactcggagt ctttcccatg tcagagcccc caaatggggg tagcaaggag cacctttctg 2460
gaacccccta tagcatccaa gtttctttct gggctctctt gccttttccc ccctttcaca 2520
gatggcaccc ctgggcatct gtccttgcct aggtgatttt ggaggttggt gccttcctgg 2580
gaactagcca ccagcttatc tgcttccctt cccctggcat cacttcccat aggcctgggg 2640
tttctagact ggggcctggc cacccctttc cccactccac gagtgagtcg gcctccagag 2700
aagactggca caattccaac tagagtcaac ccatgctgcc ctctgccctt cccactcaga 2760
tctagatcct gctttcattt ctggctagtg aagtagactt ttgtgttttt gaggtttatt 2820
agcaggtctg ctcaggaacc aaactaatga gtagctttat attgggccac cccaatatat 2880
ggctttgggg gctgaaaaag cagatgtaga ccccctccct cggatcctta ttggtgtgcc 2940
ctttagcact ccgcagactc tgcggggtga acaggagtga tgataaaatt tttcattcta 3000
aaaaaaaaaa aaaaaaaaaa a 3021

<210> 140
<211> 3747
<212> DNA
<213> Homo sapiens

<400> 140
gggcctactc tgccgccgcc gccgcccgcc cgctccagcc gccgccgccg ccgccaccgc 60
cctccaggct ccgggacccg gcccgcgcca ccgcccccgt gcgcgccccg ccgccgccgc 120
cttcgccttc gccttttgtt tcctccgctc cggcgccccc gccccggctc gcgctttgca 180
ggggacgcag cgcgcgcccc cagcgggccc gggaaaagcc gcggcgcgcg cgcgcgcctg 240
cgcggcggac ccctccttct cctccccgcg tgcgcgtgcc cttcttggct gcgcgccggc 300
gccgcctggc gggcgggagg ggaggtggca ggcgcgtttg caggaggggc gcacctcttc 360

gctcgcgcac ccccccggaa ggtagaccgg gaaggggagg cgggcgggcg gagaggagag 420

agtggcgcgc agtccagcga gggcgggggt tggctatgtg gggggtggtg caccccgcag 480

tctagacagt ctgatccggg ctgggggcgt gtacactcgg cgcacctgcg agactacaga 540

gcctcgggcc ggcacgtgtg gggagtgtgg acacgtctgc tgcgccccgc ttctcgctgc 600

tgaggggaag ggagggggcg ggcaggtgca gcggccgggc tagtgggagg gggcggcggc 660

catggagcgg gtgaacgacg cttcgtgcgg cccgtctggc tgctacacat accaggtgag 720

cagacacagc acggagatgc tgcacaacct gaaccagcag cgcaaaaacg gcgggcgctt 780

ctgcgacgtg ctcttgcggg taggcgacga gagcttccca gcgcaccgcg ccgtgctggc 840

cgcctgcagc gagtactttg agtcggtgtt cagcgcccag ttgggcgacg gcggagctgc 900

ggacgggggt ccggctgatg tagggggcgc gacggcagca ccaggcggcg gggccggggg 960

cagccgggag ctggagatgc acactatcag ctccaaggta tttggggaca ttctggactt 1020

cgcctacact tcccgcatcg tggtgcgctt ggagagcttt cccgaactca tgacggccgc 1080

caagttcctg ctgatgaggt cggttatcga gatctgccag gaagtcatca aacagtccaa 1140

cgtacagatc ctggtacccc ctgcccgcgc cgatataatg ctctttcgcc cccctgggac 1200

ctcggacttg ggcttccctt tggacatgac caacggggca gccttggcag ccaacagcaa 1260

tggcatcgcc ggcagcatgc agccagagga ggaggcagct cgggcggctg gtgcagccat 1320

tgcaggccaa gcctctttgc ctgtgttacc tggggtggac cgcttgccca tggtggctgg 1380

acccctatcc ccccaactgc tgacttcccc attccccagt gtggcatcca gtgcccctcc 1440

cctgactggc aagcgaggcc ggggccgccc aaggaaggcc aacctgctgg actcaatgtt 1500

tgggtcccca gggggcctga gggaggcagg catccttcca tgcggtctat gtggtaaggt 1560

gttcactgat gccaaccggc tccggcagca cgaggcccag cacggtgtca ccagcctcca 1620

gctgggctac atcgaccttc ctcctccgag gctgggtgag aatgggctac ccatctctga 1680

agaccccgac ggcccccgaa agaggagccg gaccaggaag caggtggctt gtgagatctg 1740

cggcaagatc ttccgtgatg tgtatcatct taaccggcac aagctgtccc actctgggga 1800

gaagccctac tcctgccctg tgtgtgggtt gcggttcaag agaaaagacc gcatgtccta 1860

ccatgtgcgg tcccatgatg ggtccgtggg caagccttac atctgccaga gctgtgggaa 1920

aggcttctcc aggcctgatc acttgaacgg acatatcaag caggtgcaca cttctgagcg 1980

gcctcacaag tgtcagacct gcaatgcttc ttttgccacc cgagaccgtc tgcgctccca 2040

cctggcctgt catgaagaca aggtgccctg ccaggtgtgt gggaagtact tgcgggcagc 2100

atacatggca gaccacctga agaagcacag cgaggggccc agcaacttct gcagtatctg 2160

taaccgaggt ctccaggcac caggagccca tcctgaatgg gggagcagcg ttccactgcg 2220

ccaggaccta tggcaacaaa gaaggccaga aatgctcaca tcaggatccg attgagagct 2280

ctgactccta tggtgacctc tcagatgcca gcgacctgaa gacgccagag aagcagagtg 2340

ccaatggctc tttctcctgc gacatggcag tccccaaaaa caaaatggag tctgatgggg 2400

agaagaagta cccatgccct gaatgtggga gcttcttccg ctctaagtcc tacttgaaca 2460

aacacatcca gaaggtgcat gtccgggctc tcgggggccc cctgggggac ctgggccctg 2520

cccttggctc acctttctct cctcagcaga acatgtctct cctcgagtcc tttgggtttc 2580

agattgttca gtcggcattt gcgtcatctt tagtagatcc tgaggttgac cagcagccca 2640

tggggcctga agggaaatga ggcagctgct gtgtccccac ggaaacaacc atctggggac 2700

tgctgggaaa tgctgtgaat gcggagggaa gtgatgtttg ggttctgtag ctgagagatt 2760

tttattcatt tttaactgcc ccccaacccc actccaactc cttctccacc acccattctc 2820

ccaatggtct ttagaaatag attttcatct gatattctgc agaaatatca atgagacttg 2880

gtatgggaca ggggcagaaa acactacata ggcctccaag gcaaaaccag tcccagtttc 2940

tttaatggga agaagctgga attcctggtg ctcaattctt agtgacccca atcctatacc 3000

caaatctatg atattctggg acctcagtga ttttggtccc ctcccacttc tctagttcgt 3060

catcctccct tcccatatcc ttcaaaagaa ccacactagg gtctccacct acttatacaa 3120

tgcggatgcc caactgtttt taaggaagcc agaagcatcc catggaccat ggggtgagtg 3180

tcctccaaga gccccctgag ctcagccctc tgcctggagg gctccagacc tttctgagcc 3240

ctgcttggag gcgagcattt tcactgctag gacaagctca gctgttgagg acacccccac 3300

cccaaatttc agttcttacg tgattttaac cattcaacat gctgttgggt tttaattctc 3360

taattattat tattattgtt attatttttt aggaccagtt gtagtgaatt gctactgaaa 3420

gctatcccag gtgatacaga gctctttgta aaccgcagtc acacattagg gttagtatta 3480

aactttgttt agatgtacca taattaactt ggctagttga ttgtttgaag tctatggaag 3540

aaatagtttt atgcaaaatt tttaaaaatg ccagtctggt cagggaagta gggggtttca 3600

atgctgttgg gaaccaggaa ggtgggacag ccggcaggta gggacattgt gtacctcagt 3660

tgtgtcacat gtgagcaagc ccaggttgac cttgtgatgt gaattgatct gatcagactg 3720

tattaaaaat gttagtacat tactcta 3747

<210> 141
<211> 4348
<212> DNA
<213> Homo sapiens

<400> 141
gggaggccca gggagaacgg ggaagggaca tttagtttga gacggtgctg agataggatc 60

atgaaggaag aggtgaaggg aattcctgta agagtggcgc tgcgttgtcg ccctctggtc 120

cccaaagaga ttagcgaggg ctgccagatg tgcctttcct tcgtgcccgg agagcctcag 180

gtggtggttg gtacagataa atccttcacc tacgattttg tatttgatcc ctctactgaa 240

caggaagaag tcttcaatac agcagtagcg ccactcataa aaggtgtatt taaaggatat 300

aatgcaacgg tcctggccta tgggcagact ggctctggaa aaacctattc aatgggaggt 360

gcatatactg cagagcaaga gaatgaacca acagttgggg ttattcctag ggtaatacaa 420

ctgctcttca aagaaattga taaaaagagt gactttgaat ttactctgaa agtgtcttac 480

ttagagattt acaatgaaga aattttggat cttctatgtc catctcgtga gaaagctcaa 540

ataaatatac gagaggatcc taaggaaggc ataaagattg tgggactcac tgagaagact 600

gttttggttg ccttggatac tgtttcctgt ttggaacagg gcaacaactc taggactgtg 660
gcctccacgg ctatgaactc ccagtcgtcc cgatctcatg ccatctttac aatctcctta 720
gagcaaggaa agaaaagtga caagaatagc agctttcgct ccaagctgca tcttgtagac 780
ctcgctggat cagaaagaca gaagaaaacc aaggctgaag gggatcgtct aaaagagggt 840
attaatatta accgaggcct cctatgcttg ggaaatgtaa tcagtgctct tggagatgac 900
aaaaagggtg gctttgcgcc ctacagagat tccaagttga ctcgactgct tcaagattct 960
ctaggaggta atagccatac tcttatgata gcctgtgtga gtcctgctga ctccaatcta 1020
gaggaaacat taaataccct tcgctatgct gacagagcaa gaaaaatcaa gaacaaacct 1080
attgttaata ttgatcccca gacagctgaa cttaatcatc taaagcaaca ggtacaacag 1140
ctacaagtct tgttgctaca ggcccatgga ggtaccctgc ctggatctat aactgtggaa 1200
ccatcagaga atctacaatc cctgatggag aagaatcagt ccctggtaga ggagaatgaa 1260
aaattaagtc gtggtctgag cgaggcagct ggtcagacag cccagatgtt ggagaggatc 1320
atttggacag agcaagcgaa tgaaaaaatg aacgccaagc tagaagagct caggcagcat 1380
gcggcctgca aactggatct tcaaaagcta gtggagactt tggaagacca ggaattgaaa 1440
gaaaatgtag agataatttg taacctgcag caattgatta cccagttatc ggatgaaact 1500
gttgcttgca tggctgcagc cattgatact gcggtggagc aagaagccca agtagaaacc 1560
agtccagaga cgagcaggtc ttctgacgct tttaccactc agcatgctct ccgtcaagcg 1620
cagatgtcta aggagctggt tgagttgaat aaagcgcttg cactgaaaga ggccctggct 1680
aggaagatga ctcagaatga cagccaactg cagcctattc agtaccaata ccaggataac 1740
ataaaagagc cagaattaga agtcatcaat ctgcaaaagg aaaaggaaga attggttctt 1800
gaacttcaga cagcaaagaa ggatgccaac caagccaagt tgagtgagcg ccgccgcaaa 1860
cgtctccagg agctggaggg tcaaattgct gatctgaaga agaaactgaa tgagcagtcc 1920
aaacttctga aactaaagga atccacagag cgtactgtct ccaaactgaa ccaggagata 1980
cggatgatga aaaaccagcg ggtacagtta atgcgtcaaa tgaaagaaga tgctgagaag 2040
tttagacagt ggaagcagaa aagagacaaa gaagtaatac agttaaaaga acgagaccgt 2100
aagaggcaat atgagctgct gaaacttgaa agaaacttcc agaaacaatc caatgtgctc 2160
agacgtaaaa cggaggaggc agcagctgcc aacaagcgtc tcaaggatgc tctccagaaa 2220
caacgggagg ttgcagataa gcggaaagag actcagagcc gtggaatgga aggcactgca 2280
gctcgagtga agaattggct tggaaacgaa attgaggtta tggtcagtac tgaggaagcc 2340
aaacgccatc tgaatgacct ccttgaagat agaaagatcc tggctcaaga tgtggctcaa 2400
ctcaaagaaa aaaaggaatc tggggagaat ccacctccta aactccggag gcgtacattc 2460
tcccttactg aagtgcgtgg tcaagtttcg gagtcagaag attctattac aaagcagatt 2520
gaaagcctag agactgaaat ggaattcagg agtgctcaga ttgctgacct acagcagaag 2580
ctgctggatg cagaaagtga agacagacca aaacaacgct gggagaatat tgccaccatt 2640
ctggaagcca agtgtgccct gaaatatttg attggagagc tggtctcctc caaaatacag 2700

gtcagcaaac ttgaaagcag cctgaaacag agcaagacca gctgtgctga catgcagaag 2760

atgctgtttg aggaacgaaa tcattttgcc gagatagaga cagagttaca agctgagctg 2820

gtcagaatgg agcaacagca ccaagagaag gtgctgtacc ttctcagcca gctgcagcaa 2880

agccaaatgg cagagaagca gttagaggaa tcagtcagtg aaaaggaaca gcagctgctg 2940

agcacactga agtgtcagga tgaagaactt gagaaaatgc gagaagtgtg tgagcaaaat 3000

cagcagcttc tccgagagaa tgaaatcatc aagcagaaac tgaccctcct ccaggtagcc 3060

agcagacaga aacatcttcc taaggatacc cttctatctc cagactcttc ttttgaatat 3120

gtccagccta agccaaaacc ttctcgtgtt aaagaaaagt tcctggagca aagcatggac 3180

atcgaggatc taaaatattg ttcagagcat tctgtgaatg agcatgagga tggtgatggt 3240

gatgatgatg agggggatga cgaggaatgg aagccaacaa aattagttaa tgtgtccagg 3300

aagaacatcc aagggtgttc ctgcaagggc tggtgtggaa acaagcaatg tgggtgcagg 3360

aagcaaaagt cagactgtgg tgtggactgt tgctgtgacc ccacaaagtg tcggaaccgc 3420

cagcaaggca aggatagctt gggcactgtt gaacggaccc aggattcaga aagctccttc 3480

aaactggagg atcctaccga ggtgacccca ggattgagct tctttaatcc cgtctgtgcc 3540

acccccaata gcaagatcct gaaagagatg tgcgatgtgg agcaggtgct gtcaaagaag 3600

actcccccag ctccctcccc ttttgacctc ccagagttga aacatgtagc aacagaatac 3660

caagaaaaca aggctccagg gaagaaaaag aaacgggctc tggccagcaa caccagcttc 3720

ttctctggct gctcccctat cgaagaagag gcccactgaa gttggagtca tcatctctac 3780

ccccagtctg gcttgggaga tgctttcagg ttgcagccag aaggggtttt ttaaatgact 3840

tctctggatt tcaggtttct tgctgttgaa aaaaggaaca aagcgttact gaaaagaagg 3900

taacctttgt tggatgtggg ccttagcctc caggtccaga ctactactct atgttctcca 3960

gaagggtgct aagtcaccta ctgaagagag aaccaactga ctttcctatt gactcatcag 4020

gaaccagtcc tcagtctggt caagttgttt cttatttgtg agcagttcag gctatctcct 4080

gatggggatg aggccaaggc tttcttatct tttggttgtc tctgcttaat ggaggagcct 4140

ggcctaggat ggaggcctgg cttagatctt tcattccacc tcaggaatga ggttgtgatc 4200

tttcctgtcc tgaccctctc tgaattatgt ttcaatagta ctcttgattg tctgccatgt 4260

tgttgaagca aatgaattat ttttaaatgt taagtaagta aataaacctt agcccgtctt 4320

tttttttttt tttttttttt tttttttt 4348

<210> 142
<211> 1130
<212> DNA
<213> Homo sapiens

<400> 142
gcacgagggt gctgactggc ttgcgtctct gccactttct ttcagcttag tggcgcgcgg 60

cgcgcgacga ccgccgggag cgtgtgcagc ggcggcggcg gaagtggccg gcgagcccgg 120

tccccgccgg caccatgctt cccttgtcac tgctgaagac ggctcagaat caccccatgt 180

tggtggagct gaaaaatggg gagacgtaca atggacacct ggtgagctgc gacaactgga    240

tgaacattaa cctgcgagaa gtcatctgca cgtccaggga cggggacaag ttctggcgga    300

tgcccgagtg ctacatccgc ggcagcacca tcaagtacct gcgcatcccc gacgagatca    360

tcgacatggt caaggaggag gtggtggcca agggccgcgg ccgcggaggc ttgcagcagc    420

agaagcagca gaaaggccgc ggcatgggcg gcgctggccg aggtgtgttt ggtggccggg    480

gccgaggtgg gatcccgggc acaggcagag gccagccaga gaagaagcct ggcagacagg    540

cgggcaaaca gtgagcgccc acccagaccg gctgctgcgc cccctcctgc cagggtggcg    600

attccgctcc acagtctcgg acggatctgc tcagaaagga agaggcaggc gccaggggga    660

acccccttcg tgttttgtga ccctcccttt taggtgaagc ccctttttct tgctaaaacc    720

ggcaattctc cggttagaaa tgttacttgg tgttttttgg ttttgtgaaa cggccgtccc    780

aaagctggct ggattcctag aagagtctgt gttgaaggca tctttcaagc cctcgctctg    840

gttctcaggg cagcattttc caggcgggtt tgttttgcat ttcttggagc ctctccgagc    900

agcaaccaga cgggagattt ttattttaag ctgttcatgc tgggactgac agcctgcagg    960

gtttccttgg gcgcggcccc aaaattgcct tcaaaacaaa cccgggacgg ttgaaagcct    1020

tcgaaccgtg caggggatgc ctcgggccct ggcccttcgc ttcctctctt gtgttatgga    1080

aataaaaaca aataaaacta cacactgcac aaaaaaaaaa aaaaaaaaaa    1130

<210>  143
<211>  2082
<212>  DNA
<213>  Homo sapiens

<400>  143
gacaggtctg tgaagcaggc aggttgctca gctgcccccg gagcggttcc tccacctgag    60

gcagactcca cgtcggctgg catgagccgg cgcccctgca gctgcgccct acggccaccc    120

cgctgctcct gcagcgccag ccccagcgca gtgacagccg ccgggcgccc tcgaccctcg    180

gatagttgta aagaagaaag ttctaccctt tctgtcaaaa tgaagtgtga ttttaattgt    240

aaccatgttc attccggact taaactggta aaacctgatg acattggaag actagtttcc    300

tacacccctg catatttgga aggttcctgt aaagactgca ttaaagacta tgaaaggctg    360

tcatgtattg ggtcaccgat tgtgagccct aggattgtac aacttgaaac tgaaagcaag    420

cgcttgcata acaaggaaaa tcaacatgtg caacagacac ttaatagtac aaatgaaata    480

gaagcactag agaccagtag actttatgaa gacagtggct attcctcatt ttctctacaa    540

agtggcctca gtgaacatga agaaggtagc ctcctggagg agaatttcgg tgacagtcta    600

caatcctgcc tgctacaaat acaaagccca gaccaatatc ccaacaaaaa cttgctgcca    660

gttcttcatt ttgaaaaagt ggtttgttca acattaaaaa agaatgcaaa acgaaatcct    720

aaagtagatc gggagatgct gaaggaaatt atagccagag gaaattttag actgcagaat    780

ataattggca gaaaaatggg cctagaatgt gtagatattc tcagcgaact ctttcgaagg    840

ggactcagac atgtcttagc aactatttta gcacaactca gtgacatgga cttaatcaat    900

gtgtctaaag tgagcacaac ttggaagaag atcctagaag atgataaggg ggcattccag    960

ttgtacagta aagcaataca aagagttacc gaaaacaaca ataaattttc acctcatgct 1020

tcaaccagag aatatgttat gttcagaacc ccactggctt ctgttcagaa atcagcagcc 1080

cagacttctc tcaaaaaaga tgctcaaacc aagttatcca atcaaggtga tcagaaaggt 1140

tctacttata gtcgacacaa tgaattctct gaggttgcca agacattgaa aaagaacgaa 1200

agcctcaaag cctgtattcg ctgtaattca cctgcaaaat atgattgcta tttacaacgg 1260

gcaacctgca aacgagaagg ctgtggattt gattattgta cgaagtgtct ctgtaattat 1320

catactacta aagactgttc agatggcaag ctcctcaaag ccagttgtaa aataggtccc 1380

ctgcctggta caaagaaaag caaaaagaat ttacgaagat tgtgatctct tattaaatca 1440

attgttactg atcatgaatg ttagttagaa aatgttaggt tttaacttaa aaaaaattgt 1500

attgtgattt tcaattttat gttgaaatcg gtgtagtatc ctgaggtttt tttcccccca 1560

gaagataaag aggatagaca acctcttaaa atatttttac aatttaatga gaaaaagttt 1620

aaaattctca atacaaatca aacaatttaa atattttaag aaaaaaggaa aagtagatag 1680

tgatactgag ggtaaaaaaa aaattgattc aattttatgg taaaggaaac ccatgcaatt 1740

ttacctagac agtcttaaat atgtctggtt ttccatctgt tagcatttca gacattttat 1800

gttcctctta ctcaattgat accaacagaa atatcaactt ctggagtcta ttaaatgtgt 1860

tgtcaccttt ctaaagcttt ttttcattgt gtgtatttcc caagaaagta tcctttgtaa 1920

aaacttgctt gttttcctta tttctgaaat ctgttttaat atttttgtat acatgtaaat 1980

atttctgtat tttttatatg tcaaagaata tgtctcttgt atgtacatat aaaaataaat 2040

tttgctcaat aaaattgtaa gcttaaaaaa aaaaaaaaaa aa 2082

<210> 144
<211> 963
<212> DNA
<213> Homo sapiens

<400> 144
ggcttccggc accggccgag gtgcgggtcg cctccagaga tcctgtgcct tcaaacccta 60

cgagtccata ctttaaaaca aaatgaagaa agtaaggctt aaggaactag agagtcgcct 120

gcaacaagtg gatggatttg aaaagcccaa gctacttctg gaacagtatc ctaccaggcc 180

gcacattgca gcatgtatgc tctatacaat ccataacact tatgatgaca ttgaaaataa 240

agtcgttgca gatctaggat gtggttgtgg agtacttagc atcggaactg caatgttagg 300

agcagagttg tgtgttggat ttgacataga tgaagacgca ttggaaatat ttaataggaa 360

tgcagaagag tttgagttac aaatattgac atggttcaat gtgctgtgtg cttattatct 420

aacagaatgt ccaagtcatt cgatacagta ttatgaatcc tccctttggg accaaaaata 480

ataaagggac agatatggct ttctaaagac tgctttggaa atggcaagaa cagcagtata 540

ttccttacac aaatcctcaa ctagagaaca tgttcaaaga aagctgcaga atggaaaatc 600

aagatagata ttatagcaga acttcgatat gacctgccag catcatacaa gtttcacaaa 660

aagaaatcag tctgatcatc taaatcaccc gaaaatttca gcaagcatcc aatcttttct 720

tctagagatt tctataaaaa tatacaaagt gatcaaaaag tttcttaaaa gcaacaacag 780

accagaaatg actcacacca ccttatagct atacaagaaa aagactagaa accaaaatgc 840

tattttaaag tagtttcttc cgccggggca gcaagagcga aactccatct caaaaaataa 900

taataataat aaataaataa agtagtttca tagaaaaaaa aaaaaaaaaa aaaaaaaaaa 960

aaa 963

<210> 145
<211> 3237
<212> DNA
<213> Homo sapiens

<400> 145
gcgaagtgaa gggtggccca ggtggggcca ggctgactga atgtatctcc tagctatgga 60

ctaaataata catgggggga aataaacaag tattcatgag ggtgaaaatg tgacccagca 120

ggaaaattac aactattttc aattgacgtt gaataggatg agtcatggaa tttaagtgat 180

ttactgaaga ttatactact ggtagataga agagctaaag aaagatggat actatgatgc 240

tgaatgtgcg gaatctgttt gagcagcttg tgcgccgggt ggagattctc agtgaaggaa 300

atgaagtcca atttatccag ttggcgaagg actttgagga tttccgtaaa aagtggcaga 360

ggactgacca tgagctgggg aaatacaagg atcttttgat gaaagcagag actgagcgaa 420

gtgctctgga tgttaagctg aagcatgcac gtaatcaggt ggatgtagag atcaaacgga 480

gacagagagc tgaggctgac tgcgaaaagc tggaacgaca gattcagctg attcgagaga 540

tgctcatgtg tgacacatct ggcagcattc aactaagcga ggagcaaaaa tcagctctgg 600

cttttctcaa cagaggccaa ccatccagca gcaatgctgg gaacaaaaga ctatcaacca 660

ttgatgaatc tggttccatt ttatcagata tcagctttga caagactgat gaatcactgg 720

attgggactc ttctttggtg aagactttca aactgaagaa gagagaaaag aggcgctcta 780

ctagccgaca gtttgttgat ggtccccctg gacctgtaaa gaaaactcgt tccattggct 840

ctgcagtaga ccaggggaat gaatccatag ttgcaaaaac tacagtgact gttcccaatg 900

atggcgggcc catcgaagct gtgtccacta ttgagactgt gccatattgg accaggagcc 960

gaaggaaaac aggtacttta caaccttgga acagtgactc caccctgaac agcaggcagc 1020

tggagccaag aactgagaca gacagtgtgg gcacgccaca gagtaatgga gggatgcgcc 1080

tgcatgactt tgtttctaag acggttatta aacctgaatc ctgtgttcca tgtggaaagc 1140

ggataaaatt tggcaaatta tctctgaagt gtcgagactg tcgtgtggtc tctcatccag 1200

aatgtcggga ccgctgtccc cttccctgca ttcctaccct gataggaaca cctgtcaaga 1260

ttggagaggg aatgctggca gactttgtgt cccagacttc tccaatgatc ccctccattg 1320

ttgtgcattg tgtaaatgag attgagcaaa gaggtctgac tgagacaggc ctgtatagga 1380

tctctggctg tgaccgcaca gtaaaagagc tgaaagagaa attcctcaga gtgaaaactg 1440

tacccctcct cagcaaagtg gatgatatcc atgctatctg tagccttcta aaagactttc 1500

ttcgaaacct caaagaacct cttctgacct ttcgccttaa cagagccttt atggaagcag 1560

cagaaatcac agatgaagac aacagcatag ctgccatgta ccaagctgtt ggtgaactgc 1620

cccaggccaa cagggacaca ttagctttcc tcatgattca cttgcagaga gtggctcaga 1680

gtccacatac taaaatggat gttgccaatc tggctaaagt ctttggccct acaatagtgg 1740

cccatgctgt gcccaatcca gacccagtga caatgttaca ggacatcaag cgtcaaccca 1800

aggtggttga gcgcctgctt tccttgcctc tggagtattg gagtcagttc atgatggtgg 1860

agcaagagaa cattgacccc ctacatgtca ttgaaaactc aaatgccttt tcaacaccac 1920

agacaccaga tattaaagtg agtttactgg gacctgtgac cactcctgaa catcagcttc 1980

tcaagactcc ttcatctagt tccctgtcac agagagtccg ttccaccctc accaagaaca 2040

ctcctagatt tgggagcaaa agcaagtctg ccactaacct aggacgacaa ggcaactttt 2100

ttgcttctcc aatgctcaag tgaagtcaca tctgcctgtt acttcccagc attgactgac 2160

tataagaaag gacacatctg tactctgctc tgcagcctcc tgtactcatt actactttta 2220

gcattctcca ggcttttact caagtttaat tgtgcatgag ggttttatta aaactatata 2280

tatctcccct tccttctcct caagtcacat aatatcagca ctttgtgctg gtcattgttg 2340

ggagctttta gatgagacat ctttccaggg gtagaagggt tagtatggaa ttggttgtga 2400

ttctttttgg ggaagggggt tattgttcct ttggcttaaa gccaaatgct gctcatagaa 2460

tgatctttct ctagtttcat ttagaactga tttccgtgag acaatgacag aaaccctacc 2520

tatctgataa gattagcttg tctcagggtg ggaagtggga gggcagggca aagaaaggat 2580

tagaccagag gatttaggat gcctccttct aagaaccaga agttctcatt ccccattatg 2640

aactgagcta taatatggag ctttcataaa aatgggatgc attgaggaca gaactagtga 2700

tgggagtatg cgtagctttg atttggatga ttaggtcttt aatagtgttg agtggcacaa 2760

ccttgtaaat gtgaaagtac aactcgtatt tatctctgat gtgccgctgg ctgaactttg 2820

ggttcatttg gggtcaaagc cagtttttct tttaaaattg aattcattct gatgcttggc 2880

ccccataccc ccaaccttgt ccagtggagc ccaacttcta aaggtcaata tatcatcctt 2940

tggcatccca actaacaata aagagtaggc tataagggaa gattgtcaat attttgtggt 3000

aagaaaagct acagtcattt tttctttgca ctttggatgc tgaaattttt cccatggaac 3060

atagccacat ctagatagat gtgagctttt tcttctgtta aaattattct taatgtctgt 3120

aaaaacgatt ttcttctgta gaatgtttga cttcgtattg acccttatct gtaaaacacc 3180

tatttgggat aatatttgga aaaaaagtaa atagcttttt caaaatgaaa aaaaaaa 3237

<210> 146
<211> 2806
<212> DNA
<213> Homo sapiens

<400> 146
ccgggacccg cccgcccgcg ggagaaatgt tgctgaagtg ctgctgaaag ggccagagat 60

gcaaggattt gggatacatt ttgaaccttt aagctgtctg acattgacct cctttcatta 120

ttaataaaga agaatcagga gcttaggatg tattaacacc aactcattaa tatactaacc 180

ggacaatgtt ctacaaacaa ttctacattg taaaggactg gattggcaca aaataaaata 240

attttatttt attcagctta taatatgact cgatggagga aaatttgata agcatgagag 300

aagaccattc ttttcatgtt cgttacagaa tggaagcttc ttgcctagag ctggccttgg 360

aaggggaacg tctatgtaaa tcaggagact gccgcgctgg cgtgtcattc tttgaagctg 420

cagttcaagt tggaactgaa gacctaaaaa cacttagcgc tatttacagc cagttgggca 480

atgcttattt ctatttgcat gattatgcca aagcattaga atatcaccat catgatttaa 540

cccttgcaag gactattgga gaccagctgg gggaagcgaa agctagtggt aatctgggaa 600

acaccttaaa agttcttggg aattttgacg aagccatagt ttgttgtcag cgacacctag 660

atatttccag agagcttaat gacaaggtgg gagaagcaag agcactttac aatcttggga 720

atgtgtatca tgccaaaggg aaaagttttg gttgccctgg tccccaggat gtaggagaat 780

ttccagaaga agtgagagat gctctgcagg cagccgtgga tttttatgag gaaaacctat 840

cattagtgac tgctttgggt gaccgagcgg cacaaggacg tgcctttgga aatcttggaa 900

acacacatta cctccttggc aacttcaggg atgcagttat agctcatgag cagcgtctcc 960

ttattgcaaa agaatttgga gataaagcag ctgaaagaag agcatatagc aaccttggaa 1020

atgcatatat atttcttggt gaatttgaaa ctgcctcgga atactacaag aagacactac 1080

tgttggcccg acagcttaaa gaccgagctg tagaagcaca gtcttgttac agtcttggaa 1140

atacatatac tttacttcaa gactatgaaa aggccattga ttatcatctg aagcacttag 1200

caattgctca agagctgaat gatagaattg gtgaaggaag agcatgttgg agcttaggaa 1260

atgcatacac agcactagga aatcatgatc aagcaatgca ttttgctgaa aagcacttgg 1320

aaatttcaag agaggttggg gataaaagtg gtgaactaac agcacgactt aatctctcag 1380

accttcaaat ggttcttggt ctgagctaca gcacaaataa ctccataatg tctgaaaata 1440

ctgaaattga tagcagtttg aatggtgtac gccccaagtt gggacgccgg catagtatgg 1500

aaaatatgga acttatgaag ttaacaccag aaaaggtaca gaactggaac agtgaaattc 1560

ttgctaagca aaaacctctt attgccaaac cttctgcaaa gctactcttt gtcaacagac 1620

tgaaggggaa aaaatacaaa acgaattcct ccactaaagt tctccaagat gccagtaatt 1680

ctattgacca ccgaattcca aattctcaga ggaaaatcag tgcagatact attggagatg 1740

aagggttctt tgacttatta agccgatttc aaagcaatag gatggatgat cagagatgtt 1800

gcttacaaga aaagaactgc catacagctt caacaacaac ttcttccact ccccctaaaa 1860

tgatgctaaa aacatcatct gttcctgtgg tatcccccaa cacggatgag tttttagatc 1920

ttcttgccag ctcacagagt cgccgtctgg atgaccagag ggctagtttc agtaatttgc 1980

cagggcttcg tctaacacaa aacagccagt cggtacttag ccacctgatg actaatgaca 2040

acaaagaggc tgatgaagat ttctttgaca tccttgtaaa atgtcaagga tccagattag 2100

atgatcaaag atgtgctcca ccacctgcta ccacaaaggg tccgacagta ccagatgaag 2160

actttttcag ccttatttta cggtcccagg gaaagagaat ggatgaacag agagttcttt 2220

tacaaagaga tcaaaacaga gacactgact ttgggctaaa ggactttttg caaaataatg 2280

ctttgttgga gtttaaaaat tcagggaaaa aatcggcaga ccattagtta ctatggattt 2340

attttttttc ctttcaaaca cggtaaggaa acaatctatt acttttttcc ttaaaaggag 2400

aatttatagc actgtaatac agcttaaaat atttttagaa tgatgtaaat agttaacctt 2460

cagtagtcta ttaaggcatt aatacttctc tggacatgcg cgtttgaggg tggaggggtc 2520

ctgtaaggtg cttcatcgtc tgtgattact gcttgggatg tgttctttgg cagcttgtga 2580

gattacttta cctagtgttt ataaagtagg aagttaagtg aatcatagat tagaatttaa 2640

tactcttatg gaaataattt tttaacatct taattgacaa tggcgttttt tttatacata 2700

accatggatg tagtgggaaa caatgttgtt tggtaaaaat aatgtacttg atcaatgtaa 2760

aaaagtatat aaaatagtct tactaaaaaa aaaaaaaaaa aaaaaa 2806

<210> 147
<211> 2189
<212> DNA
<213> Homo sapiens

<400> 147
aggggcgggc ccggcgcgcg cgggaagtct ctgtagggcg gccggctacc ctcagccgcc 60

gcgcgtcatg gccctttcgg tgcccggcta ctcaccgggc ttccgaaagc cgcccgaggt 120

agtgcggctc cgacggaaaa gggcccggag ccgtggagct gccgcctccc cgccccgtga 180

gctgacggag ccggcggccc gccgagccgc cctggtggcg gggctgcctc ttcgcccttt 240

ccctgctgcg gggggcagag gcggtggcag cggcggcggc ccggccgctg ctcggaggaa 300

ccccttcgcc cgcctggaca accgaccgcg ggtcgccgcg gagccccccg acgggccggc 360

ccgcgagcag ccggaggccc cggtcccgtt tttagattct aatcaagaaa atgatttgct 420

atgggaagag aagtttcctg aaagaacaac tgttactgaa ttacctcaga cttcacatgt 480

atcattctcc gagcctgata ttccgtcctc aaaaagtact gagttacctg tggactggag 540

tattaaaacg cgactccttt tcacctcttc tcaacccttt acctgggcag atcatttgaa 600

agcacaggaa gaagctcaag gtcttgtcca gcattgtagg gcaacagaag ttactttgcc 660

taaaagtata caggatccca aactctcctc tgagctccgt tgtaccttcc agcagagcct 720

tatctattgg ctccaccctg ctttgtcttg gctaccactg ttccctcgta ttggagctga 780

tagaaaaatg gctggaaaga caagtccttg gtcaaatgat gcaaccctgc agcatgtttt 840

aatgagtgac tggtctgtga gctttacttc tctatataat ttgctgaaga caaaactttg 900

cccctatttc tacgtttgta cctatcagtt tactgtcctg ttccgagcag caggattagc 960

tggaagtgac ttaatcacag ctctcatatc tccaacaact cgaggtttaa gagaagctat 1020

gagaaatgaa ggtattgaat tttctctgcc tttaataaaa gaaagtggcc ataagaagga 1080

gacagcatct ggaacaagct tgggatatgg ggaggagcaa gccatcagtg atgaggatga 1140

agaggaaagt ttttcctggc tggaagagat gggtgtgcaa gataaaatta aaaagccaga 1200

catactttct atcaagctgc gtaaagagaa acatgaagta caaatggatc acagacctga 1260

atctgttgtg ttggtaaaag gaatcaacac ctttacattg ctcaattttt tgattaactc 1320

taagagttta gttgctacct caggtccaca ggcaggactt cctccaaccc tcttgtcccc 1380

tgttgctttc cgaggtgcca caatgcaaat gcttaaggca cggagtgtga atgtgaagac 1440

acaagctctt tctggataca gagaccaatt tagtttggag attacaggtc ctatcatgcc 1500

tcattctctg cattcactga ccatgctgct caaatcttca cagagtggat ctttctctgc 1560

agtactgtat ccacacgagc caactgctgt atttaacatc tgcctgcaaa tggacaaagt 1620

acttgatatg gaggttgttc ataaggagct tactaactgt ggtttgcacc ctaacactct 1680

ggagcaactt agtcaaatac cgttacttgg gaaatcatct ttacggaatg tggtgctgag 1740

agactacatt tataattgga gatcctgaac accaaagtaa gcctaaaagg aatctttgag 1800

gaaaaaagcc ttctagcaag gaaattcaag attcttgaag ttgaaggaat atactgaaat 1860

gtttataaac ataactttta ctacagtagc atctaaggtt ttaaatgtgt tttactattt 1920

tcagattaga tttttagata taaagatgaa aaactcagat aactcaaatg agatatctat 1980

atccctacaa cctaattgta tattgagcag aaactgaatt aatatgcttc cttgcaaatg 2040

ggaaatcaca tagcagttac cccatggcat tgtgactaat ggcatcaggg caaaaatatg 2100

ttactttacg atttacctgt tagcaggttc ttgtttcata aaggtagaaa ataaatacag 2160

acaacatcta aaaaaaaaaa aaaaaaaaa 2189

<210> 148
<211> 2010
<212> DNA
<213> Homo sapiens

<400> 148
aggggcgggc ccggcgcgcg cgggaagtct ctgtagggcg gccggctacc ctcagccgcc 60

gcgcgtcatg gccctttcgg tgcccggcta ctcaccgggc ttccgaaagc cgcccgaggt 120

agtgcggctc cgacggaaaa gggcccggag ccgtggagct gccgcctccc cgccccgtga 180

gctgacggag ccggcggccc gccgagccgc cctggtggcg gggctgcctc ttcgcccttt 240

ccctgctgcg gggggcagag gcggtggcag cggcggcggc ccggccgctg ctcggaggaa 300

ccccttcgcc cgcctggaca accgaccgcg ggtcgccgcg gagccccccg acgggccggc 360

ccgcgagcag ccggaggccc cggtcccgtt tttagattct aatcaagaaa atgatttgct 420

atgggaagag aagtttcctg aaagaacaac tgttactgaa ttacctcaga cttcacatgt 480

atcattctcc gagcctgata ttccgtcctc aaaaagtact gagttacctg tggactggag 540

tattaaaacg cgactccttt tcacctcttc tcaacccttt acctgggcag atcatttgaa 600

agcacaggaa gaagctcaag gtcttgtcca gcattgtagg gcaacagaag ttactttgcc 660

taaaagtata caggatccca aactctcctc tgagctccgt tgtaccttcc agcagagcct 720

tatctattgg ctccaccctg ctttgtcttg gctaccactg ttccctcgta ttggagctga 780

tagaaaaatg gctggaaaga caagtccttg gtcaaatgat gcaaccctgc agcatgtttt 840

aatgagtgac tggtattgaa ttttctctgc ctttaataaa agaaagtggc cataagaagg 900

agacagcatc tggaacaagc ttgggatatg gggaggagca agccatcagt gatgaggatg 960

aagaggaaag tttttcctgg ctggaagaga tgggtgtgca agataaaatt aaaaagccag 1020

acatactttc tatcaagctg cgtaaagaga aacatgaagt acaaatggat cacagacctg 1080

aatctgttgt gttggtaaaa ggaatcaaca cctttacatt gctcaatttt ttgattaact 1140

ctaagagttt agttgctacc tcaggtccac aggcaggact tcctccaacc ctcttgtccc 1200

ctgttgcttt ccgaggtgcc acaatgcaaa tgcttaaggc acggagtgtg aatgtgaaga 1260

cacaagctct ttctggatac agagaccaat ttagtttgga gattacaggt cctatcatgc 1320

ctcattctct gcattcactg accatgctgc tcaaatcttc acagagtgga tctttctctg 1380

cagtactgta tccacacgag ccaactgctg tatttaacat ctgcctgcaa atggacaaag 1440

tacttgatat ggaggttgtt cataaggagc ttactaactg tggtttgcac cctaacactc 1500

tggagcaact tagtcaaata ccgttacttg ggaaatcatc tttacggaat gtggtgctga 1560

gagactacat ttataattgg agatcctgaa caccaaagta agcctaaaag gaatctttga 1620

ggaaaaaagc cttctagcaa ggaaattcaa gattcttgaa gttgaaggaa tatactgaaa 1680

tgtttataaa cataactttt actacagtag catctaaggt tttaaatgtg ttttactatt 1740

ttcagattag atttttagat ataaagatga aaaactcaga taactcaaat gagatatcta 1800

tatccctaca acctaattgt atattgagca gaaactgaat taatatgctt ccttgcaaat 1860

gggaaatcac atagcagtta ccccatggca ttgtgactaa tggcatcagg gcaaaaatat 1920

gttactttac gatttacctg ttagcaggtt cttgtttcat aaaggtagaa aataaataca 1980

gacaacatct aaaaaaaaaa aaaaaaaaaa 2010

<210> 149
<211> 1624
<212> DNA
<213> Homo sapiens

<400> 149
aggggcgggc ccggcgcgcg cgggaagtct ctgtagggcg gccggctacc ctcagccgcc 60

gcgcgtcatg gccctttcgg tgcccggcta ctcaccgggc ttccgaaagc cgcccgaggt 120

agtgcggctc cgacggaaaa gggcccggag ccgtggagct gccgcctccc cgccccgtga 180

gctgacggag ccggcggccc gccgagccgc cctggtggcg gggctgcctc ttcgcccttt 240

ccctgctgcg gggggcagag gcggtggcag cggcggcggc ccggccgctg ctcggaggaa 300

ccccttcgcc cgcctggaca accgaccgcg ggtcgccgcg gagccccccg acgggccggc 360

ccgcgagcag ccggaggccc cggtcccgtt tttagattct aatcaagaaa atgatttgct 420

atgggaagag aagtttcctg aaagaacaac tgttactgaa ttacctcaga cttcacatgt 480

atcattctcc gagcctgata ttccgtcctc aaaaagtact gagttacctg tggactggag 540

tattaaaacg cgactccttt tcacctcttc tcaacccttt acctgggcag atcatttgaa 600

agcacaggaa gaagctcaag gtcttgtcca gcattgtagg gcaacagaag ttactttgcc 660

taaaagtata caggatccca aactctcctc tgagctccgt tgtaccttcc agcagagcct 720

tatctattgg ctccaccctg ctttgtcttg gctaccactg ttccctcgta ttggagctga 780

tagaaaaatg gctggaaaga caagtccttg gtcaaatgat gcaaccctgc agcatgtttt 840

aatgagtgac tggcacggag tgtgaatgtg aagacacaag ctctttctgg atacagagac 900

caatttagtt tggagattac aggtcctatc atgcctcatt ctctgcattc actgaccatg 960

ctgctcaaat cttcacagag tggatctttc tctgcagtac tgtatccaca cgagccaact 1020

gctgtattta acatctgcct gcaaatggac aaagtacttg atatggaggt tgttcataag 1080

gagcttacta actgtggttt gcaccctaac actctggagc aacttagtca aataccgtta 1140

cttgggaaat catctttacg gaatgtggtg ctgagagact acatttataa ttggagatcc 1200

tgaacaccaa agtaagccta aaaggaatct ttgaggaaaa aagccttcta gcaaggaaat 1260

tcaagattct tgaagttgaa ggaatatact gaaatgttta taaacataac ttttactaca 1320

gtagcatcta aggttttaaa tgtgttttac tattttcaga ttagattttt agatataaag 1380

atgaaaaact cagataactc aaatgagata tctatatccc tacaacctaa ttgtatattg 1440

agcagaaact gaattaatat gcttccttgc aaatgggaaa tcacatagca gttaccccat 1500

ggcattgtga ctaatggcat cagggcaaaa atatgttact ttacgattta cctgttagca 1560

ggttcttgtt tcataaaggt agaaaataaa tacagacaac atctaaaaaa aaaaaaaaaa 1620

aaaa 1624

<210> 150
<211> 1215
<212> DNA
<213> Homo sapiens

<400> 150
cgtcagttgg tcacgtggtt gttcggagcg ggcgagcgga gttagcaggg ctttactgca 60

gagcgcgccg ggcactccag cgaccgtggg gatcagcgta ggtgagctgt ggccttttgc 120

gaggtgctgc agccatagct acgtgcgttc gctacgagga ttgagcgtct ccacccagta 180

agtgggcaag aggcggcagg aagtgggtac gcaggggcgc aaggcgcaca gcctctagac 240

gactcgcttt ccctccggcc aacctctgaa gccgcgtcct actttgacag ctgcagggcc 300

gcggcctggt cttctgtgct tcaccatcta cataatgaat cccagtatga agcagaaaca 360

agaagaaatc aaagagaata taaagaatag ttctgtccca agaagaactc tgaagatgat 420

tcagccttct gcatctggat ctcttgttgg aagagaaaat gagctgtccg caggcttgtc 480

caaaaggaaa catcggaatg accacttaac atctacaact tccagccctg gggttattgt 540

cccagaatct agtgaaaata aaaatcttgg aggagtcacc caggagtcat ttgatcttat 600

gattaaagaa aatccatcct ctcagtattg gaaggaagtg gcagaaaaac ggagaaaggc 660

gctgtatgaa gcacttaagg aaaatgagaa acttcataaa gaaattgaac aaaaggacaa 720

tgaaattgcc cgcctgaaaa aggagaataa agaactggca gaagtagcag aacatgtaca 780

gtatatggca gagctaatag agagactgaa tggtgaacct ctggataatt ttgaatcact 840

ggataatcag gaatttgatt ctgaagaaga aactgttgag gattctctag tggaagactc 900

agaaattggc acgtgtgctg aaggaactgt atcttcctct acggatgcaa agccatgtat 960

atgaaatgca ttaatatttg actgttgaga attttactgc cgaagtttac ctccactagt 1020

tctttgtagc agagtacata actacataat gccaactctg gaatcaaatt tccttgtttg 1080

aatcctggga ccctattgca ttaaagtaca aatactatgt atttttaatc tatgatggtt 1140

tatgtgaata ggattttctc agttgtcagc catgacttat gtttattact aaataaactt 1200

caaactcctg ttgaa 1215

<210> 151
<211> 1084
<212> DNA
<213> Homo sapiens

<400> 151
tagagcagac aagcccgccc cgccgctcct cccagcagct tctatcccgg aagttgatgc 60

cgagcgcaga tcgcttgcag cttgctagct gtgtgggctg ggaggtctgg tagggctgag 120

cttgcaagag gatcaacatg cctttggcta gagatttact acatccgtcc ttggaagagg 180

aaaagaaaaa acataaaaag aaacgcctag tacaaagtcc aaattcttac tttatggatg 240

taaaatgtcc aggttgctac aagatcacca cggttttcag ccatgctcag acagtggttc 300

tttgtgtagg ttgttcaaca gtgttgtgcc agcctacagg aggaaaggcc agactcacag 360

aagggtgttc atttagaaga aagcaacact aatgattcaa acagcttcct gaattttaat 420

tttgtgttgt ctcacagaaa gccttatcat aaattccata attctaatta atttaccaag 480

ataatgtaat tacatttggt tttgtaaggt atacagcagt aatctcctat tttggtgtca 540

gtttttcaat aaagttttga ttatgggcaa atcccctctt tttctttttt taaaatatat 600

ttgagtatgc catacattta tatatatggt gtatatgaat ttggtttaaa cattttaaaa 660

tttattctga ttagtttgtg tctttttttt tttttttgag agagagagtc ctgctctgtc 720

actcaagctg gagtgcagtg gtgcgatctc ggctcactgc aacctccgcc tcccaggtcc 780

aagcaattct cttgccttgt cctcccaagt agctgggatt ataggcacac accaccatgc 840

ctggctaatt tgtgtctcat tttcaagagt agaaacccta aatattttat tttcattcct 900

tttccaaatt gctatgaatg ggattaaagg attacagatg taaagtctat tatttgtgaa 960

ttctaaatgt agttctgctg ttgtacctgt ggaaacatct taaagaagta catattttgc 1020

acgtcctgca cgtgtacccc agaacttaaa ctataattaa aaagaatagt ttcaaaaaaa 1080

taca 1084

<210> 152
<211> 2288
<212> DNA
<213> Homo sapiens

<400> 152
   aaagttaaga gtggcgccag ggatttgaac cgcgctgacg aagtttggtg atccatcttc 60
   cgagtatcgc cgggatttcg aatcgcgatg atcatcccct ctctagagga gctggactcc 120
   ctcaagtaca gtgacctgca gaacttagcc aagagtctgg gtctccgggc caacctgagg 180
   gcaaccaagt tgttaaaagc cttgaaaggc tacattaaac atgaggcaag aaaaggaaat 240
   gagaatcagg atgaaagtca aacttctgca tcctcttgtg atgagactga gatacagatc 300
   agcaaccagg aagaagctga gagacagcca cttggccatg tcaccaaaac aaggagaagg 360
   tgcaagactg tccgtgtgga ccctgactca cagcagaatc attcagagat aaaaataagt 420
   aatcccactg aattccagaa tcatgaaaag caggaaagcc aggatctcag agctactgca 480
   aaagttcctt ctccaccaga cgagcaccaa gaagctgaga atgctgtttc ctcaggtaac 540
   agagattcaa aggtaccttc agaaggaaag aaatctctct acacagatga gtcatccaaa 600
   cctggaaaaa ataaaagaac tgcaatcact actccaaact ttaagaagct tcatgaagct 660
   cattttaagg aaatggagtc cattgatcaa tatattgaga gaaaaaagaa acattttgaa 720
   gaacacaatt ccatgaatga actgaagcag cagcccatca ataagggagg ggtcaggact 780
   ccagtacctc caagaggaag actctctgtg gcttctactc ccatcagcca acgacgctcg 840
   caaggccggt cttgtggccc tgcaagtcag agtaccttgg gtctgaaggg gtcactcaag 900
   cgctctgcta tctctgcagc taaaacgggt gtcaggtttt cagctgctac taaagataat 960
   gagcataagc gttcactgac caagactcca gccagaaagt ctgcacatgt gaccgtgtct 1020
   gggggcaccc caaaaggcga ggctgtgctt gggacacaca aattaaagac catcacgggg 1080
   aattctgctg ctgttattac cccattcaag ttgacaactg aggcaacgca gactccagtc 1140
   tccaataaga aaccagtgtt tgatcttaaa gcaagtttgt ctcgtcccct caactatgaa 1200
   ccacacaaag gaaagctaaa accatggggg caatctaaag aaaataatta tctaaatcaa 1260
   catgtcaaca gaattaactt ctacaagaaa acttacaaac aaccccatct ccagacaaag 1320
   gaagagcaac ggaagaaacg cgagcaagaa cgaaaggaga agaaagcaaa ggttttggga 1380
   atgcgaaggg gcctcatttt ggctgaagat taataatttt ttaacatctt gtaaatattc 1440
   ctgtattctc aacttttttc cttttgtaaa tttttttttt ttgctgtcat ccccacttta 1500
   gtcacgagat ctttttctgc taactgttca tagtctgtgt agtgtccatg ggttcttcat 1560
   gtgctatgat ctctgaaaag acgttatcac cttaaagctc aaattctttg ggatggtttt 1620
   tacttaagtc cattaacaat tcaggtttct aacgagaccc atcctaaaat tctgtttcta 1680
   gatttttaat gtcaagttcc caagttcccc ctgctggttc taatattaac agaactgcag 1740
   tcttctgcta gccaatagca tttacctgat ggcagctagt tatgcaagct tcaggagaat 1800
   ttgaacaata acaagaatag ggtaagctgg gatagaaagg ccacctcttc actctctata 1860
   gaatatagta acctttatga aacggggcca tatagtttgg ttatgacatc aatattttac 1920
   ctaggtgaaa ttgtttaggc ttatgtacct tcgttcaaat atcctcatgt aattgccatc 1980
   tgtcactcac tatattcaca aaaataaaac tctacaactc attctaacat tgcttactta 2040
   aaagctacat agccctatcg aaatgcgagg attaatgctt taatgctttt agagacaggg 2100
   tctcactgtg ttgcccaggc tggtctcaaa ctccaccaaa tgtacttctt attcatttta 2160
   tggaaaagac taggctttgc ttagtatcat gtccatgttt ccttcacctc agtggagctt 2220
   ctgagtttta tactgctcaa gatcgtcata aataaaattt tttctcattg tcataaaaaa 2280
   aaaaaaaa 2288

<210> 153
<211> 2285
<212> DNA
<213> Homo sapiens

<400> 153
aaagttaaga gtggcgccag ggatttgaac cgcgctgacg aagtttggtg atccatcttc 60
cgagtatcgc cgggatttcg aatcgcgatg atcatcccct ctctagagga gctggactcc 120
ctcaagtaca gtgacctgca gaacttagcc aagagtctgg gtctccgggc caacctgagg 180
gcaaccaagt tgttaaaagc cttgaaaggc tacattaaac atgaggcaag aaaaggaaat 240
gagaatcagg atgaaagtca aacttctgca tcctcttgtg atgagactga gatacagatc 300
agcaaccagg aagaagctga gagacagcca cttggccatg tcaccaaaac aaggagaagg 360
tgcaagactg tccgtgtgga ccctgactca cagcagaatc attcagagat aaaaataagt 420
aatcccactg aattccagaa tcatgaaaag caggaaagcc aggatctcag agctactgca 480
aaagttcctt ctccaccaga cgagcaccaa gaagctgaga atgctgtttc ctcaggtaac 540
agagattcaa aggtaccttc agaaggaaag aaatctctct acacagatga gtcatccaaa 600
cctggaaaaa ataaaagaac tgcaatcact actccaaact ttaagaagct tcatgaagct 660
cattttaagg aaatggagtc cattgatcaa tatattgaga gaaaaaagaa acattttgaa 720
gaacacaatt ccatgaatga actgaagcag cccatcaata agggaggggt caggactcca 780
gtacctccaa gaggaagact ctctgtggct tctactccca tcagccaacg acgctcgcaa 840
ggccggtctt gtggccctgc aagtcagagt accttgggtc tgaaggggtc actcaagcgc 900
tctgctatct ctgcagctaa aacgggtgtc aggttttcag ctgctactaa agataatgag 960
cataagcgtt cactgaccaa gactccagcc agaaagtctg cacatgtgac cgtgtctggg 1020
ggcaccccaa aaggcgaggc tgtgcttggg acacacaaat taaagaccat cacggggaat 1080
tctgctgctg ttattacccc attcaagttg acaactgagg caacgcagac tccagtctcc 1140
aataagaaac cagtgtttga tcttaaagca agtttgtctc gtcccctcaa ctatgaacca 1200
cacaaaggaa agctaaaacc atgggggcaa tctaaagaaa ataattatct aaatcaacat 1260
gtcaacagaa ttaacttcta caagaaaact tacaaacaac cccatctcca gacaaaggaa 1320
gagcaacgga agaaacgcga gcaagaacga aaggagaaga aagcaaaggt tttgggaatg 1380
cgaaggggcc tcattttggc tgaagattaa taatttttta acatcttgta aatattcctg 1440
tattctcaac ttttttcctt ttgtaaattt tttttttttg ctgtcatccc cactttagtc 1500
acgagatctt tttctgctaa ctgttcatag tctgtgtagt gtccatgggt tcttcatgtg 1560
ctatgatctc tgaaaagacg ttatcacctt aaagctcaaa ttctttggga tggtttttac 1620
ttaagtccat taacaattca ggtttctaac gagacccatc ctaaaattct gtttctagat 1680
ttttaatgtc aagttcccaa gttccccctg ctggttctaa tattaacaga actgcagtct 1740
tctgctagcc aatagcattt acctgatggc agctagttat gcaagcttca ggagaatttg 1800
aacaataaca agaatagggt aagctgggat agaaaggcca cctcttcact ctctatagaa 1860
tatagtaacc tttatgaaac ggggccatat agtttggtta tgacatcaat attttaccta 1920
ggtgaaattg tttaggctta tgtaccttcg ttcaaatatc ctcatgtaat tgccatctgt 1980
cactcactat attcacaaaa ataaaactct acaactcatt ctaacattgc ttacttaaaa 2040
gctacatagc cctatcgaaa tgcgaggatt aatgctttaa tgcttttaga gacagggtct 2100

cactgtgttg cccaggctgg tctcaaactc caccaaatgt acttcttatt cattttatgg 2160

aaaagactag gctttgctta gtatcatgtc catgtttcct tcacctcagt ggagcttctg 2220

agttttatac tgctcaagat cgtcataaat aaaatttttt ctcattgtca taaaaaaaaa 2280

aaaaa 2285

<210> 154
<211> 4221
<212> DNA
<213> Homo sapiens

<400> 154
cgataacgat ttgtgttgtg agaggcgcaa gctgcgattt ctgctgaact tggaggcatt 60

tctacgactt ttctctcagc tgaggctttt cctccgaccc tgatgctctt caattcggtg 120

ctccgccagc cccagcttgg cgtcctgaga aatggatggt cttcacaata ccctcttcaa 180

tcccttctga ctggttatca gtgcagtggt aatgatgaac acacttctta tggagaaaca 240

ggagtcccag ttcctccttt tggatgtacc ttctcttctg ctcccaatat ggaacatgta 300

ctagcagttg ccaatgaaga aggctttgtt cgattgtata acacagaatc acaaagtttc 360

agaaagaagt gcttcaaaga atggatggct cactggaatg ccgtctttga cctggcctgg 420

gttcctggtg aacttaaact tgttacagca gcaggtgatc aaacagccaa attttgggac 480

gtaaaagctg gtgagctgat tggaacatgc aaaggtcatc aatgcagcct caagtcagtt 540

gccttttcta agtttgagaa agctgtattc tgtacgggtg gaagagatgg caacattatg 600

gtctgggata ccaggtgcaa caaaaaagat gggttttata ggcaagtgaa tcaaatcagt 660

ggagctcaca atacctcaga caagcaaacc ccttcaaaac ccaagaagaa acagaattca 720

aaaggacttg ctccttctgt ggatttccag caaagtgtta ctgtggtcct ctttcaagac 780

gagaatacct tagtctcagc aggagctgtg gatgggataa tcaaagtatg ggatttacgt 840

aagaattata ctgcttatcg acaagaaccc atagcatcca agtctttcct gtacccaggt 900

agcagcactc gaaaacttgg atattcaagt ctgattttgg attccactgg ctctacttta 960

tttgctaatt gcacagacga taacatctac atgtttaata tgactgggtt gaagacttct 1020

ccagtggcta ttttcaatgg acaccagaac tctacctttt atgtaaaatc cagccttagt 1080

ccagatgacc agtttttagt cagtggctca agtgatgaag ctgcctacat atggaaggtc 1140

tccacaccct ggcaacctcc tactgtgctc ctgggtcatt ctcaagaggt cacgtctgtg 1200

tgctggtgtc catctgactt cacaaagatt gctacctgtt ctgatgacaa tacactaaaa 1260

atctggcgct tgaatagagg cttagaggag aaaccaggag gtgataaact ttccacggtg 1320

ggttgggcct ctcagaagaa aaaagagtca agacctggcc tagtaacagt aacgagtagc 1380

cagagtactc ctgccaaagc ccccagggta aagtgcaatc catccaattc ttccccgtca 1440

tccgcagctt gtgccccaag ctgtgctgga gacctccctc ttccttcaaa tactcctacg 1500

ttctctatta aaacctctcc tgccaaggcc cggtctccca tcaacagaag aggctctgtc 1560

tcctccgtct ctcccaagcc accttcatct ttcaagatgt cgattagaaa ctgggtgacc 1620

cgaacacctt cctcatcacc acccatcact ccacctgctt cggagaccaa gatcatgtct 1680

ccgagaaaag cccttattcc tgtgagccag aagtcatccc aagcagaggc ttgctctgag 1740

tctagaaata gagtaaagag gaggctagac tcaagctgtc tggagagtgt gaaacaaaag 1800

tgtgtgaaga gttgtaactg tgtgactgag cttgatggcc aagttgaaaa tcttcatttg 1860

gatctgtgct gccttgctgg taaccaggaa gaccttagta aggactctct aggtcctacc 1920

aaatcaagca aaattgaagg agctggtacc agtatctcag agcctccgtc tcctatcagt 1980

ccgtatgctt cagaaagctg tggaacgcta cctcttcctt tgagaccttg tggagaaggg 2040

tctgaaatgg taggcaaaga gaatagttcc ccagagaata aaaactggtt gttggccatg 2100

gcagccaaac ggaaggctga gaatccatct ccacgaagtc cgtcatccca gacacccaat 2160

tccaggagac agagcggaaa gacattgcca agcccggtca ccatcacgcc cagctccatg 2220

aggaaaatct gcacatactt ccatagaaag tcccaggagg acttctgtgg tcctgaacac 2280

tcaacagaat tatagattct aatctgagtg agttactgag ctttggtcca ctaaaacaag 2340

ctgagctttg gtccactaaa acaagatgaa aaatacaaga gtgactctat aactctggtc 2400

tttaagaaag ctgccttttc atttttagac aaaatctttt caacgctgaa atgtacctaa 2460

tctggttcta ctaccataat gtatatgcag cttcccgagg atgaatgctg tgtttaaatt 2520

tcataaagta aatttgtcac tctagcattt tgaatgaata gtcttcactt tttaaattat 2580

tcatcttctc tataataatg acatcccagt tcatggaggc aaaaaacaag tttcttgtta 2640

tcctgaaact ttctatgctc agtggaaagt atctgccagc cacagcatga ggcctgtgaa 2700

ggctgactga gaaatcctct gctgaagacc cctggttctg ttctgcctcc aacatgtata 2760

attttatttg aaatacataa tcttttcact atgcttttgt ggggtttttt ttaagtatgt 2820

gtaaaaatgt gatgctcaga taagtacatt tatatcagtt cagtgttaaa atgcagtctc 2880

ttgagttaaa gtcatcttta ttttaaatgc agtgataaat gtcaactctt cggagaaact 2940

aggagaacaa caacagaaag ctgtgtttgt cttttttctc tcaaatatat ctcccgtatg 3000

agatttcagg tccccatgtt ttcaccaagc aatctgctat gtcagccaac ccaacatcac 3060

tttctacagg aggttatgat ttttgccatt tactagagga agatgtttta tgaaatcaat 3120

ttggggtttg aattcaggtg cagtcatcag ttctttaggg gctgcaatgt tttaaaaaaa 3180

ataagtcatc agattttaag aaaaaagtga tgatttctta ttgatatttt tgtaacagaa 3240

tatagctctt aactgaaaat ccagaaccag aaacataaat cttgagtttc ttttcatgta 3300

cataaaaagc aatagccttt tagtatagat agccctgagc caaaaagtaa tagaattttc 3360

tctagatatt taatacagag agtgtataga ctgactctaa gttaataatg tgcaaaatat 3420

cttaaacatc cctcccctta ttcaacaatt atgtatcagt gatcttgaac cattgtttta 3480

tatttttcac ctttgtaacc tcatggaaag aggctttaca tactttctat gtactattta 3540

cttagaaggg agcccccttc cagtcatgaa acttcatttg ttttatccat atccctgagg 3600

actgtgtaga ctttatgtca gttctgtgta gactttatgt cagtttttgt cattatttga 3660

aaatctattc tgacaacttt ttaattcctt tgatcttata agttaaagct gtaacaactg 3720

aaattgcatg gatcaagtaa gcatagtttt atccagggag aaaaataaaa ggaagccata 3780

gaattgctct ggtcaaaacc aagcacacca tagccttaac tgaatattta ggaaatctgc 3840

ctaatctgct tatatttggt gtttgttttt tgactgttgg gctttgggaa gatgttattt 3900

atgaccaata tctgccagta acgctgttta tctcacttgc tttgaaagcc aatgggggaa 3960

aaaaatccat gaaaaaaaaa agattgataa agtagatgat tttgtttgta tccctaccca 4020

tctcctggca gccctactga gtgaaattgg gatacatttg gctgtcagaa attataccga 4080

gtctactggg tataacatgt ctcacttgga aagctagtac ttttaaatgg gtgccaaagg 4140

tcaactgtaa tgagataatt atccctgcct gtgtccatgt cagactttga gctgatcctg 4200

aataataaag ccttttacct t 4221

<210> 155
<211> 1483
<212> DNA
<213> Homo sapiens

<400> 155
cttagtggtg tcggggtcta gtggacagag aagactcttg gccaggcaga tggcttctcg 60

gtggcagaac atggggacct ccgtgcgccg gagatctctc cagcaccagg agcagctgga 120

ggacagcaag gagctgcagc ctgtggtcag ccatcaggag acctctgtag gggccctggg 180

gtccctgtgc agacagttcc aaaggaggct gcccctgaga accgtcaacc tcaacctccg 240

cgcagggccc tcctggaaac gcctggaaac cccagagcca ggtcagcagg gcctccaggc 300

tgcagctcgc tcagctaaga gtgctttggg tgccgtgtcc cagagaatcc aggagtcctg 360

ccaaagtggc accaagtggc tggtggagac ccaggtgaag gccaggaggc ggaagagagg 420

agcacagaag ggcagtggat ccccaactca cagcctgagc cagaagagca cccggctgtc 480

tggagccgcc cctgcccact cagccgcaga cccctgggag aaggagcatc accgcctctc 540

tgtccggatg ggctcacatg cccacccatt acggcgatca aggcgggagg ctgccttccg 600

gagcccctac tcctcaacag agcccctctg ctctcccagc gagtctgaca gtgacctaga 660

gcctgtgggg gcgggaattc agcatctcca gaagctgtcc caagagctag atgaagccat 720

tatggcggaa gagagtggtg acatcgtctc tctcattcat gactgaggaa gtgcctgcag 780

gaaacaagcc ctgtctgacc gccaaggctt catactcaag gatgtctatg cttccccgtg 840

tgcttcctgg aaaaaacccc cgggagtcgt cagtacccct gggccactgc taacaagcac 900

ctaacaaggg gcccagagcc ccctgctcca gccacatctg gacccatcag tgactgcctg 960

ccatagcctg agagtgtctt ggggagacct tgcagagggg gagaattgtt ccttctgctt 1020

tcctagggga ctcttgagct tagaaactca tcgtacactt gaccttgagc cttctatttg 1080

cctcatctat aacatgaagt gctagcatca gatatttgag agctcttagc tctgtacccg 1140

ggtgcctggt ttttggggag tcatccgcag agtcactcac ccactgtgtt tctggtgcca 1200

aggctcttga gggccccact ctcatccctc ctttccctac cagggactcg gaggaaggca 1260

taggagatat ttccaggctt acgaccctgg gctcacgggt acctatttat atgctcagtg 1320

cagagcactg tggatgtgcc aggaggggta gccctgttca agagcaattt ctgccctttg 1380

taaattattt aagaaacctg ctttgtcatt ttattagaaa gaaaccagca tgtgactttc 1440

ctagataaca ctgctttctc ataataaaga ctatttgcat ttg 1483

<210> 156
<211> 2319
<212> DNA
<213> Homo sapiens

<400> 156
gtggagtttg aattgggtgg cggttgactg tagagccgct ctctctcact ggcacagcga 60

ggttttgctc agcccttgtc tcgggaccgc agcctccgcc gagcgccatg gctcctagga 120

agggcagtag tcgggtggcc aagaccaact ccttacggag gcggaagctc gcctcctttc 180

tgaaagactt cgaccgtgaa gtggaaatac gaatcaagca aattgagtca gacaggcaga 240

acctcctcaa ggaggtggat aacctctaca acatcgagat cctgcggctc cccaaggctc 300

tgcgcgagat gaactggctt gactacttcg cccttggagg aaacaaacag gccctggaag 360

aggcggcaac agctgacctg gatatcaccg aaataaacaa actaacagca gaagctattc 420

agacacccct gaaatctgcc aaaacacgaa aggtaataca ggtagatgaa atgatagtgg 480

aagaggaaga agaagaagaa aatgaacgta agaatcttca aactgcaaga gtcaaaaggt 540

gtcctccatc caagaagaga actcagtcca tacaaggaaa aggaaaaggg aaaaggtcaa 600

gccgtgctaa cactgttacc ccagccgtgg gccgattgga ggtgtccatg gtcaaaccaa 660

ctccaggcct gacacccagg tttgactcaa gggtcttcaa gacccctggc ctgcgtactc 720

cagcagcagg agagcggatt tacaacatct cagggaatgg cagccctctt gctgacagca 780

aagagatctt cctcactgtg ccagtgggcg gcggagagag cctgcgatta ttggccagtg 840

acttgcagag gcacagtatt gcccagctgg atccagaggc cttgggaaac attaagaagc 900

tctccaaccg tctcgcccaa atctgcagca gcatacggac ccacaaatga gacaccaaag 960

ttgacaggat ggacttttaa tgggcacttc tgggaccctg aagagacttc ttcccttcag 1020

gcttattgtt tgagtgtgaa gttccagagc aaggagccat gttcctctaa gggaattcag 1080

gaattcagac gtgctagtcc cacaccagtt aggtagagct gtctgttcac cctcccatcc 1140

cagctgatcc cagtcactgc ttgctggggc catgccatgg aagcttccca tcagtctccc 1200

agctgaatcc tccctgctct ctgagctgct gccttttgcc tcctgcaact caacatcctc 1260

ttcaccctgc cctgcctgca gttgaggggg cgaagaagaa ccctgtgttc tcaggaagac 1320

tgcctccacc accgctaccc agagaacctc tgcatctggc atttctgctc tctatgcttg 1380

agaccgggag gtttaggctc agataagtga gctctgggcc atgagagggt aggtccagaa 1440

ggtgggggga actgtacaga tcagcagagc aggacagttg gcagcagtga cctcagtagg 1500

gaacatgtcc gtctaccctc tcgcactcat gacacctccc cctaccagcc ctcctcttcc 1560

tcctcctcct cctcctgtgg gaggtggtca gtgggactta gggatctttc acctgctgtg 1620

cccagtagtt ctgaagtctg cttgtggagc agtgttttat gtttatccct gtttactgaa 1680

gaccaaatac tggtttggag acaacttcca tgtcttgctc ttctacctcc ctagttagtg 1740

gaaatttgga taagggaact gtagggccca gattctggag gttttatgtc attggccaca 1800

gaataactgt ctctaagcta tccatggtcc agtggtccct gccaagtctg tagacttcag 1860

agagcacttc tctcttatgg ggttcatggg aacaggggtg ggtgtgactt gcttggtggc 1920

ctcattccat gtgtgcctgt gcctggggca tggactttgt taagcagagt cagcagtgag 1980

gtcctcattc tccagccagc ctctctgccc tggagaatca tgtgctatgt tctaagaatt 2040

tgagaactag agtcctcatc cccaggcttg aaggcacatg gctttctcat gtagggctct 2100

ctgtggtatt tgttattatt ttgcaacaag accattttag taaaacagtc ctgttcaagt 2160

tgtattcttt taagttcttt tattctcctt tccctgagat ttttgtatat attgttctga 2220

gtaatggtat ctttgagctg attgttctaa tcagagctgg tacctacttt caataaattc 2280

tggttttgtg ttttcttttg taaaaaaaaa aaaaaaaaa 2319

<210> 157
<211> 2635
<212> DNA
<213> Homo sapiens

<400> 157
ggccgacgcg agcgccgcgc ttcgcttcag ctgctagctg gcccaaggga ggcgaccgcg 60

gagggtggcg aggggcggcc aggacccgca gccccggggc cgggccggtc cggaccgcca 120

gggagggcag gtcagtgggc agatcgcgtc cgcgggattc aatctctgcc cgctctgata 180

acagtccttt tccctggcgc tcacttcgtg cctggcaccc ggctgggcgc ctcaagaccg 240

ttgtctcttc gatcgcttct ttggacttgg cgaccatttc agagatgtct tccagaagta 300

ccaaagattt aattaaaagt aagtggggat cgaagcctag taactccaaa tccgaaacta 360

cattagaaaa attaaaggga gaaattgcac acttaaagac atcagtggat gaaatcacaa 420

gtgggaaagg aaagctgact gataaagaga gacacagact tttggagaaa attcgagtcc 480

ttgaggctga gaaggagaag aatgcttatc aactcacaga gaaggacaaa gaaatacagc 540

gactgagaga ccaactgaag gccagatata gtactaccgc attgcttgaa cagctggaag 600

agacaacgag agaaggagaa aggagggagc aggtgttgaa agccttatct gaagagaaag 660

acgtattgaa acaacagttg tctgctgcaa cctcacgaat tgctgaactt gaaagcaaaa 720

ccaatacact ccgtttatca cagactgtgg ctccaaactg cttcaactca tcaataaata 780

atattcatga aatggaaata cagctgaaag atgctctgga gaaaaatcag cagtggctcg 840

tgtatgatca gcagcgggaa gtctatgtaa aaggactttt agcaaagatc tttgagttgg 900

aaaagaaaac ggaaacagct gctcattcac tcccacagca gacaaaaaag cctgaatcag 960

aaggttatct tcaagaagag aagcagaaat gttacaacga tctcttggca agtgcaaaaa 1020

aagatcttga ggttgaacga caaaccataa ctcagctgag ttttgaactg agtgaatttc 1080

gaagaaaata tgaagaaacc caaaaagaag ttcacaattt aaatcagctg ttgtattcac 1140

aaagaagggc agatgtgcaa catctggaag atgataggca taaaacagag aagatacaaa 1200

aactcaggga agagaatgat attgctaggg gaaaacttga agaagagaag aagagatccg 1260

aagagctctt atctcaggtc cagtttcttt acacatctct gctaaagcag caagaagaac 1320

aaacaagggt agctctgttg gaacaacaga tgcaggcatg tactttagac tttgaaaatg 1380

aaaaactcga ccgtcaacat gtgcagcatc aattgcttgt aattcttaag gagctccgaa 1440

aagcaagaaa tcaaataaca cagttggaat ccttgaaaca gcttcatgag tttgccatca 1500

cagagccatt agtcactttc caaggagaga ctgaaaacag agaaaaagtt gccgcctcac 1560

caaaaagtcc cactgctgca ctcaatgaaa gcctggtgga atgtcccaag tgcaatatac 1620

agtatccagc cactgagcat cgcgatctgc ttgtccatgt ggaatactgt tcaaagtagc 1680

aaaataagta tttgttttga tattaaaaga ttcaatactg tattttctgt tagcttgtgg 1740

gcattttgaa ttatatattt cacattttgc ataaaactgc ctatctacct ttgacactcc 1800

agcatgctag tgaatcatgt atcttttagg ctgctgtgca tttctcttgg cagtgatacc 1860

tccctgacat ggttcatcat caggctgcaa tgacagaatg tggtgagcag cgtctactga 1920

gactactaac attttgcact gtcaaaatac ttggtgagga aaagatagct caggttattg 1980

ctaatgggtt aatgcaccag caagcaaaat attttatgtt ttgggggttt gaaaaatcaa 2040

agataattaa ccaaggatct taactgtgtt cgcatttttt atccaagcac ttagaaaacc 2100

tacaatccta attttgatgt ccattgttaa gaggtggtga tagatactat tttttttttc 2160

atattgtata gcggttatta gaaaagttgg ggatttcttt gatctttatt gctgcttacc 2220

attgaaactt aacccagctg tgttccccaa ctctgttctg cgcacgaaac agtatctgtt 2280

tgaggcataa tcttaagtgg ccacacacaa tgttttctct tatgttatct ggcagtaact 2340

gtaacttgaa ttacattagc acattctgct tagctaaaat tgttaaaata aactttaata 2400

aacccatgta gccctctcat ttgattgaca gtattttagt tatttttggc attcttaaag 2460

ctgggcaatg taatgatcag atctttgttt gtctgaacag gtatttttat acatgctttt 2520

tgtaaaccaa aaacttttaa atttcttcag gttttctaac atgcttacca ctgggctact 2580

gtaaatgaga aaagaataaa attatttaat gttttaaaaa aaaaaaaaaa aaaaa 2635

<210> 158
<211> 4569
<212> DNA
<213> Homo sapiens

<400> 158
tcttgttgtt acgggtaacg gaagtgtggc ggcgttgggt tgagcgggct ttttggaagt 60

ttgtggcgga gttctgtgat atgagcaaca atggaccaga agattttatc tctagcagca 120

gaaaaaacag cagacaaact gcaagaattt cttcaaaccc tgagagaagg tgatttgact 180

aatctccttc agaatcaagc agtgaaagga aaagttgctg gagcactcct gagagccatc 240

ttcaaaggtt ccccctgctc tgaggaagct ggaacactta ggagacgtaa gatatacact 300

tgttgtatcc agttggtgga atcgggggat ttgcagaaag aaatagcgtc tgagatcata 360

ggattactga tgctggaggc tcaccatttt ccaggaccat tattggttga attagccaat 420

gagtttatta gtgctgtcag agaaggcagc ctagtgaatg gaaaatcttt ggagttacta 480

cctatcattc tcactgccct ggctacgaaa aaggaaaatc tggcttatgg aaaaggtgta 540

ctgagtgggg aagaatgtaa gaaacagttg attaacaccc tgtgttctgg caggtgggat 600

cagcaatatg taatccaact cacctccatg ttcaaggatg tccctctgac tgcagaagag 660

gtggaatttg tggtggaaaa agcattgagc atgttctcca agatgaatct tcaagaaata 720

ccacctttgg tctatcagct tctggttctc tcctccaagg gaagcagaaa gagtgttttg 780

gaaggaatca tagccttctt cagtgcacta gataagcagc acaatgagga acagagtggt 840

gacgagctat tggatgttgt cactgtgcca tcaggtgaac ttcgtcatgt ggaaggcacc 900

attattctac acattgtgtt tgccatcaaa ttggactatg aactaggcag agaactcgtg 960

aaacacttaa aggtaggaca gcaaggagat tccaataata acttaagtcc cttcagcatt 1020

gctcttcttc tgtctgtaac aagaatacaa agatttcagg accaggtgct tgatctttta 1080

aagacttcgg ttgtaaagag ctttaaggat cttcaactcc tccaaggctc aaaatttctt 1140

cagaatctag ttcctcatag atcttatgtt tcaaccatga tcttggaagt agtgaagaat 1200

agcgttcata gctgggacca tgttactcag ggcctcgtag aacttggttt cattttgatg 1260

gattcatatg ggccaaagaa ggttcttgat ggaaaaacta ttgaaaccag cccaagtctt 1320

tctagaatgc caaaccagca tgcatgtaag ctcggagcta atatcctgtt ggaaactttt 1380

aagatccatg agatgatcag acaagaaatt ttggagcagg tcctcaacag ggttgttacc 1440

agagcatctt ctcccatcag tcatttctta gacctgcttt caaatatcgt catgtatgca 1500

cccttagttc ttcaaagttg ttcttctaaa gtcacagaag cttttgacta tttgtccttt 1560

ctgccccttc agactgtaca aaggctgctt aaggcagtgc agccccttct caaagtcagc 1620

atgtcaatga gagactgctt gatacttgtc cttcggaaag ctatgtttgc caaccagctt 1680

gatgcccgaa aatctgcagt tgctgggttt ttgctgctcc tgaagaactt taaagtttta 1740

ggcagcctgt catcctctca gtgcagtcag tctctcagtg tcagtcaggt tcatgtggat 1800

gttcacagcc attacaattc tgtcgccaat gaaacttttt gccttgagat catggatagt 1860

ttgaggagat gcttaagcca gcaagctgat gttcgactca tgctttatga ggggttttat 1920

gatgttcttc gaaggaactc tcagctggct aattcagtca tgcaaactct gctctcacag 1980

ttaaaacagt tctatgagcc aaaacctgat ctgctgcctc ctctgaaatt agaagcttgt 2040

attctgaccc aaggagataa gatctctcta caagaaccac tggattatct gctgtgttgt 2100

attcagcatt gtttggcctg gtataagaat acagtcatac ccttacagca gggagaggag 2160

gaagaggagg aggaagaggc attctacgaa gacctagatg atatattgga gtccattact 2220

aatagaatga ttaagagtga gctggaagac tttgaactgg ataaatcagc agatttttct 2280

cagagcacca gtattggcat aaaaaataat atctgtgctt ttcttgtgat gggagtttgt 2340

gaggttttaa tagaatacaa tttctccata agtagtttca gtaagaatag gtttgaggac 2400

attctgagct tatttatgtg ttacaaaaaa ctctctgaca ttcttaatga aaaagcgggt 2460

aaagccaaaa ctaaaatggc caacaagaca agtgatagtc ttttgtccat gaaatttgtg 2520

tccagtcttc tcactgctct tttcagagtc ttgctatgga gatacacttc aattcctact 2580

tcagtggaag agtcgggaaa gaaagagaaa ggaaagagca tctcactgct gtgcttggag 2640

ggtttacaga aaatattcag tgctgtgcaa cagttctatc agcccaagat tcagcagttt 2700

ctcagagctc tggatgtcac agataaggaa ggagaagaga gagaagatgc agatgtcagt 2760

gtcactcaga gaacagcatt ccagatccgg caatttcaga ggtccttgtt gaatttactt 2820

agcagtcaag aggaagattt taatagcaaa gaagccctcc tgctagtcac ggttcttacc 2880

agtttgtcca agttactgga gccctcctct cctcagtttg tgcagatgtt atcctggaca 2940

tcaaagattt gcaaggaaaa cagccgggag gatgccttgt tttgcaagag cttgatgaac 3000

ttgctcttca gcctgcatgt ttcgtataag agtcctgtca ttctgctgcg tgacttgtcc 3060

caggatatcc acgggcatct gggagatata gaccaggatg tagaggtgga gaaaacaaac 3120

cactttgcaa tagtgaattt gagaacggct gcccccactg tctgtttact tgttctgagt 3180

caggccgaga aggttctaga agaagtggac tggctaatca ccaagcttaa gggacaagtg 3240

agccaagaaa ccttatcaga agaggcctct tctcaggcaa ccctaccaaa tcagcctgtt 3300

gagaaagcta tcatcatgca actgggaact ctgcttacat ttttccacga gctggtgcag 3360

acagctctgc catcaggcag ctgtgtggac accttgttaa aggacttgtg caaaatgtac 3420

accacactta cagcccttgt cagatattat ctccaggtgt gtcagagctc cggaggaatt 3480

ccaaaaaata tggaaaagct ggtgaagctg tctggttctc atctgacccc cctgtgttat 3540

tctttcattt cttacgtaca gaataagagt aagagcctga actatacggg agagaaaaag 3600

gagaaacctg ctgccgttgc cacagccatg gccagagttc ttcgggaaac caagccaatc 3660

cctaacctca tctttgccat agaacagtat gaaaaatttc tcatccacct ttctaagaag 3720

tccaaggtga acctgatgca gcacatgaag ctcagcacct cacgagactt caagatcaaa 3780

ggaaacatcc tagacatggt tcttcgagag gatggtgaag atgaaaatga agagggcact 3840

gcatcagagc atgggggaca gaacaaagaa ccagccaaga agaaaaggaa aaaataaatg 3900

aaatgcctga gttaatgtga actttggggc ttctgcttca tttttaccca acaagcaaca 3960

atgccccttg tcctgtagtc cacaccgatg ttggcatctt ggttctgaac ccactgaatt 4020

caactgcacc ttcagttaga aggaatcttc ttggcaggtc ctgctactga aaaatggctg 4080

gccttaggca agcccttttg caaaaagcac agctgaaagc ctgagtttgg gagcctgcac 4140

caccccgatg aagctccacg ggagcaaata cagagcctcc aggcagtgct atggtccagg 4200

ctggcttcgt ttttccaagg agcctttggt gagttcaatt atctggtaaa tatccagcgc 4260

ttcacctgaa agatagtgca aattggttag gatgccacct caagaactgt aactgagagc 4320

tcagaagtga gcaaaggagc ttaatgctaa ggtcaaaagg agagtgaaag gttgagaaca 4380

attgccacga acggtaatgt tacatgttag gagggtctgt tttcttttta tataagtgtg 4440

tcttagatat attttaaata gaaaataagc tttctgattt acttgtttgg tatttaaagc 4500

acagtttgtt tttctgtcac ctatagagtg caagaatgca ctctatagaa taaattatct 4560

ttaaacatt 4569

<210> 159
<211> 3065
<212> DNA

<213>  Homo sapiens

<400>  159
ctatttgagt ttgtggcgcg cgaggccctg cagtccgggt tggcgcttgg gtactggctg      60

ggtccgatgc tgggtacgct gcgcgccatg gagggcgagg acgtggaaga cgaccagctg     120

ctgcagaagc tcagggccag tcgccgccgc ttccagaggc gcatgcagcg gctgatagag     180

aagtacaacc agcccttcga ggacaccccg gtggtgcaaa tggccacgct gacctacgag     240

acgccacagg gattgagaat ttggggtgga agactaataa aggaaagaaa cgaaggagag     300

atccaggact cctccatgaa gcccgcggac aggacagatg gctccgtgca agctgcagcc     360

tggggtcctg agcttccctc gcaccgcaca gtcctgggag ccgattcaaa aagcggtgag     420

gtcgatgcca cgtcagacca ggaagagtca gttgcttggg ccttagcacc tgcagtgcct     480

caaagccctt tgaaaaatga attaagaagg aaatacttga cccaagtgga tatactgcta     540

caaggtgcag agtattttga gtgtgcaggt aacagagctg gaagggatgt acgtgtgact     600

ccgctgcctt cactggcctc acctgccgtg cctgcccccg gatactgcag tcgtatctcc     660

agaaagagtc ctggtgaccc agcgaaacca gcttcatctc ccagagaatg ggatcctttg     720

catccttcct ccacagacat ggccttagta cctagaaatg acagcctctc cctacaagag     780

accagtagca gcagcttctt aagcagccag ccctttgaag atgatgacat ttgcaatgtg     840

accatcagtg acctgtacgc agggatgctg cactccatga gccggctgtt gagcacaaag     900

ccatcaagca tcatctccac caaaacgttc atcatgcaaa actggaactc caggaggagg     960

cacagatata agagcaggat gaacaaaaca tattgcaaag gagccagacg ttctcagagg    1020

agctccaagg agaacttcat accctgctct gagcctgtga aagggacagg ggcattaaga    1080

gattgcaaga acgtattaga tgtttcttgc cgtaagacag gtttaaaatt ggaaaaagct    1140

tttcttgaag tcaacagacc ccaaatccat aagttagatc caagttggaa ggagcgcaaa    1200

gtgacaccct cgaagtattc ttccttgatt tacttcgact ccagtgcaac atataatctt    1260

gatgaggaaa atagatttag gacattaaaa tggttaattt ctcctgtaaa aatagtttcc    1320

agaccaacaa tacgacaggg ccatggagag aaccgtcaga gggagattga aatccgattt    1380

gatcagcttc atcgggaata ttgcctgagt cccaggaacc agcctcgccg gatgtgcctc    1440

ccggactcct gggccatgaa catgtacaga gggggtcctg cgagtcctgg tggccttcag    1500

ggcttagaaa cccgcaggct gagtttacct tccagcaaag caaaagcaaa aagtttaagt    1560

gaggcttttg aaaacctagg caaaagatct ctggaagcag gtaggtgcct gcccaagagc    1620

gattcatctt catcacttcc aaagaccaac cccacacaca gcgcaactcg cccgcagcag    1680

acatctgacc ttcacgttca gggaaatagt tctggaatat ttagaaagtc agtgtcaccc    1740

agcaaaactc tttcagtccc agataaagaa gtgccaggcc acggaaggaa tcgttacgat    1800

gaaattaaag aagaatttga caagcttcat caaaagtatt gcctcaaatc tcctgggcag    1860

atgacagtgc ctttatgtat tggagtgtct acagataaag caagtatgga agttcgatat    1920

caaacagaag gcttcttagg aaaattaaat ccagaccctc acttccaggg tttccagaag    1980

ttgccatcat cacccctggg gtgcagaaaa agtctactgg gctcaactgc aattgaggct    2040

ccttcatcta catgtgttgc tcgtgccatc acgagggatg gcacgaggga ccatcagttc 2100

cctgcaaaaa gacccaggct atcagaaccc cagggctccg gacgccaggg caattccctg 2160

ggtgcctcag atggggtgga caacaccgtc agaccgggag accagggcag ctcttcacag 2220

cccaactcag aagagagagg agagaacacg tcttacagga tggaagagaa aagtgatttc 2280

atgctagaaa aattggaaac taaaagtgtg tagctaggtt atttcggagt gttatttatc 2340

ttcccacttg ctctctgttt gtatttttgt tttgtttttg attcttgaga ctgtgaggac 2400

ttggttgact tctctgccct taaagtaaat attagtgaaa ttggttccat cagagataac 2460

ctcgagttct tggtgtagaa attatgtgaa taaagttgct caattagaat ttttagggtt 2520

ctctttgata ggcctgtttt tctgatgtgt gtgttttttt tggggggggg ttatttgttt 2580

gtttgtttgt ttgtttgttt gtttttgaga cagtctctct ctatcgccca ggctggagtg 2640

cggtggcaca atcttggctc actgcaactt ccgcctcccg ggttcaagcg attcttctgc 2700

ctcagcctcc cgagtagctg ggattacagg cgcgcgccac cacgcctggc taatttttgt 2760

agttttagta gagacggggt ttcaccatat tgaccaggct ggtctcgagc tcctggcctc 2820

gtgatccatc tgcctcggcc tcccaaagtg ctgggattat aggcgtgagc cactgctccc 2880

agccgtgtgt tcttttttaa atttagatat gtccagagaa tcctctctcc tgtttcccat 2940

ttcattcgag aatattgttt gcttgtgaga cgtaagttcg agccctgcat gcaatgaccc 3000

ttgaaggaaa ataaacagtc ctggtggtcc cagacgctcc tgcagccaca aaaaaaaaaa 3060

aaaaa 3065

<210> 160
<211> 4532
<212> DNA
<213> Homo sapiens

<400> 160
gaacgaagaa ggcgtcccgg catcggccaa gattctacat tgctcatctg ggcatctgag 60

cctccttcga agtttcctgt cacaactgtc ctcttgacag catggatgag gaggaagaca 120

atctgtctct gctgaccgca ctgctggaag aaaatgagtc agccttggat tgtaattcag 180

aagaaaataa cttcttgacg cgggaaaatg gcgagcccga cgcatttgat gagctctttg 240

atgccgacgg cgacggtgaa tcttatacag aagaggctga tgatggagaa acaggagaga 300

caagagacga aaaggaaaat ctggccactc tctttggaga tatggaggac ttaacagatg 360

aagaagaagt tcccgcatca cagtcaactg aaaatagggt cctccctgct cctgccccca 420

ggcgagagaa aacgaatgaa gagttgcaag aggaattaag gaatttgcaa gagcaaatga 480

aggccttaca agagcagcta aaagtaacaa caattaaaca gacagcaagc ccagcccgtc 540

tgcaaaaatc ccctgagaag tctccccggc cacctcttaa ggagaggaga gttcagagaa 600

ttcaggagtc aacatgcttt tctgcggagc ttgatgtccc tgcgctacca agaaccaaga 660

gggtggctcg aacaccaaag gcttcacctc cagatcccaa aagctcatct tcaaggatga 720

caagtgcacc ctcccaaccc ctacagacga tttctcggaa caaacctagt gggataacta 780

gaggtcaaat tgtggggacc ccaggaagtt ctggggaaac gactcaaccc atctgtgtgg 840
aagccttctc tggtctgcgg ctcaggcggc ctcgagtatc ctccacagaa atgaacaaga 900
aaatgaccgg ccgaaaactg atcagactgt ctcagatcaa ggaaaagatg gccagagaga 960
agctggaaga aatagattgg gtgacatttg ggttatattg gaagaaggtt acgccacaga 1020
gtgtgaatag tggaaaaacc ttcagcatat ggaaactgaa tgatcttcgt gacctgacac 1080
aatgtgtgtc cttgttctta tttggagaag ttcacaaagc gctctggaag acggagcagg 1140
ggactgtcgt agggatcctc aatgccaacc ccatgaagcc caaggatggt tcagaggagg 1200
tgtgtttatc tatcgatcat cctcagaagg tcttaattat gggtgaagct cttgacctgg 1260
gaacctgtaa agccaagaag aagaatggag agccgtgcac gcagactgtg aatttgcgtg 1320
actgtgagta ctgtcagtac catgtccagg ctcagtacaa gaagctcagc gcaaagcgtg 1380
cggatctgca gtccaccttc tctggaggac gaattccaaa gaagtttgcc cgcagaggca 1440
ccagcctcaa agaacggctg tgccaagatg gcttttacta cggaggggtt tcttctgcct 1500
cgtatgcagc ttcaattgca gcagctgtgg ctcctaagaa gaagattcaa accactctga 1560
gtaatctggt tgttaagggc acaaacttga tcatccagga aacacggcaa aaactcggaa 1620
taccccagaa gagcctgtct tgctctgagg agttcaagga actgatggac ctgccgacgt 1680
gtggagccag gaacttaaaa caacatttag ccaaagccac agcttcaggg attatgggga 1740
gcccaaaacc agccatcaag tccatctcgg cctcagcact cttgaagcaa cagaagcagc 1800
ggatgttgga gatgaggaga aggaaatcag aagaaataca gaagcgattt ctgcagagct 1860
caagtgaagt tgagagccca gctgtgccat cttcatcaag acagccccct gctcagcctc 1920
cacggacagg atccgagttc cccaggctgg agggagcccc ggccacaatg acgcccaagc 1980
tggggcgagg tgtcttggaa ggagatgatg ttctctttta tgatgagtca ccaccaccaa 2040
gaccaaaact gagtgcttta gcagaagcca aaaagttagc tgctatcacc aaattaaggg 2100
caaaaggcca ggttcttaca aaaacaaacc caaacagcat taagaagaaa caaaaggacc 2160
ctcaggacat cctggaggtg aaggaacgtg tagaaaaaaa caccatgttt tcttctcaag 2220
ctgaggatga attggagcct gccaggaaaa aaaggagaga acaacttgcc tatctggaat 2280
ctgaggaatt tcagaaaatc ctaaaagcaa aatcaaaaca cacaggcatc ctgaaagagg 2340
ccgaggctga gatgcaggag cgctactttg agccactggt gaaaaaagaa caaatggaag 2400
aaaagatgag aaacatcaga gaagtgaagt gccgtgtcgt gacatgcaag acgtgcgcct 2460
atacccactt caagctgctg gagacctgcg tcagtgagca gcatgaatac cactggcatg 2520
atggtgtgaa gaggtttttc aaatgtccct gtggaaacag aagcatctcc ttggacagac 2580
tcccgaacaa gcactgcagt aactgtggcc tctacaaatg ggaacgggac ggaatgctaa 2640
aggaaaagac tggtccaaag ataggaggag aaactctgtt accaagagga gaagaacatg 2700
ctaaatttct gaacagcctt aaataacccg aacttcagac attttcccac agacttcctg 2760
gcctcctgtg actctggaaa gcaaaggatt ggctgtgtat tgtccattga ttcctgattg 2820
acgccgtcaa aaacaaatgc ttgttaagcc cataagcttt gcctgcttac tttctgccat 2880

tgggttggtt tgataccaca tttaacattg acatttaagt ggaaaaccaa gttatcattg 2940

tcttttctaa gctcagtgtg gatgattgca ttacttcatt cactgaagtt tttgcccaaa 3000

aattggaagg taaacagaga gctatgtttc tgtatctttt ggttatagag tgttcacttc 3060

tttatcataa caaaattcta gtgtttatac gaacacccag aggcaaaaga atttggctta 3120

attctcactc caggtaagta gcttaacttc tgggcttcag ttttctcatc tgtaaaatca 3180

ggaagattgg actaagtgat cctgaaatgt attttttagc actggatttc tacaaataat 3240

aaaactttcc catctagata atgatgatca catagtcttg atgtacggac attaaaagcc 3300

agatttcttc attcaattct gttatctctg ttttactctt tgaaattgat caagccactg 3360

aatcactttg catttcagtt tatatatata gagagaaaga aggtgtctgc tcttacatta 3420

ttgtggagcc ctgtgataga aatatgtaaa atctcatatt attttttttt taattttttt 3480

attttttatg acagggtctc actatgtcac cctggctgga gtgcagtagt gcgatcgcgg 3540

cacactgcag ccttggcttc cctgggctca agcagtcctc ccacctcagt ctcccaaata 3600

gctaggacta caggcgtgcg tgaccaagcc cagctaattt ttgcattttt tgtagagatg 3660

gggttttgcc atgttgctca ggctggtctc aaactcctga gcactagcaa tccacccacc 3720

tctgtttcca aaaaaaaaaa aaaaatgaaa ggtcaacccc tatgcaaatt accacagcaa 3780

aggtttcatt caggagattc ttccatctgg gcaacctggt tttccaaata tcatttgacc 3840

taagtgaatg ttgatactag ctaaagattg ggtaaattgg ttgaattatt gtattgaagc 3900

ttgagctgta gctaaaagta atttaggttt cccctaagat gttattatgt tagggacata 3960

acacttttgg gaggttgttg tgggagatgg ttgatttagg ttttcaaaag ctagaaataa 4020

aatttacatg ccttagattt cataaaattc tgctctaatt gggtggaagg tgctgtatct 4080

aacttgtgtt cctcctaagg ttatgtccta ataactattc ttttaggagt atacttctac 4140

tttatagaag gttgcttttc tttttaattt tttctaacaa agaaaagaat aaagtattta 4200

ttaataagaa ccagaaagca cttgaaactg atgtttttaa tggctcattt agggtagatt 4260

tatttatctc attaacttaa aacagctatg tgtatgaaat aggtcacaac agaacttgaa 4320

caccaggttg gtgtctgagc aatccctttc ttatgggaaa aacaatgttc ttgtttgaac 4380

agagggtatc attgcagtca gtattcacgt gtatattgtt atataagttg tataatatgc 4440

ttgtaaaggc tgagggtgag ctgtatctgg atgccttttt acaatttgat tttaactttt 4500

aaaataaatt taaaacataa aaaaaaaaaa aa 4532

<210> 161
<211> 4535
<212> DNA
<213> Homo sapiens

<400> 161
gaacgaagaa ggcgtcccgg catcggccaa gattctacat tgctcatctg ggcatctgag 60

cctccttcga agtttcctgt cacaactgtc ctcttgacag catggatgag gaggaagaca 120

atctgtctct gctgaccgca ctgctggaag aaaatgagtc agccttggat tgtaattcag 180

aagaaaataa cttcttgacg cgggaaaatg gcgagcccga cgcatttgat gagctctttg 240

atgccgacgg cgacggtgaa tcttatacag aagaggctga tgatggagaa acaggagaga 300

caagagacga aaaggaaaat ctggccactc tctttggaga tatggaggac ttaacagatg 360

aagaagaagt tcccgcatca cagtcaactg aaaatagggt cctccctgct cctgccccca 420

ggcgagagaa aacgaatgaa gagttgcaag aggaattaag gaatttgcaa gagcaaatga 480

aggccttaca agagcagcta aaagtaacaa caattaaaca gacagcaagc ccagcccgtc 540

tgcaaaaatc ccctgtagag aagtctcccc ggccacctct taaggagagg agagttcaga 600

gaattcagga gtcaacatgc ttttctgcgg agcttgatgt ccctgcgcta ccaagaacca 660

agagggtggc tcgaacacca aaggcttcac ctccagatcc caaaagctca tcttcaagga 720

tgacaagtgc accctcccaa cccctacaga cgatttctcg gaacaaacct agtgggataa 780

ctagaggtca aattgtgggg accccaggaa gttctgggga aacgactcaa cccatctgtg 840

tggaagcctt ctctggtctg cggctcaggc ggcctcgagt atcctccaca gaaatgaaca 900

agaaaatgac cggccgaaaa ctgatcagac tgtctcagat caaggaaaag atggccagag 960

agaagctgga agaaatagat tgggtgacat ttggggttat attgaagaag gttacgccac 1020

agagtgtgaa tagtggaaaa accttcagca tatggaaact gaatgatctt cgtgacctga 1080

cacaatgtgt gtccttgttc ttatttggag aagttcacaa agcgctctgg aagacggagc 1140

aggggactgt cgtagggatc ctcaatgcca accccatgaa gcccaaggat ggttcagagg 1200

aggtgtgttt atctatcgat catcctcaga aggtcttaat tatgggtgaa gctcttgacc 1260

tgggaacctg taaagccaag aagaagaatg gagagccgtg cacgcagact gtgaatttgc 1320

gtgactgtga gtactgtcag taccatgtcc aggctcagta caagaagctc agcgcaaagc 1380

gtgcggatct gcagtccacc ttctctggag gacgaattcc aaagaagttt gcccgcagag 1440

gcaccagcct caaagaacgg ctgtgccaag atggctttta ctacggaggg gtttcttctg 1500

cctcgtatgc agcttcaatt gcagcagctg tggctcctaa gaagaagatt caaaccactc 1560

tgagtaatct ggttgttaag ggcacaaact tgatcatcca ggaaacacgg caaaaactcg 1620

gaatacccca gaagagcctg tcttgctctg aggagttcaa ggaactgatg gacctgccga 1680

cgtgtggagc caggaactta aaacaacatt tagccaaagc cacagcttca gggattatgg 1740

ggagcccaaa accagccatc aagtccatct cggcctcagc actcttgaag caacagaagc 1800

agcggatgtt ggagatgagg agaaggaaat cagaagaaat acagaagcga tttctgcaga 1860

gctcaagtga agttgagagc ccagctgtgc catcttcatc aagacagccc cctgctcagc 1920

ctccacggac aggatccgag ttccccaggc tggagggagc cccggccaca atgacgccca 1980

agctggggcg aggtgtcttg gaaggagatg atgttctctt ttatgatgag tcaccaccac 2040

caagaccaaa actgagtgct ttagcagaag ccaaaaagtt agctgctatc accaaattaa 2100

gggcaaaagg ccaggttctt acaaaaacaa acccaaacag cattaagaag aaacaaaagg 2160

accctcagga catcctggag gtgaaggaac gtgtagaaaa aaacaccatg ttttcttctc 2220

aagctgagga tgaattggag cctgccagga aaaaaaggag agaacaactt gcctatctgg 2280

aatctgagga atttcagaaa atcctaaaag caaaatcaaa acacacaggc atcctgaaag 2340

aggccgaggc tgagatgcag gagcgctact ttgagccact ggtgaaaaaa gaacaaatgg 2400

aagaaaagat gagaaacatc agagaagtga agtgccgtgt cgtgacatgc aagacgtgcg 2460

cctataccca cttcaagctg ctggagacct gcgtcagtga gcagcatgaa taccactggc 2520

atgatggtgt gaagaggttt ttcaaatgtc cctgtggaaa cagaagcatc tccttggaca 2580

gactcccgaa caagcactgc agtaactgtg gcctctacaa atgggaacgg gacggaatgc 2640

taaaggaaaa gactggtcca aagataggag gagaaactct gttaccaaga ggagaagaac 2700

atgctaaatt tctgaacagc cttaaataac ccgaacttca gacattttcc cacagacttc 2760

ctggcctcct gtgactctgg aaagcaaagg attggctgtg tattgtccat tgattcctga 2820

ttgacgccgt caaaaacaaa tgcttgttaa gcccataagc tttgcctgct tactttctgc 2880

cattgggttg gtttgatacc acatttaaca ttgacattta agtggaaaac caagttatca 2940

ttgtcttttc taagctcagt gtggatgatt gcattacttc attcactgaa gtttttgccc 3000

aaaaattgga aggtaaacag agagctatgt ttctgtatct tttggttata gagtgttcac 3060

ttctttatca taacaaaatt ctagtgttta tacgaacacc cagaggcaaa agaatttggc 3120

ttaattctca ctccaggtaa gtagcttaac ttctgggctt cagttttctc atctgtaaaa 3180

tcaggaagat tggactaagt gatcctgaaa tgtatttttt agcactggat ttctacaaat 3240

aataaaactt tcccatctag ataatgatga tcacatagtc ttgatgtacg gacattaaaa 3300

gccagatttc ttcattcaat tctgttatct ctgttttact ctttgaaatt gatcaagcca 3360

ctgaatcact ttgcatttca gtttatatat atagagagaa agaaggtgtc tgctcttaca 3420

ttattgtgga gccctgtgat agaaatatgt aaaatctcat attatttttt ttttaatttt 3480

tttatttttt atgacagggt ctcactatgt caccctggct ggagtgcagt agtgcgatcg 3540

cggcacactg cagccttggc ttccctgggc tcaagcagtc ctcccacctc agtctcccaa 3600

atagctagga ctacaggcgt gcgtgaccaa gcccagctaa tttttgcatt ttttgtagag 3660

atggggtttt gccatgttgc tcaggctggt ctcaaactcc tgagcactag caatccaccc 3720

acctctgttt ccaaaaaaaa aaaaaaaatg aaaggtcaac ccctatgcaa attaccacag 3780

caaaggtttc attcaggaga ttcttccatc tgggcaacct ggttttccaa atatcatttg 3840

acctaagtga atgttgatac tagctaaaga ttgggtaaat tggttgaatt attgtattga 3900

agcttgagct gtagctaaaa gtaatttagg tttcccctaa gatgttatta tgttagggac 3960

ataacacttt tgggaggttg ttgtgggaga tggttgattt aggttttcaa aagctagaaa 4020

taaaatttac atgccttaga tttcataaaa ttctgctcta attgggtgga aggtgctgta 4080

tctaacttgt gttcctccta aggttatgtc ctaataacta ttcttttagg agtatacttc 4140

tactttatag aaggttgctt ttctttttaa ttttttctaa caaagaaaag aataaagtat 4200

ttattaataa gaaccagaaa gcacttgaaa ctgatgtttt taatggctca tttagggtag 4260

atttatttat ctcattaact taaaacagct atgtgtatga aataggtcac aacagaactt 4320

gaacaccagg ttggtgtctg agcaatccct ttcttatggg aaaaacaatg ttcttgtttg 4380

aacagagggt atcattgcag tcagtattca cgtgtatatt gttatataag ttgtataata 4440

tgcttgtaaa ggctgagggt gagctgtatc tggatgcctt tttacaattt gattttaact 4500

tttaaaataa atttaaaaca taaaaaaaaa aaaaa 4535

<210> 162
<211> 1746
<212> DNA
<213> Homo sapiens

<400> 162
ggaggccggc tatttgaagg cggcgcgcgg actaggtgcg cacttcagtt ctcggagaga 60

agaggcggga gtggacctgg tcagccctac cccactgacc ccaccggacc caggcgcggc 120

ctccgccaca gccacagccc ctgcccctgc tgcggcgcgg cgaggcgagg cgatggccaa 180

ggtgtcggtg ctgaacgtgg cggtcctgga gaacccgagc cctttccaca gccccttccg 240

gttcgagatc agcttcgagt gcagtgaagc cctggcggac gacctggagt ggaagatcat 300

ttatgttggc tcggctgaga gtgaggaatt tgatcagatc ctagactcgg tgctggtggg 360

ccctgtgcca gcagggagac acatgtttgt ctttcaggcc gacgccccca acccatccct 420

catcccagag actgatgccg tgggtgtgac tgtggtcctc atcacctgca cctaccatgg 480

acaggagttc atccgagtgg gctactacgt caacaacgag tacctcaacc ctgagctgcg 540

tgagaacccg cccatgaagc cagatttctc ccagctccag cggaacatct tggcctcgaa 600

cccccgggtg acccgcttcc atatcaactg ggacaacaac atggacaggc tggaggccat 660

agagacccag gacccctccc tgggctgcgg cctcccactc aactgcactc ctatcaaggg 720

cttggggctc cctggctgca tccctggcct cctccctgag aactccatgg actgcatcta 780

actgcaggaa cccagagtgt cccagcacgc cgggaggggc aaccaggcct cccagcgagt 840

cctgcagggc ccatctagag gactttgggg gccatcagct gcaatccagg tctgtcaaac 900

tcagccccta ggaaagaaca ggccttgggt ctcccctagt cctggccaga aggatgatct 960

cgcttttcct ctacaggcct ataagaagca ggtacttcag ttctaaattc tgacttgtgt 1020

tcttttcgtc ttcataaatt ctaactaagg ccactgtgcc actgtgcacc cttgagtacc 1080

attgatccaa agctttccca cagacctccc tggcccacct agaggctttc ttggtcagtg 1140

cctgtcaagg ctccagtcct gctgagccaa aggctttgtc attcctttct cttcctgtac 1200

atctgagcag acccactcca gctttctggt gtcacaggcg ggaatgttag ttagtaggta 1260

gacttagatc ccatttctgt cctgctccca ggaagattct taggtcctct tcaatccagc 1320

agcccctccc agaggtgtga tcagcaggat gctgaggaac catgttgcct ttcctgtcaa 1380

tcacagccac cttcctgtta tctcctaaat ggatctggct tttcctggag gctgccatgg 1440

ttggaagatg gtatcagagg gcctgcctgg gcagtctgtc tccgggccag ggtcagggac 1500

cctctgcctc tggcagcctt aacctgtcct ctgctaggac cagggtgatt tcaagccagg 1560

gaagcaactg ggaccctgaa aactgtccct ccccagcccg ctccccctct ctgtgccctg 1620

gtccccttgc tgccatgtgg atgctgttgt gattgctgtt tgtatattat caaaatgttt 1680

ttatattaaa aatgtttggt ctgaaaatta aaagcacttc atttagaatg aaaaaaaaaa 1740

aaaaaa                                                              1746

<210>  163
<211>  1405
<212>  DNA
<213>  Homo sapiens

<400>  163
cacctcgctc gcagcctccc cagcgcagca gcccggctgt gggcctgcgg cagccgggtc      60

ttcctggtcc ccacctcctg gggccgacgg gcggcaggaa ggggctcggc gggacgcgcc     120

gtcagggacc tgaggaggaa caacggaacg cgttcggaac ggcctggact cccgagactc     180

acccgactcg tggccacacc gggagaactg aagcggcagt agccggcgga gacgcccgac     240

ccgaaggccg gctgctaggg agcagacagc tgaaccgctt gccagacgcc gaaacccagt     300

gacgccctcc accgctccac cgtgctcccg gctccccgcc cccgccgccc gcgggcccca     360

aggcgcatgc gccgcctgtc ctggaggggc ccatttccgt ccgtcgtggg gggaggcaca     420

gtgagtccac tggggcacgg cagcgtctaa gccacaagcc gagcacataa gccaggtcct     480

aacggagcct atgtgtaagt ccactactgg tgcaaggttg cacacttcta agaagagcgg     540

cgtggggggc tcggcgacct tcgcttcagt cgctcccccg tgcagtcccc tgtgcccaag     600

acacagcctg atgcttgtgc tccggtgggc ggagcttgga ggcggcggga actgcaattg     660

gtggctttga aggcgcggcg agcgggaaca gctcttgagg agtgagactg caggagatgt     720

gggccgtgcc aaagagatgg atgagactgt tgctgagttc atcaagagga ccatcttgaa     780

aatccccatg aatgaactga caacaatcct gaaggcctgg gattttttgt ctgaaaatca     840

actgcagact gtaaatttcc gacagagaaa ggaatctgta gttcagcact tgatccatct     900

gtgtgaggaa aagcgtgcaa gtatcagtga tgctgccctg ttagacatca tttatatgca     960

atttcatcag caccagaaag tttgggatgt ttttcagatg agtaaaggac caggtgaaga    1020

tgttgacctt tttgatatga aacaatttaa aaattcgttc aagaaaattc ttcagagagc    1080

attaaaaaat gtgacagtca gcttcagaga aactgaggag aatgcagtct ggattcgaat    1140

tgcctgggga acacagtaca caaagccaaa ccagtacaaa cctacctacg tggtgtacta    1200

ctcccagact ccgtacgcct tcacgtcctc ctccatgctg aggcgcaata caccgcttct    1260

gggtcaggag ttagaagcta ctgggaaaat ctacctccga caagaggaga tcattttaga    1320

tattaccgaa atgaagaaag cttgcaatta gtgaacatga aaggaaaata aaaattcctc    1380

acagtcaaaa aaaaaaaaaa aaaaa                                         1405

<210>  164
<211>  2140
<212>  DNA
<213>  Homo sapiens

<400>  164
gtggcgcgca cgcgcagctc cgcttccggg agccgctcct cgctacccac tatgctctgc      60

gacatcgacc tgtcgcaaag gccgcgtttg cggggccaat gagcgactcg ctttccgtgc     120

ggtgcggcga gtgaggcccc ggtcttcctc ctcgtcctgc cgcaggccag aacccctgac     180

ggtattcagc tgcgcgtaag tctggccggt gccatctgtc tccgcaatgc cccccaagaa 240

acaggctcag gccgggggca gcaaaaaggc ggacgaaaaa aagaaggaga agattatcga 300

agacaaaact ttcggtttga agaataagaa aggagcaaag caacagaagt ttatcaaggc 360

tgtcacacat caagttaaat ttggtcaaca aaatccacgt caggtagcac agagtgaagc 420

tgaaaagaaa ttgaagaagg atgacaagaa gaaagatttg caggagctaa atgagctgtt 480

caaacctgta gttgctgctc aaaaaataag taaaggtgca gatcccaagt ctgtagtatg 540

tgcattcttc aagcaaggac agtgtactaa aggagataag tgtaagttct cccatgactt 600

gactctggag agaaaatgtg aaaagcgaag tgtttacatt gatgcaagag atgaagaact 660

tgaaaaagat actatggata attgggatga gaaaaagctg gaagaagtag tgaacaagaa 720

gcacggtgag gcggaaaaga aaaaaccaaa aactcaaata gtgtgcaagc atttcctgga 780

agctattgaa aacaacaagt atggctggtt ttgggtatgc cctggagggg gtgatatttg 840

catgtatcgt catgcacttc ctcctggatt tgtgttgaaa aaagataaaa agaaagaaga 900

gaaagaagat gaaatttcat tagaagatct aattgagaga gagcgttctg ccctaggtcc 960

aaatgttacc aaaatcactc tagaatcttt tcttgcctgg aagaaaagga aaagacaaga 1020

aaagattgat aaacttgaac aagatatgga aagaaggaaa gctgacttca aagcagggaa 1080

agcactagtg atcagtggtc gtgaagtgtt tgaatttcgt cctgaactgg tcaatgatga 1140

tgatgaggaa gcagatgata cccgctacac ccagggaaca ggtggtgatg aggttgatga 1200

ttcagtgagt gtaaatgaca tagatttaag cctgtacatc ccaagagatg tagatgaaac 1260

aggtattact gtagccagtc ttgaaagatt cagcacatat acttcagata aagatgaaaa 1320

caaattaagt gaagcttctg gaggtagggc tgaaaatggt gaaagaagtg acttggaaga 1380

ggacaacgag agggagggaa ccggaaaatg gagccattga tgctgttcct gttgatgaaa 1440

atcttttcca ctggagagga tttggatgaa ctagaagaag aattaaatac cttgatttag 1500

aagaatgccc caaccctcgc tgaaaaaatt aagtcagcca gcacgagttg aaattgacta 1560

cattaatttc ctttccacct agaatcaaca ggatgtttat ttcctatgct gattctggag 1620

gagttacctc ctgcaaaaaa ggcatcttgt ccctacatct tctcttctga ctttggctac 1680

atctcatagt aagttcagag tagttcatga taaattgaaa atataatggt cattgcagaa 1740

aatgattgat gttgtactgt ccacccaagt aagaagtgta tctgcctttc catcttttgg 1800

ttttcatttg ggcatgtgct attaccagaa acaacaaact tatatttaaa atacccttca 1860

tttgacacag tttttaatga gtgatttaat ttcctctgta tttgtatgtt tagaagactg 1920

cctaaaacat gagcactgta cttcataaag gaaactgcgt atgcagattc agtattgtgt 1980

atctttggac aattagatgg acatttaaaa tggaacttct tttatctgac aggatcagct 2040

acaatgccct gtgttaaatt gtttaaaagt ttcccttttc ttttttgcca ataaagttgt 2100

aaataaagac catcatacat taaaaaaaaa aaaaaaaaaa 2140

<210> 165
<211> 1899
<212> DNA

<213>  Homo sapiens

<400>  165
agcgcgcgac tttttgaaag ccaggagggt tcgaattgca acggcagctg ccgggcgtat      60

gtgttggtgc tagaggcagc tgcagggtct cgctgggggc cgctcgggac caattttgaa     120

gaggtacttg gccacgactt attttcacct ccgacctttc cttccaggcg gtgagactct     180

ggactgagag tggctttcac aatggaaggg atcagtaatt tcaagacacc aagcaaatta     240

tcagaaaaaa agaaatctgt attatgttca actccaacta taaatatccc ggcctctccg     300

tttatgcaga agcttggctt tggtactggg gtaaatgtgt acctaatgaa aagatctcca     360

agaggtttgt ctcattctcc ttgggctgta aaaaagatta atcctatatg taatgatcat     420

tatcgaagtg tgtatcaaaa gagactaatg gatgaagcta agattttgaa aagccttcat     480

catccaaaca ttgttggtta tcgtgctttt actgaagcca atgatggcag tctgtgtctt     540

gctatggaat atggaggtga aaagtctcta aatgacttaa tagaagaacg atataaagcc     600

agccaagatc cttttccagc agccataatt ttaaaagttg ctttgaatat ggcaagaggg     660

ttaaagtatc tgcaccaaga aaagaaactg cttcatggag acataaagtc ttcaaatgtt     720

gtaattaaag gcgattttga aacaattaaa atctgtgatg taggagtctc tctaccactg     780

gatgaaaata tgactgtgac tgaccctgag gcttgttaca ttggcacaga gccatggaaa     840

cccaaagaag ctgtggagga gaatggtgtt attactgaca aggcagacat atttgccttt     900

ggccttactt tgtgggaaat gatgacttta tcgattccac acattaatct ttcaaatgat     960

gatgatgatg aagataaaac ttttgatgaa agtgattttg atgatgaagc atactatgca    1020

gcgttgggaa ctaggccacc tattaatatg gaagaactgg atgaatcata ccagaaagta    1080

attgaactct tctctgtatg cactaatgaa gaccctaaag atcgtccttc tgctgcacac    1140

attgttgaag ctctggaaac agatgtctag tgatcatctc agctgaagtg tggcttgcgt    1200

aaataactgt ttattccaaa atatttacat agttactatc agtagttatt agactctaaa    1260

attggcatat ttgaggacca tagtttcttg ttaacatatg gataactatt tctaatatga    1320

aatatgctta tattggctat aagcacttgg aattgtactg ggttttctgt aaagttttag    1380

aaactagcta cataagtact ttgatactgc tcatgctgac ttaaaacact agcagtaaaa    1440

cgctgtaaac tgtaacatta aattgaatga ccattacttt tattaatgat ctttcttaaa    1500

tattctatat tttaatggat ctactgacat tagcactttg tacagtacaa aataaagtct    1560

acatttgttt aaaacactga accttttgct gatgtgttta tcaaatgata actggaagct    1620

gaggagaata tgcctcaaaa agagtagctc cttggatact tcagactctg gttacagatt    1680

gtcttgatct cttggatctc ctcagatctt tggtttttgc tttaatttat taaatgtatt    1740

ttccatactg agtttaaaat ttattaattt gtaccttaag catttcccag ctgtgtaaaa    1800

acaataaaac tcaaatagga tgataaagaa taaaggacac tttgggtacc agaaaaaaaa    1860

aaaaaaaaaa aaaaaaaaaa aaaaaaaaaa aaaaaaaaa                            1899

<210>  166
<211>  1290

<212>  DNA
<213>  Homo sapiens

<400>  166
gtgagcggca acatggcgtc caggtctaag cggcgtgccg tggaaagtgg ggttccgcag      60

ccgccggatc ccccagtcca gcgcgacgag gaagaggaaa aagaagtcga aaatgaggat     120

gaagacgatg atgacagtga caaggaaaag gatgaagagg acgaggtcat tgacgaggaa     180

gtgaatattg aatttgaagc ttattcccta tcagataatg attatgacgg aattaagaaa     240

ttactgcagc agctttttct aaaggctcct gtgaacactg cagaactaac agatctctta     300

attcaacaga accatattgg gagtgtgatt aagcaaacgg atgtttcaga agacagcaat     360

gatgatatgg atgaagatga ggtttttggt ttcataagcc ttttaaattt aactgaaaga     420

aagggtaccc agtgtgttga acaaattcaa gagttggttc tacgcttctg tgagaagaac     480

tgtgaaaaga gcatggttga acagctggac aagtttttaa atgacaccac caagcctgtg     540

ggccttctcc taagtgaaag attcattaat gtccctccac agatcgctct gcccatgtac     600

cagcagcttc agaaagaact ggcgggggca cacagaacca ataagccatg tgggaagtgc     660

tacttttacc ttctgattag taagacattt gtggaagcag gaaaaaacaa ttccaaaaag     720

aaacctagca acaaaaagaa agctgcgtta atgtttgcaa atgcagagga agaatttttc     780

tatgaggagc agggaaaacc tgaggtgctt ggaggtccag acacaagaag aggattggaa     840

ccagttccga tacagcacaa tggtggttca cgggggcaag tgacagccct ggtttctctg     900

aaggctggac taattcaatc aagatcaact ctaagtgatt tccagggaac cttcatgact     960

gttggaattg ctctgtcata ataagtcagg gatatttagg aggctcatag tctcctggag    1020

ggataaaaca tctcggcacc tagtaatggt aaattagtca atatttgtta gtttcgtttg    1080

agatctcaaa tgttaggatt ttctgaagtc tcagtgtctt tcagagtttg agataccttg    1140

ttttcatttt ttctaatttt taatttaaaa gtaataaaga atattttgta atgattagga    1200

tgaaaatttt atcgtcaatt ataagccaaa atttgctaaa attaaagtca gtatacttgg    1260

aaaaaaaaaa aaaaaaaaaa aaaaaaaaaa                                      1290

<210>  167
<211>  1268
<212>  DNA
<213>  Homo sapiens

<400>  167
gtgagcggca acatggcgtc caggtctaag cggcgtgccg tggaaagtgg ggttccgcag      60

ccgccggatc ccccagtcca gcgcgacgag gaagaggaaa aagaagtcga aaatgaggat     120

gaagacgatg atgacagtga caaggaaaag gatgaagagg acgaggtcat tgacgaggaa     180

gtgaatattg aatttgaagc ttattcccta tcagataatg attatgacgg aattaagaaa     240

ttactgcagc agctttttct aaaggctcct gtgaacactg cagaactaac agatctctta     300

attcaacaga accatattgg gagtgtgatt aagcaaacgg atgtttcaga agacagcaat     360

gatgatatgg atgaagatga ggtttttggt ttcataagcc ttttaaattt aactgaaaga     420

aagggtaccc agtgtgttga acaaattcaa gagttggttc tacgcttctg tgagaagaac     480

tgtgaaaaga gcatggttga acagctggac aagtttttaa atgacaccac caagcctgtg 540

ggccttctcc taagtgaaag attcattaat gtccctccac agatcgctct gcccatgtac 600

cagcagcttc agaaagaact ggcgggggca cacagaacca ataagccatg tgggaagtgc 660

tacttttacc ttctgattag taagacattt gtggaagcag gaaaaaacaa ttccaaaaag 720

aaacctagca acaaaaagaa agctgcgtta atgtttgcaa atgcagagga agaatttttc 780

tatgagaagg caattctcaa gttcaactac tcagtgcagg aggagagcga cacttgtctg 840

ggaggcaaat ggtcttttga tgacgtacca atgacgccct tgcgaactgt gatgttaatt 900

ccaggcgaca agatgaacga aatcatggat aaactgaaag aatatctatc tgtctaaccc 960

atttccaatg gacagtgatg ggcttgtttt tgtaaaatta ccagaaaact cagtggagat 1020

ttactgaaaa actcagactt tattcagatt aagttcctct acaaaaagta gggttctgtc 1080

ccatgtgtct ctgacacatt tacaaaatac cagtttttta aaattttggt caaattatga 1140

gtggttgatt taaaaacttt tccaagaaga agaaaagcat ggagtcgtaa tttaaagaac 1200

tcaataaaaa cttctatttt ttattttaaa ataataccaa aaaaaaaaaa aaaaaaaaaa 1260

aaaaaaaa 1268

<210> 168
<211> 2338
<212> DNA
<213> Homo sapiens

<400> 168
   gtgagcggca acatggcgtc caggtctaag cggcgtgccg tggaaagtgg ggttccgcag 60
   ccgccggatc ccccagtcca gcgcgacgag gaagaggaaa aagaagtcga aaatgaggat 120
   gaagacgatg atgacagtga caaggaaaag gatgaagagg acgaggtcat tgacgaggaa 180
   gtgaatattg aatttgaagc ttattcccta tcagataatg attatgacgg aattaagaaa 240
   ttactgcagc agctttttct aaaggctcct gtgaacactg cagaactaac agatctctta 300
   attcaacaga accatattgg gagtgtgatt aagcaaacgg atgtttcaga agacagcaat 360
   gatgatatgg atgaagatga ggtttttggt ttcataagcc ttttaaattt aactgaaaga 420
   aagggtaccc agtgtgttga acaaattcaa gagttggttc tacgcttctg tgagaagaac 480
   tgtgaaaaga gcatggttga acagctggac aagtttttaa atgacaccac caagcctgtg 540
   ggccttctcc taagtgaaag attcattaat gtccctccac agatcgctct gcccatgtac 600
   cagcagcttc agaaagaact ggcgggggca cacagaacca ataagccatg tgggaagtgc 660
   tacttttacc ttctgattag taagacattt gtggaagcag gaaaaaacaa ttccaaaaag 720
   aaacctagca acaaaaagaa agctgcgtta atgtttgcaa atgcagagga agaatttttc 780
   tatgaggagc agggaaaacc tgaggtgctt ggaggtccag acacaagaag aggattggaa 840
   ccagttccga tacagcacaa tggttggtct gttcccccag tattagaata aattaaaaca 900
   tacaagccag gatgaatgaa gccaatagga aataaaaaac aggcacacaa cattatttgg 960
   ggaaaaacac ctctagtgac acattttctt catctgcact gattatttca gcaaaagaaa 1020
   tctagtatgt tttcatacat ctgatgtata aatttgctga actttgagtt agttttctta 1080
   agataatttt tcatattaag aaaattttcc ttttctaacc tattaaaata cctcactatc 1140
   atttcaaaag gttaaatgag ttgatcacta tctctgctct gtgctcctca aaatataatt 1200
   tcaaatggat ccgatctaaa tctataccta gtgcagattt gcaaaaaaag aaaaggaata 1260
   gtcattaagg atacctgtta ccatggctgc gattgttagc acttcatcta cacagtcaaa 1320
   ttcacaggac gctaagatag acttcgagag ttgtggatca agaggaaact ctgacatgat 1380
   gattccaaat tcagaaagat ttccatcatt atccagtgct gccagataat ctaagtcttc 1440
   caatgcctgc atcaaacttt ctggtgctag gaaaggaaaa aaataataat ttaagagtct 1500
   catatggcta agatggagaa acaggtactg gacttttccc tcctgcatga aacaagcaaa 1560
   caatggacaa aatatgtgaa acaacggttt gcaagccacc aaataccagg gagtgaagga 1620
   tagtgattct tgagagggca aactcaggag gggagccctg tgattgtcca gctcatgctc 1680
   cgaggagggt cccagctgca gtgtgcatgc gggggaaccc caggtggagc ctgccagcct 1740
   ccctgcagta ggaaaacaat gccagggatc cagggagact aaggtggccg gaactcgcgg 1800
   tcacagtact ggagagagac acacagatct aagtcggggg ccagagggag ctccctcaac 1860
   taggcagcac agccctactg atcagcgcat gtgtgtaagg aaccacctca ggccagagaa 1920
   agagccatgt gagaagatta gaaggaacag tggtgctgac acaggaccta gggtagggcc 1980
   tgttcccacc accctagctg gaaaacctca tgatttatgg ggcactgggt agaaaacaca 2040
   agtgagaaac cctagatggg cactgccacc tgacacagct atacagcagg acctgaaagg 2100
   atcacactgc atcctaggac aaagctcaaa agtacctctg ggactacaaa aatattcagt 2160
   gcccaagaag gtaaaatgta caatgtctag catccagtag agaggcatga aaagcaggaa 2220
   aatacattgc ataatacaga gaaaaattaa tcacaactga cctggatctc agatgctaga 2280
   aatagtagac aaagatgtta tttcccagtg caaataaaga aataaagata gcaagctg 2338

<210> 169
<211> 4775
<212> DNA
<213> Homo sapiens

<400> 169
cagtcgcgcg cggtgcagtc gggaggtgga ggcaccggct gcattgtttt cgggatcgag 60

gggtgagggc gctatggcac ccggctgcaa aactgagtta cgcagcgtga caaatggtca 120

gtctaaccaa ccaagtaatg aaggtgatgc catcaaagtt tttgtgcgaa ttcgtcctcc 180

tgcagaaaga tctgggtcag ctgatggaga gcagaactta tgcttatctg tgctgtcctc 240

cacgagtctc cggctgcact ccaaccctga gcccaagacc ttcacgtttg atcatgttgc 300

agatgtggat accactcagg aatctgtatt tgcaactgtg gctaaaagca ttgtggagtc 360

ttgcatgagc ggttataatg gtaccatctt tgcatatgga cagactggct cagggaagac 420

atttactatg atgggaccat ctgaatctga taatttttct cataacctga gaggagtaat 480

cccacgaagt tttgaatatt tgttttcctt aattgatcgt gaaaaagaaa aggctggagc 540

tggaaagagt ttcctttgta agtgttcctt tattgaaatc tacaacgagc agatatatga 600

tctactggac tctgcatcgg ctggactgta cttaagggag catatcaaga agggagtctt 660

tgttgttggt gcggtggagc aggtggtaac ctcagctgct gaagcctatc aggtgctgtc 720

tggaggatgg aggaatagac gtgtggcatc aacatcaatg aacagagaat cgtctaggtc 780

tcatgccgtc tttacaatta caatagagtc aatggagaaa agtaatgaga ttgtgaatat 840

acggacctcc ctactcaacc tggtggattt agcaggatct gaaaggcaaa aagataccca 900

tgcagaaggg atgagattga aggaagcagg taacataaat cgatcattga gctgcctggg 960

ccaagtgatt acagcacttg tcgacgtggg taatggaaaa cagagacatg tttgctacag 1020

agactccaaa cttaccttct tactacggga ttcccttgga ggtaatgcca aaacagccat 1080

aattgcaaat gttcatcctg gatccaggtg ttttggggaa accctatcaa cacttaactt 1140

tgctcaaaga gccaagctga ttaaaaacaa ggcagtagta aatgaagaca cccaaggaaa 1200

tgtgagccag ctccaagctg aagtgaagag gctcaaagaa caactggcgg agcttgcttc 1260

aggacagaca ccaccagaaa gcttcctgac cagagacaaa aagaagacta actatatgga 1320

gtatttccag gaagcaatgt tattctttaa gaaatctgaa caggaaaaga agtctctgat 1380

agaaaaagtt acccaattag aagacctcac cctcaaaaag gaaaaattta ttcaatctaa 1440

taaaatgatt gtgaaattcc gagaggatca aataatacgc ttggaaaagc tccacaagga 1500

atcccgggga ggttttctgc ctgaggagca ggatcgtttg ctctcagaat taaggaatga 1560

gattcaaact ctgcgagaac aaatagagca ccaccccaga gttgcaaagt atgctatgga 1620

aaatcattcc ctcagggagg agaatagaag actgagatta ttagagcctg tgaaaagagc 1680

tcaagaaatg gatgcccaga ccattgcaaa actagaaaaa gctttctctg aaataagtgg 1740

catggagaaa agtgacaaaa atcagcaagg attttcacct aaagctcaga aagagccatg 1800

tttgtttgca aacactgaga agttaaaagc acaactcctg caaattcaga cagagctgaa 1860

taattcaaag caagaatatg aagaattcaa agaacttact aggaaaaggc agctagaatt 1920

ggaatcagag cttcagtctt tgcaaaaagc gaaccttaat cttgaaaacc ttttggaagc 1980

aacaaaagcc tgcaagcggc aagaagtttc tcagctgaat aaaattcatg ctgaaacact 2040

taagattata actacaccaa ccaaggccta ccaacttcat tcccgaccag taccaaaatt 2100

aagccctgaa atgggaagct ttggctctct atacactcag aattctagca tattagataa 2160

tgatatatta aatgagccag ttcctcctga gatgaatgaa caagcttttg aggccatttc 2220

tgaagagctt agaacagtgc aggaacaaat gagtgctctt caagccaaac tggatgaaga 2280

agagcataaa aacctaaagc ttcagcagca tgttgacaaa ctggaacatc attctaccca 2340

aatgcaggag cttttctcat cagaaagaat tgattggacc aaacagcagg aagagcttct 2400

ctcacagttg aatgtccttg aaaagcagct tcaagagact caaactaaaa atgacttttt 2460

gaaaagtgag gtacatgacc tgcgagtagt ccttcattct gctgacaagg agctttcttc 2520

agtgaaattg gaatatagtt cattcaaaac gaatcaggag aaagaattca acaaactttc 2580

tgaaagacac atgcatgtac agcttcaatt agataatctc aggttagaaa acgaaaagct 2640

gcttgagagc aaagcctgcc tacaggattc ctatgacaac ttacaagaaa taatgaaatt 2700

tgagattgac caactttcaa gaaacctcca aaacttcaaa aaagaaaatg aaactctgaa 2760

atctgatctg aataatttga tggagcttct tgaggcagaa aaagaacgca ataacaaatt 2820

atcattacag tttgaagaag ataaagaaaa cagttctaaa gaaatcttaa aagttcttga 2880

ggctgtacgt caggagaaac agaaagagac ggccaagtgt gagcagcaga tggcaaaagt 2940

acagaaacta gaagagagct tgcttgctac tgaaaaagtg atcagttccc tggaaaagtc 3000

tagagattct gataagaaag ttgtagctga cctcatgaac cagatccagg agctaagaac 3060

atcggtctgt gagaaaacag aaactataga caccctgaaa caagaactga aggacataaa 3120

ttgcaaatac aactctgctt tggttgacag agaagagagc agagtgttga tcaagaagca 3180

ggaagtggat attctggatc tgaaagaaac ccttaggctg agaatacttt ctgaggacat 3240

agagagggat atgctctgtg aggacctggc tcatgccact gagcagctga acatgctcac 3300

agaggcctca aaaaaacact cggggctgct gcagtctgcc caggaagaac tgaccaagaa 3360

ggaagccctg attcaggaac ttcagcacaa gctaaaccaa aagaaagagg aagtagaaca 3420

gaagaagaat gaatataact tcaaaatgag gcaactagaa catgtgatgg attctgctgc 3480

tgaggatccc cagagtccta agacaccacc tcactttcaa acacatttgg caaaactcct 3540

ggaaacacaa gaacaagaga tagaagatgg aagagcctct aagacttctt tggaacacct 3600

tgtaacaaag ctaaatgaag acagagaagt caaaaatgct gaaatcctca gaatgaagga 3660

gcagttgcgt gaaatggaaa acctacgcct ggaaagtcag cagttaatag agaaaaactg 3720

gctcctgcaa ggtcagctgg atgatattaa aagacaaaag gaaaacagtg atcagaatca 3780

tccagataat caacagctga agaatgaaca agaagaaagt atcaaagaaa gacttgcaaa 3840

aagtaaaata gttgaagaaa tgctgaaaat gaaagcagac ctagaagaag tccaaagtgc 3900

cctttacaac aaagagatgg aatgccttag aatgactgat gaagtcgaac gaacccaaac 3960

tttggagtct aaagcattcc aggaaaaaga acaactgaga tcaaagctgg aagaaatgta 4020

tgaagaaaga gagagaacat cccaggagat ggaaatgtta aggaagcagg tggagtgtct 4080

tgctgaggaa aatggaaagt tggtaggtca ccaaaatttg catcagaaga ttcagtacgt 4140

agtgcgacta aagaaggaaa atgtcaggct tgctgaggag acagaaaagt tgcgtgccga 4200

aaatgtattt ttaaaagaaa agaaaagaag tgaatcttga ggattccggt cagctaccta 4260

ggcatcacct tgtttgaaga tgtttcttct cttttacaag taagacctac tcctggccac 4320

ttaggagagc tgaatttatg gaccttaatt attaaatgtt tataaggtgg tggtaaccac 4380

ctcaagtttc tgatgaacat tctgcatcca tatacaccct gtgacagtca gcagtctgct 4440

attaagtggc ctacttcaag gctttgaatc aacttaaggg aaaacctttt gtctttgtaa 4500

aaataaaagc ctgtagctaa ggtttacagt ggacattagc cagatcattt tcttcttaga 4560

ttatgccata atctcctttg attcttatgg aagttctaac aatatatggt ggttccaaca 4620

cctgcagtga gtttaatgac tgacttagta gcaggtacaa gaagcaaact tgttaatata 4680

gattattttt gtattcttac tttaggtatt ttacttgagc attttccatg actgtaaata 4740

aagccatttt ttaagataaa aaaaaaaaaa aaaaa 4775

<210> 170
<211> 1375
<212> DNA
<213> Homo sapiens

<400> 170
gtttgaaatc ggaaagttgg cggggctgcg ggagctgagc ctagagtccg gctgttggct 60

agagtgggcg cggatctggt gtggggaagg cggcgggact caggcctgcc tgcgaagcat 120

tgtcctacat aatggtagag gacgaactgg cacttttcga taaaagcata aatgaatttt 180

ggaataaatt caaaagtacg gacacctcct gtcagatggc gggactaaga gatacctaca 240

aggattccat caaagcattt gcagaaaagc tgtctgtgaa attaaaggaa gaagaacgaa 300

tggttgagat gtttctggaa tatcaaaatc agatcagcag gcaaaataag ctcattcaag 360

aaaaaaagga taacttgtta aaattgattg ctgaagtaaa aggcaaaaag caggaattgg 420

aagtactgac tgcaaatatc caggatctta aggaagaata ttctaggaag aaggaaacta 480

tttctactgc taataaagcg aatgcagaga ggttgaaaag gctgcagaaa tctgcagact 540

tgtataaaga tcgacttgga ctagaaattc gaaaaattta tggtgagaaa ttgcagttta 600

ttttcactaa tattgaccct aagaatcctg agagcccatt tatgttttcc ttacatctca 660

atgaagcaag ggactatgaa gtgtcagata gtgcccctca tcttgagggc ctagcagaat 720

ttcaagagaa tgtaaggaag accaacaatt tttcagcttt tcttgccaat gttcggaaag 780

cttttactgc cacggtttat aattaacata caaatagtgt atataaaaac ggtttatttt 840

tcttctctat tacatatctc tttttttctt gtttttatta ttattatact ttaagtttta 900

gggtacatgt gcacaatgtg caggtttgtt acatatgtat acatgtgcca tattggtgtg 960

ctgcacccat taactcgtca tttcattagg tatatctcct aatgctatcc ctcccccctc 1020

ccccaaccca caacagtccc cgttgtgtga tgttcccctt cctgtgtcca tgtgttctca 1080

ttgttcaatt cccacctagg agtgagaata tgtggtgttt ggttttttgt cctttcgata 1140

gtttgctgag aatgatggtt tccagcttca tccatgttcc tacaaaggac atgaactcat 1200

ccttttttat ggctgcatag tattccatgg tgtatatgtg ccacattttc ttaatccagt 1260

ctatcattgt tggacatttg ggttggttcc aagtctttgc tattgtgaat agtgccgaaa 1320

taaacatacg tgtgcatgtg tctttaaaaa aaaaaaaaaa aaaaaaaaaa aaaaa 1375

<210> 171
<211> 1225
<212> DNA
<213> Homo sapiens

<400> 171
ggcccacgct cgctcccgct gtcggactct gggcctggac cgcgcgtggc cggcgccaaa 60

agcgagaggc atgagcgcgt gcccgggcca gtgaactgct cggggatgga ctgcggctcg 120

gtcggcggcc agaggaccca gcgcctgccc ggccgccagc ggcttctctt cttgcccgtg 180

ggcctcagcg gaagaccggg cggcagcgaa acctcagcac gccgctgcct ctctgcgctc 240

tccgacggtc tgggggcgct gcgccctcgc gccccggcgg cccgcggcgg cgtgtcacgt 300

gcctcaccgc tgctcctcct cctgctagtg ccctccccgc gcttggccgc cgcagccccg 360

cgccggcagc tcggggactg ggagcgctcg cgcctcgggt atgccgcacc cccggccggg 420

cgcagcagcg cgtggaggtg ctcaccgggc gtcgccgctg ccgccggcgc ccttccccag 480

taccacggcc cggcgcctgc actcgtttcc tgcaggagag agctaagctt gtctgctgga 540

tccctgcagc tggagcgcaa aaggagagat ttcacctctt ctgggagcag gaaactctac 600

ttcgacactc atgccttagt gtgcttactg gaagacaatg ggtttgctac tcaacaagca 660

gaaatcattg tgtctgcatt ggtcaagatc ctggaggcca acatggacat cgtctacaaa 720

gatatggtca ccaagatgca gcaggaaatc acttttcagc aagtaatgtc tcagattgcg 780

aatgtgaaaa aggatatgat tattttggag aagagtgaat tttcagccct cagagcagaa 840

aatgagaaaa taaaactcga actacatcag ttaaaacaac aagtaatgga tgaagtgatc 900

aaagtccgaa cagataccaa attagacttc aacctagaaa agagcagagt aaaagaattg 960

tattcattga acgaaaagaa gctgctggaa ttgagaacag aaatagtggc attgcatgcc 1020

cagcaagatc gggcccttac ccagacagac aggaagatcg aaactgaggt tgctggcctc 1080

aaaaccatgc ttgagtcaca caagcttgat aatattaaat atttagcagg gtctatattt 1140

acgtgcctaa cagtagctct gggattttat cgcctgtgga tctaataaag tgtctattta 1200

aagtgaaaaa aaaaaaaaaa aaaaa 1225

<210> 172
<211> 1842
<212> DNA
<213> Homo sapiens

<400> 172
gcgcgccacg gcaggaaagg gcggtcctgc gctgtggccc acgctcgctc ccgctgtcgg 60

actctgggcc tggaccgcgc gtggccggcg ccaaaagcga gaggcatgag cgcgcgcccg 120

ggccagtgaa ctgctcgggg atggactgcg gctcggtcgg cggccagagg acccagcgcc 180

tgcccggccg ccagcggctt ctcttcttgc ccgtgggcct cagcggaaga ccgggcggca 240

gcgaaacctc agcacgccgc tgcccctctg cgctctccga cggtctgggg gcgctgcgcc 300

ctcgcgcccc ggcggcccgc ggcggcgtgt cacgtgcctc accgctgctc ctcctcctgc 360

tagtgccctc cccgcgcttg gccgccgcag ccccgcgccg gcagctcggg gactgggagc 420

gctcgcgcct cgggtatgcc gcacccccgg ccgggcgcag cggcgcgtgg aggtgctcac 480

cgggcgtcgc cgctgccgcc ggcgcccttc cccagtacca cggcccggcg cctgcactcg 540

tttcctgcag gagagagcta agcttgtctg ctggatccct gcagctggag cgcaaaagga 600

gagatttcac ctcttctggg agcaggaaac tctacttcga cactcatgcc ttagtgtgct 660

tactggaaga caatgaatct cactctttca tccaggctgg agtgcagtgg cacagtcttg 720

gcctactgca acccccacct cccggtttca agcgatcctc ccacctcatc ctcctgagta 780

gctgggatta caggcatgca ccaccacacc tggataattt ttctgtattt ttactagaga 840

cggggtttca ccatgttggc caggctggtc tcaaactgct gacctcaagt gatccaccca 900

ccttggcctc ctaaagtgct gggattacag gcgtgaacca ctgtgtccag ttgccaaacc 960

taattatgtt taaacatgaa ctcttttttt aattaaaatt ttttaaaatt ttatttacat 1020

taaaatggag atgggatcac tatgttgccc atgctggttc tgaactcctg ggctcaaaga 1080

tcctcccgcc ttggtctccc agagtgttag gattacaggc gtgagccacc acacccagcc 1140

agtgaactct tcttaataaa actttaaaat ttggcaaaag tttagtttga gtttttaaaa 1200

actaggaaga cagacgagaa aaaagaaaac agagtcagag ctaaacaatg ttaacatttt 1260

gggtttgcta ctcaacaagc agaaatcatt gtgtctgcat tggtcaagat cctggaggcc 1320

aacatggaca tcgtctacaa agatatggtc accaagatgc agcaggaaat cacttttcag 1380

caagtaatgt ctcagattgc gaatgtgaaa aaggatatga ttattttgga gaagagtgaa 1440

ttttcagccc tcagagcaga aaatgagaaa ataaaactcg aactacatca gttaaaacaa 1500

caagtaatgg atgaagtgat caaagtccga acagatacca aattagactt caacctagaa 1560

aagagcagag taaaagaatt gtattcattg aacgaaaaga agctgctgga attgagaaca 1620

gaaatagtgg cattgcatgc ccagcaagat cgggccctta cccagacaga caggaagatc 1680

gaaactgagg ttgctggcct caaaaccatg cttgagtcac acaagcttga taatattaaa 1740

tatttagcag ggtctatatt tacgtgccta acagtagctc tgggatttta tcgcctgtgg 1800

atctaataaa gtgtctattt aaagtgaaaa aaaaaaaaaa aa 1842

<210> 173
<211> 4682
<212> DNA
<213> Homo sapiens

<400> 173
cttacggcag tctcgcggga tttccccctc tcgcgggaat tatttgaacg ttcgagcggt 60

aaatactccc tggggctgtc atagaagact actcggagag cgctgcctct gggttggcgg 120

gctggcaggc tgtagccgag cgcgggcagg actcgtcccg gcagggttcc agagccatgg 180

gagcggaaag gaggctgctg tcgattaagg aggcctttcg gctggcgcag cagccgcacc 240

agaaccaggc gaagctggtg gtggcgctga gccgcaccta ccgcacgatg gatgataaga 300

cagtttttca tgaggagttc attcattacc ttaaatatgt tatggtggtc tataaacgtg 360

aaccagctgt ggagagggta atagaatttg cagcaaagtt tgttacctca tttcaccaat 420

cagatatgga agatgatgag gaagaggaag atggtggcct tttaaattat ttgtttactt 480

ttctcttaaa gtctcatgaa gcaaacagca atgcagtgag atttagagtg tgcctgctca 540

taaacaagct tttgggaagt atgccagaaa atgctcagat tgatgatgat gtgtttgata 600

aaattaataa agccatgctt attagattga aagataagat tccaaatgtg agaatacagg 660

cagttctggc gctttcacga cttcaggatc ccaaggatga tgaatgccca gtggttaatg 720

catatgctac tttgattgaa aatgattcaa atccagaagt tagacgggca gtgttatcat 780

gtattgcacc atcagcaaag actttgccaa aaattgtagg gcgcaccaag gatgtgaaag 840

aggctgtcag aaagctggct tatcaggttt tagctgaaaa ggttcatatg agagctatgt 900

ccattgctca gagagtaatg ctccttcaac aaggtcttaa tgacagatca gatgctgtga 960

aacaagctat gcagaagcat cttcttcaag gctggttacg gttctctgaa ggaaatatct 1020

tagagttgct ccatcggttg gatgtagaaa attcttctga agtggcagtc tctgttctca 1080

atgccttgtt ttcaataact cctctcagtg aactggtggg actctgtaaa aacaatgatg 1140

gcaggaaatt gattccagtg gaaacattaa ctcctgaaat tgctttgtat tggtgtgccc 1200

tttgtgaata tttgaaatca aaaggagatg aaggtgaaga atttttagag cagattttgc 1260

cagagcctgt agtatatgca gactatttat tgagttacat ccagagcatt ccagttgtta 1320

atgaagaaca cagaggtgat ttttcctata ttggaaattt gatgacaaaa gaattcatag 1380

gtcaacaatt gattctaatt attaagtctt tggataccag tgaagaagga ggaagaaaaa 1440

aactgctggc tgttttacag gagattctta ttttacccac aatcccaata tccctggttt 1500

cttttcttgt tgaaagacta ctccacatca ttatagatga taataagaga acacaaattg 1560

ttacagaaat tatctcagag attcgggcgc ccattgttac tgttggtgtt aataacgatc 1620

cagctgatgt aagaaagaaa gaactcaaga tggctgaaat aaaagttaag cttatcgaag 1680

ccaaagaagc tttggaaaat tgcattacct tacaggattt taatcgggca tcagaattaa 1740

aagaagaaat aaaagcatta gaagatgcca gaataaacct tttgaaagag acagagcaac 1800

ttgaaattaa agaagtccac atagagaaga atgatgctga aacattgcag aaatgtctta 1860

ttttatgcta tgaactgttg aagcagatgt ccatttcaac aggcttaagt gcaaccatga 1920

atggaatcat cgaatctttg attcttcctg gaataataag tattcatcct gttgtaagaa 1980

acctggctgt tttatgcttg ggatgctgtg gactacagaa tcaggatttt gcaaggaaac 2040

acttcgtatt actattgcag gttttgcaaa ttgatgatgt cacaataaaa ataagtgctt 2100

taaaggcaat ctttgaccaa ctgatgacgt tcgggattga accatttaaa actaaaaaaa 2160

tcaaaacact tcattgtgaa ggtacagaaa taaacagtga tgatgagcaa gaatcaaaag 2220

aagttgaaga gactgctaca gctaagaatg ttctgaaact cctttctgat ttcttagata 2280

gtgaggtatc tgaacttagg actggagctg cagaaggact agccaagctg atgttctctg 2340

ggcttttggt cagcagcagg attctttctc gtcttatttt gttatggtac aatcctgtga 2400

ctgaagagga tgttcaactt cgacattgcc taggcgtgtt cttccccgtg tttgcttatg 2460

caagcaggac taatcaggaa tgctttgaag aagcttttct tccaaccctg caaacactgg 2520

ccaatgcccc tgcatcttct cctttagctg aaattgatat cacaaatgtt gctgagttac 2580

ttgtagattt gacaagacca agtggattaa atcctcaggc caagacttcc caagattatc 2640

aggccttaac agtacatgac aatttggcta tgaaaatttg caatgagatc ttaacaagtc 2700

cgtgctcgcc agaaattcga gtctatacaa aagccttgag ttctttagaa ctcagtagcc 2760

atcttgcaaa agatcttctg gttctattga atgagattct ggagcaagta aaagatagga 2820

catgtctgag agctttggag aaaatcaaga ttcagttaga aaaaggaaat aaagaatttg 2880

gtgaccaagc tgaagcagca caggatgcca ccttgactac aactactttc caaaatgaag 2940

atgaaaagaa taaagaagta tatatgactc cactcagggg tgtaaaagca acccaagcat 3000

caaagtctac tcagctaaag actaacagag gacagagaaa agtgacagtt tcagctagga 3060

cgaacaggag gtgtcagact gctgaagccg actctgaaag tgatcatgaa gttccagaac 3120

cagaatcaga aatgaagatg agactaccaa gacgagccaa aaccgcagca ctagaaaaaa 3180

gtaaacttaa ccttgcccaa tttctcaatg aagatctaag ttaggaaaga cgatggaggt 3240

ggaatccttt aagattatgt ccagttattt gctttaataa agaagaagtt acccttgtca 3300

aaatcagaac aaacctgatg tctttctgaa gattttctgc tgtgcgcttc cacgttactt 3360

tggcctgtat taaagcagta gagcagcatc agttattata gtccagaaaa agtgtgcatc 3420

agtcagtcac acagatttat cacaatctga ggtgggccta ggaatctcat ttttaaatag 3480

tctctccaag tgattcttat gaactcttta tgtttaaaat catgtcatta tggaaaactt 3540

acaagtgtaa ctagctagta gcttgcattt gagaagctta tgacttagat gggcagaatc 3600

aacaaagatg aaaccgcctg aggacacatt taacaagtaa catttctagg gaaaatgaag 3660

gaagtaccac aaactggcta gaaaggagct tatcaatcac cagtgaggaa gaccagtata 3720

acgttcaaca acagttattt tgacaaaaac ttattttgtg attcctacag tgaaaacatt 3780

tttggtgata tctgcctggg aaatctctct tcctaaagta tttgtatatg ggagtccttg 3840

tttgtgaatg tttcctggat tagggaggtg tcaacataaa tgtattatta accatgaagc 3900

tgctcgctat atttttggca taacaaaata atatttattt actgtggata ataattctag 3960

tgggaatata atgtgacagg aacttctctt tatatacgct accaatttat gagcactatt 4020

cactgtcaat ttcatttctt gtcttttgaa attgacactt ggcctgactt acgaaacttg 4080

tactatatga aattggtcct cttttctgca atacccaacg aaacaccttt tctctttatt 4140

attcagaaat gtcctaacat ggatctgttt gttttaataa ttgtgctttt tttaggctta 4200

tcatctacta gaggccattt acttaaggtg aaattttaag atggagctaa agtaagatca 4260

ctggttttta gaaccaaatt gctatacata tgtgcctcat agaacttata aaaggagtca 4320

aagtttcaaa gcaagatagt tattaagcaa aaggaaaaat ggtaatgata gaaagtcagt 4380

taaaaataga tgattgttct tcattctgtt tgttggctct gtgttctcct gtgcttcaga 4440

ttccttatgt gttgttgttt taaagacaat ttgcaggggg ttgggagaag gactgaaaag 4500

gtacattaag tgtgctgtaa ggaaaagtct tagaaacata ataagctaaa atcccattca 4560

cacatggcca ggctatccaa aaagaaagga gccatgttct catgtggttt accataccaa 4620

agcttgcttt ctctggcatg ggaaaaataa atttaagcac caaaaaaaaa aaaaaaagaa 4680

aa 4682

<210> 174
<211> 3632
<212> DNA
<213> Homo sapiens

<400> 174
gtagtctttt ttttgcagaa tgggccaagg ggacagagac ttcactcggg agccagaacc 60

tgttttcaac atgggtattt taagaagaga cggagaggct gtcactgcag tggtcactgt 120

cctggaaaag taaccgttgc acggcccaga agtggctctg tggcggccaa tgggagccga 180

agcctgcgag gatgttggga attgtagttc tggcgtcggt cggtggccga gatcccgggc 240
acgctggctc tggtccacct tctccaatcc ctgcctgctg ggagaggacg atctcttgag 300
aaaggaaaga cttctgtgct cccgagaact tcctatcagg tcctggctgc agggaaacaa 360
gctgggcttt ttataattaa ggttggaaga agtcaccaca ggcagcagaa ctccatcttg 420
agatgaaata acatctacct ggacctctgg cagaatttca aggcacacac tgggctgact 480
ctggcgccat gatgttgcct tatccttcag cactgggaga tcaatactgg gaagagattt 540
tgcttccaaa gaatggggaa aatgtagaga ctatgaagaa attgacccaa aatcataaag 600
cgaaaggctt gccttctaat gatactgact gcccccagaa aaaggaggga aaggcccaaa 660
tagtggtacc agttacattc agggatgtga ctgtgatctt cacagaagca gaatggaaga 720
gactgagtcc agagcagagg aatctataca aagaagtgat gctggagaat tacaggaatc 780
ttctctcatt ggcagaacca aagccagaaa tctacacttg ttcctcctgc cttctggcct 840
tctcctgtca gcagttcctc agtcaacatg tacttcagat cttcctgggc ttatgtgcag 900
aaaatcactt ccatccaggg aattctagcc cagggcattg gaaacagcag gggcagcagt 960
attcccatgt aagctgttgg tttgaaaatg cagaaggtca ggagagagga ggtggctcca 1020
aaccctggtc tgcaaggaca gaggagagag aaacctcaag ggcattcccc agcccactcc 1080
aaagacagtc agcaagtcct agaaaaggca acatggtggt agaaacagag cccagctcag 1140
cccaaagacc aaaccctgtg cagctagaca aaggcttgaa ggaattagaa accttgagat 1200
ttggagcaat caactgtaga gagtatgaac cggaccataa cctggaatca aactttatta 1260
caaacccgag gaccctctta gggaagaagc cctacatttg cagtgattgt gggcgaagct 1320
ttaaagatag atcaaccctc atcagacacc atcgtataca ctcgatggag aagccttatg 1380
tgtgcagtga gtgcgggcga ggttttagcc agaagtccaa cctcagcaga caccagagaa 1440
cacattcaga agagaagcct tatttgtgca gggagtgtgg gcaaagcttt agaagtaagt 1500
ccatcctcaa tagacatcag tggactcact cagaggagaa gccctatgtt tgcagcgagt 1560
gtgggcgagg ctttagcgag aagtcatcct tcatcagaca ccagaggaca cactccggtg 1620
agaaacccta tgtgtgcctg gagtgtggac gaagcttttg tgataagtca accctcagaa 1680
aacaccagag gatacactca ggggagaagc cttatgtttg cagggagtgt gggcgaggct 1740
ttagccagaa ctcagatctc atcaaacacc agaggacaca cttggatgag aagccttatg 1800
tttgcaggga gtgtgggcga ggcttttgtg acaagtcaac cctcatcata cacgagcgga 1860
cgcactctgg agagaagcct tatgtgtgtg gtgagtgtgg ccgaggcttt agtcggaaat 1920
cactcctcct tgtccaccag aggacacact caggggagaa gcattatgtc tgcagggagt 1980
gtaggcgagg ttttagccag aagtcaaatc tcatcagaca ccagaggacg cactcaaatg 2040
agaagcctta tatttgcagg gaatgtgggc gaggcttttg tgacaagtca accctcattg 2100
tacatgagag gacacactca ggagagaagc cttacgtgtg cagtgagtgt ggccgaggct 2160
ttagccggaa atcactcctc cttgtccacc agaggacaca ctcaggggag aagcattatg 2220
tttgtaggga gtgtgggcga ggctttagtc ataagtcaaa tctcatcaga caccagagga 2280

cacactgacg ggagaaacct gtgtatgcag gggtcatgaa caagacctga gtgaccagtc 2340

aagcctcatg ttaccccaga gagacacatg gggagtagac cctgtgtaca cagattgtga 2400

gtgaagttcc agagatgtgt cagcccttat caggcatggg agggacacgt tcaggagagg 2460

agccttatga gtatagagta cgggcaactg tagccatcag tcagccttga gcatgcacaa 2520

aaggacacac ttaggagaga agtttatgtg tagggactgt gggaaggctt tagcaataat 2580

caacatttac cagacatcca atgacagcct caggggaaag cacccttgtc tggggagtgt 2640

tggggagcat cagtaaaaga atggacactc aggcacagag tggccctcag gaaggaggtc 2700

tttgtttgta ggatgtatgg gcaaagcttt tgtgatcaca caccacaggg agaatctgca 2760

tgtggggaca ctgtggagct ctgcccagat gaccttttca ggggtaacac cccagctgct 2820

tgagagaaca gtgttgctgc tggcagagat gcattccaga gatgcactcc gctctggaac 2880

tcactctcag ccacagggag ctgcatgcac cacaggggca atgcaccttt gcaggggtac 2940

cttctggccc caacccttga ctcaacgggg acaactccag aaggtcattc cagatccaga 3000

gatccccatc gaactgaagg atcactgggt tgcagacaca ttgcaggtca gcttcttcct 3060

ctgcccagtc ctgccctcac tccccagtga atcctcaatt ttctgtctcg ttgtctgtcc 3120

agataattga ttctaagaca tgttaggtat ataaggagtg tagataaggc ttcagccatg 3180

agtcaccccc cagtaagccc cagagtatat tgaatagaaa ttctgcatgt gtggggagaa 3240

tggacaagga cttaggaaaa agtcctcatc aaagaacagc ttttttggga caagctttac 3300

atgggtgggg agggaaaatg tgaaacacat tagcaataag ttaaacctca tcttgtacta 3360

aaggaggaca cactcaggga gaagacctct gtgggcaggg cttgtgggtg gagcttcatc 3420

ccgatgtcac tcctcaactg acttaggagg acagtcttgc taccccaagt cactacctca 3480

ctcacctctg agggattttc aggaaatgtc ttgactcccc catgtactct gtatgtgagc 3540

gaagatggca gtaactgtta aataagcatt cttttctact tcttggaatc agatgaaata 3600

aaaaagcagg ctttatttaa gtaatcaaaa aa 3632

<210> 175
<211> 2547
<212> DNA
<213> Homo sapiens

<400> 175
   ggattggctg ttcggagcgg cgaggggctt ggcgggagct tccagtcgct cgagagcgga 60
   gagcggcacc atggccccgc gggggcggcg gcggccgcgg cctcacaggt ctgagggcgc 120
   aagacgttca aagaacactt tagaaagaac acattccatg aaagataaag ctggtcaaaa 180
   gtgcaagcct attgacgtgt tcgactttcc tgataattct gatgtctcaa gcattggcag 240
   gctgggtgaa aatgagaaag atgaagaaac ttatgagacc tttgatcctc ctttacatag 300
   cacagctata tatgctgatg aagaagaatt ctccaaacat tgtggactgt ctctctcttc 360
   aactcctcca ggaaaagaag caaaaagaag ttcagacact tctggaaatg aagcaagtga 420
   aatcgaatct gtaaaaatta gtgcaaaaaa gccaggaaga aagctcaggc ccattagtga 480
   tgactctgaa agcattgaag aaagtgatac aaggagaaaa gttaaatcag cagagaaaat 540
   aagtacacaa cgtcatgagg ttattcgaac cacagcgtct tcagaacttt cagagaaacc 600
   agctgagtct gtcacttcta aaaagacagg accccttagt gcccagccct ctgttgaaaa 660
   agagaacttg gcaatagaaa gtcaatcgaa aactcagaaa aaagggaaga tatctcatga 720
   caaaaggaag aaatcaagaa gtaaagccat aggctcagat acttctgaca ttgtgcacat 780
   ttggtgtcca gaaggaatga aaaccagtga catcaaggag ttgaatattg ttttgcctga 840
   atttgagaaa acccacctag agcatcaaca aagaatagaa tctaaagttt gtaaggcagc 900
   catcgccaca ttttatgtta atgttaaaga acaattcatc aaaatgctta aagaaagcca 960
   gatgttgaca aatctgaaaa ggaagaatgc taagatgatt tcagatatcg aaaagaaaag 1020
   gcagcgtatg attgaagtcc aggatgaact gcttcggtta gagccacagc tgaaacaact 1080
   acaaacaaaa tatgatgaac ttaaagagag aaagtcttcc cttaggaatg cagcatattt 1140
   cttatctaat ttaaaacagc tttatcaaga ttattcagat gttcaagctc aagaaccaaa 1200
   cgtaaaggaa acgtatgatt catccagcct tccagctctg ttatttaaag caagaacact 1260
   tctgggagcc gaaagccatc tgcgaaatat caaccatcag ttagagaagc tccttgacca 1320
   gggatgagaa gagcagtcta ctaaaatgtg cctataggaa gactagtctc atgctgttac 1380
   cttctgaaac tgtaccttta taaatcaatt gttttgcaaa gaagttatgg cctacttaga 1440
   atctaaaatt tgttattcaa attaaatggc tgtgaacaat gttaaatagc atcagtttgt 1500
   ccaatagttt taaaggccat aatcatcttt tctggttaat atcttgagta attttaaaat 1560
   gttgacacct taatcggtcc caggtatgag ctataataaa cttgtaaaat taagttgatg 1620
   tgaacataat tttgattaat taataaggcg atttctcctg aatttacacc aaagctaatt 1680
   tttaatgaaa ttggtttaca gaagtaaaaa acaaaaattg gaaagcaaag taataaaact 1740
   tagtttatat aaacaggttg aatgatatat ttatcaaatc tcacagacat caggcaaatt 1800
   atagcctggt gacaaaagtg ttcatagtga attagttact cttgtaatac ttctataatt 1860
   agttcatcag gaatttcatc cacttcactg ttataactga gaagactgtt ctctgcagct 1920
   tcagctaatt cagcatcttc agtagcttct aaaaaataag catcatcaat gccattatcc 1980
   cagacagcat cagcagatgc acctgttgac agcctgctag gtgatggttt atgaggattc 2040
   tgggtttcat tgctcctagt ttcatctgct tcatctgttg taaactcttc ttcctttatt 2100
   tcagtggtga agggatagag agtgggatag gaaaatattt actcaggata tgtgatttaa 2160
   ccttatactc tatgttgaag taaggtatta agtgacagat actaaagtga atatgcagga 2220
   ggaatgctgt ctccgatatc tcaccgtggg aatgagtgca ctgattcaaa cgttgctgca 2280
   ctgaagctca gacacacttg aaactccaaa tttgaaatta cctacagttc tgtgcacata 2340
   cttttcaata ctccccgacg gaagagcaag ggtggattta attttttaac aagtggacag 2400
   tccagctgaa gacaaatcag aagataaatt tgctatcttg acaatggact tagtacccat 2460
   gctttaaatt ttaaagtatt tagcaaatcg taaacatgga ttgaaaaaag attaaaaaca 2520
   gttgccaaaa tataaaaaaa aaaaaaa 2547

<210>  176
<211>  3273
<212>  DNA
<213>  Homo sapiens

<400>  176
actggagtct ctgagcagcc attgaagggg aaggaactgc gggtgtgtgt gtgtatgtgt      60

gtgtgtatgt gtgtgcgcgc gtgcgtgcgt gtgtgtgcgc gcgctagtgt gtggacaagg     120

aggtgggggc agctgagtta gagtcccaac tcttggactc catttgctat tctcttcttt     180

ctcccccaca cctatctggt ggtggtagtg ggcgtttata tttgcgttcc ttttcattca     240

tttctaaatc tcttaaaaat tttgggttgg gggtattggg gaaggcagga aagggaaaag     300

gagagtagta gctgaagagc aagaggagga catggagatg aagaagaaga ttaacctgga     360

gttaaggaac agatccccgg aggaggtgac agagttagtc cttgataatt gcctgtgtgt     420

caatggggaa attgaaggcc tgaatgatac tttcaaagaa ctagaatttc tgagtatggc     480

taatgtggaa ctaagttcgc tggcccggct tcccagctta aataaacttc gaaaattgga     540

gcttagtgat aatataattt ctggaggctt ggaagtcctg gcagagaaat gtccaaatct     600

tacctacctc aatctgagtg gaaacaaaat aaaagatctc agtacagtag aagctctgca     660

aaatcttaaa aatttgaaaa gtcttgacct gtttaactgt gagatcacaa acctggaaga     720

ttatagagaa agtatttttg aactactgca gcaaatcaca tacttagatg gatttgatca     780

ggaggataat gaagcgccgg actctgaaga ggaggatgat gaggatggcg atgaagatga     840

tgaagaggaa gaggaaaatg aagctggtcc accggaagga tatgaggaag aggaggagga     900

agaggaagag gaggatgagg atgaggatga agatgaagat gaagcaggtt cagagttggg     960

agagggagaa gaggaagtgg gcctctcata cttaatgaaa gaagaaattc aggatgaaga    1020

agatgatgat gactatgttg aagaagggga agaagaggaa gaagaggaag aaggaggtct    1080

tcgaggggag aagaggaaac gagatgctga agacgatgga gaggaagaag atgactagat    1140

cattctaaga ccagattctc taatgtttct gggtgtgcaa tagagtgatc acatctttgt    1200

ttcttcatgt acgatagcta tccctacaga agataatgtg taacttttta taggaaaagt    1260

gtggttttac tatttttgcc ttatcattcc aaataagaac tagtctgtta atgatcatat    1320

tgtatgtaga gaaaaatttt cattgactcc cattgtggaa ttccctagca atttatttag    1380

acttaatttt ttaaattcaa gcttactgta ttagtcattt ttagcccata attaaaacat    1440

gatcactttt acacaggtgt agtatggtgc atttcattcc ttatttataa attaactgaa    1500

attacagttt gctataatat aaaatgacaa tagtctcttg agtggtaagt tggttatttt    1560

tttagaggtg atccaggaat ctttagtttg aaggcagtta cctttttttt tttttttttt    1620

ttgactaaga gtgtttggtt gcttttttgt cacaagtaac ttggaaaata gaagcagaat    1680

agtaaaggtt ctattcagca acatagttca tggattttgt ggaggttcta ttcagtaata    1740

tggttcatgg atttagtggt gactgataag attttatttt tgaaggaaaa attgcttata    1800

ctaagtccag agacatgcag gtgagccctt ttgtcaggct gcaaatcatg acatgccgat    1860

ggttgtttat tttgttttta ggtgtgcatt ctttttcttc ttagcaattc ctttatgatc    1920

accttccctt cttgtttcac tccctcccgc tctctcaaaa ggaacttggg aaacttgtga 1980

aacccaggaa aacctttagt cttatacctc aactaccttt cagtcctgtc tgggttttaa 2040

ataagtgaag tagaagaaat tgagtatttt ctgacataag aatatattat caatacagtt 2100

ttatgcagta agctctcctt accataaatg tttcttggtt gacaacatct aagacaatat 2160

tagtgggatg aagaaagaaa agcaggggtg cttttggaag cagtgttagt gttcctcaaa 2220

agtcggaaca attgcctgtt gatatattaa taagacatta aagtcaaatt ttaatgttgg 2280

cctctcaaat gatttggata ccactctgca aagtatttct aacctttaat tcccagtttt 2340

aaaacagata taataatagc atttaattgg aatatactag gcagctggaa aagtatttga 2400

aactaaattg acattaaaat taagatttgt tttcaagtgg atgtccatta aaagtagaaa 2460

aatatttggg ataagtgagt gtgtatttcc ttacatggct actaaataaa atataatgag 2520

tatacaagta tatctcctct tttgctatgg aggctccatg ttcaaggcaa tggcttttta 2580

aatcttggct atctaaaatt ttttcccttt gttttgaata tttgtaagtt tttaagaagt 2640

tagtgtcagc aaattaattg aagttatgct tctatactgg gacatattta aatactgagt 2700

atagtactgc tgctactgct tctacaatgt aaaatgtatg acttggtgtt ttaaagtaaa 2760

aattatgatg ttacttgtgg agaaactaaa aatgttgtac aactgaccga aagaaaaccc 2820

ttggggataa gtttagtgag gggattggaa tccccaaaaa gataacattt ttcttctgct 2880

tttaaaaact gaaattccct gttctagttc ctaacaattc tcattacata ctatgccaga 2940

ttacaaaata cttattttta aaatgaaatc tatatattga ctttcttatc aatcatctta 3000

ctgtgcaatc aaaattagag tactttggtt tgaaaacaac acttagagcc tccagataac 3060

ttttaagact tatttagctt tgtgggtggt attttcatgc aaataagtaa gggtgggttt 3120

tatattttgt agaagttttc ggtcctattt taatgctttt gtatggcagt atgtatatat 3180

tgtgttaagt tcctcaagaa tctccttaaa aactttgaag ttaatacttt tgtgcaactg 3240

tgttttgaat aaagccatga cagtgttaaa aac 3273

<210> 177
<211> 3379
<212> DNA
<213> Homo sapiens

<400> 177
ctggcctggg cctgaagtga gtgagaggca catgaagaga agtattcaag tatttataca 60

gataggaatc aagataatca acaatgtctg tcactgagga agacctgtgc caccatatga 120

aagtagtagt tcgtgtacgt ccggaaaaca ctaaagaaaa agcagctgga tttcataaag 180

tggttcatgt tgtggataaa catatcctag tttttgatcc caaacaagaa gaagtcagtt 240

ttttccatgg aaagaaaact acaaatcaaa atgttataaa gaaacaaaat aaggatctta 300

aatttgtatt tgatgctgtt tttgatgaaa cgtcaactca gtcagaagtt tttgaacaca 360

ctactaagcc aattcttcgt agttttttga atggatataa ttgcacagta cttgcctatg 420

gtgccactgg tgctgggaag acccacacta tgctaggatc agctgatgaa cctggagtga 480

tgtatctaac aatgttacac ctttacaaat gcatggatga gattaaagaa gagaaaatat 540

gtagtactgc agtttcatat ctggaggtat ataatgaaca gattcgtgat ctcttagtaa 600

attcagggcc acttgctgtc cgggaagata cccaaaaagg ggtggtcgtt catggactta 660

ctttacacca gcccaaatcc tcagaagaaa ttttacattt attggataat ggaaacaaaa 720

acaggacaca acatcccact gatatgaatg ccacatcttc tcgttctcat gctgttttcc 780

aaatttactt gcgacaacaa gacaaaacag caagtatcaa tcaaaatgtc cgtattgcca 840

agatgtcact cattgacctg gcaggatctg agcgagcaag tacttccggt gctaagggga 900

cccgatttgt agaaggcaca aatattaata gatcactttt agctcttggg aatgtcatca 960

atgccttagc agattcaaag agaaagaatc agcatatccc ttacagaaat agtaagctta 1020

ctcgcttgtt aaaggattct cttggaggaa actgtcaaac tataatgata gctgctgtta 1080

gtccttcctc tgtattctac gatgacacat ataacactct taagtatgct aaccgggcaa 1140

aggacattaa atcttctttg aagagcaatg ttcttaatgt caataatcat ataactcaat 1200

atgtaaagat ctgtaatgag cagaaggcag agattttatt gttaaaagaa aaactaaaag 1260

cctatgaaga acagaaagcc ttcactaatg aaaatgacca agcaaagtta atgatttcaa 1320

accctcagga aaaagaaatc gaaaggtttc aagaaatcct gaactgcttg ttccagaatc 1380

gagaagaaat tagacaagaa tatctgaagt tggaaatgtt acttaaagaa aatgaactta 1440

aatcattcta ccaacaacag tgccataaac aaatagaaat gatgtgttct gaagacaaag 1500

tagaaaaggc cactggaaaa cgagatcata gacttgcaat gttgaaaact cgtcgctcct 1560

acctggagaa aaggagggag gaggaattga agcaatttga tgagaatact aattggctcc 1620

atcgtgtcga aaaagaaatg ggactcttaa gtcaaaacgg tcatattcca aaggaactca 1680

agaaagatct tcattgtcac catttgcacc tccagaacaa agatttgaaa gcacaaatta 1740

gacatatgat ggatctagct tgtcttcagg aacagcaaca caggcagact gaagcagtat 1800

tgaatgcttt acttccaacc ctaagaaaac aatattgcac attaaaagaa gccggcctgt 1860

caaatgctgc ttttgaatct gacttcaaag agatcgaaca tttggtagag aggaaaaaag 1920

tggtagtttg ggctgaccaa actggcgaac aaccaaagca aaacgatcta cccgggattt 1980

ctgttcttat gaccttttca caacttggac cagttcagcc tattccttgt tgctcatctt 2040

caggtggaac taatctggtt aagattccta cagaaaaaag aactcggaga aaactaatgc 2100

catctccctt gaaaggacag catactctaa agtctccacc atctcaaagt gtgcagctca 2160

atgattctct tagcaaagaa cttcagccta ttgtatatac accagaagac tgtagaaaag 2220

cttttcaaaa tccgtctaca gtaaccttaa tgaaaccatc atcatttact acaagttttc 2280

aggctatcag ctcaaacata aacagtgata attgtctgaa aatgttgtgt gaagtagcta 2340

tccctcataa tagaagaaaa gaatgtggac aggaggactt ggactctaca tttactatat 2400

gtgaagacat caagagctcg aagtgtaaat tacccgaaca agaatcacta ccaaatgata 2460

acaaagacat tttacaacgg cttgatcctt cttcattctc aactaagcat tctatgcctg 2520

taccaagcat ggtgccatcc tacatggcaa tgactactgc tgccaaaagg aaacggaaat 2580

taacaagttc tacatcaaac agttcgttaa ctgcagacgt aaattctgga tttgccaaac 2640

gtgttcgaca agataattca agtgagaagc acttacaaga aaacaaacca acaatggaac 2700

ataaaagaaa catctgtaaa ataaatccaa gcatggttag aaaatttgga agaaatattt 2760

caaaaggaaa tctaagataa atcacttcaa aaccaagcaa aatgaagttg atcaaatctg 2820

cttttcaaag tttatccaat accctttcaa aaatatattt aaaatctttg aaagaagacc 2880

catcttaaag ctaagtttac ccaagtactt tcagcaagca gaaaaatgaa actctttgtt 2940

ttcttctttt gtgttctaaa aaaataaaat ttcaaaagaa aaggttgtct tttaagtttt 3000

ttaaatattt gttgcctttt aaaatccctg agtgtaagtt accatggtgg cagcttagtt 3060

ttactatgcc acaacaagtt gactaggaca ttttagtaaa tggtagtgag ttaaattatc 3120

tttattattt tttaaaaata agaatttaga agtggtaaaa ttatggccca agatgtattt 3180

ggttctctat tatgttttga tacattattt taatcatata tatgactttc cttttcaaaa 3240

atactttaat gtacaagtgt aaatatatgt gcccataaaa tcattgtaaa tattatttag 3300

tcatcacaaa taaaatattg tcccttgcta cttgatatat taaagatgta gattttaaag 3360

tgaaaaaaaa aaaaaaaaa 3379

<210> 178
<211> 1139
<212> DNA
<213> Homo sapiens

<400> 178
ccacgagctg ttgtgcatcc agaggtggaa ttggggcccg gcattccctc ctcgtcccgg 60

gctggccctt gcccccaccc tgcaactcct ggttgagatg ggctcagcca agagcgtccc 120

agtcacacca gcgcggcctc cgccgcacaa caagcatctg gctcgagtgg cggacccccg 180

ttcacctagt gctggcatcc tgcgcactcc catccaggtg gagagctctc cacagccagg 240

cctaccagca ggggagcaac tggagggtct taaacatgcc caggactcag atccccgctc 300

tcctactctt ggtattgcac ggacacctat gaagaccagc agtggagacc ccccaagccc 360

actggtgaaa cagctgagtg aagtatttga aactgaagac tctaaatcaa atcttccccc 420

agagcctgtt ctgcccccag aggcaccttt atcttctgaa ttggacttgc ctctgggtac 480

ccagttatct gttgaggaac agatgccacc ttggaaccag actgagttcc cctccaaaca 540

ggtgttttcc aaggaggaag caagacagcc cacagaaacc cctgtggcca gccagagctc 600

cgacaagccc tcaagggacc ctgagactcc cagatcttca ggttctatgc gcaatagatg 660

gaaaccaaac agcagcaagg tactagggag atcccccctc accatcctgc aggatgacaa 720

ctcccctggc accctgacac tacgacaggg taagcggcct tcacccctaa gtgaaaatgt 780

tagtgaacta aaggaaggag ccattcttgg aactggacga cttctgaaaa ctggaggacg 840

agcatgggag caaggccagg accatgacaa ggaaaatcag cactttccct tggtggagag 900

ctaggccctg catggcccca gcaatgcagt cacccagggc ctggtgatat ctgtgtcctc 960

tcaccccttc tttcccaggg atactgagga atggcttgtt ttcttagact cctcctcagc 1020

taccaaactg ggactcacag ctttattggg ctttctttgt gtcttgtgtg tttcttttat 1080

attaaaggaa gtaattttaa atgttacttt aaaaaggtaa aaaaaaaaaa aaaaaaaaa 1139

<210> 179
<211> 1621
<212> DNA
<213> Homo sapiens

<400> 179
agctgattcc cgggactagg ttgcgggaga aagcctgttg cgtggaagat aaggcggcgc 60

gggaagtgga cacagggtgg gctggagatc taactggact ctcgctcctg ctggctggac 120

atggaggatt tggaggaaga tgtaaggttt attgtggatg agaccttgga ctttgggggg 180

ctgtcaccat ctgacagccg tgaggaggaa gacataacag tgttggtgac tccagagaaa 240

ccacttcgac ggggcctctc ccaccgaagt gacccaaatg cagtggcacc tgccccccag 300

ggtgtgaggc tcagcctagg ccccctcagt ccagagaagc tggaggagat cctcgatgag 360

gccaaccggc tggccgctca gctggagcag tgtgccctgc aggatcggga gagcgcaggc 420

gagggcctgg ggcctcgccg agtgaagccc agtcctcggc gggagacctt tgtgctgaag 480

gatagtcctg tccgagacct gctgcccact gtgaactctt tgacgcggag caccccctcc 540

ccaagcagcc tgacgcctcg actccggagt aatgatagga aggggtcagt cagggctctc 600

cgggctacat ctggaaagag gccctccaac atgaagaggg agtcacccac ttgcaatctg 660

ttccctgcat ccaaaagccc agcatcttct cctcttaccc gatcgactcc cccagtccgg 720

gggagagccg ggcccagtgg gagagcagca gccagtgagg agaccagagc agccaagttg 780

cgggcctgcc agccgaatgc cactcaccag ccggagtgtg ccacctggca gaggtgccct 840

acctccggat tctctgtcaa ctcgaaaagg gcttccaaga ccaagcactg caggacacag 900

agtgcgggaa agtggacaca aggttcctgt ttcccagcga ctaaatcttc ctgtcatggg 960

tgccactcgc agcaatctgc agccccccag gaaagtggca gtcccaggac ctaccaggta 1020

aagagatcag gacagcaagc aagacttcag tagcaaacca ctacagtcag tacctggact 1080

cgcctctacc cagcagaccc tgactccagc agattctggc ccagggacag gaggaagaga 1140

tgccaccagg gctggtctcc caggagtaga gaccatggga aatggggtgg attaggattg 1200

agctggagaa gacttaaact ctctgggttg aaagaagatt aggggaaaag aggtcacctt 1260

ccagcagtga aatgaacaaa tagaagatga gaagtacagg caagtggttt gtctttatcc 1320

acccccactg ttgtggtcag ccccagagaa ttttatcttc ttccttggca ttggttcact 1380

ggacatttcc acgtgagcgg cctccgtagc taacctccct gccctctgag gagccatctt 1440

cctgaatcgc attctctact ggactctggc ctgcttggag aggtggcagc aggcacctgg 1500

tcttcagaaa ttgtttcctg tgaattctgt gactcctaat aggccagttt gtgataagct 1560

tactctatga gtcttcattt ttctaaaata aagtgaatgt atttttatat tctctgtaaa 1620

a 1621

<210> 180
<211> 1828
<212> DNA
<213> Homo sapiens

<400>  180
agctgattcc cgggactagg ttgcgggaga aagcctgttg cgtggaagat aaggcggcgc      60

gggaagtgga cacagggtgg gctggaggta tctaactgga ctctcgctcc tgctggctgg     120

acatggagga tttggaggaa gatgtaaggt ttattgtgga tgagaccttg gactttgggg     180

ggctgtcacc atctgacagc cgtgaggagg aagacataac agtgttggtg actccagaga     240

aaccacttcg acggggcctc tcccaccgaa gtgacccaaa tgcagtggca cctgcccccc     300

agggtgtgag gctcagccta ggccccctca gtccagagaa gctggaggag atcctcgatg     360

aggccaaccg gctggccgct cagctggagc agtgtgccct gcaggatcgg gagagcgcag     420

gcgagggcct ggggcctcgc cgagtgaagc ccagtcctcg gcgggagacc tttgtgctga     480

aggatagtcc tgtccgagac ctgctgccca ctgtgaactc tttgacgcgg agcaccccct     540

ccccaagcag cctgacgcct cgactccgga gtaatgatag gaaggggtca gtcagggctc     600

tccgggctac atctggaaag aggccctcca acatgaagag ggagtcaccc acttgcaatc     660

tgttccctgc atccaaaagc ccagcatctt ctcctcttac ccgatcgact cccccagtcc     720

gggggagagc cgggcccagt gggagagcag cagccagtga ggagaccaga gcagccaagt     780

tgcgggtgag tgggagtggg gagtttgtgg gattgaccct gaaatttctc cacccctctc     840

caccaggccc acccaccccc atcagatccg tcctggcccc acagccttct accagcaact     900

ctcaacgcct gccccggccg cagggagcag ctgctaaatc ttccagtcaa ctgcccattc     960

cctcggccat ccccaggcct gccagccgaa tgccactcac cagccggagt gtgccacctg    1020

gcagaggtgc cctacctccg gattctctgt caactcgaaa agggcttcca agaccaagca    1080

ctgcaggaca cagagtgcgg gaaagtggac acaaggttcc tgtttcccag cgactaaatc    1140

ttcctgtcat gggtgccact cgcagcaatc tgcagccccc caggaaagtg gcagtcccag    1200

gacctaccag gtaaagagat caggacagca agcaagactt cagtagcaaa ccactacagt    1260

cagtacctgg actcgcctct acccagcaga ccctgactcc agcagattct ggcccaggga    1320

caggaggaag agatgccacc agggctggtc tcccaggagt agagaccatg ggaaatgggg    1380

tggattagga ttgagctgga gaagacttaa actctctggg ttgaaagaag attaggggaa    1440

aagaggtcac cttccagcag tgaaatgaac aaatagaaga tgagaagtac aggcaagtgg    1500

tttgtcttta tccaccccca ctgttgtggt cagccccaga gaattttatc ttcttccttg    1560

gcattggttc actggacatt tccacgtgag cggcctccgt agctaacctc cctgccctct    1620

gaggagccat cttcctgaat cgcattctct actggactct ggcctgcttg gagaggtggc    1680

agcaggcacc tggtcttcag aaattgtttc ctgtgaattc tgtgactcct aataggccag    1740

tttgtgataa gcttactcta tgagtcttca tttttctaaa ataaagtgaa tgtattttta    1800

tattctctgt aaaaaaaaaa aaaaaaaa                                        1828

<210>  181
<211>  1738
<212>  DNA
<213>  Homo sapiens

<400>  181
agctgattcc cgggactagg ttgcgggaga aagcctgttg cgtggaagat aaggcggcgc      60

gggaagtgga cacagggtgg gctggaggta tctaactgga ctctcgctcc tgctggctgg     120

acatggagga tttggaggaa gatgtaaggt ttattgtgga tgagaccttg gactttgggg     180

ggctgtcacc atctgacagc cgtgaggagg aagacataac agtgttggtg actccagaga     240

aaccacttcg acggggcctc tcccaccgaa gtgacccaaa tgcagtggca cctgcccccc     300

agggtgtgag gctcagccta ggccccctca gtccagagaa gctggaggag atcctcgatg     360

aggccaaccg gctggccgct cagctggagc agtgtgccct gcaggatcgg gagagcgcag     420

gcgagggcct ggggcctcgc cgagtgaagc ccagtcctcg gcgggagacc tttgtgctga     480

aggatagtcc tgtccgagac ctgctgccca ctgtgaactc tttgacgcgg agcaccccct     540

ccccaagcag cctgacgcct cgactccgga gtaatgatag gaaggggtca gtcagggctc     600

tccgggctac atctggaaag aggccctcca acatgaagag ggagtcaccc acttgcaatc     660

tgttccctgc atccaaaagc ccagcatctt ctcctcttac ccgatcgact cccccagtcc     720

gggggagagc cgggcccagt gggagagcag cagccagccc acccaccccc atcagatccg     780

tcctggcccc acagccttct accagcaact ctcaacgcct gccccggccg cagggagcag     840

ctgctaaatc ttccagtcaa ctgcccattc cctcggccat ccccaggcct gccagccgaa     900

tgccactcac cagccggagt gtgccacctg gcagaggtgc cctacctccg gattctctgt     960

caactcgaaa agggcttcca agaccaagca ctgcaggaca cagagtgcgg gaaagtggac    1020

acaaggttcc tgtttcccag cgactaaatc ttcctgtcat gggtgccact cgcagcaatc    1080

tgcagccccc caggaaagtg gcagtcccag gacctaccag gtaaagagat caggacagca    1140

agcaagactt cagtagcaaa ccactacagt cagtacctgg actcgcctct acccagcaga    1200

ccctgactcc agcagattct ggcccaggga caggaggaag agatgccacc agggctggtc    1260

tcccaggagt agagaccatg ggaaatgggg tggattagga ttgagctgga gaagacttaa    1320

actctctggg ttgaaagaag attaggggaa aagaggtcac cttccagcag tgaaatgaac    1380

aaatagaaga tgagaagtac aggcaagtgg tttgtcttta tccaccccca ctgttgtggt    1440

cagccccaga gaattttatc ttcttccttg gcattggttc actggacatt tccacgtgag    1500

cggcctccgt agctaacctc cctgccctct gaggagccat cttcctgaat cgcattctct    1560

actggactct ggcctgcttg gagaggtggc agcaggcacc tggtcttcag aaattgtttc    1620

ctgtgaattc tgtgactcct aataggccag tttgtgataa gcttactcta tgagtcttca    1680

tttttctaaa ataaagtgaa tgtattttta tattctctgt aaaaaaaaaa aaaaaaaa      1738

<210>  182
<211>  1722
<212>  DNA
<213>  Homo sapiens

<400>  182
agctgattcc cgggactagg ttgcgggaga aagcctgttg cgtggaagat aaggcggcgc      60

gggaagtgga cacagggtgg gctggagatc taactggact ctcgctcctg ctggctggac     120

atggaggatt tggaggaaga tgtaaggttt attgtggatg agaccttgga ctttgggggg 180

ctgtcaccat ctgacagccg tgaggaggaa gacataacag tgttggtgac tccagagaaa 240

ccacttcgac ggggcctctc ccaccgaagt gacccaaatg cagtggcacc tgccccccag 300

ggtgtgaggc tcagcctagg ccccctcagt ccagagaagc tggaggagat cctcgatgag 360

gccaaccggc tggccgctca gctggagcag tgtgccctgc aggatcggga gagcgcaggc 420

gagggcctgg ggcctcgccg agtgaagccc agtcctcggc gggagacctt tgtgctgaag 480

gatagtcctg tccgagacct gctgcccact gtgaactctt tgacgcggag caccccctcc 540

ccaagcagcc tgacgcctcg actccggagt aatgatagga aggggtcagt cagggctctc 600

cgggctacat ctggaaagag gccctccaac atgaagaggg agtcacccac ttgcaatctg 660

ttccctgcat ccaaaagccc agcatcttct cctcttaccc gatcgactcc cccagtccgg 720

gggagagccg ggcccagtgg gagagcagca gccagcccac ccacccccat cagatccgtc 780

ctggccccac agccttctac cagcaactct caacgcctgc cccggccgca gggagcagct 840

gctaaatctt ccagtcaact gcccattccc tcggccatcc ccaggcctgc cagccgaatg 900

ccactcacca gccggagtgt gccacctggc agaggtgccc tacctccgga ttctctgtca 960

actcgaaaag ggcttccaag accaagcact gcaggacaca gagtgcggga aagtggacac 1020

aaggttcctg tttcccagcg actaaatctt cctgtcatgg gtgccactcg cagcaatctg 1080

cagcccccca ggaaagtggc agtcccagga cctaccaggt aaagagatca ggacagcaag 1140

caagacttca gtagcaaacc actacagtca gtacctggac tcgcctctac ccagcagacc 1200

ctgactccag cagattctgg cccagggaca ggaggaagag atgccaccag ggctggtctc 1260

ccaggagtag agaccatggg aaatggggtg gattaggatt gagctggaga agacttaaac 1320

tctctgggtt gaaagaagat taggggaaaa gaggtcacct tccagcagtg aaatgaacaa 1380

atagaagatg agaagtacag gcaagtggtt tgtctttatc cacccccact gttgtggtca 1440

gccccagaga attttatctt cttccttggc attggttcac tggacatttc cacgtgagcg 1500

gcctccgtag ctaacctccc tgccctctga ggagccatct tcctgaatcg cattctctac 1560

tggactctgg cctgcttgga gaggtggcag caggcacctg gtcttcagaa attgtttcct 1620

gtgaattctg tgactcctaa taggccagtt tgtgataagc ttactctatg agtcttcatt 1680

tttctaaaat aaagtgaatg tatttttata ttctctgtaa aa 1722

<210> 183
<211> 2510
<212> DNA
<213> Homo sapiens

<400> 183
   gcacctcgct gctccagcct ctggggcgca ttccaacctt ccagcctgcg acctgcggag 60
   aaaaaaaatt acttattttc ttgccccata cataccttga ggcgagcaaa aaaattaaat 120
   tttaaccatg agggaaatcg tgcacatcca ggctggtcag tgtggcaacc agatcggtgc 180
   caagttctgg gaggtgatca gtgatgaaca tggcatcgac cccaccggca cctaccacgg 240
   ggacagcgac ctgcagctgg accgcatctc tgtgtactac aatgaagcca caggtggcaa 300
   atatgttcct cgtgccatcc tggtggatct agaacctggg accatggact ctgttcgctc 360
   aggtcctttt ggccagatct ttagaccaga caactttgta tttggtcagt ctggggcagg 420
   taacaactgg gccaaaggcc actacacaga gggcgccgag ctggttgatt ctgtcctgga 480
   tgtggtacgg aaggaggcag agagctgtga ctgcctgcag ggcttccagc tgacccactc 540
   actgggcggg ggcacaggct ctggaatggg cactctcctt atcagcaaga tccgagaaga 600
   ataccctgat cgcatcatga ataccttcag tgtggtgcct tcacccaaag tgtctgacac 660
   cgtggtcgag ccctacaatg ccaccctctc cgtccatcag ttggtagaga atactgatga 720
   gacctattgc attgacaacg aggccctcta tgatatctgc ttccgcactc tgaagctgac 780
   cacaccaacc tacggggatc tgaaccacct tgtctcagcc accatgagtg gtgtcaccac 840
   ctgcctccgt ttccctggcc agctcaatgc tgacctccgc aagttggcag tcaacatggt 900
   ccccttccca cgtctccatt tctttatgcc tggctttgcc cctctcacca gccgtggaag 960
   ccagcagtat cgagctctca cagtgccgga actcacccag caggtcttcg atgccaagaa 1020
   catgatggct gcctgtgacc cccgccacgg ccgatacctc accgtggctg ctgtcttccg 1080
   tggtcggatg tccatgaagg aggtcgatga gcagatgctt aacgtgcaga acaagaacag 1140
   cagctacttt gtggaatgga tccccaacaa tgtcaagaca gccgtctgtg acatcccacc 1200
   tcgtggcctc aagatggcag tcaccttcat tggcaatagc acagccatcc aggagctctt 1260
   caagcgcatc tcggagcagt tcactgccat gttccgccgg aaggccttcc tccactggta 1320
   cacaggcgag ggcatggacg agatggagtt caccgaggct gagagcaaca tgaacgacct 1380
   cgtctctgag tatcagcagt accaggatgc caccgcagaa gaggaggagg atttcggtga 1440
   ggaggccgaa gaggaggcct aaggcagagc ccccatcacc tcaggcttct cagttccctt 1500
   agccgtctta ctcaactgcc cctttcctct ccctcagaat ttgtgtttgc tgcctctatc 1560
   ttgttttttg ttttttcttc tggggggggt ctagaacagt gcctggcaca tagtaggcgc 1620
   tcaataaata cttgtttgtt gaatgtctcc tctctctttc cactctggga aacctaggtt 1680
   tctgccattc tgggtgaccc tgtatttctt tctggtgccc attccatttg tccagttaat 1740
   acttcctctt aaaaatctcc aagaagctgg gtctccagat cccatttaga accaaccagg 1800
   tgctgaaaac acatgtagat aatggccatc atcctaagcc caaagtagaa aatggtagaa 1860
   ggtagtgggt agaagtcact atataaggaa ggggatggga ttttccattc taaaagtttt 1920
   ggagagggaa atccaggcta ttaaagtcac taaatttcta agtatgtcca tttcccatct 1980
   cagcttcaag ggaggtgtca gcagtattat ctccactttc aatctccctc caagctctac 2040
   tctggaggag tctgtcccac tctgtcaagt ggaatccttc cctttccaac tctacctccc 2100
   tcactcagct cctttcccct gatcagagaa agggatcaag ggggttggga ggggggaaag 2160
   agaccagcct tggtccctaa gcctccagaa acgtcttctt aatccccacc ttttcttact 2220
   cccaaaaaag aatgaacacc cctgactctg gagtggtgta tactgccaca tcagtgtttg 2280
   agtcagtccc cagaggagag gggaaccctc ctccatcttt tttgcaacat ctcatttctt 2340
   ccttttgctg ttgcttcccc cctcacacac ttggttttgt tctatcctac atttgagatt 2400
   tctattttat gttgaacttg ctgctttttt tcatattgaa aagatgacat cgccccaaga 2460
   gccaaaaata aatgggaatt gaaaaaaaaa aaaaaaaaaa aaaaaaaaaa 2510

<210> 184
<211> 10434
<212> DNA
<213> Homo sapiens

<400> 184
atggcgaacc ggcgagtggg gcgaggctgc tgggaagtga gcccgaccga gcggaggccg 60

cccgcggggc tgcggggccc cgcggccgag gaggaggcgt cttccccgcc ggtcctgtct 120

ctcagccact tctgcaggtc tcctttcctt tgcttcgggg acgttctcct gggagcctca 180

cggacgctgt ctctggccct agacaaccct aacgaggagg tggcagaagt gaagatctcc 240

cacttcccgg ccgcggacct gggcttcagt gtgtcgcagc gctgtttcgt gttgcagcct 300

aaagagaaaa ttgttatttc tgttaactgg acaccactca aagaaggccg agtaagagag 360

attatgacat ttcttgtaaa tgatgttctg aaacaccaag ctatattact aggaaatgca 420

gaagagcaga aaaagaaaaa gaggagtctt tgggatacca ttaaaaagaa gaaaatttca 480

gcctctacaa gtcacaacag aagggtttca aatattcaga atgttaataa aacatttagt 540

gtttcccaaa aagttgacag agttaggagc ccactacaag cttgtgaaaa cttggctatg 600

aatgaaggcg gtcccccaac agaaaacaat tctttaatac ttgaagaaaa taaaataccc 660

atatcaccta ttagccctgc tttcaatgaa tgccatggtg caacttgctt gccactctct 720

gtacgtcgat ctactaccta ctcatctctt catgcatcag aaaataggga actattaaat 780

gtacacagtg ccaacgtttc aaaagtttct tttaatgaga aagctgtaac tgaaacttcc 840

tttaattctg taaatgttaa tggccaaaga ggagagaata gtaaacttag tcttaccccc 900

aactgttctt caactttgaa cattacacaa agccaaatac attttctaag tccagattct 960

tttgtaaata atagtcatgg agctaataat gaactagaat tagtaacatg tctttcatca 1020

gatatgttta tgaaagataa ttcacagcct gtgcatttgg aatcaacaat tgcacatgaa 1080

atttatcaga aaattttaag tccagattct ttcataaaag ataattatgg actaaatcag 1140

gatctagaat cagagtcagt taatcctatt ttatccccta atcaattttt aaaagataac 1200

atggcatata tgtgtacatc tcagcaaaca tgtaaagtac cattatcaaa tgaaaattct 1260

caagtcccac agtctcctga agattggaga aaaagtgaag tttcgccacg tattcctgaa 1320

tgtcagggtt caaaatctcc caaagctatt tttgaagaac tagtagaaat gaagtcaaat 1380

tactacagtt ttataaaaca aaataatcct aaattttctg cagttcagga tatttctagt 1440

catagccaca ataaacaacc taagagacgt ccaatacttt ctgccactgt tactaaaagg 1500

aaggccacct gtaccagaga aaaccaaact gagattaata aaccaaaagc aaaaagatgt 1560

ctcaacagtg cagtgggtga acatgaaaaa gtaataaata atcaaaagga aaaagaagat 1620

tttcattctt atcttccaat tatagatcca atattaagta aatctaagag ttataaaaac 1680

gaggtaacac cctcttcgac aacagcttca gttgctcgga aaagaaagag cgatggaagc 1740

atggaagatg caaatgtgag agttgcaatt acagaacata cagaagtgcg agaaatcaaa 1800
agaatccatt tttctccctc agagcctaaa acatcagctg ttaagaaaac aaaaaatgtg 1860
acaacaccca tctcaaaacg tattagcaac agagagaaat taaacctgaa gaagaaaact 1920
gatttatcaa tattcagaac tccaatttct aaaacaaaca aaaggacaaa acccattatc 1980
gctgtggcac agtccagttt gaccttcata aaaccattaa aaacagatat tcccagacac 2040
ccgatgccat ttgctgcaaa aaacatgttt tatgatgaac gctggaagga aaagcaggaa 2100
cagggcttca cttggtggtt aaattttata ttaacccctg atgacttcac tgtaaaaaca 2160
aatatttctg aagtaaatgc tgctactctt cttttgggaa tagagaatca acataaaata 2220
agtgttccta gagcacctac aaaagaggaa atgtctctca gagcttatac tgctcggtgt 2280
aggttaaaca gactacgtcg tgcagcatgc cgtttgttta cttctgaaaa aatggttaaa 2340
gctattaaaa agcttgaaat tgaaattgaa gctaggcggt taattgttcg aaaagataga 2400
cacctatgga aagatgtggg agaacgtcag aaagtcctga attggctgtt gtcctacaat 2460
cctttgtggc ttcgaattgg tctagagaca acttatggag aactcatatc tttggaagat 2520
aacagtgatg tcacagggtt ggctatgttt attctgaatc gcctactttg gaatcctgat 2580
atagcagctg agtatagaca ccccactgtt cctcacctgt atagagatgg tcatgaagaa 2640
gctttgtcca agtttacatt gaaaaagtta ttgttgttgg tctgttttct tgattatgct 2700
aaaatttcca gactcattga tcatgatcct tgtctcttct gtaaagatgc cgaattcaag 2760
gctagtaaag aaatcctttt ggctttttca cgagatttcc taagtggtga aggtgacctt 2820
tcccgtcacc ttggcttatt gggattacct gttaaccatg ttcagacacc atttgatgaa 2880
tttgattttg ccgttacaaa tcttgccgta gacttgcaat gtggagtgcg ccttgtgcga 2940
accatggaac ttctcacaca gaactgggac ctctcaaaga aactcaggat tccggcaata 3000
agtcgtcttc aaaagatgca caatgttgac attgttcttc aagttcttaa atcacgagga 3060
attgaattaa gtgatgagca tggaaataca attctatcta aggatattgt ggataggcac 3120
agagaaaaaa ctctcaggtt gctttggaaa atagcgtttg cttttcaggt ggatatttcc 3180
cttaacttag atcaattaaa ggaagaaatt gcctttctaa aacacacaaa gagtataaag 3240
aaaacaatat ctctactatc atgccattct gatgatctta ttaataagaa aaaaggcaaa 3300
agggatagtg gttcctttga acaatatagt gaaaacataa agttattgat ggattgggta 3360
aatgctgttt gtgccttcta taataaaaag gtggagaatt ttacagtgtc tttctcagac 3420
ggccgtgtgt tatgttacct gatccaccat taccatcctt gctatgtgcc atttgacgct 3480
atatgtcagc gtactactca aactgtggaa tgtacgcaaa ctggttcagt ggtattaaat 3540
tcatcatctg aatctgatga cagttctctg gatatgtcac ttaaagcatt tgatcatgaa 3600
aatacttcag agctatacaa agagctccta gaaaatgaaa agaaaaattt tcacttggtt 3660
aggtctgcag ttagagacct tggtggaata cctgctatga ttaatcattc agatatgtca 3720
aatacaattc cagatgaaaa ggtggttatt acctatttgt catttctttg tgcaaggctt 3780
ttggatcttc gtaaagaaat aagagctgct cgactcatac aaacaacatg gagaaaatat 3840

aaactaaaaa cagatctcaa acgccatcag gagagagaga aagctgcaag aattattcaa 3900

ttggctgtaa tcaattttct agcaaaacaa agattgagaa aaagagttaa tgcagcactc 3960

gtcattcaga aatattggcg aagagtctta gcacagagaa aattattaat gttaaaaaag 4020

gaaaagctgg aaaaagttca aaataaagca gcatcactta ttcagggata ttggagaaga 4080

tattccacta gacaaagatt tctgaaattg aaatattatt caatcatcct gcaatctagg 4140

ataagaatga taattgctgt tacatcttat aaacgatatc tttgggctac agttacaatt 4200

cagaggcatt ggcgtgctta tttaagaaga aaacaagatc aacaaagata tgaaatgcta 4260

aaatcatcaa ctcttataat ccaatctatg ttcagaaaat ggaagcaacg taaaatgcaa 4320

tcacaagtaa aagctacagt aatattgcaa agagctttta gagaatggca tttaagaaaa 4380

caagctaaag aagaaaattc tgctattatc atacaatcat ggtatagaat gcataaagaa 4440

ttacggaagt atatttatat tagatcttgt gttgttatca ttcagaaaag atttcggtgc 4500

tttcaagccc aaaagttata taaaagaaga aaagagtcca tactaaccat ccagaagtac 4560

tacaaagcat atctgaaagg aaagattgag cgcaccaact atttgcagaa acgagctgca 4620

gccattcaat tacaagctgc ttttaggaga ctgaaagctc ataatttatg tagacaaatt 4680

agagctgctt gtgttattca gtcatactgg agaatgagac aagacagagt tcgattttta 4740

aaccttaaga agactattat caaatttcag gcacatgtaa gaaaacatca acaacgacag 4800

aaatataaga agatgaagaa agcagctgtt ataattcaga ctcatttccg agcttatatt 4860

tttgccatga aagttctagc atcttaccag aaaacacgct ctgctgtcat tgtgctgcag 4920

tctgcatata gagggatgca agccaggaaa atgtatattc acatcctcac atctgttata 4980

aagattcaat catattatcg tgcttatgtt tctaaaaagg aatttttgag cctaaaaaat 5040

gctacaataa aattgcagtc aactgttaag atgaaacaaa cacgtaaaca atatttgcat 5100

ttaagagcag ctgcactatt tatccagcaa tgttaccgtt ccaaaaaaat agctgcacaa 5160

aagagagaag agtatatgca gatgcgggaa tcttgtatca aactgcaagc atttgttaga 5220

ggataccttg tccgaaagca gatgaggtta caaagaaaag ctgttatttc actacagtct 5280

tatttcagaa tgagaaaggc tcggcagtat tatctgaaaa tgtataaagc aattattgtc 5340

attcagaatt actatcatgc atacaaagca caggtcaatc agaggaagaa cttcttgcaa 5400

gtcaaaaaag cagctacttg cttgcaagca gcttacagag gttataaagt acgccagcta 5460

atcaaacaac aatctatagc tgctcttaaa attcagtctg cttttagagg ctataataaa 5520

agggtaaaat atcaatctgt gcttcaatct ataataaaga ttcagagatg gtacagggcg 5580

tacaagactc ttcatgatac aagaacacat tttttgaaga caaaggcagc tgtgatttcc 5640

ctccagtctg cttatcgtgg ctggaaggtt cggaaacaga ttagaaggga acatcaagct 5700

gccttgaaga ttcagtctgc ttttagaatg gccaaggccc agaaacagtt tagattgttt 5760

aaaacagcag cattagtcat ccagcaaaat ttcagagcat ggactgcagg aaggaagcaa 5820

tgtatggagt atattgaact ccgtcatgcg gtactggtgc ttcaatctat gtggaaggga 5880

aaaacactga gaagacagct tcaaaggcaa cataaatgtg ctatcatcat acagtcatac 5940

tatagaatgc atgtgcaaca aaagaagtgg aaaatcatga aaaaagctgc tcttctgatt 6000

caaaagtatt atagggctta cagtattgga agagaacaga atcatttata tttgaaaaca 6060

aaagcagctg tagtaacttt acagtcagct tatcgtggta tgaaagtgag aaaaagaata 6120

aaggattgca acaaagcagc agtcactata cagtctaaat acagagctta caaaaccaaa 6180

aagaaatatg caacctatag agcttcagct attataattc agagatggta tcgaggtatt 6240

aaaattacaa accatcagca taaggagtat cttaatttga agaagacagc aattaaaatc 6300

caatctgttt atagaggtat tagagttaga agacatattc aacacatgca cagggcagcc 6360

acttttatta aagccatgtt taaaatgcat cagtcaagaa taagttacca tacaatgaga 6420

aaagcagcta ttgttattca agtaagatgt agagcatatt atcaaggtaa aatgcagcgt 6480

gaaaagtacc tgacaatttt gaaagctgtt aaagtccttc aggcaagttt tagaggagta 6540

agagttagac ggactcttag aaagatgcag actgcagcaa cactcattca gtcaaactac 6600

agaagataca gacagcaaac atactttaat aagttaaaga aaataacaaa aacagtacag 6660

caaagatact gggcaatgaa agaaagaaac atacaatttc aaaggtataa caaactgagg 6720

cattctgtaa tatacattca ggctattttt aggggaaaga aagctagaag acatttaaaa 6780

atgatgcata tagccgcaac tctcattcag aggagattta gaactctaat gatgagaaga 6840

agattcctct ctctcaagaa aactgctatt ttgattcaga gaaaatatcg ggcacatctt 6900

tgtacaaagc atcacttaca gttccttcag gtacaaaatg cagttattaa aatccagtca 6960

tcatacagaa gatggatgat aaggaaaagg atgcgagaga tgcacagggc tgctactttc 7020

atccagtcta ctttcagaat gcacagatta catatgagat atcgagcttt gaaacaggcc 7080

tccgttgtga tccaacagca ataccaagca aatagagctg caaaactgca gaggcagcat 7140

tatctcagac aaagacactc tgctgtgatc cttcaggctg cattcagggg tatgaaaact 7200

agaagacatt tgaagagtat gcattcctct gcaaccctta ttcagagtag gtttagatca 7260

ttactggtga ggagaagatt catttccctc aaaaaagcta ctatttttgt tcagaggaaa 7320

tatcgagcca ccatttgtgc caaacataaa ttgtaccaat tcttgcactt aagaaaggca 7380

gccattacaa tacagtcatc ttacagaaga ctgatggtaa agaagaagtt acaagaaatg 7440

caaagggctg cagttctcat tcaggctact ttcaggatgc acagaacata tattacattt 7500

cagacttgga aacatgcttc aattctaatt cagcaacatt atcgaacata tagagctgca 7560

aaattgcaaa gagaaaatta tatcagacaa tggcattctg ctgtggttat tcaggctgca 7620

tataaaggaa tgaaagcaag acaactttta agggaaaaac acaaagcttc tatcgtaata 7680

caaagcacct acagaatgta taggcagtat tgtttctacc aaaagcttca gtgggctaca 7740

aaaatcatac aagaaaaata tagagcaaat aaaaagaaac agaaagtatt tcaacacaat 7800

gaacttaaga aagagacttg tgttcaggca ggttttcagg acatgaacat aaaaaaacag 7860

attcaggaac agcaccaggc tgccattatt attcagaagc attgtaaagc ctttaaaata 7920

aggaagcatt atctccacct tagagcaaca gtagtttcta ttcaaagaag atacagaaaa 7980

ctaactgcag tgcgtaccca agcagttatt tgtatacagt cttattacag aggctttaaa 8040

gtacgaaagg atattcaaaa tatgcaccgg gctgccacac taattcagtc attctatcga 8100

atgcacaggg ccaaagttga ttatgaaaca aagaaaactg caattgtggt tatacagaat 8160

tattataggt tgtatgttag agtaaaaaca gaaagaaaaa actttttagc agttcagaaa 8220

tctgtacgaa ctattcaggc tgcttttaga ggcatgaaag ttagacaaaa attgaaaaat 8280

gtatcagagg aaaagatggc agccattgtt aaccaatctg cactctgctg ttacagaagt 8340

aaaactcagt atgaagctgt tcaaagtgaa ggtgttatga ttcaagagtg gtataaagct 8400

tctggccttg cttgttcaca ggaagcagag tatcattctc aaagtagggc tgcagtaaca 8460

attcaaaaag ctttttgtag aatggtcaca agaaaactgg aaacacagaa atgtgctgcc 8520

ctacggattc agttcttcct tcagatggct gtgtatcgga gaagatttgt tcagcagaaa 8580

agagctgcta tcactttaca gcattatttt aggacgtggc aaaccagaaa acagttttta 8640

ctatatagaa aagcagcagt ggttttacaa aatcactaca gagcatttct gtctgcaaaa 8700

catcaaagac aagtctattt acagatcaga agcagtgtta tcattattca agctagaagt 8760

aaaggattta tacagaaacg gaagtttcag gaaattaaaa atagcaccat aaaaattcag 8820

gctatgtgga ggagatatag agccaagaaa tatttatgta aagtgaaagc tgcctgcaag 8880

attcaagcct ggtatagatg ttggagagca cacaaagaat atctagctat attaaaagct 8940

gttaaaatta ttcaaggttg cttctatacc aaactagaga gaacacggtt tttgaatgtg 9000

agagcatcag caattatcat tcagagaaaa tggagagcta tacttcctgc aaagatagct 9060

catgaacact tcttaatgat aaaaagacat cgagctgctt gtttgatcca agcacattat 9120

agaggatata aaggaaggca ggtctttctt cggcagaaat ctgctgcttt gatcatacaa 9180

aaatatatac gagccaggga ggctggaaag catgaaagga taaaatatat tgaatttaaa 9240

aaatctacag ttatcctaca agcactggtg cgtggttggc tagtacgaaa aagattttta 9300

gaacagagag ccaaaattcg acttcttcac ttcactgcag ctgcatatta tcacctgaat 9360

gctgttagaa ttcaaagagc ctataaactt tacctggctg tgaagaatgc taacaagcag 9420

gttaattcag tcatctgtat tcagagatgg tttcgagcaa gattacaaga aaagagattt 9480

attcagaaat atcatagcat caaaaagatt gagcatgaag gtcaagaatg tctgagccag 9540

cgaaataggg ctgcatcagt aatacagaaa gcagtgcgcc attttctcct ccgtaaaaag 9600

caggaaaaat tcactagtgg aatcattaaa attcaggcat tatggagagg ctattcttgg 9660

aggaagaaaa atgattgtac aaaaattaaa gctatacgac taagtcttca agttgttaat 9720

agggagattc gagaagaaaa caaactctac aaaagaactg cacttgcact tcattacctt 9780

ttgacatata agcacctttc tgccattctt gaggccttaa aacacctaga ggtagttact 9840

agattgtctc cactttgttg tgagaacatg gcccagagtg gagcaatttc taaaatattt 9900

gttttgatcc gaagttgtaa tcgcagtatt ccttgtatgg aagtcatcag atatgctgtg 9960

caagtcttgc ttaatgtatc taagtatgag aaaactactt cagcagttta tgatgtagaa 10020

aattgtatag atatactatt ggagcttttg cagatatacc gagaaaagcc tggtaataaa 10080

gttgcagaca aaggcggaag catttttaca aaaacttgtt gtttgttggc tattttactg 10140

aagacaacaa atagagcctc tgatgtacga agtaggtcca aagttgttga ccgtatttac 10200

agtctctaca aacttacagc tcataaacat aaaatgaata ctgaaagaat actttacaag 10260

caaaagaaga attcttctat aagcattcct tttatcccag aaacacctgt aaggaccaga 10320

atagtttcaa gacttaagcc agattgggtt ttgagaagag ataacatgga agaaatcaca 10380

aatcccctgc aagctattca aatggtgatg gatacgcttg gcattcctta ttag 10434

<210> 185
<211> 429
<212> PRT
<213> Homo sapiens

<400> 185

Met Ser Gly Gly Val Tyr Gly Gly Asp Glu Val Gly Ala Leu Val Phe
1               5                   10                  15

Asp Ile Gly Ser Tyr Thr Val Arg Ala Gly Tyr Ala Gly Glu Asp Cys
            20                  25                  30

Pro Lys Val Asp Phe Pro Thr Ala Ile Gly Met Val Val Glu Arg Asp
        35                  40                  45

Asp Gly Ser Thr Leu Met Glu Ile Asp Gly Asp Lys Gly Lys Gln Gly
    50                  55                  60

Gly Pro Thr Tyr Tyr Ile Asp Thr Asn Ala Leu Arg Val Pro Arg Glu
65                  70                  75                  80

Asn Met Glu Ala Ile Ser Pro Leu Lys Asn Gly Met Val Glu Asp Trp
                85                  90                  95

Asp Ser Phe Gln Ala Ile Leu Asp His Thr Tyr Lys Met His Val Lys
            100                 105                 110

Ser Glu Ala Ser Leu His Pro Val Leu Met Ser Glu Ala Pro Trp Asn
        115                 120                 125

Thr Arg Ala Lys Arg Glu Lys Leu Thr Glu Leu Met Phe Glu His Tyr
    130                 135                 140

Asn Ile Pro Ala Phe Phe Leu Cys Lys Thr Ala Val Leu Thr Ala Phe
145                 150                 155                 160

Ala Asn Gly Arg Ser Thr Gly Leu Ile Leu Asp Ser Gly Ala Thr His
                165                 170                 175

Thr Thr Ala Ile Pro Val His Asp Gly Tyr Val Leu Gln Gln Gly Ile
            180                 185                 190

Val Lys Ser Pro Leu Ala Gly Asp Phe Ile Thr Met Gln Cys Arg Glu
        195                 200                 205

Leu Phe Gln Glu Met Asn Ile Glu Leu Val Pro Pro Tyr Met Ile Ala
    210                 215                 220

Ser Lys Glu Ala Val Arg Glu Gly Ser Pro Ala Asn Trp Lys Arg Lys
225                 230                 235                 240

Glu Lys Leu Pro Gln Val Thr Arg Ser Trp His Asn Tyr Met Cys Asn
                245                 250                 255

Cys Val Ile Gln Asp Phe Gln Ala Ser Val Leu Gln Val Ser Asp Ser
            260                 265                 270

Thr Tyr Asp Glu Gln Val Ala Ala Gln Met Pro Thr Val His Tyr Glu
        275                 280                 285

Phe Pro Asn Gly Tyr Asn Cys Asp Phe Gly Ala Glu Arg Leu Lys Ile
    290                 295                 300

Pro Glu Gly Leu Phe Asp Pro Ser Asn Val Lys Gly Leu Ser Gly Asn
305                 310                 315                 320

Thr Met Leu Gly Val Ser His Val Val Thr Thr Ser Val Gly Met Cys
                325                 330                 335

Asp Ile Asp Ile Arg Pro Gly Leu Tyr Gly Ser Val Ile Val Ala Gly
            340                 345                 350

Gly Asn Thr Leu Ile Gln Ser Phe Thr Asp Arg Leu Asn Arg Glu Leu
        355                 360                 365

Ser Gln Lys Thr Pro Pro Ser Met Arg Leu Lys Leu Ile Ala Asn Asn
    370                 375                 380

Thr Thr Val Glu Arg Arg Phe Ser Ser Trp Ile Gly Gly Ser Ile Leu
385                 390                 395                 400

Ala Ser Leu Gly Thr Phe Gln Gln Met Trp Ile Ser Lys Gln Glu Tyr
                405                 410                 415

Glu Glu Gly Gly Lys Gln Cys Val Glu Arg Lys Cys Pro
            420                 425

<210>  186
<211>  387
<212>  PRT
<213>  Homo sapiens

<400>  186

Met Val Val Glu Arg Asp Asp Gly Ser Thr Leu Met Glu Ile Asp Gly
1               5                   10                  15

Asp Lys Gly Lys Gln Gly Gly Pro Thr Tyr Tyr Ile Asp Thr Asn Ala
            20                  25                  30

Leu Arg Val Pro Arg Glu Asn Met Glu Ala Ile Ser Pro Leu Lys Asn
        35                  40                  45

Gly Met Val Glu Asp Trp Asp Ser Phe Gln Ala Ile Leu Asp His Thr
    50                  55                  60

Tyr Lys Met His Val Lys Ser Glu Ala Ser Leu His Pro Val Leu Met
65                  70                  75                  80

Ser Glu Ala Pro Trp Asn Thr Arg Ala Lys Arg Glu Lys Leu Thr Glu
                85                  90                  95

Leu Met Phe Glu His Tyr Asn Ile Pro Ala Phe Phe Leu Cys Lys Thr
            100                 105                 110

Ala Val Leu Thr Ala Phe Ala Asn Gly Arg Ser Thr Gly Leu Ile Leu
        115                 120                 125

Asp Ser Gly Ala Thr His Thr Thr Ala Ile Pro Val His Asp Gly Tyr
    130                 135                 140

Val Leu Gln Gln Gly Ile Val Lys Ser Pro Leu Ala Gly Asp Phe Ile
145                 150                 155                 160

Thr Met Gln Cys Arg Glu Leu Phe Gln Glu Met Asn Ile Glu Leu Val
                165                 170                 175

Pro Pro Tyr Met Ile Ala Ser Lys Glu Ala Val Arg Glu Gly Ser Pro
            180                 185                 190

Ala Asn Trp Lys Arg Lys Glu Lys Leu Pro Gln Val Thr Arg Ser Trp
        195                 200                 205

His Asn Tyr Met Cys Asn Cys Val Ile Gln Asp Phe Gln Ala Ser Val
    210                 215                 220

Leu Gln Val Ser Asp Ser Thr Tyr Asp Glu Gln Val Ala Ala Gln Met
225                 230                 235                 240

Pro Thr Val His Tyr Glu Phe Pro Asn Gly Tyr Asn Cys Asp Phe Gly
                245                 250                 255

Ala Glu Arg Leu Lys Ile Pro Glu Gly Leu Phe Asp Pro Ser Asn Val
            260                 265                 270

Lys Gly Leu Ser Gly Asn Thr Met Leu Gly Val Ser His Val Val Thr
        275                 280                 285

Thr Ser Val Gly Met Cys Asp Ile Asp Ile Arg Pro Gly Leu Tyr Gly
    290                 295                 300

Ser Val Ile Val Ala Gly Gly Asn Thr Leu Ile Gln Ser Phe Thr Asp

305                 310                 315                 320

Arg Leu Asn Arg Glu Leu Ser Gln Lys Thr Pro Pro Ser Met Arg Leu
                325                 330                 335

Lys Leu Ile Ala Asn Asn Thr Thr Val Glu Arg Arg Phe Ser Ser Trp
            340                 345                 350

Ile Gly Gly Ser Ile Leu Ala Ser Leu Gly Thr Phe Gln Gln Met Trp
        355                 360                 365

Ile Ser Lys Gln Glu Tyr Glu Glu Gly Gly Lys Gln Cys Val Glu Arg
    370                 375                 380

Lys Cys Pro
385

<210>  187
<211>  387
<212>  PRT
<213>  Homo sapiens

<400>  187

Met Val Val Glu Arg Asp Asp Gly Ser Thr Leu Met Glu Ile Asp Gly
1               5                   10                  15

Asp Lys Gly Lys Gln Gly Gly Pro Thr Tyr Tyr Ile Asp Thr Asn Ala
            20                  25                  30

Leu Arg Val Pro Arg Glu Asn Met Glu Ala Ile Ser Pro Leu Lys Asn
        35                  40                  45

Gly Met Val Glu Asp Trp Asp Ser Phe Gln Ala Ile Leu Asp His Thr
    50                  55                  60

Tyr Lys Met His Val Lys Ser Glu Ala Ser Leu His Pro Val Leu Met
65                  70                  75                  80

Ser Glu Ala Pro Trp Asn Thr Arg Ala Lys Arg Glu Lys Leu Thr Glu
                85                  90                  95

Leu Met Phe Glu His Tyr Asn Ile Pro Ala Phe Phe Leu Cys Lys Thr
            100                 105                 110

Ala Val Leu Thr Ala Phe Ala Asn Gly Arg Ser Thr Gly Leu Ile Leu
        115                 120                 125

Asp Ser Gly Ala Thr His Thr Thr Ala Ile Pro Val His Asp Gly Tyr
    130                 135                 140

Val Leu Gln Gln Gly Ile Val Lys Ser Pro Leu Ala Gly Asp Phe Ile
145                 150                 155                 160

Thr Met Gln Cys Arg Glu Leu Phe Gln Glu Met Asn Ile Glu Leu Val
                165                 170                 175

Pro Pro Tyr Met Ile Ala Ser Lys Glu Ala Val Arg Glu Gly Ser Pro
            180                 185                 190

Ala Asn Trp Lys Arg Lys Glu Lys Leu Pro Gln Val Thr Arg Ser Trp
        195                 200                 205

His Asn Tyr Met Cys Asn Cys Val Ile Gln Asp Phe Gln Ala Ser Val
    210                 215                 220

Leu Gln Val Ser Asp Ser Thr Tyr Asp Glu Gln Val Ala Ala Gln Met
225                 230                 235                 240

Pro Thr Val His Tyr Glu Phe Pro Asn Gly Tyr Asn Cys Asp Phe Gly
                245                 250                 255

Ala Glu Arg Leu Lys Ile Pro Glu Gly Leu Phe Asp Pro Ser Asn Val
            260                 265                 270

Lys Gly Leu Ser Gly Asn Thr Met Leu Gly Val Ser His Val Val Thr
        275                 280                 285

Thr Ser Val Gly Met Cys Asp Ile Asp Ile Arg Pro Gly Leu Tyr Gly
    290                 295                 300

Ser Val Ile Val Ala Gly Gly Asn Thr Leu Ile Gln Ser Phe Thr Asp
305                 310                 315                 320

Arg Leu Asn Arg Glu Leu Ser Gln Lys Thr Pro Pro Ser Met Arg Leu
                325                 330                 335

Lys Leu Ile Ala Asn Asn Thr Thr Val Glu Arg Arg Phe Ser Ser Trp
            340                 345                 350

Ile Gly Gly Ser Ile Leu Ala Ser Leu Gly Thr Phe Gln Gln Met Trp
        355                 360                 365

Ile Ser Lys Gln Glu Tyr Glu Glu Gly Gly Lys Gln Cys Val Glu Arg
    370                 375                 380

Lys Cys Pro
385

<210>  188
<211>  1168
<212>  PRT
<213>  Homo sapiens

<400>  188

<210>  189
<211>  1115
<212>  PRT
<213>  Homo sapiens

<400>  189

Met Ser Trp Phe Ser Gly Leu Leu Val Pro Lys Val Asp Glu Arg Lys
1               5                   10                  15

Thr Ala Trp Gly Glu Arg Asn Gly Gln Lys Arg Ser Arg Arg Arg Gly
            20                  25                  30

Thr Arg Ala Gly Gly Phe Cys Thr Pro Arg Tyr Met Ser Cys Leu Arg
        35                  40                  45

Asp Ala Glu Pro Pro Ser Pro Thr Pro Ala Gly Pro Pro Arg Cys Pro
    50                  55                  60

Trp Gln Asp Asp Ala Phe Ile Arg Arg Gly Gly Pro Gly Lys Gly Lys
65                  70                  75                  80

Glu Leu Gly Leu Arg Ala Val Ala Leu Gly Phe Glu Asp Thr Glu Val
                85                  90                  95

Thr Thr Thr Ala Gly Gly Thr Ala Glu Val Ala Pro Asp Ala Val Pro
            100                 105                 110

Arg Ser Gly Arg Ser Cys Trp Arg Arg Leu Val Gln Val Phe Gln Ser
        115                 120                 125

Lys Gln Phe Arg Ser Ala Lys Leu Glu Arg Leu Tyr Gln Arg Tyr Phe
    130                 135                 140

Phe Gln Met Asn Gln Ser Ser Leu Thr Leu Leu Met Ala Val Leu Val
145                 150                 155                 160

Leu Leu Thr Ala Val Leu Leu Ala Phe His Ala Ala Pro Ala Arg Pro
                165                 170                 175

Gln Pro Ala Tyr Val Ala Leu Leu Ala Cys Ala Ala Ala Leu Phe Val
            180                 185                 190

Gly Leu Met Val Val Cys Asn Arg His Ser Phe Arg Gln Asp Ser Met
        195                 200                 205

Trp Val Val Ser Tyr Val Val Leu Gly Ile Leu Ala Ala Val Gln Val
    210                 215                 220

Gly Gly Ala Leu Ala Ala Asp Pro Arg Ser Pro Ser Ala Gly Leu Trp
225                 230                 235                 240

Cys Pro Val Phe Phe Val Tyr Ile Ala Tyr Thr Leu Leu Pro Ile Arg
                245                 250                 255

Met Arg Ala Ala Val Leu Ser Gly Leu Gly Leu Ser Thr Leu His Leu
            260                 265                 270

Ile Leu Ala Trp Gln Leu Asn Arg Gly Asp Ala Phe Leu Trp Lys Gln
        275                 280                 285

Leu Gly Ala Asn Val Leu Leu Phe Leu Cys Thr Asn Val Ile Gly Ile
    290                 295                 300

Cys Thr His Tyr Pro Ala Glu Val Ser Gln Arg Gln Ala Phe Gln Glu
305                 310                 315                 320

Thr Arg Gly Tyr Ile Gln Ala Arg Leu His Leu Gln His Glu Asn Arg
                325                 330                 335

Gln Gln Glu Arg Leu Leu Leu Ser Val Leu Pro Gln His Val Ala Met
            340                 345                 350

Glu Met Lys Glu Asp Ile Asn Thr Lys Lys Glu Asp Met Met Phe His
        355                 360                 365

Lys Ile Tyr Ile Gln Lys His Asp Asn Val Ser Ile Leu Phe Ala Asp
    370                 375                 380

Ile Glu Gly Phe Thr Ser Leu Ala Ser Gln Cys Thr Ala Gln Glu Leu
385                 390                 395                 400

Val Met Thr Leu Asn Glu Leu Phe Ala Arg Phe Asp Lys Leu Ala Ala
                405                 410                 415

Glu Asn His Cys Leu Arg Ile Lys Ile Leu Gly Asp Cys Tyr Tyr Cys
            420                 425                 430

Val Ser Gly Leu Pro Glu Ala Arg Ala Asp His Ala His Cys Cys Val
        435                 440                 445

Glu Met Gly Val Asp Met Ile Glu Ala Ile Ser Leu Val Arg Glu Val
    450                 455                 460

Thr Gly Val Asn Val Asn Met Arg Val Gly Ile His Ser Gly Arg Val
465                 470                 475                 480

His Cys Gly Val Leu Gly Leu Arg Lys Trp Gln Phe Asp Val Trp Ser
                485                 490                 495

Asn Asp Val Thr Leu Ala Asn His Met Glu Ala Gly Gly Arg Ala Gly
            500                 505                 510

Arg Ile His Ile Thr Arg Ala Thr Leu Gln Tyr Leu Asn Gly Asp Tyr
        515                 520                 525

Glu Val Glu Pro Gly Arg Gly Gly Glu Arg Asn Ala Tyr Leu Lys Glu
    530                 535                 540

Gln His Ile Glu Thr Phe Leu Ile Leu Gly Ala Ser Gln Lys Arg Lys
545                 550                 555                 560

Glu Glu Lys Ala Met Leu Ala Lys Leu Gln Arg Thr Arg Ala Asn Ser
                565                 570                 575

Met Glu Gly Leu Met Pro Arg Trp Val Pro Asp Arg Ala Phe Ser Arg
            580                 585                 590

Thr Lys Asp Ser Lys Ala Phe Arg Gln Met Gly Ile Asp Asp Ser Ser
        595                 600                 605

Lys Asp Asn Arg Gly Thr Gln Asp Ala Leu Asn Pro Glu Asp Glu Val
    610                 615                 620

Asp Glu Phe Leu Ser Arg Ala Ile Asp Ala Arg Ser Ile Asp Gln Leu
625                 630                 635                 640

Arg Lys Asp His Val Arg Arg Phe Leu Leu Thr Phe Gln Arg Glu Asp
                645                 650                 655

Leu Glu Lys Lys Tyr Ser Arg Lys Val Asp Pro Arg Phe Gly Ala Tyr
            660                 665                 670

Val Ala Cys Ala Leu Leu Val Phe Cys Phe Ile Cys Phe Ile Gln Leu
        675                 680                 685

Leu Ile Phe Pro His Ser Thr Leu Met Leu Gly Ile Tyr Ala Ser Ile
    690                 695                 700

Phe Leu Leu Leu Leu Ile Thr Val Leu Ile Cys Ala Val Tyr Ser Cys
705                 710                 715                 720

Gly Ser Leu Phe Pro Lys Ala Leu Gln Arg Leu Ser Arg Ser Ile Val
                725                 730                 735

Arg Ser Arg Ala His Ser Thr Ala Val Gly Ile Phe Ser Val Leu Leu
            740                 745                 750

Val Phe Thr Ser Ala Ile Ala Asn Met Tyr Phe Ile Gly Asn Met Leu
        755                 760                 765

Leu Ser Leu Leu Ala Ser Ser Val Phe Leu His Ile Ser Ser Ile Gly
    770                 775                 780

Lys Leu Ala Met Ile Phe Val Leu Gly Leu Ile Tyr Leu Val Leu Leu
785                 790                 795                 800

Leu Leu Gly Pro Pro Ala Thr Ile Phe Asp Asn Tyr Asp Leu Leu Leu
                805                 810                 815

Gly Val His Gly Leu Ala Ser Ser Asn Glu Thr Phe Asp Gly Leu Asp
            820                 825                 830

Cys Pro Ala Ala Gly Arg Val Ala Leu Lys Tyr Met Thr Pro Val Ile
        835                 840                 845

Leu Leu Val Phe Ala Leu Ala Leu Tyr Leu His Ala Gln Gln Val Glu
    850                 855                 860

Ser Thr Ala Arg Leu Asp Phe Leu Trp Lys Leu Gln Ala Thr Gly Glu
865                 870                 875                 880

Lys Glu Glu Met Glu Glu Leu Gln Ala Tyr Asn Arg Arg Leu Leu His
                885                 890                 895

Asn Ile Leu Pro Lys Asp Val Ala Ala His Phe Leu Ala Arg Glu Arg
            900                 905                 910

Arg Asn Asp Glu Leu Tyr Tyr Gln Ser Cys Glu Cys Val Ala Val Met
        915                 920                 925

Phe Ala Ser Ile Ala Asn Phe Ser Glu Phe Tyr Val Glu Leu Glu Ala
    930                 935                 940

Asn Asn Glu Gly Val Glu Cys Leu Arg Leu Leu Asn Glu Ile Ile Ala
945                 950                 955                 960

Asp Phe Asp Glu Ile Ile Ser Glu Glu Arg Phe Arg Gln Leu Glu Lys
                965                 970                 975

Ile Lys Thr Ile Gly Ser Thr Tyr Met Ala Ala Ser Gly Leu Asn Ala
            980                 985                 990

Ser Thr Tyr Asp Gln Val Gly Arg  Ser His Ile Thr Ala  Leu Ala Asp
        995                 1000                 1005

Tyr Ala  Met Arg Leu Met Glu  Gln Met Lys His Ile  Asn Glu His
    1010                 1015                 1020

Ser Phe  Asn Asn Phe Gln Met  Lys Ile Gly Leu Asn  Met Gly Pro
    1025                 1030                 1035

Val Val  Ala Gly Val Ile Gly  Ala Arg Lys Pro Gln  Tyr Asp Ile
    1040                 1045                 1050

Trp Gly  Asn Thr Val Asn Val  Ser Ser Arg Met Asp  Ser Thr Gly
    1055                 1060                 1065

Val Pro  Asp Arg Ile Gln Val  Thr Thr Asp Leu Tyr  Gln Val Leu
    1070                 1075                 1080

Ala Ala  Lys Gly Tyr Gln Leu  Glu Cys Arg Gly Val  Val Lys Val
    1085                 1090                 1095

Lys Gly  Lys Gly Glu Met Thr  Thr Tyr Phe Leu Asn  Gly Gly Pro
    1100                 1105                 1110

Ser Ser
    1115

<210>  190
<211>  137
<212>  PRT
<213>  Homo sapiens

<400>  190

Met Gly Ala Pro Thr Leu Pro Pro Ala Trp Gln Pro Phe Leu Lys Asp
1               5                   10                  15

His Arg Ile Ser Thr Phe Lys Asn Trp Pro Phe Leu Glu Gly Cys Ala
            20                  25                  30

Cys Thr Pro Glu Arg Met Ala Glu Ala Gly Phe Ile His Cys Pro Thr
        35                  40                  45

Glu Asn Glu Pro Asp Leu Ala Gln Cys Phe Phe Cys Phe Lys Glu Leu
    50                  55                  60

Glu Gly Trp Glu Pro Asp Asp Asp Pro Met Gln Arg Lys Pro Thr Ile
65                  70                  75                  80

Arg Arg Lys Asn Leu Arg Lys Leu Arg Arg Lys Cys Ala Val Pro Ser
                85                  90                  95

Ser Ser Trp Leu Pro Trp Ile Glu Ala Ser Gly Arg Ser Cys Leu Val
            100                 105                 110

Pro Glu Trp Leu His His Phe Gln Gly Leu Phe Pro Gly Ala Thr Ser
        115                 120                 125

Leu Pro Val Gly Pro Leu Ala Met Ser
    130                 135

<210>  191
<211>  165
<212>  PRT

<213>  Homo sapiens

<400>  191

Met Gly Ala Pro Thr Leu Pro Pro Ala Trp Gln Pro Phe Leu Lys Asp
1               5                   10                  15

His Arg Ile Ser Thr Phe Lys Asn Trp Pro Phe Leu Glu Gly Cys Ala
            20                  25                  30

Cys Thr Pro Glu Arg Met Ala Glu Ala Gly Phe Ile His Cys Pro Thr
        35                  40                  45

Glu Asn Glu Pro Asp Leu Ala Gln Cys Phe Phe Cys Phe Lys Glu Leu
    50                  55                  60

Glu Gly Trp Glu Pro Asp Asp Asp Pro Ile Gly Pro Gly Thr Val Ala
65                  70                  75                  80

Tyr Ala Cys Asn Thr Ser Thr Leu Gly Gly Arg Gly Gly Arg Ile Thr
                85                  90                  95

Arg Glu Glu His Lys Lys His Ser Ser Gly Cys Ala Phe Leu Ser Val
            100                 105                 110

Lys Lys Gln Phe Glu Glu Leu Thr Leu Gly Glu Phe Leu Lys Leu Asp
        115                 120                 125

Arg Glu Arg Ala Lys Asn Lys Ile Ala Lys Glu Thr Asn Asn Lys Lys
    130                 135                 140

Lys Glu Phe Glu Glu Thr Ala Glu Lys Val Arg Arg Ala Ile Glu Gln
145                 150                 155                 160

Leu Ala Ala Met Asp
                165


<210>  192
<211>  142
<212>  PRT
<213>  Homo sapiens

<400>  192

Met Gly Ala Pro Thr Leu Pro Pro Ala Trp Gln Pro Phe Leu Lys Asp
1               5                   10                  15

His Arg Ile Ser Thr Phe Lys Asn Trp Pro Phe Leu Glu Gly Cys Ala
            20                  25                  30

Cys Thr Pro Glu Arg Met Ala Glu Ala Gly Phe Ile His Cys Pro Thr
        35                  40                  45

Glu Asn Glu Pro Asp Leu Ala Gln Cys Phe Phe Cys Phe Lys Glu Leu
    50                  55                  60

Glu Gly Trp Glu Pro Asp Asp Asp Pro Ile Glu Glu His Lys Lys His
65                  70                  75                  80

Ser Ser Gly Cys Ala Phe Leu Ser Val Lys Lys Gln Phe Glu Glu Leu
                85                  90                  95

Thr Leu Gly Glu Phe Leu Lys Leu Asp Arg Glu Arg Ala Lys Asn Lys
            100                 105                 110

Ile Ala Lys Glu Thr Asn Asn Lys Lys Lys Glu Phe Glu Glu Thr Ala
        115                 120                 125

Glu Lys Val Arg Arg Ala Ile Glu Gln Leu Ala Ala Met Asp
    130                 135                 140

<210>  193
<211>  1085
<212>  PRT
<213>  Homo sapiens

<400>  193

Met Asp Thr Pro Glu Asn Val Leu Gln Met Leu Glu Ala His Met Gln
1               5                   10                  15

Ser Tyr Lys Gly Asn Asp Pro Leu Gly Glu Trp Glu Arg Tyr Ile Gln
            20                  25                  30

Trp Val Glu Glu Asn Phe Pro Glu Asn Lys Glu Tyr Leu Ile Thr Leu
        35                  40                  45

Leu Glu His Leu Met Lys Glu Phe Leu Asp Lys Lys Lys Tyr His Asn
    50                  55                  60

Asp Pro Arg Phe Ile Ser Tyr Cys Leu Lys Phe Ala Glu Tyr Asn Ser
65                  70                  75                  80

Asp Leu His Gln Phe Phe Glu Phe Leu Tyr Asn His Gly Ile Gly Thr
                85                  90                  95

Leu Ser Ser Pro Leu Tyr Ile Ala Trp Ala Gly His Leu Glu Ala Gln
            100                 105                 110

Gly Glu Leu Gln His Ala Ser Ala Val Leu Gln Arg Gly Ile Gln Asn
        115                 120                 125

Gln Ala Glu Pro Arg Glu Phe Leu Gln Gln Gln Tyr Arg Leu Phe Gln
    130                 135                 140

Thr Arg Leu Thr Glu Thr His Leu Pro Ala Gln Ala Arg Thr Ser Glu
145                 150                 155                 160

Pro Leu His Asn Val Gln Val Leu Asn Gln Met Ile Thr Ser Lys Ser
                165                 170                 175

Asn Pro Gly Asn Asn Met Ala Cys Ile Ser Lys Asn Gln Gly Ser Glu
            180                 185                 190

Leu Ser Gly Val Ile Ser Ser Ala Cys Asp Lys Glu Ser Asn Met Glu
        195                 200                 205

Arg Arg Val Ile Thr Ile Ser Lys Ser Glu Tyr Ser Val His Ser Ser
    210                 215                 220

Leu Ala Ser Lys Val Asp Val Glu Gln Val Val Met Tyr Cys Lys Glu
225                 230                 235                 240

Lys Leu Ile Arg Gly Glu Ser Glu Phe Ser Phe Glu Glu Leu Arg Ala
                245                 250                 255

Gln Lys Tyr Asn Gln Arg Arg Lys His Glu Gln Trp Val Asn Glu Asp
            260                 265                 270

Arg His Tyr Met Lys Arg Lys Glu Ala Asn Ala Phe Glu Glu Gln Leu
        275                 280                 285

Leu Lys Gln Lys Met Asp Glu Leu His Lys Lys Leu His Gln Val Val
    290                 295                 300

Glu Thr Ser His Glu Asp Leu Pro Ala Ser Gln Glu Arg Ser Glu Val
305                 310                 315                 320

Asn Pro Ala Arg Met Gly Pro Ser Val Gly Ser Gln Gln Glu Leu Arg
                325                 330                 335

Ala Pro Cys Leu Pro Val Thr Tyr Gln Gln Thr Pro Val Asn Met Glu
            340                 345                 350

Lys Asn Pro Arg Glu Ala Pro Pro Val Val Pro Pro Leu Ala Asn Ala
        355                 360                 365

Ile Ser Ala Ala Leu Val Ser Pro Ala Thr Ser Gln Ser Ile Ala Pro
    370                 375                 380

Pro Val Pro Leu Lys Ala Gln Thr Val Thr Asp Ser Met Phe Ala Val
385                 390                 395                 400

Ala Ser Lys Asp Ala Gly Cys Val Asn Lys Ser Thr His Glu Phe Lys
                405                 410                 415

Pro Gln Ser Gly Ala Glu Ile Lys Glu Gly Cys Glu Thr His Lys Val
            420                 425                 430

Ala Asn Thr Ser Ser Phe His Thr Thr Pro Asn Thr Ser Leu Gly Met
        435                 440                 445

Val Gln Ala Thr Pro Ser Lys Val Gln Pro Ser Pro Thr Val His Thr
450                 455                 460

Lys Glu Ala Leu Gly Phe Ile Met Asn Met Phe Gln Ala Pro Thr Leu
465                 470                 475                 480

Pro Asp Ile Ser Asp Asp Lys Asp Glu Trp Gln Ser Leu Asp Gln Asn
                485                 490                 495

Glu Asp Ala Phe Glu Ala Gln Phe Gln Lys Asn Val Arg Ser Ser Gly
            500                 505                 510

Ala Trp Gly Val Asn Lys Ile Ile Ser Ser Leu Ser Ser Ala Phe His
        515                 520                 525

Val Phe Glu Asp Gly Asn Lys Glu Asn Tyr Gly Leu Pro Gln Pro Lys
    530                 535                 540

Asn Lys Pro Thr Gly Ala Arg Thr Phe Gly Glu Arg Ser Val Ser Arg
545                 550                 555                 560

Leu Pro Ser Lys Pro Lys Glu Glu Val Pro His Ala Glu Glu Phe Leu
                565                 570                 575

Asp Asp Ser Thr Val Trp Gly Ile Arg Cys Asn Lys Thr Leu Ala Pro
            580                 585                 590

Ser Pro Lys Ser Pro Gly Asp Phe Thr Ser Ala Ala Gln Leu Ala Ser
        595                 600                 605

Thr Pro Phe His Lys Leu Pro Val Glu Ser Val His Ile Leu Glu Asp
    610                 615                 620

Lys Glu Asn Val Val Ala Lys Gln Cys Thr Gln Ala Thr Leu Asp Ser
625                 630                 635                 640

Cys Glu Glu Asn Met Val Val Pro Ser Arg Asp Gly Lys Phe Ser Pro
                645                 650                 655

Ile Gln Glu Lys Ser Pro Lys Gln Ala Leu Ser Ser His Met Tyr Ser
            660                 665                 670

Ala Ser Leu Leu Arg Leu Ser Gln Pro Ala Ala Gly Gly Val Leu Thr
        675                 680                 685

Cys Glu Ala Glu Leu Gly Val Glu Ala Cys Arg Leu Thr Asp Thr Asp
    690                 695                 700

Ala Ala Ile Ala Glu Asp Pro Pro Asp Ala Ile Ala Gly Leu Gln Ala
705                 710                 715                 720

Glu Trp Met Gln Met Ser Ser Leu Gly Thr Val Asp Ala Pro Asn Phe
                725                 730                 735

Ile Val Gly Asn Pro Trp Asp Asp Lys Leu Ile Phe Lys Leu Leu Ser
            740                 745                 750

Gly Leu Ser Lys Pro Val Ser Ser Tyr Pro Asn Thr Phe Glu Trp Gln
        755                 760                 765

Cys Lys Leu Pro Ala Ile Lys Pro Lys Thr Glu Phe Gln Leu Gly Ser
    770                 775                 780

Lys Leu Val Tyr Val His His Leu Leu Gly Glu Gly Ala Phe Ala Gln
785                 790                 795                 800

Val Tyr Glu Ala Thr Gln Gly Asp Leu Asn Asp Ala Lys Asn Lys Gln
                805                 810                 815

Lys Phe Val Leu Lys Val Gln Lys Pro Ala Asn Pro Trp Glu Phe Tyr
            820                 825                 830

Ile Gly Thr Gln Leu Met Glu Arg Leu Lys Pro Ser Met Gln His Met
        835                 840                 845

Phe Met Lys Phe Tyr Ser Ala His Leu Phe Gln Asn Gly Ser Val Leu
    850                 855                 860

Val Gly Glu Leu Tyr Ser Tyr Gly Thr Leu Leu Asn Ala Ile Asn Leu
865                 870                 875                 880

Tyr Lys Asn Thr Pro Glu Lys Val Met Pro Gln Gly Leu Val Ile Ser
                885                 890                 895

Phe Ala Met Arg Met Leu Tyr Met Ile Glu Gln Val His Asp Cys Glu
            900                 905                 910

Ile Ile His Gly Asp Ile Lys Pro Asp Asn Phe Ile Leu Gly Asn Gly
        915                 920                 925

Phe Leu Glu Gln Asp Asp Glu Asp Asp Leu Ser Ala Gly Leu Ala Leu
    930                 935                 940

Ile Asp Leu Gly Gln Ser Ile Asp Met Lys Leu Phe Pro Lys Gly Thr
945                 950                 955                 960

Ile Phe Thr Ala Lys Cys Glu Thr Ser Gly Phe Gln Cys Val Glu Met
                965                 970                 975

Leu Ser Asn Lys Pro Trp Asn Tyr Gln Ile Asp Tyr Phe Gly Val Ala
            980                 985                 990

Ala Thr Val Tyr Cys Met Leu Phe  Gly Thr Tyr Met Lys  Val Lys Asn
        995                 1000                1005

Glu Gly  Gly Glu Cys Lys Pro  Glu Gly Leu Phe Arg  Arg Leu Pro
    1010                 1015                 1020

His Leu  Asp Met Trp Asn Glu  Phe Phe His Val Met  Leu Asn Ile
    1025                 1030                 1035

Pro Asp  Cys His His Leu Pro  Ser Leu Asp Leu Leu  Arg Gln Lys
    1040                 1045                 1050

Leu Lys  Lys Val Phe Gln Gln  His Tyr Thr Asn Lys  Ile Arg Ala
    1055                 1060                 1065

Leu Arg  Asn Arg Leu Ile Val  Leu Leu Leu Glu Cys  Lys Arg Ser
    1070                 1075                 1080

Arg Lys
    1085

<210>  194
<211>  1050
<212>  PRT
<213>  Homo sapiens

<400>  194

Met Ala Ala Val Lys Lys Glu Gly Gly Ala Leu Ser Glu Ala Met Ser
1               5                   10                  15

Leu Glu Gly Asp Glu Trp Glu Leu Ser Lys Glu Asn Val Gln Pro Leu
            20                  25                  30

Arg Gln Gly Arg Ile Met Ser Thr Leu Gln Gly Ala Leu Ala Gln Glu
        35                  40                  45

Ser Ala Cys Asn Asn Thr Leu Gln Gln Gln Lys Arg Ala Phe Glu Tyr
    50                  55                  60

Glu Ile Arg Phe Tyr Thr Gly Asn Asp Pro Leu Asp Val Trp Asp Arg
65                  70                  75                  80

Tyr Ile Ser Trp Thr Glu Gln Asn Tyr Pro Gln Gly Gly Lys Glu Ser
                85                  90                  95

Asn Met Ser Thr Leu Leu Glu Arg Ala Val Glu Ala Leu Gln Gly Glu
            100                 105                 110

Lys Arg Tyr Tyr Ser Asp Pro Arg Phe Leu Asn Leu Trp Leu Lys Leu
        115                 120                 125

Gly Arg Leu Cys Asn Glu Pro Leu Asp Met Tyr Ser Tyr Leu His Asn
    130                 135                 140

Gln Gly Ile Gly Val Ser Leu Ala Gln Phe Tyr Ile Ser Trp Ala Glu
145                 150                 155                 160

Glu Tyr Glu Ala Arg Glu Asn Phe Arg Lys Ala Asp Ala Ile Phe Gln
                165                 170                 175

Glu Gly Ile Gln Gln Lys Ala Glu Pro Leu Glu Arg Leu Gln Ser Gln
            180                 185                 190

His Arg Gln Phe Gln Ala Arg Val Ser Arg Gln Thr Leu Leu Ala Leu
        195                 200                 205

Glu Lys Glu Glu Glu Glu Glu Val Phe Glu Ser Ser Val Pro Gln Arg
    210                 215                 220

Ser Thr Leu Ala Glu Leu Lys Ser Lys Gly Lys Lys Thr Ala Arg Ala
225                 230                 235                 240

Pro Ile Ile Arg Val Gly Gly Ala Leu Lys Ala Pro Ser Gln Asn Arg
                245                 250                 255

Gly Leu Gln Asn Pro Phe Pro Gln Gln Met Gln Asn Asn Ser Arg Ile
            260                 265                 270

Thr Val Phe Asp Glu Asn Ala Asp Glu Ala Ser Thr Ala Glu Leu Ser
        275                 280                 285

Lys Pro Thr Val Gln Pro Trp Ile Ala Pro Pro Met Pro Arg Ala Lys
    290                 295                 300

Glu Asn Glu Leu Gln Ala Gly Pro Trp Asn Thr Gly Arg Ser Leu Glu
305                 310                 315                 320

His Arg Pro Arg Gly Asn Thr Ala Ser Leu Ile Ala Val Pro Ala Val
                325                 330                 335

Leu Pro Ser Phe Thr Pro Tyr Val Glu Glu Thr Ala Gln Gln Pro Val
            340                 345                 350

Met Thr Pro Cys Lys Ile Glu Pro Ser Ile Asn His Ile Leu Ser Thr
        355                 360                 365

Arg Lys Pro Gly Lys Glu Glu Gly Asp Pro Leu Gln Arg Val Gln Ser
    370                 375                 380

His Gln Gln Ala Ser Glu Glu Lys Lys Glu Lys Met Met Tyr Cys Lys
385                 390                 395                 400

Glu Lys Ile Tyr Ala Gly Val Gly Glu Phe Ser Phe Glu Glu Ile Arg
                405                 410                 415

Ala Glu Val Phe Arg Lys Lys Leu Lys Glu Gln Arg Glu Ala Glu Leu
            420                 425                 430

Leu Thr Ser Ala Glu Lys Arg Ala Glu Met Gln Lys Gln Ile Glu Glu

435                 440                 445

Met Glu Lys Lys Leu Lys Glu Ile Gln Thr Thr Gln Gln Glu Arg Thr
    450                 455                 460

Gly Asp Gln Gln Glu Glu Thr Met Pro Thr Lys Glu Thr Thr Lys Leu
465                 470                 475                 480

Gln Ile Ala Ser Glu Ser Gln Lys Ile Pro Gly Met Thr Leu Ser Ser
                485                 490                 495

Ser Val Cys Gln Val Asn Cys Cys Ala Arg Glu Thr Ser Leu Ala Glu
            500                 505                 510

Asn Ile Trp Gln Glu Gln Pro His Ser Lys Gly Pro Ser Val Pro Phe
        515                 520                 525

Ser Ile Phe Asp Glu Phe Leu Leu Ser Glu Lys Lys Asn Lys Ser Pro
    530                 535                 540

Pro Ala Asp Pro Pro Arg Val Leu Ala Gln Arg Arg Pro Leu Ala Val
545                 550                 555                 560

Leu Lys Thr Ser Glu Ser Ile Thr Ser Asn Glu Asp Val Ser Pro Asp
                565                 570                 575

Val Cys Asp Glu Phe Thr Gly Ile Glu Pro Leu Ser Glu Asp Ala Ile
            580                 585                 590

Ile Thr Gly Phe Arg Asn Val Thr Ile Cys Pro Asn Pro Glu Asp Thr
        595                 600                 605

Cys Asp Phe Ala Arg Ala Ala Arg Phe Val Ser Thr Pro Phe His Glu
    610                 615                 620

Ile Met Ser Leu Lys Asp Leu Pro Ser Asp Pro Glu Arg Leu Leu Pro
625                 630                 635                 640

Glu Glu Asp Leu Asp Val Lys Thr Ser Glu Asp Gln Gln Thr Ala Cys
                645                 650                 655

Gly Thr Ile Tyr Ser Gln Thr Leu Ser Ile Lys Lys Leu Ser Pro Ile
            660                 665                 670

Ile Glu Asp Ser Arg Glu Ala Thr His Ser Ser Gly Phe Ser Gly Ser
        675                 680                 685

Ser Ala Ser Val Ala Ser Thr Ser Ser Ile Lys Cys Leu Gln Ile Pro
    690                 695                 700

Glu Lys Leu Glu Leu Thr Asn Glu Thr Ser Glu Asn Pro Thr Gln Ser
705                 710                 715                 720

Pro Trp Cys Ser Gln Tyr Arg Arg Gln Leu Leu Lys Ser Leu Pro Glu
                725                 730                 735

Leu Ser Ala Ser Ala Glu Leu Cys Ile Glu Asp Arg Pro Met Pro Lys
            740                 745                 750

Leu Glu Ile Glu Lys Glu Ile Glu Leu Gly Asn Glu Asp Tyr Cys Ile
        755                 760                 765

Lys Arg Glu Tyr Leu Ile Cys Glu Asp Tyr Lys Leu Phe Trp Val Ala
    770                 775                 780

Pro Arg Asn Ser Ala Glu Leu Thr Val Ile Lys Val Ser Ser Gln Pro
785                 790                 795                 800

Val Pro Trp Asp Phe Tyr Ile Asn Leu Lys Leu Lys Glu Arg Leu Asn
                805                 810                 815

Glu Asp Phe Asp His Phe Cys Ser Cys Tyr Gln Tyr Gln Asp Gly Cys
            820                 825                 830

Ile Val Trp His Gln Tyr Ile Asn Cys Phe Thr Leu Gln Asp Leu Leu
        835                 840                 845

Gln His Ser Glu Tyr Ile Thr His Glu Ile Thr Val Leu Ile Ile Tyr
    850                 855                 860

Asn Leu Leu Thr Ile Val Glu Met Leu His Lys Ala Glu Ile Val His
865                 870                 875                 880

Gly Asp Leu Ser Pro Arg Cys Leu Ile Leu Arg Asn Arg Ile His Asp
                885                 890                 895

Pro Tyr Asp Cys Asn Lys Asn Asn Gln Ala Leu Lys Ile Val Asp Phe
            900                 905                 910

Ser Tyr Ser Val Asp Leu Arg Val Gln Leu Asp Val Phe Thr Leu Ser
        915                 920                 925

Gly Phe Arg Thr Val Gln Ile Leu Glu Gly Gln Lys Ile Leu Ala Asn
    930                 935                 940

Cys Ser Ser Pro Tyr Gln Val Asp Leu Phe Gly Ile Ala Asp Leu Ala
945                 950                 955                 960

His Leu Leu Leu Phe Lys Glu His Leu Gln Val Phe Trp Asp Gly Ser
                965                 970                 975

Phe Trp Lys Leu Ser Gln Asn Ile Ser Glu Leu Lys Asp Gly Glu Leu
            980                 985                 990

Trp Asn Lys Phe Phe Val Arg Ile  Leu Asn Ala Asn Asp  Glu Ala Thr

995                 1000                1005

Val Ser  Val Leu Gly Glu Leu  Ala Ala Glu Met Asn  Gly Val Phe
    1010                 1015                1020

Asp Thr  Thr Phe Gln Ser His  Leu Asn Lys Ala Leu  Trp Lys Val
    1025                 1030                1035

Gly Lys  Leu Thr Ser Pro Gly  Ala Leu Leu Phe Gln
    1040                 1045                1050

<210>  195
<211>  2225
<212>  PRT
<213>  Homo sapiens

<400>  195

<210>  196
<211>  433
<212>  PRT
<213>  Homo sapiens

<400>  196

Met Ala Leu Arg Val Thr Arg Asn Ser Lys Ile Asn Ala Glu Asn Lys
1               5                   10                  15

Ala Lys Ile Asn Met Ala Gly Ala Lys Arg Val Pro Thr Ala Pro Ala
            20                  25                  30

Ala Thr Ser Lys Pro Gly Leu Arg Pro Arg Thr Ala Leu Gly Asp Ile
        35                  40                  45

Gly Asn Lys Val Ser Glu Gln Leu Gln Ala Lys Met Pro Met Lys Lys
    50                  55                  60

Glu Ala Lys Pro Ser Ala Thr Gly Lys Val Ile Asp Lys Lys Leu Pro
65                  70                  75                  80

Lys Pro Leu Glu Lys Val Pro Met Leu Val Pro Val Pro Val Ser Glu

85                  90                  95

Pro Val Pro Glu Pro Glu Pro Glu Pro Glu Pro Glu Pro Val Lys Glu
            100                 105                 110

Glu Lys Leu Ser Pro Glu Pro Ile Leu Val Asp Thr Ala Ser Pro Ser
        115                 120                 125

Pro Met Glu Thr Ser Gly Cys Ala Pro Ala Glu Glu Asp Leu Cys Gln
    130                 135                 140

Ala Phe Ser Asp Val Ile Leu Ala Val Asn Asp Val Asp Ala Glu Asp
145                 150                 155                 160

Gly Ala Asp Pro Asn Leu Cys Ser Glu Tyr Val Lys Asp Ile Tyr Ala
                165                 170                 175

Tyr Leu Arg Gln Leu Glu Glu Glu Gln Ala Val Arg Pro Lys Tyr Leu
            180                 185                 190

Leu Gly Arg Glu Val Thr Gly Asn Met Arg Ala Ile Leu Ile Asp Trp
        195                 200                 205

Leu Val Gln Val Gln Met Lys Phe Arg Leu Leu Gln Glu Thr Met Tyr
    210                 215                 220

Met Thr Val Ser Ile Ile Asp Arg Phe Met Gln Asn Asn Cys Val Pro
225                 230                 235                 240

Lys Lys Met Leu Gln Leu Val Gly Val Thr Ala Met Phe Ile Ala Ser
                245                 250                 255

Lys Tyr Glu Glu Met Tyr Pro Pro Glu Ile Gly Asp Phe Ala Phe Val
            260                 265                 270

Thr Asp Asn Thr Tyr Thr Lys His Gln Ile Arg Gln Met Glu Met Lys
        275                 280                 285

Ile Leu Arg Ala Leu Asn Phe Gly Leu Gly Arg Pro Leu Pro Leu His
    290                 295                 300

Phe Leu Arg Arg Ala Ser Lys Ile Gly Glu Val Asp Val Glu Gln His
305                 310                 315                 320

Thr Leu Ala Lys Tyr Leu Met Glu Leu Thr Met Leu Asp Tyr Asp Met
                325                 330                 335

Val His Phe Pro Pro Ser Gln Ile Ala Ala Gly Ala Phe Cys Leu Ala
            340                 345                 350

Leu Lys Ile Leu Asp Asn Gly Glu Trp Thr Pro Thr Leu Gln His Tyr
        355                 360                 365

Leu Ser Tyr Thr Glu Glu Ser Leu Leu Pro Val Met Gln His Leu Ala
    370                 375                 380

Lys Asn Val Val Met Val Asn Gln Gly Leu Thr Lys His Met Thr Val
385                 390                 395                 400

Lys Asn Lys Tyr Ala Thr Ser Lys His Ala Lys Ile Ser Thr Leu Pro
                405                 410                 415

Gln Leu Asn Ser Ala Leu Val Gln Asp Leu Ala Lys Ala Val Ala Lys
            420                 425                 430

Val

<210>  197
<211>  297
<212>  PRT
<213>  Homo sapiens

<400>  197

Met Glu Asp Tyr Thr Lys Ile Glu Lys Ile Gly Glu Gly Thr Tyr Gly
1               5                   10                  15

Val Val Tyr Lys Gly Arg His Lys Thr Thr Gly Gln Val Val Ala Met
            20                  25                  30

Lys Lys Ile Arg Leu Glu Ser Glu Glu Glu Gly Val Pro Ser Thr Ala
        35                  40                  45

Ile Arg Glu Ile Ser Leu Leu Lys Glu Leu Arg His Pro Asn Ile Val
    50                  55                  60

Ser Leu Gln Asp Val Leu Met Gln Asp Ser Arg Leu Tyr Leu Ile Phe
65                  70                  75                  80

Glu Phe Leu Ser Met Asp Leu Lys Lys Tyr Leu Asp Ser Ile Pro Pro
                85                  90                  95

Gly Gln Tyr Met Asp Ser Ser Leu Val Lys Ser Tyr Leu Tyr Gln Ile
            100                 105                 110

Leu Gln Gly Ile Val Phe Cys His Ser Arg Arg Val Leu His Arg Asp
        115                 120                 125

Leu Lys Pro Gln Asn Leu Leu Ile Asp Asp Lys Gly Thr Ile Lys Leu
    130                 135                 140

Ala Asp Phe Gly Leu Ala Arg Ala Phe Gly Ile Pro Ile Arg Val Tyr
145                 150                 155                 160

Thr His Glu Val Val Thr Leu Trp Tyr Arg Ser Pro Glu Val Leu Leu
                165                 170                 175

Gly Ser Ala Arg Tyr Ser Thr Pro Val Asp Ile Trp Ser Ile Gly Thr
            180                 185                 190

Ile Phe Ala Glu Leu Ala Thr Lys Lys Pro Leu Phe His Gly Asp Ser
        195                 200                 205

Glu Ile Asp Gln Leu Phe Arg Ile Phe Arg Ala Leu Gly Thr Pro Asn
    210                 215                 220

Asn Glu Val Trp Pro Glu Val Glu Ser Leu Gln Asp Tyr Lys Asn Thr
225                 230                 235                 240

Phe Pro Lys Trp Lys Pro Gly Ser Leu Ala Ser His Val Lys Asn Leu
                245                 250                 255

Asp Glu Asn Gly Leu Asp Leu Leu Ser Lys Met Leu Ile Tyr Asp Pro
            260                 265                 270

Ala Lys Arg Ile Ser Gly Lys Met Ala Leu Asn His Pro Tyr Phe Asn
        275                 280                 285

Asp Leu Asp Asn Gln Ile Lys Lys Met
    290                 295

<210>  198
<211>  240
<212>  PRT
<213>  Homo sapiens

<400>  198

Met Glu Asp Tyr Thr Lys Ile Glu Lys Ile Gly Glu Gly Thr Tyr Gly
1               5                   10                  15

Val Val Tyr Lys Gly Arg His Lys Thr Thr Gly Gln Val Val Ala Met
            20                  25                  30

Lys Lys Ile Arg Leu Glu Ser Glu Glu Glu Gly Val Pro Ser Thr Ala
        35                  40                  45

Ile Arg Glu Ile Ser Leu Leu Lys Glu Leu Arg His Pro Asn Ile Val
    50                  55                  60

Ser Leu Gln Asp Val Leu Met Gln Asp Ser Arg Leu Tyr Leu Ile Phe
65                  70                  75                  80

Glu Phe Leu Ser Met Asp Leu Lys Lys Tyr Leu Asp Ser Ile Pro Pro
                85                  90                  95

Gly Gln Tyr Met Asp Ser Ser Leu Val Lys Val Val Thr Leu Trp Tyr
            100                 105                 110

Arg Ser Pro Glu Val Leu Leu Gly Ser Ala Arg Tyr Ser Thr Pro Val

115                 120                 125

Asp Ile Trp Ser Ile Gly Thr Ile Phe Ala Glu Leu Ala Thr Lys Lys
    130                 135                 140

Pro Leu Phe His Gly Asp Ser Glu Ile Asp Gln Leu Phe Arg Ile Phe
145                 150                 155                 160

Arg Ala Leu Gly Thr Pro Asn Asn Glu Val Trp Pro Glu Val Glu Ser
                165                 170                 175

Leu Gln Asp Tyr Lys Asn Thr Phe Pro Lys Trp Lys Pro Gly Ser Leu
            180                 185                 190

Ala Ser His Val Lys Asn Leu Asp Glu Asn Gly Leu Asp Leu Leu Ser
        195                 200                 205

Lys Met Leu Ile Tyr Asp Pro Ala Lys Arg Ile Ser Gly Lys Met Ala
    210                 215                 220

Leu Asn His Pro Tyr Phe Asn Asp Leu Asp Asn Gln Ile Lys Lys Met
225                 230                 235                 240

<210>  199
<211>  499
<212>  PRT
<213>  Homo sapiens

<400>  199

Met Ala Gln Phe Ala Phe Glu Ser Asp Leu His Ser Leu Leu Gln Leu
1               5                   10                  15

Asp Ala Pro Ile Pro Asn Ala Pro Pro Ala Arg Trp Gln Arg Lys Ala
            20                  25                  30

Lys Glu Ala Ala Gly Pro Ala Pro Ser Pro Met Arg Ala Ala Asn Arg
        35                  40                  45

Ser His Ser Ala Gly Arg Thr Pro Gly Arg Thr Pro Gly Lys Ser Ser
    50                  55                  60

Ser Lys Val Gln Thr Thr Pro Ser Lys Pro Gly Gly Asp Arg Tyr Ile
65                  70                  75                  80

Pro His Arg Ser Ala Ala Gln Met Glu Val Ala Ser Phe Leu Leu Ser
                85                  90                  95

Lys Glu Asn Gln Ser Glu Asn Ser Gln Thr Pro Thr Lys Lys Glu His
            100                 105                 110

Gln Lys Ala Trp Ala Leu Asn Leu Asn Gly Phe Asp Val Glu Glu Ala
        115                 120                 125

Lys Ile Leu Arg Leu Ser Gly Lys Pro Gln Asn Ala Pro Glu Gly Tyr
    130                 135                 140

Gln Asn Arg Leu Lys Val Leu Tyr Ser Gln Lys Ala Thr Pro Gly Ser
145                 150                 155                 160

Ser Arg Lys Thr Cys Arg Tyr Ile Pro Ser Leu Pro Asp Arg Ile Leu
                165                 170                 175

Asp Ala Pro Glu Ile Arg Asn Asp Tyr Tyr Leu Asn Leu Val Asp Trp
            180                 185                 190

Ser Ser Gly Asn Val Leu Ala Val Ala Leu Asp Asn Ser Val Tyr Leu
        195                 200                 205

Trp Ser Ala Ser Ser Gly Asp Ile Leu Gln Leu Leu Gln Met Glu Gln
    210                 215                 220

Pro Gly Glu Tyr Ile Ser Ser Val Ala Trp Ile Lys Glu Gly Asn Tyr
225                 230                 235                 240

Leu Ala Val Gly Thr Ser Ser Ala Glu Val Gln Leu Trp Asp Val Gln
                245                 250                 255

Gln Gln Lys Arg Leu Arg Asn Met Thr Ser His Ser Ala Arg Val Gly
            260                 265                 270

Ser Leu Ser Trp Asn Ser Tyr Ile Leu Ser Ser Gly Ser Arg Ser Gly
        275                 280                 285

His Ile His His His Asp Val Arg Val Ala Glu His His Val Ala Thr
    290                 295                 300

Leu Ser Gly His Ser Gln Glu Val Cys Gly Leu Arg Trp Ala Pro Asp
305                 310                 315                 320

Gly Arg His Leu Ala Ser Gly Gly Asn Asp Asn Leu Val Asn Val Trp
                325                 330                 335

Pro Ser Ala Pro Gly Glu Gly Gly Trp Val Pro Leu Gln Thr Phe Thr
            340                 345                 350

Gln His Gln Gly Ala Val Lys Ala Val Ala Trp Cys Pro Trp Gln Ser
        355                 360                 365

Asn Val Leu Ala Thr Gly Gly Gly Thr Ser Asp Arg His Ile Arg Ile
    370                 375                 380

Trp Asn Val Cys Ser Gly Ala Cys Leu Ser Ala Val Asp Ala His Ser
385                 390                 395                 400

Gln Val Cys Ser Ile Leu Trp Ser Pro His Tyr Lys Glu Leu Ile Ser
                405                 410                 415

Gly His Gly Phe Ala Gln Asn Gln Leu Val Ile Trp Lys Tyr Pro Thr
            420                 425                 430

Met Ala Lys Val Ala Glu Leu Lys Gly His Thr Ser Arg Val Leu Ser
        435                 440                 445

Leu Thr Met Ser Pro Asp Gly Ala Thr Val Ala Ser Ala Ala Ala Asp
    450                 455                 460

Glu Thr Leu Arg Leu Trp Arg Cys Phe Glu Leu Asp Pro Ala Arg Arg
465                 470                 475                 480

Arg Glu Arg Glu Lys Ala Ser Ala Ala Lys Ser Ser Leu Ile His Gln
                485                 490                 495

Gly Ile Arg

<210>  200
<211>  140
<212>  PRT
<213>  Homo sapiens

<400>  200

Met Gly Pro Arg Arg Arg Ser Arg Lys Pro Glu Ala Pro Arg Arg Arg
1               5                   10                  15

Ser Pro Ser Pro Thr Pro Thr Pro Gly Pro Ser Arg Arg Gly Pro Ser
            20                  25                  30

Leu Gly Ala Ser Ser His Gln His Ser Arg Arg Arg Gln Gly Trp Leu
        35                  40                  45

Lys Glu Ile Arg Lys Leu Gln Lys Ser Thr His Leu Leu Ile Arg Lys
    50                  55                  60

Leu Pro Phe Ser Arg Leu Ala Arg Glu Ile Cys Val Lys Phe Thr Arg
65                  70                  75                  80

Gly Val Asp Phe Asn Trp Gln Ala Gln Ala Leu Leu Ala Leu Gln Glu
                85                  90                  95

Ala Ala Glu Ala Phe Leu Val His Leu Phe Glu Asp Ala Tyr Leu Leu
            100                 105                 110

Thr Leu His Ala Gly Arg Val Thr Leu Phe Pro Lys Asp Val Gln Leu
        115                 120                 125

Ala Arg Arg Ile Arg Gly Leu Glu Glu Gly Leu Gly
    130                 135                 140

<210>  201

<211>  3114
<212>  PRT
<213>  Homo sapiens

<400>  201

Met Ser Trp Ala Leu Glu Glu Trp Lys Glu Gly Leu Pro Thr Arg Ala
1               5                   10                  15

Leu Gln Lys Ile Gln Glu Leu Glu Gly Gln Leu Asp Lys Leu Lys Lys
            20                  25                  30

Glu Lys Gln Gln Arg Gln Phe Gln Leu Asp Ser Leu Glu Ala Ala Leu
        35                  40                  45

Gln Lys Gln Lys Gln Lys Val Glu Asn Glu Lys Thr Glu Gly Thr Asn
    50                  55                  60

Leu Lys Arg Glu Asn Gln Arg Leu Met Glu Ile Cys Glu Ser Leu Glu
65                  70                  75                  80

Lys Thr Lys Gln Lys Ile Ser His Glu Leu Gln Val Lys Glu Ser Gln
                85                  90                  95

Val Asn Phe Gln Glu Gly Gln Leu Asn Ser Gly Lys Lys Gln Ile Glu
            100                 105                 110

Lys Leu Glu Gln Glu Leu Lys Arg Cys Lys Ser Glu Leu Glu Arg Ser
        115                 120                 125

Gln Gln Ala Ala Gln Ser Ala Asp Val Ser Leu Asn Pro Cys Asn Thr
    130                 135                 140

Pro Gln Lys Ile Phe Thr Thr Pro Leu Thr Pro Ser Gln Tyr Tyr Ser
145                 150                 155                 160

Gly Ser Lys Tyr Glu Asp Leu Lys Glu Lys Tyr Asn Lys Glu Val Glu
                165                 170                 175

Glu Arg Lys Arg Leu Glu Ala Glu Val Lys Ala Leu Gln Ala Lys Lys
            180                 185                 190

Ala Ser Gln Thr Leu Pro Gln Ala Thr Met Asn His Arg Asp Ile Ala
        195                 200                 205

Arg His Gln Ala Ser Ser Ser Val Phe Ser Trp Gln Gln Glu Lys Thr
    210                 215                 220

Pro Ser His Leu Ser Ser Asn Ser Gln Arg Thr Pro Ile Arg Arg Asp
225                 230                 235                 240

Phe Ser Ala Ser Tyr Phe Ser Gly Glu Gln Glu Val Thr Pro Ser Arg
                245                 250                 255

Ser Thr Leu Gln Ile Gly Lys Arg Asp Ala Asn Ser Ser Phe Phe Asp
            260                 265                 270

Asn Ser Ser Ser Pro His Leu Leu Asp Gln Leu Lys Ala Gln Asn Gln
        275                 280                 285

Glu Leu Arg Asn Lys Ile Asn Glu Leu Glu Leu Arg Leu Gln Gly His
    290                 295                 300

Glu Lys Glu Met Lys Gly Gln Val Asn Lys Phe Gln Glu Leu Gln Leu
305                 310                 315                 320

Gln Leu Glu Lys Ala Lys Val Glu Leu Ile Glu Lys Glu Lys Val Leu
                325                 330                 335

Asn Lys Cys Arg Asp Glu Leu Val Arg Thr Thr Ala Gln Tyr Asp Gln
            340                 345                 350

Ala Ser Thr Lys Tyr Thr Ala Leu Glu Gln Lys Leu Lys Lys Leu Thr
        355                 360                 365

Glu Asp Leu Ser Cys Gln Arg Gln Asn Ala Glu Ser Ala Arg Cys Ser
    370                 375                 380

Leu Glu Gln Lys Ile Lys Glu Lys Glu Lys Glu Phe Gln Glu Glu Leu
385                 390                 395                 400

Ser Arg Gln Gln Arg Ser Phe Gln Thr Leu Asp Gln Glu Cys Ile Gln
                405                 410                 415

Met Lys Ala Arg Leu Thr Gln Glu Leu Gln Gln Ala Lys Asn Met His
            420                 425                 430

Asn Val Leu Gln Ala Glu Leu Asp Lys Leu Thr Ser Val Lys Gln Gln
        435                 440                 445

Leu Glu Asn Asn Leu Glu Glu Phe Lys Gln Lys Leu Cys Arg Ala Glu
    450                 455                 460

Gln Ala Phe Gln Ala Ser Gln Ile Lys Glu Asn Glu Leu Arg Arg Ser
465                 470                 475                 480

Met Glu Glu Met Lys Lys Glu Asn Asn Leu Leu Lys Ser His Ser Glu
                485                 490                 495

Gln Lys Ala Arg Glu Val Cys His Leu Glu Ala Glu Leu Lys Asn Ile
            500                 505                 510

Lys Gln Cys Leu Asn Gln Ser Gln Asn Phe Ala Glu Glu Met Lys Ala
        515                 520                 525

Lys Asn Thr Ser Gln Glu Thr Met Leu Arg Asp Leu Gln Glu Lys Ile
    530                 535                 540

Asn Gln Gln Glu Asn Ser Leu Thr Leu Glu Lys Leu Lys Leu Ala Val
545                 550                 555                 560

Ala Asp Leu Glu Lys Gln Arg Asp Cys Ser Gln Asp Leu Leu Lys Lys
                565                 570                 575

Arg Glu His His Ile Glu Gln Leu Asn Asp Lys Leu Ser Lys Thr Glu
            580                 585                 590

Lys Glu Ser Lys Ala Leu Leu Ser Ala Leu Glu Leu Lys Lys Lys Glu
        595                 600                 605

Tyr Glu Glu Leu Lys Glu Glu Lys Thr Leu Phe Ser Cys Trp Lys Ser
    610                 615                 620

Glu Asn Glu Lys Leu Leu Thr Gln Met Glu Ser Glu Lys Glu Asn Leu
625                 630                 635                 640

Gln Ser Lys Ile Asn His Leu Glu Thr Cys Leu Lys Thr Gln Gln Ile
                645                 650                 655

Lys Ser His Glu Tyr Asn Glu Arg Val Arg Thr Leu Glu Met Asp Arg
            660                 665                 670

Glu Asn Leu Ser Val Glu Ile Arg Asn Leu His Asn Val Leu Asp Ser
        675                 680                 685

Lys Ser Val Glu Val Glu Thr Gln Lys Leu Ala Tyr Met Glu Leu Gln
    690                 695                 700

Gln Lys Ala Glu Phe Ser Asp Gln Lys His Gln Lys Glu Ile Glu Asn
705                 710                 715                 720

Met Cys Leu Lys Thr Ser Gln Leu Thr Gly Gln Val Glu Asp Leu Glu
                725                 730                 735

His Lys Leu Gln Leu Leu Ser Asn Glu Ile Met Asp Lys Asp Arg Cys
            740                 745                 750

Tyr Gln Asp Leu His Ala Glu Tyr Glu Ser Leu Arg Asp Leu Leu Lys
        755                 760                 765

Ser Lys Asp Ala Ser Leu Val Thr Asn Glu Asp His Gln Arg Ser Leu
    770                 775                 780

Leu Ala Phe Asp Gln Gln Pro Ala Met His His Ser Phe Ala Asn Ile
785                 790                 795                 800

Ile Gly Glu Gln Gly Ser Met Pro Ser Glu Arg Ser Glu Cys Arg Leu
                805                 810                 815

Glu Ala Asp Gln Ser Pro Lys Asn Ser Ala Ile Leu Gln Asn Arg Val
            820                 825                 830

Asp Ser Leu Glu Phe Ser Leu Glu Ser Gln Lys Gln Met Asn Ser Asp
        835                 840                 845

Leu Gln Lys Gln Cys Glu Glu Leu Val Gln Ile Lys Gly Glu Ile Glu
    850                 855                 860

Glu Asn Leu Met Lys Ala Glu Gln Met His Gln Ser Phe Val Ala Glu
865                 870                 875                 880

Thr Ser Gln Arg Ile Ser Lys Leu Gln Glu Asp Thr Ser Ala His Gln
                885                 890                 895

Asn Val Val Ala Glu Thr Leu Ser Ala Leu Glu Asn Lys Glu Lys Glu
            900                 905                 910

Leu Gln Leu Leu Asn Asp Lys Val Glu Thr Glu Gln Ala Glu Ile Gln
        915                 920                 925

Glu Leu Lys Lys Ser Asn His Leu Leu Glu Asp Ser Leu Lys Glu Leu
    930                 935                 940

Gln Leu Leu Ser Glu Thr Leu Ser Leu Glu Lys Lys Glu Met Ser Ser
945                 950                 955                 960

Ile Ile Ser Leu Asn Lys Arg Glu Ile Glu Glu Leu Thr Gln Glu Asn
                965                 970                 975

Gly Thr Leu Lys Glu Ile Asn Ala Ser Leu Asn Gln Glu Lys Met Asn
            980                 985                 990

Leu Ile Gln Lys Ser Glu Ser Phe  Ala Asn Tyr Ile Asp  Glu Arg Glu
        995                 1000                 1005

Lys Ser  Ile Ser Glu Leu Ser  Asp Gln Tyr Lys Gln  Glu Lys Leu
    1010                 1015                 1020

Ile Leu  Leu Gln Arg Cys Glu  Glu Thr Gly Asn Ala  Tyr Glu Asp
    1025                 1030                 1035

Leu Ser  Gln Lys Tyr Lys Ala  Ala Gln Glu Lys Asn  Ser Lys Leu
    1040                 1045                 1050

Glu Cys  Leu Leu Asn Glu Cys  Thr Ser Leu Cys Glu  Asn Arg Lys
    1055                 1060                 1065

Asn Glu  Leu Glu Gln Leu Lys  Glu Ala Phe Ala Lys  Glu His Gln
    1070                 1075                 1080

Glu Phe  Leu Thr Lys Leu Ala  Phe Ala Glu Glu Arg  Asn Gln Asn
    1085                 1090                 1095

Leu Met Leu Glu Leu Glu Thr Val Gln Gln Ala Leu Arg Ser Glu
    1100                1105                1110

Met Thr Asp Asn Gln Asn Asn Ser Lys Ser Glu Ala Gly Gly Leu
    1115                1120                1125

Lys Gln Glu Ile Met Thr Leu Lys Glu Glu Gln Asn Lys Met Gln
    1130                1135                1140

Lys Glu Val Asn Asp Leu Leu Gln Glu Asn Glu Gln Leu Met Lys
    1145                1150                1155

Val Met Lys Thr Lys His Glu Cys Gln Asn Leu Glu Ser Glu Pro
    1160                1165                1170

Ile Arg Asn Ser Val Lys Glu Arg Glu Ser Glu Arg Asn Gln Cys
    1175                1180                1185

Asn Phe Lys Pro Gln Met Asp Leu Glu Val Lys Glu Ile Ser Leu
    1190                1195                1200

Asp Ser Tyr Asn Ala Gln Leu Val Gln Leu Glu Ala Met Leu Arg
    1205                1210                1215

Asn Lys Glu Leu Lys Leu Gln Glu Ser Glu Lys Glu Lys Glu Cys
    1220                1225                1230

Leu Gln His Glu Leu Gln Thr Ile Arg Gly Asp Leu Glu Thr Ser
    1235                1240                1245

Asn Leu Gln Asp Met Gln Ser Gln Glu Ile Ser Gly Leu Lys Asp
    1250                1255                1260

Cys Glu Ile Asp Ala Glu Glu Lys Tyr Ile Ser Gly Pro His Glu
    1265                1270                1275

Leu Ser Thr Ser Gln Asn Asp Asn Ala His Leu Gln Cys Ser Leu
    1280                1285                1290

Gln Thr Thr Met Asn Lys Leu Asn Glu Leu Glu Lys Ile Cys Glu
    1295                1300                1305

Ile Leu Gln Ala Glu Lys Tyr Glu Leu Val Thr Glu Leu Asn Asp
    1310                1315                1320

Ser Arg Ser Glu Cys Ile Thr Ala Thr Arg Lys Met Ala Glu Glu
    1325                1330                1335

Val Gly Lys Leu Leu Asn Glu Val Lys Ile Leu Asn Asp Asp Ser
    1340                1345                1350

Gly Leu Leu His Gly Glu Leu Val Glu Asp Ile Pro Gly Gly Glu
    1355                1360                1365

Phe Gly Glu Gln Pro Asn Glu Gln His Pro Val Ser Leu Ala Pro
    1370                1375                1380

Leu Asp Glu Ser Asn Ser Tyr Glu His Leu Thr Leu Ser Asp Lys
    1385                1390                1395

Glu Val Gln Met His Phe Ala Glu Leu Gln Glu Lys Phe Leu Ser
    1400                1405                1410

Leu Gln Ser Glu His Lys Ile Leu His Asp Gln His Cys Gln Met
    1415                1420                1425

Ser Ser Lys Met Ser Glu Leu Gln Thr Tyr Val Asp Ser Leu Lys
    1430                1435                1440

Ala Glu Asn Leu Val Leu Ser Thr Asn Leu Arg Asn Phe Gln Gly
    1445                1450                1455

Asp Leu Val Lys Glu Met Gln Leu Gly Leu Glu Glu Gly Leu Val
    1460                1465                1470

Pro Ser Leu Ser Ser Ser Cys Val Pro Asp Ser Ser Ser Leu Ser
    1475                1480                1485

Ser Leu Gly Asp Ser Ser Phe Tyr Arg Ala Leu Leu Glu Gln Thr
    1490                1495                1500

Gly Asp Met Ser Leu Leu Ser Asn Leu Glu Gly Ala Val Ser Ala
    1505                1510                1515

Asn Gln Cys Ser Val Asp Glu Val Phe Cys Ser Ser Leu Gln Glu
    1520                1525                1530

Glu Asn Leu Thr Arg Lys Glu Thr Pro Ser Ala Pro Ala Lys Gly
    1535                1540                1545

Val Glu Glu Leu Glu Ser Leu Cys Glu Val Tyr Arg Gln Ser Leu
    1550                1555                1560

Glu Lys Leu Glu Glu Lys Met Glu Ser Gln Gly Ile Met Lys Asn
    1565                1570                1575

Lys Glu Ile Gln Glu Leu Glu Gln Leu Leu Ser Ser Glu Arg Gln
    1580                1585                1590

Glu Leu Asp Cys Leu Arg Lys Gln Tyr Leu Ser Glu Asn Glu Gln
    1595                1600                1605

Trp Gln Gln Lys Leu Thr Ser Val Thr Leu Glu Met Glu Ser Lys
    1610                1615                1620

Leu Ala Ala Glu Lys Lys Gln Thr Glu Gln Leu Ser Leu Glu Leu
    1625                1630                1635

Glu Val Ala Arg Leu Gln Leu Gln Gly Leu Asp Leu Ser Ser Arg
    1640                1645                1650

Ser Leu Leu Gly Ile Asp Thr Glu Asp Ala Ile Gln Gly Arg Asn
    1655                1660                1665

Glu Ser Cys Asp Ile Ser Lys Glu His Thr Ser Glu Thr Thr Glu
    1670                1675                1680

Arg Thr Pro Lys His Asp Val His Gln Ile Cys Asp Lys Asp Ala
    1685                1690                1695

Gln Gln Asp Leu Asn Leu Asp Ile Glu Lys Ile Thr Glu Thr Gly
    1700                1705                1710

Ala Val Lys Pro Thr Gly Glu Cys Ser Gly Glu Gln Ser Pro Asp
    1715                1720                1725

Thr Asn Tyr Glu Pro Pro Gly Glu Asp Lys Thr Gln Gly Ser Ser
    1730                1735                1740

Glu Cys Ile Ser Glu Leu Ser Phe Ser Gly Pro Asn Ala Leu Val
    1745                1750                1755

Pro Met Asp Phe Leu Gly Asn Gln Glu Asp Ile His Asn Leu Gln
    1760                1765                1770

Leu Arg Val Lys Glu Thr Ser Asn Glu Asn Leu Arg Leu Leu His
    1775                1780                1785

Val Ile Glu Asp Arg Asp Arg Lys Val Glu Ser Leu Leu Asn Glu
    1790                1795                1800

Met Lys Glu Leu Asp Ser Lys Leu His Leu Gln Glu Val Gln Leu
    1805                1810                1815

Met Thr Lys Ile Glu Ala Cys Ile Glu Leu Glu Lys Ile Val Gly
    1820                1825                1830

Glu Leu Lys Lys Glu Asn Ser Asp Leu Ser Glu Lys Leu Glu Tyr
    1835                1840                1845

Phe Ser Cys Asp His Gln Glu Leu Leu Gln Arg Val Glu Thr Ser
    1850                1855                1860

Glu Gly Leu Asn Ser Asp Leu Glu Met His Ala Asp Lys Ser Ser
    1865                1870                1875

Arg Glu Asp Ile Gly Asp Asn Val Ala Lys Val Asn Asp Ser Trp
    1880                1885                1890

Lys Glu Arg Phe Leu Asp Val Glu Asn Glu Leu Ser Arg Ile Arg
    1895                1900                1905

Ser Glu Lys Ala Ser Ile Glu His Glu Ala Leu Tyr Leu Glu Ala
    1910                1915                1920

Asp Leu Glu Val Val Gln Thr Glu Lys Leu Cys Leu Glu Lys Asp
    1925                1930                1935

Asn Glu Asn Lys Gln Lys Val Ile Val Cys Leu Glu Glu Glu Leu
    1940                1945                1950

Ser Val Val Thr Ser Glu Arg Asn Gln Leu Arg Gly Glu Leu Asp
    1955                1960                1965

Thr Met Ser Lys Lys Thr Thr Ala Leu Asp Gln Leu Ser Glu Lys
    1970                1975                1980

Met Lys Glu Lys Thr Gln Glu Leu Glu Ser His Gln Ser Glu Cys
    1985                1990                1995

Leu His Cys Ile Gln Val Ala Glu Ala Glu Val Lys Glu Lys Thr
    2000                2005                2010

Glu Leu Leu Gln Thr Leu Ser Ser Asp Val Ser Glu Leu Leu Lys
    2015                2020                2025

Asp Lys Thr His Leu Gln Glu Lys Leu Gln Ser Leu Glu Lys Asp
    2030                2035                2040

Ser Gln Ala Leu Ser Leu Thr Lys Cys Glu Leu Glu Asn Gln Ile
    2045                2050                2055

Ala Gln Leu Asn Lys Glu Lys Glu Leu Leu Val Lys Glu Ser Glu
    2060                2065                2070

Ser Leu Gln Ala Arg Leu Ser Glu Ser Asp Tyr Glu Lys Leu Asn
    2075                2080                2085

Val Ser Lys Ala Leu Glu Ala Ala Leu Val Glu Lys Gly Glu Phe
    2090                2095                2100

Ala Leu Arg Leu Ser Ser Thr Gln Glu Glu Val His Gln Leu Arg
    2105                2110                2115

Arg Gly Ile Glu Lys Leu Arg Val Arg Ile Glu Ala Asp Glu Lys
    2120                2125                2130

Lys Gln Leu His Ile Ala Glu Lys Leu Lys Glu Arg Glu Arg Glu
    2135                2140                2145

Asn Asp Ser Leu Lys Asp Lys Val Glu Asn Leu Glu Arg Glu Leu
    2150                2155                2160

Gln Met Ser Glu Glu Asn Gln Glu Leu Val Ile Leu Asp Ala Glu
    2165                2170                2175

Asn Ser Lys Ala Glu Val Glu Thr Leu Lys Thr Gln Ile Glu Glu
    2180                2185                2190

Met Ala Arg Ser Leu Lys Val Phe Glu Leu Asp Leu Val Thr Leu
    2195                2200                2205

Arg Ser Glu Lys Glu Asn Leu Thr Lys Gln Ile Gln Glu Lys Gln
    2210                2215                2220

Gly Gln Leu Ser Glu Leu Asp Lys Leu Leu Ser Ser Phe Lys Ser
    2225                2230                2235

Leu Leu Glu Glu Lys Glu Gln Ala Glu Ile Gln Ile Lys Glu Glu
    2240                2245                2250

Ser Lys Thr Ala Val Glu Met Leu Gln Asn Gln Leu Lys Glu Leu
    2255                2260                2265

Asn Glu Ala Val Ala Ala Leu Cys Gly Asp Gln Glu Ile Met Lys
    2270                2275                2280

Ala Thr Glu Gln Ser Leu Asp Pro Pro Ile Glu Glu Glu His Gln
    2285                2290                2295

Leu Arg Asn Ser Ile Glu Lys Leu Arg Ala Arg Leu Glu Ala Asp
    2300                2305                2310

Glu Lys Lys Gln Leu Cys Val Leu Gln Gln Leu Lys Glu Ser Glu
    2315                2320                2325

His His Ala Asp Leu Leu Lys Gly Arg Val Glu Asn Leu Glu Arg
    2330                2335                2340

Glu Leu Glu Ile Ala Arg Thr Asn Gln Glu His Ala Ala Leu Glu
    2345                2350                2355

Ala Glu Asn Ser Lys Gly Glu Val Glu Thr Leu Lys Ala Lys Ile
    2360                2365                2370

Glu Gly Met Thr Gln Ser Leu Arg Gly Leu Glu Leu Asp Val Val
    2375                2380                2385

Thr Ile Arg Ser Glu Lys Glu Asn Leu Thr Asn Glu Leu Gln Lys
    2390                2395                2400

Glu Gln Glu Arg Ile Ser Glu Leu Glu Ile Ile Asn Ser Ser Phe
    2405                2410                2415

Glu Asn Ile Leu Gln Glu Lys Glu Gln Glu Lys Val Gln Met Lys
    2420                2425                2430

Glu Lys Ser Ser Thr Ala Met Glu Met Leu Gln Thr Gln Leu Lys
    2435                2440                2445

Glu Leu Asn Glu Arg Val Ala Ala Leu His Asn Asp Gln Glu Ala
    2450                2455                2460

Cys Lys Ala Lys Glu Gln Asn Leu Ser Ser Gln Val Glu Cys Leu
    2465                2470                2475

Glu Leu Glu Lys Ala Gln Leu Leu Gln Gly Leu Asp Glu Ala Lys
    2480                2485                2490

Asn Asn Tyr Ile Val Leu Gln Ser Ser Val Asn Gly Leu Ile Gln
    2495                2500                2505

Glu Val Glu Asp Gly Lys Gln Lys Leu Glu Lys Lys Asp Glu Glu
    2510                2515                2520

Ile Ser Arg Leu Lys Asn Gln Ile Gln Asp Gln Glu Gln Leu Val
    2525                2530                2535

Ser Lys Leu Ser Gln Val Glu Gly Glu His Gln Leu Trp Lys Glu
    2540                2545                2550

Gln Asn Leu Glu Leu Arg Asn Leu Thr Val Glu Leu Glu Gln Lys
    2555                2560                2565

Ile Gln Val Leu Gln Ser Lys Asn Ala Ser Leu Gln Asp Thr Leu
    2570                2575                2580

Glu Val Leu Gln Ser Ser Tyr Lys Asn Leu Glu Asn Glu Leu Glu
    2585                2590                2595

Leu Thr Lys Met Asp Lys Met Ser Phe Val Glu Lys Val Asn Lys
    2600                2605                2610

Met Thr Ala Lys Glu Thr Glu Leu Gln Arg Glu Met His Glu Met
    2615                2620                2625

Ala Gln Lys Thr Ala Glu Leu Gln Glu Glu Leu Ser Gly Glu Lys
    2630                2635                2640

Asn Arg Leu Ala Gly Glu Leu Gln Leu Leu Leu Glu Glu Ile Lys
    2645                2650                2655

Ser Ser Lys Asp Gln Leu Lys Glu Leu Thr Leu Glu Asn Ser Glu
    2660                2665                2670

Leu Lys Lys Ser Leu Asp Cys Met His Lys Asp Gln Val Glu Lys
    2675                2680                2685

Glu Gly Lys Val Arg Glu Glu Ile Ala Glu Tyr Gln Leu Arg Leu
    2690                2695                2700

His Glu Ala Glu Lys Lys His Gln Ala Leu Leu Leu Asp Thr Asn
    2705                2710                2715

Lys Gln Tyr Glu Val Glu Ile Gln Thr Tyr Arg Glu Lys Leu Thr
    2720                2725                2730

Ser Lys Glu Glu Cys Leu Ser Ser Gln Lys Leu Glu Ile Asp Leu
    2735                2740                2745

Leu Lys Ser Ser Lys Glu Glu Leu Asn Asn Ser Leu Lys Ala Thr
    2750                2755                2760

Thr Gln Ile Leu Glu Glu Leu Lys Lys Thr Lys Met Asp Asn Leu
    2765                2770                2775

Lys Tyr Val Asn Gln Leu Lys Lys Glu Asn Glu Arg Ala Gln Gly
    2780                2785                2790

Lys Met Lys Leu Leu Ile Lys Ser Cys Lys Gln Leu Glu Glu Glu
    2795                2800                2805

Lys Glu Ile Leu Gln Lys Glu Leu Ser Gln Leu Gln Ala Ala Gln
    2810                2815                2820

Glu Lys Gln Lys Thr Gly Thr Val Met Asp Thr Lys Val Asp Glu
    2825                2830                2835

Leu Thr Thr Glu Ile Lys Glu Leu Lys Glu Thr Leu Glu Glu Lys
    2840                2845                2850

Thr Lys Glu Ala Asp Glu Tyr Leu Asp Lys Tyr Cys Ser Leu Leu
    2855                2860                2865

Ile Ser His Glu Lys Leu Glu Lys Ala Lys Glu Met Leu Glu Thr
    2870                2875                2880

Gln Val Ala His Leu Cys Ser Gln Gln Ser Lys Gln Asp Ser Arg
    2885                2890                2895

Gly Ser Pro Leu Leu Gly Pro Val Val Pro Gly Pro Ser Pro Ile
    2900                2905                2910

Pro Ser Val Thr Glu Lys Arg Leu Ser Ser Gly Gln Asn Lys Ala
    2915                2920                2925

Ser Gly Lys Arg Gln Arg Ser Ser Gly Ile Trp Glu Asn Gly Arg
    2930                2935                2940

Gly Pro Thr Pro Ala Thr Pro Glu Ser Phe Ser Lys Lys Ser Lys
    2945                2950                2955

Lys Ala Val Met Ser Gly Ile His Pro Ala Glu Asp Thr Glu Gly
    2960                2965                2970

Thr Glu Phe Glu Pro Glu Gly Leu Pro Glu Val Val Lys Lys Gly
    2975                2980                2985

Phe Ala Asp Ile Pro Thr Gly Lys Thr Ser Pro Tyr Ile Leu Arg
    2990                2995                3000

Arg Thr Thr Met Ala Thr Arg Thr Ser Pro Arg Leu Ala Ala Gln
    3005                3010                3015

Lys Leu Ala Leu Ser Pro Leu Ser Leu Gly Lys Glu Asn Leu Ala
    3020                3025                3030

Glu Ser Ser Lys Pro Thr Ala Gly Gly Ser Arg Ser Gln Lys Val
    3035                3040                3045

Lys Val Ala Gln Arg Ser Pro Val Asp Ser Gly Thr Ile Leu Arg
    3050                3055                3060

Glu Pro Thr Thr Lys Ser Val Pro Val Asn Asn Leu Pro Glu Arg
    3065                3070                3075

Ser Pro Thr Asp Ser Pro Arg Glu Gly Leu Arg Val Lys Arg Gly
    3080                3085                3090

Arg Leu Val Pro Ser Pro Lys Ala Gly Leu Glu Ser Asn Gly Ser
    3095                3100                3105

Glu Asn Cys Lys Val Gln
    3110

<210> 202
<211> 476
<212> PRT
<213> Homo sapiens

<400> 202

Met Ala Val Pro Phe Val Glu Asp Trp Asp Leu Val Gln Thr Leu Gly
1               5                   10                  15

Glu Gly Ala Tyr Gly Glu Val Gln Leu Ala Val Asn Arg Val Thr Glu
            20                  25                  30

Glu Ala Val Ala Val Lys Ile Val Asp Met Lys Arg Ala Val Asp Cys
        35                  40                  45

Pro Glu Asn Ile Lys Lys Glu Ile Cys Ile Asn Lys Met Leu Asn His
    50                  55                  60

Glu Asn Val Val Lys Phe Tyr Gly His Arg Arg Glu Gly Asn Ile Gln
65                  70                  75                  80

Tyr Leu Phe Leu Glu Tyr Cys Ser Gly Gly Glu Leu Phe Asp Arg Ile
                85                  90                  95

Glu Pro Asp Ile Gly Met Pro Glu Pro Asp Ala Gln Arg Phe Phe His
            100                 105                 110

Gln Leu Met Ala Gly Val Val Tyr Leu His Gly Ile Gly Ile Thr His
        115                 120                 125

Arg Asp Ile Lys Pro Glu Asn Leu Leu Leu Asp Glu Arg Asp Asn Leu
    130                 135                 140

Lys Ile Ser Asp Phe Gly Leu Ala Thr Val Phe Arg Tyr Asn Asn Arg
145                 150                 155                 160

Glu Arg Leu Leu Asn Lys Met Cys Gly Thr Leu Pro Tyr Val Ala Pro
                165                 170                 175

Glu Leu Leu Lys Arg Arg Glu Phe His Ala Glu Pro Val Asp Val Trp
            180                 185                 190

Ser Cys Gly Ile Val Leu Thr Ala Met Leu Ala Gly Glu Leu Pro Trp
        195                 200                 205

Asp Gln Pro Ser Asp Ser Cys Gln Glu Tyr Ser Asp Trp Lys Glu Lys
    210                 215                 220

Lys Thr Tyr Leu Asn Pro Trp Lys Lys Ile Asp Ser Ala Pro Leu Ala
225                 230                 235                 240

Leu Leu His Lys Ile Leu Val Glu Asn Pro Ser Ala Arg Ile Thr Ile
                245                 250                 255

Pro Asp Ile Lys Lys Asp Arg Trp Tyr Asn Lys Pro Leu Lys Lys Gly
            260                 265                 270

Ala Lys Arg Pro Arg Val Thr Ser Gly Gly Val Ser Glu Ser Pro Ser
        275                 280                 285

Gly Phe Ser Lys His Ile Gln Ser Asn Leu Asp Phe Ser Pro Val Asn
    290                 295                 300

Ser Ala Ser Ser Glu Glu Asn Val Lys Tyr Ser Ser Ser Gln Pro Glu
305                 310                 315                 320

Pro Arg Thr Gly Leu Ser Leu Trp Asp Thr Ser Pro Ser Tyr Ile Asp

325                 330                 335

Lys Leu Val Gln Gly Ile Ser Phe Ser Gln Pro Thr Cys Pro Asp His
            340                 345                 350

Met Leu Leu Asn Ser Gln Leu Leu Gly Thr Pro Gly Ser Ser Gln Asn
        355                 360                 365

Pro Trp Gln Arg Leu Val Lys Arg Met Thr Arg Phe Phe Thr Lys Leu
    370                 375                 380

Asp Ala Asp Lys Ser Tyr Gln Cys Leu Lys Glu Thr Cys Glu Lys Leu
385                 390                 395                 400

Gly Tyr Gln Trp Lys Lys Ser Cys Met Asn Gln Val Thr Ile Ser Thr
                405                 410                 415

Thr Asp Arg Arg Asn Asn Lys Leu Ile Phe Lys Val Asn Leu Leu Glu
            420                 425                 430

Met Asp Asp Lys Ile Leu Val Asp Phe Arg Leu Ser Lys Gly Asp Gly
        435                 440                 445

Leu Glu Phe Lys Arg His Phe Leu Lys Ile Lys Gly Lys Leu Ile Asp
    450                 455                 460

Ile Val Ser Ser Gln Lys Val Trp Leu Pro Ala Thr
465                 470                 475

<210>  203
<211>  79
<212>  PRT
<213>  Homo sapiens

<400>  203

<210>  204
<211>  391
<212>  PRT
<213>  Homo sapiens

<400> 204

<210>  205
<211>  391
<212>  PRT
<213>  Homo sapiens

<400>  205

<210>  206
<211>  255
<212>  PRT
<213>  Homo sapiens

<400>  206

Met Tyr Glu Ile Leu Lys Ala Leu Asp Tyr Cys His Ser Met Gly Ile
1               5                   10                  15

Met His Arg Asp Val Lys Pro His Asn Val Met Ile Asp His Glu His
            20                  25                  30

Arg Lys Leu Arg Leu Ile Asp Trp Gly Leu Ala Glu Phe Tyr His Pro
        35                  40                  45

Gly Gln Glu Tyr Asn Val Arg Val Ala Ser Arg Tyr Phe Lys Gly Pro
    50                  55                  60

Glu Leu Leu Val Asp Tyr Gln Met Tyr Asp Tyr Ser Leu Asp Met Trp
65                  70                  75                  80

Ser Leu Gly Cys Met Leu Ala Ser Met Ile Phe Arg Lys Glu Pro Phe
                85                  90                  95

Phe His Gly His Asp Asn Tyr Asp Gln Leu Val Arg Ile Ala Lys Val
            100                 105                 110

Leu Gly Thr Glu Asp Leu Tyr Asp Tyr Ile Asp Lys Tyr Asn Ile Glu
        115                 120                 125

Leu Asp Pro Arg Phe Asn Asp Ile Leu Gly Arg His Ser Arg Lys Arg
    130                 135                 140

Trp Glu Arg Phe Val His Ser Glu Asn Gln His Leu Val Ser Pro Glu
145                 150                 155                 160

Ala Leu Asp Phe Leu Asp Lys Leu Leu Arg Tyr Asp His Gln Ser Arg
                165                 170                 175

Leu Thr Ala Arg Glu Ala Met Glu His Pro Tyr Phe Tyr Thr Val Val
            180                 185                 190

Lys Asp Gln Ala Arg Met Gly Ser Ser Ser Met Pro Gly Gly Ser Thr
        195                 200                 205

Pro Val Ser Ser Ala Asn Met Met Ser Gly Ile Ser Ser Val Pro Thr
    210                 215                 220

Pro Ser Pro Leu Gly Pro Leu Ala Gly Ser Pro Val Ile Ala Ala Ala
225                 230                 235                 240

Asn Pro Leu Gly Met Pro Val Pro Ala Ala Ala Gly Ala Gln Gln
                245                 250                 255

<210>  207
<211>  853
<212>  PRT
<213>  Homo sapiens

<400>  207

<210>  208
<211>  833
<212>  PRT
<213>  Homo sapiens

<400>  208

Met Lys Gly Asp Thr Arg His Leu Asn Gly Glu Glu Asp Ala Gly Gly
1               5                   10                  15

Arg Glu Asp Ser Ile Leu Val Asn Gly Ala Cys Ser Asp Gln Ser Ser
            20                  25                  30

Asp Ser Pro Pro Ile Leu Glu Ala Ile Arg Thr Pro Glu Ile Arg Gly
        35                  40                  45

Arg Arg Ser Ser Ser Arg Leu Ser Lys Arg Glu Val Ser Ser Leu Leu
    50                  55                  60

Ser Tyr Thr Gln Asp Leu Thr Gly Asp Gly Asp Gly Glu Asp Gly Asp
65                  70                  75                  80

Gly Ser Asp Thr Pro Val Met Pro Lys Leu Phe Arg Glu Thr Arg Thr
                85                  90                  95

Arg Ser Glu Ser Pro Ala Val Arg Thr Arg Asn Asn Asn Ser Val Ser
            100                 105                 110

Ser Arg Glu Arg His Arg Pro Ser Pro Arg Ser Thr Arg Gly Arg Gln
        115                 120                 125

Gly Arg Asn His Val Asp Glu Ser Pro Val Glu Phe Pro Ala Thr Arg
    130                 135                 140

Ser Leu Arg Arg Arg Ala Thr Ala Ser Ala Gly Thr Pro Trp Pro Ser
145                 150                 155                 160

Pro Pro Ser Ser Tyr Leu Thr Ile Asp Leu Thr Asp Asp Thr Glu Asp
                165                 170                 175

Thr His Gly Thr Pro Gln Ser Ser Ser Thr Pro Tyr Ala Arg Leu Ala
            180                 185                 190

Gln Asp Ser Gln Gln Gly Gly Met Glu Ser Pro Gln Val Glu Ala Asp

195                 200                 205

Ser Gly Asp Gly Asp Ser Ser Glu Tyr Gln Asp Gly Lys Glu Phe Gly
    210                 215                 220

Ile Gly Asp Leu Val Trp Gly Lys Ile Lys Gly Phe Ser Trp Trp Pro
225                 230                 235                 240

Ala Met Val Val Ser Trp Lys Ala Thr Ser Lys Arg Gln Ala Met Ser
                245                 250                 255

Gly Met Arg Trp Val Gln Trp Phe Gly Asp Gly Lys Phe Ser Glu Val
            260                 265                 270

Ser Ala Asp Lys Leu Val Ala Leu Gly Leu Phe Ser Gln His Phe Asn
        275                 280                 285

Leu Ala Thr Phe Asn Lys Leu Val Ser Tyr Arg Lys Ala Met Tyr His
    290                 295                 300

Ala Leu Glu Lys Ala Arg Val Arg Ala Gly Lys Thr Phe Pro Ser Ser
305                 310                 315                 320

Pro Gly Asp Ser Leu Glu Asp Gln Leu Lys Pro Met Leu Glu Trp Ala
                325                 330                 335

His Gly Gly Phe Lys Pro Thr Gly Ile Glu Gly Leu Lys Pro Asn Asn
            340                 345                 350

Thr Gln Pro Glu Asn Lys Thr Arg Arg Arg Thr Ala Asp Asp Ser Ala
        355                 360                 365

Thr Ser Asp Tyr Cys Pro Ala Pro Lys Arg Leu Lys Thr Asn Cys Tyr
    370                 375                 380

Asn Asn Gly Lys Asp Arg Gly Asp Glu Asp Gln Ser Arg Glu Gln Met
385                 390                 395                 400

Ala Ser Asp Val Ala Asn Asn Lys Ser Ser Leu Glu Asp Gly Cys Leu
                405                 410                 415

Ser Cys Gly Arg Lys Asn Pro Val Ser Phe His Pro Leu Phe Glu Gly
            420                 425                 430

Gly Leu Cys Gln Thr Cys Arg Asp Arg Phe Leu Glu Leu Phe Tyr Met
        435                 440                 445

Tyr Asp Asp Asp Gly Tyr Gln Ser Tyr Cys Thr Val Cys Cys Glu Gly
    450                 455                 460

Arg Glu Leu Leu Leu Cys Ser Asn Thr Ser Cys Cys Arg Cys Phe Cys
465                 470                 475                 480

Val Glu Cys Leu Glu Val Leu Val Gly Thr Gly Thr Ala Ala Glu Ala
                485                 490                 495

Lys Leu Gln Glu Pro Trp Ser Cys Tyr Met Cys Leu Pro Gln Arg Cys
            500                 505                 510

His Gly Val Leu Arg Arg Arg Lys Asp Trp Asn Val Arg Leu Gln Ala
        515                 520                 525

Phe Phe Thr Ser Asp Thr Gly Leu Glu Tyr Glu Ala Pro Lys Leu Tyr
    530                 535                 540

Pro Ala Ile Pro Ala Ala Arg Arg Arg Pro Ile Arg Val Leu Ser Leu
545                 550                 555                 560

Phe Asp Gly Ile Ala Thr Gly Tyr Leu Val Leu Lys Glu Leu Gly Ile
                565                 570                 575

Lys Val Gly Lys Tyr Val Ala Ser Glu Val Cys Glu Glu Ser Ile Ala
            580                 585                 590

Val Gly Thr Val Lys His Glu Gly Asn Ile Lys Tyr Val Asn Asp Val
        595                 600                 605

Arg Asn Ile Thr Lys Lys Asn Ile Glu Glu Trp Gly Pro Phe Asp Leu
    610                 615                 620

Val Ile Gly Gly Ser Pro Cys Asn Asp Leu Ser Asn Val Asn Pro Ala
625                 630                 635                 640

Arg Lys Gly Leu Tyr Glu Gly Thr Gly Arg Leu Phe Phe Glu Phe Tyr
                645                 650                 655

His Leu Leu Asn Tyr Ser Arg Pro Lys Glu Gly Asp Asp Arg Pro Phe
            660                 665                 670

Phe Trp Met Phe Glu Asn Val Val Ala Met Lys Val Gly Asp Lys Arg
        675                 680                 685

Asp Ile Ser Arg Phe Leu Glu Cys Asn Pro Val Met Ile Asp Ala Ile
    690                 695                 700

Lys Val Ser Ala Ala His Arg Ala Arg Tyr Phe Trp Gly Asn Leu Pro
705                 710                 715                 720

Gly Met Asn Arg Pro Val Ile Ala Ser Lys Asn Asp Lys Leu Glu Leu
                725                 730                 735

Gln Asp Cys Leu Glu Tyr Asn Arg Ile Ala Lys Leu Lys Lys Val Gln
            740                 745                 750

Thr Ile Thr Thr Lys Ser Asn Ser Ile Lys Gln Gly Lys Asn Gln Leu

755                 760                 765

Phe Pro Val Val Met Asn Gly Lys Glu Asp Val Leu Trp Cys Thr Glu
    770                 775                 780

Leu Glu Arg Ile Phe Gly Phe Pro Val His Tyr Thr Asp Val Ser Asn
785                 790                 795                 800

Met Gly Arg Gly Ala Arg Gln Lys Leu Leu Gly Arg Ser Trp Ser Val
                805                 810                 815

Pro Val Ile Arg His Leu Phe Ala Pro Leu Lys Asp Tyr Phe Ala Cys
            820                 825                 830

Glu

<210>  209
<211>  770
<212>  PRT
<213>  Homo sapiens

<400>  209

Met Lys Gly Asp Thr Arg His Leu Asn Gly Glu Glu Asp Ala Gly Gly
1               5                   10                  15

Arg Glu Asp Ser Ile Leu Val Asn Gly Ala Cys Ser Asp Gln Ser Ser
            20                  25                  30

Asp Ser Pro Pro Ile Leu Glu Ala Ile Arg Thr Pro Glu Ile Arg Gly
        35                  40                  45

Arg Arg Ser Ser Ser Arg Leu Ser Lys Arg Glu Val Ser Ser Leu Leu
    50                  55                  60

Ser Tyr Thr Gln Asp Leu Thr Gly Asp Gly Asp Gly Glu Asp Gly Asp
65                  70                  75                  80

Gly Ser Asp Thr Pro Val Met Pro Lys Leu Phe Arg Glu Thr Arg Thr
                85                  90                  95

Arg Ser Glu Ser Pro Ala Val Arg Thr Arg Asn Asn Asn Ser Val Ser
            100                 105                 110

Ser Arg Glu Arg His Arg Pro Ser Pro Arg Ser Thr Arg Gly Arg Gln
        115                 120                 125

Gly Arg Asn His Val Asp Glu Ser Pro Val Glu Phe Pro Ala Thr Arg
    130                 135                 140

Ser Leu Arg Arg Arg Ala Thr Ala Ser Ala Gly Thr Pro Trp Pro Ser
145                 150                 155                 160

Pro Pro Ser Ser Tyr Leu Thr Ile Asp Leu Thr Asp Asp Thr Glu Asp
                165                 170                 175

Thr His Gly Thr Pro Gln Ser Ser Ser Thr Pro Tyr Ala Arg Leu Ala
            180                 185                 190

Gln Asp Ser Gln Gln Gly Gly Met Glu Ser Pro Gln Val Glu Ala Asp
        195                 200                 205

Ser Gly Asp Gly Asp Ser Ser Glu Tyr Gln Asp Gly Lys Glu Phe Gly
    210                 215                 220

Ile Gly Asp Leu Val Trp Gly Lys Ile Lys Gly Phe Ser Trp Trp Pro
225                 230                 235                 240

Ala Met Val Val Ser Trp Lys Ala Thr Ser Lys Arg Gln Ala Met Ser
                245                 250                 255

Gly Met Arg Trp Val Gln Trp Phe Gly Asp Gly Lys Phe Ser Glu Val
            260                 265                 270

Ser Ala Asp Lys Leu Val Ala Leu Gly Leu Phe Ser Gln His Phe Asn
        275                 280                 285

Leu Ala Thr Phe Asn Lys Leu Val Ser Tyr Arg Lys Ala Met Tyr His
    290                 295                 300

Ala Leu Glu Lys Ala Arg Val Arg Ala Gly Lys Thr Phe Pro Ser Ser
305                 310                 315                 320

Pro Gly Asp Ser Leu Glu Asp Gln Leu Lys Pro Met Leu Glu Trp Ala
                325                 330                 335

His Gly Gly Phe Lys Pro Thr Gly Ile Glu Gly Leu Lys Pro Asn Asn
            340                 345                 350

Thr Gln Pro Glu Asn Lys Thr Arg Arg Arg Thr Ala Asp Asp Ser Ala
        355                 360                 365

Thr Ser Asp Tyr Cys Pro Ala Pro Lys Arg Leu Lys Thr Asn Cys Tyr
    370                 375                 380

Asn Asn Gly Lys Asp Arg Gly Asp Glu Asp Gln Ser Arg Glu Gln Met
385                 390                 395                 400

Ala Ser Asp Val Ala Asn Asn Lys Ser Ser Leu Glu Asp Gly Cys Leu
                405                 410                 415

Ser Cys Gly Arg Lys Asn Pro Val Ser Phe His Pro Leu Phe Glu Gly
            420                 425                 430

Gly Leu Cys Gln Thr Cys Arg Asp Arg Phe Leu Glu Leu Phe Tyr Met
        435                 440                 445

Tyr Asp Asp Asp Gly Tyr Gln Ser Tyr Cys Thr Val Cys Cys Glu Gly
    450                 455                 460

Arg Glu Leu Leu Leu Cys Ser Asn Thr Ser Cys Cys Arg Cys Phe Cys
465                 470                 475                 480

Val Glu Cys Leu Glu Val Leu Val Gly Thr Gly Thr Ala Ala Glu Ala
                485                 490                 495

Lys Leu Gln Glu Pro Trp Ser Cys Tyr Met Cys Leu Pro Gln Arg Cys
            500                 505                 510

His Gly Val Leu Arg Arg Arg Lys Asp Trp Asn Val Arg Leu Gln Ala
        515                 520                 525

Phe Phe Thr Ser Asp Thr Gly Leu Glu Tyr Glu Ala Pro Lys Leu Tyr
    530                 535                 540

Pro Ala Ile Pro Ala Ala Arg Arg Arg Pro Ile Arg Val Leu Ser Leu
545                 550                 555                 560

Phe Asp Gly Ile Ala Thr Gly Tyr Leu Val Leu Lys Glu Leu Gly Ile
                565                 570                 575

Lys Val Gly Lys Tyr Val Ala Ser Glu Val Cys Glu Glu Ser Ile Ala
            580                 585                 590

Val Gly Thr Val Lys His Glu Gly Asn Ile Lys Tyr Val Asn Asp Val
        595                 600                 605

Arg Asn Ile Thr Lys Lys Asn Ile Glu Glu Trp Gly Pro Phe Asp Leu
    610                 615                 620

Val Ile Gly Gly Ser Pro Cys Asn Asp Leu Ser Asn Val Asn Pro Ala
625                 630                 635                 640

Arg Lys Gly Leu Tyr Glu Gly Thr Gly Arg Leu Phe Phe Glu Phe Tyr
                645                 650                 655

His Leu Leu Asn Tyr Ser Arg Pro Lys Glu Gly Asp Asp Arg Pro Phe
            660                 665                 670

Phe Trp Met Phe Glu Asn Val Val Ala Met Lys Val Gly Asp Lys Arg
        675                 680                 685

Asp Ile Ser Arg Phe Leu Glu Cys Asn Pro Val Met Ile Asp Ala Ile
    690                 695                 700

Lys Val Ser Ala Ala His Arg Ala Arg Tyr Phe Trp Gly Asn Leu Pro
705                 710                 715                 720

Gly Met Asn Arg Ile Phe Gly Phe Pro Val His Tyr Thr Asp Val Ser
                725                 730                 735

Asn Met Gly Arg Gly Ala Arg Gln Lys Leu Leu Gly Arg Ser Trp Ser
            740                 745                 750

Val Pro Val Ile Arg His Leu Phe Ala Pro Leu Lys Asp Tyr Phe Ala
        755                 760                 765

Cys Glu
    770

<210>  210
<211>  845
<212>  PRT
<213>  Homo sapiens

<400>  210

Met Glu Pro Ser Pro Glu Pro Pro Ser Leu Glu Ser Met Lys Gly Asp
1               5                   10                  15

Thr Arg His Leu Asn Gly Glu Glu Asp Ala Gly Gly Arg Glu Asp Ser
            20                  25                  30

Ile Leu Val Asn Gly Ala Cys Ser Asp Gln Ser Ser Asp Ser Pro Pro
        35                  40                  45

Ile Leu Glu Ala Ile Arg Thr Pro Glu Ile Arg Gly Arg Arg Ser Ser
    50                  55                  60

Ser Arg Leu Ser Lys Arg Glu Val Ser Ser Leu Leu Ser Tyr Thr Gln
65                  70                  75                  80

Asp Leu Thr Gly Asp Gly Asp Gly Glu Asp Gly Asp Gly Ser Asp Thr
                85                  90                  95

Pro Val Met Pro Lys Leu Phe Arg Glu Thr Arg Thr Arg Ser Glu Ser
            100                 105                 110

Pro Ala Val Arg Thr Arg Asn Asn Asn Ser Val Ser Ser Arg Glu Arg
        115                 120                 125

His Arg Pro Ser Pro Arg Ser Thr Arg Gly Arg Gln Gly Arg Asn His
    130                 135                 140

Val Asp Glu Ser Pro Val Glu Phe Pro Ala Thr Arg Ser Leu Arg Arg
145                 150                 155                 160

Arg Ala Thr Ala Ser Ala Gly Thr Pro Trp Pro Ser Pro Pro Ser Ser
                165                 170                 175

Tyr Leu Thr Ile Asp Leu Thr Asp Asp Thr Glu Asp Thr His Gly Thr
            180                 185                 190

Pro Gln Ser Ser Ser Thr Pro Tyr Ala Arg Leu Ala Gln Asp Ser Gln
        195                 200                 205

Gln Gly Gly Met Glu Ser Pro Gln Val Glu Ala Asp Ser Gly Asp Gly
    210                 215                 220

Asp Ser Ser Glu Tyr Gln Asp Gly Lys Glu Phe Gly Ile Gly Asp Leu
225                 230                 235                 240

Val Trp Gly Lys Ile Lys Gly Phe Ser Trp Trp Pro Ala Met Val Val
                245                 250                 255

Ser Trp Lys Ala Thr Ser Lys Arg Gln Ala Met Ser Gly Met Arg Trp
            260                 265                 270

Val Gln Trp Phe Gly Asp Gly Lys Phe Ser Glu Val Ser Ala Asp Lys
        275                 280                 285

Leu Val Ala Leu Gly Leu Phe Ser Gln His Phe Asn Leu Ala Thr Phe
    290                 295                 300

Asn Lys Leu Val Ser Tyr Arg Lys Ala Met Tyr His Ala Leu Glu Lys
305                 310                 315                 320

Ala Arg Val Arg Ala Gly Lys Thr Phe Pro Ser Ser Pro Gly Asp Ser
                325                 330                 335

Leu Glu Asp Gln Leu Lys Pro Met Leu Glu Trp Ala His Gly Gly Phe
            340                 345                 350

Lys Pro Thr Gly Ile Glu Gly Leu Lys Pro Asn Asn Thr Gln Pro Glu
        355                 360                 365

Asn Lys Thr Arg Arg Arg Thr Ala Asp Asp Ser Ala Thr Ser Asp Tyr
    370                 375                 380

Cys Pro Ala Pro Lys Arg Leu Lys Thr Asn Cys Tyr Asn Asn Gly Lys
385                 390                 395                 400

Asp Arg Gly Asp Glu Asp Gln Ser Arg Glu Gln Met Ala Ser Asp Val
                405                 410                 415

Ala Asn Asn Lys Ser Ser Leu Glu Asp Gly Cys Leu Ser Cys Gly Arg
            420                 425                 430

Lys Asn Pro Val Ser Phe His Pro Leu Phe Glu Gly Gly Leu Cys Gln
        435                 440                 445

Thr Cys Arg Asp Arg Phe Leu Glu Leu Phe Tyr Met Tyr Asp Asp Asp
    450                 455                 460

Gly Tyr Gln Ser Tyr Cys Thr Val Cys Cys Glu Gly Arg Glu Leu Leu

465                 470                 475                 480

Leu Cys Ser Asn Thr Ser Cys Cys Arg Cys Phe Cys Val Glu Cys Leu
                485                 490                 495

Glu Val Leu Val Gly Thr Gly Thr Ala Ala Glu Ala Lys Leu Gln Glu
            500                 505                 510

Pro Trp Ser Cys Tyr Met Cys Leu Pro Gln Arg Cys His Gly Val Leu
        515                 520                 525

Arg Arg Arg Lys Asp Trp Asn Val Arg Leu Gln Ala Phe Phe Thr Ser
    530                 535                 540

Asp Thr Gly Leu Glu Tyr Glu Ala Pro Lys Leu Tyr Pro Ala Ile Pro
545                 550                 555                 560

Ala Ala Arg Arg Arg Pro Ile Arg Val Leu Ser Leu Phe Asp Gly Ile
                565                 570                 575

Ala Thr Gly Tyr Leu Val Leu Lys Glu Leu Gly Ile Lys Val Gly Lys
            580                 585                 590

Tyr Val Ala Ser Glu Val Cys Glu Glu Ser Ile Ala Val Gly Thr Val
        595                 600                 605

Lys His Glu Gly Asn Ile Lys Tyr Val Asn Asp Val Arg Asn Ile Thr
    610                 615                 620

Lys Lys Asn Ile Glu Glu Trp Gly Pro Phe Asp Leu Val Ile Gly Gly
625                 630                 635                 640

Ser Pro Cys Asn Asp Leu Ser Asn Val Asn Pro Ala Arg Lys Gly Leu
                645                 650                 655

Tyr Glu Gly Thr Gly Arg Leu Phe Phe Glu Phe Tyr His Leu Leu Asn
            660                 665                 670

Tyr Ser Arg Pro Lys Glu Gly Asp Asp Arg Pro Phe Phe Trp Met Phe
        675                 680                 685

Glu Asn Val Val Ala Met Lys Val Gly Asp Lys Arg Asp Ile Ser Arg
    690                 695                 700

Phe Leu Glu Cys Asn Pro Val Met Ile Asp Ala Ile Lys Val Ser Ala
705                 710                 715                 720

Ala His Arg Ala Arg Tyr Phe Trp Gly Asn Leu Pro Gly Met Asn Arg
                725                 730                 735

Pro Val Ile Ala Ser Lys Asn Asp Lys Leu Glu Leu Gln Asp Cys Leu
            740                 745                 750

Glu Tyr Asn Arg Ile Ala Lys Leu Lys Lys Val Gln Thr Ile Thr Thr
        755                 760                 765

Lys Ser Asn Ser Ile Lys Gln Gly Lys Asn Gln Leu Phe Pro Val Val
    770                 775                 780

Met Asn Gly Lys Glu Asp Val Leu Trp Cys Thr Glu Leu Glu Arg Ile
785                 790                 795                 800

Phe Gly Phe Pro Val His Tyr Thr Asp Val Ser Asn Met Gly Arg Gly
                805                 810                 815

Ala Arg Gln Lys Leu Leu Gly Arg Ser Trp Ser Val Pro Val Ile Arg
            820                 825                 830

His Leu Phe Ala Pro Leu Lys Asp Tyr Phe Ala Cys Glu
        835                 840                 845

<210> 211
<211> 883
<212> PRT
<213> Homo sapiens

<400> 211

Met Ala Glu Asn Ser Val Leu Thr Ser Thr Thr Gly Arg Thr Ser Leu
1               5                   10                  15

Ala Asp Ser Ser Ile Phe Asp Ser Lys Val Thr Glu Ile Ser Lys Glu
            20                  25                  30

Asn Leu Leu Ile Gly Ser Thr Ser Tyr Val Glu Glu Glu Met Pro Gln
        35                  40                  45

Ile Glu Thr Arg Val Ile Leu Val Gln Glu Ala Gly Lys Gln Glu Glu
    50                  55                  60

Leu Ile Lys Ala Leu Lys Asp Ile Lys Val Gly Phe Val Lys Met Glu
65                  70                  75                  80

Ser Val Glu Glu Phe Glu Gly Leu Asp Ser Pro Glu Phe Glu Asn Val
                85                  90                  95

Phe Val Val Thr Asp Phe Gln Asp Ser Val Phe Asn Asp Leu Tyr Lys
            100                 105                 110

Ala Asp Cys Arg Val Ile Gly Pro Pro Val Val Leu Asn Cys Ser Gln
        115                 120                 125

Lys Gly Glu Pro Leu Pro Phe Ser Cys Arg Pro Leu Tyr Cys Thr Ser
    130                 135                 140

Met Met Asn Leu Val Leu Cys Phe Thr Gly Phe Arg Lys Lys Glu Glu
145                 150                 155                 160

Leu Val Arg Leu Val Thr Leu Val His His Met Gly Gly Val Ile Arg
                165                 170                 175

Lys Asp Phe Asn Ser Lys Val Thr His Leu Val Ala Asn Cys Thr Gln
            180                 185                 190

Gly Glu Lys Phe Arg Val Ala Val Ser Leu Gly Thr Pro Ile Met Lys
        195                 200                 205

Pro Glu Trp Ile Tyr Lys Ala Trp Glu Arg Arg Asn Glu Gln Asp Phe
    210                 215                 220

Tyr Ala Ala Val Asp Asp Phe Arg Asn Glu Phe Lys Val Pro Pro Phe
225                 230                 235                 240

Gln Asp Cys Ile Leu Ser Phe Leu Gly Phe Ser Asp Glu Glu Lys Thr
                245                 250                 255

Asn Met Glu Glu Met Thr Glu Met Gln Gly Gly Lys Tyr Leu Pro Leu
            260                 265                 270

Gly Asp Glu Arg Cys Thr His Leu Val Val Glu Glu Asn Ile Val Lys
        275                 280                 285

Asp Leu Pro Phe Glu Pro Ser Lys Lys Leu Tyr Val Val Lys Gln Glu
    290                 295                 300

Trp Phe Trp Gly Ser Ile Gln Met Asp Ala Arg Ala Gly Glu Thr Met
305                 310                 315                 320

Tyr Leu Tyr Glu Lys Ala Asn Thr Pro Glu Leu Lys Lys Ser Val Ser
                325                 330                 335

Met Leu Ser Leu Asn Thr Pro Asn Ser Asn Arg Lys Arg Arg Arg Leu
            340                 345                 350

Lys Glu Thr Leu Ala Gln Leu Ser Arg Glu Thr Asp Val Ser Pro Phe
        355                 360                 365

Pro Pro Arg Lys Arg Pro Ser Ala Glu His Ser Leu Ser Ile Gly Ser
    370                 375                 380

Leu Leu Asp Ile Ser Asn Thr Pro Glu Ser Ser Ile Asn Tyr Gly Asp
385                 390                 395                 400

Thr Pro Lys Ser Cys Thr Lys Ser Ser Lys Ser Ser Thr Pro Val Pro
                405                 410                 415

Ser Lys Gln Ser Ala Arg Trp Gln Val Ala Lys Glu Leu Tyr Gln Thr
            420                 425                 430

Glu Ser Asn Tyr Val Asn Ile Leu Ala Thr Ile Ile Gln Leu Phe Gln
        435                 440                 445

Val Pro Leu Glu Glu Glu Gly Gln Arg Gly Gly Pro Ile Leu Ala Pro
    450                 455                 460

Glu Glu Ile Lys Thr Ile Phe Gly Ser Ile Pro Asp Ile Phe Asp Val
465                 470                 475                 480

His Thr Lys Ile Lys Asp Asp Leu Glu Asp Leu Ile Val Asn Trp Asp
                485                 490                 495

Glu Ser Lys Ser Ile Gly Asp Ile Phe Leu Lys Tyr Ser Lys Asp Leu
            500                 505                 510

Val Lys Thr Tyr Pro Pro Phe Val Asn Phe Phe Glu Met Ser Lys Glu
        515                 520                 525

Thr Ile Ile Lys Cys Glu Lys Gln Lys Pro Arg Phe His Ala Phe Leu
    530                 535                 540

Lys Ile Asn Gln Ala Lys Pro Glu Cys Gly Arg Gln Ser Leu Val Glu
545                 550                 555                 560

Leu Leu Ile Arg Pro Val Gln Arg Leu Pro Ser Val Ala Leu Leu Leu
                565                 570                 575

Asn Asp Leu Lys Lys His Thr Ala Asp Glu Asn Pro Asp Lys Ser Thr
            580                 585                 590

Leu Glu Lys Ala Ile Gly Ser Leu Lys Glu Val Met Thr His Ile Asn
        595                 600                 605

Glu Asp Lys Arg Lys Thr Glu Ala Gln Lys Gln Ile Phe Asp Val Val
    610                 615                 620

Tyr Glu Val Asp Gly Cys Pro Ala Asn Leu Leu Ser Ser His Arg Ser
625                 630                 635                 640

Leu Val Gln Arg Val Glu Thr Ile Ser Leu Gly Glu His Pro Cys Asp
                645                 650                 655

Arg Gly Glu Gln Val Thr Leu Phe Leu Phe Asn Asp Cys Leu Glu Ile
            660                 665                 670

Ala Arg Lys Arg His Lys Val Ile Gly Thr Phe Arg Ser Pro His Gly
        675                 680                 685

Gln Thr Arg Pro Pro Ala Ser Leu Lys His Ile His Leu Met Pro Leu
    690                 695                 700

Ser Gln Ile Lys Lys Val Leu Asp Ile Arg Glu Thr Glu Asp Cys His
705                 710                 715                 720

Asn Ala Phe Ala Leu Leu Val Arg Pro Pro Thr Glu Gln Ala Asn Val
                725                 730                 735

Leu Leu Ser Phe Gln Met Thr Ser Asp Glu Leu Pro Lys Glu Asn Trp
            740                 745                 750

Leu Lys Met Leu Cys Arg His Val Ala Asn Thr Ile Cys Lys Ala Asp
        755                 760                 765

Ala Glu Asn Leu Ile Tyr Thr Ala Asp Pro Glu Ser Phe Glu Val Asn
    770                 775                 780

Thr Lys Asp Met Asp Ser Thr Leu Ser Arg Ala Ser Arg Ala Ile Lys
785                 790                 795                 800

Lys Thr Ser Lys Lys Val Thr Arg Ala Phe Ser Phe Ser Lys Thr Pro
                805                 810                 815

Lys Arg Ala Leu Arg Arg Ala Leu Met Thr Ser His Gly Ser Val Glu
            820                 825                 830

Gly Arg Ser Pro Ser Ser Asn Asp Lys His Val Met Ser Arg Leu Ser
        835                 840                 845

Ser Thr Ser Ser Leu Ala Gly Ile Pro Ser Pro Ser Leu Val Ser Leu
    850                 855                 860

Pro Ser Phe Phe Glu Arg Arg Ser His Thr Leu Ser Arg Ser Thr Thr
865                 870                 875                 880

His Leu Ile

<210>  212
<211>  751
<212>  PRT
<213>  Homo sapiens

<400>  212

<210>  213
<211>  707
<212>  PRT
<213>  Homo sapiens

<400>  213

Met Gly Gln Thr Gly Lys Lys Ser Glu Lys Gly Pro Val Cys Trp Arg
1               5                   10                  15

Lys Arg Val Lys Ser Glu Tyr Met Arg Leu Arg Gln Leu Lys Arg Phe
            20                  25                  30

Arg Arg Ala Asp Glu Val Lys Ser Met Phe Ser Ser Asn Arg Gln Lys
        35                  40                  45

Ile Leu Glu Arg Thr Glu Ile Leu Asn Gln Glu Trp Lys Gln Arg Arg
    50                  55                  60

Ile Gln Pro Val His Ile Leu Thr Ser Val Ser Ser Leu Arg Gly Thr
65                  70                  75                  80

Arg Glu Val Glu Asp Glu Thr Val Leu His Asn Ile Pro Tyr Met Gly
                85                  90                  95

Asp Glu Val Leu Asp Gln Asp Gly Thr Phe Ile Glu Glu Leu Ile Lys
            100                 105                 110

Asn Tyr Asp Gly Lys Val His Gly Asp Arg Glu Cys Gly Phe Ile Asn
        115                 120                 125

Asp Glu Ile Phe Val Glu Leu Val Asn Ala Leu Gly Gln Tyr Asn Asp
    130                 135                 140

Asp Asp Asp Asp Asp Asp Gly Asp Asp Pro Glu Glu Arg Glu Glu Lys
145                 150                 155                 160

Gln Lys Asp Leu Glu Asp His Arg Asp Asp Lys Glu Ser Arg Pro Pro
                165                 170                 175

Arg Lys Phe Pro Ser Asp Lys Ile Phe Glu Ala Ile Ser Ser Met Phe
            180                 185                 190

Pro Asp Lys Gly Thr Ala Glu Glu Leu Lys Glu Lys Tyr Lys Glu Leu
        195                 200                 205

Thr Glu Gln Gln Leu Pro Gly Ala Leu Pro Pro Glu Cys Thr Pro Asn
    210                 215                 220

Ile Asp Gly Pro Asn Ala Lys Ser Val Gln Arg Glu Gln Ser Leu His
225                 230                 235                 240

Ser Phe His Thr Leu Phe Cys Arg Arg Cys Phe Lys Tyr Asp Cys Phe
                245                 250                 255

Leu His Pro Phe His Ala Thr Pro Asn Thr Tyr Lys Arg Lys Asn Thr
            260                 265                 270

Glu Thr Ala Leu Asp Asn Lys Pro Cys Gly Pro Gln Cys Tyr Gln His
        275                 280                 285

Leu Glu Gly Ala Lys Glu Phe Ala Ala Ala Leu Thr Ala Glu Arg Ile
    290                 295                 300

Lys Thr Pro Pro Lys Arg Pro Gly Gly Arg Arg Arg Gly Arg Leu Pro
305                 310                 315                 320

Asn Asn Ser Ser Arg Pro Ser Thr Pro Thr Ile Asn Val Leu Glu Ser
                325                 330                 335

Lys Asp Thr Asp Ser Asp Arg Glu Ala Gly Thr Glu Thr Gly Gly Glu
            340                 345                 350

Asn Asn Asp Lys Glu Glu Glu Glu Lys Lys Asp Glu Thr Ser Ser Ser
        355                 360                 365

Ser Glu Ala Asn Ser Arg Cys Gln Thr Pro Ile Lys Met Lys Pro Asn
    370                 375                 380

Ile Glu Pro Pro Glu Asn Val Glu Trp Ser Gly Ala Glu Ala Ser Met
385                 390                 395                 400

Phe Arg Val Leu Ile Gly Thr Tyr Tyr Asp Asn Phe Cys Ala Ile Ala
                405                 410                 415

Arg Leu Ile Gly Thr Lys Thr Cys Arg Gln Val Tyr Glu Phe Arg Val
            420                 425                 430

Lys Glu Ser Ser Ile Ile Ala Pro Ala Pro Ala Glu Asp Val Asp Thr
        435                 440                 445

Pro Pro Arg Lys Lys Lys Arg Lys His Arg Leu Trp Ala Ala His Cys
    450                 455                 460

Arg Lys Ile Gln Leu Lys Lys Asp Gly Ser Ser Asn His Val Tyr Asn
465                 470                 475                 480

Tyr Gln Pro Cys Asp His Pro Arg Gln Pro Cys Asp Ser Ser Cys Pro
                485                 490                 495

Cys Val Ile Ala Gln Asn Phe Cys Glu Lys Phe Cys Gln Cys Ser Ser
            500                 505                 510

Glu Cys Gln Asn Arg Phe Pro Gly Cys Arg Cys Lys Ala Gln Cys Asn
        515                 520                 525

Thr Lys Gln Cys Pro Cys Tyr Leu Ala Val Arg Glu Cys Asp Pro Asp
    530                 535                 540

Leu Cys Leu Thr Cys Gly Ala Ala Asp His Trp Asp Ser Lys Asn Val
545                 550                 555                 560

Ser Cys Lys Asn Cys Ser Ile Gln Arg Gly Ser Lys Lys His Leu Leu
                565                 570                 575

Leu Ala Pro Ser Asp Val Ala Gly Trp Gly Ile Phe Ile Lys Asp Pro
            580                 585                 590

Val Gln Lys Asn Glu Phe Ile Ser Glu Tyr Cys Gly Glu Ile Ile Ser
        595                 600                 605

Gln Asp Glu Ala Asp Arg Arg Gly Lys Val Tyr Asp Lys Tyr Met Cys
    610                 615                 620

Ser Phe Leu Phe Asn Leu Asn Asn Asp Phe Val Val Asp Ala Thr Arg
625                 630                 635                 640

Lys Gly Asn Lys Ile Arg Phe Ala Asn His Ser Val Asn Pro Asn Cys
                645                 650                 655

Tyr Ala Lys Val Met Met Val Asn Gly Asp His Arg Ile Gly Ile Phe
            660                 665                 670

Ala Lys Arg Ala Ile Gln Thr Gly Glu Glu Leu Phe Phe Asp Tyr Arg
        675                 680                 685

Tyr Ser Gln Ala Asp Ala Leu Lys Tyr Val Gly Ile Glu Arg Glu Met
    690                 695                 700

Glu Ile Pro
705

<210>  214
<211>  622
<212>  PRT
<213>  Homo sapiens

<400>  214

<210> 215
<211> 380
<212> PRT
<213> Homo sapiens

<400> 215

Met Gly Ile Gln Gly Leu Ala Lys Leu Ile Ala Asp Val Ala Pro Ser
1               5                   10                  15

Ala Ile Arg Glu Asn Asp Ile Lys Ser Tyr Phe Gly Arg Lys Val Ala
            20                  25                  30

Ile Asp Ala Ser Met Ser Ile Tyr Gln Phe Leu Ile Ala Val Arg Gln
        35                  40                  45

Gly Gly Asp Val Leu Gln Asn Glu Glu Gly Glu Thr Thr Ser His Leu
    50                  55                  60

Met Gly Met Phe Tyr Arg Thr Ile Arg Met Met Glu Asn Gly Ile Lys
65                  70                  75                  80

Pro Val Tyr Val Phe Asp Gly Lys Pro Pro Gln Leu Lys Ser Gly Glu
                85                  90                  95

Leu Ala Lys Arg Ser Glu Arg Arg Ala Glu Ala Glu Lys Gln Leu Gln
            100                 105                 110

Gln Ala Gln Ala Ala Gly Ala Glu Gln Glu Val Glu Lys Phe Thr Lys
        115                 120                 125

Arg Leu Val Lys Val Thr Lys Gln His Asn Asp Glu Cys Lys His Leu
    130                 135                 140

Leu Ser Leu Met Gly Ile Pro Tyr Leu Asp Ala Pro Ser Glu Ala Glu
145                 150                 155                 160

Ala Ser Cys Ala Ala Leu Val Lys Ala Gly Lys Val Tyr Ala Ala Ala
                165                 170                 175

Thr Glu Asp Met Asp Cys Leu Thr Phe Gly Ser Pro Val Leu Met Arg
            180                 185                 190

His Leu Thr Ala Ser Glu Ala Lys Lys Leu Pro Ile Gln Glu Phe His
        195                 200                 205

Leu Ser Arg Ile Leu Gln Glu Leu Gly Leu Asn Gln Glu Gln Phe Val
    210                 215                 220

Asp Leu Cys Ile Leu Leu Gly Ser Asp Tyr Cys Glu Ser Ile Arg Gly
225                 230                 235                 240

Ile Gly Pro Lys Arg Ala Val Asp Leu Ile Gln Lys His Lys Ser Ile
                245                 250                 255

Glu Glu Ile Val Arg Arg Leu Asp Pro Asn Lys Tyr Pro Val Pro Glu
            260                 265                 270

Asn Trp Leu His Lys Glu Ala His Gln Leu Phe Leu Glu Pro Glu Val
        275                 280                 285

Leu Asp Pro Glu Ser Val Glu Leu Lys Trp Ser Glu Pro Asn Glu Glu
    290                 295                 300

Glu Leu Ile Lys Phe Met Cys Gly Glu Lys Gln Phe Ser Glu Glu Arg
305                 310                 315                 320

Ile Arg Ser Gly Val Lys Arg Leu Ser Lys Ser Arg Gln Gly Ser Thr
                325                 330                 335

Gln Gly Arg Leu Asp Asp Phe Phe Lys Val Thr Gly Ser Leu Ser Ser
            340                 345                 350

Ala Lys Arg Lys Glu Pro Glu Pro Lys Gly Ser Thr Lys Lys Lys Ala
        355                 360                 365

Lys Thr Gly Ala Ala Gly Lys Phe Lys Arg Gly Lys
    370                 375                 380

<210>  216
<211>  763
<212>  PRT
<213>  Homo sapiens

<400>  216

Met Lys Thr Ser Pro Arg Arg Pro Leu Ile Leu Lys Arg Arg Arg Leu
1               5                   10                  15

Pro Leu Pro Val Gln Asn Ala Pro Ser Glu Thr Ser Glu Glu Glu Pro
            20                  25                  30

Lys Arg Ser Pro Ala Gln Gln Glu Ser Asn Gln Ala Glu Ala Ser Lys
        35                  40                  45

Glu Val Ala Glu Ser Asn Ser Cys Lys Phe Pro Ala Gly Ile Lys Ile
    50                  55                  60

Ile Asn His Pro Thr Met Pro Asn Thr Gln Val Val Ala Ile Pro Asn
65                  70                  75                  80

Asn Ala Asn Ile His Ser Ile Ile Thr Ala Leu Thr Ala Lys Gly Lys
                85                  90                  95

Glu Ser Gly Ser Ser Gly Pro Asn Lys Phe Ile Leu Ile Ser Cys Gly
            100                 105                 110

Gly Ala Pro Thr Gln Pro Pro Gly Leu Arg Pro Gln Thr Gln Thr Ser
        115                 120                 125

Tyr Asp Ala Lys Arg Thr Glu Val Thr Leu Glu Thr Leu Gly Pro Lys
    130                 135                 140

Pro Ala Ala Arg Asp Val Asn Leu Pro Arg Pro Pro Gly Ala Leu Cys
145                 150                 155                 160

Glu Gln Lys Arg Glu Thr Cys Ala Asp Gly Glu Ala Ala Gly Cys Thr
                165                 170                 175

Ile Asn Asn Ser Leu Ser Asn Ile Gln Trp Leu Arg Lys Met Ser Ser
            180                 185                 190

Asp Gly Leu Gly Ser Arg Ser Ile Lys Gln Glu Met Glu Glu Lys Glu
        195                 200                 205

Asn Cys His Leu Glu Gln Arg Gln Val Lys Val Glu Glu Pro Ser Arg
    210                 215                 220

Pro Ser Ala Ser Trp Gln Asn Ser Val Ser Glu Arg Pro Pro Tyr Ser
225                 230                 235                 240

Tyr Met Ala Met Ile Gln Phe Ala Ile Asn Ser Thr Glu Arg Lys Arg
                245                 250                 255

Met Thr Leu Lys Asp Ile Tyr Thr Trp Ile Glu Asp His Phe Pro Tyr
            260                 265                 270

Phe Lys His Ile Ala Lys Pro Gly Trp Lys Asn Ser Ile Arg His Asn

275                 280                 285

Leu Ser Leu His Asp Met Phe Val Arg Glu Thr Ser Ala Asn Gly Lys
    290                 295                 300

Val Ser Phe Trp Thr Ile His Pro Ser Ala Asn Arg Tyr Leu Thr Leu
305                 310                 315                 320

Asp Gln Val Phe Lys Pro Leu Asp Pro Gly Ser Pro Gln Leu Pro Glu
                325                 330                 335

His Leu Glu Ser Gln Gln Lys Arg Pro Asn Pro Glu Leu Arg Arg Asn
            340                 345                 350

Met Thr Ile Lys Thr Glu Leu Pro Leu Gly Ala Arg Arg Lys Met Lys
        355                 360                 365

Pro Leu Leu Pro Arg Val Ser Ser Tyr Leu Val Pro Ile Gln Phe Pro
    370                 375                 380

Val Asn Gln Ser Leu Val Leu Gln Pro Ser Val Lys Val Pro Leu Pro
385                 390                 395                 400

Leu Ala Ala Ser Leu Met Ser Ser Glu Leu Ala Arg His Ser Lys Arg
                405                 410                 415

Val Arg Ile Ala Pro Lys Val Leu Leu Ala Glu Glu Gly Ile Ala Pro
            420                 425                 430

Leu Ser Ser Ala Gly Pro Gly Lys Glu Glu Lys Leu Leu Phe Gly Glu
        435                 440                 445

Gly Phe Ser Pro Leu Leu Pro Val Gln Thr Ile Lys Glu Glu Glu Ile
    450                 455                 460

Gln Pro Gly Glu Glu Met Pro His Leu Ala Arg Pro Ile Lys Val Glu
465                 470                 475                 480

Ser Pro Pro Leu Glu Glu Trp Pro Ser Pro Ala Pro Ser Phe Lys Glu
                485                 490                 495

Glu Ser Ser His Ser Trp Glu Asp Ser Ser Gln Ser Pro Thr Pro Arg
            500                 505                 510

Pro Lys Lys Ser Tyr Ser Gly Leu Arg Ser Pro Thr Arg Cys Val Ser
        515                 520                 525

Glu Met Leu Val Ile Gln His Arg Glu Arg Arg Glu Arg Ser Arg Ser
    530                 535                 540

Arg Arg Lys Gln His Leu Leu Pro Pro Cys Val Asp Glu Pro Glu Leu
545                 550                 555                 560

Leu Phe Ser Glu Gly Pro Ser Thr Ser Arg Trp Ala Ala Glu Leu Pro
                565                 570                 575

Phe Pro Ala Asp Ser Ser Asp Pro Ala Ser Gln Leu Ser Tyr Ser Gln
            580                 585                 590

Glu Val Gly Gly Pro Phe Lys Thr Pro Ile Lys Glu Thr Leu Pro Ile
        595                 600                 605

Ser Ser Thr Pro Ser Lys Ser Val Leu Pro Arg Thr Pro Glu Ser Trp
    610                 615                 620

Arg Leu Thr Pro Pro Ala Lys Val Gly Gly Leu Asp Phe Ser Pro Val
625                 630                 635                 640

Gln Thr Ser Gln Gly Ala Ser Asp Pro Leu Pro Asp Pro Leu Gly Leu
                645                 650                 655

Met Asp Leu Ser Thr Thr Pro Leu Gln Ser Ala Pro Pro Leu Glu Ser
            660                 665                 670

Pro Gln Arg Leu Leu Ser Ser Glu Pro Leu Asp Leu Ile Ser Val Pro
        675                 680                 685

Phe Gly Asn Ser Ser Pro Ser Asp Ile Asp Val Pro Lys Pro Gly Ser
    690                 695                 700

Pro Glu Pro Gln Val Ser Gly Leu Ala Ala Asn Arg Ser Leu Thr Glu
705                 710                 715                 720

Gly Leu Val Leu Asp Thr Met Asn Asp Ser Leu Ser Lys Ile Leu Leu
                725                 730                 735

Asp Ile Ser Phe Pro Gly Leu Asp Glu Asp Pro Leu Gly Pro Asp Asn
            740                 745                 750

Ile Asn Trp Ser Gln Phe Ile Pro Glu Leu Gln
        755                 760


<210>  217
<211>  801
<212>  PRT
<213>  Homo sapiens

<400>  217

Met Lys Thr Ser Pro Arg Arg Pro Leu Ile Leu Lys Arg Arg Arg Leu
1               5                   10                  15

Pro Leu Pro Val Gln Asn Ala Pro Ser Glu Thr Ser Glu Glu Pro
            20                  25                  30

Lys Arg Ser Pro Ala Gln Gln Glu Ser Asn Gln Ala Glu Ala Ser Lys
        35                  40                  45

Glu Val Ala Glu Ser Asn Ser Cys Lys Phe Pro Ala Gly Ile Lys Ile
    50                  55                  60

Ile Asn His Pro Thr Met Pro Asn Thr Gln Val Val Ala Ile Pro Asn
65                  70                  75                  80

Asn Ala Asn Ile His Ser Ile Ile Thr Ala Leu Thr Ala Lys Gly Lys
                85                  90                  95

Glu Ser Gly Ser Ser Gly Pro Asn Lys Phe Ile Leu Ile Ser Cys Gly
            100                 105                 110

Gly Ala Pro Thr Gln Pro Pro Gly Leu Arg Pro Gln Thr Gln Thr Ser
        115                 120                 125

Tyr Asp Ala Lys Arg Thr Glu Val Thr Leu Glu Thr Leu Gly Pro Lys
    130                 135                 140

Pro Ala Ala Arg Asp Val Asn Leu Pro Arg Pro Pro Gly Ala Leu Cys
145                 150                 155                 160

Glu Gln Lys Arg Glu Thr Cys Ala Asp Gly Glu Ala Ala Gly Cys Thr
                165                 170                 175

Ile Asn Asn Ser Leu Ser Asn Ile Gln Trp Leu Arg Lys Met Ser Ser
            180                 185                 190

Asp Gly Leu Gly Ser Arg Ser Ile Lys Gln Glu Met Glu Glu Lys Glu
        195                 200                 205

Asn Cys His Leu Glu Gln Arg Gln Val Lys Val Glu Glu Pro Ser Arg
    210                 215                 220

Pro Ser Ala Ser Trp Gln Asn Ser Val Ser Glu Arg Pro Pro Tyr Ser
225                 230                 235                 240

Tyr Met Ala Met Ile Gln Phe Ala Ile Asn Ser Thr Glu Arg Lys Arg
                245                 250                 255

Met Thr Leu Lys Asp Ile Tyr Thr Trp Ile Glu Asp His Phe Pro Tyr
            260                 265                 270

Phe Lys His Ile Ala Lys Pro Gly Trp Lys Asn Ser Ile Arg His Asn
        275                 280                 285

Leu Ser Leu His Asp Met Phe Val Arg Glu Thr Ser Ala Asn Gly Lys
    290                 295                 300

Val Ser Phe Trp Thr Ile His Pro Ser Ala Asn Arg Tyr Leu Thr Leu
305                 310                 315                 320

Asp Gln Val Phe Lys Pro Leu Asp Pro Gly Ser Pro Gln Leu Pro Glu
325 330 335

His Leu Glu Ser Gln Gln Lys Arg Pro Asn Pro Glu Leu Arg Arg Asn
                340                 345                 350

Met Thr Ile Lys Thr Glu Leu Pro Leu Gly Ala Arg Arg Lys Met Lys
            355                 360                 365

Pro Leu Leu Pro Arg Val Ser Ser Tyr Leu Val Pro Ile Gln Phe Pro
        370                 375                 380

Val Asn Gln Ser Leu Val Leu Gln Pro Ser Val Lys Val Pro Leu Pro
385 390 395 400

Leu Ala Ala Ser Leu Met Ser Ser Glu Leu Ala Arg His Ser Lys Arg
405 410 415

Val Arg Ile Ala Pro Lys Val Phe Gly Glu Gln Val Val Phe Gly Tyr
                420                 425                 430

Met Ser Lys Phe Phe Ser Gly Asp Leu Arg Asp Phe Gly Thr Pro Ile
            435                 440                 445

Thr Ser Leu Phe Asn Phe Ile Phe Leu Cys Leu Ser Val Leu Leu Ala
        450                 455                 460

Glu Glu Gly Ile Ala Pro Leu Ser Ser Ala Gly Pro Gly Lys Glu Glu
465 470 475 480

Lys Leu Leu Phe Gly Glu Gly Phe Ser Pro Leu Leu Pro Val Gln Thr
485 490 495

Ile Lys Glu Glu Glu Ile Gln Pro Gly Glu Glu Met Pro His Leu Ala
                500                 505                 510

Arg Pro Ile Lys Val Glu Ser Pro Pro Leu Glu Glu Trp Pro Ser Pro
            515                 520                 525

Ala Pro Ser Phe Lys Glu Glu Ser Ser His Ser Trp Glu Asp Ser Ser
        530                 535                 540

Gln Ser Pro Thr Pro Arg Pro Lys Lys Ser Tyr Ser Gly Leu Arg Ser
545 550 555 560

Pro Thr Arg Cys Val Ser Glu Met Leu Val Ile Gln His Arg Glu Arg
565 570 575

Arg Glu Arg Ser Arg Ser Arg Arg Lys Gln His Leu Leu Pro Pro Cys
                580                 585                 590

Val Asp Glu Pro Glu Leu Leu Phe Ser Glu Gly Pro Ser Thr Ser Arg
            595                 600                 605

Trp Ala Ala Glu Leu Pro Phe Pro Ala Asp Ser Ser Asp Pro Ala Ser
    610                 615                 620

Gln Leu Ser Tyr Ser Gln Glu Val Gly Gly Pro Phe Lys Thr Pro Ile
625                 630                 635                 640

Lys Glu Thr Leu Pro Ile Ser Ser Thr Pro Ser Lys Ser Val Leu Pro
                645                 650                 655

Arg Thr Pro Glu Ser Trp Arg Leu Thr Pro Pro Ala Lys Val Gly Gly
            660                 665                 670

Leu Asp Phe Ser Pro Val Gln Thr Ser Gln Gly Ala Ser Asp Pro Leu
        675                 680                 685

Pro Asp Pro Leu Gly Leu Met Asp Leu Ser Thr Thr Pro Leu Gln Ser
    690                 695                 700

Ala Pro Pro Leu Glu Ser Pro Gln Arg Leu Leu Ser Ser Glu Pro Leu
705                 710                 715                 720

Asp Leu Ile Ser Val Pro Phe Gly Asn Ser Ser Pro Ser Asp Ile Asp
                725                 730                 735

Val Pro Lys Pro Gly Ser Pro Glu Pro Gln Val Ser Gly Leu Ala Ala
            740                 745                 750

Asn Arg Ser Leu Thr Glu Gly Leu Val Leu Asp Thr Met Asn Asp Ser
        755                 760                 765

Leu Ser Lys Ile Leu Leu Asp Ile Ser Phe Pro Gly Leu Asp Glu Asp
    770                 775                 780

Pro Leu Gly Pro Asp Asn Ile Asn Trp Ser Gln Phe Ile Pro Glu Leu
785                 790                 795                 800

Gln

<210>  218
<211>  748
<212>  PRT
<213>  Homo sapiens

<400>  218

Met Lys Thr Ser Pro Arg Arg Pro Leu Ile Leu Lys Arg Arg Arg Leu
1               5                   10                  15

Pro Leu Pro Val Gln Asn Ala Pro Ser Glu Thr Ser Glu Glu Glu Pro
            20                  25                  30

Lys Arg Ser Pro Ala Gln Gln Glu Ser Asn Gln Ala Glu Ala Ser Lys

35                  40                  45

Glu Val Ala Glu Ser Asn Ser Cys Lys Phe Pro Ala Gly Ile Lys Ile
    50                  55                  60

Ile Asn His Pro Thr Met Pro Asn Thr Gln Val Val Ala Ile Pro Asn
65                  70                  75                  80

Asn Ala Asn Ile His Ser Ile Ile Thr Ala Leu Thr Ala Lys Gly Lys
                85                  90                  95

Glu Ser Gly Ser Ser Gly Pro Asn Lys Phe Ile Leu Ile Ser Cys Gly
            100                 105                 110

Gly Ala Pro Thr Gln Pro Pro Gly Leu Arg Pro Gln Thr Gln Thr Ser
        115                 120                 125

Tyr Asp Ala Lys Arg Thr Glu Val Thr Leu Glu Thr Leu Gly Pro Lys
    130                 135                 140

Pro Ala Ala Arg Asp Val Asn Leu Pro Arg Pro Pro Gly Ala Leu Cys
145                 150                 155                 160

Glu Gln Lys Arg Glu Thr Cys Ala Asp Gly Glu Ala Ala Gly Cys Thr
                165                 170                 175

Ile Asn Asn Ser Leu Ser Asn Ile Gln Trp Leu Arg Lys Met Ser Ser
            180                 185                 190

Asp Gly Leu Gly Ser Arg Ser Ile Lys Gln Glu Met Glu Glu Lys Glu
        195                 200                 205

Asn Cys His Leu Glu Gln Arg Gln Val Lys Val Glu Glu Pro Ser Arg
    210                 215                 220

Pro Ser Ala Ser Trp Gln Asn Ser Val Ser Glu Arg Pro Pro Tyr Ser
225                 230                 235                 240

Tyr Met Ala Met Ile Gln Phe Ala Ile Asn Ser Thr Glu Arg Lys Arg
                245                 250                 255

Met Thr Leu Lys Asp Ile Tyr Thr Trp Ile Glu Asp His Phe Pro Tyr
            260                 265                 270

Phe Lys His Ile Ala Lys Pro Gly Trp Lys Asn Ser Ile Arg His Asn
        275                 280                 285

Leu Ser Leu His Asp Met Phe Val Arg Glu Thr Ser Ala Asn Gly Lys
    290                 295                 300

Val Ser Phe Trp Thr Ile His Pro Ser Ala Asn Arg Tyr Leu Thr Leu
305                 310                 315                 320

Asp Gln Val Phe Lys Gln Gln Lys Arg Pro Asn Pro Glu Leu Arg Arg
                325                 330                 335

Asn Met Thr Ile Lys Thr Glu Leu Pro Leu Gly Ala Arg Arg Lys Met
            340                 345                 350

Lys Pro Leu Leu Pro Arg Val Ser Ser Tyr Leu Val Pro Ile Gln Phe
        355                 360                 365

Pro Val Asn Gln Ser Leu Val Leu Gln Pro Ser Val Lys Val Pro Leu
    370                 375                 380

Pro Leu Ala Ala Ser Leu Met Ser Ser Glu Leu Ala Arg His Ser Lys
385                 390                 395                 400

Arg Val Arg Ile Ala Pro Lys Val Leu Leu Ala Glu Glu Gly Ile Ala
                405                 410                 415

Pro Leu Ser Ser Ala Gly Pro Gly Lys Glu Glu Lys Leu Leu Phe Gly
            420                 425                 430

Glu Gly Phe Ser Pro Leu Leu Pro Val Gln Thr Ile Lys Glu Glu Glu
        435                 440                 445

Ile Gln Pro Gly Glu Glu Met Pro His Leu Ala Arg Pro Ile Lys Val
    450                 455                 460

Glu Ser Pro Pro Leu Glu Glu Trp Pro Ser Pro Ala Pro Ser Phe Lys
465                 470                 475                 480

Glu Glu Ser Ser His Ser Trp Glu Asp Ser Ser Gln Ser Pro Thr Pro
                485                 490                 495

Arg Pro Lys Lys Ser Tyr Ser Gly Leu Arg Ser Pro Thr Arg Cys Val
            500                 505                 510

Ser Glu Met Leu Val Ile Gln His Arg Glu Arg Arg Glu Arg Ser Arg
        515                 520                 525

Ser Arg Arg Lys Gln His Leu Leu Pro Pro Cys Val Asp Glu Pro Glu
    530                 535                 540

Leu Leu Phe Ser Glu Gly Pro Ser Thr Ser Arg Trp Ala Ala Glu Leu
545                 550                 555                 560

Pro Phe Pro Ala Asp Ser Ser Asp Pro Ala Ser Gln Leu Ser Tyr Ser
                565                 570                 575

Gln Glu Val Gly Gly Pro Phe Lys Thr Pro Ile Lys Glu Thr Leu Pro
            580                 585                 590

Ile Ser Ser Thr Pro Ser Lys Ser Val Leu Pro Arg Thr Pro Glu Ser

595                 600                 605

Trp Arg Leu Thr Pro Pro Ala Lys Val Gly Gly Leu Asp Phe Ser Pro
    610                 615                 620

Val Gln Thr Ser Gln Gly Ala Ser Asp Pro Leu Pro Asp Pro Leu Gly
625                 630                 635                 640

Leu Met Asp Leu Ser Thr Thr Pro Leu Gln Ser Ala Pro Pro Leu Glu
                645                 650                 655

Ser Pro Gln Arg Leu Leu Ser Ser Glu Pro Leu Asp Leu Ile Ser Val
            660                 665                 670

Pro Phe Gly Asn Ser Ser Pro Ser Asp Ile Asp Val Pro Lys Pro Gly
        675                 680                 685

Ser Pro Glu Pro Gln Val Ser Gly Leu Ala Ala Asn Arg Ser Leu Thr
    690                 695                 700

Glu Gly Leu Val Leu Asp Thr Met Asn Asp Ser Leu Ser Lys Ile Leu
705                 710                 715                 720

Leu Asp Ile Ser Phe Pro Gly Leu Asp Glu Asp Pro Leu Gly Pro Asp
                725                 730                 735

Asn Ile Asn Trp Ser Gln Phe Ile Pro Glu Leu Gln
            740                 745

<210>  219
<211>  128
<212>  PRT
<213>  Homo sapiens

<400>  219

Met Ala Gly Gly Lys Ala Gly Lys Asp Ser Gly Lys Ala Lys Thr Lys
1               5                   10                  15

Ala Val Ser Arg Ser Gln Arg Ala Gly Leu Gln Phe Pro Val Gly Arg
            20                  25                  30

Ile His Arg His Leu Lys Ser Arg Thr Thr Ser His Gly Arg Val Gly
        35                  40                  45

Ala Thr Ala Ala Val Tyr Ser Ala Ala Ile Leu Glu Tyr Leu Thr Ala
    50                  55                  60

Glu Val Leu Glu Leu Ala Gly Asn Ala Ser Lys Asp Leu Lys Val Lys
65                  70                  75                  80

Arg Ile Thr Pro Arg His Leu Gln Leu Ala Ile Arg Gly Asp Glu Glu
                85                  90                  95

Leu Asp Ser Leu Ile Lys Ala Thr Ile Ala Gly Gly Gly Val Ile Pro
            100                 105                 110

His Ile His Lys Ser Leu Ile Gly Lys Lys Gly Gln Gln Lys Thr Val
        115                 120                 125

<210>  220
<211>  142
<212>  PRT
<213>  Homo sapiens

<400>  220

<210>  221
<211>  142
<212>  PRT
<213>  Homo sapiens

<400>  221

<210>  222
<211>  147
<212>  PRT
<213>  Homo sapiens

<400>  222

<210>  223
<211>  390
<212>  PRT
<213>  Homo sapiens

<400>  223

Met Arg Gly Asp Arg Gly Arg Gly Arg Gly Gly Arg Phe Gly Ser Arg
1               5                   10                  15

Gly Gly Pro Gly Gly Gly Phe Arg Pro Phe Val Pro His Ile Pro Phe
            20                  25                  30

Asp Phe Tyr Leu Cys Glu Met Ala Phe Pro Arg Val Lys Pro Ala Pro
        35                  40                  45

Asp Glu Thr Ser Phe Ser Glu Ala Leu Leu Lys Arg Asn Gln Asp Leu
    50                  55                  60

Ala Pro Asn Ser Ala Glu Gln Ala Ser Ile Leu Ser Leu Val Thr Lys
65                  70                  75                  80

Ile Asn Asn Val Ile Asp Asn Leu Ile Val Ala Pro Gly Thr Phe Glu
                85                  90                  95

Val Gln Ile Glu Glu Val Arg Gln Val Gly Ser Tyr Lys Lys Gly Thr
            100                 105                 110

Met Thr Thr Gly His Asn Val Ala Asp Leu Val Val Ile Leu Lys Ile
        115                 120                 125

Leu Pro Thr Leu Glu Ala Val Ala Ala Leu Gly Asn Lys Val Val Glu
    130                 135                 140

Ser Leu Arg Ala Gln Asp Pro Ser Glu Val Leu Thr Met Leu Thr Asn
145                 150                 155                 160

Glu Thr Gly Phe Glu Ile Ser Ser Ser Asp Ala Thr Val Lys Ile Leu
                165                 170                 175

Ile Thr Thr Val Pro Pro Asn Leu Arg Lys Leu Asp Pro Glu Leu His
            180                 185                 190

Leu Asp Ile Lys Val Leu Gln Ser Ala Leu Ala Ala Ile Arg His Ala
        195                 200                 205

Arg Trp Phe Glu Glu Asn Ala Ser Gln Ser Thr Val Lys Val Leu Ile
    210                 215                 220

Arg Leu Leu Lys Asp Leu Arg Ile Arg Phe Pro Gly Phe Glu Pro Leu
225                 230                 235                 240

Thr Pro Trp Ile Leu Asp Leu Leu Gly His Tyr Ala Val Met Asn Asn
                245                 250                 255

Pro Thr Arg Gln Pro Leu Ala Leu Asn Val Ala Tyr Arg Arg Cys Leu
            260                 265                 270

Gln Ile Leu Ala Ala Gly Leu Phe Leu Pro Gly Ser Val Gly Ile Thr
        275                 280                 285

Asp Pro Cys Glu Ser Gly Asn Phe Arg Val His Thr Val Met Thr Leu
    290                 295                 300

Glu Gln Gln Asp Met Val Cys Tyr Thr Ala Gln Thr Leu Val Arg Ile
305                 310                 315                 320

Leu Ser His Gly Gly Phe Arg Lys Ile Leu Gly Gln Glu Gly Asp Ala
                325                 330                 335

Ser Tyr Leu Ala Ser Glu Ile Ser Thr Trp Asp Gly Val Ile Val Thr
            340                 345                 350

Pro Ser Glu Lys Ala Tyr Glu Lys Pro Pro Glu Lys Lys Glu Gly Glu
        355                 360                 365

Glu Glu Glu Glu Asn Thr Glu Glu Pro Pro Gln Gly Glu Glu Glu Glu
    370                 375                 380

Ser Met Glu Thr Gln Glu
385                 390

<210>  224
<211>  1056
<212>  PRT
<213>  Homo sapiens

<400>  224

<210>  225
<211>  673
<212>  PRT
<213>  Homo sapiens

<400>  225

Met Asp Pro Gln Arg Ser Pro Leu Leu Glu Val Lys Gly Asn Ile Glu
1               5                   10                  15

Leu Lys Arg Pro Leu Ile Lys Ala Pro Ser Gln Leu Pro Leu Ser Gly
            20                  25                  30

Ser Arg Leu Lys Arg Arg Pro Asp Gln Met Glu Asp Gly Leu Glu Pro
        35                  40                  45

Glu Lys Lys Arg Thr Arg Gly Leu Gly Ala Thr Thr Lys Ile Thr Thr
    50                  55                  60

Ser His Pro Arg Val Pro Ser Leu Thr Thr Val Pro Gln Thr Gln Gly
65                  70                  75                  80

Gln Thr Thr Ala Gln Lys Val Ser Lys Lys Thr Gly Pro Arg Cys Ser
                85                  90                  95

Thr Ala Ile Ala Thr Gly Leu Lys Asn Gln Lys Pro Val Pro Ala Val
            100                 105                 110

Pro Val Gln Lys Ser Gly Thr Ser Gly Val Pro Pro Met Ala Gly Gly
        115                 120                 125

Lys Lys Pro Ser Lys Arg Pro Ala Trp Asp Leu Lys Gly Gln Leu Cys
    130                 135                 140

Asp Leu Asn Ala Glu Leu Lys Arg Cys Arg Glu Arg Thr Gln Thr Leu
145                 150                 155                 160

Asp Gln Glu Asn Gln Gln Leu Gln Asp Gln Leu Arg Asp Ala Gln Gln
                165                 170                 175

Gln Val Lys Ala Leu Gly Thr Glu Arg Thr Thr Leu Glu Gly His Leu
            180                 185                 190

Ala Lys Val Gln Ala Gln Ala Glu Gln Gly Gln Gln Glu Leu Lys Asn
        195                 200                 205

Leu Arg Ala Cys Val Leu Glu Leu Glu Glu Arg Leu Ser Thr Gln Glu
    210                 215                 220

Gly Leu Val Gln Glu Leu Gln Lys Lys Gln Val Glu Leu Gln Glu Glu
225                 230                 235                 240

Arg Arg Gly Leu Met Ser Gln Leu Glu Glu Lys Glu Arg Arg Leu Gln
                245                 250                 255

Thr Ser Glu Ala Ala Leu Ser Ser Ser Gln Ala Glu Val Ala Ser Leu
            260                 265                 270

Arg Gln Glu Thr Val Ala Gln Ala Ala Leu Leu Thr Glu Arg Glu Glu
        275                 280                 285

Arg Leu His Gly Leu Glu Met Glu Arg Arg Arg Leu His Asn Gln Leu
    290                 295                 300

Gln Glu Leu Lys Gly Asn Ile Arg Val Phe Cys Arg Val Arg Pro Val
305                 310                 315                 320

Leu Pro Gly Glu Pro Thr Pro Pro Pro Gly Leu Leu Leu Phe Pro Ser
                325                 330                 335

Gly Pro Gly Gly Pro Ser Asp Pro Pro Thr Arg Leu Ser Leu Ser Arg
            340                 345                 350

Ser Asp Glu Arg Arg Gly Thr Leu Ser Gly Ala Pro Ala Pro Pro Pro
        355                 360                 365

Arg His Asp Phe Ser Phe Asp Arg Val Phe Pro Pro Gly Ser Gly Gln
    370                 375                 380

Asp Glu Val Phe Glu Glu Ile Ala Met Leu Val Gln Ser Ala Leu Asp
385                 390                 395                 400

Gly Tyr Pro Val Cys Ile Phe Ala Tyr Gly Gln Thr Gly Ser Gly Lys
                405                 410                 415

Thr Phe Thr Met Glu Gly Gly Pro Gly Gly Asp Pro Gln Leu Glu Gly
            420                 425                 430

Leu Ile Pro Arg Ala Leu Arg His Leu Phe Ser Val Ala Gln Glu Leu
        435                 440                 445

Ser Gly Gln Gly Trp Thr Tyr Ser Phe Val Ala Ser Tyr Val Glu Ile
    450                 455                 460

Tyr Asn Glu Thr Val Arg Asp Leu Leu Ala Thr Gly Thr Arg Lys Gly
465                 470                 475                 480

Gln Gly Gly Glu Cys Glu Ile Arg Arg Ala Gly Pro Gly Ser Glu Glu
                485                 490                 495

Leu Thr Val Thr Asn Ala Arg Tyr Val Pro Val Ser Cys Glu Lys Glu
            500                 505                 510

Val Asp Ala Leu Leu His Leu Ala Arg Gln Asn Arg Ala Val Ala Arg
        515                 520                 525

Thr Ala Gln Asn Glu Arg Ser Ser Arg Ser His Ser Val Phe Gln Leu
    530                 535                 540

Gln Ile Ser Gly Glu His Ser Ser Arg Gly Leu Gln Cys Gly Ala Pro
545                 550                 555                 560

Leu Ser Leu Val Asp Leu Ala Gly Ser Glu Arg Leu Asp Pro Gly Leu
                565                 570                 575

Ala Leu Gly Pro Gly Glu Arg Glu Arg Leu Arg Glu Thr Gln Ala Ile
            580                 585                 590

Asn Ser Ser Leu Ser Thr Leu Gly Leu Val Ile Met Ala Leu Ser Asn
        595                 600                 605

Lys Glu Ser His Val Pro Tyr Arg Asn Ser Lys Leu Thr Tyr Leu Leu
    610                 615                 620

Gln Asn Ser Leu Gly Gly Ser Ala Lys Met Leu Met Phe Val Asn Ile
625                 630                 635                 640

Ser Pro Leu Glu Glu Asn Val Ser Glu Ser Leu Asn Ser Leu Arg Phe
                645                 650                 655

Ala Ser Lys Val Asn Gln Cys Val Ile Gly Thr Ala Gln Ala Asn Arg
            660                 665                 670

Lys

<210>  226
<211>  529
<212>  PRT
<213>  Homo sapiens

<400>  226

<210>  227
<211>  586
<212>  PRT
<213>  Homo sapiens

<400>  227

<210>  228
<211>  205
<212>  PRT
<213>  Homo sapiens

<400>  228

Met Ala Leu Gln Leu Ser Arg Glu Gln Gly Ile Thr Leu Arg Gly Ser
1               5                   10                  15

Ala Glu Ile Val Ala Glu Phe Phe Ser Phe Gly Ile Asn Ser Ile Leu
            20                  25                  30

Tyr Gln Arg Gly Ile Tyr Pro Ser Glu Thr Phe Thr Arg Val Gln Lys
        35                  40                  45

Tyr Gly Leu Thr Leu Leu Val Thr Thr Asp Leu Glu Leu Ile Lys Tyr
    50                  55                  60

Leu Asn Asn Val Val Glu Gln Leu Lys Asp Trp Leu Tyr Lys Cys Ser
65                  70                  75                  80

Val Gln Lys Leu Val Val Val Ile Ser Asn Ile Glu Ser Gly Glu Val
                85                  90                  95

Leu Glu Arg Trp Gln Phe Asp Ile Glu Cys Asp Lys Thr Ala Lys Asp
            100                 105                 110

Asp Ser Ala Pro Arg Glu Lys Ser Gln Lys Ala Ile Gln Asp Glu Ile
        115                 120                 125

Arg Ser Val Ile Arg Gln Ile Thr Ala Thr Val Thr Phe Leu Pro Leu
    130                 135                 140

Leu Glu Val Ser Cys Ser Phe Asp Leu Leu Ile Tyr Thr Asp Lys Asp
145                 150                 155                 160

Leu Val Val Pro Glu Lys Trp Glu Glu Ser Gly Pro Gln Phe Ile Thr
                165                 170                 175

Asn Ser Glu Glu Val Arg Leu Arg Ser Phe Thr Thr Thr Ile His Lys
            180                 185                 190

Val Asn Ser Met Val Ala Tyr Lys Ile Pro Val Asn Asp
        195                 200                 205

<210>  229
<211>  904
<212>  PRT
<213>  Homo sapiens

<400>  229

<210>  230
<211>  808
<212>  PRT
<213>  Homo sapiens

<400>  230
   Met Ala Gly Thr Val Val Leu Asp Asp Val Glu Leu Arg Glu Ala Gln
   1               5                   10                  15
   Arg Asp Tyr Leu Asp Phe Leu Asp Asp Glu Glu Asp Gln Gly Ile Tyr
               20                  25                  30
   Gln Ser Lys Val Arg Glu Leu Ile Ser Asp Asn Gln Tyr Arg Leu Ile
           35                  40                  45
   Val Asn Val Asn Asp Leu Arg Arg Lys Asn Glu Lys Arg Ala Asn Arg
       50                  55                  60
   Leu Leu Asn Asn Ala Phe Glu Glu Leu Val Ala Phe Gln Arg Ala Leu
   65                  70                  75                  80
   Lys Asp Phe Val Ala Ser Ile Asp Ala Thr Tyr Ala Lys Gln Tyr Glu
                   85                  90                  95
   Glu Phe Tyr Val Gly Leu Glu Gly Ser Phe Gly Ser Lys His Val Ser
               100                 105                 110
   Pro Arg Thr Leu Thr Ser Cys Phe Leu Ser Cys Val Val Cys Val Glu
   115                 120                 125
   Gly Ile Val Thr Lys Cys Ser Leu Val Arg Pro Lys Val Val Arg Ser
       130                 135                 140
   Val His Tyr Cys Pro Ala Thr Lys Lys Thr Ile Glu Arg Arg Tyr Ser
   145                 150                 155                 160
   Asp Leu Thr Thr Leu Val Ala Phe Pro Ser Ser Ser Val Tyr Pro Thr
                   165                 170                 175
   Lys Asp Glu Glu Asn Asn Pro Leu Glu Thr Glu Tyr Gly Leu Ser Val
               180                 185                 190
   Tyr Lys Asp His Gln Thr Ile Thr Ile Gln Glu Met Pro Glu Lys Ala
           195                 200                 205
   Pro Ala Gly Gln Leu Pro Arg Ser Val Asp Val Ile Leu Asp Asp Asp
       210                 215                 220
   Leu Val Asp Lys Ala Lys Pro Gly Asp Arg Val Gln Val Val Gly Thr
   225                 230                 235                 240
   Tyr Arg Cys Leu Pro Gly Lys Lys Gly Gly Tyr Thr Ser Gly Thr Phe
                   245                 250                 255
   Arg Thr Val Leu Ile Ala Cys Asn Val Lys Gln Met Ser Lys Asp Ala
               260                 265                 270
   Gln Pro Ser Phe Ser Ala Glu Asp Ile Ala Lys Ile Lys Lys Phe Ser
           275                 280                 285
   Lys Thr Arg Ser Lys Asp Ile Phe Asp Gln Leu Ala Lys Ser Leu Ala
       290                 295                 300
   Pro Ser Ile His Gly His Asp Tyr Val Lys Lys Ala Ile Leu Cys Leu
   305                 310                 315                 320
   Leu Leu Gly Gly Val Glu Arg Asp Leu Glu Asn Gly Ser His Ile Arg
                   325                 330                 335
   Gly Asp Ile Asn Ile Leu Leu Ile Gly Asp Pro Ser Val Ala Lys Ser
               340                 345                 350
   Gln Leu Leu Arg Tyr Val Leu Cys Thr Ala Pro Arg Ala Ile Pro Thr
           355                 360                 365
   Thr Gly Arg Gly Ser Ser Gly Val Gly Leu Thr Ala Ala Val Thr Thr
       370                 375                 380
   Asp Gln Glu Thr Gly Glu Arg Arg Leu Glu Ala Gly Ala Met Val Leu
   385                 390                 395                 400
   Ala Asp Arg Gly Val Val Cys Ile Asp Glu Phe Asp Lys Met Ser Asp
                   405                 410                 415
   Met Asp Arg Thr Ala Ile His Glu Val Met Glu Gln Gly Arg Val Thr
               420                 425                 430
   Ile Ala Lys Ala Gly Ile His Ala Arg Leu Asn Ala Arg Cys Ser Val
           435                 440                 445
   Leu Ala Ala Ala Asn Pro Val Tyr Gly Arg Tyr Asp Gln Tyr Lys Thr
       450                 455                 460
   Pro Met Glu Asn Ile Gly Leu Gln Asp Ser Leu Leu Ser Arg Phe Asp
   465                 470                 475                 480
   Leu Leu Phe Ile Met Leu Asp Gln Met Asp Pro Glu Gln Asp Arg Glu
                   485                 490                 495
   Ile Ser Asp His Val Leu Arg Met His Arg Tyr Arg Ala Pro Gly Glu
               500                 505                 510
   Gln Asp Gly Asp Ala Met Pro Leu Gly Ser Ala Val Asp Ile Leu Ala
           515                 520                 525
   Thr Asp Asp Pro Asn Phe Ser Gln Glu Asp Gln Gln Asp Thr Gln Ile
       530                 535                 540
   Tyr Glu Lys His Asp Asn Leu Leu His Gly Thr Lys Lys Lys Lys Glu
   545                 550                 555                 560
   Lys Met Val Ser Ala Ala Phe Met Lys Lys Tyr Ile His Val Ala Lys
                   565                 570                 575
   Ile Ile Lys Pro Val Leu Thr Gln Glu Ser Ala Thr Tyr Ile Ala Glu
               580                 585                 590
   Glu Tyr Ser Arg Leu Arg Ser Gln Asp Ser Met Ser Ser Asp Thr Ala
           595                 600                 605
   Arg Thr Ser Pro Val Thr Ala Arg Thr Leu Glu Thr Leu Ile Arg Leu
       610                 615                 620
   Ala Thr Ala His Ala Lys Ala Arg Met Ser Lys Thr Val Asp Leu Gln
   625                 630                 635                 640
   Asp Ala Glu Glu Ala Val Glu Leu Val Gln Tyr Ala Tyr Phe Lys Lys
                   645                 650                 655
   Val Leu Glu Lys Glu Lys Lys Arg Lys Lys Arg Ser Glu Asp Glu Ser
               660                 665                 670
   Glu Thr Glu Asp Glu Glu Glu Lys Ser Gln Glu Asp Gln Glu Gln Lys
   675                 680                 685
   Arg Lys Arg Arg Lys Thr Arg Gln Pro Asp Ala Lys Asp Gly Asp Ser
       690                 695                 700
   Tyr Asp Pro Tyr Asp Phe Ser Asp Thr Glu Glu Glu Met Pro Gln Val
   705                 710                 715                 720
   His Thr Pro Lys Thr Ala Asp Ser Gln Glu Thr Lys Glu Ser Gln Lys
                   725                 730                 735
   Val Glu Leu Ser Glu Ser Arg Leu Lys Ala Phe Lys Val Ala Leu Leu
               740                 745                 750
   Asp Val Phe Arg Glu Ala His Ala Gln Ser Ile Gly Met Asn Arg Leu
           755                 760                 765
   Thr Glu Ser Ile Asn Arg Asp Ser Glu Glu Pro Phe Ser Ser Val Glu
       770                 775                 780
   Ile Gln Ala Ala Leu Ser Lys Met Gln Asp Asp Asn Gln Val Met Val
   785                 790                 795                 800
   Ser Glu Gly Ile Ile Phe Leu Ile
                   805

<210>  231
<211>  821
<212>  PRT
<213>  Homo sapiens

<400>  231

Met Asp Leu Ala Ala Ala Ala Glu Pro Gly Ala Gly Ser Gln His Leu
1               5                   10                  15

Glu Val Arg Asp Glu Val Ala Glu Lys Cys Gln Lys Leu Phe Leu Asp
            20                  25                  30

Phe Leu Glu Glu Phe Gln Ser Ser Asp Gly Glu Ile Lys Tyr Leu Gln
        35                  40                  45

Leu Ala Glu Glu Leu Ile Arg Pro Glu Arg Asn Thr Leu Val Val Ser
    50                  55                  60

Phe Val Asp Leu Glu Gln Phe Asn Gln Gln Leu Ser Thr Thr Ile Gln
65                  70                  75                  80

Glu Glu Phe Tyr Arg Val Tyr Pro Tyr Leu Cys Arg Ala Leu Lys Thr
                85                  90                  95

Phe Val Lys Asp Arg Lys Glu Ile Pro Leu Ala Lys Asp Phe Tyr Val
            100                 105                 110

Ala Phe Gln Asp Leu Pro Thr Arg His Lys Ile Arg Glu Leu Thr Ser
        115                 120                 125

Ser Arg Ile Gly Leu Leu Thr Arg Ile Ser Gly Gln Val Val Arg Thr
    130                 135                 140

His Pro Val His Pro Glu Leu Val Ser Gly Thr Phe Leu Cys Leu Asp
145                 150                 155                 160

Cys Gln Thr Val Ile Arg Asp Val Glu Gln Gln Phe Lys Tyr Thr Gln
                165                 170                 175

Pro Asn Ile Cys Arg Asn Pro Val Cys Ala Asn Arg Arg Arg Phe Leu
            180                 185                 190

Leu Asp Thr Asn Lys Ser Arg Phe Val Asp Phe Gln Lys Val Arg Ile
        195                 200                 205

Gln Glu Thr Gln Ala Glu Leu Pro Arg Gly Ser Ile Pro Arg Ser Leu
    210                 215                 220

Glu Val Ile Leu Arg Ala Glu Ala Val Glu Ser Ala Gln Ala Gly Asp
225                 230                 235                 240

Lys Cys Asp Phe Thr Gly Thr Leu Ile Val Val Pro Asp Val Ser Lys
                245                 250                 255

Leu Ser Thr Pro Gly Ala Arg Ala Glu Thr Asn Ser Arg Val Ser Gly
            260                 265                 270

Val Asp Gly Tyr Glu Thr Glu Gly Ile Arg Gly Leu Arg Ala Leu Gly
        275                 280                 285

Val Arg Asp Leu Ser Tyr Arg Leu Val Phe Leu Ala Cys Cys Val Ala
    290                 295                 300

Pro Thr Asn Pro Arg Phe Gly Gly Lys Glu Leu Arg Asp Glu Glu Gln
305                 310                 315                 320

Thr Ala Glu Ser Ile Lys Asn Gln Met Thr Val Lys Glu Trp Glu Lys
                325                 330                 335

Val Phe Glu Met Ser Gln Asp Lys Asn Leu Tyr His Asn Leu Cys Thr
            340                 345                 350

Ser Leu Phe Pro Thr Ile His Gly Asn Asp Glu Val Lys Arg Gly Val
        355                 360                 365

Leu Leu Met Leu Phe Gly Gly Val Pro Lys Thr Thr Gly Glu Gly Thr
    370                 375                 380

Ser Leu Arg Gly Asp Ile Asn Val Cys Ile Val Gly Asp Pro Ser Thr
385                 390                 395                 400

Ala Lys Ser Gln Phe Leu Lys His Val Glu Glu Phe Ser Pro Arg Ala
                405                 410                 415

Val Tyr Thr Ser Gly Lys Ala Ser Ser Ala Ala Gly Leu Thr Ala Ala
            420                 425                 430

Val Val Arg Asp Glu Glu Ser His Glu Phe Val Ile Glu Ala Gly Ala
        435                 440                 445

Leu Met Leu Ala Asp Asn Gly Val Cys Cys Ile Asp Glu Phe Asp Lys
    450                 455                 460

Met Asp Val Arg Asp Gln Val Ala Ile His Glu Ala Met Glu Gln Gln
465                 470                 475                 480

Thr Ile Ser Ile Thr Lys Ala Gly Val Lys Ala Thr Leu Asn Ala Arg
                485                 490                 495

Thr Ser Ile Leu Ala Ala Ala Asn Pro Ile Ser Gly His Tyr Asp Arg
            500                 505                 510

Ser Lys Ser Leu Lys Gln Asn Ile Asn Leu Ser Ala Pro Ile Met Ser
        515                 520                 525

Arg Phe Asp Leu Phe Phe Ile Leu Val Asp Glu Cys Asn Glu Val Thr
    530                 535                 540

Asp Tyr Ala Ile Ala Arg Arg Ile Val Asp Leu His Ser Arg Ile Glu
545                 550                 555                 560

Glu Ser Ile Asp Arg Val Tyr Ser Leu Asp Asp Ile Arg Arg Tyr Leu
                565                 570                 575

Leu Phe Ala Arg Gln Phe Lys Pro Lys Ile Ser Lys Glu Ser Glu Asp
            580                 585                 590

Phe Ile Val Glu Gln Tyr Lys His Leu Arg Gln Arg Asp Gly Ser Gly
        595                 600                 605

Val Thr Lys Ser Ser Trp Arg Ile Thr Val Arg Gln Leu Glu Ser Met
    610                 615                 620

Ile Arg Leu Ser Glu Ala Met Ala Arg Met His Cys Cys Asp Glu Val
625                 630                 635                 640

Gln Pro Lys His Val Lys Glu Ala Phe Arg Leu Leu Asn Lys Ser Ile
                645                 650                 655

Ile Arg Val Glu Thr Pro Asp Val Asn Leu Asp Gln Glu Glu Glu Ile
            660                 665                 670

Gln Met Glu Val Asp Glu Gly Ala Gly Gly Ile Asn Gly His Ala Asp
        675                 680                 685

Ser Pro Ala Pro Val Asn Gly Ile Asn Gly Tyr Asn Glu Asp Ile Asn
    690                 695                 700

Gln Glu Ser Ala Pro Lys Ala Ser Leu Arg Leu Gly Phe Ser Glu Tyr
705                 710                 715                 720

Cys Arg Ile Ser Asn Leu Ile Val Leu His Leu Arg Lys Val Glu Glu
                725                 730                 735

Glu Glu Asp Glu Ser Ala Leu Lys Arg Ser Glu Leu Val Asn Trp Tyr
            740                 745                 750

Leu Lys Glu Ile Glu Ser Glu Ile Asp Ser Glu Glu Glu Leu Ile Asn
        755                 760                 765

Lys Lys Arg Ile Ile Glu Lys Val Ile His Arg Leu Thr His Tyr Asp
    770                 775                 780

His Val Leu Ile Glu Leu Thr Gln Ala Gly Leu Lys Gly Ser Thr Glu
785                 790                 795                 800

Gly Ser Glu Ser Tyr Glu Glu Asp Pro Tyr Leu Val Val Asn Pro Asn
                805                 810                 815

Tyr Leu Leu Glu Asp
            820

<210>  232
<211>  700
<212>  PRT
<213>  Homo sapiens

<400>  232

Met Ser Arg Arg Thr Arg Cys Glu Asp Leu Asp Glu Leu His Tyr Gln
1               5                   10                  15

Asp Thr Asp Ser Asp Val Pro Glu Gln Arg Asp Ser Lys Cys Lys Val
            20                  25                  30

Lys Trp Thr His Glu Glu Asp Glu Gln Leu Arg Ala Leu Val Arg Gln
        35                  40                  45

Phe Gly Gln Gln Asp Trp Lys Phe Leu Ala Ser His Phe Pro Asn Arg
    50                  55                  60

Thr Asp Gln Gln Cys Gln Tyr Arg Trp Leu Arg Val Leu Asn Pro Asp
65                  70                  75                  80

Leu Val Lys Gly Pro Trp Thr Lys Glu Glu Asp Gln Lys Val Ile Glu
                85                  90                  95

Leu Val Lys Lys Tyr Gly Thr Lys Gln Trp Thr Leu Ile Ala Lys His
            100                 105                 110

Leu Lys Gly Arg Leu Gly Lys Gln Cys Arg Glu Arg Trp His Asn His
        115                 120                 125

Leu Asn Pro Glu Val Lys Lys Ser Cys Trp Thr Glu Glu Glu Asp Arg
    130                 135                 140

Ile Ile Cys Glu Ala His Lys Val Leu Gly Asn Arg Trp Ala Glu Ile
145                 150                 155                 160

Ala Lys Met Leu Pro Gly Arg Thr Asp Asn Ala Val Lys Asn His Trp
                165                 170                 175

Asn Ser Thr Ile Lys Arg Lys Val Asp Thr Gly Gly Phe Leu Ser Glu
            180                 185                 190

Ser Lys Asp Cys Lys Pro Pro Val Tyr Leu Leu Leu Glu Leu Glu Asp
        195                 200                 205

Lys Asp Gly Leu Gln Ser Ala Gln Pro Thr Glu Gly Gln Gly Ser Leu
    210                 215                 220

Leu Thr Asn Trp Pro Ser Val Pro Pro Thr Ile Lys Glu Glu Glu Asn
225                 230                 235                 240

Ser Glu Glu Glu Leu Ala Ala Ala Thr Thr Ser Lys Glu Gln Glu Pro
                245                 250                 255

Ile Gly Thr Asp Leu Asp Ala Val Arg Thr Pro Glu Pro Leu Glu Glu
            260                 265                 270

Phe Pro Lys Arg Glu Asp Gln Glu Gly Ser Pro Pro Glu Thr Ser Leu
        275                 280                 285

Pro Tyr Lys Trp Val Val Glu Ala Ala Asn Leu Leu Ile Pro Ala Val
    290                 295                 300

Gly Ser Ser Leu Ser Glu Ala Leu Asp Leu Ile Glu Ser Asp Pro Asp
305                 310                 315                 320

Ala Trp Cys Asp Leu Ser Lys Phe Asp Leu Pro Glu Glu Pro Ser Ala
                325                 330                 335

Glu Asp Ser Ile Asn Asn Ser Leu Val Gln Leu Gln Ala Ser His Gln
            340                 345                 350

Gln Gln Val Leu Pro Pro Arg Gln Pro Ser Ala Leu Val Pro Ser Val
        355                 360                 365

Thr Glu Tyr Arg Leu Asp Gly His Thr Ile Ser Asp Leu Ser Arg Ser

370                 375                 380

Ser Arg Gly Glu Leu Ile Pro Ile Ser Pro Ser Thr Glu Val Gly Gly
385                 390                 395                 400

Ser Gly Ile Gly Thr Pro Pro Ser Val Leu Lys Arg Gln Arg Lys Arg
                405                 410                 415

Arg Val Ala Leu Ser Pro Val Thr Glu Asn Ser Thr Ser Leu Ser Phe
            420                 425                 430

Leu Asp Ser Cys Asn Ser Leu Thr Pro Lys Ser Thr Pro Val Lys Thr
        435                 440                 445

Leu Pro Phe Ser Pro Ser Gln Phe Leu Asn Phe Trp Asn Lys Gln Asp
    450                 455                 460

Thr Leu Glu Leu Glu Ser Pro Ser Leu Thr Ser Thr Pro Val Cys Ser
465                 470                 475                 480

Gln Lys Val Val Val Thr Thr Pro Leu His Arg Asp Lys Thr Pro Leu
                485                 490                 495

His Gln Lys His Ala Ala Phe Val Thr Pro Asp Gln Lys Tyr Ser Met
            500                 505                 510

Asp Asn Thr Pro His Thr Pro Thr Pro Phe Lys Asn Ala Leu Glu Lys
        515                 520                 525

Tyr Gly Pro Leu Lys Pro Leu Pro Gln Thr Pro His Leu Glu Glu Asp
    530                 535                 540

Leu Lys Glu Val Leu Arg Ser Glu Ala Gly Ile Glu Leu Ile Ile Glu
545                 550                 555                 560

Asp Asp Ile Arg Pro Glu Lys Gln Lys Arg Lys Pro Gly Leu Arg Arg
                565                 570                 575

Ser Pro Ile Lys Lys Val Arg Lys Ser Leu Ala Leu Asp Ile Val Asp
            580                 585                 590

Glu Asp Val Lys Leu Met Met Ser Thr Leu Pro Lys Ser Leu Ser Leu
        595                 600                 605

Pro Thr Thr Ala Pro Ser Asn Ser Ser Ser Leu Thr Leu Ser Gly Ile
    610                 615                 620

Lys Glu Asp Asn Ser Leu Leu Asn Gln Gly Phe Leu Gln Ala Lys Pro
625                 630                 635                 640

Glu Lys Ala Ala Val Ala Gln Lys Pro Arg Ser His Phe Thr Thr Pro
                645                 650                 655

Ala Pro Met Ser Ser Ala Trp Lys Thr Val Ala Cys Gly Gly Thr Arg
            660                 665                 670

Asp Gln Leu Phe Met Gln Glu Lys Ala Arg Gln Leu Leu Gly Arg Leu
        675                 680                 685

Lys Pro Ser His Thr Ser Arg Thr Leu Ile Leu Ser
    690                 695                 700

<210>  233
<211>  161
<212>  PRT
<213>  Homo sapiens

<400>  233

Met Thr Leu Glu Glu Leu Val Ala Cys Asp Asn Ala Ala Gln Lys Met
1               5                   10                  15

Gln Thr Val Thr Ala Ala Val Glu Glu Leu Leu Val Ala Ala Gln Arg
            20                  25                  30

Gln Asp Arg Leu Thr Val Gly Val Tyr Glu Ser Ala Lys Leu Met Asn
        35                  40                  45

Val Asp Pro Asp Ser Val Val Leu Cys Leu Leu Ala Ile Asp Glu Glu
    50                  55                  60

Glu Glu Asp Asp Ile Ala Leu Gln Ile His Phe Thr Leu Ile Gln Ser
65                  70                  75                  80

Phe Cys Cys Asp Asn Asp Ile Asn Ile Val Arg Val Ser Gly Asn Ala
                85                  90                  95

Arg Leu Ala Gln Leu Leu Gly Glu Pro Ala Glu Thr Gln Gly Thr Thr
            100                 105                 110

Glu Ala Arg Asp Leu His Cys Leu Pro Phe Leu Gln Asn Pro His Thr
        115                 120                 125

Asp Ala Trp Lys Ser His Gly Leu Val Glu Val Ala Ser Tyr Cys Glu
    130                 135                 140

Glu Ser Arg Gly Asn Asn Gln Trp Val Pro Tyr Ile Ser Leu Gln Glu
145                 150                 155                 160

Arg

<210>  234
<211>  788
<212>  PRT
<213>  Homo sapiens

<400>  234

Met Ala Met Glu Ser Thr Ala Thr Ala Ala Val Ala Ala Glu Leu Val
1               5                   10                  15

Ser Ala Asp Lys Ile Glu Asp Val Pro Ala Pro Ser Thr Ser Ala Asp
            20                  25                  30

Lys Val Glu Ser Leu Asp Val Asp Ser Glu Ala Lys Lys Leu Leu Gly
        35                  40                  45

Leu Gly Gln Lys His Leu Val Met Gly Asp Ile Pro Ala Ala Val Asn
    50                  55                  60

Ala Phe Gln Glu Ala Ala Ser Leu Leu Gly Lys Lys Tyr Gly Glu Thr
65                  70                  75                  80

Ala Asn Glu Cys Gly Glu Ala Phe Phe Phe Tyr Gly Lys Ser Leu Leu
                85                  90                  95

Glu Leu Ala Arg Met Glu Asn Gly Val Leu Gly Asn Ala Leu Glu Gly
            100                 105                 110

Val His Val Glu Glu Glu Glu Gly Glu Lys Thr Glu Asp Glu Ser Leu
        115                 120                 125

Val Glu Asn Asn Asp Asn Ile Asp Glu Glu Ala Arg Glu Glu Leu Arg
    130                 135                 140

Glu Gln Val Tyr Asp Ala Met Gly Glu Lys Glu Glu Ala Lys Lys Thr
145                 150                 155                 160

Glu Asp Lys Ser Leu Ala Lys Pro Glu Thr Asp Lys Glu Gln Asp Ser
                165                 170                 175

Glu Met Glu Lys Gly Gly Arg Glu Asp Met Asp Ile Ser Lys Ser Ala
            180                 185                 190

Glu Glu Pro Gln Glu Lys Val Asp Leu Thr Leu Asp Trp Leu Thr Glu
        195                 200                 205

Thr Ser Glu Glu Ala Lys Gly Gly Ala Ala Pro Glu Gly Pro Asn Glu
    210                 215                 220

Ala Glu Val Thr Ser Gly Lys Pro Glu Gln Glu Val Pro Asp Ala Glu
225                 230                 235                 240

Glu Glu Lys Ser Val Ser Gly Thr Asp Val Gln Glu Glu Cys Arg Glu
                245                 250                 255

Lys Gly Gly Gln Glu Lys Gln Gly Glu Val Ile Val Ser Ile Glu Glu
            260                 265                 270

Lys Pro Lys Glu Val Ser Glu Glu Gln Pro Val Val Thr Leu Glu Lys

275                 280                 285

Gln Gly Thr Ala Val Glu Val Glu Ala Glu Ser Leu Asp Pro Thr Val
    290                 295                 300

Lys Pro Val Asp Val Gly Gly Asp Glu Pro Glu Glu Lys Val Val Thr
305                 310                 315                 320

Ser Glu Asn Glu Ala Gly Lys Ala Val Leu Glu Gln Leu Val Gly Gln
                325                 330                 335

Glu Val Pro Pro Ala Glu Glu Ser Pro Glu Val Thr Thr Glu Ala Ala
            340                 345                 350

Glu Ala Ser Ala Val Glu Ala Gly Ser Glu Val Ser Glu Lys Pro Gly
        355                 360                 365

Gln Glu Ala Pro Val Leu Pro Lys Asp Gly Ala Val Asn Gly Pro Ser
    370                 375                 380

Val Val Gly Asp Gln Thr Pro Ile Glu Pro Gln Thr Ser Ile Glu Arg
385                 390                 395                 400

Leu Thr Glu Thr Lys Asp Gly Ser Gly Leu Glu Glu Lys Val Arg Ala
                405                 410                 415

Lys Leu Val Pro Ser Gln Glu Glu Thr Lys Leu Ser Val Glu Glu Ser
            420                 425                 430

Glu Ala Ala Gly Asp Gly Val Asp Thr Lys Val Ala Gln Gly Ala Thr
        435                 440                 445

Glu Lys Ser Pro Glu Asp Lys Val Gln Ile Ala Ala Asn Glu Glu Thr
    450                 455                 460

Gln Glu Arg Glu Glu Gln Met Lys Glu Gly Glu Glu Thr Glu Gly Ser
465                 470                 475                 480

Glu Glu Asp Asp Lys Glu Asn Asp Lys Thr Glu Glu Met Pro Asn Asp
                485                 490                 495

Ser Val Leu Glu Asn Lys Ser Leu Gln Glu Asn Glu Glu Glu Glu Ile
            500                 505                 510

Gly Asn Leu Glu Leu Ala Trp Asp Met Leu Asp Leu Ala Lys Ile Ile
        515                 520                 525

Phe Lys Arg Gln Glu Thr Lys Glu Ala Gln Leu Tyr Ala Ala Gln Ala
    530                 535                 540

His Leu Lys Leu Gly Glu Val Ser Val Glu Ser Glu Asn Tyr Val Gln
545                 550                 555                 560

Ala Val Glu Glu Phe Gln Ser Cys Leu Asn Leu Gln Glu Gln Tyr Leu
                565                 570                 575

Glu Ala His Asp Arg Leu Leu Ala Glu Thr His Tyr Gln Leu Gly Leu
            580                 585                 590

Ala Tyr Gly Tyr Asn Ser Gln Tyr Asp Glu Ala Val Ala Gln Phe Ser
        595                 600                 605

Lys Ser Ile Glu Val Ile Glu Asn Arg Met Ala Val Leu Asn Glu Gln
    610                 615                 620

Val Lys Glu Ala Glu Gly Ser Ser Ala Glu Tyr Lys Lys Glu Ile Glu
625                 630                 635                 640

Glu Leu Lys Glu Leu Leu Pro Glu Ile Arg Glu Lys Ile Glu Asp Ala
                645                 650                 655

Lys Glu Ser Gln Arg Ser Gly Asn Val Ala Glu Leu Ala Leu Lys Ala
            660                 665                 670

Thr Leu Val Glu Ser Ser Thr Ser Gly Phe Thr Pro Gly Gly Gly Gly
        675                 680                 685

Ser Ser Val Ser Met Ile Ala Ser Arg Lys Pro Thr Asp Gly Ala Ser
    690                 695                 700

Ser Ser Asn Cys Val Thr Asp Ile Ser His Leu Val Arg Lys Lys Arg
705                 710                 715                 720

Lys Pro Glu Glu Glu Ser Pro Arg Lys Asp Asp Ala Lys Lys Ala Lys
                725                 730                 735

Gln Glu Pro Glu Val Asn Gly Gly Ser Gly Asp Ala Val Pro Ser Gly
            740                 745                 750

Asn Glu Val Ser Glu Asn Met Glu Glu Glu Ala Glu Asn Gln Ala Glu
        755                 760                 765

Ser Arg Ala Ala Val Glu Gly Thr Val Glu Ala Gly Ala Thr Val Glu
    770                 775                 780

Ser Thr Ala Cys
785

<210>  235
<211>  449
<212>  PRT
<213>  Homo sapiens

<400>  235

Met Ala Met Glu Ser Thr Ala Thr Ala Ala Val Ala Ala Glu Leu Val
1               5                   10                  15

Ser Ala Asp Lys Ile Glu Asp Val Pro Ala Pro Ser Thr Ser Ala Asp
            20                  25                  30

Lys Val Glu Ser Leu Asp Val Asp Ser Glu Ala Lys Lys Leu Leu Gly
        35                  40                  45

Leu Gly Gln Lys His Leu Val Met Gly Asp Ile Pro Ala Ala Val Asn
    50                  55                  60

Ala Phe Gln Glu Ala Ala Ser Leu Leu Gly Lys Lys Tyr Gly Glu Thr
65                  70                  75                  80

Ala Asn Glu Cys Gly Glu Ala Phe Phe Phe Tyr Gly Lys Ser Leu Leu
                85                  90                  95

Glu Leu Ala Arg Met Glu Asn Gly Val Leu Gly Asn Ala Leu Glu Gly
            100                 105                 110

Val His Val Glu Glu Glu Glu Gly Glu Lys Thr Glu Asp Glu Ser Leu
        115                 120                 125

Val Glu Asn Asn Asp Asn Ile Asp Glu Thr Glu Gly Ser Glu Glu Asp
    130                 135                 140

Asp Lys Glu Asn Asp Lys Thr Glu Glu Met Pro Asn Asp Ser Val Leu
145                 150                 155                 160

Glu Asn Lys Ser Leu Gln Glu Asn Glu Glu Glu Glu Ile Gly Asn Leu
                165                 170                 175

Glu Leu Ala Trp Asp Met Leu Asp Leu Ala Lys Ile Ile Phe Lys Arg
            180                 185                 190

Gln Glu Thr Lys Glu Ala Gln Leu Tyr Ala Ala Gln Ala His Leu Lys
        195                 200                 205

Leu Gly Glu Val Ser Val Glu Ser Glu Asn Tyr Val Gln Ala Val Glu
    210                 215                 220

Glu Phe Gln Ser Cys Leu Asn Leu Gln Glu Gln Tyr Leu Glu Ala His
225                 230                 235                 240

Asp Arg Leu Leu Ala Glu Thr His Tyr Gln Leu Gly Leu Ala Tyr Gly
                245                 250                 255

Tyr Asn Ser Gln Tyr Asp Glu Ala Val Ala Gln Phe Ser Lys Ser Ile
            260                 265                 270

Glu Val Ile Glu Asn Arg Met Ala Val Leu Asn Glu Gln Val Lys Glu
        275                 280                 285

Ala Glu Gly Ser Ser Ala Glu Tyr Lys Lys Glu Ile Glu Glu Leu Lys
    290                 295                 300

Glu Leu Leu Pro Glu Ile Arg Glu Lys Ile Glu Asp Ala Lys Glu Ser
305                 310                 315                 320

Gln Arg Ser Gly Asn Val Ala Glu Leu Ala Leu Lys Ala Thr Leu Val
                325                 330                 335

Glu Ser Ser Thr Ser Gly Phe Thr Pro Gly Gly Gly Gly Ser Ser Val
            340                 345                 350

Ser Met Ile Ala Ser Arg Lys Pro Thr Asp Gly Ala Ser Ser Ser Asn
        355                 360                 365

Cys Val Thr Asp Ile Ser His Leu Val Arg Lys Lys Arg Lys Pro Glu
    370                 375                 380

Glu Glu Ser Pro Arg Lys Asp Asp Ala Lys Lys Ala Lys Gln Glu Pro
385                 390                 395                 400

Glu Val Asn Gly Gly Ser Gly Asp Ala Val Pro Ser Gly Asn Glu Val
                405                 410                 415

Ser Glu Asn Met Glu Glu Glu Ala Glu Asn Gln Ala Glu Ser Arg Ala
            420                 425                 430

Ala Val Glu Gly Thr Val Glu Ala Gly Ala Thr Val Glu Ser Thr Ala
        435                 440                 445

Cys

<210>  236
<211>  790
<212>  PRT
<213>  Homo sapiens

<400>  236

Met Phe Leu Leu Leu Leu His Leu Gln Ile Lys Trp Arg Ala Thr Ile
1               5                   10                  15

Asn Leu Leu Ser Val Thr Glu Asp Gly Leu His Phe Val Glu Tyr Tyr
            20                  25                  30

Leu Asn Arg Ile Ile His Leu Asp Val Asp Ser Glu Ala Lys Lys Leu
        35                  40                  45

Leu Gly Leu Gly Gln Lys His Leu Val Met Gly Asp Ile Pro Ala Ala
    50                  55                  60

Val Asn Ala Phe Gln Glu Ala Ala Ser Leu Leu Gly Lys Lys Tyr Gly
65                  70                  75                  80

Glu Thr Ala Asn Glu Cys Gly Glu Ala Phe Phe Phe Tyr Gly Lys Ser
                85                  90                  95

Leu Leu Glu Leu Ala Arg Met Glu Asn Gly Val Leu Gly Asn Ala Leu
            100                 105                 110

Glu Gly Val His Val Glu Glu Glu Glu Gly Glu Lys Thr Glu Asp Glu
        115                 120                 125

Ser Leu Val Glu Asn Asn Asp Asn Ile Asp Glu Glu Ala Arg Glu Glu
    130                 135                 140

Leu Arg Glu Gln Val Tyr Asp Ala Met Gly Glu Lys Glu Glu Ala Lys
145                 150                 155                 160

Lys Thr Glu Asp Lys Ser Leu Ala Lys Pro Glu Thr Asp Lys Glu Gln
                165                 170                 175

Asp Ser Glu Met Glu Lys Gly Gly Arg Glu Asp Met Asp Ile Ser Lys
            180                 185                 190

Ser Ala Glu Glu Pro Gln Glu Lys Val Asp Leu Thr Leu Asp Trp Leu
        195                 200                 205

Thr Glu Thr Ser Glu Glu Ala Lys Gly Gly Ala Ala Pro Glu Gly Pro
    210                 215                 220

Asn Glu Ala Glu Val Thr Ser Gly Lys Pro Glu Gln Glu Val Pro Asp
225                 230                 235                 240

Ala Glu Glu Glu Lys Ser Val Ser Gly Thr Asp Val Gln Glu Glu Cys
                245                 250                 255

Arg Glu Lys Gly Gly Gln Glu Lys Gln Gly Glu Val Ile Val Ser Ile
            260                 265                 270

Glu Glu Lys Pro Lys Glu Val Ser Glu Glu Gln Pro Val Val Thr Leu
        275                 280                 285

Glu Lys Gln Gly Thr Ala Val Glu Val Glu Ala Glu Ser Leu Asp Pro
    290                 295                 300

Thr Val Lys Pro Val Asp Val Gly Gly Asp Glu Pro Glu Glu Lys Val
305                 310                 315                 320

Val Thr Ser Glu Asn Glu Ala Gly Lys Ala Val Leu Glu Gln Leu Val
                325                 330                 335

Gly Gln Glu Val Pro Pro Ala Glu Glu Ser Pro Glu Val Thr Thr Glu
            340                 345                 350

Ala Ala Glu Ala Ser Ala Val Glu Ala Gly Ser Glu Val Ser Glu Lys

355                 360                 365

Pro Gly Gln Glu Ala Pro Val Leu Pro Lys Asp Gly Ala Val Asn Gly
    370                 375                 380

Pro Ser Val Val Gly Asp Gln Thr Pro Ile Glu Pro Gln Thr Ser Ile
385                 390                 395                 400

Glu Arg Leu Thr Glu Thr Lys Asp Gly Ser Gly Leu Glu Glu Lys Val
                405                 410                 415

Arg Ala Lys Leu Val Pro Ser Gln Glu Glu Thr Lys Leu Ser Val Glu
            420                 425                 430

Glu Ser Glu Ala Ala Gly Asp Gly Val Asp Thr Lys Val Ala Gln Gly
        435                 440                 445

Ala Thr Glu Lys Ser Pro Glu Asp Lys Val Gln Ile Ala Ala Asn Glu
    450                 455                 460

Glu Thr Gln Glu Arg Glu Glu Gln Met Lys Glu Gly Glu Glu Thr Glu
465                 470                 475                 480

Gly Ser Glu Glu Asp Asp Lys Glu Asn Asp Lys Thr Glu Glu Met Pro
                485                 490                 495

Asn Asp Ser Val Leu Glu Asn Lys Ser Leu Gln Glu Asn Glu Glu Glu
            500                 505                 510

Glu Ile Gly Asn Leu Glu Leu Ala Trp Asp Met Leu Asp Leu Ala Lys
        515                 520                 525

Ile Ile Phe Lys Arg Gln Glu Thr Lys Glu Ala Gln Leu Tyr Ala Ala
    530                 535                 540

Gln Ala His Leu Lys Leu Gly Glu Val Ser Val Glu Ser Glu Asn Tyr
545                 550                 555                 560

Val Gln Ala Val Glu Glu Phe Gln Ser Cys Leu Asn Leu Gln Glu Gln
                565                 570                 575

Tyr Leu Glu Ala His Asp Arg Leu Leu Ala Glu Thr His Tyr Gln Leu
            580                 585                 590

Gly Leu Ala Tyr Gly Tyr Asn Ser Gln Tyr Asp Glu Ala Val Ala Gln
        595                 600                 605

Phe Ser Lys Ser Ile Glu Val Ile Glu Asn Arg Met Ala Val Leu Asn
    610                 615                 620

Glu Gln Val Lys Glu Ala Glu Gly Ser Ser Ala Glu Tyr Lys Lys Glu
625                 630                 635                 640

Ile Glu Glu Leu Lys Glu Leu Leu Pro Glu Ile Arg Glu Lys Ile Glu
                645                 650                 655

Asp Ala Lys Glu Ser Gln Arg Ser Gly Asn Val Ala Glu Leu Ala Leu
            660                 665                 670

Lys Ala Thr Leu Val Glu Ser Ser Thr Ser Gly Phe Thr Pro Gly Gly
        675                 680                 685

Gly Gly Ser Ser Val Ser Met Ile Ala Ser Arg Lys Pro Thr Asp Gly
    690                 695                 700

Ala Ser Ser Ser Asn Cys Val Thr Asp Ile Ser His Leu Val Arg Lys
705                 710                 715                 720

Lys Arg Lys Pro Glu Glu Glu Ser Pro Arg Lys Asp Asp Ala Lys Lys
                725                 730                 735

Ala Lys Gln Glu Pro Glu Val Asn Gly Gly Ser Gly Asp Ala Val Pro
            740                 745                 750

Ser Gly Asn Glu Val Ser Glu Asn Met Glu Glu Glu Ala Glu Asn Gln
        755                 760                 765

Ala Glu Ser Arg Ala Ala Val Glu Gly Thr Val Glu Ala Gly Ala Thr
    770                 775                 780

Val Glu Ser Thr Ala Cys
785                 790

<210> 237
<211> 445
<212> PRT
<213> Homo sapiens

<400> 237

Met Pro Ser Arg Ala Glu Asp Tyr Glu Val Leu Tyr Thr Ile Gly Thr
1               5                   10                  15

Gly Ser Tyr Gly Arg Cys Gln Lys Ile Arg Arg Lys Ser Asp Gly Lys
            20                  25                  30

Ile Leu Val Trp Lys Glu Leu Asp Tyr Gly Ser Met Thr Glu Ala Glu
        35                  40                  45

Lys Gln Met Leu Val Ser Glu Val Asn Leu Leu Arg Glu Leu Lys His
    50                  55                  60

Pro Asn Ile Val Arg Tyr Tyr Asp Arg Ile Ile Asp Arg Thr Asn Thr
65                  70                  75                  80

Thr Leu Tyr Ile Val Met Glu Tyr Cys Glu Gly Gly Asp Leu Ala Ser
                85                  90                  95

Val Ile Thr Lys Gly Thr Lys Glu Arg Gln Tyr Leu Asp Glu Glu Phe
            100                 105                 110

Val Leu Arg Val Met Thr Gln Leu Thr Leu Ala Leu Lys Glu Cys His
        115                 120                 125

Arg Arg Ser Asp Gly Gly His Thr Val Leu His Arg Asp Leu Lys Pro
    130                 135                 140

Ala Asn Val Phe Leu Asp Gly Lys Gln Asn Val Lys Leu Gly Asp Phe
145                 150                 155                 160

Gly Leu Ala Arg Ile Leu Asn His Asp Thr Ser Phe Ala Lys Thr Phe
                165                 170                 175

Val Gly Thr Pro Tyr Tyr Met Ser Pro Glu Gln Met Asn Arg Met Ser
            180                 185                 190

Tyr Asn Glu Lys Ser Asp Ile Trp Ser Leu Gly Cys Leu Leu Tyr Glu
        195                 200                 205

Leu Cys Ala Leu Met Pro Pro Phe Thr Ala Phe Ser Gln Lys Glu Leu
    210                 215                 220

Ala Gly Lys Ile Arg Glu Gly Lys Phe Arg Arg Ile Pro Tyr Arg Tyr
225                 230                 235                 240

Ser Asp Glu Leu Asn Glu Ile Ile Thr Arg Met Leu Asn Leu Lys Asp
                245                 250                 255

Tyr His Arg Pro Ser Val Glu Glu Ile Leu Glu Asn Pro Leu Ile Ala
            260                 265                 270

Asp Leu Val Ala Asp Glu Gln Arg Arg Asn Leu Glu Arg Arg Gly Arg
        275                 280                 285

Gln Leu Gly Glu Pro Glu Lys Ser Gln Asp Ser Ser Pro Val Leu Ser
    290                 295                 300

Glu Leu Lys Leu Lys Glu Ile Gln Leu Gln Glu Arg Glu Arg Ala Leu
305                 310                 315                 320

Lys Ala Arg Glu Glu Arg Leu Glu Gln Lys Glu Gln Glu Leu Cys Val
                325                 330                 335

Arg Glu Arg Leu Ala Glu Asp Lys Leu Ala Arg Ala Glu Asn Leu Leu
            340                 345                 350

Lys Asn Tyr Ser Leu Leu Lys Glu Arg Lys Phe Leu Ser Leu Ala Ser
        355                 360                 365

Asn Pro Glu Leu Leu Asn Leu Pro Ser Ser Val Ile Lys Lys Lys Val
    370                 375                 380

His Phe Ser Gly Glu Ser Lys Glu Asn Ile Met Arg Ser Glu Asn Ser
385                 390                 395                 400

Glu Ser Gln Leu Thr Ser Lys Ser Lys Cys Lys Asp Leu Lys Lys Arg
                405                 410                 415

Leu His Ala Ala Gln Leu Arg Ala Gln Ala Leu Ser Asp Ile Glu Lys
            420                 425                 430

Asn Tyr Gln Leu Lys Ser Arg Gln Ile Leu Gly Met Arg
        435                 440                 445

<210>  238
<211>  231
<212>  PRT
<213>  Homo sapiens

<400>  238

Met Ser Gly Glu Glu Asn Pro Ala Ser Lys Pro Thr Pro Val Gln Asp
1               5                   10                  15

Val Gln Gly Asp Gly Arg Trp Met Ser Leu His His Arg Phe Val Ala
            20                  25                  30

Asp Ser Lys Asp Lys Glu Pro Glu Val Val Phe Ile Gly Asp Ser Leu
        35                  40                  45

Val Gln Leu Met His Gln Cys Glu Ile Trp Arg Glu Leu Phe Ser Pro
    50                  55                  60

Leu His Ala Leu Asn Phe Gly Ile Gly Gly Asp Gly Thr Gln His Val
65                  70                  75                  80

Leu Trp Arg Leu Glu Asn Gly Glu Leu Glu His Ile Arg Pro Lys Ile
                85                  90                  95

Val Val Val Trp Val Gly Thr Asn Asn His Gly His Thr Ala Glu Gln
            100                 105                 110

Val Thr Gly Gly Ile Lys Ala Ile Val Gln Leu Val Asn Glu Arg Gln
        115                 120                 125

Pro Gln Ala Arg Val Val Val Leu Gly Leu Leu Pro Arg Gly Gln His
    130                 135                 140

Pro Asn Pro Leu Arg Glu Lys Asn Arg Gln Val Asn Glu Leu Val Arg
145                 150                 155                 160

Ala Ala Leu Ala Gly His Pro Arg Ala His Phe Leu Asp Ala Asp Pro
                165                 170                 175

Gly Phe Val His Ser Asp Gly Thr Ile Ser His His Asp Met Tyr Asp
            180                 185                 190

Tyr Leu His Leu Ser Arg Leu Gly Tyr Thr Pro Val Cys Arg Ala Leu
        195                 200                 205

His Ser Leu Leu Leu Arg Leu Leu Ala Gln Asp Gln Gly Gln Gly Ala
    210                 215                 220

Pro Leu Leu Glu Pro Ala Pro
225                 230

<210>  239
<211>  392
<212>  PRT
<213>  Homo sapiens

<400>  239

Met Gly Val Asn Gln Ser Val Gly Phe Pro Pro Val Thr Gly Pro His
1               5                   10                  15

Leu Val Gly Cys Gly Asp Val Met Glu Gly Gln Asn Leu Gln Gly Ser
            20                  25                  30

Phe Phe Arg Leu Phe Tyr Pro Cys Gln Lys Ala Glu Glu Thr Met Glu
        35                  40                  45

Gln Pro Leu Trp Ile Pro Arg Tyr Glu Tyr Cys Thr Gly Leu Ala Glu
    50                  55                  60

Tyr Leu Gln Phe Asn Lys Arg Cys Gly Gly Leu Leu Phe Asn Leu Ala
65                  70                  75                  80

Val Gly Ser Cys Arg Leu Pro Val Ser Trp Asn Gly Pro Phe Lys Thr
                85                  90                  95

Lys Asp Ser Gly Tyr Pro Leu Ile Ile Phe Ser His Gly Leu Gly Ala
            100                 105                 110

Phe Arg Thr Leu Tyr Ser Ala Phe Cys Met Glu Leu Ala Ser Arg Gly
        115                 120                 125

Phe Val Val Ala Val Pro Glu His Arg Asp Arg Ser Ala Ala Thr Thr
    130                 135                 140

Tyr Phe Cys Lys Gln Ala Pro Glu Glu Asn Gln Pro Thr Asn Glu Ser
145                 150                 155                 160

Leu Gln Glu Glu Trp Ile Pro Phe Arg Arg Val Glu Glu Gly Glu Lys
                165                 170                 175

Glu Phe His Val Arg Asn Pro Gln Val His Gln Arg Val Ser Glu Cys
            180                 185                 190

Leu Arg Val Leu Lys Ile Leu Gln Glu Val Thr Ala Gly Gln Thr Val
        195                 200                 205

Phe Asn Ile Leu Pro Gly Gly Leu Asp Leu Met Thr Leu Lys Gly Asn
    210                 215                 220

Ile Asp Met Ser Arg Val Ala Val Met Gly His Ser Phe Gly Gly Ala
225                 230                 235                 240

Thr Ala Ile Leu Ala Leu Ala Lys Glu Thr Gln Phe Arg Cys Ala Val
                245                 250                 255

Ala Leu Asp Ala Trp Met Phe Pro Leu Glu Arg Asp Phe Tyr Pro Lys
            260                 265                 270

Ala Arg Gly Pro Val Phe Phe Ile Asn Thr Glu Lys Phe Gln Thr Met
        275                 280                 285

Glu Ser Val Asn Leu Met Lys Lys Ile Cys Ala Gln His Glu Gln Ser
    290                 295                 300

Arg Ile Ile Thr Val Leu Gly Ser Val His Arg Ser Gln Thr Asp Phe
305                 310                 315                 320

Ala Phe Val Thr Gly Asn Leu Ile Gly Lys Phe Phe Ser Thr Glu Thr
                325                 330                 335

Arg Gly Ser Leu Asp Pro Tyr Glu Gly Gln Glu Val Met Val Arg Ala
            340                 345                 350

Met Leu Ala Phe Leu Gln Lys His Leu Asp Leu Lys Glu Asp Tyr Asn
        355                 360                 365

Gln Trp Asn Asn Leu Ile Glu Gly Ile Gly Pro Ser Leu Thr Pro Gly
    370                 375                 380

Ala Pro His His Leu Ser Ser Leu
385                 390

<210>  240
<211>  261
<212>  PRT
<213>  Homo sapiens

<400>  240

Met Phe Glu Ala Arg Leu Val Gln Gly Ser Ile Leu Lys Lys Val Leu
1               5                   10                  15

Glu Ala Leu Lys Asp Leu Ile Asn Glu Ala Cys Trp Asp Ile Ser Ser
            20                  25                  30

Ser Gly Val Asn Leu Gln Ser Met Asp Ser Ser His Val Ser Leu Val

35                  40                  45

Gln Leu Thr Leu Arg Ser Glu Gly Phe Asp Thr Tyr Arg Cys Asp Arg
    50                  55                  60

Asn Leu Ala Met Gly Val Asn Leu Thr Ser Met Ser Lys Ile Leu Lys
65                  70                  75                  80

Cys Ala Gly Asn Glu Asp Ile Ile Thr Leu Arg Ala Glu Asp Asn Ala
                85                  90                  95

Asp Thr Leu Ala Leu Val Phe Glu Ala Pro Asn Gln Glu Lys Val Ser
            100                 105                 110

Asp Tyr Glu Met Lys Leu Met Asp Leu Asp Val Glu Gln Leu Gly Ile
        115                 120                 125

Pro Glu Gln Glu Tyr Ser Cys Val Val Lys Met Pro Ser Gly Glu Phe
    130                 135                 140

Ala Arg Ile Cys Arg Asp Leu Ser His Ile Gly Asp Ala Val Val Ile
145                 150                 155                 160

Ser Cys Ala Lys Asp Gly Val Lys Phe Ser Ala Ser Gly Glu Leu Gly
                165                 170                 175

Asn Gly Asn Ile Lys Leu Ser Gln Thr Ser Asn Val Asp Lys Glu Glu
            180                 185                 190

Glu Ala Val Thr Ile Glu Met Asn Glu Pro Val Gln Leu Thr Phe Ala
        195                 200                 205

Leu Arg Tyr Leu Asn Phe Phe Thr Lys Ala Thr Pro Leu Ser Ser Thr
    210                 215                 220

Val Thr Leu Ser Met Ser Ala Asp Val Pro Leu Val Val Glu Tyr Lys
225                 230                 235                 240

Ile Ala Asp Met Gly His Leu Lys Tyr Tyr Leu Ala Pro Lys Ile Glu
                245                 250                 255

Asp Glu Glu Gly Ser
            260

<210>  241
<211>  261
<212>  PRT
<213>  Homo sapiens

<400>  241

Met Phe Glu Ala Arg Leu Val Gln Gly Ser Ile Leu Lys Lys Val Leu
1               5                   10                  15

Glu Ala Leu Lys Asp Leu Ile Asn Glu Ala Cys Trp Asp Ile Ser Ser
            20                  25                  30

Ser Gly Val Asn Leu Gln Ser Met Asp Ser Ser His Val Ser Leu Val
        35                  40                  45

Gln Leu Thr Leu Arg Ser Glu Gly Phe Asp Thr Tyr Arg Cys Asp Arg
    50                  55                  60

Asn Leu Ala Met Gly Val Asn Leu Thr Ser Met Ser Lys Ile Leu Lys
65                  70                  75                  80

Cys Ala Gly Asn Glu Asp Ile Ile Thr Leu Arg Ala Glu Asp Asn Ala
                85                  90                  95

Asp Thr Leu Ala Leu Val Phe Glu Ala Pro Asn Gln Glu Lys Val Ser
            100                 105                 110

Asp Tyr Glu Met Lys Leu Met Asp Leu Asp Val Glu Gln Leu Gly Ile
        115                 120                 125

Pro Glu Gln Glu Tyr Ser Cys Val Val Lys Met Pro Ser Gly Glu Phe
    130                 135                 140

Ala Arg Ile Cys Arg Asp Leu Ser His Ile Gly Asp Ala Val Val Ile
145                 150                 155                 160

Ser Cys Ala Lys Asp Gly Val Lys Phe Ser Ala Ser Gly Glu Leu Gly
                165                 170                 175

Asn Gly Asn Ile Lys Leu Ser Gln Thr Ser Asn Val Asp Lys Glu Glu
            180                 185                 190

Glu Ala Val Thr Ile Glu Met Asn Glu Pro Val Gln Leu Thr Phe Ala
        195                 200                 205

Leu Arg Tyr Leu Asn Phe Phe Thr Lys Ala Thr Pro Leu Ser Ser Thr
    210                 215                 220

Val Thr Leu Ser Met Ser Ala Asp Val Pro Leu Val Val Glu Tyr Lys
225                 230                 235                 240

Ile Ala Asp Met Gly His Leu Lys Tyr Tyr Leu Ala Pro Lys Ile Glu
                245                 250                 255

Asp Glu Glu Gly Ser
            260

<210>  242
<211>  603
<212>  PRT
<213>  Homo sapiens

<400>  242

Met Ser Ala Ala Val Thr Ala Gly Lys Leu Ala Arg Ala Pro Ala Asp
1               5                   10                  15

Pro Gly Lys Ala Gly Val Pro Gly Val Ala Ala Pro Gly Ala Pro Ala
            20                  25                  30

Ala Ala Pro Pro Ala Lys Glu Ile Pro Glu Val Leu Val Asp Pro Arg
        35                  40                  45

Ser Arg Arg Arg Tyr Val Arg Gly Arg Phe Leu Gly Lys Gly Gly Phe
    50                  55                  60

Ala Lys Cys Phe Glu Ile Ser Asp Ala Asp Thr Lys Glu Val Phe Ala
65                  70                  75                  80

Gly Lys Ile Val Pro Lys Ser Leu Leu Leu Lys Pro His Gln Arg Glu
                85                  90                  95

Lys Met Ser Met Glu Ile Ser Ile His Arg Ser Leu Ala His Gln His
            100                 105                 110

Val Val Gly Phe His Gly Phe Phe Glu Asp Asn Asp Phe Val Phe Val
        115                 120                 125

Val Leu Glu Leu Cys Arg Arg Arg Ser Leu Leu Glu Leu His Lys Arg
    130                 135                 140

Arg Lys Ala Leu Thr Glu Pro Glu Ala Arg Tyr Tyr Leu Arg Gln Ile
145                 150                 155                 160

Val Leu Gly Cys Gln Tyr Leu His Arg Asn Arg Val Ile His Arg Asp
                165                 170                 175

Leu Lys Leu Gly Asn Leu Phe Leu Asn Glu Asp Leu Glu Val Lys Ile
            180                 185                 190

Gly Asp Phe Gly Leu Ala Thr Lys Val Glu Tyr Asp Gly Glu Arg Lys
        195                 200                 205

Lys Thr Leu Cys Gly Thr Pro Asn Tyr Ile Ala Pro Glu Val Leu Ser
    210                 215                 220

Lys Lys Gly His Ser Phe Glu Val Asp Val Trp Ser Ile Gly Cys Ile
225                 230                 235                 240

Met Tyr Thr Leu Leu Val Gly Lys Pro Pro Phe Glu Thr Ser Cys Leu
                245                 250                 255

Lys Glu Thr Tyr Leu Arg Ile Lys Lys Asn Glu Tyr Ser Ile Pro Lys
            260                 265                 270

His Ile Asn Pro Val Ala Ala Ser Leu Ile Gln Lys Met Leu Gln Thr

275                 280                 285

Asp Pro Thr Ala Arg Pro Thr Ile Asn Glu Leu Leu Asn Asp Glu Phe
    290                 295                 300

Phe Thr Ser Gly Tyr Ile Pro Ala Arg Leu Pro Ile Thr Cys Leu Thr
305                 310                 315                 320

Ile Pro Pro Arg Phe Ser Ile Ala Pro Ser Ser Leu Asp Pro Ser Asn
                325                 330                 335

Arg Lys Pro Leu Thr Val Leu Asn Lys Gly Leu Glu Asn Pro Leu Pro
            340                 345                 350

Glu Arg Pro Arg Glu Lys Glu Glu Pro Val Val Arg Glu Thr Gly Glu
        355                 360                 365

Val Val Asp Cys His Leu Ser Asp Met Leu Gln Gln Leu His Ser Val
    370                 375                 380

Asn Ala Ser Lys Pro Ser Glu Arg Gly Leu Val Arg Gln Glu Glu Ala
385                 390                 395                 400

Glu Asp Pro Ala Cys Ile Pro Ile Phe Trp Val Ser Lys Trp Val Asp
                405                 410                 415

Tyr Ser Asp Lys Tyr Gly Leu Gly Tyr Gln Leu Cys Asp Asn Ser Val
            420                 425                 430

Gly Val Leu Phe Asn Asp Ser Thr Arg Leu Ile Leu Tyr Asn Asp Gly
        435                 440                 445

Asp Ser Leu Gln Tyr Ile Glu Arg Asp Gly Thr Glu Ser Tyr Leu Thr
    450                 455                 460

Val Ser Ser His Pro Asn Ser Leu Met Lys Lys Ile Thr Leu Leu Lys
465                 470                 475                 480

Tyr Phe Arg Asn Tyr Met Ser Glu His Leu Leu Lys Ala Gly Ala Asn
                485                 490                 495

Ile Thr Pro Arg Glu Gly Asp Glu Leu Ala Arg Leu Pro Tyr Leu Arg
            500                 505                 510

Thr Trp Phe Arg Thr Arg Ser Ala Ile Ile Leu His Leu Ser Asn Gly
        515                 520                 525

Ser Val Gln Ile Asn Phe Phe Gln Asp His Thr Lys Leu Ile Leu Cys
    530                 535                 540

Pro Leu Met Ala Ala Val Thr Tyr Ile Asp Glu Lys Arg Asp Phe Arg
545                 550                 555                 560

Thr Tyr Arg Leu Ser Leu Leu Glu Glu Tyr Gly Cys Cys Lys Glu Leu
                565                 570                 575

Ala Ser Arg Leu Arg Tyr Ala Arg Thr Met Val Asp Lys Leu Leu Ser
            580                 585                 590

Ser Arg Ser Ala Ser Asn Arg Leu Lys Ala Ser
        595                 600

<210>  243
<211>  456
<212>  PRT
<213>  Homo sapiens

<400>  243

Met Lys Glu Thr Pro Leu Ser Asn Cys Glu Arg Arg Phe Leu Leu Arg
1               5                   10                  15

Ala Ile Glu Glu Lys Lys Arg Leu Asp Gly Arg Gln Thr Tyr Asp Tyr
            20                  25                  30

Arg Asn Ile Arg Ile Ser Phe Gly Thr Asp Tyr Gly Cys Cys Ile Val
        35                  40                  45

Glu Leu Gly Lys Thr Arg Val Leu Gly Gln Val Ser Cys Glu Leu Val
    50                  55                  60

Ser Pro Lys Leu Asn Arg Ala Thr Glu Gly Ile Leu Phe Phe Asn Leu
65                  70                  75                  80

Glu Leu Ser Gln Met Ala Ala Pro Ala Phe Glu Pro Gly Arg Gln Ser
                85                  90                  95

Asp Leu Leu Val Lys Leu Asn Arg Leu Met Glu Arg Cys Leu Arg Asn
            100                 105                 110

Ser Lys Cys Ile Asp Thr Glu Ser Leu Cys Val Val Ala Gly Glu Lys
        115                 120                 125

Val Trp Gln Ile Arg Val Asp Leu His Leu Leu Asn His Asp Gly Asn
    130                 135                 140

Ile Ile Asp Ala Ala Ser Ile Ala Ala Ile Val Ala Leu Cys His Phe
145                 150                 155                 160

Arg Arg Pro Asp Val Ser Val Gln Gly Asp Glu Val Thr Leu Tyr Thr
                165                 170                 175

Pro Glu Glu Arg Asp Pro Val Pro Leu Ser Ile His His Met Pro Ile
            180                 185                 190

Cys Val Ser Phe Ala Phe Phe Gln Gln Gly Thr Tyr Leu Leu Val Asp
        195                 200                 205

Pro Asn Glu Arg Glu Glu Arg Val Met Asp Gly Leu Leu Val Ile Ala
    210                 215                 220

Met Asn Lys His Arg Glu Ile Cys Thr Ile Gln Ser Ser Gly Gly Ile
225                 230                 235                 240

Met Leu Leu Lys Asp Gln Val Leu Arg Cys Ser Lys Ile Ala Gly Val
                245                 250                 255

Lys Val Ala Glu Ile Thr Glu Leu Ile Leu Lys Ala Leu Glu Asn Asp
            260                 265                 270

Gln Lys Val Arg Lys Glu Gly Gly Lys Phe Gly Phe Ala Glu Ser Ile
        275                 280                 285

Ala Asn Gln Arg Ile Thr Ala Phe Lys Met Glu Lys Ala Pro Ile Asp
    290                 295                 300

Thr Ser Asp Val Glu Glu Lys Ala Glu Glu Ile Ile Ala Glu Ala Glu
305                 310                 315                 320

Pro Pro Ser Glu Val Val Ser Thr Pro Val Leu Trp Thr Pro Gly Thr
                325                 330                 335

Ala Gln Ile Gly Glu Gly Val Glu Asn Ser Trp Gly Asp Leu Glu Asp
            340                 345                 350

Ser Glu Lys Glu Asp Asp Glu Gly Gly Gly Asp Gln Ala Ile Ile Leu
        355                 360                 365

Asp Gly Ile Lys Met Asp Thr Gly Val Glu Val Ser Asp Ile Gly Ser
    370                 375                 380

Gln Glu Leu Gly Phe His His Val Gly Gln Thr Gly Leu Glu Phe Leu
385                 390                 395                 400

Thr Ser Asp Ala Pro Ile Ile Leu Ser Asp Ser Glu Glu Glu Glu Met
                405                 410                 415

Ile Ile Leu Glu Pro Asp Lys Asn Pro Lys Lys Ile Arg Thr Gln Thr
            420                 425                 430

Thr Ser Ala Lys Gln Glu Lys Ala Pro Ser Lys Lys Pro Val Lys Arg
        435                 440                 445

Arg Lys Lys Lys Arg Ala Ala Asn
    450                 455

<210>  244
<211>  439
<212>  PRT
<213>  Homo sapiens

<400> 244

Met Lys Glu Thr Pro Leu Ser Asn Cys Glu Arg Arg Phe Leu Leu Arg
1               5                   10                  15

Ala Ile Glu Glu Lys Lys Arg Leu Asp Gly Arg Gln Thr Tyr Asp Tyr
            20                  25                  30

Arg Asn Ile Arg Ile Ser Phe Gly Thr Asp Tyr Gly Cys Cys Ile Val
        35                  40                  45

Glu Leu Gly Lys Thr Arg Val Leu Gly Gln Val Ser Cys Glu Leu Val
    50                  55                  60

Ser Pro Lys Leu Asn Arg Ala Thr Glu Gly Ile Leu Phe Phe Asn Leu
65                  70                  75                  80

Glu Leu Ser Gln Met Ala Ala Pro Ala Phe Glu Pro Gly Arg Gln Ser
                85                  90                  95

Asp Leu Leu Val Lys Leu Asn Arg Leu Met Glu Arg Cys Leu Arg Asn
            100                 105                 110

Ser Lys Cys Ile Asp Thr Glu Ser Leu Cys Val Val Ala Gly Glu Lys
        115                 120                 125

Val Trp Gln Ile Arg Val Asp Leu His Leu Leu Asn His Asp Gly Asn
    130                 135                 140

Ile Ile Asp Ala Ala Ser Ile Ala Ala Ile Val Ala Leu Cys His Phe
145                 150                 155                 160

Arg Arg Pro Asp Val Ser Val Gln Gly Asp Glu Val Thr Leu Tyr Thr
                165                 170                 175

Pro Glu Glu Arg Asp Pro Val Pro Leu Ser Ile His His Met Pro Ile
            180                 185                 190

Cys Val Ser Phe Ala Phe Phe Gln Gln Gly Thr Tyr Leu Leu Val Asp
        195                 200                 205

Pro Asn Glu Arg Glu Glu Arg Val Met Asp Gly Leu Leu Val Ile Ala
    210                 215                 220

Met Asn Lys His Arg Glu Ile Cys Thr Ile Gln Ser Ser Gly Gly Ile
225                 230                 235                 240

Met Leu Leu Lys Asp Gln Val Leu Arg Cys Ser Lys Ile Ala Gly Val
                245                 250                 255

Lys Val Ala Glu Ile Thr Glu Leu Ile Leu Lys Ala Leu Glu Asn Asp
            260                 265                 270

Gln Lys Val Arg Lys Glu Gly Gly Lys Phe Gly Phe Ala Glu Ser Ile
        275                 280                 285

Ala Asn Gln Arg Ile Thr Ala Phe Lys Met Glu Lys Ala Pro Ile Asp
    290                 295                 300

Thr Ser Asp Val Glu Glu Lys Ala Glu Glu Ile Ile Ala Glu Ala Glu
305                 310                 315                 320

Pro Pro Ser Glu Val Val Ser Thr Pro Val Leu Trp Thr Pro Gly Thr
                325                 330                 335

Ala Gln Ile Gly Glu Gly Val Glu Asn Ser Trp Gly Asp Leu Glu Asp
            340                 345                 350

Ser Glu Lys Glu Asp Asp Glu Gly Gly Gly Asp Gln Ala Ile Ile Leu
        355                 360                 365

Asp Gly Ile Lys Met Asp Thr Gly Val Glu Val Ser Asp Ile Gly Ser
    370                 375                 380

Gln Asp Ala Pro Ile Ile Leu Ser Asp Ser Glu Glu Glu Glu Met Ile
385                 390                 395                 400

Ile Leu Glu Pro Asp Lys Asn Pro Lys Lys Ile Arg Thr Gln Thr Thr
                405                 410                 415

Ser Ala Lys Gln Glu Lys Ala Pro Ser Lys Lys Pro Val Lys Arg Arg
            420                 425                 430

Lys Lys Lys Arg Ala Ala Asn
        435

<210>  245
<211>  356
<212>  PRT
<213>  Homo sapiens

<400>  245

Met Ser Leu Trp Val Asp Lys Tyr Arg Pro Cys Ser Leu Gly Arg Leu
1               5                   10                  15

Asp Tyr His Lys Glu Gln Ala Ala Gln Leu Arg Asn Leu Val Gln Cys
            20                  25                  30

Gly Asp Phe Pro His Leu Leu Val Tyr Gly Pro Ser Gly Ala Gly Lys
        35                  40                  45

Lys Thr Arg Ile Met Cys Ile Leu Arg Glu Leu Tyr Gly Val Gly Val
    50                  55                  60

Glu Lys Leu Arg Ile Glu His Gln Thr Ile Thr Thr Pro Ser Lys Lys
65                  70                  75                  80

Lys Ile Glu Ile Ser Thr Ile Ala Ser Asn Tyr His Leu Glu Val Asn
                85                  90                  95

Pro Ser Asp Ala Gly Asn Ser Asp Arg Val Val Ile Gln Glu Met Leu
            100                 105                 110

Lys Thr Val Ala Gln Ser Gln Gln Leu Glu Thr Asn Ser Gln Arg Asp
        115                 120                 125

Phe Lys Val Val Leu Leu Thr Glu Val Asp Lys Leu Thr Lys Asp Ala
    130                 135                 140

Gln His Ala Leu Arg Arg Thr Met Glu Lys Tyr Met Ser Thr Cys Arg
145                 150                 155                 160

Leu Ile Leu Cys Cys Asn Ser Thr Ser Lys Val Ile Pro Pro Ile Arg
                165                 170                 175

Ser Arg Cys Leu Ala Val Arg Val Pro Ala Pro Ser Ile Glu Asp Ile
            180                 185                 190

Cys His Val Leu Ser Thr Val Cys Lys Lys Glu Gly Leu Asn Leu Pro
        195                 200                 205

Ser Gln Leu Ala His Arg Leu Ala Glu Lys Ser Cys Arg Asn Leu Arg
    210                 215                 220

Lys Ala Leu Leu Met Cys Glu Ala Cys Arg Val Gln Gln Tyr Pro Phe
225                 230                 235                 240

Thr Ala Asp Gln Glu Ile Pro Glu Thr Asp Trp Glu Val Tyr Leu Arg
                245                 250                 255

Glu Thr Ala Asn Ala Ile Val Ser Gln Gln Thr Pro Gln Arg Leu Leu
            260                 265                 270

Glu Val Arg Gly Arg Leu Tyr Glu Leu Leu Thr His Cys Ile Pro Pro
        275                 280                 285

Glu Ile Ile Met Lys Gly Leu Leu Ser Glu Leu Leu His Asn Cys Asp
    290                 295                 300

Gly Gln Leu Lys Gly Glu Val Ala Gln Met Ala Ala Tyr Tyr Glu His
305                 310                 315                 320

Arg Leu Gln Leu Gly Ser Lys Ala Ile Tyr His Leu Glu Ala Phe Val
                325                 330                 335

Ala Lys Phe Met Ala Leu Tyr Lys Lys Phe Met Glu Asp Gly Leu Glu
            340                 345                 350

Gly Met Met Phe
        355

<210>  246
<211>  202
<212>  PRT
<213>  Homo sapiens

<400>  246

Met Ser Leu Trp Val Asp Lys Tyr Arg Pro Cys Ser Leu Gly Arg Leu
1               5                   10                  15

Asp Tyr His Lys Glu Gln Ala Ala Gln Leu Arg Asn Leu Val Gln Cys
            20                  25                  30

Gly Asp Phe Pro His Leu Leu Val Tyr Gly Pro Ser Gly Ala Gly Lys
        35                  40                  45

Lys Thr Arg Ile Met Cys Ile Leu Arg Glu Leu Tyr Gly Val Gly Val
    50                  55                  60

Glu Lys Leu Arg Ile Glu His Gln Thr Ile Thr Thr Pro Ser Lys Lys
65                  70                  75                  80

Lys Ile Glu Ile Ser Thr Ile Ala Ser Asn Tyr His Leu Glu Val Asn
                85                  90                  95

Pro Ser Asp Ala Gly Asn Ser Asp Arg Val Val Ile Gln Glu Met Leu
            100                 105                 110

Lys Thr Val Ala Gln Ser Gln Gln Leu Glu Thr Asn Ser Gln Arg Asp
        115                 120                 125

Phe Lys Val Val Leu Leu Thr Glu Val Asp Lys Leu Thr Lys Asp Ala
    130                 135                 140

Gln His Ala Leu Arg Arg Thr Met Glu Lys Tyr Met Ser Thr Cys Arg
145                 150                 155                 160

Leu Ile Leu Cys Cys Asn Ser Thr Ser Lys Val Ile Pro Pro Ile Arg
                165                 170                 175

Ser Arg Cys Leu Ala Val Arg Val Pro Ala Pro Ser Ile Glu Asp Gly
            180                 185                 190

Arg Ser Glu Ser Ser Phe Thr Thr Gly Ser
        195                 200

<210>  247
<211>  293
<212>  PRT
<213>  Homo sapiens

<400>  247


<210>  248
<211>  1647
<212>  PRT
<213>  Homo sapiens

<400>  248

Met Ser Thr Pro Asp Pro Pro Leu Gly Gly Thr Pro Arg Pro Gly Pro
1               5                   10                  15

Ser Pro Gly Pro Gly Pro Ser Pro Gly Ala Met Leu Gly Pro Ser Pro
            20                  25                  30

Gly Pro Ser Pro Gly Ser Ala His Ser Met Met Gly Pro Ser Pro Gly
        35                  40                  45

Pro Pro Ser Ala Gly His Pro Ile Pro Thr Gln Gly Pro Gly Gly Tyr
    50                  55                  60

Pro Gln Asp Asn Met His Gln Met His Lys Pro Met Glu Ser Met His
65                  70                  75                  80

Glu Lys Gly Met Ser Asp Asp Pro Arg Tyr Asn Gln Met Lys Gly Met
                85                  90                  95

Gly Met Arg Ser Gly Gly His Ala Gly Met Gly Pro Pro Pro Ser Pro
            100                 105                 110

Met Asp Gln His Ser Gln Gly Tyr Pro Ser Pro Leu Gly Gly Ser Glu
        115                 120                 125

His Ala Ser Ser Pro Val Pro Ala Ser Gly Pro Ser Ser Gly Pro Gln
    130                 135                 140

Met Ser Ser Gly Pro Gly Gly Ala Pro Leu Asp Gly Ala Asp Pro Gln
145                 150                 155                 160

Ala Leu Gly Gln Gln Asn Arg Gly Pro Thr Pro Phe Asn Gln Asn Gln
                165                 170                 175

Leu His Gln Leu Arg Ala Gln Ile Met Ala Tyr Lys Met Leu Ala Arg
            180                 185                 190

Gly Gln Pro Leu Pro Asp His Leu Gln Met Ala Val Gln Gly Lys Arg
        195                 200                 205

Pro Met Pro Gly Met Gln Gln Gln Met Pro Thr Leu Pro Pro Pro Ser
    210                 215                 220

Val Ser Ala Thr Gly Pro Gly Pro Gly Pro Gly Pro Gly Pro Gly Pro

225                 230                 235                 240

Gly Pro Gly Pro Ala Pro Pro Asn Tyr Ser Arg Pro His Gly Met Gly
                245                 250                 255

Gly Pro Asn Met Pro Pro Pro Gly Pro Ser Gly Val Pro Pro Gly Met
            260                 265                 270

Pro Gly Gln Pro Pro Gly Gly Pro Pro Lys Pro Trp Pro Glu Gly Pro
        275                 280                 285

Met Ala Asn Ala Ala Ala Pro Thr Ser Thr Pro Gln Lys Leu Ile Pro
    290                 295                 300

Pro Gln Pro Thr Gly Arg Pro Ser Pro Ala Pro Pro Ala Val Pro Pro
305                 310                 315                 320

Ala Ala Ser Pro Val Met Pro Pro Gln Thr Gln Ser Pro Gly Gln Pro
                325                 330                 335

Ala Gln Pro Ala Pro Met Val Pro Leu His Gln Lys Gln Ser Arg Ile
            340                 345                 350

Thr Pro Ile Gln Lys Pro Arg Gly Leu Asp Pro Val Glu Ile Leu Gln
        355                 360                 365

Glu Arg Glu Tyr Arg Leu Gln Ala Arg Ile Ala His Arg Ile Gln Glu
    370                 375                 380

Leu Glu Asn Leu Pro Gly Ser Leu Ala Gly Asp Leu Arg Thr Lys Ala
385                 390                 395                 400

Thr Ile Glu Leu Lys Ala Leu Arg Leu Leu Asn Phe Gln Arg Gln Leu
                405                 410                 415

Arg Gln Glu Val Val Val Cys Met Arg Arg Asp Thr Ala Leu Glu Thr
            420                 425                 430

Ala Leu Asn Ala Lys Ala Tyr Lys Arg Ser Lys Arg Gln Ser Leu Arg
        435                 440                 445

Glu Ala Arg Ile Thr Glu Lys Leu Glu Lys Gln Gln Lys Ile Glu Gln
    450                 455                 460

Glu Arg Lys Arg Arg Gln Lys His Gln Glu Tyr Leu Asn Ser Ile Leu
465                 470                 475                 480

Gln His Ala Lys Asp Phe Lys Glu Tyr His Arg Ser Val Thr Gly Lys
                485                 490                 495

Ile Gln Lys Leu Thr Lys Ala Val Ala Thr Tyr His Ala Asn Thr Glu
            500                 505                 510

Arg Glu Gln Lys Lys Glu Asn Glu Arg Ile Glu Lys Glu Arg Met Arg
        515                 520                 525

Arg Leu Met Ala Glu Asp Glu Glu Gly Tyr Arg Lys Leu Ile Asp Gln
    530                 535                 540

Lys Lys Asp Lys Arg Leu Ala Tyr Leu Leu Gln Gln Thr Asp Glu Tyr
545                 550                 555                 560

Val Ala Asn Leu Thr Glu Leu Val Arg Gln His Lys Ala Ala Gln Val
                565                 570                 575

Ala Lys Glu Lys Lys Lys Lys Lys Lys Lys Lys Lys Ala Glu Asn Ala
            580                 585                 590

Glu Gly Gln Thr Pro Ala Ile Gly Pro Asp Gly Glu Pro Leu Asp Glu
        595                 600                 605

Thr Ser Gln Met Ser Asp Leu Pro Val Lys Val Ile His Val Glu Ser
    610                 615                 620

Gly Lys Ile Leu Thr Gly Thr Asp Ala Pro Lys Ala Gly Gln Leu Glu
625                 630                 635                 640

Ala Trp Leu Glu Met Asn Pro Gly Tyr Glu Val Ala Pro Arg Ser Asp
                645                 650                 655

Ser Glu Glu Ser Gly Ser Glu Glu Glu Glu Glu Glu Glu Glu Glu Glu
            660                 665                 670

Gln Pro Gln Ala Ala Gln Pro Pro Thr Leu Pro Val Glu Glu Lys Lys
        675                 680                 685

Lys Ile Pro Asp Pro Asp Ser Asp Asp Val Ser Glu Val Asp Ala Arg
    690                 695                 700

His Ile Ile Glu Asn Ala Lys Gln Asp Val Asp Asp Glu Tyr Gly Val
705                 710                 715                 720

Ser Gln Ala Leu Ala Arg Gly Leu Gln Ser Tyr Tyr Ala Val Ala His
                725                 730                 735

Ala Val Thr Glu Arg Val Asp Lys Gln Ser Ala Leu Met Val Asn Gly
            740                 745                 750

Val Leu Lys Gln Tyr Gln Ile Lys Gly Leu Glu Trp Leu Val Ser Leu
        755                 760                 765

Tyr Asn Asn Asn Leu Asn Gly Ile Leu Ala Asp Glu Met Gly Leu Gly
    770                 775                 780

Lys Thr Ile Gln Thr Ile Ala Leu Ile Thr Tyr Leu Met Glu His Lys

785                 790                 795                 800

Arg Ile Asn Gly Pro Phe Leu Ile Ile Val Pro Leu Ser Thr Leu Ser
                805                 810                 815

Asn Trp Ala Tyr Glu Phe Asp Lys Trp Ala Pro Ser Val Val Lys Val
            820                 825                 830

Ser Tyr Lys Gly Ser Pro Ala Ala Arg Arg Ala Phe Val Pro Gln Leu
        835                 840                 845

Arg Ser Gly Lys Phe Asn Val Leu Leu Thr Thr Tyr Glu Tyr Ile Ile
    850                 855                 860

Lys Asp Lys His Ile Leu Ala Lys Ile Arg Trp Lys Tyr Met Ile Val
865                 870                 875                 880

Asp Glu Gly His Arg Met Lys Asn His His Cys Lys Leu Thr Gln Val
                885                 890                 895

Leu Asn Thr His Tyr Val Ala Pro Arg Arg Leu Leu Leu Thr Gly Thr
            900                 905                 910

Pro Leu Gln Asn Lys Leu Pro Glu Leu Trp Ala Leu Leu Asn Phe Leu
        915                 920                 925

Leu Pro Thr Ile Phe Lys Ser Cys Ser Thr Phe Glu Gln Trp Phe Asn
    930                 935                 940

Ala Pro Phe Ala Met Thr Gly Glu Lys Val Asp Leu Asn Glu Glu Glu
945                 950                 955                 960

Thr Ile Leu Ile Ile Arg Arg Leu His Lys Val Leu Arg Pro Phe Leu
                965                 970                 975

Leu Arg Arg Leu Lys Lys Glu Val Glu Ala Gln Leu Pro Glu Lys Val
            980                 985                 990

Glu Tyr Val Ile Lys Cys Asp Met  Ser Ala Leu Gln Arg  Val Leu Tyr
        995                 1000                 1005

Arg His  Met Gln Ala Lys Gly  Val Leu Leu Thr Asp  Gly Ser Glu
    1010                 1015                 1020

Lys Asp  Lys Lys Gly Lys Gly  Gly Thr Lys Thr Leu  Met Asn Thr
    1025                 1030                 1035

Ile Met  Gln Leu Arg Lys Ile  Cys Asn His Pro Tyr  Met Phe Gln
    1040                 1045                 1050

His Ile  Glu Glu Ser Phe Ser  Glu His Leu Gly Phe  Thr Gly Gly
    1055                 1060                 1065

Ile Val Gln Gly Leu Asp Leu Tyr Arg Ala Ser Gly Lys Phe Glu
    1070                1075                1080

Leu Leu Asp Arg Ile Leu Pro Lys Leu Arg Ala Thr Asn His Lys
    1085                1090                1095

Val Leu Leu Phe Cys Gln Met Thr Ser Leu Met Thr Ile Met Glu
    1100                1105                1110

Asp Tyr Phe Ala Tyr Arg Gly Phe Lys Tyr Leu Arg Leu Asp Gly
    1115                1120                1125

Thr Thr Lys Ala Glu Asp Arg Gly Met Leu Leu Lys Thr Phe Asn
    1130                1135                1140

Glu Pro Gly Ser Glu Tyr Phe Ile Phe Leu Leu Ser Thr Arg Ala
    1145                1150                1155

Gly Gly Leu Gly Leu Asn Leu Gln Ser Ala Asp Thr Val Ile Ile
    1160                1165                1170

Phe Asp Ser Asp Trp Asn Pro His Gln Asp Leu Gln Ala Gln Asp
    1175                1180                1185

Arg Ala His Arg Ile Gly Gln Gln Asn Glu Val Arg Val Leu Arg
    1190                1195                1200

Leu Cys Thr Val Asn Ser Val Glu Glu Lys Ile Leu Ala Ala Ala
    1205                1210                1215

Lys Tyr Lys Leu Asn Val Asp Gln Lys Val Ile Gln Ala Gly Met
    1220                1225                1230

Phe Asp Gln Lys Ser Ser Ser His Glu Arg Arg Ala Phe Leu Gln
    1235                1240                1245

Ala Ile Leu Glu His Glu Glu Gln Asp Glu Ser Arg His Cys Ser
    1250                1255                1260

Thr Gly Ser Gly Ser Ala Ser Phe Ala His Thr Ala Pro Pro Pro
    1265                1270                1275

Ala Gly Val Asn Pro Asp Leu Glu Glu Pro Pro Leu Lys Glu Glu
    1280                1285                1290

Asp Glu Val Pro Asp Asp Glu Thr Val Asn Gln Met Ile Ala Arg
    1295                1300                1305

His Glu Glu Glu Phe Asp Leu Phe Met Arg Met Asp Leu Asp Arg
    1310                1315                1320

Arg Arg Glu Glu Ala Arg Asn Pro Lys Arg Lys Pro Arg Leu Met

1325                    1330                    1335

Glu Glu Asp Glu Leu Pro Ser Trp Ile Ile Lys Asp Asp Ala Glu
    1340                    1345                    1350

Val Glu Arg Leu Thr Cys Glu Glu Glu Glu Glu Lys Met Phe Gly
    1355                    1360                    1365

Arg Gly Ser Arg His Arg Lys Glu Val Asp Tyr Ser Asp Ser Leu
    1370                    1375                    1380

Thr Glu Lys Gln Trp Leu Lys Ala Ile Glu Glu Gly Thr Leu Glu
    1385                    1390                    1395

Glu Ile Glu Glu Glu Val Arg Gln Lys Lys Ser Ser Arg Lys Arg
    1400                    1405                    1410

Lys Arg Asp Ser Asp Ala Gly Ser Ser Thr Pro Thr Thr Ser Thr
    1415                    1420                    1425

Arg Ser Arg Asp Lys Asp Asp Glu Ser Lys Lys Gln Lys Lys Arg
    1430                    1435                    1440

Gly Arg Pro Pro Ala Glu Lys Leu Ser Pro Asn Pro Pro Asn Leu
    1445                    1450                    1455

Thr Lys Lys Met Lys Lys Ile Val Asp Ala Val Ile Lys Tyr Lys
    1460                    1465                    1470

Asp Ser Ser Ser Gly Arg Gln Leu Ser Glu Val Phe Ile Gln Leu
    1475                    1480                    1485

Pro Ser Arg Lys Glu Leu Pro Glu Tyr Tyr Glu Leu Ile Arg Lys
    1490                    1495                    1500

Pro Val Asp Phe Lys Lys Ile Lys Glu Arg Ile Arg Asn His Lys
    1505                    1510                    1515

Tyr Arg Ser Leu Asn Asp Leu Glu Lys Asp Val Met Leu Leu Cys
    1520                    1525                    1530

Gln Asn Ala Gln Thr Phe Asn Leu Glu Gly Ser Leu Ile Tyr Glu
    1535                    1540                    1545

Asp Ser Ile Val Leu Gln Ser Val Phe Thr Ser Val Arg Gln Lys
    1550                    1555                    1560

Ile Glu Lys Glu Asp Asp Ser Glu Gly Glu Glu Ser Glu Glu Glu
    1565                    1570                    1575

Glu Glu Gly Glu Glu Glu Gly Ser Glu Ser Glu Ser Arg Ser Val
    1580                    1585                    1590

Lys Val Lys Ile Lys Leu Gly Arg Lys Glu Lys Ala Gln Asp Arg
    1595                1600                1605

Leu Lys Gly Gly Arg Arg Arg Pro Ser Arg Gly Ser Arg Ala Lys
    1610                1615                1620

Pro Val Val Ser Asp Asp Asp Ser Glu Glu Glu Gln Glu Glu Asp
    1625                1630                1635

Arg Ser Gly Ser Gly Ser Glu Glu Asp
    1640                1645

<210> 249
<211> 231
<212> PRT
<213> Homo sapiens

<400> 249

Met Thr Val Gly Lys Ser Ser Lys Met Leu Gln His Ile Asp Tyr Arg
1               5                   10                  15

Met Arg Cys Ile Leu Gln Asp Gly Arg Ile Phe Ile Gly Thr Phe Lys
            20                  25                  30

Ala Phe Asp Lys His Met Asn Leu Ile Leu Cys Asp Cys Asp Glu Phe
        35                  40                  45

Arg Lys Ile Lys Pro Lys Asn Ser Lys Gln Ala Glu Arg Glu Glu Lys
    50                  55                  60

Arg Val Leu Gly Leu Val Leu Leu Arg Gly Glu Asn Leu Val Ser Met
65                  70                  75                  80

Thr Val Glu Gly Pro Pro Pro Lys Asp Thr Gly Ile Ala Arg Val Pro
                85                  90                  95

Leu Ala Gly Ala Ala Gly Gly Pro Gly Ile Gly Arg Ala Ala Gly Arg
            100                 105                 110

Gly Ile Pro Ala Gly Val Pro Met Pro Gln Ala Pro Ala Gly Leu Ala
        115                 120                 125

Gly Pro Val Arg Gly Val Gly Gly Pro Ser Gln Gln Val Met Thr Pro
    130                 135                 140

Gln Gly Arg Gly Thr Val Ala Ala Ala Ala Ala Ala Ala Thr Ala Ser
145                 150                 155                 160

Ile Ala Gly Ala Pro Thr Gln Tyr Pro Pro Gly Arg Gly Gly Pro Pro
                165                 170                 175

Pro Pro Met Gly Arg Gly Ala Pro Pro Pro Gly Met Met Gly Pro Pro
            180                 185                 190

Pro Gly Met Arg Pro Pro Met Gly Pro Pro Met Gly Ile Pro Pro Gly
      195                 200                 205

Arg Gly Thr Pro Met Gly Met Pro Pro Pro Gly Met Arg Pro Pro Pro
    210                 215                 220

Pro Gly Met Arg Gly Leu Leu
225                 230

<210>  250
<211>  240
<212>  PRT
<213>  Homo sapiens

<400>  250

<210>  251
<211>  403
<212>  PRT
<213>  Homo sapiens

<400>  251

Met Asp Arg Ser Lys Glu Asn Cys Ile Ser Gly Pro Val Lys Ala Thr
1               5                   10                  15

Ala Pro Val Gly Gly Pro Lys Arg Val Leu Val Thr Gln Gln Phe Pro
            20                  25                  30

Cys Gln Asn Pro Leu Pro Val Asn Ser Gly Gln Ala Gln Arg Val Leu
        35                  40                  45

Cys Pro Ser Asn Ser Ser Gln Arg Ile Pro Leu Gln Ala Gln Lys Leu
    50                  55                  60

Val Ser Ser His Lys Pro Val Gln Asn Gln Lys Gln Lys Gln Leu Gln
65                  70                  75                  80

Ala Thr Ser Val Pro His Pro Val Ser Arg Pro Leu Asn Asn Thr Gln
                85                  90                  95

Lys Ser Lys Gln Pro Leu Pro Ser Ala Pro Glu Asn Asn Pro Glu Glu
            100                 105                 110

Glu Leu Ala Ser Lys Gln Lys Asn Glu Glu Ser Lys Lys Arg Gln Trp
        115                 120                 125

Ala Leu Glu Asp Phe Glu Ile Gly Arg Pro Leu Gly Lys Gly Lys Phe
    130                 135                 140

Gly Asn Val Tyr Leu Ala Arg Glu Lys Gln Ser Lys Phe Ile Leu Ala
145                 150                 155                 160

Leu Lys Val Leu Phe Lys Ala Gln Leu Glu Lys Ala Gly Val Glu His
                165                 170                 175

Gln Leu Arg Arg Glu Val Glu Ile Gln Ser His Leu Arg His Pro Asn
            180                 185                 190

Ile Leu Arg Leu Tyr Gly Tyr Phe His Asp Ala Thr Arg Val Tyr Leu
        195                 200                 205

Ile Leu Glu Tyr Ala Pro Leu Gly Thr Val Tyr Arg Glu Leu Gln Lys
    210                 215                 220

Leu Ser Lys Phe Asp Glu Gln Arg Thr Ala Thr Tyr Ile Thr Glu Leu
225                 230                 235                 240

Ala Asn Ala Leu Ser Tyr Cys His Ser Lys Arg Val Ile His Arg Asp
                245                 250                 255

Ile Lys Pro Glu Asn Leu Leu Leu Gly Ser Ala Gly Glu Leu Lys Ile
            260                 265                 270

Ala Asp Phe Gly Trp Ser Val His Ala Pro Ser Ser Arg Arg Thr Thr
        275                 280                 285

Leu Cys Gly Thr Leu Asp Tyr Leu Pro Pro Glu Met Ile Glu Gly Arg
    290                 295                 300

Met His Asp Glu Lys Val Asp Leu Trp Ser Leu Gly Val Leu Cys Tyr
305                 310                 315                 320

Glu Phe Leu Val Gly Lys Pro Pro Phe Glu Ala Asn Thr Tyr Gln Glu
                325                 330                 335

Thr Tyr Lys Arg Ile Ser Arg Val Glu Phe Thr Phe Pro Asp Phe Val
            340                 345                 350

Thr Glu Gly Ala Arg Asp Leu Ile Ser Arg Leu Leu Lys His Asn Pro
        355                 360                 365

Ser Gln Arg Pro Met Leu Arg Glu Val Leu Glu His Pro Trp Ile Thr
    370                 375                 380

Ala Asn Ser Ser Lys Pro Ser Asn Cys Gln Asn Lys Glu Ser Ala Ser
385                 390                 395                 400

Lys Gln Ser

<210>  252
<211>  403
<212>  PRT
<213>  Homo sapiens

<400>  252

Met Asp Arg Ser Lys Glu Asn Cys Ile Ser Gly Pro Val Lys Ala Thr
1               5                   10                  15

Ala Pro Val Gly Gly Pro Lys Arg Val Leu Val Thr Gln Gln Phe Pro
            20                  25                  30

Cys Gln Asn Pro Leu Pro Val Asn Ser Gly Gln Ala Gln Arg Val Leu
        35                  40                  45

Cys Pro Ser Asn Ser Ser Gln Arg Ile Pro Leu Gln Ala Gln Lys Leu
    50                  55                  60

Val Ser Ser His Lys Pro Val Gln Asn Gln Lys Gln Lys Gln Leu Gln
65                  70                  75                  80

Ala Thr Ser Val Pro His Pro Val Ser Arg Pro Leu Asn Asn Thr Gln
                85                  90                  95

Lys Ser Lys Gln Pro Leu Pro Ser Ala Pro Glu Asn Asn Pro Glu Glu
            100                 105                 110

Glu Leu Ala Ser Lys Gln Lys Asn Glu Glu Ser Lys Lys Arg Gln Trp
        115                 120                 125

Ala Leu Glu Asp Phe Glu Ile Gly Arg Pro Leu Gly Lys Gly Lys Phe
    130                 135                 140

Gly Asn Val Tyr Leu Ala Arg Glu Lys Gln Ser Lys Phe Ile Leu Ala
145                 150                 155                 160

Leu Lys Val Leu Phe Lys Ala Gln Leu Glu Lys Ala Gly Val Glu His
                165                 170                 175

Gln Leu Arg Arg Glu Val Glu Ile Gln Ser His Leu Arg His Pro Asn
            180                 185                 190

Ile Leu Arg Leu Tyr Gly Tyr Phe His Asp Ala Thr Arg Val Tyr Leu
        195                 200                 205

Ile Leu Glu Tyr Ala Pro Leu Gly Thr Val Tyr Arg Glu Leu Gln Lys
    210                 215                 220

Leu Ser Lys Phe Asp Glu Gln Arg Thr Ala Thr Tyr Ile Thr Glu Leu
225                 230                 235                 240

Ala Asn Ala Leu Ser Tyr Cys His Ser Lys Arg Val Ile His Arg Asp
                245                 250                 255

Ile Lys Pro Glu Asn Leu Leu Leu Gly Ser Ala Gly Glu Leu Lys Ile
            260                 265                 270

Ala Asp Phe Gly Trp Ser Val His Ala Pro Ser Ser Arg Arg Thr Thr
        275                 280                 285

Leu Cys Gly Thr Leu Asp Tyr Leu Pro Pro Glu Met Ile Glu Gly Arg
    290                 295                 300

Met His Asp Glu Lys Val Asp Leu Trp Ser Leu Gly Val Leu Cys Tyr
305                 310                 315                 320

Glu Phe Leu Val Gly Lys Pro Pro Phe Glu Ala Asn Thr Tyr Gln Glu

325                 330                 335

Thr Tyr Lys Arg Ile Ser Arg Val Glu Phe Thr Phe Pro Asp Phe Val
            340                 345                 350

Thr Glu Gly Ala Arg Asp Leu Ile Ser Arg Leu Leu Lys His Asn Pro
        355                 360                 365

Ser Gln Arg Pro Met Leu Arg Glu Val Leu Glu His Pro Trp Ile Thr
    370                 375                 380

Ala Asn Ser Ser Lys Pro Ser Asn Cys Gln Asn Lys Glu Ser Ala Ser
385                 390                 395                 400

Lys Gln Ser

<210>  253
<211>  403
<212>  PRT
<213>  Homo sapiens

<400>  253

Met Asp Arg Ser Lys Glu Asn Cys Ile Ser Gly Pro Val Lys Ala Thr
1               5                   10                  15

Ala Pro Val Gly Gly Pro Lys Arg Val Leu Val Thr Gln Gln Phe Pro
            20                  25                  30

Cys Gln Asn Pro Leu Pro Val Asn Ser Gly Gln Ala Gln Arg Val Leu
        35                  40                  45

Cys Pro Ser Asn Ser Ser Gln Arg Ile Pro Leu Gln Ala Gln Lys Leu
    50                  55                  60

Val Ser Ser His Lys Pro Val Gln Asn Gln Lys Gln Lys Gln Leu Gln
65                  70                  75                  80

Ala Thr Ser Val Pro His Pro Val Ser Arg Pro Leu Asn Asn Thr Gln
                85                  90                  95

Lys Ser Lys Gln Pro Leu Pro Ser Ala Pro Glu Asn Asn Pro Glu Glu
            100                 105                 110

Glu Leu Ala Ser Lys Gln Lys Asn Glu Glu Ser Lys Lys Arg Gln Trp
        115                 120                 125

Ala Leu Glu Asp Phe Glu Ile Gly Arg Pro Leu Gly Lys Gly Lys Phe
    130                 135                 140

Gly Asn Val Tyr Leu Ala Arg Glu Lys Gln Ser Lys Phe Ile Leu Ala
145                 150                 155                 160

Leu Lys Val Leu Phe Lys Ala Gln Leu Glu Lys Ala Gly Val Glu His
                165                 170                 175

Gln Leu Arg Arg Glu Val Glu Ile Gln Ser His Leu Arg His Pro Asn
            180                 185                 190

Ile Leu Arg Leu Tyr Gly Tyr Phe His Asp Ala Thr Arg Val Tyr Leu
        195                 200                 205

Ile Leu Glu Tyr Ala Pro Leu Gly Thr Val Tyr Arg Glu Leu Gln Lys
    210                 215                 220

Leu Ser Lys Phe Asp Glu Gln Arg Thr Ala Thr Tyr Ile Thr Glu Leu
225                 230                 235                 240

Ala Asn Ala Leu Ser Tyr Cys His Ser Lys Arg Val Ile His Arg Asp
                245                 250                 255

Ile Lys Pro Glu Asn Leu Leu Leu Gly Ser Ala Gly Glu Leu Lys Ile
            260                 265                 270

Ala Asp Phe Gly Trp Ser Val His Ala Pro Ser Ser Arg Arg Thr Thr
        275                 280                 285

Leu Cys Gly Thr Leu Asp Tyr Leu Pro Pro Glu Met Ile Glu Gly Arg
    290                 295                 300

Met His Asp Glu Lys Val Asp Leu Trp Ser Leu Gly Val Leu Cys Tyr
305                 310                 315                 320

Glu Phe Leu Val Gly Lys Pro Pro Phe Glu Ala Asn Thr Tyr Gln Glu
                325                 330                 335

Thr Tyr Lys Arg Ile Ser Arg Val Glu Phe Thr Phe Pro Asp Phe Val
            340                 345                 350

Thr Glu Gly Ala Arg Asp Leu Ile Ser Arg Leu Leu Lys His Asn Pro
        355                 360                 365

Ser Gln Arg Pro Met Leu Arg Glu Val Leu Glu His Pro Trp Ile Thr
    370                 375                 380

Ala Asn Ser Ser Lys Pro Ser Asn Cys Gln Asn Lys Glu Ser Ala Ser
385                 390                 395                 400

Lys Gln Ser

<210>  254
<211>  403
<212>  PRT
<213>  Homo sapiens

<400>  254

Met Asp Arg Ser Lys Glu Asn Cys Ile Ser Gly Pro Val Lys Ala Thr
1               5                   10                  15

Ala Pro Val Gly Gly Pro Lys Arg Val Leu Val Thr Gln Gln Phe Pro
            20                  25                  30

Cys Gln Asn Pro Leu Pro Val Asn Ser Gly Gln Ala Gln Arg Val Leu
        35                  40                  45

Cys Pro Ser Asn Ser Ser Gln Arg Ile Pro Leu Gln Ala Gln Lys Leu
    50                  55                  60

Val Ser Ser His Lys Pro Val Gln Asn Gln Lys Gln Lys Gln Leu Gln
65                  70                  75                  80

Ala Thr Ser Val Pro His Pro Val Ser Arg Pro Leu Asn Asn Thr Gln
                85                  90                  95

Lys Ser Lys Gln Pro Leu Pro Ser Ala Pro Glu Asn Asn Pro Glu Glu
            100                 105                 110

Glu Leu Ala Ser Lys Gln Lys Asn Glu Glu Ser Lys Lys Arg Gln Trp
        115                 120                 125

Ala Leu Glu Asp Phe Glu Ile Gly Arg Pro Leu Gly Lys Gly Lys Phe
    130                 135                 140

Gly Asn Val Tyr Leu Ala Arg Glu Lys Gln Ser Lys Phe Ile Leu Ala
145                 150                 155                 160

Leu Lys Val Leu Phe Lys Ala Gln Leu Glu Lys Ala Gly Val Glu His
                165                 170                 175

Gln Leu Arg Arg Glu Val Glu Ile Gln Ser His Leu Arg His Pro Asn
            180                 185                 190

Ile Leu Arg Leu Tyr Gly Tyr Phe His Asp Ala Thr Arg Val Tyr Leu
        195                 200                 205

Ile Leu Glu Tyr Ala Pro Leu Gly Thr Val Tyr Arg Glu Leu Gln Lys
    210                 215                 220

Leu Ser Lys Phe Asp Glu Gln Arg Thr Ala Thr Tyr Ile Thr Glu Leu
225                 230                 235                 240

Ala Asn Ala Leu Ser Tyr Cys His Ser Lys Arg Val Ile His Arg Asp
                245                 250                 255

Ile Lys Pro Glu Asn Leu Leu Leu Gly Ser Ala Gly Glu Leu Lys Ile
            260                 265                 270

Ala Asp Phe Gly Trp Ser Val His Ala Pro Ser Ser Arg Arg Thr Thr

275                 280                 285

Leu Cys Gly Thr Leu Asp Tyr Leu Pro Pro Glu Met Ile Glu Gly Arg
    290                 295                 300

Met His Asp Glu Lys Val Asp Leu Trp Ser Leu Gly Val Leu Cys Tyr
305                 310                 315                 320

Glu Phe Leu Val Gly Lys Pro Pro Phe Glu Ala Asn Thr Tyr Gln Glu
                325                 330                 335

Thr Tyr Lys Arg Ile Ser Arg Val Glu Phe Thr Phe Pro Asp Phe Val
            340                 345                 350

Thr Glu Gly Ala Arg Asp Leu Ile Ser Arg Leu Leu Lys His Asn Pro
        355                 360                 365

Ser Gln Arg Pro Met Leu Arg Glu Val Leu Glu His Pro Trp Ile Thr
    370                 375                 380

Ala Asn Ser Ser Lys Pro Ser Asn Cys Gln Asn Lys Glu Ser Ala Ser
385                 390                 395                 400

Lys Gln Ser

<210>  255
<211>  403
<212>  PRT
<213>  Homo sapiens

<400>  255

Met Asp Arg Ser Lys Glu Asn Cys Ile Ser Gly Pro Val Lys Ala Thr
1               5                   10                  15

Ala Pro Val Gly Gly Pro Lys Arg Val Leu Val Thr Gln Gln Phe Pro
            20                  25                  30

Cys Gln Asn Pro Leu Pro Val Asn Ser Gly Gln Ala Gln Arg Val Leu
        35                  40                  45

Cys Pro Ser Asn Ser Ser Gln Arg Ile Pro Leu Gln Ala Gln Lys Leu
    50                  55                  60

Val Ser Ser His Lys Pro Val Gln Asn Gln Lys Gln Lys Gln Leu Gln
65                  70                  75                  80

Ala Thr Ser Val Pro His Pro Val Ser Arg Pro Leu Asn Asn Thr Gln
                85                  90                  95

Lys Ser Lys Gln Pro Leu Pro Ser Ala Pro Glu Asn Asn Pro Glu Glu
            100                 105                 110

Glu Leu Ala Ser Lys Gln Lys Asn Glu Glu Ser Lys Lys Arg Gln Trp
        115                 120                 125

Ala Leu Glu Asp Phe Glu Ile Gly Arg Pro Leu Gly Lys Gly Lys Phe
    130                 135                 140

Gly Asn Val Tyr Leu Ala Arg Glu Lys Gln Ser Lys Phe Ile Leu Ala
145                 150                 155                 160

Leu Lys Val Leu Phe Lys Ala Gln Leu Glu Lys Ala Gly Val Glu His
                165                 170                 175

Gln Leu Arg Arg Glu Val Glu Ile Gln Ser His Leu Arg His Pro Asn
            180                 185                 190

Ile Leu Arg Leu Tyr Gly Tyr Phe His Asp Ala Thr Arg Val Tyr Leu
        195                 200                 205

Ile Leu Glu Tyr Ala Pro Leu Gly Thr Val Tyr Arg Glu Leu Gln Lys
    210                 215                 220

Leu Ser Lys Phe Asp Glu Gln Arg Thr Ala Thr Tyr Ile Thr Glu Leu
225                 230                 235                 240

Ala Asn Ala Leu Ser Tyr Cys His Ser Lys Arg Val Ile His Arg Asp
                245                 250                 255

Ile Lys Pro Glu Asn Leu Leu Leu Gly Ser Ala Gly Glu Leu Lys Ile
            260                 265                 270

Ala Asp Phe Gly Trp Ser Val His Ala Pro Ser Ser Arg Arg Thr Thr
        275                 280                 285

Leu Cys Gly Thr Leu Asp Tyr Leu Pro Pro Glu Met Ile Glu Gly Arg
    290                 295                 300

Met His Asp Glu Lys Val Asp Leu Trp Ser Leu Gly Val Leu Cys Tyr
305                 310                 315                 320

Glu Phe Leu Val Gly Lys Pro Pro Phe Glu Ala Asn Thr Tyr Gln Glu
                325                 330                 335

Thr Tyr Lys Arg Ile Ser Arg Val Glu Phe Thr Phe Pro Asp Phe Val
            340                 345                 350

Thr Glu Gly Ala Arg Asp Leu Ile Ser Arg Leu Leu Lys His Asn Pro
        355                 360                 365

Ser Gln Arg Pro Met Leu Arg Glu Val Leu Glu His Pro Trp Ile Thr
    370                 375                 380

Ala Asn Ser Ser Lys Pro Ser Asn Cys Gln Asn Lys Glu Ser Ala Ser
385                 390                 395                 400

Lys Gln Ser

<210> 256
<211> 403
<212> PRT
<213> Homo sapiens

<400> 256

Met Asp Arg Ser Lys Glu Asn Cys Ile Ser Gly Pro Val Lys Ala Thr
1               5                   10                  15

Ala Pro Val Gly Gly Pro Lys Arg Val Leu Val Thr Gln Gln Phe Pro
            20                  25                  30

Cys Gln Asn Pro Leu Pro Val Asn Ser Gly Gln Ala Gln Arg Val Leu
        35                  40                  45

Cys Pro Ser Asn Ser Ser Gln Arg Ile Pro Leu Gln Ala Gln Lys Leu
    50                  55                  60

Val Ser Ser His Lys Pro Val Gln Asn Gln Lys Gln Lys Gln Leu Gln
65                  70                  75                  80

Ala Thr Ser Val Pro His Pro Val Ser Arg Pro Leu Asn Asn Thr Gln
                85                  90                  95

Lys Ser Lys Gln Pro Leu Pro Ser Ala Pro Glu Asn Asn Pro Glu Glu
            100                 105                 110

Glu Leu Ala Ser Lys Gln Lys Asn Glu Glu Ser Lys Lys Arg Gln Trp
        115                 120                 125

Ala Leu Glu Asp Phe Glu Ile Gly Arg Pro Leu Gly Lys Gly Lys Phe
    130                 135                 140

Gly Asn Val Tyr Leu Ala Arg Glu Lys Gln Ser Lys Phe Ile Leu Ala
145                 150                 155                 160

Leu Lys Val Leu Phe Lys Ala Gln Leu Glu Lys Ala Gly Val Glu His
                165                 170                 175

Gln Leu Arg Arg Glu Val Glu Ile Gln Ser His Leu Arg His Pro Asn
            180                 185                 190

Ile Leu Arg Leu Tyr Gly Tyr Phe His Asp Ala Thr Arg Val Tyr Leu
        195                 200                 205

Ile Leu Glu Tyr Ala Pro Leu Gly Thr Val Tyr Arg Glu Leu Gln Lys
    210                 215                 220

Leu Ser Lys Phe Asp Glu Gln Arg Thr Ala Thr Tyr Ile Thr Glu Leu

225                 230                 235                 240

Ala Asn Ala Leu Ser Tyr Cys His Ser Lys Arg Val Ile His Arg Asp
                245                 250                 255

Ile Lys Pro Glu Asn Leu Leu Leu Gly Ser Ala Gly Glu Leu Lys Ile
            260                 265                 270

Ala Asp Phe Gly Trp Ser Val His Ala Pro Ser Ser Arg Arg Thr Thr
        275                 280                 285

Leu Cys Gly Thr Leu Asp Tyr Leu Pro Pro Glu Met Ile Glu Gly Arg
    290                 295                 300

Met His Asp Glu Lys Val Asp Leu Trp Ser Leu Gly Val Leu Cys Tyr
305                 310                 315                 320

Glu Phe Leu Val Gly Lys Pro Pro Phe Glu Ala Asn Thr Tyr Gln Glu
                325                 330                 335

Thr Tyr Lys Arg Ile Ser Arg Val Glu Phe Thr Phe Pro Asp Phe Val
            340                 345                 350

Thr Glu Gly Ala Arg Asp Leu Ile Ser Arg Leu Leu Lys His Asn Pro
        355                 360                 365

Ser Gln Arg Pro Met Leu Arg Glu Val Leu Glu His Pro Trp Ile Thr
    370                 375                 380

Ala Asn Ser Ser Lys Pro Ser Asn Cys Gln Asn Lys Glu Ser Ala Ser
385                 390                 395                 400

Lys Gln Ser

<210>  257
<211>  454
<212>  PRT
<213>  Homo sapiens

<400>  257

Met Pro Glu Phe Leu Glu Asp Pro Ser Val Leu Thr Lys Asp Lys Leu
1               5                   10                  15

Lys Ser Glu Leu Val Ala Asn Asn Val Thr Leu Pro Ala Gly Glu Gln
            20                  25                  30

Arg Lys Asp Val Tyr Val Gln Leu Tyr Leu Gln His Leu Thr Ala Arg
        35                  40                  45

Asn Arg Pro Pro Leu Pro Ala Gly Thr Asn Ser Lys Gly Pro Pro Asp
    50                  55                  60

Phe Ser Ser Asp Glu Glu Arg Glu Pro Thr Pro Val Leu Gly Ser Gly
65                  70                  75                  80

Ala Ala Ala Ala Gly Arg Ser Arg Ala Ala Val Gly Arg Lys Ala Thr
                85                  90                  95

Lys Lys Thr Asp Lys Pro Arg Gln Glu Asp Lys Asp Asp Leu Asp Val
            100                 105                 110

Thr Glu Leu Thr Asn Glu Asp Leu Leu Asp Gln Leu Val Lys Tyr Gly
        115                 120                 125

Val Asn Pro Gly Pro Ile Val Gly Thr Thr Arg Lys Leu Tyr Glu Lys
    130                 135                 140

Lys Leu Leu Lys Leu Arg Glu Gln Gly Thr Glu Ser Arg Ser Ser Thr
145                 150                 155                 160

Pro Leu Pro Thr Ile Ser Ser Ser Ala Glu Asn Thr Arg Gln Asn Gly
                165                 170                 175

Ser Asn Asp Ser Asp Arg Tyr Ser Asp Asn Glu Glu Asp Ser Lys Ile
            180                 185                 190

Glu Leu Lys Leu Glu Lys Arg Glu Pro Leu Lys Gly Arg Ala Lys Thr
        195                 200                 205

Pro Val Thr Leu Lys Gln Arg Arg Val Glu His Asn Gln Ser Tyr Ser
    210                 215                 220

Gln Ala Gly Ile Thr Glu Thr Glu Trp Thr Ser Gly Ser Ser Lys Gly
225                 230                 235                 240

Gly Pro Leu Gln Ala Leu Thr Arg Glu Ser Thr Arg Gly Ser Arg Arg
                245                 250                 255

Thr Pro Arg Lys Arg Val Glu Thr Ser Glu His Phe Arg Ile Asp Gly
            260                 265                 270

Pro Val Ile Ser Glu Ser Thr Pro Ile Ala Glu Thr Ile Met Ala Ser
        275                 280                 285

Ser Asn Glu Ser Leu Val Val Asn Arg Val Thr Gly Asn Phe Lys His
    290                 295                 300

Ala Ser Pro Ile Leu Pro Ile Thr Glu Phe Ser Asp Ile Pro Arg Arg
305                 310                 315                 320

Ala Pro Lys Lys Pro Leu Thr Arg Ala Glu Val Gly Glu Lys Thr Glu
                325                 330                 335

Glu Arg Arg Val Glu Arg Asp Ile Leu Lys Glu Met Phe Pro Tyr Glu
            340                 345                 350

Ala Ser Thr Pro Thr Gly Ile Ser Ala Ser Cys Arg Arg Pro Ile Lys
        355                 360                 365

Gly Ala Ala Gly Arg Pro Leu Glu Leu Ser Asp Phe Arg Met Glu Glu
    370                 375                 380

Ser Phe Ser Ser Lys Tyr Val Pro Lys Tyr Val Pro Leu Ala Asp Val
385                 390                 395                 400

Lys Ser Glu Lys Thr Lys Lys Gly Arg Ser Ile Pro Val Trp Ile Lys
                405                 410                 415

Ile Leu Leu Phe Val Val Val Ala Val Phe Leu Phe Leu Val Tyr Gln
            420                 425                 430

Ala Met Glu Thr Asn Gln Val Asn Pro Phe Ser Asn Phe Leu His Val
        435                 440                 445

Asp Pro Arg Lys Ser Asn
    450

<210>  258
<211>  345
<212>  PRT
<213>  Homo sapiens

<400>  258

Met Pro Glu Phe Leu Glu Asp Pro Ser Val Leu Thr Lys Asp Lys Leu
1               5                   10                  15

Lys Ser Glu Leu Val Ala Asn Asn Val Thr Leu Pro Ala Gly Glu Gln
            20                  25                  30

Arg Lys Asp Val Tyr Val Gln Leu Tyr Leu Gln His Leu Thr Ala Arg
        35                  40                  45

Asn Arg Pro Pro Leu Pro Ala Gly Thr Asn Ser Lys Gly Pro Pro Asp
    50                  55                  60

Phe Ser Ser Asp Glu Glu Arg Glu Pro Thr Pro Val Leu Gly Ser Gly
65                  70                  75                  80

Ala Ala Ala Ala Gly Arg Ser Arg Ala Ala Val Gly Arg Lys Ala Thr
                85                  90                  95

Lys Lys Thr Asp Lys Pro Arg Gln Glu Asp Lys Asp Asp Leu Asp Val
            100                 105                 110

Thr Glu Leu Thr Asn Glu Asp Leu Leu Asp Gln Leu Val Lys Tyr Gly
        115                 120                 125

Val Asn Pro Gly Pro Ile Val Gly Thr Thr Arg Lys Leu Tyr Glu Lys

130                 135                 140

Lys Leu Leu Lys Leu Arg Glu Gln Gly Thr Glu Ser Arg Ser Ser Thr
145                 150                 155                 160

Pro Leu Pro Thr Ile Ser Ser Ser Ala Glu Asn Thr Arg Gln Asn Gly
                165                 170                 175

Ser Asn Asp Ser Asp Arg Tyr Ser Asp Asn Glu Glu Asp Ser Lys Ile
            180                 185                 190

Glu Leu Lys Leu Glu Lys Arg Glu Pro Leu Lys Gly Arg Ala Lys Thr
        195                 200                 205

Pro Val Thr Leu Lys Gln Arg Arg Val Glu His Asn Gln Val Gly Glu
    210                 215                 220

Lys Thr Glu Glu Arg Arg Val Glu Arg Asp Ile Leu Lys Glu Met Phe
225                 230                 235                 240

Pro Tyr Glu Ala Ser Thr Pro Thr Gly Ile Ser Ala Ser Cys Arg Arg
                245                 250                 255

Pro Ile Lys Gly Ala Ala Gly Arg Pro Leu Glu Leu Ser Asp Phe Arg
            260                 265                 270

Met Glu Glu Ser Phe Ser Ser Lys Tyr Val Pro Lys Tyr Val Pro Leu
        275                 280                 285

Ala Asp Val Lys Ser Glu Lys Thr Lys Lys Gly Arg Ser Ile Pro Val
    290                 295                 300

Trp Ile Lys Ile Leu Leu Phe Val Val Val Ala Val Phe Leu Phe Leu
305                 310                 315                 320

Val Tyr Gln Ala Met Glu Thr Asn Gln Val Asn Pro Phe Ser Asn Phe
                325                 330                 335

Leu His Val Asp Pro Arg Lys Ser Asn
            340                 345

<210>  259
<211>  694
<212>  PRT
<213>  Homo sapiens

<400>  259

Met Pro Glu Phe Leu Glu Asp Pro Ser Val Leu Thr Lys Asp Lys Leu
1               5                   10                  15

Lys Ser Glu Leu Val Ala Asn Asn Val Thr Leu Pro Ala Gly Glu Gln
            20                  25                  30

Arg Lys Asp Val Tyr Val Gln Leu Tyr Leu Gln His Leu Thr Ala Arg
        35                  40                  45

Asn Arg Pro Pro Leu Pro Ala Gly Thr Asn Ser Lys Gly Pro Pro Asp
    50                  55                  60

Phe Ser Ser Asp Glu Glu Arg Glu Pro Thr Pro Val Leu Gly Ser Gly
65                  70                  75                  80

Ala Ala Ala Ala Gly Arg Ser Arg Ala Ala Val Gly Arg Lys Ala Thr
                85                  90                  95

Lys Lys Thr Asp Lys Pro Arg Gln Glu Asp Lys Asp Asp Leu Asp Val
            100                 105                 110

Thr Glu Leu Thr Asn Glu Asp Leu Leu Asp Gln Leu Val Lys Tyr Gly
        115                 120                 125

Val Asn Pro Gly Pro Ile Val Gly Thr Thr Arg Lys Leu Tyr Glu Lys
    130                 135                 140

Lys Leu Leu Lys Leu Arg Glu Gln Gly Thr Glu Ser Arg Ser Ser Thr
145                 150                 155                 160

Pro Leu Pro Thr Ile Ser Ser Ser Ala Glu Asn Thr Arg Gln Asn Gly
                165                 170                 175

Ser Asn Asp Ser Asp Arg Tyr Ser Asp Asn Glu Glu Gly Lys Lys Lys
            180                 185                 190

Glu His Lys Lys Val Lys Ser Thr Arg Asp Ile Val Pro Phe Ser Glu
        195                 200                 205

Leu Gly Thr Thr Pro Ser Gly Gly Gly Phe Phe Gln Gly Ile Ser Phe
    210                 215                 220

Pro Glu Ile Ser Thr Arg Pro Pro Leu Gly Ser Thr Glu Leu Gln Ala
225                 230                 235                 240

Ala Lys Lys Val His Thr Ser Lys Gly Asp Leu Pro Arg Glu Pro Leu
                245                 250                 255

Val Ala Thr Asn Leu Pro Gly Arg Gly Gln Leu Gln Lys Leu Ala Ser
            260                 265                 270

Glu Arg Asn Leu Phe Ile Ser Cys Lys Ser Ser His Asp Arg Cys Leu
        275                 280                 285

Glu Lys Ser Ser Ser Ser Ser Ser Gln Pro Glu His Ser Ala Met Leu
    290                 295                 300

Val Ser Thr Ala Ala Ser Pro Ser Leu Ile Lys Glu Thr Thr Thr Gly
305                 310                 315                 320

Tyr Tyr Lys Asp Ile Val Glu Asn Ile Cys Gly Arg Glu Lys Ser Gly
                325                 330                 335

Ile Gln Pro Leu Cys Pro Glu Arg Ser His Ile Ser Asp Gln Ser Pro
            340                 345                 350

Leu Ser Ser Lys Arg Lys Ala Leu Glu Glu Ser Glu Ser Ser Gln Leu
        355                 360                 365

Ile Ser Pro Pro Leu Ala Gln Ala Ile Arg Asp Tyr Val Asn Ser Leu
    370                 375                 380

Leu Val Gln Gly Gly Val Gly Ser Leu Pro Gly Thr Ser Asn Ser Met
385                 390                 395                 400

Pro Pro Leu Asp Val Glu Asn Ile Gln Lys Arg Ile Asp Gln Ser Lys
                405                 410                 415

Phe Gln Glu Thr Glu Phe Leu Ser Pro Pro Arg Lys Val Pro Arg Leu
            420                 425                 430

Ser Glu Lys Ser Val Glu Glu Arg Asp Ser Gly Ser Phe Val Ala Phe
        435                 440                 445

Gln Asn Ile Pro Gly Ser Glu Leu Met Ser Ser Phe Ala Lys Thr Val
    450                 455                 460

Val Ser His Ser Leu Thr Thr Leu Gly Leu Glu Val Ala Lys Gln Ser
465                 470                 475                 480

Gln His Asp Lys Ile Asp Ala Ser Glu Leu Ser Phe Pro Phe His Glu
                485                 490                 495

Ser Ile Leu Lys Val Ile Glu Glu Glu Trp Gln Gln Val Asp Arg Gln
            500                 505                 510

Leu Pro Ser Leu Ala Cys Lys Tyr Pro Val Ser Ser Arg Glu Ala Thr
        515                 520                 525

Gln Ile Leu Ser Val Pro Lys Val Asp Asp Glu Ile Leu Gly Phe Ile
    530                 535                 540

Ser Glu Ala Thr Pro Leu Gly Gly Ile Gln Ala Ala Ser Thr Glu Ser
545                 550                 555                 560

Cys Asn Gln Gln Leu Asp Leu Ala Leu Cys Arg Ala Tyr Glu Ala Ala
                565                 570                 575

Ala Ser Ala Leu Gln Ile Ala Thr His Thr Ala Phe Val Ala Lys Ala
            580                 585                 590

Met Gln Ala Asp Ile Ser Gln Ala Ala Gln Ile Leu Ser Ser Asp Pro
        595                 600                 605

Ser Arg Thr His Gln Ala Leu Gly Ile Leu Ser Lys Thr Tyr Asp Ala
    610                 615                 620

Ala Ser Tyr Ile Cys Glu Ala Ala Phe Asp Glu Val Lys Met Ala Ala
625                 630                 635                 640

His Thr Met Gly Asn Ala Thr Val Gly Arg Arg Tyr Leu Trp Leu Lys
                645                 650                 655

Asp Cys Lys Ile Asn Leu Ala Ser Lys Asn Lys Leu Ala Ser Thr Pro
            660                 665                 670

Phe Lys Gly Gly Thr Leu Phe Gly Gly Glu Val Cys Lys Val Ile Lys
        675                 680                 685

Lys Arg Gly Asn Lys His
    690

<210> 260
<211> 1531
<212> PRT
<213> Homo sapiens

<400> 260

Met Glu Val Ser Pro Leu Gln Pro Val Asn Glu Asn Met Gln Val Asn
1               5                   10                  15

Lys Ile Lys Lys Asn Glu Asp Ala Lys Lys Arg Leu Ser Val Glu Arg
            20                  25                  30

Ile Tyr Gln Lys Lys Thr Gln Leu Glu His Ile Leu Leu Arg Pro Asp
        35                  40                  45

Thr Tyr Ile Gly Ser Val Glu Leu Val Thr Gln Gln Met Trp Val Tyr
    50                  55                  60

Asp Glu Asp Val Gly Ile Asn Tyr Arg Glu Val Thr Phe Val Pro Gly
65                  70                  75                  80

Leu Tyr Lys Ile Phe Asp Glu Ile Leu Val Asn Ala Ala Asp Asn Lys
                85                  90                  95

Gln Arg Asp Pro Lys Met Ser Cys Ile Arg Val Thr Ile Asp Pro Glu
            100                 105                 110

Asn Asn Leu Ile Ser Ile Trp Asn Asn Gly Lys Gly Ile Pro Val Val
        115                 120                 125

Glu His Lys Val Glu Lys Met Tyr Val Pro Ala Leu Ile Phe Gly Gln
    130                 135                 140

Leu Leu Thr Ser Ser Asn Tyr Asp Asp Asp Glu Lys Lys Val Thr Gly
145                 150                 155                 160

Gly Arg Asn Gly Tyr Gly Ala Lys Leu Cys Asn Ile Phe Ser Thr Lys
                165                 170                 175

Phe Thr Val Glu Thr Ala Ser Arg Glu Tyr Lys Lys Met Phe Lys Gln
            180                 185                 190

Thr Trp Met Asp Asn Met Gly Arg Ala Gly Glu Met Glu Leu Lys Pro
        195                 200                 205

Phe Asn Gly Glu Asp Tyr Thr Cys Ile Thr Phe Gln Pro Asp Leu Ser
    210                 215                 220

Lys Phe Lys Met Gln Ser Leu Asp Lys Asp Ile Val Ala Leu Met Val
225                 230                 235                 240

Arg Arg Ala Tyr Asp Ile Ala Gly Ser Thr Lys Asp Val Lys Val Phe
                245                 250                 255

Leu Asn Gly Asn Lys Leu Pro Val Lys Gly Phe Arg Ser Tyr Val Asp
            260                 265                 270

Met Tyr Leu Lys Asp Lys Leu Asp Glu Thr Gly Asn Ser Leu Lys Val
        275                 280                 285

Ile His Glu Gln Val Asn His Arg Trp Glu Val Cys Leu Thr Met Ser
    290                 295                 300

Glu Lys Gly Phe Gln Gln Ile Ser Phe Val Asn Ser Ile Ala Thr Ser
305                 310                 315                 320

Lys Gly Gly Arg His Val Asp Tyr Val Ala Asp Gln Ile Val Thr Lys
                325                 330                 335

Leu Val Asp Val Val Lys Lys Lys Asn Lys Gly Gly Val Ala Val Lys
            340                 345                 350

Ala His Gln Val Lys Asn His Met Trp Ile Phe Val Asn Ala Leu Ile
        355                 360                 365

Glu Asn Pro Thr Phe Asp Ser Gln Thr Lys Glu Asn Met Thr Leu Gln
    370                 375                 380

Pro Lys Ser Phe Gly Ser Thr Cys Gln Leu Ser Glu Lys Phe Ile Lys
385                 390                 395                 400

Ala Ala Ile Gly Cys Gly Ile Val Glu Ser Ile Leu Asn Trp Val Lys
                405                 410                 415

Phe Lys Ala Gln Val Gln Leu Asn Lys Lys Cys Ser Ala Val Lys His

            420                 425                 430

Asn Arg Ile Lys Gly Ile Pro Lys Leu Asp Asp Ala Asn Asp Ala Gly
        435                 440                 445

Gly Arg Asn Ser Thr Glu Cys Thr Leu Ile Leu Thr Glu Gly Asp Ser
    450                 455                 460

Ala Lys Thr Leu Ala Val Ser Gly Leu Gly Val Val Gly Arg Asp Lys
465                 470                 475                 480

Tyr Gly Val Phe Pro Leu Arg Gly Lys Ile Leu Asn Val Arg Glu Ala
                485                 490                 495

Ser His Lys Gln Ile Met Glu Asn Ala Glu Ile Asn Asn Ile Ile Lys
            500                 505                 510

Ile Val Gly Leu Gln Tyr Lys Lys Asn Tyr Glu Asp Glu Asp Ser Leu
        515                 520                 525

Lys Thr Leu Arg Tyr Gly Lys Ile Met Ile Met Thr Asp Gln Asp Gln
    530                 535                 540

Asp Gly Ser His Ile Lys Gly Leu Leu Ile Asn Phe Ile His His Asn
545                 550                 555                 560

Trp Pro Ser Leu Leu Arg His Arg Phe Leu Glu Glu Phe Ile Thr Pro
                565                 570                 575

Ile Val Lys Val Ser Lys Asn Lys Gln Glu Met Ala Phe Tyr Ser Leu
            580                 585                 590

Pro Glu Phe Glu Glu Trp Lys Ser Ser Thr Pro Asn His Lys Lys Trp
        595                 600                 605

Lys Val Lys Tyr Tyr Lys Gly Leu Gly Thr Ser Thr Ser Lys Glu Ala
    610                 615                 620

Lys Glu Tyr Phe Ala Asp Met Lys Arg His Arg Ile Gln Phe Lys Tyr
625                 630                 635                 640

Ser Gly Pro Glu Asp Asp Ala Ala Ile Ser Leu Ala Phe Ser Lys Lys
                645                 650                 655

Gln Ile Asp Asp Arg Lys Glu Trp Leu Thr Asn Phe Met Glu Asp Arg
            660                 665                 670

Arg Gln Arg Lys Leu Leu Gly Leu Pro Glu Asp Tyr Leu Tyr Gly Gln
        675                 680                 685

Thr Thr Thr Tyr Leu Thr Tyr Asn Asp Phe Ile Asn Lys Glu Leu Ile
    690                 695                 700

Leu Phe Ser Asn Ser Asp Asn Glu Arg Ser Ile Pro Ser Met Val Asp
705                 710                 715                 720

Gly Leu Lys Pro Gly Gln Arg Lys Val Leu Phe Thr Cys Phe Lys Arg
                725                 730                 735

Asn Asp Lys Arg Glu Val Lys Val Ala Gln Leu Ala Gly Ser Val Ala
            740                 745                 750

Glu Met Ser Ser Tyr His His Gly Glu Met Ser Leu Met Met Thr Ile
        755                 760                 765

Ile Asn Leu Ala Gln Asn Phe Val Gly Ser Asn Asn Leu Asn Leu Leu
    770                 775                 780

Gln Pro Ile Gly Gln Phe Gly Thr Arg Leu His Gly Gly Lys Asp Ser
785                 790                 795                 800

Ala Ser Pro Arg Tyr Ile Phe Thr Met Leu Ser Ser Leu Ala Arg Leu
                805                 810                 815

Leu Phe Pro Pro Lys Asp Asp His Thr Leu Lys Phe Leu Tyr Asp Asp
            820                 825                 830

Asn Gln Arg Val Glu Pro Glu Trp Tyr Ile Pro Ile Ile Pro Met Val
        835                 840                 845

Leu Ile Asn Gly Ala Glu Gly Ile Gly Thr Gly Trp Ser Cys Lys Ile
    850                 855                 860

Pro Asn Phe Asp Val Arg Glu Ile Val Asn Asn Ile Arg Arg Leu Met
865                 870                 875                 880

Asp Gly Glu Glu Pro Leu Pro Met Leu Pro Ser Tyr Lys Asn Phe Lys
                885                 890                 895

Gly Thr Ile Glu Glu Leu Ala Pro Asn Gln Tyr Val Ile Ser Gly Glu
            900                 905                 910

Val Ala Ile Leu Asn Ser Thr Thr Ile Glu Ile Ser Glu Leu Pro Val
        915                 920                 925

Arg Thr Trp Thr Gln Thr Tyr Lys Glu Gln Val Leu Glu Pro Met Leu
    930                 935                 940

Asn Gly Thr Glu Lys Thr Pro Pro Leu Ile Thr Asp Tyr Arg Glu Tyr
945                 950                 955                 960

His Thr Asp Thr Thr Val Lys Phe Val Val Lys Met Thr Glu Glu Lys
                965                 970                 975

Leu Ala Glu Ala Glu Arg Val Gly Leu His Lys Val Phe Lys Leu Gln

980                    985                    990

Thr Ser Leu Thr Cys Asn Ser Met  Val Leu Phe Asp His  Val Gly Cys
        995                    1000                   1005

Leu Lys  Lys Tyr Asp Thr Val  Leu Asp Ile Leu Arg  Asp Phe Phe
    1010                 1015                 1020

Glu Leu  Arg Leu Lys Tyr Tyr  Gly Leu Arg Lys Glu  Trp Leu Leu
    1025                 1030                 1035

Gly Met  Leu Gly Ala Glu Ser  Ala Lys Leu Asn Asn  Gln Ala Arg
    1040                 1045                 1050

Phe Ile  Leu Glu Lys Ile Asp  Gly Lys Ile Ile Ile  Glu Asn Lys
    1055                 1060                 1065

Pro Lys  Lys Glu Leu Ile Lys  Val Leu Ile Gln Arg  Gly Tyr Asp
    1070                 1075                 1080

Ser Asp  Pro Val Lys Ala Trp  Lys Glu Ala Gln Gln  Lys Val Pro
    1085                 1090                 1095

Asp Glu  Glu Glu Asn Glu Glu  Ser Asp Asn Glu Lys  Glu Thr Glu
    1100                 1105                 1110

Lys Ser  Asp Ser Val Thr Asp  Ser Gly Pro Thr Phe  Asn Tyr Leu
    1115                 1120                 1125

Leu Asp  Met Pro Leu Trp Tyr  Leu Thr Lys Glu Lys  Lys Asp Glu
    1130                 1135                 1140

Leu Cys  Arg Leu Arg Asn Glu  Lys Glu Gln Glu Leu  Asp Thr Leu
    1145                 1150                 1155

Lys Arg  Lys Ser Pro Ser Asp  Leu Trp Lys Glu Asp  Leu Ala Thr
    1160                 1165                 1170

Phe Ile  Glu Glu Leu Glu Ala  Val Glu Ala Lys Glu  Lys Gln Asp
    1175                 1180                 1185

Glu Gln  Val Gly Leu Pro Gly  Lys Gly Gly Lys Ala  Lys Gly Lys
    1190                 1195                 1200

Lys Thr  Gln Met Ala Glu Val  Leu Pro Ser Pro Arg  Gly Gln Arg
    1205                 1210                 1215

Val Ile  Pro Arg Ile Thr Ile  Glu Met Lys Ala Glu  Ala Glu Lys
    1220                 1225                 1230

Lys Asn  Lys Lys Lys Ile Lys  Asn Glu Asn Thr Glu  Gly Ser Pro
    1235                 1240                 1245

Gln Glu Asp Gly Val Glu Leu Glu Gly Leu Lys Gln Arg Leu Glu
    1250                1255                1260

Lys Lys Gln Lys Arg Glu Pro Gly Thr Lys Thr Lys Lys Gln Thr
    1265                1270                1275

Thr Leu Ala Phe Lys Pro Ile Lys Lys Gly Lys Lys Arg Asn Pro
    1280                1285                1290

Trp Ser Asp Ser Glu Ser Asp Arg Ser Ser Asp Glu Ser Asn Phe
    1295                1300                1305

Asp Val Pro Pro Arg Glu Thr Glu Pro Arg Arg Ala Ala Thr Lys
    1310                1315                1320

Thr Lys Phe Thr Met Asp Leu Asp Ser Asp Glu Asp Phe Ser Asp
    1325                1330                1335

Phe Asp Glu Lys Thr Asp Asp Glu Asp Phe Val Pro Ser Asp Ala
    1340                1345                1350

Ser Pro Pro Lys Thr Lys Thr Ser Pro Lys Leu Ser Asn Lys Glu
    1355                1360                1365

Leu Lys Pro Gln Lys Ser Val Val Ser Asp Leu Glu Ala Asp Asp
    1370                1375                1380

Val Lys Gly Ser Val Pro Leu Ser Ser Ser Pro Pro Ala Thr His
    1385                1390                1395

Phe Pro Asp Glu Thr Glu Ile Thr Asn Pro Val Pro Lys Lys Asn
    1400                1405                1410

Val Thr Val Lys Lys Thr Ala Ala Lys Ser Gln Ser Ser Thr Ser
    1415                1420                1425

Thr Thr Gly Ala Lys Lys Arg Ala Ala Pro Lys Gly Thr Lys Arg
    1430                1435                1440

Asp Pro Ala Leu Asn Ser Gly Val Ser Gln Lys Pro Asp Pro Ala
    1445                1450                1455

Lys Thr Lys Asn Arg Arg Lys Arg Lys Pro Ser Thr Ser Asp Asp
    1460                1465                1470

Ser Asp Ser Asn Phe Glu Lys Ile Val Ser Lys Ala Val Thr Ser
    1475                1480                1485

Lys Lys Ser Lys Gly Glu Ser Asp Asp Phe His Met Asp Phe Asp
    1490                1495                1500

Ser Ala Val Ala Pro Arg Ala Lys Ser Val Arg Ala Lys Lys Pro

1505                1510                1515

Ile Lys  Tyr Leu Glu Glu Ser  Asp Glu Asp Asp Leu  Phe
    1520                1525                1530

<210>  261
<211>  857
<212>  PRT
<213>  Homo sapiens

<400>  261

Met Glu Ser Glu Asp Leu Ser Gly Arg Glu Leu Thr Ile Asp Ser Ile
1               5                   10                  15

Met Asn Lys Val Arg Asp Ile Lys Asn Lys Phe Lys Asn Glu Asp Leu
            20                  25                  30

Thr Asp Glu Leu Ser Leu Asn Lys Ile Ser Ala Asp Thr Thr Asp Asn
        35                  40                  45

Ser Gly Thr Val Asn Gln Ile Met Met Met Ala Asn Asn Pro Glu Asp
    50                  55                  60

Trp Leu Ser Leu Leu Leu Lys Leu Glu Lys Asn Ser Val Pro Leu Ser
65                  70                  75                  80

Asp Ala Leu Leu Asn Lys Leu Ile Gly Arg Tyr Ser Gln Ala Ile Glu
                85                  90                  95

Ala Leu Pro Pro Asp Lys Tyr Gly Gln Asn Glu Ser Phe Ala Arg Ile
            100                 105                 110

Gln Val Arg Phe Ala Glu Leu Lys Ala Ile Gln Glu Pro Asp Asp Ala
        115                 120                 125

Arg Asp Tyr Phe Gln Met Ala Arg Ala Asn Cys Lys Lys Phe Ala Phe
    130                 135                 140

Val His Ile Ser Phe Ala Gln Phe Glu Leu Ser Gln Gly Asn Val Lys
145                 150                 155                 160

Lys Ser Lys Gln Leu Leu Gln Lys Ala Val Glu Arg Gly Ala Val Pro
                165                 170                 175

Leu Glu Met Leu Glu Ile Ala Leu Arg Asn Leu Asn Leu Gln Lys Lys
            180                 185                 190

Gln Leu Leu Ser Glu Glu Glu Lys Lys Asn Leu Ser Ala Ser Thr Val
        195                 200                 205

Leu Thr Ala Gln Glu Ser Phe Ser Gly Ser Leu Gly His Leu Gln Asn
    210                 215                 220

Arg Asn Asn Ser Cys Asp Ser Arg Gly Gln Thr Thr Lys Ala Arg Phe
225                 230                 235                 240

Leu Tyr Gly Glu Asn Met Pro Pro Gln Asp Ala Glu Ile Gly Tyr Arg
                245                 250                 255

Asn Ser Leu Arg Gln Thr Asn Lys Thr Lys Gln Ser Cys Pro Phe Gly
            260                 265                 270

Arg Val Pro Val Asn Leu Leu Asn Ser Pro Asp Cys Asp Val Lys Thr
        275                 280                 285

Asp Asp Ser Val Val Pro Cys Phe Met Lys Arg Gln Thr Ser Arg Ser
    290                 295                 300

Glu Cys Arg Asp Leu Val Val Pro Gly Ser Lys Pro Ser Gly Asn Asp
305                 310                 315                 320

Ser Cys Glu Leu Arg Asn Leu Lys Ser Val Gln Asn Ser His Phe Lys
                325                 330                 335

Glu Pro Leu Val Ser Asp Glu Lys Ser Ser Glu Leu Ile Ile Thr Asp
            340                 345                 350

Ser Ile Thr Leu Lys Asn Lys Thr Glu Ser Ser Leu Leu Ala Lys Leu
        355                 360                 365

Glu Glu Thr Lys Glu Tyr Gln Glu Pro Glu Val Pro Glu Ser Asn Gln
    370                 375                 380

Lys Gln Trp Gln Ser Lys Arg Lys Ser Glu Cys Ile Asn Gln Asn Pro
385                 390                 395                 400

Ala Ala Ser Ser Asn His Trp Gln Ile Pro Glu Leu Ala Arg Lys Val
                405                 410                 415

Asn Thr Glu Gln Lys His Thr Thr Phe Glu Gln Pro Val Phe Ser Val
            420                 425                 430

Ser Lys Gln Ser Pro Pro Ile Ser Thr Ser Lys Trp Phe Asp Pro Lys
        435                 440                 445

Ser Ile Cys Lys Thr Pro Ser Ser Asn Thr Leu Asp Asp Tyr Met Ser
    450                 455                 460

Cys Phe Arg Thr Pro Val Val Lys Asn Asp Phe Pro Pro Ala Cys Gln
465                 470                 475                 480

Leu Ser Thr Pro Tyr Gly Gln Pro Ala Cys Phe Gln Gln Gln Gln His
                485                 490                 495

Gln Ile Leu Ala Thr Pro Leu Gln Asn Leu Gln Val Leu Ala Ser Ser
            500                 505                 510

Ser Ala Asn Glu Cys Ile Ser Val Lys Gly Arg Ile Tyr Ser Ile Leu
        515                 520                 525

Lys Gln Ile Gly Ser Gly Gly Ser Ser Lys Val Phe Gln Val Leu Asn
    530                 535                 540

Glu Lys Lys Gln Ile Tyr Ala Ile Lys Tyr Val Asn Leu Glu Glu Ala
545                 550                 555                 560

Asp Asn Gln Thr Leu Asp Ser Tyr Arg Asn Glu Ile Ala Tyr Leu Asn
                565                 570                 575

Lys Leu Gln Gln His Ser Asp Lys Ile Ile Arg Leu Tyr Asp Tyr Glu
            580                 585                 590

Ile Thr Asp Gln Tyr Ile Tyr Met Val Met Glu Cys Gly Asn Ile Asp
        595                 600                 605

Leu Asn Ser Trp Leu Lys Lys Lys Lys Ser Ile Asp Pro Trp Glu Arg
    610                 615                 620

Lys Ser Tyr Trp Lys Asn Met Leu Glu Ala Val His Thr Ile His Gln
625                 630                 635                 640

His Gly Ile Val His Ser Asp Leu Lys Pro Ala Asn Phe Leu Ile Val
                645                 650                 655

Asp Gly Met Leu Lys Leu Ile Asp Phe Gly Ile Ala Asn Gln Met Gln
            660                 665                 670

Pro Asp Thr Thr Ser Val Val Lys Asp Ser Gln Val Gly Thr Val Asn
        675                 680                 685

Tyr Met Pro Pro Glu Ala Ile Lys Asp Met Ser Ser Ser Arg Glu Asn
    690                 695                 700

Gly Lys Ser Lys Ser Lys Ile Ser Pro Lys Ser Asp Val Trp Ser Leu
705                 710                 715                 720

Gly Cys Ile Leu Tyr Tyr Met Thr Tyr Gly Lys Thr Pro Phe Gln Gln
                725                 730                 735

Ile Ile Asn Gln Ile Ser Lys Leu His Ala Ile Ile Asp Pro Asn His
            740                 745                 750

Glu Ile Glu Phe Pro Asp Ile Pro Glu Lys Asp Leu Gln Asp Val Leu
        755                 760                 765

Lys Cys Cys Leu Lys Arg Asp Pro Lys Gln Arg Ile Ser Ile Pro Glu
    770                 775                 780

Leu Leu Ala His Pro Tyr Val Gln Ile Gln Thr His Pro Val Asn Gln
785                 790                 795                 800

Met Ala Lys Gly Thr Thr Glu Glu Met Lys Tyr Val Leu Gly Gln Leu
                805                 810                 815

Val Gly Leu Asn Ser Pro Asn Ser Ile Leu Lys Ala Ala Lys Thr Leu
            820                 825                 830

Tyr Glu His Tyr Ser Gly Gly Glu Ser His Asn Ser Ser Ser Ser Lys
        835                 840                 845

Thr Phe Glu Lys Lys Arg Gly Lys Lys
    850                 855

<210>  262
<211>  313
<212>  PRT
<213>  Homo sapiens

<400>  262

Met Pro Val Ala Gly Ser Glu Leu Pro Arg Arg Pro Leu Pro Pro Ala
1               5                   10                  15

Ala Gln Glu Arg Asp Ala Glu Pro Arg Pro Pro His Gly Glu Leu Gln
            20                  25                  30

Tyr Leu Gly Gln Ile Gln His Ile Leu Arg Cys Gly Val Arg Lys Asp
        35                  40                  45

Asp Arg Thr Gly Thr Gly Thr Leu Ser Val Phe Gly Met Gln Ala Arg
    50                  55                  60

Tyr Ser Leu Arg Asp Glu Phe Pro Leu Leu Thr Thr Lys Arg Val Phe
65                  70                  75                  80

Trp Lys Gly Val Leu Glu Glu Leu Leu Trp Phe Ile Lys Gly Ser Thr
                85                  90                  95

Asn Ala Lys Glu Leu Ser Ser Lys Gly Val Lys Ile Trp Asp Ala Asn
            100                 105                 110

Gly Ser Arg Asp Phe Leu Asp Ser Leu Gly Phe Ser Thr Arg Glu Glu
        115                 120                 125

Gly Asp Leu Gly Pro Val Tyr Gly Phe Gln Trp Arg His Phe Gly Ala
    130                 135                 140

Glu Tyr Arg Asp Met Glu Ser Asp Tyr Ser Gly Gln Gly Val Asp Gln
145                 150                 155                 160

Leu Gln Arg Val Ile Asp Thr Ile Lys Thr Asn Pro Asp Asp Arg Arg
                165                 170                 175

Ile Ile Met Cys Ala Trp Asn Pro Arg Asp Leu Pro Leu Met Ala Leu
            180                 185                 190

Pro Pro Cys His Ala Leu Cys Gln Phe Tyr Val Val Asn Ser Glu Leu
        195                 200                 205

Ser Cys Gln Leu Tyr Gln Arg Ser Gly Asp Met Gly Leu Gly Val Pro
    210                 215                 220

Phe Asn Ile Ala Ser Tyr Ala Leu Leu Thr Tyr Met Ile Ala His Ile
225                 230                 235                 240

Thr Gly Leu Lys Pro Gly Asp Phe Ile His Thr Leu Gly Asp Ala His
                245                 250                 255

Ile Tyr Leu Asn His Ile Glu Pro Leu Lys Ile Gln Leu Gln Arg Glu
            260                 265                 270

Pro Arg Pro Phe Pro Lys Leu Arg Ile Leu Arg Lys Val Glu Lys Ile
        275                 280                 285

Asp Asp Phe Lys Ala Glu Asp Phe Gln Ile Glu Gly Tyr Asn Pro His
    290                 295                 300

Pro Thr Ile Lys Met Glu Met Ala Val
305                 310

<210> 263
<211> 304
<212> PRT
<213> Homo sapiens

<400> 263

Met Gly Val Phe Cys Leu Gly Pro Trp Gly Leu Gly Arg Lys Leu Arg
1               5                   10                  15

Thr Pro Gly Lys Gly Pro Leu Gln Leu Leu Ser Arg Leu Cys Gly Asp
            20                  25                  30

His Leu Gln Ala Ile Pro Ala Lys Lys Ala Pro Ala Gly Gln Glu Glu
        35                  40                  45

Pro Gly Thr Pro Pro Ser Ser Pro Leu Ser Ala Glu Gln Leu Asp Arg
    50                  55                  60

Ile Gln Arg Asn Lys Ala Ala Ala Leu Leu Arg Leu Ala Ala Arg Asn
65                  70                  75                  80

Val Pro Val Gly Phe Gly Glu Ser Trp Lys Lys His Leu Ser Gly Glu
                85                  90                  95

Phe Gly Lys Pro Tyr Phe Ile Lys Leu Met Gly Phe Val Ala Glu Glu
            100                 105                 110

Arg Lys His Tyr Thr Val Tyr Pro Pro Pro His Gln Val Phe Thr Trp
        115                 120                 125

Thr Gln Met Cys Asp Ile Lys Asp Val Lys Val Val Ile Leu Gly Gln
    130                 135                 140

Asp Pro Tyr His Gly Pro Asn Gln Ala His Gly Leu Cys Phe Ser Val
145                 150                 155                 160

Gln Arg Pro Val Pro Pro Pro Pro Ser Leu Glu Asn Ile Tyr Lys Glu
                165                 170                 175

Leu Ser Thr Asp Ile Glu Asp Phe Val His Pro Gly His Gly Asp Leu
            180                 185                 190

Ser Gly Trp Ala Lys Gln Gly Val Leu Leu Leu Asn Ala Val Leu Thr
        195                 200                 205

Val Arg Ala His Gln Ala Asn Ser His Lys Glu Arg Gly Trp Glu Gln
    210                 215                 220

Phe Thr Asp Ala Val Val Ser Trp Leu Asn Gln Asn Ser Asn Gly Leu
225                 230                 235                 240

Val Phe Leu Leu Trp Gly Ser Tyr Ala Gln Lys Lys Gly Ser Ala Ile
                245                 250                 255

Asp Arg Lys Arg His His Val Leu Gln Thr Ala His Pro Ser Pro Leu
            260                 265                 270

Ser Val Tyr Arg Gly Phe Phe Gly Cys Arg His Phe Ser Lys Thr Asn
        275                 280                 285

Glu Leu Leu Gln Lys Ser Gly Lys Lys Pro Ile Asp Trp Lys Glu Leu
    290                 295                 300

<210>  264
<211>  313
<212>  PRT
<213>  Homo sapiens

<400>  264

Met Ile Gly Gln Lys Thr Leu Tyr Ser Phe Phe Ser Pro Ser Pro Ala
1               5                   10                  15

Arg Lys Arg His Ala Pro Ser Pro Glu Pro Ala Val Gln Gly Thr Gly
            20                  25                  30

Val Ala Gly Val Pro Glu Glu Ser Gly Asp Ala Ala Ala Ile Pro Ala
        35                  40                  45

Lys Lys Ala Pro Ala Gly Gln Glu Glu Pro Gly Thr Pro Pro Ser Ser

50                  55                  60

Pro Leu Ser Ala Glu Gln Leu Asp Arg Ile Gln Arg Asn Lys Ala Ala
65                  70                  75                  80

Ala Leu Leu Arg Leu Ala Ala Arg Asn Val Pro Val Gly Phe Gly Glu
                85                  90                  95

Ser Trp Lys Lys His Leu Ser Gly Glu Phe Gly Lys Pro Tyr Phe Ile
            100                 105                 110

Lys Leu Met Gly Phe Val Ala Glu Glu Arg Lys His Tyr Thr Val Tyr
        115                 120                 125

Pro Pro Pro His Gln Val Phe Thr Trp Thr Gln Met Cys Asp Ile Lys
    130                 135                 140

Asp Val Lys Val Val Ile Leu Gly Gln Asp Pro Tyr His Gly Pro Asn
145                 150                 155                 160

Gln Ala His Gly Leu Cys Phe Ser Val Gln Arg Pro Val Pro Pro Pro
                165                 170                 175

Pro Ser Leu Glu Asn Ile Tyr Lys Glu Leu Ser Thr Asp Ile Glu Asp
            180                 185                 190

Phe Val His Pro Gly His Gly Asp Leu Ser Gly Trp Ala Lys Gln Gly
        195                 200                 205

Val Leu Leu Leu Asn Ala Val Leu Thr Val Arg Ala His Gln Ala Asn
    210                 215                 220

Ser His Lys Glu Arg Gly Trp Glu Gln Phe Thr Asp Ala Val Val Ser
225                 230                 235                 240

Trp Leu Asn Gln Asn Ser Asn Gly Leu Val Phe Leu Leu Trp Gly Ser
                245                 250                 255

Tyr Ala Gln Lys Lys Gly Ser Ala Ile Asp Arg Lys Arg His His Val
            260                 265                 270

Leu Gln Thr Ala His Pro Ser Pro Leu Ser Val Tyr Arg Gly Phe Phe
        275                 280                 285

Gly Cys Arg His Phe Ser Lys Thr Asn Glu Leu Leu Gln Lys Ser Gly
    290                 295                 300

Lys Lys Pro Ile Asp Trp Lys Glu Leu
305                 310

<210>  265
<211>  396
<212>  PRT

<213>  Homo sapiens

<400>  265

Met Pro Arg Val Lys Ala Ala Gln Ala Gly Arg Gln Ser Ser Ala Lys
1               5                   10                  15

Arg His Leu Ala Glu Gln Phe Ala Val Gly Glu Ile Ile Thr Asp Met
            20                  25                  30

Ala Lys Lys Glu Trp Lys Val Gly Leu Pro Ile Gly Gln Gly Gly Phe
        35                  40                  45

Gly Cys Ile Tyr Leu Ala Asp Met Asn Ser Ser Glu Ser Val Gly Ser
    50                  55                  60

Asp Ala Pro Cys Val Val Lys Val Glu Pro Ser Asp Asn Gly Pro Leu
65                  70                  75                  80

Phe Thr Glu Leu Lys Phe Tyr Gln Arg Ala Ala Lys Pro Glu Gln Ile
                85                  90                  95

Gln Lys Trp Ile Arg Thr Arg Lys Leu Lys Tyr Leu Gly Val Pro Lys
            100                 105                 110

Tyr Trp Gly Ser Gly Leu His Asp Lys Asn Gly Lys Ser Tyr Arg Phe
        115                 120                 125

Met Ile Met Asp Arg Phe Gly Ser Asp Leu Gln Lys Ile Tyr Glu Ala
    130                 135                 140

Asn Ala Lys Arg Phe Ser Arg Lys Thr Val Leu Gln Leu Ser Leu Arg
145                 150                 155                 160

Ile Leu Asp Ile Leu Glu Tyr Ile His Glu His Glu Tyr Val His Gly
                165                 170                 175

Asp Ile Lys Ala Ser Asn Leu Leu Leu Asn Tyr Lys Asn Pro Asp Gln
            180                 185                 190

Val Tyr Leu Val Asp Tyr Gly Leu Ala Tyr Arg Tyr Cys Pro Glu Gly
        195                 200                 205

Val His Lys Glu Tyr Lys Glu Asp Pro Lys Arg Cys His Asp Gly Thr
    210                 215                 220

Ile Glu Phe Thr Ser Ile Asp Ala His Asn Gly Val Ala Pro Ser Arg
225                 230                 235                 240

Arg Gly Asp Leu Glu Ile Leu Gly Tyr Cys Met Ile Gln Trp Leu Thr
                245                 250                 255

Gly His Leu Pro Trp Glu Asp Asn Leu Lys Asp Pro Lys Tyr Val Arg
            260                 265                 270

Asp Ser Lys Ile Arg Tyr Arg Glu Asn Ile Ala Ser Leu Met Asp Lys
        275                 280                 285

Cys Phe Pro Glu Lys Asn Lys Pro Gly Glu Ile Ala Lys Tyr Met Glu
    290                 295                 300

Thr Val Lys Leu Leu Asp Tyr Thr Glu Lys Pro Leu Tyr Glu Asn Leu
305                 310                 315                 320

Arg Asp Ile Leu Leu Gln Gly Leu Lys Ala Ile Gly Ser Lys Asp Asp
                325                 330                 335

Gly Lys Leu Asp Leu Ser Val Val Glu Asn Gly Gly Leu Lys Ala Lys
            340                 345                 350

Thr Ile Thr Lys Lys Arg Lys Lys Glu Ile Glu Glu Ser Lys Glu Pro
        355                 360                 365

Gly Val Glu Asp Thr Glu Trp Ser Asn Thr Gln Thr Glu Glu Ala Ile
    370                 375                 380

Gln Thr Arg Ser Arg Thr Arg Lys Arg Val Gln Lys
385                 390                 395

<210>  266
<211>  380
<212>  PRT
<213>  Homo sapiens

<400>  266

Met Leu Val Ile Pro Pro Gly Leu Ser Glu Glu Glu Glu Ala Leu Gln
1               5                   10                  15

Lys Lys Phe Asn Lys Leu Lys Lys Lys Lys Lys Ala Leu Leu Ala Leu
            20                  25                  30

Lys Lys Gln Ser Ser Ser Ser Thr Thr Ser Gln Gly Gly Val Lys Arg
        35                  40                  45

Ser Leu Ser Glu Gln Pro Val Met Asp Thr Ala Thr Ala Thr Glu Gln
    50                  55                  60

Ala Lys Gln Leu Val Lys Ser Gly Ala Ile Ser Ala Ile Lys Ala Glu
65                  70                  75                  80

Thr Lys Asn Ser Gly Phe Lys Arg Ser Arg Thr Leu Glu Gly Lys Leu
                85                  90                  95

Lys Asp Pro Glu Lys Gly Pro Val Pro Thr Phe Gln Pro Phe Gln Arg
            100                 105                 110

Ser Ile Ser Ala Asp Asp Asp Leu Gln Glu Ser Ser Arg Arg Pro Gln

115                 120                 125

Arg Lys Ser Leu Tyr Glu Ser Phe Val Ser Ser Ser Asp Arg Leu Arg
    130                 135                 140

Glu Leu Gly Pro Asp Gly Glu Glu Ala Glu Gly Pro Gly Ala Gly Asp
145                 150                 155                 160

Gly Pro Pro Arg Ser Phe Asp Trp Gly Tyr Glu Glu Arg Ser Gly Ala
                165                 170                 175

His Ser Ser Ala Ser Pro Pro Arg Ser Arg Ser Arg Asp Arg Ser His
            180                 185                 190

Glu Arg Asn Arg Asp Arg Asp Arg Asp Arg Glu Arg Asp Arg Asp Arg
        195                 200                 205

Asp Arg Asp Arg Asp Arg Glu Arg Asp Arg Asp Arg Asp Arg Asp Arg
    210                 215                 220

Asp Arg Asp Arg Glu Arg Asp Arg Asp Arg Glu Arg Asp Arg Asp Arg
225                 230                 235                 240

Asp Arg Glu Gly Pro Phe Arg Arg Ser Asp Ser Phe Pro Glu Arg Arg
                245                 250                 255

Ala Pro Arg Lys Gly Asn Thr Leu Tyr Val Tyr Gly Glu Asp Met Thr
            260                 265                 270

Pro Thr Leu Leu Arg Gly Ala Phe Ser Pro Phe Gly Asn Ile Ile Asp
        275                 280                 285

Leu Ser Met Asp Pro Pro Arg Asn Cys Ala Phe Val Thr Tyr Glu Lys
    290                 295                 300

Met Glu Ser Ala Asp Gln Ala Val Ala Glu Leu Asn Gly Thr Gln Val
305                 310                 315                 320

Glu Ser Val Gln Leu Lys Val Asn Ile Ala Arg Lys Gln Pro Met Leu
                325                 330                 335

Asp Ala Ala Thr Gly Lys Ser Val Trp Gly Ser Leu Ala Val Gln Asn
            340                 345                 350

Ser Pro Lys Gly Cys His Arg Asp Lys Arg Thr Gln Ile Val Tyr Ser
        355                 360                 365

Asp Asp Val Tyr Lys Glu Asn Leu Val Asp Gly Phe
    370                 375                 380

<210>  267
<211>  693
<212>  PRT

<213>  Homo sapiens

<400>  267

Met Ala Leu Cys Asn Gly Asp Ser Lys Leu Glu Asn Ala Gly Gly Asp
1               5                   10                  15

Leu Lys Asp Gly His His His Tyr Glu Gly Ala Val Val Ile Leu Asp
            20                  25                  30

Ala Gly Ala Gln Tyr Gly Lys Val Ile Asp Arg Arg Val Arg Glu Leu
        35                  40                  45

Phe Val Gln Ser Glu Ile Phe Pro Leu Glu Thr Pro Ala Phe Ala Ile
    50                  55                  60

Lys Glu Gln Gly Phe Arg Ala Ile Ile Ile Ser Gly Gly Pro Asn Ser
65                  70                  75                  80

Val Tyr Ala Glu Asp Ala Pro Trp Phe Asp Pro Ala Ile Phe Thr Ile
                85                  90                  95

Gly Lys Pro Val Leu Gly Ile Cys Tyr Gly Met Gln Met Met Asn Lys
            100                 105                 110

Val Phe Gly Gly Thr Val His Lys Lys Ser Val Arg Glu Asp Gly Val
        115                 120                 125

Phe Asn Ile Ser Val Asp Asn Thr Cys Ser Leu Phe Arg Gly Leu Gln
    130                 135                 140

Lys Glu Glu Val Val Leu Leu Thr His Gly Asp Ser Val Asp Lys Val
145                 150                 155                 160

Ala Asp Gly Phe Lys Val Val Ala Arg Ser Gly Asn Ile Val Ala Gly
                165                 170                 175

Ile Ala Asn Glu Ser Lys Lys Leu Tyr Gly Ala Gln Phe His Pro Glu
            180                 185                 190

Val Gly Leu Thr Glu Asn Gly Lys Val Ile Leu Lys Asn Phe Leu Tyr
        195                 200                 205

Asp Ile Ala Gly Cys Ser Gly Thr Phe Thr Val Gln Asn Arg Glu Leu
    210                 215                 220

Glu Cys Ile Arg Glu Ile Lys Glu Arg Val Gly Thr Ser Lys Val Leu
225                 230                 235                 240

Val Leu Leu Ser Gly Gly Val Asp Ser Thr Val Cys Thr Ala Leu Leu
                245                 250                 255

Asn Arg Ala Leu Asn Gln Glu Gln Val Ile Ala Val His Ile Asp Asn
            260                 265                 270

Gly Phe Met Arg Lys Arg Glu Ser Gln Ser Val Glu Glu Ala Leu Lys
        275                 280                 285

Lys Leu Gly Ile Gln Val Lys Val Ile Asn Ala Ala His Ser Phe Tyr
    290                 295                 300

Asn Gly Thr Thr Thr Leu Pro Ile Ser Asp Glu Asp Arg Thr Pro Arg
305                 310                 315                 320

Lys Arg Ile Ser Lys Thr Leu Asn Met Thr Thr Ser Pro Glu Glu Lys
                325                 330                 335

Arg Lys Ile Ile Gly Asp Thr Phe Val Lys Ile Ala Asn Glu Val Ile
            340                 345                 350

Gly Glu Met Asn Leu Lys Pro Glu Glu Val Phe Leu Ala Gln Gly Thr
        355                 360                 365

Leu Arg Pro Asp Leu Ile Glu Ser Ala Ser Leu Val Ala Ser Gly Lys
    370                 375                 380

Ala Glu Leu Ile Lys Thr His His Asn Asp Thr Glu Leu Ile Arg Lys
385                 390                 395                 400

Leu Arg Glu Glu Gly Lys Val Ile Glu Pro Leu Lys Asp Phe His Lys
                405                 410                 415

Asp Glu Val Arg Ile Leu Gly Arg Glu Leu Gly Leu Pro Glu Glu Leu
            420                 425                 430

Val Ser Arg His Pro Phe Pro Gly Pro Gly Leu Ala Ile Arg Val Ile
        435                 440                 445

Cys Ala Glu Glu Pro Tyr Ile Cys Lys Asp Phe Pro Glu Thr Asn Asn
    450                 455                 460

Ile Leu Lys Ile Val Ala Asp Phe Ser Ala Ser Val Lys Lys Pro His
465                 470                 475                 480

Thr Leu Leu Gln Arg Val Lys Ala Cys Thr Thr Glu Glu Asp Gln Glu
                485                 490                 495

Lys Leu Met Gln Ile Thr Ser Leu His Ser Leu Asn Ala Phe Leu Leu
            500                 505                 510

Pro Ile Lys Thr Val Gly Val Gln Gly Asp Cys Arg Ser Tyr Ser Tyr
        515                 520                 525

Val Cys Gly Ile Ser Ser Lys Asp Glu Pro Asp Trp Glu Ser Leu Ile
    530                 535                 540

Phe Leu Ala Arg Leu Ile Pro Arg Met Cys His Asn Val Asn Arg Val
545                 550                 555                 560

Val Tyr Ile Phe Gly Pro Pro Val Lys Glu Pro Pro Thr Asp Val Thr
                565                 570                 575

Pro Thr Phe Leu Thr Thr Gly Val Leu Ser Thr Leu Arg Gln Ala Asp
            580                 585                 590

Phe Glu Ala His Asn Ile Leu Arg Glu Ser Gly Tyr Ala Gly Lys Ile
        595                 600                 605

Ser Gln Met Pro Val Ile Leu Thr Pro Leu His Phe Asp Arg Asp Pro
    610                 615                 620

Leu Gln Lys Gln Pro Ser Cys Gln Arg Ser Val Val Ile Arg Thr Phe
625                 630                 635                 640

Ile Thr Ser Asp Phe Met Thr Gly Ile Pro Ala Thr Pro Gly Asn Glu
                645                 650                 655

Ile Pro Val Glu Val Val Leu Lys Met Val Thr Glu Ile Lys Lys Ile
            660                 665                 670

Pro Gly Ile Ser Arg Ile Met Tyr Asp Leu Thr Ser Lys Pro Pro Gly
        675                 680                 685

Thr Thr Glu Trp Glu
    690

<210>  268
<211>  1208
<212>  PRT
<213>  Homo sapiens

<400>  268
   Met Asp Leu His Met Met Asn Cys Glu Leu Leu Ala Thr Cys Ser Ala
   1               5                   10                  15
   Leu Gly Tyr Leu Glu Gly Asp Thr Tyr His Lys Glu Pro Asp Cys Leu
               20                  25                  30
   Glu Ser Val Lys Asp Leu Ile Arg Tyr Leu Arg His Glu Asp Glu Thr
           35                  40                  45
   Arg Asp Val Arg Gln Gln Leu Gly Ala Ala Gln Ile Leu Gln Ser Asp
       50                  55                  60
   Leu Leu Pro Ile Leu Thr Gln His His Gln Asp Lys Pro Leu Phe Asp
   65                  70                  75                  80
   Ala Val Ile Arg Leu Met Val Asn Leu Thr Gln Pro Ala Leu Leu Cys
                   85                  90                  95
   Phe Gly Asn Leu Pro Lys Glu Pro Ser Phe Arg His His Phe Leu Gln
               100                 105                 110
   Val Leu Thr Tyr Leu Gln Ala Tyr Lys Glu Ala Phe Ala Ser Glu Lys
           115                 120                 125
   Ala Phe Gly Val Leu Ser Glu Thr Leu Tyr Glu Leu Leu Gln Leu Gly
       130                 135                 140
   Trp Glu Glu Arg Gln Glu Glu Asp Asn Leu Leu Ile Glu Arg Ile Leu
   145                 150                 155                 160
   Leu Leu Val Arg Asn Ile Leu His Val Pro Ala Asp Leu Asp Gln Glu
                   165                 170                 175
   Lys Lys Ile Asp Asp Asp Ala Ser Ala His Asp Gln Leu Leu Trp Ala
               180                 185                 190
   Ile His Leu Ser Gly Leu Asp Asp Leu Leu Leu Phe Leu Ala Ser Ser
           195                 200                 205
   Ser Ala Glu Glu Gln Trp Ser Leu His Val Leu Glu Ile Val Ser Leu
       210                 215                 220
   Met Phe Arg Asp Gln Asn Pro Glu Gln Leu Ala Gly Val Gly Gln Gly
   225                 230                 235                 240
   Arg Leu Ala Gln Glu Arg Ser Ala Asp Phe Ala Glu Leu Glu Val Leu
                   245                 250                 255
   Arg Gln Arg Glu Met Ala Glu Lys Lys Thr Arg Ala Leu Gln Arg Gly
               260                 265                 270
   Asn Arg His Ser Arg Phe Gly Gly Ser Tyr Ile Val Gln Gly Leu Lys
           275                 280                 285
   Ser Ile Gly Glu Arg Asp Leu Ile Phe His Lys Gly Leu His Asn Leu
       290                 295                 300
   Arg Asn Tyr Ser Ser Asp Leu Gly Lys Gln Pro Lys Lys Val Pro Lys
   305                 310                 315                 320
   Arg Arg Gln Ala Ala Arg Glu Leu Ser Ile Gln Arg Arg Ser Ala Leu
                   325                 330                 335
   Asn Val Arg Leu Phe Leu Arg Asp Phe Cys Ser Glu Phe Leu Glu Asn
               340                 345                 350
   Cys Tyr Asn Arg Leu Met Gly Ser Val Lys Asp His Leu Leu Arg Glu
           355                 360                 365
   Lys Ala Gln Gln His Asp Glu Thr Tyr Tyr Met Trp Ala Leu Ala Phe
   370                 375                 380
   Phe Met Ala Phe Asn Arg Ala Ala Ser Phe Arg Pro Gly Leu Val Ser
   385                 390                 395                 400
   Glu Thr Leu Ser Val Arg Thr Phe His Phe Ile Glu Gln Asn Leu Thr
                   405                 410                 415
   Asn Tyr Tyr Glu Met Met Leu Thr Asp Arg Lys Glu Ala Ala Ser Trp
               420                 425                 430
   Ala Arg Arg Met His Leu Ala Leu Lys Ala Tyr Gln Glu Leu Leu Ala
           435                 440                 445
   Thr Val Asn Glu Met Asp Leu Ser Pro Asp Glu Ala Val Arg Glu Ser
       450                 455                 460
   Ser Arg Ile Ile Lys Asn Asn Ile Phe Tyr Val Met Glu Tyr Arg Glu
   465                 470                 475                 480
   Leu Phe Leu Ala Leu Phe Arg Lys Phe Asp Glu Arg Cys Gln Pro Arg
                   485                 490                 495
   Ser Phe Leu Arg Asp Leu Val Glu Thr Thr His Leu Phe Leu Lys Met
               500                 505                 510
   Leu Glu Arg Phe Cys Arg Ser Arg Gly Asn Leu Val Val Gln Asn Lys
           515                 520                 525
   Gln Lys Lys Arg Arg Lys Lys Lys Lys Lys Val Leu Asp Gln Ala Ile
       530                 535                 540
   Val Ser Gly Asn Val Pro Ser Ser Pro Glu Glu Val Glu Ala Val Trp
   545                 550                 555                 560
   Pro Ala Leu Ala Glu Gln Leu Gln Cys Cys Ala Gln Asn Ser Glu Leu
                   565                 570                 575
   Ser Met Asp Ser Val Val Pro Phe Asp Ala Ala Ser Glu Val Pro Val
               580                 585                 590
   Glu Glu Gln Arg Ala Glu Ala Met Val Arg Ile Gln Asp Cys Leu Leu
           595                 600                 605
   Ala Gly Gln Ala Pro Gln Ala Leu Thr Leu Leu Arg Ser Ala Arg Glu
       610                 615                 620
   Val Trp Pro Glu Gly Asp Val Phe Gly Ser Gln Asp Ile Ser Pro Glu
   625                 630                 635                 640
   Glu Glu Ile Gln Leu Leu Lys Gln Ile Leu Ser Ala Pro Leu Pro Arg
                   645                 650                 655
   Gln Gln Gly Pro Glu Glu Arg Gly Ala Glu Glu Glu Glu Glu Glu Glu
               660                 665                 670
   Glu Glu Glu Glu Glu Glu Leu Gln Val Val Gln Val Ser Glu Lys Glu
           675                 680                 685
   Phe Asn Phe Leu Asp Tyr Leu Lys Arg Phe Ala Cys Ser Thr Val Val
       690                 695                 700
   Arg Ala Tyr Val Leu Leu Leu Arg Ser Tyr Gln Gln Asn Ser Ala His
   705                 710                 715                 720
   Thr Asn His Cys Ile Val Lys Met Leu His Arg Leu Ala His Asp Leu
                   725                 730                 735
   Lys Met Glu Ala Leu Leu Phe Gln Leu Ser Val Phe Cys Leu Phe Asn
               740                 745                 750
   Arg Leu Leu Ser Asp Pro Ala Ala Gly Ala Tyr Lys Glu Leu Val Thr
           755                 760                 765
   Phe Ala Lys Tyr Ile Leu Gly Lys Phe Phe Ala Leu Ala Ala Val Asn
       770                 775                 780
   Gln Lys Ala Phe Val Glu Leu Leu Phe Trp Lys Asn Thr Ala Val Val
   785                 790                 795                 800
   Arg Glu Met Thr Glu Gly Tyr Gly Ser Leu Asp Asp Arg Ser Ser Ser
                   805                 810                 815
   Arg Arg Ala Pro Thr Trp Ser Pro Glu Glu Glu Ala His Leu Gln Glu
               820                 825                 830
   Leu Tyr Leu Ala Asn Lys Asp Val Glu Gly Gln Asp Val Val Glu Ala
           835                 840                 845
   Ile Leu Ala His Leu Asn Thr Val Pro Arg Thr Arg Lys Gln Ile Ile
       850                 855                 860
   His His Leu Val Gln Met Gly Leu Ala Asp Ser Val Lys Asp Phe Gln
   865                 870                 875                 880
   Arg Lys Gly Thr His Ile Val Leu Trp Thr Gly Asp Gln Glu Leu Glu
                   885                 890                 895
   Leu Gln Arg Leu Phe Glu Glu Phe Arg Asp Ser Asp Asp Val Leu Gly
               900                 905                 910
   His Ile Met Lys Asn Ile Thr Ala Lys Arg Ser Arg Ala Arg Ile Val
           915                 920                 925
   Asp Lys Leu Leu Ala Leu Gly Leu Val Ala Glu Arg Arg Glu Leu Tyr
   930 935 940
   Lys Lys Arg Gln Lys Lys Leu Ala Ser Ser Ile Leu Pro Asn Gly Ala
   945 950 955 960
   Glu Ser Leu Lys Asp Phe Cys Gln Glu Asp Leu Glu Glu Glu Glu Asn
   965 970 975
   Leu Pro Glu Glu Asp Ser Glu Glu Glu Glu Glu Gly Gly Ser Glu Ala
   980 985 990
   Glu Gln Val Gln Gly Ser Leu Val Leu Ser Asn Glu Asn Leu Gly Gln
   995 1000 1005
   Ser Leu His Gln Glu Gly Phe Ser Ile Pro Leu Leu Trp Leu Gln
   1010 1015 1020
   Asn Cys Leu Ile Arg Ala Ala Asp Asp Arg Glu Glu Asp Gly Cys
   1025 1030 1035
   Ser Gln Ala Val Pro Leu Val Pro Leu Thr Glu Glu Asn Glu Glu
   1040 1045 1050
   Ala Met Glu Asn Glu Gln Phe Gln Gln Leu Leu Arg Lys Leu Gly
   1055 1060 1065
   Val Arg Pro Pro Ala Ser Gly Gln Glu Thr Phe Trp Arg Ile Pro
   1070 1075 1080
   Ala Lys Leu Ser Pro Thr Gln Leu Arg Arg Ala Ala Ala Ser Leu
   1085 1090 1095
   Ser Gln Pro Glu Glu Glu Gln Lys Leu Gln Pro Glu Leu Gln Pro
   1100 1105 1110
   Lys Val Pro Gly Glu Gln Gly Ser Asp Glu Glu His Cys Lys Glu
   1115 1120 1125
   His Arg Ala Gln Ala Leu Arg Ala Leu Leu Leu Ala His Lys Lys
   1130 1135 1140
   Lys Ala Gly Leu Ala Ser Pro Glu Glu Glu Asp Ala Val Gly Lys
   1145 1150 1155
   Glu Pro Leu Lys Ala Ala Pro Lys Lys Arg Gln Leu Leu Asp Ser
   1160 1165 1170
   Asp Glu Glu Gln Glu Glu Asp Glu Gly Arg Asn Arg Ala Pro Glu
   1175 1180 1185
   Leu Gly Ala Pro Gly Ile Gln Lys Lys Lys Arg Tyr Gln Ile Glu
   1190 1195 1200
   Asp Asp  Glu Asp Asp
       1205

<210>  269
<211>  620
<212>  PRT
<213>  Homo sapiens

<400>  269

Met Arg Arg Ser Glu Val Leu Ala Glu Glu Ser Ile Val Cys Leu Gln
1               5                   10                  15

Lys Ala Leu Asn His Leu Arg Glu Ile Trp Glu Leu Ile Gly Ile Pro
            20                  25                  30

Glu Asp Gln Arg Leu Gln Arg Thr Glu Val Val Lys Lys His Ile Lys
        35                  40                  45

Glu Leu Leu Asp Met Met Ile Ala Glu Glu Glu Ser Leu Lys Glu Arg
    50                  55                  60

Leu Ile Lys Ser Ile Ser Val Cys Gln Lys Glu Leu Asn Thr Leu Cys
65                  70                  75                  80

Ser Glu Leu His Val Glu Pro Phe Gln Glu Glu Gly Glu Thr Thr Ile
                85                  90                  95

Leu Gln Leu Glu Lys Asp Leu Arg Thr Gln Val Glu Leu Met Arg Lys
            100                 105                 110

Gln Lys Lys Glu Arg Lys Gln Glu Leu Lys Leu Leu Gln Glu Gln Asp
        115                 120                 125

Gln Glu Leu Cys Glu Ile Leu Cys Met Pro His Tyr Asp Ile Asp Ser
    130                 135                 140

Ala Ser Val Pro Ser Leu Glu Glu Leu Asn Gln Phe Arg Gln His Val
145                 150                 155                 160

Thr Thr Leu Arg Glu Thr Lys Ala Ser Arg Arg Glu Glu Phe Val Ser
                165                 170                 175

Ile Lys Arg Gln Ile Ile Leu Cys Met Glu Glu Leu Asp His Thr Pro
            180                 185                 190

Asp Thr Ser Phe Glu Arg Asp Val Val Cys Glu Asp Glu Asp Ala Phe
        195                 200                 205

Cys Leu Ser Leu Glu Asn Ile Ala Thr Leu Gln Lys Leu Leu Arg Gln
    210                 215                 220

Leu Glu Met Gln Lys Ser Gln Asn Glu Ala Val Cys Glu Gly Leu Arg
225                 230                 235                 240

Thr Gln Ile Arg Glu Leu Trp Asp Arg Leu Gln Ile Pro Glu Glu Glu
                245                 250                 255

Arg Glu Ala Val Ala Thr Ile Met Ser Gly Ser Lys Ala Lys Val Arg
            260                 265                 270

Lys Ala Leu Gln Leu Glu Val Asp Arg Leu Glu Glu Leu Lys Met Gln
        275                 280                 285

Asn Met Lys Lys Val Ile Glu Ala Ile Arg Val Glu Leu Val Gln Tyr
    290                 295                 300

Trp Asp Gln Cys Phe Tyr Ser Gln Glu Gln Arg Gln Ala Phe Ala Pro
305                 310                 315                 320

Phe Cys Ala Glu Asp Tyr Thr Glu Ser Leu Leu Gln Leu His Asp Ala
                325                 330                 335

Glu Ile Val Arg Leu Lys Asn Tyr Tyr Glu Val His Lys Glu Leu Phe
            340                 345                 350

Glu Gly Val Gln Lys Trp Glu Glu Thr Trp Arg Leu Phe Leu Glu Phe
        355                 360                 365

Glu Arg Lys Ala Ser Asp Pro Asn Arg Phe Thr Asn Arg Gly Gly Asn
    370                 375                 380

Leu Leu Lys Glu Glu Lys Gln Arg Ala Lys Leu Gln Lys Met Leu Pro
385                 390                 395                 400

Lys Leu Glu Glu Glu Leu Lys Ala Arg Ile Glu Leu Trp Glu Gln Glu
                405                 410                 415

His Ser Lys Ala Phe Met Val Asn Gly Gln Lys Phe Met Glu Tyr Val
            420                 425                 430

Ala Glu Gln Trp Glu Met His Arg Leu Glu Lys Glu Arg Ala Lys Gln
        435                 440                 445

Glu Arg Gln Leu Lys Asn Lys Lys Gln Thr Glu Thr Glu Met Leu Tyr
    450                 455                 460

Gly Ser Ala Pro Arg Thr Pro Ser Lys Arg Arg Gly Leu Ala Pro Asn
465                 470                 475                 480

Thr Pro Gly Lys Ala Arg Lys Leu Asn Thr Thr Thr Met Ser Asn Ala
                485                 490                 495

Thr Ala Asn Ser Ser Ile Arg Pro Ile Phe Gly Gly Thr Val Tyr His
            500                 505                 510

Ser Pro Val Ser Arg Leu Pro Pro Ser Gly Ser Lys Pro Val Ala Ala
        515                 520                 525

Ser Thr Cys Ser Gly Lys Lys Thr Pro Arg Thr Gly Arg His Gly Ala
    530                 535                 540

Asn Lys Glu Asn Leu Glu Leu Asn Gly Ser Ile Leu Ser Gly Gly Tyr
545                 550                 555                 560

Pro Gly Ser Ala Pro Leu Gln Arg Asn Phe Ser Ile Asn Ser Val Ala
                565                 570                 575

Ser Thr Tyr Ser Glu Phe Ala Lys Asp Pro Ser Leu Ser Asp Ser Ser
            580                 585                 590

Thr Val Gly Leu Gln Arg Glu Leu Ser Lys Ala Ser Lys Ser Asp Ala
        595                 600                 605

Thr Ser Gly Ile Leu Asn Ser Thr Asn Ile Gln Ser
    610                 615                 620

<210>  270
<211>  606
<212>  PRT
<213>  Homo sapiens

<400>  270

Met Arg Arg Ser Glu Val Leu Ala Glu Glu Ser Ile Val Cys Leu Gln
1               5                   10                  15

Lys Ala Leu Asn His Leu Arg Glu Ile Trp Glu Leu Ile Gly Ile Pro
            20                  25                  30

Glu Asp Gln Arg Leu Gln Arg Thr Glu Val Val Lys Lys His Ile Lys
        35                  40                  45

Glu Leu Leu Asp Met Met Ile Ala Glu Glu Glu Ser Leu Lys Glu Arg
    50                  55                  60

Leu Ile Lys Ser Ile Ser Val Cys Gln Lys Glu Leu Asn Thr Leu Cys
65                  70                  75                  80

Ser Glu Leu His Val Glu Pro Phe Gln Glu Glu Gly Glu Thr Thr Ile
                85                  90                  95

Leu Gln Leu Glu Lys Asp Leu Arg Thr Gln Val Glu Leu Met Arg Lys
            100                 105                 110

Gln Lys Lys Glu Arg Lys Gln Glu Leu Lys Leu Leu Gln Glu Gln Asp
        115                 120                 125

Gln Glu Leu Cys Glu Ile Leu Cys Met Pro His Tyr Asp Ile Asp Ser
    130                 135                 140

Ala Ser Val Pro Ser Leu Glu Glu Leu Asn Gln Phe Arg Gln His Val
145                 150                 155                 160

Thr Thr Leu Arg Glu Thr Lys Ala Ser Arg Arg Glu Glu Phe Val Ser
                165                 170                 175

Ile Lys Arg Gln Ile Ile Leu Cys Met Glu Glu Leu Asp His Thr Pro
            180                 185                 190

Asp Thr Ser Phe Glu Arg Asp Val Val Cys Glu Asp Glu Asp Ala Phe
        195                 200                 205

Cys Leu Ser Leu Glu Asn Ile Ala Thr Leu Gln Lys Leu Leu Arg Gln
    210                 215                 220

Leu Glu Met Gln Lys Ser Gln Asn Glu Ala Val Cys Glu Gly Leu Arg
225                 230                 235                 240

Thr Gln Ile Arg Glu Leu Trp Asp Arg Leu Gln Ile Pro Glu Glu Glu
                245                 250                 255

Arg Glu Ala Val Ala Thr Ile Met Ser Gly Ser Lys Ala Lys Val Arg
            260                 265                 270

Lys Ala Leu Gln Leu Glu Val Asp Arg Leu Glu Glu Leu Lys Met Gln
        275                 280                 285

Asn Met Lys Lys Val Ile Glu Ala Ile Arg Val Glu Leu Val Gln Tyr
    290                 295                 300

Trp Asp Gln Cys Phe Tyr Ser Gln Glu Gln Arg Gln Ala Phe Ala Pro
305                 310                 315                 320

Phe Cys Ala Glu Asp Tyr Thr Glu Ser Leu Leu Gln Leu His Asp Ala
                325                 330                 335

Glu Ile Val Arg Leu Lys Asn Tyr Tyr Glu Val His Lys Glu Leu Phe
            340                 345                 350

Glu Gly Val Gln Lys Trp Glu Glu Thr Trp Arg Leu Phe Leu Glu Phe
        355                 360                 365

Glu Arg Lys Ala Ser Asp Pro Asn Arg Phe Thr Asn Arg Gly Gly Asn
    370                 375                 380

Leu Leu Lys Glu Glu Lys Gln Arg Ala Lys Leu Gln Lys Met Leu Pro
385                 390                 395                 400

Lys Leu Glu Glu Glu Leu Lys Ala Arg Ile Glu Leu Trp Glu Gln Glu
                405                 410                 415

His Ser Lys Ala Phe Met Val Asn Gly Gln Lys Phe Met Glu Tyr Val

420                 425                 430

Ala Glu Gln Trp Glu Met His Arg Leu Glu Lys Glu Arg Ala Lys Gln
        435                 440                 445

Glu Arg Gln Leu Lys Asn Lys Lys Gln Thr Glu Thr Glu Met Leu Tyr
    450                 455                 460

Gly Ser Ala Pro Arg Thr Pro Ser Lys Arg Arg Gly Leu Ala Pro Asn
465                 470                 475                 480

Thr Pro Gly Lys Ala Arg Lys Leu Asn Thr Thr Thr Met Ser Asn Ala
                485                 490                 495

Thr Ala Asn Ser Ser Ile Arg Pro Ile Phe Gly Gly Thr Val Tyr His
            500                 505                 510

Ser Pro Val Ser Arg Leu Pro Pro Ser Gly Ser Lys Pro Val Ala Ala
        515                 520                 525

Ser Thr Cys Ser Gly Lys Lys Thr Pro Arg Thr Gly Arg His Gly Ala
    530                 535                 540

Asn Lys Glu Asn Leu Glu Leu Asn Gly Ser Ile Leu Ser Gly Gly Tyr
545                 550                 555                 560

Pro Gly Ser Ala Pro Leu Gln Arg Asn Phe Ser Ile Asn Ser Val Ala
                565                 570                 575

Ser Thr Tyr Ser Glu Phe Ala Arg Glu Leu Ser Lys Ala Ser Lys Ser
            580                 585                 590

Asp Ala Thr Ser Gly Ile Leu Asn Ser Thr Asn Ile Gln Ser
        595                 600                 605

<210>  271
<211>  566
<212>  PRT
<213>  Homo sapiens

<400>  271

Met Arg Arg Ser Glu Val Leu Ala Glu Glu Ser Ile Val Cys Leu Gln
1               5                   10                  15

Lys Ala Leu Asn His Leu Arg Glu Ile Trp Glu Leu Ile Gly Ile Pro
            20                  25                  30

Glu Asp Gln Arg Leu Gln Arg Thr Glu Val Val Lys Lys His Ile Lys
        35                  40                  45

Glu Leu Leu Asp Met Met Ile Ala Glu Glu Glu Ser Leu Lys Glu Arg
    50                  55                  60

Leu Ile Lys Ser Ile Ser Val Cys Gln Lys Glu Leu Asn Thr Leu Cys
65                  70                  75                  80

Ser Glu Leu His Val Glu Pro Phe Gln Glu Glu Gly Glu Thr Thr Ile
                85                  90                  95

Leu Gln Leu Glu Lys Asp Leu Arg Thr Gln Val Glu Leu Met Arg Lys
            100                 105                 110

Gln Lys Lys Glu Arg Lys Gln Glu Leu Lys Leu Leu Gln Glu Gln Asp
        115                 120                 125

Gln Glu Leu Cys Glu Ile Leu Cys Met Pro His Tyr Asp Ile Asp Ser
    130                 135                 140

Ala Ser Val Pro Ser Leu Glu Glu Leu Asn Gln Phe Arg Gln His Val
145                 150                 155                 160

Thr Thr Leu Arg Glu Thr Lys Ala Ser Arg Arg Glu Glu Phe Val Ser
                165                 170                 175

Ile Lys Arg Gln Ile Ile Leu Cys Met Glu Glu Leu Asp His Thr Pro
            180                 185                 190

Asp Thr Ser Phe Glu Arg Asp Val Val Cys Glu Asp Glu Asp Ala Phe
        195                 200                 205

Cys Leu Ser Leu Glu Asn Ile Ala Thr Leu Gln Lys Leu Leu Arg Gln
    210                 215                 220

Leu Glu Met Gln Lys Ser Gln Asn Glu Ala Val Cys Glu Gly Leu Arg
225                 230                 235                 240

Thr Gln Ile Arg Glu Leu Trp Asp Arg Leu Gln Ile Pro Glu Glu Glu
                245                 250                 255

Arg Glu Ala Val Ala Thr Ile Met Ser Gly Ser Lys Ala Lys Val Arg
            260                 265                 270

Lys Ala Leu Gln Leu Glu Val Asp Arg Leu Glu Glu Leu Lys Met Gln
        275                 280                 285

Asn Met Lys Lys Val Ile Glu Ala Ile Arg Val Glu Leu Val Gln Tyr
    290                 295                 300

Trp Asp Gln Cys Phe Tyr Ser Gln Glu Gln Arg Gln Ala Phe Ala Pro
305                 310                 315                 320

Phe Cys Ala Glu Asp Tyr Thr Glu Ser Leu Leu Gln Leu His Asp Ala
                325                 330                 335

Glu Ile Val Arg Leu Lys Asn Tyr Tyr Glu Val His Lys Glu Leu Phe
            340                 345                 350

Glu Gly Val Gln Lys Trp Glu Glu Thr Trp Arg Leu Phe Leu Glu Phe
        355                 360                 365

Glu Arg Lys Ala Ser Asp Pro Asn Arg Phe Thr Asn Arg Gly Gly Asn
    370                 375                 380

Leu Leu Lys Glu Glu Lys Gln Arg Ala Lys Leu Gln Lys Met Leu Pro
385                 390                 395                 400

Lys Leu Glu Glu Glu Leu Lys Ala Arg Ile Glu Leu Trp Glu Gln Glu
                405                 410                 415

His Ser Lys Ala Phe Met Val Asn Gly Gln Lys Phe Met Glu Tyr Val
            420                 425                 430

Ala Glu Gln Trp Glu Met His Arg Leu Glu Lys Glu Arg Ala Lys Gln
        435                 440                 445

Glu Arg Gln Leu Lys Asn Lys Lys Gln Thr Glu Thr Glu Met Leu Tyr
    450                 455                 460

Gly Ser Ala Pro Arg Thr Pro Ser Lys Arg Arg Gly Leu Ala Pro Asn
465                 470                 475                 480

Thr Pro Gly Lys Ala Arg Lys Leu Asn Thr Thr Thr Met Ser Asn Ala
                485                 490                 495

Thr Ala Asn Ser Ser Ile Arg Pro Ile Phe Gly Gly Thr Val Tyr His
            500                 505                 510

Ser Pro Val Ser Arg Leu Pro Pro Ser Gly Ser Lys Pro Val Ala Ala
        515                 520                 525

Ser Thr Cys Ser Gly Lys Lys Thr Pro Arg Thr Gly Arg His Gly Ala
    530                 535                 540

Asn Lys Glu Asn Leu Glu Leu Asn Gly Ser Ile Leu Ser Ala Arg Thr
545                 550                 555                 560

Phe Lys Gly Phe Gln Ile
                565

<210>  272
<211>  398
<212>  PRT
<213>  Homo sapiens

<400>  272

Met Ala Leu Leu Arg Arg Pro Thr Val Ser Ser Asp Leu Glu Asn Ile
1               5                   10                  15

Asp Thr Gly Val Asn Ser Lys Val Lys Ser His Val Thr Ile Arg Arg

20                  25                  30

Thr Val Leu Glu Glu Ile Gly Asn Arg Val Thr Thr Arg Ala Ala Gln
        35                  40                  45

Val Ala Lys Lys Ala Gln Asn Thr Lys Val Pro Val Gln Pro Thr Lys
    50                  55                  60

Thr Thr Asn Val Asn Lys Gln Leu Lys Pro Thr Ala Ser Val Lys Pro
65                  70                  75                  80

Val Gln Met Glu Lys Leu Ala Pro Lys Gly Pro Ser Pro Thr Pro Glu
                85                  90                  95

Asp Val Ser Met Lys Glu Glu Asn Leu Cys Gln Ala Phe Ser Asp Ala
            100                 105                 110

Leu Leu Cys Lys Ile Glu Asp Ile Asp Asn Glu Asp Trp Glu Asn Pro
        115                 120                 125

Gln Leu Cys Ser Asp Tyr Val Lys Asp Ile Tyr Gln Tyr Leu Arg Gln
    130                 135                 140

Leu Glu Val Leu Gln Ser Ile Asn Pro His Phe Leu Asp Gly Arg Asp
145                 150                 155                 160

Ile Asn Gly Arg Met Arg Ala Ile Leu Val Asp Trp Leu Val Gln Val
                165                 170                 175

His Ser Lys Phe Arg Leu Leu Gln Glu Thr Leu Tyr Met Cys Val Gly
            180                 185                 190

Ile Met Asp Arg Phe Leu Gln Val Gln Pro Val Ser Arg Lys Lys Leu
        195                 200                 205

Gln Leu Val Gly Ile Thr Ala Leu Leu Leu Ala Ser Lys Tyr Glu Glu
    210                 215                 220

Met Phe Ser Pro Asn Ile Glu Asp Phe Val Tyr Ile Thr Asp Asn Ala
225                 230                 235                 240

Tyr Thr Ser Ser Gln Ile Arg Glu Met Glu Thr Leu Ile Leu Lys Glu
                245                 250                 255

Leu Lys Phe Glu Leu Gly Arg Pro Leu Pro Leu His Phe Leu Arg Arg
            260                 265                 270

Ala Ser Lys Ala Gly Glu Val Asp Val Glu Gln His Thr Leu Ala Lys
        275                 280                 285

Tyr Leu Met Glu Leu Thr Leu Ile Asp Tyr Asp Met Val His Tyr His
    290                 295                 300

Pro Ser Lys Val Ala Ala Ala Ala Ser Cys Leu Ser Gln Lys Val Leu
305                 310                 315                 320

Gly Gln Gly Lys Trp Asn Leu Lys Gln Gln Tyr Tyr Thr Gly Tyr Thr
                325                 330                 335

Glu Asn Glu Val Leu Glu Val Met Gln His Met Ala Lys Asn Val Val
            340                 345                 350

Lys Val Asn Glu Asn Leu Thr Lys Phe Ile Ala Ile Lys Asn Lys Tyr
        355                 360                 365

Ala Ser Ser Lys Leu Leu Lys Ile Ser Met Ile Pro Gln Leu Asn Ser
    370                 375                 380

Lys Ala Val Lys Asp Leu Ala Ser Pro Leu Ile Gly Arg Ser
385                 390                 395

<210>  273
<211>  803
<212>  PRT
<213>  Homo sapiens

<400>  273

Met Gly Ile Gln Gly Leu Leu Gln Phe Ile Lys Glu Ala Ser Glu Pro
1               5                   10                  15

Ile His Val Arg Lys Tyr Lys Gly Gln Val Val Ala Val Asp Thr Tyr
            20                  25                  30

Cys Trp Leu His Lys Gly Ala Ile Ala Cys Ala Glu Lys Leu Ala Lys
        35                  40                  45

Gly Glu Pro Thr Asp Arg Tyr Val Gly Phe Cys Met Lys Phe Val Asn
    50                  55                  60

Met Leu Leu Ser His Gly Ile Lys Pro Ile Leu Val Phe Asp Gly Cys
65                  70                  75                  80

Thr Leu Pro Ser Lys Lys Glu Val Glu Arg Ser Arg Arg Glu Arg Arg
                85                  90                  95

Gln Ala Asn Leu Leu Lys Gly Lys Gln Leu Leu Arg Glu Gly Lys Val
            100                 105                 110

Ser Glu Ala Arg Glu Cys Phe Thr Arg Ser Ile Asn Ile Thr His Ala
        115                 120                 125

Met Ala His Lys Val Ile Lys Ala Ala Arg Ser Gln Gly Val Asp Cys
    130                 135                 140

Leu Val Ala Pro Tyr Glu Ala Asp Ala Gln Leu Ala Tyr Leu Asn Lys
145                 150                 155                 160

Ala Gly Ile Val Gln Ala Ile Ile Thr Glu Asp Ser Asp Leu Leu Ala
                165                 170                 175

Phe Gly Cys Lys Lys Val Ile Leu Lys Met Asp Gln Phe Gly Asn Gly
            180                 185                 190

Leu Glu Ile Asp Gln Ala Arg Leu Gly Met Cys Arg Gln Leu Gly Asp
        195                 200                 205

Val Phe Thr Glu Glu Lys Phe Arg Tyr Met Cys Ile Leu Ser Gly Cys
    210                 215                 220

Asp Tyr Leu Ser Ser Leu Arg Gly Ile Gly Leu Ala Lys Ala Cys Lys
225                 230                 235                 240

Val Leu Arg Leu Ala Asn Asn Pro Asp Ile Val Lys Val Ile Lys Lys
                245                 250                 255

Ile Gly His Tyr Leu Lys Met Asn Ile Thr Val Pro Glu Asp Tyr Ile
            260                 265                 270

Asn Gly Phe Ile Arg Ala Asn Asn Thr Phe Leu Tyr Gln Leu Val Phe
        275                 280                 285

Asp Pro Ile Lys Arg Lys Leu Ile Pro Leu Asn Ala Tyr Glu Asp Asp
    290                 295                 300

Val Asp Pro Glu Thr Leu Ser Tyr Ala Gly Gln Tyr Val Asp Asp Ser
305                 310                 315                 320

Ile Ala Leu Gln Ile Ala Leu Gly Asn Lys Asp Ile Asn Thr Phe Glu
                325                 330                 335

Gln Ile Asp Asp Tyr Asn Pro Asp Thr Ala Met Pro Ala His Ser Arg
            340                 345                 350

Ser His Ser Trp Asp Asp Lys Thr Cys Gln Lys Ser Ala Asn Val Ser
        355                 360                 365

Ser Ile Trp His Arg Asn Tyr Ser Pro Arg Pro Glu Ser Gly Thr Val
    370                 375                 380

Ser Asp Ala Pro Gln Leu Lys Glu Asn Pro Ser Thr Val Gly Val Glu
385                 390                 395                 400

Arg Val Ile Ser Thr Lys Gly Leu Asn Leu Pro Arg Lys Ser Ser Ile
                405                 410                 415

Val Lys Arg Pro Arg Ser Ala Glu Leu Ser Glu Asp Asp Leu Leu Ser
            420                 425                 430

Gln Tyr Ser Leu Ser Phe Thr Lys Lys Thr Lys Lys Asn Ser Ser Glu
        435                 440                 445

Gly Asn Lys Ser Leu Ser Phe Ser Glu Val Phe Val Pro Asp Leu Val
    450                 455                 460

Asn Gly Pro Thr Asn Lys Lys Ser Val Ser Thr Pro Pro Arg Thr Arg
465                 470                 475                 480

Asn Lys Phe Ala Thr Phe Leu Gln Arg Lys Asn Glu Glu Ser Gly Ala
                485                 490                 495

Val Val Val Pro Gly Thr Arg Ser Arg Phe Phe Cys Ser Ser Asp Ser
            500                 505                 510

Thr Asp Cys Val Ser Asn Lys Val Ser Ile Gln Pro Leu Asp Glu Thr
        515                 520                 525

Ala Val Thr Asp Lys Glu Asn Asn Leu His Glu Ser Glu Tyr Gly Asp
    530                 535                 540

Gln Glu Gly Lys Arg Leu Val Asp Thr Asp Val Ala Arg Asn Ser Ser
545                 550                 555                 560

Asp Asp Ile Pro Asn Asn His Ile Pro Gly Asp His Ile Pro Asp Lys
                565                 570                 575

Ala Thr Val Phe Thr Asp Glu Glu Ser Tyr Ser Phe Glu Ser Ser Lys
            580                 585                 590

Phe Thr Arg Thr Ile Ser Pro Pro Thr Leu Gly Thr Leu Arg Ser Cys
        595                 600                 605

Phe Ser Trp Ser Gly Gly Leu Gly Asp Phe Ser Arg Thr Pro Ser Pro
    610                 615                 620

Ser Pro Ser Thr Ala Leu Gln Gln Phe Arg Arg Lys Ser Asp Ser Pro
625                 630                 635                 640

Thr Ser Leu Pro Glu Asn Asn Met Ser Asp Val Ser Gln Leu Lys Ser
                645                 650                 655

Glu Glu Ser Ser Asp Asp Glu Ser His Pro Leu Arg Glu Gly Ala Cys
            660                 665                 670

Ser Ser Gln Ser Gln Glu Ser Gly Glu Phe Ser Leu Gln Ser Ser Asn
        675                 680                 685

Ala Ser Lys Leu Ser Gln Cys Ser Ser Lys Asp Ser Asp Ser Glu Glu
    690                 695                 700

Ser Asp Cys Asn Ile Lys Leu Leu Asp Ser Gln Ser Asp Gln Thr Ser
705                 710                 715                 720

Lys Leu Cys Leu Ser His Phe Ser Lys Lys Asp Thr Pro Leu Arg Asn
                725                 730                 735

Lys Val Pro Gly Leu Tyr Lys Ser Ser Ser Ala Asp Ser Leu Ser Thr
            740                 745                 750

Thr Lys Ile Lys Pro Leu Gly Pro Ala Arg Ala Ser Gly Leu Ser Lys
        755                 760                 765

Lys Pro Ala Ser Ile Gln Lys Arg Lys His His Asn Ala Glu Asn Lys
    770                 775                 780

Pro Gly Leu Gln Ile Lys Leu Asn Glu Leu Trp Lys Asn Phe Gly Phe
785                 790                 795                 800

Lys Lys Phe

<210>  274
<211>  846
<212>  PRT
<213>  Homo sapiens

<400>  274

Met Gly Ile Gln Gly Leu Leu Gln Phe Ile Lys Glu Ala Ser Glu Pro
1               5                   10                  15

Ile His Val Arg Lys Tyr Lys Gly Gln Val Val Ala Val Asp Thr Tyr
            20                  25                  30

Cys Trp Leu His Lys Gly Ala Ile Ala Cys Ala Glu Lys Leu Ala Lys
        35                  40                  45

Gly Glu Pro Thr Asp Arg Tyr Val Gly Phe Cys Met Lys Phe Val Asn
    50                  55                  60

Met Leu Leu Ser His Gly Ile Lys Pro Ile Leu Val Phe Asp Gly Cys
65                  70                  75                  80

Thr Leu Pro Ser Lys Lys Glu Val Glu Arg Ser Arg Arg Glu Arg Arg
                85                  90                  95

Gln Ala Asn Leu Leu Lys Gly Lys Gln Leu Leu Arg Glu Gly Lys Val
            100                 105                 110

Ser Glu Ala Arg Glu Cys Phe Thr Arg Ser Ile Asn Ile Thr His Ala
        115                 120                 125

Met Ala His Lys Val Ile Lys Ala Ala Arg Ser Gln Gly Val Asp Cys
    130                 135                 140

Leu Val Ala Pro Tyr Glu Ala Asp Ala Gln Leu Ala Tyr Leu Asn Lys

145                 150                 155                 160

Ala Gly Ile Val Gln Ala Ile Ile Thr Glu Asp Ser Asp Leu Leu Ala
                165                 170                 175

Phe Gly Cys Lys Lys Val Ile Leu Lys Met Asp Gln Phe Gly Asn Gly
            180                 185                 190

Leu Glu Ile Asp Gln Ala Arg Leu Gly Met Cys Arg Gln Leu Gly Asp
        195                 200                 205

Val Phe Thr Glu Glu Lys Phe Arg Tyr Met Cys Ile Leu Ser Gly Cys
    210                 215                 220

Asp Tyr Leu Ser Ser Leu Arg Gly Ile Gly Leu Ala Lys Ala Cys Lys
225                 230                 235                 240

Val Leu Arg Leu Ala Asn Asn Pro Asp Ile Val Lys Val Ile Lys Lys
                245                 250                 255

Ile Gly His Tyr Leu Lys Met Asn Ile Thr Val Pro Glu Asp Tyr Ile
            260                 265                 270

Asn Gly Phe Ile Arg Ala Asn Asn Thr Phe Leu Tyr Gln Leu Val Phe
        275                 280                 285

Asp Pro Ile Lys Arg Lys Leu Ile Pro Leu Asn Ala Tyr Glu Asp Asp
    290                 295                 300

Val Asp Pro Glu Thr Leu Ser Tyr Ala Gly Gln Tyr Val Asp Asp Ser
305                 310                 315                 320

Ile Ala Leu Gln Ile Ala Leu Gly Asn Lys Asp Ile Asn Thr Phe Glu
                325                 330                 335

Gln Ile Asp Asp Tyr Asn Pro Asp Thr Ala Met Pro Ala His Ser Arg
            340                 345                 350

Ser His Ser Trp Asp Asp Lys Thr Cys Gln Lys Ser Ala Asn Val Ser
        355                 360                 365

Ser Ile Trp His Arg Asn Tyr Ser Pro Arg Pro Glu Ser Gly Thr Val
    370                 375                 380

Ser Asp Ala Pro Gln Leu Lys Glu Asn Pro Ser Thr Val Gly Val Glu
385                 390                 395                 400

Arg Val Ile Ser Thr Lys Gly Leu Asn Leu Pro Arg Lys Ser Ser Ile
                405                 410                 415

Val Lys Arg Pro Arg Ser Ala Glu Leu Ser Glu Asp Asp Leu Leu Ser
            420                 425                 430

Gln Tyr Ser Leu Ser Phe Thr Lys Lys Thr Lys Lys Asn Ser Ser Glu
        435                 440                 445

Gly Asn Lys Ser Leu Ser Phe Ser Glu Val Phe Val Pro Asp Leu Val
    450                 455                 460

Asn Gly Pro Thr Asn Lys Lys Ser Val Ser Thr Pro Pro Arg Thr Arg
465                 470                 475                 480

Asn Lys Phe Ala Thr Phe Leu Gln Arg Lys Asn Glu Glu Ser Gly Ala
                485                 490                 495

Val Val Val Pro Gly Thr Arg Ser Arg Phe Phe Cys Ser Ser Asp Ser
            500                 505                 510

Thr Asp Cys Val Ser Asn Lys Val Ser Ile Gln Pro Leu Asp Glu Thr
        515                 520                 525

Ala Val Thr Asp Lys Glu Asn Asn Leu His Glu Ser Glu Tyr Gly Asp
    530                 535                 540

Gln Glu Gly Lys Arg Leu Val Asp Thr Asp Val Ala Arg Asn Ser Ser
545                 550                 555                 560

Asp Asp Ile Pro Asn Asn His Ile Pro Gly Asp His Ile Pro Asp Lys
                565                 570                 575

Ala Thr Val Phe Thr Asp Glu Glu Ser Tyr Ser Phe Glu Ser Ser Lys
            580                 585                 590

Phe Thr Arg Thr Ile Ser Pro Pro Thr Leu Gly Thr Leu Arg Ser Cys
        595                 600                 605

Phe Ser Trp Ser Gly Gly Leu Gly Asp Phe Ser Arg Thr Pro Ser Pro
    610                 615                 620

Ser Pro Ser Thr Ala Leu Gln Gln Phe Arg Arg Lys Ser Asp Ser Pro
625                 630                 635                 640

Thr Ser Leu Pro Glu Asn Asn Met Ser Asp Val Ser Gln Leu Lys Ser
                645                 650                 655

Glu Glu Ser Ser Asp Asp Glu Ser His Pro Leu Arg Glu Gly Ala Cys
            660                 665                 670

Ser Ser Gln Ser Gln Glu Ser Gly Glu Phe Ser Leu Gln Ser Ser Asn
        675                 680                 685

Ala Ser Lys Leu Ser Gln Cys Ser Ser Lys Asp Ser Asp Ser Glu Glu
    690                 695                 700

Ser Asp Cys Asn Ile Lys Leu Leu Asp Ser Gln Ser Asp Gln Thr Ser

705                 710                 715                 720

Lys Leu Cys Leu Ser His Phe Ser Lys Lys Asp Thr Pro Leu Arg Asn
                725                 730                 735

Lys Val Pro Gly Leu Tyr Lys Ser Ser Ser Ala Asp Ser Leu Ser Thr
            740                 745                 750

Thr Lys Ile Lys Pro Leu Gly Pro Ala Arg Ala Ser Gly Leu Ser Lys
        755                 760                 765

Lys Pro Ala Ser Ile Gln Lys Arg Lys His His Asn Ala Glu Asn Lys
    770                 775                 780

Pro Gly Leu Gln Ile Lys Leu Asn Glu Leu Trp Lys Asn Phe Gly Phe
785                 790                 795                 800

Lys Lys Asp Ser Glu Lys Leu Pro Pro Cys Lys Lys Pro Leu Ser Pro
                805                 810                 815

Val Arg Asp Asn Ile Gln Leu Thr Pro Glu Ala Glu Glu Asp Ile Phe
            820                 825                 830

Asn Lys Pro Glu Cys Gly Arg Val Gln Arg Ala Ile Phe Gln
        835                 840                 845

<210>  275
<211>  846
<212>  PRT
<213>  Homo sapiens

<400>  275

Met Gly Ile Gln Gly Leu Leu Gln Phe Ile Lys Glu Ala Ser Glu Pro
1               5                   10                  15

Ile His Val Arg Lys Tyr Lys Gly Gln Val Val Ala Val Asp Thr Tyr
            20                  25                  30

Cys Trp Leu His Lys Gly Ala Ile Ala Cys Ala Glu Lys Leu Ala Lys
        35                  40                  45

Gly Glu Pro Thr Asp Arg Tyr Val Gly Phe Cys Met Lys Phe Val Asn
    50                  55                  60

Met Leu Leu Ser His Gly Ile Lys Pro Ile Leu Val Phe Asp Gly Cys
65                  70                  75                  80

Thr Leu Pro Ser Lys Lys Glu Val Glu Arg Ser Arg Arg Glu Arg Arg
                85                  90                  95

Gln Ala Asn Leu Leu Lys Gly Lys Gln Leu Leu Arg Glu Gly Lys Val
            100                 105                 110

Ser Glu Ala Arg Glu Cys Phe Thr Arg Ser Ile Asn Ile Thr His Ala
        115                 120                 125

Met Ala His Lys Val Ile Lys Ala Ala Arg Ser Gln Gly Val Asp Cys
    130                 135                 140

Leu Val Ala Pro Tyr Glu Ala Asp Ala Gln Leu Ala Tyr Leu Asn Lys
145                 150                 155                 160

Ala Gly Ile Val Gln Ala Ile Ile Thr Glu Asp Ser Asp Leu Leu Ala
                165                 170                 175

Phe Gly Cys Lys Lys Val Ile Leu Lys Met Asp Gln Phe Gly Asn Gly
            180                 185                 190

Leu Glu Ile Asp Gln Ala Arg Leu Gly Met Cys Arg Gln Leu Gly Asp
        195                 200                 205

Val Phe Thr Glu Glu Lys Phe Arg Tyr Met Cys Ile Leu Ser Gly Cys
    210                 215                 220

Asp Tyr Leu Ser Ser Leu Arg Gly Ile Gly Leu Ala Lys Ala Cys Lys
225                 230                 235                 240

Val Leu Arg Leu Ala Asn Asn Pro Asp Ile Val Lys Val Ile Lys Lys
                245                 250                 255

Ile Gly His Tyr Leu Lys Met Asn Ile Thr Val Pro Glu Asp Tyr Ile
            260                 265                 270

Asn Gly Phe Ile Arg Ala Asn Asn Thr Phe Leu Tyr Gln Leu Val Phe
        275                 280                 285

Asp Pro Ile Lys Arg Lys Leu Ile Pro Leu Asn Ala Tyr Glu Asp Asp
    290                 295                 300

Val Asp Pro Glu Thr Leu Ser Tyr Ala Gly Gln Tyr Val Asp Asp Ser
305                 310                 315                 320

Ile Ala Leu Gln Ile Ala Leu Gly Asn Lys Asp Ile Asn Thr Phe Glu
                325                 330                 335

Gln Ile Asp Asp Tyr Asn Pro Asp Thr Ala Met Pro Ala His Ser Arg
            340                 345                 350

Ser His Ser Trp Asp Asp Lys Thr Cys Gln Lys Ser Ala Asn Val Ser
        355                 360                 365

Ser Ile Trp His Arg Asn Tyr Ser Pro Arg Pro Glu Ser Gly Thr Val
    370                 375                 380

Ser Asp Ala Pro Gln Leu Lys Glu Asn Pro Ser Thr Val Gly Val Glu
385                 390                 395                 400

Arg Val Ile Ser Thr Lys Gly Leu Asn Leu Pro Arg Lys Ser Ser Ile
                405                 410                 415

Val Lys Arg Pro Arg Ser Ala Glu Leu Ser Glu Asp Asp Leu Leu Ser
            420                 425                 430

Gln Tyr Ser Leu Ser Phe Thr Lys Lys Thr Lys Lys Asn Ser Ser Glu
        435                 440                 445

Gly Asn Lys Ser Leu Ser Phe Ser Glu Val Phe Val Pro Asp Leu Val
    450                 455                 460

Asn Gly Pro Thr Asn Lys Lys Ser Val Ser Thr Pro Pro Arg Thr Arg
465                 470                 475                 480

Asn Lys Phe Ala Thr Phe Leu Gln Arg Lys Asn Glu Glu Ser Gly Ala
                485                 490                 495

Val Val Val Pro Gly Thr Arg Ser Arg Phe Phe Cys Ser Ser Asp Ser
            500                 505                 510

Thr Asp Cys Val Ser Asn Lys Val Ser Ile Gln Pro Leu Asp Glu Thr
        515                 520                 525

Ala Val Thr Asp Lys Glu Asn Asn Leu His Glu Ser Glu Tyr Gly Asp
    530                 535                 540

Gln Glu Gly Lys Arg Leu Val Asp Thr Asp Val Ala Arg Asn Ser Ser
545                 550                 555                 560

Asp Asp Ile Pro Asn Asn His Ile Pro Gly Asp His Ile Pro Asp Lys
                565                 570                 575

Ala Thr Val Phe Thr Asp Glu Glu Ser Tyr Ser Phe Glu Ser Ser Lys
            580                 585                 590

Phe Thr Arg Thr Ile Ser Pro Pro Thr Leu Gly Thr Leu Arg Ser Cys
        595                 600                 605

Phe Ser Trp Ser Gly Gly Leu Gly Asp Phe Ser Arg Thr Pro Ser Pro
    610                 615                 620

Ser Pro Ser Thr Ala Leu Gln Gln Phe Arg Arg Lys Ser Asp Ser Pro
625                 630                 635                 640

Thr Ser Leu Pro Glu Asn Asn Met Ser Asp Val Ser Gln Leu Lys Ser
                645                 650                 655

Glu Glu Ser Ser Asp Asp Glu Ser His Pro Leu Arg Glu Gly Ala Cys
            660                 665                 670

Ser Ser Gln Ser Gln Glu Ser Gly Glu Phe Ser Leu Gln Ser Ser Asn
        675                 680                 685

Ala Ser Lys Leu Ser Gln Cys Ser Ser Lys Asp Ser Asp Ser Glu Glu
    690                 695                 700

Ser Asp Cys Asn Ile Lys Leu Leu Asp Ser Gln Ser Asp Gln Thr Ser
705                 710                 715                 720

Lys Leu Cys Leu Ser His Phe Ser Lys Lys Asp Thr Pro Leu Arg Asn
                725                 730                 735

Lys Val Pro Gly Leu Tyr Lys Ser Ser Ser Ala Asp Ser Leu Ser Thr
            740                 745                 750

Thr Lys Ile Lys Pro Leu Gly Pro Ala Arg Ala Ser Gly Leu Ser Lys
        755                 760                 765

Lys Pro Ala Ser Ile Gln Lys Arg Lys His His Asn Ala Glu Asn Lys
    770                 775                 780

Pro Gly Leu Gln Ile Lys Leu Asn Glu Leu Trp Lys Asn Phe Gly Phe
785                 790                 795                 800

Lys Lys Asp Ser Glu Lys Leu Pro Pro Cys Lys Lys Pro Leu Ser Pro
                805                 810                 815

Val Arg Asp Asn Ile Gln Leu Thr Pro Glu Ala Glu Glu Asp Ile Phe
            820                 825                 830

Asn Lys Pro Glu Cys Gly Arg Val Gln Arg Ala Ile Phe Gln
        835                 840                 845

<210>  276
<211>  202
<212>  PRT
<213>  Homo sapiens

<400>  276

Met Ala Thr Leu Ile Tyr Val Asp Lys Glu Asn Gly Glu Pro Gly Thr
1               5                   10                  15

Arg Val Val Ala Lys Asp Gly Leu Lys Leu Gly Ser Gly Pro Ser Ile
            20                  25                  30

Lys Ala Leu Asp Gly Arg Ser Gln Val Ser Thr Pro Arg Phe Gly Lys
        35                  40                  45

Thr Phe Asp Ala Pro Pro Ala Leu Pro Lys Ala Thr Arg Lys Ala Leu
    50                  55                  60

Gly Thr Val Asn Arg Ala Thr Glu Lys Ser Val Lys Thr Lys Gly Pro
65                  70                  75                  80

Leu Lys Gln Lys Gln Pro Ser Phe Ser Ala Lys Lys Met Thr Glu Lys
                85                  90                  95

Thr Val Lys Ala Lys Ser Ser Val Pro Ala Ser Asp Asp Ala Tyr Pro
            100                 105                 110

Glu Ile Glu Lys Phe Phe Pro Phe Asn Pro Leu Asp Phe Glu Ser Phe
        115                 120                 125

Asp Leu Pro Glu Glu His Gln Ile Ala His Leu Pro Leu Ser Gly Val
    130                 135                 140

Pro Leu Met Ile Leu Asp Glu Glu Arg Glu Leu Glu Lys Leu Phe Gln
145                 150                 155                 160

Leu Gly Pro Pro Ser Pro Val Lys Met Pro Ser Pro Pro Trp Glu Ser
                165                 170                 175

Asn Leu Leu Gln Ser Pro Ser Ser Ile Leu Ser Thr Leu Asp Val Glu
            180                 185                 190

Leu Pro Pro Val Cys Cys Asp Ile Asp Ile
        195                 200

<210>  277
<211>  144
<212>  PRT
<213>  Homo sapiens

<400>  277

Met Ala Asp Arg Leu Thr Gln Leu Gln Asp Ala Val Asn Ser Leu Ala
1               5                   10                  15

Asp Gln Phe Cys Asn Ala Ile Gly Val Leu Gln Gln Cys Gly Pro Pro
            20                  25                  30

Ala Ser Phe Asn Asn Ile Gln Thr Ala Ile Asn Lys Asp Gln Pro Ala
        35                  40                  45

Asn Pro Thr Glu Glu Tyr Ala Gln Leu Phe Ala Ala Leu Ile Ala Arg
    50                  55                  60

Thr Ala Lys Asp Ile Asp Val Leu Ile Asp Ser Leu Pro Ser Glu Glu
65                  70                  75                  80

Ser Thr Ala Ala Leu Gln Ala Ala Ser Leu Tyr Lys Leu Glu Glu Glu
                85                  90                  95

Asn His Glu Ala Ala Thr Cys Leu Glu Asp Val Val Tyr Arg Gly Asp
            100                 105                 110

Met Leu Leu Glu Lys Ile Gln Ser Ala Leu Ala Asp Ile Ala Gln Ser
        115                 120                 125

Gln Leu Lys Thr Arg Ser Gly Thr His Ser Gln Ser Leu Pro Asp Ser
    130                 135                 140

<210>  278
<211>  856
<212>  PRT
<213>  Homo sapiens

<400>  278

Met Lys Ser Ala Arg Ala Lys Thr Pro Arg Lys Pro Thr Val Lys Lys
1               5                   10                  15

Gly Ser Gln Thr Asn Leu Lys Asp Pro Val Gly Val Tyr Cys Arg Val
            20                  25                  30

Arg Pro Leu Gly Phe Pro Asp Gln Glu Cys Cys Ile Glu Val Ile Asn
        35                  40                  45

Asn Thr Thr Val Gln Leu His Thr Pro Glu Gly Tyr Arg Leu Asn Arg
    50                  55                  60

Asn Gly Asp Tyr Lys Glu Thr Gln Tyr Ser Phe Lys Gln Val Phe Gly
65                  70                  75                  80

Thr His Thr Thr Gln Lys Glu Leu Phe Asp Val Val Ala Asn Pro Leu
                85                  90                  95

Val Asn Asp Leu Ile His Gly Lys Asn Gly Leu Leu Phe Thr Tyr Gly
            100                 105                 110

Val Thr Gly Ser Gly Lys Thr His Thr Met Thr Gly Ser Pro Gly Glu
        115                 120                 125

Gly Gly Leu Leu Pro Arg Cys Leu Asp Met Ile Phe Asn Ser Ile Gly
    130                 135                 140

Ser Phe Gln Ala Lys Arg Tyr Val Phe Lys Ser Asn Asp Arg Asn Ser
145                 150                 155                 160

Met Asp Ile Gln Cys Glu Val Asp Ala Leu Leu Glu Arg Gln Lys Arg
                165                 170                 175

Glu Ala Met Pro Asn Pro Lys Thr Ser Ser Ser Lys Arg Gln Val Asp
            180                 185                 190

Pro Glu Phe Ala Asp Met Ile Thr Val Gln Glu Phe Cys Lys Ala Glu
        195                 200                 205

Glu Val Asp Glu Asp Ser Val Tyr Gly Val Phe Val Ser Tyr Ile Glu
    210                 215                 220

Ile Tyr Asn Asn Tyr Ile Tyr Asp Leu Leu Glu Glu Val Pro Phe Asp

225                 230                 235                 240

Pro Ile Lys Pro Lys Pro Pro Gln Ser Lys Leu Leu Arg Glu Asp Lys
                245                 250                 255

Asn His Asn Met Tyr Val Ala Gly Cys Thr Glu Val Glu Val Lys Ser
            260                 265                 270

Thr Glu Glu Ala Phe Glu Val Phe Trp Arg Gly Gln Lys Lys Arg Arg
        275                 280                 285

Ile Ala Asn Thr His Leu Asn Arg Glu Ser Ser Arg Ser His Ser Val
    290                 295                 300

Phe Asn Ile Lys Leu Val Gln Ala Pro Leu Asp Ala Asp Gly Asp Asn
305                 310                 315                 320

Val Leu Gln Glu Lys Glu Gln Ile Thr Ile Ser Gln Leu Ser Leu Val
                325                 330                 335

Asp Leu Ala Gly Ser Glu Arg Thr Asn Arg Thr Arg Ala Glu Gly Asn
            340                 345                 350

Arg Leu Arg Glu Ala Gly Asn Ile Asn Gln Ser Leu Met Thr Leu Arg
        355                 360                 365

Thr Cys Met Asp Val Leu Arg Glu Asn Gln Met Tyr Gly Thr Asn Lys
    370                 375                 380

Met Val Pro Tyr Arg Asp Ser Lys Leu Thr His Leu Phe Lys Asn Tyr
385                 390                 395                 400

Phe Asp Gly Glu Gly Lys Val Arg Met Ile Val Cys Val Asn Pro Lys
                405                 410                 415

Ala Glu Asp Tyr Glu Glu Asn Leu Gln Val Met Arg Phe Ala Glu Val
            420                 425                 430

Thr Gln Glu Val Glu Val Ala Arg Pro Val Asp Lys Ala Ile Cys Gly
        435                 440                 445

Leu Thr Pro Gly Arg Arg Tyr Arg Asn Gln Pro Arg Gly Pro Val Gly
    450                 455                 460

Asn Glu Pro Leu Val Thr Asp Val Val Leu Gln Ser Phe Pro Pro Leu
465                 470                 475                 480

Pro Ser Cys Glu Ile Leu Asp Ile Asn Asp Glu Gln Thr Leu Pro Arg
                485                 490                 495

Leu Ile Glu Ala Leu Glu Lys Arg His Asn Leu Arg Gln Met Met Ile
            500                 505                 510

Asp Glu Phe Asn Lys Gln Ser Asn Ala Phe Lys Ala Leu Leu Gln Glu
        515                 520                 525

Phe Asp Asn Ala Val Leu Ser Lys Glu Asn His Met Gln Gly Lys Leu
    530                 535                 540

Asn Glu Lys Glu Lys Met Ile Ser Gly Gln Lys Leu Glu Ile Glu Arg
545                 550                 555                 560

Leu Glu Lys Lys Asn Lys Thr Leu Glu Tyr Lys Ile Glu Ile Leu Glu
                565                 570                 575

Lys Thr Thr Thr Ile Tyr Glu Glu Asp Lys Arg Asn Leu Gln Gln Glu
            580                 585                 590

Leu Glu Thr Gln Asn Gln Lys Leu Gln Arg Gln Phe Ser Asp Lys Arg
        595                 600                 605

Arg Leu Glu Ala Arg Leu Gln Gly Met Val Thr Glu Thr Thr Met Lys
    610                 615                 620

Trp Glu Lys Glu Cys Glu Arg Arg Val Ala Ala Lys Gln Leu Glu Met
625                 630                 635                 640

Gln Asn Lys Leu Trp Val Lys Asp Glu Lys Leu Lys Gln Leu Lys Ala
                645                 650                 655

Ile Val Thr Glu Pro Lys Thr Glu Lys Pro Glu Arg Pro Ser Arg Glu
            660                 665                 670

Arg Asp Arg Glu Lys Val Thr Gln Arg Ser Val Ser Pro Ser Pro Val
        675                 680                 685

Pro Leu Leu Phe Gln Pro Asp Gln Asn Ala Pro Pro Ile Arg Leu Arg
    690                 695                 700

His Arg Arg Ser Arg Ser Ala Gly Asp Arg Trp Val Asp His Lys Pro
705                 710                 715                 720

Ala Ser Asn Met Gln Thr Glu Thr Val Met Gln Pro His Val Pro His
                725                 730                 735

Ala Ile Thr Val Ser Val Ala Asn Glu Lys Ala Leu Ala Lys Cys Glu
            740                 745                 750

Lys Tyr Met Leu Thr His Gln Glu Leu Ala Ser Asp Gly Glu Ile Glu
        755                 760                 765

Thr Lys Leu Ile Lys Gly Asp Ile Tyr Lys Thr Arg Gly Gly Gly Gln
    770                 775                 780

Ser Val Gln Phe Thr Asp Ile Glu Thr Leu Lys Gln Glu Ser Pro Asn

785                 790                 795                 800

Gly Ser Arg Lys Arg Arg Ser Ser Thr Val Ala Pro Ala Gln Pro Asp
                805                 810                 815

Gly Ala Glu Ser Glu Trp Thr Asp Val Glu Thr Arg Cys Ser Val Ala
            820                 825                 830

Val Glu Met Arg Ala Gly Ser Gln Leu Gly Pro Gly Tyr Gln His His
        835                 840                 845

Ala Gln Pro Lys Arg Lys Lys Pro
    850                 855

<210>  279
<211>  960
<212>  PRT
<213>  Homo sapiens

<400>  279

<210>  280
<211>  2120
<212>  PRT
<213>  Homo sapiens

<400>  280

Met Arg Ser Phe Lys Arg Val Asn Phe Gly Thr Leu Leu Ser Ser Gln
1               5                   10                  15

Lys Glu Ala Glu Glu Leu Leu Pro Asp Leu Lys Glu Phe Leu Ser Asn
                20                  25                  30

Pro Pro Ala Gly Phe Pro Ser Ser Arg Ser Asp Ala Glu Arg Arg Gln
        35                  40                  45

Ala Cys Asp Ala Ile Leu Arg Ala Cys Asn Gln Gln Leu Thr Ala Lys
    50                  55                  60

Leu Ala Cys Pro Arg His Leu Gly Ser Leu Leu Glu Leu Ala Glu Leu
65                  70                  75                  80

Ala Cys Asp Gly Tyr Leu Val Ser Thr Pro Gln Arg Pro Pro Leu Tyr
                85                  90                  95

Leu Glu Arg Ile Leu Phe Val Leu Leu Arg Asn Ala Ala Ala Gln Gly
            100                 105                 110

Ser Pro Glu Ala Thr Leu Arg Leu Ala Gln Pro Leu His Ala Cys Leu
        115                 120                 125

Val Gln Cys Ser Arg Glu Ala Ala Pro Gln Asp Tyr Glu Ala Val Ala
    130                 135                 140

Arg Gly Ser Phe Ser Leu Leu Trp Lys Gly Ala Glu Ala Leu Leu Glu
145                 150                 155                 160

Arg Arg Ala Ala Phe Ala Ala Arg Leu Lys Ala Leu Ser Phe Leu Val
                165                 170                 175

Leu Leu Glu Asp Glu Ser Thr Pro Cys Glu Val Pro His Phe Ala Ser
            180                 185                 190

Pro Thr Ala Cys Arg Ala Val Ala Ala His Gln Leu Phe Asp Ala Ser
        195                 200                 205

Gly His Gly Leu Asn Glu Ala Asp Ala Asp Phe Leu Asp Asp Leu Leu
    210                 215                 220

Ser Arg His Val Ile Arg Ala Leu Val Gly Glu Arg Gly Ser Ser Ser
225                 230                 235                 240

Gly Leu Leu Ser Pro Gln Arg Ala Leu Cys Leu Leu Glu Leu Thr Leu
                245                 250                 255

Glu His Cys Arg Arg Phe Cys Trp Ser Arg His His Asp Lys Ala Ile
            260                 265                 270

Ser Ala Val Glu Lys Ala His Ser Tyr Leu Arg Asn Thr Asn Leu Ala
        275                 280                 285

Pro Ser Leu Gln Leu Cys Gln Leu Gly Val Lys Leu Leu Gln Val Gly
    290                 295                 300

Glu Glu Gly Pro Gln Ala Val Ala Lys Leu Leu Ile Lys Ala Ser Ala

305                 310                 315                 320

Val Leu Ser Lys Ser Met Glu Ala Pro Ser Pro Pro Leu Arg Ala Leu
                325                 330                 335

Tyr Glu Ser Cys Gln Phe Phe Leu Ser Gly Leu Glu Arg Gly Thr Lys
            340                 345                 350

Arg Arg Tyr Arg Leu Asp Ala Ile Leu Ser Leu Phe Ala Phe Leu Gly
        355                 360                 365

Gly Tyr Cys Ser Leu Leu Gln Gln Leu Arg Asp Asp Gly Val Tyr Gly
    370                 375                 380

Gly Ser Ser Lys Gln Gln Gln Ser Phe Leu Gln Met Tyr Phe Gln Gly
385                 390                 395                 400

Leu His Leu Tyr Thr Val Val Val Tyr Asp Phe Ala Gln Gly Cys Gln
                405                 410                 415

Ile Val Asp Leu Ala Asp Leu Thr Gln Leu Val Asp Ser Cys Lys Ser
            420                 425                 430

Thr Val Val Trp Met Leu Glu Ala Leu Glu Gly Leu Ser Gly Gln Glu
        435                 440                 445

Leu Thr Asp His Met Gly Met Thr Ala Ser Tyr Thr Ser Asn Leu Ala
    450                 455                 460

Tyr Ser Phe Tyr Ser His Lys Leu Tyr Ala Glu Ala Cys Ala Ile Ser
465                 470                 475                 480

Glu Pro Leu Cys Gln His Leu Gly Leu Val Lys Pro Gly Thr Tyr Pro
                485                 490                 495

Glu Val Pro Pro Glu Lys Leu His Arg Cys Phe Arg Leu Gln Val Glu
            500                 505                 510

Ser Leu Lys Lys Leu Gly Lys Gln Ala Gln Gly Cys Lys Met Val Ile
        515                 520                 525

Leu Trp Leu Ala Ala Leu Gln Pro Cys Ser Pro Glu His Met Ala Glu
    530                 535                 540

Pro Val Thr Phe Trp Val Arg Val Lys Met Asp Ala Ala Arg Ala Gly
545                 550                 555                 560

Asp Lys Glu Leu Gln Leu Lys Thr Leu Arg Asp Ser Leu Ser Gly Trp
                565                 570                 575

Asp Pro Glu Thr Leu Ala Leu Leu Leu Arg Glu Glu Leu Gln Ala Tyr
            580                 585                 590

Lys Ala Val Arg Ala Asp Thr Gly Gln Glu Arg Phe Asn Ile Ile Cys
        595                 600                 605

Asp Leu Leu Glu Leu Ser Pro Glu Glu Thr Pro Ala Gly Ala Trp Ala
    610                 615                 620

Arg Ala Thr His Leu Val Glu Leu Ala Gln Val Leu Cys Tyr His Asp
625                 630                 635                 640

Phe Thr Gln Gln Thr Asn Cys Ser Ala Leu Asp Ala Ile Arg Glu Ala
                645                 650                 655

Leu Gln Leu Leu Asp Ser Val Arg Pro Glu Ala Gln Ala Arg Asp Gln
            660                 665                 670

Leu Leu Asp Asp Lys Ala Gln Ala Leu Leu Trp Leu Tyr Ile Cys Thr
        675                 680                 685

Leu Glu Ala Lys Ile Gln Glu Gly Ile Glu Arg Asp Arg Arg Ala Gln
    690                 695                 700

Ala Pro Gly Asn Leu Glu Glu Phe Glu Val Asn Asp Leu Asn Tyr Glu
705                 710                 715                 720

Asp Lys Leu Gln Glu Asp Arg Phe Leu Tyr Ser Asn Ile Ala Phe Asn
                725                 730                 735

Leu Ala Ala Asp Ala Ala Gln Ser Lys Cys Leu Asp Gln Ala Leu Ala
            740                 745                 750

Leu Trp Lys Glu Leu Leu Thr Lys Gly Gln Ala Pro Ala Val Arg Cys
        755                 760                 765

Leu Gln Gln Thr Ala Ala Ser Leu Gln Ile Leu Ala Ala Leu Tyr Gln
    770                 775                 780

Leu Val Ala Lys Pro Met Gln Ala Leu Glu Val Leu Leu Leu Leu Arg
785                 790                 795                 800

Ile Val Ser Glu Arg Leu Lys Asp His Ser Lys Ala Ala Gly Ser Ser
                805                 810                 815

Cys His Ile Thr Gln Leu Leu Leu Thr Leu Gly Cys Pro Ser Tyr Ala
            820                 825                 830

Gln Leu His Leu Glu Glu Ala Ala Ser Ser Leu Lys His Leu Asp Gln
        835                 840                 845

Thr Thr Asp Thr Tyr Leu Leu Leu Ser Leu Thr Cys Asp Leu Leu Arg
    850                 855                 860

Ser Gln Leu Tyr Trp Thr His Gln Lys Val Thr Lys Gly Val Ser Leu

865                 870                 875                 880

Leu Leu Ser Val Leu Arg Asp Pro Ala Leu Gln Lys Ser Ser Lys Ala
                885                 890                 895

Trp Tyr Leu Leu Arg Val Gln Val Leu Gln Leu Val Ala Ala Tyr Leu
            900                 905                 910

Ser Leu Pro Ser Asn Asn Leu Ser His Ser Leu Trp Glu Gln Leu Cys
        915                 920                 925

Ala Gln Gly Trp Gln Thr Pro Glu Ile Ala Leu Ile Asp Ser His Lys
    930                 935                 940

Leu Leu Arg Ser Ile Ile Leu Leu Leu Met Gly Ser Asp Ile Leu Ser
945                 950                 955                 960

Thr Gln Lys Ala Ala Val Glu Thr Ser Phe Leu Asp Tyr Gly Glu Asn
                965                 970                 975

Leu Val Gln Lys Trp Gln Val Leu Ser Glu Val Leu Ser Cys Ser Glu
            980                 985                 990

Lys Leu Val Cys His Leu Gly Arg  Leu Gly Ser Val Ser  Glu Ala Lys
        995                 1000                1005

Ala Phe  Cys Leu Glu Ala Leu  Lys Leu Thr Thr Lys  Leu Gln Ile
    1010                1015                1020

Pro Arg  Gln Cys Ala Leu Phe  Leu Val Leu Lys Gly  Glu Leu Glu
    1025                1030                1035

Leu Ala  Arg Asn Asp Ile Asp  Leu Cys Gln Ser Asp  Leu Gln Gln
    1040                1045                1050

Val Leu  Phe Leu Leu Glu Ser  Cys Thr Glu Phe Gly  Gly Val Thr
    1055                1060                1065

Gln His  Leu Asp Ser Val Lys  Lys Val His Leu Gln  Lys Gly Lys
    1070                1075                1080

Gln Gln  Ala Gln Val Pro Cys  Pro Pro Gln Leu Pro  Glu Glu Glu
    1085                1090                1095

Leu Phe  Leu Arg Gly Pro Ala  Leu Glu Leu Val Ala  Thr Val Ala
    1100                1105                1110

Lys Glu  Pro Gly Pro Ile Ala  Pro Ser Thr Asn Ser  Ser Pro Val
    1115                1120                1125

Leu Lys  Thr Lys Pro Gln Pro  Ile Pro Asn Phe Leu  Ser His Ser
    1130                1135                1140

Pro Thr Cys Asp Cys Ser Leu Cys Ala Ser Pro Val Leu Thr Ala
    1145                1150                1155

Val Cys Leu Arg Trp Val Leu Val Thr Ala Gly Val Arg Leu Ala
    1160                1165                1170

Met Gly His Gln Ala Gln Gly Leu Asp Leu Leu Gln Val Val Leu
    1175                1180                1185

Lys Gly Cys Pro Glu Ala Ala Glu Arg Leu Thr Gln Ala Leu Gln
    1190                1195                1200

Ala Ser Leu Asn His Lys Thr Pro Pro Ser Leu Val Pro Ser Leu
    1205                1210                1215

Leu Asp Glu Ile Leu Ala Gln Ala Tyr Thr Leu Leu Ala Leu Glu
    1220                1225                1230

Gly Leu Asn Gln Pro Ser Asn Glu Ser Leu Gln Lys Val Leu Gln
    1235                1240                1245

Ser Gly Leu Lys Phe Val Ala Ala Arg Ile Pro His Leu Glu Pro
    1250                1255                1260

Trp Arg Ala Ser Leu Leu Leu Ile Trp Ala Leu Thr Lys Leu Gly
    1265                1270                1275

Gly Leu Ser Cys Cys Thr Thr Gln Leu Phe Ala Ser Ser Trp Gly
    1280                1285                1290

Trp Gln Pro Pro Leu Ile Lys Ser Val Pro Gly Ser Glu Pro Ser
    1295                1300                1305

Lys Thr Gln Gly Gln Lys Arg Ser Gly Arg Gly Arg Gln Lys Leu
    1310                1315                1320

Ala Ser Ala Pro Leu Arg Leu Asn Asn Thr Ser Gln Lys Gly Leu
    1325                1330                1335

Glu Gly Arg Gly Leu Pro Cys Thr Pro Lys Pro Pro Asp Arg Ile
    1340                1345                1350

Arg Gln Ala Gly Pro His Val Pro Phe Thr Val Phe Glu Glu Val
    1355                1360                1365

Cys Pro Thr Glu Ser Lys Pro Glu Val Pro Gln Ala Pro Arg Val
    1370                1375                1380

Gln Gln Arg Val Gln Thr Arg Leu Lys Val Asn Phe Ser Asp Asp
    1385                1390                1395

Ser Asp Leu Glu Asp Pro Val Ser Ala Glu Ala Trp Leu Ala Glu

1400                 1405                 1410

Glu Pro Lys Arg Arg Gly Thr Ala Ser Arg Gly Arg Gly Arg Ala
    1415                 1420                 1425

Arg Lys Gly Leu Ser Leu Lys Thr Asp Ala Val Val Ala Pro Gly
    1430                 1435                 1440

Ser Ala Pro Gly Asn Pro Gly Leu Asn Gly Arg Ser Arg Arg Ala
    1445                 1450                 1455

Lys Lys Val Ala Ser Arg His Cys Glu Glu Arg Arg Pro Gln Arg
    1460                 1465                 1470

Ala Ser Asp Gln Ala Arg Pro Gly Pro Glu Ile Met Arg Thr Ile
    1475                 1480                 1485

Pro Glu Glu Glu Leu Thr Asp Asn Trp Arg Lys Met Ser Phe Glu
    1490                 1495                 1500

Ile Leu Arg Gly Ser Asp Gly Glu Asp Ser Ala Ser Gly Gly Lys
    1505                 1510                 1515

Thr Pro Ala Pro Gly Pro Glu Ala Ala Ser Gly Glu Trp Glu Leu
    1520                 1525                 1530

Leu Arg Leu Asp Ser Ser Lys Lys Lys Leu Pro Ser Pro Cys Pro
    1535                 1540                 1545

Asp Lys Glu Ser Asp Lys Asp Leu Gly Pro Arg Leu Gln Leu Pro
    1550                 1555                 1560

Ser Ala Pro Val Ala Thr Gly Leu Ser Thr Leu Asp Ser Ile Cys
    1565                 1570                 1575

Asp Ser Leu Ser Val Ala Phe Arg Gly Ile Ser His Cys Pro Pro
    1580                 1585                 1590

Ser Gly Leu Tyr Ala His Leu Cys Arg Phe Leu Ala Leu Cys Leu
    1595                 1600                 1605

Gly His Arg Asp Pro Tyr Ala Thr Ala Phe Leu Val Thr Glu Ser
    1610                 1615                 1620

Val Ser Ile Thr Cys Arg His Gln Leu Leu Thr His Leu His Arg
    1625                 1630                 1635

Gln Leu Ser Lys Ala Gln Lys His Arg Gly Ser Leu Glu Ile Ala
    1640                 1645                 1650

Asp Gln Leu Gln Gly Leu Ser Leu Gln Glu Met Pro Gly Asp Val
    1655                 1660                 1665

Pro Leu Ala Arg Ile Gln Arg Leu Phe Ser Phe Arg Ala Leu Glu
    1670                1675                1680

Ser Gly His Phe Pro Gln Pro Glu Lys Glu Ser Phe Gln Glu Arg
    1685                1690                1695

Leu Ala Leu Ile Pro Ser Gly Val Thr Val Cys Val Leu Ala Leu
    1700                1705                1710

Ala Thr Leu Gln Pro Gly Thr Val Gly Asn Thr Leu Leu Leu Thr
    1715                1720                1725

Arg Leu Glu Lys Asp Ser Pro Pro Val Ser Val Gln Ile Pro Thr
    1730                1735                1740

Gly Gln Asn Lys Leu His Leu Arg Ser Val Leu Asn Glu Phe Asp
    1745                1750                1755

Ala Ile Gln Lys Ala Gln Lys Glu Asn Ser Ser Cys Thr Asp Lys
    1760                1765                1770

Arg Glu Trp Trp Thr Gly Arg Leu Ala Leu Asp His Arg Met Glu
    1775                1780                1785

Val Leu Ile Ala Ser Leu Glu Lys Ser Val Leu Gly Cys Trp Lys
    1790                1795                1800

Gly Leu Leu Leu Pro Ser Ser Glu Glu Pro Gly Pro Ala Gln Glu
    1805                1810                1815

Ala Ser Arg Leu Gln Glu Leu Leu Gln Asp Cys Gly Trp Lys Tyr
    1820                1825                1830

Pro Asp Arg Thr Leu Leu Lys Ile Met Leu Ser Gly Ala Gly Ala
    1835                1840                1845

Leu Thr Pro Gln Asp Ile Gln Ala Leu Ala Tyr Gly Leu Cys Pro
    1850                1855                1860

Thr Gln Pro Glu Arg Ala Gln Glu Leu Leu Asn Glu Ala Val Gly
    1865                1870                1875

Arg Leu Gln Gly Leu Thr Val Pro Ser Asn Ser His Leu Val Leu
    1880                1885                1890

Val Leu Asp Lys Asp Leu Gln Lys Leu Pro Trp Glu Ser Met Pro
    1895                1900                1905

Ser Leu Gln Ala Leu Pro Val Thr Arg Leu Pro Ser Phe Arg Phe
    1910                1915                1920

Leu Leu Ser Tyr Ser Ile Ile Lys Glu Tyr Gly Ala Ser Pro Val

1925                 1930                 1935

Leu Ser Gln Gly Val Asp Pro Arg Ser Thr Phe Tyr Val Leu Asn
    1940                 1945                 1950

Pro His Asn Asn Leu Ser Ser Thr Glu Glu Gln Phe Arg Ala Asn
    1955                 1960                 1965

Phe Ser Ser Glu Ala Gly Trp Arg Gly Val Val Gly Glu Val Pro
    1970                 1975                 1980

Arg Pro Glu Gln Val Gln Glu Ala Leu Thr Lys His Asp Leu Tyr
    1985                 1990                 1995

Ile Tyr Ala Gly His Gly Ala Gly Ala Arg Phe Leu Asp Gly Gln
    2000                 2005                 2010

Ala Val Leu Arg Leu Ser Cys Arg Ala Val Ala Leu Leu Phe Gly
    2015                 2020                 2025

Cys Ser Ser Ala Ala Leu Ala Val His Gly Asn Leu Glu Gly Ala
    2030                 2035                 2040

Gly Ile Val Leu Lys Tyr Ile Met Ala Gly Cys Pro Leu Phe Leu
    2045                 2050                 2055

Gly Asn Leu Trp Asp Val Thr Asp Arg Asp Ile Asp Arg Tyr Thr
    2060                 2065                 2070

Glu Ala Leu Leu Gln Gly Trp Leu Gly Ala Gly Pro Gly Ala Pro
    2075                 2080                 2085

Leu Leu Tyr Tyr Val Asn Gln Ala Arg Gln Ala Pro Arg Leu Lys
    2090                 2095                 2100

Tyr Leu Ile Gly Ala Ala Pro Ile Ala Tyr Gly Leu Pro Val Ser
    2105                 2110                 2115

Leu Arg
    2120

<210>  281
<211>  2209
<212>  PRT
<213>  Homo sapiens

<400>  281

Met Trp Asn Asp Ile Glu Leu Leu Thr Asn Asp Asp Thr Gly Ser Gly
1               5                   10                  15

Tyr Leu Ser Val Gly Ser Arg Lys Glu His Gly Thr Ala Leu Tyr Gln
            20                  25                  30

Val Asp Leu Leu Val Lys Ile Ser Ser Glu Lys Ala Ser Leu Asn Pro
        35                  40                  45

Lys Ile Gln Ala Cys Ser Leu Ser Asp Gly Phe Ile Ile Val Ala Asp
    50                  55                  60

Gln Ser Val Ile Leu Leu Asp Ser Ile Cys Arg Ser Leu Gln Leu His
65                  70                  75                  80

Leu Val Phe Asp Thr Glu Val Asp Val Val Gly Leu Cys Gln Glu Gly
                85                  90                  95

Lys Phe Leu Leu Val Gly Glu Arg Ser Gly Asn Leu His Leu Ile His
            100                 105                 110

Val Thr Ser Lys Gln Thr Leu Leu Thr Asn Ala Phe Val Gln Lys Ala
        115                 120                 125

Asn Asp Glu Asn Arg Arg Thr Tyr Gln Asn Leu Val Ile Glu Lys Asp
    130                 135                 140

Gly Ser Asn Glu Gly Thr Tyr Tyr Met Leu Leu Leu Thr Tyr Ser Gly
145                 150                 155                 160

Phe Phe Cys Ile Thr Asn Leu Gln Leu Leu Lys Ile Gln Gln Ala Ile
                165                 170                 175

Glu Asn Val Asp Phe Ser Thr Ala Lys Lys Leu Gln Gly Gln Ile Lys
            180                 185                 190

Ser Ser Phe Ile Ser Thr Glu Asn Tyr His Thr Leu Gly Cys Leu Ser
        195                 200                 205

Leu Val Ala Gly Asp Leu Ala Ser Glu Val Pro Val Ile Ile Gly Gly
    210                 215                 220

Thr Gly Asn Cys Ala Phe Ser Lys Trp Glu Pro Asp Ser Ser Lys Lys
225                 230                 235                 240

Gly Met Thr Val Lys Asn Leu Ile Asp Ala Glu Ile Ile Lys Gly Ala
                245                 250                 255

Lys Lys Phe Gln Leu Ile Asp Asn Leu Leu Phe Val Leu Asp Thr Asp
            260                 265                 270

Asn Val Leu Ser Leu Trp Asp Ile Tyr Thr Leu Thr Pro Val Trp Asn
        275                 280                 285

Trp Pro Ser Leu His Val Glu Glu Phe Leu Leu Thr Thr Glu Ala Asp
    290                 295                 300

Ser Pro Ser Ser Val Thr Trp Gln Gly Ile Thr Asn Leu Lys Leu Ile
305                 310                 315                 320

Ala Leu Thr Ala Ser Ala Asn Lys Lys Met Lys Asn Leu Met Val Tyr
                325                 330                 335

Ser Leu Pro Thr Met Glu Ile Leu Tyr Ser Leu Glu Val Ser Ser Val
            340                 345                 350

Ser Ser Leu Val Gln Thr Gly Ile Ser Thr Asp Thr Ile Tyr Leu Leu
        355                 360                 365

Glu Gly Val Cys Lys Asn Asp Pro Lys Leu Ser Glu Asp Ser Val Ser
    370                 375                 380

Val Leu Val Leu Arg Cys Leu Thr Glu Ala Leu Pro Glu Asn Arg Leu
385                 390                 395                 400

Ser Arg Leu Leu His Lys His Arg Phe Ala Glu Ala Glu Ser Phe Ala
                405                 410                 415

Ile Gln Phe Gly Leu Asp Val Glu Leu Val Tyr Lys Val Lys Ser Asn
            420                 425                 430

His Ile Leu Glu Lys Leu Ala Leu Ser Ser Val Asp Ala Ser Glu Gln
        435                 440                 445

Thr Glu Trp Gln Gln Leu Val Asp Asp Ala Lys Glu Asn Leu His Lys
    450                 455                 460

Ile Gln Asp Asp Glu Phe Val Val Asn Tyr Cys Leu Lys Ala Gln Trp
465                 470                 475                 480

Ile Thr Tyr Glu Thr Thr Gln Glu Met Leu Asn Tyr Ala Lys Thr Arg
                485                 490                 495

Leu Leu Lys Lys Glu Asp Lys Thr Ala Leu Ile Tyr Ser Asp Gly Leu
            500                 505                 510

Lys Glu Val Leu Arg Ala His Ala Lys Leu Thr Thr Phe Tyr Gly Ala
        515                 520                 525

Phe Gly Pro Glu Lys Phe Ser Gly Ser Ser Trp Ile Glu Phe Leu Asn
    530                 535                 540

Asn Glu Asp Asp Leu Lys Asp Ile Phe Leu Gln Leu Lys Glu Gly Asn
545                 550                 555                 560

Leu Val Cys Ala Gln Tyr Leu Trp Leu Arg His Arg Ala Asn Phe Glu
                565                 570                 575

Ser Arg Phe Asp Val Lys Met Leu Glu Ser Leu Leu Asn Ser Met Ser
            580                 585                 590

Ala Ser Val Ser Leu Gln Lys Leu Cys Pro Trp Phe Lys Asn Asp Val
        595                 600                 605

Ile Pro Phe Val Arg Arg Thr Val Pro Glu Gly Gln Ile Ile Leu Ala
    610                 615                 620

Lys Trp Leu Glu Gln Ala Ala Arg Asn Leu Glu Leu Thr Asp Lys Ala
625                 630                 635                 640

Asn Trp Pro Glu Asn Gly Leu Gln Leu Ala Glu Ile Phe Phe Thr Ala
                645                 650                 655

Glu Lys Thr Asp Glu Leu Gly Leu Ala Ser Ser Trp His Trp Ile Ser
            660                 665                 670

Leu Lys Asp Tyr Gln Asn Thr Glu Glu Val Cys Gln Leu Arg Thr Leu
        675                 680                 685

Val Asn Asn Leu Arg Glu Leu Ile Thr Leu His Arg Lys Tyr Asn Cys
    690                 695                 700

Lys Leu Ala Leu Ser Asp Phe Glu Lys Glu Asn Thr Thr Thr Ile Val
705                 710                 715                 720

Phe Arg Met Phe Asp Lys Val Leu Ala Pro Glu Leu Ile Pro Ser Ile
                725                 730                 735

Leu Glu Lys Phe Ile Arg Val Tyr Met Arg Glu His Asp Leu Gln Glu
            740                 745                 750

Glu Glu Leu Leu Leu Leu Tyr Ile Glu Asp Leu Leu Asn Arg Cys Ser
        755                 760                 765

Ser Lys Ser Thr Ser Leu Phe Glu Thr Ala Trp Glu Ala Lys Ala Met
    770                 775                 780

Ala Val Ile Ala Cys Leu Ser Asp Thr Asp Leu Ile Phe Asp Ala Val
785                 790                 795                 800

Leu Lys Ile Met Tyr Ala Ala Val Val Pro Trp Ser Ala Ala Val Glu
                805                 810                 815

Gln Leu Val Lys Gln His Leu Glu Met Asp His Pro Lys Val Lys Leu
            820                 825                 830

Leu Gln Glu Ser Tyr Lys Leu Met Glu Met Lys Lys Leu Leu Arg Gly
        835                 840                 845

Tyr Gly Ile Arg Glu Val Asn Leu Leu Asn Lys Glu Ile Met Arg Val
    850                 855                 860

Val Arg Tyr Ile Leu Lys Gln Asp Val Pro Ser Ser Leu Glu Asp Ala
865                 870                 875                 880

Leu Lys Val Ala Gln Ala Phe Met Leu Ser Asp Asp Glu Ile Tyr Ser
                885                 890                 895

Leu Arg Ile Ile Asp Leu Ile Asp Arg Glu Gln Gly Glu Asp Cys Leu
            900                 905                 910

Leu Leu Leu Lys Ser Leu Pro Pro Ala Glu Ala Glu Lys Thr Ala Glu
        915                 920                 925

Arg Val Ile Ile Trp Ala Arg Leu Ala Leu Gln Glu Glu Pro Asp His
    930                 935                 940

Ser Lys Glu Gly Lys Ala Trp Arg Met Ser Val Ala Lys Thr Ser Val
945                 950                 955                 960

Asp Ile Leu Lys Ile Leu Cys Asp Ile Gln Lys Asp Asn Leu Gln Lys
                965                 970                 975

Lys Asp Glu Cys Glu Glu Met Leu Lys Leu Phe Lys Glu Val Ala Ser
            980                 985                 990

Leu Gln Glu Asn Phe Glu Val Phe  Leu Ser Phe Glu Asp  Tyr Ser Asn
        995                 1000                 1005

Ser Ser  Leu Val Ala Asp Leu  Arg Glu Gln His Ile  Lys Ala His
    1010                 1015                 1020

Glu Val  Ala Gln Ala Lys His  Lys Pro Gly Ser Thr  Pro Glu Pro
    1025                 1030                 1035

Ile Ala  Ala Glu Val Arg Ser  Pro Ser Met Glu Ser  Lys Leu His
    1040                 1045                 1050

Arg Gln  Ala Leu Ala Leu Gln  Met Ser Lys Gln Glu  Leu Glu Ala
    1055                 1060                 1065

Glu Leu  Thr Leu Arg Ala Leu  Lys Asp Gly Asn Ile  Lys Thr Ala
    1070                 1075                 1080

Leu Lys  Lys Cys Ser Asp Leu  Phe Lys Tyr His Cys  Asn Ala Asp
    1085                 1090                 1095

Thr Gly  Lys Leu Leu Phe Leu  Thr Cys Gln Lys Leu  Cys Gln Met
    1100                 1105                 1110

Leu Ala  Asp Asn Val Pro Val  Thr Val Pro Val Gly  Leu Asn Leu
    1115                 1120                 1125

Pro Ser  Met Ile His Asp Leu  Ala Ser Gln Ala Ala  Thr Ile Cys
    1130                 1135                 1140

Ser Pro Asp Phe Leu Leu Asp Ala Leu Glu Leu Cys Lys His Thr
    1145                1150                1155

Leu Met Ala Val Glu Leu Ser Arg Gln Cys Gln Met Asp Asp Cys
    1160                1165                1170

Gly Ile Leu Met Lys Ala Ser Phe Gly Thr His Lys Asp Pro Tyr
    1175                1180                1185

Glu Glu Trp Ser Tyr Ser Asp Phe Phe Ser Glu Asp Gly Ile Val
    1190                1195                1200

Leu Glu Ser Gln Met Val Leu Pro Val Ile Tyr Glu Leu Ile Ser
    1205                1210                1215

Ser Leu Val Pro Leu Ala Glu Ser Lys Arg Tyr Pro Leu Glu Ser
    1220                1225                1230

Thr Ser Leu Pro Tyr Cys Ser Leu Asn Glu Gly Asp Gly Leu Val
    1235                1240                1245

Leu Pro Val Ile Asn Ser Ile Ser Ala Leu Leu Gln Asn Leu Gln
    1250                1255                1260

Glu Ser Ser Gln Trp Glu Leu Ala Leu Arg Phe Val Val Gly Ser
    1265                1270                1275

Phe Gly Thr Cys Leu Gln His Ser Val Ser Asn Phe Met Asn Ala
    1280                1285                1290

Thr Leu Ser Glu Lys Leu Phe Gly Glu Thr Thr Leu Val Lys Ser
    1295                1300                1305

Arg His Val Val Met Glu Leu Lys Glu Lys Ala Val Ile Phe Ile
    1310                1315                1320

Arg Glu Asn Ala Thr Thr Leu Leu His Lys Val Phe Asn Cys Arg
    1325                1330                1335

Leu Val Asp Leu Asp Leu Ala Leu Gly Tyr Cys Thr Leu Leu Pro
    1340                1345                1350

Gln Lys Asp Val Phe Glu Asn Leu Trp Lys Leu Ile Asp Lys Ala
    1355                1360                1365

Trp Gln Asn Tyr Asp Lys Ile Leu Ala Ile Ser Leu Val Gly Ser
    1370                1375                1380

Glu Leu Ala Ser Leu Tyr Gln Glu Ile Glu Met Gly Leu Lys Phe
    1385                1390                1395

Arg Glu Leu Ser Thr Asp Ala Gln Trp Gly Ile Arg Leu Gly Lys
    1400                1405                1410

Leu Gly Ile Ser Phe Gln Pro Val Phe Arg Gln His Phe Leu Thr
    1415                1420                1425

Lys Lys Asp Leu Ile Lys Ala Leu Val Glu Asn Ile Asp Met Asp
    1430                1435                1440

Thr Ser Leu Ile Leu Glu Tyr Cys Ser Thr Phe Gln Leu Asp Cys
    1445                1450                1455

Asp Ala Val Leu Gln Leu Phe Ile Glu Thr Leu Leu His Asn Thr
    1460                1465                1470

Asn Ala Gly Gln Gly Gln Gly Asp Ala Ser Met Asp Ser Ala Lys
    1475                1480                1485

Arg Arg His Pro Lys Leu Leu Ala Lys Ala Leu Glu Met Val Pro
    1490                1495                1500

Leu Leu Thr Ser Thr Lys Asp Leu Val Ile Ser Leu Ser Gly Ile
    1505                1510                1515

Leu His Lys Leu Asp Pro Tyr Asp Tyr Glu Met Ile Glu Val Val
    1520                1525                1530

Leu Lys Val Ile Glu Arg Ala Asp Glu Lys Ile Thr Asn Ile Asn
    1535                1540                1545

Ile Asn Gln Ala Leu Ser Ile Leu Lys His Leu Lys Ser Tyr Arg
    1550                1555                1560

Arg Ile Ser Pro Pro Val Asp Leu Glu Tyr Gln Tyr Met Leu Glu
    1565                1570                1575

His Val Ile Thr Leu Pro Ser Ala Ala Gln Thr Arg Leu Pro Phe
    1580                1585                1590

His Leu Ile Phe Phe Gly Thr Ala Gln Asn Phe Trp Lys Ile Leu
    1595                1600                1605

Ser Thr Glu Leu Ser Glu Glu Ser Phe Pro Thr Leu Leu Leu Ile
    1610                1615                1620

Ser Lys Leu Met Lys Phe Ser Leu Asp Thr Leu Tyr Val Ser Thr
    1625                1630                1635

Ala Lys His Val Phe Glu Lys Lys Leu Lys Pro Lys Leu Leu Lys
    1640                1645                1650

Leu Thr Gln Ala Lys Ser Ser Thr Leu Ile Asn Lys Glu Ile Thr
    1655                1660                1665

Lys Ile Thr Gln Thr Ile Glu Ser Cys Leu Leu Ser Ile Val Asn
    1670                1675                1680

Pro Glu Trp Ala Val Ala Ile Ala Ile Ser Leu Ala Gln Asp Ile
    1685                1690                1695

Pro Glu Gly Ser Phe Lys Ile Ser Ala Leu Lys Phe Cys Leu Tyr
    1700                1705                1710

Leu Ala Glu Arg Trp Leu Gln Asn Ile Pro Ser Gln Asp Glu Lys
    1715                1720                1725

Arg Glu Lys Ala Glu Ala Leu Leu Lys Lys Leu His Ile Gln Tyr
    1730                1735                1740

Arg Arg Ser Gly Thr Glu Ala Val Leu Ile Ala His Lys Leu Asn
    1745                1750                1755

Thr Glu Glu Tyr Leu Arg Val Ile Gly Lys Pro Ala His Leu Ile
    1760                1765                1770

Val Ser Leu Tyr Glu His Pro Ser Ile Asn Gln Arg Ile Gln Asn
    1775                1780                1785

Ser Ser Gly Thr Asp Tyr Pro Asp Ile His Ala Ala Ala Lys Glu
    1790                1795                1800

Ile Ala Glu Val Asn Glu Ile Asn Leu Glu Lys Val Trp Asp Met
    1805                1810                1815

Leu Leu Glu Lys Trp Leu Cys Pro Ser Thr Lys Pro Gly Glu Lys
    1820                1825                1830

Pro Ser Glu Leu Phe Glu Leu Gln Glu Asp Glu Ala Leu Arg Arg
    1835                1840                1845

Val Gln Tyr Leu Leu Leu Ser Arg Pro Ile Asp Tyr Ser Ser Arg
    1850                1855                1860

Met Leu Phe Val Phe Ala Thr Ser Thr Thr Thr Thr Leu Gly Met
    1865                1870                1875

His Gln Leu Thr Phe Ala His Arg Thr Arg Ala Leu Gln Cys Leu
    1880                1885                1890

Phe Tyr Leu Ala Asp Lys Glu Thr Ile Glu Ser Leu Phe Lys Lys
    1895                1900                1905

Pro Ile Glu Glu Val Lys Ser Tyr Leu Arg Cys Ile Thr Phe Leu
    1910                1915                1920

Ala Ser Phe Glu Thr Leu Asn Ile Pro Ile Thr Tyr Glu Leu Phe
    1925                1930                1935

Cys Ser Ser Pro Lys Glu Gly Met Ile Lys Gly Leu Trp Lys Asn
    1940                1945                1950

His Ser His Glu Ser Met Ala Val Arg Leu Val Thr Glu Leu Cys
    1955                1960                1965

Leu Glu Tyr Lys Ile Tyr Asp Leu Gln Leu Trp Asn Gly Leu Leu
    1970                1975                1980

Gln Lys Leu Leu Gly Phe Asn Met Ile Pro Tyr Leu Arg Lys Val
    1985                1990                1995

Leu Lys Ala Ile Ser Ser Ile His Ser Leu Trp Gln Val Pro Tyr
    2000                2005                2010

Phe Ser Lys Ala Trp Gln Arg Val Ile Gln Ile Pro Leu Leu Ser
    2015                2020                2025

Ala Ser Cys Pro Leu Ser Pro Asp Gln Leu Ser Asp Cys Ser Glu
    2030                2035                2040

Ser Leu Ile Ala Val Leu Glu Cys Pro Val Ser Gly Asp Leu Asp
    2045                2050                2055

Leu Ile Gly Val Ala Arg Gln Tyr Ile Gln Leu Glu Leu Pro Ala
    2060                2065                2070

Phe Ala Leu Ala Cys Leu Met Leu Met Pro His Ser Glu Lys Arg
    2075                2080                2085

His Gln Gln Ile Lys Asn Phe Leu Gly Ser Cys Asp Pro Gln Val
    2090                2095                2100

Ile Leu Lys Gln Leu Glu Glu His Met Asn Thr Gly Gln Leu Ala
    2105                2110                2115

Gly Phe Ser His Gln Ile Arg Ser Leu Ile Leu Asn Asn Ile Ile
    2120                2125                2130

Asn Lys Lys Glu Phe Gly Ile Leu Ala Lys Thr Lys Tyr Phe Gln
    2135                2140                2145

Met Leu Lys Met His Ala Met Asn Thr Asn Asn Ile Thr Glu Leu
    2150                2155                2160

Val Asn Tyr Leu Ala Asn Asp Leu Ser Leu Asp Glu Ala Ser Val
    2165                2170                2175

Leu Ile Thr Glu Tyr Ser Lys His Cys Gly Lys Pro Val Pro Pro
    2180                2185                2190

Asp Thr Ala Pro Cys Glu Ile Leu Lys Met Phe Leu Ser Gly Leu
    2195                2200                2205

Ser

<210> 282
<211> 846
<212> PRT
<213> Homo sapiens

<400> 282

Met Ser Ser Ser His Phe Ala Ser Arg His Arg Lys Asp Ile Ser Thr
1               5                   10                  15

Glu Met Ile Arg Thr Lys Ile Ala His Arg Lys Ser Leu Ser Gln Lys
            20                  25                  30

Glu Asn Arg His Lys Glu Tyr Glu Arg Asn Arg His Phe Gly Leu Lys
        35                  40                  45

Asp Val Asn Ile Pro Thr Leu Glu Gly Arg Ile Leu Val Glu Leu Asp
    50                  55                  60

Glu Thr Ser Gln Glu Leu Val Pro Glu Lys Thr Asn Val Lys Pro Arg
65                  70                  75                  80

Ala Met Lys Thr Ile Leu Gly Asp Gln Arg Lys Gln Met Leu Gln Lys
                85                  90                  95

Tyr Lys Glu Glu Lys Gln Leu Gln Lys Leu Lys Glu Gln Arg Glu Lys
            100                 105                 110

Ala Lys Arg Gly Ile Phe Lys Val Gly Arg Tyr Arg Pro Asp Met Pro
        115                 120                 125

Cys Phe Leu Leu Ser Asn Gln Asn Ala Val Lys Ala Glu Pro Lys Lys
    130                 135                 140

Ala Ile Pro Ser Ser Val Arg Ile Thr Arg Ser Lys Ala Lys Asp Gln
145                 150                 155                 160

Met Glu Gln Thr Lys Ile Asp Asn Glu Ser Asp Val Arg Ala Ile Arg
                165                 170                 175

Pro Gly Pro Arg Gln Thr Ser Glu Lys Lys Val Ser Asp Lys Glu Lys
            180                 185                 190

Lys Val Val Gln Pro Val Met Pro Thr Ser Leu Arg Met Thr Arg Ser
        195                 200                 205

Ala Thr Gln Ala Ala Lys Gln Val Pro Arg Thr Val Ser Ser Thr Thr
    210                 215                 220

Ala Arg Lys Pro Val Thr Arg Ala Ala Asn Glu Asn Glu Pro Glu Gly
225                 230                 235                 240

Lys Val Pro Ser Lys Gly Arg Pro Ala Lys Asn Val Glu Thr Lys Pro
                245                 250                 255

Asp Lys Gly Ile Ser Cys Lys Val Asp Ser Glu Glu Asn Thr Leu Asn
            260                 265                 270

Ser Gln Thr Asn Ala Thr Ser Gly Met Asn Pro Asp Gly Val Leu Ser
        275                 280                 285

Lys Met Glu Asn Leu Pro Glu Ile Asn Thr Ala Lys Ile Lys Gly Lys
    290                 295                 300

Asn Ser Phe Ala Pro Lys Asp Phe Met Phe Gln Pro Leu Asp Gly Leu
305                 310                 315                 320

Lys Thr Tyr Gln Val Thr Pro Met Thr Pro Arg Ser Ala Asn Ala Phe
                325                 330                 335

Leu Thr Pro Ser Tyr Thr Trp Thr Pro Leu Lys Thr Glu Val Asp Glu
            340                 345                 350

Ser Gln Ala Thr Lys Glu Ile Leu Ala Gln Lys Cys Lys Thr Tyr Ser
        355                 360                 365

Thr Lys Thr Ile Gln Gln Asp Ser Asn Lys Leu Pro Cys Pro Leu Gly
    370                 375                 380

Pro Leu Thr Val Trp His Glu Glu His Val Leu Asn Lys Asn Glu Ala
385                 390                 395                 400

Thr Thr Lys Asn Leu Asn Gly Leu Pro Ile Lys Glu Val Pro Ser Leu
                405                 410                 415

Glu Arg Asn Glu Gly Arg Ile Ala Gln Pro His His Gly Val Pro Tyr
            420                 425                 430

Phe Arg Asn Ile Leu Gln Ser Glu Thr Glu Lys Leu Thr Ser His Cys
        435                 440                 445

Phe Glu Trp Asp Arg Lys Leu Glu Leu Asp Ile Pro Asp Asp Ala Lys
    450                 455                 460

Asp Leu Ile Arg Thr Ala Val Gly Gln Thr Arg Leu Leu Met Lys Glu
465                 470                 475                 480

Arg Phe Lys Gln Phe Glu Gly Leu Val Asp Asp Cys Glu Tyr Lys Arg
                485                 490                 495

Gly Ile Lys Glu Thr Thr Cys Thr Asp Leu Asp Gly Phe Trp Asp Met

            500                 505                 510

Val Ser Phe Gln Ile Glu Asp Val Ile His Lys Phe Asn Asn Leu Ile
        515                 520                 525

Lys Leu Glu Glu Ser Gly Trp Gln Val Asn Asn Asn Met Asn His Asn
    530                 535                 540

Met Asn Lys Asn Val Phe Arg Lys Lys Val Val Ser Gly Ile Ala Ser
545                 550                 555                 560

Lys Pro Lys Gln Asp Asp Ala Gly Arg Ile Ala Ala Arg Asn Arg Leu
                565                 570                 575

Ala Ala Ile Lys Asn Ala Met Arg Glu Arg Ile Arg Gln Glu Glu Cys
            580                 585                 590

Ala Glu Thr Ala Val Ser Val Ile Pro Lys Glu Val Asp Lys Ile Val
        595                 600                 605

Phe Asp Ala Gly Phe Phe Arg Val Glu Ser Pro Val Lys Leu Phe Ser
    610                 615                 620

Gly Leu Ser Val Ser Ser Glu Gly Pro Ser Gln Arg Leu Gly Thr Pro
625                 630                 635                 640

Lys Ser Val Asn Lys Ala Val Ser Gln Ser Arg Asn Glu Met Gly Ile
                645                 650                 655

Pro Gln Gln Thr Thr Ser Pro Glu Asn Ala Gly Pro Gln Asn Thr Lys
            660                 665                 670

Ser Glu His Val Lys Lys Thr Leu Phe Leu Ser Ile Pro Glu Ser Arg
        675                 680                 685

Ser Ser Ile Glu Asp Ala Gln Cys Pro Gly Leu Pro Asp Leu Ile Glu
    690                 695                 700

Glu Asn His Val Val Asn Lys Thr Asp Leu Lys Val Asp Cys Leu Ser
705                 710                 715                 720

Ser Glu Arg Met Ser Leu Pro Leu Leu Ala Gly Gly Val Ala Asp Asp
                725                 730                 735

Ile Asn Thr Asn Lys Lys Glu Gly Ile Ser Asp Val Val Glu Gly Met
            740                 745                 750

Glu Leu Asn Ser Ser Ile Thr Ser Gln Asp Val Leu Met Ser Ser Pro
        755                 760                 765

Glu Lys Asn Thr Ala Ser Gln Asn Ser Ile Leu Glu Glu Gly Glu Thr
    770                 775                 780

Lys Ile Ser Gln Ser Glu Leu Phe Asp Asn Lys Ser Leu Thr Thr Glu
785                 790                 795                 800

Cys His Leu Leu Asp Ser Pro Gly Leu Asn Cys Ser Asn Pro Phe Thr
                805                 810                 815

Gln Leu Glu Arg Arg His Gln Glu His Ala Arg His Ile Ser Phe Gly
            820                 825                 830

Gly Asn Leu Ile Thr Phe Ser Pro Leu Gln Pro Gly Glu Phe
        835                 840                 845

<210>  283
<211>  651
<212>  PRT
<213>  Homo sapiens

<400>  283

Met Lys Asp Tyr Asp Glu Leu Leu Lys Tyr Tyr Glu Leu His Glu Thr
1               5                   10                  15

Ile Gly Thr Gly Gly Phe Ala Lys Val Lys Leu Ala Cys His Ile Leu
            20                  25                  30

Thr Gly Glu Met Val Ala Ile Lys Ile Met Asp Lys Asn Thr Leu Gly
        35                  40                  45

Ser Asp Leu Pro Arg Ile Lys Thr Glu Ile Glu Ala Leu Lys Asn Leu
    50                  55                  60

Arg His Gln His Ile Cys Gln Leu Tyr His Val Leu Glu Thr Ala Asn
65                  70                  75                  80

Lys Ile Phe Met Val Leu Glu Tyr Cys Pro Gly Gly Glu Leu Phe Asp
                85                  90                  95

Tyr Ile Ile Ser Gln Asp Arg Leu Ser Glu Glu Glu Thr Arg Val Val
            100                 105                 110

Phe Arg Gln Ile Val Ser Ala Val Ala Tyr Val His Ser Gln Gly Tyr
        115                 120                 125

Ala His Arg Asp Leu Lys Pro Glu Asn Leu Leu Phe Asp Glu Tyr His
    130                 135                 140

Lys Leu Lys Leu Ile Asp Phe Gly Leu Cys Ala Lys Pro Lys Gly Asn
145                 150                 155                 160

Lys Asp Tyr His Leu Gln Thr Cys Cys Gly Ser Leu Ala Tyr Ala Ala
                165                 170                 175

Pro Glu Leu Ile Gln Gly Lys Ser Tyr Leu Gly Ser Glu Ala Asp Val
            180                 185                 190

Trp Ser Met Gly Ile Leu Leu Tyr Val Leu Met Cys Gly Phe Leu Pro
        195                 200                 205

Phe Asp Asp Asp Asn Val Met Ala Leu Tyr Lys Lys Ile Met Arg Gly
    210                 215                 220

Lys Tyr Asp Val Pro Lys Trp Leu Ser Pro Ser Ser Ile Leu Leu Leu
225                 230                 235                 240

Gln Gln Met Leu Gln Val Asp Pro Lys Lys Arg Ile Ser Met Lys Asn
                245                 250                 255

Leu Leu Asn His Pro Trp Ile Met Gln Asp Tyr Asn Tyr Pro Val Glu
            260                 265                 270

Trp Gln Ser Lys Asn Pro Phe Ile His Leu Asp Asp Asp Cys Val Thr
        275                 280                 285

Glu Leu Ser Val His His Arg Asn Asn Arg Gln Thr Met Glu Asp Leu
    290                 295                 300

Ile Ser Leu Trp Gln Tyr Asp His Leu Thr Ala Thr Tyr Leu Leu Leu
305                 310                 315                 320

Leu Ala Lys Lys Ala Arg Gly Lys Pro Val Arg Leu Arg Leu Ser Ser
                325                 330                 335

Phe Ser Cys Gly Gln Ala Ser Ala Thr Pro Phe Thr Asp Ile Lys Ser
            340                 345                 350

Asn Asn Trp Ser Leu Glu Asp Val Thr Ala Ser Asp Lys Asn Tyr Val
        355                 360                 365

Ala Gly Leu Ile Asp Tyr Asp Trp Cys Glu Asp Asp Leu Ser Thr Gly
    370                 375                 380

Ala Ala Thr Pro Arg Thr Ser Gln Phe Thr Lys Tyr Trp Thr Glu Ser
385                 390                 395                 400

Asn Gly Val Glu Ser Lys Ser Leu Thr Pro Ala Leu Cys Arg Thr Pro
                405                 410                 415

Ala Asn Lys Leu Lys Asn Lys Glu Asn Val Tyr Thr Pro Lys Ser Ala
            420                 425                 430

Val Lys Asn Glu Glu Tyr Phe Met Phe Pro Glu Pro Lys Thr Pro Val
        435                 440                 445

Asn Lys Asn Gln His Lys Arg Glu Ile Leu Thr Thr Pro Asn Arg Tyr
    450                 455                 460

Thr Thr Pro Ser Lys Ala Arg Asn Gln Cys Leu Lys Glu Thr Pro Ile
465                 470                 475                 480

Lys Ile Pro Val Asn Ser Thr Gly Thr Asp Lys Leu Met Thr Gly Val
                485                 490                 495

Ile Ser Pro Glu Arg Arg Cys Arg Ser Val Glu Leu Asp Leu Asn Gln
            500                 505                 510

Ala His Met Glu Glu Thr Pro Lys Arg Lys Gly Ala Lys Val Phe Gly
        515                 520                 525

Ser Leu Glu Arg Gly Leu Asp Lys Val Ile Thr Val Leu Thr Arg Ser
    530                 535                 540

Lys Arg Lys Gly Ser Ala Arg Asp Gly Pro Arg Arg Leu Lys Leu His
545                 550                 555                 560

Tyr Asn Val Thr Thr Thr Arg Leu Val Asn Pro Asp Gln Leu Leu Asn
                565                 570                 575

Glu Ile Met Ser Ile Leu Pro Lys Lys His Val Asp Phe Val Gln Lys
            580                 585                 590

Gly Tyr Thr Leu Lys Cys Gln Thr Gln Ser Asp Phe Gly Lys Val Thr
        595                 600                 605

Met Gln Phe Glu Leu Glu Val Cys Gln Leu Gln Lys Pro Asp Val Val
    610                 615                 620

Gly Ile Arg Arg Gln Arg Leu Lys Gly Asp Ala Trp Val Tyr Lys Arg
625                 630                 635                 640

Leu Val Glu Asp Ile Leu Ser Ser Cys Lys Val
                645                 650

<210>  284
<211>  196
<212>  PRT
<213>  Homo sapiens

<400>  284

Met Phe Cys Glu Lys Ala Met Glu Leu Ile Arg Glu Leu His Arg Ala
1               5                   10                  15

Pro Glu Gly Gln Leu Pro Ala Phe Asn Glu Asp Gly Leu Arg Gln Val
            20                  25                  30

Leu Glu Glu Met Lys Ala Leu Tyr Glu Gln Asn Gln Ser Asp Val Asn
        35                  40                  45

Glu Ala Lys Ser Gly Gly Arg Ser Asp Leu Ile Pro Thr Ile Lys Phe
    50                  55                  60

Arg His Cys Ser Leu Leu Arg Asn Arg Arg Cys Thr Val Ala Tyr Leu
65                  70                  75                  80

Tyr Asp Arg Leu Leu Arg Ile Arg Ala Leu Arg Trp Glu Tyr Gly Ser
                85                  90                  95

Ile Leu Pro Asn Ala Leu Arg Phe His Met Ala Ala Glu Glu Met Glu
            100                 105                 110

Trp Phe Asn Asn Tyr Lys Arg Ser Leu Ala Thr Tyr Met Arg Ser Leu
        115                 120                 125

Gly Gly Asp Glu Gly Leu Asp Ile Thr Gln Asp Met Lys Pro Pro Lys
    130                 135                 140

Ser Leu Tyr Ile Glu Val Arg Cys Leu Lys Asp Tyr Gly Glu Phe Glu
145                 150                 155                 160

Val Asp Asp Gly Thr Ser Val Leu Leu Lys Lys Asn Ser Gln His Phe
                165                 170                 175

Leu Pro Arg Trp Lys Cys Glu Gln Leu Ile Arg Gln Gly Val Leu Glu
            180                 185                 190

His Ile Leu Ser
        195

<210>  285
<211>  1401
<212>  PRT
<213>  Homo sapiens

<400>  285

Met Ala Pro Gln Met Tyr Glu Phe His Leu Pro Leu Ser Pro Glu Glu
1               5                   10                  15

Leu Leu Lys Ser Gly Gly Val Asn Gln Tyr Val Val Gln Glu Val Leu
            20                  25                  30

Ser Ile Lys His Leu Pro Pro Gln Leu Arg Ala Phe Gln Ala Ala Phe
        35                  40                  45

Arg Ala Gln Gly Pro Leu Ala Met Leu Gln His Phe Asp Thr Ile Tyr
    50                  55                  60

Ser Ile Leu His His Phe Arg Ser Ile Asp Pro Gly Leu Lys Glu Asp
65                  70                  75                  80

Thr Leu Glu Phe Leu Ile Lys Val Val Ser Arg His Ser Gln Glu Leu
                85                  90                  95

Pro Ala Ile Leu Asp Asp Thr Thr Leu Ser Gly Ser Asp Arg Asn Ala
            100                 105                 110

His Leu Asn Ala Leu Lys Met Asn Cys Tyr Ala Leu Ile Arg Leu Leu
        115                 120                 125

Glu Ser Phe Glu Thr Met Ala Ser Gln Thr Asn Leu Val Asp Leu Asp
    130                 135                 140

Leu Gly Gly Lys Gly Lys Lys Ala Arg Thr Lys Ala Ala His Gly Phe
145                 150                 155                 160

Asp Trp Glu Glu Glu Arg Gln Pro Ile Leu Gln Leu Leu Thr Gln Leu
                165                 170                 175

Leu Gln Leu Asp Ile Arg His Leu Trp Asn His Ser Ile Ile Glu Glu
            180                 185                 190

Glu Phe Val Ser Leu Val Thr Gly Cys Cys Tyr Arg Leu Leu Glu Asn
        195                 200                 205

Pro Thr Ile Asn His Gln Lys Asn Arg Pro Thr Arg Glu Ala Ile Thr
    210                 215                 220

His Leu Leu Gly Val Ala Leu Thr Arg Tyr Asn His Met Leu Ser Ala
225                 230                 235                 240

Thr Val Lys Ile Ile Gln Met Leu Gln His Phe Glu His Leu Ala Pro
                245                 250                 255

Val Leu Val Ala Ala Val Ser Leu Trp Ala Thr Asp Tyr Gly Met Lys
            260                 265                 270

Ser Ile Val Gly Glu Ile Val Arg Glu Ile Gly Gln Lys Cys Pro Gln
        275                 280                 285

Glu Leu Ser Arg Asp Pro Ser Gly Thr Lys Gly Phe Ala Ala Phe Leu
    290                 295                 300

Thr Glu Leu Ala Glu Arg Val Pro Ala Ile Leu Met Ser Ser Met Cys
305                 310                 315                 320

Ile Leu Leu Asp His Leu Asp Gly Glu Asn Tyr Met Met Arg Asn Ala
                325                 330                 335

Val Leu Ala Ala Met Ala Glu Met Val Leu Gln Val Leu Ser Gly Asp
            340                 345                 350

Gln Leu Glu Ala Ala Ala Arg Asp Thr Arg Asp Gln Phe Leu Asp Thr
        355                 360                 365

Leu Gln Ala His Gly His Asp Val Asn Ser Phe Val Arg Ser Arg Val
    370                 375                 380

Leu Gln Leu Phe Thr Arg Ile Val Gln Gln Lys Ala Leu Pro Leu Thr
385                 390                 395                 400

Arg Phe Gln Ala Val Val Ala Leu Ala Val Gly Arg Leu Ala Asp Lys
                405                 410                 415

Ser Val Leu Val Cys Lys Asn Ala Ile Gln Leu Leu Ala Ser Phe Leu
            420                 425                 430

Ala Asn Asn Pro Phe Ser Cys Lys Leu Ser Asp Ala Asp Leu Ala Gly
        435                 440                 445

Pro Leu Gln Lys Glu Thr Gln Lys Leu Gln Glu Met Arg Ala Gln Arg
    450                 455                 460

Arg Thr Ala Ala Ala Ser Ala Val Leu Asp Pro Glu Glu Glu Trp Glu
465                 470                 475                 480

Ala Met Leu Pro Glu Leu Lys Ser Thr Leu Gln Gln Leu Leu Gln Leu
                485                 490                 495

Pro Gln Gly Glu Glu Glu Ile Pro Glu Gln Ile Ala Asn Thr Glu Thr
            500                 505                 510

Thr Glu Asp Val Lys Gly Arg Ile Tyr Gln Leu Leu Ala Lys Ala Ser
        515                 520                 525

Tyr Lys Lys Ala Ile Ile Leu Thr Arg Glu Ala Thr Gly His Phe Gln
    530                 535                 540

Glu Ser Glu Pro Phe Ser His Ile Asp Pro Glu Glu Ser Glu Glu Thr
545                 550                 555                 560

Arg Leu Leu Asn Ile Leu Gly Leu Ile Phe Lys Gly Pro Ala Ala Ser
                565                 570                 575

Thr Gln Glu Lys Asn Pro Arg Glu Ser Thr Gly Asn Met Val Thr Gly
            580                 585                 590

Gln Thr Val Cys Lys Asn Lys Pro Asn Met Ser Asp Pro Glu Glu Ser
        595                 600                 605

Arg Gly Asn Asp Glu Leu Val Lys Gln Glu Met Leu Val Gln Tyr Leu
    610                 615                 620

Gln Asp Ala Tyr Ser Phe Ser Arg Lys Ile Thr Glu Ala Ile Gly Ile
625                 630                 635                 640

Ile Ser Lys Met Met Tyr Glu Asn Thr Thr Thr Val Val Gln Glu Val
                645                 650                 655

Ile Glu Phe Phe Val Met Val Phe Gln Phe Gly Val Pro Gln Ala Leu
            660                 665                 670

Phe Gly Val Arg Arg Met Leu Pro Leu Ile Trp Ser Lys Glu Pro Gly
        675                 680                 685

Val Arg Glu Ala Val Leu Asn Ala Tyr Arg Gln Leu Tyr Leu Asn Pro
    690                 695                 700

Lys Gly Asp Ser Ala Arg Ala Lys Ala Gln Ala Leu Ile Gln Asn Leu
705                 710                 715                 720

Ser Leu Leu Leu Val Asp Ala Ser Val Gly Thr Ile Gln Cys Leu Glu
                725                 730                 735

Glu Ile Leu Cys Glu Phe Val Gln Lys Asp Glu Leu Lys Pro Ala Val
            740                 745                 750

Thr Gln Leu Leu Trp Glu Arg Ala Thr Glu Lys Val Ala Cys Cys Pro
        755                 760                 765

Leu Glu Arg Cys Ser Ser Val Met Leu Leu Gly Met Met Ala Arg Gly
    770                 775                 780

Lys Pro Glu Ile Val Gly Ser Asn Leu Asp Thr Leu Val Ser Ile Gly
785                 790                 795                 800

Leu Asp Glu Lys Phe Pro Gln Asp Tyr Arg Leu Ala Gln Gln Val Cys
                805                 810                 815

His Ala Ile Ala Asn Ile Ser Asp Arg Arg Lys Pro Ser Leu Gly Lys
            820                 825                 830

Arg His Pro Pro Phe Arg Leu Pro Gln Glu His Arg Leu Phe Glu Arg
        835                 840                 845

Leu Arg Glu Thr Val Thr Lys Gly Phe Val His Pro Asp Pro Leu Trp
    850                 855                 860

Ile Pro Phe Lys Glu Val Ala Val Thr Leu Ile Tyr Gln Leu Ala Glu
865                 870                 875                 880

Gly Pro Glu Val Ile Cys Ala Gln Ile Leu Gln Gly Cys Ala Lys Gln
                885                 890                 895

Ala Leu Glu Lys Leu Glu Glu Lys Arg Thr Ser Gln Glu Asp Pro Lys
            900                 905                 910

Glu Ser Pro Ala Met Leu Pro Thr Phe Leu Leu Met Asn Leu Leu Ser
        915                 920                 925

Leu Ala Gly Asp Val Ala Leu Gln Gln Leu Val His Leu Glu Gln Ala
    930                 935                 940

Val Ser Gly Glu Leu Cys Arg Arg Arg Val Leu Arg Glu Glu Gln Glu
945                 950                 955                 960

His Lys Thr Lys Asp Pro Lys Glu Lys Asn Thr Ser Ser Glu Thr Thr
                965                 970                 975

Met Glu Glu Glu Leu Gly Leu Val Gly Ala Thr Ala Asp Asp Thr Glu
            980                 985                 990

Ala Glu Leu Ile Arg Gly Ile Cys  Glu Met Glu Leu Leu  Asp Gly Lys
        995                 1000                 1005

Gln Thr  Leu Ala Ala Phe Val  Pro Leu Leu Leu Lys  Val Cys Asn
    1010                 1015                 1020

Asn Pro  Gly Leu Tyr Ser Asn  Pro Asp Leu Ser Ala  Ala Ala Ser
    1025                 1030                 1035

Leu Ala  Leu Gly Lys Phe Cys  Met Ile Ser Ala Thr  Phe Cys Asp
    1040                 1045                 1050

Ser Gln  Leu Arg Leu Leu Phe  Thr Met Leu Glu Lys  Ser Pro Leu
    1055                 1060                 1065

Pro Ile  Val Arg Ser Asn Leu  Met Val Ala Thr Gly  Asp Leu Ala
    1070                 1075                 1080

Ile Arg  Phe Pro Asn Leu Val  Asp Pro Trp Thr Pro  His Leu Tyr
    1085                 1090                 1095

Ala Arg  Leu Arg Asp Pro Ala  Gln Gln Val Arg Lys  Thr Ala Gly
    1100                 1105                 1110

Leu Val  Met Thr His Leu Ile  Leu Lys Asp Met Val  Lys Val Lys
    1115                 1120                 1125

Gly Gln  Val Ser Glu Met Ala  Val Leu Leu Ile Asp  Pro Glu Pro
    1130                 1135                 1140

Gln Ile  Ala Ala Leu Ala Lys  Asn Phe Phe Asn Glu  Leu Ser His
    1145                 1150                 1155

Lys Gly  Asn Ala Ile Tyr Asn  Leu Leu Pro Asp Ile  Ile Ser Arg
    1160                 1165                 1170

Leu Ser  Asp Pro Glu Leu Gly  Val Glu Glu Glu Pro  Phe His Thr
    1175                 1180                 1185

Ile Met  Lys Gln Leu Leu Ser  Tyr Ile Thr Lys Asp  Lys Gln Thr
    1190                 1195                 1200

Glu Ser  Leu Val Glu Lys Leu  Cys Gln Arg Phe Arg  Thr Ser Arg
    1205                 1210                 1215

Thr Glu Arg Gln Gln Arg Asp Leu Ala Tyr Cys Val Ser Gln Leu
    1220                1225                1230

Pro Leu Thr Glu Arg Gly Leu Arg Lys Met Leu Asp Asn Phe Asp
    1235                1240                1245

Cys Phe Gly Asp Lys Leu Ser Asp Glu Ser Ile Phe Ser Ala Phe
    1250                1255                1260

Leu Ser Val Val Gly Lys Leu Arg Arg Gly Ala Lys Pro Glu Gly
    1265                1270                1275

Lys Ala Ile Ile Asp Glu Phe Glu Gln Lys Leu Arg Ala Cys His
    1280                1285                1290

Thr Arg Gly Leu Asp Gly Ile Lys Glu Leu Glu Ile Gly Gln Ala
    1295                1300                1305

Gly Ser Gln Arg Ala Pro Ser Ala Lys Lys Pro Ser Ser Gly Ser
    1310                1315                1320

Arg Tyr Gln Pro Leu Ala Ser Thr Ala Ser Asp Asn Asp Phe Val
    1325                1330                1335

Thr Pro Glu Pro Arg Arg Thr Thr Arg Arg His Pro Asn Thr Gln
    1340                1345                1350

Gln Arg Ala Ser Lys Lys Lys Pro Lys Val Val Phe Ser Ser Asp
    1355                1360                1365

Glu Ser Ser Glu Glu Asp Leu Ser Ala Glu Met Thr Glu Asp Glu
    1370                1375                1380

Thr Pro Lys Lys Thr Thr Pro Ile Leu Arg Ala Ser Ala Arg Arg
    1385                1390                1395

His Arg Ser
    1400

<210>  286
<211>  1648
<212>  PRT
<213>  Homo sapiens

<400>  286

Met Ser Leu His Ser Thr His Asn Arg Asn Asn Ser Gly Asp Ile Leu
1               5                   10                  15

Asp Ile Pro Ser Ser Gln Asn Ser Ser Ser Leu Asn Ala Leu Thr His
            20                  25                  30

Ser Ser Arg Leu Lys Leu His Leu Lys Ser Asp Met Ser Glu Cys Glu

35                  40                  45

Asn Asp Asp Pro Leu Leu Arg Ser Ala Gly Lys Val Arg Asp Ile Asn
    50                  55                  60

Arg Thr Tyr Val Ile Ser Ala Ser Arg Lys Thr Ala Asp Met Pro Leu
65                  70                  75                  80

Thr Pro Asn Pro Val Gly Arg Leu Ala Leu Gln Arg Arg Thr Thr Arg
                85                  90                  95

Asn Lys Glu Ser Ser Leu Leu Val Ser Glu Leu Glu Asp Thr Thr Glu
            100                 105                 110

Lys Thr Ala Glu Thr Arg Leu Thr Leu Gln Arg Arg Ala Lys Thr Asp
        115                 120                 125

Ser Ala Glu Lys Trp Lys Thr Ala Glu Ile Asp Ser Val Lys Met Thr
    130                 135                 140

Leu Asn Val Gly Gly Glu Thr Glu Asn Asn Gly Val Ser Lys Glu Ser
145                 150                 155                 160

Arg Thr Asn Val Arg Ile Val Asn Asn Ala Lys Asn Ser Phe Val Ala
                165                 170                 175

Ser Ser Val Pro Leu Asp Glu Asp Pro Gln Val Ile Glu Met Met Ala
            180                 185                 190

Asp Lys Lys Tyr Lys Glu Thr Phe Ser Ala Pro Ser Arg Ala Asn Glu
        195                 200                 205

Asn Val Ala Leu Lys Tyr Ser Ser Asn Arg Pro Pro Ile Ala Ser Leu
    210                 215                 220

Ser Gln Thr Glu Val Val Arg Ser Gly His Leu Thr Thr Lys Pro Thr
225                 230                 235                 240

Gln Ser Lys Leu Asp Ile Lys Val Leu Gly Thr Gly Asn Leu Tyr His
                245                 250                 255

Arg Ser Ile Gly Lys Glu Ile Ala Lys Thr Ser Asn Lys Phe Gly Ser
            260                 265                 270

Leu Glu Lys Arg Thr Pro Thr Lys Cys Thr Thr Glu His Lys Leu Thr
        275                 280                 285

Thr Lys Cys Ser Leu Pro Gln Leu Lys Ser Pro Ala Pro Ser Ile Leu
    290                 295                 300

Lys Asn Arg Met Ser Asn Leu Gln Val Lys Gln Arg Pro Lys Ser Ser
305                 310                 315                 320

Phe Leu Ala Asn Lys Gln Glu Arg Ser Ala Glu Asn Thr Ile Leu Pro
                325                 330                 335

Glu Glu Glu Thr Val Val Gln Asn Thr Ser Ala Gly Lys Asp Pro Leu
            340                 345                 350

Lys Val Glu Asn Ser Gln Val Thr Val Ala Val Arg Val Arg Pro Phe
        355                 360                 365

Thr Lys Arg Glu Lys Ile Glu Lys Ala Ser Gln Val Val Phe Met Ser
    370                 375                 380

Gly Lys Glu Ile Thr Val Glu His Pro Asp Thr Lys Gln Val Tyr Asn
385                 390                 395                 400

Phe Ile Tyr Asp Val Ser Phe Trp Ser Phe Asp Glu Cys His Pro His
                405                 410                 415

Tyr Ala Ser Gln Thr Thr Val Tyr Glu Lys Leu Ala Ala Pro Leu Leu
            420                 425                 430

Glu Arg Ala Phe Glu Gly Phe Asn Thr Cys Leu Phe Ala Tyr Gly Gln
        435                 440                 445

Thr Gly Ser Gly Lys Ser Tyr Thr Met Met Gly Phe Ser Glu Glu Pro
    450                 455                 460

Gly Ile Ile Pro Arg Phe Cys Glu Asp Leu Phe Ser Gln Val Ala Arg
465                 470                 475                 480

Lys Gln Thr Gln Glu Val Ser Tyr His Ile Glu Met Ser Phe Phe Glu
                485                 490                 495

Val Tyr Asn Glu Lys Ile His Asp Leu Leu Val Cys Lys Asp Glu Asn
            500                 505                 510

Gly Gln Arg Lys Gln Pro Leu Arg Val Arg Glu His Pro Val Tyr Gly
        515                 520                 525

Pro Tyr Val Glu Ala Leu Ser Met Asn Ile Val Ser Ser Tyr Ala Asp
    530                 535                 540

Ile Gln Ser Trp Leu Glu Leu Gly Asn Lys Gln Arg Ala Thr Ala Ala
545                 550                 555                 560

Thr Gly Met Asn Asp Lys Ser Ser Arg Ser His Ser Val Phe Thr Leu
                565                 570                 575

Val Met Thr Gln Thr Lys Thr Glu Phe Val Glu Gly Glu Glu His Asp
            580                 585                 590

His Arg Ile Thr Ser Arg Ile Asn Leu Ile Asp Leu Ala Gly Ser Glu

595                 600                 605

Arg Cys Ser Thr Ala His Thr Asn Gly Asp Arg Leu Lys Glu Gly Val
    610                 615                 620

Ser Ile Asn Lys Ser Leu Leu Thr Leu Gly Lys Val Ile Ser Ala Leu
625                 630                 635                 640

Ser Glu Gln Ala Asn Gln Arg Ser Val Phe Ile Pro Tyr Arg Glu Ser
                645                 650                 655

Val Leu Thr Trp Leu Leu Lys Glu Ser Leu Gly Gly Asn Ser Lys Thr
            660                 665                 670

Ala Met Ile Ala Thr Ile Ser Pro Ala Ala Ser Asn Ile Glu Glu Thr
        675                 680                 685

Leu Ser Thr Leu Arg Tyr Ala Asn Gln Ala Arg Leu Ile Val Asn Ile
    690                 695                 700

Ala Lys Val Asn Glu Asp Met Asn Ala Lys Leu Ile Arg Glu Leu Lys
705                 710                 715                 720

Ala Glu Ile Ala Lys Leu Lys Ala Ala Gln Arg Asn Ser Arg Asn Ile
                725                 730                 735

Asp Pro Glu Arg Tyr Arg Leu Cys Arg Gln Glu Ile Thr Ser Leu Arg
            740                 745                 750

Met Lys Leu His Gln Gln Glu Arg Asp Met Ala Glu Met Gln Arg Val
        755                 760                 765

Trp Lys Glu Lys Phe Glu Gln Ala Glu Lys Arg Lys Leu Gln Glu Thr
    770                 775                 780

Lys Glu Leu Gln Lys Ala Gly Ile Met Phe Gln Met Asp Asn His Leu
785                 790                 795                 800

Pro Asn Leu Val Asn Leu Asn Glu Asp Pro Gln Leu Ser Glu Met Leu
                805                 810                 815

Leu Tyr Met Ile Lys Glu Gly Thr Thr Thr Val Gly Lys Tyr Lys Pro
            820                 825                 830

Asn Ser Ser His Asp Ile Gln Leu Ser Gly Val Leu Ile Ala Asp Asp
        835                 840                 845

His Cys Thr Ile Lys Asn Phe Gly Gly Thr Val Ser Ile Ile Pro Val
    850                 855                 860

Gly Glu Ala Lys Thr Tyr Val Asn Gly Lys His Ile Leu Glu Ile Thr
865                 870                 875                 880

Val Leu Arg His Gly Asp Arg Val Ile Leu Gly Gly Asp His Tyr Phe
                885                 890                 895

Arg Phe Asn His Pro Val Glu Val Gln Lys Gly Lys Arg Pro Ser Gly
            900                 905                 910

Arg Asp Thr Pro Ile Ser Glu Gly Pro Lys Asp Phe Glu Phe Ala Lys
        915                 920                 925

Asn Glu Leu Leu Met Ala Gln Arg Ser Gln Leu Glu Ala Glu Ile Lys
    930                 935                 940

Glu Ala Gln Leu Lys Ala Lys Glu Glu Met Met Gln Gly Ile Gln Ile
945                 950                 955                 960

Ala Lys Glu Met Ala Gln Gln Glu Leu Ser Ser Gln Lys Ala Ala Tyr
                965                 970                 975

Glu Ser Lys Ile Lys Ala Leu Glu Ala Glu Leu Arg Glu Glu Ser Gln
            980                 985                 990

Arg Lys Lys Met Gln Glu Ile Asn  Asn Gln Lys Ala Asn  His Lys Ile
        995                 1000                 1005

Glu Glu  Leu Glu Lys Ala Lys  Gln His Leu Glu Gln  Glu Ile Tyr
    1010                 1015                 1020

Val Asn  Lys Lys Arg Leu Glu  Met Glu Thr Leu Ala  Thr Lys Gln
    1025                 1030                 1035

Ala Leu  Glu Asp His Ser Ile  Arg His Ala Arg Ile  Leu Glu Ala
    1040                 1045                 1050

Leu Glu  Thr Glu Lys Gln Lys  Ile Ala Lys Glu Val  Gln Ile Leu
    1055                 1060                 1065

Gln Gln  Asn Arg Asn Asn Arg  Asp Lys Thr Phe Thr  Val Gln Thr
    1070                 1075                 1080

Thr Trp  Ser Ser Met Lys Leu  Ser Met Met Ile Gln  Glu Ala Asn
    1085                 1090                 1095

Ala Ile  Ser Ser Lys Leu Lys  Thr Tyr Tyr Val Phe  Gly Arg His
    1100                 1105                 1110

Asp Ile  Ser Asp Lys Ser Ser  Ser Asp Thr Ser Ile  Arg Val Arg
    1115                 1120                 1125

Asn Leu  Lys Leu Gly Ile Ser  Thr Phe Trp Ser Leu  Glu Lys Phe
    1130                 1135                 1140

Glu Ser  Lys Leu Ala Ala Met  Lys Glu Leu Tyr Glu  Ser Asn Gly

1145                 1150                 1155

Ser Asn Arg Gly Glu Asp Ala Phe Cys Asp Pro Glu Asp Glu Trp
    1160                 1165                 1170

Glu Pro Asp Ile Thr Asp Ala Pro Val Ser Ser Leu Ser Arg Arg
    1175                 1180                 1185

Arg Ser Arg Ser Leu Met Lys Asn Arg Arg Ile Ser Gly Cys Leu
    1190                 1195                 1200

His Asp Ile Gln Val His Pro Ile Lys Asn Leu His Ser Ser His
    1205                 1210                 1215

Ser Ser Gly Leu Met Asp Lys Ser Ser Thr Ile Tyr Ser Asn Ser
    1220                 1225                 1230

Ala Glu Ser Phe Leu Pro Gly Ile Cys Lys Glu Leu Ile Gly Ser
    1235                 1240                 1245

Ser Leu Asp Phe Phe Gly Gln Ser Tyr Asp Glu Glu Arg Thr Ile
    1250                 1255                 1260

Ala Asp Ser Leu Ile Asn Ser Phe Leu Lys Ile Tyr Asn Gly Leu
    1265                 1270                 1275

Phe Ala Ile Ser Lys Ala His Glu Glu Gln Asp Glu Glu Ser Gln
    1280                 1285                 1290

Asp Asn Leu Phe Ser Ser Asp Arg Ala Ile Gln Ser Leu Thr Ile
    1295                 1300                 1305

Gln Thr Ala Cys Ala Phe Glu Gln Leu Val Val Leu Met Lys His
    1310                 1315                 1320

Trp Leu Ser Asp Leu Leu Pro Cys Thr Asn Ile Ala Arg Leu Glu
    1325                 1330                 1335

Asp Glu Leu Arg Gln Glu Val Lys Lys Leu Gly Gly Tyr Leu Gln
    1340                 1345                 1350

Leu Phe Leu Gln Gly Cys Cys Leu Asp Ile Ser Ser Met Ile Lys
    1355                 1360                 1365

Glu Ala Gln Lys Asn Ala Ile Gln Ile Val Gln Gln Ala Val Lys
    1370                 1375                 1380

Tyr Val Gly Gln Leu Ala Val Leu Lys Gly Ser Lys Leu His Phe
    1385                 1390                 1395

Leu Glu Asn Gly Asn Asn Lys Ala Ala Ser Val Gln Glu Glu Phe
    1400                 1405                 1410

Met Asp Ala Val Cys Asp Gly Val Gly Leu Gly Met Lys Ile Leu
    1415                1420                1425

Leu Asp Ser Gly Leu Glu Lys Ala Lys Glu Leu Gln His Glu Leu
    1430                1435                1440

Phe Arg Gln Cys Thr Lys Asn Glu Val Thr Lys Glu Met Lys Thr
    1445                1450                1455

Asn Ala Met Gly Leu Ile Arg Ser Leu Glu Asn Ile Phe Ala Glu
    1460                1465                1470

Ser Lys Ile Lys Ser Phe Arg Arg Gln Val Gln Glu Glu Asn Phe
    1475                1480                1485

Glu Tyr Gln Asp Phe Lys Arg Met Val Asn Arg Ala Pro Glu Phe
    1490                1495                1500

Leu Lys Leu Lys His Cys Leu Glu Lys Ala Ile Glu Ile Ile Ile
    1505                1510                1515

Ser Ala Leu Lys Gly Cys His Ser Asp Ile Asn Leu Leu Gln Thr
    1520                1525                1530

Cys Val Glu Ser Ile Arg Asn Leu Ala Ser Asp Phe Tyr Ser Asp
    1535                1540                1545

Phe Ser Val Pro Ser Thr Ser Val Gly Ser Tyr Glu Ser Arg Val
    1550                1555                1560

Thr His Ile Val His Gln Glu Leu Glu Ser Leu Ala Lys Ser Leu
    1565                1570                1575

Leu Phe Cys Phe Glu Ser Glu Glu Ser Pro Asp Leu Leu Lys Pro
    1580                1585                1590

Trp Glu Thr Tyr Asn Gln Asn Thr Lys Glu Glu His Gln Gln Ser
    1595                1600                1605

Lys Ser Ser Gly Ile Asp Gly Ser Lys Asn Lys Gly Val Pro Lys
    1610                1615                1620

Arg Val Tyr Glu Leu His Gly Ser Ser Pro Ala Val Ser Ser Glu
    1625                1630                1635

Glu Cys Thr Pro Ser Arg Ile Gln Trp Val
    1640                1645

<210> 287
<211> 778
<212> PRT
<213> Homo sapiens

<400> 287

Met Thr Thr Arg Gln Ala Thr Lys Asp Pro Leu Leu Arg Gly Val Ser
1               5                   10                  15

Pro Thr Pro Ser Lys Ile Pro Val Arg Ser Gln Lys Arg Thr Pro Phe
            20                  25                  30

Pro Thr Val Thr Ser Cys Ala Val Asp Gln Glu Asn Gln Asp Pro Arg
        35                  40                  45

Arg Trp Val Gln Lys Pro Pro Leu Asn Ile Gln Arg Pro Leu Val Asp
    50                  55                  60

Ser Ala Gly Pro Arg Pro Lys Ala Arg His Gln Ala Glu Thr Ser Gln
65                  70                  75                  80

Arg Leu Val Gly Ile Ser Gln Pro Arg Asn Pro Leu Glu Glu Leu Arg
                85                  90                  95

Pro Ser Pro Arg Gly Gln Asn Val Gly Pro Gly Pro Pro Ala Gln Thr
            100                 105                 110

Glu Ala Pro Gly Thr Ile Glu Phe Val Ala Asp Pro Ala Ala Leu Ala
        115                 120                 125

Thr Ile Leu Ser Gly Glu Gly Val Lys Ser Cys His Leu Gly Arg Gln
    130                 135                 140

Pro Ser Leu Ala Lys Arg Val Leu Val Arg Gly Ser Gln Gly Gly Thr
145                 150                 155                 160

Thr Gln Arg Val Gln Gly Val Arg Ala Ser Ala Tyr Leu Ala Pro Arg
                165                 170                 175

Thr Pro Thr His Arg Leu Asp Pro Ala Arg Ala Ser Cys Phe Ser Arg
            180                 185                 190

Leu Glu Gly Pro Gly Pro Arg Gly Arg Thr Leu Cys Pro Gln Arg Leu
        195                 200                 205

Gln Ala Leu Ile Ser Pro Ser Gly Pro Ser Phe His Pro Ser Thr His
    210                 215                 220

Pro Ser Phe Gln Glu Leu Arg Arg Glu Thr Ala Gly Ser Ser Arg Thr
225                 230                 235                 240

Ser Val Ser Gln Ala Ser Gly Leu Leu Leu Glu Thr Pro Val Gln Pro
                245                 250                 255

Ala Phe Ser Leu Pro Lys Gly Glu Arg Glu Val Val Thr His Ser Asp
            260                 265                 270

Glu Gly Gly Val Ala Ser Leu Gly Leu Ala Gln Arg Val Pro Leu Arg
        275                 280                 285

Glu Asn Arg Glu Met Ser His Thr Arg Asp Ser His Asp Ser His Leu
    290                 295                 300

Met Pro Ser Pro Ala Pro Val Ala Gln Pro Leu Pro Gly His Val Val
305                 310                 315                 320

Pro Cys Pro Ser Pro Phe Gly Arg Ala Gln Arg Val Pro Ser Pro Gly
                325                 330                 335

Pro Pro Thr Leu Thr Ser Tyr Ser Val Leu Arg Arg Leu Thr Val Gln
            340                 345                 350

Pro Lys Thr Arg Phe Thr Pro Met Pro Ser Thr Pro Arg Val Gln Gln
        355                 360                 365

Ala Gln Trp Leu Arg Gly Val Ser Pro Gln Ser Cys Ser Glu Asp Pro
    370                 375                 380

Ala Leu Pro Trp Glu Gln Val Ala Val Arg Leu Phe Asp Gln Glu Ser
385                 390                 395                 400

Cys Ile Arg Ser Leu Glu Gly Ser Gly Lys Pro Pro Val Ala Thr Pro
                405                 410                 415

Ser Gly Pro His Ser Asn Arg Thr Pro Ser Leu Gln Glu Val Lys Ile
            420                 425                 430

Gln Arg Ile Gly Ile Leu Gln Gln Leu Leu Arg Gln Glu Val Glu Gly
        435                 440                 445

Leu Val Gly Gly Gln Cys Val Pro Leu Asn Gly Gly Ser Ser Leu Asp
    450                 455                 460

Met Val Glu Leu Gln Pro Leu Leu Thr Glu Ile Ser Arg Thr Leu Asn
465                 470                 475                 480

Ala Thr Glu His Asn Ser Gly Thr Ser His Leu Pro Gly Leu Leu Lys
                485                 490                 495

His Ser Gly Leu Pro Lys Pro Cys Leu Pro Glu Glu Cys Gly Glu Pro
            500                 505                 510

Gln Pro Cys Pro Pro Ala Glu Pro Gly Pro Pro Glu Ala Phe Cys Arg
        515                 520                 525

Ser Glu Pro Glu Ile Pro Glu Pro Ser Leu Gln Glu Gln Leu Glu Val
    530                 535                 540

Pro Glu Pro Tyr Pro Pro Ala Glu Pro Arg Pro Leu Glu Ser Cys Cys
545                 550                 555                 560

Arg Ser Glu Pro Glu Ile Pro Glu Ser Ser Arg Gln Glu Gln Leu Glu
                565                 570                 575

Val Pro Glu Pro Cys Pro Pro Ala Glu Pro Arg Pro Leu Glu Ser Tyr
            580                 585                 590

Cys Arg Ile Glu Pro Glu Ile Pro Glu Ser Ser Arg Gln Glu Gln Leu
        595                 600                 605

Glu Val Pro Glu Pro Cys Pro Pro Ala Glu Pro Gly Pro Leu Gln Pro
    610                 615                 620

Ser Thr Gln Gly Gln Ser Gly Pro Pro Gly Pro Cys Pro Arg Val Glu
625                 630                 635                 640

Leu Gly Ala Ser Glu Pro Cys Thr Leu Glu His Arg Ser Leu Glu Ser
                645                 650                 655

Ser Leu Pro Pro Cys Cys Ser Gln Trp Ala Pro Ala Thr Thr Ser Leu
            660                 665                 670

Ile Phe Ser Ser Gln His Pro Leu Cys Ala Ser Pro Pro Ile Cys Ser
        675                 680                 685

Leu Gln Ser Leu Arg Pro Pro Ala Gly Gln Ala Gly Leu Ser Asn Leu
    690                 695                 700

Ala Pro Arg Thr Leu Ala Leu Arg Glu Arg Leu Lys Ser Cys Leu Thr
705                 710                 715                 720

Ala Ile His Cys Phe His Glu Ala Arg Leu Asp Asp Glu Cys Ala Phe
                725                 730                 735

Tyr Thr Ser Arg Ala Pro Pro Ser Gly Pro Thr Arg Val Cys Thr Asn
            740                 745                 750

Pro Val Ala Thr Leu Leu Glu Trp Gln Asp Ala Leu Cys Phe Ile Pro
        755                 760                 765

Val Gly Ser Ala Ala Pro Gln Gly Ser Pro
    770                 775

<210>  288
<211>  956
<212>  PRT
<213>  Homo sapiens

<400>  288

Met Leu Glu Glu Leu Glu Cys Gly Ala Pro Gly Ala Arg Gly Ala Ala
1               5                   10                  15

Thr Ala Met Asp Cys Lys Asp Arg Pro Ala Phe Pro Val Lys Lys Leu

20                  25                  30

Ile Gln Ala Arg Leu Pro Phe Lys Arg Leu Asn Leu Val Pro Lys Gly
    35                  40                  45

Lys Ala Asp Asp Met Ser Asp Asp Gln Gly Thr Ser Val Gln Ser Lys
    50                  55                  60

Ser Pro Asp Leu Glu Ala Ser Leu Asp Thr Leu Glu Asn Asn Cys His
65                  70                  75                  80

Val Gly Ser Asp Ile Asp Phe Arg Pro Lys Leu Val Asn Gly Lys Gly
                85                  90                  95

Pro Leu Asp Asn Phe Leu Arg Asn Arg Ile Glu Thr Ser Ile Gly Gln
            100                 105                 110

Ser Thr Val Ile Ile Asp Leu Thr Glu Asp Ser Asn Glu Gln Pro Asp
    115                 120                 125

Ser Leu Val Asp His Asn Lys Leu Asn Ser Glu Ala Ser Pro Ser Arg
    130                 135                 140

Glu Ala Ile Asn Gly Gln Arg Glu Asp Thr Gly Asp Gln Gln Gly Leu
145                 150                 155                 160

Leu Lys Ala Ile Gln Asn Asp Lys Leu Ala Phe Pro Gly Glu Thr Leu
                165                 170                 175

Ser Asp Ile Pro Cys Lys Thr Glu Glu Glu Gly Val Gly Cys Gly Gly
            180                 185                 190

Ala Gly Arg Arg Gly Asp Ser Gln Glu Cys Ser Pro Arg Ser Cys Pro
    195                 200                 205

Glu Leu Thr Ser Gly Pro Arg Met Cys Pro Arg Lys Glu Gln Asp Ser
    210                 215                 220

Trp Ser Glu Ala Gly Gly Ile Leu Phe Lys Gly Lys Val Pro Met Val
225                 230                 235                 240

Val Leu Gln Asp Ile Leu Ala Val Arg Pro Pro Gln Ile Lys Ser Leu
                245                 250                 255

Pro Ala Thr Pro Gln Gly Lys Asn Met Thr Pro Glu Ser Glu Val Leu
            260                 265                 270

Glu Ser Phe Pro Glu Glu Asp Ser Val Leu Ser His Ser Ser Leu Ser
    275                 280                 285

Ser Pro Ser Ser Thr Ser Ser Pro Glu Gly Pro Pro Ala Pro Pro Lys
    290                 295                 300

Gln His Ser Ser Thr Ser Pro Phe Pro Thr Ser Thr Pro Leu Arg Arg
305                 310                 315                 320

Ile Thr Lys Lys Phe Val Lys Gly Ser Thr Glu Lys Asn Lys Leu Arg
                325                 330                 335

Leu Gln Arg Asp Gln Glu Arg Leu Gly Lys Gln Leu Lys Leu Arg Ala
            340                 345                 350

Glu Arg Glu Glu Lys Glu Lys Leu Lys Glu Glu Ala Lys Arg Ala Lys
        355                 360                 365

Glu Glu Ala Lys Lys Lys Lys Glu Glu Glu Lys Glu Leu Lys Glu Lys
    370                 375                 380

Glu Arg Arg Glu Lys Arg Glu Lys Asp Glu Lys Glu Lys Ala Glu Lys
385                 390                 395                 400

Gln Arg Leu Lys Glu Glu Arg Arg Lys Glu Arg Gln Glu Ala Leu Glu
                405                 410                 415

Ala Lys Leu Glu Glu Lys Arg Lys Lys Glu Glu Glu Lys Arg Leu Arg
            420                 425                 430

Glu Glu Glu Lys Arg Ile Lys Ala Glu Lys Ala Glu Ile Thr Arg Phe
        435                 440                 445

Phe Gln Lys Pro Lys Thr Pro Gln Ala Pro Lys Thr Leu Ala Gly Ser
    450                 455                 460

Cys Gly Lys Phe Ala Pro Phe Glu Ile Lys Glu His Met Val Leu Ala
465                 470                 475                 480

Pro Arg Arg Arg Thr Ala Phe His Pro Asp Leu Cys Ser Gln Leu Asp
                485                 490                 495

Gln Leu Leu Gln Gln Gln Ser Gly Glu Phe Ser Phe Leu Lys Asp Leu
            500                 505                 510

Lys Gly Arg Gln Pro Leu Arg Ser Gly Pro Thr His Val Ser Thr Arg
        515                 520                 525

Asn Ala Asp Ile Phe Asn Ser Asp Val Val Ile Val Glu Arg Gly Lys
    530                 535                 540

Gly Asp Gly Val Pro Glu Arg Arg Lys Phe Gly Arg Met Lys Leu Leu
545                 550                 555                 560

Gln Phe Cys Glu Asn His Arg Pro Ala Tyr Trp Gly Thr Trp Asn Lys
                565                 570                 575

Lys Thr Ala Leu Ile Arg Ala Arg Asp Pro Trp Ala Gln Asp Thr Lys

580                 585                 590

Leu Leu Asp Tyr Glu Val Asp Ser Asp Glu Glu Trp Glu Glu Glu Glu
        595                 600                 605

Pro Gly Glu Ser Leu Ser His Ser Glu Gly Asp Asp Asp Asp Asp Met
    610                 615                 620

Gly Glu Asp Glu Asp Glu Asp Asp Gly Phe Phe Val Pro His Gly Tyr
625                 630                 635                 640

Leu Ser Glu Asp Glu Gly Val Thr Glu Glu Cys Ala Asp Pro Glu Asn
                645                 650                 655

His Lys Val Arg Gln Lys Leu Lys Ala Lys Glu Trp Asp Glu Phe Leu
            660                 665                 670

Ala Lys Gly Lys Arg Phe Arg Val Leu Gln Pro Val Lys Ile Gly Cys
        675                 680                 685

Val Trp Ala Ala Asp Arg Asp Cys Ala Gly Asp Asp Leu Lys Val Leu
    690                 695                 700

Gln Gln Phe Ala Ala Cys Phe Leu Glu Thr Leu Pro Ala Gln Glu Glu
705                 710                 715                 720

Gln Thr Pro Lys Ala Ser Lys Arg Glu Arg Arg Asp Glu Gln Ile Leu
                725                 730                 735

Ala Gln Leu Leu Pro Leu Leu His Gly Asn Val Asn Gly Ser Lys Val
            740                 745                 750

Ile Ile Arg Glu Phe Gln Glu His Cys Arg Arg Gly Leu Leu Ser Asn
        755                 760                 765

His Thr Gly Ser Pro Arg Ser Pro Ser Thr Thr Tyr Leu His Thr Pro
    770                 775                 780

Thr Pro Ser Glu Asp Ala Ala Ile Pro Ser Lys Ser Arg Leu Lys Arg
785                 790                 795                 800

Leu Ile Ser Glu Asn Ser Val Tyr Glu Lys Arg Pro Asp Phe Arg Met
                805                 810                 815

Cys Trp Tyr Val His Pro Gln Val Leu Gln Ser Phe Gln Gln Glu His
            820                 825                 830

Leu Pro Val Pro Cys Gln Trp Ser Tyr Val Thr Ser Val Pro Ser Ala
        835                 840                 845

Pro Lys Glu Asp Ser Gly Ser Val Pro Ser Thr Gly Pro Ser Gln Gly
    850                 855                 860

Thr Pro Ile Ser Leu Lys Arg Lys Ser Ala Gly Ser Met Cys Ile Thr
865                 870                 875                 880

Gln Phe Met Lys Lys Arg Arg His Asp Gly Gln Ile Gly Ala Glu Asp
                885                 890                 895

Met Asp Gly Phe Gln Ala Asp Thr Glu Glu Glu Glu Glu Glu Glu Gly
            900                 905                 910

Asp Cys Met Ile Val Asp Val Pro Asp Ala Ala Glu Val Gln Ala Pro
        915                 920                 925

Cys Gly Ala Ala Ser Gly Ala Gly Gly Gly Val Gly Val Asp Thr Gly
    930                 935                 940

Lys Ala Thr Leu Thr Ala Ser Pro Leu Gly Ala Ser
945                 950                 955

<210>  289
<211>  534
<212>  PRT
<213>  Homo sapiens

<400>  289

Met Pro Val Ala Gly Gly Lys Ala Asn Lys Asp Arg Thr Glu Asp Lys
1               5                   10                  15

Gln Asp Gly Met Pro Gly Arg Ser Trp Ala Ser Lys Arg Val Ser Glu
            20                  25                  30

Ser Val Lys Ala Leu Leu Leu Lys Gly Lys Ala Pro Val Asp Pro Glu
        35                  40                  45

Cys Thr Ala Lys Val Gly Lys Ala His Val Tyr Cys Glu Gly Asn Asp
    50                  55                  60

Val Tyr Asp Val Met Leu Asn Gln Thr Asn Leu Gln Phe Asn Asn Asn
65                  70                  75                  80

Lys Tyr Tyr Leu Ile Gln Leu Leu Glu Asp Asp Ala Gln Arg Asn Phe
                85                  90                  95

Ser Val Trp Met Arg Trp Gly Arg Val Gly Lys Met Gly Gln His Ser
            100                 105                 110

Leu Val Ala Cys Ser Gly Asn Leu Asn Lys Ala Lys Glu Ile Phe Gln
        115                 120                 125

Lys Lys Phe Leu Asp Lys Thr Lys Asn Asn Trp Glu Asp Arg Glu Lys
    130                 135                 140

Phe Glu Lys Val Pro Gly Lys Tyr Asp Met Leu Gln Met Asp Tyr Ala
145                 150                 155                 160

Thr Asn Thr Gln Asp Glu Glu Glu Thr Lys Lys Glu Glu Ser Leu Lys
                165                 170                 175

Ser Pro Leu Lys Pro Glu Ser Gln Leu Asp Leu Arg Val Gln Glu Leu
            180                 185                 190

Ile Lys Leu Ile Cys Asn Val Gln Ala Met Glu Glu Met Met Met Glu
        195                 200                 205

Met Lys Tyr Asn Thr Lys Lys Ala Pro Leu Gly Lys Leu Thr Val Ala
    210                 215                 220

Gln Ile Lys Ala Gly Tyr Gln Ser Leu Lys Lys Ile Glu Asp Cys Ile
225                 230                 235                 240

Arg Ala Gly Gln His Gly Arg Ala Leu Met Glu Ala Cys Asn Glu Phe
                245                 250                 255

Tyr Thr Arg Ile Pro His Asp Phe Gly Leu Arg Thr Pro Pro Leu Ile
            260                 265                 270

Arg Thr Gln Lys Glu Leu Ser Glu Lys Ile Gln Leu Leu Glu Ala Leu
        275                 280                 285

Gly Asp Ile Glu Ile Ala Ile Lys Leu Val Lys Thr Glu Leu Gln Ser
    290                 295                 300

Pro Glu His Pro Leu Asp Gln His Tyr Arg Asn Leu His Cys Ala Leu
305                 310                 315                 320

Arg Pro Leu Asp His Glu Ser Tyr Glu Phe Lys Val Ile Ser Gln Tyr
                325                 330                 335

Leu Gln Ser Thr His Ala Pro Thr His Ser Asp Tyr Thr Met Thr Leu
            340                 345                 350

Leu Asp Leu Phe Glu Val Glu Lys Asp Gly Glu Lys Glu Ala Phe Arg
        355                 360                 365

Glu Asp Leu His Asn Arg Met Leu Leu Trp His Gly Ser Arg Met Ser
    370                 375                 380

Asn Trp Val Gly Ile Leu Ser His Gly Leu Arg Ile Ala His Pro Glu
385                 390                 395                 400

Ala Pro Ile Thr Gly Tyr Met Phe Gly Lys Gly Ile Tyr Phe Ala Asp
                405                 410                 415

Met Ser Ser Lys Ser Ala Asn Tyr Cys Phe Ala Ser Arg Leu Lys Asn
            420                 425                 430

Thr Gly Leu Leu Leu Leu Ser Glu Val Ala Leu Gly Gln Cys Asn Glu
        435                 440                 445

Leu Leu Glu Ala Asn Pro Lys Ala Glu Gly Leu Leu Gln Gly Lys His
    450                 455                 460

Ser Thr Lys Gly Leu Gly Lys Met Ala Pro Ser Ser Ala His Phe Val
465                 470                 475                 480

Thr Leu Asn Gly Ser Thr Val Pro Leu Gly Pro Ala Ser Asp Thr Gly
                485                 490                 495

Ile Leu Asn Pro Asp Gly Tyr Thr Leu Asn Tyr Asn Glu Tyr Ile Val
            500                 505                 510

Tyr Asn Pro Asn Gln Val Arg Met Arg Tyr Leu Leu Lys Val Gln Phe
        515                 520                 525

Asn Phe Leu Gln Leu Trp
    530

<210>  290
<211>  925
<212>  PRT
<213>  Homo sapiens

<400>  290

Met Pro Arg Lys Gly Thr Gln Pro Ser Thr Ala Arg Arg Arg Glu Glu
1               5                   10                  15

Gly Pro Pro Pro Pro Ser Pro Asp Gly Ala Ser Ser Asp Ala Glu Pro
            20                  25                  30

Glu Pro Pro Ser Gly Arg Thr Glu Ser Pro Ala Thr Ala Ala Ala Met
        35                  40                  45

Thr Asn Glu Ala Gly Ala Pro Arg Leu Met Ile Thr His Ile Val Asn
    50                  55                  60

Gln Asn Phe Lys Ser Tyr Ala Gly Glu Lys Ile Leu Gly Pro Phe His
65                  70                  75                  80

Lys Arg Phe Ser Cys Ile Ile Gly Pro Asn Gly Ser Gly Lys Ser Asn
                85                  90                  95

Val Ile Asp Ser Met Leu Phe Val Phe Gly Tyr Arg Ala Gln Lys Ile
            100                 105                 110

Arg Ser Lys Lys Leu Ser Val Leu Ile His Asn Ser Asp Glu His Lys
        115                 120                 125

Asp Ile Gln Ser Cys Thr Val Glu Val His Phe Gln Lys Ile Ile Asp
    130                 135                 140

Lys Glu Gly Asp Asp Tyr Glu Val Ile Pro Asn Ser Asn Phe Tyr Val
145                 150                 155                 160

Ser Arg Thr Ala Cys Arg Asp Asn Thr Ser Val Tyr His Ile Ser Gly
                165                 170                 175

Lys Lys Lys Thr Phe Lys Asp Val Gly Asn Leu Leu Arg Ser His Gly
            180                 185                 190

Ile Asp Leu Asp His Asn Arg Phe Leu Ile Leu Gln Gly Glu Val Glu
        195                 200                 205

Gln Ile Ala Met Met Lys Pro Lys Gly Gln Thr Glu His Asp Glu Gly
    210                 215                 220

Met Leu Glu Tyr Leu Glu Asp Ile Ile Gly Cys Gly Arg Leu Asn Glu
225                 230                 235                 240

Pro Ile Lys Val Leu Cys Arg Arg Val Glu Ile Leu Asn Glu His Arg
                245                 250                 255

Gly Glu Lys Leu Asn Arg Val Lys Met Val Glu Lys Glu Lys Asp Ala
            260                 265                 270

Leu Glu Gly Glu Lys Asn Ile Ala Ile Glu Phe Leu Thr Leu Glu Asn
        275                 280                 285

Glu Ile Phe Arg Lys Lys Asn His Val Cys Gln Tyr Tyr Ile Tyr Glu
    290                 295                 300

Leu Gln Lys Arg Ile Ala Glu Met Glu Thr Gln Lys Glu Lys Ile His
305                 310                 315                 320

Glu Asp Thr Lys Glu Ile Asn Glu Lys Ser Asn Ile Leu Ser Asn Glu
                325                 330                 335

Met Lys Ala Lys Asn Lys Asp Val Lys Asp Thr Glu Lys Lys Leu Asn
            340                 345                 350

Lys Ile Thr Lys Phe Ile Glu Glu Asn Lys Glu Lys Phe Thr Gln Leu
        355                 360                 365

Asp Leu Glu Asp Val Gln Val Arg Glu Lys Leu Lys His Ala Thr Ser
    370                 375                 380

Lys Ala Lys Lys Leu Glu Lys Gln Leu Gln Lys Asp Lys Glu Lys Val
385                 390                 395                 400

Glu Glu Phe Lys Ser Ile Pro Ala Lys Ser Asn Asn Ile Ile Asn Glu
                405                 410                 415

Thr Thr Thr Arg Asn Asn Ala Leu Glu Lys Glu Lys Glu Lys Glu Glu

420                 425                 430

Lys Lys Leu Lys Glu Val Met Asp Ser Leu Lys Gln Glu Thr Gln Gly
        435                 440                 445

Leu Gln Lys Glu Lys Glu Ser Arg Glu Lys Glu Leu Met Gly Phe Ser
    450                 455                 460

Lys Ser Val Asn Glu Ala Arg Ser Lys Met Asp Val Ala Gln Ser Glu
465                 470                 475                 480

Leu Asp Ile Tyr Leu Ser Arg His Asn Thr Ala Val Ser Gln Leu Thr
                485                 490                 495

Lys Ala Lys Glu Ala Leu Ile Ala Ala Ser Glu Thr Leu Lys Glu Arg
            500                 505                 510

Lys Ala Ala Ile Arg Asp Ile Glu Gly Lys Leu Pro Gln Thr Glu Gln
        515                 520                 525

Glu Leu Lys Glu Lys Glu Lys Glu Leu Gln Lys Leu Thr Gln Glu Glu
    530                 535                 540

Thr Asn Phe Lys Ser Leu Val His Asp Leu Phe Gln Lys Val Glu Glu
545                 550                 555                 560

Ala Lys Ser Ser Leu Ala Met Asn Arg Ser Arg Gly Lys Val Leu Asp
                565                 570                 575

Ala Ile Ile Gln Glu Lys Lys Ser Gly Arg Ile Pro Gly Ile Tyr Gly
            580                 585                 590

Arg Leu Gly Asp Leu Gly Ala Ile Asp Glu Lys Tyr Asp Val Ala Ile
        595                 600                 605

Ser Ser Cys Cys His Ala Leu Asp Tyr Ile Val Val Asp Ser Ile Asp
    610                 615                 620

Ile Ala Gln Glu Cys Val Asn Phe Leu Lys Arg Gln Asn Ile Gly Val
625                 630                 635                 640

Ala Thr Phe Ile Gly Leu Asp Lys Met Ala Val Trp Ala Lys Lys Met
                645                 650                 655

Thr Glu Ile Gln Thr Pro Glu Asn Thr Pro Arg Leu Phe Asp Leu Val
            660                 665                 670

Lys Val Lys Asp Glu Lys Ile Arg Gln Ala Phe Tyr Phe Ala Leu Arg
        675                 680                 685

Asp Thr Leu Val Ala Asp Asn Leu Asp Gln Ala Thr Arg Val Ala Tyr
    690                 695                 700

Gln Lys Asp Arg Arg Trp Arg Val Val Thr Leu Gln Gly Gln Ile Ile
705                 710                 715                 720

Glu Gln Ser Gly Thr Met Thr Gly Gly Gly Ser Lys Val Met Lys Gly
                725                 730                 735

Arg Met Gly Ser Ser Leu Val Ile Glu Ile Ser Glu Glu Glu Val Asn
            740                 745                 750

Lys Met Glu Ser Gln Leu Gln Asn Asp Ser Lys Lys Ala Met Gln Ile
        755                 760                 765

Gln Glu Gln Lys Val Gln Leu Glu Glu Arg Val Val Lys Leu Arg His
    770                 775                 780

Ser Glu Arg Glu Met Arg Asn Thr Leu Glu Lys Phe Thr Ala Ser Ile
785                 790                 795                 800

Gln Arg Leu Ile Glu Gln Glu Glu Tyr Leu Asn Val Gln Val Lys Glu
                805                 810                 815

Leu Glu Ala Asn Val Leu Ala Thr Ala Pro Asp Lys Lys Lys Gln Lys
            820                 825                 830

Leu Leu Glu Glu Asn Val Ser Ala Phe Lys Thr Glu Tyr Asp Ala Val
        835                 840                 845

Ala Glu Lys Ala Gly Lys Val Glu Ala Glu Val Lys Arg Leu His Asn
    850                 855                 860

Thr Ile Val Glu Ile Asn Asn His Lys Leu Lys Ala Gln Gln Asp Lys
865                 870                 875                 880

Leu Asp Lys Ile Asn Lys Gln Leu Asp Glu Cys Ala Ser Ala Ile Thr
                885                 890                 895

Lys Ala Gln Val Ala Ile Lys Thr Ala Asp Arg Pro Ser Lys Gly Thr
            900                 905                 910

Arg Leu Cys Leu Ala Tyr Arg Glu Arg Asn Lys Arg Tyr
        915                 920                 925

<210>  291
<211>  1288
<212>  PRT
<213>  Homo sapiens

<400>  291

Met Pro Arg Lys Gly Thr Gln Pro Ser Thr Ala Arg Arg Arg Glu Glu
1               5                   10                  15

Gly Pro Pro Pro Pro Ser Pro Asp Gly Ala Ser Ser Asp Ala Glu Pro
            20                  25                  30

Glu Pro Pro Ser Gly Arg Thr Glu Ser Pro Ala Thr Ala Ala Glu Thr
        35                  40                  45

Ala Ser Glu Glu Leu Asp Asn Arg Ser Leu Glu Glu Ile Leu Asn Ser
    50                  55                  60

Ile Pro Pro Pro Pro Pro Pro Ala Met Thr Asn Glu Ala Gly Ala Pro
65                  70                  75                  80

Arg Leu Met Ile Thr His Ile Val Asn Gln Asn Phe Lys Ser Tyr Ala
                85                  90                  95

Gly Glu Lys Ile Leu Gly Pro Phe His Lys Arg Phe Ser Cys Ile Ile
            100                 105                 110

Gly Pro Asn Gly Ser Gly Lys Ser Asn Val Ile Asp Ser Met Leu Phe
        115                 120                 125

Val Phe Gly Tyr Arg Ala Gln Lys Ile Arg Ser Lys Lys Leu Ser Val
    130                 135                 140

Leu Ile His Asn Ser Asp Glu His Lys Asp Ile Gln Ser Cys Thr Val
145                 150                 155                 160

Glu Val His Phe Gln Lys Ile Ile Asp Lys Glu Gly Asp Asp Tyr Glu
                165                 170                 175

Val Ile Pro Asn Ser Asn Phe Tyr Val Ser Arg Thr Ala Cys Arg Asp
            180                 185                 190

Asn Thr Ser Val Tyr His Ile Ser Gly Lys Lys Lys Thr Phe Lys Asp
        195                 200                 205

Val Gly Asn Leu Leu Arg Ser His Gly Ile Asp Leu Asp His Asn Arg
    210                 215                 220

Phe Leu Ile Leu Gln Gly Glu Val Glu Gln Ile Ala Met Met Lys Pro
225                 230                 235                 240

Lys Gly Gln Thr Glu His Asp Glu Gly Met Leu Glu Tyr Leu Glu Asp
                245                 250                 255

Ile Ile Gly Cys Gly Arg Leu Asn Glu Pro Ile Lys Val Leu Cys Arg
            260                 265                 270

Arg Val Glu Ile Leu Asn Glu His Arg Gly Glu Lys Leu Asn Arg Val
        275                 280                 285

Lys Met Val Glu Lys Glu Lys Asp Ala Leu Glu Gly Glu Lys Asn Ile
    290                 295                 300

Ala Ile Glu Phe Leu Thr Leu Glu Asn Glu Ile Phe Arg Lys Lys Asn
305                 310                 315                 320

His Val Cys Gln Tyr Tyr Ile Tyr Glu Leu Gln Lys Arg Ile Ala Glu
                325                 330                 335

Met Glu Thr Gln Lys Glu Lys Ile His Glu Asp Thr Lys Glu Ile Asn
            340                 345                 350

Glu Lys Ser Asn Ile Leu Ser Asn Glu Met Lys Ala Lys Asn Lys Asp
        355                 360                 365

Val Lys Asp Thr Glu Lys Lys Leu Asn Lys Ile Thr Lys Phe Ile Glu
    370                 375                 380

Glu Asn Lys Glu Lys Phe Thr Gln Leu Asp Leu Glu Asp Val Gln Val
385                 390                 395                 400

Arg Glu Lys Leu Lys His Ala Thr Ser Lys Ala Lys Lys Leu Glu Lys
                405                 410                 415

Gln Leu Gln Lys Asp Lys Glu Lys Val Glu Glu Phe Lys Ser Ile Pro
            420                 425                 430

Ala Lys Ser Asn Asn Ile Ile Asn Glu Thr Thr Thr Arg Asn Asn Ala
        435                 440                 445

Leu Glu Lys Glu Lys Glu Lys Glu Glu Lys Lys Leu Lys Glu Val Met
    450                 455                 460

Asp Ser Leu Lys Gln Glu Thr Gln Gly Leu Gln Lys Glu Lys Glu Ser
465                 470                 475                 480

Arg Glu Lys Glu Leu Met Gly Phe Ser Lys Ser Val Asn Glu Ala Arg
                485                 490                 495

Ser Lys Met Asp Val Ala Gln Ser Glu Leu Asp Ile Tyr Leu Ser Arg
            500                 505                 510

His Asn Thr Ala Val Ser Gln Leu Thr Lys Ala Lys Glu Ala Leu Ile
        515                 520                 525

Ala Ala Ser Glu Thr Leu Lys Glu Arg Lys Ala Ala Ile Arg Asp Ile
    530                 535                 540

Glu Gly Lys Leu Pro Gln Thr Glu Gln Glu Leu Lys Glu Lys Glu Lys
545                 550                 555                 560

Glu Leu Gln Lys Leu Thr Gln Glu Glu Thr Asn Phe Lys Ser Leu Val
                565                 570                 575

His Asp Leu Phe Gln Lys Val Glu Glu Ala Lys Ser Ser Leu Ala Met
            580                 585                 590

Asn Arg Ser Arg Gly Lys Val Leu Asp Ala Ile Ile Gln Glu Lys Lys
        595                 600                 605

Ser Gly Arg Ile Pro Gly Ile Tyr Gly Arg Leu Gly Asp Leu Gly Ala
    610                 615                 620

Ile Asp Glu Lys Tyr Asp Val Ala Ile Ser Ser Cys Cys His Ala Leu
625                 630                 635                 640

Asp Tyr Ile Val Val Asp Ser Ile Asp Ile Ala Gln Glu Cys Val Asn
                645                 650                 655

Phe Leu Lys Arg Gln Asn Ile Gly Val Ala Thr Phe Ile Gly Leu Asp
            660                 665                 670

Lys Met Ala Val Trp Ala Lys Lys Met Thr Glu Ile Gln Thr Pro Glu
        675                 680                 685

Asn Thr Pro Arg Leu Phe Asp Leu Val Lys Val Lys Asp Glu Lys Ile
    690                 695                 700

Arg Gln Ala Phe Tyr Phe Ala Leu Arg Asp Thr Leu Val Ala Asp Asn
705                 710                 715                 720

Leu Asp Gln Ala Thr Arg Val Ala Tyr Gln Lys Asp Arg Arg Trp Arg
                725                 730                 735

Val Val Thr Leu Gln Gly Gln Ile Ile Glu Gln Ser Gly Thr Met Thr
            740                 745                 750

Gly Gly Gly Ser Lys Val Met Lys Gly Arg Met Gly Ser Ser Leu Val
        755                 760                 765

Ile Glu Ile Ser Glu Glu Glu Val Asn Lys Met Glu Ser Gln Leu Gln
    770                 775                 780

Asn Asp Ser Lys Lys Ala Met Gln Ile Gln Glu Gln Lys Val Gln Leu
785                 790                 795                 800

Glu Glu Arg Val Val Lys Leu Arg His Ser Glu Arg Glu Met Arg Asn
                805                 810                 815

Thr Leu Glu Lys Phe Thr Ala Ser Ile Gln Arg Leu Ile Glu Gln Glu
            820                 825                 830

Glu Tyr Leu Asn Val Gln Val Lys Glu Leu Glu Ala Asn Val Leu Ala
        835                 840                 845

Thr Ala Pro Asp Lys Lys Lys Gln Lys Leu Leu Glu Glu Asn Val Ser
    850                 855                 860

Ala Phe Lys Thr Glu Tyr Asp Ala Val Ala Glu Lys Ala Gly Lys Val
865                 870                 875                 880

Glu Ala Glu Val Lys Arg Leu His Asn Thr Ile Val Glu Ile Asn Asn
                885                 890                 895

His Lys Leu Lys Ala Gln Gln Asp Lys Leu Asp Lys Ile Asn Lys Gln
            900                 905                 910

Leu Asp Glu Cys Ala Ser Ala Ile Thr Lys Ala Gln Val Ala Ile Lys
        915                 920                 925

Thr Ala Asp Arg Asn Leu Gln Lys Ala Gln Asp Ser Val Leu Arg Thr
    930                 935                 940

Glu Lys Glu Ile Lys Asp Thr Glu Lys Glu Val Asp Asp Leu Thr Ala
945                 950                 955                 960

Glu Leu Lys Ser Leu Glu Asp Lys Ala Ala Glu Val Val Lys Asn Thr
                965                 970                 975

Asn Ala Ala Glu Glu Ser Leu Pro Glu Ile Gln Lys Glu His Arg Asn
            980                 985                 990

Leu Leu Gln Glu Leu Lys Val Ile  Gln Glu Asn Glu His  Ala Leu Gln
        995                 1000                1005

Lys Asp  Ala Leu Ser Ile Lys  Leu Lys Leu Glu Gln  Ile Asp Gly
    1010                 1015                 1020

His Ile  Ala Glu His Asn Ser  Lys Ile Lys Tyr Trp  His Lys Glu
    1025                 1030                 1035

Ile Ser  Lys Ile Ser Leu His  Pro Ile Glu Asp Asn  Pro Ile Glu
    1040                 1045                 1050

Glu Ile  Ser Val Leu Ser Pro  Glu Asp Leu Glu Ala  Ile Lys Asn
    1055                 1060                 1065

Pro Asp  Ser Ile Thr Asn Gln  Ile Ala Leu Leu Glu  Ala Arg Cys
    1070                 1075                 1080

His Glu  Met Lys Pro Asn Leu  Gly Ala Ile Ala Glu  Tyr Lys Lys
    1085                 1090                 1095

Lys Glu  Glu Leu Tyr Leu Gln  Arg Val Ala Glu Leu  Asp Lys Ile
    1100                 1105                 1110

Thr Tyr  Glu Arg Asp Ser Phe  Arg Gln Ala Tyr Glu  Asp Leu Arg
    1115                 1120                 1125

Lys Gln  Arg Leu Asn Glu Phe  Met Ala Gly Phe Tyr  Ile Ile Thr
    1130                 1135                 1140

Asn Lys Leu Lys Glu Asn Tyr Gln Met Leu Thr Leu Gly Gly Asp
    1145                1150                1155

Ala Glu Leu Glu Leu Val Asp Ser Leu Asp Pro Phe Ser Glu Gly
    1160                1165                1170

Ile Met Phe Ser Val Arg Pro Pro Lys Lys Ser Trp Lys Lys Ile
    1175                1180                1185

Phe Asn Leu Ser Gly Gly Glu Lys Thr Leu Ser Ser Leu Ala Leu
    1190                1195                1200

Val Phe Ala Leu His His Tyr Lys Pro Thr Pro Leu Tyr Phe Met
    1205                1210                1215

Asp Glu Ile Asp Ala Ala Leu Asp Phe Lys Asn Val Ser Ile Val
    1220                1225                1230

Ala Phe Tyr Ile Tyr Glu Gln Thr Lys Asn Ala Gln Phe Ile Ile
    1235                1240                1245

Ile Ser Leu Arg Asn Asn Met Phe Glu Ile Ser Asp Arg Leu Ile
    1250                1255                1260

Gly Ile Tyr Lys Thr Tyr Asn Ile Thr Lys Ser Val Ala Val Asn
    1265                1270                1275

Pro Lys Glu Ile Ala Ser Lys Gly Leu Cys
    1280                1285

<210>  292
<211>  1288
<212>  PRT
<213>  Homo sapiens

<400>  292

Met Pro Arg Lys Gly Thr Gln Pro Ser Thr Ala Arg Arg Arg Glu Glu
1               5                   10                  15

Gly Pro Pro Pro Pro Ser Pro Asp Gly Ala Ser Ser Asp Ala Glu Pro
            20                  25                  30

Glu Pro Pro Ser Gly Arg Thr Glu Ser Pro Ala Thr Ala Ala Glu Thr
        35                  40                  45

Ala Ser Glu Glu Leu Asp Asn Arg Ser Leu Glu Glu Ile Leu Asn Ser
    50                  55                  60

Ile Pro Pro Pro Pro Pro Pro Ala Met Thr Asn Glu Ala Gly Ala Pro
65                  70                  75                  80

Arg Leu Met Ile Thr His Ile Val Asn Gln Asn Phe Lys Ser Tyr Ala

85                 90                 95

Gly Glu Lys Ile Leu Gly Pro Phe His Lys Arg Phe Ser Cys Ile Ile
            100                 105                 110

Gly Pro Asn Gly Ser Gly Lys Ser Asn Val Ile Asp Ser Met Leu Phe
        115                 120                 125

Val Phe Gly Tyr Arg Ala Gln Lys Ile Arg Ser Lys Lys Leu Ser Val
    130                 135                 140

Leu Ile His Asn Ser Asp Glu His Lys Asp Ile Gln Ser Cys Thr Val
145                 150                 155                 160

Glu Val His Phe Gln Lys Ile Ile Asp Lys Glu Gly Asp Asp Tyr Glu
                165                 170                 175

Val Ile Pro Asn Ser Asn Phe Tyr Val Ser Arg Thr Ala Cys Arg Asp
            180                 185                 190

Asn Thr Ser Val Tyr His Ile Ser Gly Lys Lys Lys Thr Phe Lys Asp
        195                 200                 205

Val Gly Asn Leu Leu Arg Ser His Gly Ile Asp Leu Asp His Asn Arg
    210                 215                 220

Phe Leu Ile Leu Gln Gly Glu Val Glu Gln Ile Ala Met Met Lys Pro
225                 230                 235                 240

Lys Gly Gln Thr Glu His Asp Glu Gly Met Leu Glu Tyr Leu Glu Asp
                245                 250                 255

Ile Ile Gly Cys Gly Arg Leu Asn Glu Pro Ile Lys Val Leu Cys Arg
            260                 265                 270

Arg Val Glu Ile Leu Asn Glu His Arg Gly Glu Lys Leu Asn Arg Val
        275                 280                 285

Lys Met Val Glu Lys Glu Lys Asp Ala Leu Glu Gly Glu Lys Asn Ile
    290                 295                 300

Ala Ile Glu Phe Leu Thr Leu Glu Asn Glu Ile Phe Arg Lys Lys Asn
305                 310                 315                 320

His Val Cys Gln Tyr Tyr Ile Tyr Glu Leu Gln Lys Arg Ile Ala Glu
                325                 330                 335

Met Glu Thr Gln Lys Glu Lys Ile His Glu Asp Thr Lys Glu Ile Asn
            340                 345                 350

Glu Lys Ser Asn Ile Leu Ser Asn Glu Met Lys Ala Lys Asn Lys Asp
        355                 360                 365

Val Lys Asp Thr Glu Lys Lys Leu Asn Lys Ile Thr Lys Phe Ile Glu
    370                 375                 380

Glu Asn Lys Glu Lys Phe Thr Gln Leu Asp Leu Glu Asp Val Gln Val
385                 390                 395                 400

Arg Glu Lys Leu Lys His Ala Thr Ser Lys Ala Lys Lys Leu Glu Lys
                405                 410                 415

Gln Leu Gln Lys Asp Lys Glu Lys Val Glu Glu Phe Lys Ser Ile Pro
            420                 425                 430

Ala Lys Ser Asn Asn Ile Ile Asn Glu Thr Thr Thr Arg Asn Asn Ala
        435                 440                 445

Leu Glu Lys Glu Lys Glu Lys Glu Glu Lys Lys Leu Lys Glu Val Met
    450                 455                 460

Asp Ser Leu Lys Gln Glu Thr Gln Gly Leu Gln Lys Glu Lys Glu Ser
465                 470                 475                 480

Arg Glu Lys Glu Leu Met Gly Phe Ser Lys Ser Val Asn Glu Ala Arg
                485                 490                 495

Ser Lys Met Asp Val Ala Gln Ser Glu Leu Asp Ile Tyr Leu Ser Arg
            500                 505                 510

His Asn Thr Ala Val Ser Gln Leu Thr Lys Ala Lys Glu Ala Leu Ile
        515                 520                 525

Ala Ala Ser Glu Thr Leu Lys Glu Arg Lys Ala Ala Ile Arg Asp Ile
    530                 535                 540

Glu Gly Lys Leu Pro Gln Thr Glu Gln Glu Leu Lys Glu Lys Glu Lys
545                 550                 555                 560

Glu Leu Gln Lys Leu Thr Gln Glu Glu Thr Asn Phe Lys Ser Leu Val
                565                 570                 575

His Asp Leu Phe Gln Lys Val Glu Glu Ala Lys Ser Ser Leu Ala Met
            580                 585                 590

Asn Arg Ser Arg Gly Lys Val Leu Asp Ala Ile Ile Gln Glu Lys Lys
        595                 600                 605

Ser Gly Arg Ile Pro Gly Ile Tyr Gly Arg Leu Gly Asp Leu Gly Ala
    610                 615                 620

Ile Asp Glu Lys Tyr Asp Val Ala Ile Ser Ser Cys Cys His Ala Leu
625                 630                 635                 640

Asp Tyr Ile Val Val Asp Ser Ile Asp Ile Ala Gln Glu Cys Val Asn

645                 650                 655

Phe Leu Lys Arg Gln Asn Ile Gly Val Ala Thr Phe Ile Gly Leu Asp
            660                 665                 670

Lys Met Ala Val Trp Ala Lys Lys Met Thr Glu Ile Gln Thr Pro Glu
        675                 680                 685

Asn Thr Pro Arg Leu Phe Asp Leu Val Lys Val Lys Asp Glu Lys Ile
    690                 695                 700

Arg Gln Ala Phe Tyr Phe Ala Leu Arg Asp Thr Leu Val Ala Asp Asn
705                 710                 715                 720

Leu Asp Gln Ala Thr Arg Val Ala Tyr Gln Lys Asp Arg Arg Trp Arg
                725                 730                 735

Val Val Thr Leu Gln Gly Gln Ile Ile Glu Gln Ser Gly Thr Met Thr
            740                 745                 750

Gly Gly Gly Ser Lys Val Met Lys Gly Arg Met Gly Ser Ser Leu Val
        755                 760                 765

Ile Glu Ile Ser Glu Glu Glu Val Asn Lys Met Glu Ser Gln Leu Gln
    770                 775                 780

Asn Asp Ser Lys Lys Ala Met Gln Ile Gln Glu Gln Lys Val Gln Leu
785                 790                 795                 800

Glu Glu Arg Val Val Lys Leu Arg His Ser Glu Arg Glu Met Arg Asn
                805                 810                 815

Thr Leu Glu Lys Phe Thr Ala Ser Ile Gln Arg Leu Ile Glu Gln Glu
            820                 825                 830

Glu Tyr Leu Asn Val Gln Val Lys Glu Leu Glu Ala Asn Val Leu Ala
        835                 840                 845

Thr Ala Pro Asp Lys Lys Lys Gln Lys Leu Leu Glu Glu Asn Val Ser
    850                 855                 860

Ala Phe Lys Thr Glu Tyr Asp Ala Val Ala Glu Lys Ala Gly Lys Val
865                 870                 875                 880

Glu Ala Glu Val Lys Arg Leu His Asn Thr Ile Val Glu Ile Asn Asn
                885                 890                 895

His Lys Leu Lys Ala Gln Gln Asp Lys Leu Asp Lys Ile Asn Lys Gln
            900                 905                 910

Leu Asp Glu Cys Ala Ser Ala Ile Thr Lys Ala Gln Val Ala Ile Lys
        915                 920                 925

Thr Ala Asp Arg Asn Leu Gln Lys Ala Gln Asp Ser Val Leu Arg Thr
    930                 935                 940

Glu Lys Glu Ile Lys Asp Thr Glu Lys Glu Val Asp Asp Leu Thr Ala
945                 950                 955                 960

Glu Leu Lys Ser Leu Glu Asp Lys Ala Ala Glu Val Val Lys Asn Thr
                965                 970                 975

Asn Ala Ala Glu Glu Ser Leu Pro Glu Ile Gln Lys Glu His Arg Asn
            980                 985                 990

Leu Leu Gln Glu Leu Lys Val Ile Gln Glu Asn Glu His Ala Leu Gln
        995                 1000                1005

Lys Asp Ala Leu Ser Ile Lys Leu Lys Leu Glu Gln Ile Asp Gly
    1010                1015                1020

His Ile Ala Glu His Asn Ser Lys Ile Lys Tyr Trp His Lys Glu
    1025                1030                1035

Ile Ser Lys Ile Ser Leu His Pro Ile Glu Asp Asn Pro Ile Glu
    1040                1045                1050

Glu Ile Ser Val Leu Ser Pro Glu Asp Leu Glu Ala Ile Lys Asn
    1055                1060                1065

Pro Asp Ser Ile Thr Asn Gln Ile Ala Leu Leu Glu Ala Arg Cys
    1070                1075                1080

His Glu Met Lys Pro Asn Leu Gly Ala Ile Ala Glu Tyr Lys Lys
    1085                1090                1095

Lys Glu Glu Leu Tyr Leu Gln Arg Val Ala Glu Leu Asp Lys Ile
    1100                1105                1110

Thr Tyr Glu Arg Asp Ser Phe Arg Gln Ala Tyr Glu Asp Leu Arg
    1115                1120                1125

Lys Gln Arg Leu Asn Glu Phe Met Ala Gly Phe Tyr Ile Ile Thr
    1130                1135                1140

Asn Lys Leu Lys Glu Asn Tyr Gln Met Leu Thr Leu Gly Gly Asp
    1145                1150                1155

Ala Glu Leu Glu Leu Val Asp Ser Leu Asp Pro Phe Ser Glu Gly
    1160                1165                1170

Ile Met Phe Ser Val Arg Pro Pro Lys Lys Ser Trp Lys Lys Ile
    1175                1180                1185

Phe Asn Leu Ser Gly Gly Glu Lys Thr Leu Ser Ser Leu Ala Leu

1190                 1195                 1200

Val Phe  Ala Leu His His Tyr  Lys Pro Thr Pro Leu  Tyr Phe Met
    1205                 1210                 1215

Asp Glu  Ile Asp Ala Ala Leu  Asp Phe Lys Asn Val  Ser Ile Val
    1220                 1225                 1230

Ala Phe  Tyr Ile Tyr Glu Gln  Thr Lys Asn Ala Gln  Phe Ile Ile
    1235                 1240                 1245

Ile Ser  Leu Arg Asn Asn Met  Phe Glu Ile Ser Asp  Arg Leu Ile
    1250                 1255                 1260

Gly Ile  Tyr Lys Thr Tyr Asn  Ile Thr Lys Ser Val  Ala Val Asn
    1265                 1270                 1275

Pro Lys  Glu Ile Ala Ser Lys  Gly Leu Cys
    1280                 1285

<210>  293
<211>  890
<212>  PRT
<213>  Homo sapiens

<400>  293

Met Ser Gln Gly Ile Leu Ser Pro Pro Ala Gly Leu Leu Ser Asp Asp
1               5                   10                  15

Asp Val Val Val Ser Pro Met Phe Glu Ser Thr Ala Ala Asp Leu Gly
            20                  25                  30

Ser Val Val Arg Lys Asn Leu Leu Ser Asp Cys Ser Val Val Ser Thr
        35                  40                  45

Ser Leu Glu Asp Lys Gln Gln Val Pro Ser Glu Asp Ser Met Glu Lys
    50                  55                  60

Val Lys Val Tyr Leu Arg Val Arg Pro Leu Leu Pro Ser Glu Leu Glu
65                  70                  75                  80

Arg Gln Glu Asp Gln Gly Cys Val Arg Ile Glu Asn Val Glu Thr Leu
                85                  90                  95

Val Leu Gln Ala Pro Lys Asp Ser Phe Ala Leu Lys Ser Asn Glu Arg
            100                 105                 110

Gly Ile Gly Gln Ala Thr His Arg Phe Thr Phe Ser Gln Ile Phe Gly
        115                 120                 125

Pro Glu Val Gly Gln Ala Ser Phe Phe Asn Leu Thr Val Lys Glu Met
    130                 135                 140

Val Lys Asp Val Leu Lys Gly Gln Asn Trp Leu Ile Tyr Thr Tyr Gly
145                 150                 155                 160

Val Thr Asn Ser Gly Lys Thr His Thr Ile Gln Gly Thr Ile Lys Asp
                165                 170                 175

Gly Gly Ile Leu Pro Arg Ser Leu Ala Leu Ile Phe Asn Ser Leu Gln
            180                 185                 190

Gly Gln Leu His Pro Thr Pro Asp Leu Lys Pro Leu Leu Ser Asn Glu
        195                 200                 205

Val Ile Trp Leu Asp Ser Lys Gln Ile Arg Gln Glu Glu Met Lys Lys
    210                 215                 220

Leu Ser Leu Leu Asn Gly Gly Leu Gln Glu Glu Glu Leu Ser Thr Ser
225                 230                 235                 240

Leu Lys Arg Ser Val Tyr Ile Glu Ser Arg Ile Gly Thr Ser Thr Ser
                245                 250                 255

Phe Asp Ser Gly Ile Ala Gly Leu Ser Ser Ile Ser Gln Cys Thr Ser
            260                 265                 270

Ser Ser Gln Leu Asp Glu Thr Ser His Arg Trp Ala Gln Pro Asp Thr
        275                 280                 285

Ala Pro Leu Pro Val Pro Ala Asn Ile Arg Phe Ser Ile Trp Ile Ser
    290                 295                 300

Phe Phe Glu Ile Tyr Asn Glu Leu Leu Tyr Asp Leu Leu Glu Pro Pro
305                 310                 315                 320

Ser Gln Gln Arg Lys Arg Gln Thr Leu Arg Leu Cys Glu Asp Gln Asn
                325                 330                 335

Gly Asn Pro Tyr Val Lys Asp Leu Asn Trp Ile His Val Gln Asp Ala
            340                 345                 350

Glu Glu Ala Trp Lys Leu Leu Lys Val Gly Arg Lys Asn Gln Ser Phe
        355                 360                 365

Ala Ser Thr His Leu Asn Gln Asn Ser Ser Arg Ser His Ser Ile Phe
    370                 375                 380

Ser Ile Arg Ile Leu His Leu Gln Gly Glu Gly Asp Ile Val Pro Lys
385                 390                 395                 400

Ile Ser Glu Leu Ser Leu Cys Asp Leu Ala Gly Ser Glu Arg Cys Lys
                405                 410                 415

Asp Gln Lys Ser Gly Glu Arg Leu Lys Glu Ala Gly Asn Ile Asn Thr
            420                 425                 430

Ser Leu His Thr Leu Gly Arg Cys Ile Ala Ala Leu Arg Gln Asn Gln
        435                 440                 445

Gln Asn Arg Ser Lys Gln Asn Leu Val Pro Phe Arg Asp Ser Lys Leu
    450                 455                 460

Thr Arg Val Phe Gln Gly Phe Phe Thr Gly Arg Gly Arg Ser Cys Met
465                 470                 475                 480

Ile Val Asn Val Asn Pro Cys Ala Ser Thr Tyr Asp Glu Thr Leu His
                485                 490                 495

Val Ala Lys Phe Ser Ala Ile Ala Ser Gln Leu Val His Ala Pro Pro
            500                 505                 510

Met Gln Leu Gly Phe Pro Ser Leu His Ser Phe Ile Lys Glu His Ser
        515                 520                 525

Leu Gln Val Ser Pro Ser Leu Glu Lys Gly Ala Lys Ala Asp Thr Gly
    530                 535                 540

Leu Asp Asp Asp Ile Glu Asn Glu Ala Asp Ile Ser Met Tyr Gly Lys
545                 550                 555                 560

Glu Glu Leu Leu Gln Val Val Glu Ala Met Lys Thr Leu Leu Leu Lys
                565                 570                 575

Glu Arg Gln Glu Lys Leu Gln Leu Glu Met His Leu Arg Asp Glu Ile
            580                 585                 590

Cys Asn Glu Met Val Glu Gln Met Gln Gln Arg Glu Gln Trp Cys Ser
        595                 600                 605

Glu His Leu Asp Thr Gln Lys Glu Leu Leu Glu Glu Met Tyr Glu Glu
    610                 615                 620

Lys Leu Asn Ile Leu Lys Glu Ser Leu Thr Ser Phe Tyr Gln Glu Glu
625                 630                 635                 640

Ile Gln Glu Arg Asp Glu Lys Ile Glu Glu Leu Glu Ala Leu Leu Gln
                645                 650                 655

Glu Ala Arg Gln Gln Ser Val Ala His Gln Gln Ser Gly Ser Glu Leu
            660                 665                 670

Ala Leu Arg Arg Ser Gln Arg Leu Ala Ala Ser Ala Ser Thr Gln Gln
        675                 680                 685

Leu Gln Glu Val Lys Ala Lys Leu Gln Gln Cys Lys Ala Glu Leu Asn
    690                 695                 700

Ser Thr Thr Glu Glu Leu His Lys Tyr Gln Lys Met Leu Glu Pro Pro
705                 710                 715                 720

Pro Ser Ala Lys Pro Phe Thr Ile Asp Val Asp Lys Lys Leu Glu Glu
                725                 730                 735

Gly Gln Lys Asn Ile Arg Leu Leu Arg Thr Glu Leu Gln Lys Leu Gly
            740                 745                 750

Glu Ser Leu Gln Ser Ala Glu Arg Ala Cys Cys His Ser Thr Gly Ala
        755                 760                 765

Gly Lys Leu Arg Gln Ala Leu Thr Thr Cys Asp Asp Ile Leu Ile Lys
    770                 775                 780

Gln Asp Gln Thr Leu Ala Glu Leu Gln Asn Asn Met Val Leu Val Lys
785                 790                 795                 800

Leu Asp Leu Arg Lys Lys Ala Ala Cys Ile Ala Glu Gln Tyr His Thr
                805                 810                 815

Val Leu Lys Leu Gln Gly Gln Val Ser Ala Lys Lys Arg Leu Gly Thr
            820                 825                 830

Asn Gln Glu Asn Gln Gln Pro Asn Gln Gln Pro Pro Gly Lys Lys Pro
        835                 840                 845

Phe Leu Arg Asn Leu Leu Pro Arg Thr Pro Thr Cys Gln Ser Ser Thr
    850                 855                 860

Asp Cys Ser Pro Tyr Ala Arg Ile Leu Arg Ser Arg Arg Ser Pro Leu
865                 870                 875                 880

Leu Lys Ser Gly Pro Phe Gly Lys Lys Tyr
                885                 890

<210>  294
<211>  229
<212>  PRT
<213>  Homo sapiens

<400>  294

Met Thr Pro Gln Leu Leu Leu Ala Leu Val Leu Trp Ala Ser Cys Pro
1               5                   10                  15

Pro Cys Ser Gly Arg Lys Gly Pro Pro Ala Ala Leu Thr Leu Pro Arg
            20                  25                  30

Val Gln Cys Arg Ala Ser Arg Tyr Pro Ile Ala Val Asp Cys Ser Trp
        35                  40                  45

Thr Leu Pro Pro Ala Pro Asn Ser Thr Ser Pro Val Ser Phe Ile Ala
    50                  55                  60

Thr Tyr Arg Leu Gly Met Ala Ala Arg Gly His Ser Trp Pro Cys Leu
65                  70                  75                  80

Gln Gln Thr Pro Thr Ser Thr Ser Cys Thr Ile Thr Asp Val Gln Leu
                85                  90                  95

Phe Ser Met Ala Pro Tyr Val Leu Asn Val Thr Ala Val His Pro Trp
            100                 105                 110

Gly Ser Ser Ser Ser Phe Val Pro Phe Ile Thr Glu His Ile Ile Lys
        115                 120                 125

Pro Asp Pro Pro Glu Gly Val Arg Leu Ser Pro Leu Ala Glu Arg Gln
    130                 135                 140

Leu Gln Val Gln Trp Glu Pro Pro Gly Ser Trp Pro Phe Pro Glu Ile
145                 150                 155                 160

Phe Ser Leu Lys Tyr Trp Ile Arg Tyr Lys Arg Gln Gly Ala Ala Arg
                165                 170                 175

Phe His Arg Val Gly Pro Ile Glu Ala Thr Ser Phe Ile Leu Arg Ala
            180                 185                 190

Val Arg Pro Arg Ala Arg Tyr Tyr Val Gln Val Ala Ala Gln Asp Leu
        195                 200                 205

Thr Asp Tyr Gly Glu Leu Ser Asp Trp Ser Leu Pro Ala Thr Ala Thr
    210                 215                 220

Met Ser Leu Gly Lys
225

<210>  295
<211>  633
<212>  PRT
<213>  Homo sapiens

<400>  295

Met Ala Asn Gln Val Asn Gly Asn Ala Val Gln Leu Lys Glu Glu Glu
1               5                   10                  15

Glu Pro Met Asp Thr Ser Ser Val Thr His Thr Glu His Tyr Lys Thr
            20                  25                  30

Leu Ile Glu Ala Gly Leu Pro Gln Lys Val Ala Glu Arg Leu Asp Glu
        35                  40                  45

Ile Phe Gln Thr Gly Leu Val Ala Tyr Val Asp Leu Asp Glu Arg Ala
    50                  55                  60

Ile Asp Ala Leu Arg Glu Phe Asn Glu Glu Gly Ala Leu Ser Val Leu
65                  70                  75                  80

Gln Gln Phe Lys Glu Ser Asp Leu Ser His Val Gln Asn Lys Ser Ala
                85                  90                  95

Phe Leu Cys Gly Val Met Lys Thr Tyr Arg Gln Arg Glu Lys Gln Gly
            100                 105                 110

Ser Lys Val Gln Glu Ser Thr Lys Gly Pro Asp Glu Ala Lys Ile Lys
        115                 120                 125

Ala Leu Leu Glu Arg Thr Gly Tyr Thr Leu Asp Val Thr Thr Gly Gln
    130                 135                 140

Arg Lys Tyr Gly Gly Pro Pro Pro Asp Ser Val Tyr Ser Gly Val Gln
145                 150                 155                 160

Pro Gly Ile Gly Thr Glu Val Phe Val Gly Lys Ile Pro Arg Asp Leu
                165                 170                 175

Tyr Glu Asp Glu Leu Val Pro Leu Phe Glu Lys Ala Gly Pro Ile Trp
            180                 185                 190

Asp Leu Arg Leu Met Met Asp Pro Leu Ser Gly Gln Asn Arg Gly Tyr
        195                 200                 205

Ala Phe Ile Thr Phe Cys Gly Lys Glu Ala Ala Gln Glu Ala Val Lys
    210                 215                 220

Leu Cys Asp Ser Tyr Glu Ile Arg Pro Gly Lys His Leu Gly Val Cys
225                 230                 235                 240

Ile Ser Val Ala Asn Asn Arg Leu Phe Val Gly Ser Ile Pro Lys Asn
                245                 250                 255

Lys Thr Lys Glu Asn Ile Leu Glu Glu Phe Ser Lys Val Thr Glu Gly
            260                 265                 270

Leu Val Asp Val Ile Leu Tyr His Gln Pro Asp Asp Lys Lys Lys Asn
        275                 280                 285

Arg Gly Phe Cys Phe Leu Glu Tyr Glu Asp His Lys Ser Ala Ala Gln
    290                 295                 300

Ala Arg Arg Arg Leu Met Ser Gly Lys Val Lys Val Trp Gly Asn Val
305                 310                 315                 320

Val Thr Val Glu Trp Ala Asp Pro Val Glu Glu Pro Asp Pro Glu Val
                325                 330                 335

Met Ala Lys Val Lys Val Leu Phe Val Arg Asn Leu Ala Thr Thr Val
            340                 345                 350

Thr Glu Glu Ile Leu Glu Lys Ser Phe Ser Glu Phe Gly Lys Leu Glu
        355                 360                 365

Arg Val Lys Lys Leu Lys Asp Tyr Ala Phe Val His Phe Glu Asp Arg
    370                 375                 380

Gly Ala Ala Val Lys Ala Met Asp Glu Met Asn Gly Lys Glu Ile Glu
385                 390                 395                 400

Gly Glu Glu Ile Glu Ile Val Leu Ala Lys Pro Pro Asp Lys Lys Arg
                405                 410                 415

Lys Glu Arg Gln Ala Ala Arg Gln Ala Ser Arg Ser Thr Ala Tyr Glu
            420                 425                 430

Asp Tyr Tyr Tyr His Pro Pro Pro Arg Met Pro Pro Pro Ile Arg Gly
        435                 440                 445

Arg Gly Arg Gly Gly Gly Arg Gly Gly Tyr Gly Tyr Pro Pro Asp Tyr
    450                 455                 460

Tyr Gly Tyr Glu Asp Tyr Tyr Asp Asp Tyr Tyr Gly Tyr Asp Tyr His
465                 470                 475                 480

Asp Tyr Arg Gly Gly Tyr Glu Asp Pro Tyr Tyr Gly Tyr Asp Asp Gly
                485                 490                 495

Tyr Ala Val Arg Gly Arg Gly Gly Gly Arg Gly Gly Arg Gly Ala Pro
            500                 505                 510

Pro Pro Pro Arg Gly Arg Gly Ala Pro Pro Pro Arg Gly Arg Ala Gly
        515                 520                 525

Tyr Ser Gln Arg Gly Ala Pro Leu Gly Pro Pro Arg Gly Ser Arg Gly
    530                 535                 540

Gly Arg Gly Gly Pro Ala Gln Gln Gln Arg Gly Arg Gly Ser Arg Gly
545                 550                 555                 560

Ser Arg Gly Asn Arg Gly Gly Asn Val Gly Gly Lys Arg Lys Ala Asp
                565                 570                 575

Gly Tyr Asn Gln Pro Asp Ser Lys Arg Arg Gln Thr Asn Asn Gln Gln
            580                 585                 590

Asn Trp Gly Ser Gln Pro Ile Ala Gln Gln Pro Leu Gln Gln Gly Gly
        595                 600                 605

Asp Tyr Ser Gly Asn Tyr Gly Tyr Asn Asn Asp Asn Gln Glu Phe Tyr
    610                 615                 620

Gln Asp Thr Tyr Gly Gln Gln Trp Lys
625                 630

<210>  296
<211>  445
<212>  PRT
<213>  Homo sapiens

<400>  296

Met Arg Glu Ile Val His Leu Gln Ala Gly Gln Cys Gly Asn Gln Ile
1               5                   10                  15

Gly Ala Lys Phe Trp Glu Val Ile Ser Asp Glu His Gly Ile Asp Pro
            20                  25                  30

Thr Gly Thr Tyr His Gly Asp Ser Asp Leu Gln Leu Glu Arg Ile Asn
        35                  40                  45

Val Tyr Tyr Asn Glu Ala Thr Gly Gly Lys Tyr Val Pro Arg Ala Val
    50                  55                  60

Leu Val Asp Leu Glu Pro Gly Thr Met Asp Ser Val Arg Ser Gly Pro
65                  70                  75                  80

Phe Gly Gln Ile Phe Arg Pro Asp Asn Phe Val Phe Gly Gln Ser Gly
                85                  90                  95

Ala Gly Asn Asn Trp Ala Lys Gly His Tyr Thr Glu Gly Ala Glu Leu
            100                 105                 110

Val Asp Ser Val Leu Asp Val Val Arg Lys Glu Ala Glu Ser Cys Asp
        115                 120                 125

Cys Leu Gln Gly Phe Gln Leu Thr His Ser Leu Gly Gly Gly Thr Gly
    130                 135                 140

Ser Gly Met Gly Thr Leu Leu Ile Ser Lys Ile Arg Glu Glu Tyr Pro
145                 150                 155                 160

Asp Arg Ile Met Asn Thr Phe Ser Val Val Pro Ser Pro Lys Val Ser
                165                 170                 175

Asp Thr Val Val Glu Pro Tyr Asn Ala Thr Leu Ser Val His Gln Leu
            180                 185                 190

Val Glu Asn Thr Asp Glu Thr Tyr Cys Ile Asp Asn Glu Ala Leu Tyr
        195                 200                 205

Asp Ile Cys Phe Arg Thr Leu Lys Leu Thr Thr Pro Thr Tyr Gly Asp
    210                 215                 220

Leu Asn His Leu Val Ser Ala Thr Met Ser Gly Val Thr Thr Cys Leu
225                 230                 235                 240

Arg Phe Pro Gly Gln Leu Asn Ala Asp Leu Arg Lys Leu Ala Val Asn

245                 250                 255

Met Val Pro Phe Pro Arg Leu His Phe Phe Met Pro Gly Phe Ala Pro
            260                 265                 270

Leu Thr Ser Arg Gly Ser Gln Gln Tyr Arg Ala Leu Thr Val Pro Glu
        275                 280                 285

Leu Thr Gln Gln Met Phe Asp Ala Lys Asn Met Met Ala Ala Cys Asp
    290                 295                 300

Pro Arg His Gly Arg Tyr Leu Thr Val Ala Ala Val Phe Arg Gly Arg
305                 310                 315                 320

Met Ser Met Lys Glu Val Asp Glu Gln Met Leu Asn Val Gln Asn Lys
                325                 330                 335

Asn Ser Ser Tyr Phe Val Glu Trp Ile Pro Asn Asn Val Lys Thr Ala
            340                 345                 350

Val Cys Asp Ile Pro Pro Arg Gly Leu Lys Met Ser Ala Thr Phe Ile
        355                 360                 365

Gly Asn Ser Thr Ala Ile Gln Glu Leu Phe Lys Arg Ile Ser Glu Gln
    370                 375                 380

Phe Thr Ala Met Phe Arg Arg Lys Ala Phe Leu His Trp Tyr Thr Gly
385                 390                 395                 400

Glu Gly Met Asp Glu Met Glu Phe Thr Glu Ala Glu Ser Asn Met Asn
                405                 410                 415

Asp Leu Val Ser Glu Tyr Gln Gln Tyr Gln Asp Ala Thr Ala Glu Glu
            420                 425                 430

Glu Gly Glu Phe Glu Glu Glu Ala Glu Glu Glu Val Ala
        435                 440                 445

<210>  297
<211>  642
<212>  PRT
<213>  Homo sapiens

<400>  297

Met Lys Arg Ser Ser Val Ser Ser Gly Gly Ala Gly Arg Leu Ser Met
1               5                   10                  15

Gln Glu Leu Arg Ser Gln Asp Val Asn Lys Gln Gly Leu Tyr Thr Pro
            20                  25                  30

Gln Thr Lys Glu Lys Pro Thr Phe Gly Lys Leu Ser Ile Asn Lys Pro
        35                  40                  45

Thr Ser Glu Arg Lys Val Ser Leu Phe Gly Lys Arg Thr Ser Gly His
    50                  55                  60

Gly Ser Arg Asn Ser Gln Leu Gly Ile Phe Ser Ser Ser Glu Lys Ile
65                  70                  75                  80

Lys Asp Pro Arg Pro Leu Asn Asp Lys Ala Phe Ile Gln Gln Cys Ile
                85                  90                  95

Arg Gln Leu Cys Glu Phe Leu Thr Glu Asn Gly Tyr Ala His Asn Val
            100                 105                 110

Ser Met Lys Ser Leu Gln Ala Pro Ser Val Lys Asp Phe Leu Lys Ile
        115                 120                 125

Phe Thr Phe Leu Tyr Gly Phe Leu Cys Pro Ser Tyr Glu Leu Pro Asp
    130                 135                 140

Thr Lys Phe Glu Glu Glu Val Pro Arg Ile Phe Lys Asp Leu Gly Tyr
145                 150                 155                 160

Pro Phe Ala Leu Ser Lys Ser Ser Met Tyr Thr Val Gly Ala Pro His
                165                 170                 175

Thr Trp Pro His Ile Val Ala Ala Leu Val Trp Leu Ile Asp Cys Ile
            180                 185                 190

Lys Ile His Thr Ala Met Lys Glu Ser Ser Pro Leu Phe Asp Asp Gly
        195                 200                 205

Gln Pro Trp Gly Glu Glu Thr Glu Asp Gly Ile Met His Asn Lys Leu
    210                 215                 220

Phe Leu Asp Tyr Thr Ile Lys Cys Tyr Glu Ser Phe Met Ser Gly Ala
225                 230                 235                 240

Asp Ser Phe Asp Glu Met Asn Ala Glu Leu Gln Ser Lys Leu Lys Asp
                245                 250                 255

Leu Phe Asn Val Asp Ala Phe Lys Leu Glu Ser Leu Glu Ala Lys Asn
            260                 265                 270

Arg Ala Leu Asn Glu Gln Ile Ala Arg Leu Glu Gln Glu Arg Glu Lys
        275                 280                 285

Glu Pro Asn Arg Leu Glu Ser Leu Arg Lys Leu Lys Ala Ser Leu Gln
    290                 295                 300

Gly Asp Val Gln Lys Tyr Gln Ala Tyr Met Ser Asn Leu Glu Ser His
305                 310                 315                 320

Ser Ala Ile Leu Asp Gln Lys Leu Asn Gly Leu Asn Glu Glu Ile Ala
                325                 330                 335

Arg Val Glu Leu Glu Cys Glu Thr Ile Lys Gln Glu Asn Thr Arg Leu
            340                 345                 350

Gln Asn Ile Ile Asp Asn Gln Lys Tyr Ser Val Ala Asp Ile Glu Arg
        355                 360                 365

Ile Asn His Glu Arg Asn Glu Leu Gln Gln Thr Ile Asn Lys Leu Thr
    370                 375                 380

Lys Asp Leu Glu Ala Glu Gln Gln Lys Leu Trp Asn Glu Glu Leu Lys
385                 390                 395                 400

Tyr Ala Arg Gly Lys Glu Ala Ile Glu Thr Gln Leu Ala Glu Tyr His
                405                 410                 415

Lys Leu Ala Arg Lys Leu Lys Leu Ile Pro Lys Gly Ala Glu Asn Ser
            420                 425                 430

Lys Gly Tyr Asp Phe Glu Ile Lys Phe Asn Pro Glu Ala Gly Ala Asn
        435                 440                 445

Cys Leu Val Lys Tyr Arg Ala Gln Val Tyr Val Pro Leu Lys Glu Leu
    450                 455                 460

Leu Asn Glu Thr Glu Glu Glu Ile Asn Lys Ala Leu Asn Lys Lys Met
465                 470                 475                 480

Gly Leu Glu Asp Thr Leu Glu Gln Leu Asn Ala Met Ile Thr Glu Ser
                485                 490                 495

Lys Arg Ser Val Arg Thr Leu Lys Glu Glu Val Gln Lys Leu Asp Asp
            500                 505                 510

Leu Tyr Gln Gln Lys Ile Lys Glu Ala Glu Glu Glu Asp Glu Lys Cys
        515                 520                 525

Ala Ser Glu Leu Glu Ser Leu Glu Lys His Lys His Leu Leu Glu Ser
    530                 535                 540

Thr Val Asn Gln Gly Leu Ser Glu Ala Met Asn Glu Leu Asp Ala Val
545                 550                 555                 560

Gln Arg Glu Tyr Gln Leu Val Val Gln Thr Thr Thr Glu Glu Arg Arg
                565                 570                 575

Lys Val Gly Asn Asn Leu Gln Arg Leu Leu Glu Met Val Ala Thr His
            580                 585                 590

Val Gly Ser Val Glu Lys His Leu Glu Glu Gln Ile Ala Lys Val Asp
        595                 600                 605

Arg Glu Tyr Glu Glu Cys Met Ser Glu Asp Leu Ser Glu Asn Ile Lys
    610                 615                 620

Glu Ile Arg Asp Lys Tyr Glu Lys Lys Ala Thr Leu Ile Lys Ser Ser
625                 630                 635                 640

Glu Glu

<210>  298
<211>  594
<212>  PRT
<213>  Homo sapiens

<400>  298

Met Val Leu Leu His Val Leu Phe Glu His Ala Val Gly Tyr Ala Leu
1               5                   10                  15

Leu Ala Leu Lys Glu Val Glu Glu Ile Ser Leu Leu Gln Pro Gln Val
            20                  25                  30

Glu Glu Ser Val Leu Asn Leu Gly Lys Phe His Ser Ile Val Arg Leu
        35                  40                  45

Val Ala Phe Cys Pro Phe Ala Ser Ser Gln Val Ala Leu Glu Asn Ala
    50                  55                  60

Asn Ala Val Ser Glu Gly Val Val His Glu Asp Leu Arg Leu Leu Leu
65                  70                  75                  80

Glu Thr His Leu Pro Ser Lys Lys Lys Lys Val Leu Leu Gly Val Gly
                85                  90                  95

Asp Pro Lys Ile Gly Ala Ala Ile Gln Glu Glu Leu Gly Tyr Asn Cys
            100                 105                 110

Gln Thr Gly Gly Val Ile Ala Glu Ile Leu Arg Gly Val Arg Leu His
        115                 120                 125

Phe His Asn Leu Val Lys Gly Leu Thr Asp Leu Ser Ala Cys Lys Ala
    130                 135                 140

Gln Leu Gly Leu Gly His Ser Tyr Ser Arg Ala Lys Val Lys Phe Asn
145                 150                 155                 160

Val Asn Arg Val Asp Asn Met Ile Ile Gln Ser Ile Ser Leu Leu Asp
                165                 170                 175

Gln Leu Asp Lys Asp Ile Asn Thr Phe Ser Met Arg Val Arg Glu Trp
            180                 185                 190

Tyr Gly Tyr His Phe Pro Glu Leu Val Lys Ile Ile Asn Asp Asn Ala
        195                 200                 205

Thr Tyr Cys Arg Leu Ala Gln Phe Ile Gly Asn Arg Arg Glu Leu Asn
    210                 215                 220

Glu Asp Lys Leu Glu Lys Leu Glu Glu Leu Thr Met Asp Gly Ala Lys
225                 230                 235                 240

Ala Lys Ala Ile Leu Asp Ala Ser Arg Ser Ser Met Gly Met Asp Ile
                245                 250                 255

Ser Ala Ile Asp Leu Ile Asn Ile Glu Ser Phe Ser Ser Arg Val Val
            260                 265                 270

Ser Leu Ser Glu Tyr Arg Gln Ser Leu His Thr Tyr Leu Arg Ser Lys
        275                 280                 285

Met Ser Gln Val Ala Pro Ser Leu Ser Ala Leu Ile Gly Glu Ala Val
    290                 295                 300

Gly Ala Arg Leu Ile Ala His Ala Gly Ser Leu Thr Asn Leu Ala Lys
305                 310                 315                 320

Tyr Pro Ala Ser Thr Val Gln Ile Leu Gly Ala Glu Lys Ala Leu Phe
                325                 330                 335

Arg Ala Leu Lys Thr Arg Gly Asn Thr Pro Lys Tyr Gly Leu Ile Phe
            340                 345                 350

His Ser Thr Phe Ile Gly Arg Ala Ala Ala Lys Asn Lys Gly Arg Ile
        355                 360                 365

Ser Arg Tyr Leu Ala Asn Lys Cys Ser Ile Ala Ser Arg Ile Asp Cys
    370                 375                 380

Phe Ser Glu Val Pro Thr Ser Val Phe Gly Glu Lys Leu Arg Glu Gln
385                 390                 395                 400

Val Glu Glu Arg Leu Ser Phe Tyr Glu Thr Gly Glu Ile Pro Arg Lys
                405                 410                 415

Asn Leu Asp Val Met Lys Glu Ala Met Val Gln Ala Glu Glu Ala Ala
            420                 425                 430

Ala Glu Ile Thr Arg Lys Leu Glu Lys Gln Glu Lys Lys Arg Leu Lys
        435                 440                 445

Lys Glu Lys Lys Arg Leu Ala Ala Leu Ala Leu Ala Ser Ser Glu Asn
    450                 455                 460

Ser Ser Ser Thr Pro Glu Glu Cys Glu Glu Met Ser Glu Lys Pro Lys
465                 470                 475                 480

Lys Lys Lys Lys Gln Lys Pro Gln Glu Val Pro Gln Glu Asn Gly Met

485                 490                 495

Glu Asp Pro Ser Ile Ser Phe Ser Lys Pro Lys Lys Lys Lys Ser Phe
            500                 505                 510

Ser Lys Glu Glu Leu Met Ser Ser Asp Leu Glu Glu Thr Ala Gly Ser
        515                 520                 525

Thr Ser Ile Pro Lys Arg Lys Lys Ser Thr Pro Lys Glu Glu Thr Val
    530                 535                 540

Asn Asp Pro Glu Glu Ala Gly His Arg Ser Gly Ser Lys Lys Lys Arg
545                 550                 555                 560

Lys Phe Ser Lys Glu Glu Pro Val Ser Ser Gly Pro Glu Glu Ala Val
                565                 570                 575

Gly Lys Ser Ser Ser Lys Lys Lys Lys Lys Phe His Lys Ala Ser Gln
            580                 585                 590

Glu Asp


<210>  299
<211>  299
<212>  PRT
<213>  Homo sapiens

<400>  299

<210>  300
<211>  335
<212>  PRT
<213>  Homo sapiens

<400>  300

Met Glu Ala Gly Ala Ala Glu Ala Ala Val Ala Ala Val Glu Glu Val
1               5                   10                  15

Gly Ser Ala Gly Gln Phe Glu Glu Leu Leu Arg Leu Lys Ala Lys Ser
            20                  25                  30

Leu Leu Val Val His Phe Trp Ala Pro Trp Ala Pro Gln Cys Ala Gln
        35                  40                  45

Met Asn Glu Val Met Ala Glu Leu Ala Lys Glu Leu Pro Gln Val Ser
    50                  55                  60

Phe Val Lys Leu Glu Ala Glu Gly Val Pro Glu Val Ser Glu Lys Tyr
65                  70                  75                  80

Glu Ile Ser Ser Val Pro Thr Phe Leu Phe Phe Lys Asn Ser Gln Lys
                85                  90                  95

Ile Asp Arg Leu Asp Gly Ala His Ala Pro Glu Leu Thr Lys Lys Val
            100                 105                 110

Gln Arg His Ala Ser Ser Gly Ser Phe Leu Pro Ser Ala Asn Glu His
        115                 120                 125

Leu Lys Glu Asp Leu Asn Leu Arg Leu Lys Lys Leu Thr His Ala Ala
    130                 135                 140

Pro Cys Met Leu Phe Met Lys Gly Thr Pro Gln Glu Pro Arg Cys Gly
145                 150                 155                 160

Phe Ser Lys Gln Met Val Glu Ile Leu His Lys His Asn Ile Gln Phe
                165                 170                 175

Ser Ser Phe Asp Ile Phe Ser Asp Glu Glu Val Arg Gln Gly Leu Lys
            180                 185                 190

Ala Tyr Ser Ser Trp Pro Thr Tyr Pro Gln Leu Tyr Val Ser Gly Glu
        195                 200                 205

Leu Ile Gly Gly Leu Asp Ile Ile Lys Glu Leu Glu Ala Ser Glu Glu
    210                 215                 220

Leu Asp Thr Ile Cys Pro Lys Ala Pro Lys Leu Glu Glu Arg Leu Lys
225                 230                 235                 240

Val Leu Thr Asn Lys Ala Ser Val Met Leu Phe Met Lys Gly Asn Lys
                245                 250                 255

Gln Glu Ala Lys Cys Gly Phe Ser Lys Gln Ile Leu Glu Ile Leu Asn
            260                 265                 270

Ser Thr Gly Val Glu Tyr Glu Thr Phe Asp Ile Leu Glu Asp Glu Glu
        275                 280                 285

Val Arg Gln Gly Leu Lys Ala Tyr Ser Asn Trp Pro Thr Tyr Pro Gln
    290                 295                 300

Leu Tyr Val Lys Gly Glu Leu Val Gly Gly Leu Asp Ile Val Lys Glu
305                 310                 315                 320

Leu Lys Glu Asn Gly Glu Leu Leu Pro Ile Leu Arg Gly Glu Asn
                325                 330                 335

<210>  301
<211>  335
<212>  PRT
<213>  Homo sapiens

<400> 301

Met Val Arg Pro Val Arg His Lys Lys Pro Val Asn Tyr Ser Gln Phe
1               5                   10                  15

Asp His Ser Asp Ser Asp Asp Asp Phe Val Ser Ala Thr Val Pro Leu
            20                  25                  30

Asn Lys Lys Ser Arg Thr Ala Pro Lys Glu Leu Lys Gln Asp Lys Pro
        35                  40                  45

Lys Pro Asn Leu Asn Asn Leu Arg Lys Glu Glu Ile Pro Val Gln Glu
    50                  55                  60

Lys Thr Pro Lys Lys Arg Met Ala Leu Asp Asp Lys Leu Tyr Gln Arg
65                  70                  75                  80

Asp Leu Glu Val Ala Leu Ala Leu Ser Val Lys Glu Leu Pro Thr Val
                85                  90                  95

Thr Thr Asn Val Gln Asn Ser Gln Asp Lys Ser Ile Glu Lys His Gly
            100                 105                 110

Ser Ser Lys Ile Glu Thr Met Asn Lys Ser Pro His Ile Ser Asn Cys
        115                 120                 125

Ser Val Ala Ser Asp Tyr Leu Asp Leu Asp Lys Ile Thr Val Glu Asp
    130                 135                 140

Asp Val Gly Gly Val Gln Gly Lys Arg Lys Ala Ala Ser Lys Ala Ala
145                 150                 155                 160

Ala Gln Gln Arg Lys Ile Leu Leu Glu Gly Ser Asp Gly Asp Ser Ala
                165                 170                 175

Asn Asp Thr Glu Pro Asp Phe Ala Pro Gly Glu Asp Ser Glu Asp Asp
            180                 185                 190

Ser Asp Phe Cys Glu Ser Glu Asp Asn Asp Glu Asp Phe Ser Met Arg
        195                 200                 205

Lys Ser Lys Val Lys Glu Ile Lys Lys Lys Glu Val Lys Val Lys Ser
    210                 215                 220

Pro Val Glu Lys Lys Glu Lys Lys Ser Lys Ser Lys Cys Asn Ala Leu
225                 230                 235                 240

Val Thr Ser Val Asp Ser Ala Pro Ala Ala Val Lys Ser Glu Ser Gln
                245                 250                 255

Ser Leu Pro Lys Lys Val Ser Leu Ser Ser Asp Thr Thr Arg Lys Pro
            260                 265                 270

Leu Glu Ile Arg Ser Pro Ser Ala Glu Ser Lys Lys Pro Lys Trp Val
        275                 280                 285

Pro Pro Ala Ala Ser Gly Gly Ser Arg Ser Ser Ser Ser Pro Leu Val
    290                 295                 300

Val Val Ser Val Lys Ser Pro Asn Gln Ser Leu Arg Leu Gly Leu Ser
305                 310                 315                 320

Arg Leu Ala Arg Val Lys Pro Leu His Pro Asn Ala Thr Ser Thr
                325                 330                 335

<210>  302
<211>  565
<212>  PRT
<213>  Homo sapiens

<400>  302

Met Ser Gly Arg Ser Lys Arg Glu Ser Arg Gly Ser Thr Arg Gly Lys
1               5                   10                  15

Arg Glu Ser Glu Ser Arg Gly Ser Ser Gly Arg Val Lys Arg Glu Arg
            20                  25                  30

Asp Arg Glu Arg Glu Pro Glu Ala Ala Ser Ser Arg Gly Ser Pro Val
        35                  40                  45

Arg Val Lys Arg Glu Phe Glu Pro Ala Ser Ala Arg Glu Ala Pro Ala
    50                  55                  60

Ser Val Val Pro Phe Val Arg Val Lys Arg Glu Arg Glu Val Asp Glu
65                  70                  75                  80

Asp Ser Glu Pro Glu Arg Glu Val Arg Ala Lys Asn Gly Arg Val Asp
                85                  90                  95

Ser Glu Asp Arg Arg Ser Arg His Cys Pro Tyr Leu Asp Thr Ile Asn
            100                 105                 110

Arg Ser Val Leu Asp Phe Asp Phe Glu Lys Leu Cys Ser Ile Ser Leu
        115                 120                 125

Ser His Ile Asn Ala Tyr Ala Cys Leu Val Cys Gly Lys Tyr Phe Gln
    130                 135                 140

Gly Arg Gly Leu Lys Ser His Ala Tyr Ile His Ser Val Gln Phe Ser
145                 150                 155                 160

His His Val Phe Leu Asn Leu His Thr Leu Lys Phe Tyr Cys Leu Pro
                165                 170                 175

Asp Asn Tyr Glu Ile Ile Asp Ser Ser Leu Glu Asp Ile Thr Tyr Val
            180                 185                 190

Leu Lys Pro Thr Phe Thr Lys Gln Gln Ile Ala Asn Leu Asp Lys Gln
        195                 200                 205

Ala Lys Leu Ser Arg Ala Tyr Asp Gly Thr Thr Tyr Leu Pro Gly Ile
    210                 215                 220

Val Gly Leu Asn Asn Ile Lys Ala Asn Asp Tyr Ala Asn Ala Val Leu
225                 230                 235                 240

Gln Ala Leu Ser Asn Val Pro Pro Leu Arg Asn Tyr Phe Leu Glu Glu
                245                 250                 255

Asp Asn Tyr Lys Asn Ile Lys Arg Pro Pro Gly Asp Ile Met Phe Leu
            260                 265                 270

Leu Val Gln Arg Phe Gly Glu Leu Met Arg Lys Leu Trp Asn Pro Arg
        275                 280                 285

Asn Phe Lys Ala His Val Ser Pro His Glu Met Leu Gln Ala Val Val
    290                 295                 300

Leu Cys Ser Lys Lys Thr Phe Gln Ile Thr Lys Gln Gly Asp Gly Val
305                 310                 315                 320

Asp Phe Leu Ser Trp Phe Leu Asn Ala Leu His Ser Ala Leu Gly Gly
                325                 330                 335

Thr Lys Lys Lys Lys Lys Thr Ile Val Thr Asp Val Phe Gln Gly Ser
            340                 345                 350

Met Arg Ile Phe Thr Lys Lys Leu Pro His Pro Asp Leu Pro Ala Glu
        355                 360                 365

Glu Lys Glu Gln Leu Leu His Asn Asp Glu Tyr Gln Glu Thr Met Val
    370                 375                 380

Glu Ser Thr Phe Met Tyr Leu Thr Leu Asp Leu Pro Thr Ala Pro Leu
385                 390                 395                 400

Tyr Lys Asp Glu Lys Glu Gln Leu Ile Ile Pro Gln Val Pro Leu Phe
                405                 410                 415

Asn Ile Leu Ala Lys Phe Asn Gly Ile Thr Glu Lys Glu Tyr Lys Thr
            420                 425                 430

Tyr Lys Glu Asn Phe Leu Lys Arg Phe Gln Leu Thr Lys Leu Pro Pro
        435                 440                 445

Tyr Leu Ile Phe Cys Ile Lys Arg Phe Thr Lys Asn Asn Phe Phe Val
    450                 455                 460

Glu Lys Asn Pro Thr Ile Val Asn Phe Pro Ile Thr Asn Val Asp Leu

465                 470                 475                 480

Arg Glu Tyr Leu Ser Glu Glu Val Gln Ala Val His Lys Asn Thr Thr
                485                 490                 495

Tyr Asp Leu Ile Ala Asn Ile Val His Asp Gly Lys Pro Ser Glu Gly
            500                 505                 510

Ser Tyr Arg Ile His Val Leu His His Gly Thr Gly Lys Trp Tyr Glu
        515                 520                 525

Leu Gln Asp Leu Gln Val Thr Asp Ile Leu Pro Gln Met Ile Thr Leu
    530                 535                 540

Ser Glu Ala Tyr Ile Gln Ile Trp Lys Arg Arg Asp Asn Asp Glu Thr
545                 550                 555                 560

Asn Gln Gln Gly Ala
                565

<210>  303
<211>  463
<212>  PRT
<213>  Homo sapiens

<400>  303

Met Ala Thr Val Thr Ala Thr Thr Lys Val Pro Glu Ile Arg Asp Val
1               5                   10                  15

Thr Arg Ile Glu Arg Ile Gly Ala His Ser His Ile Arg Gly Leu Gly
            20                  25                  30

Leu Asp Asp Ala Leu Glu Pro Arg Gln Ala Ser Gln Gly Met Val Gly
        35                  40                  45

Gln Leu Ala Ala Arg Arg Ala Ala Gly Val Val Leu Glu Met Ile Arg
    50                  55                  60

Glu Gly Lys Ile Ala Gly Arg Ala Val Leu Ile Ala Gly Gln Pro Gly
65                  70                  75                  80

Thr Gly Lys Thr Ala Ile Ala Met Gly Met Ala Gln Ala Leu Gly Pro
                85                  90                  95

Asp Thr Pro Phe Thr Ala Ile Ala Gly Ser Glu Ile Phe Ser Leu Glu
            100                 105                 110

Met Ser Lys Thr Glu Ala Leu Thr Gln Ala Phe Arg Arg Ser Ile Gly
        115                 120                 125

Val Arg Ile Lys Glu Glu Thr Glu Ile Ile Glu Gly Glu Val Val Glu
    130                 135                 140

Ile Gln Ile Asp Arg Pro Ala Thr Gly Thr Gly Ser Lys Val Gly Lys
145                 150                 155                 160

Leu Thr Leu Lys Thr Thr Glu Met Glu Thr Ile Tyr Asp Leu Gly Thr
                165                 170                 175

Lys Met Ile Glu Ser Leu Thr Lys Asp Lys Val Gln Ala Gly Asp Val
            180                 185                 190

Ile Thr Ile Asp Lys Ala Thr Gly Lys Ile Ser Lys Leu Gly Arg Ser
        195                 200                 205

Phe Thr Arg Ala Arg Asp Tyr Asp Ala Met Gly Ser Gln Thr Lys Phe
    210                 215                 220

Val Gln Cys Pro Asp Gly Glu Leu Gln Lys Arg Lys Glu Val Val His
225                 230                 235                 240

Thr Val Ser Leu His Glu Ile Asp Val Ile Asn Ser Arg Thr Gln Gly
                245                 250                 255

Phe Leu Ala Leu Phe Ser Gly Asp Thr Gly Glu Ile Lys Ser Glu Val
            260                 265                 270

Arg Glu Gln Ile Asn Ala Lys Val Ala Glu Trp Arg Glu Glu Gly Lys
        275                 280                 285

Ala Glu Ile Ile Pro Gly Val Leu Phe Ile Asp Glu Val His Met Leu
    290                 295                 300

Asp Ile Glu Ser Phe Ser Phe Leu Asn Arg Ala Leu Glu Ser Asp Met
305                 310                 315                 320

Ala Pro Val Leu Ile Met Ala Thr Asn Arg Gly Ile Thr Arg Ile Arg
                325                 330                 335

Gly Thr Ser Tyr Gln Ser Pro His Gly Ile Pro Ile Asp Leu Leu Asp
            340                 345                 350

Arg Leu Leu Ile Val Ser Thr Thr Pro Tyr Ser Glu Lys Asp Thr Lys
        355                 360                 365

Gln Ile Leu Arg Ile Arg Cys Glu Glu Glu Asp Val Glu Met Ser Glu
    370                 375                 380

Asp Ala Tyr Thr Val Leu Thr Arg Ile Gly Leu Glu Thr Ser Leu Arg
385                 390                 395                 400

Tyr Ala Ile Gln Leu Ile Thr Ala Ala Ser Leu Val Cys Arg Lys Arg
                405                 410                 415

Lys Gly Thr Glu Val Gln Val Asp Asp Ile Lys Arg Val Tyr Ser Leu
            420                 425                 430

Phe Leu Asp Glu Ser Arg Ser Thr Gln Tyr Met Lys Glu Tyr Gln Asp
        435                 440                 445

Ala Phe Leu Phe Asn Glu Leu Lys Gly Glu Thr Met Asp Thr Ser
    450                 455                 460

<210>  304
<211>  725
<212>  PRT
<213>  Homo sapiens

<400>  304

Met Ala Met Asp Ser Ser Leu Gln Ala Arg Leu Phe Pro Gly Leu Ala
1               5                   10                  15

Ile Lys Ile Gln Arg Ser Asn Gly Leu Ile His Ser Ala Asn Val Arg
            20                  25                  30

Thr Val Asn Leu Glu Lys Ser Cys Val Ser Val Glu Trp Ala Glu Gly
        35                  40                  45

Gly Ala Thr Lys Gly Lys Glu Ile Asp Phe Asp Asp Val Ala Ala Ile
    50                  55                  60

Asn Pro Glu Leu Leu Gln Leu Leu Pro Leu His Pro Lys Asp Asn Leu
65                  70                  75                  80

Pro Leu Gln Glu Asn Val Thr Ile Gln Lys Gln Lys Arg Arg Ser Val
                85                  90                  95

Asn Ser Lys Ile Pro Ala Pro Lys Glu Ser Leu Arg Ser Arg Ser Thr
            100                 105                 110

Arg Met Ser Thr Val Ser Glu Leu Arg Ile Thr Ala Gln Glu Asn Asp
        115                 120                 125

Met Glu Val Glu Leu Pro Ala Ala Ala Asn Ser Arg Lys Gln Phe Ser
    130                 135                 140

Val Pro Pro Ala Pro Thr Arg Pro Ser Cys Pro Ala Val Ala Glu Ile
145                 150                 155                 160

Pro Leu Arg Met Val Ser Glu Glu Met Glu Glu Gln Val His Ser Ile
                165                 170                 175

Arg Gly Ser Ser Ser Ala Asn Pro Val Asn Ser Val Arg Arg Lys Ser
            180                 185                 190

Cys Leu Val Lys Glu Val Glu Lys Met Lys Asn Lys Arg Glu Glu Lys
        195                 200                 205

Lys Ala Gln Asn Ser Glu Met Arg Met Lys Arg Ala Gln Glu Tyr Asp

210                 215                 220

Ser Ser Phe Pro Asn Trp Glu Phe Ala Arg Met Ile Lys Glu Phe Arg
225                 230                 235                 240

Ala Thr Leu Glu Cys His Pro Leu Thr Met Thr Asp Pro Ile Glu Glu
                245                 250                 255

His Arg Ile Cys Val Cys Val Arg Lys Arg Pro Leu Asn Lys Gln Glu
            260                 265                 270

Leu Ala Lys Lys Glu Ile Asp Val Ile Ser Ile Pro Ser Lys Cys Leu
        275                 280                 285

Leu Leu Val His Glu Pro Lys Leu Lys Val Asp Leu Thr Lys Tyr Leu
    290                 295                 300

Glu Asn Gln Ala Phe Cys Phe Asp Phe Ala Phe Asp Glu Thr Ala Ser
305                 310                 315                 320

Asn Glu Val Val Tyr Arg Phe Thr Ala Arg Pro Leu Val Gln Thr Ile
                325                 330                 335

Phe Glu Gly Gly Lys Ala Thr Cys Phe Ala Tyr Gly Gln Thr Gly Ser
            340                 345                 350

Gly Lys Thr His Thr Met Gly Gly Asp Leu Ser Gly Lys Ala Gln Asn
        355                 360                 365

Ala Ser Lys Gly Ile Tyr Ala Met Ala Ser Arg Asp Val Phe Leu Leu
    370                 375                 380

Lys Asn Gln Pro Cys Tyr Arg Lys Leu Gly Leu Glu Val Tyr Val Thr
385                 390                 395                 400

Phe Phe Glu Ile Tyr Asn Gly Lys Leu Phe Asp Leu Leu Asn Lys Lys
                405                 410                 415

Ala Lys Leu Arg Val Leu Glu Asp Gly Lys Gln Gln Val Gln Val Val
            420                 425                 430

Gly Leu Gln Glu His Leu Val Asn Ser Ala Asp Asp Val Ile Lys Met
        435                 440                 445

Leu Asp Met Gly Ser Ala Cys Arg Thr Ser Gly Gln Thr Phe Ala Asn
    450                 455                 460

Ser Asn Ser Ser Arg Ser His Ala Cys Phe Gln Ile Ile Leu Arg Ala
465                 470                 475                 480

Lys Gly Arg Met His Gly Lys Phe Ser Leu Val Asp Leu Ala Gly Asn
                485                 490                 495

Glu Arg Gly Ala Asp Thr Ser Ser Ala Asp Arg Gln Thr Arg Met Glu
            500                 505                 510

Gly Ala Glu Ile Asn Lys Ser Leu Leu Ala Leu Lys Glu Cys Ile Arg
        515                 520                 525

Ala Leu Gly Gln Asn Lys Ala His Thr Pro Phe Arg Glu Ser Lys Leu
    530                 535                 540

Thr Gln Val Leu Arg Asp Ser Phe Ile Gly Glu Asn Ser Arg Thr Cys
545                 550                 555                 560

Met Ile Ala Thr Ile Ser Pro Gly Ile Ser Ser Cys Glu Tyr Thr Leu
                565                 570                 575

Asn Thr Leu Arg Tyr Ala Asp Arg Val Lys Glu Leu Ser Pro His Ser
            580                 585                 590

Gly Pro Ser Gly Glu Gln Leu Ile Gln Met Glu Thr Glu Glu Met Glu
        595                 600                 605

Ala Cys Ser Asn Gly Ala Leu Ile Pro Gly Asn Leu Ser Lys Glu Glu
    610                 615                 620

Glu Glu Leu Ser Ser Gln Met Ser Ser Phe Asn Glu Ala Met Thr Gln
625                 630                 635                 640

Ile Arg Glu Leu Glu Glu Lys Ala Met Glu Glu Leu Lys Glu Ile Ile
                645                 650                 655

Gln Gln Gly Pro Asp Trp Leu Glu Leu Ser Glu Met Thr Glu Gln Pro
            660                 665                 670

Asp Tyr Asp Leu Glu Thr Phe Val Asn Lys Ala Glu Ser Ala Leu Ala
        675                 680                 685

Gln Gln Ala Lys His Phe Ser Ala Leu Arg Asp Val Ile Lys Ala Leu
    690                 695                 700

Arg Leu Ala Met Gln Leu Glu Glu Gln Ala Ser Arg Gln Ile Ser Ser
705                 710                 715                 720

Lys Lys Arg Pro Gln
                725

<210>  305
<211>  179
<212>  PRT
<213>  Homo sapiens

<400>  305

Met Ala Ser Gln Asn Arg Asp Pro Ala Ala Thr Ser Val Ala Ala Ala
1               5                   10                  15

Arg Lys Gly Ala Glu Pro Ser Gly Gly Ala Ala Arg Gly Pro Val Gly
            20                  25                  30

Lys Arg Leu Gln Gln Glu Leu Met Thr Leu Met Met Ser Gly Asp Lys
        35                  40                  45

Gly Ile Ser Ala Phe Pro Glu Ser Asp Asn Leu Phe Lys Trp Val Gly
    50                  55                  60

Thr Ile His Gly Ala Ala Gly Thr Val Tyr Glu Asp Leu Arg Tyr Lys
65                  70                  75                  80

Leu Ser Leu Glu Phe Pro Ser Gly Tyr Pro Tyr Asn Ala Pro Thr Val
                85                  90                  95

Lys Phe Leu Thr Pro Cys Tyr His Pro Asn Val Asp Thr Gln Gly Asn
            100                 105                 110

Ile Cys Leu Asp Ile Leu Lys Glu Lys Trp Ser Ala Leu Tyr Asp Val
        115                 120                 125

Arg Thr Ile Leu Leu Ser Ile Gln Ser Leu Leu Gly Glu Pro Asn Ile
    130                 135                 140

Asp Ser Pro Leu Asn Thr His Ala Ala Glu Leu Trp Lys Asn Pro Thr
145                 150                 155                 160

Ala Phe Lys Lys Tyr Leu Gln Glu Thr Tyr Ser Lys Gln Val Thr Ser
                165                 170                 175

Gln Glu Pro

<210>  306
<211>  161
<212>  PRT
<213>  Homo sapiens

<400>  306

Met Ala Ser Gln Asn Arg Asp Pro Ala Ala Thr Ser Val Ala Ala Ala
1               5                   10                  15

Arg Lys Gly Ala Glu Pro Ser Gly Gly Ala Ala Arg Gly Pro Val Gly
            20                  25                  30

Lys Arg Leu Gln Gln Glu Leu Met Thr Leu Met Met Ser Gly Asp Lys
        35                  40                  45

Gly Ile Ser Ala Phe Pro Glu Ser Asp Asn Leu Phe Lys Trp Val Gly
    50                  55                  60

Thr Ile His Gly Ala Ala Gly Thr Ala Val Gly Ser Ile Arg Thr Ser

65                  70                  75                  80

Ser Thr Val Cys Leu Leu Ser Gly Pro Arg Glu Thr Gln Asp Ser Ser
                85                  90                  95

Lys Pro Leu Val Trp Gly Leu Gly Trp Asp Met Arg Leu Leu Leu Glu
            100                 105                 110

Leu Thr Leu Gln Leu Phe Leu Gln Met Pro Glu Pro Asn Ile Asp Ser
        115                 120                 125

Pro Leu Asn Thr His Ala Ala Glu Leu Trp Lys Asn Pro Thr Ala Phe
    130                 135                 140

Lys Lys Tyr Leu Gln Glu Thr Tyr Ser Lys Gln Val Thr Ser Gln Glu
145                 150                 155                 160

Pro

<210>  307
<211>  150
<212>  PRT
<213>  Homo sapiens

<400>  307

Met Ala Ser Gln Asn Arg Asp Pro Ala Ala Thr Ser Val Ala Ala Ala
1               5                   10                  15

Arg Lys Gly Ala Glu Pro Ser Gly Gly Ala Ala Arg Gly Pro Val Gly
            20                  25                  30

Lys Arg Leu Gln Gln Glu Leu Met Thr Leu Met Val Tyr Glu Asp Leu
        35                  40                  45

Arg Tyr Lys Leu Ser Leu Glu Phe Pro Ser Gly Tyr Pro Tyr Asn Ala
    50                  55                  60

Pro Thr Val Lys Phe Leu Thr Pro Cys Tyr His Pro Asn Val Asp Thr
65                  70                  75                  80

Gln Gly Asn Ile Cys Leu Asp Ile Leu Lys Glu Lys Trp Ser Ala Leu
                85                  90                  95

Tyr Asp Val Arg Thr Ile Leu Leu Ser Ile Gln Ser Leu Leu Gly Glu
            100                 105                 110

Pro Asn Ile Asp Ser Pro Leu Asn Thr His Ala Ala Glu Leu Trp Lys
        115                 120                 125

Asn Pro Thr Ala Phe Lys Lys Tyr Leu Gln Glu Thr Tyr Ser Lys Gln
    130                 135                 140

Val Thr Ser Gln Glu Pro
145                 150

<210>  308
<211>  140
<212>  PRT
<213>  Homo sapiens

<400>  308

Met Thr Leu Met Met Ser Gly Asp Lys Gly Ile Ser Ala Phe Pro Glu
1               5                   10                  15

Ser Asp Asn Leu Phe Lys Trp Val Gly Thr Ile His Gly Ala Ala Gly
            20                  25                  30

Thr Val Tyr Glu Asp Leu Arg Tyr Lys Leu Ser Leu Glu Phe Pro Ser
        35                  40                  45

Gly Tyr Pro Tyr Asn Ala Pro Thr Val Lys Phe Leu Thr Pro Cys Tyr
    50                  55                  60

His Pro Asn Val Asp Thr Gln Gly Asn Ile Cys Leu Asp Ile Leu Lys
65                  70                  75                  80

Glu Lys Trp Ser Ala Leu Tyr Asp Val Arg Thr Ile Leu Leu Ser Ile
                85                  90                  95

Gln Ser Leu Leu Gly Glu Pro Asn Ile Asp Ser Pro Leu Asn Thr His
            100                 105                 110

Ala Ala Glu Leu Trp Lys Asn Pro Thr Ala Phe Lys Lys Tyr Leu Gln
        115                 120                 125

Glu Thr Tyr Ser Lys Gln Val Thr Ser Gln Glu Pro
    130                 135                 140

<210>  309
<211>  140
<212>  PRT
<213>  Homo sapiens

<400>  309

Met Thr Leu Met Met Ser Gly Asp Lys Gly Ile Ser Ala Phe Pro Glu
1               5                   10                  15

Ser Asp Asn Leu Phe Lys Trp Val Gly Thr Ile His Gly Ala Ala Gly
            20                  25                  30

Thr Val Tyr Glu Asp Leu Arg Tyr Lys Leu Ser Leu Glu Phe Pro Ser
        35                  40                  45

Gly Tyr Pro Tyr Asn Ala Pro Thr Val Lys Phe Leu Thr Pro Cys Tyr
    50                  55                  60

His Pro Asn Val Asp Thr Gln Gly Asn Ile Cys Leu Asp Ile Leu Lys
65                  70                  75                  80

Glu Lys Trp Ser Ala Leu Tyr Asp Val Arg Thr Ile Leu Leu Ser Ile
                85                  90                  95

Gln Ser Leu Leu Gly Glu Pro Asn Ile Asp Ser Pro Leu Asn Thr His
            100                 105                 110

Ala Ala Glu Leu Trp Lys Asn Pro Thr Ala Phe Lys Lys Tyr Leu Gln
        115                 120                 125

Glu Thr Tyr Ser Lys Gln Val Thr Ser Gln Glu Pro
    130                 135                 140

<210>  310
<211>  50
<212>  PRT
<213>  Homo sapiens

<400>  310

Met Ala Ser Gln Asn Arg Asp Pro Ala Ala Thr Ser Val Ala Ala Ala
1               5                   10                  15

Arg Lys Gly Ala Glu Pro Ser Gly Gly Ala Ala Arg Gly Pro Val Gly
            20                  25                  30

Lys Arg Leu Gln Gln Glu Leu Met Thr Leu Met Asn Pro Thr Leu Ile
        35                  40                  45

Val Pro
    50

<210>  311
<211>  230
<212>  PRT
<213>  Homo sapiens

<400>  311

Met Glu Ala Ala Glu Thr Glu Ala Glu Ala Ala Ala Leu Glu Val Leu
1               5                   10                  15

Ala Glu Val Ala Gly Ile Leu Glu Pro Val Gly Leu Gln Glu Glu Ala
            20                  25                  30

Glu Leu Pro Ala Lys Ile Leu Val Glu Phe Val Val Asp Ser Gln Lys
        35                  40                  45

Lys Asp Lys Leu Leu Cys Ser Gln Leu Gln Val Ala Asp Phe Leu Gln
    50                  55                  60

Asn Ile Leu Ala Gln Glu Asp Thr Ala Lys Gly Leu Asp Pro Leu Ala
65                  70                  75                  80

Ser Glu Asp Thr Ser Arg Gln Lys Ala Ile Ala Ala Lys Glu Gln Trp
                85                  90                  95

Lys Glu Leu Lys Ala Thr Tyr Arg Glu His Val Glu Ala Ile Lys Ile
            100                 105                 110

Gly Leu Thr Lys Ala Leu Thr Gln Met Glu Glu Ala Gln Arg Lys Arg
        115                 120                 125

Thr Gln Leu Arg Glu Ala Phe Glu Gln Leu Gln Ala Lys Lys Gln Met
    130                 135                 140

Ala Met Glu Lys Arg Arg Ala Val Gln Asn Gln Trp Gln Leu Gln Gln
145                 150                 155                 160

Glu Lys His Leu Gln His Leu Ala Glu Val Ser Ala Glu Gly Lys Leu
                165                 170                 175

Leu Phe Pro Glu Ala Glu Ala Glu Ala Glu Asn Leu Pro Asp Asp Lys
            180                 185                 190

Pro Gln Gln Pro Thr Arg Pro Gln Glu Gln Ser Thr Gly Asp Thr Met
        195                 200                 205

Gly Arg Asp Pro Gly Val Ser Phe Lys Ala Val Gly Leu Gln Pro Ala
    210                 215                 220

Gly Asp Val Asn Leu Pro
225                 230

<210>  312
<211>  48
<212>  PRT
<213>  Homo sapiens

<400>  312

Met Glu Ala Ala Glu Thr Glu Ala Glu Ala Ala Ala Leu Glu Val Leu
1               5                   10                  15

Ala Glu Val Ala Gly Ile Leu Glu Pro Val Gly Leu Gln Glu Glu Ala
            20                  25                  30

Glu Leu Pro Ala Lys Ile Leu Val Glu Phe Val Val Val Cys Thr Arg
        35                  40                  45

<210>  313
<211>  277
<212>  PRT
<213>  Homo sapiens

<400>  313

Met Glu Ala Ala Glu Thr Glu Ala Glu Ala Ala Ala Leu Glu Val Leu
1               5                   10                  15

Ala Glu Val Ala Gly Ile Leu Glu Pro Val Gly Leu Gln Glu Glu Ala
            20                  25                  30

Glu Leu Pro Ala Lys Ile Leu Val Glu Phe Val Val Asp Ser Gln Lys
        35                  40                  45

Lys Asp Lys Leu Leu Cys Ser Gln Leu Gln Val Ala Asp Phe Leu Gln
    50                  55                  60

Asn Ile Leu Ala Gln Glu Asp Thr Ala Lys Gly Leu Asp Pro Leu Ala
65                  70                  75                  80

Ser Glu Asp Thr Ser Arg Gln Lys Ala Ile Ala Ala Lys Glu Gln Trp
                85                  90                  95

Lys Glu Leu Lys Ala Thr Tyr Arg Glu His Val Glu Ala Ile Lys Ile
            100                 105                 110

Gly Leu Thr Lys Ala Leu Thr Gln Met Glu Glu Ala Gln Arg Lys Arg
        115                 120                 125

Thr Gln Leu Arg Glu Ala Phe Glu Gln Leu Gln Ala Lys Lys Gln Met
    130                 135                 140

Ala Met Glu Lys Arg Arg Ala Val Gln Asn Gln Trp Gln Leu Gln Gln
145                 150                 155                 160

Glu Lys His Leu Gln His Leu Ala Glu Val Ser Ala Glu Val Arg Glu
                165                 170                 175

Arg Lys Thr Gly Thr Gln Gln Glu Leu Asp Arg Val Phe Gln Lys Leu
            180                 185                 190

Gly Asn Leu Lys Gln Gln Ala Glu Gln Glu Arg Asp Lys Leu Gln Arg
        195                 200                 205

Tyr Gln Thr Phe Leu Gln Leu Leu Tyr Thr Leu Gln Gly Lys Leu Leu
    210                 215                 220

Phe Pro Glu Ala Glu Ala Glu Ala Glu Asn Leu Pro Asp Asp Lys Pro
225                 230                 235                 240

Gln Gln Pro Thr Arg Pro Gln Glu Gln Ser Thr Gly Asp Thr Met Gly
                245                 250                 255

Arg Asp Pro Gly Val Ser Phe Lys Ala Val Gly Leu Gln Pro Ala Gly
            260                 265                 270

Asp Val Asn Leu Pro
        275

<210>  314
<211>  277

<212>  PRT
<213>  Homo sapiens

<400>  314

Met Glu Ala Ala Glu Thr Glu Ala Glu Ala Ala Ala Leu Glu Val Leu
1               5                   10                  15

Ala Glu Val Ala Gly Ile Leu Glu Pro Val Gly Leu Gln Glu Glu Ala
            20                  25                  30

Glu Leu Pro Ala Lys Ile Leu Val Glu Phe Val Val Asp Ser Gln Lys
        35                  40                  45

Lys Asp Lys Leu Leu Cys Ser Gln Leu Gln Val Ala Asp Phe Leu Gln
    50                  55                  60

Asn Ile Leu Ala Gln Glu Asp Thr Ala Lys Gly Leu Asp Pro Leu Ala
65                  70                  75                  80

Ser Glu Asp Thr Ser Arg Gln Lys Ala Ile Ala Ala Lys Glu Gln Trp
                85                  90                  95

Lys Glu Leu Lys Ala Thr Tyr Arg Glu His Val Glu Ala Ile Lys Ile
            100                 105                 110

Gly Leu Thr Lys Ala Leu Thr Gln Met Glu Glu Ala Gln Arg Lys Arg
        115                 120                 125

Thr Gln Leu Arg Glu Ala Phe Glu Gln Leu Gln Ala Lys Lys Gln Met
    130                 135                 140

Ala Met Glu Lys Arg Arg Ala Val Gln Asn Gln Trp Gln Leu Gln Gln
145                 150                 155                 160

Glu Lys His Leu Gln His Leu Ala Glu Val Ser Ala Glu Val Arg Glu
                165                 170                 175

Arg Lys Thr Gly Thr Gln Gln Glu Leu Asp Arg Val Phe Gln Lys Leu
            180                 185                 190

Gly Asn Leu Lys Gln Gln Ala Glu Gln Glu Arg Asp Lys Leu Gln Arg
        195                 200                 205

Tyr Gln Thr Phe Leu Gln Leu Leu Tyr Thr Leu Gln Gly Lys Leu Leu
    210                 215                 220

Phe Pro Glu Ala Glu Ala Glu Ala Glu Asn Leu Pro Asp Asp Lys Pro
225                 230                 235                 240

Gln Gln Pro Thr Arg Pro Gln Glu Gln Ser Thr Gly Asp Thr Met Gly
                245                 250                 255

Arg Asp Pro Gly Val Ser Phe Lys Ala Val Gly Leu Gln Pro Ala Gly

260                 265                 270

Asp Val Asn Leu Pro
        275

<210>  315
<211>  747
<212>  PRT
<213>  Homo sapiens

<400>  315

Met Ser Gln Val Lys Ser Ser Tyr Ser Tyr Asp Ala Pro Ser Asp Phe
1               5                   10                  15

Ile Asn Phe Ser Ser Leu Asp Asp Glu Gly Asp Thr Gln Asn Ile Asp
            20                  25                  30

Ser Trp Phe Glu Glu Lys Ala Asn Leu Glu Asn Lys Leu Leu Gly Lys
        35                  40                  45

Asn Gly Thr Gly Gly Leu Phe Gln Gly Lys Thr Pro Leu Arg Lys Ala
    50                  55                  60

Asn Leu Gln Gln Ala Ile Val Thr Pro Leu Lys Pro Val Asp Asn Thr
65                  70                  75                  80

Tyr Tyr Lys Glu Ala Glu Lys Glu Asn Leu Val Glu Gln Ser Ile Pro
                85                  90                  95

Ser Asn Ala Cys Ser Ser Leu Glu Val Glu Ala Ala Ile Ser Arg Lys
            100                 105                 110

Thr Pro Ala Gln Pro Gln Arg Arg Ser Leu Arg Leu Ser Ala Gln Lys
        115                 120                 125

Asp Leu Glu Gln Lys Glu Lys His His Val Lys Met Lys Ala Lys Arg
    130                 135                 140

Cys Ala Thr Pro Val Ile Ile Asp Glu Ile Leu Pro Ser Lys Lys Met
145                 150                 155                 160

Lys Val Ser Asn Asn Lys Lys Lys Pro Glu Glu Glu Gly Ser Ala His
                165                 170                 175

Gln Asp Thr Ala Glu Lys Asn Ala Ser Ser Pro Glu Lys Ala Lys Gly
            180                 185                 190

Arg His Thr Val Pro Cys Met Pro Pro Ala Lys Gln Lys Phe Leu Lys
        195                 200                 205

Ser Thr Glu Glu Gln Glu Leu Glu Lys Ser Met Lys Met Gln Gln Glu
    210                 215                 220

Val Val Glu Met Arg Lys Lys Asn Glu Glu Phe Lys Lys Leu Ala Leu
225                 230                 235                 240

Ala Gly Ile Gly Gln Pro Val Lys Lys Ser Val Ser Gln Val Thr Lys
                245                 250                 255

Ser Val Asp Phe His Phe Arg Thr Asp Glu Arg Ile Lys Gln His Pro
            260                 265                 270

Lys Asn Gln Glu Glu Tyr Lys Glu Val Asn Phe Thr Ser Glu Leu Arg
        275                 280                 285

Lys His Pro Ser Ser Pro Ala Arg Val Thr Lys Gly Cys Thr Ile Val
    290                 295                 300

Lys Pro Phe Asn Leu Ser Gln Gly Lys Lys Arg Thr Phe Asp Glu Thr
305                 310                 315                 320

Val Ser Thr Tyr Val Pro Leu Ala Gln Gln Val Glu Asp Phe His Lys
                325                 330                 335

Arg Thr Pro Asn Arg Tyr His Leu Arg Ser Lys Lys Asp Asp Ile Asn
            340                 345                 350

Leu Leu Pro Ser Lys Ser Ser Val Thr Lys Ile Cys Arg Asp Pro Gln
        355                 360                 365

Thr Pro Val Leu Gln Thr Lys His Arg Ala Arg Ala Val Thr Cys Lys
    370                 375                 380

Ser Thr Ala Glu Leu Glu Ala Glu Glu Leu Glu Lys Leu Gln Gln Tyr
385                 390                 395                 400

Lys Phe Lys Ala Arg Glu Leu Asp Pro Arg Ile Leu Glu Gly Gly Pro
                405                 410                 415

Ile Leu Pro Lys Lys Pro Pro Val Lys Pro Pro Thr Glu Pro Ile Gly
            420                 425                 430

Phe Asp Leu Glu Ile Glu Lys Arg Ile Gln Glu Arg Glu Ser Lys Lys
        435                 440                 445

Lys Thr Glu Asp Glu His Phe Glu Phe His Ser Arg Pro Cys Pro Thr
    450                 455                 460

Lys Ile Leu Glu Asp Val Val Gly Val Pro Glu Lys Lys Val Leu Pro
465                 470                 475                 480

Ile Thr Val Pro Lys Ser Pro Ala Phe Ala Leu Lys Asn Arg Ile Arg
                485                 490                 495

Met Pro Thr Lys Glu Asp Glu Glu Glu Asp Glu Pro Val Val Ile Lys
            500                 505                 510

Ala Gln Pro Val Pro His Tyr Gly Val Pro Phe Lys Pro Gln Ile Pro
        515                 520                 525

Glu Ala Arg Thr Val Glu Ile Cys Pro Phe Ser Phe Asp Ser Arg Asp
    530                 535                 540

Lys Glu Arg Gln Leu Gln Lys Glu Lys Lys Ile Lys Glu Leu Gln Lys
545                 550                 555                 560

Gly Glu Val Pro Lys Phe Lys Ala Leu Pro Leu Pro His Phe Asp Thr
                565                 570                 575

Ile Asn Leu Pro Glu Lys Lys Val Lys Asn Val Thr Gln Ile Glu Pro
            580                 585                 590

Phe Cys Leu Glu Thr Asp Arg Arg Gly Ala Leu Lys Ala Gln Thr Trp
        595                 600                 605

Lys His Gln Leu Glu Glu Glu Leu Arg Gln Gln Lys Glu Ala Ala Cys
    610                 615                 620

Phe Lys Ala Arg Pro Asn Thr Val Ile Ser Gln Glu Pro Phe Val Pro
625                 630                 635                 640

Lys Lys Glu Lys Lys Ser Val Ala Glu Gly Leu Ser Gly Ser Leu Val
                645                 650                 655

Gln Glu Pro Phe Gln Leu Ala Thr Glu Lys Arg Ala Lys Glu Arg Gln
            660                 665                 670

Glu Leu Glu Lys Arg Met Ala Glu Val Glu Ala Gln Lys Ala Gln Gln
        675                 680                 685

Leu Glu Glu Ala Arg Leu Gln Glu Glu Glu Gln Lys Lys Glu Glu Leu
    690                 695                 700

Ala Arg Leu Arg Arg Glu Leu Val His Lys Ala Asn Pro Ile Arg Lys
705                 710                 715                 720

Tyr Gln Gly Leu Glu Ile Lys Ser Ser Asp Gln Pro Leu Thr Val Pro
                725                 730                 735

Val Ser Pro Lys Phe Ser Thr Arg Phe His Cys
            740                 745

<210>  316
<211>  3526
<212>  PRT
<213>  Homo sapiens

<400>  316

Met Asn Met Val Lys Arg Ile Met Gly Arg Pro Arg Gln Glu Glu Cys

1               5                   10                  15

Ser Pro Gln Asp Asn Ala Leu Gly Leu Met His Leu Arg Arg Leu Phe
            20                  25                  30

Thr Glu Leu Cys His Pro Pro Arg His Met Thr Gln Lys Glu Gln Glu
        35                  40                  45

Glu Lys Leu Tyr Met Met Leu Pro Val Phe Asn Arg Val Phe Gly Asn
    50                  55                  60

Ala Pro Pro Asn Thr Met Thr Glu Lys Phe Ser Asp Leu Leu Gln Phe
65                  70                  75                  80

Thr Thr Gln Val Ser Arg Leu Met Val Thr Glu Ile Arg Arg Arg Ala
                85                  90                  95

Ser Asn Lys Ser Thr Glu Ala Ala Ser Arg Ala Ile Val Gln Phe Leu
            100                 105                 110

Glu Ile Asn Gln Ser Glu Glu Ala Ser Arg Gly Trp Met Leu Leu Thr
        115                 120                 125

Thr Ile Asn Leu Leu Ala Ser Ser Gly Gln Lys Thr Val Asp Cys Met
    130                 135                 140

Thr Thr Met Ser Val Pro Ser Thr Leu Val Lys Cys Leu Tyr Leu Phe
145                 150                 155                 160

Phe Asp Leu Pro His Val Pro Glu Ala Val Gly Gly Ala Gln Asn Glu
                165                 170                 175

Leu Pro Leu Ala Glu Arg Arg Gly Leu Leu Gln Lys Val Phe Val Gln
            180                 185                 190

Ile Leu Val Lys Leu Cys Ser Phe Val Ser Pro Ala Glu Glu Leu Ala
        195                 200                 205

Gln Lys Asp Asp Leu Gln Leu Leu Phe Ser Ala Ile Thr Ser Trp Cys
    210                 215                 220

Pro Pro Tyr Asn Leu Pro Trp Arg Lys Ser Ala Gly Glu Val Leu Met
225                 230                 235                 240

Thr Ile Ser Arg His Gly Leu Ser Val Asn Val Val Lys Tyr Ile His
                245                 250                 255

Glu Lys Glu Cys Leu Ser Thr Cys Val Gln Asn Met Gln Gln Ser Asp
            260                 265                 270

Asp Leu Ser Pro Leu Glu Ile Val Glu Met Phe Ala Gly Leu Ser Cys
        275                 280                 285

Phe Leu Lys Asp Ser Ser Asp Val Ser Gln Thr Leu Leu Asp Asp Phe
    290                 295                 300

Arg Ile Trp Gln Gly Tyr Asn Phe Leu Cys Asp Leu Leu Leu Arg Leu
305                 310                 315                 320

Glu Gln Ala Lys Glu Ala Glu Ser Lys Asp Ala Leu Lys Asp Leu Val
                325                 330                 335

Asn Leu Ile Thr Ser Leu Thr Thr Tyr Gly Val Ser Glu Leu Lys Pro
            340                 345                 350

Ala Gly Ile Thr Thr Gly Ala Pro Phe Leu Leu Pro Gly Phe Ala Val
        355                 360                 365

Pro Gln Pro Ala Gly Lys Gly His Ser Val Arg Asn Val Gln Ala Phe
    370                 375                 380

Ala Val Leu Gln Asn Ala Phe Leu Lys Ala Lys Thr Ser Phe Leu Ala
385                 390                 395                 400

Gln Ile Ile Leu Asp Ala Ile Thr Asn Ile Tyr Met Ala Asp Asn Ala
                405                 410                 415

Asn Tyr Phe Ile Leu Glu Ser Gln His Thr Leu Ser Gln Phe Ala Glu
            420                 425                 430

Lys Ile Ser Lys Leu Pro Glu Val Gln Asn Lys Tyr Phe Glu Met Leu
        435                 440                 445

Glu Phe Val Val Phe Ser Leu Asn Tyr Ile Pro Cys Lys Glu Leu Ile
    450                 455                 460

Ser Val Ser Ile Leu Leu Lys Ser Ser Ser Ser Tyr His Cys Ser Ile
465                 470                 475                 480

Ile Ala Met Lys Thr Leu Leu Lys Phe Thr Arg His Asp Tyr Ile Phe
                485                 490                 495

Lys Asp Val Phe Arg Glu Val Gly Leu Leu Glu Val Met Val Asn Leu
            500                 505                 510

Leu His Lys Tyr Ala Ala Leu Leu Lys Asp Pro Thr Gln Ala Leu Asn
        515                 520                 525

Glu Gln Gly Asp Ser Arg Asn Asn Ser Ser Val Glu Asp Gln Lys His
    530                 535                 540

Leu Ala Leu Leu Val Met Glu Thr Leu Thr Val Leu Leu Gln Gly Ser
545                 550                 555                 560

Asn Thr Asn Ala Gly Ile Phe Arg Glu Phe Gly Gly Ala Arg Cys Ala

                565                 570                 575

His Asn Ile Val Lys Tyr Pro Gln Cys Arg Gln His Ala Leu Met Thr
            580                 585                 590

Ile Gln Gln Leu Val Leu Ser Pro Asn Gly Asp Asp Asp Met Gly Thr
        595                 600                 605

Leu Leu Gly Leu Met His Ser Ala Pro Pro Thr Glu Leu Gln Leu Lys
    610                 615                 620

Thr Asp Ile Leu Arg Ala Leu Leu Ser Val Leu Arg Glu Ser His Arg
625                 630                 635                 640

Ser Arg Thr Val Phe Arg Lys Val Gly Gly Phe Val Tyr Ile Thr Ser
                645                 650                 655

Leu Leu Val Ala Met Glu Arg Ser Leu Ser Cys Pro Pro Lys Asn Gly
            660                 665                 670

Trp Glu Lys Val Asn Gln Asn Gln Val Phe Glu Leu Leu His Thr Val
        675                 680                 685

Phe Cys Thr Leu Thr Ala Ala Met Arg Tyr Glu Pro Ala Asn Ser His
    690                 695                 700

Phe Phe Lys Thr Glu Ile Gln Tyr Glu Lys Leu Ala Asp Ala Val Arg
705                 710                 715                 720

Phe Leu Gly Cys Phe Ser Asp Leu Arg Lys Ile Ser Ala Met Asn Val
                725                 730                 735

Phe Pro Ser Asn Thr Gln Pro Phe Gln Arg Leu Leu Glu Glu Asp Val
            740                 745                 750

Ile Ser Ile Glu Ser Val Ser Pro Thr Leu Arg His Cys Ser Lys Leu
        755                 760                 765

Phe Ile Tyr Leu Tyr Lys Val Ala Thr Asp Ser Phe Asp Ser Arg Ala
    770                 775                 780

Glu Gln Ile Pro Pro Cys Leu Thr Ser Glu Ser Ser Leu Pro Ser Pro
785                 790                 795                 800

Trp Gly Thr Pro Ala Leu Ser Arg Lys Arg His Ala Tyr His Ser Val
                805                 810                 815

Ser Thr Pro Pro Val Tyr Pro Pro Lys Asn Val Ala Asp Leu Lys Leu
            820                 825                 830

His Val Thr Thr Ser Ser Leu Gln Ser Ser Asp Ala Val Ile Ile His
        835                 840                 845

Pro Gly Ala Met Leu Ala Met Leu Asp Leu Leu Ala Ser Val Gly Ser
    850                 855                 860

Val Thr Gln Pro Glu His Ala Leu Asp Leu Gln Leu Ala Val Ala Asn
865                 870                 875                 880

Ile Leu Gln Ser Leu Val His Thr Glu Arg Asn Gln Gln Val Met Cys
                885                 890                 895

Glu Ala Gly Leu His Ala Arg Leu Leu Gln Arg Cys Ser Ala Ala Leu
            900                 905                 910

Ala Asp Glu Asp His Ser Leu His Pro Pro Leu Gln Arg Met Phe Glu
        915                 920                 925

Arg Leu Ala Ser Gln Ala Leu Glu Pro Met Val Leu Arg Glu Phe Leu
    930                 935                 940

Arg Leu Ala Ser Pro Leu Asn Cys Gly Ala Trp Asp Lys Lys Leu Leu
945                 950                 955                 960

Lys Gln Tyr Arg Val His Lys Pro Ser Ser Leu Ser Tyr Glu Pro Glu
                965                 970                 975

Met Arg Ser Ser Met Ile Thr Ser Leu Glu Gly Leu Gly Thr Asp Asn
            980                 985                 990

Val Phe Ser Leu His Glu Asp Asn His Tyr Arg Ile Ser Lys Ser Leu
        995                 1000                1005

Val Lys Ser Ala Glu Gly Ser Thr Val Pro Leu Thr Arg Val Lys
    1010                1015                1020

Cys Leu Val Ser Met Thr Thr Pro His Asp Ile Arg Leu His Gly
    1025                1030                1035

Ser Ser Val Thr Pro Ala Phe Val Glu Phe Asp Thr Ser Leu Glu
    1040                1045                1050

Gly Phe Gly Cys Leu Phe Leu Pro Ser Leu Ala Pro His Asn Ala
    1055                1060                1065

Pro Thr Asn Asn Thr Val Thr Thr Gly Leu Ile Asp Gly Ala Val
    1070                1075                1080

Val Ser Gly Ile Gly Ser Gly Glu Arg Phe Phe Pro Pro Pro Ser
    1085                1090                1095

Gly Leu Ser Tyr Ser Ser Trp Phe Cys Ile Glu His Phe Ser Ser
    1100                1105                1110

Pro Pro Asn Asn His Pro Val Arg Leu Leu Thr Val Val Arg Arg

1115                 1120                 1125

Ala Asn Ser Ser Glu Gln His Tyr Val Cys Leu Ala Ile Val Leu
    1130                 1135                 1140

Ser Ala Lys Asp Arg Ser Leu Ile Val Ser Thr Lys Glu Glu Leu
    1145                 1150                 1155

Leu Gln Asn Tyr Val Asp Asp Phe Ser Glu Glu Ser Ser Phe Tyr
    1160                 1165                 1170

Glu Ile Leu Pro Cys Cys Ala Arg Phe Arg Cys Gly Glu Leu Ile
    1175                 1180                 1185

Ile Glu Gly Gln Trp His His Leu Val Leu Val Met Ser Lys Gly
    1190                 1195                 1200

Met Leu Lys Asn Ser Thr Ala Ala Leu Tyr Ile Asp Gly Gln Leu
    1205                 1210                 1215

Val Asn Thr Val Lys Leu His Tyr Val His Ser Thr Pro Gly Gly
    1220                 1225                 1230

Ser Gly Ser Ala Asn Pro Pro Val Val Ser Thr Val Tyr Ala Tyr
    1235                 1240                 1245

Ile Gly Thr Pro Pro Ala Gln Arg Gln Ile Ala Ser Leu Val Trp
    1250                 1255                 1260

Arg Leu Gly Pro Thr His Phe Leu Glu Glu Val Leu Pro Ser Ser
    1265                 1270                 1275

Asn Val Thr Thr Ile Tyr Glu Leu Gly Pro Asn Tyr Val Gly Ser
    1280                 1285                 1290

Phe Gln Ala Val Cys Met Pro Cys Lys Asp Ala Lys Ser Glu Gly
    1295                 1300                 1305

Val Val Pro Ser Pro Val Ser Leu Val Pro Glu Glu Lys Val Ser
    1310                 1315                 1320

Phe Gly Leu Tyr Ala Leu Ser Val Ser Ser Leu Thr Val Ala Arg
    1325                 1330                 1335

Ile Arg Lys Val Tyr Asn Lys Leu Asp Ser Lys Ala Ile Ala Lys
    1340                 1345                 1350

Gln Leu Gly Ile Ser Ser His Glu Asn Ala Thr Pro Val Lys Leu
    1355                 1360                 1365

Ile His Asn Ser Ala Gly His Leu Asn Gly Ser Ala Arg Thr Ile
    1370                 1375                 1380

Gly Ala Ala Leu Ile Gly Tyr Leu Gly Val Arg Thr Phe Val Pro
    1385                1390                1395

Lys Pro Val Ala Thr Thr Leu Gln Tyr Val Gly Gly Ala Ala Ala
    1400                1405                1410

Ile Leu Gly Leu Val Ala Met Ala Ser Asp Val Glu Gly Leu Tyr
    1415                1420                1425

Ala Ala Val Lys Ala Leu Val Cys Val Val Lys Ser Asn Pro Leu
    1430                1435                1440

Ala Ser Lys Glu Met Glu Arg Ile Lys Gly Tyr Gln Leu Leu Ala
    1445                1450                1455

Met Leu Leu Lys Lys Lys Arg Ser Leu Leu Asn Ser His Ile Leu
    1460                1465                1470

His Leu Thr Phe Ser Leu Val Gly Thr Val Asp Ser Gly His Glu
    1475                1480                1485

Thr Ser Ile Ile Pro Asn Ser Thr Ala Phe Gln Asp Leu Leu Cys
    1490                1495                1500

Asp Phe Glu Val Trp Leu His Ala Pro Tyr Glu Leu His Leu Ser
    1505                1510                1515

Leu Phe Glu His Phe Ile Glu Leu Leu Thr Glu Ser Ser Glu Ala
    1520                1525                1530

Ser Lys Asn Ala Lys Leu Met Arg Glu Phe Gln Leu Ile Pro Lys
    1535                1540                1545

Leu Leu Leu Thr Leu Arg Asp Met Ser Leu Ser Gln Pro Thr Ile
    1550                1555                1560

Ala Ala Ile Ser Asn Val Leu Ser Phe Leu Leu Gln Gly Phe Pro
    1565                1570                1575

Ser Ser Asn Asp Leu Leu Arg Phe Gly Gln Phe Ile Ser Ser Thr
    1580                1585                1590

Leu Pro Thr Phe Ala Val Cys Glu Lys Phe Val Val Met Glu Ile
    1595                1600                1605

Asn Asn Glu Glu Lys Leu Asp Thr Gly Thr Glu Glu Glu Phe Gly
    1610                1615                1620

Gly Leu Val Ser Ala Asn Leu Ile Leu Leu Arg Asn Arg Leu Leu
    1625                1630                1635

Asp Ile Leu Leu Lys Leu Ile Tyr Thr Ser Lys Glu Lys Thr Ser

1640                    1645                    1650

Ile Asn Leu Gln Ala Cys Glu Glu Leu Val Lys Thr Leu Gly Phe
    1655                    1660                    1665

Asp Trp Ile Met Met Phe Met Glu Glu His Leu His Ser Thr Thr
    1670                    1675                    1680

Val Thr Ala Ala Met Arg Ile Leu Val Val Leu Leu Ser Asn Gln
    1685                    1690                    1695

Ser Ile Leu Ile Lys Phe Lys Glu Gly Leu Ser Gly Gly Gly Trp
    1700                    1705                    1710

Leu Glu Gln Thr Asp Ser Val Leu Thr Asn Lys Ile Gly Thr Val
    1715                    1720                    1725

Leu Gly Phe Asn Val Gly Arg Ser Ala Gly Gly Arg Ser Thr Val
    1730                    1735                    1740

Arg Glu Ile Asn Arg Asp Ala Cys His Phe Pro Gly Phe Pro Val
    1745                    1750                    1755

Leu Gln Ser Phe Leu Pro Lys His Thr Asn Val Pro Ala Leu Tyr
    1760                    1765                    1770

Phe Leu Leu Met Ala Leu Phe Leu Gln Gln Pro Val Ser Glu Leu
    1775                    1780                    1785

Pro Glu Asn Leu Gln Val Ser Val Pro Val Ile Ser Cys Arg Ser
    1790                    1795                    1800

Lys Gln Gly Cys Gln Phe Asp Leu Asp Ser Ile Trp Thr Phe Ile
    1805                    1810                    1815

Phe Gly Val Pro Ala Ser Ser Gly Thr Val Val Ser Ser Ile His
    1820                    1825                    1830

Asn Val Cys Thr Glu Ala Val Phe Leu Leu Leu Gly Met Leu Arg
    1835                    1840                    1845

Ser Met Leu Thr Ser Pro Trp Gln Ser Glu Glu Glu Gly Ser Trp
    1850                    1855                    1860

Leu Arg Glu Tyr Pro Val Thr Leu Met Gln Phe Phe Arg Tyr Leu
    1865                    1870                    1875

Tyr His Asn Val Pro Asp Leu Ala Ser Met Trp Met Ser Pro Asp
    1880                    1885                    1890

Phe Leu Cys Ala Leu Ala Ala Thr Val Phe Pro Phe Asn Ile Arg
    1895                    1900                    1905

Pro Tyr Ser Glu Met Val Thr Asp Leu Asp Asp Glu Val Gly Ser
    1910                1915                1920

Pro Ala Glu Glu Phe Lys Ala Phe Ala Ala Asp Thr Gly Met Asn
    1925                1930                1935

Arg Ser Gln Ser Glu Tyr Cys Asn Val Gly Thr Lys Thr Tyr Leu
    1940                1945                1950

Thr Asn His Pro Ala Lys Lys Phe Val Phe Asp Phe Met Arg Val
    1955                1960                1965

Leu Ile Ile Asp Asn Leu Cys Leu Thr Pro Ala Ser Lys Gln Thr
    1970                1975                1980

Pro Leu Ile Asp Leu Leu Leu Glu Ala Ser Pro Glu Arg Ser Thr
    1985                1990                1995

Arg Thr Gln Gln Lys Glu Phe Gln Thr Tyr Ile Leu Asp Ser Val
    2000                2005                2010

Met Asp His Leu Leu Ala Ala Asp Val Leu Leu Gly Glu Asp Ala
    2015                2020                2025

Ser Leu Pro Ile Thr Ser Gly Gly Ser Tyr Gln Val Leu Val Asn
    2030                2035                2040

Asn Val Phe Tyr Phe Thr Gln Arg Val Val Asp Lys Leu Trp Gln
    2045                2050                2055

Gly Met Phe Asn Lys Glu Ser Lys Leu Leu Ile Asp Phe Ile Ile
    2060                2065                2070

Gln Leu Ile Ala Gln Ser Lys Arg Arg Ser Gln Gly Leu Ser Leu
    2075                2080                2085

Asp Ala Val Tyr His Cys Leu Asn Arg Thr Ile Leu Tyr Gln Phe
    2090                2095                2100

Ser Arg Ala His Lys Thr Val Pro Gln Gln Val Ala Leu Leu Asp
    2105                2110                2115

Ser Leu Arg Val Leu Thr Val Asn Arg Asn Leu Ile Leu Gly Pro
    2120                2125                2130

Gly Asn His Asp Gln Glu Phe Ile Ser Cys Leu Ala His Cys Leu
    2135                2140                2145

Ile Asn Leu His Val Gly Ser Asn Val Asp Gly Phe Gly Leu Glu
    2150                2155                2160

Ala Glu Ala Arg Met Thr Thr Trp His Ile Met Ile Pro Ser Asp

2165                    2170                    2175

Ile Glu Pro Asp Gly Ser Tyr Ser Gln Asp Ile Ser Glu Gly Arg
    2180                    2185                    2190

Gln Leu Leu Ile Lys Ala Val Asn Arg Val Trp Thr Glu Leu Ile
    2195                    2200                    2205

His Ser Lys Lys Gln Val Leu Glu Glu Leu Phe Lys Val Thr Leu
    2210                    2215                    2220

Pro Val Asn Glu Arg Gly His Val Asp Ile Ala Thr Ala Arg Pro
    2225                    2230                    2235

Leu Ile Glu Glu Ala Ala Leu Lys Cys Trp Gln Asn His Leu Ala
    2240                    2245                    2250

His Glu Lys Lys Cys Ile Ser Arg Gly Glu Ala Leu Ala Pro Thr
    2255                    2260                    2265

Thr Gln Ser Lys Leu Ser Arg Val Ser Ser Gly Phe Gly Leu Ser
    2270                    2275                    2280

Lys Leu Thr Gly Ser Arg Arg Asn Arg Lys Glu Ser Gly Leu Asn
    2285                    2290                    2295

Lys His Ser Leu Ser Thr Gln Glu Ile Ser Gln Trp Met Phe Thr
    2300                    2305                    2310

His Ile Ala Val Val Arg Asp Leu Val Asp Thr Gln Tyr Lys Glu
    2315                    2320                    2325

Tyr Gln Glu Arg Gln Gln Asn Ala Leu Lys Tyr Val Thr Glu Glu
    2330                    2335                    2340

Trp Cys Gln Ile Glu Cys Glu Leu Leu Arg Glu Arg Gly Leu Trp
    2345                    2350                    2355

Gly Pro Pro Ile Gly Ser His Leu Asp Lys Trp Met Leu Glu Met
    2360                    2365                    2370

Thr Glu Gly Pro Cys Arg Met Arg Lys Lys Met Val Arg Asn Asp
    2375                    2380                    2385

Met Phe Tyr Asn His Tyr Pro Tyr Val Pro Glu Thr Glu Gln Glu
    2390                    2395                    2400

Thr Asn Val Ala Ser Glu Ile Pro Ser Lys Gln Pro Glu Thr Pro
    2405                    2410                    2415

Asp Asp Ile Pro Gln Lys Lys Pro Ala Arg Tyr Arg Arg Ala Val
    2420                    2425                    2430

Ser Tyr Asp Ser Lys Glu Tyr Tyr Met Arg Leu Ala Ser Gly Asn
    2435                2440                2445

Pro Ala Ile Val Gln Asp Ala Ile Val Glu Ser Ser Glu Gly Glu
    2450                2455                2460

Ala Ala Gln Gln Glu Pro Glu His Gly Glu Asp Thr Ile Ala Lys
    2465                2470                2475

Val Lys Gly Leu Val Lys Pro Pro Leu Lys Arg Ser Arg Ser Ala
    2480                2485                2490

Pro Asp Gly Gly Asp Glu Glu Asn Gln Glu Gln Leu Gln Asp Gln
    2495                2500                2505

Ile Ala Glu Gly Ser Ser Ile Glu Glu Glu Glu Lys Thr Asp Asn
    2510                2515                2520

Ala Thr Leu Leu Arg Leu Leu Glu Glu Gly Glu Lys Ile Gln His
    2525                2530                2535

Met Tyr Arg Cys Ala Arg Val Gln Gly Leu Asp Thr Ser Glu Gly
    2540                2545                2550

Leu Leu Leu Phe Gly Lys Glu His Phe Tyr Val Ile Asp Gly Phe
    2555                2560                2565

Thr Met Thr Ala Thr Arg Glu Ile Arg Asp Ile Glu Thr Leu Pro
    2570                2575                2580

Pro Asn Met His Glu Pro Ile Ile Pro Arg Gly Ala Arg Gln Gly
    2585                2590                2595

Pro Ser Gln Leu Lys Arg Thr Cys Ser Ile Phe Ala Tyr Glu Asp
    2600                2605                2610

Ile Lys Glu Val His Lys Arg Arg Tyr Leu Leu Gln Pro Ile Ala
    2615                2620                2625

Val Glu Val Phe Ser Gly Asp Gly Arg Asn Tyr Leu Leu Ala Phe
    2630                2635                2640

Gln Lys Gly Ile Arg Asn Lys Val Tyr Gln Arg Phe Leu Ala Val
    2645                2650                2655

Val Pro Ser Leu Thr Asp Ser Ser Glu Ser Val Ser Gly Gln Arg
    2660                2665                2670

Pro Asn Thr Ser Val Glu Gln Gly Ser Gly Leu Leu Ser Thr Leu
    2675                2680                2685

Val Gly Glu Lys Ser Val Thr Gln Arg Trp Glu Arg Gly Glu Ile

2690                2695                2700

Ser Asn Phe Gln Tyr Leu Met His Leu Asn Thr Leu Ala Gly Arg
    2705                2710                2715

Ser Tyr Asn Asp Leu Met Gln Tyr Pro Val Phe Pro Trp Ile Leu
    2720                2725                2730

Ala Asp Tyr Asp Ser Glu Glu Val Asp Leu Thr Asn Pro Lys Thr
    2735                2740                2745

Phe Arg Asn Leu Ala Lys Pro Met Gly Ala Gln Thr Asp Glu Arg
    2750                2755                2760

Leu Ala Gln Tyr Lys Lys Arg Tyr Lys Asp Trp Glu Asp Pro Asn
    2765                2770                2775

Gly Glu Thr Pro Ala Tyr His Tyr Gly Thr His Tyr Ser Ser Ala
    2780                2785                2790

Met Ile Val Ala Ser Tyr Leu Val Arg Met Glu Pro Phe Thr Gln
    2795                2800                2805

Ile Phe Leu Arg Leu Gln Gly Gly His Phe Asp Leu Ala Asp Arg
    2810                2815                2820

Met Phe His Ser Val Arg Glu Ala Trp Tyr Ser Ala Ser Lys His
    2825                2830                2835

Asn Met Ala Asp Val Lys Glu Leu Ile Pro Glu Phe Phe Tyr Leu
    2840                2845                2850

Pro Glu Phe Leu Phe Asn Ser Asn Asn Phe Asp Leu Gly Cys Lys
    2855                2860                2865

Gln Asn Gly Thr Lys Leu Gly Asp Val Ile Leu Pro Pro Trp Ala
    2870                2875                2880

Lys Gly Asp Pro Arg Glu Phe Ile Arg Val His Arg Glu Ala Leu
    2885                2890                2895

Glu Cys Asp Tyr Val Ser Ala His Leu His Glu Trp Ile Asp Leu
    2900                2905                2910

Ile Phe Gly Tyr Lys Gln Gln Gly Pro Ala Ala Val Glu Ala Val
    2915                2920                2925

Asn Val Phe His His Leu Phe Tyr Glu Gly Gln Val Asp Ile Tyr
    2930                2935                2940

Asn Ile Asn Asp Pro Leu Lys Glu Thr Ala Thr Ile Gly Phe Ile
    2945                2950                2955

Asn Asn Phe Gly Gln Ile Pro Lys Gln Leu Phe Lys Lys Pro His
    2960                2965                2970

Pro Pro Lys Arg Val Arg Ser Arg Leu Asn Gly Asp Asn Ala Gly
    2975                2980                2985

Ile Ser Val Leu Pro Gly Ser Thr Ser Asp Lys Ile Phe Phe His
    2990                2995                3000

His Leu Asp Asn Leu Arg Pro Ser Leu Thr Pro Val Lys Glu Leu
    3005                3010                3015

Lys Glu Pro Val Gly Gln Ile Val Cys Thr Asp Lys Gly Ile Leu
    3020                3025                3030

Ala Val Glu Gln Asn Lys Val Leu Ile Pro Pro Thr Trp Asn Lys
    3035                3040                3045

Thr Phe Ala Trp Gly Tyr Ala Asp Leu Ser Cys Arg Leu Gly Thr
    3050                3055                3060

Tyr Glu Ser Asp Lys Ala Met Thr Val Tyr Glu Cys Leu Ser Glu
    3065                3070                3075

Trp Gly Gln Ile Leu Cys Ala Ile Cys Pro Asn Pro Lys Leu Val
    3080                3085                3090

Ile Thr Gly Gly Thr Ser Thr Val Val Cys Val Trp Glu Met Gly
    3095                3100                3105

Thr Ser Lys Glu Lys Ala Lys Thr Val Thr Leu Lys Gln Ala Leu
    3110                3115                3120

Leu Gly His Thr Asp Thr Val Thr Cys Ala Thr Ala Ser Leu Ala
    3125                3130                3135

Tyr His Ile Ile Val Ser Gly Ser Arg Asp Arg Thr Cys Ile Ile
    3140                3145                3150

Trp Asp Leu Asn Lys Leu Ser Phe Leu Thr Gln Leu Arg Gly His
    3155                3160                3165

Arg Ala Pro Val Ser Ala Leu Cys Ile Asn Glu Leu Thr Gly Asp
    3170                3175                3180

Ile Val Ser Cys Ala Gly Thr Tyr Ile His Val Trp Ser Ile Asn
    3185                3190                3195

Gly Asn Pro Ile Val Ser Val Asn Thr Phe Thr Gly Arg Ser Gln
    3200                3205                3210

Gln Ile Ile Cys Cys Cys Met Ser Glu Met Asn Glu Trp Asp Thr

3215                3220                3225

Gln Asn Val Ile Val Thr Gly His Ser Asp Gly Val Val Arg Phe
    3230                3235                3240

Trp Arg Met Glu Phe Leu Gln Val Pro Glu Thr Pro Ala Pro Glu
    3245                3250                3255

Pro Ala Glu Val Leu Glu Met Gln Glu Asp Cys Pro Glu Ala Gln
    3260                3265                3270

Ile Gly Gln Glu Ala Gln Asp Glu Asp Ser Ser Asp Ser Glu Ala
    3275                3280                3285

Asp Glu Gln Ser Ile Ser Gln Asp Pro Lys Asp Thr Pro Ser Gln
    3290                3295                3300

Pro Ser Ser Thr Ser His Arg Pro Arg Ala Ala Ser Cys Arg Ala
    3305                3310                3315

Thr Ala Ala Trp Cys Thr Asp Ser Gly Ser Asp Asp Ser Arg Arg
    3320                3325                3330

Trp Ser Asp Gln Leu Ser Leu Asp Glu Lys Asp Gly Phe Ile Phe
    3335                3340                3345

Val Asn Tyr Ser Glu Gly Gln Thr Arg Ala His Leu Gln Gly Pro
    3350                3355                3360

Leu Ser His Pro His Pro Asn Pro Ile Glu Val Arg Asn Tyr Ser
    3365                3370                3375

Arg Leu Lys Pro Gly Tyr Arg Trp Glu Arg Gln Leu Val Phe Arg
    3380                3385                3390

Ser Lys Leu Thr Met His Thr Ala Phe Asp Arg Lys Asp Asn Ala
    3395                3400                3405

His Pro Ala Glu Val Thr Ala Leu Gly Ile Ser Lys Asp His Ser
    3410                3415                3420

Arg Ile Leu Val Gly Asp Ser Arg Gly Arg Val Phe Ser Trp Ser
    3425                3430                3435

Val Ser Asp Gln Pro Gly Arg Ser Ala Ala Asp His Trp Val Lys
    3440                3445                3450

Asp Glu Gly Gly Asp Ser Cys Ser Gly Cys Ser Val Arg Phe Ser
    3455                3460                3465

Leu Thr Glu Arg Arg His His Cys Arg Asn Cys Gly Gln Leu Phe
    3470                3475                3480

Cys Gln Lys Cys Ser Arg Phe Gln Ser Glu Ile Lys Arg Leu Lys
    3485                3490                3495

Ile Ser Ser Pro Val Arg Val Cys Gln Asn Cys Tyr Tyr Asn Leu
    3500                3505                3510

Gln His Glu Arg Gly Ser Glu Asp Gly Pro Arg Asn Cys
    3515                3520                3525

<210> 317
<211> 795
<212> PRT
<213> Homo sapiens

<400> 317

Met Asn Met Val Lys Arg Ile Met Gly Arg Pro Arg Gln Glu Glu Cys
1               5                   10                  15

Ser Pro Gln Asp Asn Ala Leu Gly Leu Met His Leu Arg Arg Leu Phe
            20                  25                  30

Thr Glu Leu Cys His Pro Pro Arg His Met Thr Gln Lys Glu Gln Glu
        35                  40                  45

Glu Lys Leu Tyr Met Met Leu Pro Val Phe Asn Arg Val Phe Gly Asn
    50                  55                  60

Ala Pro Pro Asn Thr Met Thr Glu Lys Phe Ser Asp Leu Leu Gln Phe
65                  70                  75                  80

Thr Thr Gln Val Ser Arg Leu Met Val Thr Glu Ile Arg Arg Arg Ala
                85                  90                  95

Ser Asn Lys Ser Thr Glu Ala Ala Ser Arg Ala Ile Val Gln Phe Leu
            100                 105                 110

Glu Ile Asn Gln Ser Glu Glu Ala Ser Arg Gly Trp Met Leu Leu Thr
        115                 120                 125

Thr Ile Asn Leu Leu Ala Ser Ser Gly Gln Lys Thr Val Asp Cys Met
    130                 135                 140

Thr Thr Met Ser Val Pro Ser Thr Leu Val Lys Cys Leu Tyr Leu Phe
145                 150                 155                 160

Phe Asp Leu Pro His Val Pro Glu Ala Val Gly Gly Ala Gln Asn Glu
                165                 170                 175

Leu Pro Leu Ala Glu Arg Arg Gly Leu Leu Gln Lys Val Phe Val Gln
            180                 185                 190

Ile Leu Val Lys Leu Cys Ser Phe Val Ser Pro Ala Glu Glu Leu Ala
        195                 200                 205

Gln Lys Asp Asp Leu Gln Leu Leu Phe Ser Ala Ile Thr Ser Trp Cys
    210                 215                 220

Pro Pro Tyr Asn Leu Pro Trp Arg Lys Ser Ala Gly Glu Val Leu Met
225                 230                 235                 240

Thr Ile Ser Arg His Gly Leu Ser Val Asn Val Val Lys Tyr Ile His
                245                 250                 255

Glu Lys Glu Cys Leu Ser Thr Cys Val Gln Asn Met Gln Gln Ser Asp
            260                 265                 270

Asp Leu Ser Pro Leu Glu Ile Val Glu Met Phe Ala Gly Leu Ser Cys
        275                 280                 285

Phe Leu Lys Asp Ser Ser Asp Val Ser Gln Thr Leu Leu Asp Asp Phe
    290                 295                 300

Arg Ile Trp Gln Gly Tyr Asn Phe Leu Cys Asp Leu Leu Leu Arg Leu
305                 310                 315                 320

Glu Gln Ala Lys Glu Ala Glu Ser Lys Asp Ala Leu Lys Asp Leu Val
                325                 330                 335

Asn Leu Ile Thr Ser Leu Thr Thr Tyr Gly Val Ser Glu Leu Lys Pro
            340                 345                 350

Ala Gly Ile Thr Thr Gly Ala Pro Phe Leu Leu Pro Gly Phe Ala Val
        355                 360                 365

Pro Gln Pro Ala Gly Lys Gly His Ser Val Arg Asn Val Gln Ala Phe
    370                 375                 380

Ala Val Leu Gln Asn Ala Phe Leu Lys Ala Lys Thr Ser Phe Leu Ala
385                 390                 395                 400

Gln Ile Ile Leu Asp Ala Ile Thr Asn Ile Tyr Met Ala Asp Asn Ala
                405                 410                 415

Asn Tyr Phe Ile Leu Glu Ser Gln His Thr Leu Ser Gln Phe Ala Glu
            420                 425                 430

Lys Ile Ser Lys Leu Pro Glu Val Gln Asn Lys Tyr Phe Glu Met Leu
        435                 440                 445

Glu Phe Val Val Phe Ser Leu Asn Tyr Ile Pro Cys Lys Glu Leu Ile
    450                 455                 460

Ser Val Ser Ile Leu Leu Lys Ser Ser Ser Ser Tyr His Cys Ser Ile
465                 470                 475                 480

Ile Ala Met Lys Thr Leu Leu Lys Phe Thr Arg His Asp Tyr Ile Phe
                485                 490                 495

Lys Asp Val Phe Arg Glu Val Gly Leu Leu Glu Val Met Val Asn Leu
            500                 505                 510

Leu His Lys Tyr Ala Ala Leu Leu Lys Asp Pro Thr Gln Ala Leu Asn
        515                 520                 525

Glu Gln Gly Asp Ser Arg Asn Asn Ser Ser Val Glu Asp Gln Lys His
    530                 535                 540

Leu Ala Leu Leu Val Met Glu Thr Leu Thr Val Leu Leu Gln Gly Ser
545                 550                 555                 560

Asn Thr Asn Ala Gly Ile Phe Arg Glu Phe Gly Gly Ala Arg Cys Ala
                565                 570                 575

His Asn Ile Val Lys Tyr Pro Gln Cys Arg Gln His Ala Leu Met Thr
            580                 585                 590

Ile Gln Gln Leu Val Leu Ser Pro Asn Gly Asp Asp Asp Met Gly Thr
        595                 600                 605

Leu Leu Gly Leu Met His Ser Ala Pro Pro Thr Glu Leu Gln Leu Lys
    610                 615                 620

Thr Asp Ile Leu Arg Ala Leu Leu Ser Val Leu Arg Glu Ser His Arg
625                 630                 635                 640

Ser Arg Thr Val Phe Arg Lys Val Gly Gly Phe Val Tyr Ile Thr Ser
                645                 650                 655

Leu Leu Val Ala Met Glu Arg Ser Leu Ser Cys Pro Pro Lys Asn Gly
            660                 665                 670

Trp Glu Lys Val Asn Gln Asn Gln Val Phe Glu Leu Leu His Thr Val
        675                 680                 685

Phe Cys Thr Leu Thr Ala Ala Met Arg Tyr Glu Pro Ala Asn Ser His
    690                 695                 700

Phe Phe Lys Thr Glu Ile Gln Tyr Glu Lys Leu Ala Asp Ala Val Arg
705                 710                 715                 720

Phe Leu Gly Cys Phe Ser Asp Leu Arg Lys Ile Ser Ala Met Asn Val
                725                 730                 735

Phe Pro Ser Asn Thr Gln Pro Phe Gln Arg Leu Leu Glu Glu Asp Val
            740                 745                 750

Ile Ser Ile Glu Ser Val Ser Pro Thr Leu Arg His Cys Ser Lys Leu
        755                 760                 765

Phe Ile Tyr Leu Tyr Lys Val Ala Thr Asp Ser Phe Asp Arg Tyr Gly
    770                 775                 780

Ser Ala Leu Ser Ser Glu Leu Lys Gly Val Tyr
785                 790                 795

<210>  318
<211>  281
<212>  PRT
<213>  Homo sapiens

<400>  318

Met Glu Phe Leu Gln Val Pro Glu Thr Pro Ala Pro Glu Pro Ala Glu
1               5                   10                  15

Val Leu Glu Met Gln Glu Asp Cys Pro Glu Ala Gln Ile Gly Gln Glu
            20                  25                  30

Ala Gln Asp Glu Asp Ser Ser Asp Ser Glu Ala Asp Glu Gln Ser Ile
        35                  40                  45

Ser Gln Asp Pro Lys Asp Thr Pro Ser Gln Pro Ser Ser Thr Ser His
    50                  55                  60

Arg Pro Arg Ala Ala Ser Cys Arg Ala Thr Ala Ala Trp Cys Thr Asp
65                  70                  75                  80

Ser Gly Ser Asp Asp Ser Arg Arg Trp Ser Asp Gln Leu Ser Leu Asp
                85                  90                  95

Glu Lys Asp Gly Phe Ile Phe Val Asn Tyr Ser Glu Gly Gln Thr Arg
            100                 105                 110

Ala His Leu Gln Gly Pro Leu Ser His Pro His Pro Asn Pro Ile Glu
        115                 120                 125

Val Arg Asn Tyr Ser Arg Leu Lys Pro Gly Tyr Arg Trp Glu Arg Gln
    130                 135                 140

Leu Val Phe Arg Ser Lys Leu Thr Met His Thr Ala Phe Asp Arg Lys
145                 150                 155                 160

Asp Asn Ala His Pro Ala Glu Val Thr Ala Leu Gly Ile Ser Lys Asp
                165                 170                 175

His Ser Arg Ile Leu Val Gly Asp Ser Arg Gly Arg Val Phe Ser Trp
            180                 185                 190

Ser Val Ser Asp Gln Pro Gly Arg Ser Ala Ala Asp His Trp Val Lys
        195                 200                 205

Asp Glu Gly Gly Asp Ser Cys Ser Gly Cys Ser Val Arg Phe Ser Leu

210                 215                 220

Thr Glu Arg Arg His His Cys Arg Asn Cys Gly Gln Leu Phe Cys Gln
225                 230                 235                 240

Lys Cys Ser Arg Phe Gln Ser Glu Ile Lys Arg Leu Lys Ile Ser Ser
                245                 250                 255

Pro Val Arg Val Cys Gln Asn Cys Tyr Tyr Asn Leu Gln His Glu Arg
            260                 265                 270

Gly Ser Glu Asp Gly Pro Arg Asn Cys
        275                 280

<210>  319
<211>  1253
<212>  PRT
<213>  Homo sapiens

<400>  319

Met Leu Ser Leu Arg Leu Gln Thr Gly Pro Glu Lys Ala Glu Leu Gln
1               5                   10                  15

Glu Leu Asn Ala Arg Leu Tyr Asp Tyr Val Cys Arg Val Arg Glu Leu
            20                  25                  30

Glu Arg Glu Asn Leu Leu Leu Glu Glu Glu Leu Arg Gly Arg Arg Gly
        35                  40                  45

Arg Glu Gly Leu Trp Ala Glu Gly Gln Ala Arg Cys Ala Glu Glu Ala
    50                  55                  60

Arg Ser Leu Arg Gln Gln Leu Asp Glu Leu Ser Trp Ala Thr Ala Leu
65                  70                  75                  80

Ala Glu Gly Glu Arg Asp Ala Leu Arg Arg Glu Leu Arg Glu Leu Gln
                85                  90                  95

Arg Leu Asp Ala Glu Glu Arg Ala Ala Arg Gly Arg Leu Asp Ala Glu
            100                 105                 110

Leu Gly Ala Gln Gln Arg Glu Leu Gln Glu Ala Leu Gly Ala Arg Ala
        115                 120                 125

Ala Leu Glu Ala Leu Leu Gly Arg Leu Gln Ala Glu Arg Arg Gly Leu
    130                 135                 140

Asp Ala Ala His Glu Arg Asp Val Arg Glu Leu Arg Ala Arg Ala Ala
145                 150                 155                 160

Ser Leu Thr Met His Phe Arg Ala Arg Ala Thr Gly Pro Ala Ala Pro
                165                 170                 175

Pro Pro Arg Leu Arg Glu Val His Asp Ser Tyr Ala Leu Leu Val Ala
            180                 185                 190

Glu Ser Trp Arg Glu Thr Val Gln Leu Tyr Glu Asp Glu Val Arg Glu
        195                 200                 205

Leu Glu Glu Ala Leu Arg Arg Gly Gln Glu Ser Arg Leu Gln Ala Glu
    210                 215                 220

Glu Glu Thr Arg Leu Cys Ala Gln Glu Ala Glu Ala Leu Arg Arg Glu
225                 230                 235                 240

Ala Leu Gly Leu Glu Gln Leu Arg Ala Arg Leu Glu Asp Ala Leu Leu
                245                 250                 255

Arg Met Arg Glu Glu Tyr Gly Ile Gln Ala Glu Glu Arg Gln Arg Val
            260                 265                 270

Ile Asp Cys Leu Glu Asp Glu Lys Ala Thr Leu Thr Leu Ala Met Ala
        275                 280                 285

Asp Trp Leu Arg Asp Tyr Gln Asp Leu Leu Gln Val Lys Thr Gly Leu
    290                 295                 300

Ser Leu Glu Val Ala Thr Tyr Arg Ala Leu Leu Glu Gly Glu Ser Asn
305                 310                 315                 320

Pro Glu Ile Val Ile Trp Ala Glu His Val Glu Asn Met Pro Ser Glu
                325                 330                 335

Phe Arg Asn Lys Ser Tyr His Tyr Thr Asp Ser Leu Leu Gln Arg Glu
            340                 345                 350

Asn Glu Arg Asn Leu Phe Ser Arg Gln Lys Ala Pro Leu Ala Ser Phe
        355                 360                 365

Asn His Ser Ser Ala Leu Tyr Ser Asn Leu Ser Gly His Arg Gly Ser
    370                 375                 380

Gln Thr Gly Thr Ser Ile Gly Gly Asp Ala Arg Arg Gly Phe Leu Gly
385                 390                 395                 400

Ser Gly Tyr Ser Ser Ser Ala Thr Thr Gln Gln Glu Asn Ser Tyr Gly
                405                 410                 415

Lys Ala Val Ser Ser Gln Thr Asn Val Arg Thr Phe Ser Pro Thr Tyr
            420                 425                 430

Gly Leu Leu Arg Asn Thr Glu Ala Gln Val Lys Thr Phe Pro Asp Arg
        435                 440                 445

Pro Lys Ala Gly Asp Thr Arg Glu Val Pro Val Tyr Ile Gly Glu Asp
    450                 455                 460

Ser Thr Ile Ala Arg Glu Ser Tyr Arg Asp Arg Arg Asp Lys Val Ala
465                 470                 475                 480

Ala Gly Ala Ser Glu Ser Thr Arg Ser Asn Glu Arg Thr Val Ile Leu
                485                 490                 495

Gly Lys Lys Thr Glu Val Lys Ala Thr Arg Glu Gln Glu Arg Asn Arg
            500                 505                 510

Pro Glu Thr Ile Arg Thr Lys Pro Glu Glu Lys Met Phe Asp Ser Lys
        515                 520                 525

Glu Lys Ala Ser Glu Glu Arg Asn Leu Arg Trp Glu Glu Leu Thr Lys
    530                 535                 540

Leu Asp Lys Glu Ala Arg Gln Arg Glu Ser Gln Gln Met Lys Glu Lys
545                 550                 555                 560

Ala Lys Glu Lys Asp Ser Leu Lys Glu Lys Ser Val Arg Glu Arg Glu
                565                 570                 575

Val Pro Ile Ser Leu Glu Val Ser Gln Asp Arg Arg Ala Glu Val Ser
            580                 585                 590

Pro Lys Gly Leu Gln Thr Pro Val Lys Asp Ala Gly Gly Gly Thr Gly
        595                 600                 605

Arg Glu Ala Glu Ala Arg Glu Leu Arg Phe Arg Leu Gly Thr Ser Asp
    610                 615                 620

Ala Thr Gly Ser Leu Gln Gly Asp Ser Met Thr Glu Thr Val Ala Glu
625                 630                 635                 640

Asn Ile Val Thr Ser Ile Leu Lys Gln Phe Thr Gln Ser Pro Glu Thr
                645                 650                 655

Glu Ala Ser Ala Asp Ser Phe Pro Asp Thr Lys Val Thr Tyr Val Asp
            660                 665                 670

Arg Lys Glu Leu Pro Gly Glu Arg Lys Thr Lys Thr Glu Ile Leu Val
        675                 680                 685

Glu Ser Lys Leu Thr Glu Asp Val Asp Val Ser Asp Glu Ala Gly Leu
    690                 695                 700

Asp Tyr Leu Leu Ser Lys Asp Ile Lys Glu Val Gly Leu Lys Gly Lys
705                 710                 715                 720

Ser Ala Glu Gln Met Ile Gly Asp Ile Ile Asn Leu Gly Leu Lys Gly
                725                 730                 735

Arg Glu Gly Arg Ala Lys Val Val Asn Val Glu Ile Val Glu Glu Pro
            740                 745                 750

Val Ser Tyr Val Ser Gly Glu Lys Pro Glu Glu Phe Ser Val Pro Phe
        755                 760                 765

Lys Val Glu Glu Val Glu Asp Val Ser Pro Gly Pro Trp Gly Leu Val
    770                 775                 780

Lys Glu Glu Glu Gly Tyr Gly Glu Ser Asp Val Thr Phe Ser Val Asn
785                 790                 795                 800

Gln His Arg Arg Thr Lys Gln Pro Gln Glu Asn Thr Thr His Val Glu
                805                 810                 815

Glu Val Thr Glu Ala Gly Asp Ser Glu Gly Glu Gln Ser Tyr Phe Val
            820                 825                 830

Ser Thr Pro Asp Glu His Pro Gly Gly His Asp Arg Asp Asp Gly Ser
        835                 840                 845

Val Tyr Gly Gln Ile His Ile Glu Glu Glu Ser Thr Ile Arg Tyr Ser
    850                 855                 860

Trp Gln Asp Glu Ile Val Gln Gly Thr Arg Arg Arg Thr Gln Lys Asp
865                 870                 875                 880

Gly Ala Val Gly Glu Lys Val Val Lys Pro Leu Asp Val Pro Ala Pro
                885                 890                 895

Ser Leu Glu Gly Asp Leu Gly Ser Thr His Trp Lys Glu Gln Ala Arg
            900                 905                 910

Ser Gly Glu Phe His Ala Glu Pro Thr Val Ile Glu Lys Glu Ile Lys
        915                 920                 925

Ile Pro His Glu Phe His Thr Ser Met Lys Gly Ile Ser Ser Lys Glu
    930                 935                 940

Pro Arg Gln Gln Leu Val Glu Val Ile Gly Gln Leu Glu Glu Thr Leu
945                 950                 955                 960

Pro Glu Arg Met Arg Glu Glu Leu Ser Ala Leu Thr Arg Glu Gly Gln
                965                 970                 975

Gly Gly Pro Gly Ser Val Ser Val Asp Val Lys Lys Val Gln Gly Ala
            980                 985                 990

Gly Gly Ser Ser Val Thr Leu Val  Ala Glu Val Asn Val  Ser Gln Thr
        995                 1000                 1005

Val Asp  Ala Asp Arg Leu Asp  Leu Glu Glu Val Ser  Lys Asp Glu
    1010                 1015                 1020

Ala Ser Glu Met Glu Lys Ala Val Glu Ser Val Val Arg Glu Ser
    1025                1030                1035

Leu Ser Arg Gln Arg Ser Pro Ala Pro Gly Ser Pro Asp Glu Glu
    1040                1045                1050

Gly Gly Ala Glu Ala Pro Ala Ala Gly Ile Arg Phe Arg Arg Trp
    1055                1060                1065

Ala Thr Arg Glu Leu Tyr Ile Pro Ser Gly Glu Ser Glu Val Ala
    1070                1075                1080

Gly Gly Ala Ser His Ser Ser Gly Gln Arg Thr Pro Gln Gly Pro
    1085                1090                1095

Val Ser Ala Thr Val Glu Val Ser Ser Pro Thr Gly Phe Ala Gln
    1100                1105                1110

Ser Gln Val Leu Glu Asp Val Ser Gln Ala Ala Arg His Ile Lys
    1115                1120                1125

Leu Gly Pro Ser Glu Val Trp Arg Thr Glu Arg Met Ser Tyr Glu
    1130                1135                1140

Gly Pro Thr Ala Glu Val Val Glu Met Asp Val Ser Asn Val Glu
    1145                1150                1155

Ala Ile Arg Ser Arg Thr Gln Glu Ala Gly Ala Leu Gly Val Ser
    1160                1165                1170

Asp Arg Gly Ser Trp Arg Asp Ala Asp Ser Arg Asn Asp Gln Ala
    1175                1180                1185

Val Gly Val Ser Phe Lys Ala Ser Ala Gly Glu Gly Asp Gln Ala
    1190                1195                1200

His Arg Glu Gln Gly Lys Glu Gln Ala Met Phe Asp Lys Lys Val
    1205                1210                1215

Gln Leu Gln Arg Met Val Asp Gln Arg Ser Val Ile Ser Asp Glu
    1220                1225                1230

Lys Lys Val Ala Leu Leu Tyr Leu Asp Asn Glu Glu Glu Glu Asn
    1235                1240                1245

Asp Gly His Trp Phe
    1250

<210>  320
<211>  1565
<212>  PRT
<213>  Homo sapiens

<400> 320
   Met Leu Ser Leu Arg Leu Gln Thr Gly Pro Glu Lys Ala Glu Leu Gln
   1               5                   10                  15
   Glu Leu Asn Ala Arg Leu Tyr Asp Tyr Val Cys Arg Val Arg Glu Leu
               20                  25                  30
   Glu Arg Glu Asn Leu Leu Leu Glu Glu Glu Leu Arg Gly Arg Arg Gly
           35                  40                  45
   Arg Glu Gly Leu Trp Ala Glu Gly Gln Ala Arg Cys Ala Glu Glu Ala
       50                  55                  60
   Arg Ser Leu Arg Gln Gln Leu Asp Glu Leu Ser Trp Ala Thr Ala Leu
   65                  70                  75                  80
   Ala Glu Gly Glu Arg Asp Ala Leu Arg Arg Glu Leu Arg Glu Leu Gln
                   85                  90                  95
   Arg Leu Asp Ala Glu Glu Arg Ala Ala Arg Gly Arg Leu Asp Ala Glu
               100                 105                 110
   Leu Gly Ala Gln Gln Arg Glu Leu Gln Glu Ala Leu Gly Ala Arg Ala
           115                 120                 125
   Ala Leu Glu Ala Leu Leu Gly Arg Leu Gln Ala Glu Arg Arg Gly Leu
       130                 135                 140
   Asp Ala Ala His Glu Arg Asp Val Arg Glu Leu Arg Ala Arg Ala Ala
   145                 150                 155                 160
   Ser Leu Thr Met His Phe Arg Ala Arg Ala Thr Gly Pro Ala Ala Pro
                   165                 170                 175
   Pro Pro Arg Leu Arg Glu Val His Asp Ser Tyr Ala Leu Leu Val Ala
               180                 185                 190
   Glu Ser Trp Arg Glu Thr Val Gln Leu Tyr Glu Asp Glu Val Arg Glu
           195                 200                 205
   Leu Glu Glu Ala Leu Arg Arg Gly Gln Glu Ser Arg Leu Gln Ala Glu
       210                 215                 220
   Glu Glu Thr Arg Leu Cys Ala Gln Glu Ala Glu Ala Leu Arg Arg Glu
   225                 230                 235                 240
   Ala Leu Gly Leu Glu Gln Leu Arg Ala Arg Leu Glu Asp Ala Leu Leu
                   245                 250                 255
   Arg Met Arg Glu Glu Tyr Gly Ile Gln Ala Glu Glu Arg Gln Arg Val
               260                 265                 270
   Ile Asp Cys Leu Glu Asp Glu Lys Ala Thr Leu Thr Leu Ala Met Ala
           275                 280                 285
   Asp Trp Leu Arg Asp Tyr Gln Asp Leu Leu Gln Val Lys Thr Gly Leu
       290                 295                 300
   Ser Leu Glu Val Ala Thr Tyr Arg Ala Leu Leu Glu Gly Glu Ser Asn
   305                 310                 315                 320
   Pro Glu Ile Val Ile Trp Ala Glu His Val Glu Asn Met Pro Ser Glu
                   325                 330                 335
   Phe Arg Asn Lys Ser Tyr His Tyr Thr Asp Ser Leu Leu Gln Arg Glu
               340                 345                 350
   Asn Glu Arg Asn Leu Phe Ser Arg Gln Lys Ala Pro Leu Ala Ser Phe
           355                 360                 365
   Asn His Ser Ser Ala Leu Tyr Ser Asn Leu Ser Gly His Arg Gly Ser
       370                 375                 380
   Gln Thr Gly Thr Ser Ile Gly Gly Asp Ala Arg Arg Gly Phe Leu Gly
   385                 390                 395                 400
   Ser Gly Tyr Ser Ser Ser Ala Thr Thr Gln Gln Glu Asn Ser Tyr Gly
                   405                 410                 415
   Lys Ala Val Ser Ser Gln Thr Asn Val Arg Thr Phe Ser Pro Thr Tyr
               420                 425                 430
   Gly Leu Leu Arg Asn Thr Glu Ala Gln Val Lys Thr Phe Pro Asp Arg
           435                 440                 445
   Pro Lys Ala Gly Asp Thr Arg Glu Val Pro Val Tyr Ile Gly Glu Asp
       450                 455                 460
   Ser Thr Ile Ala Arg Glu Ser Tyr Arg Asp Arg Arg Asp Lys Val Ala
   465                 470                 475                 480
   Ala Gly Ala Ser Glu Ser Thr Arg Ser Asn Glu Arg Thr Val Ile Leu
                   485                 490                 495
   Gly Lys Lys Thr Glu Val Lys Ala Thr Arg Glu Gln Glu Arg Asn Arg
               500                 505                 510
   Pro Glu Thr Ile Arg Thr Lys Pro Glu Glu Lys Met Phe Asp Ser Lys
           515                 520                 525
   Glu Lys Ala Ser Glu Glu Arg Asn Leu Arg Trp Glu Glu Leu Thr Lys
       530                 535                 540
   Leu Asp Lys Glu Ala Arg Gln Arg Glu Ser Gln Gln Met Lys Glu Lys
   545                 550                 555                 560
   Ala Lys Glu Lys Asp Ser Leu Lys Glu Lys Ser Val Arg Glu Arg Glu
                   565                 570                 575
   Val Pro Ile Ser Leu Glu Val Ser Gln Asp Arg Arg Ala Glu Val Ser
               580                 585                 590
   Pro Lys Gly Leu Gln Thr Pro Val Lys Asp Ala Gly Gly Gly Thr Gly
           595                 600                 605
   Arg Glu Ala Glu Ala Arg Glu Leu Arg Phe Arg Leu Gly Thr Ser Asp
       610                 615                 620
   Ala Thr Gly Ser Leu Gln Gly Asp Ser Met Thr Glu Thr Val Ala Glu
   625                 630                 635                 640
   Asn Ile Val Thr Ser Ile Leu Lys Gln Phe Thr Gln Ser Pro Glu Thr
                   645                 650                 655
   Glu Ala Ser Ala Asp Ser Phe Pro Asp Thr Lys Val Thr Tyr Val Asp
               660                 665                 670
   Arg Lys Glu Leu Pro Gly Glu Arg Lys Thr Lys Thr Glu Ile Leu Val
           675                 680                 685
   Glu Ser Lys Leu Thr Glu Asp Val Asp Val Ser Asp Glu Ala Gly Leu
       690                 695                 700
   Asp Tyr Leu Leu Ser Lys Asp Ile Lys Glu Val Gly Leu Lys Gly Lys
   705                 710                 715                 720
   Ser Ala Glu Gln Met Ile Gly Asp Ile Ile Asn Leu Gly Leu Lys Gly
                   725                 730                 735
   Arg Glu Gly Arg Ala Lys Val Val Asn Val Glu Ile Val Glu Glu Pro
               740                 745                 750
   Val Ser Tyr Val Ser Gly Glu Lys Pro Glu Glu Phe Ser Val Pro Phe
           755                 760                 765
   Lys Val Glu Glu Val Glu Asp Val Ser Pro Gly Pro Trp Gly Leu Val
       770                 775                 780
   Lys Glu Glu Glu Gly Tyr Gly Glu Ser Asp Val Thr Phe Ser Val Asn
   785                 790                 795                 800
   Gln His Arg Arg Thr Lys Gln Pro Gln Glu Asn Thr Thr His Val Glu
                   805                 810                 815
   Glu Val Thr Glu Ala Gly Asp Ser Glu Gly Glu Gln Ser Tyr Phe Val
               820                 825                 830
   Ser Thr Pro Asp Glu His Pro Gly Gly His Asp Arg Asp Asp Gly Ser
   835 840 845
   Val Tyr Gly Gln Ile His Ile Glu Glu Glu Ser Thr Ile Arg Tyr Ser
   850 855 860
   Trp Gln Asp Glu Ile Val Gln Gly Thr Arg Arg Arg Thr Gln Lys Asp
   865 870 875 880
   Gly Ala Val Gly Glu Lys Val Val Lys Pro Leu Asp Val Pro Ala Pro
   885 890 895
   Ser Leu Glu Gly Asp Leu Gly Ser Thr His Trp Lys Glu Gln Ala Arg
   900 905 910
   Ser Gly Glu Phe His Ala Glu Pro Thr Val Ile Glu Lys Glu Ile Lys
   915 920 925
   Ile Pro His Glu Phe His Thr Ser Met Lys Gly Ile Ser Ser Lys Glu
   930 935 940
   Pro Arg Gln Gln Leu Val Glu Val Ile Gly Gln Leu Glu Glu Thr Leu
   945 950 955 960
   Pro Glu Arg Met Arg Glu Glu Leu Ser Ala Leu Thr Arg Glu Gly Gln
   965 970 975
   Gly Gly Pro Gly Ser Val Ser Val Asp Val Lys Lys Val Gln Gly Ala
   980 985 990
   Gly Gly Ser Ser Val Thr Leu Val Ala Glu Val Asn Val Ser Gln Thr
   995 1000 1005
   Val Asp Ala Asp Arg Leu Asp Leu Glu Glu Val Ser Lys Asp Glu
   1010 1015 1020
   Ala Ser Glu Met Glu Lys Ala Val Glu Ser Val Val Arg Glu Ser
   1025 1030 1035
   Leu Ser Arg Gln Arg Ser Pro Ala Pro Gly Ser Pro Asp Glu Glu
   1040 1045 1050
   Gly Gly Ala Glu Ala Pro Ala Ala Gly Ile Arg Phe Arg Arg Trp
   1055 1060 1065
   Ala Thr Arg Glu Leu Tyr Ile Pro Ser Gly Glu Ser Glu Val Ala
   1070 1075 1080
   Gly Gly Ala Ser His Ser Ser Gly Gln Arg Thr Pro Gln Gly Pro
   1085 1090 1095
   Val Ser Ala Thr Val Glu Val Ser Ser Pro Thr Gly Phe Ala Gln
   1100                 1105                 1110
   Ser Gln  Val Leu Glu Asp Val  Ser Gln Ala Ala Arg  His Ile Lys
       1115                 1120                 1125
   Leu Gly  Pro Ser Glu Val Trp  Arg Thr Glu Arg Met  Ser Tyr Glu
       1130                 1135                 1140
   Gly Pro  Thr Ala Glu Val Val  Gly Val Ser Ala Gly  Gly Asp Leu
       1145                 1150                 1155
   Ser Gln  Ala Ala Ser Pro Thr  Gly Ala Ser Arg Ser  Val Arg His
       1160                 1165                 1170
   Val Thr  Leu Gly Pro Gly Gln  Ser Pro Leu Ser Arg  Glu Val Ile
       1175                 1180                 1185
   Phe Leu  Gly Pro Ala Pro Ala  Cys Pro Glu Ala Trp  Gly Ser Pro
       1190                 1195                 1200
   Glu Pro  Gly Pro Ala Glu Ser  Ser Ala Asp Met Asp  Gly Ser Gly
       1205                 1210                 1215
   Arg His  Ser Thr Phe Gly Cys  Arg Gln Phe His Ala  Glu Lys Glu
       1220                 1225                 1230
   Ile Ile  Phe Gln Gly Pro Ile  Ser Ala Ala Gly Lys  Val Gly Asp
       1235                 1240                 1245
   Tyr Phe  Ala Thr Glu Glu Ser  Val Gly Thr Gln Thr  Ser Val Arg
       1250                 1255                 1260
   Gln Leu  Gln Leu Gly Pro Lys  Glu Gly Phe Ser Gly  Gln Ile Gln
       1265                 1270                 1275
   Phe Thr  Ala Pro Leu Ser Asp  Lys Val Glu Leu Gly  Val Thr Gly
       1280                 1285                 1290
   Asp Ser  Val His Met Glu Gly  Leu Pro Gly Ser Ser  Thr Ser Ile
       1295                 1300                 1305
   Arg His  Ile Ser Ile Gly Pro  Gln Arg His Gln Thr  Thr Gln Gln
       1310                 1315                 1320
   Ile Val  Tyr His Gly Leu Val  Pro Gln Leu Gly Glu  Ser Gly Asp
       1325                 1330                 1335
   Ser Glu  Ser Thr Val His Gly  Glu Gly Ser Ala Asp  Val His Gln
       1340                 1345                 1350
   Ala Thr  His Ser His Thr Ser  Gly Arg Gln Thr Val  Met Thr Glu
       1355                 1360                 1365
   Lys Ser Thr Phe Gln Ser Val Val Ser Glu Ser Pro Gln Glu Asp
   1370 1375 1380
   Ser Ala Glu Asp Thr Ser Gly Ala Glu Met Thr Ser Gly Val Ser
   1385 1390 1395
   Arg Ser Phe Arg His Ile Arg Leu Gly Pro Thr Glu Thr Glu Thr
   1400 1405 1410
   Ser Glu His Ile Ala Ile Arg Gly Pro Val Ser Arg Thr Phe Val
   1415 1420 1425
   Leu Ala Gly Ser Ala Asp Ser Pro Glu Leu Gly Lys Leu Ala Asp
   1430 1435 1440
   Ser Ser Arg Thr Leu Arg His Ile Ala Pro Gly Pro Lys Glu Thr
   1445 1450 1455
   Ser Phe Thr Phe Gln Met Asp Val Ser Asn Val Glu Ala Ile Arg
   1460 1465 1470
   Ser Arg Thr Gln Glu Ala Gly Ala Leu Gly Val Ser Asp Arg Gly
   1475 1480 1485
   Ser Trp Arg Asp Ala Asp Ser Arg Asn Asp Gln Ala Val Gly Val
   1490 1495 1500
   Ser Phe Lys Ala Ser Ala Gly Glu Gly Asp Gln Ala His Arg Glu
   1505 1510 1515
   Gln Gly Lys Glu Gln Ala Met Phe Asp Lys Lys Val Gln Leu Gln
   1520 1525 1530
   Arg Met Val Asp Gln Arg Ser Val Ile Ser Asp Glu Lys Lys Val
   1535 1540 1545
   Ala Leu Leu Tyr Leu Asp Asn Glu Glu Glu Glu Asn Asp Gly His
   1550 1555 1560
   Trp Phe
   1565

<210> 321
<211> 687
<212> PRT
<213> Homo sapiens

<400> 321

Met Glu Arg Val Asn Asp Ala Ser Cys Gly Pro Ser Gly Cys Tyr Thr
1               5                   10                  15

Tyr Gln Val Ser Arg His Ser Thr Glu Met Leu His Asn Leu Asn Gln
20 25 . 30

Gln Arg Lys Asn Gly Gly Arg Phe Cys Asp Val Leu Leu Arg Val Gly
35 40 45

Asp Glu Ser Phe Pro Ala His Arg Ala Val Leu Ala Ala Cys Ser Glu
50 55 60

Tyr Phe Glu Ser Val Phe Ser Ala Gln Leu Gly Asp Gly Gly Ala Ala
65 70 75 80

Asp Gly Gly Pro Ala Asp Val Gly Gly Ala Thr Ala Ala Pro Gly Gly
85 90 95

Gly Ala Gly Gly Ser Arg Glu Leu Glu Met His Thr Ile Ser Ser Lys
100 105 110

Val Phe Gly Asp Ile Leu Asp Phe Ala Tyr Thr Ser Arg Ile Val Val
115 120 125

Arg Leu Glu Ser Phe Pro Glu Leu Met Thr Ala Ala Lys Phe Leu Leu
130 135 140

Met Arg Ser Val Ile Glu Ile Cys Gln Glu Val Ile Lys Gln Ser Asn
145 150 155 160

Val Gln Ile Leu Val Pro Pro Ala Arg Ala Asp Ile Met Leu Phe Arg
165 170 175

Pro Pro Gly Thr Ser Asp Leu Gly Phe Pro Leu Asp Met Thr Asn Gly
180 185 190

Ala Ala Leu Ala Ala Asn Ser Asn Gly Ile Ala Gly Ser Met Gln Pro
195 200 205

Glu Glu Glu Ala Ala Arg Ala Ala Gly Ala Ala Ile Ala Gly Gln Ala
210 215 220

Ser Leu Pro Val Leu Pro Gly Val Asp Arg Leu Pro Met Val Ala Gly
225 230 235 240

Pro Leu Ser Pro Gln Leu Leu Thr Ser Pro Phe Pro Ser Val Ala Ser
245 250 255

Ser Ala Pro Pro Leu Thr Gly Lys Arg Gly Arg Gly Arg Pro Arg Lys
260 265 270

Ala Asn Leu Leu Asp Ser Met Phe Gly Ser Pro Gly Gly Leu Arg Glu
275 280 285

Ala Gly Ile Leu Pro Cys Gly Leu Cys Gly Lys Val Phe Thr Asp Ala
290 295 300

Asn Arg Leu Arg Gln His Glu Ala Gln His Gly Val Thr Ser Leu Gln
305                 310                 315                 320

Leu Gly Tyr Ile Asp Leu Pro Pro Pro Arg Leu Gly Glu Asn Gly Leu
                325                 330                 335

Pro Ile Ser Glu Asp Pro Asp Gly Pro Arg Lys Arg Ser Arg Thr Arg
            340                 345                 350

Lys Gln Val Ala Cys Glu Ile Cys Gly Lys Ile Phe Arg Asp Val Tyr
        355                 360                 365

His Leu Asn Arg His Lys Leu Ser His Ser Gly Glu Lys Pro Tyr Ser
    370                 375                 380

Cys Pro Val Cys Gly Leu Arg Phe Lys Arg Lys Asp Arg Met Ser Tyr
385                 390                 395                 400

His Val Arg Ser His Asp Gly Ser Val Gly Lys Pro Tyr Ile Cys Gln
                405                 410                 415

Ser Cys Gly Lys Gly Phe Ser Arg Pro Asp His Leu Asn Gly His Ile
            420                 425                 430

Lys Gln Val His Thr Ser Glu Arg Pro His Lys Cys Gln Thr Cys Asn
        435                 440                 445

Ala Ser Phe Ala Thr Arg Asp Arg Leu Arg Ser His Leu Ala Cys His
    450                 455                 460

Glu Asp Lys Val Pro Cys Gln Val Cys Gly Lys Tyr Leu Arg Ala Ala
465                 470                 475                 480

Tyr Met Ala Asp His Leu Lys Lys His Ser Glu Gly Pro Ser Asn Phe
                485                 490                 495

Cys Ser Ile Cys Asn Arg Gly Phe Ser Ser Ala Ser Tyr Leu Lys Val
            500                 505                 510

His Val Lys Thr His His Gly Val Pro Leu Pro Gln Val Ser Arg His
        515                 520                 525

Gln Glu Pro Ile Leu Asn Gly Gly Ala Ala Phe His Cys Ala Arg Thr
    530                 535                 540

Tyr Gly Asn Lys Glu Gly Gln Lys Cys Ser His Gln Asp Pro Ile Glu
545                 550                 555                 560

Ser Ser Asp Ser Tyr Gly Asp Leu Ser Asp Ala Ser Asp Leu Lys Thr
                565                 570                 575

Pro Glu Lys Gln Ser Ala Asn Gly Ser Phe Ser Cys Asp Met Ala Val
            580                 585                 590

Pro Lys Asn Lys Met Glu Ser Asp Gly Glu Lys Lys Tyr Pro Cys Pro
        595                 600                 605

Glu Cys Gly Ser Phe Phe Arg Ser Lys Ser Tyr Leu Asn Lys His Ile
    610                 615                 620

Gln Lys Val His Val Arg Ala Leu Gly Gly Pro Leu Gly Asp Leu Gly
625                 630                 635                 640

Pro Ala Leu Gly Ser Pro Phe Ser Pro Gln Gln Asn Met Ser Leu Leu
                645                 650                 655

Glu Ser Phe Gly Phe Gln Ile Val Gln Ser Ala Phe Ala Ser Ser Leu
            660                 665                 670

Val Asp Pro Glu Val Asp Gln Gln Pro Met Gly Pro Glu Gly Lys
        675                 680                 685

<210>  322
<211>  641
<212>  PRT
<213>  Homo sapiens

<400>  322

Met Glu Arg Val Asn Asp Ala Ser Cys Gly Pro Ser Gly Cys Tyr Thr
1               5                   10                  15

Tyr Gln Val Ser Arg His Ser Thr Glu Met Leu His Asn Leu Asn Gln
            20                  25                  30

Gln Arg Lys Asn Gly Gly Arg Phe Cys Asp Val Leu Leu Arg Val Gly
        35                  40                  45

Asp Glu Ser Phe Pro Ala His Arg Ala Val Leu Ala Ala Cys Ser Glu
    50                  55                  60

Tyr Phe Glu Ser Val Phe Ser Ala Gln Leu Gly Asp Gly Gly Ala Ala
65                  70                  75                  80

Asp Gly Gly Pro Ala Asp Val Gly Gly Ala Thr Ala Ala Pro Gly Gly
                85                  90                  95

Gly Ala Gly Gly Ser Arg Glu Leu Glu Met His Thr Ile Ser Ser Lys
            100                 105                 110

Val Phe Gly Asp Ile Leu Asp Phe Ala Tyr Thr Ser Arg Ile Val Val
        115                 120                 125

Arg Leu Glu Ser Phe Pro Glu Leu Met Thr Ala Ala Lys Phe Leu Leu
    130                 135                 140

Met Arg Ser Val Ile Glu Ile Cys Gln Glu Val Ile Lys Gln Ser Asn

145                 150                 155                 160

Val Gln Ile Leu Val Pro Pro Ala Arg Ala Asp Ile Met Leu Phe Arg
                165                 170                 175

Pro Pro Gly Thr Ser Asp Leu Gly Phe Pro Leu Asp Met Thr Asn Gly
            180                 185                 190

Ala Ala Leu Ala Ala Asn Ser Asn Gly Ile Ala Gly Ser Met Gln Pro
        195                 200                 205

Glu Glu Glu Ala Ala Arg Ala Ala Gly Ala Ala Ile Ala Gly Gln Ala
    210                 215                 220

Ser Leu Pro Val Leu Pro Gly Val Asp Arg Leu Pro Met Val Ala Gly
225                 230                 235                 240

Pro Leu Ser Pro Gln Leu Leu Thr Ser Pro Phe Pro Ser Val Ala Ser
                245                 250                 255

Ser Ala Pro Pro Leu Thr Gly Lys Arg Gly Arg Gly Arg Pro Arg Lys
            260                 265                 270

Ala Asn Leu Leu Asp Ser Met Phe Gly Ser Pro Gly Gly Leu Arg Glu
        275                 280                 285

Ala Gly Ile Leu Pro Cys Gly Leu Cys Gly Lys Val Phe Thr Asp Ala
    290                 295                 300

Asn Arg Leu Arg Gln His Glu Ala Gln His Gly Val Thr Ser Leu Gln
305                 310                 315                 320

Leu Gly Tyr Ile Asp Leu Pro Pro Pro Arg Leu Gly Glu Asn Gly Leu
                325                 330                 335

Pro Ile Ser Glu Asp Pro Asp Gly Pro Arg Lys Arg Ser Arg Thr Arg
            340                 345                 350

Lys Gln Val Ala Cys Glu Ile Cys Gly Lys Ile Phe Arg Asp Val Tyr
        355                 360                 365

His Leu Asn Arg His Lys Leu Ser His Ser Gly Glu Lys Pro Tyr Ser
    370                 375                 380

Cys Pro Val Cys Gly Leu Arg Phe Lys Arg Lys Asp Arg Met Ser Tyr
385                 390                 395                 400

His Val Arg Ser His Asp Gly Ser Val Gly Lys Pro Tyr Ile Cys Gln
                405                 410                 415

Ser Cys Gly Lys Gly Phe Ser Arg Pro Asp His Leu Asn Gly His Ile
            420                 425                 430

Lys Gln Val His Thr Ser Glu Arg Pro His Lys Cys Gln Thr Cys Asn
        435                 440                 445

Ala Ser Phe Ala Thr Arg Asp Arg Leu Arg Ser His Leu Ala Cys His
    450                 455                 460

Glu Asp Lys Val Pro Cys Gln Val Cys Gly Lys Tyr Leu Arg Ala Ala
465                 470                 475                 480

Tyr Met Ala Asp His Leu Lys Lys His Ser Glu Gly Pro Ser Asn Phe
                485                 490                 495

Cys Ser Ile Cys Asn Arg Glu Gly Gln Lys Cys Ser His Gln Asp Pro
            500                 505                 510

Ile Glu Ser Ser Asp Ser Tyr Gly Asp Leu Ser Asp Ala Ser Asp Leu
        515                 520                 525

Lys Thr Pro Glu Lys Gln Ser Ala Asn Gly Ser Phe Ser Cys Asp Met
    530                 535                 540

Ala Val Pro Lys Asn Lys Met Glu Ser Asp Gly Glu Lys Lys Tyr Pro
545                 550                 555                 560

Cys Pro Glu Cys Gly Ser Phe Phe Arg Ser Lys Ser Tyr Leu Asn Lys
                565                 570                 575

His Ile Gln Lys Val His Val Arg Ala Leu Gly Gly Pro Leu Gly Asp
            580                 585                 590

Leu Gly Pro Ala Leu Gly Ser Pro Phe Ser Pro Gln Gln Asn Met Ser
        595                 600                 605

Leu Leu Glu Ser Phe Gly Phe Gln Ile Val Gln Ser Ala Phe Ala Ser
    610                 615                 620

Ser Leu Val Asp Pro Glu Val Asp Gln Gln Pro Met Gly Pro Glu Gly
625                 630                 635                 640

Lys

<210>  323
<211>  537
<212>  PRT
<213>  Homo sapiens

<400>  323

Met Glu Arg Val Asn Asp Ala Ser Cys Gly Pro Ser Gly Cys Tyr Thr
1               5                   10                  15

Tyr Gln Val Ser Arg His Ser Thr Glu Met Leu His Asn Leu Asn Gln
            20                  25                  30

Gln Arg Lys Asn Gly Gly Arg Phe Cys Asp Val Leu Leu Arg Val Gly
        35                  40                  45

Asp Glu Ser Phe Pro Ala His Arg Ala Val Leu Ala Ala Cys Ser Glu
    50                  55                  60

Tyr Phe Glu Ser Val Phe Ser Ala Gln Leu Gly Asp Gly Gly Ala Ala
65                  70                  75                  80

Asp Gly Gly Pro Ala Asp Val Gly Gly Ala Thr Ala Ala Pro Gly Gly
                85                  90                  95

Gly Ala Gly Gly Ser Arg Glu Leu Glu Met His Thr Ile Ser Ser Lys
            100                 105                 110

Val Phe Gly Asp Ile Leu Asp Phe Ala Tyr Thr Ser Arg Ile Val Val
        115                 120                 125

Arg Leu Glu Ser Phe Pro Glu Leu Met Thr Ala Ala Lys Phe Leu Leu
    130                 135                 140

Met Arg Ser Val Ile Glu Ile Cys Gln Glu Val Ile Lys Gln Ser Asn
145                 150                 155                 160

Val Gln Ile Leu Val Pro Pro Ala Arg Ala Asp Ile Met Leu Phe Arg
                165                 170                 175

Pro Pro Gly Thr Ser Asp Leu Gly Phe Pro Leu Asp Met Thr Asn Gly
            180                 185                 190

Ala Ala Leu Ala Ala Asn Ser Asn Gly Ile Ala Gly Ser Met Gln Pro
        195                 200                 205

Glu Glu Glu Ala Ala Arg Ala Ala Gly Ala Ala Ile Ala Gly Gln Ala
    210                 215                 220

Ser Leu Pro Val Leu Pro Gly Val Asp Arg Leu Pro Met Val Ala Gly
225                 230                 235                 240

Pro Leu Ser Pro Gln Leu Leu Thr Ser Pro Phe Pro Ser Val Ala Ser
                245                 250                 255

Ser Ala Pro Pro Leu Thr Gly Lys Arg Gly Arg Gly Arg Pro Arg Lys
            260                 265                 270

Ala Asn Leu Leu Asp Ser Met Phe Gly Ser Pro Gly Gly Leu Arg Glu
        275                 280                 285

Ala Gly Ile Leu Pro Cys Gly Leu Cys Gly Lys Val Phe Thr Asp Ala
    290                 295                 300

Asn Arg Leu Arg Gln His Glu Ala Gln His Gly Val Thr Ser Leu Gln
305                 310                 315                 320

Leu Gly Tyr Ile Asp Leu Pro Pro Pro Arg Leu Gly Glu Asn Gly Leu
                325                 330                 335

Pro Ile Ser Glu Asp Pro Asp Gly Pro Arg Lys Arg Ser Arg Thr Arg
            340                 345                 350

Lys Gln Val Ala Cys Glu Ile Cys Gly Lys Ile Phe Arg Asp Val Tyr
        355                 360                 365

His Leu Asn Arg His Lys Leu Ser His Ser Gly Glu Lys Pro Tyr Ser
    370                 375                 380

Cys Pro Val Cys Gly Leu Arg Phe Lys Arg Lys Asp Arg Met Ser Tyr
385                 390                 395                 400

His Val Arg Ser His Asp Gly Ser Val Gly Lys Pro Tyr Ile Cys Gln
                405                 410                 415

Ser Cys Gly Lys Gly Phe Ser Arg Pro Asp His Leu Asn Gly His Ile
            420                 425                 430

Lys Gln Val His Thr Ser Glu Arg Pro His Lys Cys Gln Val Trp Val
        435                 440                 445

Gly Ser Ser Ser Gly Leu Pro Pro Leu Glu Pro Leu Pro Ser Asp Leu
    450                 455                 460

Pro Ser Trp Asp Phe Ala Gln Pro Ala Leu Trp Arg Ser Ser His Ser
465                 470                 475                 480

Val Pro Asp Thr Ala Phe Ser Leu Ser Leu Lys Lys Ser Phe Pro Ala
                485                 490                 495

Leu Glu Asn Leu Gly Pro Ala His Ser Ser Asn Thr Leu Phe Cys Pro
            500                 505                 510

Ala Pro Pro Gly Tyr Leu Arg Gln Gly Trp Thr Thr Pro Glu Gly Ser
        515                 520                 525

Arg Ala Phe Thr Gln Trp Pro Val Gly
    530                 535

<210>  324
<211>  537
<212>  PRT
<213>  Homo sapiens

<400>  324

Met Glu Arg Val Asn Asp Ala Ser Cys Gly Pro Ser Gly Cys Tyr Thr
1               5                   10                  15

Tyr Gln Val Ser Arg His Ser Thr Glu Met Leu His Asn Leu Asn Gln
            20                  25                  30

Gln Arg Lys Asn Gly Gly Arg Phe Cys Asp Val Leu Leu Arg Val Gly
        35                  40                  45

Asp Glu Ser Phe Pro Ala His Arg Ala Val Leu Ala Ala Cys Ser Glu
    50                  55                  60

Tyr Phe Glu Ser Val Phe Ser Ala Gln Leu Gly Asp Gly Gly Ala Ala
65                  70                  75                  80

Asp Gly Gly Pro Ala Asp Val Gly Gly Ala Thr Ala Ala Pro Gly Gly
                85                  90                  95

Gly Ala Gly Gly Ser Arg Glu Leu Glu Met His Thr Ile Ser Ser Lys
            100                 105                 110

Val Phe Gly Asp Ile Leu Asp Phe Ala Tyr Thr Ser Arg Ile Val Val
        115                 120                 125

Arg Leu Glu Ser Phe Pro Glu Leu Met Thr Ala Ala Lys Phe Leu Leu
    130                 135                 140

Met Arg Ser Val Ile Glu Ile Cys Gln Glu Val Ile Lys Gln Ser Asn
145                 150                 155                 160

Val Gln Ile Leu Val Pro Pro Ala Arg Ala Asp Ile Met Leu Phe Arg
                165                 170                 175

Pro Pro Gly Thr Ser Asp Leu Gly Phe Pro Leu Asp Met Thr Asn Gly
            180                 185                 190

Ala Ala Leu Ala Ala Asn Ser Asn Gly Ile Ala Gly Ser Met Gln Pro
        195                 200                 205

Glu Glu Glu Ala Ala Arg Ala Ala Gly Ala Ala Ile Ala Gly Gln Ala
    210                 215                 220

Ser Leu Pro Val Leu Pro Gly Val Asp Arg Leu Pro Met Val Ala Gly
225                 230                 235                 240

Pro Leu Ser Pro Gln Leu Leu Thr Ser Pro Phe Pro Ser Val Ala Ser
                245                 250                 255

Ser Ala Pro Pro Leu Thr Gly Lys Arg Gly Arg Gly Arg Pro Arg Lys
            260                 265                 270

Ala Asn Leu Leu Asp Ser Met Phe Gly Ser Pro Gly Gly Leu Arg Glu
        275                 280                 285

Ala Gly Ile Leu Pro Cys Gly Leu Cys Gly Lys Val Phe Thr Asp Ala

290                 295                 300

Asn Arg Leu Arg Gln His Glu Ala Gln His Gly Val Thr Ser Leu Gln
305                 310                 315                 320

Leu Gly Tyr Ile Asp Leu Pro Pro Pro Arg Leu Gly Glu Asn Gly Leu
                325                 330                 335

Pro Ile Ser Glu Asp Pro Asp Gly Pro Arg Lys Arg Ser Arg Thr Arg
            340                 345                 350

Lys Gln Val Ala Cys Glu Ile Cys Gly Lys Ile Phe Arg Asp Val Tyr
        355                 360                 365

His Leu Asn Arg His Lys Leu Ser His Ser Gly Glu Lys Pro Tyr Ser
    370                 375                 380

Cys Pro Val Cys Gly Leu Arg Phe Lys Arg Lys Asp Arg Met Ser Tyr
385                 390                 395                 400

His Val Arg Ser His Asp Gly Ser Val Gly Lys Pro Tyr Ile Cys Gln
                405                 410                 415

Ser Cys Gly Lys Gly Phe Ser Arg Pro Asp His Leu Asn Gly His Ile
            420                 425                 430

Lys Gln Val His Thr Ser Glu Arg Pro His Lys Cys Gln Thr Cys Asn
        435                 440                 445

Ala Ser Phe Ala Thr Arg Asp Arg Leu Arg Ser His Leu Ala Cys His
    450                 455                 460

Glu Asp Lys Val Pro Cys Gln Val Cys Gly Lys Tyr Leu Arg Ala Ala
465                 470                 475                 480

Tyr Met Ala Asp His Leu Lys Lys His Ser Glu Gly Pro Ser Asn Phe
                485                 490                 495

Cys Ser Ile Cys Asn Arg Gly Leu Gln Ala Pro Gly Ala His Pro Glu
            500                 505                 510

Trp Gly Ser Ser Val Pro Leu Arg Gln Asp Leu Trp Gln Gln Arg Arg
        515                 520                 525

Pro Glu Met Leu Thr Ser Gly Ser Asp
    530                 535

<210>  325
<211>  1232
<212>  PRT
<213>  Homo sapiens

<400>  325

Met Lys Glu Glu Val Lys Gly Ile Pro Val Arg Val Ala Leu Arg Cys
1               5                   10                  15

Arg Pro Leu Val Pro Lys Glu Ile Ser Glu Gly Cys Gln Met Cys Leu
            20                  25                  30

Ser Phe Val Pro Gly Glu Pro Gln Val Val Val Gly Thr Asp Lys Ser
        35                  40                  45

Phe Thr Tyr Asp Phe Val Phe Asp Pro Ser Thr Glu Gln Glu Glu Val
    50                  55                  60

Phe Asn Thr Ala Val Ala Pro Leu Ile Lys Gly Val Phe Lys Gly Tyr
65                  70                  75                  80

Asn Ala Thr Val Leu Ala Tyr Gly Gln Thr Gly Ser Gly Lys Thr Tyr
                85                  90                  95

Ser Met Gly Gly Ala Tyr Thr Ala Glu Gln Glu Asn Glu Pro Thr Val
            100                 105                 110

Gly Val Ile Pro Arg Val Ile Gln Leu Leu Phe Lys Glu Ile Asp Lys
        115                 120                 125

Lys Ser Asp Phe Glu Phe Thr Leu Lys Val Ser Tyr Leu Glu Ile Tyr
    130                 135                 140

Asn Glu Glu Ile Leu Asp Leu Leu Cys Pro Ser Arg Glu Lys Ala Gln
145                 150                 155                 160

Ile Asn Ile Arg Glu Asp Pro Lys Glu Gly Ile Lys Ile Val Gly Leu
                165                 170                 175

Thr Glu Lys Thr Val Leu Val Ala Leu Asp Thr Val Ser Cys Leu Glu
            180                 185                 190

Gln Gly Asn Asn Ser Arg Thr Val Ala Ser Thr Ala Met Asn Ser Gln
        195                 200                 205

Ser Ser Arg Ser His Ala Ile Phe Thr Ile Ser Leu Glu Gln Gly Lys
    210                 215                 220

Lys Ser Asp Lys Asn Ser Ser Phe Arg Ser Lys Leu His Leu Val Asp
225                 230                 235                 240

Leu Ala Gly Ser Glu Arg Gln Lys Lys Thr Lys Ala Glu Gly Asp Arg
                245                 250                 255

Leu Lys Glu Gly Ile Asn Ile Asn Arg Gly Leu Leu Cys Leu Gly Asn
            260                 265                 270

Val Ile Ser Ala Leu Gly Asp Asp Lys Lys Gly Gly Phe Ala Pro Tyr
        275                 280                 285

Arg Asp Ser Lys Leu Thr Arg Leu Leu Gln Asp Ser Leu Gly Gly Asn
    290                 295                 300

Ser His Thr Leu Met Ile Ala Cys Val Ser Pro Ala Asp Ser Asn Leu
305                 310                 315                 320

Glu Glu Thr Leu Asn Thr Leu Arg Tyr Ala Asp Arg Ala Arg Lys Ile
                325                 330                 335

Lys Asn Lys Pro Ile Val Asn Ile Asp Pro Gln Thr Ala Glu Leu Asn
            340                 345                 350

His Leu Lys Gln Gln Val Gln Gln Leu Gln Val Leu Leu Leu Gln Ala
        355                 360                 365

His Gly Gly Thr Leu Pro Gly Ser Ile Thr Val Glu Pro Ser Glu Asn
    370                 375                 380

Leu Gln Ser Leu Met Glu Lys Asn Gln Ser Leu Val Glu Glu Asn Glu
385                 390                 395                 400

Lys Leu Ser Arg Gly Leu Ser Glu Ala Ala Gly Gln Thr Ala Gln Met
                405                 410                 415

Leu Glu Arg Ile Ile Trp Thr Glu Gln Ala Asn Glu Lys Met Asn Ala
            420                 425                 430

Lys Leu Glu Glu Leu Arg Gln His Ala Ala Cys Lys Leu Asp Leu Gln
        435                 440                 445

Lys Leu Val Glu Thr Leu Glu Asp Gln Glu Leu Lys Glu Asn Val Glu
    450                 455                 460

Ile Ile Cys Asn Leu Gln Gln Leu Ile Thr Gln Leu Ser Asp Glu Thr
465                 470                 475                 480

Val Ala Cys Met Ala Ala Ala Ile Asp Thr Ala Val Glu Gln Glu Ala
                485                 490                 495

Gln Val Glu Thr Ser Pro Glu Thr Ser Arg Ser Ser Asp Ala Phe Thr
            500                 505                 510

Thr Gln His Ala Leu Arg Gln Ala Gln Met Ser Lys Glu Leu Val Glu
        515                 520                 525

Leu Asn Lys Ala Leu Ala Leu Lys Glu Ala Leu Ala Arg Lys Met Thr
    530                 535                 540

Gln Asn Asp Ser Gln Leu Gln Pro Ile Gln Tyr Gln Tyr Gln Asp Asn
545                 550                 555                 560

Ile Lys Glu Pro Glu Leu Glu Val Ile Asn Leu Gln Lys Glu Lys Glu
                565                 570                 575

Glu Leu Val Leu Glu Leu Gln Thr Ala Lys Lys Asp Ala Asn Gln Ala
            580                 585                 590

Lys Leu Ser Glu Arg Arg Arg Lys Arg Leu Gln Glu Leu Glu Gly Gln
        595                 600                 605

Ile Ala Asp Leu Lys Lys Lys Leu Asn Glu Gln Ser Lys Leu Leu Lys
    610                 615                 620

Leu Lys Glu Ser Thr Glu Arg Thr Val Ser Lys Leu Asn Gln Glu Ile
625                 630                 635                 640

Arg Met Met Lys Asn Gln Arg Val Gln Leu Met Arg Gln Met Lys Glu
                645                 650                 655

Asp Ala Glu Lys Phe Arg Gln Trp Lys Gln Lys Arg Asp Lys Glu Val
            660                 665                 670

Ile Gln Leu Lys Glu Arg Asp Arg Lys Arg Gln Tyr Glu Leu Leu Lys
        675                 680                 685

Leu Glu Arg Asn Phe Gln Lys Gln Ser Asn Val Leu Arg Arg Lys Thr
    690                 695                 700

Glu Glu Ala Ala Ala Ala Asn Lys Arg Leu Lys Asp Ala Leu Gln Lys
705                 710                 715                 720

Gln Arg Glu Val Ala Asp Lys Arg Lys Glu Thr Gln Ser Arg Gly Met
                725                 730                 735

Glu Gly Thr Ala Ala Arg Val Lys Asn Trp Leu Gly Asn Glu Ile Glu
            740                 745                 750

Val Met Val Ser Thr Glu Glu Ala Lys Arg His Leu Asn Asp Leu Leu
        755                 760                 765

Glu Asp Arg Lys Ile Leu Ala Gln Asp Val Ala Gln Leu Lys Glu Lys
    770                 775                 780

Lys Glu Ser Gly Glu Asn Pro Pro Pro Lys Leu Arg Arg Arg Thr Phe
785                 790                 795                 800

Ser Leu Thr Glu Val Arg Gly Gln Val Ser Glu Ser Glu Asp Ser Ile
                805                 810                 815

Thr Lys Gln Ile Glu Ser Leu Glu Thr Glu Met Glu Phe Arg Ser Ala
            820                 825                 830

Gln Ile Ala Asp Leu Gln Gln Lys Leu Leu Asp Ala Glu Ser Glu Asp
        835                 840                 845

Arg Pro Lys Gln Arg Trp Glu Asn Ile Ala Thr Ile Leu Glu Ala Lys
    850                 855                 860

Cys Ala Leu Lys Tyr Leu Ile Gly Glu Leu Val Ser Ser Lys Ile Gln
865                 870                 875                 880

Val Ser Lys Leu Glu Ser Ser Leu Lys Gln Ser Lys Thr Ser Cys Ala
                885                 890                 895

Asp Met Gln Lys Met Leu Phe Glu Glu Arg Asn His Phe Ala Glu Ile
            900                 905                 910

Glu Thr Glu Leu Gln Ala Glu Leu Val Arg Met Glu Gln Gln His Gln
        915                 920                 925

Glu Lys Val Leu Tyr Leu Leu Ser Gln Leu Gln Gln Ser Gln Met Ala
    930                 935                 940

Glu Lys Gln Leu Glu Glu Ser Val Ser Glu Lys Glu Gln Gln Leu Leu
945                 950                 955                 960

Ser Thr Leu Lys Cys Gln Asp Glu Glu Leu Glu Lys Met Arg Glu Val
                965                 970                 975

Cys Glu Gln Asn Gln Gln Leu Leu Arg Glu Asn Glu Ile Ile Lys Gln
            980                 985                 990

Lys Leu Thr Leu Leu Gln Val Ala  Ser Arg Gln Lys His  Leu Pro Lys
        995                 1000                1005

Asp Thr  Leu Leu Ser Pro Asp  Ser Ser Phe Glu Tyr  Val Gln Pro
    1010                1015                1020

Lys Pro  Lys Pro Ser Arg Val  Lys Glu Lys Phe Leu  Glu Gln Ser
    1025                1030                1035

Met Asp  Ile Glu Asp Leu Lys  Tyr Cys Ser Glu His  Ser Val Asn
    1040                1045                1050

Glu His  Glu Asp Gly Asp Gly  Asp Asp Asp Glu Gly  Asp Asp Glu
    1055                1060                1065

Glu Trp  Lys Pro Thr Lys Leu  Val Asn Val Ser Arg  Lys Asn Ile
    1070                1075                1080

Gln Gly  Cys Ser Cys Lys Gly  Trp Cys Gly Asn Lys  Gln Cys Gly
    1085                1090                1095

Cys Arg  Lys Gln Lys Ser Asp  Cys Gly Val Asp Cys  Cys Cys Asp
    1100                1105                1110

Pro Thr Lys Cys Arg Asn Arg Gln Gln Gly Lys Asp Ser Leu Gly
    1115                1120                1125

Thr Val Glu Arg Thr Gln Asp Ser Glu Ser Ser Phe Lys Leu Glu
    1130                1135                1140

Asp Pro Thr Glu Val Thr Pro Gly Leu Ser Phe Phe Asn Pro Val
    1145                1150                1155

Cys Ala Thr Pro Asn Ser Lys Ile Leu Lys Glu Met Cys Asp Val
    1160                1165                1170

Glu Gln Val Leu Ser Lys Lys Thr Pro Pro Ala Pro Ser Pro Phe
    1175                1180                1185

Asp Leu Pro Glu Leu Lys His Val Ala Thr Glu Tyr Gln Glu Asn
    1190                1195                1200

Lys Ala Pro Gly Lys Lys Lys Lys Arg Ala Leu Ala Ser Asn Thr
    1205                1210                1215

Ser Phe Phe Ser Gly Cys Ser Pro Ile Glu Glu Glu Ala His
    1220                1225                1230

<210> 326
<211> 139
<212> PRT
<213> Homo sapiens

<400> 326

Met Leu Pro Leu Ser Leu Leu Lys Thr Ala Gln Asn His Pro Met Leu
1               5                   10                  15

Val Glu Leu Lys Asn Gly Glu Thr Tyr Asn Gly His Leu Val Ser Cys
            20                  25                  30

Asp Asn Trp Met Asn Ile Asn Leu Arg Glu Val Ile Cys Thr Ser Arg
        35                  40                  45

Asp Gly Asp Lys Phe Trp Arg Met Pro Glu Cys Tyr Ile Arg Gly Ser
    50                  55                  60

Thr Ile Lys Tyr Leu Arg Ile Pro Asp Glu Ile Ile Asp Met Val Lys
65                  70                  75                  80

Glu Glu Val Val Ala Lys Gly Arg Gly Arg Gly Gly Leu Gln Gln Gln
                85                  90                  95

Lys Gln Gln Lys Gly Arg Gly Met Gly Gly Ala Gly Arg Gly Val Phe
            100                 105                 110

Gly Gly Arg Gly Arg Gly Gly Ile Pro Gly Thr Gly Arg Gly Gln Pro
        115                 120                 125

Glu Lys Lys Pro Gly Arg Gln Ala Gly Lys Gln
    130                 135

<210>  327
<211>  447
<212>  PRT
<213>  Homo sapiens

<400>  327

Met Ser Arg Arg Pro Cys Ser Cys Ala Leu Arg Pro Pro Arg Cys Ser
1               5                   10                  15

Cys Ser Ala Ser Pro Ser Ala Val Thr Ala Ala Gly Arg Pro Arg Pro
            20                  25                  30

Ser Asp Ser Cys Lys Glu Glu Ser Ser Thr Leu Ser Val Lys Met Lys
        35                  40                  45

Cys Asp Phe Asn Cys Asn His Val His Ser Gly Leu Lys Leu Val Lys
    50                  55                  60

Pro Asp Asp Ile Gly Arg Leu Val Ser Tyr Thr Pro Ala Tyr Leu Glu
65                  70                  75                  80

Gly Ser Cys Lys Asp Cys Ile Lys Asp Tyr Glu Arg Leu Ser Cys Ile
                85                  90                  95

Gly Ser Pro Ile Val Ser Pro Arg Ile Val Gln Leu Glu Thr Glu Ser
            100                 105                 110

Lys Arg Leu His Asn Lys Glu Asn Gln His Val Gln Gln Thr Leu Asn
        115                 120                 125

Ser Thr Asn Glu Ile Glu Ala Leu Glu Thr Ser Arg Leu Tyr Glu Asp
    130                 135                 140

Ser Gly Tyr Ser Ser Phe Ser Leu Gln Ser Gly Leu Ser Glu His Glu
145                 150                 155                 160

Glu Gly Ser Leu Leu Glu Glu Asn Phe Gly Asp Ser Leu Gln Ser Cys
                165                 170                 175

Leu Leu Gln Ile Gln Ser Pro Asp Gln Tyr Pro Asn Lys Asn Leu Leu
            180                 185                 190

Pro Val Leu His Phe Glu Lys Val Val Cys Ser Thr Leu Lys Lys Asn
        195                 200                 205

Ala Lys Arg Asn Pro Lys Val Asp Arg Glu Met Leu Lys Glu Ile Ile
    210                 215                 220

Ala Arg Gly Asn Phe Arg Leu Gln Asn Ile Ile Gly Arg Lys Met Gly
225                 230                 235                 240

Leu Glu Cys Val Asp Ile Leu Ser Glu Leu Phe Arg Arg Gly Leu Arg
                245                 250                 255

His Val Leu Ala Thr Ile Leu Ala Gln Leu Ser Asp Met Asp Leu Ile
            260                 265                 270

Asn Val Ser Lys Val Ser Thr Thr Trp Lys Lys Ile Leu Glu Asp Asp
        275                 280                 285

Lys Gly Ala Phe Gln Leu Tyr Ser Lys Ala Ile Gln Arg Val Thr Glu
    290                 295                 300

Asn Asn Asn Lys Phe Ser Pro His Ala Ser Thr Arg Glu Tyr Val Met
305                 310                 315                 320

Phe Arg Thr Pro Leu Ala Ser Val Gln Lys Ser Ala Ala Gln Thr Ser
                325                 330                 335

Leu Lys Lys Asp Ala Gln Thr Lys Leu Ser Asn Gln Gly Asp Gln Lys
            340                 345                 350

Gly Ser Thr Tyr Ser Arg His Asn Glu Phe Ser Glu Val Ala Lys Thr
        355                 360                 365

Leu Lys Lys Asn Glu Ser Leu Lys Ala Cys Ile Arg Cys Asn Ser Pro
    370                 375                 380

Ala Lys Tyr Asp Cys Tyr Leu Gln Arg Ala Thr Cys Lys Arg Glu Gly
385                 390                 395                 400

Cys Gly Phe Asp Tyr Cys Thr Lys Cys Leu Cys Asn Tyr His Thr Thr
                405                 410                 415

Lys Asp Cys Ser Asp Gly Lys Leu Leu Lys Ala Ser Cys Lys Ile Gly
            420                 425                 430

Pro Leu Pro Gly Thr Lys Lys Ser Lys Lys Asn Leu Arg Arg Leu
        435                 440                 445

<210>  328
<211>  132
<212>  PRT
<213>  Homo sapiens

<400>  328

Met Lys Lys Val Arg Leu Lys Glu Leu Glu Ser Arg Leu Gln Gln Val
1               5                   10                  15

Asp Gly Phe Glu Lys Pro Lys Leu Leu Leu Glu Gln Tyr Pro Thr Arg
            20                  25                  30

Pro His Ile Ala Ala Cys Met Leu Tyr Thr Ile His Asn Thr Tyr Asp

        35                  40                  45

Asp Ile Glu Asn Lys Val Val Ala Asp Leu Gly Cys Gly Cys Gly Val
    50                  55                  60

Leu Ser Ile Gly Thr Ala Met Leu Gly Ala Glu Leu Cys Val Gly Phe
65                  70                  75                  80

Asp Ile Asp Glu Asp Ala Leu Glu Ile Phe Asn Arg Asn Ala Glu Glu
                85                  90                  95

Phe Glu Leu Gln Ile Leu Thr Trp Phe Asn Val Leu Cys Ala Tyr Tyr
            100                 105                 110

Leu Thr Glu Cys Pro Ser His Ser Ile Gln Tyr Tyr Glu Ser Ser Leu
        115                 120                 125

Trp Asp Gln Lys
    130

<210> 329
<211> 632
<212> PRT
<213> Homo sapiens

<400> 329

Met Asp Thr Met Met Leu Asn Val Arg Asn Leu Phe Glu Gln Leu Val
1               5                   10                  15

Arg Arg Val Glu Ile Leu Ser Glu Gly Asn Glu Val Gln Phe Ile Gln
            20                  25                  30

Leu Ala Lys Asp Phe Glu Asp Phe Arg Lys Lys Trp Gln Arg Thr Asp
        35                  40                  45

His Glu Leu Gly Lys Tyr Lys Asp Leu Leu Met Lys Ala Glu Thr Glu
    50                  55                  60

Arg Ser Ala Leu Asp Val Lys Leu Lys His Ala Arg Asn Gln Val Asp
65                  70                  75                  80

Val Glu Ile Lys Arg Arg Gln Arg Ala Glu Ala Asp Cys Glu Lys Leu
                85                  90                  95

Glu Arg Gln Ile Gln Leu Ile Arg Glu Met Leu Met Cys Asp Thr Ser
            100                 105                 110

Gly Ser Ile Gln Leu Ser Glu Glu Gln Lys Ser Ala Leu Ala Phe Leu
        115                 120                 125

Asn Arg Gly Gln Pro Ser Ser Ser Asn Ala Gly Asn Lys Arg Leu Ser
    130                 135                 140

Thr Ile Asp Glu Ser Gly Ser Ile Leu Ser Asp Ile Ser Phe Asp Lys
145                 150                 155                 160

Thr Asp Glu Ser Leu Asp Trp Asp Ser Ser Leu Val Lys Thr Phe Lys
                165                 170                 175

Leu Lys Lys Arg Glu Lys Arg Arg Ser Thr Ser Arg Gln Phe Val Asp
            180                 185                 190

Gly Pro Pro Gly Pro Val Lys Lys Thr Arg Ser Ile Gly Ser Ala Val
        195                 200                 205

Asp Gln Gly Asn Glu Ser Ile Val Ala Lys Thr Thr Val Thr Val Pro
    210                 215                 220

Asn Asp Gly Gly Pro Ile Glu Ala Val Ser Thr Ile Glu Thr Val Pro
225                 230                 235                 240

Tyr Trp Thr Arg Ser Arg Arg Lys Thr Gly Thr Leu Gln Pro Trp Asn
                245                 250                 255

Ser Asp Ser Thr Leu Asn Ser Arg Gln Leu Glu Pro Arg Thr Glu Thr
            260                 265                 270

Asp Ser Val Gly Thr Pro Gln Ser Asn Gly Gly Met Arg Leu His Asp
        275                 280                 285

Phe Val Ser Lys Thr Val Ile Lys Pro Glu Ser Cys Val Pro Cys Gly
    290                 295                 300

Lys Arg Ile Lys Phe Gly Lys Leu Ser Leu Lys Cys Arg Asp Cys Arg
305                 310                 315                 320

Val Val Ser His Pro Glu Cys Arg Asp Arg Cys Pro Leu Pro Cys Ile
                325                 330                 335

Pro Thr Leu Ile Gly Thr Pro Val Lys Ile Gly Glu Gly Met Leu Ala
            340                 345                 350

Asp Phe Val Ser Gln Thr Ser Pro Met Ile Pro Ser Ile Val Val His
        355                 360                 365

Cys Val Asn Glu Ile Glu Gln Arg Gly Leu Thr Glu Thr Gly Leu Tyr
    370                 375                 380

Arg Ile Ser Gly Cys Asp Arg Thr Val Lys Glu Leu Lys Glu Lys Phe
385                 390                 395                 400

Leu Arg Val Lys Thr Val Pro Leu Leu Ser Lys Val Asp Asp Ile His
                405                 410                 415

Ala Ile Cys Ser Leu Leu Lys Asp Phe Leu Arg Asn Leu Lys Glu Pro
            420                 425                 430

Leu Leu Thr Phe Arg Leu Asn Arg Ala Phe Met Glu Ala Ala Glu Ile
        435                 440                 445

Thr Asp Glu Asp Asn Ser Ile Ala Ala Met Tyr Gln Ala Val Gly Glu
    450                 455                 460

Leu Pro Gln Ala Asn Arg Asp Thr Leu Ala Phe Leu Met Ile His Leu
465                 470                 475                 480

Gln Arg Val Ala Gln Ser Pro His Thr Lys Met Asp Val Ala Asn Leu
                485                 490                 495

Ala Lys Val Phe Gly Pro Thr Ile Val Ala His Ala Val Pro Asn Pro
            500                 505                 510

Asp Pro Val Thr Met Leu Gln Asp Ile Lys Arg Gln Pro Lys Val Val
        515                 520                 525

Glu Arg Leu Leu Ser Leu Pro Leu Glu Tyr Trp Ser Gln Phe Met Met
    530                 535                 540

Val Glu Gln Glu Asn Ile Asp Pro Leu His Val Ile Glu Asn Ser Asn
545                 550                 555                 560

Ala Phe Ser Thr Pro Gln Thr Pro Asp Ile Lys Val Ser Leu Leu Gly
                565                 570                 575

Pro Val Thr Thr Pro Glu His Gln Leu Leu Lys Thr Pro Ser Ser Ser
            580                 585                 590

Ser Leu Ser Gln Arg Val Arg Ser Thr Leu Thr Lys Asn Thr Pro Arg
        595                 600                 605

Phe Gly Ser Lys Ser Lys Ser Ala Thr Asn Leu Gly Arg Gln Gly Asn
    610                 615                 620

Phe Phe Ala Ser Pro Met Leu Lys
625                 630

<210>  330
<211>  677
<212>  PRT
<213>  Homo sapiens

<400>  330

Met Arg Glu Asp His Ser Phe His Val Arg Tyr Arg Met Glu Ala Ser
1               5                   10                  15

Cys Leu Glu Leu Ala Leu Glu Gly Glu Arg Leu Cys Lys Ser Gly Asp
            20                  25                  30

Cys Arg Ala Gly Val Ser Phe Phe Glu Ala Ala Val Gln Val Gly Thr

35                  40                  45

Glu Asp Leu Lys Thr Leu Ser Ala Ile Tyr Ser Gln Leu Gly Asn Ala
    50                  55                  60

Tyr Phe Tyr Leu His Asp Tyr Ala Lys Ala Leu Glu Tyr His His His
65                  70                  75                  80

Asp Leu Thr Leu Ala Arg Thr Ile Gly Asp Gln Leu Gly Glu Ala Lys
                85                  90                  95

Ala Ser Gly Asn Leu Gly Asn Thr Leu Lys Val Leu Gly Asn Phe Asp
            100                 105                 110

Glu Ala Ile Val Cys Cys Gln Arg His Leu Asp Ile Ser Arg Glu Leu
        115                 120                 125

Asn Asp Lys Val Gly Glu Ala Arg Ala Leu Tyr Asn Leu Gly Asn Val
    130                 135                 140

Tyr His Ala Lys Gly Lys Ser Phe Gly Cys Pro Gly Pro Gln Asp Val
145                 150                 155                 160

Gly Glu Phe Pro Glu Glu Val Arg Asp Ala Leu Gln Ala Ala Val Asp
                165                 170                 175

Phe Tyr Glu Glu Asn Leu Ser Leu Val Thr Ala Leu Gly Asp Arg Ala
            180                 185                 190

Ala Gln Gly Arg Ala Phe Gly Asn Leu Gly Asn Thr His Tyr Leu Leu
        195                 200                 205

Gly Asn Phe Arg Asp Ala Val Ile Ala His Glu Gln Arg Leu Leu Ile
    210                 215                 220

Ala Lys Glu Phe Gly Asp Lys Ala Ala Glu Arg Arg Ala Tyr Ser Asn
225                 230                 235                 240

Leu Gly Asn Ala Tyr Ile Phe Leu Gly Glu Phe Glu Thr Ala Ser Glu
                245                 250                 255

Tyr Tyr Lys Lys Thr Leu Leu Leu Ala Arg Gln Leu Lys Asp Arg Ala
            260                 265                 270

Val Glu Ala Gln Ser Cys Tyr Ser Leu Gly Asn Thr Tyr Thr Leu Leu
        275                 280                 285

Gln Asp Tyr Glu Lys Ala Ile Asp Tyr His Leu Lys His Leu Ala Ile
    290                 295                 300

Ala Gln Glu Leu Asn Asp Arg Ile Gly Glu Gly Arg Ala Cys Trp Ser
305                 310                 315                 320

Leu Gly Asn Ala Tyr Thr Ala Leu Gly Asn His Asp Gln Ala Met His
                325                 330                 335

Phe Ala Glu Lys His Leu Glu Ile Ser Arg Glu Val Gly Asp Lys Ser
            340                 345                 350

Gly Glu Leu Thr Ala Arg Leu Asn Leu Ser Asp Leu Gln Met Val Leu
        355                 360                 365

Gly Leu Ser Tyr Ser Thr Asn Asn Ser Ile Met Ser Glu Asn Thr Glu
    370                 375                 380

Ile Asp Ser Ser Leu Asn Gly Val Arg Pro Lys Leu Gly Arg Arg His
385                 390                 395                 400

Ser Met Glu Asn Met Glu Leu Met Lys Leu Thr Pro Glu Lys Val Gln
                405                 410                 415

Asn Trp Asn Ser Glu Ile Leu Ala Lys Gln Lys Pro Leu Ile Ala Lys
            420                 425                 430

Pro Ser Ala Lys Leu Leu Phe Val Asn Arg Leu Lys Gly Lys Lys Tyr
        435                 440                 445

Lys Thr Asn Ser Ser Thr Lys Val Leu Gln Asp Ala Ser Asn Ser Ile
    450                 455                 460

Asp His Arg Ile Pro Asn Ser Gln Arg Lys Ile Ser Ala Asp Thr Ile
465                 470                 475                 480

Gly Asp Glu Gly Phe Phe Asp Leu Leu Ser Arg Phe Gln Ser Asn Arg
                485                 490                 495

Met Asp Asp Gln Arg Cys Cys Leu Gln Glu Lys Asn Cys His Thr Ala
            500                 505                 510

Ser Thr Thr Thr Ser Ser Thr Pro Pro Lys Met Met Leu Lys Thr Ser
        515                 520                 525

Ser Val Pro Val Val Ser Pro Asn Thr Asp Glu Phe Leu Asp Leu Leu
    530                 535                 540

Ala Ser Ser Gln Ser Arg Arg Leu Asp Asp Gln Arg Ala Ser Phe Ser
545                 550                 555                 560

Asn Leu Pro Gly Leu Arg Leu Thr Gln Asn Ser Gln Ser Val Leu Ser
                565                 570                 575

His Leu Met Thr Asn Asp Asn Lys Glu Ala Asp Glu Asp Phe Phe Asp
            580                 585                 590

Ile Leu Val Lys Cys Gln Gly Ser Arg Leu Asp Asp Gln Arg Cys Ala

595                 600                 605

Pro Pro Pro Ala Thr Thr Lys Gly Pro Thr Val Pro Asp Glu Asp Phe
    610                 615                 620

Phe Ser Leu Ile Leu Arg Ser Gln Gly Lys Arg Met Asp Glu Gln Arg
625                 630                 635                 640

Val Leu Leu Gln Arg Asp Gln Asn Arg Asp Thr Asp Phe Gly Leu Lys
                645                 650                 655

Asp Phe Leu Gln Asn Asn Ala Leu Leu Glu Phe Lys Asn Ser Gly Lys
            660                 665                 670

Lys Ser Ala Asp His
        675

<210>  331
<211>  566
<212>  PRT
<213>  Homo sapiens

<400>  331

<210>  332
<211>  263
<212>  PRT
<213>  Homo sapiens

<400>  332

Met Ala Leu Ser Val Pro Gly Tyr Ser Pro Gly Phe Arg Lys Pro Pro
1               5                   10                  15

Glu Val Val Arg Leu Arg Arg Lys Arg Ala Arg Ser Arg Gly Ala Ala
            20                  25                  30

Ala Ser Pro Pro Arg Glu Leu Thr Glu Pro Ala Ala Arg Arg Ala Ala
        35                  40                  45

Leu Val Ala Gly Leu Pro Leu Arg Pro Phe Pro Ala Ala Gly Gly Arg
    50                  55                  60

Gly Gly Gly Ser Gly Gly Gly Pro Ala Ala Ala Arg Arg Asn Pro Phe
65                  70                  75                  80

Ala Arg Leu Asp Asn Arg Pro Arg Val Ala Ala Glu Pro Pro Asp Gly
                85                  90                  95

Pro Ala Arg Glu Gln Pro Glu Ala Pro Val Pro Phe Leu Asp Ser Asn
            100                 105                 110

Gln Glu Asn Asp Leu Leu Trp Glu Glu Lys Phe Pro Glu Arg Thr Thr

115                 120                 125

Val Thr Glu Leu Pro Gln Thr Ser His Val Ser Phe Ser Glu Pro Asp
    130                 135                 140

Ile Pro Ser Ser Lys Ser Thr Glu Leu Pro Val Asp Trp Ser Ile Lys
145                 150                 155                 160

Thr Arg Leu Leu Phe Thr Ser Ser Gln Pro Phe Thr Trp Ala Asp His
                165                 170                 175

Leu Lys Ala Gln Glu Glu Ala Gln Gly Leu Val Gln His Cys Arg Ala
            180                 185                 190

Thr Glu Val Thr Leu Pro Lys Ser Ile Gln Asp Pro Lys Leu Ser Ser
        195                 200                 205

Glu Leu Arg Cys Thr Phe Gln Gln Ser Leu Ile Tyr Trp Leu His Pro
    210                 215                 220

Ala Leu Ser Trp Leu Pro Leu Phe Pro Arg Ile Gly Ala Asp Arg Lys
225                 230                 235                 240

Met Ala Gly Lys Thr Ser Pro Trp Ser Asn Asp Ala Thr Leu Gln His
                245                 250                 255

Val Leu Met Ser Asp Trp Tyr
            260

<210>  333
<211>  265
<212>  PRT
<213>  Homo sapiens

<400>  333

Met Ala Leu Ser Val Pro Gly Tyr Ser Pro Gly Phe Arg Lys Pro Pro
1               5                   10                  15

Glu Val Val Arg Leu Arg Arg Lys Arg Ala Arg Ser Arg Gly Ala Ala
            20                  25                  30

Ala Ser Pro Pro Arg Glu Leu Thr Glu Pro Ala Ala Arg Arg Ala Ala
        35                  40                  45

Leu Val Ala Gly Leu Pro Leu Arg Pro Phe Pro Ala Ala Gly Gly Arg
    50                  55                  60

Gly Gly Gly Ser Gly Gly Gly Pro Ala Ala Ala Arg Arg Asn Pro Phe
65                  70                  75                  80

Ala Arg Leu Asp Asn Arg Pro Arg Val Ala Ala Glu Pro Pro Asp Gly
                85                  90                  95

Pro Ala Arg Glu Gln Pro Glu Ala Pro Val Pro Phe Leu Asp Ser Asn
            100                 105                 110

Gln Glu Asn Asp Leu Leu Trp Glu Glu Lys Phe Pro Glu Arg Thr Thr
        115                 120                 125

Val Thr Glu Leu Pro Gln Thr Ser His Val Ser Phe Ser Glu Pro Asp
    130                 135                 140

Ile Pro Ser Ser Lys Ser Thr Glu Leu Pro Val Asp Trp Ser Ile Lys
145                 150                 155                 160

Thr Arg Leu Leu Phe Thr Ser Ser Gln Pro Phe Thr Trp Ala Asp His
                165                 170                 175

Leu Lys Ala Gln Glu Glu Ala Gln Gly Leu Val Gln His Cys Arg Ala
            180                 185                 190

Thr Glu Val Thr Leu Pro Lys Ser Ile Gln Asp Pro Lys Leu Ser Ser
        195                 200                 205

Glu Leu Arg Cys Thr Phe Gln Gln Ser Leu Ile Tyr Trp Leu His Pro
    210                 215                 220

Ala Leu Ser Trp Leu Pro Leu Phe Pro Arg Ile Gly Ala Asp Arg Lys
225                 230                 235                 240

Met Ala Gly Lys Thr Ser Pro Trp Ser Asn Asp Ala Thr Leu Gln His
                245                 250                 255

Val Leu Met Ser Asp Trp His Gly Val
            260                 265

<210>  334
<211>  209
<212>  PRT
<213>  Homo sapiens

<400>  334

<210>  335
<211>  84
<212>  PRT
<213>  Homo sapiens

<400>  335

Met Pro Leu Ala Arg Asp Leu Leu His Pro Ser Leu Glu Glu Glu Lys
1               5                   10                  15

Lys Lys His Lys Lys Lys Arg Leu Val Gln Ser Pro Asn Ser Tyr Phe
            20                  25                  30

Met Asp Val Lys Cys Pro Gly Cys Tyr Lys Ile Thr Thr Val Phe Ser
        35                  40                  45

His Ala Gln Thr Val Val Leu Cys Val Gly Cys Ser Thr Val Leu Cys
    50                  55                  60

Gln Pro Thr Gly Gly Lys Ala Arg Leu Thr Glu Gly Cys Ser Phe Arg
65                  70                  75                  80

Arg Lys Gln His

<210>  336
<211>  226

<212>  PRT
<213>  Homo sapiens

<400>  336

Met Asn Glu Leu Lys Gln Gln Pro Ile Asn Lys Gly Gly Val Arg Thr
1               5                   10                  15

Pro Val Pro Pro Arg Gly Arg Leu Ser Val Ala Ser Thr Pro Ile Ser
            20                  25                  30

Gln Arg Arg Ser Gln Gly Arg Ser Cys Gly Pro Ala Ser Gln Ser Thr
        35                  40                  45

Leu Gly Leu Lys Gly Ser Leu Lys Arg Ser Ala Ile Ser Ala Ala Lys
    50                  55                  60

Thr Gly Val Arg Phe Ser Ala Ala Thr Lys Asp Asn Glu His Lys Arg
65                  70                  75                  80

Ser Leu Thr Lys Thr Pro Ala Arg Lys Ser Ala His Val Thr Val Ser
                85                  90                  95

Gly Gly Thr Pro Lys Gly Glu Ala Val Leu Gly Thr His Lys Leu Lys
            100                 105                 110

Thr Ile Thr Gly Asn Ser Ala Ala Val Ile Thr Pro Phe Lys Leu Thr
        115                 120                 125

Thr Glu Ala Thr Gln Thr Pro Val Ser Asn Lys Lys Pro Val Phe Asp
    130                 135                 140

Leu Lys Ala Ser Leu Ser Arg Pro Leu Asn Tyr Glu Pro His Lys Gly
145                 150                 155                 160

Lys Leu Lys Pro Trp Gly Gln Ser Lys Glu Asn Asn Tyr Leu Asn Gln
                165                 170                 175

His Val Asn Arg Ile Asn Phe Tyr Lys Lys Thr Tyr Lys Gln Pro His
            180                 185                 190

Leu Gln Thr Lys Glu Glu Gln Arg Lys Lys Arg Glu Gln Glu Arg Lys
        195                 200                 205

Glu Lys Lys Ala Lys Val Leu Gly Met Arg Arg Gly Leu Ile Leu Ala
    210                 215                 220

Glu Asp
225

<210>  337
<211>  440
<212>  PRT
<213>  Homo sapiens

<400> 337

Met Ile Ile Pro Ser Leu Glu Glu Leu Asp Ser Leu Lys Tyr Ser Asp
1               5                   10                  15

Leu Gln Asn Leu Ala Lys Ser Leu Gly Leu Arg Ala Asn Leu Arg Ala
            20                  25                  30

Thr Lys Leu Leu Lys Ala Leu Lys Gly Tyr Ile Lys His Glu Ala Arg
        35                  40                  45

Lys Gly Asn Glu Asn Gln Asp Glu Ser Gln Thr Ser Ala Ser Ser Cys
    50                  55                  60

Asp Glu Thr Glu Ile Gln Ile Ser Asn Gln Glu Glu Ala Glu Arg Gln
65                  70                  75                  80

Pro Leu Gly His Val Thr Lys Thr Arg Arg Arg Cys Lys Thr Val Arg
                85                  90                  95

Val Asp Pro Asp Ser Gln Gln Asn His Ser Glu Ile Lys Ile Ser Asn
            100                 105                 110

Pro Thr Glu Phe Gln Asn His Glu Lys Gln Glu Ser Gln Asp Leu Arg
        115                 120                 125

Ala Thr Ala Lys Val Pro Ser Pro Pro Asp Glu His Gln Glu Ala Glu
    130                 135                 140

Asn Ala Val Ser Ser Gly Asn Arg Asp Ser Lys Val Pro Ser Glu Gly
145                 150                 155                 160

Lys Lys Ser Leu Tyr Thr Asp Glu Ser Ser Lys Pro Gly Lys Asn Lys
                165                 170                 175

Arg Thr Ala Ile Thr Thr Pro Asn Phe Lys Lys Leu His Glu Ala His
            180                 185                 190

Phe Lys Glu Met Glu Ser Ile Asp Gln Tyr Ile Glu Arg Lys Lys Lys
        195                 200                 205

His Phe Glu Glu His Asn Ser Met Asn Glu Leu Lys Gln Pro Ile Asn
    210                 215                 220

Lys Gly Gly Val Arg Thr Pro Val Pro Pro Arg Gly Arg Leu Ser Val
225                 230                 235                 240

Ala Ser Thr Pro Ile Ser Gln Arg Arg Ser Gln Gly Arg Ser Cys Gly
                245                 250                 255

Pro Ala Ser Gln Ser Thr Leu Gly Leu Lys Gly Ser Leu Lys Arg Ser
            260                 265                 270

Ala Ile Ser Ala Ala Lys Thr Gly Val Arg Phe Ser Ala Ala Thr Lys
        275                 280                 285

Asp Asn Glu His Lys Arg Ser Leu Thr Lys Thr Pro Ala Arg Lys Ser
    290                 295                 300

Ala His Val Thr Val Ser Gly Gly Thr Pro Lys Gly Glu Ala Val Leu
305                 310                 315                 320

Gly Thr His Lys Leu Lys Thr Ile Thr Gly Asn Ser Ala Ala Val Ile
                325                 330                 335

Thr Pro Phe Lys Leu Thr Thr Glu Ala Thr Gln Thr Pro Val Ser Asn
            340                 345                 350

Lys Lys Pro Val Phe Asp Leu Lys Ala Ser Leu Ser Arg Pro Leu Asn
        355                 360                 365

Tyr Glu Pro His Lys Gly Lys Leu Lys Pro Trp Gly Gln Ser Lys Glu
    370                 375                 380

Asn Asn Tyr Leu Asn Gln His Val Asn Arg Ile Asn Phe Tyr Lys Lys
385                 390                 395                 400

Thr Tyr Lys Gln Pro His Leu Gln Thr Lys Glu Glu Gln Arg Lys Lys
                405                 410                 415

Arg Glu Gln Glu Arg Lys Glu Lys Lys Ala Lys Val Leu Gly Met Arg
            420                 425                 430

Arg Gly Leu Ile Leu Ala Glu Asp
        435                 440

<210>  338
<211>  730
<212>  PRT
<213>  Homo sapiens

<400>  338

Met Leu Phe Asn Ser Val Leu Arg Gln Pro Gln Leu Gly Val Leu Arg
1               5                   10                  15

Asn Gly Trp Ser Ser Gln Tyr Pro Leu Gln Ser Leu Leu Thr Gly Tyr
            20                  25                  30

Gln Cys Ser Gly Asn Asp Glu His Thr Ser Tyr Gly Glu Thr Gly Val
        35                  40                  45

Pro Val Pro Pro Phe Gly Cys Thr Phe Ser Ser Ala Pro Asn Met Glu
    50                  55                  60

His Val Leu Ala Val Ala Asn Glu Glu Gly Phe Val Arg Leu Tyr Asn
65                  70                  75                  80

Thr Glu Ser Gln Ser Phe Arg Lys Lys Cys Phe Lys Glu Trp Met Ala
                85                  90                  95

His Trp Asn Ala Val Phe Asp Leu Ala Trp Val Pro Gly Glu Leu Lys
            100                 105                 110

Leu Val Thr Ala Ala Gly Asp Gln Thr Ala Lys Phe Trp Asp Val Lys
        115                 120                 125

Ala Gly Glu Leu Ile Gly Thr Cys Lys Gly His Gln Cys Ser Leu Lys
    130                 135                 140

Ser Val Ala Phe Ser Lys Phe Glu Lys Ala Val Phe Cys Thr Gly Gly
145                 150                 155                 160

Arg Asp Gly Asn Ile Met Val Trp Asp Thr Arg Cys Asn Lys Lys Asp
                165                 170                 175

Gly Phe Tyr Arg Gln Val Asn Gln Ile Ser Gly Ala His Asn Thr Ser
            180                 185                 190

Asp Lys Gln Thr Pro Ser Lys Pro Lys Lys Lys Gln Asn Ser Lys Gly
        195                 200                 205

Leu Ala Pro Ser Val Asp Phe Gln Gln Ser Val Thr Val Val Leu Phe
    210                 215                 220

Gln Asp Glu Asn Thr Leu Val Ser Ala Gly Ala Val Asp Gly Ile Ile
225                 230                 235                 240

Lys Val Trp Asp Leu Arg Lys Asn Tyr Thr Ala Tyr Arg Gln Glu Pro
                245                 250                 255

Ile Ala Ser Lys Ser Phe Leu Tyr Pro Gly Ser Ser Thr Arg Lys Leu
            260                 265                 270

Gly Tyr Ser Ser Leu Ile Leu Asp Ser Thr Gly Ser Thr Leu Phe Ala
        275                 280                 285

Asn Cys Thr Asp Asp Asn Ile Tyr Met Phe Asn Met Thr Gly Leu Lys
    290                 295                 300

Thr Ser Pro Val Ala Ile Phe Asn Gly His Gln Asn Ser Thr Phe Tyr
305                 310                 315                 320

Val Lys Ser Ser Leu Ser Pro Asp Asp Gln Phe Leu Val Ser Gly Ser
                325                 330                 335

Ser Asp Glu Ala Ala Tyr Ile Trp Lys Val Ser Thr Pro Trp Gln Pro
            340                 345                 350

Pro Thr Val Leu Leu Gly His Ser Gln Glu Val Thr Ser Val Cys Trp

355                 360                 365

Cys Pro Ser Asp Phe Thr Lys Ile Ala Thr Cys Ser Asp Asp Asn Thr
    370                 375                 380

Leu Lys Ile Trp Arg Leu Asn Arg Gly Leu Glu Glu Lys Pro Gly Gly
385                 390                 395                 400

Asp Lys Leu Ser Thr Val Gly Trp Ala Ser Gln Lys Lys Lys Glu Ser
                405                 410                 415

Arg Pro Gly Leu Val Thr Val Thr Ser Ser Gln Ser Thr Pro Ala Lys
            420                 425                 430

Ala Pro Arg Val Lys Cys Asn Pro Ser Asn Ser Ser Pro Ser Ser Ala
        435                 440                 445

Ala Cys Ala Pro Ser Cys Ala Gly Asp Leu Pro Leu Pro Ser Asn Thr
    450                 455                 460

Pro Thr Phe Ser Ile Lys Thr Ser Pro Ala Lys Ala Arg Ser Pro Ile
465                 470                 475                 480

Asn Arg Arg Gly Ser Val Ser Ser Val Ser Pro Lys Pro Pro Ser Ser
                485                 490                 495

Phe Lys Met Ser Ile Arg Asn Trp Val Thr Arg Thr Pro Ser Ser Ser
            500                 505                 510

Pro Pro Ile Thr Pro Pro Ala Ser Glu Thr Lys Ile Met Ser Pro Arg
        515                 520                 525

Lys Ala Leu Ile Pro Val Ser Gln Lys Ser Ser Gln Ala Glu Ala Cys
    530                 535                 540

Ser Glu Ser Arg Asn Arg Val Lys Arg Arg Leu Asp Ser Ser Cys Leu
545                 550                 555                 560

Glu Ser Val Lys Gln Lys Cys Val Lys Ser Cys Asn Cys Val Thr Glu
                565                 570                 575

Leu Asp Gly Gln Val Glu Asn Leu His Leu Asp Leu Cys Cys Leu Ala
            580                 585                 590

Gly Asn Gln Glu Asp Leu Ser Lys Asp Ser Leu Gly Pro Thr Lys Ser
        595                 600                 605

Ser Lys Ile Glu Gly Ala Gly Thr Ser Ile Ser Glu Pro Pro Ser Pro
    610                 615                 620

Ile Ser Pro Tyr Ala Ser Glu Ser Cys Gly Thr Leu Pro Leu Pro Leu
625                 630                 635                 640

Arg Pro Cys Gly Glu Gly Ser Glu Met Val Gly Lys Glu Asn Ser Ser
                645                 650                 655

Pro Glu Asn Lys Asn Trp Leu Leu Ala Met Ala Ala Lys Arg Lys Ala
            660                 665                 670

Glu Asn Pro Ser Pro Arg Ser Pro Ser Ser Gln Thr Pro Asn Ser Arg
        675                 680                 685

Arg Gln Ser Gly Lys Thr Leu Pro Ser Pro Val Thr Ile Thr Pro Ser
    690                 695                 700

Ser Met Arg Lys Ile Cys Thr Tyr Phe His Arg Lys Ser Gln Glu Asp
705                 710                 715                 720

Phe Cys Gly Pro Glu His Ser Thr Glu Leu
                725                 730

<210>  339
<211>  238
<212>  PRT
<213>  Homo sapiens

<400>  339

Met Ala Ser Arg Trp Gln Asn Met Gly Thr Ser Val Arg Arg Arg Ser
1               5                   10                  15

Leu Gln His Gln Glu Gln Leu Glu Asp Ser Lys Glu Leu Gln Pro Val
            20                  25                  30

Val Ser His Gln Glu Thr Ser Val Gly Ala Leu Gly Ser Leu Cys Arg
        35                  40                  45

Gln Phe Gln Arg Arg Leu Pro Leu Arg Thr Val Asn Leu Asn Leu Arg
    50                  55                  60

Ala Gly Pro Ser Trp Lys Arg Leu Glu Thr Pro Glu Pro Gly Gln Gln
65                  70                  75                  80

Gly Leu Gln Ala Ala Ala Arg Ser Ala Lys Ser Ala Leu Gly Ala Val
                85                  90                  95

Ser Gln Arg Ile Gln Glu Ser Cys Gln Ser Gly Thr Lys Trp Leu Val
            100                 105                 110

Glu Thr Gln Val Lys Ala Arg Arg Arg Lys Arg Gly Ala Gln Lys Gly
        115                 120                 125

Ser Gly Ser Pro Thr His Ser Leu Ser Gln Lys Ser Thr Arg Leu Ser
    130                 135                 140

Gly Ala Ala Pro Ala His Ser Ala Ala Asp Pro Trp Glu Lys Glu His
145                 150                 155                 160

His Arg Leu Ser Val Arg Met Gly Ser His Ala His Pro Leu Arg Arg
                165                 170                 175

Ser Arg Arg Glu Ala Ala Phe Arg Ser Pro Tyr Ser Ser Thr Glu Pro
            180                 185                 190

Leu Cys Ser Pro Ser Glu Ser Asp Ser Asp Leu Glu Pro Val Gly Ala
        195                 200                 205

Gly Ile Gln His Leu Gln Lys Leu Ser Gln Glu Leu Asp Glu Ala Ile
    210                 215                 220

Met Ala Glu Glu Ser Gly Asp Ile Val Ser Leu Ile His Asp
225                 230                 235

<210>  340
<211>  280
<212>  PRT
<213>  Homo sapiens

<400>  340

Met Ala Pro Arg Lys Gly Ser Ser Arg Val Ala Lys Thr Asn Ser Leu
1               5                   10                  15

Arg Arg Arg Lys Leu Ala Ser Phe Leu Lys Asp Phe Asp Arg Glu Val
            20                  25                  30

Glu Ile Arg Ile Lys Gln Ile Glu Ser Asp Arg Gln Asn Leu Leu Lys
        35                  40                  45

Glu Val Asp Asn Leu Tyr Asn Ile Glu Ile Leu Arg Leu Pro Lys Ala
    50                  55                  60

Leu Arg Glu Met Asn Trp Leu Asp Tyr Phe Ala Leu Gly Gly Asn Lys
65                  70                  75                  80

Gln Ala Leu Glu Glu Ala Ala Thr Ala Asp Leu Asp Ile Thr Glu Ile
                85                  90                  95

Asn Lys Leu Thr Ala Glu Ala Ile Gln Thr Pro Leu Lys Ser Ala Lys
            100                 105                 110

Thr Arg Lys Val Ile Gln Val Asp Glu Met Ile Val Glu Glu Glu Glu
        115                 120                 125

Glu Glu Glu Asn Glu Arg Lys Asn Leu Gln Thr Ala Arg Val Lys Arg
    130                 135                 140

Cys Pro Pro Ser Lys Lys Arg Thr Gln Ser Ile Gln Gly Lys Gly Lys
145                 150                 155                 160

Gly Lys Arg Ser Ser Arg Ala Asn Thr Val Thr Pro Ala Val Gly Arg

165                 170                 175

Leu Glu Val Ser Met Val Lys Pro Thr Pro Gly Leu Thr Pro Arg Phe
            180                 185                 190

Asp Ser Arg Val Phe Lys Thr Pro Gly Leu Arg Thr Pro Ala Ala Gly
        195                 200                 205

Glu Arg Ile Tyr Asn Ile Ser Gly Asn Gly Ser Pro Leu Ala Asp Ser
    210                 215                 220

Lys Glu Ile Phe Leu Thr Val Pro Val Gly Gly Gly Glu Ser Leu Arg
225                 230                 235                 240

Leu Leu Ala Ser Asp Leu Gln Arg His Ser Ile Ala Gln Leu Asp Pro
                245                 250                 255

Glu Ala Leu Gly Asn Ile Lys Lys Leu Ser Asn Arg Leu Ala Gln Ile
            260                 265                 270

Cys Ser Ser Ile Arg Thr His Lys
        275                 280

<210>  341
<211>  464
<212>  PRT
<213>  Homo sapiens

<400>  341

Met Ser Ser Arg Ser Thr Lys Asp Leu Ile Lys Ser Lys Trp Gly Ser
1               5                   10                  15

Lys Pro Ser Asn Ser Lys Ser Glu Thr Thr Leu Glu Lys Leu Lys Gly
            20                  25                  30

Glu Ile Ala His Leu Lys Thr Ser Val Asp Glu Ile Thr Ser Gly Lys
        35                  40                  45

Gly Lys Leu Thr Asp Lys Glu Arg His Arg Leu Leu Glu Lys Ile Arg
    50                  55                  60

Val Leu Glu Ala Glu Lys Glu Lys Asn Ala Tyr Gln Leu Thr Glu Lys
65                  70                  75                  80

Asp Lys Glu Ile Gln Arg Leu Arg Asp Gln Leu Lys Ala Arg Tyr Ser
                85                  90                  95

Thr Thr Ala Leu Leu Glu Gln Leu Glu Glu Thr Thr Arg Glu Gly Glu
            100                 105                 110

Arg Arg Glu Gln Val Leu Lys Ala Leu Ser Glu Glu Lys Asp Val Leu
        115                 120                 125

Lys Gln Gln Leu Ser Ala Ala Thr Ser Arg Ile Ala Glu Leu Glu Ser
    130                 135                 140

Lys Thr Asn Thr Leu Arg Leu Ser Gln Thr Val Ala Pro Asn Cys Phe
145                 150                 155                 160

Asn Ser Ser Ile Asn Asn Ile His Glu Met Glu Ile Gln Leu Lys Asp
                165                 170                 175

Ala Leu Glu Lys Asn Gln Gln Trp Leu Val Tyr Asp Gln Gln Arg Glu
            180                 185                 190

Val Tyr Val Lys Gly Leu Leu Ala Lys Ile Phe Glu Leu Glu Lys Lys
        195                 200                 205

Thr Glu Thr Ala Ala His Ser Leu Pro Gln Gln Thr Lys Lys Pro Glu
    210                 215                 220

Ser Glu Gly Tyr Leu Gln Glu Glu Lys Gln Lys Cys Tyr Asn Asp Leu
225                 230                 235                 240

Leu Ala Ser Ala Lys Lys Asp Leu Glu Val Glu Arg Gln Thr Ile Thr
                245                 250                 255

Gln Leu Ser Phe Glu Leu Ser Glu Phe Arg Arg Lys Tyr Glu Glu Thr
            260                 265                 270

Gln Lys Glu Val His Asn Leu Asn Gln Leu Leu Tyr Ser Gln Arg Arg
        275                 280                 285

Ala Asp Val Gln His Leu Glu Asp Asp Arg His Lys Thr Glu Lys Ile
    290                 295                 300

Gln Lys Leu Arg Glu Glu Asn Asp Ile Ala Arg Gly Lys Leu Glu Glu
305                 310                 315                 320

Glu Lys Lys Arg Ser Glu Glu Leu Leu Ser Gln Val Gln Phe Leu Tyr
                325                 330                 335

Thr Ser Leu Leu Lys Gln Gln Glu Glu Gln Thr Arg Val Ala Leu Leu
            340                 345                 350

Glu Gln Gln Met Gln Ala Cys Thr Leu Asp Phe Glu Asn Glu Lys Leu
        355                 360                 365

Asp Arg Gln His Val Gln His Gln Leu Leu Val Ile Leu Lys Glu Leu
    370                 375                 380

Arg Lys Ala Arg Asn Gln Ile Thr Gln Leu Glu Ser Leu Lys Gln Leu
385                 390                 395                 400

His Glu Phe Ala Ile Thr Glu Pro Leu Val Thr Phe Gln Gly Glu Thr
                405                 410                 415

Glu Asn Arg Glu Lys Val Ala Ala Ser Pro Lys Ser Pro Thr Ala Ala
            420                 425                 430

Leu Asn Glu Ser Leu Val Glu Cys Pro Lys Cys Asn Ile Gln Tyr Pro
        435                 440                 445

Ala Thr Glu His Arg Asp Leu Leu Val His Val Glu Tyr Cys Ser Lys
    450                 455                 460

<210>  342
<211>  1268
<212>  PRT
<213>  Homo sapiens

<400>  342

<210> 343
<211> 748
<212> PRT
<213> Homo sapiens

<400> 343

Met Leu Gly Thr Leu Arg Ala Met Glu Gly Glu Asp Val Glu Asp Asp
1               5                   10                  15

Gln Leu Leu Gln Lys Leu Arg Ala Ser Arg Arg Arg Phe Gln Arg Arg
            20                  25                  30

Met Gln Arg Leu Ile Glu Lys Tyr Asn Gln Pro Phe Glu Asp Thr Pro
        35                  40                  45

Val Val Gln Met Ala Thr Leu Thr Tyr Glu Thr Pro Gln Gly Leu Arg
    50                  55                  60

Ile Trp Gly Gly Arg Leu Ile Lys Glu Arg Asn Glu Gly Glu Ile Gln
65                  70                  75                  80

Asp Ser Ser Met Lys Pro Ala Asp Arg Thr Asp Gly Ser Val Gln Ala
                85                  90                  95

Ala Ala Trp Gly Pro Glu Leu Pro Ser His Arg Thr Val Leu Gly Ala
            100                 105                 110

Asp Ser Lys Ser Gly Glu Val Asp Ala Thr Ser Asp Gln Glu Glu Ser
        115                 120                 125

Val Ala Trp Ala Leu Ala Pro Ala Val Pro Gln Ser Pro Leu Lys Asn
    130                 135                 140

Glu Leu Arg Arg Lys Tyr Leu Thr Gln Val Asp Ile Leu Leu Gln Gly
145                 150                 155                 160

Ala Glu Tyr Phe Glu Cys Ala Gly Asn Arg Ala Gly Arg Asp Val Arg
                165                 170                 175

Val Thr Pro Leu Pro Ser Leu Ala Ser Pro Ala Val Pro Ala Pro Gly
            180                 185                 190

Tyr Cys Ser Arg Ile Ser Arg Lys Ser Pro Gly Asp Pro Ala Lys Pro
        195                 200                 205

Ala Ser Ser Pro Arg Glu Trp Asp Pro Leu His Pro Ser Ser Thr Asp
    210                 215                 220

Met Ala Leu Val Pro Arg Asn Asp Ser Leu Ser Leu Gln Glu Thr Ser
225                 230                 235                 240

Ser Ser Ser Phe Leu Ser Ser Gln Pro Phe Glu Asp Asp Asp Ile Cys
                245                 250                 255

Asn Val Thr Ile Ser Asp Leu Tyr Ala Gly Met Leu His Ser Met Ser
            260                 265                 270

Arg Leu Leu Ser Thr Lys Pro Ser Ser Ile Ile Ser Thr Lys Thr Phe
        275                 280                 285

Ile Met Gln Asn Trp Asn Ser Arg Arg Arg His Arg Tyr Lys Ser Arg
    290                 295                 300

Met Asn Lys Thr Tyr Cys Lys Gly Ala Arg Arg Ser Gln Arg Ser Ser
305                 310                 315                 320

Lys Glu Asn Phe Ile Pro Cys Ser Glu Pro Val Lys Gly Thr Gly Ala
                325                 330                 335

Leu Arg Asp Cys Lys Asn Val Leu Asp Val Ser Cys Arg Lys Thr Gly
            340                 345                 350

Leu Lys Leu Glu Lys Ala Phe Leu Glu Val Asn Arg Pro Gln Ile His
        355                 360                 365

Lys Leu Asp Pro Ser Trp Lys Glu Arg Lys Val Thr Pro Ser Lys Tyr
    370                 375                 380

Ser Ser Leu Ile Tyr Phe Asp Ser Ser Ala Thr Tyr Asn Leu Asp Glu
385                 390                 395                 400

Glu Asn Arg Phe Arg Thr Leu Lys Trp Leu Ile Ser Pro Val Lys Ile
                405                 410                 415

Val Ser Arg Pro Thr Ile Arg Gln Gly His Gly Glu Asn Arg Gln Arg
            420                 425                 430

Glu Ile Glu Ile Arg Phe Asp Gln Leu His Arg Glu Tyr Cys Leu Ser
        435                 440                 445

Pro Arg Asn Gln Pro Arg Arg Met Cys Leu Pro Asp Ser Trp Ala Met
    450                 455                 460

Asn Met Tyr Arg Gly Gly Pro Ala Ser Pro Gly Gly Leu Gln Gly Leu
465                 470                 475                 480

Glu Thr Arg Arg Leu Ser Leu Pro Ser Ser Lys Ala Lys Ala Lys Ser
                485                 490                 495

Leu Ser Glu Ala Phe Glu Asn Leu Gly Lys Arg Ser Leu Glu Ala Gly
            500                 505                 510

Arg Cys Leu Pro Lys Ser Asp Ser Ser Ser Ser Leu Pro Lys Thr Asn
        515                 520                 525

Pro Thr His Ser Ala Thr Arg Pro Gln Gln Thr Ser Asp Leu His Val
    530                 535                 540

Gln Gly Asn Ser Ser Gly Ile Phe Arg Lys Ser Val Ser Pro Ser Lys
545                 550                 555                 560

Thr Leu Ser Val Pro Asp Lys Glu Val Pro Gly His Gly Arg Asn Arg
                565                 570                 575

Tyr Asp Glu Ile Lys Glu Glu Phe Asp Lys Leu His Gln Lys Tyr Cys
            580                 585                 590

Leu Lys Ser Pro Gly Gln Met Thr Val Pro Leu Cys Ile Gly Val Ser
        595                 600                 605

Thr Asp Lys Ala Ser Met Glu Val Arg Tyr Gln Thr Glu Gly Phe Leu
    610                 615                 620

Gly Lys Leu Asn Pro Asp Pro His Phe Gln Gly Phe Gln Lys Leu Pro
625                 630                 635                 640

Ser Ser Pro Leu Gly Cys Arg Lys Ser Leu Leu Gly Ser Thr Ala Ile
                645                 650                 655

Glu Ala Pro Ser Ser Thr Cys Val Ala Arg Ala Ile Thr Arg Asp Gly
            660                 665                 670

Thr Arg Asp His Gln Phe Pro Ala Lys Arg Pro Arg Leu Ser Glu Pro
        675                 680                 685

Gln Gly Ser Gly Arg Gln Gly Asn Ser Leu Gly Ala Ser Asp Gly Val
    690                 695                 700

Asp Asn Thr Val Arg Pro Gly Asp Gln Gly Ser Ser Ser Gln Pro Asn
705                 710                 715                 720

Ser Glu Glu Arg Gly Glu Asn Thr Ser Tyr Arg Met Glu Glu Lys Ser
                725                 730                 735

Asp Phe Met Leu Glu Lys Leu Glu Thr Lys Ser Val
            740                 745

<210>  344
<211>  874
<212>  PRT
<213>  Homo sapiens

<400>  344

Met Asp Glu Glu Glu Asp Asn Leu Ser Leu Leu Thr Ala Leu Leu Glu
1               5                   10                  15

Glu Asn Glu Ser Ala Leu Asp Cys Asn Ser Glu Glu Asn Asn Phe Leu
            20                  25                  30

Thr Arg Glu Asn Gly Glu Pro Asp Ala Phe Asp Glu Leu Phe Asp Ala
        35                  40                  45

Asp Gly Asp Gly Glu Ser Tyr Thr Glu Glu Ala Asp Asp Gly Glu Thr

50                  55                  60

Gly Glu Thr Arg Asp Glu Lys Glu Asn Leu Ala Thr Leu Phe Gly Asp
65                  70                  75                  80

Met Glu Asp Leu Thr Asp Glu Glu Glu Val Pro Ala Ser Gln Ser Thr
                85                  90                  95

Glu Asn Arg Val Leu Pro Ala Pro Ala Pro Arg Arg Glu Lys Thr Asn
            100                 105                 110

Glu Glu Leu Gln Glu Glu Leu Arg Asn Leu Gln Glu Gln Met Lys Ala
        115                 120                 125

Leu Gln Glu Gln Leu Lys Val Thr Thr Ile Lys Gln Thr Ala Ser Pro
    130                 135                 140

Ala Arg Leu Gln Lys Ser Pro Glu Lys Ser Pro Arg Pro Pro Leu Lys
145                 150                 155                 160

Glu Arg Arg Val Gln Arg Ile Gln Glu Ser Thr Cys Phe Ser Ala Glu
                165                 170                 175

Leu Asp Val Pro Ala Leu Pro Arg Thr Lys Arg Val Ala Arg Thr Pro
            180                 185                 190

Lys Ala Ser Pro Pro Asp Pro Lys Ser Ser Ser Ser Arg Met Thr Ser
        195                 200                 205

Ala Pro Ser Gln Pro Leu Gln Thr Ile Ser Arg Asn Lys Pro Ser Gly
    210                 215                 220

Ile Thr Arg Gly Gln Ile Val Gly Thr Pro Gly Ser Ser Gly Glu Thr
225                 230                 235                 240

Thr Gln Pro Ile Cys Val Glu Ala Phe Ser Gly Leu Arg Leu Arg Arg
                245                 250                 255

Pro Arg Val Ser Ser Thr Glu Met Asn Lys Lys Met Thr Gly Arg Lys
            260                 265                 270

Leu Ile Arg Leu Ser Gln Ile Lys Glu Lys Met Ala Arg Glu Lys Leu
        275                 280                 285

Glu Glu Ile Asp Trp Val Thr Phe Gly Val Ile Leu Lys Lys Val Thr
    290                 295                 300

Pro Gln Ser Val Asn Ser Gly Lys Thr Phe Ser Ile Trp Lys Leu Asn
305                 310                 315                 320

Asp Leu Arg Asp Leu Thr Gln Cys Val Ser Leu Phe Leu Phe Gly Glu
                325                 330                 335

Val His Lys Ala Leu Trp Lys Thr Glu Gln Gly Thr Val Val Gly Ile
            340                 345                 350

Leu Asn Ala Asn Pro Met Lys Pro Lys Asp Gly Ser Glu Glu Val Cys
        355                 360                 365

Leu Ser Ile Asp His Pro Gln Lys Val Leu Ile Met Gly Glu Ala Leu
    370                 375                 380

Asp Leu Gly Thr Cys Lys Ala Lys Lys Lys Asn Gly Glu Pro Cys Thr
385                 390                 395                 400

Gln Thr Val Asn Leu Arg Asp Cys Glu Tyr Cys Gln Tyr His Val Gln
                405                 410                 415

Ala Gln Tyr Lys Lys Leu Ser Ala Lys Arg Ala Asp Leu Gln Ser Thr
            420                 425                 430

Phe Ser Gly Gly Arg Ile Pro Lys Lys Phe Ala Arg Arg Gly Thr Ser
        435                 440                 445

Leu Lys Glu Arg Leu Cys Gln Asp Gly Phe Tyr Tyr Gly Gly Val Ser
    450                 455                 460

Ser Ala Ser Tyr Ala Ala Ser Ile Ala Ala Ala Val Ala Pro Lys Lys
465                 470                 475                 480

Lys Ile Gln Thr Thr Leu Ser Asn Leu Val Val Lys Gly Thr Asn Leu
                485                 490                 495

Ile Ile Gln Glu Thr Arg Gln Lys Leu Gly Ile Pro Gln Lys Ser Leu
            500                 505                 510

Ser Cys Ser Glu Glu Phe Lys Glu Leu Met Asp Leu Pro Thr Cys Gly
        515                 520                 525

Ala Arg Asn Leu Lys Gln His Leu Ala Lys Ala Thr Ala Ser Gly Ile
    530                 535                 540

Met Gly Ser Pro Lys Pro Ala Ile Lys Ser Ile Ser Ala Ser Ala Leu
545                 550                 555                 560

Leu Lys Gln Gln Lys Gln Arg Met Leu Glu Met Arg Arg Arg Lys Ser
                565                 570                 575

Glu Glu Ile Gln Lys Arg Phe Leu Gln Ser Ser Ser Glu Val Glu Ser
            580                 585                 590

Pro Ala Val Pro Ser Ser Ser Arg Gln Pro Pro Ala Gln Pro Pro Arg
        595                 600                 605

Thr Gly Ser Glu Phe Pro Arg Leu Glu Gly Ala Pro Ala Thr Met Thr

610                 615                 620

Pro Lys Leu Gly Arg Gly Val Leu Glu Gly Asp Asp Val Leu Phe Tyr
625                 630                 635                 640

Asp Glu Ser Pro Pro Pro Arg Pro Lys Leu Ser Ala Leu Ala Glu Ala
                645                 650                 655

Lys Lys Leu Ala Ala Ile Thr Lys Leu Arg Ala Lys Gly Gln Val Leu
            660                 665                 670

Thr Lys Thr Asn Pro Asn Ser Ile Lys Lys Lys Gln Lys Asp Pro Gln
        675                 680                 685

Asp Ile Leu Glu Val Lys Glu Arg Val Glu Lys Asn Thr Met Phe Ser
    690                 695                 700

Ser Gln Ala Glu Asp Glu Leu Glu Pro Ala Arg Lys Lys Arg Arg Glu
705                 710                 715                 720

Gln Leu Ala Tyr Leu Glu Ser Glu Glu Phe Gln Lys Ile Leu Lys Ala
                725                 730                 735

Lys Ser Lys His Thr Gly Ile Leu Lys Glu Ala Glu Ala Glu Met Gln
            740                 745                 750

Glu Arg Tyr Phe Glu Pro Leu Val Lys Lys Glu Gln Met Glu Glu Lys
        755                 760                 765

Met Arg Asn Ile Arg Glu Val Lys Cys Arg Val Val Thr Cys Lys Thr
    770                 775                 780

Cys Ala Tyr Thr His Phe Lys Leu Leu Glu Thr Cys Val Ser Glu Gln
785                 790                 795                 800

His Glu Tyr His Trp His Asp Gly Val Lys Arg Phe Phe Lys Cys Pro
                805                 810                 815

Cys Gly Asn Arg Ser Ile Ser Leu Asp Arg Leu Pro Asn Lys His Cys
            820                 825                 830

Ser Asn Cys Gly Leu Tyr Lys Trp Glu Arg Asp Gly Met Leu Lys Glu
        835                 840                 845

Lys Thr Gly Pro Lys Ile Gly Gly Glu Thr Leu Leu Pro Arg Gly Glu
    850                 855                 860

Glu His Ala Lys Phe Leu Asn Ser Leu Lys
865                 870

<210>  345
<211>  875
<212>  PRT

<213>  Homo sapiens

<400>  345

Met Asp Glu Glu Glu Asp Asn Leu Ser Leu Leu Thr Ala Leu Leu Glu
1               5                   10                  15

Glu Asn Glu Ser Ala Leu Asp Cys Asn Ser Glu Glu Asn Asn Phe Leu
            20                  25                  30

Thr Arg Glu Asn Gly Glu Pro Asp Ala Phe Asp Glu Leu Phe Asp Ala
        35                  40                  45

Asp Gly Asp Gly Glu Ser Tyr Thr Glu Glu Ala Asp Asp Gly Glu Thr
    50                  55                  60

Gly Glu Thr Arg Asp Glu Lys Glu Asn Leu Ala Thr Leu Phe Gly Asp
65                  70                  75                  80

Met Glu Asp Leu Thr Asp Glu Glu Glu Val Pro Ala Ser Gln Ser Thr
                85                  90                  95

Glu Asn Arg Val Leu Pro Ala Pro Ala Pro Arg Arg Glu Lys Thr Asn
            100                 105                 110

Glu Glu Leu Gln Glu Glu Leu Arg Asn Leu Gln Glu Gln Met Lys Ala
        115                 120                 125

Leu Gln Glu Gln Leu Lys Val Thr Thr Ile Lys Gln Thr Ala Ser Pro
    130                 135                 140

Ala Arg Leu Gln Lys Ser Pro Val Glu Lys Ser Pro Arg Pro Pro Leu
145                 150                 155                 160

Lys Glu Arg Arg Val Gln Arg Ile Gln Glu Ser Thr Cys Phe Ser Ala
                165                 170                 175

Glu Leu Asp Val Pro Ala Leu Pro Arg Thr Lys Arg Val Ala Arg Thr
            180                 185                 190

Pro Lys Ala Ser Pro Pro Asp Pro Lys Ser Ser Ser Ser Arg Met Thr
        195                 200                 205

Ser Ala Pro Ser Gln Pro Leu Gln Thr Ile Ser Arg Asn Lys Pro Ser
    210                 215                 220

Gly Ile Thr Arg Gly Gln Ile Val Gly Thr Pro Gly Ser Ser Gly Glu
225                 230                 235                 240

Thr Thr Gln Pro Ile Cys Val Glu Ala Phe Ser Gly Leu Arg Leu Arg
                245                 250                 255

Arg Pro Arg Val Ser Ser Thr Glu Met Asn Lys Lys Met Thr Gly Arg
            260                 265                 270

Lys Leu Ile Arg Leu Ser Gln Ile Lys Glu Lys Met Ala Arg Glu Lys
        275                 280                 285

Leu Glu Glu Ile Asp Trp Val Thr Phe Gly Val Ile Leu Lys Lys Val
    290                 295                 300

Thr Pro Gln Ser Val Asn Ser Gly Lys Thr Phe Ser Ile Trp Lys Leu
305                 310                 315                 320

Asn Asp Leu Arg Asp Leu Thr Gln Cys Val Ser Leu Phe Leu Phe Gly
                325                 330                 335

Glu Val His Lys Ala Leu Trp Lys Thr Glu Gln Gly Thr Val Val Gly
            340                 345                 350

Ile Leu Asn Ala Asn Pro Met Lys Pro Lys Asp Gly Ser Glu Glu Val
        355                 360                 365

Cys Leu Ser Ile Asp His Pro Gln Lys Val Leu Ile Met Gly Glu Ala
    370                 375                 380

Leu Asp Leu Gly Thr Cys Lys Ala Lys Lys Lys Asn Gly Glu Pro Cys
385                 390                 395                 400

Thr Gln Thr Val Asn Leu Arg Asp Cys Glu Tyr Cys Gln Tyr His Val
                405                 410                 415

Gln Ala Gln Tyr Lys Lys Leu Ser Ala Lys Arg Ala Asp Leu Gln Ser
            420                 425                 430

Thr Phe Ser Gly Gly Arg Ile Pro Lys Lys Phe Ala Arg Arg Gly Thr
        435                 440                 445

Ser Leu Lys Glu Arg Leu Cys Gln Asp Gly Phe Tyr Tyr Gly Gly Val
    450                 455                 460

Ser Ser Ala Ser Tyr Ala Ala Ser Ile Ala Ala Ala Val Ala Pro Lys
465                 470                 475                 480

Lys Lys Ile Gln Thr Thr Leu Ser Asn Leu Val Val Lys Gly Thr Asn
                485                 490                 495

Leu Ile Ile Gln Glu Thr Arg Gln Lys Leu Gly Ile Pro Gln Lys Ser
            500                 505                 510

Leu Ser Cys Ser Glu Glu Phe Lys Glu Leu Met Asp Leu Pro Thr Cys
        515                 520                 525

Gly Ala Arg Asn Leu Lys Gln His Leu Ala Lys Ala Thr Ala Ser Gly
    530                 535                 540

Ile Met Gly Ser Pro Lys Pro Ala Ile Lys Ser Ile Ser Ala Ser Ala
545                 550                 555                 560

Leu Leu Lys Gln Gln Lys Gln Arg Met Leu Glu Met Arg Arg Arg Lys
                565                 570                 575

Ser Glu Glu Ile Gln Lys Arg Phe Leu Gln Ser Ser Ser Glu Val Glu
            580                 585                 590

Ser Pro Ala Val Pro Ser Ser Ser Arg Gln Pro Pro Ala Gln Pro Pro
        595                 600                 605

Arg Thr Gly Ser Glu Phe Pro Arg Leu Glu Gly Ala Pro Ala Thr Met
    610                 615                 620

Thr Pro Lys Leu Gly Arg Gly Val Leu Glu Gly Asp Asp Val Leu Phe
625                 630                 635                 640

Tyr Asp Glu Ser Pro Pro Pro Arg Pro Lys Leu Ser Ala Leu Ala Glu
                645                 650                 655

Ala Lys Lys Leu Ala Ala Ile Thr Lys Leu Arg Ala Lys Gly Gln Val
            660                 665                 670

Leu Thr Lys Thr Asn Pro Asn Ser Ile Lys Lys Lys Gln Lys Asp Pro
        675                 680                 685

Gln Asp Ile Leu Glu Val Lys Glu Arg Val Glu Lys Asn Thr Met Phe
    690                 695                 700

Ser Ser Gln Ala Glu Asp Glu Leu Glu Pro Ala Arg Lys Lys Arg Arg
705                 710                 715                 720

Glu Gln Leu Ala Tyr Leu Glu Ser Glu Glu Phe Gln Lys Ile Leu Lys
                725                 730                 735

Ala Lys Ser Lys His Thr Gly Ile Leu Lys Glu Ala Glu Ala Glu Met
            740                 745                 750

Gln Glu Arg Tyr Phe Glu Pro Leu Val Lys Lys Glu Gln Met Glu Glu
        755                 760                 765

Lys Met Arg Asn Ile Arg Glu Val Lys Cys Arg Val Val Thr Cys Lys
    770                 775                 780

Thr Cys Ala Tyr Thr His Phe Lys Leu Leu Glu Thr Cys Val Ser Glu
785                 790                 795                 800

Gln His Glu Tyr His Trp His Asp Gly Val Lys Arg Phe Phe Lys Cys
                805                 810                 815

Pro Cys Gly Asn Arg Ser Ile Ser Leu Asp Arg Leu Pro Asn Lys His
            820                 825                 830

Cys Ser Asn Cys Gly Leu Tyr Lys Trp Glu Arg Asp Gly Met Leu Lys
        835                 840                 845

Glu Lys Thr Gly Pro Lys Ile Gly Gly Glu Thr Leu Leu Pro Arg Gly
    850                 855                 860

Glu Glu His Ala Lys Phe Leu Asn Ser Leu Lys
865                 870                 875

<210>  346
<211>  202
<212>  PRT
<213>  Homo sapiens

<400>  346

Met Ala Lys Val Ser Val Leu Asn Val Ala Val Leu Glu Asn Pro Ser
1               5                   10                  15

Pro Phe His Ser Pro Phe Arg Phe Glu Ile Ser Phe Glu Cys Ser Glu
            20                  25                  30

Ala Leu Ala Asp Asp Leu Glu Trp Lys Ile Ile Tyr Val Gly Ser Ala
        35                  40                  45

Glu Ser Glu Glu Phe Asp Gln Ile Leu Asp Ser Val Leu Val Gly Pro
    50                  55                  60

Val Pro Ala Gly Arg His Met Phe Val Phe Gln Ala Asp Ala Pro Asn
65                  70                  75                  80

Pro Ser Leu Ile Pro Glu Thr Asp Ala Val Gly Val Thr Val Val Leu
                85                  90                  95

Ile Thr Cys Thr Tyr His Gly Gln Glu Phe Ile Arg Val Gly Tyr Tyr
            100                 105                 110

Val Asn Asn Glu Tyr Leu Asn Pro Glu Leu Arg Glu Asn Pro Pro Met
        115                 120                 125

Lys Pro Asp Phe Ser Gln Leu Gln Arg Asn Ile Leu Ala Ser Asn Pro
    130                 135                 140

Arg Val Thr Arg Phe His Ile Asn Trp Asp Asn Asn Met Asp Arg Leu
145                 150                 155                 160

Glu Ala Ile Glu Thr Gln Asp Pro Ser Leu Gly Cys Gly Leu Pro Leu
                165                 170                 175

Asn Cys Thr Pro Ile Lys Gly Leu Gly Leu Pro Gly Cys Ile Pro Gly
            180                 185                 190

Leu Leu Pro Glu Asn Ser Met Asp Cys Ile

195                 200


<210>  347
<211>  204
<212>  PRT
<213>  Homo sapiens

<400>  347

Met Asp Glu Thr Val Ala Glu Phe Ile Lys Arg Thr Ile Leu Lys Ile
1               5                   10                  15

Pro Met Asn Glu Leu Thr Thr Ile Leu Lys Ala Trp Asp Phe Leu Ser
            20                  25                  30

Glu Asn Gln Leu Gln Thr Val Asn Phe Arg Gln Arg Lys Glu Ser Val
        35                  40                  45

Val Gln His Leu Ile His Leu Cys Glu Glu Lys Arg Ala Ser Ile Ser
    50                  55                  60

Asp Ala Ala Leu Leu Asp Ile Ile Tyr Met Gln Phe His Gln His Gln
65                  70                  75                  80

Lys Val Trp Asp Val Phe Gln Met Ser Lys Gly Pro Gly Glu Asp Val
                85                  90                  95

Asp Leu Phe Asp Met Lys Gln Phe Lys Asn Ser Phe Lys Lys Ile Leu
            100                 105                 110

Gln Arg Ala Leu Lys Asn Val Thr Val Ser Phe Arg Glu Thr Glu Glu
        115                 120                 125

Asn Ala Val Trp Ile Arg Ile Ala Trp Gly Thr Gln Tyr Thr Lys Pro
    130                 135                 140

Asn Gln Tyr Lys Pro Thr Tyr Val Val Tyr Tyr Ser Gln Thr Pro Tyr
145                 150                 155                 160

Ala Phe Thr Ser Ser Ser Met Leu Arg Arg Asn Thr Pro Leu Leu Gly
                165                 170                 175

Gln Glu Leu Glu Ala Thr Gly Lys Ile Tyr Leu Arg Gln Glu Glu Ile
            180                 185                 190

Ile Leu Asp Ile Thr Glu Met Lys Lys Ala Cys Asn
        195                 200


<210>  348
<211>  397
<212>  PRT
<213>  Homo sapiens

<400>  348

Met Pro Pro Lys Lys Gln Ala Gln Ala Gly Gly Ser Lys Lys Ala Asp

1               5                   10                  15

Glu Lys Lys Lys Glu Lys Ile Ile Glu Asp Lys Thr Phe Gly Leu Lys
            20                  25                  30

Asn Lys Lys Gly Ala Lys Gln Gln Lys Phe Ile Lys Ala Val Thr His
        35                  40                  45

Gln Val Lys Phe Gly Gln Gln Asn Pro Arg Gln Val Ala Gln Ser Glu
    50                  55                  60

Ala Glu Lys Lys Leu Lys Lys Asp Asp Lys Lys Lys Asp Leu Gln Glu
65                  70                  75                  80

Leu Asn Glu Leu Phe Lys Pro Val Val Ala Ala Gln Lys Ile Ser Lys
                85                  90                  95

Gly Ala Asp Pro Lys Ser Val Val Cys Ala Phe Phe Lys Gln Gly Gln
            100                 105                 110

Cys Thr Lys Gly Asp Lys Cys Lys Phe Ser His Asp Leu Thr Leu Glu
        115                 120                 125

Arg Lys Cys Glu Lys Arg Ser Val Tyr Ile Asp Ala Arg Asp Glu Glu
    130                 135                 140

Leu Glu Lys Asp Thr Met Asp Asn Trp Asp Glu Lys Lys Leu Glu Glu
145                 150                 155                 160

Val Val Asn Lys Lys His Gly Glu Ala Glu Lys Lys Lys Pro Lys Thr
                165                 170                 175

Gln Ile Val Cys Lys His Phe Leu Glu Ala Ile Glu Asn Asn Lys Tyr
            180                 185                 190

Gly Trp Phe Trp Val Cys Pro Gly Gly Gly Asp Ile Cys Met Tyr Arg
        195                 200                 205

His Ala Leu Pro Pro Gly Phe Val Leu Lys Lys Asp Lys Lys Lys Glu
    210                 215                 220

Glu Lys Glu Asp Glu Ile Ser Leu Glu Asp Leu Ile Glu Arg Glu Arg
225                 230                 235                 240

Ser Ala Leu Gly Pro Asn Val Thr Lys Ile Thr Leu Glu Ser Phe Leu
                245                 250                 255

Ala Trp Lys Lys Arg Lys Arg Gln Glu Lys Ile Asp Lys Leu Glu Gln
            260                 265                 270

Asp Met Glu Arg Arg Lys Ala Asp Phe Lys Ala Gly Lys Ala Leu Val
        275                 280                 285

Ile Ser Gly Arg Glu Val Phe Glu Phe Arg Pro Glu Leu Val Asn Asp
    290                 295                 300

Asp Asp Glu Glu Ala Asp Asp Thr Arg Tyr Thr Gln Gly Thr Gly Gly
305                 310                 315                 320

Asp Glu Val Asp Asp Ser Val Ser Val Asn Asp Ile Asp Leu Ser Leu
                325                 330                 335

Tyr Ile Pro Arg Asp Val Asp Glu Thr Gly Ile Thr Val Ala Ser Leu
            340                 345                 350

Glu Arg Phe Ser Thr Tyr Thr Ser Asp Lys Asp Glu Asn Lys Leu Ser
        355                 360                 365

Glu Ala Ser Gly Gly Arg Ala Glu Asn Gly Glu Arg Ser Asp Leu Glu
    370                 375                 380

Glu Asp Asn Glu Arg Glu Gly Thr Gly Lys Trp Ser His
385                 390                 395

<210>  349
<211>  322
<212>  PRT
<213>  Homo sapiens

<400>  349

Met Glu Gly Ile Ser Asn Phe Lys Thr Pro Ser Lys Leu Ser Glu Lys
1               5                   10                  15

Lys Lys Ser Val Leu Cys Ser Thr Pro Thr Ile Asn Ile Pro Ala Ser
            20                  25                  30

Pro Phe Met Gln Lys Leu Gly Phe Gly Thr Gly Val Asn Val Tyr Leu
        35                  40                  45

Met Lys Arg Ser Pro Arg Gly Leu Ser His Ser Pro Trp Ala Val Lys
    50                  55                  60

Lys Ile Asn Pro Ile Cys Asn Asp His Tyr Arg Ser Val Tyr Gln Lys
65                  70                  75                  80

Arg Leu Met Asp Glu Ala Lys Ile Leu Lys Ser Leu His His Pro Asn
                85                  90                  95

Ile Val Gly Tyr Arg Ala Phe Thr Glu Ala Asn Asp Gly Ser Leu Cys
            100                 105                 110

Leu Ala Met Glu Tyr Gly Gly Glu Lys Ser Leu Asn Asp Leu Ile Glu
        115                 120                 125

Glu Arg Tyr Lys Ala Ser Gln Asp Pro Phe Pro Ala Ala Ile Ile Leu
    130                 135                 140

Lys Val Ala Leu Asn Met Ala Arg Gly Leu Lys Tyr Leu His Gln Glu
145                 150                 155                 160

Lys Lys Leu Leu His Gly Asp Ile Lys Ser Ser Asn Val Val Ile Lys
                165                 170                 175

Gly Asp Phe Glu Thr Ile Lys Ile Cys Asp Val Gly Val Ser Leu Pro
            180                 185                 190

Leu Asp Glu Asn Met Thr Val Thr Asp Pro Glu Ala Cys Tyr Ile Gly
        195                 200                 205

Thr Glu Pro Trp Lys Pro Lys Glu Ala Val Glu Glu Asn Gly Val Ile
    210                 215                 220

Thr Asp Lys Ala Asp Ile Phe Ala Phe Gly Leu Thr Leu Trp Glu Met
225                 230                 235                 240

Met Thr Leu Ser Ile Pro His Ile Asn Leu Ser Asn Asp Asp Asp Asp
                245                 250                 255

Glu Asp Lys Thr Phe Asp Glu Ser Asp Phe Asp Asp Glu Ala Tyr Tyr
            260                 265                 270

Ala Ala Leu Gly Thr Arg Pro Pro Ile Asn Met Glu Glu Leu Asp Glu
        275                 280                 285

Ser Tyr Gln Lys Val Ile Glu Leu Phe Ser Val Cys Thr Asn Glu Asp
    290                 295                 300

Pro Lys Asp Arg Pro Ser Ala Ala His Ile Val Glu Ala Leu Glu Thr
305                 310                 315                 320

Asp Val

<210>  350
<211>  322
<212>  PRT
<213>  Homo sapiens

<400>  350

Met Ala Ser Arg Ser Lys Arg Arg Ala Val Glu Ser Gly Val Pro Gln
1               5                   10                  15

Pro Pro Asp Pro Pro Val Gln Arg Asp Glu Glu Glu Glu Lys Glu Val
            20                  25                  30

Glu Asn Glu Asp Glu Asp Asp Asp Asp Ser Asp Lys Glu Lys Asp Glu
        35                  40                  45

Glu Asp Glu Val Ile Asp Glu Glu Val Asn Ile Glu Phe Glu Ala Tyr

50                  55                  60

Ser Leu Ser Asp Asn Asp Tyr Asp Gly Ile Lys Lys Leu Leu Gln Gln
65                  70                  75                  80

Leu Phe Leu Lys Ala Pro Val Asn Thr Ala Glu Leu Thr Asp Leu Leu
                85                  90                  95

Ile Gln Gln Asn His Ile Gly Ser Val Ile Lys Gln Thr Asp Val Ser
            100                 105                 110

Glu Asp Ser Asn Asp Asp Met Asp Glu Asp Glu Val Phe Gly Phe Ile
        115                 120                 125

Ser Leu Leu Asn Leu Thr Glu Arg Lys Gly Thr Gln Cys Val Glu Gln
    130                 135                 140

Ile Gln Glu Leu Val Leu Arg Phe Cys Glu Lys Asn Cys Glu Lys Ser
145                 150                 155                 160

Met Val Glu Gln Leu Asp Lys Phe Leu Asn Asp Thr Thr Lys Pro Val
                165                 170                 175

Gly Leu Leu Leu Ser Glu Arg Phe Ile Asn Val Pro Pro Gln Ile Ala
            180                 185                 190

Leu Pro Met Tyr Gln Gln Leu Gln Lys Glu Leu Ala Gly Ala His Arg
        195                 200                 205

Thr Asn Lys Pro Cys Gly Lys Cys Tyr Phe Tyr Leu Leu Ile Ser Lys
    210                 215                 220

Thr Phe Val Glu Ala Gly Lys Asn Asn Ser Lys Lys Lys Pro Ser Asn
225                 230                 235                 240

Lys Lys Lys Ala Ala Leu Met Phe Ala Asn Ala Glu Glu Glu Phe Phe
                245                 250                 255

Tyr Glu Glu Gln Gly Lys Pro Glu Val Leu Gly Gly Pro Asp Thr Arg
            260                 265                 270

Arg Gly Leu Glu Pro Val Pro Ile Gln His Asn Gly Gly Ser Arg Gly
        275                 280                 285

Gln Val Thr Ala Leu Val Ser Leu Lys Ala Gly Leu Ile Gln Ser Arg
    290                 295                 300

Ser Thr Leu Ser Asp Phe Gln Gly Thr Phe Met Thr Val Gly Ile Ala
305                 310                 315                 320

Leu Ser

<210> 351
<211> 314
<212> PRT
<213> Homo sapiens

<400> 351

Met Ala Ser Arg Ser Lys Arg Arg Ala Val Glu Ser Gly Val Pro Gln
1               5                   10                  15

Pro Pro Asp Pro Pro Val Gln Arg Asp Glu Glu Glu Glu Lys Glu Val
            20                  25                  30

Glu Asn Glu Asp Glu Asp Asp Asp Asp Ser Asp Lys Glu Lys Asp Glu
        35                  40                  45

Glu Asp Glu Val Ile Asp Glu Glu Val Asn Ile Glu Phe Glu Ala Tyr
    50                  55                  60

Ser Leu Ser Asp Asn Asp Tyr Asp Gly Ile Lys Lys Leu Leu Gln Gln
65                  70                  75                  80

Leu Phe Leu Lys Ala Pro Val Asn Thr Ala Glu Leu Thr Asp Leu Leu
                85                  90                  95

Ile Gln Gln Asn His Ile Gly Ser Val Ile Lys Gln Thr Asp Val Ser
            100                 105                 110

Glu Asp Ser Asn Asp Asp Met Asp Glu Asp Glu Val Phe Gly Phe Ile
        115                 120                 125

Ser Leu Leu Asn Leu Thr Glu Arg Lys Gly Thr Gln Cys Val Glu Gln
    130                 135                 140

Ile Gln Glu Leu Val Leu Arg Phe Cys Glu Lys Asn Cys Glu Lys Ser
145                 150                 155                 160

Met Val Glu Gln Leu Asp Lys Phe Leu Asn Asp Thr Thr Lys Pro Val
                165                 170                 175

Gly Leu Leu Leu Ser Glu Arg Phe Ile Asn Val Pro Pro Gln Ile Ala
            180                 185                 190

Leu Pro Met Tyr Gln Gln Leu Gln Lys Glu Leu Ala Gly Ala His Arg
        195                 200                 205

Thr Asn Lys Pro Cys Gly Lys Cys Tyr Phe Tyr Leu Leu Ile Ser Lys
    210                 215                 220

Thr Phe Val Glu Ala Gly Lys Asn Asn Ser Lys Lys Lys Pro Ser Asn
225                 230                 235                 240

Lys Lys Lys Ala Ala Leu Met Phe Ala Asn Ala Glu Glu Glu Phe Phe
                245                 250                 255

Tyr Glu Lys Ala Ile Leu Lys Phe Asn Tyr Ser Val Gln Glu Glu Ser
            260                 265                 270

Asp Thr Cys Leu Gly Gly Lys Trp Ser Phe Asp Asp Val Pro Met Thr
        275                 280                 285

Pro Leu Arg Thr Val Met Leu Ile Pro Gly Asp Lys Met Asn Glu Ile
    290                 295                 300

Met Asp Lys Leu Lys Glu Tyr Leu Ser Val
305                 310

<210>  352
<211>  292
<212>  PRT
<213>  Homo sapiens

<400>  352

Met Ala Ser Arg Ser Lys Arg Arg Ala Val Glu Ser Gly Val Pro Gln
1               5                   10                  15

Pro Pro Asp Pro Pro Val Gln Arg Asp Glu Glu Glu Glu Lys Glu Val
            20                  25                  30

Glu Asn Glu Asp Glu Asp Asp Asp Asp Ser Asp Lys Glu Lys Asp Glu
        35                  40                  45

Glu Asp Glu Val Ile Asp Glu Glu Val Asn Ile Glu Phe Glu Ala Tyr
    50                  55                  60

Ser Leu Ser Asp Asn Asp Tyr Asp Gly Ile Lys Lys Leu Leu Gln Gln
65                  70                  75                  80

Leu Phe Leu Lys Ala Pro Val Asn Thr Ala Glu Leu Thr Asp Leu Leu
                85                  90                  95

Ile Gln Gln Asn His Ile Gly Ser Val Ile Lys Gln Thr Asp Val Ser
            100                 105                 110

Glu Asp Ser Asn Asp Asp Met Asp Glu Asp Glu Val Phe Gly Phe Ile
        115                 120                 125

Ser Leu Leu Asn Leu Thr Glu Arg Lys Gly Thr Gln Cys Val Glu Gln
    130                 135                 140

Ile Gln Glu Leu Val Leu Arg Phe Cys Glu Lys Asn Cys Glu Lys Ser
145                 150                 155                 160

Met Val Glu Gln Leu Asp Lys Phe Leu Asn Asp Thr Thr Lys Pro Val
                165                 170                 175

Gly Leu Leu Leu Ser Glu Arg Phe Ile Asn Val Pro Pro Gln Ile Ala

180                 185                 190

Leu Pro Met Tyr Gln Gln Leu Gln Lys Glu Leu Ala Gly Ala His Arg
        195                 200                 205

Thr Asn Lys Pro Cys Gly Lys Cys Tyr Phe Tyr Leu Leu Ile Ser Lys
    210                 215                 220

Thr Phe Val Glu Ala Gly Lys Asn Asn Ser Lys Lys Lys Pro Ser Asn
225                 230                 235                 240

Lys Lys Lys Ala Ala Leu Met Phe Ala Asn Ala Glu Glu Glu Phe Phe
                245                 250                 255

Tyr Glu Glu Gln Gly Lys Pro Glu Val Leu Gly Gly Pro Asp Thr Arg
            260                 265                 270

Arg Gly Leu Glu Pro Val Pro Ile Gln His Asn Gly Trp Ser Val Pro
        275                 280                 285

Pro Val Leu Glu
    290

<210>  353
<211>  1388
<212>  PRT
<213>  Homo sapiens

<400>  353

Met Ala Pro Gly Cys Lys Thr Glu Leu Arg Ser Val Thr Asn Gly Gln
1               5                   10                  15

Ser Asn Gln Pro Ser Asn Glu Gly Asp Ala Ile Lys Val Phe Val Arg
            20                  25                  30

Ile Arg Pro Pro Ala Glu Arg Ser Gly Ser Ala Asp Gly Glu Gln Asn
        35                  40                  45

Leu Cys Leu Ser Val Leu Ser Ser Thr Ser Leu Arg Leu His Ser Asn
    50                  55                  60

Pro Glu Pro Lys Thr Phe Thr Phe Asp His Val Ala Asp Val Asp Thr
65                  70                  75                  80

Thr Gln Glu Ser Val Phe Ala Thr Val Ala Lys Ser Ile Val Glu Ser
                85                  90                  95

Cys Met Ser Gly Tyr Asn Gly Thr Ile Phe Ala Tyr Gly Gln Thr Gly
            100                 105                 110

Ser Gly Lys Thr Phe Thr Met Met Gly Pro Ser Glu Ser Asp Asn Phe
        115                 120                 125

Ser His Asn Leu Arg Gly Val Ile Pro Arg Ser Phe Glu Tyr Leu Phe
    130                 135                 140

Ser Leu Ile Asp Arg Glu Lys Glu Lys Ala Gly Ala Gly Lys Ser Phe
145                 150                 155                 160

Leu Cys Lys Cys Ser Phe Ile Glu Ile Tyr Asn Glu Gln Ile Tyr Asp
                165                 170                 175

Leu Leu Asp Ser Ala Ser Ala Gly Leu Tyr Leu Arg Glu His Ile Lys
            180                 185                 190

Lys Gly Val Phe Val Val Gly Ala Val Glu Gln Val Val Thr Ser Ala
        195                 200                 205

Ala Glu Ala Tyr Gln Val Leu Ser Gly Gly Trp Arg Asn Arg Arg Val
    210                 215                 220

Ala Ser Thr Ser Met Asn Arg Glu Ser Ser Arg Ser His Ala Val Phe
225                 230                 235                 240

Thr Ile Thr Ile Glu Ser Met Glu Lys Ser Asn Glu Ile Val Asn Ile
                245                 250                 255

Arg Thr Ser Leu Leu Asn Leu Val Asp Leu Ala Gly Ser Glu Arg Gln
            260                 265                 270

Lys Asp Thr His Ala Glu Gly Met Arg Leu Lys Glu Ala Gly Asn Ile
        275                 280                 285

Asn Arg Ser Leu Ser Cys Leu Gly Gln Val Ile Thr Ala Leu Val Asp
    290                 295                 300

Val Gly Asn Gly Lys Gln Arg His Val Cys Tyr Arg Asp Ser Lys Leu
305                 310                 315                 320

Thr Phe Leu Leu Arg Asp Ser Leu Gly Gly Asn Ala Lys Thr Ala Ile
                325                 330                 335

Ile Ala Asn Val His Pro Gly Ser Arg Cys Phe Gly Glu Thr Leu Ser
            340                 345                 350

Thr Leu Asn Phe Ala Gln Arg Ala Lys Leu Ile Lys Asn Lys Ala Val
        355                 360                 365

Val Asn Glu Asp Thr Gln Gly Asn Val Ser Gln Leu Gln Ala Glu Val
    370                 375                 380

Lys Arg Leu Lys Glu Gln Leu Ala Glu Leu Ala Ser Gly Gln Thr Pro
385                 390                 395                 400

Pro Glu Ser Phe Leu Thr Arg Asp Lys Lys Lys Thr Asn Tyr Met Glu
                405                 410                 415

Tyr Phe Gln Glu Ala Met Leu Phe Phe Lys Lys Ser Glu Gln Glu Lys
            420                 425                 430

Lys Ser Leu Ile Glu Lys Val Thr Gln Leu Glu Asp Leu Thr Leu Lys
        435                 440                 445

Lys Glu Lys Phe Ile Gln Ser Asn Lys Met Ile Val Lys Phe Arg Glu
    450                 455                 460

Asp Gln Ile Ile Arg Leu Glu Lys Leu His Lys Glu Ser Arg Gly Gly
465                 470                 475                 480

Phe Leu Pro Glu Glu Gln Asp Arg Leu Leu Ser Glu Leu Arg Asn Glu
                485                 490                 495

Ile Gln Thr Leu Arg Glu Gln Ile Glu His His Pro Arg Val Ala Lys
            500                 505                 510

Tyr Ala Met Glu Asn His Ser Leu Arg Glu Glu Asn Arg Arg Leu Arg
        515                 520                 525

Leu Leu Glu Pro Val Lys Arg Ala Gln Glu Met Asp Ala Gln Thr Ile
    530                 535                 540

Ala Lys Leu Glu Lys Ala Phe Ser Glu Ile Ser Gly Met Glu Lys Ser
545                 550                 555                 560

Asp Lys Asn Gln Gln Gly Phe Ser Pro Lys Ala Gln Lys Glu Pro Cys
                565                 570                 575

Leu Phe Ala Asn Thr Glu Lys Leu Lys Ala Gln Leu Leu Gln Ile Gln
            580                 585                 590

Thr Glu Leu Asn Asn Ser Lys Gln Glu Tyr Glu Glu Phe Lys Glu Leu
        595                 600                 605

Thr Arg Lys Arg Gln Leu Glu Leu Glu Ser Glu Leu Gln Ser Leu Gln
    610                 615                 620

Lys Ala Asn Leu Asn Leu Glu Asn Leu Leu Glu Ala Thr Lys Ala Cys
625                 630                 635                 640

Lys Arg Gln Glu Val Ser Gln Leu Asn Lys Ile His Ala Glu Thr Leu
                645                 650                 655

Lys Ile Ile Thr Thr Pro Thr Lys Ala Tyr Gln Leu His Ser Arg Pro
            660                 665                 670

Val Pro Lys Leu Ser Pro Glu Met Gly Ser Phe Gly Ser Leu Tyr Thr
        675                 680                 685

Gln Asn Ser Ser Ile Leu Asp Asn Asp Ile Leu Asn Glu Pro Val Pro
    690                 695                 700

Pro Glu Met Asn Glu Gln Ala Phe Glu Ala Ile Ser Glu Glu Leu Arg
705                 710                 715                 720

Thr Val Gln Glu Gln Met Ser Ala Leu Gln Ala Lys Leu Asp Glu Glu
                725                 730                 735

Glu His Lys Asn Leu Lys Leu Gln Gln His Val Asp Lys Leu Glu His
            740                 745                 750

His Ser Thr Gln Met Gln Glu Leu Phe Ser Ser Glu Arg Ile Asp Trp
        755                 760                 765

Thr Lys Gln Gln Glu Glu Leu Leu Ser Gln Leu Asn Val Leu Glu Lys
    770                 775                 780

Gln Leu Gln Glu Thr Gln Thr Lys Asn Asp Phe Leu Lys Ser Glu Val
785                 790                 795                 800

His Asp Leu Arg Val Val Leu His Ser Ala Asp Lys Glu Leu Ser Ser
                805                 810                 815

Val Lys Leu Glu Tyr Ser Ser Phe Lys Thr Asn Gln Glu Lys Glu Phe
            820                 825                 830

Asn Lys Leu Ser Glu Arg His Met His Val Gln Leu Gln Leu Asp Asn
        835                 840                 845

Leu Arg Leu Glu Asn Glu Lys Leu Leu Glu Ser Lys Ala Cys Leu Gln
    850                 855                 860

Asp Ser Tyr Asp Asn Leu Gln Glu Ile Met Lys Phe Glu Ile Asp Gln
865                 870                 875                 880

Leu Ser Arg Asn Leu Gln Asn Phe Lys Lys Glu Asn Glu Thr Leu Lys
                885                 890                 895

Ser Asp Leu Asn Asn Leu Met Glu Leu Leu Glu Ala Glu Lys Glu Arg
            900                 905                 910

Asn Asn Lys Leu Ser Leu Gln Phe Glu Glu Asp Lys Glu Asn Ser Ser
        915                 920                 925

Lys Glu Ile Leu Lys Val Leu Glu Ala Val Arg Gln Glu Lys Gln Lys
    930                 935                 940

Glu Thr Ala Lys Cys Glu Gln Gln Met Ala Lys Val Gln Lys Leu Glu
945                 950                 955                 960

Glu Ser Leu Leu Ala Thr Glu Lys Val Ile Ser Ser Leu Glu Lys Ser
                965                 970                 975

Arg Asp Ser Asp Lys Lys Val Val Ala Asp Leu Met Asn Gln Ile Gln
            980                 985                 990

Glu Leu Arg Thr Ser Val Cys Glu  Lys Thr Glu Thr Ile  Asp Thr Leu
        995                 1000                 1005

Lys Gln  Glu Leu Lys Asp Ile  Asn Cys Lys Tyr Asn  Ser Ala Leu
    1010                 1015                 1020

Val Asp  Arg Glu Glu Ser Arg  Val Leu Ile Lys Lys  Gln Glu Val
    1025                 1030                 1035

Asp Ile  Leu Asp Leu Lys Glu  Thr Leu Arg Leu Arg  Ile Leu Ser
    1040                 1045                 1050

Glu Asp  Ile Glu Arg Asp Met  Leu Cys Glu Asp Leu  Ala His Ala
    1055                 1060                 1065

Thr Glu  Gln Leu Asn Met Leu  Thr Glu Ala Ser Lys  Lys His Ser
    1070                 1075                 1080

Gly Leu  Leu Gln Ser Ala Gln  Glu Glu Leu Thr Lys  Lys Glu Ala
    1085                 1090                 1095

Leu Ile  Gln Glu Leu Gln His  Lys Leu Asn Gln Lys  Lys Glu Glu
    1100                 1105                 1110

Val Glu  Gln Lys Lys Asn Glu  Tyr Asn Phe Lys Met  Arg Gln Leu
    1115                 1120                 1125

Glu His  Val Met Asp Ser Ala  Ala Glu Asp Pro Gln  Ser Pro Lys
    1130                 1135                 1140

Thr Pro  Pro His Phe Gln Thr  His Leu Ala Lys Leu  Leu Glu Thr
    1145                 1150                 1155

Gln Glu  Gln Glu Ile Glu Asp  Gly Arg Ala Ser Lys  Thr Ser Leu
    1160                 1165                 1170

Glu His  Leu Val Thr Lys Leu  Asn Glu Asp Arg Glu  Val Lys Asn
    1175                 1180                 1185

Ala Glu  Ile Leu Arg Met Lys  Glu Gln Leu Arg Glu  Met Glu Asn
    1190                 1195                 1200

Leu Arg  Leu Glu Ser Gln Gln  Leu Ile Glu Lys Asn  Trp Leu Leu
    1205                 1210                 1215

Gln Gly  Gln Leu Asp Asp Ile  Lys Arg Gln Lys Glu  Asn Ser Asp
    1220                 1225                 1230

Gln Asn His Pro Asp Asn Gln Gln Leu Lys Asn Glu Gln Glu Glu
    1235                1240                1245

Ser Ile Lys Glu Arg Leu Ala Lys Ser Lys Ile Val Glu Glu Met
    1250                1255                1260

Leu Lys Met Lys Ala Asp Leu Glu Glu Val Gln Ser Ala Leu Tyr
    1265                1270                1275

Asn Lys Glu Met Glu Cys Leu Arg Met Thr Asp Glu Val Glu Arg
    1280                1285                1290

Thr Gln Thr Leu Glu Ser Lys Ala Phe Gln Glu Lys Glu Gln Leu
    1295                1300                1305

Arg Ser Lys Leu Glu Glu Met Tyr Glu Glu Arg Glu Arg Thr Ser
    1310                1315                1320

Gln Glu Met Glu Met Leu Arg Lys Gln Val Glu Cys Leu Ala Glu
    1325                1330                1335

Glu Asn Gly Lys Leu Val Gly His Gln Asn Leu His Gln Lys Ile
    1340                1345                1350

Gln Tyr Val Val Arg Leu Lys Lys Glu Asn Val Arg Leu Ala Glu
    1355                1360                1365

Glu Thr Glu Lys Leu Arg Ala Glu Asn Val Phe Leu Lys Glu Lys
    1370                1375                1380

Lys Arg Ser Glu Ser
    1385

<210>  354
<211>  224
<212>  PRT
<213>  Homo sapiens

<400>  354

Met Val Glu Asp Glu Leu Ala Leu Phe Asp Lys Ser Ile Asn Glu Phe
1               5                   10                  15

Trp Asn Lys Phe Lys Ser Thr Asp Thr Ser Cys Gln Met Ala Gly Leu
            20                  25                  30

Arg Asp Thr Tyr Lys Asp Ser Ile Lys Ala Phe Ala Glu Lys Leu Ser
        35                  40                  45

Val Lys Leu Lys Glu Glu Glu Arg Met Val Glu Met Phe Leu Glu Tyr
    50                  55                  60

Gln Asn Gln Ile Ser Arg Gln Asn Lys Leu Ile Gln Glu Lys Lys Asp
65                  70                  75                  80

Asn Leu Leu Lys Leu Ile Ala Glu Val Lys Gly Lys Lys Gln Glu Leu
                85                  90                  95

Glu Val Leu Thr Ala Asn Ile Gln Asp Leu Lys Glu Glu Tyr Ser Arg
            100                 105                 110

Lys Lys Glu Thr Ile Ser Thr Ala Asn Lys Ala Asn Ala Glu Arg Leu
        115                 120                 125

Lys Arg Leu Gln Lys Ser Ala Asp Leu Tyr Lys Asp Arg Leu Gly Leu
    130                 135                 140

Glu Ile Arg Lys Ile Tyr Gly Glu Lys Leu Gln Phe Ile Phe Thr Asn
145                 150                 155                 160

Ile Asp Pro Lys Asn Pro Glu Ser Pro Phe Met Phe Ser Leu His Leu
                165                 170                 175

Asn Glu Ala Arg Asp Tyr Glu Val Ser Asp Ser Ala Pro His Leu Glu
            180                 185                 190

Gly Leu Ala Glu Phe Gln Glu Asn Val Arg Lys Thr Asn Asn Phe Ser
        195                 200                 205

Ala Phe Leu Ala Asn Val Arg Lys Ala Phe Thr Ala Thr Val Tyr Asn
    210                 215                 220

<210>  355
<211>  359
<212>  PRT
<213>  Homo sapiens

<400>  355

Met Asp Cys Gly Ser Val Gly Gly Gln Arg Thr Gln Arg Leu Pro Gly
1               5                   10                  15

Arg Gln Arg Leu Leu Phe Leu Pro Val Gly Leu Ser Gly Arg Pro Gly
            20                  25                  30

Gly Ser Glu Thr Ser Ala Arg Arg Cys Leu Ser Ala Leu Ser Asp Gly
        35                  40                  45

Leu Gly Ala Leu Arg Pro Arg Ala Pro Ala Ala Arg Gly Gly Val Ser
    50                  55                  60

Arg Ala Ser Pro Leu Leu Leu Leu Leu Leu Val Pro Ser Pro Arg Leu
65                  70                  75                  80

Ala Ala Ala Ala Pro Arg Arg Gln Leu Gly Asp Trp Glu Arg Ser Arg
                85                  90                  95

Leu Gly Tyr Ala Ala Pro Pro Ala Gly Arg Ser Ser Ala Trp Arg Cys
            100                 105                 110

Ser Pro Gly Val Ala Ala Ala Ala Gly Ala Leu Pro Gln Tyr His Gly
        115                 120                 125

Pro Ala Pro Ala Leu Val Ser Cys Arg Arg Glu Leu Ser Leu Ser Ala
    130                 135                 140

Gly Ser Leu Gln Leu Glu Arg Lys Arg Arg Asp Phe Thr Ser Ser Gly
145                 150                 155                 160

Ser Arg Lys Leu Tyr Phe Asp Thr His Ala Leu Val Cys Leu Leu Glu
                165                 170                 175

Asp Asn Gly Phe Ala Thr Gln Gln Ala Glu Ile Ile Val Ser Ala Leu
            180                 185                 190

Val Lys Ile Leu Glu Ala Asn Met Asp Ile Val Tyr Lys Asp Met Val
        195                 200                 205

Thr Lys Met Gln Gln Glu Ile Thr Phe Gln Gln Val Met Ser Gln Ile
    210                 215                 220

Ala Asn Val Lys Lys Asp Met Ile Ile Leu Glu Lys Ser Glu Phe Ser
225                 230                 235                 240

Ala Leu Arg Ala Glu Asn Glu Lys Ile Lys Leu Glu Leu His Gln Leu
                245                 250                 255

Lys Gln Gln Val Met Asp Glu Val Ile Lys Val Arg Thr Asp Thr Lys
            260                 265                 270

Leu Asp Phe Asn Leu Glu Lys Ser Arg Val Lys Glu Leu Tyr Ser Leu
        275                 280                 285

Asn Glu Lys Lys Leu Leu Glu Leu Arg Thr Glu Ile Val Ala Leu His
    290                 295                 300

Ala Gln Gln Asp Arg Ala Leu Thr Gln Thr Asp Arg Lys Ile Glu Thr
305                 310                 315                 320

Glu Val Ala Gly Leu Lys Thr Met Leu Glu Ser His Lys Leu Asp Asn
                325                 330                 335

Ile Lys Tyr Leu Ala Gly Ser Ile Phe Thr Cys Leu Thr Val Ala Leu
            340                 345                 350

Gly Phe Tyr Arg Leu Trp Ile
        355

<210>  356
<211>  257
<212>  PRT
<213>  Homo sapiens

<400>  356

Met Asp Cys Gly Ser Val Gly Gly Gln Arg Thr Gln Arg Leu Pro Gly
1               5                   10                  15

Arg Gln Arg Leu Leu Phe Leu Pro Val Gly Leu Ser Gly Arg Pro Gly
            20                  25                  30

Gly Ser Glu Thr Ser Ala Arg Arg Cys Pro Ser Ala Leu Ser Asp Gly
        35                  40                  45

Leu Gly Ala Leu Arg Pro Arg Ala Pro Ala Ala Arg Gly Gly Val Ser
    50                  55                  60

Arg Ala Ser Pro Leu Leu Leu Leu Leu Leu Val Pro Ser Pro Arg Leu
65                  70                  75                  80

Ala Ala Ala Ala Pro Arg Arg Gln Leu Gly Asp Trp Glu Arg Ser Arg
                85                  90                  95

Leu Gly Tyr Ala Ala Pro Pro Ala Gly Arg Ser Gly Ala Trp Arg Cys
            100                 105                 110

Ser Pro Gly Val Ala Ala Ala Ala Gly Ala Leu Pro Gln Tyr His Gly
        115                 120                 125

Pro Ala Pro Ala Leu Val Ser Cys Arg Arg Glu Leu Ser Leu Ser Ala
    130                 135                 140

Gly Ser Leu Gln Leu Glu Arg Lys Arg Arg Asp Phe Thr Ser Ser Gly
145                 150                 155                 160

Ser Arg Lys Leu Tyr Phe Asp Thr His Ala Leu Val Cys Leu Leu Glu
                165                 170                 175

Asp Asn Glu Ser His Ser Phe Ile Gln Ala Gly Val Gln Trp His Ser
            180                 185                 190

Leu Gly Leu Leu Gln Pro Pro Pro Pro Gly Phe Lys Arg Ser Ser His
        195                 200                 205

Leu Ile Leu Leu Ser Ser Trp Asp Tyr Arg His Ala Pro Pro His Leu
    210                 215                 220

Asp Asn Phe Ser Val Phe Leu Leu Glu Thr Gly Phe His His Val Gly
225                 230                 235                 240

Gln Ala Gly Leu Lys Leu Leu Thr Ser Ser Asp Pro Pro Thr Leu Ala
                245                 250                 255

Ser

<210>  357
<211>  1015
<212>  PRT
<213>  Homo sapiens

<400>  357

Met Gly Ala Glu Arg Arg Leu Leu Ser Ile Lys Glu Ala Phe Arg Leu
1               5                   10                  15

Ala Gln Gln Pro His Gln Asn Gln Ala Lys Leu Val Val Ala Leu Ser
            20                  25                  30

Arg Thr Tyr Arg Thr Met Asp Asp Lys Thr Val Phe His Glu Glu Phe
        35                  40                  45

Ile His Tyr Leu Lys Tyr Val Met Val Val Tyr Lys Arg Glu Pro Ala
    50                  55                  60

Val Glu Arg Val Ile Glu Phe Ala Ala Lys Phe Val Thr Ser Phe His
65                  70                  75                  80

Gln Ser Asp Met Glu Asp Asp Glu Glu Glu Glu Asp Gly Gly Leu Leu
                85                  90                  95

Asn Tyr Leu Phe Thr Phe Leu Leu Lys Ser His Glu Ala Asn Ser Asn
            100                 105                 110

Ala Val Arg Phe Arg Val Cys Leu Leu Ile Asn Lys Leu Leu Gly Ser
        115                 120                 125

Met Pro Glu Asn Ala Gln Ile Asp Asp Asp Val Phe Asp Lys Ile Asn
    130                 135                 140

Lys Ala Met Leu Ile Arg Leu Lys Asp Lys Ile Pro Asn Val Arg Ile
145                 150                 155                 160

Gln Ala Val Leu Ala Leu Ser Arg Leu Gln Asp Pro Lys Asp Asp Glu
                165                 170                 175

Cys Pro Val Val Asn Ala Tyr Ala Thr Leu Ile Glu Asn Asp Ser Asn
            180                 185                 190

Pro Glu Val Arg Arg Ala Val Leu Ser Cys Ile Ala Pro Ser Ala Lys
        195                 200                 205

Thr Leu Pro Lys Ile Val Gly Arg Thr Lys Asp Val Lys Glu Ala Val
    210                 215                 220

Arg Lys Leu Ala Tyr Gln Val Leu Ala Glu Lys Val His Met Arg Ala
225                 230                 235                 240

Met Ser Ile Ala Gln Arg Val Met Leu Leu Gln Gln Gly Leu Asn Asp
                245                 250                 255

Arg Ser Asp Ala Val Lys Gln Ala Met Gln Lys His Leu Leu Gln Gly
            260                 265                 270

Trp Leu Arg Phe Ser Glu Gly Asn Ile Leu Glu Leu Leu His Arg Leu
        275                 280                 285

Asp Val Glu Asn Ser Ser Glu Val Ala Val Ser Val Leu Asn Ala Leu
    290                 295                 300

Phe Ser Ile Thr Pro Leu Ser Glu Leu Val Gly Leu Cys Lys Asn Asn
305                 310                 315                 320

Asp Gly Arg Lys Leu Ile Pro Val Glu Thr Leu Thr Pro Glu Ile Ala
                325                 330                 335

Leu Tyr Trp Cys Ala Leu Cys Glu Tyr Leu Lys Ser Lys Gly Asp Glu
            340                 345                 350

Gly Glu Glu Phe Leu Glu Gln Ile Leu Pro Glu Pro Val Val Tyr Ala
        355                 360                 365

Asp Tyr Leu Leu Ser Tyr Ile Gln Ser Ile Pro Val Val Asn Glu Glu
    370                 375                 380

His Arg Gly Asp Phe Ser Tyr Ile Gly Asn Leu Met Thr Lys Glu Phe
385                 390                 395                 400

Ile Gly Gln Gln Leu Ile Leu Ile Ile Lys Ser Leu Asp Thr Ser Glu
                405                 410                 415

Glu Gly Gly Arg Lys Lys Leu Leu Ala Val Leu Gln Glu Ile Leu Ile
            420                 425                 430

Leu Pro Thr Ile Pro Ile Ser Leu Val Ser Phe Leu Val Glu Arg Leu
        435                 440                 445

Leu His Ile Ile Ile Asp Asp Asn Lys Arg Thr Gln Ile Val Thr Glu
    450                 455                 460

Ile Ile Ser Glu Ile Arg Ala Pro Ile Val Thr Val Gly Val Asn Asn
465                 470                 475                 480

Asp Pro Ala Asp Val Arg Lys Lys Glu Leu Lys Met Ala Glu Ile Lys
                485                 490                 495

Val Lys Leu Ile Glu Ala Lys Glu Ala Leu Glu Asn Cys Ile Thr Leu
            500                 505                 510

Gln Asp Phe Asn Arg Ala Ser Glu Leu Lys Glu Glu Ile Lys Ala Leu
        515                 520                 525

Glu Asp Ala Arg Ile Asn Leu Leu Lys Glu Thr Glu Gln Leu Glu Ile
    530                 535                 540

Lys Glu Val His Ile Glu Lys Asn Asp Ala Glu Thr Leu Gln Lys Cys
545                 550                 555                 560

Leu Ile Leu Cys Tyr Glu Leu Leu Lys Gln Met Ser Ile Ser Thr Gly
                565                 570                 575

Leu Ser Ala Thr Met Asn Gly Ile Ile Glu Ser Leu Ile Leu Pro Gly
            580                 585                 590

Ile Ile Ser Ile His Pro Val Val Arg Asn Leu Ala Val Leu Cys Leu
        595                 600                 605

Gly Cys Cys Gly Leu Gln Asn Gln Asp Phe Ala Arg Lys His Phe Val
    610                 615                 620

Leu Leu Leu Gln Val Leu Gln Ile Asp Asp Val Thr Ile Lys Ile Ser
625                 630                 635                 640

Ala Leu Lys Ala Ile Phe Asp Gln Leu Met Thr Phe Gly Ile Glu Pro
                645                 650                 655

Phe Lys Thr Lys Lys Ile Lys Thr Leu His Cys Glu Gly Thr Glu Ile
            660                 665                 670

Asn Ser Asp Asp Glu Gln Glu Ser Lys Glu Val Glu Glu Thr Ala Thr
        675                 680                 685

Ala Lys Asn Val Leu Lys Leu Leu Ser Asp Phe Leu Asp Ser Glu Val
    690                 695                 700

Ser Glu Leu Arg Thr Gly Ala Ala Glu Gly Leu Ala Lys Leu Met Phe
705                 710                 715                 720

Ser Gly Leu Leu Val Ser Ser Arg Ile Leu Ser Arg Leu Ile Leu Leu
                725                 730                 735

Trp Tyr Asn Pro Val Thr Glu Glu Asp Val Gln Leu Arg His Cys Leu
            740                 745                 750

Gly Val Phe Phe Pro Val Phe Ala Tyr Ala Ser Arg Thr Asn Gln Glu
        755                 760                 765

Cys Phe Glu Glu Ala Phe Leu Pro Thr Leu Gln Thr Leu Ala Asn Ala
    770                 775                 780

Pro Ala Ser Ser Pro Leu Ala Glu Ile Asp Ile Thr Asn Val Ala Glu
785                 790                 795                 800

Leu Leu Val Asp Leu Thr Arg Pro Ser Gly Leu Asn Pro Gln Ala Lys
                805                 810                 815

Thr Ser Gln Asp Tyr Gln Ala Leu Thr Val His Asp Asn Leu Ala Met
            820                 825                 830

Lys Ile Cys Asn Glu Ile Leu Thr Ser Pro Cys Ser Pro Glu Ile Arg
        835                 840                 845

Val Tyr Thr Lys Ala Leu Ser Ser Leu Glu Leu Ser Ser His Leu Ala
    850                 855                 860

Lys Asp Leu Leu Val Leu Leu Asn Glu Ile Leu Glu Gln Val Lys Asp
865                 870                 875                 880

Arg Thr Cys Leu Arg Ala Leu Glu Lys Ile Lys Ile Gln Leu Glu Lys
                885                 890                 895

Gly Asn Lys Glu Phe Gly Asp Gln Ala Glu Ala Ala Gln Asp Ala Thr
            900                 905                 910

Leu Thr Thr Thr Thr Phe Gln Asn Glu Asp Glu Lys Asn Lys Glu Val
        915                 920                 925

Tyr Met Thr Pro Leu Arg Gly Val Lys Ala Thr Gln Ala Ser Lys Ser
    930                 935                 940

Thr Gln Leu Lys Thr Asn Arg Gly Gln Arg Lys Val Thr Val Ser Ala
945                 950                 955                 960

Arg Thr Asn Arg Arg Cys Gln Thr Ala Glu Ala Asp Ser Glu Ser Asp
                965                 970                 975

His Glu Val Pro Glu Pro Glu Ser Glu Met Lys Met Arg Leu Pro Arg
            980                 985                 990

Arg Ala Lys Thr Ala Ala Leu Glu  Lys Ser Lys Leu Asn  Leu Ala Gln
        995                 1000                 1005

Phe Leu  Asn Glu Asp Leu Ser
    1010                 1015

<210>  358
<211>  599
<212>  PRT
<213>  Homo sapiens

<400>  358

Met Met Leu Pro Tyr Pro Ser Ala Leu Gly Asp Gln Tyr Trp Glu Glu
1               5                   10                  15

Ile Leu Leu Pro Lys Asn Gly Glu Asn Val Glu Thr Met Lys Lys Leu
            20                  25                  30

Thr Gln Asn His Lys Ala Lys Gly Leu Pro Ser Asn Asp Thr Asp Cys

35                  40                  45

Pro Gln Lys Lys Glu Gly Lys Ala Gln Ile Val Val Pro Val Thr Phe
    50                  55                  60

Arg Asp Val Thr Val Ile Phe Thr Glu Ala Glu Trp Lys Arg Leu Ser
65                  70                  75                  80

Pro Glu Gln Arg Asn Leu Tyr Lys Glu Val Met Leu Glu Asn Tyr Arg
                85                  90                  95

Asn Leu Leu Ser Leu Ala Glu Pro Lys Pro Glu Ile Tyr Thr Cys Ser
            100                 105                 110

Ser Cys Leu Leu Ala Phe Ser Cys Gln Gln Phe Leu Ser Gln His Val
        115                 120                 125

Leu Gln Ile Phe Leu Gly Leu Cys Ala Glu Asn His Phe His Pro Gly
    130                 135                 140

Asn Ser Ser Pro Gly His Trp Lys Gln Gln Gly Gln Gln Tyr Ser His
145                 150                 155                 160

Val Ser Cys Trp Phe Glu Asn Ala Glu Gly Gln Glu Arg Gly Gly Gly
                165                 170                 175

Ser Lys Pro Trp Ser Ala Arg Thr Glu Glu Arg Glu Thr Ser Arg Ala
            180                 185                 190

Phe Pro Ser Pro Leu Gln Arg Gln Ser Ala Ser Pro Arg Lys Gly Asn
        195                 200                 205

Met Val Val Glu Thr Glu Pro Ser Ser Ala Gln Arg Pro Asn Pro Val
    210                 215                 220

Gln Leu Asp Lys Gly Leu Lys Glu Leu Glu Thr Leu Arg Phe Gly Ala
225                 230                 235                 240

Ile Asn Cys Arg Glu Tyr Glu Pro Asp His Asn Leu Glu Ser Asn Phe
                245                 250                 255

Ile Thr Asn Pro Arg Thr Leu Leu Gly Lys Lys Pro Tyr Ile Cys Ser
            260                 265                 270

Asp Cys Gly Arg Ser Phe Lys Asp Arg Ser Thr Leu Ile Arg His His
        275                 280                 285

Arg Ile His Ser Met Glu Lys Pro Tyr Val Cys Ser Glu Cys Gly Arg
    290                 295                 300

Gly Phe Ser Gln Lys Ser Asn Leu Ser Arg His Gln Arg Thr His Ser
305                 310                 315                 320

Glu Glu Lys Pro Tyr Leu Cys Arg Glu Cys Gly Gln Ser Phe Arg Ser
                325                 330                 335

Lys Ser Ile Leu Asn Arg His Gln Trp Thr His Ser Glu Glu Lys Pro
            340                 345                 350

Tyr Val Cys Ser Glu Cys Gly Arg Gly Phe Ser Glu Lys Ser Ser Phe
        355                 360                 365

Ile Arg His Gln Arg Thr His Ser Gly Glu Lys Pro Tyr Val Cys Leu
    370                 375                 380

Glu Cys Gly Arg Ser Phe Cys Asp Lys Ser Thr Leu Arg Lys His Gln
385                 390                 395                 400

Arg Ile His Ser Gly Glu Lys Pro Tyr Val Cys Arg Glu Cys Gly Arg
                405                 410                 415

Gly Phe Ser Gln Asn Ser Asp Leu Ile Lys His Gln Arg Thr His Leu
            420                 425                 430

Asp Glu Lys Pro Tyr Val Cys Arg Glu Cys Gly Arg Gly Phe Cys Asp
        435                 440                 445

Lys Ser Thr Leu Ile Ile His Glu Arg Thr His Ser Gly Glu Lys Pro
    450                 455                 460

Tyr Val Cys Gly Glu Cys Gly Arg Gly Phe Ser Arg Lys Ser Leu Leu
465                 470                 475                 480

Leu Val His Gln Arg Thr His Ser Gly Glu Lys His Tyr Val Cys Arg
                485                 490                 495

Glu Cys Arg Arg Gly Phe Ser Gln Lys Ser Asn Leu Ile Arg His Gln
            500                 505                 510

Arg Thr His Ser Asn Glu Lys Pro Tyr Ile Cys Arg Glu Cys Gly Arg
        515                 520                 525

Gly Phe Cys Asp Lys Ser Thr Leu Ile Val His Glu Arg Thr His Ser
    530                 535                 540

Gly Glu Lys Pro Tyr Val Cys Ser Glu Cys Gly Arg Gly Phe Ser Arg
545                 550                 555                 560

Lys Ser Leu Leu Leu Val His Gln Arg Thr His Ser Gly Glu Lys His
                565                 570                 575

Tyr Val Cys Arg Glu Cys Gly Arg Gly Phe Ser His Lys Ser Asn Leu
            580                 585                 590

Ile Arg His Gln Arg Thr His

        595

<210>  359
<211>  418
<212>  PRT
<213>  Homo sapiens

<400>  359

Met Ala Pro Arg Gly Arg Arg Arg Pro Arg Pro His Arg Ser Glu Gly
1               5                   10                  15

Ala Arg Arg Ser Lys Asn Thr Leu Glu Arg Thr His Ser Met Lys Asp
            20                  25                  30

Lys Ala Gly Gln Lys Cys Lys Pro Ile Asp Val Phe Asp Phe Pro Asp
        35                  40                  45

Asn Ser Asp Val Ser Ser Ile Gly Arg Leu Gly Glu Asn Glu Lys Asp
    50                  55                  60

Glu Glu Thr Tyr Glu Thr Phe Asp Pro Pro Leu His Ser Thr Ala Ile
65                  70                  75                  80

Tyr Ala Asp Glu Glu Glu Phe Ser Lys His Cys Gly Leu Ser Leu Ser
                85                  90                  95

Ser Thr Pro Pro Gly Lys Glu Ala Lys Arg Ser Ser Asp Thr Ser Gly
            100                 105                 110

Asn Glu Ala Ser Glu Ile Glu Ser Val Lys Ile Ser Ala Lys Lys Pro
        115                 120                 125

Gly Arg Lys Leu Arg Pro Ile Ser Asp Asp Ser Glu Ser Ile Glu Glu
    130                 135                 140

Ser Asp Thr Arg Arg Lys Val Lys Ser Ala Glu Lys Ile Ser Thr Gln
145                 150                 155                 160

Arg His Glu Val Ile Arg Thr Thr Ala Ser Ser Glu Leu Ser Glu Lys
                165                 170                 175

Pro Ala Glu Ser Val Thr Ser Lys Lys Thr Gly Pro Leu Ser Ala Gln
            180                 185                 190

Pro Ser Val Glu Lys Glu Asn Leu Ala Ile Glu Ser Gln Ser Lys Thr
        195                 200                 205

Gln Lys Lys Gly Lys Ile Ser His Asp Lys Arg Lys Lys Ser Arg Ser
    210                 215                 220

Lys Ala Ile Gly Ser Asp Thr Ser Asp Ile Val His Ile Trp Cys Pro
225                 230                 235                 240

Glu Gly Met Lys Thr Ser Asp Ile Lys Glu Leu Asn Ile Val Leu Pro
                245                 250                 255

Glu Phe Glu Lys Thr His Leu Glu His Gln Gln Arg Ile Glu Ser Lys
            260                 265                 270

Val Cys Lys Ala Ala Ile Ala Thr Phe Tyr Val Asn Val Lys Glu Gln
        275                 280                 285

Phe Ile Lys Met Leu Lys Glu Ser Gln Met Leu Thr Asn Leu Lys Arg
    290                 295                 300

Lys Asn Ala Lys Met Ile Ser Asp Ile Glu Lys Lys Arg Gln Arg Met
305                 310                 315                 320

Ile Glu Val Gln Asp Glu Leu Leu Arg Leu Glu Pro Gln Leu Lys Gln
                325                 330                 335

Leu Gln Thr Lys Tyr Asp Glu Leu Lys Glu Arg Lys Ser Ser Leu Arg
            340                 345                 350

Asn Ala Ala Tyr Phe Leu Ser Asn Leu Lys Gln Leu Tyr Gln Asp Tyr
        355                 360                 365

Ser Asp Val Gln Ala Gln Glu Pro Asn Val Lys Glu Thr Tyr Asp Ser
    370                 375                 380

Ser Ser Leu Pro Ala Leu Leu Phe Lys Ala Arg Thr Leu Leu Gly Ala
385                 390                 395                 400

Glu Ser His Leu Arg Asn Ile Asn His Gln Leu Glu Lys Leu Leu Asp
                405                 410                 415

Gln Gly

<210>  360
<211>  268
<212>  PRT
<213>  Homo sapiens

<400>  360

Met Glu Met Lys Lys Lys Ile Asn Leu Glu Leu Arg Asn Arg Ser Pro
1               5                   10                  15

Glu Glu Val Thr Glu Leu Val Leu Asp Asn Cys Leu Cys Val Asn Gly
            20                  25                  30

Glu Ile Glu Gly Leu Asn Asp Thr Phe Lys Glu Leu Glu Phe Leu Ser
        35                  40                  45

Met Ala Asn Val Glu Leu Ser Ser Leu Ala Arg Leu Pro Ser Leu Asn
    50                  55                  60

Lys Leu Arg Lys Leu Glu Leu Ser Asp Asn Ile Ile Ser Gly Gly Leu
65                  70      '           75                  80

Glu Val Leu Ala Glu Lys Cys Pro Asn Leu Thr Tyr Leu Asn Leu Ser
                85                  90                  95

Gly Asn Lys Ile Lys Asp Leu Ser Thr Val Glu Ala Leu Gln Asn Leu
            100                 105                 110

Lys Asn Leu Lys Ser Leu Asp Leu Phe Asn Cys Glu Ile Thr Asn Leu
        115                 120                 125

Glu Asp Tyr Arg Glu Ser Ile Phe Glu Leu Leu Gln Gln Ile Thr Tyr
    130                 135                 140

Leu Asp Gly Phe Asp Gln Glu Asp Asn Glu Ala Pro Asp Ser Glu Glu
145                 150                 155                 160

Glu Asp Asp Glu Asp Gly Asp Glu Asp Asp Glu Glu Glu Glu Glu Asn
                165                 170                 175

Glu Ala Gly Pro Pro Glu Gly Tyr Glu Glu Glu Glu Glu Glu Glu Glu
            180                 185                 190

Glu Glu Asp Glu Asp Glu Asp Glu Asp Glu Asp Glu Ala Gly Ser Glu
        195                 200                 205

Leu Gly Glu Gly Glu Glu Glu Val Gly Leu Ser Tyr Leu Met Lys Glu
    210                 215                 220

Glu Ile Gln Asp Glu Glu Asp Asp Asp Asp Tyr Val Glu Glu Gly Glu
225                 230                 235                 240

Glu Glu Glu Glu Glu Glu Glu Gly Gly Leu Arg Gly Glu Lys Arg Lys
                245                 250                 255

Arg Asp Ala Glu Asp Asp Gly Glu Glu Glu Asp Asp
            260                 265

<210>  361
<211>  898
<212>  PRT
<213>  Homo sapiens

<400>  361

Met Ser Val Thr Glu Glu Asp Leu Cys His His Met Lys Val Val Val
1               5                   10                  15

Arg Val Arg Pro Glu Asn Thr Lys Glu Lys Ala Ala Gly Phe His Lys
            20                  25                  30

Val Val His Val Val Asp Lys His Ile Leu Val Phe Asp Pro Lys Gln
        35                  40                  45

Glu Glu Val Ser Phe Phe His Gly Lys Lys Thr Thr Asn Gln Asn Val
    50                  55                  60

Ile Lys Lys Gln Asn Lys Asp Leu Lys Phe Val Phe Asp Ala Val Phe
65                  70                  75                  80

Asp Glu Thr Ser Thr Gln Ser Glu Val Phe Glu His Thr Thr Lys Pro
                85                  90                  95

Ile Leu Arg Ser Phe Leu Asn Gly Tyr Asn Cys Thr Val Leu Ala Tyr
            100                 105                 110

Gly Ala Thr Gly Ala Gly Lys Thr His Thr Met Leu Gly Ser Ala Asp
        115                 120                 125

Glu Pro Gly Val Met Tyr Leu Thr Met Leu His Leu Tyr Lys Cys Met
    130                 135                 140

Asp Glu Ile Lys Glu Glu Lys Ile Cys Ser Thr Ala Val Ser Tyr Leu
145                 150                 155                 160

Glu Val Tyr Asn Glu Gln Ile Arg Asp Leu Leu Val Asn Ser Gly Pro
                165                 170                 175

Leu Ala Val Arg Glu Asp Thr Gln Lys Gly Val Val Val His Gly Leu
            180                 185                 190

Thr Leu His Gln Pro Lys Ser Ser Glu Glu Ile Leu His Leu Leu Asp
        195                 200                 205

Asn Gly Asn Lys Asn Arg Thr Gln His Pro Thr Asp Met Asn Ala Thr
    210                 215                 220

Ser Ser Arg Ser His Ala Val Phe Gln Ile Tyr Leu Arg Gln Gln Asp
225                 230                 235                 240

Lys Thr Ala Ser Ile Asn Gln Asn Val Arg Ile Ala Lys Met Ser Leu
                245                 250                 255

Ile Asp Leu Ala Gly Ser Glu Arg Ala Ser Thr Ser Gly Ala Lys Gly
            260                 265                 270

Thr Arg Phe Val Glu Gly Thr Asn Ile Asn Arg Ser Leu Leu Ala Leu
        275                 280                 285

Gly Asn Val Ile Asn Ala Leu Ala Asp Ser Lys Arg Lys Asn Gln His
    290                 295                 300

Ile Pro Tyr Arg Asn Ser Lys Leu Thr Arg Leu Leu Lys Asp Ser Leu
305                 310                 315                 320

Gly Gly Asn Cys Gln Thr Ile Met Ile Ala Ala Val Ser Pro Ser Ser
                325                 330                 335

Val Phe Tyr Asp Asp Thr Tyr Asn Thr Leu Lys Tyr Ala Asn Arg Ala
            340                 345                 350

Lys Asp Ile Lys Ser Ser Leu Lys Ser Asn Val Leu Asn Val Asn Asn
        355                 360                 365

His Ile Thr Gln Tyr Val Lys Ile Cys Asn Glu Gln Lys Ala Glu Ile
    370                 375                 380

Leu Leu Leu Lys Glu Lys Leu Lys Ala Tyr Glu Glu Gln Lys Ala Phe
385                 390                 395                 400

Thr Asn Glu Asn Asp Gln Ala Lys Leu Met Ile Ser Asn Pro Gln Glu
                405                 410                 415

Lys Glu Ile Glu Arg Phe Gln Glu Ile Leu Asn Cys Leu Phe Gln Asn
            420                 425                 430

Arg Glu Glu Ile Arg Gln Glu Tyr Leu Lys Leu Glu Met Leu Leu Lys
        435                 440                 445

Glu Asn Glu Leu Lys Ser Phe Tyr Gln Gln Gln Cys His Lys Gln Ile
    450                 455                 460

Glu Met Met Cys Ser Glu Asp Lys Val Glu Lys Ala Thr Gly Lys Arg
465                 470                 475                 480

Asp His Arg Leu Ala Met Leu Lys Thr Arg Arg Ser Tyr Leu Glu Lys
                485                 490                 495

Arg Arg Glu Glu Glu Leu Lys Gln Phe Asp Glu Asn Thr Asn Trp Leu
            500                 505                 510

His Arg Val Glu Lys Glu Met Gly Leu Leu Ser Gln Asn Gly His Ile
        515                 520                 525

Pro Lys Glu Leu Lys Lys Asp Leu His Cys His His Leu His Leu Gln
    530                 535                 540

Asn Lys Asp Leu Lys Ala Gln Ile Arg His Met Met Asp Leu Ala Cys
545                 550                 555                 560

Leu Gln Glu Gln Gln His Arg Gln Thr Glu Ala Val Leu Asn Ala Leu
                565                 570                 575

Leu Pro Thr Leu Arg Lys Gln Tyr Cys Thr Leu Lys Glu Ala Gly Leu
            580                 585                 590

Ser Asn Ala Ala Phe Glu Ser Asp Phe Lys Glu Ile Glu His Leu Val
        595                 600                 605

Glu Arg Lys Lys Val Val Val Trp Ala Asp Gln Thr Gly Glu Gln Pro
    610                 615                 620

Lys Gln Asn Asp Leu Pro Gly Ile Ser Val Leu Met Thr Phe Ser Gln
625                 630                 635                 640

Leu Gly Pro Val Gln Pro Ile Pro Cys Cys Ser Ser Ser Gly Gly Thr
                645                 650                 655

Asn Leu Val Lys Ile Pro Thr Glu Lys Arg Thr Arg Arg Lys Leu Met
            660                 665                 670

Pro Ser Pro Leu Lys Gly Gln His Thr Leu Lys Ser Pro Pro Ser Gln
        675                 680                 685

Ser Val Gln Leu Asn Asp Ser Leu Ser Lys Glu Leu Gln Pro Ile Val
    690                 695                 700

Tyr Thr Pro Glu Asp Cys Arg Lys Ala Phe Gln Asn Pro Ser Thr Val
705                 710                 715                 720

Thr Leu Met Lys Pro Ser Ser Phe Thr Thr Ser Phe Gln Ala Ile Ser
                725                 730                 735

Ser Asn Ile Asn Ser Asp Asn Cys Leu Lys Met Leu Cys Glu Val Ala
            740                 745                 750

Ile Pro His Asn Arg Arg Lys Glu Cys Gly Gln Glu Asp Leu Asp Ser
        755                 760                 765

Thr Phe Thr Ile Cys Glu Asp Ile Lys Ser Ser Lys Cys Lys Leu Pro
    770                 775                 780

Glu Gln Glu Ser Leu Pro Asn Asp Asn Lys Asp Ile Leu Gln Arg Leu
785                 790                 795                 800

Asp Pro Ser Ser Phe Ser Thr Lys His Ser Met Pro Val Pro Ser Met
                805                 810                 815

Val Pro Ser Tyr Met Ala Met Thr Thr Ala Ala Lys Arg Lys Arg Lys
            820                 825                 830

Leu Thr Ser Ser Thr Ser Asn Ser Ser Leu Thr Ala Asp Val Asn Ser
        835                 840                 845

Gly Phe Ala Lys Arg Val Arg Gln Asp Asn Ser Ser Glu Lys His Leu
    850                 855                 860

Gln Glu Asn Lys Pro Thr Met Glu His Lys Arg Asn Ile Cys Lys Ile
865                 870                 875                 880

Asn Pro Ser Met Val Arg Lys Phe Gly Arg Asn Ile Ser Lys Gly Asn
                885                 890                 895

Leu Arg

<210>  362
<211>  268
<212>  PRT
<213>  Homo sapiens

<400>  362

Met Gly Ser Ala Lys Ser Val Pro Val Thr Pro Ala Arg Pro Pro Pro
1               5                   10                  15

His Asn Lys His Leu Ala Arg Val Ala Asp Pro Arg Ser Pro Ser Ala
            20                  25                  30

Gly Ile Leu Arg Thr Pro Ile Gln Val Glu Ser Ser Pro Gln Pro Gly
        35                  40                  45

Leu Pro Ala Gly Glu Gln Leu Glu Gly Leu Lys His Ala Gln Asp Ser
    50                  55                  60

Asp Pro Arg Ser Pro Thr Leu Gly Ile Ala Arg Thr Pro Met Lys Thr
65                  70                  75                  80

Ser Ser Gly Asp Pro Pro Ser Pro Leu Val Lys Gln Leu Ser Glu Val
                85                  90                  95

Phe Glu Thr Glu Asp Ser Lys Ser Asn Leu Pro Pro Glu Pro Val Leu
            100                 105                 110

Pro Pro Glu Ala Pro Leu Ser Ser Glu Leu Asp Leu Pro Leu Gly Thr
        115                 120                 125

Gln Leu Ser Val Glu Glu Gln Met Pro Pro Trp Asn Gln Thr Glu Phe
    130                 135                 140

Pro Ser Lys Gln Val Phe Ser Lys Glu Glu Ala Arg Gln Pro Thr Glu
145                 150                 155                 160

Thr Pro Val Ala Ser Gln Ser Ser Asp Lys Pro Ser Arg Asp Pro Glu
                165                 170                 175

Thr Pro Arg Ser Ser Gly Ser Met Arg Asn Arg Trp Lys Pro Asn Ser
            180                 185                 190

Ser Lys Val Leu Gly Arg Ser Pro Leu Thr Ile Leu Gln Asp Asp Asn
        195                 200                 205

Ser Pro Gly Thr Leu Thr Leu Arg Gln Gly Lys Arg Pro Ser Pro Leu
    210                 215                 220

Ser Glu Asn Val Ser Glu Leu Lys Glu Gly Ala Ile Leu Gly Thr Gly
225                 230                 235                 240

Arg Leu Leu Lys Thr Gly Gly Arg Ala Trp Glu Gln Gly Gln Asp His
                245                 250                 255

Asp Lys Glu Asn Gln His Phe Pro Leu Val Glu Ser
            260                 265

<210>  363
<211>  310
<212>  PRT
<213>  Homo sapiens

<400>  363

Met Glu Asp Leu Glu Glu Asp Val Arg Phe Ile Val Asp Glu Thr Leu
1               5                   10                  15

Asp Phe Gly Gly Leu Ser Pro Ser Asp Ser Arg Glu Glu Glu Asp Ile
            20                  25                  30

Thr Val Leu Val Thr Pro Glu Lys Pro Leu Arg Arg Gly Leu Ser His
        35                  40                  45

Arg Ser Asp Pro Asn Ala Val Ala Pro Ala Pro Gln Gly Val Arg Leu
    50                  55                  60

Ser Leu Gly Pro Leu Ser Pro Glu Lys Leu Glu Glu Ile Leu Asp Glu
65                  70                  75                  80

Ala Asn Arg Leu Ala Ala Gln Leu Glu Gln Cys Ala Leu Gln Asp Arg
                85                  90                  95

Glu Ser Ala Gly Glu Gly Leu Gly Pro Arg Arg Val Lys Pro Ser Pro
            100                 105                 110

Arg Arg Glu Thr Phe Val Leu Lys Asp Ser Pro Val Arg Asp Leu Leu
        115                 120                 125

Pro Thr Val Asn Ser Leu Thr Arg Ser Thr Pro Ser Pro Ser Ser Leu
    130                 135                 140

Thr Pro Arg Leu Arg Ser Asn Asp Arg Lys Gly Ser Val Arg Ala Leu
145                 150                 155                 160

Arg Ala Thr Ser Gly Lys Arg Pro Ser Asn Met Lys Arg Glu Ser Pro
                165                 170                 175

Thr Cys Asn Leu Phe Pro Ala Ser Lys Ser Pro Ala Ser Ser Pro Leu
            180                 185                 190

Thr Arg Ser Thr Pro Pro Val Arg Gly Arg Ala Gly Pro Ser Gly Arg
        195                 200                 205

Ala Ala Ala Ser Glu Glu Thr Arg Ala Ala Lys Leu Arg Ala Cys Gln
    210                 215                 220

Pro Asn Ala Thr His Gln Pro Glu Cys Ala Thr Trp Gln Arg Cys Pro
225                 230                 235                 240

Thr Ser Gly Phe Ser Val Asn Ser Lys Arg Ala Ser Lys Thr Lys His
                245                 250                 255

Cys Arg Thr Gln Ser Ala Gly Lys Trp Thr Gln Gly Ser Cys Phe Pro
            260                 265                 270

Ala Thr Lys Ser Ser Cys His Gly Cys His Ser Gln Gln Ser Ala Ala
        275                 280                 285

Pro Gln Glu Ser Gly Ser Pro Arg Thr Tyr Gln Val Lys Arg Ser Gly
    290                 295                 300

Gln Gln Ala Arg Leu Gln
305                 310

<210>  364
<211>  363
<212>  PRT
<213>  Homo sapiens

<400>  364

Met Glu Asp Leu Glu Glu Asp Val Arg Phe Ile Val Asp Glu Thr Leu
1               5                   10                  15

Asp Phe Gly Gly Leu Ser Pro Ser Asp Ser Arg Glu Glu Glu Asp Ile
            20                  25                  30

Thr Val Leu Val Thr Pro Glu Lys Pro Leu Arg Arg Gly Leu Ser His
        35                  40                  45

Arg Ser Asp Pro Asn Ala Val Ala Pro Ala Pro Gln Gly Val Arg Leu
    50                  55                  60

Ser Leu Gly Pro Leu Ser Pro Glu Lys Leu Glu Glu Ile Leu Asp Glu
65                  70                  75                  80

Ala Asn Arg Leu Ala Ala Gln Leu Glu Gln Cys Ala Leu Gln Asp Arg
                85                  90                  95

Glu Ser Ala Gly Glu Gly Leu Gly Pro Arg Arg Val Lys Pro Ser Pro
            100                 105                 110

Arg Arg Glu Thr Phe Val Leu Lys Asp Ser Pro Val Arg Asp Leu Leu
        115                 120                 125

Pro Thr Val Asn Ser Leu Thr Arg Ser Thr Pro Ser Pro Ser Ser Leu

130                 135                 140

Thr Pro Arg Leu Arg Ser Asn Asp Arg Lys Gly Ser Val Arg Ala Leu
145                 150                 155                 160

Arg Ala Thr Ser Gly Lys Arg Pro Ser Asn Met Lys Arg Glu Ser Pro
                165                 170                 175

Thr Cys Asn Leu Phe Pro Ala Ser Lys Ser Pro Ala Ser Ser Pro Leu
            180                 185                 190

Thr Arg Ser Thr Pro Pro Val Arg Gly Arg Ala Gly Pro Ser Gly Arg
        195                 200                 205

Ala Ala Ala Ser Glu Glu Thr Arg Ala Ala Lys Leu Arg Val Ser Gly
    210                 215                 220

Ser Gly Glu Phe Val Gly Leu Thr Leu Lys Phe Leu His Pro Ser Pro
225                 230                 235                 240

Pro Gly Pro Pro Thr Pro Ile Arg Ser Val Leu Ala Pro Gln Pro Ser
                245                 250                 255

Thr Ser Asn Ser Gln Arg Leu Pro Arg Pro Gln Gly Ala Ala Ala Lys
            260                 265                 270

Ser Ser Ser Gln Leu Pro Ile Pro Ser Ala Ile Pro Arg Pro Ala Ser
        275                 280                 285

Arg Met Pro Leu Thr Ser Arg Ser Val Pro Pro Gly Arg Gly Ala Leu
    290                 295                 300

Pro Pro Asp Ser Leu Ser Thr Arg Lys Gly Leu Pro Arg Pro Ser Thr
305                 310                 315                 320

Ala Gly His Arg Val Arg Glu Ser Gly His Lys Val Pro Val Ser Gln
                325                 330                 335

Arg Leu Asn Leu Pro Val Met Gly Ala Thr Arg Ser Asn Leu Gln Pro
            340                 345                 350

Pro Arg Lys Val Ala Val Pro Gly Pro Thr Arg
        355                 360

<210>  365
<211>  333
<212>  PRT
<213>  Homo sapiens

<400>  365

Met Glu Asp Leu Glu Glu Asp Val Arg Phe Ile Val Asp Glu Thr Leu
1               5                   10                  15

Asp Phe Gly Gly Leu Ser Pro Ser Asp Ser Arg Glu Glu Glu Asp Ile
            20                  25                  30

Thr Val Leu Val Thr Pro Glu Lys Pro Leu Arg Arg Gly Leu Ser His
        35                  40                  45

Arg Ser Asp Pro Asn Ala Val Ala Pro Ala Pro Gln Gly Val Arg Leu
    50                  55                  60

Ser Leu Gly Pro Leu Ser Pro Glu Lys Leu Glu Glu Ile Leu Asp Glu
65                  70                  75                  80

Ala Asn Arg Leu Ala Ala Gln Leu Glu Gln Cys Ala Leu Gln Asp Arg
                85                  90                  95

Glu Ser Ala Gly Glu Gly Leu Gly Pro Arg Arg Val Lys Pro Ser Pro
            100                 105                 110

Arg Arg Glu Thr Phe Val Leu Lys Asp Ser Pro Val Arg Asp Leu Leu
        115                 120                 125

Pro Thr Val Asn Ser Leu Thr Arg Ser Thr Pro Ser Pro Ser Ser Leu
    130                 135                 140

Thr Pro Arg Leu Arg Ser Asn Asp Arg Lys Gly Ser Val Arg Ala Leu
145                 150                 155                 160

Arg Ala Thr Ser Gly Lys Arg Pro Ser Asn Met Lys Arg Glu Ser Pro
                165                 170                 175

Thr Cys Asn Leu Phe Pro Ala Ser Lys Ser Pro Ala Ser Ser Pro Leu
            180                 185                 190

Thr Arg Ser Thr Pro Pro Val Arg Gly Arg Ala Gly Pro Ser Gly Arg
        195                 200                 205

Ala Ala Ala Ser Pro Pro Thr Pro Ile Arg Ser Val Leu Ala Pro Gln
    210                 215                 220

Pro Ser Thr Ser Asn Ser Gln Arg Leu Pro Arg Pro Gln Gly Ala Ala
225                 230                 235                 240

Ala Lys Ser Ser Ser Gln Leu Pro Ile Pro Ser Ala Ile Pro Arg Pro
                245                 250                 255

Ala Ser Arg Met Pro Leu Thr Ser Arg Ser Val Pro Pro Gly Arg Gly
            260                 265                 270

Ala Leu Pro Pro Asp Ser Leu Ser Thr Arg Lys Gly Leu Pro Arg Pro
        275                 280                 285

Ser Thr Ala Gly His Arg Val Arg Glu Ser Gly His Lys Val Pro Val
    290                 295                 300

Ser Gln Arg Leu Asn Leu Pro Val Met Gly Ala Thr Arg Ser Asn Leu
305                 310                 315                 320

Gln Pro Pro Arg Lys Val Ala Val Pro Gly Pro Thr Arg
                325                 330

<210>  366
<211>  333
<212>  PRT
<213>  Homo sapiens

<400>  366

Met Glu Asp Leu Glu Glu Asp Val Arg Phe Ile Val Asp Glu Thr Leu
1               5                   10                  15

Asp Phe Gly Gly Leu Ser Pro Ser Asp Ser Arg Glu Glu Glu Asp Ile
            20                  25                  30

Thr Val Leu Val Thr Pro Glu Lys Pro Leu Arg Arg Gly Leu Ser His
        35                  40                  45

Arg Ser Asp Pro Asn Ala Val Ala Pro Ala Pro Gln Gly Val Arg Leu
    50                  55                  60

Ser Leu Gly Pro Leu Ser Pro Glu Lys Leu Glu Glu Ile Leu Asp Glu
65                  70                  75                  80

Ala Asn Arg Leu Ala Ala Gln Leu Glu Gln Cys Ala Leu Gln Asp Arg
                85                  90                  95

Glu Ser Ala Gly Glu Gly Leu Gly Pro Arg Arg Val Lys Pro Ser Pro
            100                 105                 110

Arg Arg Glu Thr Phe Val Leu Lys Asp Ser Pro Val Arg Asp Leu Leu
        115                 120                 125

Pro Thr Val Asn Ser Leu Thr Arg Ser Thr Pro Ser Pro Ser Ser Leu
    130                 135                 140

Thr Pro Arg Leu Arg Ser Asn Asp Arg Lys Gly Ser Val Arg Ala Leu
145                 150                 155                 160

Arg Ala Thr Ser Gly Lys Arg Pro Ser Asn Met Lys Arg Glu Ser Pro
                165                 170                 175

Thr Cys Asn Leu Phe Pro Ala Ser Lys Ser Pro Ala Ser Ser Pro Leu
            180                 185                 190

Thr Arg Ser Thr Pro Pro Val Arg Gly Arg Ala Gly Pro Ser Gly Arg
        195                 200                 205

Ala Ala Ala Ser Pro Pro Thr Pro Ile Arg Ser Val Leu Ala Pro Gln

210                 215                 220

Pro Ser Thr Ser Asn Ser Gln Arg Leu Pro Arg Pro Gln Gly Ala Ala
225                 230                 235                 240

Ala Lys Ser Ser Ser Gln Leu Pro Ile Pro Ser Ala Ile Pro Arg Pro
                245                 250                 255

Ala Ser Arg Met Pro Leu Thr Ser Arg Ser Val Pro Pro Gly Arg Gly
            260                 265                 270

Ala Leu Pro Pro Asp Ser Leu Ser Thr Arg Lys Gly Leu Pro Arg Pro
        275                 280                 285

Ser Thr Ala Gly His Arg Val Arg Glu Ser Gly His Lys Val Pro Val
    290                 295                 300

Ser Gln Arg Leu Asn Leu Pro Val Met Gly Ala Thr Arg Ser Asn Leu
305                 310                 315                 320

Gln Pro Pro Arg Lys Val Ala Val Pro Gly Pro Thr Arg
                325                 330

<210>  367
<211>  444
<212>  PRT
<213>  Homo sapiens

<400>  367

<210>  368
<211>  3477
<212>  PRT
<213>  Homo sapiens

<400>  368
   Met Ala Asn Arg Arg Val Gly Arg Gly Cys Trp Glu Val Ser Pro Thr
   1               5                   10                  15
   Glu Arg Arg Pro Pro Ala Gly Leu Arg Gly Pro Ala Ala Glu Glu Glu
               20                  25                  30
   Ala Ser Ser Pro Pro Val Leu Ser Leu Ser His Phe Cys Arg Ser Pro
           35                  40                  45
   Phe Leu Cys Phe Gly Asp Val Leu Leu Gly Ala Ser Arg Thr Leu Ser
       50                  55                  60
   Leu Ala Leu Asp Asn Pro Asn Glu Glu Val Ala Glu Val Lys Ile Ser
   65                  70                  75                  80
   His Phe Pro Ala Ala Asp Leu Gly Phe Ser Val Ser Gln Arg Cys Phe
                   85                  90                  95
   Val Leu Gln Pro Lys Glu Lys Ile Val Ile Ser Val Asn Trp Thr Pro
               100                 105                 110
   Leu Lys Glu Gly Arg Val Arg Glu Ile Met Thr Phe Leu Val Asn Asp
           115                 120                 125
   Val Leu Lys His Gln Ala Ile Leu Leu Gly Asn Ala Glu Glu Gln Lys
       130                 135                 140
   Lys Lys Lys Arg Ser Leu Trp Asp Thr Ile Lys Lys Lys Lys Ile Ser
   145                 150                 155                 160
   Ala Ser Thr Ser His Asn Arg Arg Val Ser Asn Ile Gln Asn Val Asn
                   165                 170                 175
   Lys Thr Phe Ser Val Ser Gln Lys Val Asp Arg Val Arg Ser Pro Leu
               180                 185                 190
   Gln Ala Cys Glu Asn Leu Ala Met Asn Glu Gly Gly Pro Pro Thr Glu
           195                 200                 205
   Asn Asn Ser Leu Ile Leu Glu Glu Asn Lys Ile Pro Ile Ser Pro Ile
   210                 215                 220
   Ser Pro Ala Phe Asn Glu Cys His Gly Ala Thr Cys Leu Pro Leu Ser
   225                 230                 235                 240
   Val Arg Arg Ser Thr Thr Tyr Ser Ser Leu His Ala Ser Glu Asn Arg
                   245                 250                 255
   Glu Leu Leu Asn Val His Ser Ala Asn Val Ser Lys Val Ser Phe Asn
               260                 265                 270
   Glu Lys Ala Val Thr Glu Thr Ser Phe Asn Ser Val Asn Val Asn Gly
           275                 280                 285
   Gln Arg Gly Glu Asn Ser Lys Leu Ser Leu Thr Pro Asn Cys Ser Ser
       290                 295                 300
   Thr Leu Asn Ile Thr Gln Ser Gln Ile His Phe Leu Ser Pro Asp Ser
   305                 310                 315                 320
   Phe Val Asn Asn Ser His Gly Ala Asn Asn Glu Leu Glu Leu Val Thr
                   325                 330                 335
   Cys Leu Ser Ser Asp Met Phe Met Lys Asp Asn Ser Gln Pro Val His
               340                 345                 350
   Leu Glu Ser Thr Ile Ala His Glu Ile Tyr Gln Lys Ile Leu Ser Pro
           355                 360                 365
   Asp Ser Phe Ile Lys Asp Asn Tyr Gly Leu Asn Gln Asp Leu Glu Ser
       370                 375                 380
   Glu Ser Val Asn Pro Ile Leu Ser Pro Asn Gln Phe Leu Lys Asp Asn
   385                 390                 395                 400
   Met Ala Tyr Met Cys Thr Ser Gln Gln Thr Cys Lys Val Pro Leu Ser
                   405                 410                 415
   Asn Glu Asn Ser Gln Val Pro Gln Ser Pro Glu Asp Trp Arg Lys Ser
               420                 425                 430
   Glu Val Ser Pro Arg Ile Pro Glu Cys Gln Gly Ser Lys Ser Pro Lys
           435                 440                 445
   Ala Ile Phe Glu Glu Leu Val Glu Met Lys Ser Asn Tyr Tyr Ser Phe
       450                 455                 460
   Ile Lys Gln Asn Asn Pro Lys Phe Ser Ala Val Gln Asp Ile Ser Ser
   465                 470                 475                 480
   His Ser His Asn Lys Gln Pro Lys Arg Arg Pro Ile Leu Ser Ala Thr
                   485                 490                 495
   Val Thr Lys Arg Lys Ala Thr Cys Thr Arg Glu Asn Gln Thr Glu Ile
               500                 505                 510
   Asn Lys Pro Lys Ala Lys Arg Cys Leu Asn Ser Ala Val Gly Glu His
           515                 520                 525
   Glu Lys Val Ile Asn Asn Gln Lys Glu Lys Glu Asp Phe His Ser Tyr
       530                 535                 540
   Leu Pro Ile Ile Asp Pro Ile Leu Ser Lys Ser Lys Ser Tyr Lys Asn
   545                 550                 555                 560
   Glu Val Thr Pro Ser Ser Thr Thr Ala Ser Val Ala Arg Lys Arg Lys
                   565                 570                 575
   Ser Asp Gly Ser Met Glu Asp Ala Asn Val Arg Val Ala Ile Thr Glu
               580                 585                 590
   His Thr Glu Val Arg Glu Ile Lys Arg Ile His Phe Ser Pro Ser Glu
           595                 600                 605
   Pro Lys Thr Ser Ala Val Lys Lys Thr Lys Asn Val Thr Thr Pro Ile
       610                 615                 620
   Ser Lys Arg Ile Ser Asn Arg Glu Lys Leu Asn Leu Lys Lys Lys Thr
   625                 630                 635                 640
   Asp Leu Ser Ile Phe Arg Thr Pro Ile Ser Lys Thr Asn Lys Arg Thr
                   645                 650                 655
   Lys Pro Ile Ile Ala Val Ala Gln Ser Ser Leu Thr Phe Ile Lys Pro
               660                 665                 670
   Leu Lys Thr Asp Ile Pro Arg His Pro Met Pro Phe Ala Ala Lys Asn
           675                 680                 685
   Met Phe Tyr Asp Glu Arg Trp Lys Glu Lys Gln Glu Gln Gly Phe Thr
       690                 695                 700
   Trp Trp Leu Asn Phe Ile Leu Thr Pro Asp Asp Phe Thr Val Lys Thr
   705                 710                 715                 720
   Asn Ile Ser Glu Val Asn Ala Ala Thr Leu Leu Leu Gly Ile Glu Asn
                   725                 730                 735
   Gln His Lys Ile Ser Val Pro Arg Ala Pro Thr Lys Glu Glu Met Ser
               740                 745                 750
   Leu Arg Ala Tyr Thr Ala Arg Cys Arg Leu Asn Arg Leu Arg Arg Ala
           755                 760                 765
   Ala Cys Arg Leu Phe Thr Ser Glu Lys Met Val Lys Ala Ile Lys Lys
   770                 775                 780
   Leu Glu Ile Glu Ile Glu Ala Arg Arg Leu Ile Val Arg Lys Asp Arg
   785                 790                 795                 800
   His Leu Trp Lys Asp Val Gly Glu Arg Gln Lys Val Leu Asn Trp Leu
                   805                 810                 815
   Leu Ser Tyr Asn Pro Leu Trp Leu Arg Ile Gly Leu Glu Thr Thr Tyr
               820                 825                 830
   Gly Glu Leu Ile Ser Leu Glu Asp Asn Ser Asp Val Thr Gly Leu Ala
           835                 840                 845
   Met Phe Ile Leu Asn Arg Leu Leu Trp Asn Pro Asp Ile Ala Ala Glu
       850                 855                 860
   Tyr Arg His Pro Thr Val Pro His Leu Tyr Arg Asp Gly His Glu Glu
   865                 870                 875                 880
   Ala Leu Ser Lys Phe Thr Leu Lys Lys Leu Leu Leu Leu Val Cys Phe
                   885                 890                 895
   Leu Asp Tyr Ala Lys Ile Ser Arg Leu Ile Asp His Asp Pro Cys Leu
               900                 905                 910
   Phe Cys Lys Asp Ala Glu Phe Lys Ala Ser Lys Glu Ile Leu Leu Ala
           915                 920                 925
   Phe Ser Arg Asp Phe Leu Ser Gly Glu Gly Asp Leu Ser Arg His Leu
       930                 935                 940
   Gly Leu Leu Gly Leu Pro Val Asn His Val Gln Thr Pro Phe Asp Glu
   945                 950                 955                 960
   Phe Asp Phe Ala Val Thr Asn Leu Ala Val Asp Leu Gln Cys Gly Val
                   965                 970                 975
   Arg Leu Val Arg Thr Met Glu Leu Leu Thr Gln Asn Trp Asp Leu Ser
               980                 985                 990
   Lys Lys Leu Arg Ile Pro Ala Ile  Ser Arg Leu Gln Lys  Met His Asn
           995                 1000                 1005
   Val Asp  Ile Val Leu Gln Val  Leu Lys Ser Arg Gly  Ile Glu Leu
       1010                 1015                 1020
   Ser Asp  Glu His Gly Asn Thr  Ile Leu Ser Lys Asp  Ile Val Asp
       1025                 1030                 1035
   Arg His  Arg Glu Lys Thr Leu  Arg Leu Leu Trp Lys  Ile Ala Phe
       1040                 1045                 1050
   Ala Phe Gln Val Asp Ile Ser Leu Asn Leu Asp Gln Leu Lys Glu
   1055 1060 1065
   Glu Ile Ala Phe Leu Lys His Thr Lys Ser Ile Lys Lys Thr Ile
   1070 1075 1080
   Ser Leu Leu Ser Cys His Ser Asp Asp Leu Ile Asn Lys Lys Lys
   1085 1090 1095
   Gly Lys Arg Asp Ser Gly Ser Phe Glu Gln Tyr Ser Glu Asn Ile
   1100 1105 1110
   Lys Leu Leu Met Asp Trp Val Asn Ala Val Cys Ala Phe Tyr Asn
   1115 1120 1125
   Lys Lys Val Glu Asn Phe Thr Val Ser Phe Ser Asp Gly Arg Val
   1130 1135 1140
   Leu Cys Tyr Leu Ile His His Tyr His Pro Cys Tyr Val Pro Phe
   1145 1150 1155
   Asp Ala Ile Cys Gln Arg Thr Thr Gln Thr Val Glu Cys Thr Gln
   1160 1165 1170
   Thr Gly Ser Val Val Leu Asn Ser Ser Ser Glu Ser Asp Asp Ser
   1175 1180 1185
   Ser Leu Asp Met Ser Leu Lys Ala Phe Asp His Glu Asn Thr Ser
   1190 1195 1200
   Glu Leu Tyr Lys Glu Leu Leu Glu Asn Glu Lys Lys Asn Phe His
   1205 1210 1215
   Leu Val Arg Ser Ala Val Arg Asp Leu Gly Gly Ile Pro Ala Met
   1220 1225 1230
   Ile Asn His Ser Asp Met Ser Asn Thr Ile Pro Asp Glu Lys Val
   1235 1240 1245
   Val Ile Thr Tyr Leu Ser Phe Leu Cys Ala Arg Leu Leu Asp Leu
   1250 1255 1260
   Arg Lys Glu Ile Arg Ala Ala Arg Leu Ile Gln Thr Thr Trp Arg
   1265 1270 1275
   Lys Tyr Lys Leu Lys Thr Asp Leu Lys Arg His Gln Glu Arg Glu
   1280 1285 1290
   Lys Ala Ala Arg Ile Ile Gln Leu Ala Val Ile Asn Phe Leu Ala
   1295 1300 1305
   Lys Gln Arg Leu Arg Lys Arg Val Asn Ala Ala Leu Val Ile Gln
   1310                1315                1320
   Lys Tyr Trp Arg Arg Val Leu Ala Gln Arg Lys Leu Leu Met Leu
       1325                1330                1335
   Lys Lys Glu Lys Leu Glu Lys Val Gln Asn Lys Ala Ala Ser Leu
       1340                1345                1350
   Ile Gln Gly Tyr Trp Arg Arg Tyr Ser Thr Arg Gln Arg Phe Leu
       1355                1360                1365
   Lys Leu Lys Tyr Tyr Ser Ile Ile Leu Gln Ser Arg Ile Arg Met
       1370                1375                1380
   Ile Ile Ala Val Thr Ser Tyr Lys Arg Tyr Leu Trp Ala Thr Val
       1385                1390                1395
   Thr Ile Gln Arg His Trp Arg Ala Tyr Leu Arg Arg Lys Gln Asp
       1400                1405                1410
   Gln Gln Arg Tyr Glu Met Leu Lys Ser Ser Thr Leu Ile Ile Gln
       1415                1420                1425
   Ser Met Phe Arg Lys Trp Lys Gln Arg Lys Met Gln Ser Gln Val
       1430                1435                1440
   Lys Ala Thr Val Ile Leu Gln Arg Ala Phe Arg Glu Trp His Leu
       1445                1450                1455
   Arg Lys Gln Ala Lys Glu Glu Asn Ser Ala Ile Ile Ile Gln Ser
       1460                1465                1470
   Trp Tyr Arg Met His Lys Glu Leu Arg Lys Tyr Ile Tyr Ile Arg
       1475                1480                1485
   Ser Cys Val Val Ile Ile Gln Lys Arg Phe Arg Cys Phe Gln Ala
       1490                1495                1500
   Gln Lys Leu Tyr Lys Arg Arg Lys Glu Ser Ile Leu Thr Ile Gln
       1505                1510                1515
   Lys Tyr Tyr Lys Ala Tyr Leu Lys Gly Lys Ile Glu Arg Thr Asn
       1520                1525                1530
   Tyr Leu Gln Lys Arg Ala Ala Ala Ile Gln Leu Gln Ala Ala Phe
       1535                1540                1545
   Arg Arg Leu Lys Ala His Asn Leu Cys Arg Gln Ile Arg Ala Ala
       1550                1555                1560
   Cys Val Ile Gln Ser Tyr Trp Arg Met Arg Gln Asp Arg Val Arg
       1565                1570                1575
   Phe Leu Asn Leu Lys Lys Thr Ile Ile Lys Phe Gln Ala His Val
   1580 1585 1590
   Arg Lys His Gln Gln Arg Gln Lys Tyr Lys Lys Met Lys Lys Ala
   1595 1600 1605
   Ala Val Ile Ile Gln Thr His Phe Arg Ala Tyr Ile Phe Ala Met
   1610 1615 1620
   Lys Val Leu Ala Ser Tyr Gln Lys Thr Arg Ser Ala Val Ile Val
   1625 1630 1635
   Leu Gln Ser Ala Tyr Arg Gly Met Gln Ala Arg Lys Met Tyr Ile
   1640 1645 1650
   His Ile Leu Thr Ser Val Ile Lys Ile Gln Ser Tyr Tyr Arg Ala
   1655 1660 1665
   Tyr Val Ser Lys Lys Glu Phe Leu Ser Leu Lys Asn Ala Thr Ile
   1670 1675 1680
   Lys Leu Gln Ser Thr Val Lys Met Lys Gln Thr Arg Lys Gln Tyr
   1685 1690 1695
   Leu His Leu Arg Ala Ala Ala Leu Phe Ile Gln Gln Cys Tyr Arg
   1700 1705 1710
   Ser Lys Lys Ile Ala Ala Gln Lys Arg Glu Glu Tyr Met Gln Met
   1715 1720 1725
   Arg Glu Ser Cys Ile Lys Leu Gln Ala Phe Val Arg Gly Tyr Leu
   1730 1735 1740
   Val Arg Lys Gln Met Arg Leu Gln Arg Lys Ala Val Ile Ser Leu
   1745 1750 1755
   Gln Ser Tyr Phe Arg Met Arg Lys Ala Arg Gln Tyr Tyr Leu Lys
   1760 1765 1770
   Met Tyr Lys Ala Ile Ile Val Ile Gln Asn Tyr Tyr His Ala Tyr
   1775 1780 1785
   Lys Ala Gln Val Asn Gln Arg Lys Asn Phe Leu Gln Val Lys Lys
   1790 1795 1800
   Ala Ala Thr Cys Leu Gln Ala Ala Tyr Arg Gly Tyr Lys Val Arg
   1805 1810 1815
   Gln Leu Ile Lys Gln Gln Ser Ile Ala Ala Leu Lys Ile Gln Ser
   1820 1825 1830
   Ala Phe Arg Gly Tyr Asn Lys Arg Val Lys Tyr Gln Ser Val Leu
   1835                 1840                 1845
   Gln Ser Ile Ile Lys Ile Gln Arg Trp Tyr Arg Ala Tyr Lys Thr
       1850                 1855                 1860
   Leu His Asp Thr Arg Thr His Phe Leu Lys Thr Lys Ala Ala Val
       1865                 1870                 1875
   Ile Ser Leu Gln Ser Ala Tyr Arg Gly Trp Lys Val Arg Lys Gln
       1880                 1885                 1890
   Ile Arg Arg Glu His Gln Ala Ala Leu Lys Ile Gln Ser Ala Phe
       1895                 1900                 1905
   Arg Met Ala Lys Ala Gln Lys Gln Phe Arg Leu Phe Lys Thr Ala
       1910                 1915                 1920
   Ala Leu Val Ile Gln Gln Asn Phe Arg Ala Trp Thr Ala Gly Arg
       1925                 1930                 1935
   Lys Gln Cys Met Glu Tyr Ile Glu Leu Arg His Ala Val Leu Val
       1940                 1945                 1950
   Leu Gln Ser Met Trp Lys Gly Lys Thr Leu Arg Arg Gln Leu Gln
       1955                 1960                 1965
   Arg Gln His Lys Cys Ala Ile Ile Ile Gln Ser Tyr Tyr Arg Met
       1970                 1975                 1980
   His Val Gln Gln Lys Lys Trp Lys Ile Met Lys Lys Ala Ala Leu
       1985                 1990                 1995
   Leu Ile Gln Lys Tyr Tyr Arg Ala Tyr Ser Ile Gly Arg Glu Gln
       2000                 2005                 2010
   Asn His Leu Tyr Leu Lys Thr Lys Ala Ala Val Val Thr Leu Gln
       2015                 2020                 2025
   Ser Ala Tyr Arg Gly Met Lys Val Arg Lys Arg Ile Lys Asp Cys
       2030                 2035                 2040
   Asn Lys Ala Ala Val Thr Ile Gln Ser Lys Tyr Arg Ala Tyr Lys
       2045                 2050                 2055
   Thr Lys Lys Lys Tyr Ala Thr Tyr Arg Ala Ser Ala Ile Ile Ile
       2060                 2065                 2070
   Gln Arg Trp Tyr Arg Gly Ile Lys Ile Thr Asn His Gln His Lys
       2075                 2080                 2085
   Glu Tyr Leu Asn Leu Lys Lys Thr Ala Ile Lys Ile Gln Ser Val
       2090                 2095                 2100
   Tyr Arg Gly Ile Arg Val Arg Arg His Ile Gln His Met His Arg
   2105 2110 2115
   Ala Ala Thr Phe Ile Lys Ala Met Phe Lys Met His Gln Ser Arg
   2120 2125 2130
   Ile Ser Tyr His Thr Met Arg Lys Ala Ala Ile Val Ile Gln Val
   2135 2140 2145
   Arg Cys Arg Ala Tyr Tyr Gln Gly Lys Met Gln Arg Glu Lys Tyr
   2150 2155 2160
   Leu Thr Ile Leu Lys Ala Val Lys Val Leu Gln Ala Ser Phe Arg
   2165 2170 2175
   Gly Val Arg Val Arg Arg Thr Leu Arg Lys Met Gln Thr Ala Ala
   2180 2185 2190
   Thr Leu Ile Gln Ser Asn Tyr Arg Arg Tyr Arg Gln Gln Thr Tyr
   2195 2200 2205
   Phe Asn Lys Leu Lys Lys Ile Thr Lys Thr Val Gln Gln Arg Tyr
   2210 2215 2220
   Trp Ala Met Lys Glu Arg Asn Ile Gln Phe Gln Arg Tyr Asn Lys
   2225 2230 2235
   Leu Arg His Ser Val Ile Tyr Ile Gln Ala Ile Phe Arg Gly Lys
   2240 2245 2250
   Lys Ala Arg Arg His Leu Lys Met Met His Ile Ala Ala Thr Leu
   2255 2260 2265
   Ile Gln Arg Arg Phe Arg Thr Leu Met Met Arg Arg Arg Phe Leu
   2270 2275 2280
   Ser Leu Lys Lys Thr Ala Ile Leu Ile Gln Arg Lys Tyr Arg Ala
   2285 2290 2295
   His Leu Cys Thr Lys His His Leu Gln Phe Leu Gln Val Gln Asn
   2300 2305 2310
   Ala Val Ile Lys Ile Gln Ser Ser Tyr Arg Arg Trp Met Ile Arg
   2315 2320 2325
   Lys Arg Met Arg Glu Met His Arg Ala Ala Thr Phe Ile Gln Ser
   2330 2335 2340
   Thr Phe Arg Met His Arg Leu His Met Arg Tyr Arg Ala Leu Lys
   2345 2350 2355
   Gln Ala Ser Val Val Ile Gln Gln Gln Tyr Gln Ala Asn Arg Ala
   2360                2365                2370
   Ala Lys Leu Gln Arg Gln His Tyr Leu Arg Gln Arg His Ser Ala
       2375                2380                2385
   Val Ile Leu Gln Ala Ala Phe Arg Gly Met Lys Thr Arg Arg His
       2390                2395                2400
   Leu Lys Ser Met His Ser Ser Ala Thr Leu Ile Gln Ser Arg Phe
       2405                2410                2415
   Arg Ser Leu Leu Val Arg Arg Arg Phe Ile Ser Leu Lys Lys Ala
       2420                2425                2430
   Thr Ile Phe Val Gln Arg Lys Tyr Arg Ala Thr Ile Cys Ala Lys
       2435                2440                2445
   His Lys Leu Tyr Gln Phe Leu His Leu Arg Lys Ala Ala Ile Thr
       2450                2455                2460
   Ile Gln Ser Ser Tyr Arg Arg Leu Met Val Lys Lys Lys Leu Gln
       2465                2470                2475
   Glu Met Gln Arg Ala Ala Val Leu Ile Gln Ala Thr Phe Arg Met
       2480                2485                2490
   His Arg Thr Tyr Ile Thr Phe Gln Thr Trp Lys His Ala Ser Ile
       2495                2500                2505
   Leu Ile Gln Gln His Tyr Arg Thr Tyr Arg Ala Ala Lys Leu Gln
       2510                2515                2520
   Arg Glu Asn Tyr Ile Arg Gln Trp His Ser Ala Val Val Ile Gln
       2525                2530                2535
   Ala Ala Tyr Lys Gly Met Lys Ala Arg Gln Leu Leu Arg Glu Lys
       2540                2545                2550
   His Lys Ala Ser Ile Val Ile Gln Ser Thr Tyr Arg Met Tyr Arg
       2555                2560                2565
   Gln Tyr Cys Phe Tyr Gln Lys Leu Gln Trp Ala Thr Lys Ile Ile
       2570                2575                2580
   Gln Glu Lys Tyr Arg Ala Asn Lys Lys Lys Gln Lys Val Phe Gln
       2585                2590                2595
   His Asn Glu Leu Lys Lys Glu Thr Cys Val Gln Ala Gly Phe Gln
       2600                2605                2610
   Asp Met Asn Ile Lys Lys Gln Ile Gln Glu Gln His Gln Ala Ala
       2615                2620                2625
   Ile Ile Ile Gln Lys His Cys Lys Ala Phe Lys Ile Arg Lys His
   2630 2635 2640
   Tyr Leu His Leu Arg Ala Thr Val Val Ser Ile Gln Arg Arg Tyr
   2645 2650 2655
   Arg Lys Leu Thr Ala Val Arg Thr Gln Ala Val Ile Cys Ile Gln
   2660 2665 2670
   Ser Tyr Tyr Arg Gly Phe Lys Val Arg Lys Asp Ile Gln Asn Met
   2675 2680 2685
   His Arg Ala Ala Thr Leu Ile Gln Ser Phe Tyr Arg Met His Arg
   2690 2695 2700
   Ala Lys Val Asp Tyr Glu Thr Lys Lys Thr Ala Ile Val Val Ile
   2705 2710 2715
   Gln Asn Tyr Tyr Arg Leu Tyr Val Arg Val Lys Thr Glu Arg Lys
   2720 2725 2730
   Asn Phe Leu Ala Val Gln Lys Ser Val Arg Thr Ile Gln Ala Ala
   2735 2740 2745
   Phe Arg Gly Met Lys Val Arg Gln Lys Leu Lys Asn Val Ser Glu
   2750 2755 2760
   Glu Lys Met Ala Ala Ile Val Asn Gln Ser Ala Leu Cys Cys Tyr
   2765 2770 2775
   Arg Ser Lys Thr Gln Tyr Glu Ala Val Gln Ser Glu Gly Val Met
   2780 2785 2790
   Ile Gln Glu Trp Tyr Lys Ala Ser Gly Leu Ala Cys Ser Gln Glu
   2795 2800 2805
   Ala Glu Tyr His Ser Gln Ser Arg Ala Ala Val Thr Ile Gln Lys
   2810 2815 2820
   Ala Phe Cys Arg Met Val Thr Arg Lys Leu Glu Thr Gln Lys Cys
   2825 2830 2835
   Ala Ala Leu Arg Ile Gln Phe Phe Leu Gln Met Ala Val Tyr Arg
   2840 2845 2850
   Arg Arg Phe Val Gln Gln Lys Arg Ala Ala Ile Thr Leu Gln His
   2855 2860 2865
   Tyr Phe Arg Thr Trp Gln Thr Arg Lys Gln Phe Leu Leu Tyr Arg
   2870 2875 2880
   Lys Ala Ala Val Val Leu Gln Asn His Tyr Arg Ala Phe Leu Ser
   2885                    2890                    2895
   Ala Lys His Gln Arg Gln Val Tyr Leu Gln Ile Arg Ser Ser Val
       2900                    2905                    2910
   Ile Ile Ile Gln Ala Arg Ser Lys Gly Phe Ile Gln Lys Arg Lys
       2915                    2920                    2925
   Phe Gln Glu Ile Lys Asn Ser Thr Ile Lys Ile Gln Ala Met Trp
       2930                    2935                    2940
   Arg Arg Tyr Arg Ala Lys Lys Tyr Leu Cys Lys Val Lys Ala Ala
       2945                    2950                    2955
   Cys Lys Ile Gln Ala Trp Tyr Arg Cys Trp Arg Ala His Lys Glu
       2960                    2965                    2970
   Tyr Leu Ala Ile Leu Lys Ala Val Lys Ile Ile Gln Gly Cys Phe
       2975                    2980                    2985
   Tyr Thr Lys Leu Glu Arg Thr Arg Phe Leu Asn Val Arg Ala Ser
       2990                    2995                    3000
   Ala Ile Ile Ile Gln Arg Lys Trp Arg Ala Ile Leu Pro Ala Lys
       3005                    3010                    3015
   Ile Ala His Glu His Phe Leu Met Ile Lys Arg His Arg Ala Ala
       3020                    3025                    3030
   Cys Leu Ile Gln Ala His Tyr Arg Gly Tyr Lys Gly Arg Gln Val
       3035                    3040                    3045
   Phe Leu Arg Gln Lys Ser Ala Ala Leu Ile Ile Gln Lys Tyr Ile
       3050                    3055                    3060
   Arg Ala Arg Glu Ala Gly Lys His Glu Arg Ile Lys Tyr Ile Glu
       3065                    3070                    3075
   Phe Lys Lys Ser Thr Val Ile Leu Gln Ala Leu Val Arg Gly Trp
       3080                    3085                    3090
   Leu Val Arg Lys Arg Phe Leu Glu Gln Arg Ala Lys Ile Arg Leu
       3095                    3100                    3105
   Leu His Phe Thr Ala Ala Ala Tyr Tyr His Leu Asn Ala Val Arg
       3110                    3115                    3120
   Ile Gln Arg Ala Tyr Lys Leu Tyr Leu Ala Val Lys Asn Ala Asn
       3125                    3130                    3135
   Lys Gln Val Asn Ser Val Ile Cys Ile Gln Arg Trp Phe Arg Ala
       3140                    3145                    3150
   Arg Leu Gln Glu Lys Arg Phe Ile Gln Lys Tyr His Ser Ile Lys
   3155 3160 3165
   Lys Ile Glu His Glu Gly Gln Glu Cys Leu Ser Gln Arg Asn Arg
   3170 3175 3180
   Ala Ala Ser Val Ile Gln Lys Ala Val Arg His Phe Leu Leu Arg
   3185 3190 3195
   Lys Lys Gln Glu Lys Phe Thr Ser Gly Ile Ile Lys Ile Gln Ala
   3200 3205 3210
   Leu Trp Arg Gly Tyr Ser Trp Arg Lys Lys Asn Asp Cys Thr Lys
   3215 3220 3225
   Ile Lys Ala Ile Arg Leu Ser Leu Gln Val Val Asn Arg Glu Ile
   3230 3235 3240
   Arg Glu Glu Asn Lys Leu Tyr Lys Arg Thr Ala Leu Ala Leu His
   3245 3250 3255
   Tyr Leu Leu Thr Tyr Lys His Leu Ser Ala Ile Leu Glu Ala Leu
   3260 3265 3270
   Lys His Leu Glu Val Val Thr Arg Leu Ser Pro Leu Cys Cys Glu
   3275 3280 3285
   Asn Met Ala Gln Ser Gly Ala Ile Ser Lys Ile Phe Val Leu Ile
   3290 3295 3300
   Arg Ser Cys Asn Arg Ser Ile Pro Cys Met Glu Val Ile Arg Tyr
   3305 3310 3315
   Ala Val Gln Val Leu Leu Asn Val Ser Lys Tyr Glu Lys Thr Thr
   3320 3325 3330
   Ser Ala Val Tyr Asp Val Glu Asn Cys Ile Asp Ile Leu Leu Glu
   3335 3340 3345
   Leu Leu Gln Ile Tyr Arg Glu Lys Pro Gly Asn Lys Val Ala Asp
   3350 3355 3360
   Lys Gly Gly Ser Ile Phe Thr Lys Thr Cys Cys Leu Leu Ala Ile
   3365 3370 3375
   Leu Leu Lys Thr Thr Asn Arg Ala Ser Asp Val Arg Ser Arg Ser
   3380 3385 3390
   Lys Val Val Asp Arg Ile Tyr Ser Leu Tyr Lys Leu Thr Ala His
   3395 3400 3405
   Lys His Lys Met Asn Thr Glu Arg Ile Leu Tyr Lys Gln Lys Lys
   3410                 3415                 3420
   Asn Ser Ser Ile Ser Ile Pro Phe Ile Pro Glu Thr Pro Val Arg
       3425                 3430                 3435
   Thr Arg Ile Val Ser Arg Leu Lys Pro Asp Trp Val Leu Arg Arg
       3440                 3445                 3450
   Asp Asn Met Glu Glu Ile Thr Asn Pro Leu Gln Ala Ile Gln Met
       3455                 3460                 3465
   Val Met Asp Thr Leu Gly Ile Pro Tyr
       3470                 3475

## Description

The present invention relates to a composition of nucleic acids useful as markers for the response to a multimodal therapeutic strategy in ovarian cancer, to a composition of nucleic acids useful as markers for different tumor types of ovarian carcinoma and useful as markers for predicting the outcome of a surgical procedure in ovarian cancer and assessment of patient prognosis. The present invention also relates to a matrix on which such nucleic acid compositions have been immobilized. Furthermore, the present invention relates to uses of such nucleic acid compositions. Moreover, the present invention relates to compositions of proteins encoded by said nucleic acid compositions, and also relates to kits comprising such nucleic acid compositions, matrices and/or protein compositions. Furthermore, the present invention relates to a method of classifying a patient with ovarian cancer as belonging to a group amenable or not amenable to surgical procedure, and to a method of predicting the outcome of such surgical procedure.

Cancer is and remains one of the leading causes of mortality throughout the world. Various treatments are available, but none of these can be considered as providing a general cure for the disease. Cancer manifests itself in various organs, including lung, liver, intestines, reproductive organs, skin etc. Of these, ovarian carcinoma represents the gynecologic tumor with the highest rate of mortality despite advances in diagnosis and treatments. Numerous attempts have been made to characterize ovarian carcinoma in terms of gene expression patterns (see for example Peiró et al, American Journal of Clinical Pathology, 2002, 118(6):922-929). One of the possibilities of treating ovarian carcinoma is a classical surgical approach, whereby the bulk of the tumor is physically removed by surgical means. However, the outcome of such surgical procedure is often not clear and frequently cannot be predicted. In many instances, an ovarian cancer tumor is not operable in the sense that there is tumor tissue remaining at the sight of surgery after the surgical procedure. In a study by Berchuck et al (American Journal of Obstetrics and Gynecology (2004), 190, 910-925), an attempt was made to identify gene expression signatures that correlate with the success of a surgical therapy. The authors of this study used supervised statistical approaches and identified an expression pattern of 32 genes which showed some correlation with the success of a surgical intervention. However, the prediction accuracy of success using such expression pattern was only 72.7%.

Accordingly, it was an object of the present invention to provide for means that allow a more accurate prediction of the outcome of surgical procedure. Moreover, it is an object of the present invention to provide for means allowing the molecular classification of ovarian cancer tissue into either a type amenable to surgical procedure, or a type not amenable to surgical procedure. Moreover, it was an object of the present invention to provide means that allow the treating physician to make a decision as to whether a patient should be treated by surgical procedures at all, or whether such patient should rather be treated by non-invasive methods, such as administration of anti-cancer-drugs, possibly in combination with surgical procedures.

All the objects of the present invention are solved by a composition of nucleic acids comprising at least two different nucleic acids having nucleic acid sequences selected from the group comprising SEQ ID NO:1-184 and/or the complementary counterparts thereof, and/or partial sequences thereof comprising 18 contiguous nucleotides or more and/or the complementary counterparts thereof hybridizing to said nucleic acids having nucleic acid sequences selected from the group comprising SEQ ID NO:1-184.

In preferred embodiments, said composition of nucleic acids comprises at least three, preferably at least five, more preferably at least 10, more preferably at least 20, even more preferably at least 50, even more preferably at least 100, and most preferably at least 184 different nucleic acids having nucleic acid sequences selected from the group comprising SEQ ID NO:1-184 and/or the complementary counterparts thereof, and/or partial sequences thereof comprising 18 contiguous nucleotides or more and/or the complementary counterparts thereof hybridizing to said nucleic acids having nucleic acid sequences selected from the group comprising SEQ ID NO:1-184.

The objects of the present invention are also solved by a matrix, preferably a microarray, having a surface on which a nucleic acid composition according to the present invention has been immobilized.

The objects of the present invention are also solved by an in-vitro-use of the composition according to the present invention, and/or the matrix according to the present invention for classifying ovarian cancer tissue as belonging to a first or second type of two different types of ovarian cancer, said first type of ovarian cancer being characterized in that it is amenable to a surgical procedure such that after said surgical procedure there is no macroscopically detectable tumor tissue remaining in the body of a patient affected by ovarian cancer, and said second type of ovarian cancer being characterized in that it is not amenable to a surgical procedure such that after said surgical procedure there is a macroscopically detectable tumor tissue remaining in the body of a patient affected by ovarian cancer.

In one embodiment, the in-vitro-use according to the present invention is for predicting the outcome of a surgical procedure in a patient affected by ovarian cancer, said outcome being a complete removal of all ovarian cancer tissue from the patient's body as a result of said surgical procedure, if said patient is classified as being affected by said first type of ovarian cancer, and said outcome being a remaining of ovarian cancer tissue in the patient's body as a result of said surgical procedure, if said patient is classified as being affected by said second type of ovarian cancer.

Preferably, the in-vitro-use according to the present invention is for predicting the patient prognosis of a patient affected by ovarian cancer, said prognosis being related to the time from surgery to recurrent disease or death of the patient.

Preferably, the in-vitro-use according to the present invention comprises:
- providing a sample of ovarian cancer tissue of a first patient having ovarian cancer of a type unidentified in respect of its amenability to a surgical procedure,
- providing a sample of ovarian cancer tissue of a second patient having ovarian cancer of said first or said second type,
- detecting first expression levels of said nucleic acids in said sample of said first patient,
- detecting second expression levels of said nucleic acids in said sample of said second patient,
- comparing said first expression levels with said second expression levels of said nucleic acids in said sample taken from said second patient affected by ovarian cancer of said first or said second type,
- determining differences between said first and said second expression levels and classifying said first patient as having ovarian cancer belonging to the type opposite to the ovarian cancer of said second patient, if there are differences between said first and said second expression levels, or classifying said first patient as having ovarian cancer belonging to the same type of ovarian cancer as said ovarian cancer of said second patient, if there are no differences between said first and said second expression levels.

In a preferred embodiment, the in-vitro-use according to the present invention comprises:
- providing a sample of ovarian cancer tissue of a test patient having ovarian cancer of a type unidentified in respect of its amenability to a surgical procedure,
- providing a collection of samples of ovarian cancer tissues of patients having ovarian cancer of said first type,
- providing a collection of samples of ovarian cancer tissues of patients having ovarian cancer of said second type,
- detecting expression levels of said nucleic acids in said sample of said test patient,
- detecting expression levels of said nucleic acids in said collections of samples of ovarian cancer tissues of patients having ovarian cancer of said first and second type, respectively,
- comparing said expression levels of said nucleic acids in said collection of samples of patients affected by ovarian cancer of said first type with said expression levels of said nucleic acids in said collection of samples of patients affected by ovarian cancer of said second type,
- fitting a classification model to said expression levels of said nucleic acids in said collection of samples of patients affected by ovarian cancer of said first and said second type, with machine learning algorithms, preferably with one of the following methods: support vector machines (SVM), linear discriminate analysis (LDA), or nearest-mean-classification (NMC)
- classifying said test patient as having ovarian cancer of said first type or said second type, based on said classification model.

Preferably, said expression levels are detected at the mRNA-stage, cDNA stage or the protein stage.

In one embodiment, wherein differences between said first and said second expression levels are detected, said differences are a fold change in expression level of said nucleic acids > 1.3 or < -1.2, wherein preferably, said fold change is in the range of from 1.3 to 6.4 and/or in the range of from -1.2 to -7.2.

In one embodiment, the in-vitro-use according to the present invention is for classifying a patient having ovarian cancer as requiring different types of chemotherapy based on the patient's ovarian cancer type, characterized in that a patient having ovarian cancer of said first type may be treated by or may require standard chemotherapy, alone or in combination with a surgical procedure, said standard chemotherapy preferably being an adjuvant chemotherapy, more preferably an administration of one or several of carboplatin, taxol, even more preferably a combination of both carboplatin and taxol, and a patient having ovarian cancer of said second type may be treated or may require a modified chemotherapy, alone or in combination with a surgical procedure ,said modified chemotherapy being a chemotherapy different from said standard chemotherapy, preferably a neoadjuvant preoperative chemotherapy.

In one embodiment, the in-vitro-use according to the present invention is for classifying a patient having ovarian cancer as requiring different types of adjuvant therapy based on the patient's ovarian cancer type, characterized in that a patient having ovarian cancer of said first type may be treated by or may require standard chemotherapy after a surgical procedure, and a patient having ovarian cancer of said second type may be treated or may require an additional therapy using molecular targeted substances tailored to inhibit or upregulate target nucleic acids or proteins selected from the group comprising SEQ ID NO:1-184, and SEQ ID NO: 185-368.

The objects of the present invention are also solved by a composition of proteins being encoded by a composition of nucleic acids according to the present invention, said nucleic acids having nucleic acid sequences selected from the group comprising SEQ ID NO:1-184, characterized in that said composition of proteins comprises at least two different proteins having sequences selected from the group comprising SEQ ID NO:185-368.

In preferred embodiments, the composition of proteins according to the present invention comprises at least three, preferably at least 5, more preferably at least 10, more pref erably at least 20, even more preferably at least 50, even more preferably at least 100, and most preferably at least 184 different proteins having sequences selected from the group comprising SEQ ID NO:185-368.

The objects of the present invention are also solved by a composition of antibodies, each antibody raised against a different target protein of said protein composition according to the present invention.

The objects of the present invention are also solved by an in-vitro-use of a composition of antibodies according to the present invention for classifying ovarian cancer tissue as belonging to a first or a second type of two different types of ovarian cancer, said first type of ovarian cancer being characterized in that it is amenable to a surgical procedure such that after said surgical procedure there is no macroscopically detectable tumor tissue remaining in the body of a patient affected by ovarian cancer, and said second type of ovarian cancer being characterized in that it is not amenable to a surgical procedure such that after said surgical procedure there is a macroscopically detectable tumor tissue remaining in the body of a patient affected by ovarian cancer.

In one embodiment, the in-vitro-use of the composition of antibodies is for predicting the outcome of a surgical procedure in a patient affected by ovarian cancer, said outcome being a complete removal of all ovarian cancer tissue from the patient's body, to a macroscopically detectable extent, as a result of said surgical procedure, if said patient is classified as being affected by said first type of ovarian cancer, and said outcome being a remaining of ovarian cancer tissue in the patient's body, to a macroscopically detectable extent, as a result of said surgical procedure, if said patient is classified as being affected by said second type of ovarian cancer.

In one embodiment, the in-vitro-use of the composition of antibodies is for predicting the patient prognosis of a patient affected by ovarian cancer, said prognosis being related to the time from surgery to recurrent disease or death of the patient.

In one embodiment, the in-vitro-use of the composition of antibodies comprises
- providing a sample of ovarian cancer tissue of a first patient having ovarian cancer of a type unidentified in respect of its amenability to a surgical procedure,
- providing a sample of ovarian cancer tissue of a second patient having ovarian cancer of said first or said second type,
- detecting first expression levels of said target proteins in said sample of said first patient, using said composition of antibodies,
- detecting second expression levels of said target proteins in said sample of said second patient, using said composition of antibodies
- comparing said first expression levels with said second expression levels of said target proteins in said sample taken from said second patient affected by ovarian cancer of said first or said second type,
- determining differences between said first and said second expression levels and classifying said first patient as having ovarian cancer belonging to the type opposite to the ovarian cancer of said second patient, if there are differences between said first and said second expression levels, or classifying said first patient having ovarian cancer belonging to the same type of ovarian cancer as said ovarian cancer of said second patient, if there are no differences between said first and said second expression levels.

In one embodiment, the in-vitro-use of the composition of antibodies comprises:
- providing a sample of ovarian cancer tissue of a test patient having ovarian cancer of a type unidentified in respect of its amenability to a surgical procedure,
- providing a collection of samples of ovarian cancer tissues of patients having ovarian cancer of said first type,
- providing a collection of samples of ovarian cancer tissues of patients having ovarian cancer of said second type,
- detecting expression levels of said target proteins in said sample of said test patient,
- detecting expression levels of said target proteins in said collections of samples of ovarian cancer tissues of patients having ovarian cancer of said first and second type, respectively,
- comparing said expression levels of said target proteins in said collection of samples of patients affected by ovarian cancer of said first type with said expression levels of said target proteins in said collection of samples of patients affected by ovarian cancer of said second type,
- fitting a classification model to said expression levels of said target proteins in said collection of samples of patients affected by ovarian cancer of said first and said second type, with machine learning algorithms, preferably with one of the following methods: support vector machines (SVM), linear discriminate analysis (LDA), or nearest-mean-classification (NMC)
- classifying said test patient as having ovarian cancer of said first type or said second type, based on said classification model.

In one embodiment, the in-vitro-use of the composition of antibodies is for classifying a patient having ovarian cancer as requiring different types of chemotherapy based on the patient's ovarian cancer type, characterized in that a patient having ovarian cancer of said first type may be treated by or may require standard chemotherapy, alone or in combination with a surgical procedure, said standard chemotherapy preferably being an adjuvant chemotherapy, more preferably an administration of one or several of carboplatin, taxol, even more preferably a combination of both carboplatin and taxol, and a patient having ovarian cancer of said second type may be treated or may require a modified chemotherapy, alone or in combination with a surgical procedure, said modified chemotherapy being different from said standard chemotherapy, preferably a neoadjuvant preoperative chemotherapy.

In one embodiment, the in-vitro-use of the composition of antibodies is for classifying a patient having ovarian cancer as requiring different types of adjuvant therapy based on the patient's ovarian cancer type, characterized in that a patient having ovarian cancer of said first type may be treated by or may require standard chemotherapy after a surgical procedure, and a patient having ovarian cancer of said second type may be treated by or may require an additional therapy using molecular targeted substances tailored to inhibit or upregulate target nucleic acids or proteins selected from the group comprising SEQ ID NO:1-184, and SEQ ID NO:185-368.

The objects of the present invention are also solved by an in-vitro-method of classifying a patient with ovarian cancer as having ovarian cancer of a first type or a second type, said first type of ovarian cancer being characterized in that it is amenable to a surgical procedure such that after said surgical procedure there is no macroscopically detectable tumor tissue remaining in the body of a patient affected by ovarian cancer, and said second type of ovarian cancer being characterized in that it is not amenable to a surgical procedure such that after said surgical procedure there is a macroscopically detectable tumor tissue remaining in the body of a patient affected by ovarian cancer, -using the nucleic acid composition, and/or the matrix, and/or the composition of antibodies according to the present invention

In one embodiment said method is for predicting the outcome of a surgical procedure in a patient affected by ovarian cancer, said outcome being a complete removal of all ovarian cancer tissue from the patient's body as a result of said surgical procedure, if said patient is classified as being affected by said first type of ovarian cancer, and said outcome being a remaining of ovarian cancer tissue in the patient's body as a result of said surgical procedure, if said patient is classified as being affected by said second type of ovarian cancer.

In one embodiment said method is for predicting the patient prognosis of a patient affected by ovarian cancer, said prognosis being related to the time from surgery to recurrent disease or death of the patient.

In one embodiment said method comprises
- providing a sample of ovarian cancer tissue of a first patient having ovarian cancer of a type unidentified in respect of its amenability to a surgical procedure,
- providing a sample of ovarian cancer tissue of a second patient having ovarian cancer of said first or said second type,
- detecting first expression levels of said nucleic acids or of said target proteins in said sample of said first patient,
- detecting second expression levels of said nucleic acids or of said target proteins in said sample of said second patient,
- comparing said first expression levels with said second expression levels of said nucleic acids or said target proteins in said sample taken from said second patient affected by ovarian cancer of said first or said second type,
- determining differences between said first and said second expression levels and classifying said first patient as having ovarian cancer belonging to the type opposite to the ovarian cancer of said second patient, if there are differences between said first and said second expression levels, or classifying said first patient as having ovarian cancer belonging to the same type of ovarian cancer as said ovarian cancer of said second patient, if there are no differences between said first and said second expression levels.

Preferably said method comprises:
- providing a sample of ovarian cancer tissue of a test patient having ovarian cancer of a type unidentified in respect of its amenability to a surgical procedure,
- providing a collection of samples of ovarian cancer tissues of patients having ovarian cancer of said first type,
- providing a collection of samples of ovarian cancer tissues of patients having ovarian cancer of said second type,
- detecting expression levels of said nucleic acids or said target proteins in said sample of said test patient,
- detecting expression levels of said nucleic acids or said target proteins in said collections of samples of ovarian cancer tissues of patients having ovarian cancer of said first and said second type, respectively,
- comparing said expression levels of said nucleic acids or said target proteins in said collection of samples of patients affected by ovarian cancer of said first type with said expression levels of said nucleic acids or said target proteins in said collection of samples of patients affected by ovarian cancer of said second type,
- fitting a classification model to said expression levels of said nucleic acids or said target proteins in said collection of samples of patients affected by ovarian cancer of said first and said second type, with machine learning algorithms, preferably with one of the following methods: support vector machines (SVM), linear discriminate analysis (LDA), or nearest-mean-classification (NMC)
- classifying said test patient as having ovarian cancer of said first type or said second type, based on said classification model.

The objects of the present invention are also solved by a kit for classifying ovarian cancer tissue as belonging to a first or a second type of two different types of ovarian cancer, said first type of ovarian cancer being characterized in that it is amenable to a surgical procedure such that after said surgical procedure there is no macroscopically detectable tumor tissue remaining in the body of a patient affected by ovarian cancer, and said second type of ovarian cancer being characterized in that it is not amenable to a surgical procedure such that after said surgical procedure there is a macroscopically detectable tumor tissue remaining in the body of a patient affected by ovarian cancer, said kit comprising a nucleic acid composition, and/or a matrix, and/or a composition of antibodies according to the present invention.

Preferably said kit further comprising reagents for performing a hybridization reaction and/or reagents for performing an antigen-antibody-reaction.

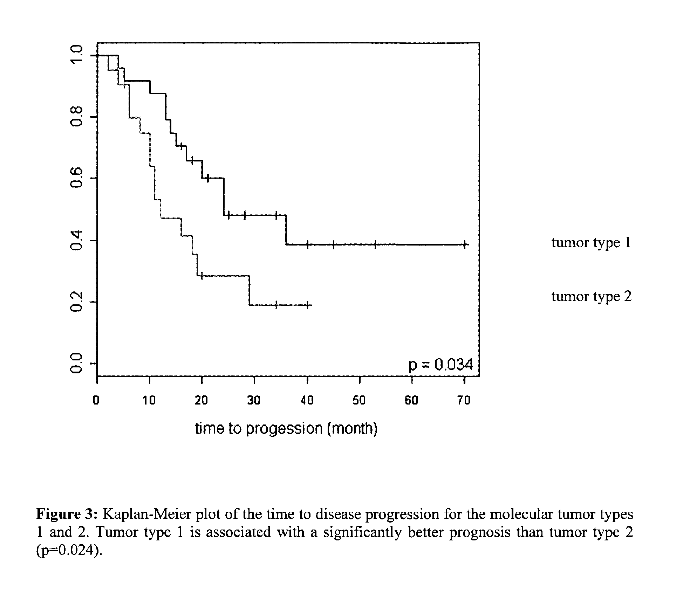

To reach acceptable prediction accuracies for a surgical outcome, the present inventors developed a new analysis approach that includes supervised as well unsupervised methods. The new strategy led to the successful identification of two well-separated molecular types (called type 1 and type 2) of ovarian tumors (figurel), characterized by a molecular signature represented by the a forementioned composition of nucleic acids in accordance with the present invention. Further, strong correlations are present between the molecular type of an ovarian tumor (type 1 or type 2) and the success of a surgical intervention, see table 3.
The present invention deals with the determination of different tumor types in ovarian cancer that can be used for prediction of accuracy of surgical procedures as well as for estimation of patient prognosis in ovarian cancer. Indeed, ovarian tumors of molecular type 1 and type 2 have a different prognosis as they differ in the time to a progression of the cancer disease (figure 3).

In this application, sometimes reference is made to "partial sequences thereof comprising 18 contiguous nucleotides". This term is herein used in a construction like:

"A composition of nucleic acids comprising at least x different nucleic acids having nucleic acid sequences selected from the group comprising SEQ ID NO:1-184, and/or partial sequences thereof comprising 18 contiguous nucleotides or more". This term signifies that within the composition of nucleic acids, instead of the full length nucleic acid sequence selected from SEQ ID NO:1-184, there may be also or instead only nucleic acid molecules having partial sequences of SEQ ID NO:1-184. Such partial sequences comprise 18 contiguous nucleotides or more, as they appear in the indicated SEQ ID NO:1-184 and can therefore be easily determined by someone skilled in the art merely by inspecting the sequences as disclosed in SEQ ID NO:1-184.

The present application also sometimes makes reference in connection with said partial sequences comprising 18 contiguous nucleotides or more, to "complementary counterparts thereof hybridizing to said nucleic acids having nucleic acid sequences selected from the group comprising SEQ ID NO:1-184". This term is meant to refer to nucleic acid probes which have sequences that are complementary over their length to said nucleic acid sequences selected from the group comprising SEQ ID NO:1-184. Such nucleic acid probes are therefore capable of hybridizing to the full length nucleic acid sequences selected from the group comprising SEQ ID NO:1-184, in complementary positions. Such nucleic acid probes are typically used in hybridization experiments, in order to detect the presence or absence of a full length nucleic acid sequence in a sample, and/or the quantity thereof.

A "complementary counterpart" of a nucleic acid sequence, as used herein, is meant to signify a nucleic sequence, the sequence of which is determined by the rules of standard "Watson-Crick"-pairing, i.e. in the sense that where one nucleic acid sequence has an A, the complementary nucleic acid sequence thereto, in this position has a T or U, if it is RNA, and vice cersa. Furthermore, if one nucleic acid sequence has a G in a specific position, the complementary counterpart thereof in this position has a C, and vice versa. Hence, by following the standard A-T-pairing and the G-C-pairing, the "complementary counterparts" of a sequence can be easily determined. By definition, such a complementary counterpart is capable of hybridizing to the sequence to which it is complementary.

The term "comprising at least five different nucleic acids having nucleic acid sequences selected from the group comprising SEQ ID NO:1-184", as used herein is meant to signify that these five different nucleic acids differ in their respective sequence, wherein each sequence of each of these nucleic acids is selected from the group SEQ ID NO:1-184.

The term "matrix", as used herein, is mean to refer to any material or support capable of having nucleic acids immobilized thereon. In a preferred embodiment said matrix is a solid material, preferably a plane solid material, or beads of a solid material, having a surface on which nucleic acids may be immobilized. In a particularly preferred embodiment, such matrix is a so-called "micro array" which, basically, is a plane surface on which a number of nucleic acid sequences have been immobilized. Micro arrays are prepared by selecting probes which comprise a polynucleotide sequence, and then immobilizing such probes to a solid support or surface. The probes may comprise DNA-sequences, RNA-sequences, or co-polymer sequences of DNA and RNA. The polynucleotide sequences of the probes may also comprise DNA-and/or RNA-analogues, or combinations thereof. For example in such a micro array, the aforementioned complementary counterparts of said partial sequences may be immobilized, which therefore serve as probes, as they are capable of hybridizing to the nucleic acids having nucleic acid sequences s elected from the group comprising SEQ ID NO:1-184. The probe sequences can be synthesized enzymatically in vivo, enzymatically in vitro (e.g. by PCR), or non-enzymatically in vitro. The probe or probes used according to the present invention are preferably immobilized to a solid support which may be either porous or non-porous. Such porous or non-porous support may be a nitrocellulose membrane or nylon membrane or filter or a g lass or plastic surface, to which the polynucleotide probes are covalently attached at either their 3'-end or their 5'-end. In particularly preferred embodiments, a micro array comprises a support or surface with an ordered array of binding sites (e.g. hybridization sites) or "probes", each representing one of the nucleic acid sequences selected from the group comprising SEQ ID NO:1-184 and/or the complementary counterparts thereof, and/or partial sequences thereof comprising 18 contiguous nucleotides or more and/or the complementary counterparts thereof hybridizing to said nucleic acids having nucleic acid sequences selected from the group comprising SEQ ID NO:1-184. Preferably, the micro arrays are addressable arrays, and more preferably positionally addressable arrays. More specifically, each probe of the array is preferably located at a known, predetermined position on the solid support such that the identity (i.e. sequence) of each probe can be determined from its position in the array (i.e. on the support or surface). In preferred embodiments, each probe is covalently attached to the solid support at a single site.

Micro arrays share certain characteristics. The arrays are reproducible, allowing multiple copies of a given array to be produced and easily compared with each other. Preferably, micro arrays are made from materials that are stable under binding (e.g. nucleic acid hybridization) conditions. The micro arrays are preferably small, e.g. between 1 cm² and 25 cm², between 12 cm² and 13 cm² or 3 cm². However, larger arrays are also contemplated and may be preferable, e.g. for use in screening arrays. Preferably a given binding site or unique set of binding sites in the micro array will specifically bind (e.g. hybridize) to the product of a single gene in a cell (e.g. to a specific mRNA, or to a specific cDNA derived therefrom). However, other related or similar sequences might cross hybridize to a given binding site.

The micro arrays of the present invention include one or more test probes, each of which has a polynucleotide sequence that is complementary to a subsequence of RNA or DNA to be detected. Preferably, the position of each probe on the solid surface is known. Indeed, the micro arrays are preferably positionally addressable arrays. Specifically, each probe of the array is preferably located at a known, predetermined position on the solid support such that the identity (i.e. sequence) of each probe can be determined from its position on the array (i.e. on the support or surface).

According to the invention, the micro array is an array (i.e. a matrix) in which each position represents one of the sequences of the composition described herein. For example, each position can contain a DNA or DNA analogue based on genomic DNA to which a particular RNA or cDNA transcribed from that genetic marker can specifically hybridize. The DNA or DNA analogue can be e.g. a synthetic oligomer or a gene fragment. In one embodiment, probes representing each of the nucleic acids comprised in the composition according to the present invention, is present on the array. In a preferred embodiment, the array comprises at least five different nucleic acids having nucleic acid sequences selected from the group comprising SEQ ID NO:1-184 and/or the complementary counterparts thereof, and/or partial sequences thereof comprising 18 contiguous nucleotides or more and/or the complementary counterparts thereof hybridizing to said nucleic acid having nucleic acid sequences selected from the group comprising SEQ ID NO:1-184, preferably 10, more preferably 20, even more preferably 50, yet more preferably 100, and most preferably all 184 of such different nucleic acids.

Methods of preparing micro arrays according to the present invention are for example disclosed in WO 02/103320, the disclosure of which is incorporated herein in its entirety by reference thereto.

The term "a composition of nucleic acids" is not limited to a particular state of the nucleic acids contained therein. As used herein such term is meant to signify that such nucleic acid composition may be e.g. in a liquid (i.e. solution) form or a solid (e.g. freeze dried) form, and the nucleic acids of such composition may be freely diffusing in such composition or may be covalently attached to other entities, e.g. a surface of a matrix, such as a microarray, which surface/support then also forms parts of the composition. The term "composition" is merely meant to denote the fact that the nucleic acids comprised therein are considered as one entity/combination. A matrix having all nucleic acids belonging to such composition immobilised thereon consequently has the composition of nucleic acids immobilised thereon.

The term "said first type of ovarian cancer is amenable to a surgical procedure" is meant to signify that, after said surgical procedure, there is no macroscopically detectable tumor tissue remaining in the body of a patient affected by ovarian cancer, i.e. said first type of ovarian cancer has a high likelihood of being successfully operated on. In contrast thereto, "said second type of ovarian cancer is not amenable to a surgical procedure", means that, after such surgical procedure, there is a macroscopically detectable tumor tissue remaining in the body of a patient affected by ovarian cancer, i.e. such second type of ovarian cancer does not have a high likelihood of being successfully operated on. "Amenability to a surgical procedure" therefore is a measure for a cancer being likely to be healed or at least alleviated by a surgical procedure alone.

The term "expression level of a nucleic acid" or "of a target protein", as used herein, is meant to signify a quantitative measure for how much a nucleic acid/target protein is expressed. Such quantitative measure may be in concentration units or may be in reading units (e.g. fluorescence) from readings taken from a micro array. In this respect, expression of a nucleic acid may be measured at the mRNA stage or the cDNA stage, reversely transcribed from such mRNA, or may be at the protein stage. If expression level is measured at the mRNA/cDNA stage, this is usually done by performing nucleic acid hybridization experiments using a matrix, e.g. a micro array, in accordance with the present invention. If an expression level is measured at the protein stage, this is usually done by detecting the expressed protein(s) using an antibody (antibodies) against such protein(s) and performing an antigen-antibody-reactions, e.g. in an immunohistochemical staining. Such immunohistochemical assays suitable for performing the present invention are known to someone skilled in the art and are for example described in Slamon et al. Science, 1989, 244, 707-712.

Sometimes herein reference is made to a "target protein" comprised in a protein composition according to the present invention. Such term "target protein" is meant to signify any protein within such protein composition to which an antibody within an antibody composition according to the present invention is directed. As an example, if the protein composition according to the present invention comprises 83 different proteins, each of these 83 different proteins may be considered a target protein, provided a corresponding antibody exists and is used in an antibody composition according to the present invention, so as to detect and, possibly, quantify the presence of such target protein in a sample of (ovarian or other) cancer tissue for which the amenability to surgical procedure or the susceptibility towards treatment by an anti-cancer-drug is to be predicted.

Methods for raising antibodies against target proteins are known by those skilled in the art and are for example described in Barlow et al. Clin Obstet Gynecol 1983, 10, 187-196 which is hereby incorporated herein by reference in its entirety.

The term "fold change" refers to the comparison between two expression levels as described above and is a measure for the difference between such two expression levels. It indicates the factor by which one would have to multiply one expression level so as to arrive at the second expression level. For example a fold change of 2.75 means that one expression level is 2.75 times bigger than the other expression level. A fold change of -4.3 means that one expression level is 4.3 times smaller than the other expression level.

The terms "ovarian cancer" and "ovarian carcinoma", as used herein, are used synonymously throughout the text. These terms are meant to include both primary tumors, i.e. tumors appearing for the first time, and recurrent tumors, i.e. tumors that appear or reappear after a patient has been operated on and/or undergone chemotherapy. Hence the present invention is suitable for both forms, i.e. primary and recurrent.
The term "recurrent disease" or "recurrence of disease", as used herein, is meant to refer to a first clinical or pathological evidence of tumor manifestation after an anti-cancer-therapy, preferably after surgery. Clinical evidence may e.g. be an elevation of tumor markers such as CA125, or the development of new masses in radiographic imaging. Pathological evidence may e.g. be the demonstration of tumor cells in tissue biopsies obtained after the initial anti-cancer therapy, e.g. surgery.
A "primary ovarian carcinoma", as used herein, refers to the first manifestation of avarian carcinoma in a patient who has not had evidence of the disease before.
A "recurrent ovarian carcinoma", as used herein, refers to a second, third, fourth etc. manifestation of ovarian cancer in a patient who has been diagnosed and treated for ovarian carcinoma before.

In a first aspect according to the present invention, the present inventors have identified a number of genes/gene markers (SEQ ID NO:1-184) and their respective protein products (SEQ ID NO:185-368) which are particularly useful for classifying tissue from an ovarian cancer tumor into two types that differ from each other in terms of their operability by surgical procedures, suggesting that these two types of ovarian cancer tissues/tumor tissues differ at a molecular level and have their own molecular signature, hence also the term "molecular type" which is sometimes used herein. The first type of such two types of ovarian cancer tissue is amenable to surgical procedures, i.e. it can be operated on with a high likelihood of no tumor tissue remaining in a patient's body after such surgical procedure, and with a high likelihood of a complete remission of the disease. In contrast thereto, the second type of ovarian cancer/tumor tissue is characterized by a low likelihood of being successfully operated on, i.e. after a surgical procedure, there will be tumor tissue remaining in a patient's body, and there will be no complete remission of the disease using a surgical procedure alone. In a second aspect, the two types of ovarian cancer differ also from each other in that, should a chemotherapy be necessary, either upon recurrence of the disease or at the primary outbreak, the two types may be treated or may require different types of chemotherapy, the first type of tumor tissue requiring standard chemotherapy, alone or possibly in combination with a surgical intervention, the second type of tumor tissue requiring modified chemotherapy, alone or possibly in combination with a surgical intervention.

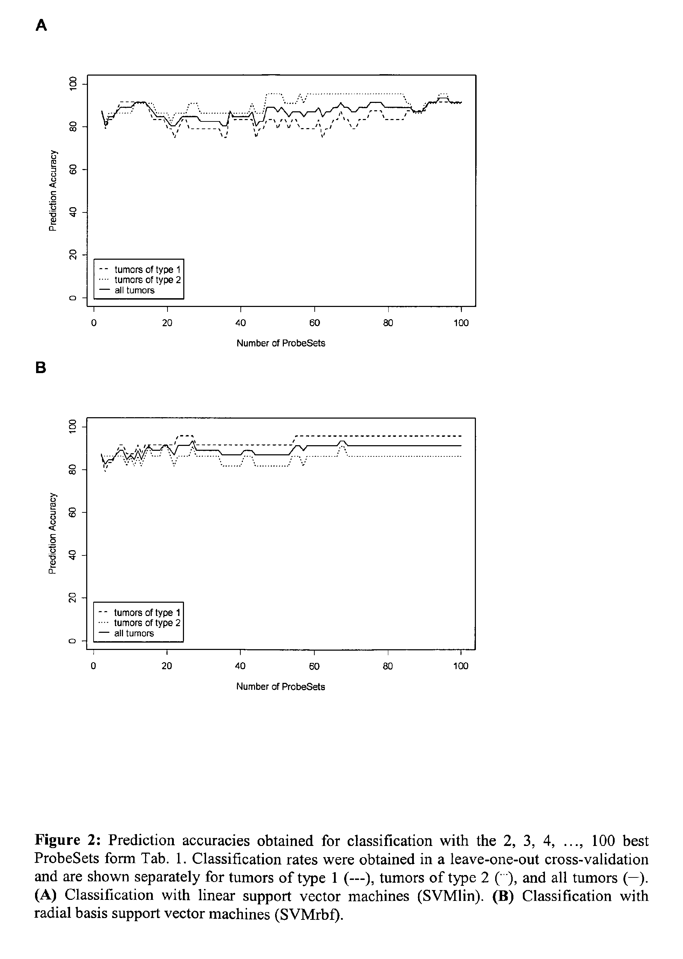

In theory, any of the 184 nucleic acid sequences may serve as a marker for classifying tumor tissue into these two types. Using any of these nucleic acid sequences as a single marker results in a successful predictability/classification rate of >80% and with some sequences and combinations, it is even greater 90%, in accordance with figure 2. Therefore, the >80% rate may be increased, when using 2, 3, 5, 10, 20, 50, 100, or even all 184 nucleic acid sequences (and/or the complementary counterparts thereof, and/or partial sequences thereof comprising 18 contiguous nucleotides or more and/or the complementary counterparts thereof hybridizing to said nucleic acids having nucleic acid sequences selected from the group comprising SEQ ID NO:1-184) for classifying ovarian cancer tissue of an unknown type and predicting its operability. In some of these cases, a successful prediction/classification rate of 90% or more can be reached, see figure 2.

As used herein, the terms "ovarian cancer (tumor) of a first type" and "ovarian cancer tumor type 1" are used synonymously throughout the text. The same applies to "ovarian cancer (tumor) of a second type" and "ovarian cancer tumor type 2"

Without wishing to be bound by any theory, the present invention is a real and substantial improvement compared to Berchuck et al., as the present inventors have used a completely different approach. Berchuck et al. first used clinical data to define both groups of tumors and then, in a second step, used supervised statistics to search for differential expressed genes in both predefined groups. This approach appears to have failed due to the fact that, as the present inventors found, clinical data may contain many mis classifications. Furthermore, it may well be that a skilled surgeon in a large operative center may be able to successfully remove a tumor, while the same tumor cannot be removed by another surgeon with less experience. Therefore, the clinical data when used first and separately can not be used as a sole basis for definition of tumor classes. In the academic discussions Berchuck et al.'s approach was generally judged to be unsuccessful, there have been no further validating studies published.
In contrast thereto, the present invention's approach was completely different. The present inventors did not compare two poorly defined clinical classes of tumors, but, in a first step, started to search for different molecular types of tumors by unsupervised statistics, that can be clearly separated by molecular analysis without the use of clinical information. Only ina second, subsequent step the molecular classes were then correlated with clinical data. The results achieved in terms of the molecular signature are clearly different, and this can also be seen by the imbalance of the prediction in both types of tumors shown in table 3. In the group of tumors that are predicted to be difficult to remove surgically by the molecular data, there is however a relevant subset of cases where the skilled surgeon at the university hospital has been able to do successful surgery. In the other group, which is predicted to be easy to remove by molecular data, all but one case have had successful surgery. This imbalance on one hand reflects the clinical situation, but on the other hand validates the present inventors' findings as opposed to the approach taken by Berchuck et al.
The present inventors have used Berchuck's statistical approach as an alternative method for methodological comparisons, and have not been able to get reliable predictive values therefrom The differences in both methods are also visible from the fact that the gene lists generated are completely different.

The results of the present inventors' finding in respect of the individual marker nucleic acids can be summarized in the following tables 1 and 2, sorted by SEQ ID in Table 2, which show the identity of the nucleic acid, its Affymetrix-probe-database entry or entries (ProbeSetID), its GeneID at the Entrez Gene database of the National Center for Biotechnology Information (NCBI), the fold change indicating how much such nucleic acid is differentially upregulated (positive numbers) or downregulated (negative numbers) when comparing one type of ovarian cancer tumor with the other type of ovarian cancer tumor, the threshold (as defined further below), its accuracy of a successful prediction/classification, when using such nucleic acid alone, its genomic location (Cytoband), and the identity of the corresponding protein sequence. Moreover the correlation between success of a surgical intervention and type of tumor is demonstrated in table 3 below.

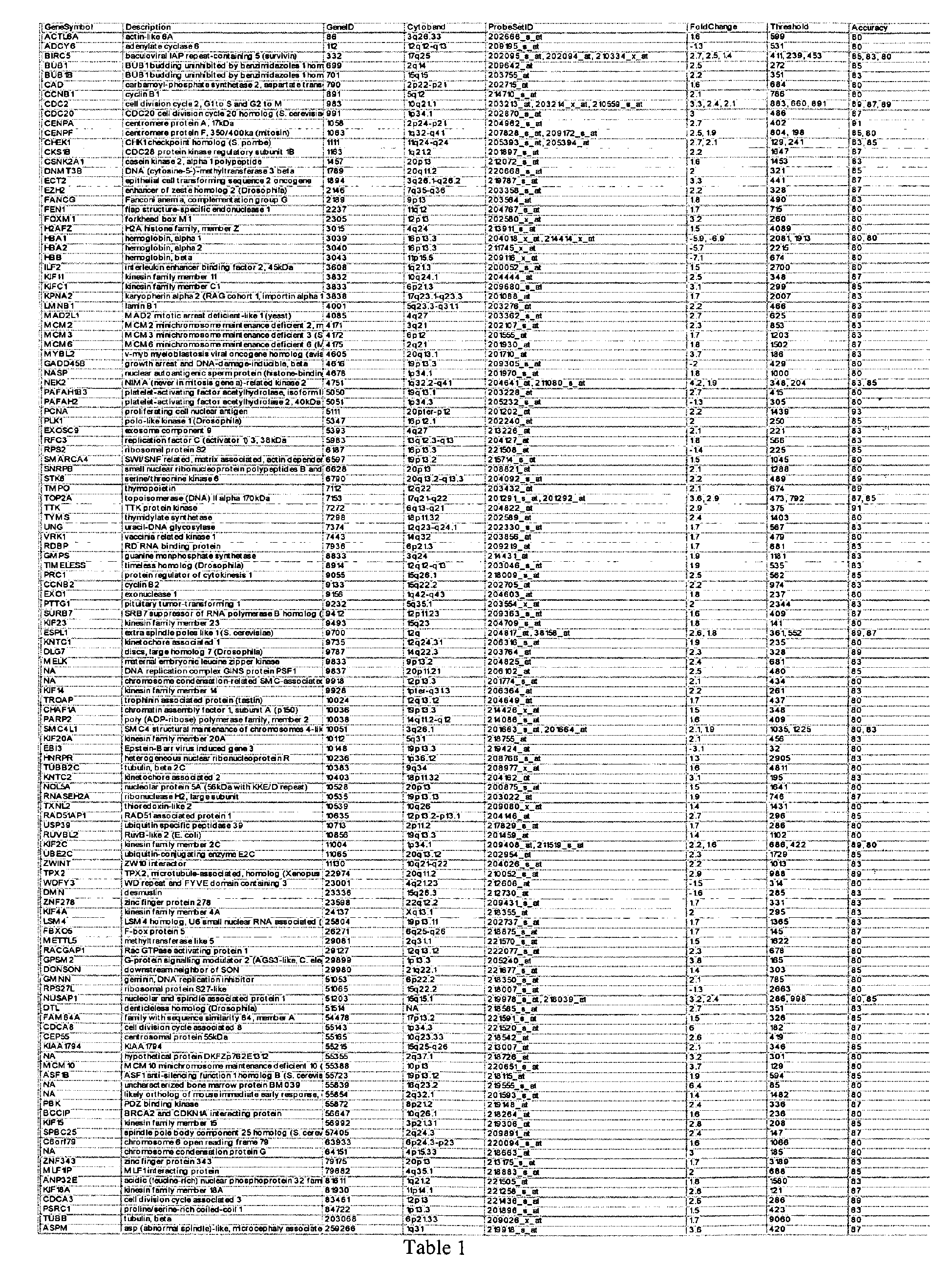

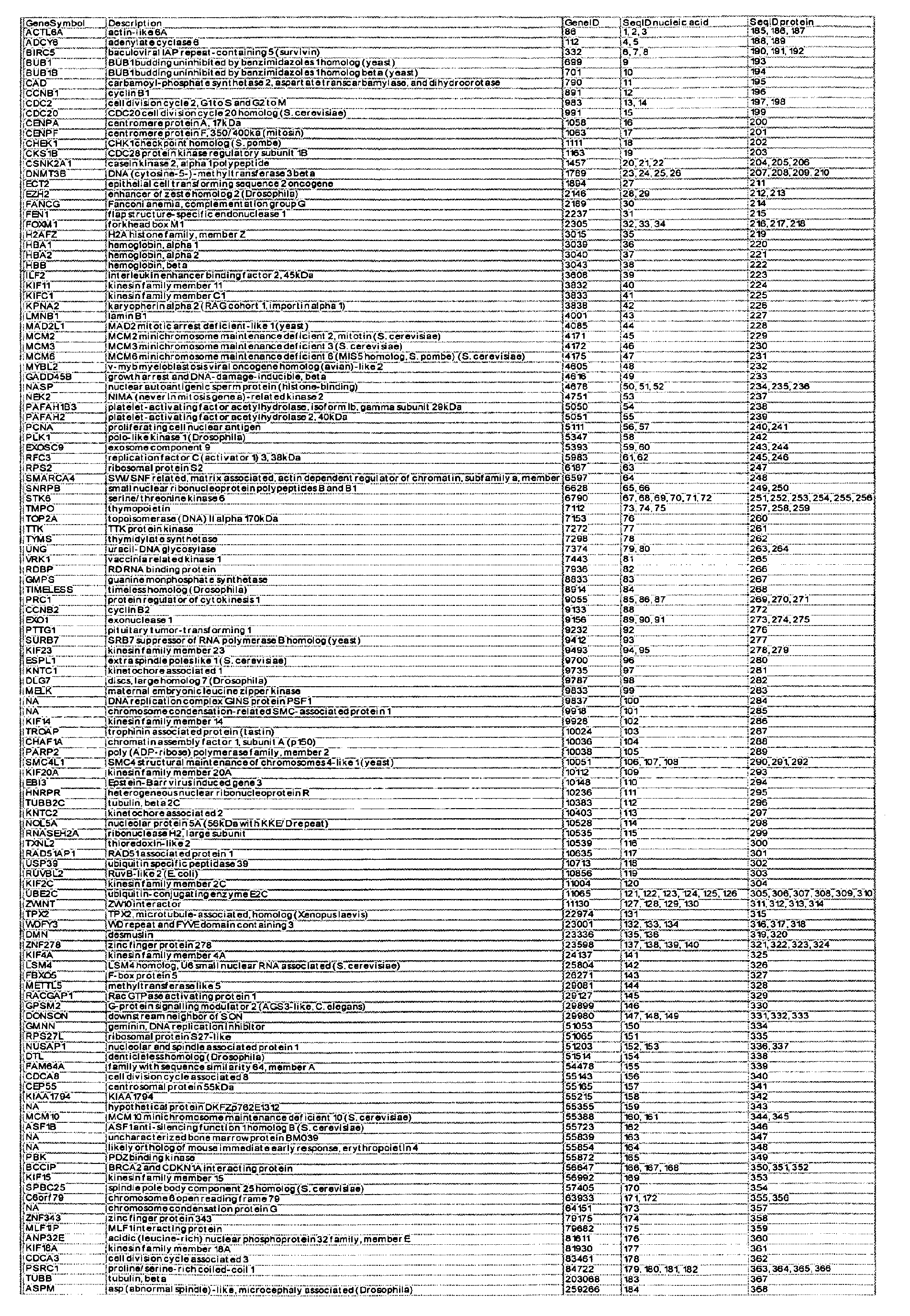

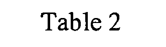

More specifically, Table 1: shows a list of 125 marker genes for the distinction between tumor type 1 and tumor type 2. Mapping of the marker genes to the corresponding Affymetrix ProbeSetIDs. Marker ProbeSetIDs were identified by assuming single-gene classification accuracies ≥ 80%. For each probe set, the fold change between tumors of type 1 and type 2, a threshold expression level, and the classification accuracy are shown.

The threshold expression level is the average of the mean expression level in tumors of type 1 and the mean expression level in tumors of type 2 calculated on the logarithmic scale and exponented back.

In accordance with the present invention, the threshold expression levels can be useful for classification of a new, unidentified ovarian cancer tumor as type 1 or type 2: If the considered gene is up-regulated between the tumors of type 1 and type 2 (positive fold change), the new tumor is considered to be of type 1, if the gene's measured expression level is greater than the threshold, and the tumor is considered to be of type 2, if the gene's measured expression level is less than the threshold. If the considered gene is down-regulated between the tumors of type 1 and type 2 (negative fold change), the new tumor is considered to be of type 1, if the gene's measured expression level is less than the threshold, and the tumor is considered to be of type 2, if the gene's measured expression level is greater than the threshold. Similar considerations apply to combinations of genes/proteins, such as combinations of a least 2, 3, 5, 10, 20, 50, 100 or 184 sequences selected from SEQ ID NO:1 - 184, and 185 - 368, respectively.

The single-gene classification accuracy is defined as follows: If the gene is up-regulated in tumors of type 1 compared to tumors of type 2, the number of tumors of type 1 with expression levels above the threshold and the number of tumors of type 2 with expression levels below the threshold is calculated. The sum of these both number divided by the total number of tumors is the classification accuracy. If the gene is down-regulated in tumors of type 1 compared to tumors of type 2, the number of tumors of type 1 with expression levels below the threshold and the number of tumors of type 2 with expression levels above the threshold is calculated. Again, the sum of these both number divided by the total number of tumors is the classification accuracy.

Table 2: shows a list of the same 125 marker genes as in table 1 for the distinction of tumor type 1 and tumor type 2, wherein the marker genes are mapped to the corresponding 184 nucleotide and 184 protein sequences with SeqIDs referring to the sequence listing ("SEQ ID nucleic acid" = SEQ ID NO: 1- 184 of the nucleic acids, "SEQ ID protein" = SEQ ID NO:185 - 368 of the corresponding proteins).

It should be noted that some of the 125 marker genes appear in the databases with more than one sequence, in which case all such sequences are referred to and used in the present application. Hence there are 184 nucleic acids and 184 corresponding protein sequences cited in the sequence listing of this application, despite there only being 125 marker genes in tables 1 and 2.

Table 3: shows a significant correlation of the molecular tumor types 1 and 2 with the success of a surgical intervention (p=0.014). With a sole exception, all tumors of type 1 were amenable to the surgical procedure such that no macroscopically detectable tumor tissue ("macroscopic free") was left in the body of the patient. On the other hand, 2/3 of the tumors of type 2 could not be operated without leaving macroscopic detectable tumor tissue in the body of the patient ("tumor tissue left").

**Table 3**

| | macroscopic free | tumor tissue left | Sum |
|---|---|---|---|
| tumor type 1 | 11 | 1 | 12 |
| tumor type 2 | 6 | 9 | 15 |
| sum | 17 | 10 | 27 |

This finding correlates with the results shown in figure 3 wherein patients having ovarian cancer of type 1 show a significantly better prognosis in terms of disease progression (i.e. a longer time until disease progression) as compared to patients having ovarian cancer of type 2.

The term "to classify a patient having ovarian cancer as requiring chemotherapy" is meant to signify that such patient, if classified into this category, is in need of chemotherapy, despite a previous surgical procedure, in order to experience an alleviation ofher disease condition, preferably a healing of her disease condition.

The term "to classify a patient having ovarian cancer as requiring different types of chemotherapy based on the patient's ovarian cancer type", as used herein, is meant to signify that the classification of the patient is done on the basis of her ovarian cancer type, i.e. her having ovarian cancer of the first or the second type in accordance with the present invention.
The term "chemotherapy", as used herein, is meant to signify any therapy involving the administration of one or several anti-cancer-drugs.
The term "different types of chemotherapy", as used herein, is meant to refer to and distinguish between "standard chemotherapy for ovarian cancer" and "modified chemotherapy for ovarian cancer". A "standard chemotherapy" for ovarian cancer, as used herein, is meant to signify any chemotherapy that is accepted as the generally applicable chemotherapy in accordance with national and international guidelines for therapy of ovarian cancer. Such guidelines are e.g. developed and maintained by organizations such as "Deutsche Krebsgesellschaft", Arbeitsgemeinschaft Gynäkologische Onkologie (AGO), or the Cochrane Foundation. Currently and more specifically, the standard chemotherapy for the treatment of ovarian cancer is based on the administration of carboplatin and taxol. It is envisaged by the present invention that ovarian cancers of the first type, as defined in the present invention, due to their molecular signature as defined by the present invention may be treated by such standard chemotherapy, whatever such standard may be at the time, whereas ovarian cancers of the second type, are not amenable to such treatment and may require a modified chemotherapy. A "modified chemotherapy", as used herein, is meant to signify any chemotherapy that is not accepted as "standard chemotherapy", as defined above, and does not use a medication accepted as a standard for ovarian cancer.

The term "adjuvant chemotherapy", as used herein, is meant to signify any type of chemotherapy that is given after a surgical intervention.
The term "neoadjuvant chemotherapy", as used herein, is meant to signify any type of chemotherapy that is given before a surgical intervention.

The term "macroscopically detectable tumor tissue remaining in the body of a patient", as used herein, is meant to signify that such tumor tissue in order to be macroscopically detectable is visible by the eye of the surgeon and/or palpable by the surgeon.

A cancerous tissue is "susceptible to treatment using one or several anti-cancer-drugs", as used herein, if such cancerous tissue does not have or develop a resistance to such one or several anti-cancer-drugs. The term "susceptible to treatment using one or several anti-cancer-drugs", as used herein, is meant to be the opposite to "resistant treatment using one or several anti-cancer-drugs". "Resistance to treatment by one or several anti-cancer-drugs", as used herein, is meant to signify that a tissue-cell showing such resistance has or develops mechanisms by which the cytostatic/cytotoxic action of such one or several anti-cancer-drugs is inhibited and/or avoided and/or circumvented, and therefore such drug does not show the desired effect in such resistant cell/tissue.

The term "predicting the outcome of an anti-cancer-therapy", as used herein, is meant to signify an estimation of the chances of success of such anti-cancer-therapy with the aim of being able to judge whether or not such anti-cancer-therapy should be effected at all. Such anti-cancer-therapy may comprise surgical procedures and/or administration of one or several anti-cancer-drugs. The "administration of one or several anti-cancer-drugs", as used herein, is also sometimes referred to as chemotherapy or molecular targeted therapy and is used synonymously with this term.

The term "predicting the prognosis of a patient with ovarian cancer", as used herein, is meant to signify an estimation of the chances of recurrent disease or death of patient after cancer therapy. More specifically,it is also meant to signify an estimation of the time after a surgical procedure until recurrence of the disease and/or death of the patient. Such anti-cancer-therapy may comprise surgical procedures and/or administration of one or several anti-cancer-drugs. The "administration of one or several anti-cancer-drugs", as used herein, is also sometimes referred to as chemotherapy or molecular targeted therapy and is used synonymously with this term.
The term "cancerous tissue" is meant to signify any tissue showing signs of malign growth or behavior. The term "ovarian cancer" and "ovarian cancer tissue" is meant to signify any ovarian tissue diagnosed as or suspected of showing malign growth or behavior.

Machine learning algorithms, such as support vector machines (SVM), linear discriminate analysis (LDA) and nearest-mean-classification (NMC), are known to someone skilled in the art and are e.g. described in R.-E. Fan, P.-H. Chen, and C.-J. Lin. Working set selection using the second order information for training SVM. Journal of Machine Learning Research 6, 1889-1918, 2005; Venables, W. N. and Ripley, B. D. (2002) Modern Applied Statistics with S. Fourth edition. Springer; Wessels LF, Reinders MJ, Hart AA, Veenman CJ, Dai H, He YD, van't Veer LJ. A protocol for building and evaluating predictors of disease state based on microarray data. Bioinformatics. 2005 Oct 1;21(19):3755-62.

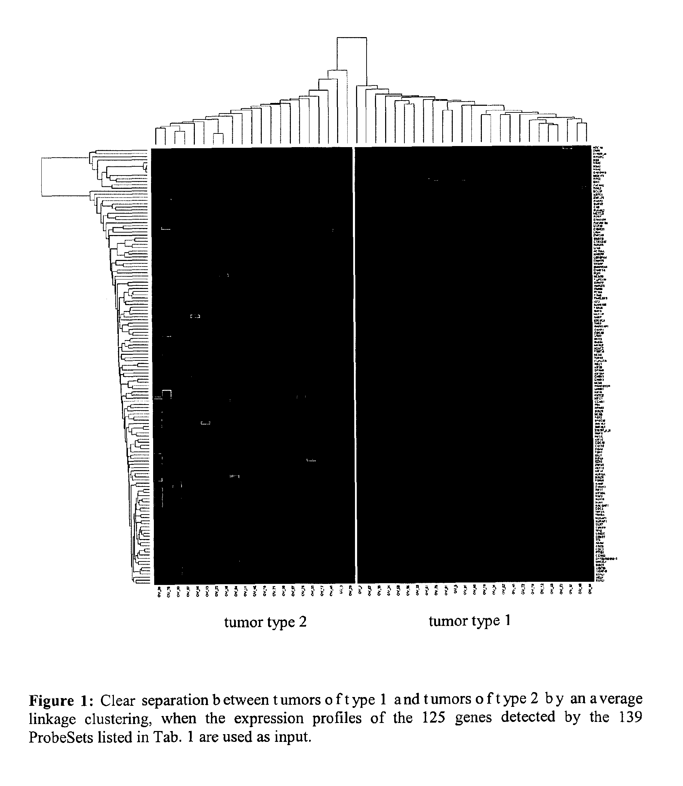

In the following, reference is made to the figures, wherein
Figure 1 shows a clustering of expression profiles of 46 ovarian carcinomas with respect to a 125 gene-signature, displaying a clear distribution into two molecularly defined tumor types. 139 ProbeSets were used for the 125 genes. Correlations between the right cluster ("tumor type 1") and the left cluster ("tumor type 2") with the success of the surgical intervention are investigated and shown in Table 3. The table includes only the 27 tumors with an advanced stage (FIGO stage III-IV) of the cancer disease and annotations about the debulking status (makroscopic free or tumor tissue left) available. In 12 cases belonging to the cluster shown on the right of the white vertical line (tumor type 1), there was no macroscopically detectable rest of tumor tissue remaining in the patient's body in all patients except for one. In the cases belonging to the cluster left of the white vertical line (tumor type 2), there was a macroscopically detectable rest of tumor tissue remaining in the patient's body in 2/3 of the 15 cases.
Figure 2 shows the prediction rates that could be obtained by classification with the measurements of 2, 3, 4, 5, ..., 100 probes for subsets of the 125 genes mentioned in Table 1.
Figure 3 shows the different prognosis of patient of both tumor groups. It can be seen from the Kaplan-Meier-plot that patients having ovarian cancer of the first type have a much better prognosis than patients having ovarian cancer of the second type. The time for diesease progression e.g. as measured by recurrence, is longer in patients having tumors of the first type, as compared to patients of the second type.

Furthermore reference is made to the sequence listing and tables wherein SEQ ID NO: 1 - 184 refer to nucleic acid sequences that are useful in accordance with the present invention, and SEQ ID NO: 185 - 368 refer to the respective proteins encoded by SEQ ID NO:1 - 184, wherein the nucleic acid having SEQ ID NO: 1 encodes the protein having SEQ ID NO: 185, the nucleic acid having SEQ ID NO: 2 encodes the protein having SEQ ID NO: 186, and so forth.

Furthermore, reference is made to the following specific description and examples which are meant to illustrate, not to limit the present invention.

### Examples

### 1. Investigation of mRNA expression profiles in ovarian carcinomas for determination of tumor types and prediction of success of surgical procedures

A collection of tumor tissue samples from primary ovarian carcinomas (n = 46) was examined with the aim of a molecular characterization of the samples in term of the gene expression (hybridization to Affymetrix-gene chips of the HG-U 133A-type). The aim of the bioinformatics analysis of the molecular data was to obtain gene signatures, the regulation of which can be correlated with the success of therapeutic measures in ovarian carcinomas. As a result, a molecular pattern, in terms of a number of nucleic acid sequences was identified, associated with the amenability of tumors towards surgical procedures (no macroscopically detectable tumor tissue in the patient's body after surgical procedure, or remaining of tumor tissue in the patient's body after surgical procedure). Tissue samples were obtained at the initial laparotomy with the aim of maximal tumor reduction. Tissue was dissected by a senior pathologist in the operating room. For histopathological diagnosis, tissue was fixed in 4% neutral buffered formaldehyde and embedded in paraffin. Routine H&E sections were performed for histopathological evaluation. The stage of tumors was assessed using the International Federation of Gynecology and Obstetrics (FIGO) staging system and the Silverberg grading system. Representative tumor tissue was immediately frozen in liquid nitrogen and stored at -80°C until analysis. Before array analysis, the tissue was histologically controlled for the presence of vital tumor cells by frozen sections and H&E staining. Only those samples containing at least 50% vital tumor cells are suitable for analysis.

RNA was extracted from tumor tissue using standard extraction procedures and the RNA quality was controlled using a bioanalyzer. There were considerable pathologic and clinical data available for all 46 tumor tissue samples, amongst others, the last date of examination with the diagnosis of a complete remission or continuation of the disease. As a starting point of the analysis, the inventors compared groups of patients with a particularly bad course of disease (progression of disease after a short period of time) with groups of patients with a particularly positive course of disease (long term remission). In this comparison, the inventors separated out the two groups of patients the expression patterns of which differed the most, i.e. in the largest number of genes. One part of the strategy of the present inventors was the identification of a statistically significant accumulation of differentially expressed genes in comparison with a zero model, which had been constructed by repeated permutation of the data. The result of this procedure were two groups of patients having substantially different molecular profiles and an associated list of differentially expressed genes. Most of the data mining was performed using the statistical language R, a programming and visualization environment that is especially useful for the analysis of high-density molecular data. In order to detect alterations between different groups of tumors, fold changes, the single-gene prediction accuracies, and p-values using Welch's t-statistics were calculated for the array data. Differential expressed genes were identified by thresholds on fold changes, single-gene prediction accuracies, p-values or combinations of these. The results of different selection procedures were validated by a permutation analysis. To this end, the data set was shuffled by 100 random permutations of the tumors and the number of identified genes was calculated for each of the permutations. Then, the observed number of modified genes between both tumor groups (nobs) was compared to the mean number that was expected from the permutation distribution (nₑₓₚ). A p-value was calculated in order to assess the significance of finding n_{obs} or more modified genes. The false discovery rate (FDR) for the generated gene list was defined as the ratio between the number of observed significant genes and the number of genes that were expected to be significant by chance (nₑₓₚ/n_{obs}).
In a next step, the present inventors grouped all 46 tissue samples by means of a clustering in relation to the expression of these genes. As a result thereof, an obvious division of the cohort of patients into two equally large clusters (n=22, and n=24) could be observed one of which happened to contain the patients having a particularly bad course of disease, and the other one containing the patients showing a particularly positive course of disease. In the last step of the analysis, this list of genes was refined by identifying the genes that were differentially expressed between these two clusters. The result is a final list of genes, the expression pattern of which is shown in figure 1, and which list of genes is explicitly shown in the tables 1 and 2 shown above.

In a subsequent examination of both tissue clusters for correlations with clinical data, it occurred to the inventors that there was a connection of the two groups with the amenability of the tumors to surgical procedures. In this examination, the present inventors limited them-selves to advanced ovarian carcinomas (FIGO stage III or IV, according to the classification of the International Federation of Gynecology and Obstetrics (FIGO). For these advanced ovarian carcinomas information was available whether or not, after surgical procedure, there was no macroscopically detectable tumor rest or, if there was, the size of the remaining tumor (n=27). One of the two patient clusters shown in figure 1 contains only patients who could be operated in such a manner that the tumor had been entirely removed, and there was no macroscopically detectable tumor rest remaining in the body of the patient. In contrast thereto, the other molecular cluster contained patients, 2/3 of which showed macroscopically detectable remaining tumors. The correlation of the molecular tissue cluster with the success of surgical procedure (entire removal vs. tumor remaining) was statistically significant (p=0.014). These results indicate that each detected tissue cluster corresponded to a different molecular tumor type, the differentiation of which is important for diagnosis and treatment of the ovarian carcinoma.

Subsequently, the present inventors examined, if and at which accuracy a prediction of a tumor type thus defined is possible by using gene expression profiles. For this purpose the investigator proceeded in two steps: first, the 2, 3, 4, 5, ..., 100 most informative probes were selected by filtering with respect to the single-gene classification power, second, machine learning algorithms, such as two variants of support vector machines (R.-E. Fan, P.-H. Chen, and C.-J. Lin. Working set selection using the second order information for training SVM. Journal of Machine Learning Research 6, 1889-1918, 2005; ), linear discriminate analysis (Venables, W. N. and Ripley, B. D. (2002) Modem Applied Statistics with S. Fourth edition. Springer), and nearest-mean-classification (Wessels LF, Reinders MJ, Hart AA, Veenman CJ, Dai H, He YD, van't Veer LJ. A protocol for building and evaluating predictors of disease state based on microarray data. Bioinformatics. 2005 Oct 1;21(19):3755-62), were applied for classification. The best results, reaching prediction rates of about 90%, were obtained with the support vector machine approache (SVMlin and SVMrbf), see figure 2.

### 2. Investigation of protein markers in ovarian carcinomas for determination of tumor types and prediction of success of surgical procedures

Formalin-fixed, paraffin-embedded tissue is used to determine the histopathological characteristics of the tumor. This tissue is further used in standard immunohistological procedures using antibodies against targets for the above mentioned gene list to determine the expression of selected markers. To increase the sensitivity, antibodies against several different markers are combined to a diagnostic set. To evaluate the specificity of the antibodies additional blocking experiments with the respective blocking peptide will be performed. Immunohistochemstry is evaluated using a scoring system that investigates the staining intensity and the percentage of positive cells. Marker expression is evaluated according to the percentage of positive cells and the intensity of staining. The percentage of positive cells is scored as: 0 (0%); 1 (<10%); 2 (10-50%); 3 (50-80%); 4 (>80%). The staining intensity is scored as: 0 (negative), 1 (weak), 2 (moderate) and 3 (strong). For the immunoreactive score (IRS) the percentage of positive cells and staining intensity were multiplicated, resulting in a value between 0 and 12. To separate cases with a low or a strong expression of the marker protein and to define a cut point that might be reproducible for future studies, cases with a IRS of 0-6 are combined to one group with negative to low merker protein expression ("marker-negative"), while cases with an IRS of 7-12 are combined into a marker-positive group. The minimum requirements for a case to be scored as positive would be either a moderate expression in more than 80% of cells or a strong expression in more than 50% of cells. By this approach it is possible to identify those patients that show a particular high marker expression in their tumor tissue. This data can be used to classify the individual tumors as being a part of group 1 or group 2 and can be used as a basis for therapeutic decisions on the extent of surgical intervention and the type of chemotherapy that is needed for adequate therapy of the patient.

### Prototypes

### 1. Prototype of micro-array

Different kinds of DNA-chips can be used for this kind of assay, for example spotted microar-rays manufactured by attaching cDNA clones or synthetic oligonucleotides to a surface (for example glas) or GeneChips® arrays (Affymetrix, Santa Clara, CA) manufactured by photolithographic in-situ synthetization of oligonucleotide probe sets onto glas slides. Assays for diagnostic purpose can be accomplished with the standard genomic GeneChips (e.g. the HG-U133A array) or with customized GeneChips offered in Affymetrix' Custom Express® Array Program or in the NimbleExpress™ Array Program. Customized GeneChips could comprise exactly the 139 probe sets listed in table 1, a subset of these probe sets or a superset of these probe sets.

Affymetrix describes the GeneChip manufracturing process as follows: GeneChip microar-rays are a classic Silicon Valley innovation, combining several disciplines to create a new technology and more useful research tool. The integration of semiconductor fabrication techniques, solid phase chemistry, combinatorial chemistry, molecular biology, and sophisticated robotics results in a unique photolithographic manufacturing process that produces GeneChip arrays with millions of probes on a small glass chip. The photolithographic process begins by coating a quartz wafer with a light-sensitive chemical compound that prevents coupling between the wafer and the first nucleotide of the DNA probe being created. Lithographic masks are used to either block or transmit light onto specific locations of the wafer surface. The surface is then flooded with a solution containing either adenine, thymine, cytosine, or guanine, and coupling occurs only in those regions on the glass that have been deprotected through illumination. The coupled nucleotide also bears a light-sensitive protecting group, so the cycle can be repeated. In this way, the microarray is built as the probes are synthesized through repeated cycles of deprotection a nd coupling. The process is repeated until the p robes reach their full length, usually 18 or more nucleotides, e.g. 25 nucleotides. Commercially available arrays are typically manufactured at a density of over 1.3 million unique features per array. Depending on the demands of the experiment and the number of probes required per array, each quartz wafer can be diced into tens or hundreds of individual arrays.

RNA isolation, preparation and hybridization can be executed according to standard protocols, as they are described for example in Gyorffy B, Surowiak P, Kiesslich O, Denkert C, Schafer R, Dietel M, Lage H.Gene expression profiling of 30 cancer cell lines predicts resistance towards 11 anticancer drugs at clinically achieved concentrations. Int J Cancer, in press. RNA is isolated from the tumor tissue using the Qiagen Rneasy Mini Kit, following the manufacturer's protocol (Qiagen GmbH, Hilden, Germany). The total isolated RNA is quantified by UV-spectroscopy and its quality is checked by analysis on a LabChip (BioAnalyzer, AGILENT Technologies, Santa Clara, CA). Samples are stored at -80°C until RNA hybridization. cDNA is synthesized from 5 g total RNA, starting with the annealing to 5 pmol/l T7-(dT)24 primer (HPLC purified, MWG-Biotech, Ebersberg, Germany) at 70°C for 10 min. Reverse transcription, second-strand synthesis and cleanup of double-stranded cDNA is performed according to the Affymetrix protocols. Synthesis of biotin-labeled cRNA is performed using the BioArray High Yield RNA Transcription kit (Enzo Diagnostics, Farmingdale, NY). cRNA concentration is determined by UV-spectroscopy and the distribution of cRNA fragment sizes are checked b y analyzing the samples on a LabChip (BioAnalyzer). The fragmented cRNA is hybridized to the HG-U133 array (Affymetrix, Santa Clara, CA) or a customized Affymetrix array designed and produced for diagnosis based on the present invention at 45°C for 16 hr. Subsequent washing and staining of the arrays is performed using the GeneChip fluidics station protocol EukGE-WS2. Finally, probe arrays are scanned using the GeneChip System confocal scanner (Hewlett-Packard, Santa Clara, CA).

### 2. Prototype of immunoassay

For this assay, paraffin-embedded formalin-fixed tissue is used. This standardized test will work with different staining protocols. The most important variables that has to be adjusted are the concentration of the antibody and the parameters of the antigen retrieval procedure. Sildes are dewaxed by incubation for 3 x 3 mins in xylene and are subsequently rehydrated for 2 x 3 min in 100% alcohol, followed by 2 x 3 min in 96% alcohol and 3 min 80% alcohol. Slides are rinsed in water. For antigen retrieval, citrate buffer (0,378g citric acid; 2,421 g Tri-Natriumcitrat-Dihydrat in 1 litre distilled water) is used. Slides are boiled in citrate puffer in a pressure cooker for 5 min, then the pressure cooker is allowed to cool slowly down. Slides are washed in running water, followed by tris-buffered saline and transferred to a staining dish. Slides are incubated with blocking solution for 5-10 m in, followed by incubation with the primary antibody at 4°C overnight. Slides are washed once in tris-buffered saline with 0.1% Tween20, followed by two wash steps with tris-buffered saline. Link solution (Super sensitive multi-link (BioGenex) is applied to cover sections and slides are incubated for 20 minsat room temperature, followed by two wash steps with tris-buffered saline, one wash step with tris-buffered saline with 0.1% Tween20 and two additional wash steps with tris-buffered saline. Sections are covered with Alkaline Phosphatase Conjugated Streptavidin solution (Bio Genex) and are incubated for 20mins at room temperature. Again one wash step with tris-buffered saline with 0.1% Tween20 and two additional wash steps with tris-buffered saline are performed. Slides are incubated with substrate until color develops (2-10 mins) and washed in water. Counterstaining is performed in haemalaun Mayer for 20 seconds, slides are rinsed in running water and mounted.
Immunohistochemical stainings are evaluated using a scoring system that has already been described above that investigates the staining intensity and the percentage of positive cells. The percentage of positive cells is scored as: 0 (0%); 1 (<10%); 2 (10-50%); 3 (50-80%); 4 (>80%). The staining intensity is scored as: 0 (negative), 1 (weak), 2 (moderate) and 3 (strong). The staining intensity of several marker proteins is combined to determine the tumor group.

The features of the present invention disclosed in the specification, the claims and/or in the accompanying drawings, may, both separately, and in any combination thereof, be material for realising the invention in various forms thereof.

## Claims

1. A composition of nucleic acids comprising at least two different nucleic acids having nucleic acid sequences selected from the group comprising SEQ ID NO:1-184 and/or the complementary counterparts thereof, and/or partial sequences thereof comprising 18 contiguous nucleotides or more and/or the complementary counterparts thereof hybridizing to said nucleic acids having nucleic acid sequences selected from the group comprising SEQ ID NO:1-184.

2. The composition of nucleic acids according to claim 1, comprising at least three different nucleic acids having nucleic acid sequences selected from the group comprising SEQ ID NO:1-184 and/or the complementary counterparts thereof, and/or partial sequences thereof comprising 18 contiguous nucleotides or more and/or the complementary counterparts thereof hybridizing to said nucleic acids having nucleic acid sequences selected from the group comprising SEQ ID NO:1-184.

3. The composition of nucleic acids according to any of the foregoing claims, comprising at least five different nucleic acids having nucleic acid sequences selected from the group comprising SEQ ID NO:1-184 and/or the complementary counterparts thereof, and/or partial sequences thereof comprising 18 contiguous nucleotides or more and/or the complementary counterparts thereof hybridizing to said nucleic acids having nucleic acid sequences selected from the group comprising SEQ ID NO:1-184.

4. The composition according to any of the foregoing claims, comprising at least 10 different nucleic acids having nucleic acid sequences selected from the group comprising SEQ ID NO:1-184 and/or the complementary counterparts thereof, and/or partial sequences thereof comprising 18 contiguous nucleotides or more and/or the complementary counterparts thereof hybridizing to said nucleic acids having nucleic acid sequences selected from the group comprising SEQ ID NO:1-184.

5. The composition according to any of the foregoing claims, comprising at least 20 different nucleic acids having nucleic acid sequences selected from the group comprising SEQ ID NO:1-184 and/or the complementary counterparts thereof, and/or partial sequences thereof comprising 18 contiguous nucleotides or more and/or the complementary counterparts thereof hybridizing to said nucleic acids having nucleic acid sequences selected from the group comprising SEQ ID NO:1-184.

6. The composition according to any of the foregoing claims, comprising at least 50 different nucleic acids having nucleic acid sequences selected from the group comprising SEQ ID NO:1-184 and/or the complementary counterparts thereof, and/or partial sequences thereof comprising 18 contiguous nucleotides or more and/or the complementary counterparts thereof hybridizing to said nucleic acids having nucleic acid sequences selected from the group comprising SEQ ID NO:1-184.

7. The composition according to any of the foregoing claims, comprising at least 100 different nucleic acids having nucleic acid sequences selected from the group comprising SEQ ID NO:1-184 and/or the complementary counterparts thereof, and/or partial sequences thereof comprising 18 contiguous nucleotides or more and/or the complementary counterparts thereof hybridizing to said nucleic acids having nucleic acid sequences selected from the group comprising SEQ ID NO:1-184.

8. The composition according to any of the foregoing claims, comprising at least 184 different nucleic acids having nucleic acid sequences selected from the group comprising SEQ ID NO:1-184 and/or the complementary counterparts thereof, and/or partial sequences thereof comprising 18 contiguous nucleotides or more and/or the complementary counterparts thereof hybridizing to said nucleic acids having nucleic acid sequences selected from the group comprising SEQ ID NO:1-184.

9. A matrix, preferably a microarray, having a surface on which a nucleic acid composition according to any of the foregoing claims has been immobilized.

10. In-vitro-use of the composition according to any of claims 1-8 and/or the matrix according to claim 9 for classifying ovarian cancer tissue as belonging to a first or second type of two different types of ovarian cancer, said first type of ovarian cancer being **characterized in that** it is amenable to a surgical procedure such that after said surgical procedure there is no macroscopically detectable tumor tissue remaining in the body of a patient affected by ovarian cancer, and said second type of ovarian cancer being **characterized in that** it is not amenable to a surgical procedure such that after said surgical procedure there is a macroscopically detectable tumor tissue remaining in the body of a patient affected by ovarian cancer.

11. In-vitro-use according to claim 10 for predicting the outcome of a surgical procedure in a patient affected by ovarian cancer, said outcome being a complete removal of all ovarian cancer tissue from the patient's body as a result of said surgical procedure, if said patient is classified as being affected by said first type of ovarian cancer, and said outcome being a remaining of ovarian cancer tissue in the patient's body as a result of said surgical procedure, if said patient is classified as being affected by said second type of ovarian cancer.

12. In-vitro-use according to any of claims 10-11 for predicting the patient prognosis of a patient affected by ovarian cancer, said prognosis being related to the time from surgery to recurrent disease or death of the patient.

13. In-vitro-use according to any of claims 10-12, comprising:
- providing a sample of ovarian cancer tissue of a first patient having ovarian cancer of a type unidentified in respect of its amenability to a surgical procedure,
- providing a sample of ovarian cancer tissue of a second patient having ovarian cancer of said first or said second type,
- detecting first expression levels of said nucleic acids in said sample of said first patient,
- detecting second expression levels of said nucleic acids in said sample of said second patient,
- comparing said first expression levels with said second expression levels of said nucleic acids in said sample taken from said second patient affected by ovarian cancer of said first or said second type,
- determining differences between said first and said second expression levels and classifying said first patient as having ovarian cancer belonging to the type opposite to the ovarian cancer of said second patient, if there are differences between said first and said second expression levels, or classifying said first patient as having ovarian cancer belonging to the same type of ovarian cancer as said ovarian cancer of said second patient, if there are no differences between said first and said second expression levels.

14. In-vitro-use according to any of claims 10-13, comprising:
- providing a sample of ovarian cancer tissue of a test patient having ovarian cancer of a type unidentified in respect of its amenability to a surgical procedure,
- providing a collection of samples of ovarian cancer tissues of patients having ovarian cancer of said first type,
- providing a collection of samples of ovarian cancer tissues of patients having ovarian cancer of said second type,
- detecting expression levels of said nucleic acids in said sample of said test patient,
- detecting expression levels of said nucleic acids in said collections of samples of ovarian cancer tissues of patients having ovarian cancer of said first and second type, respectively,
- comparing said expression levels of said nucleic acids in said collection of samples of patients affected by ovarian cancer of said first type with said expression levels of said nucleic acids in said collection of samples of patients affected by ovarian cancer of said second type,
- fitting a classification model to said expression levels of said nucleic acids in said collection of samples of patients affected by ovarian cancer of said first and said second type, with machine learning algorithms, preferably with one of the following methods: support vector machines (SVM), linear discriminate analysis (LDA), or nearest-mean-classification (NMC)
- classifying said test patient as having ovarian cancer of said first type or said second type, based on said classification model.

15. In-vitro-use according to any of claims 13-14, wherein said expression levels are detected at the mRNA-stage, cDNA stage or the protein stage.

16. In-vitro-use according to claim 13, wherein said differences are a fold change in expression level of said nucleic acids > 1.3 or < -1.2.

17. In-vitro-use according to claim 16, wherein said fold change is in the range of from 1.3 to 6.4 and/or in the range of from -1.2 to -7.2.

18. In-vitro-use according to any of claims 10-17 for classifying a patient having ovarian cancer as requiring different types of chemotherapy based on the patient's ovarian cancer type, **characterized in that** a patient having ovarian cancer of said first type may be treated by or may require standard chemotherapy, alone or in combination with a surgical procedure, said standard chemotherapy preferably being an adjuvant chemotherapy, more preferably an administration of one or several of carboplatin, taxol, even more preferably a combination of both carboplatin and taxol, and a patient having ovarian cancer of said second type may be treated or may require a modified chemotherapy, alone or in combination with a surgical procedure ,said modified chemotherapy being a chemotherapy different from said standard chemotherapy, preferably a neoadjuvant preoperative chemotherapy.

19. In-vitro-use according to any of claims 10-18 for classifying a patient having ovarian cancer as requiring different types of adjuvant therapy based on the patient's ovarian cancer type, **characterized in that** a patient having ovarian cancer of said first type may be treated by or may require standard chemotherapy after a surgical procedure, and a patient having ovarian cancer of said second type may be treated or may require an additional therapy using molecular targeted substances tailored to inhibit or upregulate target nucleic acids or proteins selected from the group comprising SEQ ID NO:1-184, and SEQ ID NO: 185-368.

20. A composition of proteins being encoded by a composition of nucleic acids according to any of claims 1-8, said nucleic acids having nucleic acid sequences selected from the group comprising SEQ ID NO:1-184, **characterized in that** said composition of proteins comprises at least two different proteins having sequences selected from the group comprising SEQ ID NO:185-368.

21. The composition according to claim 20, **characterized in that** said composition comprises at least three different proteins having sequences selected from the group comprising SEQ ID NO:185-368.

22. Composition according to any of claims 20-21, **characterized in that** said composition of proteins comprises at least 5 different proteins having sequences selected from the group comprising SEQ ID NO: 185-368.

23. Composition according to any of claims 20-22, **characterized in that** said composition of proteins comprises at least 10 different proteins having sequences selected from the group comprising SEQ ID NO: 185-368.

24. Composition according to any of claims 20-23, **characterized in that** said composition of proteins comprises at least 20 different proteins having sequences selected from the group comprising SEQ ID NO: 185-368.

25. Composition according to any of claims 20-24, **characterized in that** said composition of proteins comprises at least 50 different proteins having sequences selected from the group comprising SEQ ID NO: 185-368.

26. Composition according to any of claims 20-25, **characterized in that** said composition of proteins comprises at least 100 different proteins having sequences selected from the group comprising SEQ ID NO: 185-368.

27. Composition according to any of claims 20-26, **characterized in that** said composition of proteins comprises at least 184 different proteins having sequences selected from the group comprising SEQ ID NO: 185-368.

28. A composition of antibodies, each antibody raised against a different target protein of said protein composition according to any of claims 20-27.

29. In-vitro-use of a composition of antibodies according to claim 28 for classifying ovarian cancer tissue as belonging to a first or a second type of two different types of ovarian cancer, said first type of ovarian cancer being **characterized in that** it is amenable to a surgical procedure such that after said surgical procedure there is no macroscopically detectable tumor tissue remaining in the body of a patient affected by ovarian cancer, and said second type of ovarian cancer being **characterized in that** it is not amenable to a surgical procedure such that after said surgical procedure there is a macroscopically detectable tumor tissue remaining in the body of a patient affected by ovarian cancer.

30. In-vitro-use according to claim 29 for predicting the outcome of a surgical procedure in a patient affected by ovarian cancer, said outcome being a complete removal of all ovarian cancer tissue from the patient's body, to a macroscopically detectable extent, as a result of said surgical procedure, if said patient is classified as being affected by said first type of ovarian cancer, and said outcome being a remaining of ovarian cancer tissue in the patient's body, to a macroscopically detectable extent, as a result of said surgical procedure, if said patient is classified as being affected by said second type of ovarian cancer.

31. In-vitro-use according any of claims 29-30 for predicting the patient prognosis of a patient affected by ovarian cancer, said prognosis being related to the time from surgery to recurrent disease or death of the patient.

32. In-vitro-use according to any of claims 29-31, comprising
- providing a sample of ovarian cancer tissue of a first patient having ovarian cancer of a type unidentified in respect of its amenability to a surgical procedure,
- providing a sample of ovarian cancer tissue of a second patient having ovarian cancer of said first or said second type,
- detecting first expression levels of said target proteins in said sample of said first patient,
- detecting second expression levels of said target proteins in said sample of said second patient,
- comparing said first expression levels with said second expression levels of said target proteins in said sample taken from said second patient affected by ovarian cancer of said first or said second type,
- determining differences between said first and said second expression levels and classifying said first patient as having ovarian cancer belonging to the type opposite to the ovarian cancer of said second patient, if there are differences between said first and said second expression levels, or classifying said first patient having ovarian cancer belonging to the same type of ovarian cancer as said ovarian cancer of said second patient, if there are no differences between said first and said second expression levels.

33. In-vitro-use according to any of claims 29-32, comprising:
- providing a sample of ovarian cancer tissue of a test patient having ovarian cancer of a type unidentified in respect of its amenability to a surgical procedure,
- providing a collection of samples of ovarian cancer tissues of patients having ovarian cancer of said first type,
- providing a collection of samples of ovarian cancer tissues of patients having ovarian cancer of said second type,
- detecting expression levels of said target proteins in said sample of said test patient,
- detecting expression levels of said target proteins in said collections of samples of ovarian cancer tissues of patients having ovarian cancer of said first and second type, respectively,
- comparing said expression levels of said target proteins in said collection of samples of patients affected by ovarian cancer of said first type with said expression levels of said target proteins in said collection of samples of patients affected by ovarian cancer of said second type,
- fitting a classification model to said expression levels of said target proteins in said collection of samples of patients affected by ovarian cancer of said first and said second type, with machine learning algorithms, preferably with one of the following methods: support vector machines (SVM), linear discriminate analysis (LDA), or nearest-mean-classification (NMC)
- classifying said test patient as having ovarian cancer of said first type or said second type, based on said classification model.

34. In-vitro-use according to any of claims 29-33 for classifying a patient having ovarian cancer as requiring different types of chemotherapy based on the patient's ovarian cancer type, **characterized in that** a patient having ovarian cancer of said first type may be treated by or may require standard chemotherapy, alone or in combination with a surgical procedure, said standard chemotherapy preferably being an adjuvant chemotherapy, more preferably an administration of one or several of carboplatin, taxol, even more preferably a combination of both carboplatin and taxol, and a patient having ovarian cancer of said second type may be treated or may require a modified chemotherapy, alone or in combination with a surgical procedure, said modified chemotherapy being different from said standard chemotherapy, preferably a neoadjuvant preoperative chemotherapy.

35. In-vitro-use according to any of claims 29-34 for classifying a patient having ovarian cancer as requiring different types of adjuvant therapy based on the patient's ovarian cancer type, **characterized in that** a patient having ovarian cancer of said first type may be treated by or may require standard chemotherapy after a surgical procedure, and a patient having ovarian cancer of said second type may be treated by or may require an additional therapy using molecular targeted substances tailored to inhibit or upregulate target nucleic acids or proteins selected from the group comprising SEQ ID NO:1-184, and SEQ ID NO:185-368.

36. An in-vitro-method of classifying a patient with ovarian cancer as having ovarian cancer of a first type or a second type, said first type of ovarian cancer being **characterized in that** it is amenable to a surgical procedure such that after said surgical procedure there is no macroscopically detectable tumor tissue remaining in the body of a patient affected by ovarian cancer, and said second type of ovarian cancer being **characterized in that** it is not amenable to a surgical procedure such that after said surgical procedure there is a macroscopically detectable tumor tissue remaining in the body of a patient affected by ovarian cancer,
- using the nucleic acid composition according to any of claims 1-8, and/or the matrix according to claim 9, and/or the composition of antibodies according to claim 28.

37. The method according to claim 36 for predicting the outcome of a surgical procedure in a patient affected by ovarian cancer, said outcome being a complete removal of all ovarian cancer tissue from the patient's body as a result of said surgical procedure, if said patient is classified as being affected by said first type of ovarian cancer, and said outcome being a remaining of ovarian cancer tissue in the patient's body as a result of said surgical procedure, if said patient is classified as being affected by said second type of ovarian cancer.

38. The method according to any of claims 36-37, for predicting the patient prognosis of a patient affected by ovarian cancer, said prognosis being related to the time from surgery to recurrent disease or death of the patient.

39. The method according to any of claims 36-38, comprising
- providing a sample of ovarian cancer tissue of a first patient having ovarian cancer of a type unidentified in respect of its amenability to a surgical procedure,
- providing a sample of ovarian cancer tissue of a second patient having ovarian cancer of said first or said second type,
- detecting first expression levels of said nucleic acids or of said target proteins in said sample of said first patient,
- detecting second expression levels of said nucleic acids or of said target proteins in said sample of said second patient,
- comparing said first expression levels with said second expression levels of said nucleic acids or said target proteins in said sample taken from said second patient affected by ovarian cancer of said first or said second type,
- determining differences between said first and said second expression levels and classifying said first patient as having ovarian cancer belonging to the type opposite to the ovarian cancer of said second patient, if there are differences between said first and said second expression levels, or classifying said first patient as having ovarian cancer belonging to the same type of ovarian cancer as said ovarian cancer of said second patient, if there are no differences between said first and said second expression levels.

40. The method according to any of claims 36-39, comprising:
- providing a sample of ovarian cancer tissue of a test patient having ovarian cancer of a type unidentified in respect of its amenability to a surgical procedure,
- providing a collection of samples of ovarian cancer tissues of patients having ovarian cancer of said first type,
- providing a collection of samples of ovarian cancer tissues of patients having ovarian cancer of said second type,
- detecting expression levels of said nucleic acids or said target proteins in said sample of said test patient,
- detecting expression levels of said nucleic acids or said target proteins in said collections of samples of ovarian cancer tissues of patients having ovarian cancer of said first and said second type, respectively,
- comparing said expression levels of said nucleic acids or said target proteins in said collection of samples of patients affected by ovarian cancer of said first type with said expression levels of said nucleic acids or said target proteins in said collection of samples of patients affected by ovarian cancer of said second type,
- fitting a classification model to said expression levels of said nucleic acids or said target proteins in said collection of samples of patients affected by ovarian cancer of said first and said second type, with machine learning algorithms, preferably with one of the following methods: support vector machines (SVM), linear discriminate analysis (LDA), or nearest-mean-classification (NMC)
- classifying said test patient as having ovarian cancer of said first type or said second type, based on said classification model.

41. A kit for classifying ovarian cancer tissue as belonging to a first or a second type of two different types of ovarian cancer, said first type of ovarian cancer being **characterized in that** it is amenable to a surgical procedure such that after said surgical procedure there is no macroscopically detectable tumor tissue remaining in the body of a patient affected by ovarian cancer, and said second type of ovarian cancer being **characterized in that** it is not amenable to a surgical procedure such that after said surgical procedure there is a macroscopically detectable tumor tissue remaining in the body of a patient affected by ovarian cancer, said kit comprising a nucleic acid composition according to any of claims 1-8 and/or a matrix according to claim 9 and/or a composition of antibodies according to claim 28.

42. The kit according to claim 41 further comprising reagents for performing a hybridization reaction and/or reagents for performing an antigen-antibody-reaction.
